# EUROPEAN PATENT SPECIFICATION

(11) **EP 3 976 604 B1**
(45) Date of publication and mention of the grant of the patent: **21.01.2026**
(21) Application number: 20814548.2
(22) Date of filing: 29.05.2020
(51) Int. Cl.: C07D 413/10, C07D 413/06, C07D 417/14, A61K 31/454, A61K 31/5377, A61K 31/444, A61K 31/517, A61K 31/4245, A61K 31/427, A61P 35/00

(54) **1,3,4-OXADIAZOLE DERIVATIVE COMPOUNDS AS HISTONE DEACETYLASE 6 INHIBITOR, AND THE PHARMACEUTICAL COMPOSITION COMPRISING THE SAME**
1,3,4-OXADIAZOL-DERIVATVERBINDUNGEN ALS HISTON-DEACETYLASE-6-HEMMER UND PHARMAZEUTISCHE ZUSAMMENSETZUNG DAMIT
COMPOSÉS DÉRIVÉS DE 1,3,4-OXADIAZOLE UTILISÉS COMME INHIBITEURS D'HISTONE DÉSACÉTYLASE 6, ET COMPOSITION PHARMACEUTIQUE LES COMPRENANT

(30) Priority: 31.05.2019 KR 20190064665
(43) Date of publication of application: 06.04.2022
(73) Proprietor: Chong Kun Dang Pharmaceutical Corp., Seoul 03742 (KR)
(72) Inventor: LEE, Chang Sik, Yongin-si Gyeonggi-do 16995 (KR); OH, Jung Taek, Yongin-si Gyeonggi-do 16995 (KR); YUN, Hokeun, Yongin-si Gyeonggi-do 16995 (KR); SONG, Hyeseung, Yongin-si Gyeonggi-do 16995 (KR); KIM, Hyunjin Michael, Yongin-si Gyeonggi-do 16995 (KR)
(74) Representative: Mewburn Ellis LLP
(86) International application number: PCT/IB2020/055109
(87) International publication number: WO 2020/240492

(56) References cited:
- WO-A1-2012/136492
- WO-A1-2017/222951
- US-A1- 2015 307 497
- US-A1- 2016 244 449
- US-A1- 2017 145 012
- US-A1- 2018 215 743

## Description

### Technical Field

The present invention relates to 1,3,4-oxadiazole derivative compounds having a histone deacetylase 6 (HDAC6) inhibitory activity, stereoisomers thereof, pharmaceutically acceptable salts thereof, a use thereof in preparation of a medicament, a pharmaceutical composition comprising the same, a therapeutic method using the composition, and a method for preparing the same.

### Background

In cells, a post-translational modification such as acetylation serves as a very important regulatory module at the hub of biological processes and is also strictly controlled by a number of enzymes. As a core protein constituting chromatin, histone functions as an axis, around which DNA winds, and thus helps a DNA condensation. Also, a balance between acetylation and deacetylation of histone plays a very important role in gene expression.

As an enzyme for removing an acetyl group from lysine residue of histone protein, which constitutes chromatin, histone deacetylase (HDAC) is known to be associated with gene silencing and induce a cell cycle arrest, angiogenic inhibition, immunoregulation, apoptosis, etc. (Hassig et al., Curr. Opin. Chem. Biol. 1997, 1, 300-308). Also, it is reported that the inhibition of HDAC enzyme functions induces cancer cells into committing apoptosis for themselves by lowering an activity of cancer cell survival-related factors and activating cancer cell death-related factors in the body (Warrell et al., J. Natl. Cancer Inst. 1998, 90, 1621-1625).

For humans, 18 HDACs are known and classified into four classes according to homology with yeast HDAC. In this case, eleven HDACs using zinc as a cofactor may be divided into three groups: Class I (HDAC1, 2, 3, 8), Class II (lla: HDAC4, 5, 7, 9; Ilb: HDAC6, 10) and Class IV (HDAC11). Further, seven HDACs of Class III (SIRT 1-7) use NAD+ as a cofactor instead of zinc (Bolden et al., Nat. Rev. Drug Discov. 2006, 5(9), 769-784).

Various HDAC inhibitors are now in a preclinical or clinical development stage, but only non-selective HDAC inhibitors have been known as an anti-cancer agent so far. Vorinostat (SAHA) and romidepsin (FK228) have obtained an approval as a therapeutic agent for cutaneous T-cell lymphoma, while panobinostat (LBH-589) has won an approval as a therapeutic agent for multiple myeloma. However, it is known that the non-selective HDAC inhibitors generally bring about side effects such as fatigue, nausea and the like at high doses (Piekarz et al., Pharmaceuticals 2010, 3, 2751-2767). It is reported that the side effects are caused by the inhibition of class I HDACs. Due to the side effects, etc., the non-selective HDAC inhibitors have been subject to restriction on drug development in other fields than an anticancer agent (Witt et al., Cancer Letters 277 (2009) 8.21).

Meanwhile, it is reported that the selective inhibition of class II HDACs would not show toxicity, which have occurred in the inhibition of class I HDACs. In case of developing the selective HDAC inhibitors, it would be likely to solve side effects such as toxicity, etc., caused by the non-selective inhibition of HDACs. Accordingly, there is a chance that the selective HDAC inhibitors may be developed as an effective therapeutic agent for various diseases (Matthias et al., Mol. Cell. Biol. 2008, 28, 1688-1701).

HDAC6, one of the class Ilb HDACs, is known to be mainly present in cytoplasma and contain a tubulin protein, thus being involved in the deacetylation of a number of non-histone substrates (HSP90, cortactin, etc.) (Yao et al., Mol. Cell 2005, 18, 601-607). HDAC6 has two catalytic domains, in which a zinc finger domain of C-terminal may bind to an ubiquitinated protein. HDAC6 is known to have a number of non-histone proteins as a substrate, and thus play an important role in various diseases such as cancers, inflammatory diseases, autoimmune diseases, neurological diseases, neurodegenerative disorders and the like (Santo et al., Blood 2012 119: 2579-2589; Vishwakarma et al., International Immunopharmacology 2013, 16, 72-78; Hu et al., J. Neurol. Sci. 2011, 304, 1-8).

A structural feature that various HDAC inhibitors have in common is comprised of a cap group, a linker and a zinc binding group (ZBG) as shown in a following structure of vorinostat. Many researchers have conducted a study on the inhibitory activity with regard to enzymes and selectivity through a structural modification of the cap group and the linker. Out of the groups, it is known that the zinc binding group plays a more important role in the enzyme inhibitory activity and selectivity (Wiest et al., J. Org. Chem. 2013 78: 5051-5065; Methot et al., Bioorg. Med. Chem. Lett. 2008, 18, 973-978).

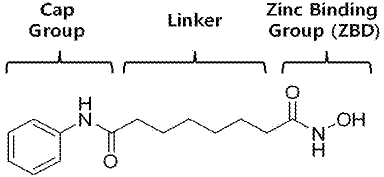

Most of said zinc binding group is comprised of hydroxamic acid or benzamide, out of which hydroxamic acid derivatives show a strong HDAC inhibitory effect, but have a problem with low bioavailability and serious off-target activity. Benzamide contains aniline and thus has a problem in that it may produce toxic metabolites in vivo (Woster et al., Med. Chem. Commun. 2015, online publication).

Accordingly, to treat cancers, inflammatory diseases, autoimmune diseases, neurological diseases, neurodegenerative disorders and the like, there is a need to develop a selective HDAC6 inhibitor which has a zinc binding group with improved bioavailability, while causing no side effects unlike the non-selective inhibitors having side effects.

### [Related Art Reference]

### [Patent Document]

International Patent Publication No. WO 2011/091213 (publicized on Jul. 28, 2011): ACY-1215
International Patent Publication No. WO 2011/011186 (publicized on Jan. 27, 2011): Tubastatin
International Patent Publication No. WO 2013/052110 (publicized on Apr. 11, 2013): Sloan-K
International Patent Publication No. WO 2013/041407 (publicized on Mar. 28, 2013): Cellzome
International Patent Publication No. WO 2013/134467 (publicized on Sep. 12, 2013): Kozi
International Patent Publication No. WO 2013/008162 (publicized on Jan. 17, 2013): Novartis
International Patent Publication No. WO 2013/080120 (publicized on Jun. 06, 2013): Novartis
International Patent Publication No. WO 2013/066835 (publicized on May 10, 2013): Tempero
International Patent Publication No. WO 2013/066838 (publicized on May 10, 2013): Tempero
International Patent Publication No. WO 2013/066833 (publicized on May 10, 2013): Tempero
International Patent Publication No. WO 2013/066839 (publicized on May 10, 2013): Tempero International Patent Publication WO2017/222951 (publicized on December 28, 2017); MSD

**Detailed Description of the Invention** Any references to methods of treatment in the subsequent paragraphs of this description are to be interpreted as references to the compounds, pharmaceutical compositions and medicaments of the present invention for use in a method for treatment of the human (or animal) body by therapy (or for diagnosis).

### Technical Problem

An objective of the present invention is to provide 1,3,4-oxadiazole derivative compounds having a selective HDAC6 inhibitory activity, stereoisomers thereof or pharmaceutically acceptable salts thereof.

Another objective of the present invention is to provide a pharmaceutical composition comprising 1,3,4-oxadiazole derivative compounds having a selective HDAC6 inhibitory activity, stereoisomers thereof or pharmaceutically acceptable salts thereof.

Still another objective of the present invention is to provide a method for preparing the same.

Still another objective of the present invention is to provide a pharmaceutical composition comprising said compounds for preventing or treating HDAC6 activity-related diseases including cancers, inflammatory diseases, autoimmune diseases, neurological diseases or neurodegenerative disorders.

Still another objective of the present invention is to provide a use thereof for preventing or treating HDAC6 activity-related diseases.

Still another objective of the present invention is to provide a use thereof in preparation of a medicament for preventing or treating HDAC6 activity-related diseases.

Still another objective of the present invention is to provide a method for treating HDAC6 activity-related diseases, comprising administering a therapeutically effective amount of a pharmaceutical composition comprising said compounds.

### Technical Solution

The present inventors have found 1,3,4-oxadiazole derivative compounds having a histone deacetylase 6 (HDAC6) inhibitory activity and have used the same in preventing or treating HDAC6 activity-related diseases, thereby completing the present invention.

### 1,3,4-oxadiazole derivative compounds

The 1,3,4-oxadiazole derivative compounds of the present invention, stereoisomers thereof or pharmaceutically acceptable salts thereof are represented by a following chemical formula I: 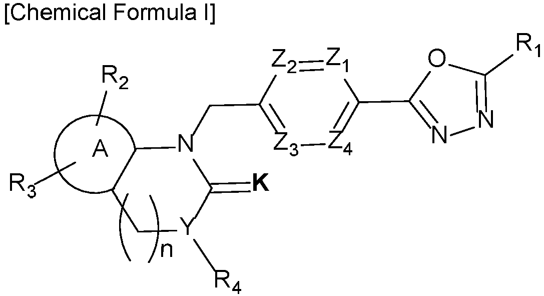 wherein,
Z₁ to Z₄ are each independently N or CRₐ, in which Rₐ is H, X, C₁-C₄ alkyl or O-(C₁-C₄ alkyl) and Rₐ can be different from each other when CRₐ is 2 or more;
K is O or S;
R₁ is CX₃ or CX₂H; 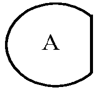 is C₆-C₁₂ arylene or C₂-C₁₀ heteroarylene;
R₂ and R₃ are each independently H, X, C₁-C₄ alkyl, C₁-C₄ haloalkyl, C₆-C₁₂ aryl, C₂-C₁₀ heteroaryl, C₃-C₁₀ cycloalkyl, C₂-C₁₀ heterocycloalkyl, C₂-C₁₀ heterocycloalkenyl, N(R_{b})(R_{c}), NH-(C₁-C₄ alkyl)-N(R_{b})(R_{c}), NH-O-(C₁-C₄ alkyl) or NHC(=O)-R₅,
in which at least one H of C₁-C₄ alkyl, C₆-C₁₂ aryl, C₂-C₁₀ heteroaryl, C₂-C₁₀ heterocycloalkenyl or C₂-C₁₀ heterocycloalkyl can be each independently substituted with X, C₁-C₄ alkyl, C₁-C₄ haloalkyl, C₃-C₁₀ cycloalkyl, C₆-C₁₂ aryl, C₂-C₁₀ heteroaryl, C₂-C₁₀ heterocycloalkyl, (C₁-C₄ alkyl)-(C₂-C₁₀ heteroaryl), (C₁-C₄ alkyl)-(C₂-C₁₀ heterocycloalkyl), (C₂-C₁₀ heterocycloalkyl)-(C₁-C₄ alkyl), (C₂-C₁₀ heterocycloalkyl)-(C₁-C₄ haloalkyl), (C₂-C₁₀ heterocycloalkyl)-(C₃-C₁₀ cycloalkyl), (C₂-C₁₀ heterocycloalkyl)-(C₂-C₁₀ heterocycloalkyl), (C₂-C₁₀ heterocycloalkyl)-(C₁-C₄ alkyl)-(C₃-C₁₀ halocycloalkyl), (C₁-C₄ alkyl)-(C₃-C₁₀ cycloalkyl), S(O₂)-(C₁-C₄ alkyl), C(=O)-N(R_{b})(R_{c}), C(=O)-R₆, -N(R_{b})(R_{c}), (C₁-C₄ alkyl)-N(R_{b})(R_{c}), O-(C₁-C₄ alkyl), (C₁-C₄ alkyl)-O-(C₁-C₄ alkyl), C(=O)O-(C₂-C₁₀ heterocycloalkyl), (C₁-C₄ alkyl)-C(=O)-R₇ or (C₂-C₁₀ heterocycloalkyl)-C(=O)-R₈;
Y is CH, N, O or S {in which R₄ is null when Y is O or S};
R₄ is H, C₁-C₄ alkyl, C₃-C₇ cycloalkyl, C₂-C₁₀ heterocycloalkyl, (C₁-C₄ alkyl)-(C₂-C₁₀ heterocycloalkyl), C₆-C₁₂ aryl, C₂-C₁₀ heteroaryl or C(=O)-R₉,
in which at least one H of C₁-C₄ alkyl, C₂-C₁₀ heterocycloalkyl or (C₁-C₄ alkyl)-(C₂-C₁₀ heterocycloalkyl) can be each independently substituted with X, C₁-C₄ alkyl, C₁-C₄ haloalkyl, O-(C₁-C₄ alkyl), -N(R_{b})(R_{c}), C₃-C₁₀ cycloalkyl, C₂-C₁₀ heterocycloalkyl, (C₂-C₁₀ heterocycloalkyl)-C(=O)-R₁₀, S(O₂)-(C₁-C₄ alkyl), C(=O)-R₁₁, (C₁-C₄ alkyl)-O-(C₁-C₄ alkyl), C₆-C₁₂ aryl, C₂-C₁₀ heteroaryl, (C₁-C₄ alkyl)-(C₂-C₁₀ heteroaryl), (C₂-C₁₀ heterocycloalkyl)-(C₂-C₁₀ heterocycloalkyl) or C(=O)-(C₁-C₄ alkyl)-O-(C₁-C₄ alkyl);
R₅, R₆, R₇, R₈, R₉, R₁₀ and R₁₁ are each independently H, C₁-C₄ alkyl, (C₁-C₄ alkyl)-OH, (C₁-C₄ alkyl)-O-(C₁-C₄ alkyl), C₂-C₁₀ heterocycloalkyl, C₆-C₁₂ aryl, C₂-C₁₀ heteroaryl, O-(C₁-C₄ alkyl), C₃-C₇ cycloalkyl or (C₁-C₄ alkyl)-N(R_{b})(R_{c}),
in which at least one H of C₆-C₁₂ aryl or C₂-C₁₀ heteroaryl can be each independently substituted with C₁-C₄ alkyl, X or C₁-C₄ haloalkyl;
R_{b} and R_{c} are each independently H, C₁-C₄ alkyl, C₆-C₁₂ aryl, C₂-C₁₀ heteroaryl, C₃-C₁₀ cycloalkyl, C₂-C₁₀ heterocycloalkyl, (C₁-C₄ alkyl)NH(C₁-C₄ alkyl), (C₁-C₄ alkyl)N(C₁-C₄ alkyl)₂, (C₁-C₄ alkyl)-O-(C₁-C₄ alkyl), C(=O)-(C₁-C₄ alkyl), C(=O)-(C₂-C₁₀ heteroaryl), C(=O)-(C₂-C₁₀ heterocycloalkyl) or C(=O)-(C₃-C₁₀ cycloalkyl);
X is an halogen atom; and
n is any one integer selected from 0, 1, 2 and 3.

In one embodiment, in the above chemical formula I,
Z₁ to Z₄ are each independently N or CRₐ, in which Rₐ is H, X, C₁-C₄ alkyl or O-(C₁-C₄ alkyl) and Rₐ can be different from each other when CRₐ is 2 or more;
K is O or S;
R₁ is CX₃ or CX₂H; 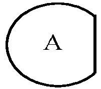 is C₆-C₁₂ arylene or C₂-C₁₀ heteroarylene,
in which C₂-C₁₀ heteroarylene can comprise at least one N;
R₂ and R₃ are each independently H, X, C₁-C₄ haloalkyl, C₆-C₁₂ aryl, C₂-C₁₀ heterocycloalkyl, C₂-C₁₀ heterocycloalkenyl, N(R_{b})(R_{c}) or C₂-C₁₀ heteroaryl,
in which at least one H of C₆-C₁₂ aryl, C₂-C₁₀ heteroaryl, C₂-C₁₀ heterocycloalkenyl or C₂-C₁₀ heterocycloalkyl can be each independently substituted with X, C₁-C₄ alkyl, C₁-C₄ haloalkyl, C₃-C₁₀ cycloalkyl, C₂-C₁₀ heterocycloalkyl, (C₁-C₄ alkyl)-(C₂-C₁₀ heteroaryl), (C₁-C₄ alkyl)-(C₂-C₁₀ heterocycloalkyl), (C₂-C₁₀ heterocycloalkyl)-(C₁-C₄ alkyl), (C₂-C₁₀ heterocycloalkyl)-(C₁-C₄ haloalkyl), (C₂-C₁₀ heterocycloalkyl)-(C₃-C₁₀ cycloalkyl), (C₂-C₁₀ heterocycloalkyl)-(C₂-C₁₀ heterocycloalkyl), (C₂-C₁₀ heterocycloalkyl)-(C₁-C₄ alkyl)-(C₃-C₁₀ halocycloalkyl), S(O₂)-(C₁-C₄ alkyl), C(=O)-R₆, O-(C₁-C₄ alkyl) or (C₂-C₁₀ heterocycloalkyl)-C(=O)-R₈;
Y is CH, N, O or S {in which R₄ is null when Y is O or S};
R₄ is C₁-C₄ alkyl, C₂-C₁₀ heterocycloalkyl, C₂-C₁₀ heteroaryl or (C₁-C₄ alkyl)-(C₂-C₁₀ heterocycloalkyl),
in which at least one H of C₁-C₄ alkyl, C₂-C₁₀ heterocycloalkyl or (C₁-C₄ alkyl)-(C₂-C₁₀ heterocycloalkyl) can be each independently substituted with C₁-C₄ alkyl, C₁-C₄ haloalkyl, O-(C₁-C₄ alkyl), -N(R_{b})(R_{c}), C₃-C₁₀ cycloalkyl, C₂-C₁₀ heterocycloalkyl, (C₂-C₁₀ heterocycloalkyl)-C(=O)-R₁₀, S(O₂)-(C₁-C₄ alkyl), C(=O)-R₁₁, (C₁-C₄ alkyl)-O-(C₁-C₄ alkyl), C₆-C₁₂ aryl, (C₂-C₁₀ heterocycloalkyl)-(C₂-C₁₀ heterocycloalkyl) or (C₁-C₄ alkyl)-(C₂-C₁₀ heteroaryl);
R₆, R₈, R₁₀ and R₁₁ are each independently C₁-C₄ alkyl, (C₁-C₄ alkyl)-OH, (C₁-C₄ alkyl)-O-(C₁-C₄ alkyl), C₂-C₁₀ heterocycloalkyl, C₆-C₁₂ aryl, C₂-C₁₀ heteroaryl or O-(C₁-C₄ alkyl),
in which at least one H of C₆-C₁₂ aryl or C₂-C₁₀ heteroaryl can be each independently substituted with C₁-C₄ alkyl or C₁-C₄ haloalkyl;
R_{b} and R_{c} are each independently H, C₁-C₄ alkyl or C(=O)-(C₁-C₄ alkyl);
X is an halogen atom; and
n is one integer selected from 0, 1, 2 and 3.

In one embodiment, in the above chemical formula I, C₂-C₁₀ heterocycloalkyl is 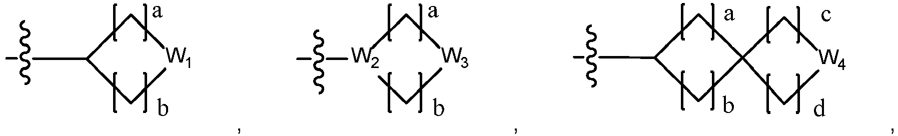 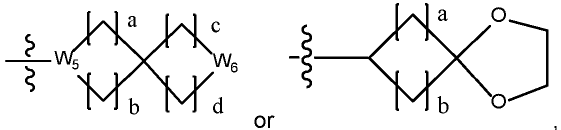 in which W₁ to W₆ are each independently N, NH, O, S or SO₂, and
a to d are each independently an integer of 1, 2 or 3.

In one embodiment, the compounds represented by the above chemical formula I may comprise the compounds represented by a following chemical formula II: 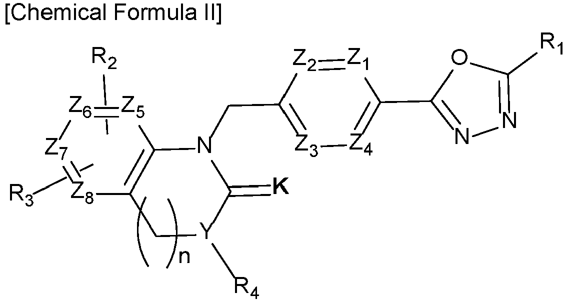 wherein,
Z₁ to Z₄ are each independently N or CRₐ, in which Rₐ is H, X, C₁-C₄ alkyl or O-(C₁-C₄ alkyl) and Rₐ can be different from each other when CRₐ is 2 or more;
Z₅ to Z₈ are each independently CR₂, CR₃, CH or N, in which Z₅ to Z₈ comprise CR₂, CR₃, CH and N, comprise CR₂, CR₃ and two Ns, or comprise CR₂, CR₃ and two CHs;
K is O or S;
R₁ is CX₃ or CX₂H;
R₂ and R₃ are each independently H, X, C₁-C₄ alkyl, C₁-C₄ haloalkyl, C₆-C₁₂ aryl, C₂-C₁₀ heteroaryl, C₃-C₁₀ cycloalkyl, C₂-C₁₀ heterocycloalkyl, C₂-C₁₀ heterocycloalkenyl, N(R_{b})(R_{c}), NH-(C₁-C₄ alkyl)-N(R_{b})(R_{c}), NH-O-(C₁-C₄ alkyl) or NHC(=O)-R₅,
in which at least one H of C₁-C₄ alkyl, C₆-C₁₂ aryl, C₂-C₁₀ heteroaryl, C₂-C₁₀ heterocycloalkenyl or C₂-C₁₀ heterocycloalkyl can be each independently substituted with X, C₁-C₄ alkyl, C₁-C₄ haloalkyl, C₃-C₁₀ cycloalkyl, C₆-C₁₂ aryl, C₂-C₁₀ heteroaryl, C₂-C₁₀ heterocycloalkyl, (C₁-C₄ alkyl)-(C₂-C₁₀ heteroaryl), (C₁-C₄ alkyl)-(C₂-C₁₀ heterocycloalkyl), (C₂-C₁₀ heterocycloalkyl)-(C₁-C₄ alkyl), (C₂-C₁₀ heterocycloalkyl)-(C₁-C₄ haloalkyl), (C₂-C₁₀ heterocycloalkyl)-(C₃-C₁₀ cycloalkyl), (C₂-C₁₀ heterocycloalkyl)-(C₂-C₁₀ heterocycloalkyl), (C₂-C₁₀ heterocycloalkyl)-(C₁-C₄ alkyl)-(C₃-C₁₀ halocycloalkyl), (C₁-C₄ alkyl)-(C₃-C₁₀ cycloalkyl), S(O₂)-(C₁-C₄ alkyl), C(=O)-N(R_{b})(R_{c}), C(=O)-R₆, -N(R_{b})(R_{c}), (C₁-C₄ alkyl)-N(R_{b})(R_{c}), O-(C₁-C₄ alkyl), (C₁-C₄ alkyl)-O-(C₁-C₄ alkyl), C(=O)O-(C₂-C₁₀ heterocycloalkyl), (C₁-C₄ alkyl)-C(=O)-R₇ or (C₂-C₁₀ heterocycloalkyl)-C(=O)-R₈;
Y is CH, N, O or S {in which R₄ is null when Y is O or S};
R₄ is H, C₁-C₄ alkyl, C₃-C₇ cycloalkyl, C₂-C₁₀ heterocycloalkyl, (C₁-C₄ alkyl)-(C₂-C₁₀ heterocycloalkyl), C₆-C₁₂ aryl, C₂-C₁₀ heteroaryl or C(=O)-R₉,
in which at least one H of C₁-C₄ alkyl, C₂-C₁₀ heterocycloalkyl or (C₁-C₄ alkyl)-(C₂-C₁₀ heterocycloalkyl) may be each independently substituted with X, C₁-C₄ alkyl, C₁-C₄ haloalkyl, O-(C₁-C₄ alkyl), -N(R_{b})(R_{c}), C₃-C₁₀ cycloalkyl, C₂-C₁₀ heterocycloalkyl, (C₂-C₁₀ heterocycloalkyl)-C(=O)-R₁₀, S(O₂)-(C₁-C₄ alkyl), C(=O)-R₁₁, (C₁-C₄ alkyl)-O-(C₁-C₄ alkyl), C₆-C₁₂ aryl, C₂-C₁₀ heteroaryl, (C₁-C₄ alkyl)-(C₂-C₁₀ heteroaryl), (C₂-C₁₀ heterocycloalkyl)-(C₂-C₁₀ heterocycloalkyl) or C(=O)-(C₁-C₄ alkyl)-O-(C₁-C₄ alkyl);
R₅, R₆, R₇, R₈, R₉, R₁₀ and R₁₁ are each independently H, C₁-C₄ alkyl, (C₁-C₄ alkyl)-OH, (C₁-C₄ alkyl)-O-(C₄-C₄ alkyl), C₂-C₁₀ heterocycloalkyl, C₆-C₁₂ aryl, C₂-C₁₀ heteroaryl, O-(C₁-C₄ alkyl), C₃-C₇ cycloalkyl or (C₁-C₄ alkyl)-N(R_{b})(R_{c}),
in which at least one H of C₆-C₁₂ aryl or C₂-C₁₀ heteroaryl can be each independently substituted with C₁-C₄ alkyl, X or C₁-C₄ haloalkyl;
R_{b} and R_{c} are each independently H, C₁-C₄ alkyl, C₆-C₁₂ aryl, C₂-C₁₀ heteroaryl, C₃-C₁₀ cycloalkyl, C₂-C₁₀ heterocycloalkyl, (C₁-C₄ alkyl)NH(C₁-C₄ alkyl), (C₁-C₄ alkyl)N(C₁-C₄ alkyl)₂, (C₁-C₄ alkyl)-O-(C₁-C₄ alkyl), C(=O)-C₁-C₄ alkyl, C(=O)-C₂-C₁₀ heteroaryl, C(=O)-(C₂-C₁₀ heterocycloalkyl) or C(=O)-(C₃-C₁₀ cycloalkyl);
X is an halogen atom; and
n is any one integer selected from 0, 1, 2 and 3.

In one embodiment, in the above chemical formula II,
Z₁ to Z₄ are each independently N or CRₐ, in which Rₐ is H, X, C₁-C₄ alkyl or O-(C₁-C₄ alkyl) and Rₐ can be different from each other when CRₐ is 2 or more;
Z₅ to Z₈ are each independently CR₂, CR₃, CH or N, in which Z₅ to Z₈ comprise CR₂, CR₃, CH and N or comprise CR₂, CR₃ and two CHs (however, Z₆ is N when Z₅ to Z₈ comprise CR₂, CR₃, CH and N);
K is O or S;
R₁ is CX₃ or CX₂H;
R₂ and R₃ are each independently H, X, C₁-C₄ haloalkyl, C₆-C₁₂ aryl, C₂-C₁₀ heterocycloalkyl, C₂-C₁₀ heterocycloalkenyl, N(R_{b})(R_{c}) or C₂-C₁₀ heteroaryl,
in which at least one H of C₆-C₁₂ aryl, C₂-C₁₀ heteroaryl, C₂-C₁₀ heterocycloalkenyl or C₂-C₁₀ heterocycloalkyl can be each independently substituted with X, C₁-C₄ alkyl, C₁-C₄ haloalkyl, C₃-C₁₀ cycloalkyl, C₂-C₁₀ heterocycloalkyl, (C₁-C₄ alkyl)-(C₂-C₁₀ heteroaryl), (C₁-C₄ alkyl)-(C₂-C₁₀ heterocycloalkyl), (C₂-C₁₀ heterocycloalkyl)-(C₁-C₄ alkyl), (C₂-C₁₀ heterocycloalkyl)-(C₁-C₄ haloalkyl), (C₂-C₁₀ heterocycloalkyl)-(C₃-C₁₀ cycloalkyl), (C₂-C₁₀ heterocycloalkyl)-(C₂-C₁₀ heterocycloalkyl), (C₂-C₁₀ heterocycloalkyl)-(C₁-C₄ alkyl)-(C₃-C₁₀ halocycloalkyl), S(O₂)-(C₁-C₄ alkyl), C(=O)-R₆, O-(C₁-C₄ alkyl) or (C₂-C₁₀ heterocycloalkyl)-C(=O)-R₈;
Y is CH, N, O or S {in which R₄ is null when Y is O or S};
R₄ is C₁-C₄ alkyl, C₂-C₁₀ heterocycloalkyl, C₂-C₁₀ heteroaryl or (C₁-C₄ alkyl)-(C₂-C₁₀ heterocycloalkyl),
in which at least one H of C₁-C₄ alkyl, C₂-C₁₀ heterocycloalkyl or (C₁-C₄ alkyl)-(C₂-C₁₀ heterocycloalkyl) can be each independently substituted with C₁-C₄ alkyl, C₁-C₄ haloalkyl, O-(C₁-C₄ alkyl), -N(R_{b})(R_{c}), C₃-C₁₀ cycloalkyl, C₂-C₁₀ heterocycloalkyl, (C₂-C₁₀ heterocycloalkyl)-C(=O)-R₁₀, S(O₂)-(C₁-C₄ alkyl), C(=O)-R₁₁, (C₁-C₄ alkyl)-O-(C₁-C₄ alkyl), C₆-C₁₂ aryl, (C₂-C₁₀ heterocycloalkyl)-(C₂-C₁₀ heterocycloalkyl) or (C₁-C₄ alkyl)-(C₂-C₁₀ heteroaryl);
R₆, R₈, R₁₀ and R₁₁ are each independently C₁-C₄ alkyl, (C₁-C₄ alkyl)-OH, (C₁-C₄ alkyl)-O-(C₁-C₄ alkyl), C₂-C₁₀ heterocycloalkyl, C₆-C₁₂ aryl, C₂-C₁₀ heteroaryl or O-(C₁-C₄ alkyl),
in which at least one H of C₆-C₁₂ aryl or C₂-C₁₀ heteroaryl can be each independently substituted with C₁-C₄ alkyl or C₁-C₄ haloalkyl;
R_{b} and R_{c} are each independently H, C₁-C₄ alkyl or C(=O)-(C₁-C₄ alkyl);
X is an halogen atom; and
n is any one integer selected from 0, 1, 2 and 3.

In one embodiment, in the above chemical formula II,
Z₁ to Z₄ are each independently N or CRₐ, in which Rₐ is H, X, C₁-C₄ alkyl or O-(C₁-C₄ alkyl) and Rₐ can be different from each other when CRₐ is 2 or more;
Z₅ to Z₈ are each independently CR₂, CR₃, CH or N, in which Z₅ to Z₈ comprise CR₂, CR₃, CH and N or comprise CR₂, CR₃ and two CHs (however, Z₆ is N when Z₅ to Z₈ comprise CR₂, CR₃, CH and N);
K is O or S;
R₁ is CX₃ or CX₂H;
R₂ and R₃ are each independently H, X, C₁-C₄ haloalkyl, C₆-C₁₂ aryl, C₂-C₁₀ heterocycloalkyl, C₂-C₁₀ heterocycloalkenyl, N(R_{b})(R_{c}) or C₂-C₁₀ heteroaryl,
in which at least one H of C₆-C₁₂ aryl, C₂-C₁₀ heteroaryl, C₂-C₁₀ heterocycloalkenyl or C₂-C₁₀ heterocycloalkyl can be each independently substituted with X, C₁-C₄ alkyl, C₁-C₄ haloalkyl, C₃-C₁₀ cycloalkyl, C₂-C₁₀ heterocycloalkyl, (C₁-C₄ alkyl)-(C₂-C₁₀ heteroaryl), (C₁-C₄ alkyl)-(C₂-C₁₀ heterocycloalkyl), (C₂-C₁₀ heterocycloalkyl)-(C₁-C₄ alkyl), (C₂-C₁₀ heterocycloalkyl)-(C₁-C₄ haloalkyl), (C₂-C₁₀ heterocycloalkyl)-(C₃-C₁₀ cycloalkyl), (C₂-C₁₀ heterocycloalkyl)-(C₂-C₁₀ heterocycloalkyl), (C₂-C₁₀ heterocycloalkyl)-(C₁-C₄ alkyl)-(C₃-C₁₀ halocycloalkyl), S(O₂)-(C₁-C₄ alkyl), C(=O)-R₆, O-(C₁-C₄ alkyl) or (C₂-C₁₀ heterocycloalkyl)-C(=O)-R₈;
Y is N, O or S {in which R₄ is null when Y is O or S};
R₄ is C₁-C₄ alkyl, C₂-C₁₀ heterocycloalkyl, C₂-C₁₀ heteroaryl or (C₁-C₄ alkyl)-(C₂-C₁₀ heterocycloalkyl),
in which at least one H of C₁-C₄ alkyl, C₂-C₁₀ heterocycloalkyl or (C₁-C₄ alkyl)-(C₂-C₁₀ heterocycloalkyl) can be each independently substituted with C₁-C₄ alkyl, C₁-C₄ haloalkyl, O-(C₁-C₄ alkyl), -N(R_{b})(R_{c}), C₃-C₁₀ cycloalkyl, C₂-C₁₀ heterocycloalkyl, (C₂-C₁₀ heterocycloalkyl)-C(=O)-R₁₀, S(O₂)-(C₁-C₄ alkyl), C(=O)-R₁₁, (C₁-C₄ alkyl)-O-(C₁-C₄ alkyl), C₆-C₁₂ aryl, (C₂-C₁₀ heterocycloalkyl)-(C₂-C₁₀ heterocycloalkyl) or (C₁-C₄ alkyl)-(C₂-C₁₀ heteroaryl);
R₆, R₈, R₁₀ and R₁₁ are each independently C₁-C₄ alkyl, (C₁-C₄ alkyl)-OH, (C₁-C₄ alkyl)-O-(C₁-C₄ alkyl), C₂-C₁₀ heterocycloalkyl, C₆-C₁₂ aryl, C₂-C₁₀ heteroaryl or O-(C₁-C₄ alkyl),
in which at least one H of C₆-C₁₂ aryl or C₂-C₁₀ heteroaryl can be each independently substituted with C₁-C₄ alkyl or C₁-C₄ haloalkyl;
R_{b} and R_{c} are each independently H, C₁-C₄ alkyl or C(=O)-(C₁-C₄ alkyl);
X is an halogen atom; and
n is any one integer selected from 0, 1, 2 and 3.

In one embodiment, in the above chemical formula I,
Z₁ to Z₄ are each independently N or CRₐ, in which Rₐ is H or X, and Rₐ can be different from each other when CRₐ is 2 or more;
K is O;
R₁ is CF₃ or CF₂H; 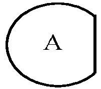 is phenylene or pyridinylene,
R₂ and R₃ are each independently H, X, C₁-C₄ haloalkyl, C₆-C₁₂ aryl, C₂-C₁₀ heterocycloalkyl, C₂-C₁₀ heterocycloalkenyl, N(R_{b})(R_{c}) or C₂-C₅ heteroaryl,
in which at least one H of C₆-C₁₂ aryl, C₂-C₅ heteroaryl, C₂-C₁₀ heterocycloalkenyl or C₂-C₁₀ heterocycloalkyl can be each independently substituted with X, C₁-C₄ alkyl, C₁-C₄ haloalkyl, C₃-C₁₀ cycloalkyl, C₂-C₁₀ heterocycloalkyl, (C₁-C₄ alkyl)-(C₂-C₁₀ heteroaryl), (C₁-C₄ alkyl)-(C₂-C₁₀ heterocycloalkyl), (C₂-C₁₀ heterocycloalkyl)-(C₁-C₄ alkyl), (C₂-C₁₀ heterocycloalkyl)-(C₁-C₄ haloalkyl), (C₂-C₁₀ heterocycloalkyl)-(C₃-C₁₀ cycloalkyl), (C₂-C₁₀ heterocycloalkyl)-(C₂-C₁₀ heterocycloalkyl), (C₂-C₁₀ heterocycloalkyl)-(C₁-C₄ alkyl)-(C₃-C₁₀ halocycloalkyl), S(O₂)-(C₁-C₄ alkyl), C(=O)-R₆, O-(C₁-C₄ alkyl) or (C₂-C₁₀ heterocycloalkyl)-C(=O)-R₈;
Y is N, O or S {in which R₄ is null when Y is O or S};
R₄ is C₁-C₄ alkyl, C₂-C₁₀ heterocycloalkyl, C₂-C₁₀ heteroaryl or (C₁-C₄ alkyl)-(C₂-C₁₀ heterocycloalkyl),
in which at least one H of C₁-C₄ alkyl, C₂-C₁₀ heterocycloalkyl or (C₁-C₄ alkyl)-(C₂-C₁₀ heterocycloalkyl) can be each independently substituted with C₁-C₄ alkyl, C₁-C₄ haloalkyl, O-(C₁-C₄ alkyl), -N(R_{b})(R_{c}), C₃-C₁₀ cycloalkyl, C₂-C₁₀ heterocycloalkyl, (C₂-C₁₀ heterocycloalkyl)-C(=O)-R₁₀, S(O₂)-(C₁-C₄ alkyl), C(=O)-R₁₁, (C₁-C₄ alkyl)-O-(C₁-C₄ alkyl), C₆-C₁₂ aryl, (C₂-C₁₀ heterocycloalkyl)-(C₂-C₁₀ heterocycloalkyl) or (C₁-C₄ alkyl)-(C₂-C₁₀ heteroaryl);
R₆ is C₁-C₄ alkyl, O-(C₁-C₄ alkyl) or (C₁-C₄ alkyl)-OH;
R₈ is O-(C₁-C₄ alkyl);
R₁₀ is C₁-C₄ alkyl;
R₁₁ is C₁-C₄ alkyl, (C₁-C₄ alkyl)-OH, (C₁-C₄ alkyl)-O-(C₁-C₄ alkyl), C₂-C₁₀ heterocycloalkyl, C₆-C₁₂ aryl, C₂-C₁₀ heteroaryl or O-(C₁-C₄ alkyl), in which at least one H of C₆-C₁₂ aryl or C₂-C₁₀ heteroaryl can be each independently substituted with C₁-C₄ alkyl or C₁-C₄ haloalkyl;
R_{b} and R_{c} are each independently H, C₁-C₄ alkyl or C(=O)-(C₁-C₄ alkyl);
X is F, CI or Br; and
n is 0 or 1.

### Advantageous Effects

According to the present invention, 1,3,4-oxadiazole derivative compounds represented by the above chemical formula I, stereoisomers thereof or pharmaceutically acceptable salts thereof have not only an HDAC6 inhibitory activity, but also a remarkably excellent effect of preventing or treating HDAC6 activity-related diseases by selectively inhibiting HDAC6.

Also, the inventive 1,3,4-oxadiazole derivative compounds having a selective HDAC6 inhibitory activity, stereoisomers thereof or pharmaceutically acceptable salts thereof can be used to prevent or treat HDAC6 activity-related diseases such as cancers, inflammatory diseases, autoimmune diseases, neurological diseases or neurodegenerative disorders, etc.

### Best Mode for Invention

Terms used in the present application are used only to describe a certain exemplary embodiment and are not intended to limit the present invention. Singular forms are to include plural forms, unless otherwise clearly indicated by context. In the present application, the terms such as "comprise," "have" or the like shall be intended to designate a presence of features, steps, structures or combinations thereof described herein and shall not be construed to exclude a possible presence or addition of one or more other features, steps, structures or combinations thereof in advance.

All the terms used herein including technical or scientific terms have the same meaning as commonly understood by those ordinary skilled in the art, to which the present invention pertains, unless defined otherwise. Such terms as those defined in a generally used dictionary are to be interpreted to have the meanings equal to the contextual meanings in the relevant art, and are not to be interpreted to have ideal or excessively formal meanings, unless clearly defined in the present application.

In the present invention, the term "substituted" represents a moiety having a substituent which replaces at least one hydrogen on carbon of a main chain. The "substitution," "substitutable with~" or "substituted with~" are defined to include implicit conditions, in which the substitution follows a permitted valency of a substituted atom and a substituent and induces a compound stabilized by substitution, for example, a compound which is not naturally modified by rearrangement, cyclization, removal, etc.

In the present invention, "C_{x-y}" means having carbon atoms in a range of x to y.

In the present invention, "alkyl" means a linear (or straight-chain) saturated hydrocarbon group or a branched (or side-chain) saturated hydrocarbon group, and includes methyl, ethyl, n-propyl, isopropyl, n-butyl, sec-butyl, isobutyl, tert-butyl, n-pentyl, n-hexyl, n-heptyl, etc.

In the present invention, "haloalkyl" means a functional group in which at least one hydrogen of the alkyl defined above is substituted with a halogen atom of F, Cl, Br or I.

In the present invention, "alkylene" means a divalent functional group which is induced from the alkyl group as defined above.

In the present invention, "aryl" includes a monocyclic aromatic structure or a polycyclic aromatic structure, as well as a structure in which a saturated hydrocarbon ring is fused into the monocyclic aromatic or polycyclic aromatic group. Aryl includes a phenyl, biphenyl, naphthalenyl, tetrahydronaphthalenyl, anthracenyl, phenanthrenyl, pyrenyl, etc.

In the present invention, "heteroaryl" means a monocyclic or polycyclic hetero ring in which at least one carbon atom of the aryl defined above is substituted with nitrogen (N), oxygen (O) or sulfur (S). Heteroaryl includes pyridinyl, thiophenyl, triazolyl, tetrazolyl, benzodioxolyl, benzothiazolyl, benzothiophenyl, quinolinyl, indolyl, isoindolyl, benzofuranyl, benzopyrrolyl, furanyl, pyrrolyl, thiazolyl, isothiazolyl, imidazolyl, pyrazolyl, oxazolyl, isoxazolyl, pyrazinyl, pyridazinyl, pyrimidinyl, isoquinolinyl, carbazolyl, benzoxazolyl, benzodioxazolyl, benzodioxinyl, benzimidazolyl, dihydrobenzothiophenyl, dihydrobenzofuranyl, purinyl, indolizinyl, chromanyl, chromenyl, dihydrobenzodioxinyl, etc., but is not limited thereto.

In the present invention, "cycloalkyl" means a saturated hydrocarbon ring generally having a specified number of carbon atoms, and the saturated hydrocarbon ring collectively refers to monocyclic and polycyclic structures, and a ring structure in which at least two rings share at least one carbon atom (for example, spiro ring, bridged ring, etc.). Cycloalkyl includes cyclohexyl, cycloheptanyl, cyclooctanyl, tetrahydronaphthalenyl, etc, but is not limited thereto.

In the present invention, "halocycloalkyl" means a functional group in which at least one hydrogen of the cycloalkyl defined above is substituted with a halogen atom of F, Cl, Br or I.

In the present invention, "heterocycloalkyl" includes saturated monocyclic and polycyclic hetero rings containing one to four hetero atoms independently selected from nitrogen (N), oxygen (O) and sulfur (S), and a ring structure in which at least two rings share at least one carbon atom (for example, spiro ring, bridged ring, etc.).

In the present invention, heterocycloalkyl can comprise 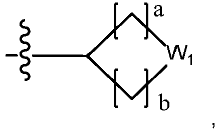 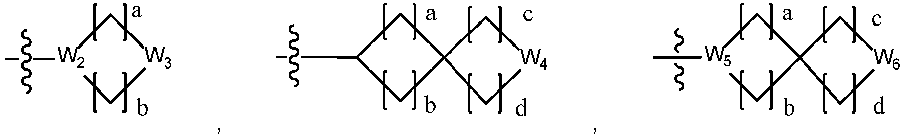 or 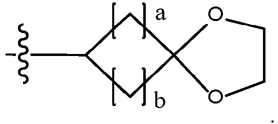 In this case, W₁ to W₆ are each independently N, NH, O, S or SO₂ and a to d are each independently an integer of 1, 2 or 3.

In the present invention, the specific examples of heterocycloalkyl may include oxiranyl, oxetanyl, morpholinyl, thiethanyl, azetidine, pyrrolidinyl, piperidinyl, tetrahydrofuranyl, tetrahydrothiophenyl, tetrahydropyranyl, tetrahydrothiopyranyl, tetrahydrothiopyran 1,1-dioxide, 6-azabicyclo[3.2.1]octanyl, 2-oxa-6-azaspiro[3.3]heptanyl, 2-oxaspiro[3.3]heptanyl, 1,4-dioxaspiro[4.5]decanyl, etc., but are not particularly limited thereto.

In the present invention, "heterocycloalkenyl" means a structure including at least one carbon-carbon double bond or carbon-nitrogen double bond out of monocyclic and polycyclic hetero rings containing one to four hetero atoms independently selected from nitrogen (N), oxygen (O) and sulfur (S). Heterocycloalkenyl includes tetrahydropyridinyl, dihydropyranyl, dihydrothiopyranyl, dihydropyrrolyl, dihydrofuranyl, dihydrothiophenyl, etc., but is not limited thereto.

In the present invention, 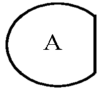 represents a structure fused by sharing two carbon atoms with another ring, and the two shared/fused carbon atoms mean two arranged in a row. With regard to 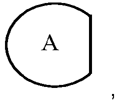 "arylene" means phenylene, naphthalenylene, etc., and "heteroarylene" means pyrimidylene, pyridylene, etc. containing at least one heteroatom, for example, N, O or S in said arylene. In this case, said arylene and said heteroarylene are fused by sharing two carbon atoms with another ring (a ring containing Y of the chemical formula I, having a structure represented by 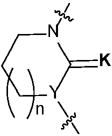 ). In this case, the two carbon atoms fused by sharing arylene or heteroarylene are two arranged in a row out of carbon atoms constituting another ring (a ring containing Y of the chemical formula I). As one example, if 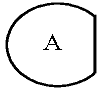 is phenylene, the chemical formula I may contain a structure of

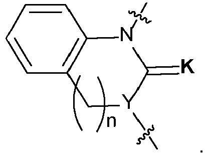

In the present invention, "stereoisomers" include a diastereomer and an optical isomer, in which the optical isomer includes not only an enantiomer, but also a mixture of the enantiomer and even a racemate.

In the present invention, "pharmaceutically acceptable salts" mean the salts conventionally used in a pharmaceutical industry. For example, there are inorganic ion salts prepared from calcium, potassium, sodium, magnesium, etc.; inorganic acid salts prepared from hydrochloric acid, nitric acid, phosphoric acid, bromic acid, iodic acid, perchloric acid, sulfuric acid, hydroiodic acid, etc.; organic acid salts prepared from acetic acid, trifluoroacetic acid, citric acid, maleic acid, succinic acid, oxalic acid, benzoic acid, tartaric acid, fumaric acid, mandelic acid, propionic acid, lactic acid, glycolic acid, gluconic acid, galacturonic acid, glutamic acid, glutaric acid, glucuronic acid, aspartic acid, ascorbric acid, carbonic acid, vanillic acid, etc.; sulfonic acid salts prepared from methanesulfonic acid, ethanesulfonic acid, benzenesulfonic acid, p-toluenesulfonic acid, naphthalenesulfonic acid, etc.; amino acid salts prepared from glycine, arginine, lysine, etc.; amine salts prepared from trimethylamine, triethylamine, ammonia, pyridine, picoline, etc.; and the like, but types of salts meant in the present invention are not limited to those listed salts.

In the present invention, preferable salts may include hydrochloride, phosphate, sulfate, trifluoroacetate, citrate, bromate, maleate or tartrate.

### Composition comprising 1,3,4-oxadiazole derivative compounds, use thereof and therapeutic method using the same

The present invention provides a pharmaceutical composition comprising 1,3,4-oxadiazole derivative compounds represented by the above chemical formula I, stereoisomers thereof or pharmaceutically acceptable salts thereof as an effective component.

The present invention provides a pharmaceutical composition for preventing or treating histone deacetylase (HDAC)-mediated diseases, comprising 1,3,4-oxadiazole derivative compounds represented by the above chemical formula I, stereoisomers thereof or pharmaceutically acceptable salts thereof as an effective component. Preferably, the present invention provides a pharmaceutical composition for preventing or treating histone deacetylase 6 (HDAC6) activity-related diseases. The above chemical formula I is the same as defined above.

The pharmaceutical composition of the present invention selectively inhibits HDAC6, thereby showing a remarkable effect on preventing or treating HDAC6 activity-related diseases.

The HDAC6 activity-related diseases include at least one selected form the consisting of infectious diseases such as prion disease; neoplasm such as benign tumor (for example, myelodysplastic syndrome) or malignant tumor (for example, multiple myeloma, lymphoma, leukemia, lung cancer, colorectal cancer, colon cancer, prostate cancer, urothelial carcinoma, breast cancer, melanoma, skin cancer, liver cancer, brain cancer, stomach cancer, ovarian cancer, pancreatic cancer, head and neck cancer, oral cancer or glioma); endocrinopathy, nutritional and metabolic diseases such as Wilson's disease, amyloidosis or diabetes; mental and behavioral disorders such as depression or rett syndrome; neurological diseases such as central nervous system atrophy (for example, Huntington's disease, spinal muscular atrophy (SMA), spinocerebellar ataxia (SCA)), neurodegenerative disease (for example, Alzheimer's disease), motor disorder (for example, Parkinson's disease), neuropathy (for example, hereditary neuropathy (Charcot-Marie-Tooth disease), sporadic neuropathy, inflammatory neuropathy, drug-induced neuropathy, motor neuropathy (for example, amyotrophic lateral sclerosis (ALS)), central nervous system demyelinating disease (for example, multiple sclerosis (MS)), or the like; eye and ocular adnexal diseases such as uveitis; circulatory diseases such as atrial fibrillation, stroke or the like; respiratory diseases such as asthma; digestive diseases such as alcoholic liver disease, inflammatory bowel disease, Crohn's disease, ulcerative bowel disease or the like; skin and subcutaneous tissue diseases such as psoriasis; musculoskeletal system and connective tissue diseases such as rheumatoid arthritis, osteoarthritis, systemic lupus erythematosis (SLE) or the like; or teratosis, deformities and chromosomal aberration such as autosomal dominant polycystic kidney disease, and also include other symptoms or diseases related to abnormal functions of histone deacetylase.

Said pharmaceutically acceptable salts are the same as described in the pharmaceutically acceptable salts of 1,3,4-oxadiazole derivative compounds of the present invention.

For administration, the pharmaceutical composition of the present invention may further comprise at least one type of a pharmaceutically acceptable carrier, in addition to said 1,3,4-oxadiazole derivative compounds, stereoisomers thereof or pharmaceutically acceptable salts thereof. The pharmaceutically acceptable carrier used may include saline solution, sterilized water, Ringer's solution, buffered saline, dextrose solution, maltodextrin solution, glycerol, ethanol and a mixture of at least one component thereof, and other conventional additives such as antioxidants, buffer solutions, bacteriostatic agents, etc., may be added thereto, if needed. Also, such pharmaceutical composition may be formulated into injectable dosage forms such as aqueous solutions, suspensions, emulsions, etc., pills, capsules, granules or tablets in such a way that diluents, dispersing agents, surfactants, binders and lubricants are additionally added thereto. Thus, the composition of the present invention may be patches, liquids and solutions, pills, capsules, granules, tablets, suppositories, etc. Such preparations may be prepared according to a conventional method used for formulation in the art or a method disclosed in Remington's Pharmaceutical Science (latest edition), Mack Publishing Company, Easton PA, and such composition may be formulated into various preparations depending on each disease or component.

The composition of the present invention may be orally or parenterally administered (for example, applied intravenously, hypodermically, intraperitoneally or locally) according to a targeted method, in which a dosage thereof varies in a range thereof depending on a patient's weight, age, gender, health condition, diet, an administration time, an administration method, an excretion rate, a severity of a disease and the like. A daily dosage of 1,3,4-oxadiazole derivative compounds of the present invention, stereoisomers thereof or pharmaceutically acceptable salts thereof may be about 1 to 1000 mg/kg, preferably 5 to 100 mg/kg, and may be administered at one time a day or several times a day by dividing the daily dosage of the compounds.

In addition to said 1,3,4-oxadiazole derivative compounds, stereoisomers thereof or pharmaceutically acceptable salts thereof, said pharmaceutical composition of the present invention may further comprise at least one effective component which shows a medicinal effect the same thereas or similar thereto.

The present invention provides a method for preventing or treating histone deacetylase 6 (HDAC6) activity-related diseases, comprising administering a therapeutically effective amount of said 1,3,4-oxadiazole derivative compounds, stereoisomers thereof or pharmaceutically acceptable salts thereof.

As used herein, the term "therapeutically effective amount" refers to an amount of said 1,3,4-oxadiazole derivative compounds, stereoisomers thereof or pharmaceutically acceptable salts thereof, which are effective in preventing or treating HDAC6 activity-related diseases.

Also, the present invention provides a method for selectively inhibiting HDAC6 by administering said 1,3,4-oxadiazole derivative compounds, stereoisomers thereof or pharmaceutically acceptable salts thereof into mammals including humans.

The method for preventing or treating HDAC6 activity-related diseases according to the present invention includes not only dealing with the diseases themselves before expression of their symptoms, but also inhibiting or avoiding such symptoms by administering said 1,3,4-oxadiazole derivative compounds, stereoisomers thereof or pharmaceutically acceptable salts thereof. In managing the disease, a preventive or therapeutic dose of a certain active component may vary depending on a nature and severity of the disease or condition and a route of administering the active component. A dose and a frequency thereof may vary depending on an individual patient's age, weight and reactions. A suitable dose and usage may be easily selected by those skilled in the art, naturally considering such factors. Also, the method for preventing or treating HDAC6 activity-related diseases according to the present invention may further comprise administering a therapeutically effective amount of an additional active agent, which is helpful in treating the diseases, along with the compounds represented by the above chemical formula I, and the additional active agent may exhibit a synergy effect or an additive effect together with said 1,3,4-oxadiazole derivative compounds, stereoisomers thereof or pharmaceutically acceptable salts thereof.

The present invention also provides a use of the compounds represented by the above chemical formula I, stereoisomers thereof or pharmaceutically acceptable salts thereof for preventing or treating histone deacetylase 6 (HDAC6) activity-related diseases. For preventing or treating HDAC6 activity-related diseases, said 1,3,4-oxadiazole derivative compounds, stereoisomers thereof or pharmaceutically acceptable salts thereof may be combined with acceptable adjuvants, diluents, carriers, etc., and may be prepared into a complex preparation together with other active agents and thus have a synergy action of active components.

The present invention also provides a use of the compounds represented by the above chemical formula I, stereoisomers thereof or pharmaceutically acceptable salts thereof in preparation of a medicament for treating histone deacetylase 6 (HDAC6) activity-related diseases. For preparing a medicament, said 1,3,4-oxadiazole derivative compounds, stereoisomers thereof or pharmaceutically acceptable salts thereof may be combined with acceptable adjuvants, diluents, carriers, etc., and may be prepared into a complex preparation together with other active agents and thus have a synergy action of active components.

Matters mentioned in the use, composition and therapeutic method of the present invention are equally applied, if not contradictory to each other.

In one embodiment, the 1,3,4-oxadiazole derivative compounds represented by the chemical formula I of the present invention, stereoisomers thereof or pharmaceutically acceptable salts thereof include the compounds as shown in a following table 1.

**[Table 1]**

| Compound | Structure | Compo und | Structure |
|---|---|---|---|
| 1 | 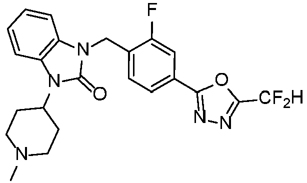 | 2 | 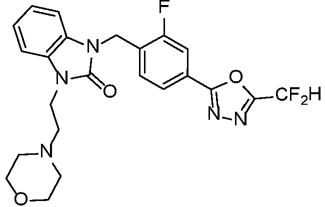 |
| 3 | 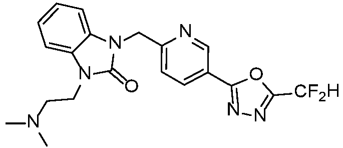 | 4 | 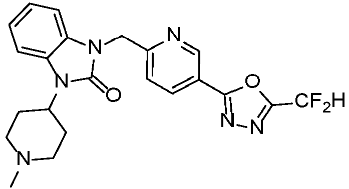 |
| 5 | 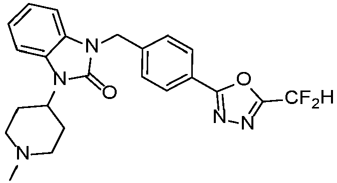 | 6 | 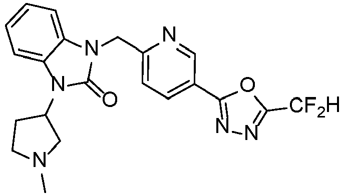 |
| 7 | 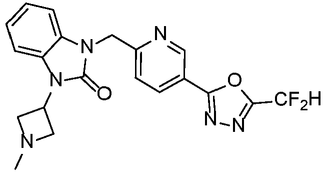 | 8 | 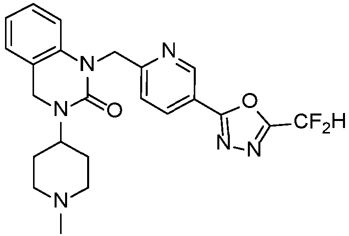 |
| 9 | 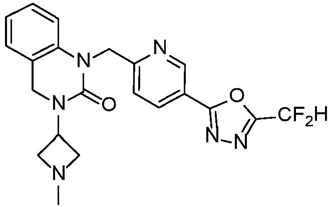 | 10 | 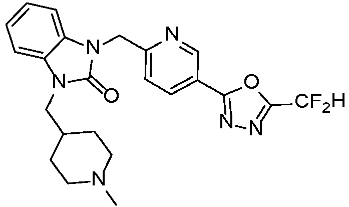 |
| 11 | 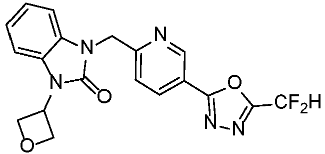 | 12 | 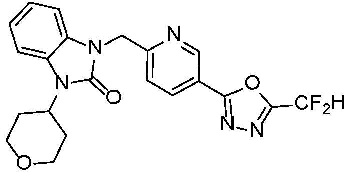 |
| 13 | 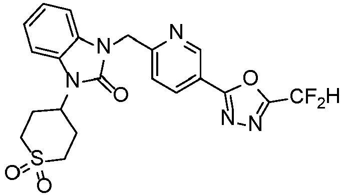 | 14 | 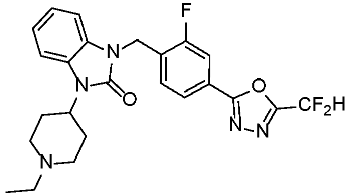 |
| 15 | 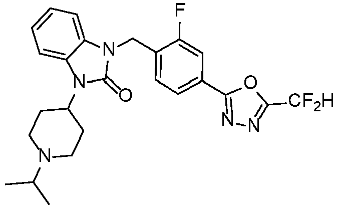 | 16 | 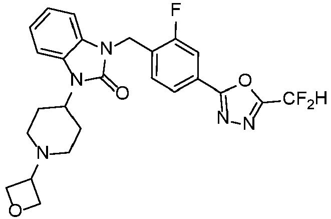 |
| 17 | 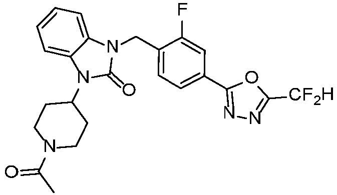 | 18 | 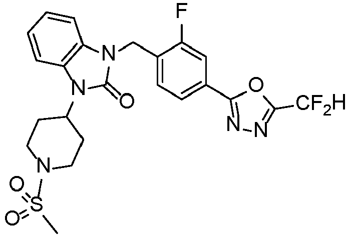 |
| 19 | 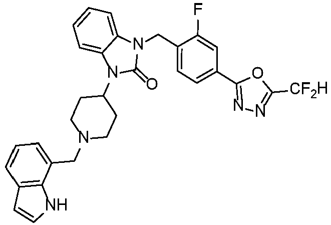 | 20 | 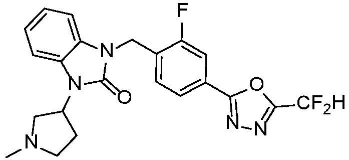 |
| 21 | 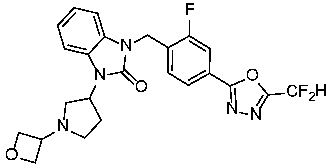 | 22 | 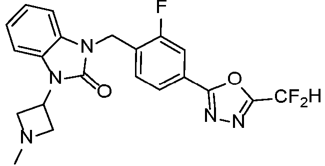 |
| 23 | 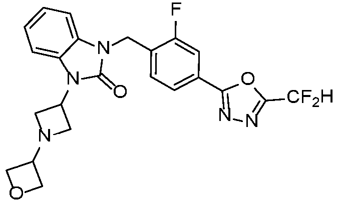 | 24 | 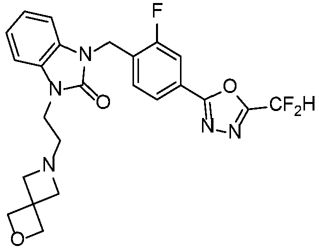 |
| 25 | 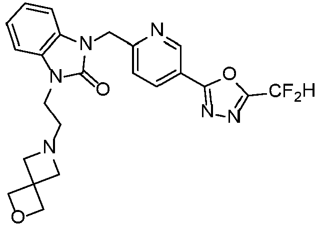 | 26 | 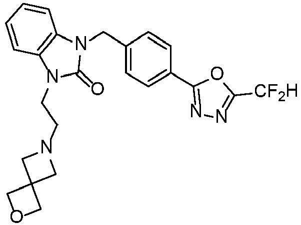 |
| 27 | 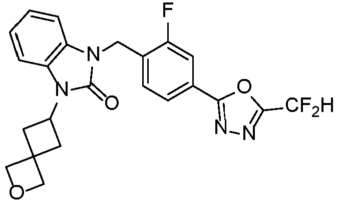 | 28 | 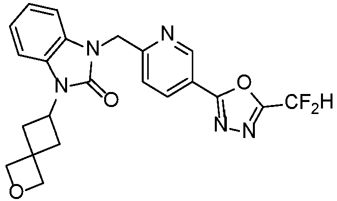 |
| 29 | 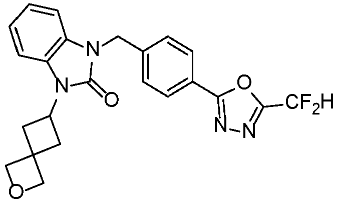 | 30 | 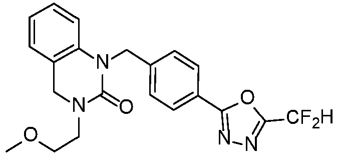 |
| 31 | 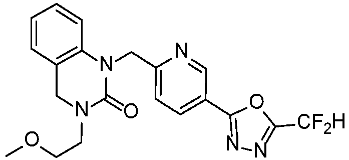 | 32 | 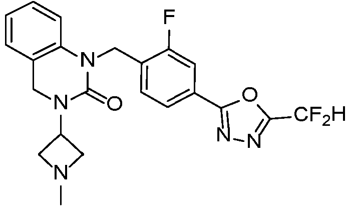 |
| 33 | 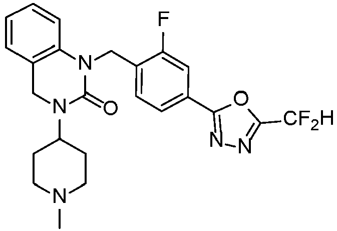 | 34 | 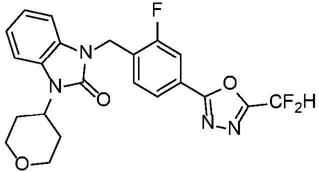 |
| 35 | 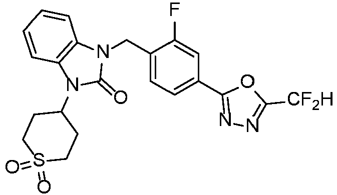 | 36 | 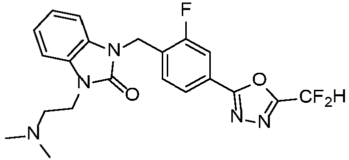 |
| 37 | 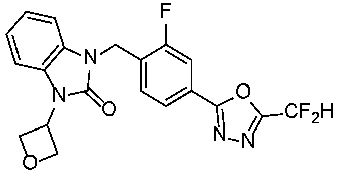 | 38 | 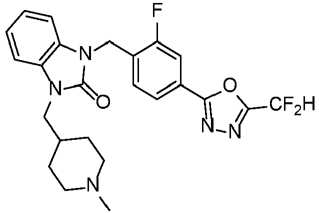 |
| 39 | 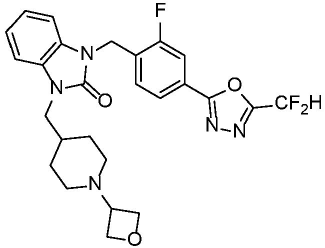 | 40 | 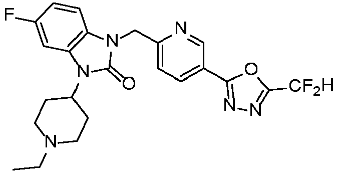 |
| 41 | 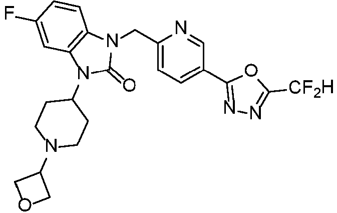 | 42 | 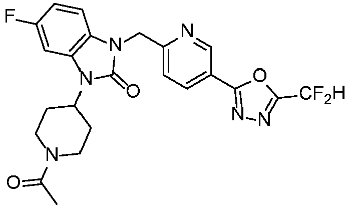 |
| 43 | 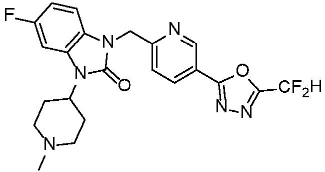 | 44 | 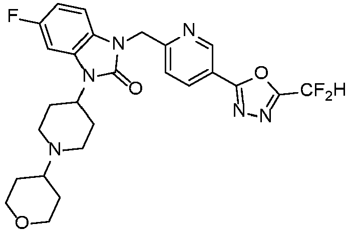 |
| 45 | 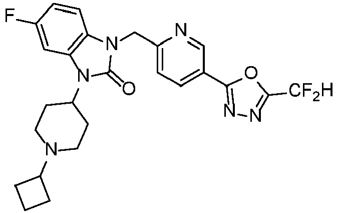 | 46 | 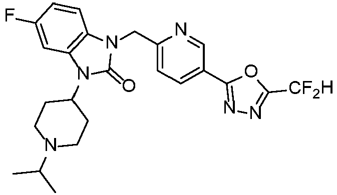 |
| 47 | 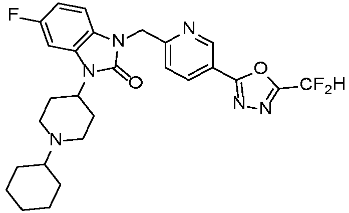 | 48 | 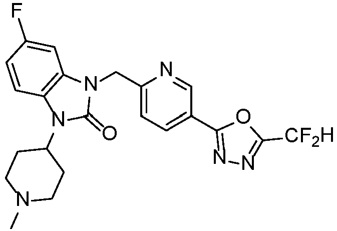 |
| 49 | 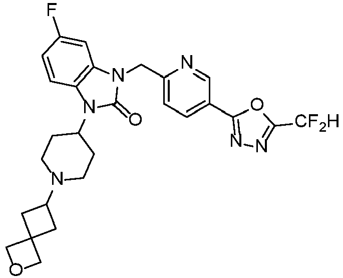 | 50 | 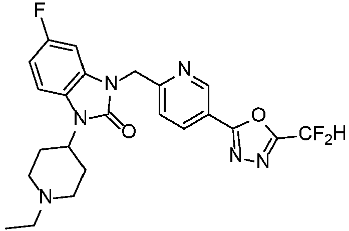 |
| 51 | 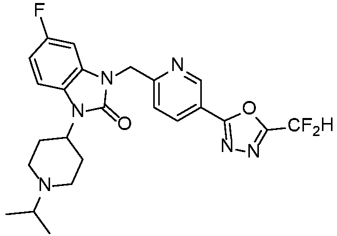 | 52 | 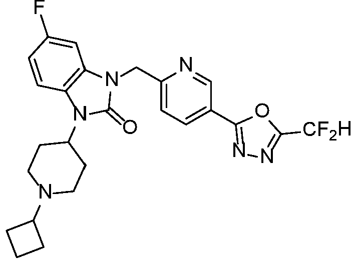 |
| 53 | 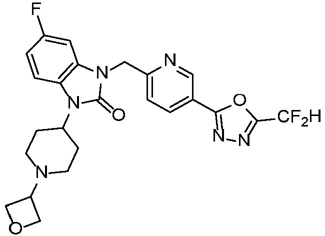 | 54 | 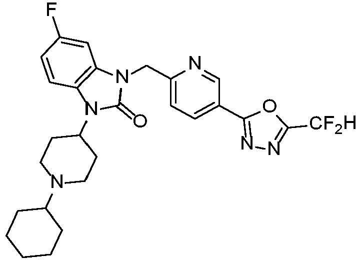 |
| 55 | 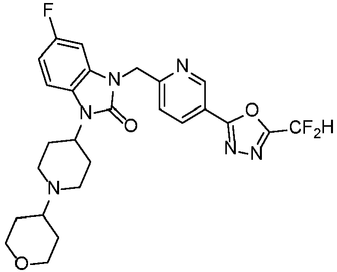 | 56 | 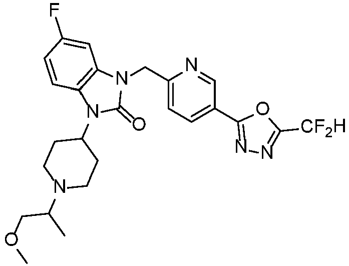 |
| 57 | 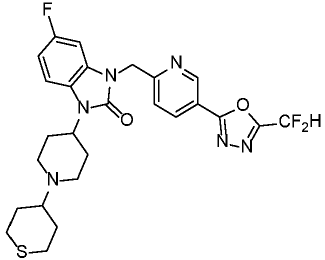 | 58 | 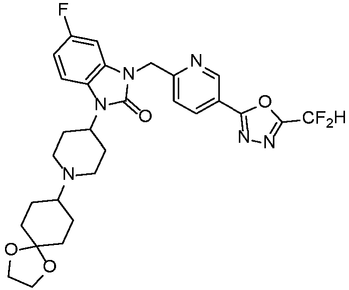 |
| 59 | 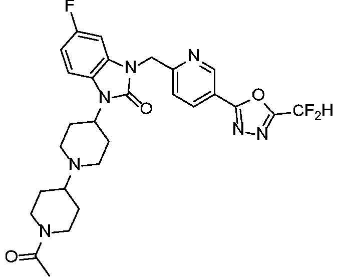 | 60 | 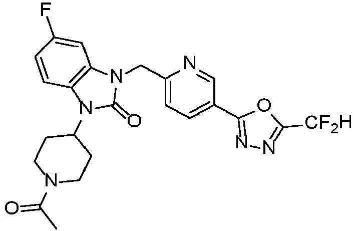 |
| 61 | 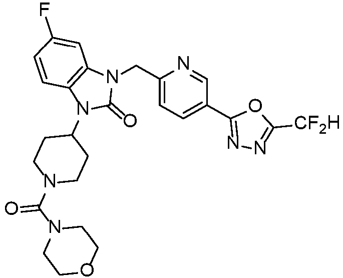 | 62 | 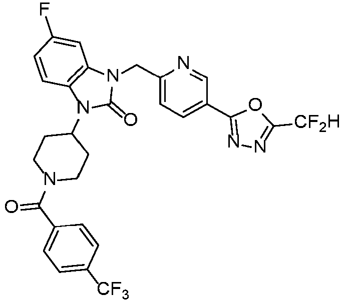 |
| 63 | 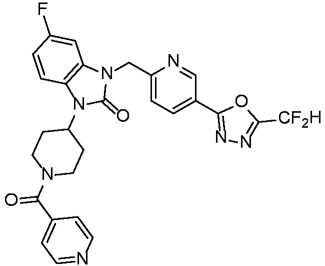 | 64 | 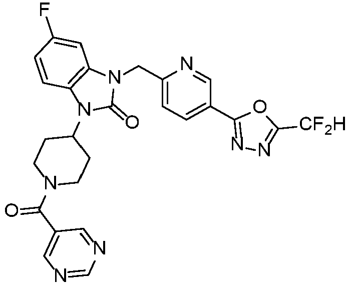 |
| 65 | 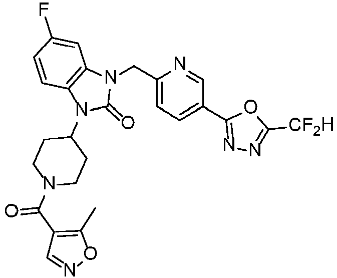 | 66 | 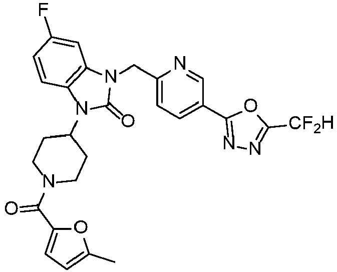 |
| 67 | 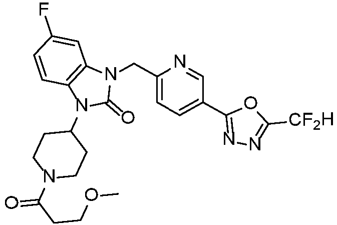 | 68 | 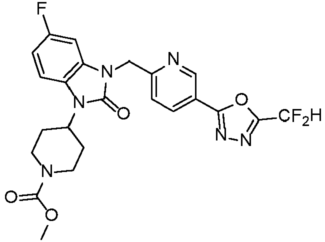 |
| 69 | 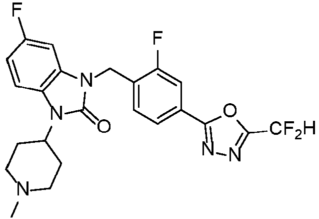 | 70 | 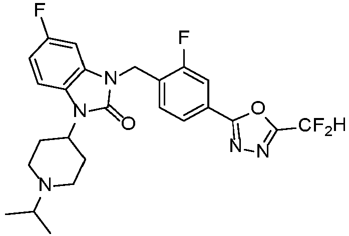 |
| 71 | 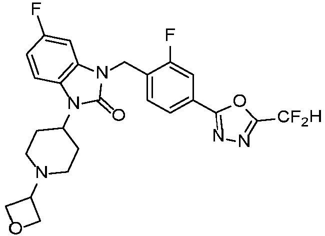 | 72 | 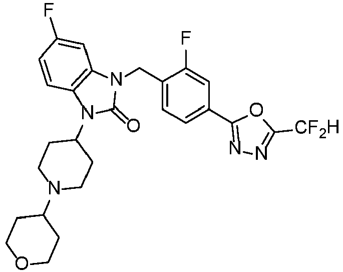 |
| 73 | 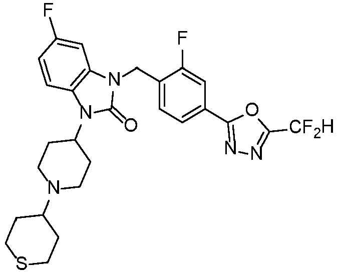 | 74 | 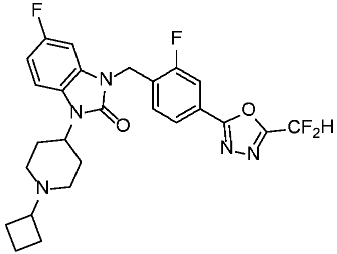 |
| 75 | 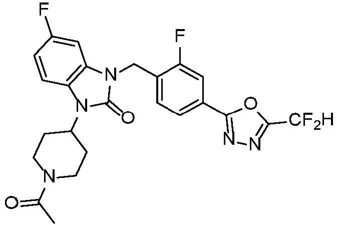 | 76 | 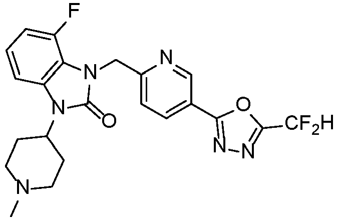 |
| 77 | 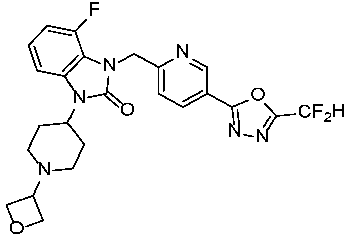 | 78 | 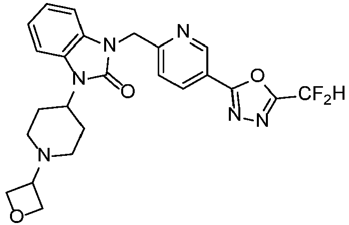 |
| 79 | 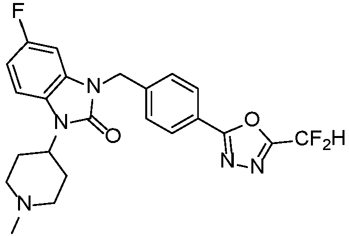 | 80 | 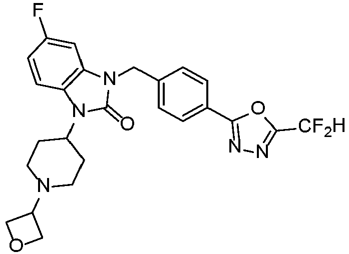 |
| 81 | 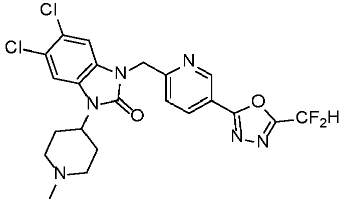 | 82 | 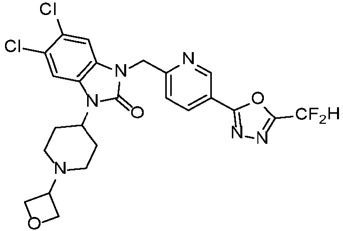 |
| 83 | 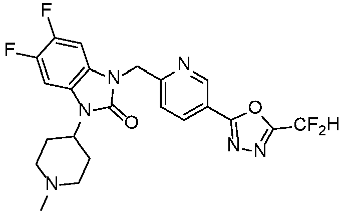 | 84 | 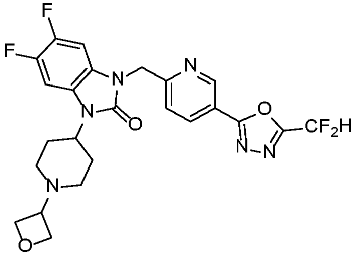 |
| 85 | 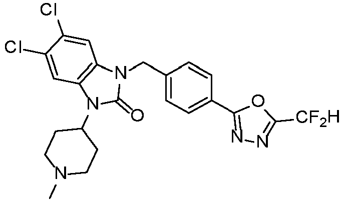 | 86 | 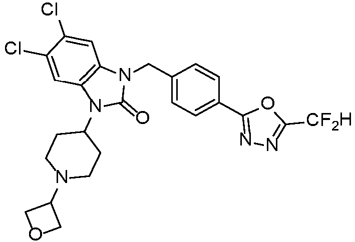 |
| 87 | 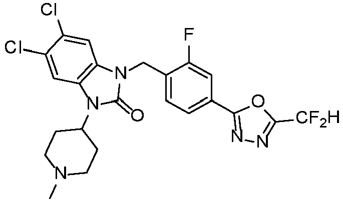 | 88 | 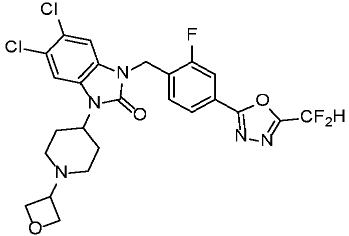 |
| 89 | 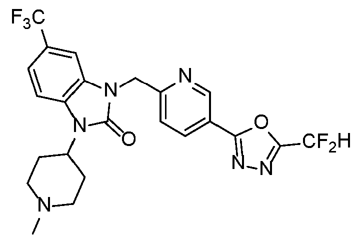 | 90 | 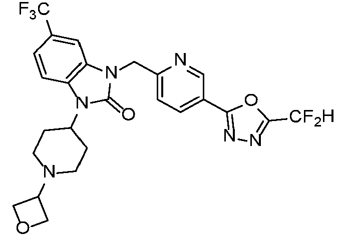 |
| 91 | 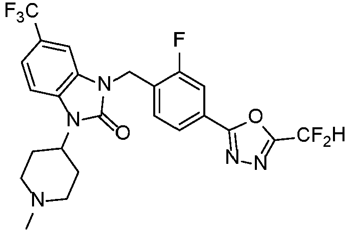 | 92 | 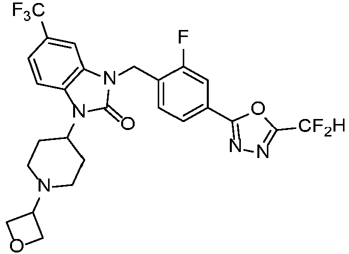 |
| 93 | 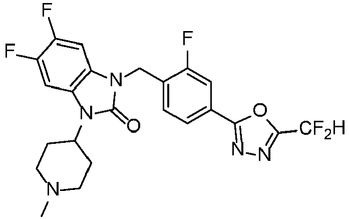 | 94 | 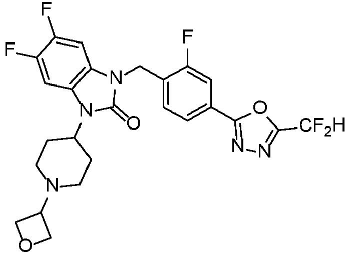 |
| 95 | 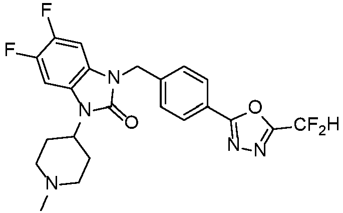 | 96 | 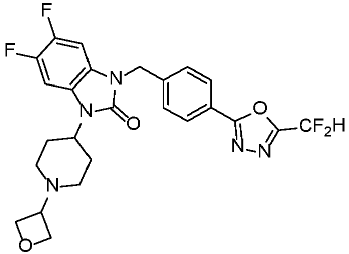 |
| 97 | 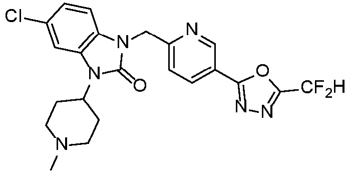 | 98 | 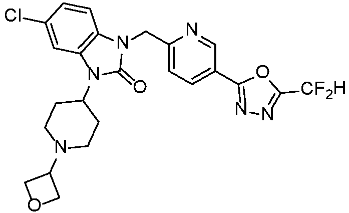 |
| 99 | 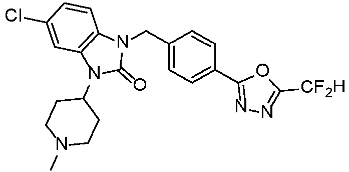 | 100 | 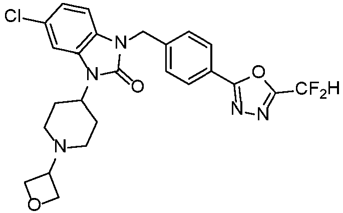 |
| 101 | 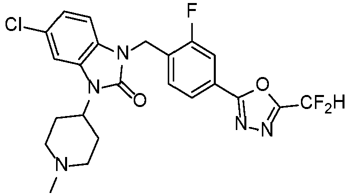 | 102 | 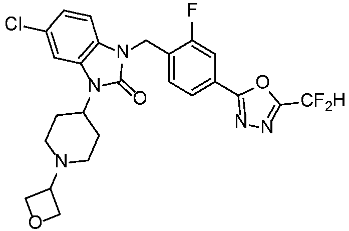 |
| 103 | 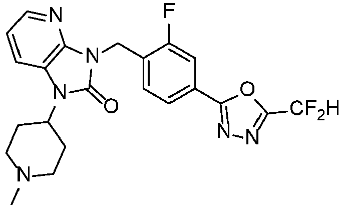 | 104 | 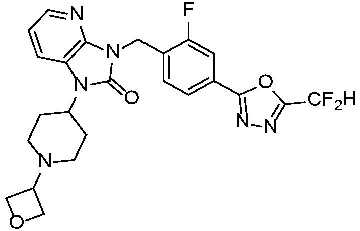 |
| 105 | 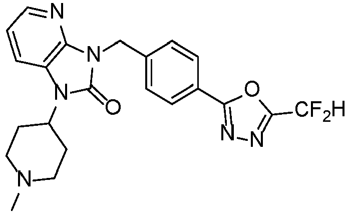 | 106 | 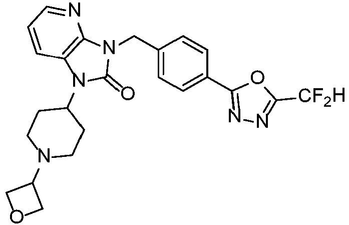 |
| 107 | 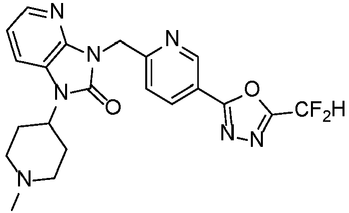 | 108 | 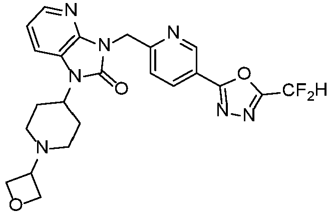 |
| 109 | 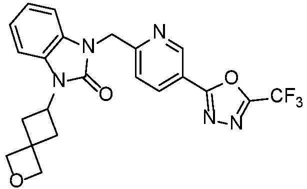 | 110 | 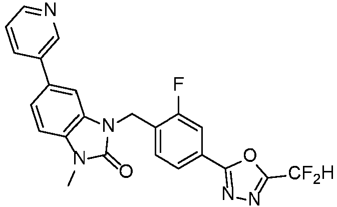 |
| 111 | 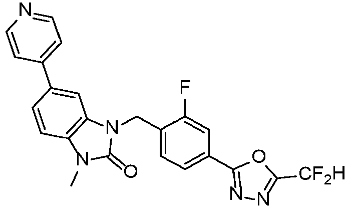 | 112 | 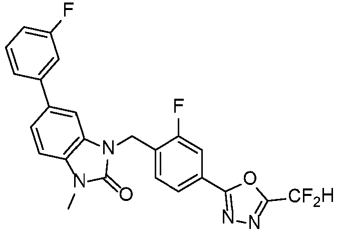 |
| 113 | 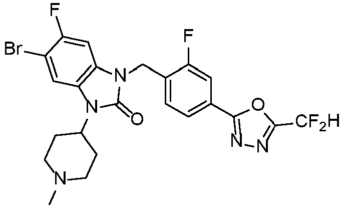 | 114 | 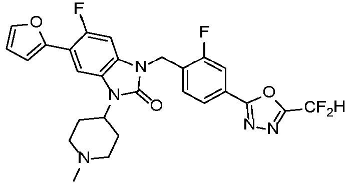 |
| 115 | 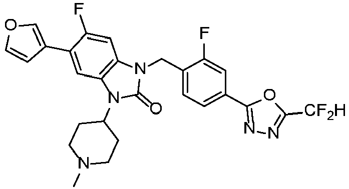 | 116 | 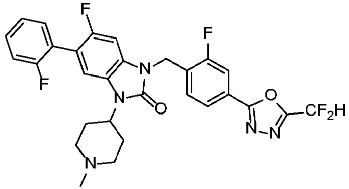 |
| 117 | 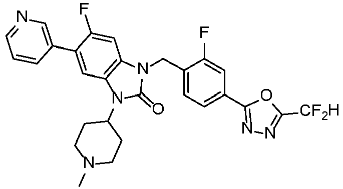 | 118 | 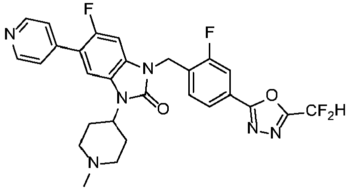 |
| 119 | 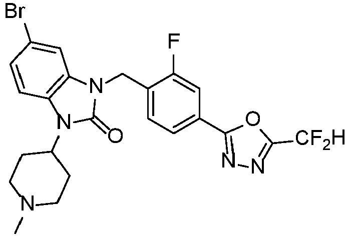 | 120 | 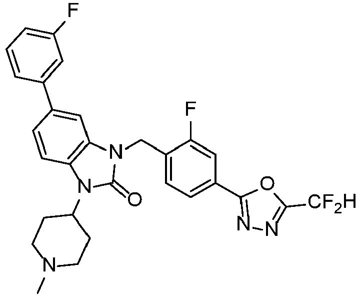 |
| 121 | 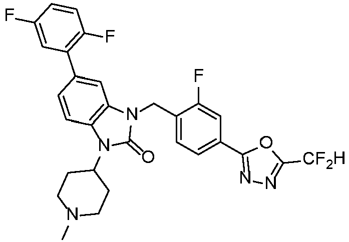 | 122 | 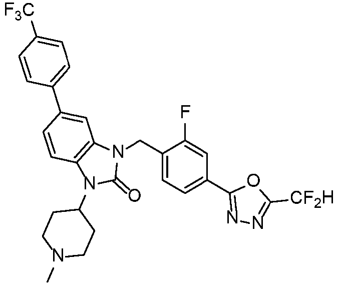 |
| 123 | 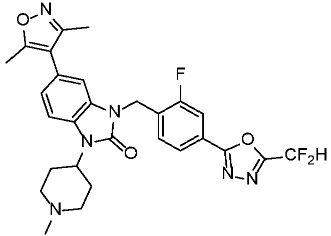 | 124 | 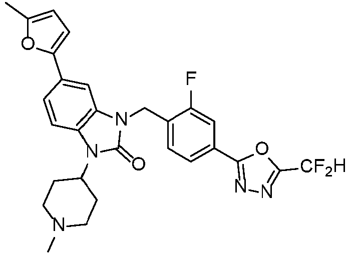 |
| 125 | 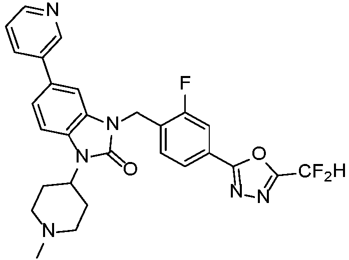 | 126 | 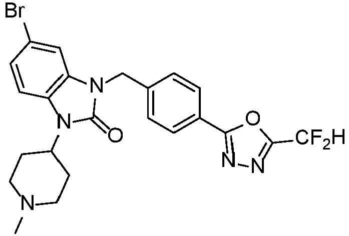 |
| 127 | 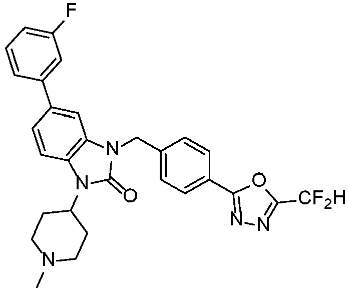 | 128 | 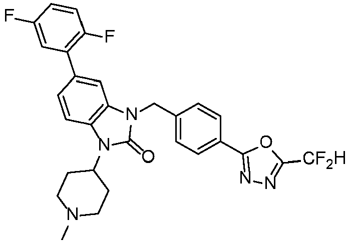 |
| 129 | 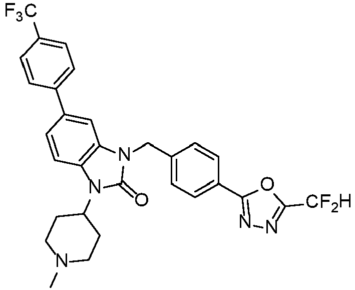 | 130 | 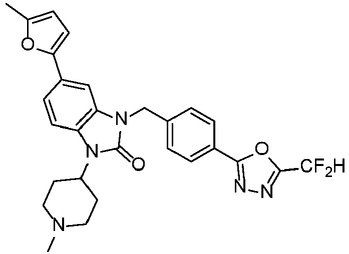 |
| 131 | 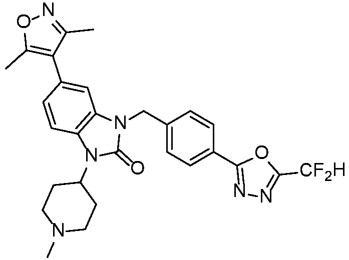 | 132 | 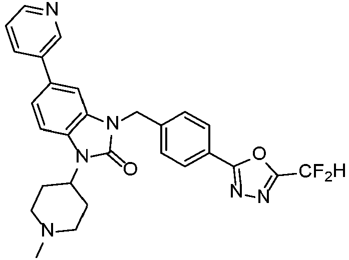 |
| 133 | 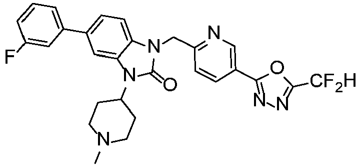 | 134 | 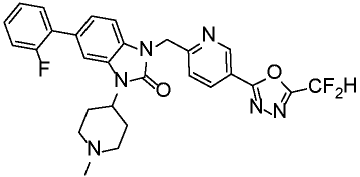 |
| 135 | 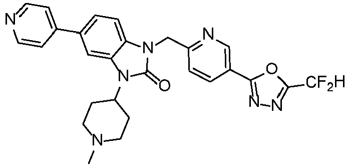 | 136 | 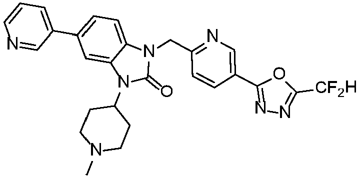 |
| 137 | 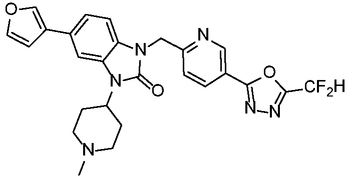 | 138 | 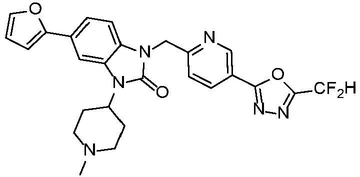 |
| 139 | 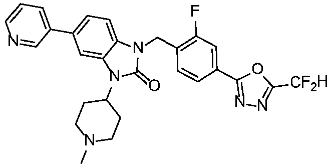 | 140 | 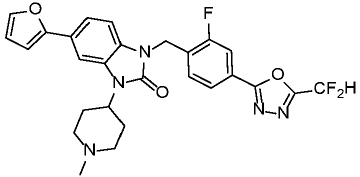 |
| 141 | 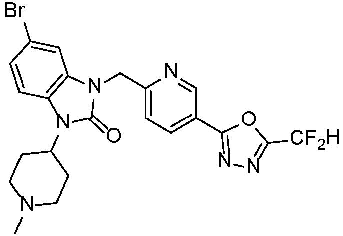 | 142 | 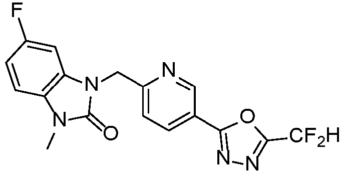 |
| 143 | 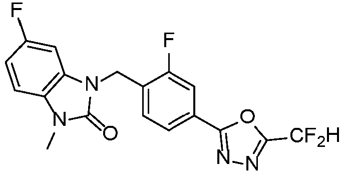 | 144 | 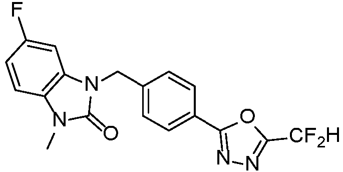 |
| 145 | 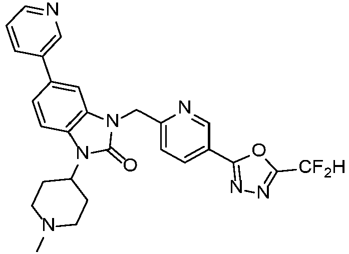 | 146 | 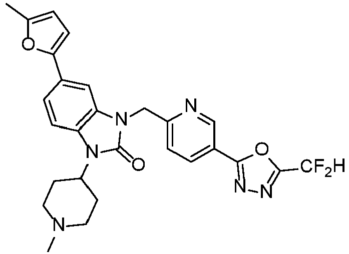 |
| 147 | 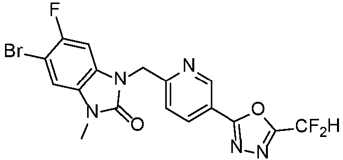 | 148 | 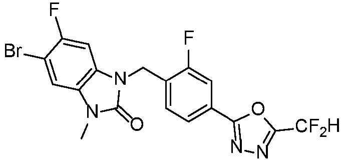 |
| 149 | 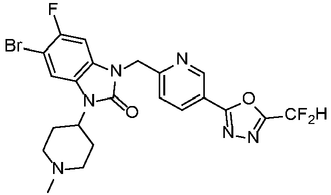 | 150 | 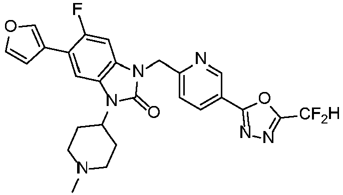 |
| 151 | 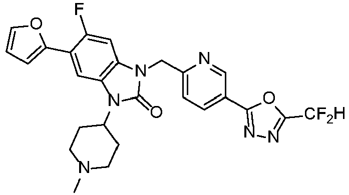 | 152 | 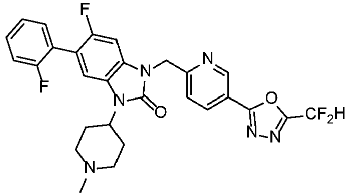 |
| 153 | 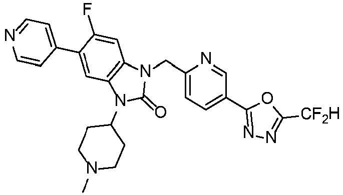 | 154 | 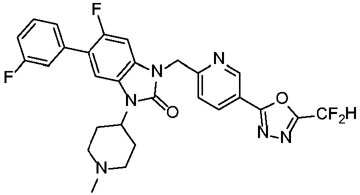 |
| 155 | 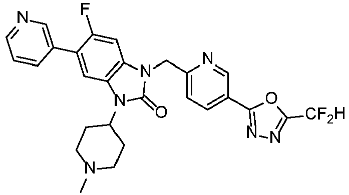 | 156 | 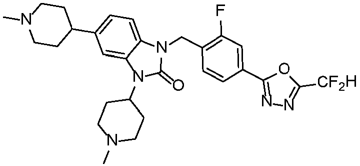 |
| 157 | 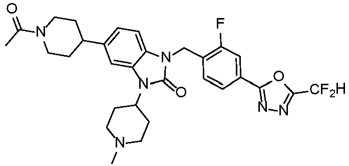 | 158 | 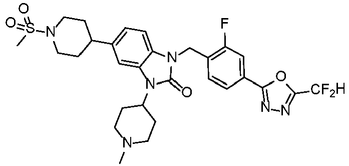 |
| 159 | 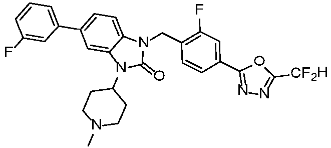 | 160 | 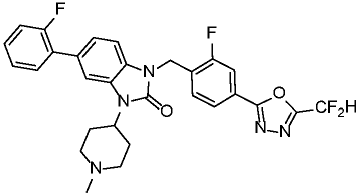 |
| 161 | 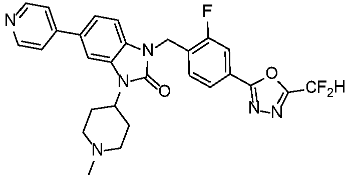 | 162 | 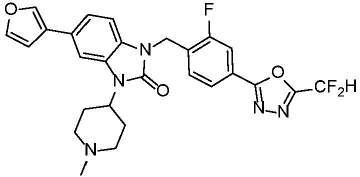 |
| 163 | 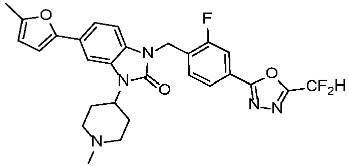 | 164 | 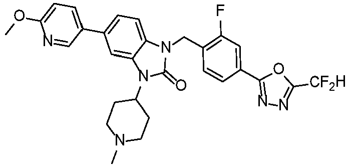 |
| 165 | 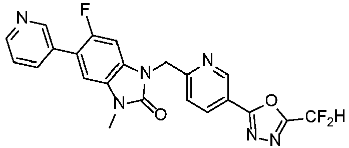 | 166 | 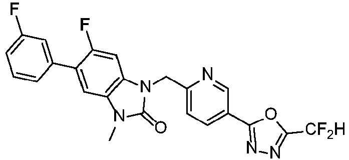 |
| 167 | 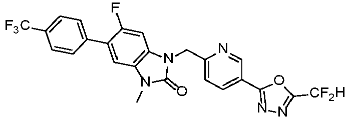 | 168 | 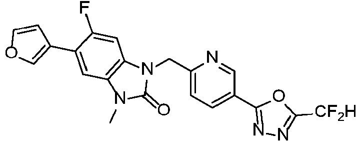 |
| 169 | 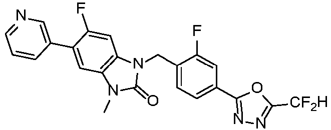 | 170 | 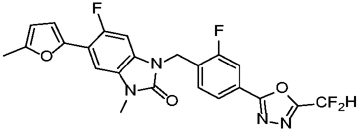 |
| 171 | 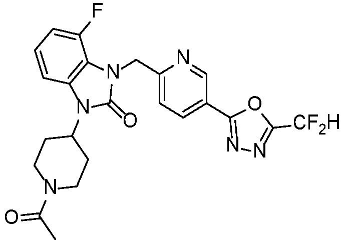 | 172 | 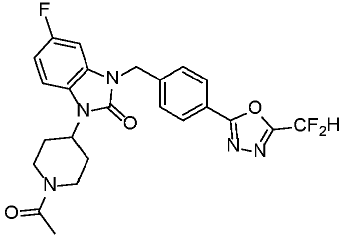 |
| 173 | 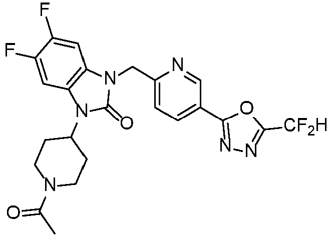 | 174 | 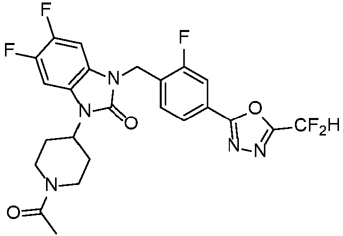 |
| 175 | 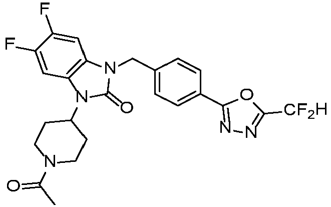 | 176 | 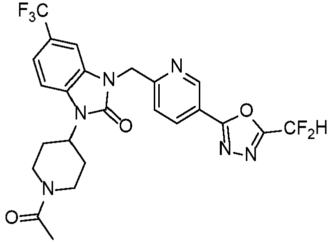 |
| 177 | 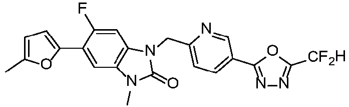 | 178 | 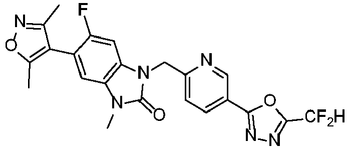 |
| 179 | 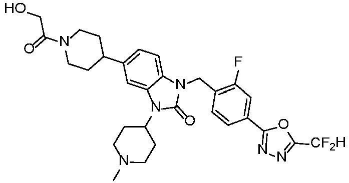 | 180 | 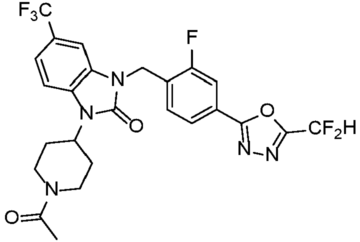 |
| 181 | 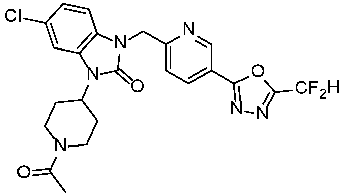 | 182 | 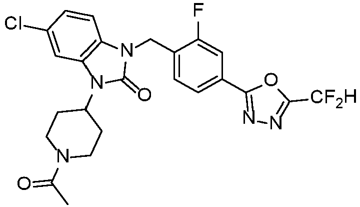 |
| 183 | 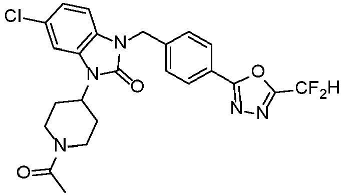 | 184 | 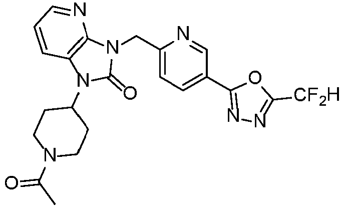 |
| 185 | 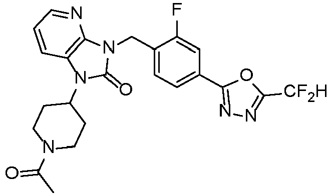 | 186 | 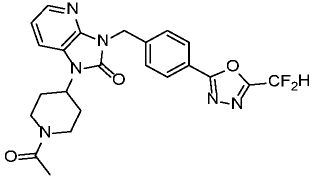 |
| 187 | 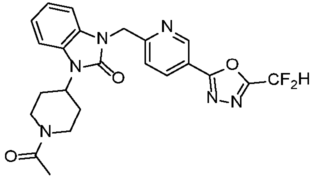 | 188 | 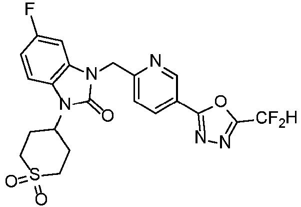 |
| 189 | 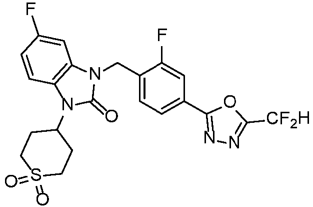 | 190 | 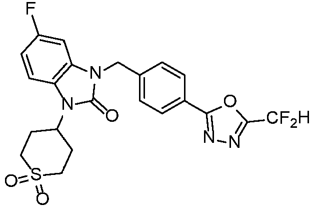 |
| 191 | 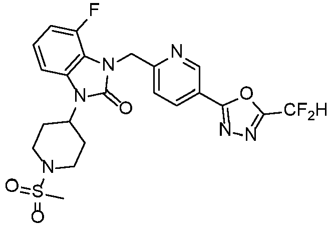 | 192 | 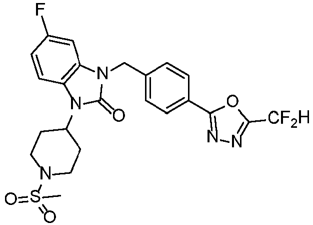 |
| 193 | 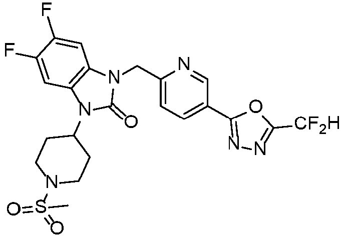 | 194 | 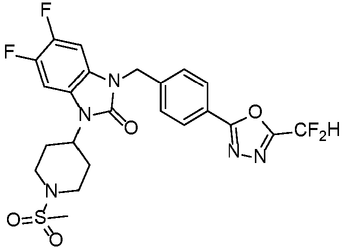 |
| 195 | 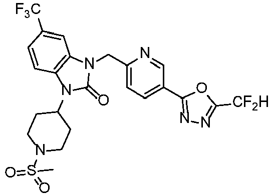 | 196 | 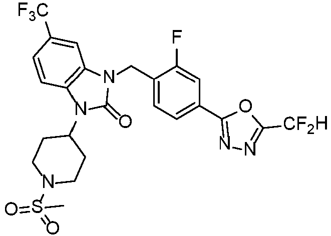 |
| 197 | 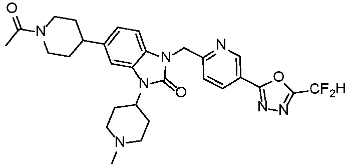 | 198 | 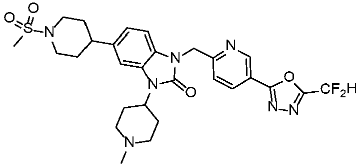 |
| 199 | 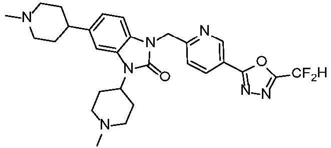 | 200 | 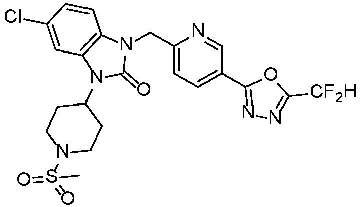 |
| 201 | 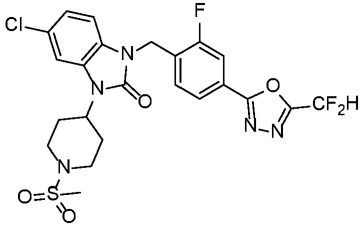 | 202 | 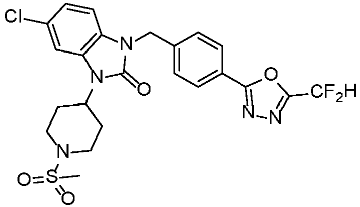 |
| 203 | 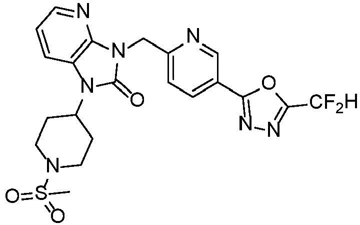 | 204 | 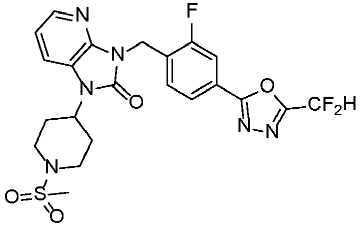 |
| 205 | 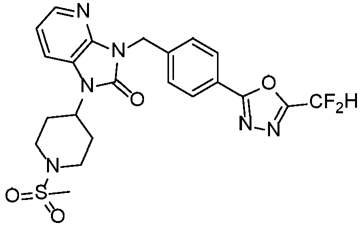 | 206 | 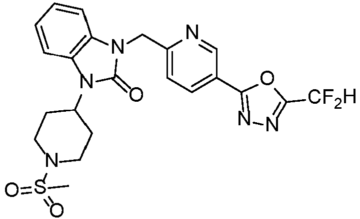 |
| 207 | 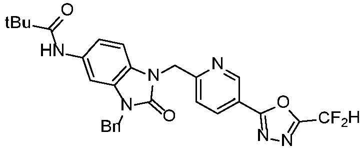 | 208 | 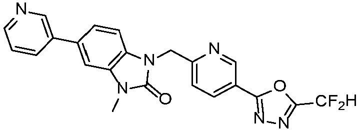 |
| 209 | 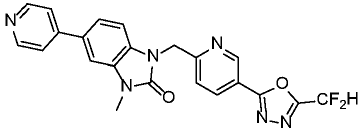 | 210 | 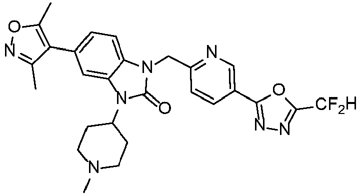 |
| 211 | 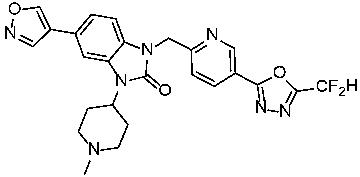 | 212 | 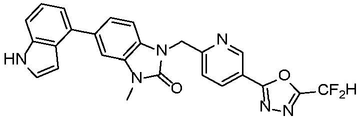 |
| 213 | 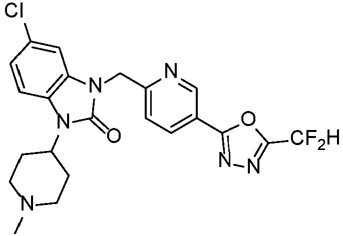 | 214 | 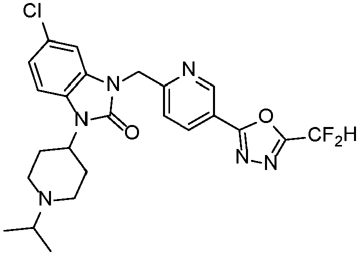 |
| 215 | 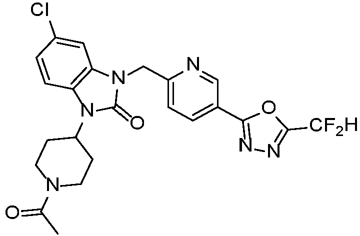 | 216 | 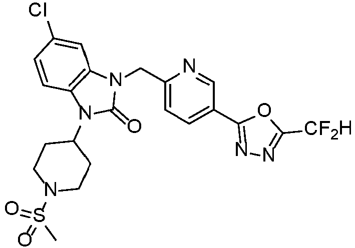 |
| 217 | 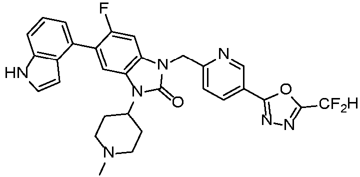 | 218 | 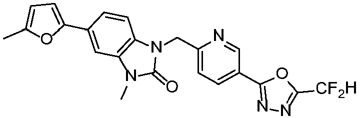 |
| 219 | 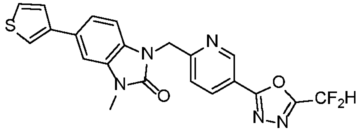 | 220 | 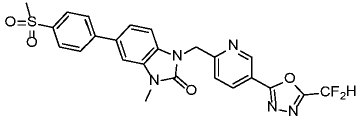 |
| 221 | 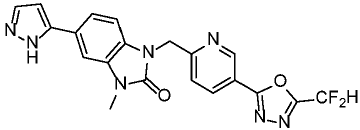 | 222 | 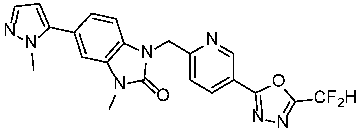 |
| 223 | 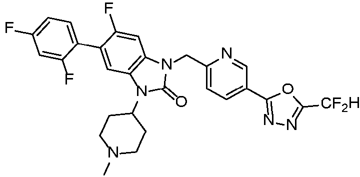 | 224 | 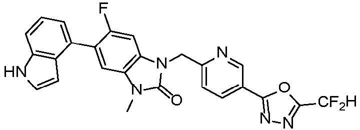 |
| 225 | 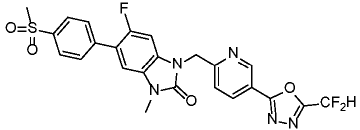 | 226 | 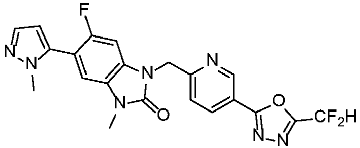 |
| 227 | 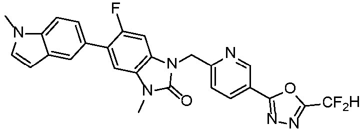 | 228 | 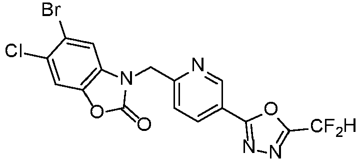 |
| 229 | 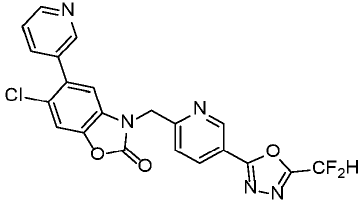 | 230 | 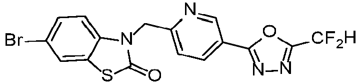 |
| 231 | 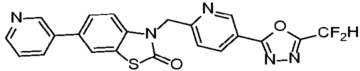 | 232 | 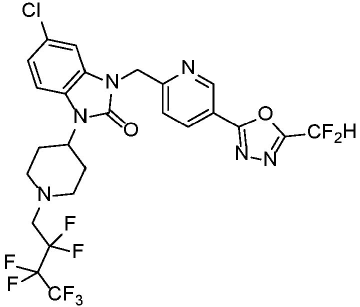 |
| 233 | 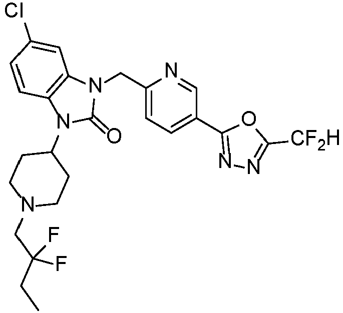 | 234 | 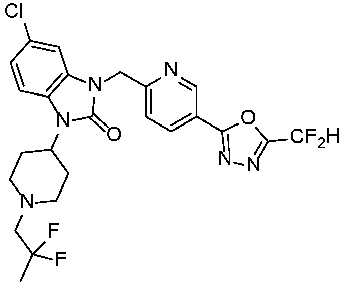 |
| 235 | 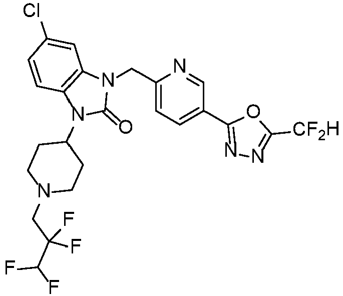 | 236 | 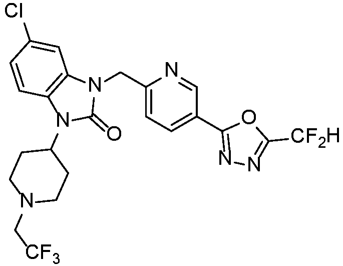 |
| 237 | 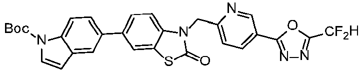 | 238 | 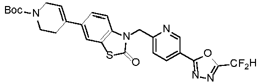 |
| 239 | 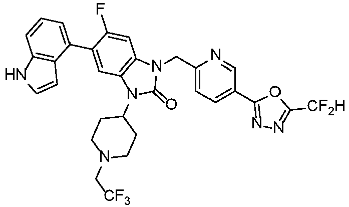 | 240 | 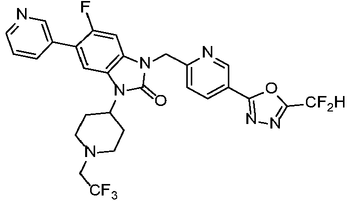 |
| 241 | 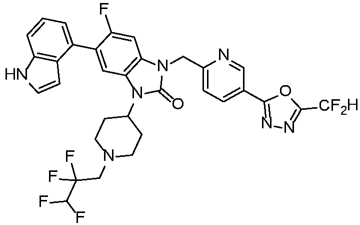 | 242 | 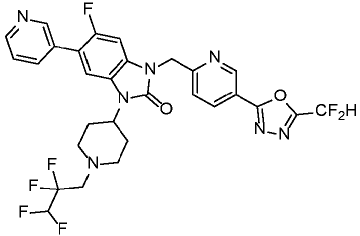 |
| 243 | 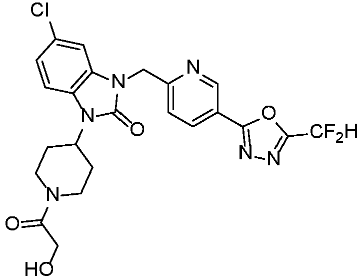 | 244 | 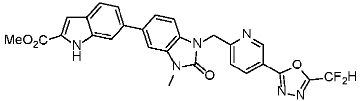 |
| 245 | 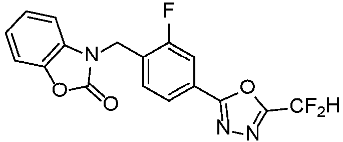 | 246 | 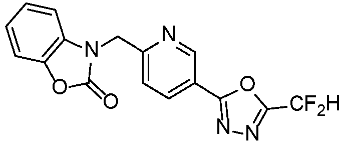 |
| 247 | 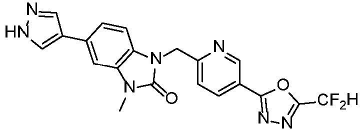 | 248 | 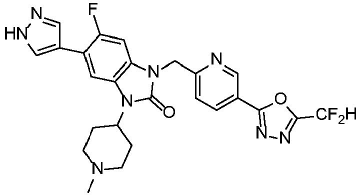 |
| 249 | 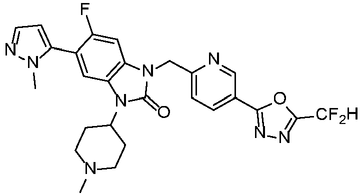 | 250 | 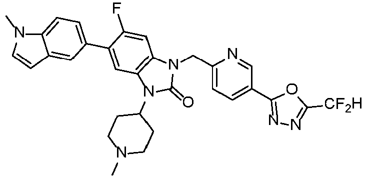 |
| 251 | 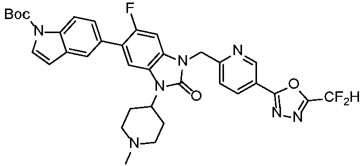 | 252 | 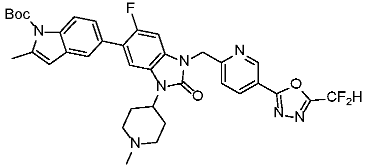 |
| 253 | 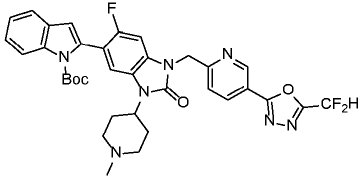 | 254 | 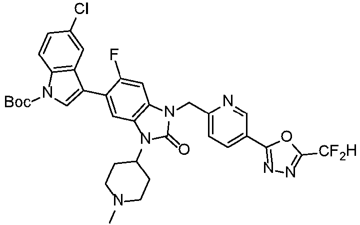 |
| 255 | 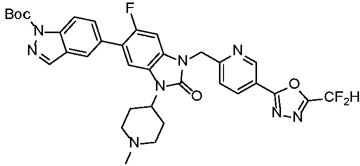 | 256 | 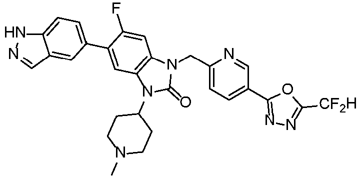 |
| 257 | 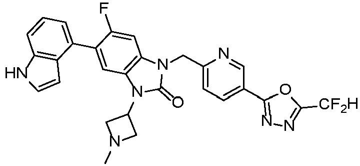 | 258 | 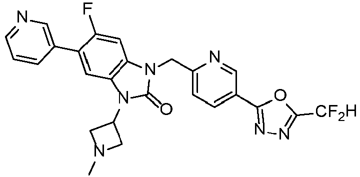 |
| 259 | 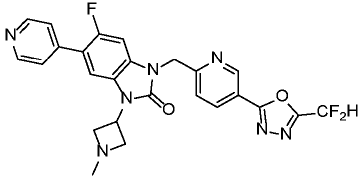 | 260 | 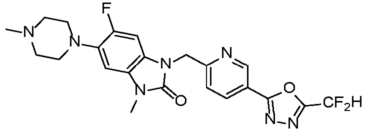 |
| 261 | 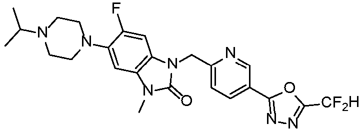 | 262 | 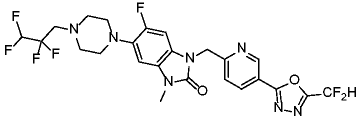 |
| 263 | 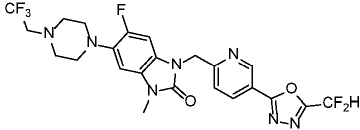 | 264 | 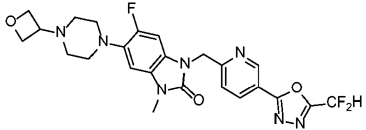 |
| 265 | 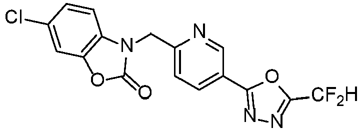 | 266 | 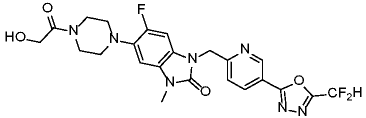 |
| 267 | 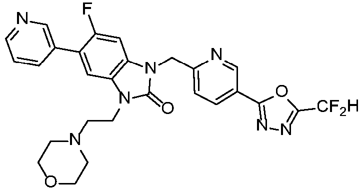 | 268 | 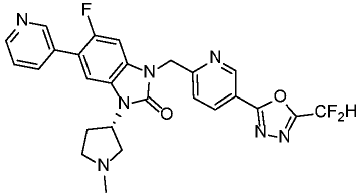 |
| 269 | 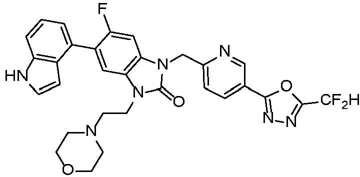 | 270 | 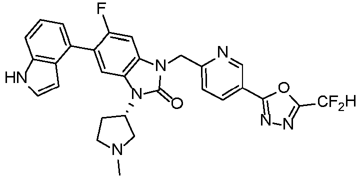 |
| 271 | 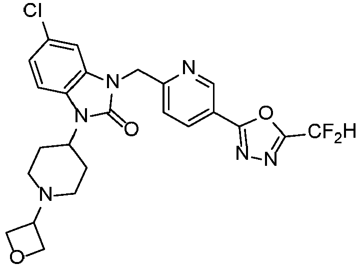 | 272 | 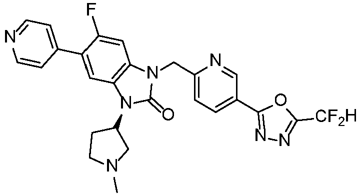 |
| 273 | 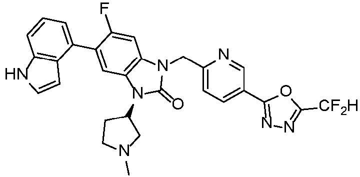 | 274 | 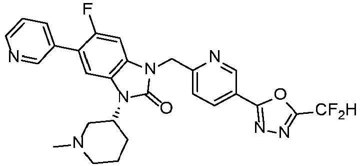 |
| 275 | 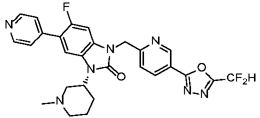 | 276 | 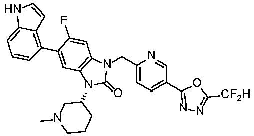 |
| 277 | 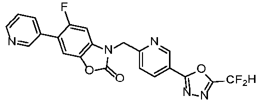 | 278 | 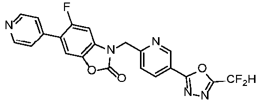 |
| 279 | 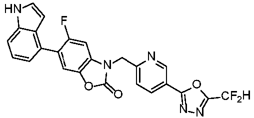 | 280 | 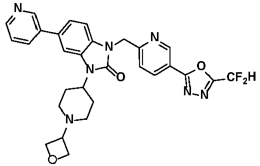 |
| 281 | 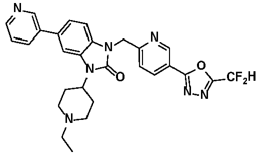 | 282 | 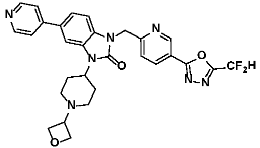 |
| 283 | 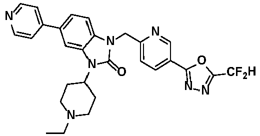 | 284 | 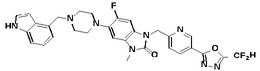 |
| 285 | 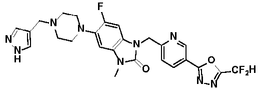 | 286 | 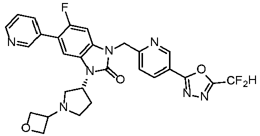 |
| 287 | 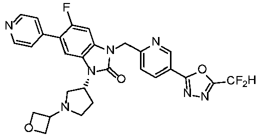 | 288 | 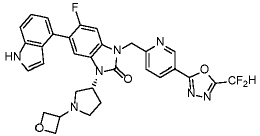 |
| 289 | 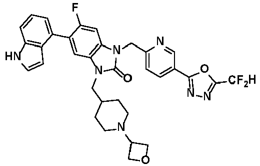 | 290 | 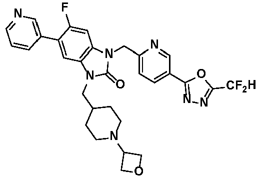 |
| 291 | 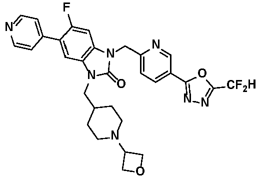 | 292 | 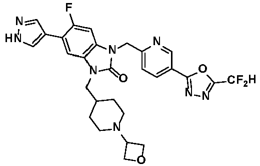 |
| 293 | 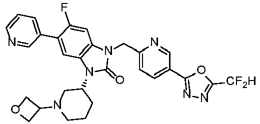 | 294 | 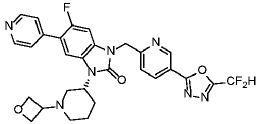 |
| 295 | 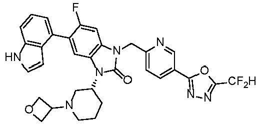 | 296 | 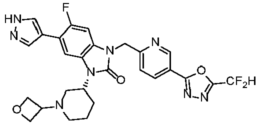 |
| 297 | 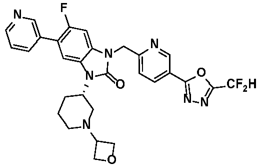 | 298 | 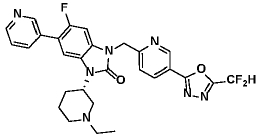 |
| 299 | 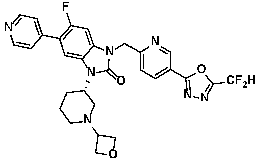 | 300 | 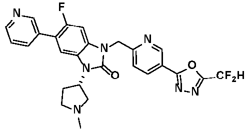 |
| 301 | 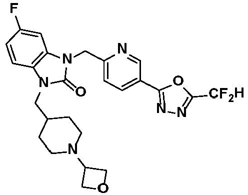 | 302 | 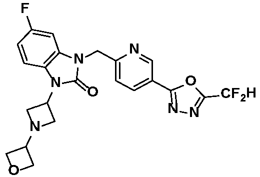 |
| 303 | 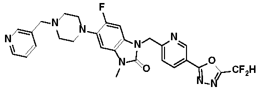 | 304 | 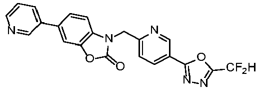 |
| 305 | 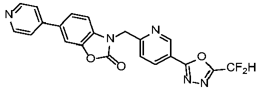 | 306 | 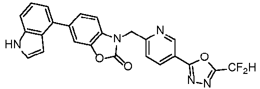 |
| 307 | 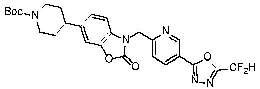 | 308 | 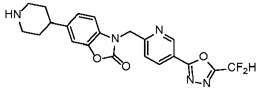 |
| 309 | 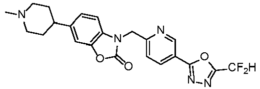 | 310 | 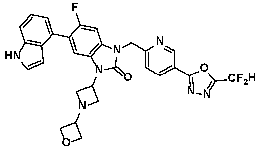 |
| 311 | 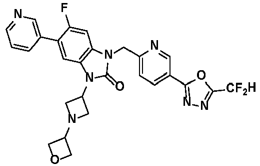 | 312 | 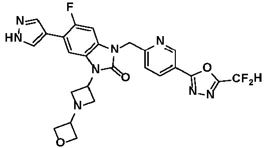 |
| 313 | 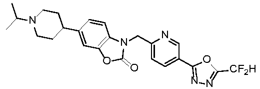 | 314 | 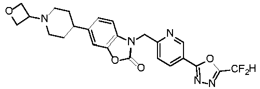 |
| 315 | 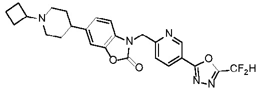 | 316 | 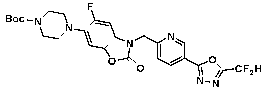 |
| 317 | 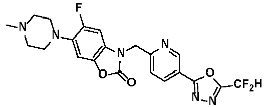 | 318 | 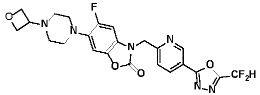 |
| 319 | 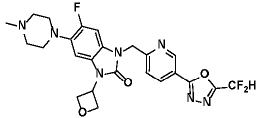 | 320 | 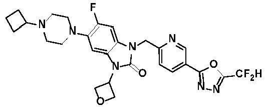 |
| 321 | 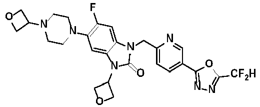 | 322 | 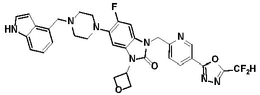 |
| 323 | 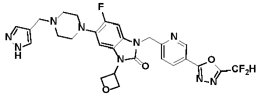 | 324 | 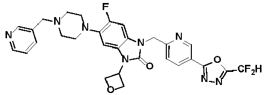 |
| 325 | 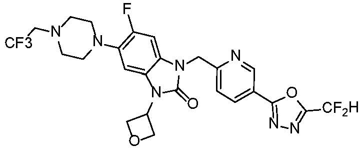 | 326 | 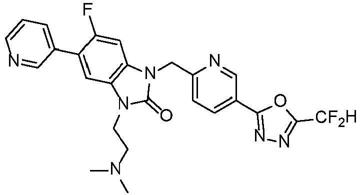 |
| 327 | 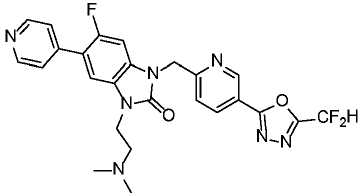 | 328 | 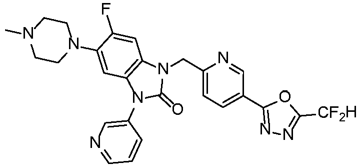 |
| 329 | 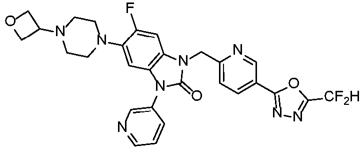 | 330 | 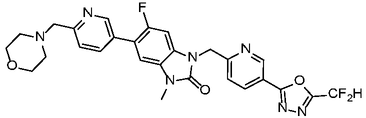 |
| | | | |
| 331 | 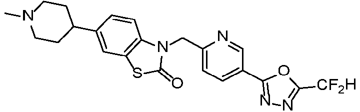 | 332 | 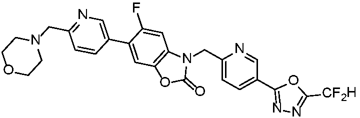 |
| 333 | 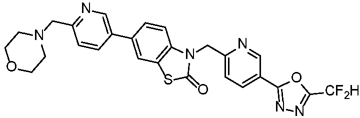 | 334 | 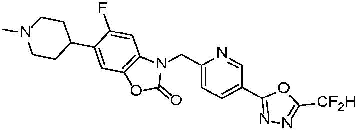 |
| 335 | 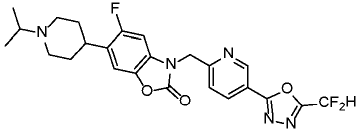 | 336 | 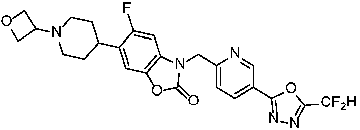 |
| 337 | 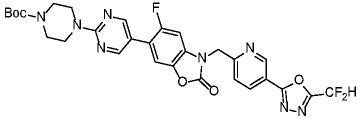 | 338 | 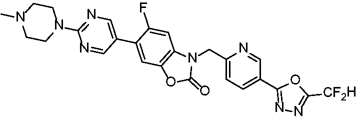 |
| 339 | 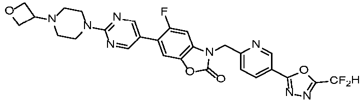 | 340 | 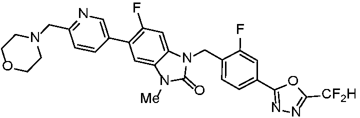 |
| 341 | 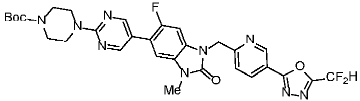 | 342 | 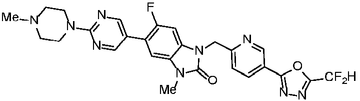 |
| 343 | 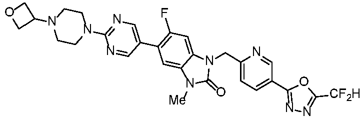 | 344 | 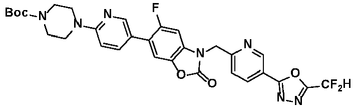 |
| 345 | 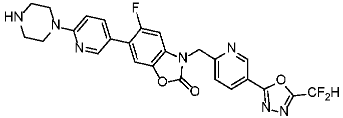 | 346 | 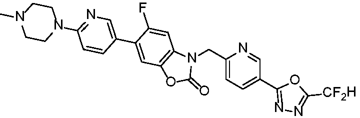 |
| 347 | 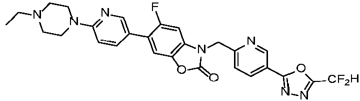 | 348 | 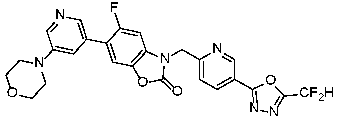 |
| 349 | 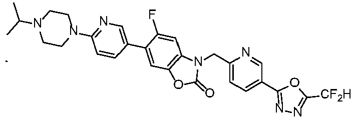 | 350 | 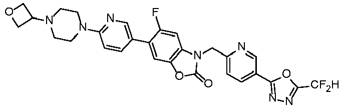 |
| 351 | 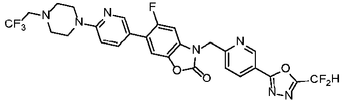 | 352 | 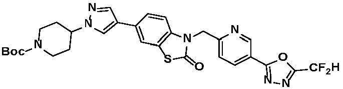 |
| 353 | 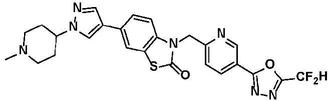 | 354 | 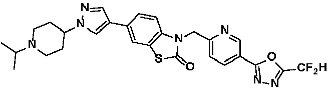 |
| 355 | 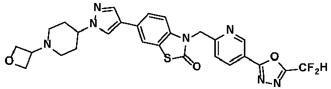 | 356 | 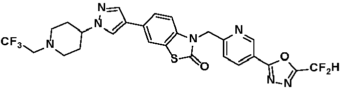 |
| 357 | 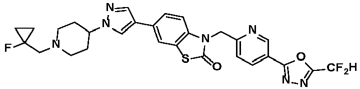 | 358 | 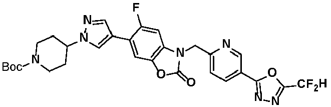 |
| 359 | 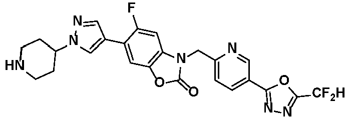 | 360 | 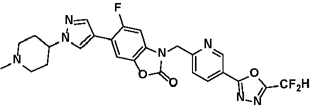 |
| 361 | 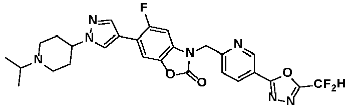 | 362 | 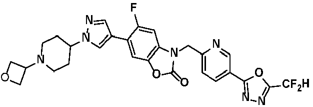 |
| 363 | 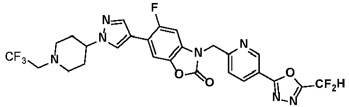 | 364 | 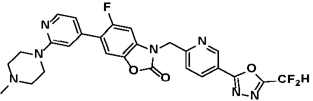 |
| 365 | 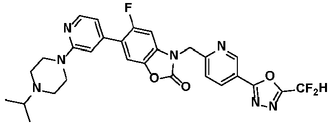 | 366 | 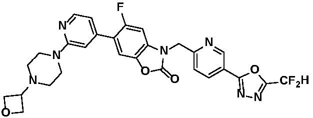 |
| 367 | 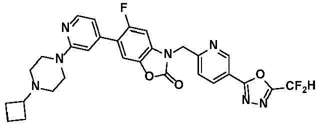 | 368 | 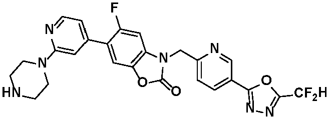 |

### A method for preparing 1,3,4-oxadiazole derivative compounds

The present invention provides a method for preparing 1,3,4-oxadiazole derivative compounds represented by the chemical formula I, stereoisomers thereof or pharmaceutically acceptable salts thereof.

A preferable method for preparing the 1,3,4-oxadiazole derivative compounds represented by the chemical formula I, stereoisomers thereof or pharmaceutically acceptable salts thereof is the same as shown in following reaction formulas 1 to 17, and even a preparation method modified at a level apparent to those skilled in the art is also included therein.

In each of [Reaction Formula 1] to [Reaction Formula 17], "A" may be phenylene as arylene, but is not particularly limited thereto. "A" may be heteroarylene. Also, in [Reaction Formula 1] to [Reaction Formula 17], R₁ to R₅, Z₁ to Z₄, a, b and X are each substantially the same as defined in the chemical formula I, and L₃ represents C₁₋₄ alkylene. In [Reaction Formula 1] to [Reaction Formula 17], "Halo" means a halogen atom of F, Cl, Br or I. Also, in [Reaction Formula 1] to [Reaction Formula 17], "PG" means a "protecting group" and may include tert-butyloxycarbonyl (Boc), benzyloxycarbonyl (Cbz) or the like. Furthermore, in [Reaction Formula 1] to [Reaction Formula 17], "X₁" means O or S.

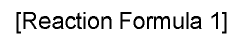

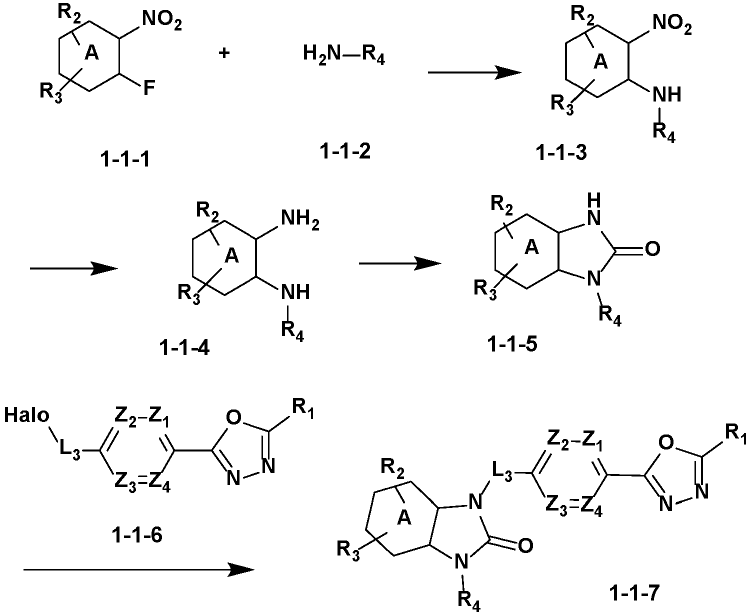

The above [Reaction Formula 1] shows a synthesis method of 1,3,4-oxadiazole compounds having a benzimidazolone structure, and a compound of the chemical formula 1-1-1 reacts with a compound of the chemical formula 1-1-2 so as to prepare a compound of the chemical formula 1-1-3. Then, the resulting compound is used to prepare a compound of the chemical formula 1-1-4. After that, the compound of the chemical formula 1-1-4 is subjected to a cyclization reaction so as to prepare a compound of the chemical formula 1-1-5. After that, the resulting compound is subjected to a substitution reaction with a compound of the chemical formula 1-1-6 so as to prepare a compound of the chemical formula 1-1-7.

In the present invention, the examples of the compounds prepared according to a method as shown in the above reaction formula 1 include compounds **2, 3, 11 to 13, 24 to 26, 34 to 37, 142 to 144, 147, 148** and the like.

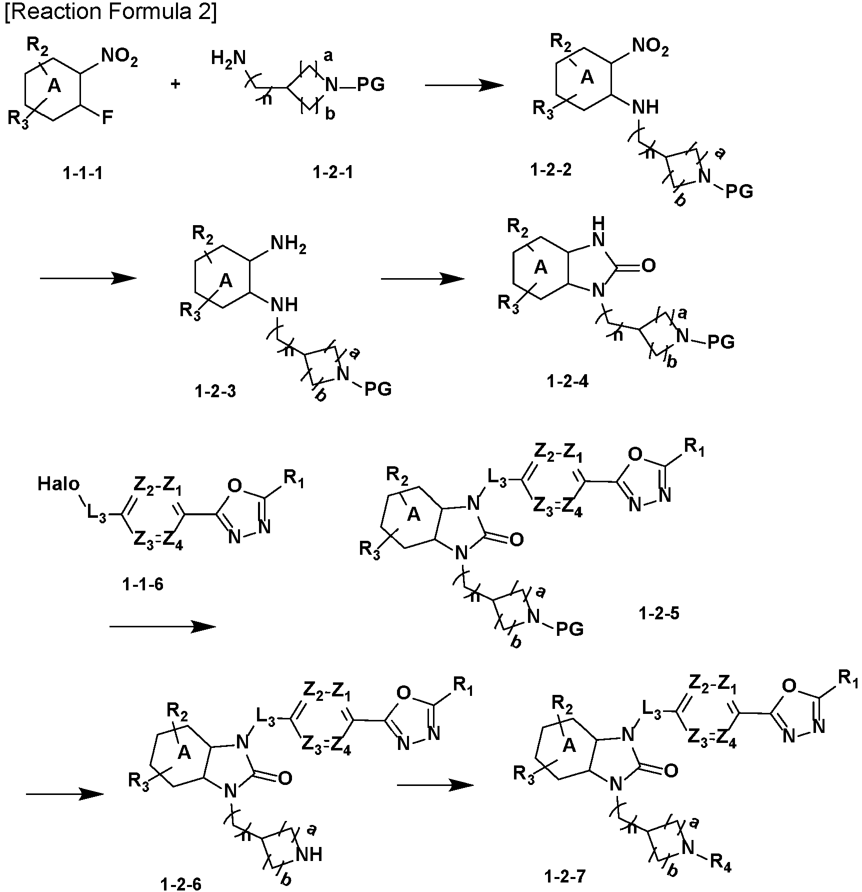

The above [Reaction Formula 2] shows a synthesis method of 1,3,4-oxadiazole compounds having a benzimidazolone structure, and a compound of the chemical formula 1-1-1 is subjected to a substitution reaction with a compound of the chemical formula 1-2-1 so as to prepare a compound of the chemical formula 1-2-2, and then is subjected to a reduction reaction so as to prepare a compound of the chemical formula 1-2-3. After that, the compound of the chemical formula 1-2-3 is subjected to a cyclization reaction so as to prepare a compound of the chemical formula 1-2-4, and then is subjected to a substitution reaction with a compound of the chemical formula 1-1-6 so as to prepare a compound of the chemical formula 1-2-5. A protecting group is removed from the compound of the chemical formula 1-2-5 so as to prepare a compound of the chemical formula 1-2-6, and then a substitution reaction, a reductive amination reaction and an acylation reaction are performed to prepare a compound of the chemical formula 1-2-7.

In the present invention, the examples of the compounds prepared according to a method as shown in the above reaction formula 2 include compounds **40** to **77, 79** to **108, 113, 125, 132, 141, 149, 172 to 176, 180 to 186, 191 to 196, 200 to 206, 213 to 216, 232 to 236, 243, 271, 301, 302** and the like.

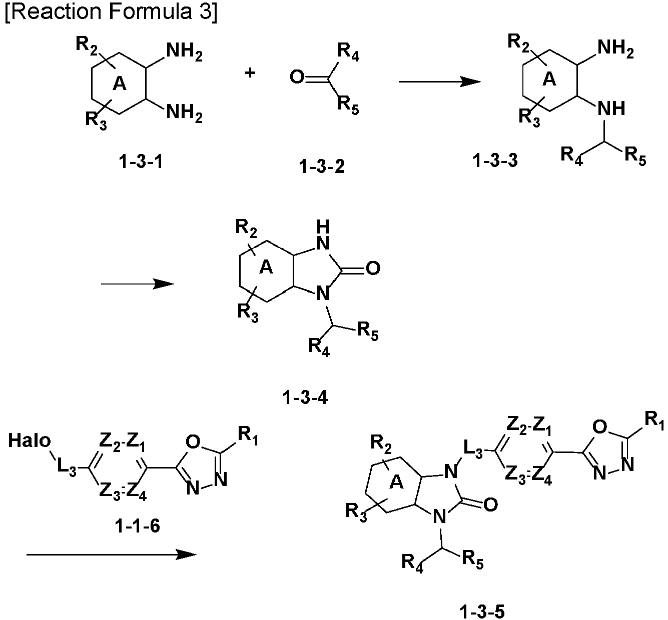

The above [Reaction Formula 3] shows a synthesis method of 1,3,4-oxadiazole compounds having a benzimidazolone structure, and a compound of the chemical formula 1-3-1 is subjected to a reductive amination reaction with a compound of the chemical formula 1-3-2 so as to prepare a compound of the chemical formula 1-3-3, and then is subjected to a cyclization reaction so as to prepare a compound of the chemical formula 1-3-4. After that, the compound of the chemical formula 1-3-4 is subjected to a substitution reaction with a compound of the chemical formula 1-1-6 so as to prepare a compound of the chemical formula 1-3-5.

In the present invention, the examples of the compounds prepared according to a method as shown in the above reaction formula 3 include compounds **27, 28, 29, 109, 188, 189, 190** and the like.

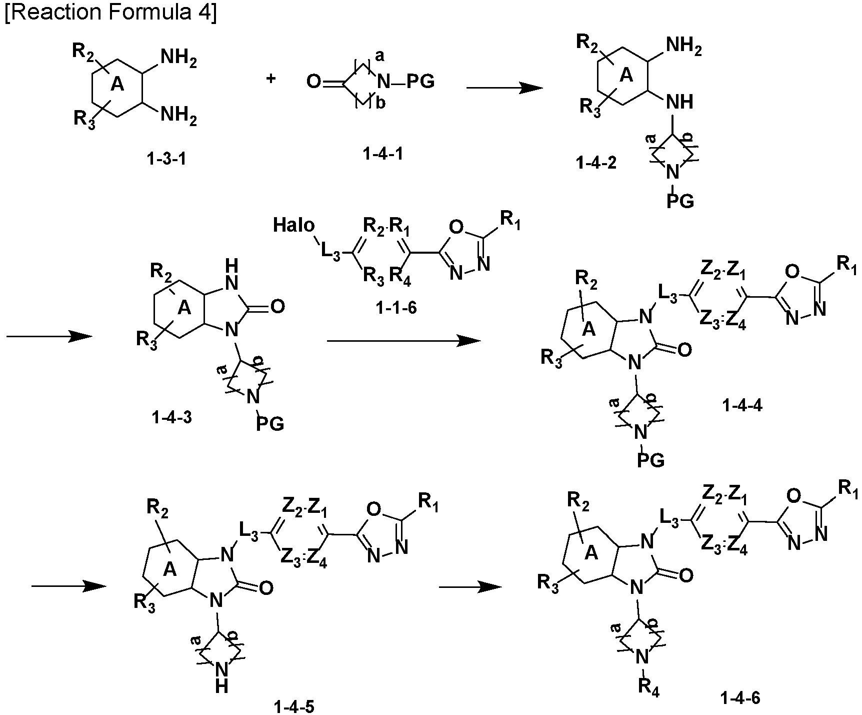

The above [Reaction Formula 4] shows a synthesis method of 1,3,4-oxadiazole compounds having a benzimidazolone structure, and a compound of the chemical formula 1-3-1 is subjected to a reductive amination reaction with a compound of the chemical formula 1-4-1 so as to prepare a compound of the chemical formula 1-4-2, and then is subjected to a cyclization reaction so as to prepare a compound of the chemical formula 1-4-3. After that, the compound of the chemical formula 1-4-3 is subjected to a substitution reaction with a compound of the chemical formula 1-1-6 so as to prepare a compound of the chemical formula 1-4-4. A protecting group is removed from the compound of the chemical formula 1-4-4 so as to prepare a compound of the chemical formula 1-4-5, and then a substitution reaction, a reductive amination reaction and an acylation reaction are performed to prepare a compound of the chemical formula 1-4-6.

In the present invention, the examples of the compounds prepared according to a method as shown in the above reaction formula 4 include compounds **1, 4 to 7, 14 to 23, 78, 187** and the like.

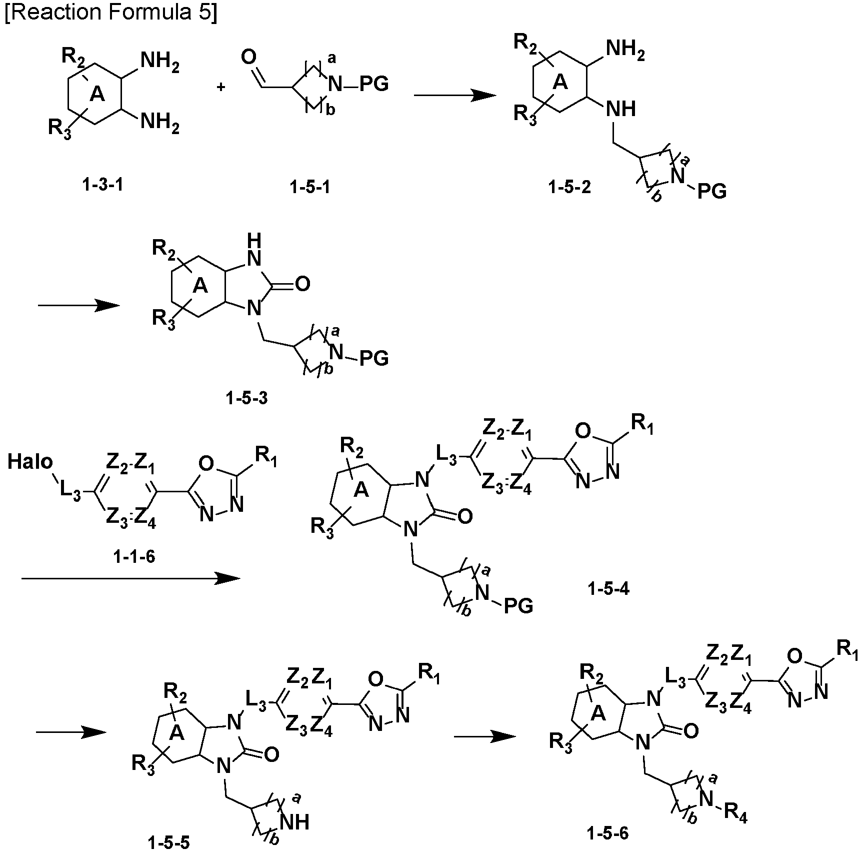

The above [Reaction Formula 5] shows a synthesis method of 1,3,4-oxadiazole compounds having a benzimidazolone structure, and a compound of the chemical formula 1-3-1 is subjected to a reductive amination reaction with a compound of the chemical formula 1-5-1 so as to prepare a compound of the chemical formula 1-5-2, and then is subjected to a cyclization reaction so as to prepare a compound of the chemical formula 1-5-3. After that, the compound of the chemical formula 1-5-3 is subjected to a substitution reaction with a compound of the chemical formula 1-1-6 so as to prepare a compound of the chemical formula 1-5-4. A protecting group is removed from the compound of the chemical formula 1-5-4 so as to prepare a compound of the chemical formula 1-5-5, and then a substitution reaction, a reductive amination reaction and an acylation reaction are performed to prepare a compound of the chemical formula 1-5-6.

In the present invention, the examples of the compounds prepared according to a method as shown in the above reaction formula 5 include compounds **10, 38, 39** and the like.

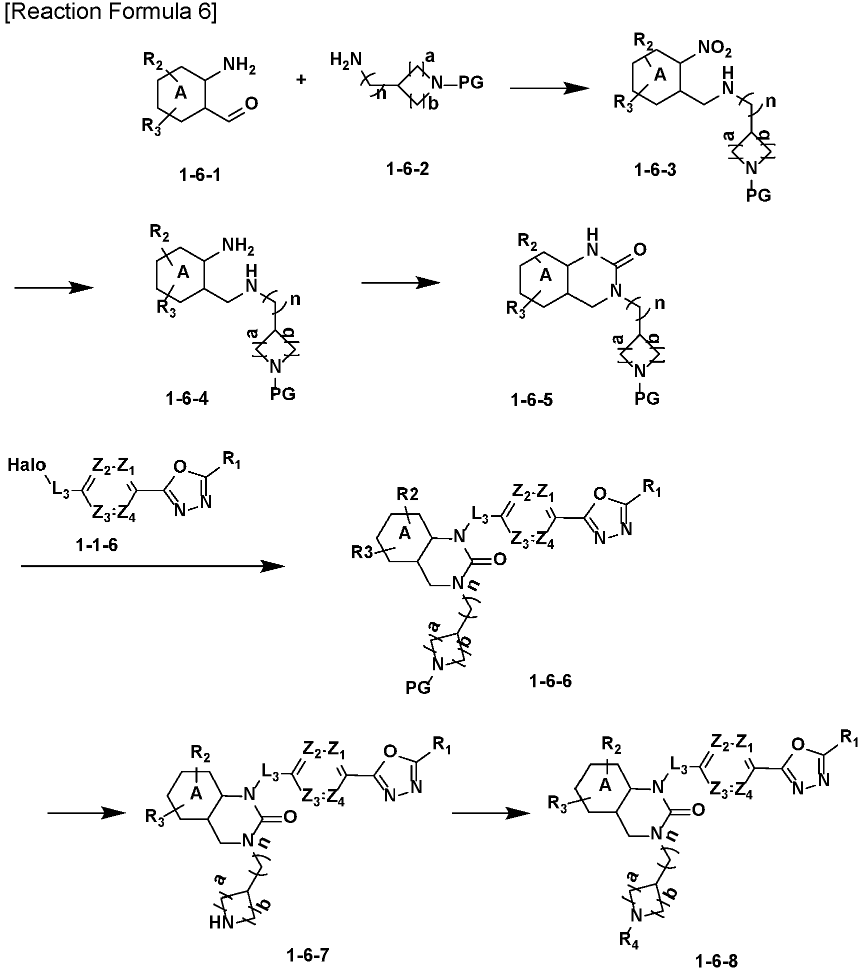

The above [Reaction Formula 6] shows a synthesis method of 1,3,4-oxadiazole compounds having a 3,4-dihydro quinazoline-2(1*H*)-one structure, and a compound of the chemical formula 1-6-1 is subjected to a reductive amination reaction with a compound of the chemical formula 1-6-2 so as to prepare a compound of the chemical formula 1-6-3, and then is subjected to a reduction reaction so as to prepare a compound of the chemical formula 1-6-4. After that, a cyclization reaction is performed to prepare a compound of the chemical formula 1-6-5, after which the compound of the chemical formula 1-6-5 is subjected to a substitution reaction with a compound of the chemical formula 1-1-6 so as to prepare a compound of the chemical formula 1-6-6. A protecting group is removed from the compound of the chemical formula 1-6-6 so as to prepare a compound of the chemical formula 1-6-7, and then a substitution reaction, a reductive amination reaction and an acylation reaction are performed to prepare a compound of the chemical formula 1-6-8.

In the present invention, the examples of the compounds prepared according to a method as shown in the above reaction formula 6 include compounds **8, 9, 32, 33** and the like.

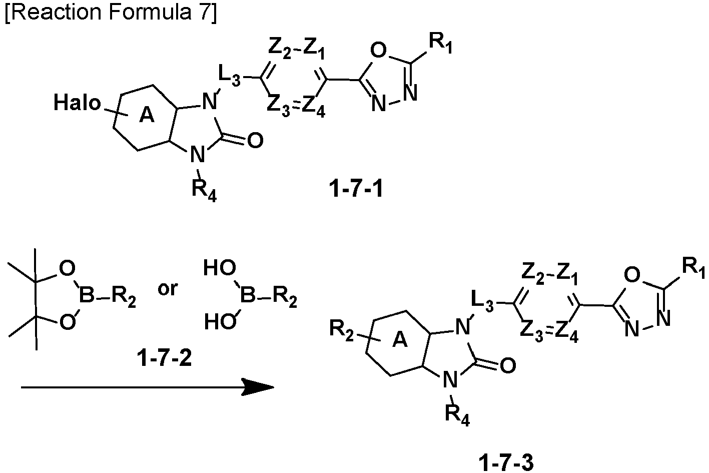

The above [Reaction Formula 7] shows a synthesis method of 1,3,4-oxadiazole compounds having a benzimidazolone structure, and a compound of the chemical formula 1-7-1 is subjected to C-C coupling (Suzuki reaction) with a compound of the chemical formula 1-7-2 so as to prepare a compound of the chemical formula 1-7-3.

In the present invention, the examples of the compounds prepared according to a method as shown in the above reaction formula 7 include compounds **110 to 124, 126 to 131, 133 to 140, 145, 146, 150 to 155, 159 to 170, 177, 178, 208 to 212, 217 to 227, 239 to 244, 247 to 259, 267 to 270, 272 to 283, 286 to 300, 304 to 306, 310 to 312, 326, 327, 332, 333, 340, 341** and the like.

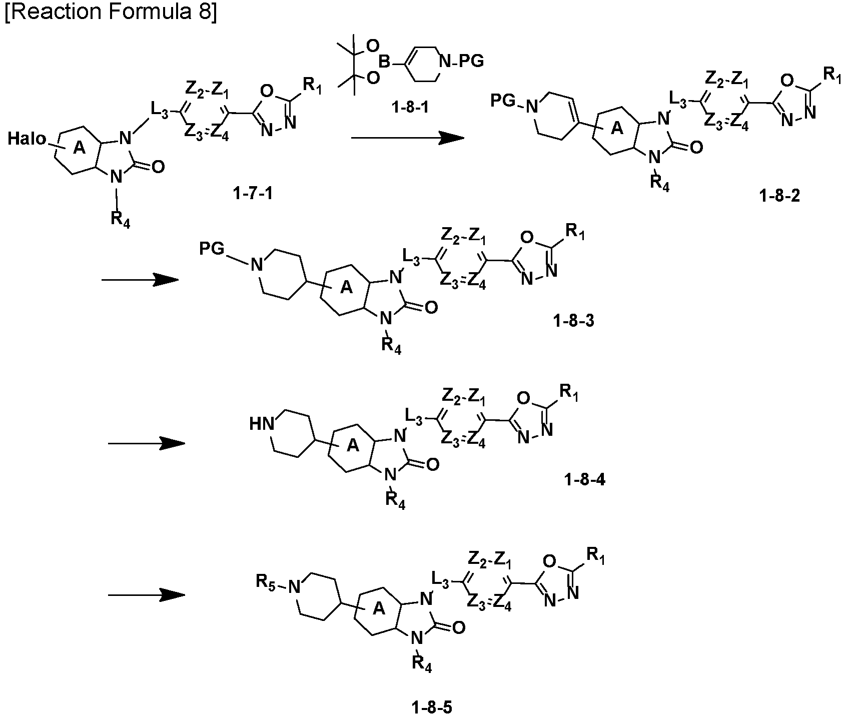

The above [Reaction Formula 8] shows a synthesis method of 1,3,4-oxadiazole compounds having a benzimidazolone structure, and a compound of the chemical formula 1-7-1 is subjected to C-C coupling (Suzuki reaction) with a compound of the chemical formula 1-8-1 so as to prepare a compound of the chemical formula 1-8-2, after which a reduction reaction is performed to prepare a compound of the chemical formula 1-8-3. After that, a protecting group is removed from the compound of the chemical formula 1-8-3 so as to prepare a compound of the chemical formula 1-8-4, and then a substitution reaction, a reductive amination reaction and an acylation reaction are performed to prepare a compound of the chemical formula 1-8-5.

In the present invention, the examples of the compounds prepared according to a method as shown in the above reaction formula 8 include compounds **156, 157, 158, 179, 197, 198, 199, 307 to 309, 313 to 315, 331, 334 to 336** and the like.

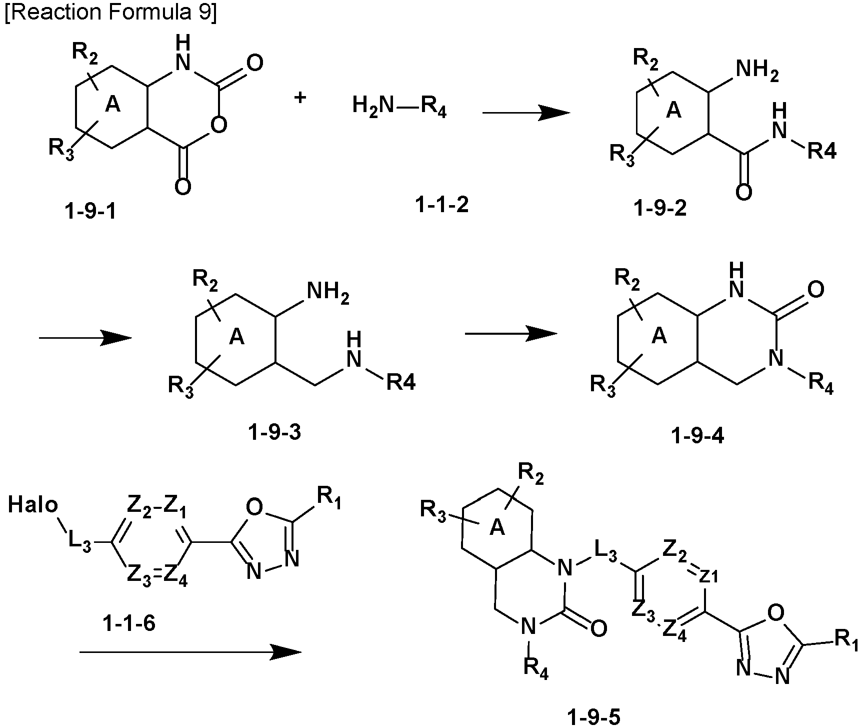

The above [Reaction Formula 9] shows a synthesis method of 1,3,4-oxadiazole compounds having a 3,4-dihydro quinazoline-2(1H)-one structure, and a compound of the chemical formula 1-9-1 reacts with a compound of the chemical formula 1-1-2 so as to prepare a compound of the chemical formula 1-9-2, and then a reduction reaction is performed to prepare a compound of the chemical formula 1-9-3. After that, the compound of the chemical formula 1-9-3 is subjected to a cyclization reaction so as to prepare a compound of the chemical formula 1-9-4, and then is subjected to a substitution reaction with a compound of the chemical formula 1-1-6 so as to prepare a compound of the chemical formula 1-9-5.

In the present invention, the examples of the compounds prepared according to a method as shown in the above reaction formula 9 include compounds **30, 31** and the like.

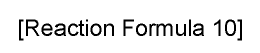

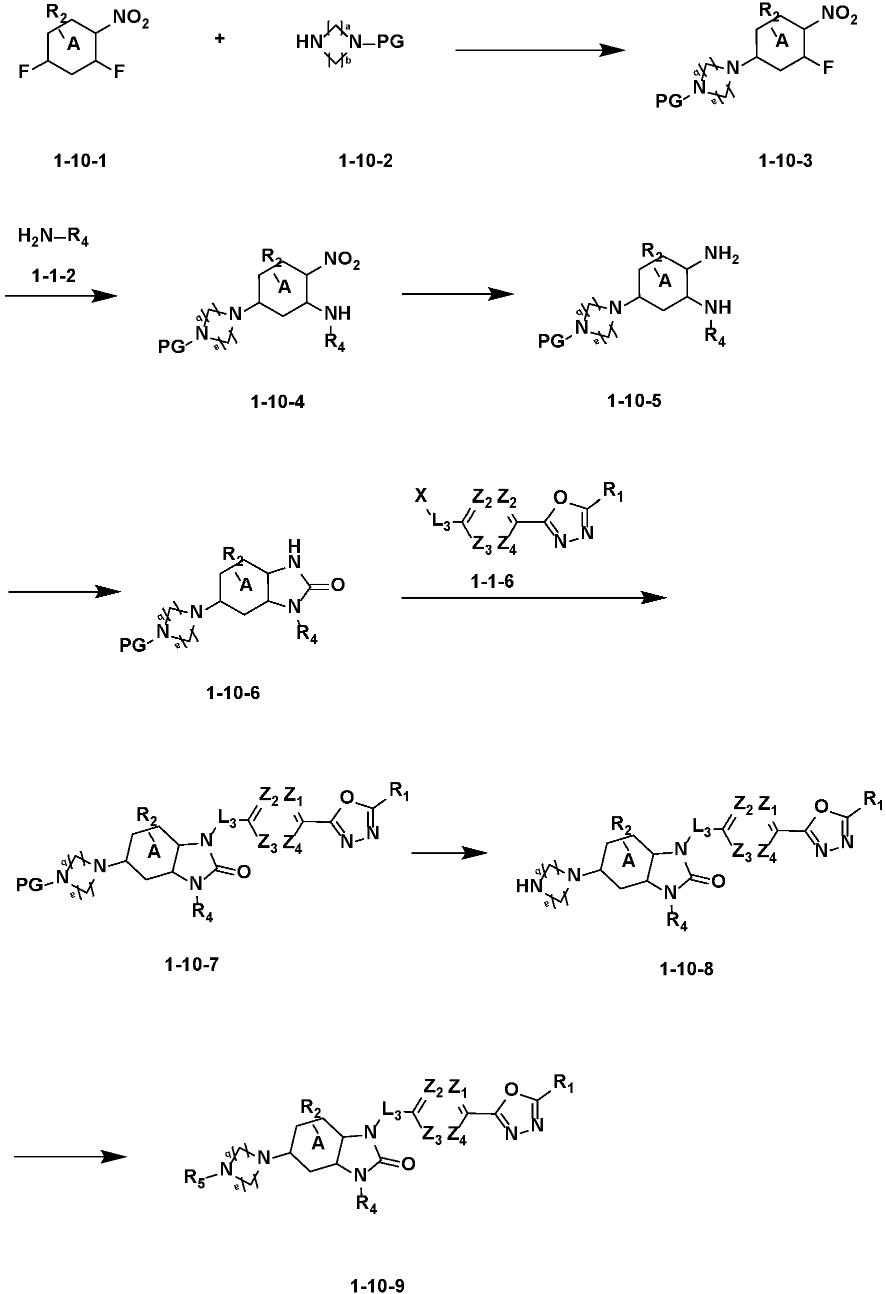

The above [Reaction Formula 10] shows a synthesis method of 1,3,4-oxadiazole compounds having a benzimidazolone structure, and a compound of the chemical formula 1-10-1 reacts with a compound of the chemical formula 1-10-2 so as to prepare a compound of the chemical formula 1-10-3, and then is subjected to a substitution reaction with a compound of the chemical formula 1-1-2 so as to prepare a compound of the chemical formula 1-10-4. Then, the compound of the chemical formula 1-10-4 is subjected to a reduction reaction so as to prepare a compound of the chemical formula 1-10-5, and then a cyclization reaction is performed to prepare a compound of the chemical formula 1-10-6. After that, the compound of the chemical formula 1-10-6 is subjected to a substitution reaction with a compound of the chemical formula 1-1-6 so as to prepare a compound of the chemical formula 1-10-7. A protecting group is removed from the compound of the chemical formula 1-10-7 so as to prepare a compound of the chemical formula 1-10-8, and then a reductive amination reaction, an alkylation reaction and an acylation reaction are performed to prepare a compound of the chemical formula 1-10-9.

In the present invention, the examples of the compounds prepared according to a method as shown in the above reaction formula 10 include compounds **260 to 264, 266, 284, 285, 303, 319 to 325, 328, 329, 330** and the like.

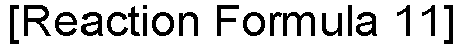

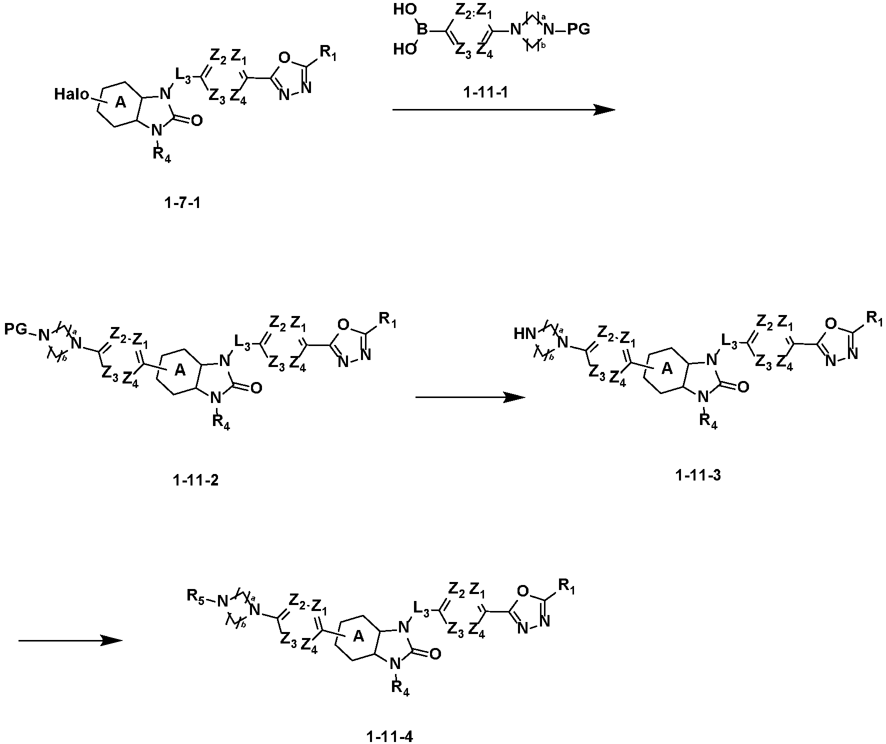

The above [Reaction Formula 11] shows a synthesis method of 1,3,4-oxadiazole compounds having a benzimidazolone structure, and a compound of the chemical formula 1-7-1 is subjected to C-C coupling (Suzuki reaction) with a compound of the chemical formula 1-11-1 so as to prepare a compound of the chemical formula 1-11-2. A protecting group is removed from the compound of the chemical formula 1-11-2 so as to prepare a compound of the chemical formula 1-11-3, and then a reductive amination reaction, an alkylation reaction and an acylation reaction are performed to prepare a compound of the chemical formula 1-11-4.

In the present invention, the examples of the compounds prepared according to a method as shown in the above reaction formula 11 include compounds **337 to 339, 342 to 344, 358 to 368,** etc.

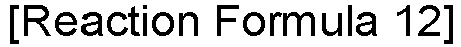

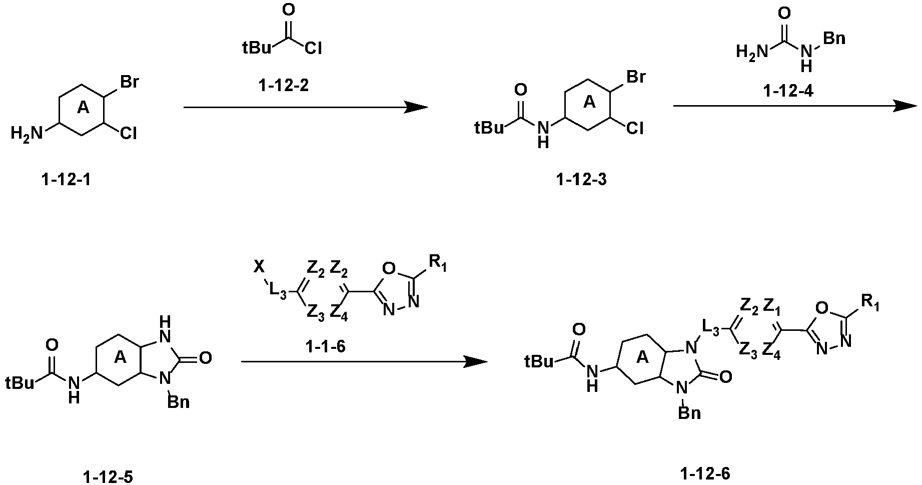

The above [Reaction Formula 12] shows a synthesis method of 1,3,4-oxadiazole compounds having a benzimidazolone structure, and a compound of the chemical formula 1-12-1 reacts with a compound of the chemical formula 1-12-2 so as to prepare a compound of the chemical formula 1-12-3. Then, the resulting compound is subjected to a reaction with a compound of the chemical formula 1-12-4 so as to prepare a cyclized compound of the chemical formula 1-12-5. After that, the compound of the chemical formula 1-12-5 is subjected to a substitution reaction with a compound of the chemical formula 1-1-6 so as to prepare a compound of the chemical formula 1-12-6.

In the present invention, the examples of the compounds prepared according to a method as shown in the above reaction formula 12 include a compound **207** and the like.

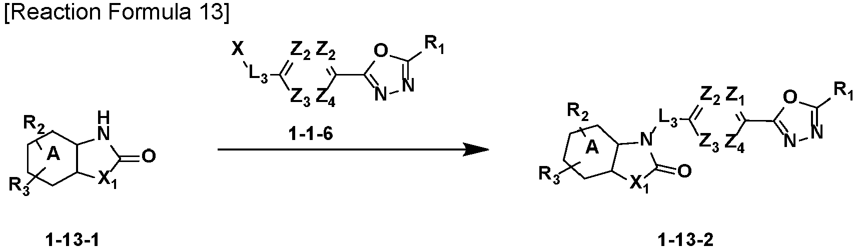

The above [Reaction Formula 13] shows a synthesis method of 1,3,4-oxadiazole compounds having a benzoxazolone or benzothiazolone structure, and a compound of the chemical formula 1-13-1 is subjected to a substitution reaction with a compound of the chemical formula 1-1-6 so as to prepare a compound of the chemical formula 1-13-2.

In the present invention, the examples of the compounds prepared according to a method as shown in the above reaction formula 13 include compounds **228, 230, 245, 246, 265** and the like.

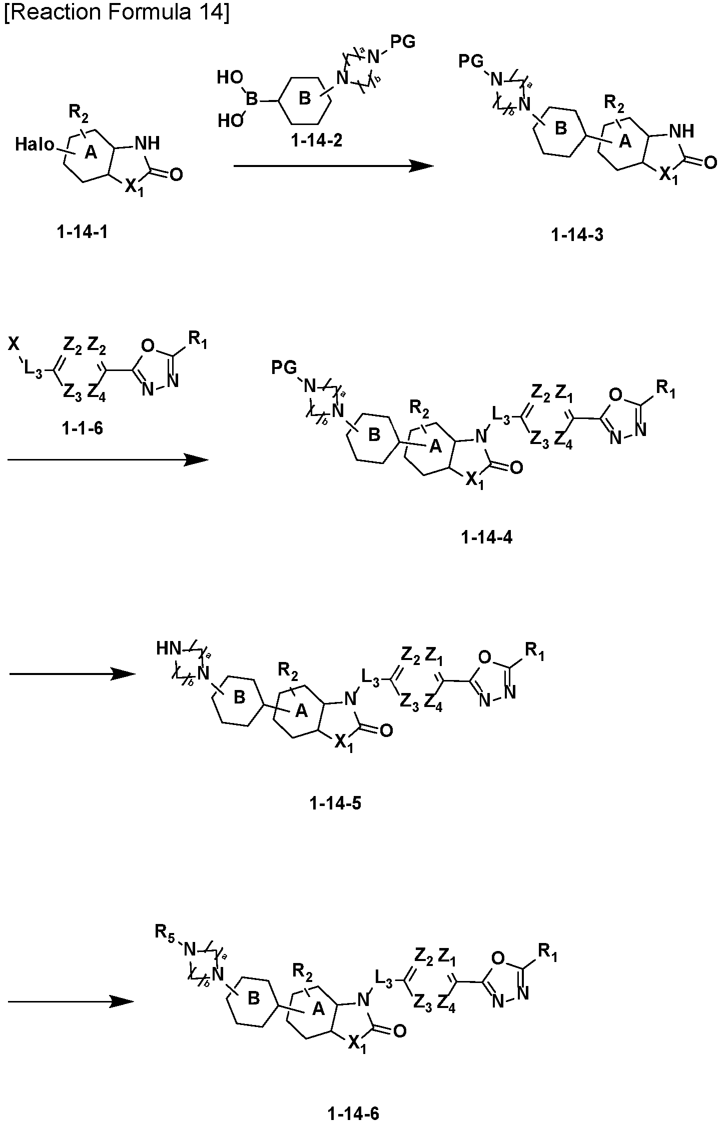

The above [Reaction Formula 14] shows a synthesis method of 1,3,4-oxadiazole compounds having a benzoxazolone or benzothiazolone structure, and a compound of the chemical formula 1-14-1 is subjected to C-C coupling (Suzuki reaction) with a compound of the chemical formula 1-14-2 so as to prepare a compound of the chemical formula 1-14-3. The compound of the chemical formula 1-14-3 is subjected to a substitution reaction with a compound of the chemical formula 1-1-6 so as to prepare a compound of the chemical formula 1-14-4. A protecting group is removed from the compound of the chemical formula 1-14-4 so as to prepare a compound of the chemical formula 1-14-5, and then a reductive amination reaction, an alkylation reaction and an acylation reaction are performed to prepare a compound of the chemical formula 1-14-6.

In the present invention, the examples of the compounds prepared according to a method as shown in the above reaction formula 14 include compounds **345 to 351** and the like.

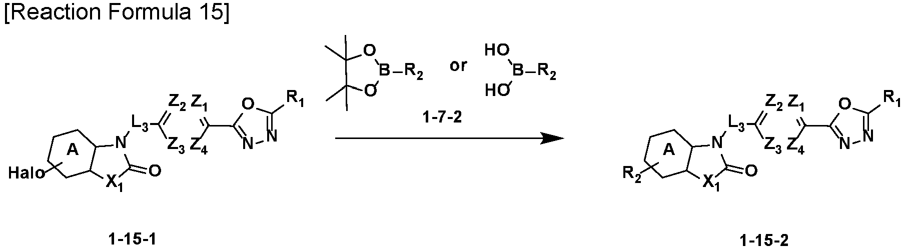

The above [Reaction Formula 15] shows a synthesis method of 1,3,4-oxadiazole compounds having a benzoxazolone or benzothiazolone structure, and a compound of the chemical formula 1-15-1 is subjected to C-C coupling (Suzuki reaction) with a compound of the chemical formula 1-7-2 so as to prepare a compound of the chemical formula 1-15-2.

In the present invention, the examples of the compounds prepared according to a method as shown in the above reaction formula 15 include compounds **229, 231, 237, 238** and the like.

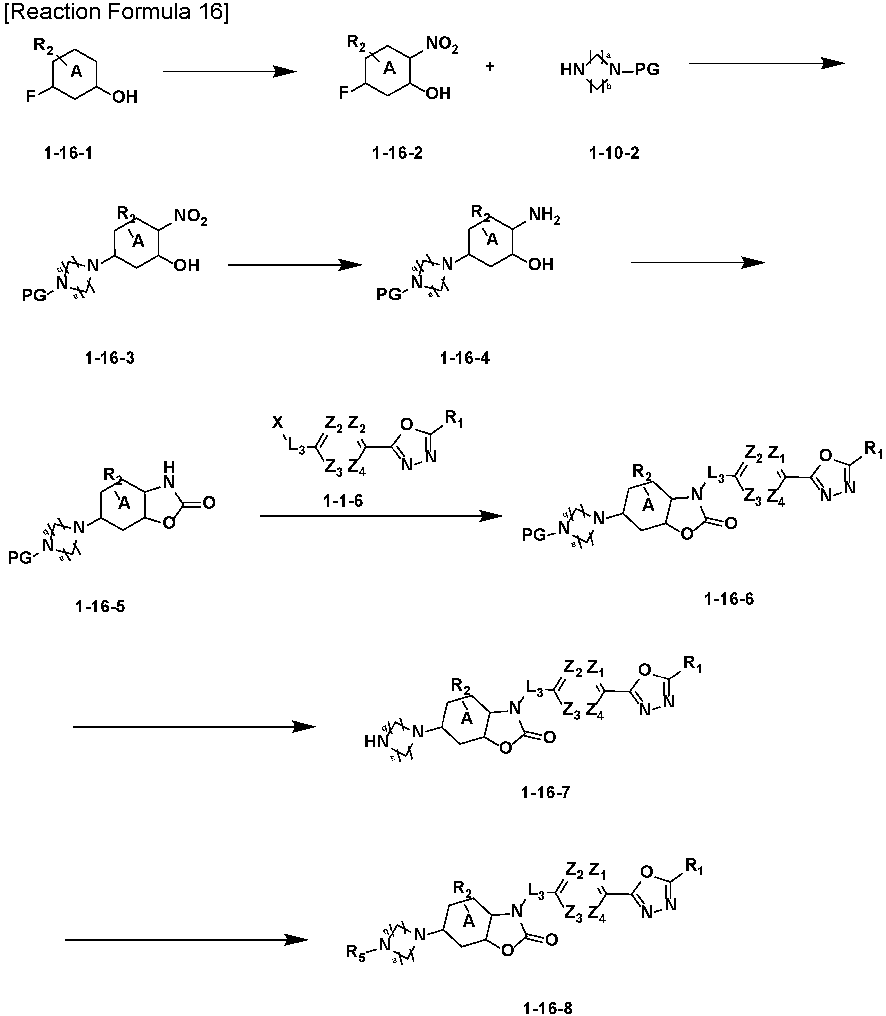

The above [Reaction Formula 16] shows a synthesis method of 1,3,4-oxadiazole compounds having a benzoxazolone structure, and a compound of the chemical formula 1-16-1 is subjected to a nitration reaction so as to prepare a compound of the chemical formula 1-16-2, and then is subjected to a reaction with a compound of the chemical formula 1-10-2 so as to prepare a compound of the chemical formula 1-16-3. After that, the resulting compound is subjected to a reduction reaction with the compound of the chemical formula 1-16-3 so as to prepare a compound of the chemical formula 1-16-4, and then a cyclization reaction is performed to prepare a compound of the chemical formula 1-16-5. Then, the compound of the chemical formula 1-16-5 is subjected to a substitution reaction with a compound of the chemical formula 1-1-6 so as to prepare a compound of the chemical formula 1-16-6. A protecting group is removed from the compound of the chemical formula 1-16-6 so as to prepare a compound of the chemical formula 1-16-7, and then a reductive amination reaction, an alkylation reaction and an acylation reaction are performed to prepare a compound of the chemical formula 1-16-8.

In the present invention, the examples of the compounds prepared according to a method as shown in the above reaction formula 16 include compounds **316 to 318** and the like.

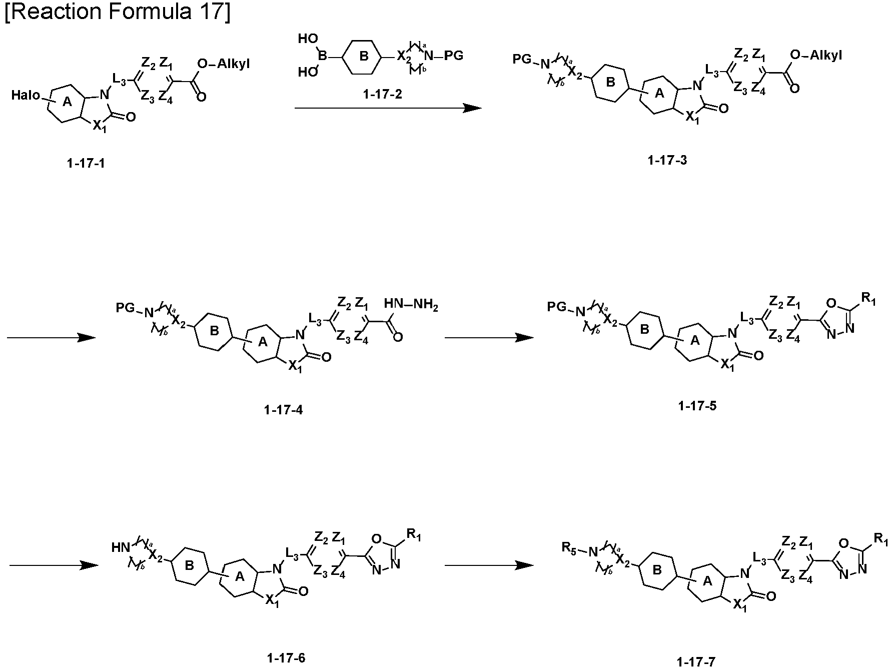

The above [Reaction Formula 17] shows a synthesis method of 1,3,4-oxadiazole compounds having a benzoxazolone or benzothiazolone structure, and a compound of the chemical formula 1-17-1 is subjected to C-C coupling (Suzuki reaction) with a compound of the chemical formula 1-17-2, to which a protecting group is added, so as to prepare a compound of the chemical formula 1-17-3, then is subjected to a reaction with hydrazine so as to prepare a compound of the chemical formula 1-17-4, and then is subjected to a reaction with difluoroacetic anhydride so as to prepare a compound of the chemical formula 1-17-5. After that, a protecting group is removed from the compound of the chemical formula 1-17-5 so as to prepare a compound of the chemical formula 1-17-6, and then a reductive amination reaction, an alkylation reaction and an acylation reaction are performed to prepare a compound of the chemical formula 1-17-7.

In the present invention, the examples of the compounds prepared according to a method as shown in the above reaction formula 17 include compounds **352 to 357** and the like.

Hereinafter, the present invention will be described in more detail through the following examples and experimental examples. However, the following examples and the like are provided only for the purpose of illustrating the present invention, and thus the scope of the present invention is not limited thereto.

### Preparation of 1,3,4-oxadiazole derivative compounds

A specific method for preparing the compounds represented by the chemical formula I is the same as follows.

### Example 1: Synthesis of Compound 1, 1-(4-(5-(difluoromethyl)-1,3,4-oxadiazole-2-yl)-2-fluorobenzyl)-3-(1-methylpiperidine-4-yl)-1,3-dih ydro-2H-benzo[d]imidazole-2-one

### [Step 1] Synthesis of tert-butyl 4-((2-aminophenyl)amino)piperidine-1-carboxylate

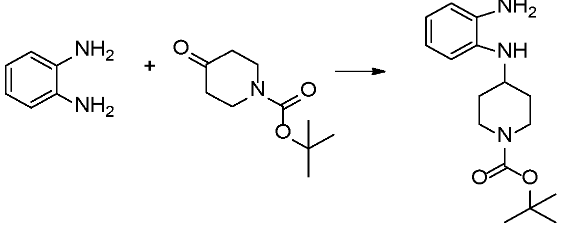

Tert-butyl 4-oxopiperidine-1-carboxylate (3.000 g, 15.056 mmol), benzene-1,2-diamine (4.885 g, 45.169 mmol), sodium triacetoxyborohydride (6.382 g, 30.113 mmol) and acetic acid (1.724 mL, 30.113 mmol) were dissolved in dichloromethane (50 mL) at room temperature, after which the resulting solution was stirred at the same temperature for 12 hours. Water was poured into the reaction mixture and an extraction was performed with dichloromethane. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 40 g cartridge; ethyl acetate/hexane = 0 to 50%) and concentrated to obtain a title compound (3.500 g, 79.8%) in a colorless oil form.

### [Step 2] Synthesis of tert-butyl 4-(2-oxo-2,3-dihydro-1H-benzo[d]imidazole-1-yl)piperidine-1-carboxylate

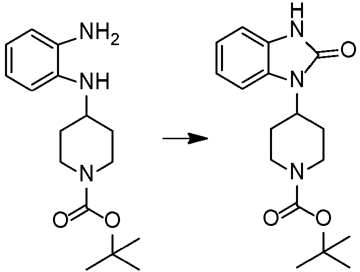

The tert-butyl 4-((2-aminophenyl)amino)piperidine-1-carboxylate (3.500 g, 12.011 mmol) prepared in the step 1 was dissolved in dichloromethane (5 mL), after which trimethylamine (1.674 mL, 12.011 mmol) and triphosgene (14.257 g, 48.044 mmol) were added thereinto at 0°C, then stirred at the same temperature for 30 minutes, and then further stirred at room temperature for 2 hours. Water was poured into the reaction mixture and an extraction was performed with dichloromethane. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; ethyl acetate/hexane = 0 to 50%) and concentrated to obtain a title compound (1.000 g, 26.2%) in a white solid form.

### [Step 3] Synthesis of tert-butyl 4-(3-(2-fluoro-4-(methoxycarbonyl)benzyl)-2-oxo-2,3-dihydro-1H-benzo[d]imidazole-1-yl)piperidi ne-1-carboxylate

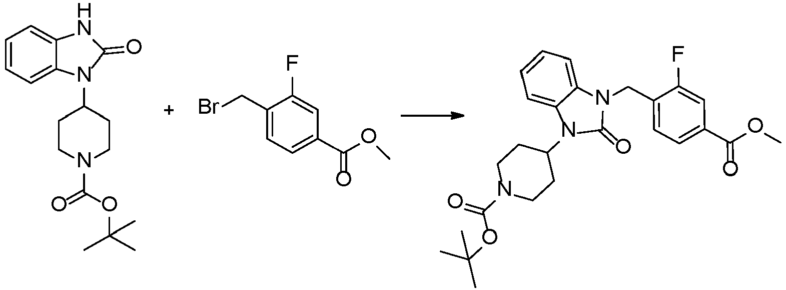

The tert-butyl 4-(2-oxo-2,3-dihydro-1H-benzo[d]imidazole-1-yl)piperidine-1-carboxylate (1.000 g, 3.151 mmol) prepared in the step 2 was dissolved in N,N-dimethylformamide (30 mL) at 0°C, after which sodium hydride (60.00%, 0.189 g, 4.726 mmol) was added into the resulting solution and stirred at the same temperature for 30 minutes. Methyl 4-(bromomethyl)-3-fluorobenzoate (0.778 g, 3.151 mmol) was added into the reaction mixture and further stirred at room temperature for 12 hours. Water was poured into the reaction mixture and an extraction was performed with ethyl acetate. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; ethyl acetate/hexane = 0 to 30%) and concentrated to obtain a title compound (0.670 g, 44.0%) in a colorless oil form.

### [Step 4] Synthesis of tert-butyl 4-(3-(2-fluoro-4-(hydrazinecarbonyl)benzyl)-2-oxo-2,3-dihydro-1H-benzo[d]imidazole-1-yl)piperid ine-1-carboxylate

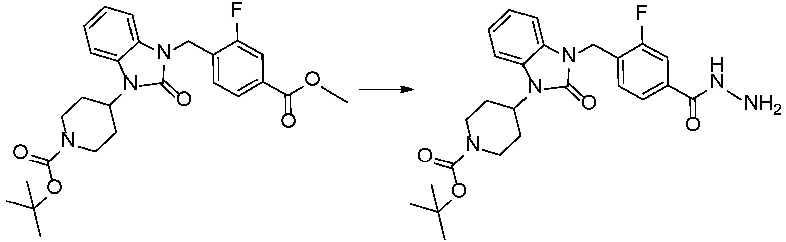

The tert-butyl 4-(3-(2-fluoro-4-(methoxycarbonyl)benzyl)-2-oxo-2,3-dihydro-1H-benzo[d]imidazole-1-yl)piperidi ne-1-carboxylate (0.670 g, 1.386 mmol) prepared in the step 3 and hydrazine monohydrate (1.347 mL, 27.712 mmol) were dissolved in ethanol (10 mL) at 90°C, after which the resulting solution was stirred at the same temperature for 12 hours and then a reaction was finished by lowering a temperature to room temperature. Solvent was removed from the reaction mixture under reduced pressure, after which a title compound was used without an additional purification process (0.670 g, 100.0%, colorless oil).

### [Step 5] Synthesis of tert-butyl 4-(3-(4-(5-(difluoromethyl)-1,3,4-oxadiazole-2-yl)-2-fluorobenzyl)-2-oxo-2,3-dihydro-1H-benzo[d]i midazole-1-yl)piperidine-1-carboxylate

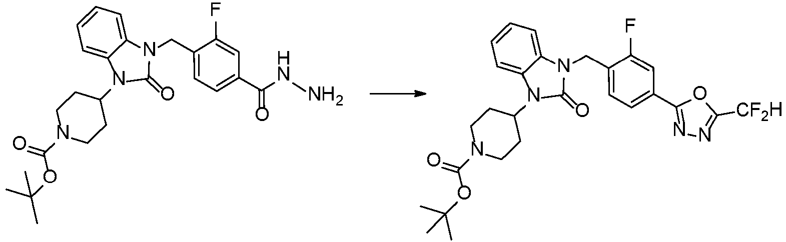

The tert-butyl 4-(3-(2-fluoro-4-(hydrazinecarbonyl)benzyl)-2-oxo-2,3-dihydro-1H-benzo[d]imidazole-1-yl)piperid ine-1-carboxylate (0.670 g, 1.386 mmol) prepared in the step 4, 2,2-difluoroacetic anhydride (0.517 mL, 4.157 mmol) and imidazole (0.283 g, 4.157 mmol) were dissolved in dichloromethane (10 mL) at 45°C, after which the resulting solution was stirred at the same temperature for 12 hours, and then a reaction was finished by lowering a temperature to room temperature. Water was poured into the reaction mixture and an extraction was performed with dichloromethane. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; ethyl acetate/hexane = 0 to 50%) and concentrated to obtain a title compound (0.600 g, 79.7%) in a white foam solid form.

### [Step 6] Synthesis of 1-(4-(5-(difluoromethyl)-1,3,4-oxadiazole-2-yl)-2-fluorobenzyl)-3-(piperidine-4-yl)-1,3-dihydro-2H -benzo[d]imidazole-2-one 2,2,2-trifluoroacetate

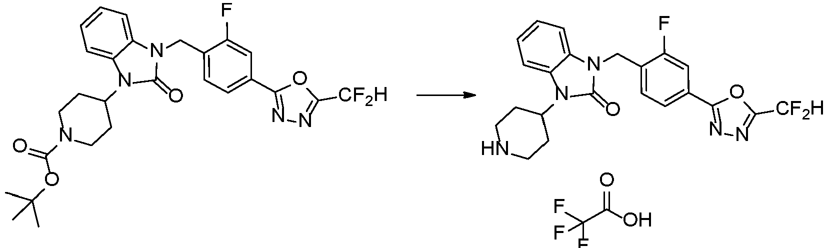

The tert-butyl 4-(3-(4-(5-(difluoromethyl)-1,3,4-oxadiazole-2-yl)-2-fluorobenzyl)-2-oxo-2,3-dihydro-1H-benzo[d]i midazole-1-yl)piperidine-1-carboxylate (0.300 g, 0.552 mmol) prepared in the step 5 and trifluoroacetic acid (0.845 mL, 11.039 mmol) were dissolved in dichloromethane (10 mL) at room temperature, after which the resulting solution was stirred at the same temperature for 3 hours. Solvent was removed from the reaction mixture under reduced pressure, after which a title compound was used without an additional purification process (0.290 g, 94.3%, brown oil).

### [Step 7] Synthesis of the compound 1

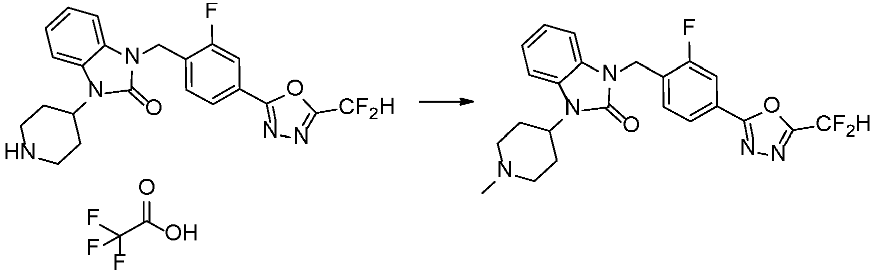

The 1-(4-(5-(difluoromethyl)-1,3,4-oxadiazole-2-yl)-2-fluorobenzyl)-3-(piperidine-4-yl)-1,3-dihydro-2H -benzo[d]imidazole-2-one 2,2,2-trifluoroacetate (0.289 g, 0.518 mmol) prepared in the step 6, formaldehyde (0.031 g, 1.037 mmol), N,N-diisopropylethylamine (0.090 mL, 0.518 mmol) and sodium triacetoxyborohydride (0.220 g, 1.037 mmol) were dissolved in dichloromethane (10 mL) at room temperature, after which the resulting solution was stirred at the same temperature for 12 hours. Water was poured into the reaction mixture and an extraction was performed with dichloromethane. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; methanol/dichloromethane = 0 to 12%) and concentrated to obtain a title compound (0.220 g, 92.8%) in a colorless oil form.

**¹H NMR** (400 MHz, CDCl₃) δ 7.87 - 7.82 (m, 2H), 7.47 - 7.39 (m, 2H), 7.12 - 7.04 (m, 2H), 7.06 (s, 0.25H), 6.98 - 6.95 (m, 1H), 6.93 (s, 0.5H), 6.80 (s, 0.25H), 5.21 (s, 2H), 4.55 - 4.51 (m, 1H), 3.22 (d, *J* = 11.8 Hz, 2H), 2.66 - 2.60 (m, 2H), 2.40 - 2.34 (m, 2H), 1.91 - 1.87 (m, 2H); **LRMS** (ES) m/z 458.0 (M⁺ + 1).

### Example 2: Synthesis of Compound 2, 1-(4-(5-(difluoromethyl)-1,3,4-oxadiazole-2-yl)-2-fluorobenzyl)-3-(2-morpholinoethyl)-1,3-dihydro -2H-benzo[d]imidazole-2-one

### [Step 1] Synthesis of N-(2-morpholinoethyl)-2-nitroaniline

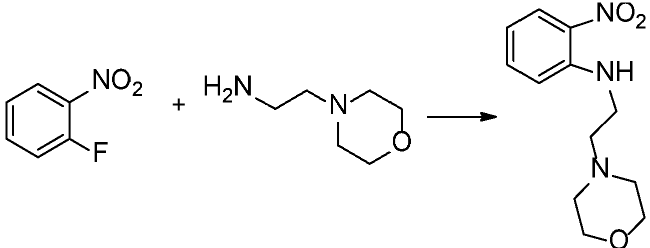

1-fluoro-2-nitrobenzene (6.000 g, 42.523 mmol), 2-morpholinoethane-1-amine (6.090 g, 46.775 mmol) and potassium carbonate (11.754 g, 85.046 mmol) were dissolved in tetrahydrofuran (50 mL) at room temperature, after which the resulting solution was stirred at the same temperature for 12 hours. Water was poured into the reaction mixture and an extraction was performed with ethyl acetate. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 40 g cartridge; ethyl acetate/hexane = 0 to 50%) and concentrated to obtain a title compound (5.500 g, 51.5%) in a colorless oil form.

### [Step 2] Synthesis of N¹-(2-morpholinoethyl)benzene-1,2-diamine

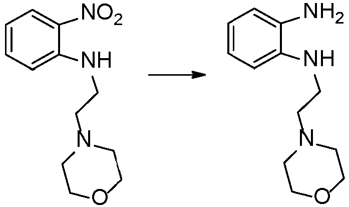

The N-(2-morpholinoethyl)-2-nitroaniline (4.500 g, 17.908 mmol) prepared in the step 1 was dissolved in methanol (30 mL) at room temperature, after which 10%-Pd/C (450 mg) was slowly added thereinto and stirred at the same temperature for 12 hours in the presence of a hydrogen balloon attached thereto. The reaction mixture was filtered via a celite pad to remove a solid therefrom, after which solvent was removed from the resulting filtrate under reduced pressure, and then a title compound was used without an additional purification process (3.500 g, 88.3%, brown oil).

### [Step 3] Synthesis of 1-(2-morpholinoethyl)-1,3-dihydro-2H-benzo[d]imidazole-2-one

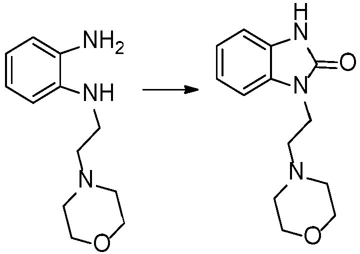

The N¹-(2-morpholinoethyl)benzene-1,2-diamine (2.770 g, 12.517 mmol) prepared in the step 2 and 1,1'-carbonyldiimidazole (CDI, 2.030 g, 12.517 mmol) were dissolved in tetrahydrofuran (30 mL) at room temperature, after which the resulting solution was stirred at the same temperature for 12 hours. Water was poured into the reaction mixture and an extraction was performed with ethyl acetate. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; ethyl acetate/hexane = 0 to 50%) and concentrated to obtain a title compound (2.240 g, 72.4%) in a colorless oil form.

### [Step 4] Synthesis of methyl 3-fluoro-4-((3-(2-morpholinoethyl)-2-oxo-2,3-dihydro-1H-benzo[d]imidazole-1-yl)methyl)benzoat e

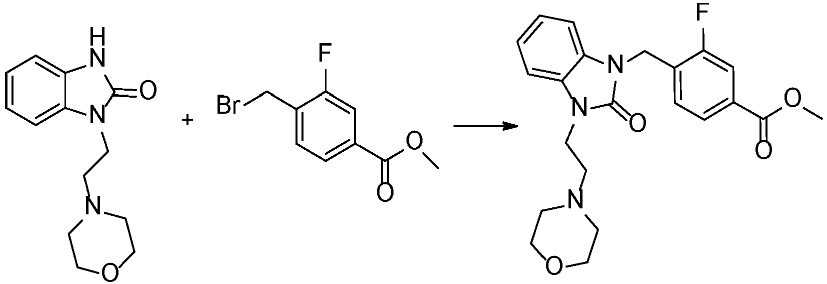

The 1-(2-morpholinoethyl)-1,3-dihydro-2H-benzo[d]imidazole-2-one (3.000 g, 12.131 mmol) prepared in the step 3 was dissolved in N,N-dimethylformamide (30 mL) at 0°C, after which sodium hydride (60.00%, 0.728 g, 18.197 mmol) was added into the resulting solution and stirred at the same temperature for 30 minutes. Methyl 4-(bromomethyl)-3-fluorobenzoate (2.997 g, 12.131 mmol) was added into the reaction mixture and further stirred at room temperature for 18 hours. A precipitated solid was filtered, then washed with hexane, and then dried to obtain a title compound (2.200 g, 43.9%) in a colorless oil form.

### [Step 5] Synthesis of 3-fluoro-4-((3-(2-morpholinoethyl)-2-oxo-2,3-dihydro-1H-benzo[d]imidazole-1-yl)methyl)benzohy drazide

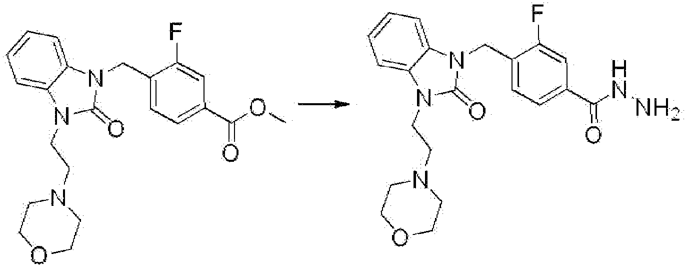

The methyl 3-fluoro-4-((3-(2-morpholinoethyl)-2-oxo-2,3-dihydro-1H-benzo[d]imidazole-1-yl)methyl)benzoat e (2.200 g, 5.321 mmol) prepared in the step 4 and hydrazine monohydrate (5.172 mL, 106.422 mmol) were dissolved in ethanol (20 mL) at 90°C, after which the resulting solution was stirred at the same temperature for 12 hours, and then a reaction was finished by lowering a temperature to room temperature. Solvent was removed from the reaction mixture under reduced pressure, after which a title compound was used without an additional purification process (2.200 g, 100.0%, colorless oil).

### [Step 6] Synthesis of the compound 2

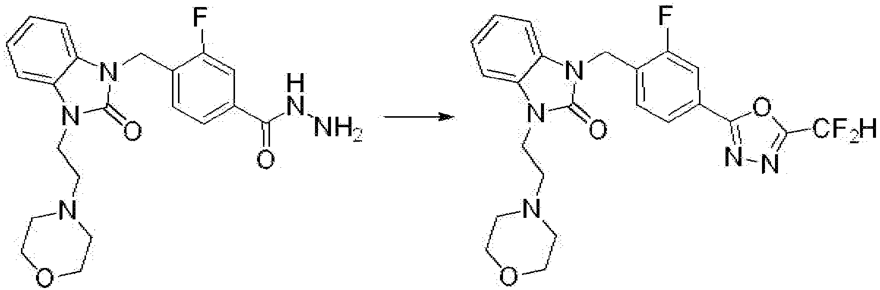

The 3-fluoro-4-((3-(2-morpholinoethyl)-2-oxo-2,3-dihydro-1H-benzo[d]imidazole-1-yl)methyl)benzohy drazide (2.150 g, 5.200 mmol) prepared in the step 5, 2,2-difluoroacetic anhydride (1.939 mL, 15.600 mmol) and triethylamine (2.174 mL, 15.600 mmol) were dissolved in dichloromethane (20 mL) at 45°C, after which the resulting solution was stirred at the same temperature for 12 hours, and then a reaction was finished by lowering a temperature to room temperature. Water was poured into the reaction mixture and an extraction was performed with dichloromethane. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; methanol/dichloromethane = 0 to 10%) and concentrated to obtain a title compound (1.550 g, 63.0%) in a colorless oil form.

**¹H NMR** (400 MHz, CDCl₃) δ 7.90 - 7.85 (m, 2H), 7.52 (dd, *J* = 8.0, 7.5 Hz, 1H), 7.19 - 7.13 (m, 2H), 7.12 - 7.09 (m, 1H), 7.06 (s, 0.25H), 6.98 (d, *J =* 7.6 Hz, 1H), 6.93 (s, 0.5H), 6.80 (s, 0.25H), 5.21 (s, 2H), 4.36 (t, *J* = 6.9 Hz, 2H), 3.96 (t, *J* = 4.8 Hz, 4H), 3.51 - 3.24 (m, 6H); **LRMS** (ES) m/z 474.3 (M⁺ + 1).

### Example 3: Synthesis of Compound 3, 1-((5-(5-(difluoromethyl)-1,3,4-oxadiazole-2-yl)pyridine-2-yl)methyl)-3-(2-(dimethylamino)ethyl)-1 ,3-dihydro-2H-benzo[d]imidazole-2-one

### [Step 1] Synthesis of N¹,N¹-dimethyl-N²-(2-nitrophenyl)ethane-1,2-diamine

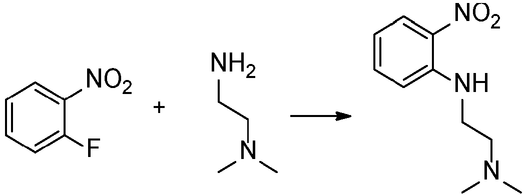

1-fluoro-2-nitrobenzene (2.000 g, 14.174 mmol), N¹,N¹-dimethylethane-1,2-diamine (1.548 mL, 14.174 mmol) and potassium carbonate (3.918 g, 28.349 mmol) were dissolved in tetrahydrofuran (30 mL) at 70°C, after which the resulting solution was stirred at the same temperature for 12 hours, and then a reaction was finished by lowering a temperature to room temperature. Water was poured into the reaction mixture and an extraction was performed with ethyl acetate. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 40 g cartridge; ethyl acetate/hexane = 0 to 50%) and concentrated to obtain a title compound (2.500 g, 84.3%) in a red oil form.

### [Step 2] Synthesis of N¹-(2-(dimethylamino)ethyl)benzene-1,2-diamine

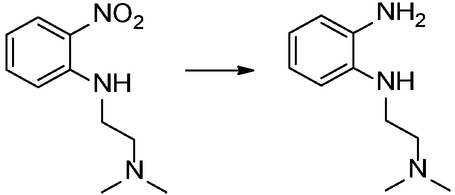

The N¹,N¹-dimethyl-N²-(2-nitrophenyl)ethane-1,2-diamine (2.500 g, 11.947 mmol) prepared in the step 1 was dissolved in methanol (30 mL) at room temperature, after which 10%-Pd/C (250 mg) was slowly added thereinto and stirred at the same temperature for 12 hours in the presence of a hydrogen balloon attached thereto. The reaction mixture was filtered via a celite pad to remove a solid therefrom, after which solvent was removed from the resulting filtrate under reduced pressure, and then a title compound was used without an additional purification process (2.100 g, 98.0%, black oil).

### [Step 3] Synthesis of 1-(2-(dimethylamino)ethyl)-1,3-dihydro-2H-benzo[d]imidazole-2-one

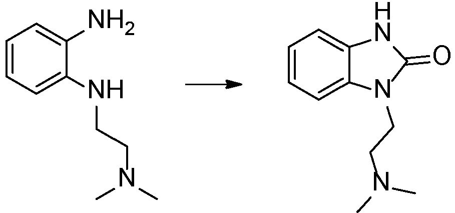

The N¹-(2-(dimethylamino)ethyl)benzene-1,2-diamine (2.100 g, 11.714 mmol) prepared in the step 2 and 1,1'-carbonyldiimidazole (CDI, 2.089 g, 12.886 mmol) were dissolved in tetrahydrofuran (10 mL) at room temperature, after which the resulting solution was stirred at the same temperature for 12 hours. Water was poured into the reaction mixture and an extraction was performed with ethyl acetate. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; ethyl acetate/hexane = 0 to 70%) and concentrated to obtain a title compound (1.700 g, 70.7%) in a black oil form.

### [Step 4] Synthesis of the compound 3

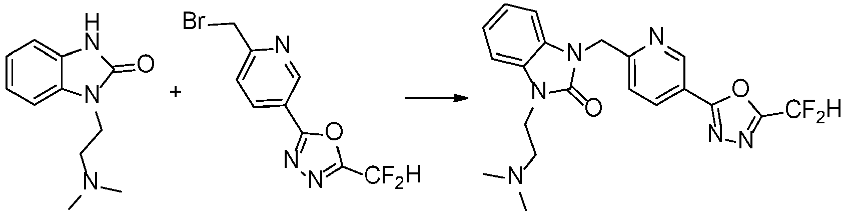

The 1-(2-(dimethylamino)ethyl)-1,3-dihydro-2H-benzo[d]imidazole-2-one (0.300 g, 1.462 mmol) prepared in the step 3 was dissolved in N,N-dimethylformamide (20 mL) at 0°C, after which sodium hydride (60.00%, 0.088 g, 2.192 mmol) was added into the resulting solution and stirred at the same temperature for 30 minutes. 2-(6-(bromomethyl)pyridine-3-yl)-5-(difluoromethyl)-1,3,4-oxadiazole (0.466 g, 1.608 mmol) was added into the reaction mixture and further stirred at room temperature for 3 hours. Water was poured into the reaction mixture and an extraction was performed with ethyl acetate. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; ethyl acetate/hexane = 0 to 50%) and concentrated to obtain a title compound (0.300 g, 49.5%) in a colorless oil form.

**¹H NMR** (400 MHz, CDCl₃) δ 9.28 (dd, *J* = 2.2, 0.7 Hz, 1H), 8.30 (dd, *J* = 8.2, 2.2 Hz, 1H), 7.40 (dd, *J* = 8.0, 0.4 Hz, 1H), 7.11 - 7.02 (m, 3H), 7.06 (s, 0.25H), 6.95 - 6.93 (m, 1H), 6.93 (s, 0.5H), 6.80 (s, 0.25H), 5.29 (s, 2H), 4.07 (t, *J* = 7.1 Hz, 2H), 2.70 (t, *J =* 7.1 Hz, 2H), 2.34 (s, 6H); **LRMS** (ES) m/z 415.4 (M⁺+ 1).

### Example 4: Synthesis of Compound 4, 1-((5-(5-(difluoromethyl)-1,3,4-oxadiazole-2-yl)pyridine-2-yl)methyl)-3-(1-methylpiperidine-4-yl)-1 ,3-dihydro-2H-benzo[d]imidazole-2-one

### [Step 1] Synthesis of tert-butyl 4-(3-((5-(5-(difluoromethyl)-1,3,4-oxadiazole-2-yl)pyridine-2-yl)methyl)-2-oxo-2,3-dihydro-1H-be nzo[d]imidazole-1-yl)piperidine-1-carboxylate

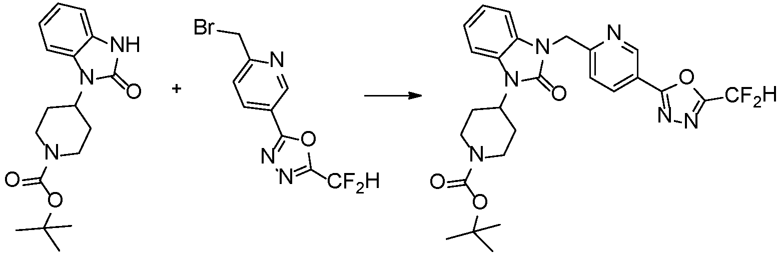

Tert-butyl 4-(2-oxo-2,3-dihydro-1H-benzo[d]imidazole-1-yl)piperidine-1-carboxylate (0.200 g, 0.630 mmol) was dissolved in N,N-dimethylformamide (20 mL) at 0°C, after which sodium hydride (60.00%, 0.038 g, 0.945 mmol) was added into the resulting solution and stirred at the same temperature for 30 minutes. 2-(6-(bromomethyl)pyridine-3-yl)-5-(difluoromethyl)-1,3,4-oxadiazole (0.201 g, 0.693 mmol) was added into the reaction mixture and further stirred at room temperature for 3 hours. Water was poured into the reaction mixture and an extraction was performed with ethyl acetate. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; ethyl acetate/hexane = 0 to 50%) and concentrated to obtain a title compound (0.210 g, 63.3%) in a colorless oil form.

### [Step 2] Synthesis of 1-((5-(5-(difluoromethyl)-1,3,4-oxadiazole-2-yl)pyridine-2-yl)methyl)-3-(piperidine-4-yl)-1,3-dihydr o-2H-benzo[d]imidazole-2-one 2,2,2-trifluoroacetate

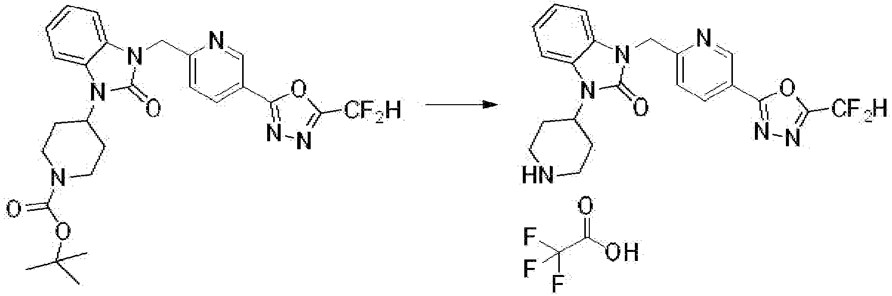

The tert-butyl 4-(3-((5-(5-(difluoromethyl)-1,3,4-oxadiazole-2-yl)pyridine-2-yl)methyl)-2-oxo-2,3-dihydro-1H-be nzo[d]imidazole-1-yl)piperidine-1-carboxylate (0.250 g, 0.475 mmol) prepared in the step 1 and trifluoroacetic acid (0.727 mL, 9.496 mmol) were dissolved in dichloromethane (5 mL) at room temperature, after which the resulting solution was stirred at the same temperature for 2 hours. A title compound was used without an additional purification process (0.250 g, 97.4%, yellow oil).

### [Step 3] Synthesis of the compound 4

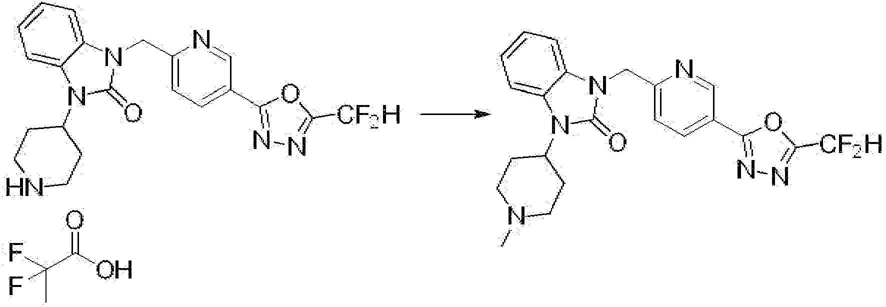

The 1-((5-(5-(difluoromethyl)-1,3,4-oxadiazole-2-yl)pyridine-2-yl)methyl)-3-(piperidine-4-yl)-1,3-dihydr o-2H-benzo[d]imidazole-2-one 2,2,2-trifluoroacetate (0.141 g, 0.261 mmol) prepared in the step 2 and N,N-diisopropylethylamine (0.045 mL, 0.261 mmol) were dissolved in dichloromethane (10 mL), after which the resulting solution was stirred at room temperature for 30 minutes, and then formaldehyde (37.00% solution, 0.039 mL, 0.522 mmol) and sodium triacetoxyborohydride (0.111 g, 0.522 mmol) were added thereinto and further stirred at the same temperature for 12 hours. Water was poured into the reaction mixture and an extraction was performed with dichloromethane. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; methanol/dichloromethane = 0 to 10%) and concentrated to obtain a title compound (0.080 g, 69.6%) in a colorless oil form.

**¹H NMR** (400 MHz, CDCl₃) δ 9.29 (d, *J* = 2.2 Hz, 1H), 8.33 (dd, *J* = 8.2, 2.2 Hz, 1H), 7.44 - 7.39 (m, 2H), 7.13 - 7.03 (m, 2H), 7.07 (s, 0.25H), 6.97 (d, *J* = 1.3 Hz, 1H), 6.95 (s, 0.5H), 6.82 (s, 0.25H), 5.30 (s, 2H), 4.68 - 4.62 (m, 1H), 3.48 - 3.43 (m, 2H), 2.85 - 2.79 (m, 2H), 2.67 (s, 3H), 2.00 - 1.96 (m, 2H), 1.45 - 1.41 (m, 2H); **LRMS** (ES) m/z 441.5 (M⁺+ 1).

### Example 5: Synthesis of Compound 5, 1-(4-(5-(difluoromethyl)-1,3,4-oxadiazole-2-yl)benzyl)-3-(1-methylpiperidine-4-yl)-1,3-dihydro-2H -benzo[d]imidazole-2-one

### [Step 1] Synthesis of tert-butyl 4-(3-(4-(5-(difluoromethyl)-1,3,4-oxadiazole-2-yl)benzyl)-2-oxo-2,3-dihydro-1H-benzo[d]imidazol e-1-yl)piperidine-1-carboxylate

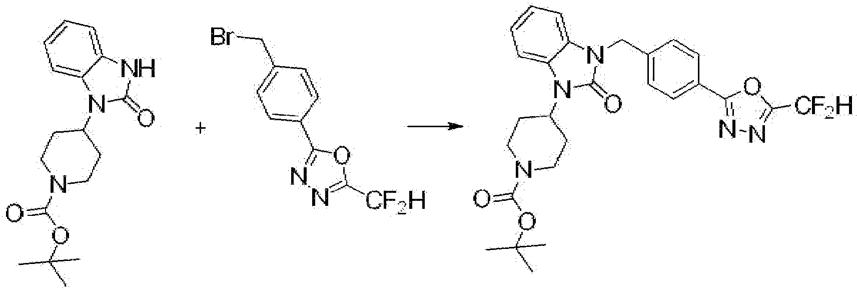

Tert-butyl 4-(2-oxo-2,3-dihydro-1H-benzo[d]imidazole-1-yl)piperidine-1-carboxylate (0.200 g, 0.630 mmol) was dissolved in N,N-dimethylformamide (20 mL) at 0°C, after which sodium hydride (60.00%, 0.038 g, 0.945 mmol) was added into the resulting solution and stirred at the same temperature for 30 minutes. 2-(4-(bromomethyl)phenyl)-5-(difluoromethyl)-1,3,4-oxadiazole (0.200 g, 0.693 mmol) was added into the reaction mixture and further stirred at room temperature for 3 hours. Water was poured into the reaction mixture and an extraction was performed with ethyl acetate. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; ethyl acetate/hexane = 0 to 50%) and concentrated to obtain a title compound (0.150 g, 45.3%) in a colorless oil form.

### [Step 2] Synthesis of 1-(4-(5-(difluoromethyl)-1,3,4-oxadiazole-2-yl)benzyl)-3-(piperidine-4-yl)-1,3-dihydro-2H-benzo[d ]imidazole-2-one 2,2,2-trifluoroacetate

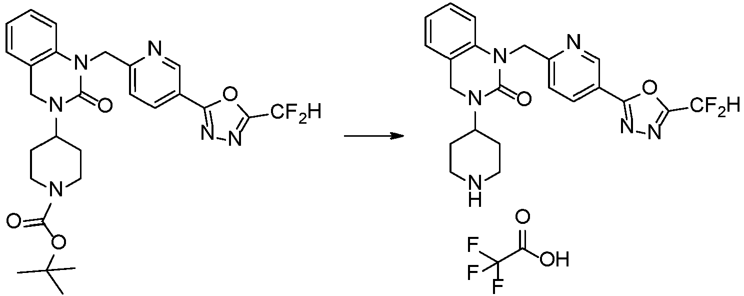

The tert-butyl 4-(3-(4-(5-(difluoromethyl)-1,3,4-oxadiazole-2-yl)benzyl)-2-oxo-2,3-dihydro-1H-benzo[d]imidazol e-1-yl)piperidine-1-carboxylate (0.150 g, 0.285 mmol) prepared in the step 1 and trifluoroacetic acid (0.437 mL, 5.708 mmol) were dissolved in dichloromethane (5 mL) at room temperature, after which the resulting solution was stirred at the same temperature for 2 hours. A title compound was used without an additional purification process (0.150 g, 97.4%, yellow oil).

### [Step 3] Synthesis of the compound 5

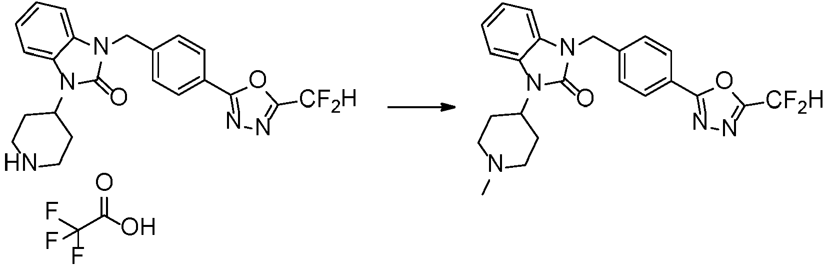

The 1-(4-(5-(difluoromethyl)-1,3,4-oxadiazole-2-yl)benzyl)-3-(piperidine-4-yl)-1,3-dihydro-2H-benzo[d ]imidazole-2-one 2,2,2-trifluoroacetate (0.150 g, 0.278 mmol) prepared in the step 2 and N,N-diisopropylethylamine (0.048 mL, 0.278 mmol) were dissolved in dichloromethane (10 mL), after which the resulting solution was stirred at room temperature for 30 minutes, and then formaldehyde (37.00% solution, 0.041 mL, 0.556 mmol) and sodium triacetoxyborohydride (0.118 g, 0.556 mmol) were added thereinto and further stirred at the same temperature for 12 hours. Water was poured into the reaction mixture and an extraction was performed with dichloromethane. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; methanol/dichloromethane = 0 to 10%) and concentrated to obtain a title compound (0.090 g, 73.7%) in a colorless oil form.

**¹H NMR** (400 MHz, CDCl₃) δ 8.05 (dd, *J* = 6.7, 1.7 Hz, 2H), 7.47 - 7.40 (m, 3H), 7.10 - 6.99 (m, 2H), 7.06 (s, 0.25H), 6.91 (s, 0.5H), 6.86 (dd, *J* = 7.8, 0.9 Hz, 1H), 6.78 (s, 0.25H), 5.13 (s, 2H), 4.68 - 4.62 (m, 1H), 3.48 - 3.44 (m, 2H), 2.83 - 2.68 (m, 5H), 1.97 (dd, *J* = 12.6, 2.5 Hz, 2H), 1.44 - 1.39 (m, 2H); **LRMS** (ES) m/z 440.3 (M⁺ + 1).

### Example 6: Synthesis of Compound 6, 1-((5-(5-(difluoromethyl)-1,3,4-oxadiazole-2-yl)pyridine-2-yl)methyl)-3-(1-methylpyrrolidine-3-yl)-1,3-dihydro-2H-benzo[d]imidazole-2-one

### [Step 1] Synthesis of benzyl 3-((2-aminophenyl)amino)pyrrolidine-1-carboxylate

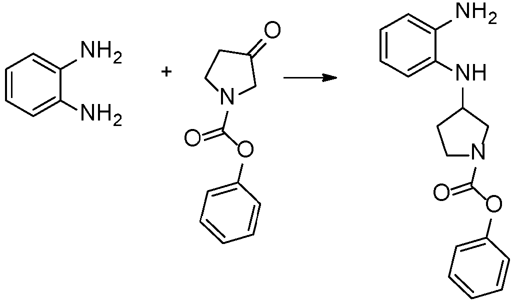

Benzyl 3-oxopyrrolidine-1-carboxylate (2.000 g, 9.122 mmol), benzene-1,2-diamine (2.959 g, 27.367 mmol), sodium triacetoxyborohydride (3.867 g, 18.245 mmol) and acetic acid (1.044 mL, 18.245 mmol) were dissolved in 1,2-dichloroethane (20 mL) at room temperature, after which the resulting solution was stirred at the same temperature for 12 hours. Water was poured into the reaction mixture and an extraction was performed with dichloromethane. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 40 g cartridge; ethyl acetate/hexane = 0 to 50%) and concentrated to obtain a title compound (1.620 g, 57.0%) in a yellow solid form.

### [Step 2] Synthesis of benzyl 3-(2-oxo-2,3-dihydro-1H-benzo[d]imidazole-1-yl)pyrrolidine-1-carboxylate

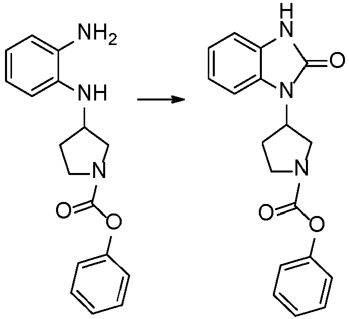

The benzyl 3-oxopyrrolidine-1-carboxylate (1.620 g, 7.389 mmol) prepared in the step 1 and 1,1'-carbonyldiimidazole (CDI, 1.198 g, 7.389 mmol) were dissolved in tetrahydrofuran (20 mL) at room temperature, after which the resulting solution was stirred at the same temperature for 12 hours. Water was poured into the reaction mixture and an extraction was performed with ethyl acetate. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; ethyl acetate/hexane = 0 to 50%) and concentrated to obtain a title compound (0.900 g, 36.1%) in a brown foam solid form.

### [Step 3] Synthesis of benzyl 3-(3-((5-(5-(difluoromethyl)-1,3,4-oxadiazole-2-yl)pyridine-2-yl)methyl)-2-oxo-2,3-dihydro-1H-be nzo[d]imidazole-1-yl)pyrrolidine-1-carboxylate

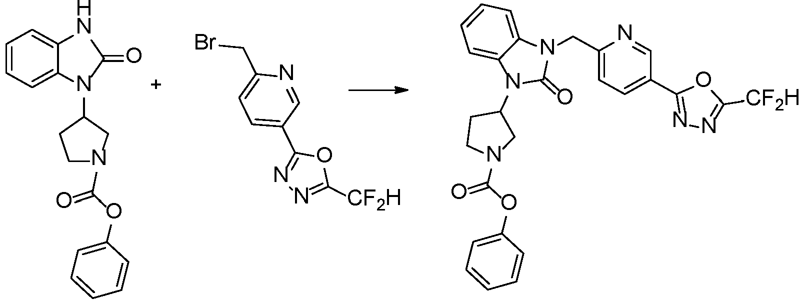

The benzyl 3-(2-oxo-2,3-dihydro-1H-benzo[d]imidazole-1-yl)pyrrolidine-1-carboxylate (0.200 g, 0.593 mmol) prepared in the step 2 was dissolved in N,N-dimethylformamide (20 mL) at 0°C, after which sodium hydride (60.00%, 0.036 g, 0.889 mmol) was added into the resulting solution and stirred at the same temperature for 30 minutes. 2-(6-(bromomethyl)pyridine-3-yl)-5-(difluoromethyl)-1,3,4-oxadiazole (0.189 g, 0.652 mmol) was added into the reaction mixture and further stirred at room temperature for 3 hours. Water was poured into the reaction mixture and an extraction was performed with ethyl acetate. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; ethyl acetate/hexane = 0 to 50%) and concentrated to obtain a title compound (0.180 g, 55.6%) in a colorless oil form.

### [Step 4] Synthesis of 1-((5-(5-(difluoromethyl)-1,3,4-oxadiazole-2-yl)pyridine-2-yl)methyl)-3-(pyrrolidine-3-yl)-1,3-dihyd ro-2H-benzo[d]imidazole-2-one

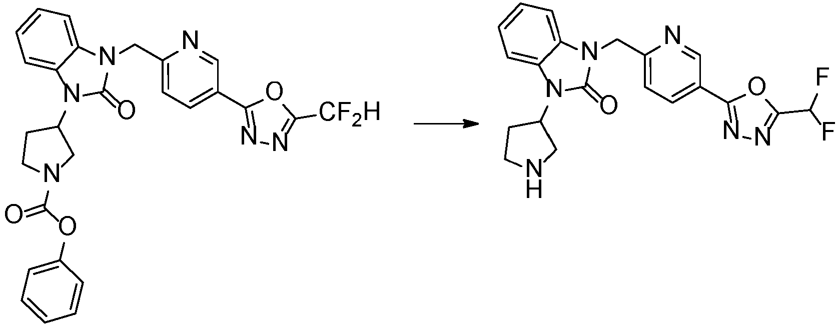

The benzyl 3-(3-((5-(5-(difluoromethyl)-1,3,4-oxadiazole-2-yl)pyridine-2-yl)methyl)-2-oxo-2,3-dihydro-1H-be nzo[d]imidazole-1-yl)pyrrolidine-1-carboxylate (0.180 g, 0.329 mmol) prepared in the step 3 was dissolved in methanol (10 mL) at room temperature, after which 10%-Pd/C (18 mg) was slowly added thereinto and stirred at the same temperature for 12 hours in the presence of a hydrogen balloon attached thereto. A title compound was used without an additional purification process (0.070 g, 51.5%, white solid).

### [Step 5] Synthesis of the compound 6

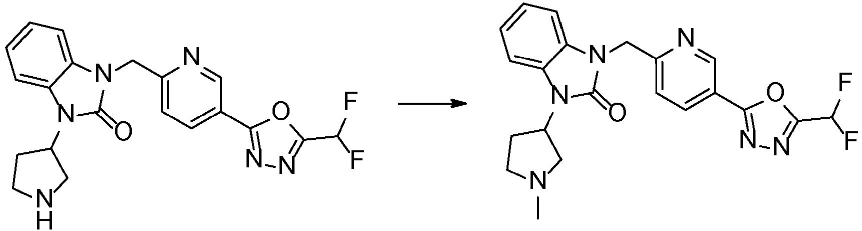

The 1-((5-(5-(difluoromethyl)-1,3,4-oxadiazole-2-yl)pyridine-2-yl)methyl)-3-(pyrrolidine-3-yl)-1,3-dihyd ro-2H-benzo[d]imidazole-2-one (0.070 g, 0.170 mmol) prepared in the step 4, sodium triacetoxyborohydride (0.072 g, 0.339 mmol) and formaldehyde (37.00% solution, 0.025 mL, 0.339 mmol) were dissolved in dichloromethane (10 mL) at room temperature, after which the resulting solution was stirred at the same temperature for 30 minutes. Water was poured into the reaction mixture and an extraction was performed with dichloromethane. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; methanol/dichloromethane = 0 to 10%) and concentrated to obtain a title compound (0.040 g, 55.3%) in a colorless oil form.

**¹H NMR** (400 MHz, CDCl₃) δ 9.31 (dd, *J* = 2.2, 0.9 Hz, 1H), 8.34 (dd, *J* = 8.2, 2.2 Hz, 1H), 7.55 - 7.53 (m, 1H), 7.43 - 7.41 (m, 1H), 7.16 - 7.10 (m, 1H), 7.08 - 7.02 (m, 1H), 7.06 (s, 0.25H), 6.97 - 6.95 (m, 1H), 6.93 (s, 0.5H), 6.81 (s, 0.25H), 5.31 - 5.23 (m, 3H), 3.23 - 3.21 (m, 2H), 3.12 - 3.07 (m, 1H), 2.86 - 2.84 (m, 1H), 2.57 (s, 3H), 2.45 - 2.34 (m, 2H); **LRMS** (ES) m/z 427.4 (M⁺ + 1).

### Example 7: Synthesis of Compound 7, 1-((5-(5-(difluoromethyl)-1,3,4-oxadiazole-2-yl)pyridine-2-yl)methyl)-3-(1-methylazetidine-3-yl)-1, 3-dihydro-2H-benzo[d]imidazole-2-one

### [Step 1] Synthesis of tert-butyl 3-((2-aminophenyl)amino)azetidine-1-carboxylate

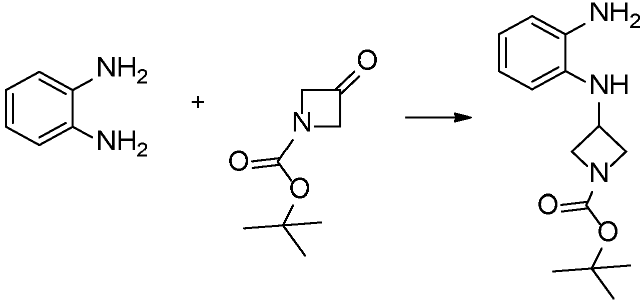

Tert-butyl 3-oxoazetidine-1-carboxylate (2.000 g, 11.682 mmol), benzene-1,2-diamine (3.790 g, 35.047 mmol), sodium triacetoxyborohydride (4.952 g, 23.364 mmol) and acetic acid (1.337 mL, 23.364 mmol) were dissolved in 1,2-dichloroethane (30 mL) at room temperature, after which the resulting solution was stirred at the same temperature for 12 hours. Water was poured into the reaction mixture and an extraction was performed with dichloromethane. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; ethyl acetate/hexane = 0 to 50%) and concentrated to obtain a title compound (1.900 g, 61.8%) in a yellow solid form.

### [Step 2] Synthesis of tert-butyl 3-(2-oxo-2,3-dihydro-1H-benzo[d]imidazole-1-yl)azetidine-1-carboxylate

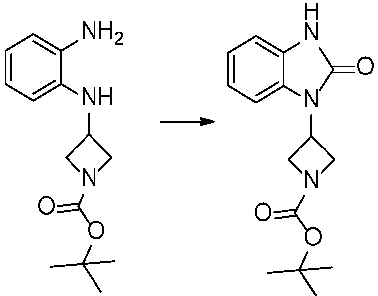

The tert-butyl 3-((2-aminophenyl)amino)azetidine-1-carboxylate (1.900 g, 7.215 mmol) prepared in the step 1 and 1,1'-carbonyldiimidazole (CDI, 1.170 g, 7.215 mmol) were dissolved in tetrahydrofuran (20 mL) at room temperature, after which the resulting solution was stirred at the same temperature for 12 hours. Water was poured into the reaction mixture and an extraction was performed with ethyl acetate. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; ethyl acetate/hexane = 0 to 50%) and concentrated to obtain a title compound (0.900 g, 43.1%) in a brown foam solid form.

### [Step 3] Synthesis of tert-butyl 3-(3-((5-(5-(difluoromethyl)-1,3,4-oxadiazole-2-yl)pyridine-2-yl)methyl)-2-oxo-2,3-dihydro-1H-be nzo[d]imidazole-1-yl)azetidine-1-carboxylate

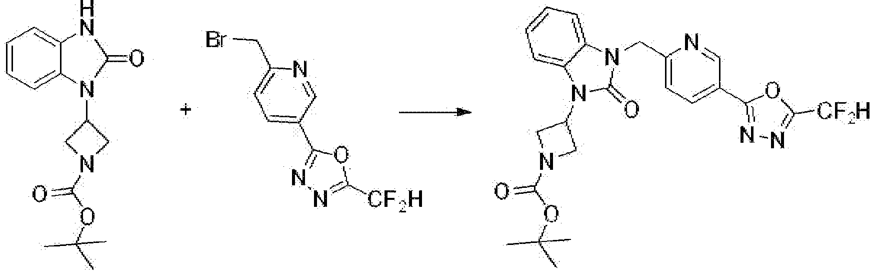

The tert-butyl 3-(2-oxo-2,3-dihydro-1H-benzo[d]imidazole-1-yl)azetidine-1-carboxylate (0.200 g, 0.691 mmol) prepared in the step 2 was dissolved in N,N-dimethylformamide (20 mL) at 0°C, after which sodium hydride (60.00%, 0.041 g, 1.037 mmol) was added into the resulting solution and stirred at the same temperature for 30 minutes. 2-(6-(bromomethyl)pyridine-3-yl)-5-(difluoromethyl)-1,3,4-oxadiazole (0.221 g, 0.760 mmol) was added into the reaction mixture and further stirred at room temperature for 3 hours. Water was poured into the reaction mixture and an extraction was performed with ethyl acetate. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; ethyl acetate/hexane = 0 to 50%) and concentrated to obtain a title compound (0.100 g, 29.0%) in a colorless oil form.

### [Step 4] Synthesis of 1-(azetidine-3-yl)-3-((5-(5-(difluoromethyl)-1,3,4-oxadiazole-2-yl)pyridine-2-yl)methyl)-1,3-dihydr o-2H-benzodimidazole-2-one 2,2,2-trifluoroacetate

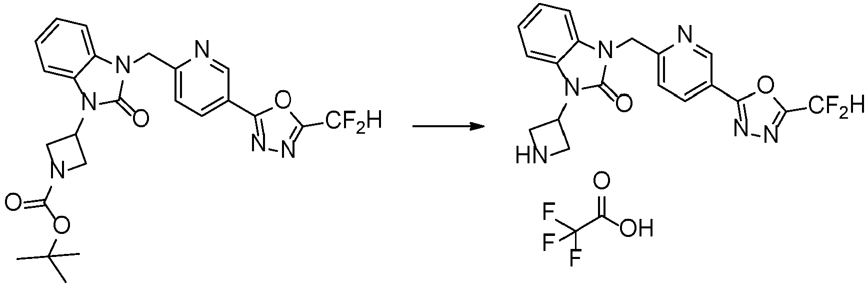

The tert-butyl 3-(3-((5-(5-(difluoromethyl)-1,3,4-oxadiazole-2-yl)pyridine-2-yl)methyl)-2-oxo-2,3-dihydro-1H-be nzo[d]imidazole-1-yl)azetidine-1-carboxylate (0.100 g, 0.201 mmol) prepared in the step 3 and trifluoroacetic acid (0.307 mL, 4.012 mmol) were dissolved in dichloromethane (5 mL) at room temperature, after which the resulting solution was stirred at the same temperature for 2 hours. A title compound was used without an additional purification process (0.100 g, 97.3%, yellow oil).

### [Step 5] Synthesis of the compound 7

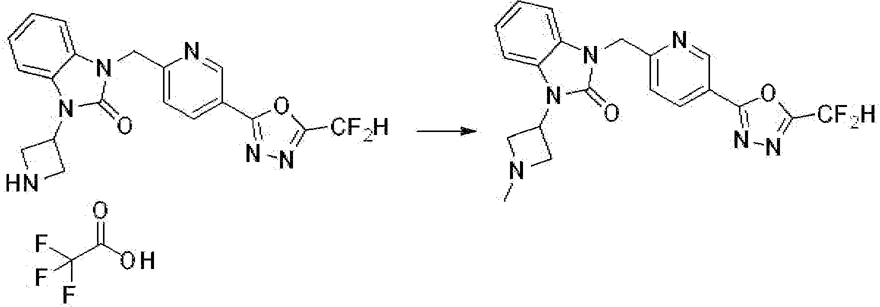

The 1-(azetidine-3-yl)-3-((5-(5-(difluoromethyl)-1,3,4-oxadiazole-2-yl)pyridine-2-yl)methyl)-1,3-dihydr o-2H-benzo[d]imidazole-2-one 2,2,2-trifluoroacetate (0.100 g, 0.195 mmol) prepared in the step 4 and N,N-diisopropylethylamine (0.034 mL, 0.195 mmol) were dissolved in dichloromethane (10 mL), after which the resulting solution was stirred at room temperature for 30 minutes, and then formaldehyde (37.00% solution, 0.029 mL, 0.390 mmol) and sodium triacetoxyborohydride (0.083 g, 0.390 mmol) were added thereinto and further stirred at the same temperature for 12 hours. Water was poured into the reaction mixture and an extraction was performed with dichloromethane. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; methanol/dichloromethane = 0 to 10%) and concentrated to obtain a title compound (0.050 g, 62.1%) in a colorless oil form.

**¹H NMR** (400 MHz, CDCl₃) δ 9.29 (dd, *J* = 2.2, 0.7 Hz, 1H), 8.37 (dd, *J* = 8.2, 2.2 Hz, 1H), 7.47 (dd, *J* = 8.2, 0.5 Hz, 1H), 7.21 - 7.01 (m, 3H), 7.07 (s, 0.25H), 6.97 (d, *J =* 19.6 Hz, 1H), 6.95 (s, 0.5H), 6.82 (s, 0.25H), 5.40 - 5.36 (m, 1H), 5.30 (s, 2H), 4.79 - 4.65 (m, 4H), 3.10 (s, 3H); LRMS (ES) m/z 413.4 (M⁺+ 1).

### Example 8: Synthesis of Compound 8, 1-((5-(5-(difluoromethyl)-1,3,4-oxadiazole-2-yl)pyridine-2-yl)methyl)-3-(1-methylpiperidine-4-yl)-3 ,4-dihydroquinazoline-2(1H)-one

### [Step 1] Synthesis of tert-butyl 4-((2-nitrobenzyl)amino)piperidine-1-carboxylate

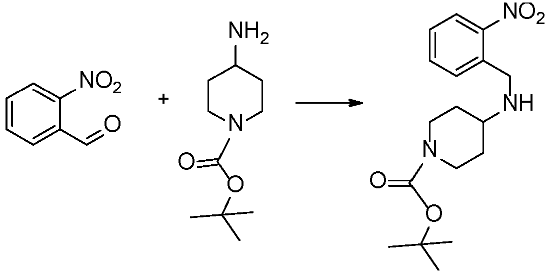

2-nitrobenzaldehyde (3.000 g, 19.852 mmol), tert-butyl 4-aminopiperidine-1-carboxylate (3.976 g, 19.852 mmol), sodium triacetoxyborohydride (8.415 g, 39.704 mmol) and acetic acid (2.273 mL, 39.704 mmol) were dissolved in 1,2-dichloroethane (30 mL) at room temperature, after which the resulting solution was stirred at the same temperature for 12 hours. Water was poured into the reaction mixture and an extraction was performed with dichloromethane. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; ethyl acetate/hexane = 0 to 50%) and concentrated to obtain a title compound (2.500 g, 37.5%) in a colorless oil form.

### [Step 2] Synthesis of tert-butyl 4-((2-aminobenzyl)amino)piperidine-1-carboxylate

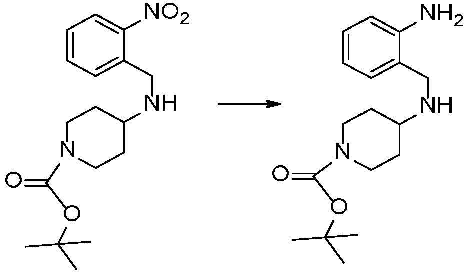

The tert-butyl 4-((2-nitrobenzyl)amino)piperidine-1-carboxylate (2.500 g, 7.454 mmol) prepared in the step 1 was dissolved in methanol (30 mL) at room temperature, after which 10%-Pd/C (250 mg) was slowly added thereinto and stirred at the same temperature for 12 hours in the presence of a hydrogen balloon attached thereto. The reaction mixture was filtered via a celite pad to remove a solid therefrom, after which solvent was removed from the resulting filtrate under reduced pressure, and then a title compound was used without an additional purification process (2.000 g, 87.9%, colorless oil).

### [Step 3] Synthesis of tert-butyl 4-(2-oxo-1,4-dihydroquinazoline-3(2H)-yl)piperidine-1-carboxylate

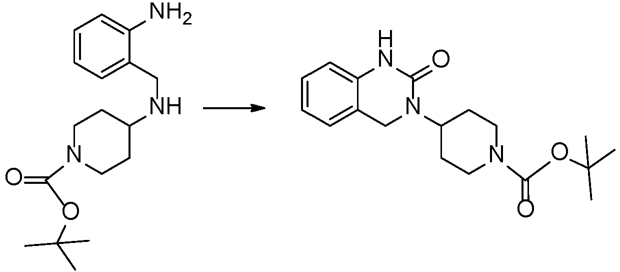

The tert-butyl 4-((2-aminobenzyl)amino)piperidine-1-carboxylate (2.000 g, 6.548 mmol) prepared in the step 2 and 1,1'-carbonyldiimidazole (CDI, 1.168 g, 7.203 mmol) were dissolved in tetrahydrofuran (30 mL) at room temperature, after which the resulting solution was stirred at the same temperature for 12 hours. Water was poured into the reaction mixture and an extraction was performed with ethyl acetate. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; ethyl acetate/hexane = 0 to 50%) and concentrated to obtain a title compound (0.900 g, 41.5%) in a white solid form.

### [Step 4] Synthesis of tert-butyl 4-(1-((5-(5-(difluoromethyl)-1,3,4-oxadiazole-2-yl)pyridine-2-yl)methyl)-2-oxo-1,4-dihydroquinazo line-3(2H)-yl)piperidine-1-carboxylate

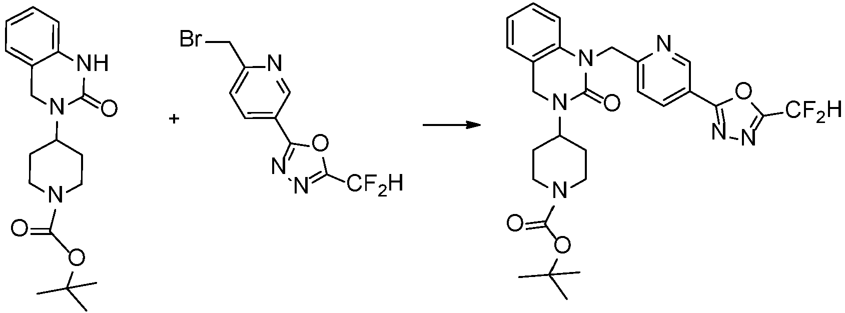

The tert-butyl 4-(2-oxo-1,4-dihydroquinazoline-3(2H)-yl)piperidine-1-carboxylate (0.200 g, 0.603 mmol) prepared in the step 3 was dissolved in N,N-dimethylformamide (20 mL) at 0°C, after which sodium hydride (60.00%, 0.036 g, 0.905 mmol) was added into the resulting solution and stirred at the same temperature for 30 minutes. 2-(6-(bromomethyl)pyridine-3-yl)-5-(difluoromethyl)-1,3,4-oxadiazole (0.193 g, 0.664 mmol) was added into the reaction mixture and further stirred at room temperature for 3 hours. Water was poured into the reaction mixture and an extraction was performed with ethyl acetate. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; ethyl acetate/hexane = 0 to 50%) and concentrated to obtain a title compound (0.160 g, 49.0%) in a colorless oil form.

### [Step 5] Synthesis of 1-((5-(5-(difluoromethyl)-1,3,4-oxadiazole-2-yl)pyridine-2-yl)methyl)-3-(piperidine-4-yl)-3,4-dihydr oquinazoline-2(1H)-one 2,2,2-trifluoroacetate

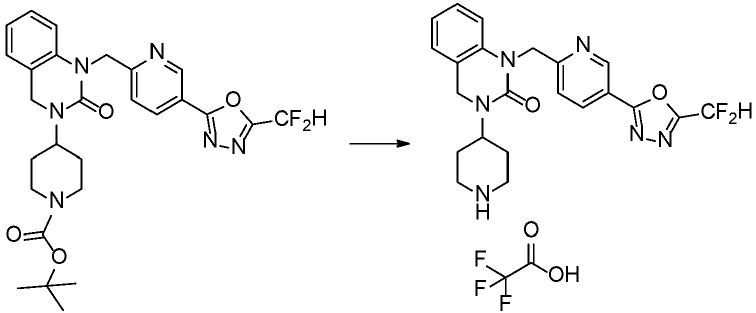

The tert-butyl 4-(1-((5-(5-(difluoromethyl)-1,3,4-oxadiazole-2-yl)pyridine-2-yl)methyl)-2-oxo-1,4-dihydroquinazo line-3(2H)-yl)piperidine-1-carboxylate (0.160 g, 0.296 mmol) prepared in the step 4 and trifluoroacetic acid (0.453 mL, 5.920 mmol) were dissolved in dichloromethane (5 mL) at room temperature, after which the resulting solution was stirred at the same temperature for 2 hours. A title compound was used without an additional purification process (0.160 g, 97.5%, yellow oil).

### [Step 6] Synthesis of the compound 8

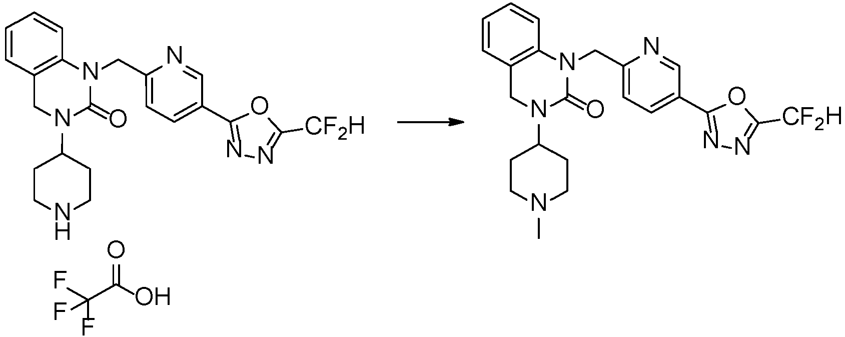

The 1-((5-(5-(difluoromethyl)-1,3,4-oxadiazole-2-yl)pyridine-2-yl)methyl)-3-(piperidine-4-yl)-3,4-dihydr oquinazoline-2(1H)-one 2,2,2-trifluoroacetate (0.160 g, 0.289 mmol) prepared in the step 5 and N,N-diisopropylethylamine (0.050 mL, 0.289 mmol) were dissolved in dichloromethane (10 mL), after which the resulting solution was stirred at room temperature for 30 minutes, and then formaldehyde (37.00% solution, 0.043 mL, 0.577 mmol) and sodium triacetoxyborohydride (0.122 g, 0.577 mmol) were added thereinto and further stirred at the same temperature for 12 hours. Water was poured into the reaction mixture and an extraction was performed with dichloromethane. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; methanol/dichloromethane = 0 to 10%) and concentrated to obtain a title compound (0.110 g, 83.9%) in a colorless oil form.

**¹H NMR** (400 MHz, CDCl₃) δ 9.31 - 9.30 (m, 1H), 8.30 (dd, *J* = 8.3, 2.2 Hz, 1H), 7.40 (d, *J* = 8.3 Hz, 1H), 7.16 - 7.12 (m, 2H), 7.07 (s, 0.25H), 7.02 - 7.01 (m, 1H), 6.94 (s, 0.5H), 6.81 (s, 0.25H), 6.72 (d, *J* = 8.1 Hz, 1H), 5.31 (s, 2H), 4.64 - 4.58 (m, 1H), 4.41 (s, 2H), 3.49 - 3.39 (m, 2H), 2.64 -2.58 (m, 5H), 2.35 -2.26 (m, 2H), 1.91 - 1.88 (m, 2H), 1.47 - 1.44 (m, 2H); **LRMS** (ES) m/z 455.4 (M⁺ + 1).

### Example 9: Synthesis of Compound 9, 1-((5-(5-(difluoromethyl)-1,3,4-oxadiazole-2-yl)pyridine-2-yl)methyl)-3-(1-methylazetidine-3-yl)-3, 4-dihydroquinazoline-2(1H)-one

### [Step 1] Synthesis of tert-butyl 3-((2-nitrobenzyl)amino)azetidine-1-carboxylate

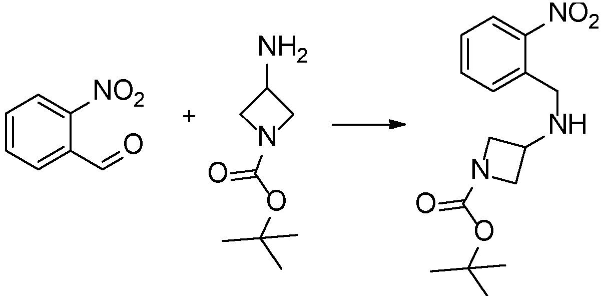

2-nitrobenzaldehyde (3.000 g, 19.852 mmol), tert-butyl 3-aminoazetidine-1-carboxylate (3.419 g, 19.852 mmol), sodium triacetoxyborohydride (8.415 g, 39.704 mmol) and acetic acid (2.273 mL, 39.704 mmol) were dissolved in 1,2-dichloroethane (30 mL) at room temperature, after which the resulting solution was stirred at the same temperature for 12 hours. Water was poured into the reaction mixture and an extraction was performed with dichloromethane. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; ethyl acetate/hexane = 0 to 50%) and concentrated to obtain a title compound (3.600 g, 59.0%) in a colorless oil form.

### [Step 2] Synthesis of tert-butyl 3-((2-aminobenzyl)amino)azetidine-1-carboxylate

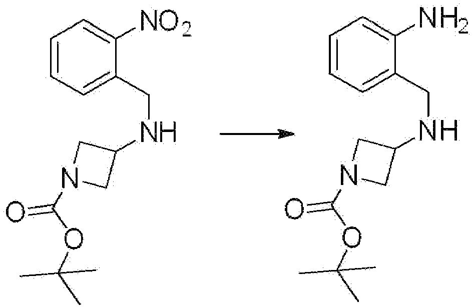

The tert-butyl 3-((2-nitrobenzyl)amino)azetidine-1-carboxylate (3.600 g, 11.713 mmol) prepared in the step 1 was dissolved in methanol (30 mL) at room temperature, after which 10%-Pd/C (360 mg) was slowly added thereinto and stirred at the same temperature for 12 hours in the presence of a hydrogen balloon attached thereto. The reaction mixture was filtered via a celite pad to remove a solid therefrom, after which solvent was removed from the resulting filtrate under reduced pressure, and then a title compound was used without an additional purification process (3.000 g, 92.3%, colorless oil).

### [Step 3] Synthesis of tert-butyl 3-(2-oxo-1,4-dihydroquinazoline-3(2H)-yl)azetidine-1-carboxylate

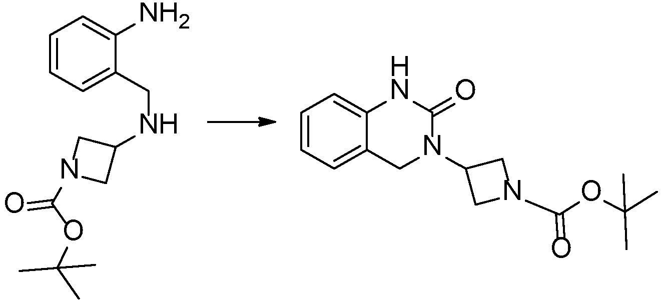

The tert-butyl 3-((2-aminobenzyl)amino)azetidine-1-carboxylate (3.000 g, 10.816 mmol) prepared in the step 2 and 1,1'-carbonyldiimidazole (CDI, 1.929 g, 11.897 mmol) were dissolved in tetrahydrofuran (30 mL) at room temperature, after which the resulting solution was stirred at the same temperature for 12 hours. Water was poured into the reaction mixture and an extraction was performed with ethyl acetate. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; ethyl acetate/hexane = 0 to 50%) and concentrated to obtain a title compound (1.300 g, 39.6%) in a white solid form.

### [Step 4] Synthesis of tert-butyl 3-(1-((5-(5-(difluoromethyl)-1,3,4-oxadiazole-2-yl)pyridine-2-yl)methyl)-2-oxo-1,4-dihydroquinazo line-3(2H)-yl)azetidine-1-carboxylate

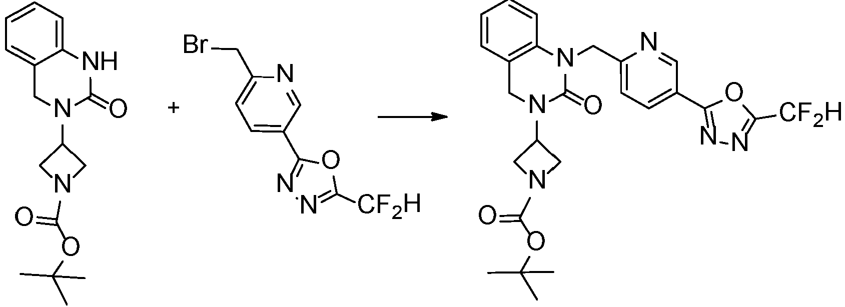

The tert-butyl 3-(2-oxo-1,4-dihydroquinazoline-3(2H)-yl)azetidine-1-carboxylate (0.200 g, 0.659 mmol) prepared in the step 3 was dissolved in N,N-dimethylformamide (20 mL) at 0°C, after which sodium hydride (60.00%, 0.040 g, 0.989 mmol) was added into the resulting solution and stirred at the same temperature for 30 minutes. 2-(6-(bromomethyl)pyridine-3-yl)-5-(difluoromethyl)-1,3,4-oxadiazole (0.210 g, 0.725 mmol) was added into the reaction mixture and further stirred at room temperature for 3 hours. Water was poured into the reaction mixture and an extraction was performed with ethyl acetate. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; ethyl acetate/hexane = 0 to 50%) and concentrated to obtain a title compound (0.150 g, 44.4%) in a colorless oil form.

### [Step 5] Synthesis of 3-(azetidine-3-yl)-1-((5-(5-(difluoromethyl)-1,3,4-oxadiazole-2-yl)pyridine-2-yl)methyl)-3,4-dihydr oquinazoline-2(1H)-one 2,2,2-trifluoroacetate

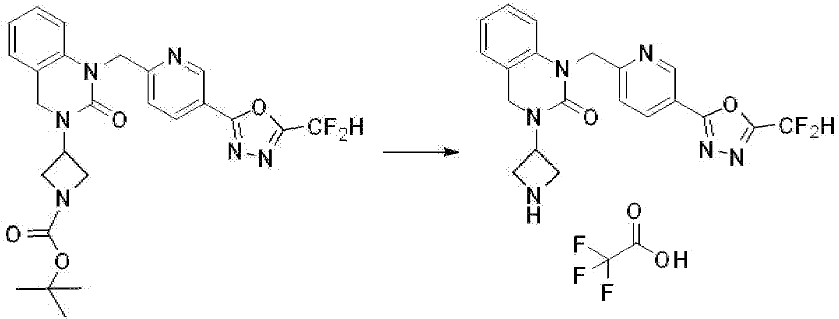

The tert-butyl 3-(1-((5-(5-(difluoromethyl)-1,3,4-oxadiazole-2-yl)pyridine-2-yl)methyl)-2-oxo-1,4-dihydroquinazo line-3(2H)-yl)azetidine-1-carboxylate (0.150 g, 0.293 mmol) prepared in the step 4 and trifluoroacetic acid (0.448 mL, 5.853 mmol) were dissolved in dichloromethane (5 mL) at room temperature, after which the resulting solution was stirred at the same temperature for 2 hours. A title compound was used without an additional purification process (0.150 g, 97.4%, yellow oil).

### [Step 6] Synthesis of the compound 9

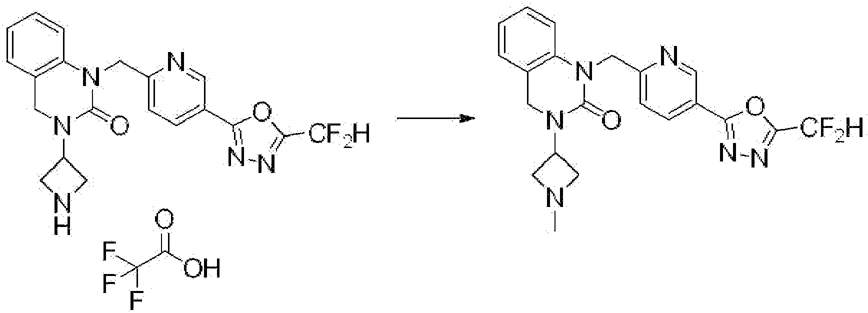

The 3-(azetidine-3-yl)-1-((5-(5-(difluoromethyl)-1,3,4-oxadiazole-2-yl)pyridine-2-yl)methyl)-3,4-dihydr oquinazoline-2(1H)-one 2,2,2-trifluoroacetate (0.150 g, 0.285 mmol) prepared in the step 5 and N,N-diisopropylethylamine (0.050 mL, 0.285 mmol) were dissolved in dichloromethane (10 mL), after which the resulting solution was stirred at room temperature for 30 minutes, and then formaldehyde (37.00% solution, 0.042 mL, 0.570 mmol) and sodium triacetoxyborohydride (0.121 g, 0.570 mmol) were added thereinto and further stirred at the same temperature for 12 hours. Water was poured into the reaction mixture and an extraction was performed with dichloromethane. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; methanol/dichloromethane = 0 to 10%) and concentrated to obtain a title compound (0.100 g, 82.3%) in a colorless oil form.

**¹H NMR** (400 MHz, CDCl₃) δ 9.31 (dd, *J* = 2.2, 0.8 Hz, 1H), 8.34 (dd, *J* = 8.3, 2.2 Hz, 1H), 7.42 (dd, J = 8.3, 0.8 Hz, 1H), 7.19 - 7.12 (m, 2H), 7.08 (s, 0.25H), 7.08 - 7.03 (m, 1H), 7.01 (s, 0.5H), 6.95 (s, 0.25H), 6.74 (d, *J* = 8.0 Hz, 1H), 5.31 (s, 2H), 4.71 - 4.67 (m, 1H), 4.49 (s, 2H), 4.32 - 4.28 (m, 2H), 4.19 - 4.15 (m, 2H), 2.83 (s, 3H); **LRMS** (ES) m/z 427.3 (M⁺+ 1).

### Example 10: Synthesis of Compound 10, 1-((5-(5-(difluoromethyl)-1,3,4-oxadiazole-2-yl)pyridine-2-yl)methyl)-3-((1-methylpiperidine-4-yl) methyl)-1,3-dihydro-2H-benzo[d]imidazole-2-one

### [Step 1] Synthesis of tert-butyl 4-(((2-aminophenyl)amino)methyl)piperidine-1-carboxylate

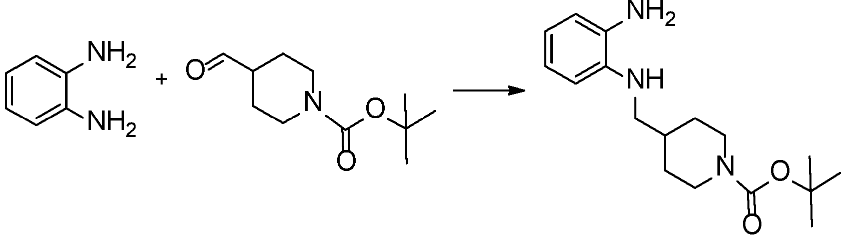

Benzene-1,2-diamine (1.700 g, 15.720 mmol), tert-butyl 4-formylpiperidine-1-carboxylate (10.059 g, 47.161 mmol), sodium triacetoxyborohydride (6.664 g, 31.441 mmol) and acetic acid (1.800 mL, 31.441 mmol) were dissolved in 1,2-dichloroethane (30 mL) at room temperature, after which the resulting solution was stirred at the same temperature for 12 hours. Water was poured into the reaction mixture and an extraction was performed with dichloromethane. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; ethyl acetate/hexane = 0 to 50%) and concentrated to obtain a title compound (1.350 g, 28.1%) in a white solid form.

### [Step 2] Synthesis of tert-butyl 4-((2-oxo-2,3-dihydro-1H-benzo[d]imidazole-1-yl)methyl)piperidine-1-carboxylate

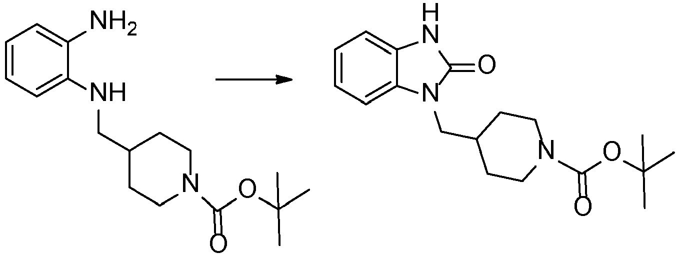

The tert-butyl 4-(((2-aminophenyl)amino)methyl)piperidine-1-carboxylate (1.350 g, 4.420 mmol) prepared in the step 1 and 1,1'-carbonyldiimidazole (CDI, 0.788 g, 4.862 mmol) were dissolved in tetrahydrofuran (10 mL) at room temperature, after which the resulting solution was stirred at the same temperature for 12 hours. Water was poured into the reaction mixture and an extraction was performed with ethyl acetate. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; ethyl acetate/hexane = 0 to 30%) and concentrated to obtain a title compound (1 g, 68.3%) in a white solid form.

### [Step 3] Synthesis of tert-butyl 4-((3-((5-(5-(difluoromethyl)-1,3,4-oxadiazole-2-yl)pyridine-2-yl)methyl)-2-oxo-2,3-dihydro-1H-be nzo[d]imidazole-1-yl)methyl)piperidine-1-carboxylate

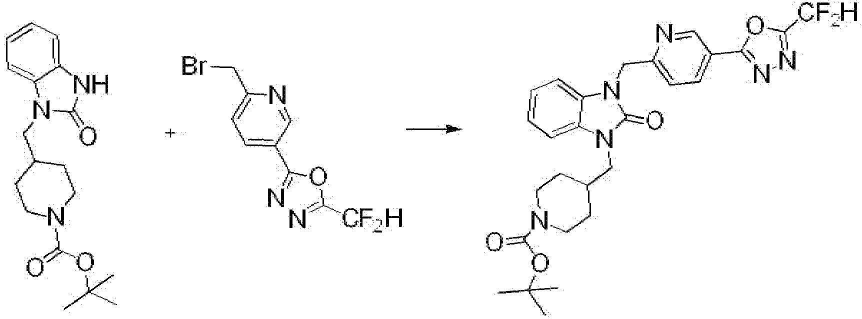

The tert-butyl 4-((2-oxo-2,3-dihydro-1H-benzo[d]imidazole-1-yl)methyl)piperidine-1-carboxylate (0.200 g, 0.603 mmol) prepared in the step 2 was dissolved in N,N-dimethylformamide (20 mL) at 0°C, after which sodium hydride (60.00%, 0.036 g, 0.905 mmol) was added into the resulting solution and stirred at the same temperature for 30 minutes. 2-(6-(bromomethyl)pyridine-3-yl)-5-(difluoromethyl)-1,3,4-oxadiazole (0.193 g, 0.664 mmol) was added into the reaction mixture and further stirred at room temperature for 3 hours. Water was poured into the reaction mixture and an extraction was performed with ethyl acetate. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; ethyl acetate/hexane = 0 to 50%) and concentrated to obtain a title compound (0.150 g, 46.0%) in a colorless oil form.

### [Step 4] Synthesis of 1-((5-(5-(difluoromethyl)-1,3,4-oxadiazole-2-yl)pyridine-2-yl)methyl)-3-(piperidine-4-ylmethyl)-1,3 -dihydro-2H-benzo[d]imidazole-2-one 2,2,2-trifluoroacetate

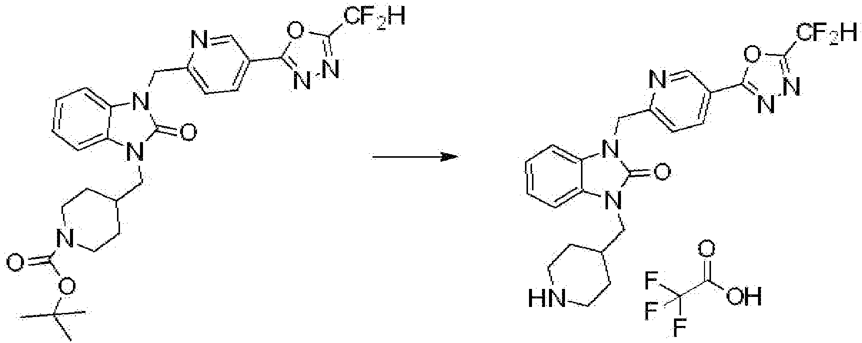

The tert-butyl 4-((3-((5-(5-(difluoromethyl)-1,3,4-oxadiazole-2-yl)pyridine-2-yl)methyl)-2-oxo-2,3-dihydro-1H-be nzo[d]imidazole-1-yl)methyl)piperidine-1-carboxylate (0.150 g, 0.277 mmol) prepared in the step 3 and trifluoroacetic acid (0.425 mL, 5.550 mmol) were dissolved in dichloromethane (5 mL) at room temperature, after which the resulting solution was stirred at the same temperature for 2 hours. A title compound was used without an additional purification process (0.150 g, 97.5%, yellow oil).

### [Step 5] Synthesis of the compound 10

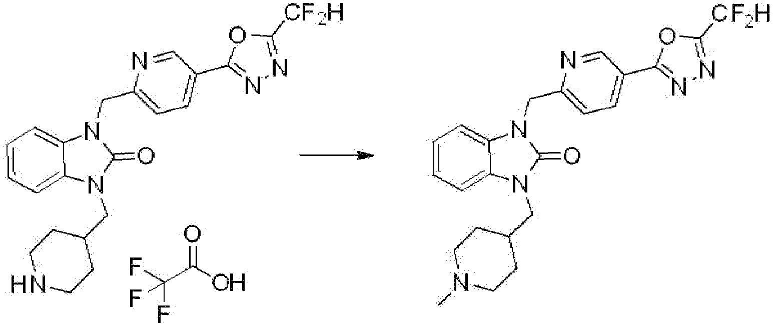

The 1-(4-(5-(difluoromethyl)-1,3,4-oxadiazole-2-yl)benzyl)-3-(piperidine-4-ylmethyl)-1,3-dihydro-2H-b enzo[d]imidazole-2-one 2,2,2-trifluoroacetate (0.150 g, 0.271 mmol) prepared in the step 4 and N,N-diisopropylethylamine (0.047 mL, 0.271 mmol) were dissolved in dichloromethane (10 mL), after which the resulting solution was stirred at room temperature for 30 minutes, and then formaldehyde (37.00% solution, 0.040 mL, 0.542 mmol) and sodium triacetoxyborohydride (0.115 g, 0.542 mmol) were added thereinto and further stirred at the same temperature for 12 hours. Water was poured into the reaction mixture and an extraction was performed with dichloromethane. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; methanol/dichloromethane = 0 to 10%) and concentrated to obtain a title compound (0.080 g, 65.1%) in a colorless oil form.

**¹H NMR** (400 MHz, CDCl₃) δ 9.29 (d, *J* = 1.6 Hz, 1H), 8.34 (dd, *J* = 8.2, 2.1 Hz, 1H), 7.42 (d, *J* = 8.0 Hz, 1H), 7.15 - 6.69 (m, 4H), 7.07 (s, 0.25H), 6.96 (s, 0.5H), 6.81 (s, 0.25H), 5.31 (s, 2H), 3.88 (d, *J* = 7.2 Hz, 2H), 3.42 - 3.39 (m, 2H), 2.67 (s, 3H), 2.55 - 2.50 (m, 2H), 2.05 - 2.04 (m, 1H), 1.93 - 1.84 (m, 2H), 1.47 - 1.42 (m, 2H); **LRMS** (ES) m/z 455.4 (M⁺+ 1).

### Example 11: Synthesis of Compound 11, 1-((5-(5-(difluoromethyl)-1,3,4-oxadiazole-2-yl)pyridine-2-yl)methyl)-3-(oxetan-3-yl)-1,3-dihydro-2H-benzo[d]imidazole-2-one

### [Step 1] Synthesis of N-(2-nitrophenyl)oxetan-3-amine

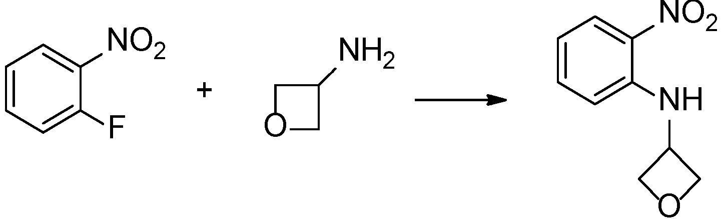

1-fluoro-2-nitrobenzene (2.000 g, 14.174 mmol), oxetan-3-amine (1.140 g, 15.592 mmol) and potassium carbonate (3.918 g, 28.349 mmol) were dissolved in tetrahydrofuran (30 mL) at 70°C, after which the resulting solution was stirred at the same temperature for 12 hours, and then a reaction was finished by lowering a temperature to room temperature. Water was poured into the reaction mixture and an extraction was performed with ethyl acetate. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 40 g cartridge; ethyl acetate/hexane = 0 to 30%) and concentrated to obtain a title compound (1.750 g, 63.6%) in a colorless oil form.

### [Step 2] Synthesis of N¹-(oxetan-3-yl)benzene-1,2-diamine

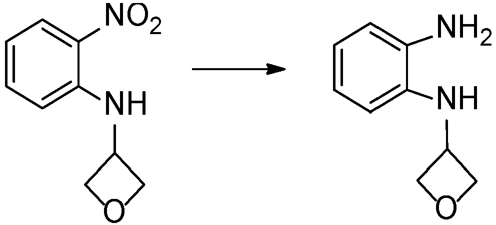

The N-(2-nitrophenyl)oxetan-3-amine (1.750 g, 9.012 mmol) prepared in the step 1 was dissolved in methanol (30 mL) at room temperature, after which 10%-Pd/C (170 mg) was slowly added thereinto and stirred at the same temperature for 12 hours in the presence of a hydrogen balloon attached thereto. The reaction mixture was filtered via a celite pad to remove a solid therefrom, after which solvent was removed from the resulting filtrate under reduced pressure, and then a title compound was used without an additional purification process (1.000 g, 67.6%, yellow oil).

### [Step 3] Synthesis of 1-(oxetan-3-yl)-1,3-dihydro-2H-benzo[d]imidazole-2-one

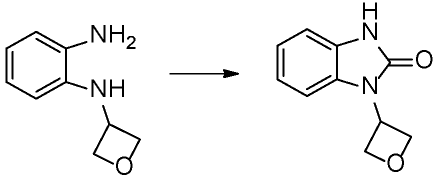

The N¹-(oxetan-3-yl)benzene-1,2-diamine (1.000 g, 6.090 mmol) prepared in the step 2 and 1,1'-carbonyldiimidazole (CDI, 1.086 g, 6.699 mmol) were dissolved in tetrahydrofuran (10 mL) at room temperature, after which the resulting solution was stirred at the same temperature for 12 hours. Water was poured into the reaction mixture and an extraction was performed with ethyl acetate. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; ethyl acetate/hexane = 0 to 50%) and concentrated to obtain a title compound (0.400 g, 34.5%) in a brown solid form.

### [Step 4] Synthesis of the compound 11

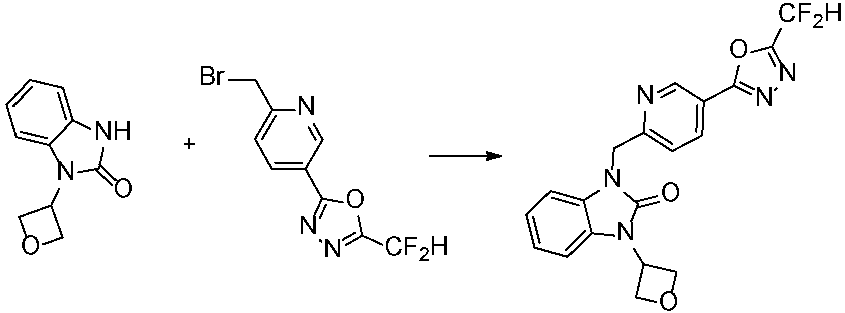

The 1-(oxetan-3-yl)-1,3-dihydro-2H-benzo[d]imidazole-2-one (0.100 g, 0.526 mmol) prepared in the step 3 was dissolved in N,N-dimethylformamide (10 mL) at 0°C, after which sodium hydride (60.00%, 0.032 g, 0.789 mmol) was added into the resulting solution and stirred at the same temperature for 30 minutes. 2-(6-(bromomethyl)pyridine-3-yl)-5-(difluoromethyl)-1,3,4-oxadiazole (0.168 g, 0.578 mmol) was added into the reaction mixture and further stirred at room temperature for 3 hours. Water was poured into the reaction mixture and an extraction was performed with ethyl acetate. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; ethyl acetate/hexane = 0 to 70%) and concentrated to obtain a title compound (0.110 g, 52.4%) in a colorless oil form.

**¹H NMR** (400 MHz, CDCl₃) δ 9.31 - 9.30 (m, 1H), 8.35 (dd, *J* = 8.2, 2.2 Hz, 1H), 7.75 - 7.73 (m, 1H), 7.45 (dd, *J* = 8.2, 0.8 Hz, 1H), 7.22 (td, *J* = 7.7, 1.3 Hz, 1H), 7.14 (td, *J* = 7.7, 1.1 Hz, 1H), 7.06 - 7.04 (m, 1H), 7.07 (s, 0.25H), 6.94 (s, 0.5H), 6.81 (s, 0.25H), 5.77 - 5.70 (m, 1H), 5.30 (s, 2H), 5.26 - 5.23 (m, 2H), 5.18 - 5.15 (m, 2H); **LRMS** (ES) m/z 400.3 (M⁺+ 1).

### Example 12: Synthesis of Compound 12, 1-((5-(5-(difluoromethyl)-1,3,4-oxadiazole-2-yl)pyridine-2-yl)methyl)-3-(tetrahydro-2H-pyran-4-yl) -1,3-dihydro-2H-benzo[d]imidazole-2-one

### [Step 1] Synthesis of N-(2-nitrophenyl)tetrahydro-2H-pyran-4-amine

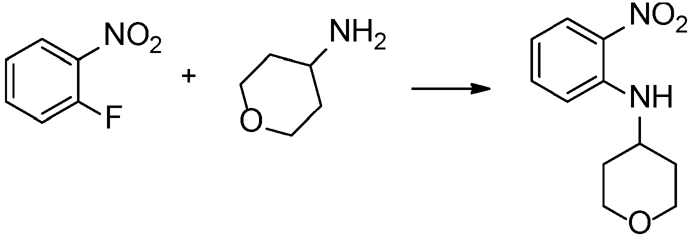

1-fluoro-2-nitrobenzene (2.000 g, 14.174 mmol), tetrahydro-2H-pyran-4-amine (1.577 g, 15.592 mmol) and potassium carbonate (3.918 g, 28.349 mmol) were dissolved in tetrahydrofuran (30 mL) at 70°C, after which the resulting solution was stirred at the same temperature for 12 hours, and then a reaction was finished by lowering a temperature to room temperature. Water was poured into the reaction mixture and an extraction was performed with ethyl acetate. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 40 g cartridge; ethyl acetate/hexane = 0 to 30%) and concentrated to obtain a title compound (1.800 g, 57.1%) in a yellow oil form.

### [Step 2] Synthesis of N¹-(tetrahydro-2H-pyran-4-yl)benzene-1,2-diamine

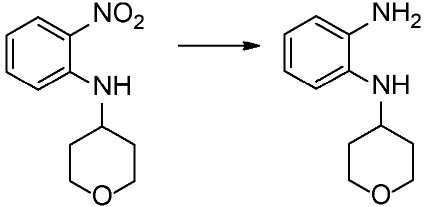

The N-(2-nitrophenyl)tetrahydro-2H-pyran-4-amine (1.800 g, 8.099 mmol) prepared in the step 1 was dissolved in methanol (30 mL) at room temperature, after which 0.1%-Pd/C (180 mg) was slowly added thereinto and stirred at the same temperature for 12 hours in the presence of a hydrogen balloon attached thereto. The reaction mixture was filtered via a celite pad to remove a solid therefrom, after which solvent was removed from the resulting filtrate under reduced pressure, and then a title compound was used without an additional purification process (1.710 g, 109.8%, yellow oil).

### [Step 3] Synthesis of 1-(tetrahydro-2H-pyran-4-yl)-1,3-dihydro-2H-benzo[d]imidazole-2-one

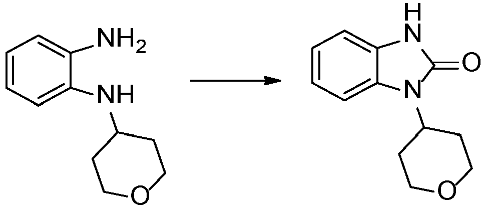

The N¹-(tetrahydro-2H-pyran-4-yl)benzene-1,2-diamine (1.710 g, 8.894 mmol) prepared in the step 2 and 1,1'-carbonyldiimidazole (CDI, 1.586 g, 9.784 mmol) were dissolved in tetrahydrofuran (10 mL) at room temperature, after which the resulting solution was stirred at the same temperature for 12 hours. Water was poured into the reaction mixture and an extraction was performed with ethyl acetate. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; ethyl acetate/hexane = 0 to 50%) and concentrated to obtain a title compound (0.900 g, 46.4%) in a black solid form.

### [Step 4] Synthesis of the compound 12

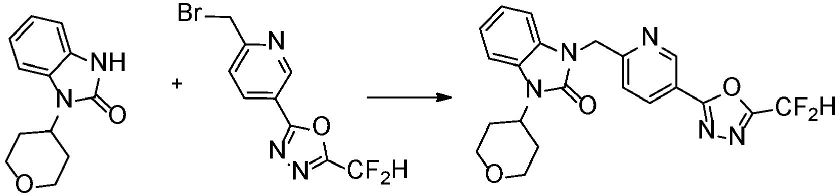

The 1-(tetrahydro-2H-pyran-4-yl)-1,3-dihydro-2H-benzo[d]imidazole-2-one (0.100 g, 0.458 mmol) prepared in the step 3 was dissolved in N,N-dimethylformamide (10 mL) at 0°C, after which sodium hydride (60.00%, 0.027 g, 0.687 mmol) was added into the resulting solution and stirred at the same temperature for 30 minutes. 2-(6-(bromomethyl)pyridine-3-yl)-5-(difluoromethyl)-1,3,4-oxadiazole (0.146 g, 0.504 mmol) was added into the reaction mixture and further stirred at room temperature for 3 hours. Water was poured into the reaction mixture and an extraction was performed with ethyl acetate. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; ethyl acetate/hexane = 0 to 70%) and concentrated to obtain a title compound (0.080 g, 40.9%) in a colorless oil form.

**¹H NMR** (400 MHz, CDCl₃) δ 9.29 (dd, *J =* 2.2, 0.8 Hz, 1H), 8.32 (dd, *J* = 8.2, 2.2 Hz, 1H), 7.41 - 7.39 (m, 1H), 7.28 - 7.26 (m, 1H), 7.11 - 7.00 (m, 3H), 7.07 (s, 0.25H), 6.94 (s, 0.5H), 6.81 (s, 0.25H), 5.30 (s, 2H), 4.68 - 4.61 (m, 1H), 4.17 - 4.13 (m, 2H), 3.61 - 3.54 (m, 2H), 2.59 - 2.48 (m, 2H), 1.84 - 1.80 (m, 2H); **LRMS** (ES) m/z 428.3 (M⁺ + 1).

### Example 13: Synthesis of Compound 13, 1-((5-(5-(difluoromethyl)-1,3,4-oxadiazole-2-yl)pyridine-2-yl)methyl)-3-(1,1-dioxydotetrahydro-2H -thiopyran-4-yl)-1,3-dihydro-2H-benzo[d]imidazole-2-one

### [Step 1] Synthesis of 4-((2-nitrophenyl)amino)tetrahydro-2H-thiopyran 1,1-dioxide

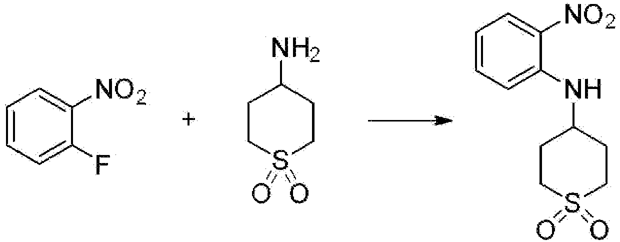

1-fluoro-2-nitrobenzene (1.710 g, 12.119 mmol), 4-aminotetrahydro-2H-thiopyran 1,1-dioxide (1.808 g, 12.119 mmol) and triethylamine (3.378 mL, 24.238 mmol) were dissolved in N,N-dimethylformamide (30 mL) at 90°C, after which the resulting solution was stirred at the same temperature for 24 hours, and then a reaction was finished by lowering a temperature to room temperature. Water was poured into the reaction mixture and an extraction was performed with ethyl acetate. An organic layer was washed with saturated sodium chloride aqueous solution, then dehydrated with anhydrous sodium sulfate, then filtered, and then concentrated under reduced pressure. Ethyl acetate (20 mL) and hexane (10 mL) were inserted into the resulting concentrate and stirred to filter out a precipitated solid, then washed with hexane, and then dried to obtain a title compound (1.730 g, 52.8%) in a yellow solid form.

### [Step 2] Synthesis of 4-((2-aminophenyl)amino)tetrahydro-2H-thiopyran 1,1-dioxide

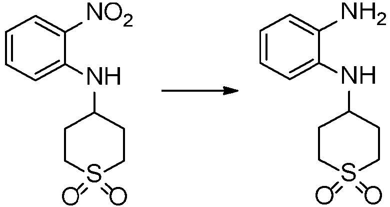

The 4-((2-nitrophenyl)amino)tetrahydro-2H-thiopyran 1,1-dioxide (1.730 g, 8.268 mmol) prepared in the step 1 was dissolved in methanol (30 mL) at room temperature, after which 10%-Pd/C (170 mg) was slowly added thereinto and stirred at the same temperature for 12 hours in the presence of a hydrogen balloon attached thereto. The reaction mixture was filtered via a celite pad to remove a solid therefrom, after which solvent was removed from the resulting filtrate under reduced pressure, and then a title compound was used without an additional purification process (0.310 g, 15.6%, white solid).

### [Step 3] Synthesis of 1-(1,1-dioxydotetrahydro-2H-thiopyran-4-yl)-1,3-dihydro-2H-benzo[d]imidazole-2-one

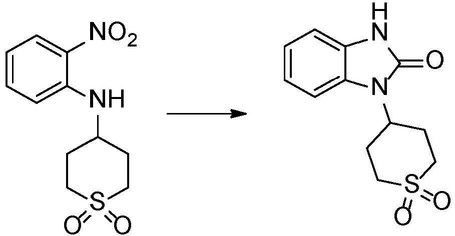

The 4-((2-aminophenyl)amino)tetrahydro-2H-thiopyran 1,1-dioxide (0.310 g, 1.290 mmol) prepared in the step 2 and 1,1'-carbonyldiimidazole (CDI, 0.230 g, 1.419 mmol) were dissolved in tetrahydrofuran (10 mL) at room temperature, after which the resulting solution was stirred at the same temperature for 12 hours. Water was poured into the reaction mixture and an extraction was performed with ethyl acetate. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; ethyl acetate/hexane = 0 to 70%) and concentrated to obtain a title compound (0.220 g, 64.0%) in a white solid form.

### [Step 4] Synthesis of the compound 13

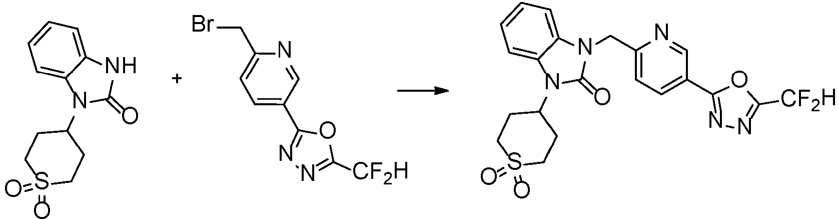

The 1-(1,1-dioxydotetrahydro-2H-thiopyran-4-yl)-1,3-dihydro-2H-benzo[d]imidazole-2-one (0.100 g, 0.375 mmol) prepared in the step 3 was dissolved in N,N-dimethylformamide (10 mL) at 0°C, after which sodium hydride (60.00%, 0.023 g, 0.563 mmol) was added into the resulting solution and stirred at the same temperature for 30 minutes. 2-(6-(bromomethyl)pyridine-3-yl)-5-(difluoromethyl)-1,3,4-oxadiazole (0.120 g, 0.413 mmol) was added into the reaction mixture and further stirred at room temperature for 3 hours. Water was poured into the reaction mixture and an extraction was performed with ethyl acetate. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; ethyl acetate/hexane = 0 to 70%) and concentrated to obtain a title compound (0.100 g, 56.0%) in a colorless oil form.

**¹H NMR** (400 MHz, CDCl₃) δ 9.27 (dd, *J* = 2.2, 0.8 Hz, 1H), 8.33 (dd, *J* = 8.2, 2.2 Hz, 1H), 7.42 (dd, *J* = 8.2, 0.5 Hz, 1H), 7.29 - 7.27 (m, 1H), 7.12 - 7.01 (m, 3H), 7.07 (s, 1H), 6.94 (s, 1H), 6.81 (s, 1H), 5.28 (s, 2H), 3.32 - 3.08 (m, 6H), 2.28 - 2.24 (m, 2H); LRMS (ES) m/z 476.4 (M⁺ + 1).

### Example 14: Synthesis of Compound 14, 1-(4-(5-(difluoromethyl)-1,3,4-oxadiazole-2-yl)-2-fluorobenzyl)-3-(1-ethylpiperidine-4-yl)-1,3-dihy dro-2H-benzo[d]imidazole-2-one

### [Step 1] Synthesis of the compound 14

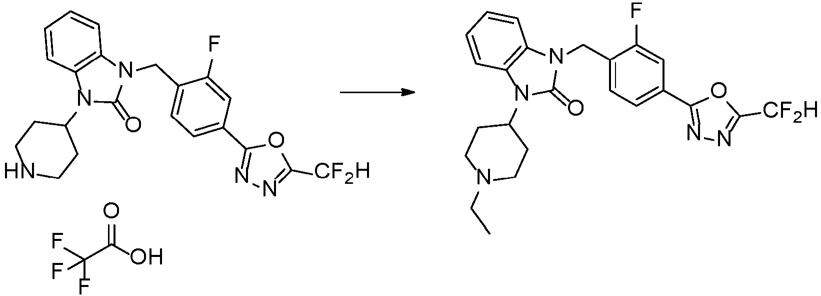

1-(4-(5-(difluoromethyl)-1,3,4-oxadiazole-2-yl)-2-fluorobenzyl)-3-(piperidine-4-yl)-1,3-dih ydro-2H-benzo[d]imidazole-2-one 2,2,2-trifluoroacetate (0.323 g, 0.579 mmol), acetaldehyde (0.051 g, 1.159 mmol), N,N-diisopropylethylamine (0.101 mL, 0.579 mmol) and sodium triacetoxyborohydride (0.246 g, 1.159 mmol) were dissolved in dichloromethane (10 mL) at room temperature, after which the resulting solution was stirred at the same temperature for 8 hours. Water was poured into the reaction mixture and an extraction was performed with dichloromethane. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; methanol/dichloromethane = 0 to 10%) and concentrated to obtain a title compound (0.180 g, 65.9%) in a colorless oil form.

**¹H NMR** (400 MHz, CDCl₃) δ 7.85 - 7.82 (m, 2H), 7.48 - 7.42 (m, 1H), 7.36 - 7.32 (m, 1H), 7.10 - 7.07 (m, 2H), 7.05 - 7.02 (m, 1H), 7.07 (s, 1H), 6.94 (s, 1H), 6.79 (s, 1H), 5.21 (s, 2H), 4.51 - 4.46 (m, 1H), 3.17 - 3.14 (m, 2H), 2.52 - 2.49 (m, 4H), 2.19 - 2.13 (m, 2H), 1.90 - 1.87 (m, 2H), 1.17 - 1.14 (m, 3H).

### Example 15: Synthesis of Compound 15, 1-(4-(5-(difluoromethyl)-1,3,4-oxadiazole-2-yl)-2-fluorobenzyl)-3-(1-isopropylpiperidine-4-yl)-1,3-dihydro-2H-benzo[d]imidazole-2-one

### [Step 1] Synthesis of the compound 15

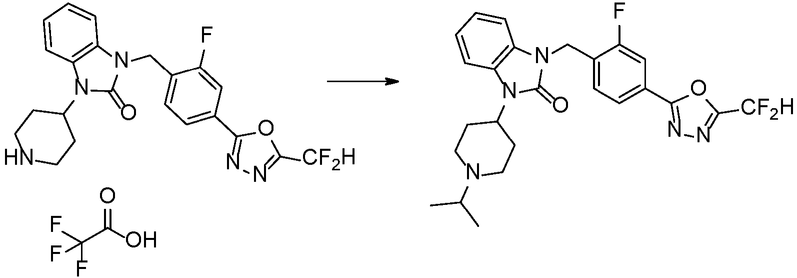

1-(4-(5-(difluoromethyl)-1,3,4-oxadiazole-2-yl)-2-fluorobenzyl)-3-(piperidine-4-yl)-1,3-dih ydro-2H-benzo[d]imidazole-2-one 2,2,2-trifluoroacetate (0.300 g, 0.538 mmol), acetone (0.080 mL, 1.076 mmol), N,N-diisopropylethylamine (0.094 mL, 0.538 mmol) and sodium triacetoxyborohydride (0.228 g, 1.076 mmol) were dissolved in dichloromethane (10 mL) at room temperature, after which the resulting solution was stirred at the same temperature for 8 hours. Water was poured into the reaction mixture and an extraction was performed with dichloromethane. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; methanol/dichloromethane = 0 to 10%) and concentrated to obtain a title compound (0.200 g, 76.5%) in a colorless oil form.

**¹H NMR** (400 MHz, CDCl₃) δ 7.88 - 7.83 (m, 2H), 7.50 - 7.43 (m, 2H), 7.13 - 7.05 (m, 2H), 6.98 - 6.95 (m, 1H), 7.07 (s, 0.25H), 6.92 (s, 0.5H), 6.79 (s, 0.25H), 5.21 (s, 2H), 4.60 - 4.58 (m, 1H), 3.32 - 3.29 (m, 3H), 2.70 - 2.62 (m, 4H), 1.96 - 1.93 (m, 2H), 1.29 - 1.23 (m, 6H); LRMS (ES) m/z 486.3 (M⁺ + 1).

### Example 16: Synthesis of Compound 16, 1-(4-(5-(difluoromethyl)-1,3,4-oxadiazole-2-yl)-2-fluorobenzyl)-3-(1-(oxetan-3-yl)piperidine-4-yl)-1,3-dihydro-2H-benzo[d]imidazole-2-one

### [Step 1] Synthesis of the compound 16

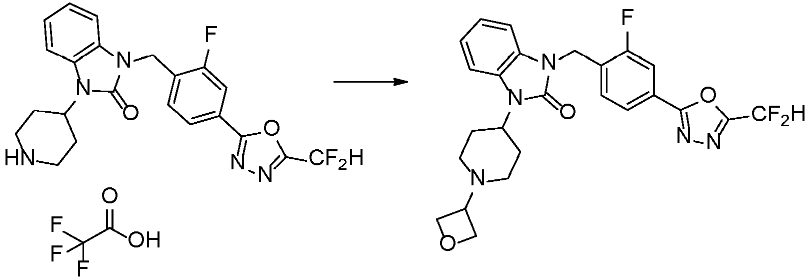

1-(4-(5-(difluoromethyl)-1,3,4-oxadiazole-2-yl)-2-fluorobenzyl)-3-(piperidine-4-yl)-1,3-dih ydro-2H-benzo[d]imidazole-2-one 2,2,2-trifluoroacetate (0.300 g, 0.538 mmol), oxetan-3-one (0.078 g, 1.076 mmol), N,N-diisopropylethylamine (0.094 mL, 0.538 mmol) and sodium triacetoxyborohydride (0.228 g, 1.076 mmol) were dissolved in dichloromethane (10 mL) at room temperature, after which the resulting solution was stirred at the same temperature for 8 hours. Water was poured into the reaction mixture and an extraction was performed with dichloromethane. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; methanol/dichloromethane = 0 to 10%) and concentrated to obtain a title compound (0.180 g, 67.0%) in a colorless oil form.

**¹H NMR** (400 MHz, CDCl₃) δ 7.85 - 7.81 (m, 2H), 7.46 - 7.42 (m, 1H), 7.36 - 7.31 (m, 1H), 7.20 - 7.05 (m, 2H), 6.98 - 6.90 (m, 1H), 7.07 (s, 0.25H), 6.91 (s, 0.5H), 6.78 (s, 0.25H), 5.20 (s, 2H), 4.71 - 4.64 (m, 4H), 4.50 - 4.46 (m, 1H), 3.57 - 3.54 (m, 1H), 2.95 - 2.92 (m, 2H), 2.54 - 2.49 (m, 2H), 2.05 - 2.02 (m, 2H), 1.89 - 1.86 (m, 2H); LRMS (ES) m/z 500.4 (M⁺ + 1).

### Example 17: Synthesis of Compound 17, 1-(1-acetylpiperidine-4-yl)-3-(4-(5-(difluoromethyl)-1,3,4-oxadiazole-2-yl)-2-fluorobenzyl)-1,3-dih ydro-2H-benzo[d]imidazole-2-one

### [Step 1] Synthesis of the compound 17

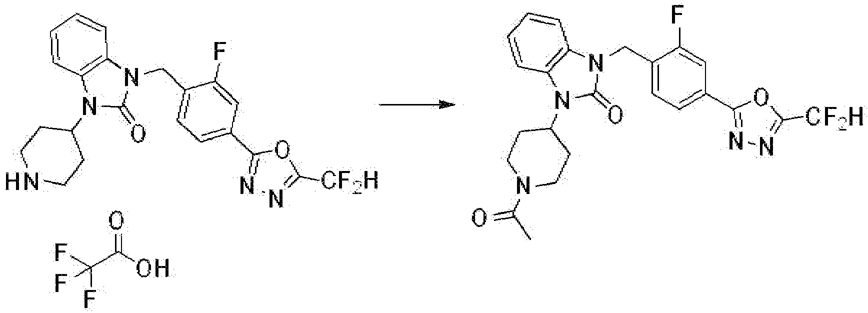

1-(4-(5-(difluoromethyl)-1,3,4-oxadiazole-2-yl)-2-fluorobenzyl)-3-(piperidine-4-yl)-1,3-dih ydro-2H-benzo[d]imidazole-2-one 2,2,2-trifluoroacetate (0.300 g, 0.538 mmol), acetyl chloride (0.077 mL, 1.076 mmol), and N,N-diisopropylethylamine (0.187 mL, 1.076 mmol) were dissolved in dichloromethane (10 mL) at room temperature, after which the resulting solution was stirred at the same temperature for 8 hours. Water was poured into the reaction mixture and an extraction was performed with dichloromethane. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; methanol/dichloromethane = 0 to 10%) and concentrated to obtain a title compound (0.110 g, 42.1%) in a colorless oil form.

**¹H NMR** (400 MHz, CDCl₃) δ 7.90 - 7.83 (m, 2H), 7.48 - 7.44 (m, 1H), 7.16 - 7.11 (m, 3H), 7.09 - 7.04 (m, 1H), 7.07 (s, 1H), 6.92 (s, 1H), 6.79 (s, 1H), 5.20 (s, 2H), 4.91 - 4.87 (m, 1H), 4.65 - 4.60 (m, 1H), 4.15 - 4.11 (m, 1H), 3.30 - 3.26 (m, 1H), 2.73 - 2.69 (m, 1H), 2.40 - 2.32 (m, 2H), 2.18 (s, 3H), 1.98 - 1.94 (m, 2H); LRMS (ES) m/z 486.3 (M⁺ + 1).

### Example 18: Synthesis of Compound 18, 1-(4-(5-(difluoromethyl)-1,3,4-oxadiazole-2-yl)-2-fluorobenzyl)-3-(1-(methylsulfonyl)piperidine-4-yl)-1,3-dihydro-2H-benzo[d]imidazole-2-one

### [Step 1] Synthesis of the compound 18

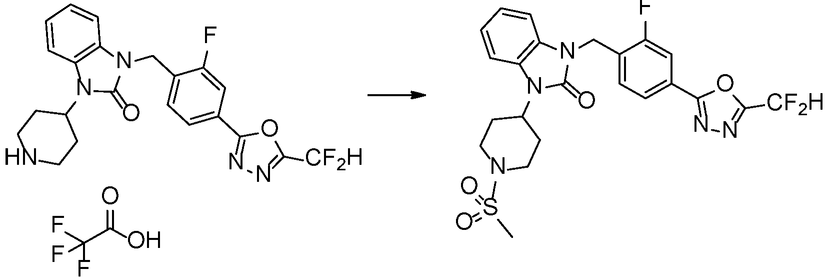

1-(4-(5-(difluoromethyl)-1,3,4-oxadiazole-2-yl)-2-fluorobenzyl)-3-(piperidine-4-yl)-1,3-dih ydro-2H-benzo[d]imidazole-2-one 2,2,2-trifluoroacetate (0.300 g, 0.538 mmol), methanesulfonyl chloride (0.083 mL, 1.076 mmol) and N,N-diisopropylethylamine (0.187 mL, 1.076 mmol) were dissolved in dichloromethane (10 mL) at room temperature, after which the resulting solution was stirred at the same temperature for 8 hours. Water was poured into the reaction mixture and an extraction was performed with dichloromethane. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; methanol/dichloromethane = 0 to 10%) and concentrated to obtain a title compound (0.150 g, 53.4%) in a white solid form.

**¹H NMR** (400 MHz, CDCl₃) δ 7.90 - 7.84 (m, 2H), 7.48 - 7.44 (m, 1H), 7.26 - 7.24 (m, 1H), 7.15 - 7.06 (m, 2H), 7.07 (s, 0.25H), 7.06 - 7.01 (m, 1H), 6.91 (s, 0.5H), 6.79 (s, 0.25H), 5.22 (s, 2H), 4.60 - 4.56 (m, 1H), 4.07 - 4.04 (m, 2H), 2.94 - 2.87 (m, 5H), 2.61 - 2.56 (m, 2H), 2.06 - 1.98 (m, 2H); **LRMS** (ES) m/z 507.2 (M⁺ + 1).

### Example 19: Synthesis of Compound 19, 1-(1-((1H-indole-7-yl)methyl)piperidine-4-yl)-3-(4-(5-(difluoromethyl)-1,3,4-oxadiazole-2-yl)-2-flu orobenzyl)-1,3-dihydro-2H-benzo[d]imidazole-2-one

### [Step 1] Synthesis of the compound 19

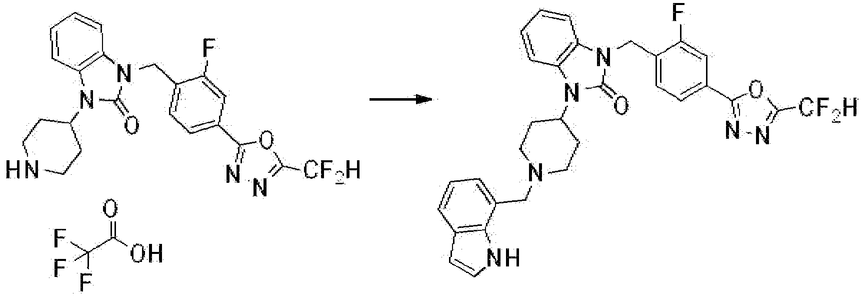

1-(4-(5-(difluoromethyl)-1,3,4-oxadiazole-2-yl)-2-fluorobenzyl)-3-(piperidine-4-yl)-1,3-dih ydro-2H-benzo[d]imidazole-2-one 2,2,2-trifluoroacetate (0.200 g, 0.359 mmol), 1H-indole-7-carbaldehyde (0.078 g, 0.538 mmol), N,N-diisopropylethylamine (0.062 mL, 0.359 mmol) and sodium triacetoxyborohydride (0.152 g, 0.718 mmol) were dissolved in dichloromethane (10 mL) at room temperature, after which the resulting solution was stirred at the same temperature for 8 hours. Water was poured into the reaction mixture and an extraction was performed with dichloromethane. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; methanol/dichloromethane = 0 to 10%) and concentrated to obtain a title compound (0.110 g, 53.5%) in a colorless oil form.

**¹H NMR** (400 MHz, CDCl₃) δ 9.80 (br s, 1H), 7.88 - 7.83 (m, 2H), 7.62 (d, *J =* 7.6 Hz, 1H), 7.49 - 7.46 (m, 1H), 7.33 - 7.32 (m, 1H), 7.26 - 7.23 (m, 1H), 7.16 - 6.98 (m, 5H), 7.07 (s, 0.25H), 6.92 (s, 0.5H), 6.79 (s, 0.25H), 6.59 - 6.57 (m, 1H), 5.21 (s, 2H), 4.39 - 4.37 (m, 1H), 3.96 (s, 2H), 3.19 - 3.17 (m, 2H), 2.64 - 2.60 (m, 2H), 2.07 - 2.02 (m, 2H), 1.91 - 1.88 (m, 2H); LRMS (ES) m/z 574.4 (M⁺ + 1).

### Example 20: Synthesis of Compound 20, 1-(4-(5-(difluoromethyl)-1,3,4-oxadiazole-2-yl)-2-fluorobenzyl)-3-(1-methylpyrrolidine-3-yl)-1,3-di hydro-2H-benzo[d]imidazole-2-one

### [Step 1] Synthesis of benzyl 3-(3-(2-fluoro-4-(methoxycarbonyl)benzyl)-2-oxo-2,3-dihydro-1H-benzo[d]imidazole-1-yl)pyrrolidi ne-1-carboxylate

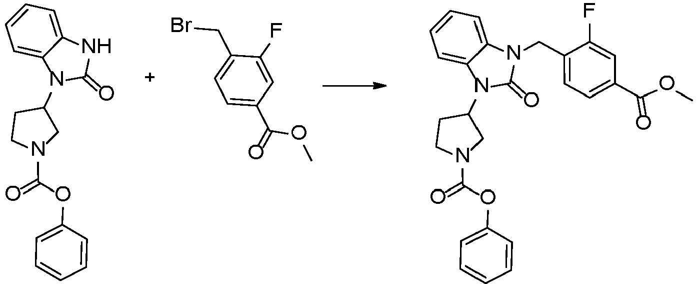

Benzyl 3-(2-oxo-2,3-dihydro-1H-benzo[d]imidazole-1-yl)pyrrolidine-1-carboxylate (0.580 g, 1.719 mmol) was dissolved in N,N-dimethylformamide (30 mL) at 0°C, after which sodium hydride (60.00%, 0.103 g, 2.579 mmol) was added into the resulting solution and stirred at the same temperature for 30 minutes. Methyl 4-(bromomethyl)-3-fluorobenzoate (0.425 g, 1.719 mmol) was added into the reaction mixture and further stirred at room temperature for 3 hours. Water was poured into the reaction mixture and an extraction was performed with ethyl acetate. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 40 g cartridge; ethyl acetate/hexane = 0 to 50%) and concentrated to obtain a title compound (0.780 g, 90.1%) in a colorless oil form.

### [Step 2] Synthesis of benzyl 3-(3-(2-fluoro-4-(hydrazinecarbonyl)benzyl)-2-oxo-2,3-dihydro-1H-benzo[d]imidazole-1-yl)pyrroli dine-1-carboxylate

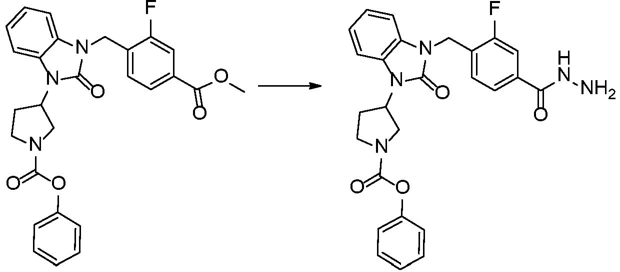

The benzyl 3-(3-(2-fluoro-4-(methoxycarbonyl)benzyl)-2-oxo-2,3-dihydro-1H-benzo[d]imidazole-1-yl)pyrrolidi ne-1-carboxylate (0.780 g, 1.549 mmol) prepared in the step 1 and hydrazine monohydrate (1.506 mL, 30.981 mmol) were dissolved in ethanol (10 mL) at 80°C, after which the resulting solution was stirred at the same temperature for 12 hours, and then a reaction was finished by lowering a temperature to room temperature. Solvent was removed from the reaction mixture under reduced pressure, after which a title compound was used without an additional purification process (0.780 g, 100.0%, colorless oil).

### [Step 3] Synthesis of benzyl 3-(3-(4-(5-(difluoromethyl)-1,3,4-oxadiazole-2-yl)-2-fluorobenzyl)-2-oxo-2,3-dihydro-1H-benzo[d]i midazole-1-yl)pyrrolidine-1-carboxylate

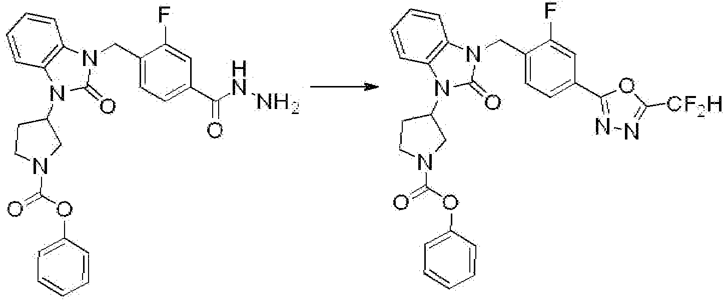

The benzyl 3-(3-(2-fluoro-4-(hydrazinecarbonyl)benzyl)-2-oxo-2,3-dihydro-1H-benzo[d]imidazole-1-yl)pyrroli dine-1-carboxylate (0.780 g, 1.549 mmol) prepared in the step 2, 2,2-difluoroacetic anhydride (0.578 mL, 4.647 mmol) and imidazole (0.316 g, 4.647 mmol) were dissolved in dichloromethane (30 mL) at 45°C, after which the resulting solution was stirred at the same temperature for 12 hours, and then a reaction was finished by lowering a temperature to room temperature. Water was poured into the reaction mixture and an extraction was performed with dichloromethane. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 40 g cartridge; ethyl acetate/hexane = 0 to 30%) and concentrated to obtain a title compound (0.700 g, 80.2%) in a colorless oil form.

### [Step 4] Synthesis of 1-(4-(5-(difluoromethyl)-1,3,4-oxadiazole-2-yl)-2-fluorobenzyl)-3-(pyrrolidine-3-yl)-1,3-dihydro-2H -benzo[d]imidazole-2-one

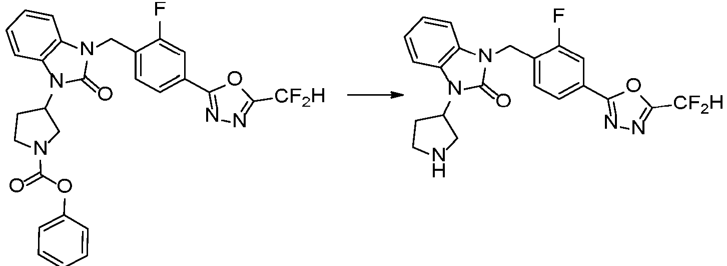

The benzyl 3-(3-(4-(5-(difluoromethyl)-1,3,4-oxadiazole-2-yl)-2-fluorobenzyl)-2-oxo-2,3-dihydro-1H-benzo[d]i midazole-1-yl)pyrrolidine-1-carboxylate (0.700 g, 1.242 mmol) prepared in the step 3 was dissolved in methanol (20 mL) at room temperature, after which 10%-Pd/C (70 mg) was slowly added thereinto and stirred at the same temperature for 12 hours in the presence of a hydrogen balloon attached thereto. The reaction mixture was filtered via a celite pad to remove a solid therefrom, after which solvent was removed from the resulting filtrate under reduced pressure, and then a title compound was used without an additional purification process (0.500 g, 93.7%, white solid).

### [Step 5] Synthesis of the compound 20

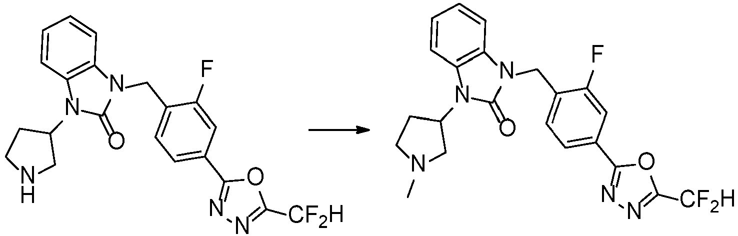

The 1-(4-(5-(difluoromethyl)-1,3,4-oxadiazole-2-yl)-2-fluorobenzyl)-3-(pyrrolidine-3-yl)-1,3-dihydro-2H -benzo[d]imidazole-2-one (0.190 g, 0.442 mmol) prepared in the step 4, formaldehyde (0.027 g, 0.885 mmol) and sodium triacetoxyborohydride (0.188 g, 0.885 mmol) were dissolved in dichloromethane (10 mL) at room temperature, after which the resulting solution was stirred at the same temperature for 12 hours. Water was poured into the reaction mixture and an extraction was performed with dichloromethane. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; methanol/dichloromethane = 0 to 10%) and concentrated to obtain a title compound (0.110 g, 56.1%) in a colorless oil form.

¹H NMR (400 MHz, CDCl₃) δ 7.87 - 7.83 (m, 2H), 7.60 - 7.58 (m, 1H), 7.46 (t, *J* = 7.8 Hz, 1H), 7.14 - 7.05 (m, 2H), 6.97 - 6.92 (m, 1H), 7.07 (s, 0.25H), 6.92 (s, 0.5H), 6.79 (s, 0.25H), 5.26 - 5.23 (m, 1H), 5.20 (s, 2H), 3.17 - 3.13 (m, 2H), 2.98 - 2.93 (m, 1H), 2.73 - 2.68 (m, 1H), 2.51 (s, 3H), 2.38 - 2.31 (m, 2H); **LRMS** (ES) m/z 444.4 (M⁺ + 1).

### Example 21: Synthesis of Compound 21, 1-(4-(5-(difluoromethyl)-1,3,4-oxadiazole-2-yl)-2-fluorobenzyl)-3-(1-(oxetan-3-yl)pyrrolidine-3-yl)-1,3-dihydro-2H-benzo[d]imidazole-2-one

### [Step 1] Synthesis of the compound 21

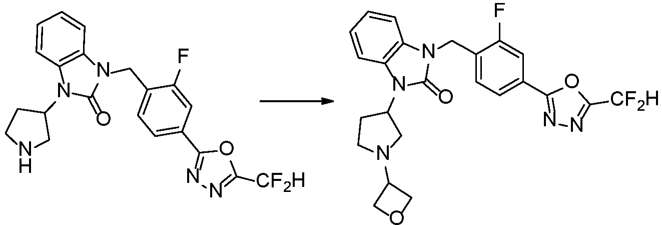

1-(4-(5-(difluoromethyl)-1,3,4-oxadiazole-2-yl)-2-fluorobenzyl)-3-(pyrrolidine-3-yl)-1,3-di hydro-2H-benzo[d]imidazole-2-one (0.210 g, 0.489 mmol), oxetan-3-one (0.070 g, 0.978 mmol) and sodium triacetoxyborohydride (0.207 g, 0.978 mmol) were dissolved in dichloromethane (10 mL) at room temperature, after which the resulting solution was stirred at the same temperature for 12 hours. Water was poured into the reaction mixture and an extraction was performed with dichloromethane. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; methanol/dichloromethane = 0 to 10%) and concentrated to obtain a title compound (0.100 g, 42.1%) in a colorless oil form.

**¹H NMR** (400 MHz, CDCl₃) δ 7.88 - 7.77 (m, 3H), 7.49 - 7.45 (m, 1H), 7.18 - 7.05 (m, 2H), 6.99 - 6.97 (m, 1H), 7.07 (s, 0.25H), 6.92 (s, 0.5H), 6.79 (s, 0.25H), 5.30 - 5.28 (m, 1H), 5.21 (s, 2H), 4.78 - 4.65 (m, 4H), 3.73 - 3.70 (m, 1H), 3.12 - 3.08 (m, 2H), 2.71 - 2.66 (m, 1H), 2.42 - 2.28 (m, 3H); **LRMS** (ES) m/z 486.4 (M⁺ + 1).

### Example 22: Synthesis of Compound 22, 1-(4-(5-(difluoromethyl)-1,3,4-oxadiazole-2-yl)-2-fluorobenzyl)-3-(1-methylazetidine-3-yl)-1,3-dih ydro-2H-benzo[d]imidazole-2-one

### [Step 1] Synthesis of tert-butyl 3-(3-(2-fluoro-4-(methoxycarbonyl)benzyl)-2-oxo-2,3-dihydro-1H-benzo[d]imidazole-1-yl)azetidin e-1-carboxylate

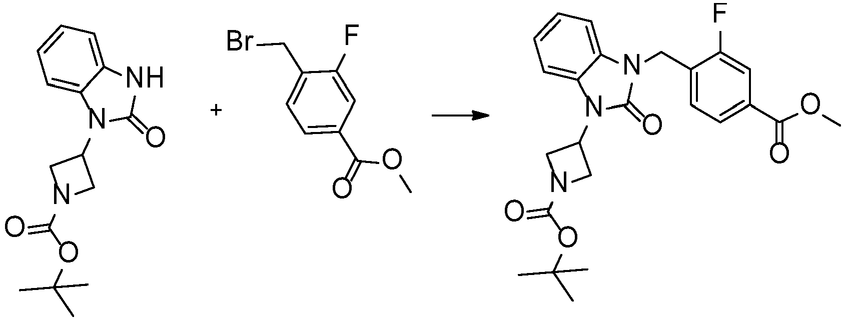

Tert-butyl 3-(2-oxo-2,3-dihydro-1H-benzo[d]imidazole-1-yl)azetidine-1-carboxylate (0.570 g, 1.970 mmol) was dissolved in N,N-dimethylformamide (30 mL) at 0°C, after which sodium hydride (60.00%, 0.118 g, 2.955 mmol) was added into the resulting solution and stirred at the same temperature for 30 minutes. Methyl 4-(bromomethyl)-3-fluorobenzoate (0.487 g, 1.970 mmol) was added into the reaction mixture and further stirred at room temperature for 3 hours. Water was poured into the reaction mixture and an extraction was performed with ethyl acetate. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 40 g cartridge; ethyl acetate/hexane = 0 to 50%) and concentrated to obtain a title compound (0.610 g, 68.0%) in a colorless oil form.

### [Step 2] Synthesis of tert-butyl 3-(3-(2-fluoro-4-(hydrazinecarbonyl)benzyl)-2-oxo-2,3-dihydro-1H-benzo[d]imidazole-1-yl)azetidi ne-1-carboxylate

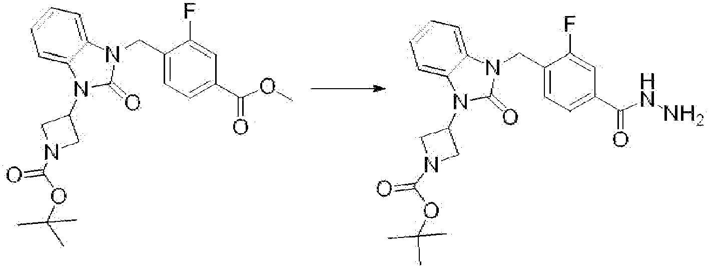

The tert-butyl 3-(3-(2-fluoro-4-(methoxycarbonyl)benzyl)-2-oxo-2,3-dihydro-1H-benzo[d]imidazole-1-yl)azetidin e-1-carboxylate (0.610 g, 1.339 mmol) prepared in the step 1 and hydrazine monohydrate (1.302 mL, 26.784 mmol) were dissolved in ethanol (10 mL) at 80°C, after which the resulting solution was stirred at the same temperature for 12 hours, and then a reaction was finished by lowering a temperature to room temperature. Solvent was removed from the reaction mixture under reduced pressure, after which a title compound was used without an additional purification process (0.610 g, 100.0%, colorless oil).

### [Step 3] Synthesis of tert-butyl 3-(3-(4-(5-(difluoromethyl)-1,3,4-oxadiazole-2-yl)-2-fluorobenzyl)-2-oxo-2,3-dihydro-1H-benzo[d]i midazole-1-yl)azetidine-1-carboxylate

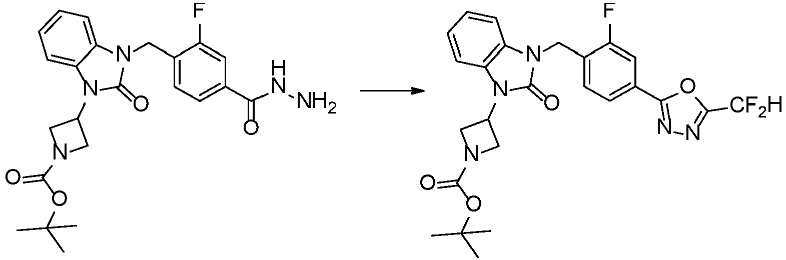

The tert-butyl 3-(3-(2-fluoro-4-(hydrazinecarbonyl)benzyl)-2-oxo-2,3-dihydro-1H-benzo[d]imidazole-1-yl)azetidi ne-1-carboxylate (0.610 g, 1.339 mmol) prepared in the step 2, 2,2-difluoroacetic anhydride (0.499 mL, 4.018 mmol) and imidazole (0.274 g, 4.018 mmol) were dissolved in dichloromethane (30 mL) at 45°C, after which the resulting solution was stirred at the same temperature for 12 hours, and then a reaction was finished by lowering a temperature to room temperature. Water was poured into the reaction mixture and an extraction was performed with dichloromethane. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 40 g cartridge; ethyl acetate/hexane = 0 to 30%) and concentrated to obtain a title compound (0.600 g, 86.9%) in a colorless oil form.

### [Step 4] Synthesis of 1-(azetidine-3-yl)-3-(4-(5-(difluoromethyl)-1,3,4-oxadiazole-2-yl)-2-fluorobenzyl)-1,3-dihydro-2H-benzo[d]imidazole-2-one 2,2,2-trifluoroacetate

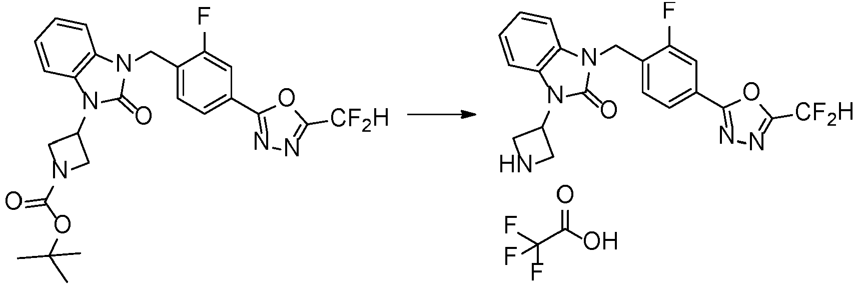

The tert-butyl 3-(3-(4-(5-(difluoromethyl)-1,3,4-oxadiazole-2-yl)-2-fluorobenzyl)-2-oxo-2,3-dihydro-1H-benzo[d]i midazole-1-yl)azetidine-1-carboxylate (0.600 g, 1.164 mmol) prepared in the step 3 and trifluoroacetic acid (1.783 mL, 23.279 mmol) were dissolved in dichloromethane (20 mL) at room temperature, after which the resulting solution was stirred at the same temperature for 12 hours. Solvent was removed from the reaction mixture under reduced pressure, after which a title compound was used without an additional purification process (0.600 g, 97.4%, yellow oil).

### [Step 5] Synthesis of the compound 22

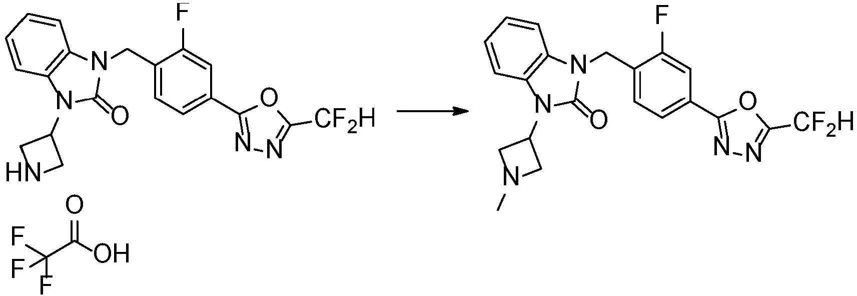

The 1-(azetidine-3-yl)-3-(4-(5-(difluoromethyl)-1,3,4-oxadiazole-2-yl)-2-fluorobenzyl)-1,3-dihydro-2H-benzo[d]imidazole-2-one 2,2,2-trifluoroacetate (0.300 g, 0.567 mmol) prepared in the step 4 and N,N-diisopropylethylamine (0.099 mL, 0.567 mmol) were dissolved in dichloromethane (10 mL), after which the resulting solution was stirred at room temperature for 30 minutes, and then formaldehyde (0.034 g, 1.133 mmol) and sodium triacetoxyborohydride (0.240 g, 1.133 mmol) were added thereinto and further stirred at the same temperature for 12 hours. Water was poured into the reaction mixture and an extraction was performed with dichloromethane. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; methanol/dichloromethane = 0 to 10%) and concentrated to obtain a title compound (0.160 g, 65.8%) in a colorless oil form.

¹H NMR (400 MHz, CDCl₃) δ 7.87 - 7.84 (m, 2H), 7.50 - 7.42 (m, 2H), 7.17 - 7.05 (m, 2H), 7.00 - 6.98 (m, 1H), 7.05 (s, 0.25H), 6.92 (s, 0.5H), 6.79 (s, 0.5H), 5.21 - 5.15 (m, 3H), 4.24 - 4.16 (m, 4H), 2.75 (s, 3H); LRMS (ES) m/z 430.4 (M⁺ + 1).

### Example 23: Synthesis of Compound 23, 1-(4-(5-(difluoromethyl)-1,3,4-oxadiazole-2-yl)-2-fluorobenzyl)-3-(1-(oxetan-3-yl)azetidine-3-yl)-1 ,3-dihydro-2H-benzo[d]imidazole-2-one

### [Step 1] Synthesis of the compound 23

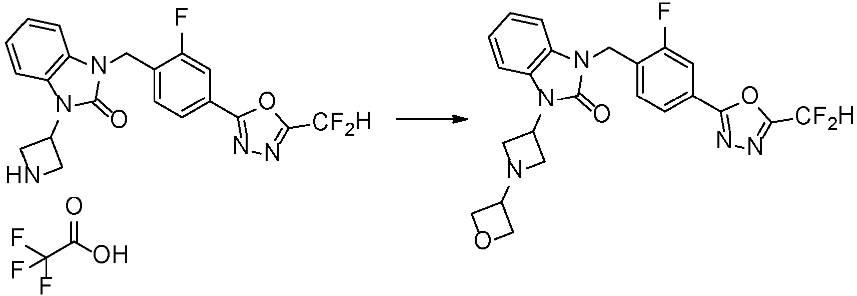

1-(azetidine-3-yl)-3-(4-(5-(difluoromethyl)-1,3,4-oxadiazole-2-yl)-2-fluorobenzyl)-1,3-dihy dro-2H-benzo[d]imidazole-2-one 2,2,2-trifluoroacetate (0.300 g, 0.567 mmol) and N,N-diisopropylethylamine (0.099 mL, 0.567 mmol) were dissolved in dichloromethane (10 mL), after which the resulting solution was stirred at room temperature for 30 minutes, and then oxetan-3-one (0.082 g, 1.133 mmol) and sodium triacetoxyborohydride (0.240 g, 1.133 mmol) were added thereinto and further stirred at the same temperature for 12 hours. Water was poured into the reaction mixture and an extraction was performed with dichloromethane. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; methanol/dichloromethane = 0 to 10%) and concentrated to obtain a title compound (0.100 g, 37.4%) in a colorless oil form.

**¹H NMR** (400 MHz, CDCl₃) δ 7.87 - 7.83 (m, 2H), 7.63 - 7.61 (m, 1H), 7.48 - 7.45 (m, 1H), 7.18 - 7.09 (m, 2H), 7.05 (s, 0.25H), 7.00 - 6.98 (m, 1H), 6.92 (s, 0.5H), 6.79 (s, 0.25H), 5.19 (s, 2H), 5.15 - 5.10 (m, 1H), 4.80 - 4.77 (m, 2H), 4.65 - 4.62 (m, 2H), 3.99 - 3.96 (m, 3H), 3.87 - 3.83 (m, 2H); LRMS (ES) m/z 472.3 (M⁺ + 1).

### Example 24: Synthesis of Compound 24, 1-(4-(5-(difluoromethyl)-1,3,4-oxadiazole-2-yl)benzyl)-3-(2-oxaspiro[3.3]heptane-6-yl)-1,3-dihydr o-2H-benzo[d]imidazole-2-one

### [Step 1] Synthesis of N-(2-(2-oxa-6-azaspiro[3.3]heptane-6-yl)ethyl)-2-nitroaniline

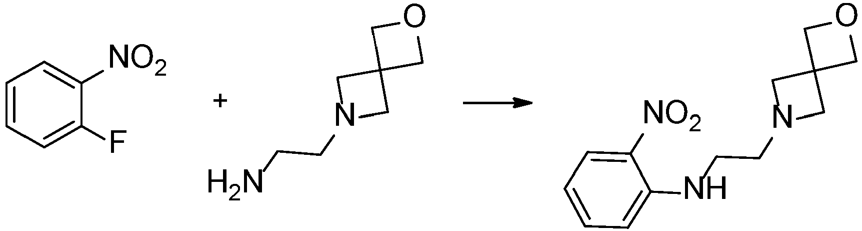

1-fluoro-2-nitrobenzene (0.400 g, 2.835 mmol), 2-(2-oxa-6-azaspiro[3.3]heptane-6-yl)ethane-1-amine (0.403 g, 2.835 mmol) and potassium carbonate (0.784 g, 5.670 mmol) were dissolved in tetrahydrofuran (20 mL) at room temperature, after which the resulting solution was heated under reflux for 2 hours, and then a reaction was finished by lowering a temperature to room temperature. Saturated sodium hydrogen carbonate aqueous solution was poured into the reaction mixture and an extraction was performed with dichloromethane. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; methanol/dichloromethane = 0 to 5%) and concentrated to obtain a title compound (0.388 g, 52.0%) in a yellow oil form.

### [Step 2] Synthesis of N¹-(2-(2-oxa-6-azaspiro[3.3]heptane-6-yl)ethyl)benzene-1,2-diamine

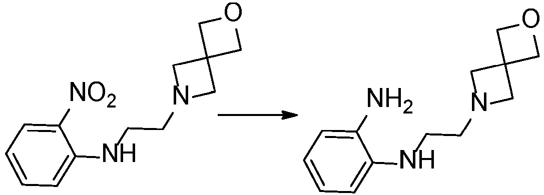

The N-(2-(2-oxa-6-azaspiro[3.3]heptane-6-yl)ethyl)-2-nitroaniline (0.388 g, 1.474 mmol) prepared in the step 1 was dissolved in ethanol (15 mL) and stirred at room temperature, after which 10%-Pd/C (40 mg) was slowly added thereinto at the same temperature and stirred at the same temperature for 3 hours in the presence of a hydrogen balloon attached thereto. The reaction mixture was filtered via a celite pad to remove a solid therefrom, after which solvent was removed from the resulting filtrate under reduced pressure, and then a title compound was used without an additional purification process (0.366 g, 106.5%, brown oil).

### [Step 3] Synthesis of 1-(2-morpholinoethyl)-1,3-dihydro-2H-benzo[d]imidazole-2-one

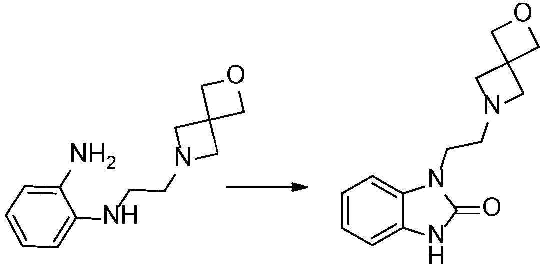

The N¹-(2-(2-oxa-6-azaspiro[3.3]heptane-6-yl)ethyl)benzene-1,2-diamine (0.366 g, 1.569 mmol) prepared in the step 2 and 1,1'-carbonyldiimidazole (0.254 g, 1.569 mmol) were dissolved in tetrahydrofuran (10 mL) at room temperature, after which the resulting solution was stirred at the same temperature for 18 hours. Solvent was removed from the reaction mixture under reduced pressure, after which water was poured into a resulting concentrate, and an extraction was performed with dichloromethane, then filtered via a plastic filter to remove a solid residue and an aqueous solution layer therefrom, and then concentrated under reduced pressure. The resulting concentrate was purified via column chromatography (SiO₂, 12 g cartridge; methanol/dichloromethane = 0 to 10%) and concentrated to obtain a title compound (0.330 g, 81.1%) in a brown oil form.

### [Step 4] Synthesis of the compound 24

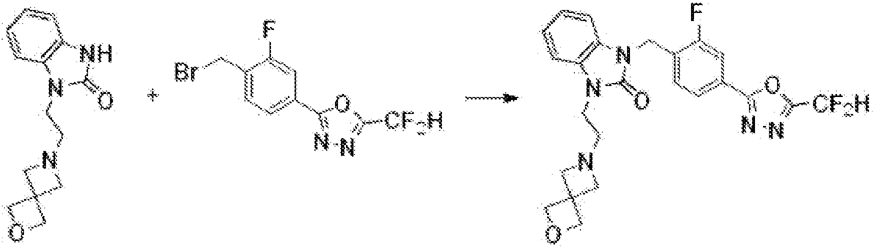

The 1-(2-morpholinoethyl)-1,3-dihydro-2H-benzo[d]imidazole-2-one (0.100 g, 0.386 mmol) prepared in the step 3 and sodium hydride (60.00%, 0.017 g, 0.424 mmol) were dissolved in N,N-dimethylformamide (4 mL) at 0°C, after which 2-(4-(bromomethyl)-3-fluorophenyl)-5-(difluoromethyl)-1,3,4-oxadiazole (0.130 g, 0.424 mmol) was added into the resulting solution and stirred at room temperature for 1 hour. Solvent was removed from the reaction mixture under reduced pressure, after which saturated sodium hydrogen carbonate aqueous solution was poured into a resulting concentrate, and an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 10%) and concentrated to obtain a title compound (0.027 g, 14.4%) in a white solid form.

**¹H NMR** (400 MHz, CDCl₃) δ 7.88 MHz- 7.82 (m, 2H), 7.46 (t, *J* = 7.6 Hz, 1H), 7.15 -6.79 (m, 5H), 5.21 (s, 2H), 4.72 (s, 4H), 3.91 (t, *J =* 6.8 Hz, 2H), 3.42 (s, 4H), 2.77 (t, *J =* 6.8 Hz, 2H); **LRMS** (ES) m/z 486.4 (M⁺ + H).

### Example 25: Synthesis of Compound 25, 1-(2-(2-oxa-6-azaspiro[3.3]heptane-6-yl)ethyl)-3-((5-(5-(difluoromethyl)-1,3,4-oxadiazole-2-yl)pyr idine-2-yl)methyl)-1,3-dihydro-2H-benzo[d]imidazole-2-one

### [Step 1] Synthesis of the compound 25

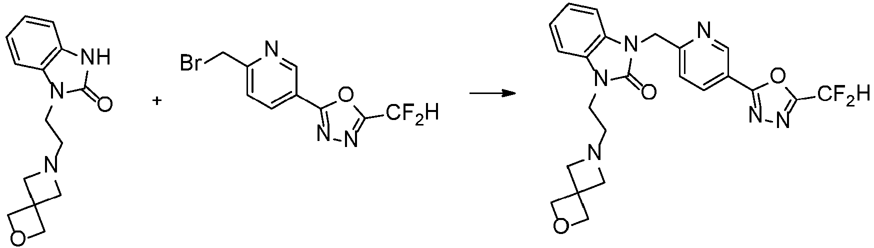

The 1-(2-(2-oxa-6-azaspiro[3.3]heptane-6-yl)ethyl)-1,3-dihydro-2H-benzo[d]imidazole-2-one (0.115 g, 0.443 mmol) prepared in the step 3 of the compound 24 and sodium hydride (60.00%, 0.020 g, 0.488 mmol) were dissolved in N,N-dimethylformamide (4 mL) at 0°C, after which 2-(6-(bromomethyl)pyridine-3-yl)-5-(difluoromethyl)-1,3,4-oxadiazole (0.142 g, 0.488 mmol) was added into the resulting solution, and then stirred at room temperature for 1 hour. Solvent was removed from the reaction mixture under reduced pressure, after which saturated sodium hydrogen carbonate aqueous solution was poured into a resulting concentrate, and an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 10%) and concentrated to obtain a title compound (0.083 g, 40.0%) in a brown oil form.

¹H NMR (400 MHz, CDCl₃) δ 9.27 (dd, *J =* 2.2, 0.8 Hz, 1H), 8.30 (dd, *J* = 8.2, 2.2 Hz, 1H), 7.38 (dd, *J* = 8.3, 0.7 Hz, 1H), 7.10 - 7.09 (m, 1H), 7.08 - 6.79 (m, 4H), 5.28 (s, 2H), 4.70 (s, 4H), 3.89 (t, *J* = 6.8 Hz, 2H), 3.40 (s, 4H), 2.76 (t, *J* = 6.8 Hz, 2H); LRMS (ES) m/z 469.4 (M⁺ + H).

### Example 26: Synthesis of Compound 26, 1-(2-(2-oxa-6-azaspiro[3.3]heptane-6-yl)ethyl)-3-(4-(5-(difluoromethyl)-1,3,4-oxadiazole-2-yl)ben zyl)-1,3-dihydro-2H-benzo[d]imidazole-2-one

### [Step 1] Synthesis of the compound 26

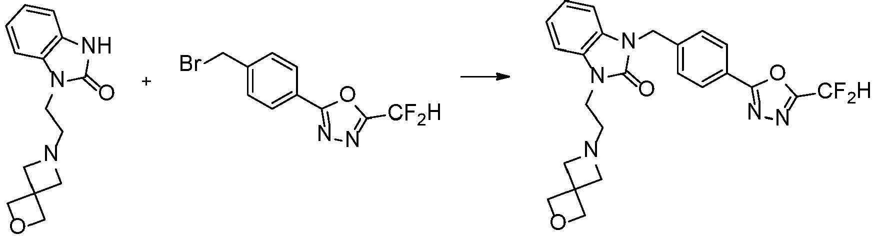

The 1-(2-(2-oxa-6-azaspiro[3.3]heptane-6-yl)ethyl)-1,3-dihydro-2H-benzo[d]imidazole-2-one (0.115 g, 0.443 mmol) prepared in the step 3 of the compound 24 and sodium hydride (60.00%, 0.020 g, 0.488 mmol) were dissolved in N,N-dimethylformamide (4 mL) at 0°C, after which 2-(6-(bromomethyl)pyridine-3-yl)-5-(difluoromethyl)-1,3,4-oxadiazole (0.142 g, 0.488 mmol) was added into the resulting solution, and then stirred at room temperature for 1 hour. Solvent was removed from the reaction mixture under reduced pressure, after which saturated sodium hydrogen carbonate aqueous solution was poured into a resulting concentrate, and an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 10%) and concentrated to obtain a title compound (0.083 g, 40.0%) in a brown oil form.

**¹H NMR** (400 MHz, CDCl₃) δ 9.27 (dd, *J =* 2.2, 0.8 Hz, 1H), 8.30 (dd, *J* = 8.2, 2.2 Hz, 1H), 7.38 (dd, *J* = 8.3, 0.7 Hz, 1H), 7.10 - 7.09 (m, 1H), 7.08 - 6.79 (m, 4H), 5.28 (s, 2H), 4.70 (s, 4H), 3.89 (t, *J* = 6.8 Hz, 2H), 3.40 (s, 4H), 2.76 (t, *J* = 6.8 Hz, 2H); LRMS (ES) m/z 469.4 (M⁺ + H).

### Example 27: Synthesis of Compound 27, 1-(4-(5-(difluoromethyl)-1,3,4-oxadiazole-2-yl)-2-fluorobenzyl)-3-(2-oxaspiro[3.3]heptane-6-yl)-1, 3-dihydro-2H-benzo[d]imidazole-2-one

### [Step 1] Synthesis of N¹-(2-oxaspiro[3.3]heptane-6-yl)benzene-1,2-diamine

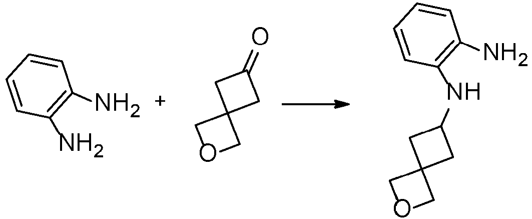

2-oxaspiro[3.3]heptane-6-one (1.000 g, 9.247 mmol) and 1,2-phenylenediamine (2.074 g, 18.495 mmol) were dissolved in dichloromethane (50 mL) at room temperature, after which sodium triacetoxyborohydride (2.352 g, 11.097 mmol) was added into the resulting solution and stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 24 g cartridge; ethyl acetate/hexane = 10 to 50%) and concentrated to obtain a title compound (1.120 g, 59.3%) in an orange solid form.

### [Step 2] Synthesis of 1-(2-oxaspiro[3.3]heptane-6-yl)-1,3-dihydro-2H-benzo[d]imidazole-2-one

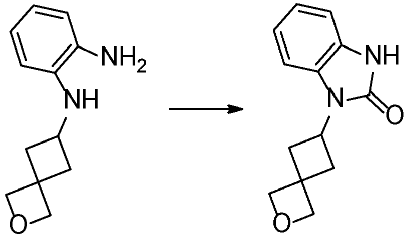

The N¹-(2-oxaspiro[3.3]heptane-6-yl)benzene-1,2-diamine (1.120 g, 5.483 mmol) prepared in the step 1 and 1,1'-carbonyldiimidazole (0.889 g, 5.483 mmol) were dissolved in tetrahydrofuran (20 mL) at room temperature, after which the resulting solution was stirred at the same temperature for 3 hours. Solvent was removed from the reaction mixture under reduced pressure, after which water was poured into the resulting concentrate, and then an extraction was performed with dichloromethane. An organic layer was washed with saturated sodium chloride aqueous solution, then dehydrated with anhydrous magnesium sulfate, then filtered, and then concentrated under reduced pressure. Ethyl acetate was put into the resulting concentrate and stirred, after which a precipitated solid was filtered, then washed with ethyl acetate, and then dried to obtain a title compound (0.556 g, 44.0%) in a pink solid form.

### [Step 3] Synthesis of the compound 27

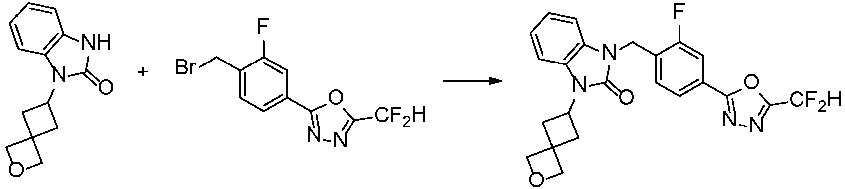

The 1-(2-oxaspiro[3.3]heptane-6-yl)-1,3-dihydro-2H-benzo[d]imidazole-2-one (0.100 g, 0.434 mmol) prepared in the step 2 and sodium hydride (60.00%, 0.019 g, 0.478 mmol) were dissolved in N,N-dimethylformamide (3 mL) at 0°C, after which 2-(4-(bromomethyl)-3-fluorophenyl)-5-(difluoromethyl)-1,3,4-oxadiazole (0.147 g, 0.478 mmol) was added into the resulting solution, and then stirred at room temperature for 18 hours. Water was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; ethyl acetate/hexane = 10 to 50%) and concentrated to obtain a title compound (0.136 g, 68.6%) in a white solid form.

¹H NMR (400 MHz, CDCl₃) δ 7.88 - 7.84 (m, 2H), 7.47 (t, *J* = 7.8 Hz, 1H), 7.14 - 6.79 (m, 5H), 5.19 (s, 2H), 4.87 (s, 2H), 4.82 (s, 2H), 4.74 - 4.68 (m, 1H), 3.19 - 3.14 (m, 2H), 2.85 - 2.79 (m, 2H); **LRMS** (ES) m/z 457.4 (M⁺ + H).

### Example 28: Synthesis of Compound 28, 1-((5-(5-(difluoromethyl)-1,3,4-oxadiazole-2-yl)pyridine-2-yl)methyl)-3-(2-oxaspiro[3.3]heptane-6 -yl)-1,3-dihydro-2H-benzo[d]imidazole-2-one

### [Step 1] Synthesis of the compound 28

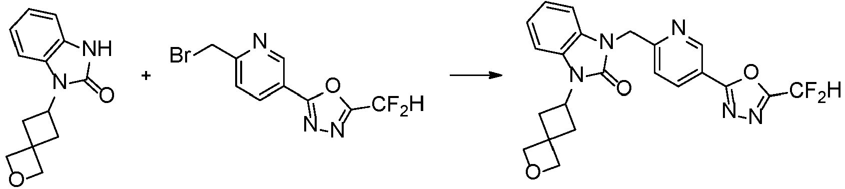

The 1-(2-oxaspiro[3.3]heptane-6-yl)-1,3-dihydro-2H-benzo[d]imidazole-2-one (0.100 g, 0.434 mmol) prepared in the step 2 of the compound 27 and sodium hydride (60.00%, 0.019 g, 0.478 mmol) were dissolved in N,N-dimethylformamide (3 mL) at 0°C, after which 2-(6-(bromomethyl)pyridine-3-yl)-5-(difluoromethyl)-1,3,4-oxadiazole (0.139 g, 0.478 mmol) was added into the resulting solution, and then stirred at room temperature for 18 hours. Water was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; ethyl acetate/hexane = 10 to 50%) and concentrated to obtain a title compound (0.118 g, 61.8%) in an light orange solid form.

**¹H NMR** (400 MHz, CDCl₃) δ 9.28 (dd, *J* = 2.2, 0.7 Hz, 1H), 8.32 (dd, *J* = 8.2, 2.2 Hz, 1H), 7.41 (d, *J* = 8.2 Hz, 1H), 7.10 - 6.81 (m, 5H), 5.27 (s, 2H), 4.86 (s, 2H), 4.80 (s, 2H), 4.74 - 4.70 (m, 1H), 3.19 - 3.16 (m, 2H), 2.84 - 2.78 (m, 2H); LRMS (ES) m/z 440.4 (M⁺ + H).

### Example 29: Synthesis of Compound 29, 1-(4-(5-(difluoromethyl)-1,3,4-oxadiazole-2-yl)benzyl)-3-(2-oxaspiro[3.3]heptane-6-yl)-1,3-dihydr o-2H-benzo[d]imidazole-2-one

### [Step 1] Synthesis of the compound 29

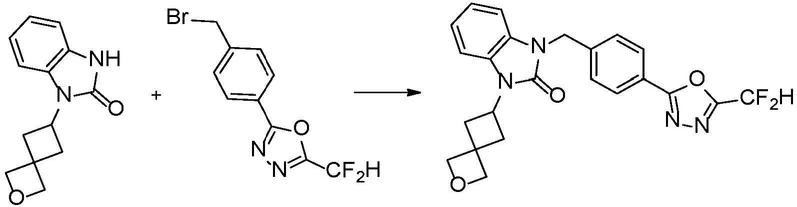

The 1-(2-oxaspiro[3.3]heptane-6-yl)-1,3-dihydro-2H-benzo[d]imidazole-2-one (0.100 g, 0.434 mmol) prepared in the step 2 of the compound 27 and sodium hydride (60.00%, 0.019 g, 0.478 mmol) were dissolved in N,N-dimethylformamide (3 mL) at 0°C, after which 2-(4-(bromomethyl)phenyl)-5-(difluoromethyl)-1,3,4-oxadiazole (0.138 g, 0.478 mmol) was added into the resulting solution and then stirred at room temperature for 18 hours. Water was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; ethyl acetate/hexane = 10 to 50%) and concentrated to obtain a title compound (0.137 g, 72.0%) in a white solid form.

**¹H NMR** (400 MHz, CDCl₃) δ 8.09 (d, *J* = 7.7 Hz, 2H), 7.49 (d, *J* = 7.8 Hz, 2H), 7.12 - 6.79 (m, 5H), 5.14 (s, 2H), 4.87 (s, 2H), 4.82 (s, 2H), 4.75 -4.71 (m, 1H), 3.17 (t, *J =* 10.3 Hz, 2H), 2.82 (t, *J* = 9.9 Hz, 2H); **LRMS** (ES) m/z 439.4 (M⁺ + H).

### Example 30: Synthesis of Compound 30, 1-(4-(5-(difluoromethyl)-1,3,4-oxadiazole-2-yl)benzyl)-3-(2-methoxyethyl)-3,4-dihydroquinazoline -2(1H)-one

### [Step 1] Synthesis of 2-amino-N-(2-methoxyethyl)benzamide

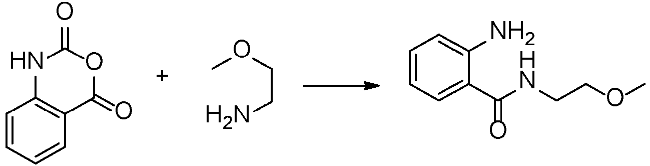

2H-benzo[d][1,3]oxazine-2,4(1H)-dione (10.000 g, 61.301 mmol), 2-methoxyethane-1-amine (4.604 g, 61.301 mmol) and triethylamine (8.544 mL, 61.301 mmol) were dissolved in ethanol (50 mL) at 80°C, after which the resulting solution was stirred at the same temperature for 12 hours, and then a reaction was finished by lowering a temperature to room temperature. Water was poured into the reaction mixture and an extraction was performed with ethyl acetate. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 40 g cartridge; ethyl acetate/hexane = 0 to 30%) and concentrated to obtain a title compound (9.800 g, 82.3%) in a colorless oil form.

### [Step 2] Synthesis of 2-(((2-methoxyethyl)amino)methyl)aniline

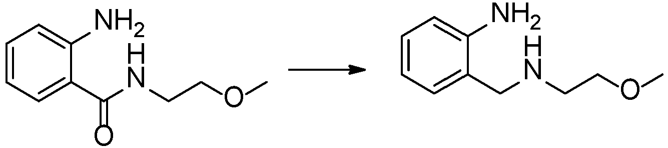

The 2-amino-N-(2-methoxyethyl)benzamide (3.670 g, 18.895 mmol) prepared in the step 1 was dissolved in dichloromethane (5 mL), after which lithium aluminum hydride (2.00 M solution in THF, 25.508 mL, 51.017 mmol) was added thereinto at 0°C, then stirred at the same temperature for 30 hours, then further stirred at 60°C for 12 hours, and then a reaction was finished by lowering a temperature to room temperature. Saturated ammonium chloride aqueous solution was poured into the reaction mixture and an extraction was performed with ethyl acetate. An organic layer was washed with saturated sodium chloride aqueous solution, then dehydrated with anhydrous sodium sulfate, then filtered, and then concentrated under reduced pressure. A title compound was used without an additional purification process (3.300 g, 96.9%, colorless oil).

### [Step 3] Synthesis of 3-(2-methoxyethyl)-3,4-dihydroquinazoline-2(1 H)-one

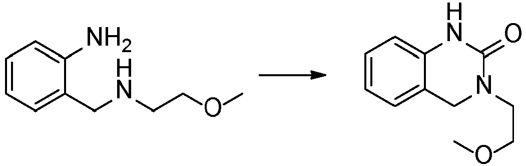

The 2-(((2-methoxyethyl)amino)methyl)aniline (2.700 g, 14.979 mmol) prepared in the step 2 and 1,1'-carbonyldiimidazole (CDI, 2.429 g, 14.979 mmol) were dissolved in tetrahydrofuran (30 mL) at room temperature, after which the resulting solution was stirred at the same temperature for 12 hours. Water was poured into the reaction mixture and an extraction was performed with dichloromethane. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; ethyl acetate/hexane = 0 to 50%) and concentrated to obtain a title compound (2.000 g, 64.7%) in a white solid form.

### [Step 4] Synthesis of the compound 30

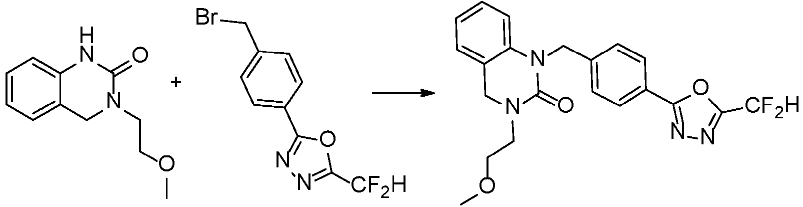

The 3-(2-methoxyethyl)-3,4-dihydroquinazoline-2(1H)-one (0.100 g, 0.485 mmol) prepared in the step 3 was dissolved in N,N-dimethylformamide (10 mL) at 0°C, after which sodium hydride (60.00%, 0.029 g, 0.727 mmol) was added into the resulting solution and stirred at the same temperature for 30 minutes. 2-(4-(bromomethyl)phenyl)-5-(difluoromethyl)-1,3,4-oxadiazole (0.140 g, 0.485 mmol) was added into the reaction mixture and further stirred at room temperature for 2 hours. Water was poured into the reaction mixture and an extraction was performed with ethyl acetate. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; ethyl acetate/hexane = 0 to 50%) and concentrated to obtain a title compound (0.060 g, 29.9%) in a colorless oil form.

**¹H NMR** (400 MHz, CDCl₃) δ 8.07 - 8.05 (m, 2H), 7.46 - 7.44 (m, 2H), 7.10 - 7.08 (m, 2H), 7.04 (s, 0.25H), 6.98 - 6.96 (m, 1H), 6.91 (s, 0.5H), 6.78 (s, 0.25H), 6.62 - 6.59 (m, 1H), 5.21 (s, 2H), 4.65 (s, 2H), 3.71 - 3.70 (m, 4H), 3.41 (s, 3H); LRMS (ES) m/z 415.3 (M⁺ + 1).

### Example 31: Synthesis of Compound 31, 1-((5-(5-(difluoromethyl)-1,3,4-oxadiazole-2-yl)pyridine-2-yl)methyl)-3-(2-methoxyethyl)-3,4-dihy droquinazoline-2(1H)-one

### [Step 1] Synthesis of 1-((5-(5-(difluoromethyl)-1,3,4-oxadiazole-2-yl)pyridine-2-yl)methyl)-3-(2-methoxyethyl)-3,4-dihy droquinazoline-2(1H)-one

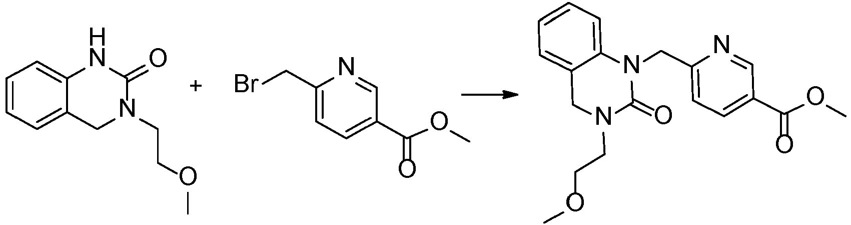

3-(2-methoxyethyl)-3,4-dihydroquinazoline-2(1H)-one (0.100 g, 0.485 mmol) was dissolved in N,N-dimethylformamide (10 mL) at 0°C, after which sodium hydride (60.00%, 0.029 g, 0.727 mmol) was added into the resulting solution and stirred at the same temperature for 30 minutes. 2-(6-(bromomethyl)pyridine-3-yl)-5-(difluoromethyl)-1,3,4-oxadiazole (0.141 g, 0.485 mmol) was added into the reaction mixture and further stirred at room temperature for 2 hours. Water was poured into the reaction mixture and an extraction was performed with ethyl acetate. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; ethyl acetate/hexane = 0 to 50%) and concentrated to obtain a title compound (0.080 g, 39.7%) in a colorless oil form.

### [Step 2] Synthesis of 6-((3-(2-methoxyethyl)-2-oxo-3,4-dihydroquinazoline-1(2H)-yl)methyl)nicotinohydrazide

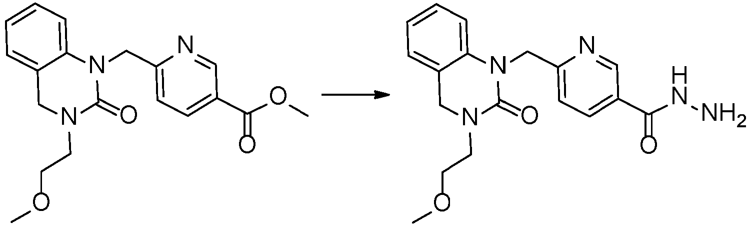

The methyl 6-((3-(2-methoxyethyl)-2-oxo-3,4-dihydroquinazoline-1 (2H)-yl)methyl)nicotinate (0.300 g, 0.844 mmol) prepared in the step 1 and hydrazine monohydrate (0.821 mL, 16.883 mmol) were dissolved in ethanol (20 mL) at 80°C, after which the resulting solution was stirred at the same temperature for 12 hours, and then a reaction was finished by lowering a temperature to room temperature. Solvent was removed from the reaction mixture under reduced pressure, after which a title compound was used without an additional purification process (0.300 g, 100.0%, white solid).

### [Step 3] Synthesis of the compound 31

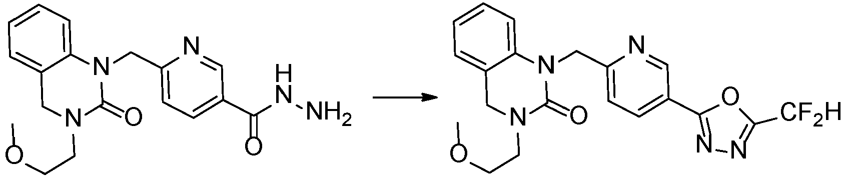

The 6-((3-(2-methoxyethyl)-2-oxo-3,4-dihydroquinazoline-1(2H)-yl)methyl)nicotinohydrazide (0.240 g, 0.675 mmol) prepared in the step 2, 2,2-difluoroacetic anhydride (0.252 mL, 2.026 mmol) and imidazole (0.138 g, 2.026 mmol) were dissolved in dichloromethane (10 mL) at 45°C, after which the resulting solution was stirred at the same temperature for 12 hours, and then a reaction was finished by lowering a temperature to room temperature. Water was poured into the reaction mixture and an extraction was performed with dichloromethane. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; ethyl acetate/hexane = 0 to 50%) and concentrated to obtain a title compound (0.150 g, 53.5%) in a colorless oil form.

**¹H NMR** (400 MHz, CDCl₃) δ 9.29 (dd, *J =* 2.2, 0.8 Hz, 1H), 8.29 (dd, *J* = 8.3, 2.2 Hz, 1H), 7.44 - 7.42 (m, 1H), 7.10 - 7.07 (m, 2H), 7.07 (s, 0.25H), 6.99 - 6.96 (m, 1H), 6.94 (s, 0.5H), 6.81 (s, 0.25H), 6.69 - 6.67 (m, 1H), 5.31 (s, 2H), 4.65 (s, 2H), 3.72 - 3.69 (m, 4H), 3.40 (s, 3H); **LRMS** (ES) m/z 416.4 (M⁺ + 1).

### Example 32: Synthesis of the compound 32, 1-(4-(5-(difluoromethyl)-1,3,4-oxadiazole-2-yl)-2-fluorobenzyl)-3-(1-methylazetidine-3-yl)-3,4-dih ydroquinazoline-2(1H)-one

### [Step 1] Synthesis of tert-butyl 3-(1-(4-(5-(difluoromethyl)-1,3,4-oxadiazole-2-yl)-2-fluorobenzyl)-2-oxo-1,4-dihydroquinazoline-3 (2H)-yl)azetidine-1-carboxylate

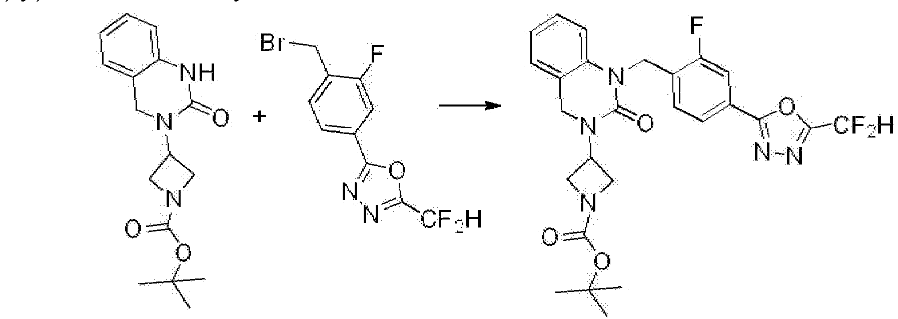

Tert-butyl 3-(2-oxo-1,4-dihydroquinazoline-3(2H)-yl)azetidine-1-carboxylate (0.469 g, 1.546 mmol) was dissolved in N,N-dimethylformamide (10 mL) at 0°C, after which sodium hydride (60.00%, 0.093 g, 2.319 mmol) was added into the resulting solution and stirred at the same temperature for 30 minutes. 2-(4-(bromomethyl)-3-fluorophenyl)-5-(difluoromethyl)-1,3,4-oxadiazole (0.475 g, 1.546 mmol) was added into the reaction mixture and further stirred at room temperature for 2 hours. Water was poured into the reaction mixture and an extraction was performed with ethyl acetate. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; ethyl acetate/hexane = 0 to 50%) and concentrated to obtain a title compound (0.410 g, 50.1%) in a colorless oil form.

### [Step 2] Synthesis of 3-(azetidine-3-yl)-1-(4-(5-(difluoromethyl)-1,3,4-oxadiazole-2-yl)-2-fluorobenzyl)-3,4-dihydroquin azoline-2(1H)-one 2,2,2-trifluoroacetate

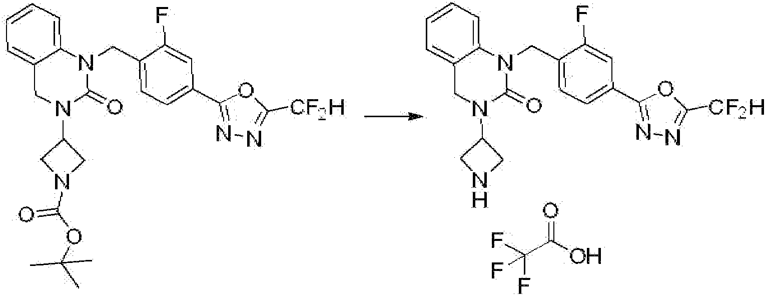

The tert-butyl 3-(1-(4-(5-(difluoromethyl)-1,3,4-oxadiazole-2-yl)-2-fluorobenzyl)-2-oxo-1,4-dihydroquinazoline-3 (2H)-yl)azetidine-1-carboxylate (0.420 g, 0.793 mmol) prepared in the step 1 and trifluoroacetic acid (0.607 mL, 7.932 mmol) were dissolved in dichloromethane (10 mL) at room temperature, after which the resulting solution was stirred at the same temperature for three hours. Solvent was removed from the reaction mixture under reduced pressure, after which a title compound was used without an additional purification process (0.420 g, 97.4%, brown oil).

### [Step 3] Synthesis of the compound 32

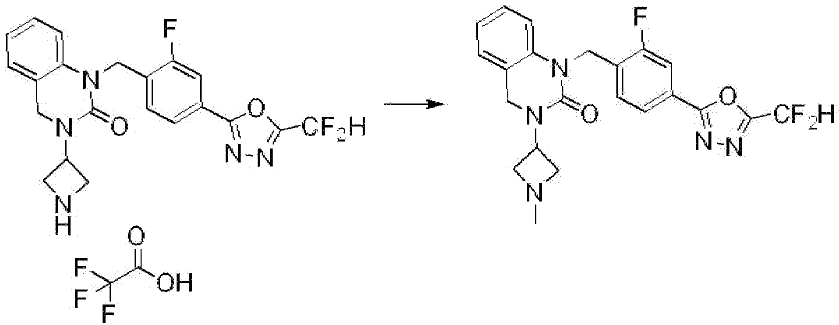

The 3-(azetidine-3-yl)-1-(4-(5-(difluoromethyl)-1,3,4-oxadiazole-2-yl)-2-fluorobenzyl)-3,4-dihydroquin azoline-2(1H)-one 2,2,2-trifluoroacetate (0.200 g, 0.368 mmol) prepared in the step 2, formaldehyde (0.022 g, 0.736 mmol), N,N-diisopropylethylamine (0.064 mL, 0.368 mmol) and sodium triacetoxyborohydride (0.156 g, 0.736 mmol) were dissolved in dichloromethane (5 mL) at room temperature, after which the resulting solution was stirred at the same temperature for 12 hours. Water was poured into the reaction mixture and an extraction was performed with dichloromethane. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; ethyl acetate/hexane = 0 to 30%) and concentrated to obtain a title compound (0.110 g, 67.4%) in a colorless oil form.

**¹H NMR** (400 MHz, CDCl₃) δ 7.87 - 7.80 (m, 2H), 7.30 - 7.28 (m, 1H), 7.15 - 7.12 (m, 2H), 7.05 (s, 0.25H), 7.05 - 7.00 (m, 1H), 6.92 (s, 0.5H), 6.79 (s, 0.25H), 6.62 (d, *J =* 8.2 Hz, 1H), 5.24 (d, *J* = 3.8 Hz, 2H), 4.70 - 4.60 (m, 1H), 4.52 (s, 2H), 4.00 - 3.93 (m, 2H), 2.87 - 2.85 (m, 1H), 2.78 - 2.76 (m, 1H); **LRMS** (ES) m/z 476.4 (M⁺ + 1).

### Example 33: Synthesis of Compound 33, 1-(4-(5-(difluoromethyl)-1,3,4-oxadiazole-2-yl)-2-fluorobenzyl)-3-(1-methylpiperidine-4-yl)-3,4-dih ydroquinazoline-2(1H)-one

### [Step 1] Synthesis of tert-butyl 4-(1-(4-(5-(difluoromethyl)-1,3,4-oxadiazole-2-yl)-2-fluorobenzyl)-2-oxo-1,4-dihydroquinazoline-3 (2H)-yl)piperidine-1-carboxylate

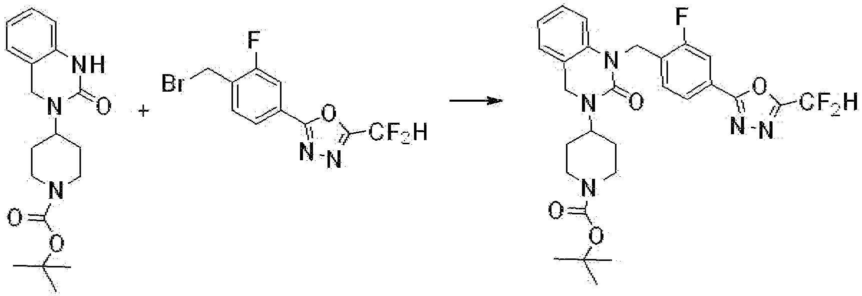

Tert-butyl 4-(2-oxo-1,4-dihydroquinazoline-3(2H)-yl)piperidine-1-carboxylate (0.685 g, 2.067 mmol) was dissolved in N,N-dimethylformamide (10 mL) at 0°C, after which sodium hydride (60.00%, 0.124 g, 3.100 mmol) was added into the resulting solution and stirred at the same temperature for 30 minutes. 2-(4-(bromomethyl)-3-fluorophenyl)-5-(difluoromethyl)-1,3,4-oxadiazole (0.635 g, 2.067 mmol) was added into the reaction mixture and further stirred at room temperature for 2 hours. Water was poured into the reaction mixture and an extraction was performed with ethyl acetate. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; ethyl acetate/hexane = 0 to 50%) and concentrated to obtain a title compound (0.530 g, 46.0%) in a colorless oil form.

### [Step 2] Synthesis of 1-(4-(5-(difluoromethyl)-1,3,4-oxadiazole-2-yl)-2-fluorobenzyl)-3-(piperidine-4-yl)-3,4-dihydroquin azoline-2(1H)-one 2,2,2-trifluoroacetate

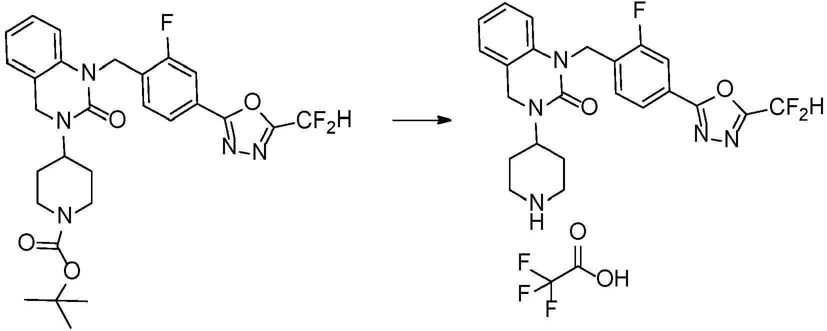

The tert-butyl 4-(1-(4-(5-(difluoromethyl)-1,3,4-oxadiazole-2-yl)-2-fluorobenzyl)-2-oxo-1,4-dihydroquinazoline-3 (2H)-yl)piperidine-1-carboxylate (0.530 g, 0.951 mmol) prepared in the step 1 and trifluoroacetic acid (0.728 mL, 9.506 mmol) were dissolved in dichloromethane (10 mL) at room temperature, after which the resulting solution was stirred at the same temperature for 3 hours. Solvent was removed from the reaction mixture under reduced pressure, after which a title compound was used without an additional purification process (0.530 g, 97.6%, brown oil).

### [Step 3] Synthesis of the compound 33

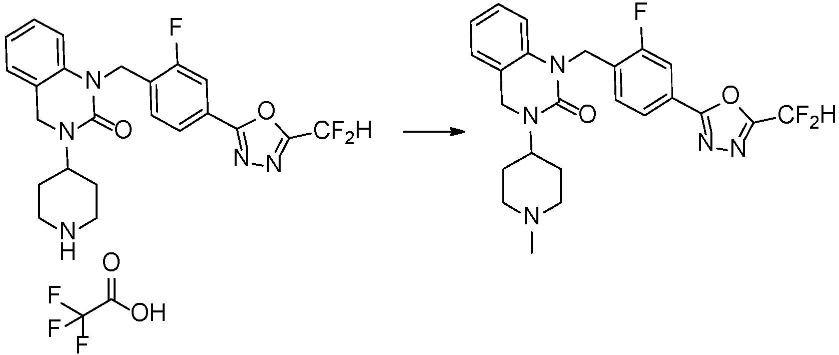

The 1-(4-(5-(difluoromethyl)-1,3,4-oxadiazole-2-yl)-2-fluorobenzyl)-3-(piperidine-4-yl)-3,4-dihydroquin azoline-2(1H)-one 2,2,2-trifluoroacetate (0.200 g, 0.350 mmol) prepared in the step 2, formaldehyde (0.021 g, 0.700 mmol), N,N-diisopropylethylamine (0.061 mL, 0.350 mmol) and sodium triacetoxyborohydride (0.148 g, 0.700 mmol) were dissolved in dichloromethane (5 mL) at room temperature, after which the resulting solution was stirred at the same temperature for 12 hours. Water was poured into the reaction mixture and an extraction was performed with dichloromethane. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; ethyl acetate/hexane = 0 to 30%) and concentrated to obtain a title compound (0.100 g, 60.6%) in a white foam solid form.

**¹H NMR** (400 MHz, CDCl₃) δ 7.86 - 7.78 (m, 2H), 7.28 - 7.24 (m, 1H), 7.18 - 7.14 (m, 2H), 7.05 (s, 0.25H), 7.05 - 7.00 (m, 1H), 6.92 (s, 0.5H), 6.79 (s, 0.25H), 6.63 (d, *J =* 7.9 Hz, 1H), 5.22 (s, 2H), 4.68 - 4.61 (m, 1H), 4.39 (s, 2H), 3.56 - 3.53 (m, 2H), 2.81 - 2.74 (m, 5H), 2.43 - 2.34 (m, 2H), 1.95 - 1.91 (m, 2H); **LRMS** (ES) m/z 472.4 (M⁺ + 1).

### Example 34: Synthesis of Compound 34, 1-(4-(5-(difluoromethyl)-1,3,4-oxadiazole-2-yl)-2-fluorobenzyl)-3-(tetrahydro-2H-pyran-4-yl)-1,3-d ihydro-2H-benzo[d]imidazole-2-one

### [Step 1] Synthesis of the compound 34

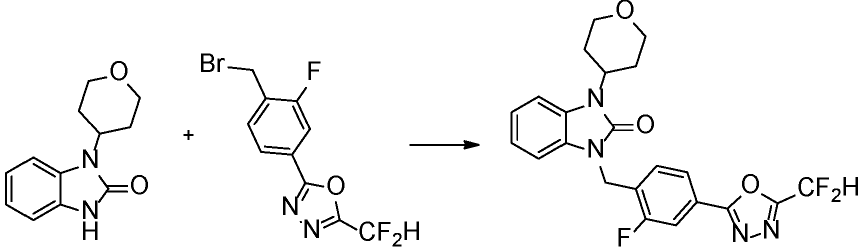

1-(tetrahydro-2H-pyran-4-yl)-1,3-dihydro-2H-benzo[d]imidazole-2-one (0.100 g, 0.458 mmol) was dissolved in N,N-dimethylformamide (5 mL) at 0°C, after which sodium hydride (60.00%, 0.027 g, 0.687 mmol) was added into the resulting solution and stirred at the same temperature for 30 minutes. 2-(4-(bromomethyl)-3-fluorophenyl)-5-(difluoromethyl)-1,3,4-oxadiazole (0.141 g, 0.458 mmol) was added into the reaction mixture and further stirred at room temperature for 2 hours. Water was poured into the reaction mixture and an extraction was performed with ethyl acetate. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; ethyl acetate/hexane = 0 to 50%) and concentrated to obtain a title compound (0.070 g, 34.4%) in a colorless oil form.

**¹H NMR** (400 MHz, CDCl₃) δ 7.89 - 7.84 (m, 2H), 7.48 (t, *J* = 7.8 Hz, 1H), 7.30 - 7.25 (m, 1H), 7.14 - 7.05 (m, 2H), 7.05 (s, 0.25H), 7.02 - 6.98 (m, 1H), 6.92 (s, 0.5H), 6.79 (s, 0.25H), 5.22 (s, 2H), 4.69 - 4.63 (m, 1H), 4.19 - 4.13 (m, 2H), 3.63 - 3.57 (m, 2H), 2.60 - 2.50 (m, 2H), 1.85 - 1.81 (m, 2H); LRMS (ES) m/z 445.3 (M⁺ + 1).

### Example 35: Synthesis of Compound 35, 1-(4-(5-(difluoromethyl)-1,3,4-oxadiazole-2-yl)-2-fluorobenzyl)-3-(1,1-dioxydotetrahydro-2H-thiop yran-4-yl)-1,3-dihydro-2H-benzo[d]imidazole-2-one

### [Step 1] Synthesis of the compound 35

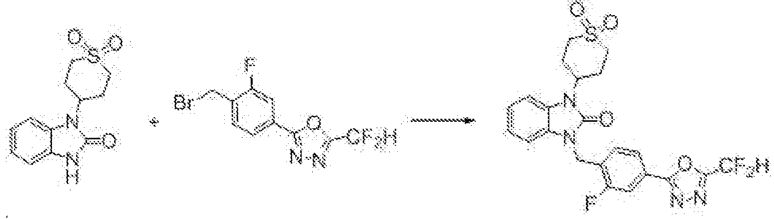

1-(1,1-dioxydotetrahydro-2H-thiopyran-4-yl)-1,3-dihydro-2H-benzo[d]imidazole-2-one (0.150 g, 0.563 mmol) was dissolved in N,N-dimethylformamide (5 mL) at 0°C, after which sodium hydride (60.00%, 0.034 g, 0.845 mmol) was added into the resulting solution and stirred at the same temperature for 30 minutes. 2-(4-(bromomethyl)-3-fluorophenyl)-5-(difluoromethyl)-1,3,4-oxadiazole (0.173 g, 0.563 mmol) was added into the reaction mixture and further stirred at room temperature for 2 hours. Water was poured into the reaction mixture and an extraction was performed with ethyl acetate. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; ethyl acetate/hexane = 0 to 50%) and concentrated to obtain a title compound (0.080 g, 28.8%) in a white solid form.

**¹H NMR** (400 MHz, CDCl₃) δ 7.89 - 7.85 (m, 2H), 7.50 - 7.46 (m, 1H), 7.31 - 7.28 (m, 1H), 7.17 - 7.10 (m, 2H), 7.05 (s, 0.25H), 7.03 - 7.01 (m, 1H), 6.92 (s, 0.5H), 6.79 (s, 0.25H), 5.21 (s, 2H), 4.80 - 4.73 (m, 1H), 3.30 - 2.97 (m, 6H), 2.29 - 2.24 (m, 2H); **LRMS** (ES) m/z 493.3 (M⁺ + 1).

### Example 36: Synthesis of Compound 36, 1-(4-(5-(difluoromethyl)-1,3,4-oxadiazole-2-yl)-2-fluorobenzyl)-3-(2-(dimethylamino)ethyl)-1,3-dih ydro-2H-benzo[d]imidazole-2-one

### [Step 1] Synthesis of the compound 36

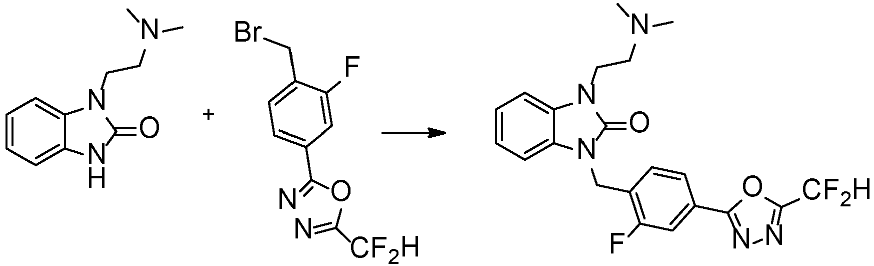

1-(2-(dimethylamino)ethyl)-1,3-dihydro-2H-benzo[d]imidazole-2-one (0.150 g, 0.731 mmol) was dissolved in N,N-dimethylformamide (5 mL) at 0°C, after which sodium hydride (60.00%, 0.044 g, 1.096 mmol) was added into the resulting solution and stirred at the same temperature for 30 minutes. 2-(4-(bromomethyl)-3-fluorophenyl)-5-(difluoromethyl)-1,3,4-oxadiazole (0.224 g, 0.731 mmol) was added into the reaction mixture and further stirred at room temperature for 2 hours. Water was poured into the reaction mixture and an extraction was performed with ethyl acetate. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; ethyl acetate/hexane = 0 to 50%) and concentrated to obtain a title compound (0.110 g, 34.9%) in a colorless oil form.

**¹H NMR** (400 MHz, CDCl₃) δ 7.87 ~ 7.82 (m, 2H), 7.50 ~ 7.45 (m, 1H), 7.13 ~ 7.06 (m, 3H), 7.05 (s, 0.25H), 6.96 ~ 6.94 (m, 1H), 6.92 (s, 0.5H), 6.79 (s, 0.25H), 5.21 (s, 2H), 4.08 (t, *J =* 7.1 Hz, 2H), 2.71 (t, *J =* 7.1 Hz, 2H), 2.36 (s, 6H).

### Example 37: Synthesis of Compound 37, 1-(4-(5-(difluoromethyl)-1,3,4-oxadiazole-2-yl)-2-fluorobenzyl)-3-(oxetan-3-yl)-1,3-dihydro-2H-be nzo[d]imidazole-2-one

### [Step 1] Synthesis of the compound 37

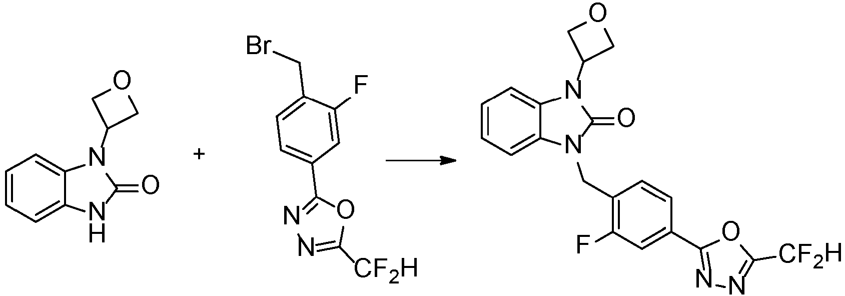

1-(oxetan-3-yl)-1,3-dihydro-2H-benzo[d]imidazole-2-one (0.200 g, 1.052 mmol) was dissolved in N,N-dimethylformamide (10 mL) at 0°C, after which sodium hydride (60.00%, 0.063 g, 1.577 mmol) was added into the resulting solution and stirred at the same temperature for 30 minutes. 2-(4-(bromomethyl)-3-fluorophenyl)-5-(difluoromethyl)-1,3,4-oxadiazole (0.646 g, 2.103 mmol) was added into the reaction mixture and further stirred at room temperature for 2 hours. Water was poured into the reaction mixture and an extraction was performed with ethyl acetate. An organic layer was washed with saturated aqueous solution, then dehydrated with anhydrous sodium sulfate, then filtered, and then concentrated under reduced pressure. The resulting concentrate was purified via column chromatography (SiO₂, 12 g cartridge; ethyl acetate/hexane = 0 to 50%) and concentrated to obtain a title compound (0.160 g, 36.5%) in a colorless oil form.

**¹H NMR** (400 MHz, CDCl₃) δ 7.85 - 7.81 (m, 2H), 7.72 - 7.70 (m, 1H), 7.48 - 7.45 (m, 1H), 7.21 - 7.11 (m, 2H), 7.04 (s, 0.25H), 7.04 - 7.01 (m, 1H), 6.92 (s, 0.5H), 6.79 (s, 0.25H), 5.72 - 5.66 (m, 1H), 5.21 - 5.11 (m, 6H).

### Example 38: Synthesis of Compound 38, 1-(4-(5-(difluoromethyl)-1,3,4-oxadiazole-2-yl)-2-fluorobenzyl)-3-(1-methylpiperidine-4-yl)methyl) -1,3-dihydro-2H-benzo[d]imidazole-2-one

### [Step 1] Synthesis of tert-butyl 4-((3-(4-(5-(difluoromethyl)-1,3,4-oxadiazole-2-yl)-2-fluorobenzyl)-2-oxo-2,3-dihydro-1H-benzo[d ]imidazole-1-yl)methyl)piperidine-1-carboxylate

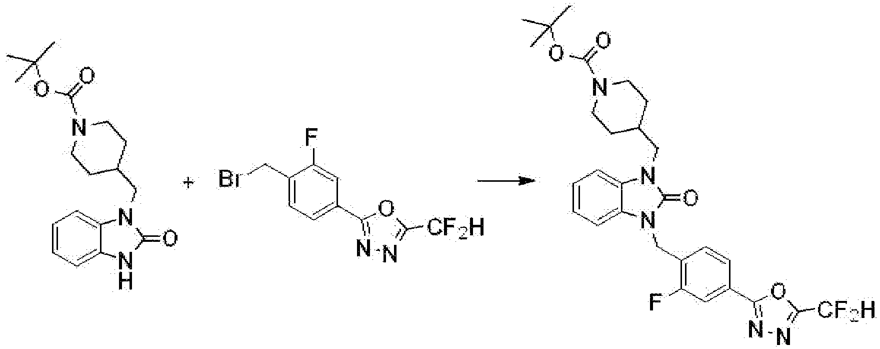

Tert-butyl 4-((2-oxo-2,3-dihydro-1H-benzo[d]imidazole-1-yl)methyl)piperidine-1-carboxylate (0.437 g, 1.319 mmol) was dissolved in N,N-dimethylformamide (10 mL) at 0°C, after which sodium hydride (60.00%, 0.079 g, 1.978 mmol) was added into the resulting solution and stirred at the same temperature for 30 minutes. 2-(4-(bromomethyl)-3-fluorophenyl)-5-(difluoromethyl)-1,3,4-oxadiazole (0.810 g, 2.637 mmol) was added into the reaction mixture and further stirred at room temperature for 2 hours. Water was poured into the reaction mixture and an extraction was performed with ethyl acetate. An organic layer was washed with saturated aqueous solution, then dehydrated with anhydrous sodium sulfate, then filtered, and then concentrated under reduced pressure. The resulting concentrate was purified via column chromatography (SiO₂, 12 g cartridge; ethyl acetate/hexane = 0 to 50%) and concentrated to obtain a title compound (0.550 g, 74.8%) in a colorless oil form.

### [Step 2] Synthesis of 1-(4-(5-(difluoromethyl)-1,3,4-oxadiazole-2-yl)-2-fluorobenzyl)-3-(piperidine-4-ylmethyl)-1,3-dihy dro-2H-benzo[d]imidazole-2-one 2,2,2-trifluoroacetate

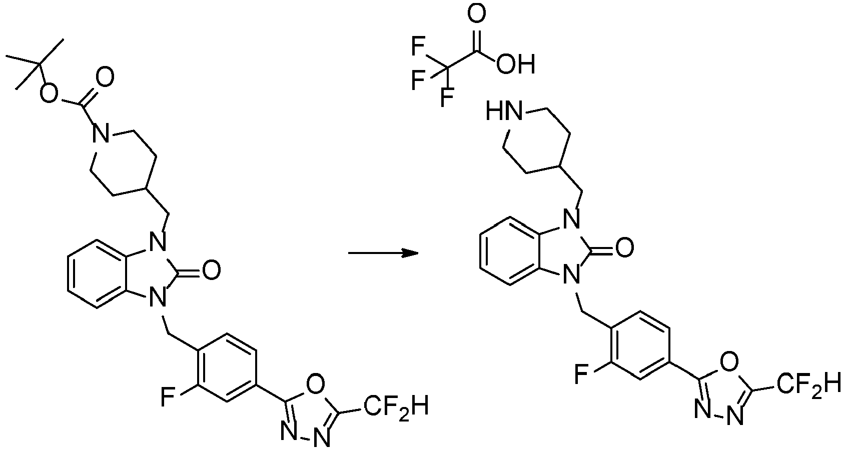

The tert-butyl 4-((3-(4-(5-(difluoromethyl)-1,3,4-oxadiazole-2-yl)-2-fluorobenzyl)-2-oxo-2,3-dihydro-1H-benzo[d ]imidazole-1-yl)methyl)piperidine-1-carboxylate (0.550 g, 0.986 mmol) prepared in the step 1 and trifluoroacetic acid (1.511 mL, 19.728 mmol) were dissolved in dichloromethane (10 mL) at room temperature, after which the resulting solution was stirred at the same temperature for 3 hours. Solvent was removed from the reaction mixture under reduced pressure, after which a title compound was used without an additional purification process (0.570 g, 101.1%, brown oil).

### [Step 3] Synthesis of the compound 38

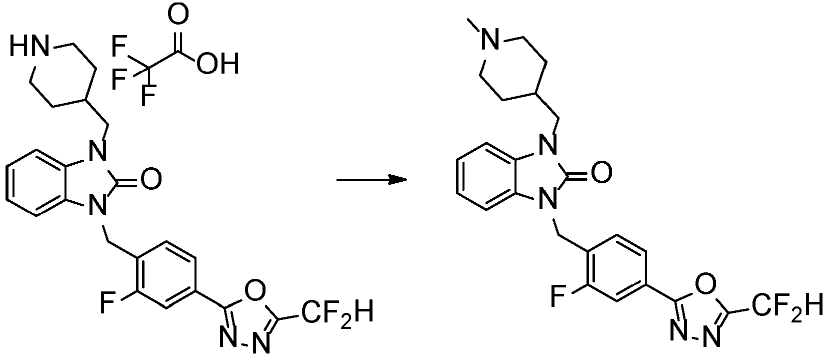

The 1-(4-(5-(difluoromethyl)-1,3,4-oxadiazole-2-yl)-2-fluorobenzyl)-3-(piperidine-4-ylmethyl)-1,3-dihy dro-2H-benzo[d]imidazole-2-one 2,2,2-trifluoroacetate (0.200 g, 0.350 mmol) prepared in the step 2 and N,N-diisopropylethylamine (0.061 mL, 0.350 mmol) were dissolved in dichloromethane (5 mL), after which the resulting solution was stirred at room temperature for 30 minutes, and then formaldehyde (0.021 g, 0.700 mmol) and sodium triacetoxyborohydride (1.558 g, 7.349 mmol) were added thereinto and further stirred at the same temperature for 12 hours. Water was poured into the reaction mixture and an extraction was performed with dichloromethane. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; methanol/dichloromethane = 0 to 10%) and concentrated to obtain a title compound (0.150 g, 90.9%) in a colorless oil form.

**LRMS** (ES) m/z 558.4 (M⁺ + 1).

### Example 39: Synthesis of Compound 39, 1-(4-(5-(difluoromethyl)-1,3,4-oxadiazole-2-yl)-2-fluorobenzyl)-3-((1-(oxetan-3-yl)piperidine-4-yl) methyl)-1,3-dihydro-2H-benzo[d]imidazole-2-one

### [Step 1] Synthesis of the compound 39

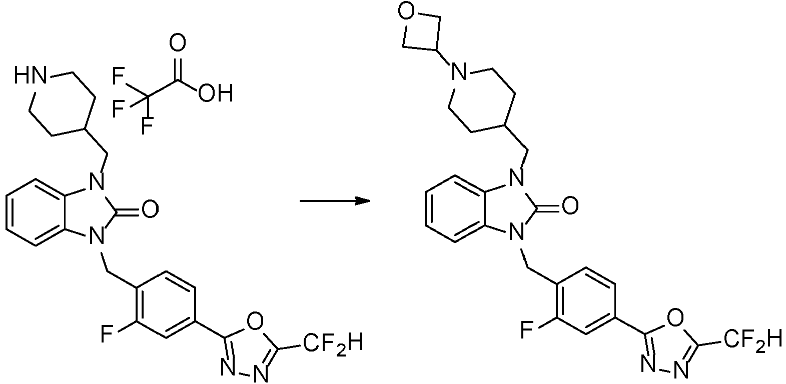

1-(4-(5-(difluoromethyl)-1,3,4-oxadiazole-2-yl)-2-fluorobenzyl)-3-(piperidine-4-ylmethyl)-1,3-dihydro-2H-benzo[d]imidazole-2-one 2,2,2-trifluoroacetate (0.200 g, 0.350 mmol) and N,N-diisopropylethylamine (0.061 mL, 0.350 mmol) were dissolved in dichloromethane (5 mL), after which the resulting solution was stirred at room temperature for 30 minutes, and then oxetan-3-one (0.050 g, 0.700 mmol) and sodium triacetoxyborohydride (1.558 g, 7.349 mmol) were added thereinto and further stirred at the same temperature for 12 hours. Water was poured into the reaction mixture and an extraction was performed with dichloromethane. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; methanol/dichloromethane = 0 to 10%) and concentrated to obtain a title compound (0.100 g, 55.6%) in a colorless oil form.

**¹H NMR** (400 MHz, CDCl₃) δ 7.86 ~ 7.81 (m, 2H), 7.45 (dd, *J =* 8.2, 7.5 Hz, 1H), 7.13 ~ 7.01 (m, 3H), 7.02 (s, 1H), 6.96 ~ 6.94 (m, 1H), 6.91 (s, 1H), 6.78 (s, 1H), 5.00 (s, 2H), 4.65 ~ 4.58 (m, 4H), 3.83 (d, *J =* 7.1 Hz, 2H), 3.47 ~ 3.43 (m, 1H), 2.77 ~ 2.74 (m, 2H), 2.00 ~ 1.90 (m, 1H), 1.84 ~ 1.72 (m, 4H), 1.51 ~ 1.47 (m, 2H).

### Example 40: Synthesis of Compound 40, 1-((5-(5-(difluoromethyl)-1,3,4-oxadiazole-2-yl)pyridine-2-yl)methyl)-3-(1-ethylpiperidine-4-yl)-5-fl uoro-1,3-dihydro-2H-benzo[d]imidazole-2-one

### [Step 1] Synthesis of tert-butyl 4-((5-fluoro-2-nitrophenyl)amino)piperidine-1-carboxylate

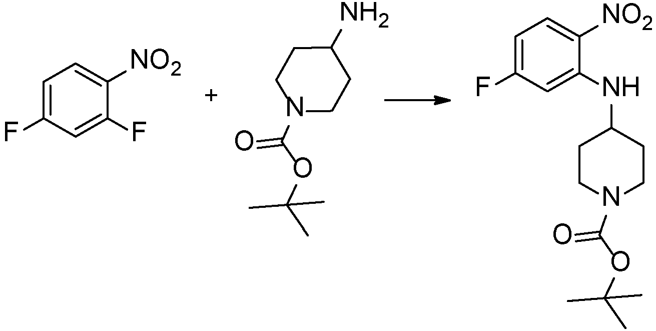

2,4-difluoro-1-nitrobenzene (2.000 g, 12.572 mmol), tert-butyl 4-aminopiperidine-1-carboxylate (2.518 g, 12.572 mmol), potassium carbonate (2.085 g, 15.086 mmol) and potassium iodide (0.021 g, 0.126 mmol) were dissolved in N,N-dimethylformamide (30 mL) at 80°C, after which the resulting solution was stirred at the same temperature for 18 hours, and then a reaction was finished by lowering a temperature to room temperature. Water was poured into the reaction mixture and an extraction was performed with ethyl acetate. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; ethyl acetate/hexane = 0 to 30%) and concentrated to obtain a title compound (3.550 g, 83.2%) in a yellow liquid form.

### [Step 2] Synthesis of tert-butyl 4-((2-amino-5-fluorophenyl)amino)piperidine-1-carboxylate

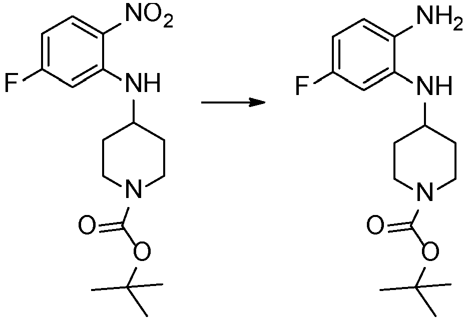

The tert-butyl 4-((5-fluoro-2-nitrophenyl)amino)piperidine-1-carboxylate (3.550 g, 10.461 mmol) prepared in the step 1 and Pd/C (45.00%, 0.247 g, 1.046 mmol) were dissolved in methanol (45 mL) at room temperature, after which the resulting solution was stirred at the same temperature for 18 hours in the presence of a hydrogen balloon attached thereto. The reaction mixture was filtered via a celite pad to remove a solid therefrom, after which solvent was removed from the resulting filtrate under reduced pressure. Then, the resulting concentrate was purified via column chromatography (SiO₂, 40 g cartridge; ethyl acetate/hexane = 0 to 30%) and concentrated to obtain a title compound (2.460 g, 76.0%) in a red solid form.

### [Step 3] Synthesis of tert-butyl 4-(6-fluoro-2-oxo-2,3-dihydro-1H-benzo[d]imidazole-1-yl)piperidine-1-carboxylate

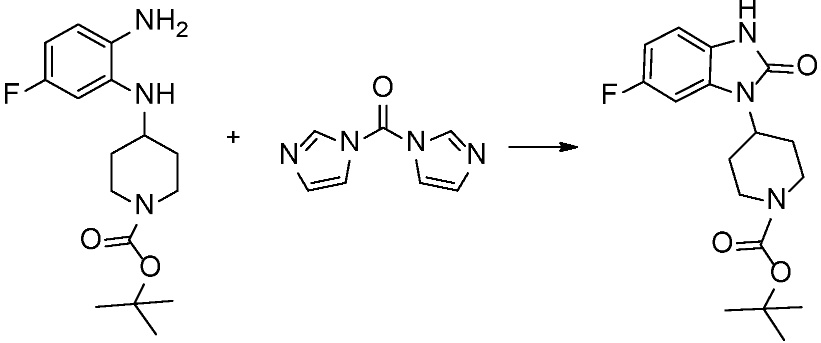

The tert-butyl 4-((2-amino-5-fluorophenyl)amino)piperidine-1-carboxylate (6.520 g, 21.074 mmol) prepared in the step 2 was dissolved in N,N-dimethylformamide (210 mL) at 0°C, after which di(1H-imidazole-1-yl)methanone (4.101 g, 25.288 mmol) was added into the resulting solution and stirred at room temperature for 18 hours. Water was poured into the reaction mixture and an extraction was performed with ethyl acetate. An organic layer was washed with saturated sodium chloride aqueous solution, then dehydrated with anhydrous magnesium sulfate, then filtered, and then concentrated under reduced pressure. The resulting concentrate was purified via column chromatography (SiO₂, 120 g cartridge; ethyl acetate/hexane = 0 to 80%) and concentrated to obtain a title compound in a violet solid form.

### [Step 4] Synthesis of tert-butyl 4-(3-((5-(5-(difluoromethyl)-1,3,4-oxadiazole-2-yl)pyridine-2-yl)methyl)-6-fluoro-2-oxo-2,3-dihydr o-1H-benzo[d]imidazole-1-yl)piperidine-1-carboxylate

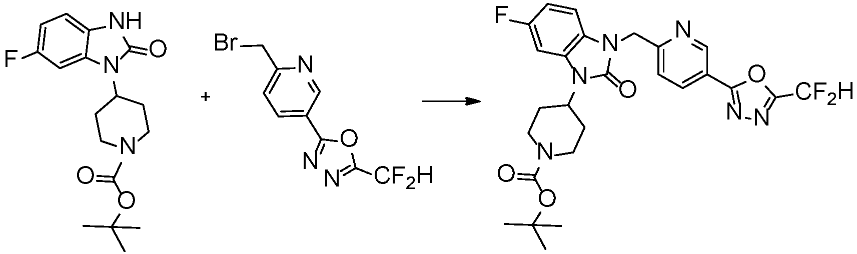

The tert-butyl 4-(6-fluoro-2-oxo-2,3-dihydro-1H-benzo[d]imidazole-1-yl)piperidine-1-carboxylate (1.200 g, 3.578 mmol) prepared in the step 3 was dissolved in N,N-dimethylformamide (20 mL) at 0°C, after which sodium hydride (60.00%, 0.215 g, 5.367 mmol) was added into the resulting solution and stirred at the same temperature for 20 minutes. 2-(6-(bromomethyl)pyridine-3-yl)-5-(difluoromethyl)-1,3,4-oxadiazole (1.557 g, 5.367 mmol) was added into the reaction mixture and further stirred at room temperature for 3 hours. Saturated ammonium chloride aqueous solution was poured into the reaction mixture and an extraction was performed with ethyl acetate. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 40 g cartridge; ethyl acetate/hexane = 0 to 60%) and concentrated to obtain a title compound (1.400 g, 71.9%) in a brown solid form.

### [Step 5] Synthesis of 1-((5-(5-(difluoromethyl)-1,3,4-oxadiazole-2-yl)pyridine-2-yl)methyl)-5-fluoro-3-(piperidine-4-yl)-1 ,3-dihydro-2H-benzo[d]imidazole-2-one

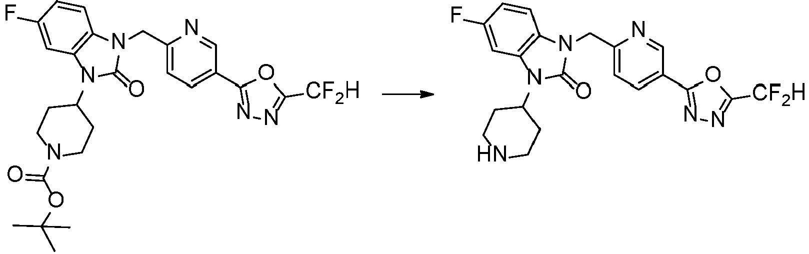

The tert-butyl 4-(3-((5-(5-(difluoromethyl)-1,3,4-oxadiazole-2-yl)pyridine-2-yl)methyl)-6-fluoro-2-oxo-2,3-dihydr o-1H-benzo[d]imidazole-1-yl)piperidine-1-carboxylate (1.400 g, 2.571 mmol) prepared in the step 4 was dissolved in dichloromethane (5 mL), after which trifluoroacetic acid (3.937 mL, 51.420 mmol) was added into the resulting solution at 0°C and stirred at room temperature for 18 hours. Saturated sodium hydrogen carbonate aqueous solution was poured into the reaction mixture and an extraction was performed with dichloromethane. An organic layer was washed with saturated sodium chloride aqueous solution, then dehydrated with anhydrous magnesium sulfate, then filtered, and then concentrated under reduced pressure. A title compound was used without an additional purification process (1.100 g, 96.3%, brown solid).

### [Step 6] Synthesis of the compound 40

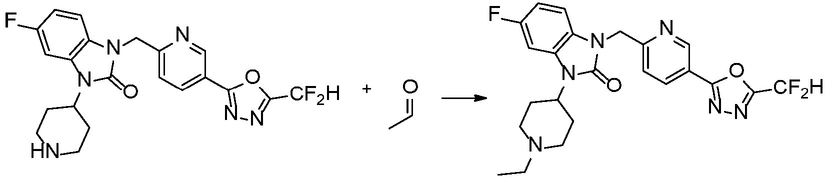

The 1-((5-(5-(difluoromethyl)-1,3,4-oxadiazole-2-yl)pyridine-2-yl)methyl)-5-fluoro-3-(piperidine-4-yl)-1 ,3-dihydro-2H-benzo[d]imidazole-2-one (0.100 g, 0.225 mmol) prepared in the step 5, acetaldehyde (0.020 g, 0.450 mmol), acetic acid (0.014 mL, 0.248 mmol) and sodium triacetoxyborohydride (0.095 g, 0.450 mmol) were dissolved in dichloromethane (1.5 mL) at room temperature, after which the resulting solution was stirred at the same temperature for 18 hours. Water was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 12 g cartridge; methanol/dichloromethane = 0 to 10%) and concentrated to obtain a title compound (0.071 g, 66.4%) in a brown liquid form.

**¹H NMR** (400 MHz, CDCl₃) δ 9.25 (dd, *J* = 2.1, 0.6 Hz, 1H), 8.31 (dd, *J* = 8.2, 2.2 Hz, 1H), 7.38 (d, *J* = 8.2 Hz, 1H), 7.18 (dd, *J* = 9.2, 2.3 Hz, 1H), 6.92 (t, *J* = 51.6 Hz, 1H), 6.86 (dd, *J* = 8.6, 4.5 Hz, 1H), 6.71 (td, *J* = 9.0, 1.7 Hz, 1H), 5.24 (s, 2H), 4.51 - 4.42 (m, 1H), 3.21 (d, *J =* 11.8 Hz, 2H), 2.60 - 2.46 (m, 4H), 2.21 (td, *J =* 12.1, 2.0 Hz, 2H), 1.86 (dd, *J* = 12.1, 2.3 Hz, 2H), 1.15 (t, *J* = 7.2 Hz, 3H); **LRMS** (ES) m/z 473.3 (M⁺ + 1).

### Example 41: Synthesis of Compound 41, 1-((5-(5-(difluoromethyl)-1,3,4-oxadiazole-2-yl)pyridine-2-yl)methyl)-5-fluoro-3-(1-(oxetan-3-yl)pi peridine-4-yl)-1,3-dihydro-2H-benzo[d]imidazole-2-one

### [Step 1] Synthesis of the compound 41

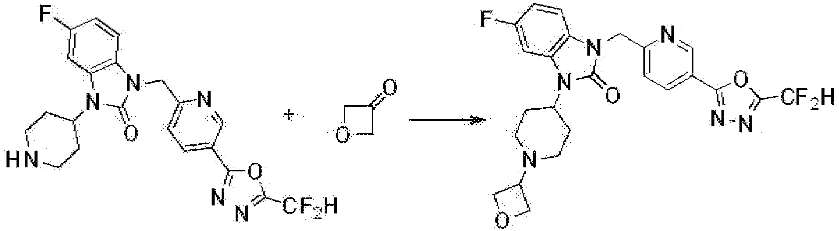

The 1-((5-(5-(difluoromethyl)-1,3,4-oxadiazole-2-yl)pyridine-2-yl)methyl)-5-fluoro-3-(piperidine-4-yl)-1 ,3-dihydro-2H-benzo[d]imidazole-2-one (0.100 g, 0.225 mmol) prepared in the step 5 of the compound 40, oxetan-3-one (0.032 g, 0.450 mmol), acetic acid (0.014 mL, 0.248 mmol) and sodium triacetoxyborohydride (0.095 g, 0.450 mmol) were dissolved in dichloromethane (1.5 mL) at room temperature, after which the resulting solution was stirred at the same temperature for 18 hours. Water was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 12 g cartridge; methanol/dichloromethane = 0 to 10%) and concentrated to obtain a title compound (0.084 g, 74.1%) in a brown liquid form.

**¹H NMR** (400 MHz, CDCl₃) δ 9.25 (dd, *J* = 2.2, 0.7 Hz, 1H), 8.31 (dd, *J* = 8.2, 2.2 Hz, 1H), 7.39 (dd, *J =* 8.2, 0.6 Hz, 1H), 7.11 (dd, *J =* 9.1, 2.3 Hz, 1H), 6.92 (t, *J =* 51.6 Hz, 1H), 6.87 (dd, *J* = 8.6, 4.5 Hz, 1H), 6.72 (td, *J* = 9.0, 2.3 Hz, 1H), 5.24 (s, 2H), 4.66 (dt, *J* = 10.6, 5.2 Hz, 4H), 4.46 - 4.38 (m, 1H), 3.58 - 3.52 (m, 1H), 2.94 - 2.92 (m, 2H), 2.50 - 2.40 (m, 2H), 2.07 - 2.04 (m, 2H), 1.86 (dd, *J* = 12.1, 2.4 Hz, 2H); **LRMS** (ES) m/z 501.4 (M⁺ + 1).

### Example 42: Synthesis of Compound 42, 3-(1-acetylpiperidine-4-yl)-1-((5-(5-(difluoromethyl)-1,3,4-oxadiazole-2-yl)pyridine-2-yl)methyl)-5-fluoro-1,3-dihydro-2H-benzo[d]imidazole-2-one

### [Step 1] Synthesis of the compound 42

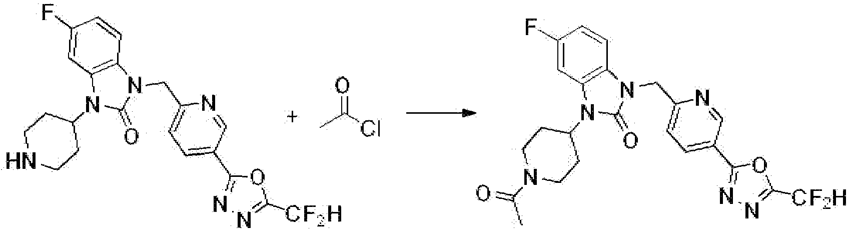

The 1-((5-(5-(difluoromethyl)-1,3,4-oxadiazole-2-yl)pyridine-2-yl)methyl)-5-fluoro-3-(piperidine-4-yl)-1 ,3-dihydro-2H-benzo[d]imidazole-2-one (0.100 g, 0.225 mmol) prepared in the step 5 of the compound 40, acetyl chloride (0.032 mL, 0.450 mmol), and triethylamine (0.063 mL, 0.450 mmol) were dissolved in dichloromethane (1.5 mL) at room temperature, after which the resulting solution was stirred at the same temperature for 18 hours. Water was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 12 g cartridge; ethyl acetate/hexane = 0 to 100%) and concentrated to obtain a title compound (0.030 g, 27.4%) in a brown liquid form.

¹H NMR (400 MHz, CDCl₃) δ 9.27 (s, 1H), 8.33 (d, *J* = 8.2 Hz, 1H), 7.41 (d, *J* = 8.2 Hz, 1H), 7.06 - 6.73 (m, 4H), 5.24 (s, 2H), 4.88 (d, *J =* 13.0 Hz, 1H), 4.53 (t, *J =* 12.3 Hz, 1H), 4.02 (d, *J = 12.4* Hz, 1H), 3.24 (t, *J =* 12.7 Hz, 1H), 2.67 (t, *J =* 12.8 Hz, 1H), 2.36 - 2.25 (m, 2H), 2.17 (s, 3H), 1.93 (t, *J =* 13.3 Hz, 2H); **LRMS** (ES) m/z 487.4 (M⁺ + 1).

### Example 43: Synthesis of Compound 43, 1-((5-(5-(difluoromethyl)-1,3,4-oxadiazole-2-yl)pyridine-2-yl)methyl)-5-fluoro-3-(1-methylpiperidin e-4-yl)-1,3-dihydro-2H-benzo[d]imidazole-2-one

### [Step 1] Synthesis of the compound 43

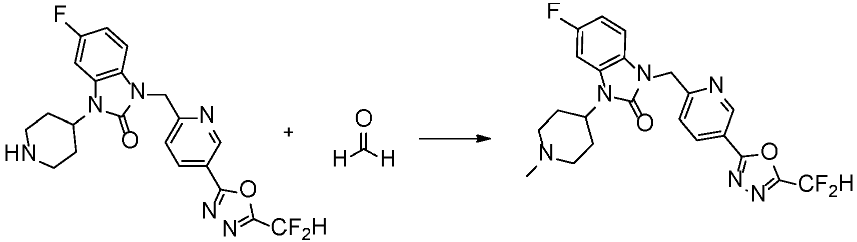

The 1-((5-(5-(difluoromethyl)-1,3,4-oxadiazole-2-yl)pyridine-2-yl)methyl)-5-fluoro-3-(piperidine-4-yl)-1 ,3-dihydro-2H-benzo[d]imidazole-2-one (0.100 g, 0.225 mmol) prepared in the step 5 of the compound 40, acetic acid (0.014 mL, 0.248 mmol), sodium triacetoxyborohydride (0.095 g, 0.450 mmol) and formaldehyde (0.014 g, 0.450 mmol) were dissolved in dichloromethane (1.5 mL) at room temperature, after which the resulting solution was stirred at the same temperature for 18 hours. Water was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 12 g cartridge; methanol/dichloromethane = 0 to 15%) and concentrated to obtain a title compound (0.051 g, 49.8%) in a brown liquid form.

**¹H NMR** (400 MHz, CDCl₃) δ 9.26 (d, *J* = 2.0 Hz, 1H), 8.31 (dd, *J* = 8.2, 2.2 Hz, 1H), 7.38 (d, *J =* 8.2 Hz, 1H), 7.16 (dd, *J =* 9.2, 2.3 Hz, 1H), 6.92 (t, *J =* 51.6 Hz, 1H), 6.86 (dd, *J =* 8.6, 4.5 Hz, 1H), 6.72 (td, *J =* 9.0, 2.2 Hz, 1H), 5.24 (s, 2H), 4.51 - 4.42 (m, 1H), 3.13 (d, *J =* 11.8 Hz, 2H), 2.58 - 2.48 (m, 2H), 2.40 (s, 3H), 2.28 (td, *J =* 12.1, 1.9 Hz, 2H), 1.84 (dd, *J =* 12.3, 2.3 Hz, 2H); **LRMS** (ES) m/z 459.3 (M⁺ + 1).

### Example 44: Synthesis of Compound 44, 1-((5-(5-(difluoromethyl)-1,3,4-oxadiazole-2-yl)pyridine-2-yl)methyl)-5-fluoro-3-(1-(tetrahydro-2H-pyran-4-yl)piperidine-4-yl)-1,3-dihydro-2H-benzo[d]imidazole-2-one

### [Step 1] Synthesis of the compound 44

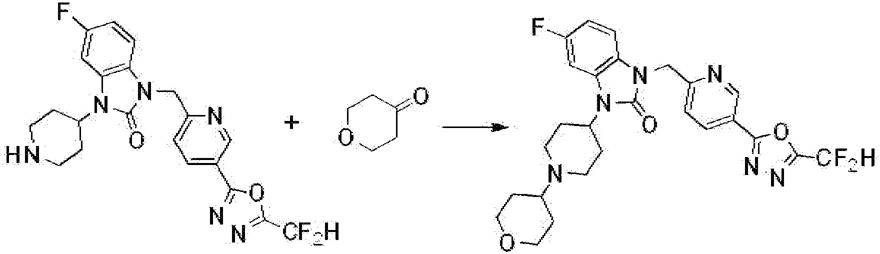

The 1-((5-(5-(difluoromethyl)-1,3,4-oxadiazole-2-yl)pyridine-2-yl)methyl)-5-fluoro-3-(piperidine-4-yl)-1 ,3-dihydro-2H-benzo[d]imidazole-2-one (0.100 g, 0.225 mmol) prepared in the step 5 of the compound 40, tetrahydro-4H-pyran-4-one (0.045 g, 0.450 mmol), acetic acid (0.014 mL, 0.248 mmol) and sodium triacetoxyborohydride (0.095 g, 0.450 mmol) were dissolved in dichloromethane (2 mL) at room temperature, after which the resulting solution was stirred at the same temperature for 18 hours. Water was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.076 g, 63.5%) in a yellow liquid form.

**¹H NMR** (400 MHz, CDCl₃) δ 9.27 (dd, *J =* 2.2, 0.8 Hz, 1H), 8.32 (dd, *J* = 8.2, 2.2 Hz, 1H), 7.40 (d, *J =* 8.2 Hz, 1H), 7.17 (dd, *J* = 9.2, 1.7 Hz, 1H), 6.92 (t, *J* = 51.6 Hz, 1H), 6.87 (dd, *J* = 8.6, 4.5 Hz, 1H), 6.73 (td, *J* = 9.0, 2.3 Hz, 1H), 5.25 (s, 2H), 4.46 - 4.39 (m, 1H), 4.05 (dd, *J* = 11.3, 3.9 Hz, 2H), 3.42 - 3.37 (m, 2H), 3.19 (d, *J =* 8.8 Hz, 2H), 2.70 - 2.65 (m, 1H), 2.50 - 2.39 (m, 4H), 1.90 - 1.80 (m, 4H), 1.72 - 1.61 (m, 2H); **LRMS** (ES) m/z 539.6 (M⁺ + 1).

### Example 45: Synthesis of Compound 45, 3-(1-cyclobutylpiperidine-4-yl)-1-((5-(5-(difluoromethyl)-1,3,4-oxadiazole-2-yl)pyridine-2-yl)methy I)-5-fluoro-1,3-dihydro-2H-benzo[d]imidazole-2-one

### [Step 1] Synthesis of the compound 45

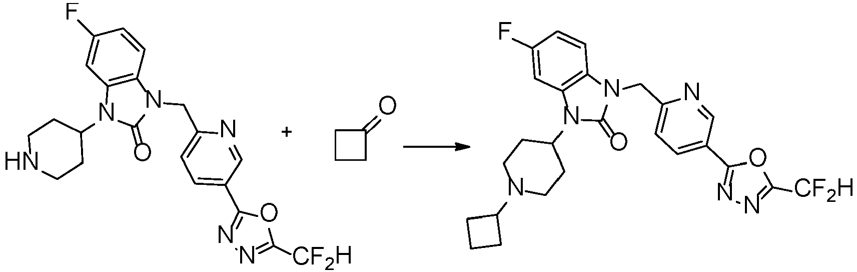

The 1-((5-(5-(difluoromethyl)-1,3,4-oxadiazole-2-yl)pyridine-2-yl)methyl)-5-fluoro-3-(piperidine-4-yl)-1 ,3-dihydro-2H-benzo[d]imidazole-2-one (0.100 g, 0.225 mmol) prepared in the synthesis step 5 of the compound 40, cyclobutanone (0.032 g, 0.450 mmol), acetic acid (0.014 mL, 0.248 mmol) and sodium triacetoxyborohydride (0.095 g, 0.450 mmol) were dissolved in dichloromethane (2 mL) at room temperature, after which the resulting solution was stirred at the same temperature for 18 hours. Water was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.097 g, 86.5%) in a yellow liquid form.

**¹H NMR** (400 MHz, CDCl₃) δ 9.28 (dd, *J* = 2.2, 0.8 Hz, 1H), 8.33 (dd, *J* = 8.2, 2.2 Hz, 1H), 7.40 (dd, *J* = 8.2, 0.7 Hz, 1H), 7.22 - 7.19 (m, 1H), 6.93 (t, *J* = 51.6 Hz, 1H), 6.87 (dd, *J* = 8.6, 4.5 Hz, 1H), 6.74 (td, *J* = 9.0, 2.1 Hz, 1H), 5.26 (s, 2H), 4.51 -4.43 (m, 1H), 3.17 (d, *J* = 11.7 Hz, 2H), 2.92 - 2.84 (m, 1H), 2.55 - 2.46 (m, 2H), 2.11 - 2.01 (m, 6H), 1.86 (dd, *J =* 12.2, 2.3 Hz, 2H), 1.79 - 1.66 (m, 2H); **LRMS** (ES) m/z 499.4 (M⁺ + 1).

### Example 46: Synthesis of Compound 46, 1-((5-(5-(difluoromethyl)-1,3,4-oxadiazole-2-yl)pyridine-2-yl)methyl)-5-fluoro-3-(1-isopropylpiperi dine-4-yl)-1,3-dihydro-2H-benzo[d]imidazole-2-one

### [Step 1] Synthesis of the compound 46

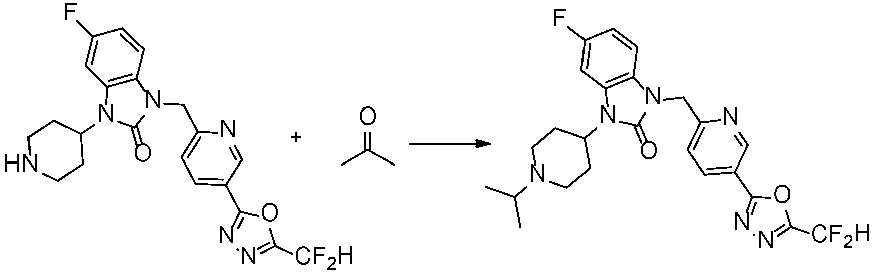

The 1-((5-(5-(difluoromethyl)-1,3,4-oxadiazole-2-yl)pyridine-2-yl)methyl)-5-fluoro-3-(piperidine-4-yl)-1 ,3-dihydro-2H-benzo[d]imidazole-2-one (0.100 g, 0.225 mmol) prepared in the step 5 of the compound 40, propane-2-one (0.026 g, 0.450 mmol), acetic acid (0.014 mL, 0.248 mmol) and sodium triacetoxyborohydride (0.095 g, 0.450 mmol) were dissolved in dichloromethane (2 mL) at room temperature, after which the resulting solution was stirred at the same temperature for 18 hours. Water was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.076 g, 69.6%) in a yellow liquid form.

**¹H NMR** (400 MHz, CDCl₃) δ 9.26 (dd, *J =* 2.2, 0.7 Hz, 1H), 8.31 (dd, *J* = 8.2, 2.2 Hz, 1H), 7.38 (d, *J =* 8.2 Hz, 1H), 7.30 (dd, *J* = 9.2, 2.3 Hz, 1H), 6.92 (t, *J =* 51.6 Hz, 1H), 6.86 (dd, *J =* 8.6, 4.5 Hz, 1H), 6.72 (td, *J =* 9.0, 2.2 Hz, 1H), 5.24 (s, 2H), 4.56 - 4.48 (m, 1H), 3.26 (d, *J* = 11.1 Hz, 2H), 3.18 - 3.11 (m, 1H), 2.70 - 2.52 (m, 4H), 1.90 (dd, *J =* 12.2, 2.8 Hz, 2H), 1.19 (d, *J* = 6.6 Hz, 6H); **LRMS** (ES) m/z 487.5 (M⁺ + 1).

### Example 47: Synthesis of Compound 47, 3-(1-cyclohexylpiperidine-4-yl)-1-((5-(5-(difluoromethyl)-1,3,4-oxadiazole-2-yl)pyridine-2-yl)meth yl)-5-fluoro-1,3-dihydro-2H-benzo[d]imidazole-2-one

### [Step 1] Synthesis of the compound 47

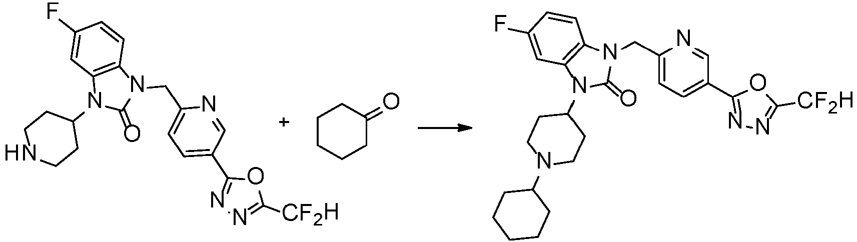

The 1-((5-(5-(difluoromethyl)-1,3,4-oxadiazole-2-yl)pyridine-2-yl)methyl)-5-fluoro-3-(piperidine-4-yl)-1 ,3-dihydro-2H-benzo[d]imidazole-2-one (0.100 g, 0.225 mmol) prepared in the step 5 of the compound 40, cyclohexanone (0.044 g, 0.450 mmol), acetic acid (0.014 mL, 0.248 mmol) and sodium triacetoxyborohydride (0.095 g, 0.450 mmol) were dissolved in dichloromethane (2 mL) at room temperature, after which the resulting solution was stirred at the same temperature for 18 hours. Water was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.094 g, 79.4%) in a yellow solid form.

**¹H NMR** (400 MHz, CDCl₃) δ 9.26 (dd, *J* = 2.2, 0.8 Hz, 1H), 8.32 (dd, *J* = 8.2, 2.2 Hz, 1H), 7.39 - 7.35 (m, 2H), 6.92 (t, *J =* 51.6 Hz, 1H), 6.86 (dd, *J =* 8.6, 4.5 Hz, 1H), 6.73 (td, *J =* 9.0, 2.3 Hz, 1H), 5.24 (s, 2H), 4.61 - 4.54 (m, 1H), 3.40 (d, *J* = 9.5 Hz, 2H), 2.90 - 2.68 (m, 5H), 2.06 - 2.04 (m, 2H), 1.94 - 1.86 (m, 4H), 1.70 - 1.67 (m, 1H), 1.41 - 1.26 (m, 4H), 1.18 - 1.08 (m, 1H); **LRMS** (ES) m/z 527.5 (M⁺ + 1).

### Example 48: Synthesis of Compound 48, 3-((5-(5-(difluoromethyl)-1,3,4-oxadiazole-2-yl)pyridine-2-yl)methyl)-5-fluoro-1-(1-methylpiperidin e-4-yl)-1,3-dihydro-2H-benzo[d]imidazole-2-one

### [Step 1] Synthesis of tert-butyl 4-((4-fluoro-2-nitrophenyl)amino)piperidine-1-carboxylate

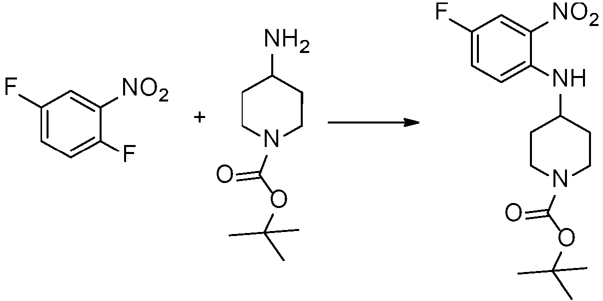

1,4-difluoro-2-nitrobenzene (2.000 g, 12.572 mmol), tert-butyl 4-aminopiperidine-1-carboxylate (2.518 g, 12.572 mmol), potassium carbonate (2.085 g, 15.086 mmol) and potassium iodide (0.021 g, 0.126 mmol) were dissolved in N,N-dimethylformamide (30 mL) at 80°C, after which the resulting solution was stirred at the same temperature for 18 hours, and then a reaction was finished by lowering a temperature to room temperature. Water was poured into the reaction mixture and an extraction was performed with ethyl acetate. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; ethyl acetate/hexane = 0 to 30%) and concentrated to obtain a title compound (4.230 g, 99.1%) in a red solid form.

### [Step 2] Synthesis of tert-butyl 4-((2-amino-4-fluorophenyl)amino)piperidine-1-carboxylate

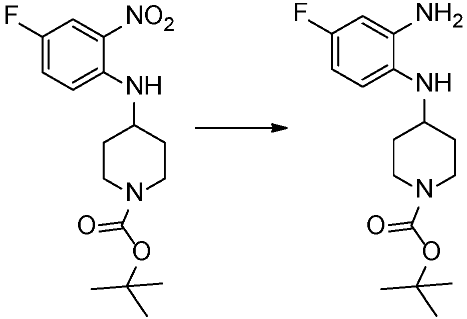

The tert-butyl 4-((4-fluoro-2-nitrophenyl)amino)piperidine-1-carboxylate (4.230 g, 12.464 mmol) prepared in the step 1 and Pd/C (10%, 0.295 g, 1.246 mmol) were mixed in methanol (50 mL) at room temperature, after which the resulting suspending solution was stirred at the same temperature for 18 hours in the presence of a hydrogen balloon. The reaction mixture was filtered via a celite pad to remove a solid therefrom, after which solvent was removed from the resulting filtrate under reduced pressure. Then, the resulting concentrate was purified via column chromatography (SiO₂, 12 g cartridge; ethyl acetate/hexane = 0 to 30%) and concentrated to obtain a title compound (3.260 g, 84.5%) in a red solid form.

### [Step 3] Synthesis of tert-butyl 4-(5-fluoro-2-oxo-2,3-dihydro-1H-benzo[d]imidazole-1-yl)piperidine-1-carboxylate

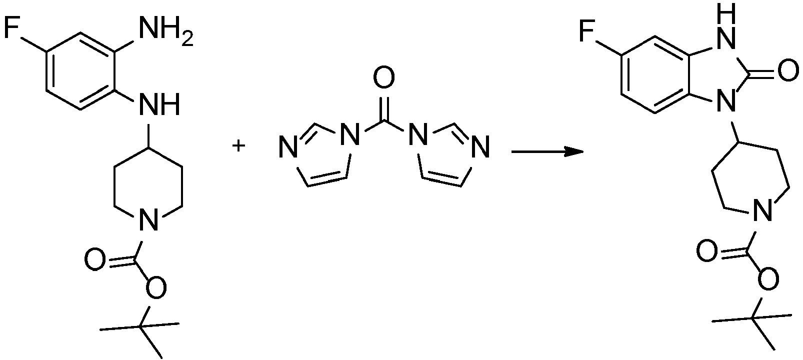

The tert-butyl 4-((2-amino-4-fluorophenyl)amino)piperidine-1-carboxylate (7.300 g, 23.595 mmol) prepared in the step 2 and di(1H-imidazole-1-yl)methanone (4.017 g, 24.775 mmol) were dissolved in N,N-dimethylformamide (150 mL) at 0°C, after which the resulting solution was stirred at room temperature for 18 hours. Solvent was removed from the reaction mixture under reduced pressure, after which water was poured into the resulting concentrate, and then an extraction was performed with ethyl acetate. An organic layer was washed with saturated sodium chloride aqueous solution, then dehydrated with anhydrous magnesium sulfate, then filtered, and then concentrated under reduced pressure. The resulting concentrate was purified via column chromatography (SiO₂, 80 g cartridge; ethyl acetate/hexane = 0 to 50%) and concentrated to obtain a title compound (6.950 g, 87.8%) in a brown solid form.

### [Step 4] Synthesis of tert-butyl 4-(3-((5-(5-(difluoromethyl)-1,3,4-oxadiazole-2-yl)pyridine-2-yl)methyl)-5-fluoro-2-oxo-2,3-dihydr o-1H-benzo[d]imidazole-1-yl)piperidine-1-carboxylate

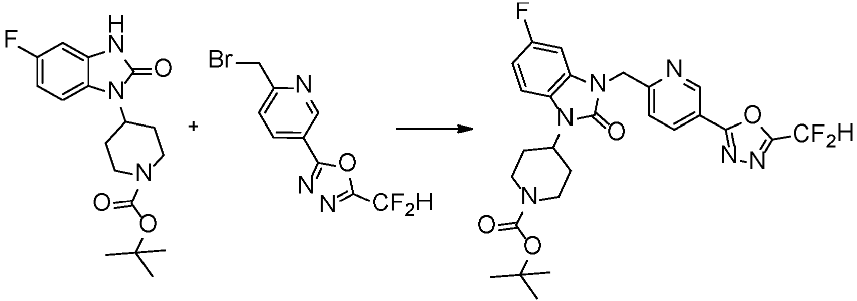

The 2-(6-(bromomethyl)pyridine-3-yl)-5-(difluoromethyl)-1,3,4-oxadiazole (4.000 g, 13.790 mmol) prepared in the step 3 was dissolved in N,N-dimethylformamide (140 mL) at 0°C, after which sodium hydride (60.00%, 0.717 g, 17.927 mmol) was added into the resulting solution and stirred at the same temperature for 30 minutes. Tert-butyl 4-(5-fluoro-2-oxo-2,3-dihydro-1H-benzo[d]imidazole-1-yl)piperidine-1-carboxylate (6.012 g, 17.927 mmol) was added into the reaction mixture and further stirred at room temperature for 5 hours. Water was poured into the reaction mixture and an extraction was performed with ethyl acetate. An organic layer was washed with saturated sodium chloride aqueous solution, then dehydrated with anhydrous magnesium sulfate, then filtered, and then concentrated under reduced pressure. The resulting concentrate was purified via column chromatography (SiO₂, 120 g cartridge; ethyl acetate/hexane = 0 to 40%) and concentrated to obtain a title compound (4.680 g, 62.3%) in an orange solid form.

### [Step 5] Synthesis of 3-((5-(5-(difluoromethyl)-1,3,4-oxadiazole-2-yl)pyridine-2-yl)methyl)-5-fluoro-1-(piperidine-4-yl)-1 ,3-dihydro-2H-benzo[d]imidazole-2-one

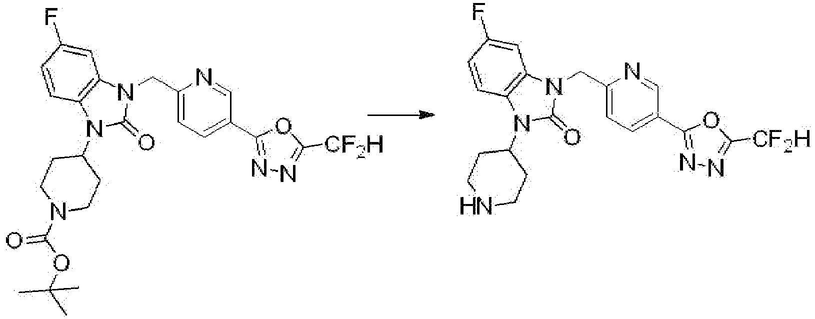

The tert-butyl 4-(3-((5-(5-(difluoromethyl)-1,3,4-oxadiazole-2-yl)pyridine-2-yl)methyl)-5-fluoro-2-oxo-2,3-dihydr o-1H-benzo[d]imidazole-1-yl)piperidine-1-carboxylate (4.680 g, 8.594 mmol) prepared in the step 4 was dissolved in dichloromethane (80 mL) at 0°C, after which trifluoroacetic acid (13.162 mL, 171.888 mmol) was added into the resulting solution and stirred at room temperature for 4.5 hours. Saturated sodium hydrogen carbonate aqueous solution was poured into the reaction mixture and an extraction was performed with dichloromethane. An organic layer was washed with saturated sodium chloride aqueous solution, then dehydrated with anhydrous magnesium sulfate, then filtered, and then concentrated under reduced pressure. The resulting concentrate was purified via column chromatography (SiO₂, 80 g cartridge; methanol/dichloromethane = 0 to 10%) and concentrated to obtain a title compound (3.710 g, 97.1%) in a light brown solid form.

### [Step 6] Synthesis of the compound 48

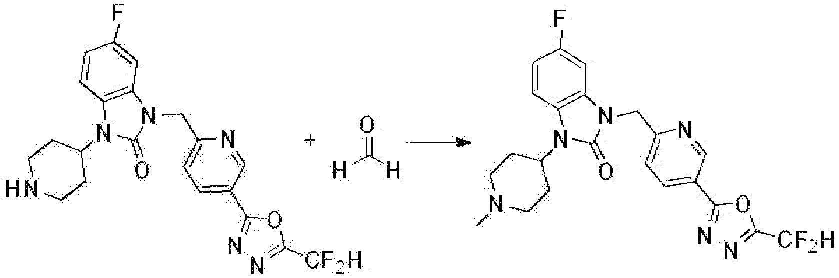

The 1-((5-(5-(difluoromethyl)-1,3,4-oxadiazole-2-yl)pyridine-2-yl)methyl)-5-fluoro-3-(piperidine-4-yl)-1 ,3-dihydro-2H-benzo[d]imidazole-2-one (0.100 g, 0.225 mmol) prepared in the step 5, formaldehyde (0.014 g, 0.450 mmol), acetic acid (0.014 mL, 0.248 mmol) and sodium triacetoxyborohydride (0.095 g, 0.450 mmol) were dissolved in dichloromethane (2 mL) at room temperature, after which the resulting solution was stirred at the same temperature for 18 hours. Water was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.065 g, 63.0%) in a yellow liquid form.

**¹H NMR** (400 MHz, CDCl₃) δ 9.27 (s, 1H), 8.33 (d, *J* = 8.0 Hz, 1H), 7.41 (d, *J =* 7.8 Hz, 1H), 7.26 - 7.24 (m, 1H), 7.05 - 6.74 (m, 3H), 5.23 (s, 2H), 4.45 - 4.39 (m, 1H), 3.04 (d, *J =* 10.6 Hz, 2H), 2.50 - 2.44 (m, 2H), 2.36 (s, 3H), 2.19 (t, *J* = 11.3 Hz, 2H), 1.84 (d, *J* = 10.6 Hz, 2H); LRMS (ES) m/z 459.3 (M⁺ + 1).

### Example 49: Synthesis of Compound 49, 1-(1-(2-oxaspiro[3.3]heptane-6-yl)piperidine-4-yl)-3-((5-(5-(difluoromethyl)-1,3,4-oxadiazole-2-yl) pyridine-2-yl)methyl)-5-fluoro-1,3-dihydro-2H-benzo[d]imidazole-2-one

### [Step 1] Synthesis of the compound 49

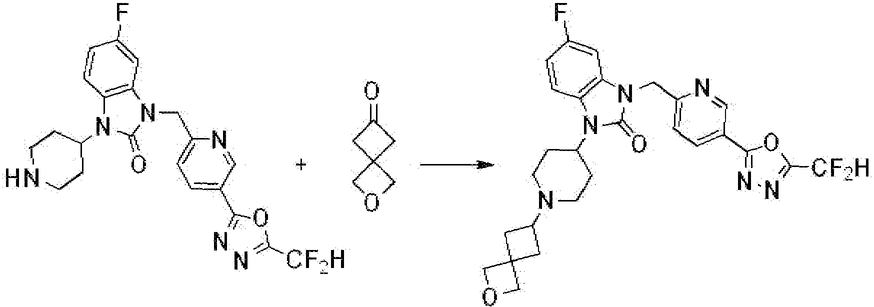

The 3-((5-(5-(difluoromethyl)-1,3,4-oxadiazole-2-yl)pyridine-2-yl)methyl)-5-fluoro-1-(piperidine-4-yl)-1 ,3-dihydro-2H-benzo[d]imidazole-2-one (0.100 g, 0.225 mmol) prepared in the step 5 of the compound 48, 2-oxaspiro[3.3]heptane-6-one (0.050 g, 0.450 mmol), acetic acid (0.014 mL, 0.248 mmol) and sodium triacetoxyborohydride (0.095 g, 0.450 mmol) were dissolved in dichloromethane (2 mL) at room temperature, after which the resulting solution was stirred at the same temperature for 18 hours. Water was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.070 g, 57.3%) in a white solid form.

**¹H NMR** (400 MHz, CDCl₃) δ 9.27 (d, *J* = 1.7 Hz, 1H), 8.33 (dd, *J* = 8.2, 2.2 Hz, 1H), 7.41 (d, *J =* 7.2 Hz, 1H), 7.22 (s, 1H), 7.05 - 6.74 (m, 3H), 5.23 (s, 2H), 4.72 (s, 2H), 4.61 (s, 2H), 4.45 - 4.30 (m, 1H), 3.15 - 2.95 (m, 2H), 2.90 - 2.50 (m, 1H), 2.50 - 2.25 (m, 4H), 2.20 - 1.85 (m, 6H); **LRMS** (ES) m/z 541.6 (M⁺ + 1).

### Example 50: Synthesis of Compound 50, 3-((5-(5-(difluoromethyl)-1,3,4-oxadiazole-2-yl)pyridine-2-yl)methyl)-1-(1-ethylpiperidine-4-yl)-5-fl uoro-1,3-dihydro-2H-benzo[d]imidazole-2-one

### [Step 1] Synthesis of the compound 50

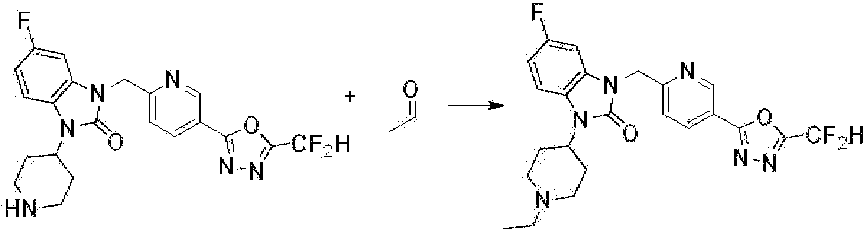

The 1-((5-(5-(difluoromethyl)-1,3,4-oxadiazole-2-yl)pyridine-2-yl)methyl)-5-fluoro-3-(piperidine-4-yl)-1 ,3-dihydro-2H-benzo[d]imidazole-2-one (0.100 g, 0.225 mmol) prepared in the step 5 of the compound 48, acetaldehyde (0.020 g, 0.450 mmol), acetic acid (0.014 mL, 0.248 mmol) and sodium triacetoxyborohydride (0.095 g, 0.450 mmol) were dissolved in dichloromethane (2 mL) at room temperature, after which the resulting solution was stirred at the same temperature for 18 hours. Water was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 10%) and concentrated to obtain a title compound (0.063 g, 59.4%) in a yellow liquid form.

**¹H NMR** (400 MHz, CDCl₃) δ 9.27 (s, 1H), 8.32 (d, *J* = 8.2 Hz, 1H), 7.41 (d, *J* = 8.2 Hz, 1H), 7.28 - 7.23 (m, 1H), 6.93 (t, *J* = 51.6 Hz, 1H), 6.77 - 6.73 (m, 2H), 5.23 (s, 2H), 4.45 - 4.39 (m, 1H), 3.12 (d, *J =* 11.5 Hz, 2H), 2.50 - 2.40 (m, 4H), 2.12 (t, *J =* 11.8 Hz, 2H), 1.85 (d, *J =* 11.7 Hz, 2H), 1.12 (t, *J* = 7.1 Hz, 3H); **LRMS** (ES) m/z 473.6 (M⁺+ 1).

### Example 51: Synthesis of Compound 51, 3-((5-(5-(difluoromethyl)-1,3,4-oxadiazole-2-yl)pyridine-2-yl)methyl)-5-fluoro-1-(1-isopropylpiperi dine-4-yl)-1,3-dihydro-2H-benzo[d]imidazole-2-one

### [Step 1] Synthesis of the compound 51

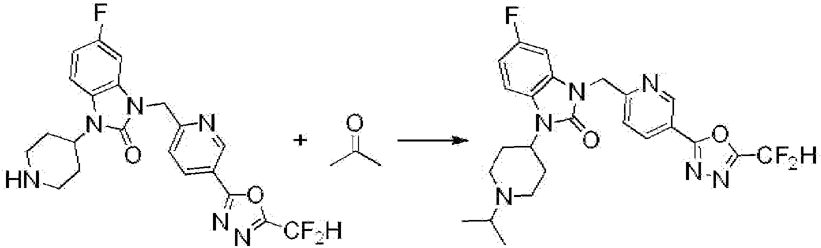

The 3-((5-(5-(difluoromethyl)-1,3,4-oxadiazole-2-yl)pyridine-2-yl)methyl)-5-fluoro-1-(piperidine-4-yl)-1 ,3-dihydro-2H-benzo[d]imidazole-2-one (0.100 g, 0.225 mmol) prepared in the step 5 of the compound 48, propane-2-one (0.026 g, 0.450 mmol), acetic acid (0.014 mL, 0.248 mmol) and sodium triacetoxyborohydride (0.095 g, 0.450 mmol) were dissolved in dichloromethane (2 mL) at room temperature, after which the resulting solution was stirred at the same temperature for 18 hours. Water was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 10%) and concentrated to obtain a title compound (0.068 g, 62.5%) in a yellow liquid form.

**¹H NMR** (400 MHz, CDCl₃) δ 9.27 (t, *J =* 1.1 Hz, 1H), 8.32 (dd, *J* = 8.2, 2.2 Hz, 1H), 7.41 (d, *J* = 8.3 Hz, 1H), 7.31 - 7.28 (m, 1H), 6.92 (t, *J* = 51.6 Hz, 1H), 6.78 - 6.72 (m, 2H), 5.23 (s, 2H), 4.42 - 4.35 (m, 1H), 3.04 (d, *J =* 9.9 Hz, 2H), 2.87 - 2.80 (m, 1H), 2.49 - 2.34 (m, 4H), 1.87 - 1.84 (m, 2H), 1.09 (d, *J =* 6.5 Hz, 6H); **LRMS** (ES) m/z 487.6 (M⁺ + 1).

### Example 52: Synthesis of Compound 52, 1-(1-cyclobutylpiperidine-4-yl)-3-((5-(5-(difluoromethyl)-1,3,4-oxadiazole-2-yl)pyridine-2-yl)methy I)-5-fluoro-1,3-dihydro-2H-benzo[d]imidazole-2-one

### [Step 1] Synthesis of the compound 52

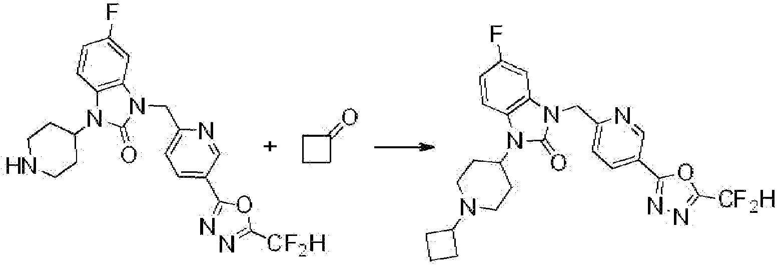

The 3-((5-(5-(difluoromethyl)-1,3,4-oxadiazole-2-yl)pyridine-2-yl)methyl)-5-fluoro-1-(piperidine-4-yl)-1 ,3-dihydro-2H-benzo[d]imidazole-2-one (0.100 g, 0.225 mmol) prepared in the step 5 of the compound 45, cyclobutanone (0.032 g, 0.450 mmol), acetic acid (0.014 mL, 0.248 mmol) and sodium triacetoxyborohydride (0.095 g, 0.450 mmol) were dissolved in dichloromethane (2 mL) at room temperature, after which the resulting solution was stirred at the same temperature for 18 hours. Water was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 10%) and concentrated to obtain a title compound (0.096 g, 85.3%) in a transparent liquid form.

**¹H NMR** (400 MHz, CDCl₃) δ 9.27 (t, *J =* 1.1 Hz, 1H), 8.32 (dd, *J =* 8.2, 2.2 Hz, 1H), 7.40 (d, *J* = 8.2 Hz, 1H), 7.29 - 7.26 (m, 1H), 6.92 (t, *J = 51.6* Hz, 1H), 6.79 - 6.73 (m, 2H), 5.23 (s, 2H), 4.45 - 4.37 (m, 1H), 3.06 (d, *J =* 11.4 Hz, 2H), 2.82 - 2.74 (m, 1H), 2.46 - 2.37 (m, 2H), 2.09 - 2.03 (m, 2H), 1.96 - 1.85 (m, 6H), 1.76 - 1.64 (m, 2H); **LRMS** (ES) m/z 499.6 (M⁺ + 1).

### Example 53: Synthesis of Compound 53, 3-((5-(5-(difluoromethyl)-1,3,4-oxadiazole-2-yl)pyridine-2-yl)methyl)-5-fluoro-1-(1-(oxetan-3-yl)pi peridine-4-yl)-1,3-dihydro-2H-benzo[d]imidazole-2-one

### [Step 1] Synthesis of the compound 53

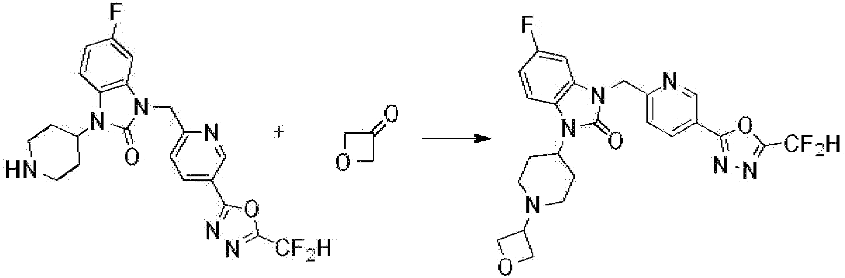

The 3-((5-(5-(difluoromethyl)-1,3,4-oxadiazole-2-yl)pyridine-2-yl)methyl)-5-fluoro-1-(piperidine-4-yl)-1 ,3-dihydro-2H-benzo[d]imidazole-2-one (0.100 g, 0.225 mmol) prepared in the step 5 of the compound 48, oxetan-3-one (0.032 g, 0.450 mmol), acetic acid (0.014 mL, 0.248 mmol) and sodium triacetoxyborohydride(0.095 g, 0.450 mmol) were dissolved in dichloromethane (2 mL) at room temperature, after which the resulting solution was stirred at the same temperature for 18 hours. Water was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 10%) and concentrated to obtain a title compound (0.110 g, 97.6%) in a light yellow solid form.

**¹H NMR** (400 MHz, CDCl₃) δ 9.26 (t, *J =* 1.1 Hz, 1H), 8.32 (dd, *J* = 8.2, 2.1 Hz, 1H), 7.41 (d, *J* = 8.2 Hz, 1H), 7.23 (dd, *J* = 8.4, 4.2 Hz, 1H), 6.92 (t, *J* = 51.6 Hz, 1H), 6.79 - 6.74 (m, 2H), 5.22 (s, 2H), 4.68 - 4.60 (m, 4H), 4.46 - 4.37 (m, 1H), 3.56 - 3.49 (m, 1H), 2.90 (d, *J* = 11.5 Hz, 2H), 2.50 - 2.40 (m, 2H), 2.05 - 1.99 (m, 2H), 1.87 - 1.85 (m, 2H); LRMS (ES) m/z 501.5 (M⁺ + 1).

### Example 54: Synthesis of Compound 54, 1-(1-cyclohexylpiperidine-4-yl)-3-((5-(5-(difluoromethyl)-1,3,4-oxadiazole-2-yl)pyridine-2-yl)meth yl)-5-fluoro-1,3-dihydro-2H-benzo[d]imidazole-2-one

### [Step 1] Synthesis of the compound 54

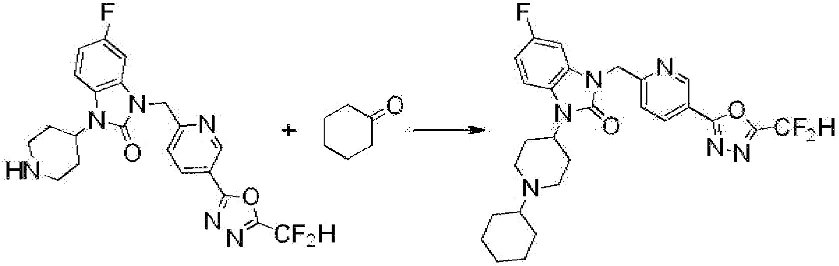

The 3-((5-(5-(difluoromethyl)-1,3,4-oxadiazole-2-yl)pyridine-2-yl)methyl)-5-fluoro-1-(piperidine-4-yl)-1 ,3-dihydro-2H-benzo[d]imidazole-2-one (0.100 g, 0.225 mmol) prepared in the step 5 of the compound 48, cyclohexanone (0.044 g, 0.450 mmol), acetic acid (0.014 mL, 0.248 mmol) and sodium triacetoxyborohydride (0.095 g, 0.450 mmol) were dissolved in dichloromethane (2 mL) at room temperature, after which the resulting solution was stirred at the same temperature for 18 hours. Water was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 10%) and concentrated to obtain a title compound (0.077 g, 64.9%) in a light yellow solid form.

**¹H NMR** (400 MHz, CDCl₃) δ 9.26 (d, *J =* 1.7 Hz, 1H), 8.32 (dd, *J* = 8.2, 2.2 Hz, 1H), 7.40 (d, *J* = 8.0 Hz, 1H), 7.29 - 7.26 (m, 1H), 6.92 (t, *J* = 51.6 Hz, 1H), 6.78 - 6.72 (m, 2H), 5.23 (s, 2H), 4.40 - 4.35 (m, 1H), 3.06 - 3.05 (m, 2H), 2.46 - 2.37 (m, 5H), 1.88 - 1.79 (m, 6H), 1.63 (d, *J =* 12.2 Hz, 1H), 1.30 - 1.19 (m, 4H), 1.14 - 1.07 (m, 1H); **LRMS** (ES) m/z 527.6 (M⁺+ 1).

### Example 55: Synthesis of Compound 55, 3-((5-(5-(difluoromethyl)-1,3,4-oxadiazole-2-yl)pyridine-2-yl)methyl)-5-fluoro-1-(1-(tetrahydro-2H-pyran-4-yl)piperidine-4-yl)-1,3-dihydro-2H-benzo[d]imidazole-2-one

### [Step 1] Synthesis of the compound 55

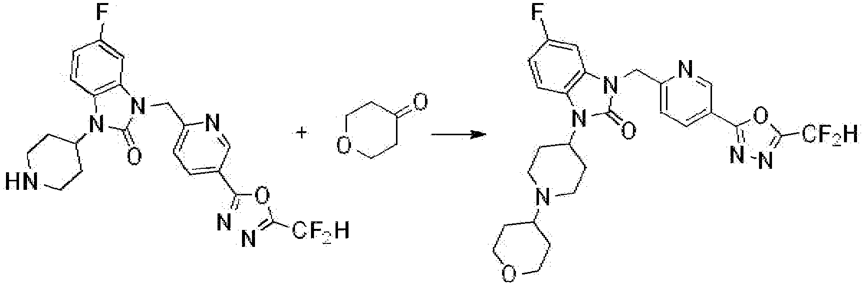

The 3-((5-(5-(difluoromethyl)-1,3,4-oxadiazole-2-yl)pyridine-2-yl)methyl)-5-fluoro-1-(piperidine-4-yl)-1 ,3-dihydro-2H-benzo[d]imidazole-2-one (0.100 g, 0.225 mmol) prepared in the step 5 of the compound 48, tetrahydro-4H-pyran-4-one (0.045 g, 0.450 mmol), acetic acid (0.014 mL, 0.248 mmol) and sodium triacetoxyborohydride (0.095 g, 0.450 mmol) were dissolved in dichloromethane (2 mL) at room temperature, after which the resulting solution was stirred at the same temperature for 18 hours. Water was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 10%) and concentrated to obtain a title compound (0.070 g, 58.9%) in a light yellow solid form.

**¹H NMR** (400 MHz, CDCl₃) δ 9.27 (d, *J =* 1.6 Hz, 1H), 8.32 (dd, *J* = 8.2, 2.2 Hz, 1H), 7.41 (d, *J* = 8.2 Hz, 1H), 7.30 - 7.20 (m, 1H), 6.92 (t, *J =* 51.7 Hz, 1H), 6.79 - 6.73 (m, 2H), 5.23 (s, 2H), 4.42 - 4.35 (m, 1H), 4.03 (dd, *J =* 11.2, 3.8 Hz, 2H), 3.41 - 3.35 (m, 2H), 3.13 (d, *J =* 8.2 Hz, 2H), 2.65-2.50 (m, 1H), 2.44 - 2.34 (m, 4H), 1.89 - 1.85 (m, 2H), 1.80 - 1.77 (m, 2H), 1.68 - 1.67 (m, 2H); LRMS (ES) m/z 529.4 (M⁺ + 1).

### Example 56: Synthesis of Compound 56, 3-((5-(5-(difluoromethyl)-1,3,4-oxadiazole-2-yl)pyridine-2-yl)methyl)-5-fluoro-1-(1-(1-methoxypro pane-2-yl)piperidine-4-yl)-1,3-dihydro-2H-benzo[d]imidazole-2-one

### [Step 1] Synthesis of the compound 56

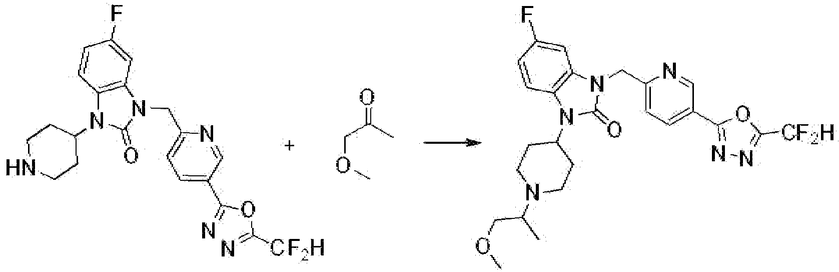

The 3-((5-(5-(difluoromethyl)-1,3,4-oxadiazole-2-yl)pyridine-2-yl)methyl)-5-fluoro-1-(piperidine-4-yl)-1 ,3-dihydro-2H-benzo[d]imidazole-2-one (0.100 g, 0.225 mmol) prepared in the step 5 of the compound 48, 1-methoxypropane-2-one (0.040 g, 0.450 mmol), acetic acid (0.014 mL, 0.248 mmol) and sodium triacetoxyborohydride (0.095 g, 0.450 mmol) were dissolved in dichloromethane (2 mL) at room temperature, after which the resulting solution was stirred at the same temperature for 18 hours. Water was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.039 g, 33.6%) in a yellow liquid form.

**¹H NMR** (400 MHz, CDCl₃) δ 9.26 (d, *J =* 2.1 Hz, 1H), 8.32 (dd, *J* = 8.2, 2.2 Hz, 1H), 7.40 (d, *J* = 8.5 Hz, 1H), 7.36 - 7.20 (m, 1H), 6.92 (t, *J = 51.6* Hz, 1H), 6.79 - 6.72 (m, 2H), 5.23 (s, 2H), 4.46 - 4.35 (m, 1H), 3.49 (dd, *J* = 9.7, 6.0 Hz, 1H), 3.35 - 3.28 (m, 4H), 3.10 - 2.95 (m, 2H), 2.94 - 2.86 (m, 1H), 2.50 - 2.40 (m, 4H), 1.84 - 1.82 (m, 2H), 1.07 (d, *J* = 6.6 Hz, 3H); **LRMS** (ES) m/z 517.4 (M⁺ + 1).

### Example 57: Synthesis of Compound 57, 3-((5-(5-(difluoromethyl)-1,3,4-oxadiazole-2-yl)pyridine-2-yl)methyl)-5-fluoro-1-(1-(tetrahydro-2H-thiopyran-4-yl)piperidine-4-yl)-1,3-dihydro-2H-benzo[d]imidazole-2-one

### [Step 1] Synthesis of the compound 57

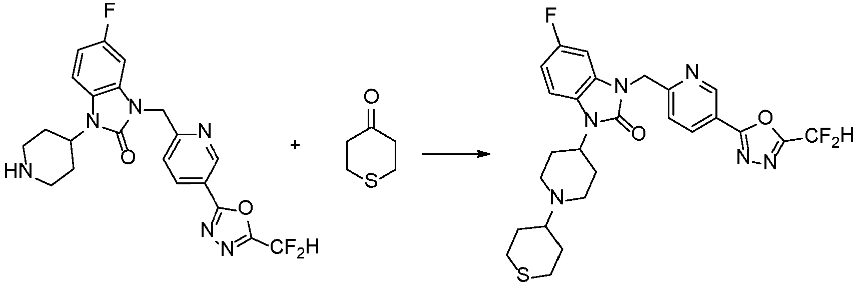

The 3-((5-(5-(difluoromethyl)-1,3,4-oxadiazole-2-yl)pyridine-2-yl)methyl)-5-fluoro-1-(piperidine-4-yl)-1 ,3-dihydro-2H-benzo[d]imidazole-2-one (0.100 g, 0.225 mmol) prepared in the step 5 of the compound 48, tetrahydro-4H-thiopyran-4-one (0.052 g, 0.450 mmol), acetic acid (0.014 mL, 0.248 mmol) and sodium triacetoxyborohydride (0.095 g, 0.450 mmol) were dissolved in dichloromethane (2 mL) at room temperature, after which the resulting solution was stirred at the same temperature for 18 hours. Water was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.025 g, 20.2%) in a white solid form.

**¹H NMR** (400 MHz, CDCl₃) δ 9.27 (d, *J* = 2.0 Hz, 1H), 8.33 (dd, *J* = 8.2, 2.2 Hz, 1H), 7.41 (d, *J* = 8.2 Hz, 1H), 7.35 - 7.15 (m, 1H), 7.05 - 6.73 (m, 3H), 5.23 (s, 2H), 4.50 - 4.30 (m, 1H), 3.20 - 2.85 (m, 2H), 2.73 - 2.70 (m, 4H), 2.70 - 2.35 (m, 4H), 2.25 - 2.15 (m, 2H), 1.19 - 1.85 (m, 3H), 1.78 - 1.69 (m, 2H); LRMS (ES) m/z 545.6 (M⁺+ 1).

### Example 58: Synthesis of Compound 58, 1-(1-(1,4-dioxaspiro[4.5]decane-8-yl)piperidine-4-yl)-3-((5-(5-(difluoromethyl)-1,3,4-oxadiazole-2 -yl)pyridine-2-yl)methyl)-5-fluoro-1,3-dihydro-2H-benzo[d]imidazole-2-one

### [Step 1] Synthesis of the compound 58

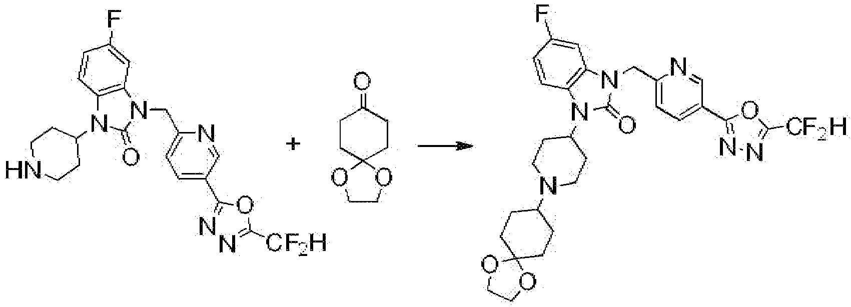

The 3-((5-(5-(difluoromethyl)-1,3,4-oxadiazole-2-yl)pyridine-2-yl)methyl)-5-fluoro-1-(piperidine-4-yl)-1 ,3-dihydro-2H-benzo[d]imidazole-2-one (0.100 g, 0.225 mmol) prepared in the step 5 of the compound 48, 1,4-dioxaspiro[4.5]decane-8-one (0.070 g, 0.450 mmol) and sodium triacetoxyborohydride (0.095 g, 0.450 mmol) were dissolved in dichloromethane (2 mL) at room temperature, after which the resulting solution was stirred at the same temperature for 18 hours. Water was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.050 g, 38.2%) in a white solid form.

**¹H NMR** (400 MHz, CDCl₃) δ 9.26 (dd, *J =* 2.2, 0.8 Hz, 1H), 8.32 (dd, *J* = 8.2, 2.2 Hz, 1H), 7.40 (dd, *J* = 8.2, 0.7 Hz, 1H), 7.27 - 7.24 (m, 1H), 7.05 - 6.72 (m, 3H), 5.22 (s, 2H), 4.40 - 4.35 (m, 1H), 3.93 (s, 4H), 3.06 - 3.04 (m, 2H), 2.51 - 2.42 (m, 4H), 1.85 - 1.81 (m, 6H), 1.67 - 1.52 (m, 4H); **LRMS** (ES) m/z 585.6 (M⁺ + 1).

### Example 59: Synthesis of Compound 59, 1-(1'-acetyl-[1,4'-bipiperidine]-4-yl)-3-((5-(5-(difluoromethyl)-1,3,4-oxadiazole-2-yl)pyridine-2-yl) methyl)-5-fluoro-1,3-dihydro-2H-benzo[d]imidazole-2-one

### [Step 1] Synthesis of the compound 59

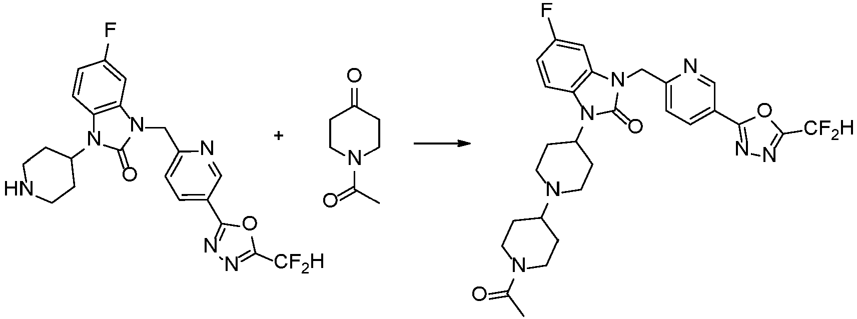

The 3-((5-(5-(difluoromethyl)-1,3,4-oxadiazole-2-yl)pyridine-2-yl)methyl)-5-fluoro-1-(piperidine-4-yl)-1 ,3-dihydro-2H-benzo[d]imidazole-2-one (0.100 g, 0.225 mmol) prepared in the step 5 of the compound 48, 1-acetylpiperidine-4-one (0.064 g, 0.450 mmol) and sodium triacetoxyborohydride (0.095 g, 0.450 mmol) were dissolved in dichloromethane (2 mL) at room temperature, after which the resulting solution was stirred at the same temperature for 18 hours. Water was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.063 g, 48.9%) in a yellow solid form.

**¹H NMR** (400 MHz, CDCl₃) δ 9.24 (dd, *J* = 2.1, 0.6 Hz, 1H), 8.31 (dd, *J* = 8.2, 2.2 Hz, 1H), 7.39 (d, *J =* 8.2 Hz, 1H), 7.23 - 7.19 (m, 1H), 6.92 (t, *J* = 51.6 Hz, 1H), 6.77 - 6.72 (m, 2H), 5.21 (s, 2H), 4.67 - 4.63 (m, 1H), 4.36 - 4.35 (m, 1H), 3.88 - 3.85 (m, 1H), 3.07 - 3.06 (m, 3H), 2.63 - 2.56 (m, 2H), 2.56 - 2.39 (m, 4H), 2.07 (s, 3H), 1.91 - 1.84 (m, 4H), 1.52 - 1.38 (m, 2H); **LRMS** (ES) m/z 570.6 (M⁺ + 1).

### Example 60: Synthesis of Compound 60, 1-(1-acetylpiperidine-4-yl)-3-((5-(5-(difluoromethyl)-1,3,4-oxadiazole-2-yl)pyridine-2-yl)methyl)-5-fluoro-1,3-dihydro-2H-benzo[d]imidazole-2-one

### [Step 1] Synthesis of the compound 60

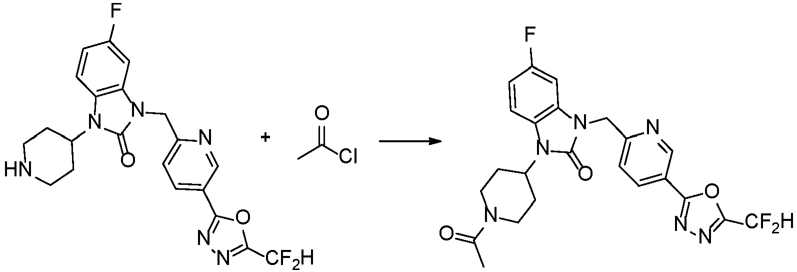

The 3-((5-(5-(difluoromethyl)-1,3,4-oxadiazole-2-yl)pyridine-2-yl)methyl)-5-fluoro-1-(piperidine-4-yl)-1 ,3-dihydro-2H-benzo[d]imidazole-2-one (0.100 g, 0.225 mmol) prepared in the step 5 of the compound 48, triethylamine (0.063 mL, 0.450 mmol) and acetyl chloride (0.032 mL, 0.450 mmol) were dissolved in dichloromethane (2 mL) at room temperature, after which the resulting solution was stirred at the same temperature for 18 hours. Water was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.085 g, 77.7%) in a yellow liquid form.

**¹H NMR** (400 MHz, CDCl₃) δ 9.26 (dd, *J* = 10.9, 9.5 Hz, 1H), 8.33 (dd, *J* = 8.2, 2.2 Hz, 1H), 7.42 (dd, *J* = 8.2, 0.7 Hz, 1H), 7.05 - 6.73 (m, 4H), 5.22 (d, *J =* 1.9 Hz, 2H), 4.84 (dt, *J* = 13.6, 2.0 Hz, 1H), 4.59 - 4.50 (m, 1H), 4.01 - 3.98 (m, 1H), 3.23 (td, *J* = 13.3, 2.2 Hz, 1H), 2.67 (td, *J* = 13.1, 2.3 Hz, 1H), 2.37 - 2.21 (m, 2H), 2.15 (s, 3H), 1.96 - 1.87 (m, 2H); **LRMS** (ES) m/z 487.6 (M⁺ + 1).

### Example 61: Synthesis of Compound 61, 3-((5-(5-(difluoromethyl)-1,3,4-oxadiazole-2-yl)pyridine-2-yl)methyl)-5-fluoro-1-(1-(morpholine-4-carbonyl)piperidine-4-yl)-1,3-dihydro-2H-benzo[d]imidazole-2-one

### [Step 1] Synthesis of the compound 61

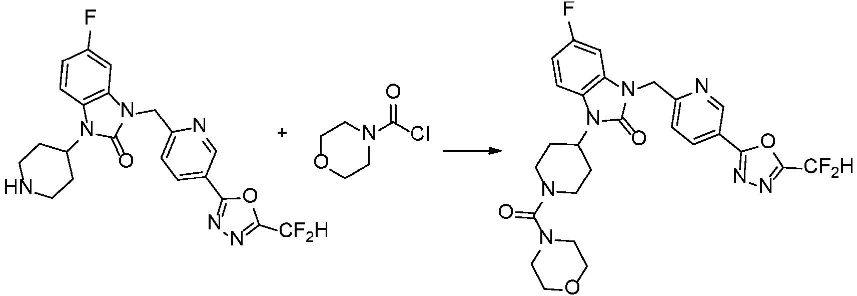

The 3-((5-(5-(difluoromethyl)-1,3,4-oxadiazole-2-yl)pyridine-2-yl)methyl)-5-fluoro-1-(piperidine-4-yl)-1 ,3-dihydro-2H-benzo[d]imidazole-2-one (0.100 g, 0.225 mmol) prepared in the step 5 of the compound 48 was dissolved in dichloromethane (2 mL) at room temperature, after which triethylamine (0.063 mL, 0.450 mmol) was added into the resulting solution and stirred at the same temperature for 10 minutes. Morpholine-4-carbonyl chloride (0.067 g, 0.450 mmol) was added into the reaction mixture and further stirred at the same temperature for 18 hours. Water was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; dichloromethane/methanol = 0 to 5%) and concentrated to obtain a title compound (0.057 g, 45.1%) in a transparent liquid form.

**¹H NMR** (400 MHz, CDCl₃) δ 9.27 (d, *J =* 2.1 Hz, 1H), 8.34 (dd, *J* = 8.2, 2.2 Hz, 1H), 7.43 (d, *J* = 8.2 Hz, 1H), 7.06 - 6.75 (m, 4H), 5.23 (s, 2H), 4.51 - 4.43 (m, 1H), 3.88 (d, *J* = 13.6 Hz, 2H), 3.70 (t, *J* = 4.7 Hz, 4H), 3.31 (t, *J* = 4.7 Hz, 4H), 2.95 (t, *J =* 12.0 Hz, 2H), 2.44 - 2.33 (m, 2H), 1.87 (dd, *J =* 12.2, 2.3 Hz, 2H); **LRMS** (ES) m/z 558.6 (M⁺ + 1).

### Example 62: Synthesis of Compound 62, 3-((5-(5-(difluoromethyl)-1,3,4-oxadiazole-2-yl)pyridine-2-yl)methyl)-5-fluoro-1-(1-(4-(trifluoromet hyl)benzoyl)piperidine-4-yl)-1,3-dihydro-2H-benzo[d]imidazole-2-one

### [Step 1] Synthesis of the compound 62

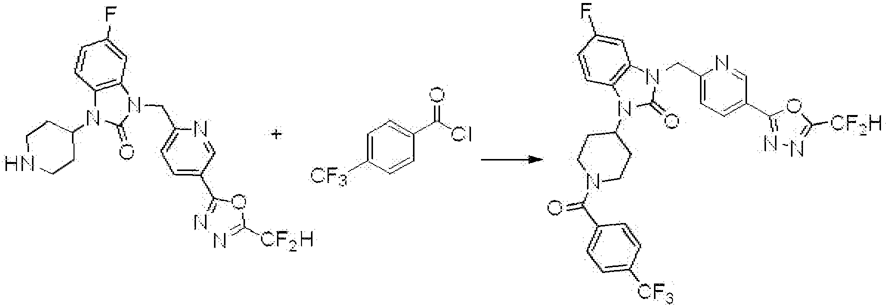

The 3-((5-(5-(difluoromethyl)-1,3,4-oxadiazole-2-yl)pyridine-2-yl)methyl)-5-fluoro-1-(piperidine-4-yl)-1 ,3-dihydro-2H-benzo[d]imidazole-2-one (0.100 g, 0.225 mmol) prepared in the step 5 of the compound 48 was dissolved in dichloromethane (2 mL) at room temperature, after which triethylamine (0.063 mL, 0.450 mmol) was added into the resulting solution and stirred at the same temperature for 10 minutes. 4-(trifluoromethyl)benzoyl chloride (0.094 g, 0.450 mmol) was added into the reaction mixture and further stirred at the same temperature for 18 hours. Water was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; dichloromethane/methanol = 0 to 5%) and concentrated to obtain a title compound (0.106 g, 76.2%) in a yellow liquid form.

**¹H NMR** (400 MHz, CDCl₃) δ 9.26 (d, *J* = 14.6 Hz, 1H), 8.33 (dd, *J* = 8.2, 2.2 Hz, 1H), 7.68, 7.57 (ABq, *J* = 42.1, 8.3 Hz, 4H), 7.43 (d, *J =* 8.2 Hz, 1H), 7.05 - 6.76 (m, 4H), 5.22 (s, 2H), 5.10 - 4.80 (m, 1H), 4.58 - 4.50 (m, 1H), 4.00 - 3.68 (m, 1H), 3.35 - 3.05 (m, 1H), 3.05 - 2.75 (m, 1H), 2.60 - 2.25 (m, 2H), 2.15 - 1.65 (m, 2H); **LRMS** (ES) m/z 617.6 (M⁺ + 1).

### Example 63: Synthesis of Compound 63, 3-((5-(5-(difluoromethyl)-1,3,4-oxadiazole-2-yl)pyridine-2-yl)methyl)-5-fluoro-1-(1-isonicotinoylpip eridine-4-yl)-1,3-dihydro-2H-benzo[d]imidazole-2-one

### [Step 1] Synthesis of the compound 63

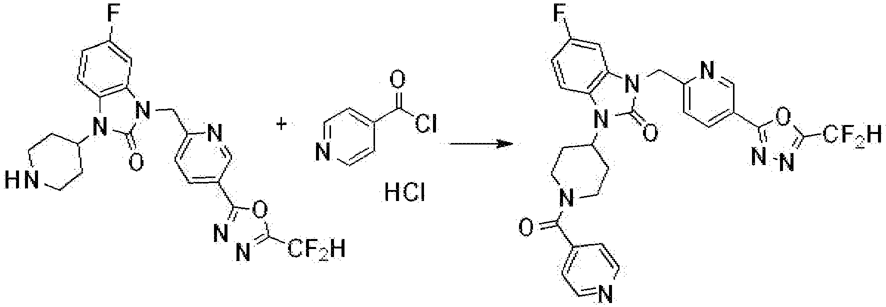

The 3-((5-(5-(difluoromethyl)-1,3,4-oxadiazole-2-yl)pyridine-2-yl)methyl)-5-fluoro-1-(piperidine-4-yl)-1 ,3-dihydro-2H-benzo[d]imidazole-2-one (0.100 g, 0.225 mmol) prepared in the step 5 of the compound 48, triethylamine (0.094 mL, 0.675 mmol) and isonicotinoyl chloride hydrochloride (0.080 g, 0.450 mmol) were dissolved in dichloromethane (2 mL), after which the resulting solution was stirred at room temperature for 10 minutes and further stirred at the same temperature for 18 hours. Water was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; dichloromethane/methanol = 0 to 5%) and concentrated to obtain a title compound (0.074 g, 59.8%) in a transparent liquid form.

**¹H NMR** (400 MHz, CDCl₃) δ 9.24 (d, *J =* 2.2 Hz, 1H), 8.69 (d, *J =* 5.8 Hz, 2H), 8.32 (dd, *J =* 8.2, 2.2 Hz, 1H), 7.42 (d, *J =* 8.2 Hz, 1H), 7.34 - 7.32 (m, 2H), 7.05 - 6.76 (m, 4H), 5.20 (d, *J =* 1.0 Hz, 2H), 4.93 (d, *J =* 11.2 Hz, 1H), 4.56 - 4.48 (m, 1H), 3.80 (d, *J =* 12.4 Hz, 1H), 3.21 (t, *J* = 12.3 Hz, 1H), 2.90 (t, *J =* 11.9 Hz, 1H), 2.49 - 2.34 (m, 2H), 2.01 (d, *J =* 9.7 Hz, 1H), 1.86 (d, *J* = 10.9 Hz, 1H); **LRMS** (ES) m/z 551.6 (M⁺ + 2).

### Example 64: Synthesis of Compound 64, 3-((5-(5-(difluoromethyl)-1,3,4-oxadiazole-2-yl)pyridine-2-yl)methyl)-5-fluoro-1-(1-(pyrimidine-5-c arbonyl)piperidine-4-yl)-1,3-dihydro-2H-benzo[d]imidazole-2-one

### [Step 1] Synthesis of the compound 64

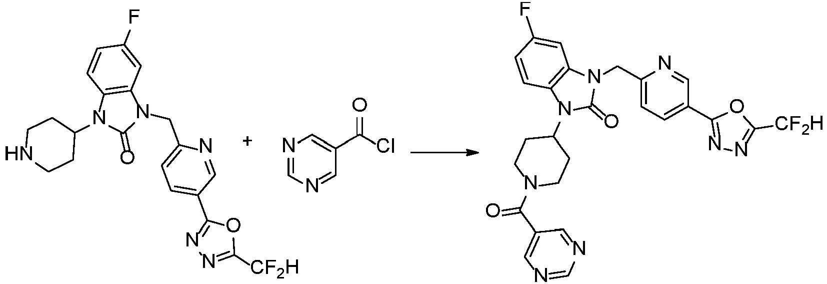

The 3-((5-(5-(difluoromethyl)-1,3,4-oxadiazole-2-yl)pyridine-2-yl)methyl)-5-fluoro-1-(piperidine-4-yl)-1 ,3-dihydro-2H-benzo[d]imidazole-2-one (0.100 g, 0.225 mmol) prepared in the step 5 of the compound 48 was dissolved in dichloromethane (2 mL) at room temperature, after which triethylamine (0.063 mL, 0.450 mmol) was added into the resulting solution and stirred at the same temperature for 10 minutes. Pyrimidine-5-carbonyl chloride (0.064 g, 0.450 mmol) was added into the reaction mixture and further stirred at the same temperature for 18 hours. Water was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; dichloromethane/methanol = 0 to 5%) and concentrated to obtain a title compound (0.065 g, 52.6%) in a yellow liquid form.

**¹H NMR** (400 MHz, CDCl₃) δ 9.25 (s, 1H), 9.24 (dd, *J* = 2.1, 0.6 Hz, 1H), 8.85 (s, 2H), 8.33 (dd, *J* = 8.2, 2.2 Hz, 1H), 7.42 (dd, *J* = 8.2, 0.5 Hz, 1H), 7.04 - 6.75 (m, 4H), 5.21 (s, 2H), 5.08 - 4.75 (m, 1H), 4.59 - 4.51 (m, 1H), 4.00 - 3.75 (m, 1H), 3.50 - 3.15 (m, 1H), 3.10 - 2.85 (m, 1H), 2.60 - 2.33 (m, 2H), 2.20 - 1.80 (m, 2H); **LRMS** (ES) m/z 551.6 (M⁺ + 1).

### Example 65: Synthesis of Compound 65, 3-((5-(5-(difluoromethyl)-1,3,4-oxadiazole-2-yl)pyridine-2-yl)methyl)-5-fluoro-1-(1-(5-methylisoox azole-4-carbonyl)piperidine-4-yl)-1,3-dihydro-2H-benzo[d]imidazole-2-one

### [Step 1] Synthesis of the compound 65

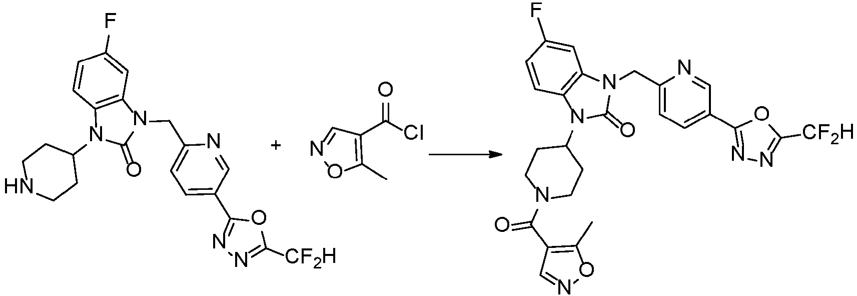

The 3-((5-(5-(difluoromethyl)-1,3,4-oxadiazole-2-yl)pyridine-2-yl)methyl)-5-fluoro-1-(piperidine-4-yl)-1 ,3-dihydro-2H-benzo[d]imidazole-2-one (0.100 g, 0.225 mmol) prepared in the step 5 of the compound 48 was dissolved in dichloromethane (2 mL) at room temperature, after which triethylamine (0.063 mL, 0.450 mmol) was added into the resulting solution and stirred at the same temperature for 10 minutes. 5-methylisooxazole-4-carbonyl chloride (0.065 g, 0.450 mmol) was added into the reaction mixture and further stirred at the same temperature for 18 hours. Water was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; dichloromethane/methanol = 0 to 5%) and concentrated to obtain a title compound (0.036 g, 28.9%) in a white solid form.

**¹H NMR** (400 MHz, CDCl₃) δ 9.27 (d, *J =* 1.6 Hz, 1H), 8.35 (dd, *J =* 8.2, 2.2 Hz, 1H), 7.44 (d, *J* = 8.1 Hz, 1H), 7.05 - 6.76 (m, 4H), 5.23 (s, 2H), 4.82 (d, *J* = 14.0 Hz, 2H), 4.64 - 4.56 (m, 1H), 3.06 (t, *J* = 13.0 Hz, 2H), 2.48 - 2.36 (m, 5H), 2.01 (dd, *J* = 12.8, 2.5 Hz, 2H); **LRMS** (ES) m/z 554.6 (M⁺ + 1).

### Example 66: Synthesis of Compound 66, 3-((5-(5-(difluoromethyl)-1,3,4-oxadiazole-2-yl)pyridine-2-yl)methyl)-5-fluoro-1-(1-(5-methylfuran-2-carbonyl)piperidine-4-yl)-1,3-dihydro-2H-benzo[d]imidazole-2-one

### [Step 1] Synthesis of the compound 66

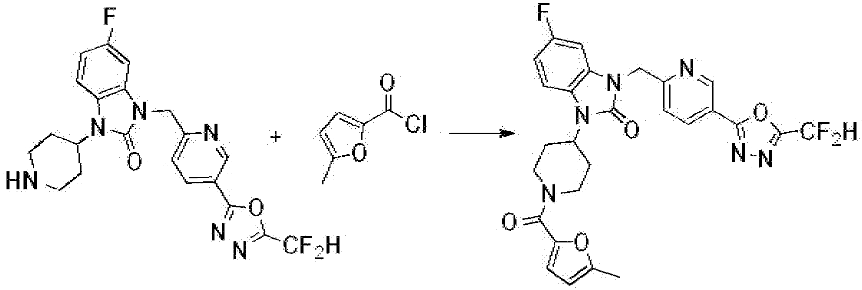

The 3-((5-(5-(difluoromethyl)-1,3,4-oxadiazole-2-yl)pyridine-2-yl)methyl)-5-fluoro-1-(piperidine-4-yl)-1 ,3-dihydro-2H-benzo[d]imidazole-2-one (0.100 g, 0.225 mmol) prepared in the step 5 of the compound 48 was dissolved in dichloromethane (2 mL) at room temperature, after which triethylamine (0.063 mL, 0.450 mmol) was added into the resulting solution and stirred at the same temperature for 10 minutes. 5-methylfuran-2-carbonyl chloride (0.065 g, 0.450 mmol) was added into the reaction mixture and further stirred at the same temperature for 18 hours. Water was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; dichloromethane/methanol = 0 to 5%) and concentrated to obtain a title compound (0.089 g, 71.8%) in a brown liquid form.

**¹H NMR** (400 MHz, CDCl₃) δ 9.25 (dd, *J* = 2.2, 0.8 Hz, 1H), 8.32 (dd, *J* = 8.2, 2.2 Hz, 1H), 7.42 (dd, *J* = 8.2, 0.7 Hz, 1H), 7.05 - 6.72 (m, 5H), 6.08 (dd, *J* = 3.4, 1.0 Hz, 1H), 5.22 (s, 2H), 4.79 (d, *J =* 13.6 Hz, 2H), 4.67 - 4.58 (m, 1H), 3.20 - 2.95 (m, 2H), 2.43 - 2.34 (m, 5H), 1.95 (dd, *J* = 12.1, 2.2 Hz, 2H); **LRMS** (ES) m/z 553.6 (M⁺ + 1).

### Example 67: Synthesis of Compound 67, 3-((5-(5-(difluoromethyl)-1,3,4-oxadiazole-2-yl)pyridine-2-yl)methyl)-5-fluoro-1-(1-(3-methoxypro panoyl)piperidine-4-yl)-1,3-dihydro-2H-benzo[d]imidazole-2-one

### [Step 1] Synthesis of the compound 67

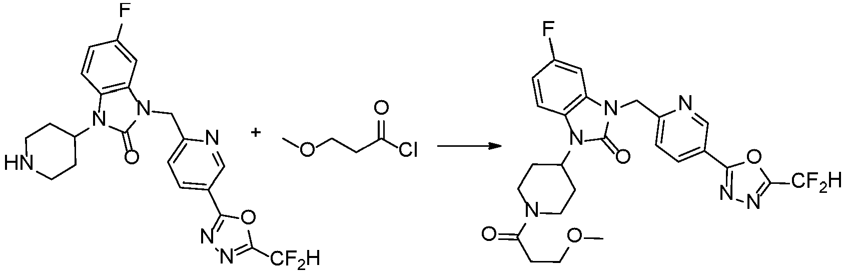

The 3-((5-(5-(difluoromethyl)-1,3,4-oxadiazole-2-yl)pyridine-2-yl)methyl)-5-fluoro-1-(piperidine-4-yl)-1 ,3-dihydro-2H-benzo[d]imidazole-2-one (0.100 g, 0.225 mmol) prepared in the step 5 of the compound 48 was dissolved in dichloromethane (2 mL) at room temperature, after which triethylamine (0.063 mL, 0.450 mmol) was added into the resulting solution and stirred at the same temperature for 10 minutes. 3-methoxypropanoyl chloride (0.055 g, 0.450 mmol) was added into the reaction mixture and further stirred at the same temperature for 18 hours. Water was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; dichloromethane/methanol = 0 to 5%) and concentrated to obtain a title compound (0.065 g, 54.5%) in a white solid form.

**¹H NMR** (400 MHz, CDCl₃) δ 9.23 (t, *J =* 1.1 Hz, 1H), 8.30 (dd, *J =* 8.2, 2.2 Hz, 1H), 7.40 (d, *J* = 8.2 Hz, 1H), 7.04 - 6.69 (m, 4H), 5.20 (s, 2H), 4.84 (d, *J* = 13.3 Hz, 2H), 4.58 - 4.51 (m, 1H), 4.08 (d, *J* = 13.5 Hz, 2H), 3.76 - 3.65 (m, 2H), 3.34 (s, 3H), 3.18 (t, *J* = 8.3 Hz, 2H), 2.74 - 2.54 (m, 3H), 2.35 - 2.19 (m, 2H), 1.88 (t, *J =* 11.5 Hz, 2H); LRMS (ES) m/z 531.6 (M⁺ + 1).

### Example 68: Synthesis of Compound 68, Methyl 4-(3-((5-(5-(difluoromethyl)-1,3,4-oxadiazole-2-yl)pyridine-2-yl)methyl)-5-fluoro-2-oxo-2,3-dihydr o-1H-benzo[d]imidazole-1-yl)piperidine-1-carboxylate

### [Step 1] Synthesis of the compound 68

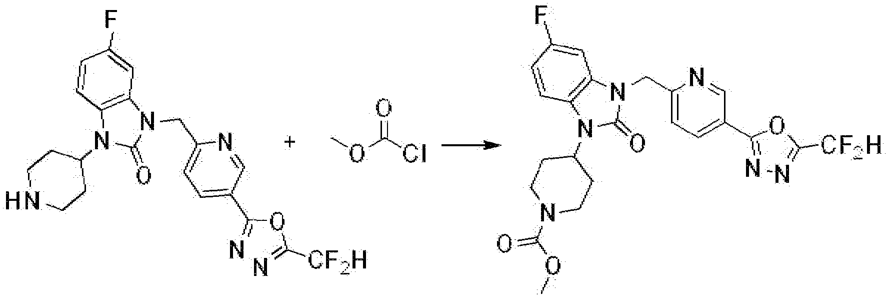

The 3-((5-(5-(difluoromethyl)-1,3,4-oxadiazole-2-yl)pyridine-2-yl)methyl)-5-fluoro-1-(piperidine-4-yl)-1 ,3-dihydro-2H-benzo[d]imidazole-2-one (0.100 g, 0.225 mmol) prepared in the step 5 of the compound 48 was dissolved in dichloromethane (2 mL) at room temperature, after which triethylamine (0.063 mL, 0.450 mmol) was added into the resulting solution and stirred at the same temperature for 10 minutes. Methyl carbonochloridate (0.043 g, 0.450 mmol) was added into the reaction mixture and further stirred at the same temperature for 18 hours. Water was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.034 g, 30.0%) in a white solid form.

**¹H NMR** (400 MHz, CDCl₃) δ 9.27 (d, *J =* 2.0 Hz, 1H), 8.33 (dd, *J* = 8.2, 2.2 Hz, 1H), 7.42 (d, *J* = 8.2 Hz, 1H), 7.05 - 6.74 (m, 4H), 5.23 (s, 2H), 4.54 - 4.20 (m, 3H), 3.73 (s, 3H), 3.05 - 2.80 (m, 2H), 2.35 - 2.25 (m, 2H), 1.87 (d, *J =* 11.4 Hz, 2H); **LRMS** (ES) m/z 503.6 (M⁺ + 1).

### Example 69: Synthesis of Compound 69, 3-(4-(5-(difluoromethyl)-1,3,4-oxadiazole-2-yl)-2-fluorobenzyl)-5-fluoro-1-(1-methylpiperidine-4-yl )-1,3-dihydro-2H-benzo[d]imidazole-2-one

### [Step 1] Synthesis of tert-butyl 4-(3-(4-(5-(difluoromethyl)-1,3,4-oxadiazole-2-yl)-2-fluorobenzyl)-5-fluoro-2-oxo-2,3-dihydro-1H-benzo[d]imidazole-1-yl)piperidine-1-carboxylate

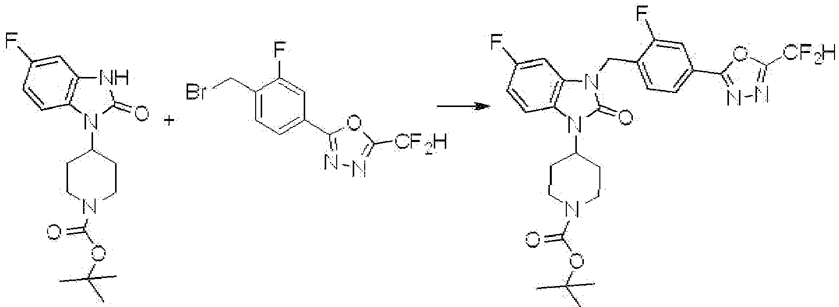

The tert-butyl 4-(5-fluoro-2-oxo-2,3-dihydro-1H-benzo[d]imidazole-1-yl)piperidine-1-carboxylate (1.300 g, 3.876 mmol) prepared in the step 3 of the compound 48 was dissolved in N,N-dimethylformamide (40 mL) at 0°C, after which sodium hydride (60.00%, 0.202 g, 5.039 mmol) was added into the resulting solution and stirred at the same temperature for 30 minutes. 2-(4-(bromomethyl)-3-fluorophenyl)-5-(difluoromethyl)-1,3,4-oxadiazole (1.547 g, 5.039 mmol) was added into the reaction mixture and further stirred at room temperature for 4 hours. Water was poured into the reaction mixture and an extraction was performed with ethyl acetate. An organic layer was washed with saturated sodium chloride aqueous solution, then dehydrated with anhydrous magnesium sulfate, then filtered, and then concentrated under reduced pressure. The resulting concentrate was purified via column chromatography (SiO₂, 40 g cartridge; ethyl acetate/hexane = 0 to 60%) and concentrated to obtain a title compound (1.420 g, 65.2%) in a light brown solid form.

### [Step 2] Synthesis of 3-(4-(5-(difluoromethyl)-1,3,4-oxadiazole-2-yl)-2-fluorobenzyl)-5-fluoro-1-(piperidine-4-yl)-1,3-dih ydro-2H-benzo[d]imidazole-2-one

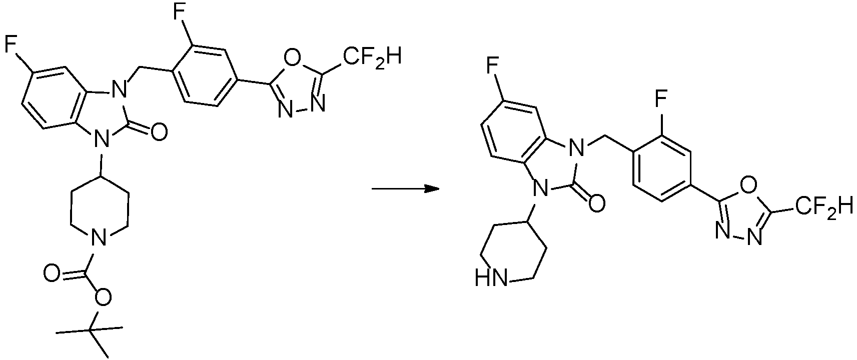

The tert-butyl 4-(3-(4-(5-(difluoromethyl)-1,3,4-oxadiazole-2-yl)-2-fluorobenzyl)-5-fluoro-2-oxo-2,3-dihydro-1H-benzo[d]imidazole-1-yl)piperidine-1-carboxylate (1.420 g, 2.529 mmol) prepared in the step 1 was dissolved in dichloromethane (15 mL) at 0°C, after which trifluoroacetic acid (1.936 mL, 25.288 mmol) was added into the resulting solution and stirred at room temperature for 5 hours. Saturated sodium hydrogen carbonate aqueous solution was poured into the reaction mixture and an extraction was performed with dichloromethane. An organic layer was washed with saturated sodium chloride aqueous solution, then dehydrated with anhydrous magnesium sulfate, then filtered, and then concentrated under reduced pressure. The resulting concentrate was purified via column chromatography (SiO₂, 40 g cartridge; ethyl acetate/hexane = 50 to 100%) and concentrated to obtain a product, after which the resulting product was purified again via chromatography (SiO₂, 40 g cartridge; dichloromethane/methanol = 0 to 30%) and concentrated to obtain a title compound (0.940 g, 80.6%) in a light yellow solid form.

### [Step 3] Synthesis of the compound 69

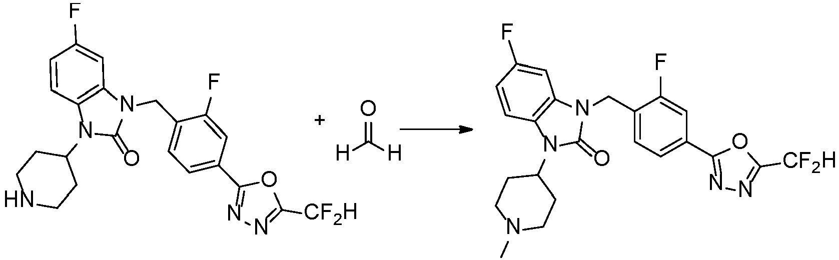

The 3-(4-(5-(difluoromethyl)-1,3,4-oxadiazole-2-yl)-2-fluorobenzyl)-5-fluoro-1-(piperidine-4-yl)-1,3-dih ydro-2H-benzo[d]imidazole-2-one (0.100 g, 0.217 mmol) prepared in the step 2, sodium triacetoxyborohydride (0.092 g, 0.433 mmol), acetic acid (0.012 mL, 0.217 mmol) and formaldehyde (0.013 g, 0.433 mmol) were dissolved in dichloromethane (2 mL) at room temperature, after which the resulting solution was stirred at the same temperature for 18 hours. Water was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; dichloromethane/methanol = 0 to 5%) and concentrated to obtain a title compound (0.037 g, 35.7%) in a white solid form.

**¹H NMR** (400 MHz, CDCl₃) δ 7.85 - 7.82 (m, 2H), 7.44 (t, *J* = 7.8 Hz, 1H), 7.23 (dd, *J* = 8.7, 4.3 Hz, 1H), 7.03 - 6.68 (m, 3H), 5.14 (s, 2H), 4.44 - 4.36 (m, 1H), 3.02 (d, *J* = 11.7 Hz, 2H), 2.51 - 2.40 (m, 2H), 2.34 (s, 3H), 2.16 (td, *J =* 12.0, 2.1 Hz, 2H), 1.84 - 1.80 (m, 2H); **LRMS** (ES) m/z 476.4 (M⁺ + 1).

### Example 70: Synthesis of Compound 70, 3-(4-(5-(difluoromethyl)-1,3,4-oxadiazole-2-yl)-2-fluorobenzyl)-5-fluoro-1-(1-isopropylpiperidine-4 -yl)-1,3-dihydro-2H-benzo[d]imidazole-2-one

### [Step 1] Synthesis of the compound 70

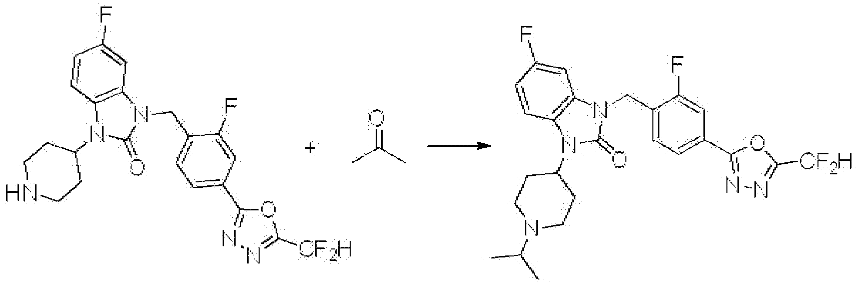

The 3-(4-(5-(difluoromethyl)-1,3,4-oxadiazole-2-yl)-2-fluorobenzyl)-5-fluoro-1-(piperidine-4-yl)-1,3-dih ydro-2H-benzo[d]imidazole-2-one (0.100 g, 0.217 mmol) prepared in the step 2 of the compound 69, sodium triacetoxyborohydride (0.092 g, 0.433 mmol), acetic acid (0.012 mL, 0.217 mmol) and propane-2-one (0.025 g, 0.433 mmol) were dissolved in dichloromethane (2 mL) at room temperature, after which the resulting solution was stirred at the same temperature for 18 hours. Water was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; dichloromethane/methanol = 0 to 5%) and concentrated to obtain a title compound (0.070 g, 63.9%) in a white solid form.

**¹H NMR** (400 MHz, CDCl₃) δ 7.85 - 7.82 (m, 2H), 7.44 (t, *J* = 7.8 Hz, 1H), 7.28 (dd, J = 9.0, 4.6 Hz, 1H), 6.90 - 6.68 (m, 3H), 5.28 (s, 2H), 4.40 - 4.33 (m, 1H), 3.02 (d, *J* = 9.5 Hz, 2H), 2.84-2.78 (m, 1H), 2.46 - 2.31 (m, 4H), 1.84 (d, *J =* 11.4 Hz, 2H), 1.07 (d, *J =* 6.6 Hz, 6H); **LRMS** (ES) m/z 504.6 (M⁺ + 1).

### Example 71: Synthesis of Compound 71, 3-(4-(5-(difluoromethyl)-1,3,4-oxadiazole-2-yl)-2-fluorobenzyl)-5-fluoro-1-(1-(oxetan-3-yl)piperidi ne-4-yl)-1,3-dihydro-2H-benzo[d]imidazole-2-one

### [Step 1] Synthesis of the compound 71

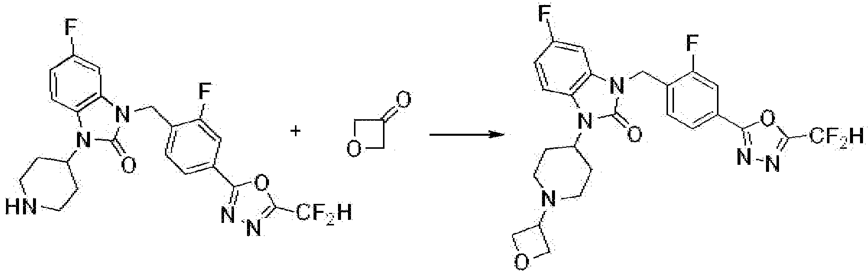

The 3-(4-(5-(difluoromethyl)-1,3,4-oxadiazole-2-yl)-2-fluorobenzyl)-5-fluoro-1-(piperidine-4-yl)-1,3-dih ydro-2H-benzo[d]imidazole-2-one (0.100 g, 0.217 mmol) prepared in the step 2 of the compound 69, sodium triacetoxyborohydride (0.092 g, 0.433 mmol), acetic acid (0.012 mL, 0.217 mmol) and oxetan-3-one (0.031 g, 0.433 mmol) were dissolved in dichloromethane (2 mL) at room temperature, after which the resulting solution was stirred at the same temperature for 18 hours. Water was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; dichloromethane/methanol = 0 to 5%) and concentrated to obtain a title compound (0.077 g, 68.9%) in a white solid form.

**¹H NMR** (400 MHz, CDCl₃) δ 7.84 - 7.82 (m, 2H), 7.44 (t, *J* = 7.8 Hz, 1H), 7.23 (dd, J = 8.7, 4.3 Hz, 1H), 7.02 - 6.69 (m, 3H), 5.13 (s, 2H), 4.64 (dt, *J* = 20.7, 6.4 Hz, 4H), 4.45 - 4.36 (m, 1H), 3.56 - 3.49 (m, 1H), 2.90 (d, *J* = 11.5 Hz, 2H), 2.50 - 2.39 (m, 2H), 2.02 (t, *J* = 10.9 Hz, 2H), 1.85 (dd, *J =* 12.1, 2.3 Hz, 2H); **LRMS** (ES) m/z 518.6 (M⁺ + 1).

### Example 72: Synthesis of Compound 72, 3-(4-(5-(difluoromethyl)-1,3,4-oxadiazole-2-yl)-2-fluorobenzyl)-5-fluoro-1-(1-(tetrahydro-2H-pyra n-4-yl)piperidine-4-yl)-1,3-dihydro-2H-benzo[d]imidazole-2-one

### [Step 1] Synthesis of the compound 72

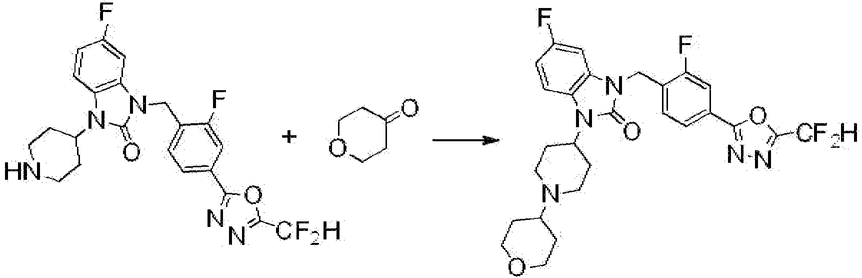

The 3-(4-(5-(difluoromethyl)-1,3,4-oxadiazole-2-yl)-2-fluorobenzyl)-5-fluoro-1-(piperidine-4-yl)-1,3-dih ydro-2H-benzo[d]imidazole-2-one (0.100 g, 0.217 mmol) prepared in the step 2 of the compound 69, sodium triacetoxyborohydride (0.092 g, 0.433 mmol), acetic acid (0.012 mL, 0.217 mmol) and tetrahydro-4H-pyran-4-one (0.043 g, 0.433 mmol) were dissolved in dichloromethane (2 mL) at room temperature, after which the resulting solution was stirred at the same temperature for 18 hours. Water was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; dichloromethane/methanol = 0 to 5%) and concentrated to obtain a title compound (0.048 g, 40.4%) in a white solid form.

**¹H NMR** (400 MHz, CDCl₃) δ 7.84 - 7.82 (m, 2H), 7.44 (t, *J* = 7.8 Hz, 1H), 7.23 (dd, J = 8.7, 4.3 Hz, 1H), 7.02 - 6.69 (m, 3H), 5.13 (s, 2H), 4.64 (dt, *J* = 20.7, 6.4 Hz, 4H), 4.45 - 4.36 (m, 1H), 3.56 - 3.49 (m, 1H), 2.90 (d, *J* = 11.5 Hz, 2H), 2.50 - 2.39 (m, 2H), 2.02 (t, *J* = 10.9 Hz, 2H), 1.85 (dd, *J =* 12.1, 2.3 Hz, 2H); **LRMS** (ES) m/z 546.6 (M⁺ + 1).

### Example 73: Synthesis of Compound 73, 3-(4-(5-(difluoromethyl)-1,3,4-oxadiazole-2-yl)-2-fluorobenzyl)-5-fluoro-1-(1-(tetrahydro-2H-thiop yran-4-yl)piperidine-4-yl)-1,3-dihydro-2H-benzo[d]imidazole-2-one

### [Step 1] Synthesis of the compound 73

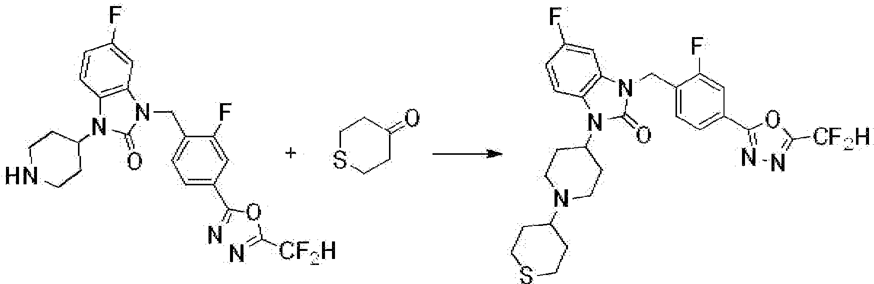

The 3-(4-(5-(difluoromethyl)-1,3,4-oxadiazole-2-yl)-2-fluorobenzyl)-5-fluoro-1-(piperidine-4-yl)-1,3-dih ydro-2H-benzo[d]imidazole-2-one (0.100 g, 0.217 mmol) prepared in the step 2 of the compound 69, sodium triacetoxyborohydride (0.092 g, 0.433 mmol), acetic acid (0.012 mL, 0.217 mmol) and tetrahydro-4H-thiopyran-4-one (0.050 g, 0.433 mmol) were dissolved in dichloromethane (2 mL) at room temperature, after which the resulting solution was stirred at the same temperature for 18 hours. Water was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; dichloromethane/methanol = 0 to 5%) and concentrated to obtain a title compound (0.054 g, 44.4%) in a white solid form.

**¹H NMR** (400 MHz, CDCl₃) δ 7.85 - 7.82 (m, 2H), 7.44 (t, *J* = 7.8 Hz, 1H), 7.22 (dd, *J* = 8.6, 4.2 Hz, 1H), 7.03 - 6.68 (m, 3H), 5.14 (s, 2H), 4.38 - 4.30 (m, 1H), 2.97 (d, *J* = 11.0 Hz, 2H), 2.71-2.69 (m, 4H), 2.53 - 2.33 (m, 5H), 2.16 - 2.13 (m, 2H), 1.83 (dd, *J* = 11.6, 2.3 Hz, 2H), 1.77 - 1.67 (m, 2H); **LRMS** (ES) m/z 562.6 (M⁺ + 1).

### Example 74: Synthesis of Compound 74, 1-(1-cyclobutylpiperidine-4-yl)-3-(4-(5-(difluoromethyl)-1,3,4-oxadiazole-2-yl)-2-fluorobenzyl)-5-fl uoro-1,3-dihydro-2H-benzo[d]imidazole-2-one

### [Step 1] Synthesis of the compound 74

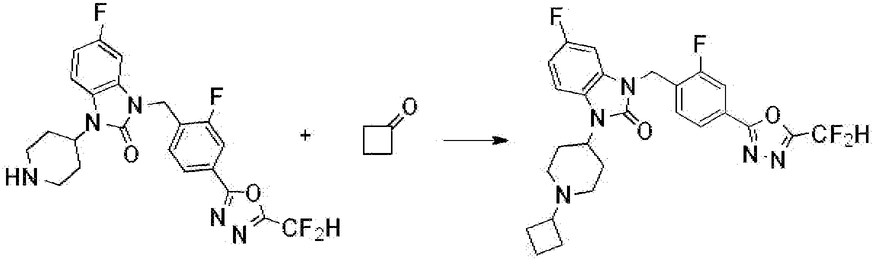

The 3-(4-(5-(difluoromethyl)-1,3,4-oxadiazole-2-yl)-2-fluorobenzyl)-5-fluoro-1-(piperidine-4-yl)-1,3-dih ydro-2H-benzo[d]imidazole-2-one (0.100 g, 0.217 mmol) prepared in the step 2 of the compound 69, sodium triacetoxyborohydride (0.092 g, 0.433 mmol), acetic acid (0.012 mL, 0.217 mmol) and cyclobutanone (0.030 g, 0.433 mmol) were dissolved in dichloromethane (2 mL) at room temperature, after which the resulting solution was stirred at the same temperature for 18 hours. Water was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; dichloromethane/methanol = 0 to 5%) and concentrated to obtain a title compound (0.079 g, 70.6%) in a white solid form.

**¹H NMR** (400 MHz, CDCl₃) δ 7.81 (d, *J =* 8.6 Hz, 2H), 7.47 - 7.40 (m, 2H), 7.03 - 6.77 (m, 2H), 6.68 (dd, *J* = 8.3, 2.5 Hz, 1H), 5.12 (s, 2H), 4.56 - 4.48 (m, 1H), 3.29 (d, *J* = 11.7 Hz, 2H), 3.06 - 2.98 (m, 1H), 2.76 - 2.67 (m, 2H), 2.28 - 2.20 (m, 4H), 2.15 - 2.08 (m, 2H), 1.89 - 1.63 (m, 4H); **LRMS** (ES) m/z 516.5 (M⁺ + 1).

### Example 75: Synthesis of Compound 75, 3-(4-(5-(difluoromethyl)-1,3,4-oxadiazole-2-yl)-2-fluorobenzyl)-5-fluoro-1-(1-(tetrahydro-2H-thiop yran-4-yl)piperidine-4-yl)-1,3-dihydro-2H-benzo[d]imidazole-2-one

### [Step 1] Synthesis of the compound 75

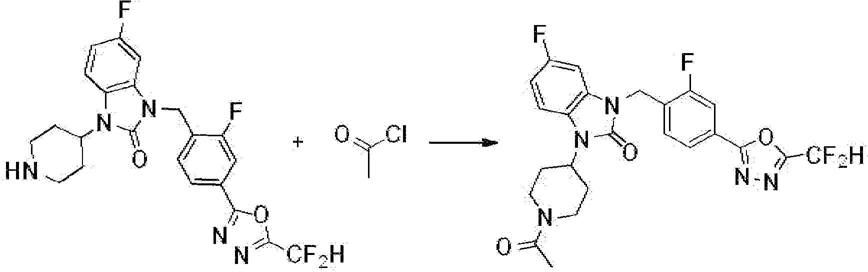

The 3-(4-(5-(difluoromethyl)-1,3,4-oxadiazole-2-yl)-2-fluorobenzyl)-5-fluoro-1-(piperidine-4-yl)-1,3-dih ydro-2H-benzo[d]imidazole-2-one (0.100 g, 0.217 mmol) prepared in the step 2 of the compound 69, triethylamine (0.060 mL, 0.433 mmol) and acetyl chloride (0.023 mL, 0.325 mmol) were dissolved in dichloromethane (2 mL) at room temperature, after which the resulting solution was stirred at the same temperature for 18 hours. Water was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; dichloromethane/methanol = 0 to 5%) and concentrated to obtain a title compound (0.101 g, 92.9%) in a white solid form.

**¹H NMR** (400 MHz, CDCl₃) δ 7.82 - 7.79 (m, 2H), 7.44 (t, *J* = 7.7 Hz, 1H), 7.01 (dd, *J* = 8.2, 3.9 Hz, 1H), 6.99 - 6.69 (m, 3H), 5.12 (s, 2H), 4.83 (d, *J* = 13.6 Hz, 1H), 4.55 - 4.47 (m, 1H), 3.99 (d, *J =* 13.8 Hz, 1H), 3.24 - 3.18 (m, 1H), 2.67 - 2.61 (m, 1H), 2.36 - 2.20 (m, 2H), 2.13 (s, 3H), 1.89 (t, *J =* 11.4 Hz, 2H); **LRMS** (ES) m/z 504.6 (M⁺ + 1).

### Example 76: Synthesis of Compound 76, 3-((5-(5-(difluoromethyl)-1,3,4-oxadiazole-2-yl)pyridine-2-yl)methyl)-4-fluoro-1-(1-methylpiperidin e-4-yl)-1,3-dihydro-2H-benzo[d]imidazole-2-one

### [Step 1] Synthesis of tert-butyl 4-((3-fluoro-2-nitrophenyl)amino)piperidine-1-carboxylate

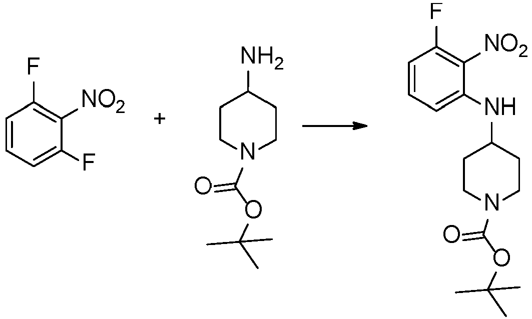

1,3-difluoro-2-nitrobenzene (3.000 g, 18.857 mmol), tert-butyl 4-aminopiperidine-1-carboxylate (3.777 g, 18.857 mmol), potassium carbonate (3.127 g, 22.629 mmol) and potassium iodide (0.031 g, 0.189 mmol) were dissolved in N,N-dimethylformamide (150 mL) at room temperature, after which the resulting solution was heated under reflux for 18 hours, and then a reaction was finished by lowering a temperature to room temperature. Solvent was removed from the reaction mixture under reduced pressure, after which water was poured into the resulting concentrate, and then an extraction was performed with ethyl acetate. An organic layer was washed with saturated sodium chloride aqueous solution, then dehydrated with anhydrous magnesium sulfate, then filtered, and then concentrated under reduced pressure. The resulting concentrate was purified via column chromatography (SiO₂, 40 g cartridge; ethyl acetate/hexane = 0 to 40%) and concentrated to obtain a title compound (4.300 g, 67.2%) in a red solid form.

### [Step 2] Synthesis of tert-butyl 4-((2-amino-3-fluorophenyl)amino)piperidine-1-carboxylate

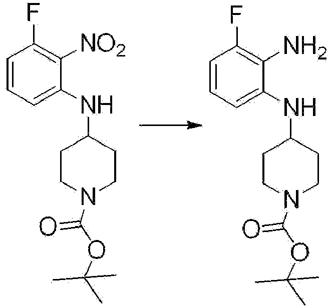

The tert-butyl 4-((3-fluoro-2-nitrophenyl)amino)piperidine-1-carboxylate (4.300 g, 12.671 mmol) prepared in the step 1 was dissolved in methanol (120 mL) at room temperature, after which the resulting solution was stirred at the same temperature for 18 hours in the presence of a hydrogen balloon. The reaction mixture was filtered via celite to remove a solid therefrom, after which solvent was removed from the resulting filtrate under reduced pressure. Then, the resulting concentrate was purified via column chromatography (SiO₂, 40 g cartridge; ethyl acetate/hexane = 0 to 70%) and concentrated to obtain a title compound (3.260 g, 83.2%) in a yellow solid form.

### [Step 3] Synthesis of tert-butyl 4-(4-fluoro-2-oxo-2,3-dihydro-1H-benzo[d]imidazole-1-yl)piperidine-1-carboxylate

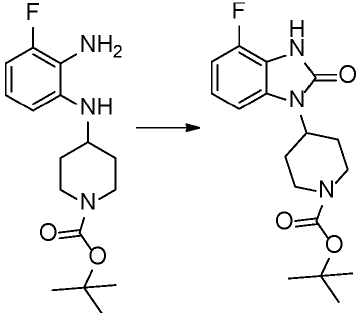

The tert-butyl 4-((2-amino-3-fluorophenyl)amino)piperidine-1-carboxylate (3.260 g, 10.537 mmol) prepared in the step 2 was dissolved in N,N-dimethylformamide (100 mL) at 0°C, after which 1,1'-carbonyldiimidazole (CDI, 1.879 g, 11.591 mmol) was added into the resulting solution and stirred at room temperature for 18 hours. Water was poured into the reaction mixture and an extraction was performed with dichloromethane. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 80 g cartridge; ethyl acetate/hexane = 0 to 70%) and concentrated to obtain a title compound (2.320 g, 65.7%) in a violet solid form.

### [Step 4] Synthesis of tert-butyl 4-(4-fluoro-2-oxo-2,3-dihydro-1H-benzo[d]imidazole-1-yl)piperidine-1-carboxylate

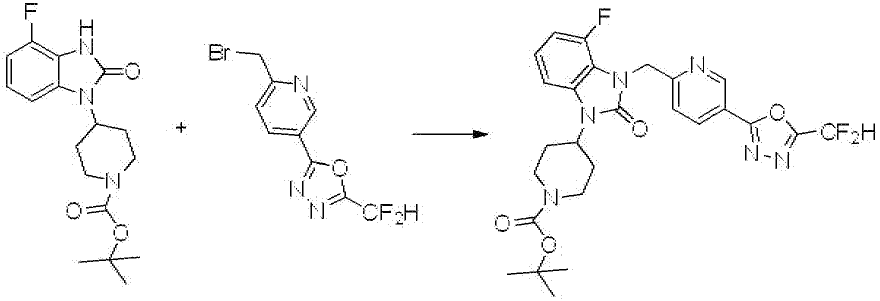

The tert-butyl 4-((2-amino-3-fluorophenyl)amino)piperidine-1-carboxylate (3.260 g, 10.537 mmol) prepared in the step 3 was dissolved in N,N-dimethylformamide (100 mL) at 0°C, after which 1,1'-carbonyldiimidazole (CDI, 1.879 g, 11.591 mmol) was added into the resulting solution and stirred at room temperature for 18 hours. Water was poured into the reaction mixture and an extraction was performed with dichloromethane. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 80 g cartridge; ethyl acetate/hexane = 0 to 70%) and concentrated to obtain a title compound (2.320 g, 65.7%) in a violet solid form.

### [Step 5] Synthesis of 3-((5-(5-(difluoromethyl)-1,3,4-oxadiazole-2-yl)pyridine-2-yl)methyl)-4-fluoro-1-(piperidine-4-yl)-1 ,3-dihydro-2H-benzo[d]imidazole-2-one

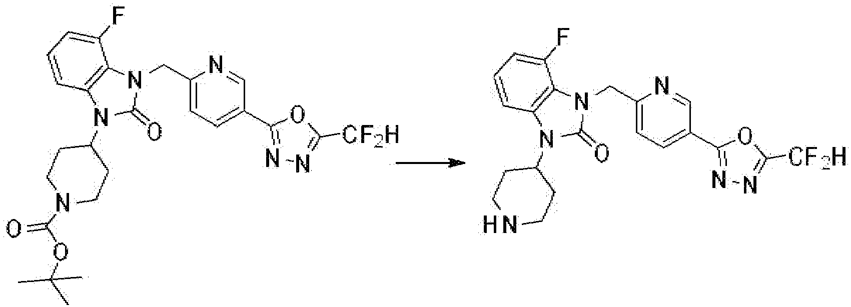

The tert-butyl 4-(3-((5-(5-(difluoromethyl)-1,3,4-oxadiazole-2-yl)pyridine-2-yl)methyl)-4-fluoro-2-oxo-2,3-dihydr o-1H-benzo[d]imidazole-1-yl)piperidine-1-carboxylate (2.650 g, 4.866 mmol) prepared in the step 4 was dissolved in dichloromethane (50 mL) at 0°C, after which trifluoroacetic acid (7.453 mL, 97.330 mmol) was added into the resulting solution and stirred at room temperature for 5 hours. Saturated sodium hydrogen carbonate aqueous solution was poured into the reaction mixture and an extraction was performed with dichloromethane. An organic layer was washed with saturated sodium chloride aqueous solution, then dehydrated with anhydrous magnesium sulfate, then filtered, and then concentrated under reduced pressure. The resulting concentrate was purified via column chromatography (SiO₂, 40 g cartridge; methanol/dichloromethane = 0 to 50%) and concentrated to obtain a title compound (1.800 g, 83.2%) in a brown solid form.

### [Step 6] Synthesis of the compound 76

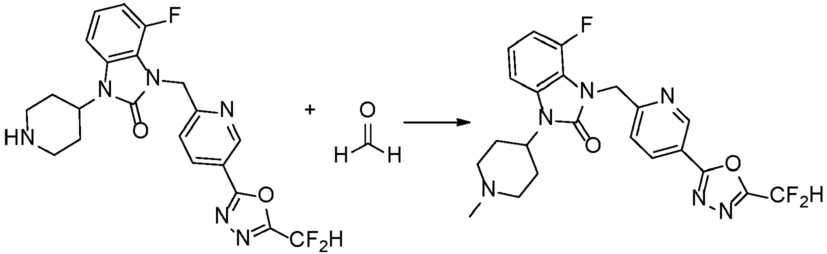

The 3-((5-(5-(difluoromethyl)-1,3,4-oxadiazole-2-yl)pyridine-2-yl)methyl)-4-fluoro-1-(piperidine-4-yl)-1 ,3-dihydro-2H-benzo[d]imidazole-2-one (0.100 g, 0.225 mmol) prepared in the step 5, sodium triacetoxyborohydride (0.095 g, 0.450 mmol), acetic acid (0.013 mL, 0.225 mmol) and formaldehyde (0.014 g, 0.450 mmol) were dissolved in dichloromethane (2 mL) at room temperature, after which the resulting solution was stirred at the same temperature for 18 hours. Water was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; dichloromethane/methanol = 0 to 5%) and concentrated to obtain a title compound (0.040 g, 39.0%) in a white solid form.

**¹H NMR** (400 MHz, CDCl₃) δ 9.24 (d, *J =* 2.1 Hz, 1H), 8.31 (dd, *J* = 8.2, 2.2 Hz, 1H), 7.32 (d, *J =* 8.2 Hz, 1H), 7.14 (d, *J* = 8.0 Hz, 1H), 7.04 - 6.72 (m, 3H), 5.42 (s, 2H), 4.47 - 4.38 (m, 1H), 3.02 (d, *J =* 11.6 Hz, 2H), 2.54 - 2.44 (m, 2H), 2.34 (s, 3H), 2.19 - 2.13 (m, 2H), 1.87 - 1.83 (m, 2H); **LRMS** (ES) m/z 459.3 (M⁺+ 1).

### Example 77: Synthesis of Compound 77, 3-((5-(5-(difluoromethyl)-1,3,4-oxadiazole-2-yl)pyridine-2-yl)methyl)-4-fluoro-1-(1-(oxetan-3-yl)pi peridine-4-yl)-1,3-dihydro-2H-benzo[d]imidazole-2-one

### [Step 1] Synthesis of the compound 77

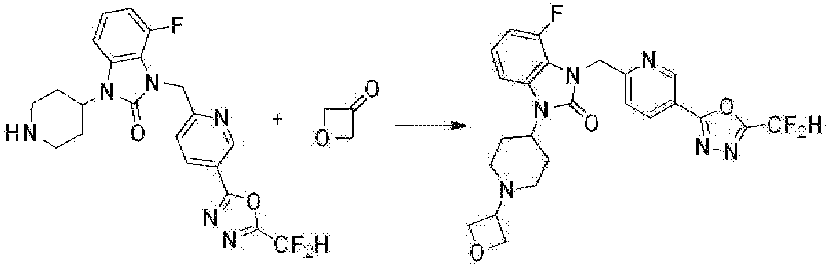

The 3-((5-(5-(difluoromethyl)-1,3,4-oxadiazole-2-yl)pyridine-2-yl)methyl)-4-fluoro-1-(piperidine-4-yl)-1 ,3-dihydro-2H-benzo[d]imidazole-2-one (0.100 g, 0.225 mmol) prepared in the step 5 of the compound 76, sodium triacetoxyborohydride (0.095 g, 0.450 mmol), acetic acid (0.013 mL, 0.225 mmol) and oxetan-3-one (0.032 g, 0.450 mmol) were dissolved in dichloromethane (2 mL) at room temperature, after which the resulting solution was stirred at the same temperature for 18 hours. Water was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; dichloromethane/methanol = 0 to 5%) and concentrated to obtain a title compound (0.047 g, 41.6%) in a white solid form.

**¹H NMR** (400 MHz, CDCl₃) δ 9.22 (d, *J =* 2.0 Hz, 1H), 8.31 (dd, *J* = 8.2, 2.2 Hz, 1H), 7.32 (d, *J* = 8.2 Hz, 1H), 7.15 (d, *J =* 7.9 Hz, 1H), 7.04 - 6.72 (m, 3H), 5.41 (s, 2H), 4.64 (dt, *J =* 22.3, 6.4 Hz, 4H), 4.47 - 4.39 (m, 1H), 3.55 - 3.49 (m, 1H), 2.90 (d, *J* = 11.5 Hz, 2H), 2.53 - 2.43 (m, 2H), 2.01 (td, *J =* 11.8, 1.9 Hz, 2H), 1.89 - 1.85 (m, 2H); **LRMS** (ES) m/z 501.6 (M⁺ + 1).

### Example 78: Synthesis of Compound 78, 1-((5-(5-(difluoromethyl)-1,3,4-oxadiazole-2-yl)pyridine-2-yl)methyl)-3-(1-(oxetan-3-yl)piperidine-4-yl)-1,3-dihydro-2H-benzo[d]imidazole-2-one

### [Step 1] Synthesis of the compound 78

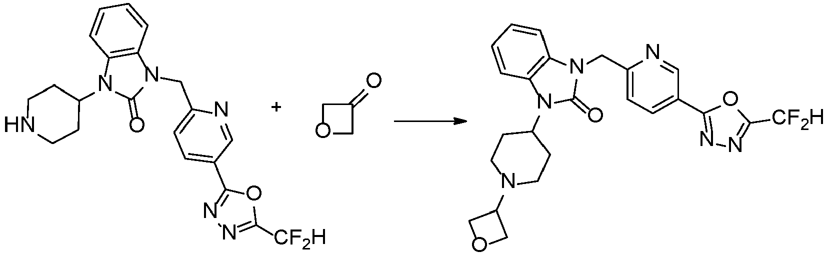

1-((5-(5-(difluoromethyl)-1,3,4-oxadiazole-2-yl)pyridine-2-yl)methyl)-3-(piperidine-4-yl)-1, 3-dihydro-2H-benzo[d]imidazole-2-one (0.100 g, 0.235 mmol), sodium triacetoxyborohydride (0.099 g, 0.469 mmol), acetic acid (0.013 mL, 0.235 mmol) and oxetan-3-one (0.034 g, 0.469 mmol) were dissolved in dichloromethane (2 mL) at room temperature, after which the resulting solution was stirred at the same temperature for 18 hours. Water was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; dichloromethane/methanol = 0 to 5%) and concentrated to obtain a title compound (0.053 g, 46.8%) in a white solid form.

**¹H NMR** (400 MHz, CDCl₃) δ 9.25 (dd, *J =* 2.2, 0.7 Hz, 1H), 8.28 (dd, *J* = 8.2, 2.2 Hz, 1H), 7.34 (dd, *J* = 12.3, 4.0 Hz, 2H), 7.09 - 6.78 (m, 4H), 5.26 (d, *J* = 2.4 Hz, 2H), 4.64 (dt, *J* = 19.7, 6.4 Hz, 4H), 4.47 - 4.39 (m, 1H), 3.55 - 3.49 (m, 1H), 2.89 (d, *J* = 11.5 Hz, 2H), 2.54 - 2.44 (m, 2H), 2.01 (td, *J =* 11.8, 1.9 Hz, 2H), 1.88 - 1.84 (m, 2H); **LRMS** (ES) m/z 483.0 (M⁺ + 1).

### Example 79: Synthesis of Compound 79, 3-(4-(5-(difluoromethyl)-1,3,4-oxadiazole-2-yl)benzyl)-5-fluoro-1-(1-methylpiperidine-4-yl)-1,3-dih ydro-2H-benzo[d]imidazole-2-one

### [Step 1] Synthesis of tert-butyl 4-(3-(4-(5-(difluoromethyl)-1,3,4-oxadiazole-2-yl)benzyl)-5-fluoro-2-oxo-2,3-dihydro-1H-benzo[d]i midazole-1-yl)piperidine-1-carboxylate

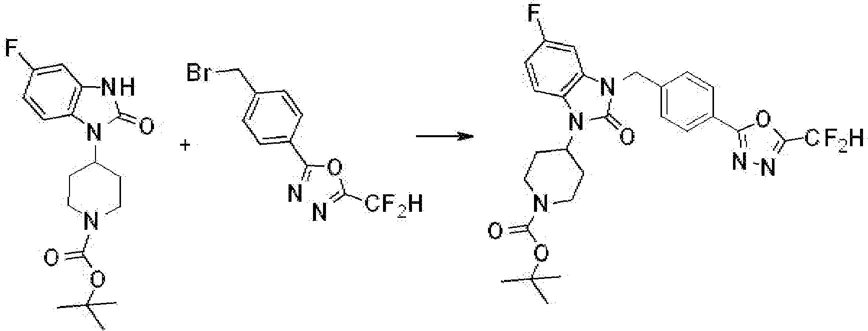

Tert-butyl 4-(5-fluoro-2-oxo-2,3-dihydro-1H-benzo[d]imidazole-1-yl)piperidine-1-carboxylate (1.300 g, 3.876 mmol) was dissolved in N,N-dimethylformamide (40 mL) at 0°C, after which sodium hydride (60.00%, 0.202 g, 5.039 mmol) was added into the resulting solution and stirred at the same temperature for 30 minutes. 2-(4-(bromomethyl)phenyl)-5-(difluoromethyl)-1,3,4-oxadiazole (1.457 g, 5.039 mmol) was added into the reaction mixture and further stirred at room temperature for 4 hours. Water was poured into the reaction mixture and an extraction was performed with ethyl acetate. An organic layer was washed with saturated sodium chloride aqueous solution, then dehydrated with anhydrous magnesium sulfate, then filtered, and then concentrated under reduced pressure. The resulting concentrate was purified via column chromatography (SiO₂, 40 g cartridge; ethyl acetate/hexane = 0 to 70%) and concentrated to obtain a title compound (1.880 g, 89.2%) in a light brown solid form.

### [Step 2] Synthesis of 3-(4-(5-(difluoromethyl)-1,3,4-oxadiazole-2-yl)benzyl)-5-fluoro-1-(piperidine-4-yl)-1,3-dihydro-2H -benzo[d]imidazole-2-one

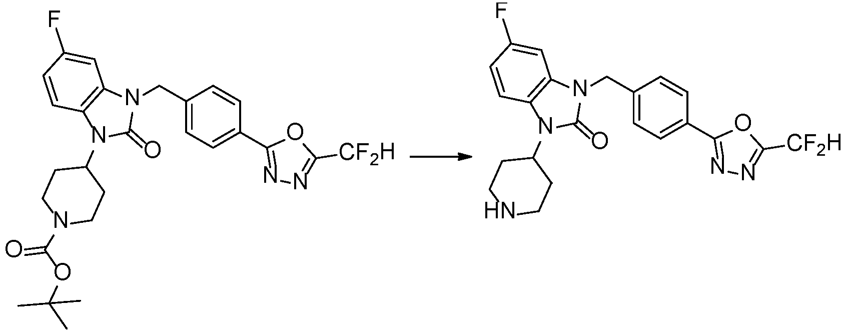

The tert-butyl 4-(3-(4-(5-(difluoromethyl)-1,3,4-oxadiazole-2-yl)benzyl)-5-fluoro-2-oxo-2,3-dihydro-1H-benzo[d]i midazole-1-yl)piperidine-1-carboxylate (1.880 g, 3.459 mmol) prepared in the step 1 was dissolved in dichloromethane (20 mL) at 0°C, after which trifluoroacetic acid (2.649 mL, 34.587 mmol) was added into the resulting solution and stirred at room temperature for 5 hours. Saturated sodium hydrogen carbonate aqueous solution was poured into the reaction mixture and an extraction was performed with dichloromethane. An organic layer was washed with saturated sodium chloride aqueous solution, then dehydrated with anhydrous magnesium sulfate, then filtered, and then concentrated under reduced pressure. The resulting concentrate was purified via column chromatography (SiO₂, 40 g cartridge; ethyl acetate/hexane = 50 to 100%) and concentrated to obtain a product, after which the resulting product was purified again via chromatography (SiO₂, 40 g cartridge; dichloromethane/methanol = 0 to 40%) and concentrated to obtain a title compound (1.230 g, 80.2%) in a light yellow solid form.

### [Step 3] Synthesis of the compound 79

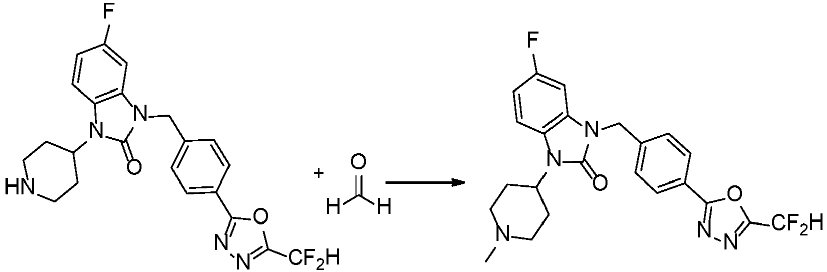

The 3-(4-(5-(difluoromethyl)-1,3,4-oxadiazole-2-yl)benzyl)-5-fluoro-1-(piperidine-4-yl)-1,3-dihydro-2H -benzo[d]imidazole-2-one (0.100 g, 0.226 mmol) prepared in the step 2, sodium triacetoxyborohydride (0.096 g, 0.451 mmol), acetic acid (0.013 mL, 0.226 mmol) and formaldehyde (0.014 g, 0.451 mmol) were dissolved in dichloromethane (2 mL) at room temperature, after which the resulting solution was stirred at the same temperature for 18 hours. Water was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; dichloromethane/methanol = 0 to 5%) and concentrated to obtain a title compound (0.067 g, 65.2%) in a white solid form.

**¹H NMR** (400 MHz, CDCl₃) δ 8.05 (d, *J =* 8.3 Hz, 2H), 7.44 (d, *J =* 8.3 Hz, 2H), 7.21 (dd, *J* = 8.7, 4.3 Hz, 1H), 6.89 (t, *J =* 51.7 Hz, 1H), 6.73 (td, *J* = 9.4, 2.8 Hz, 1H), 6.56 (dd, *J* = 8.4, 2.5 Hz, 1H), 5.09 (s, 2H), 4.44 - 4.36 (m, 1H), 3.02 - 2.99 (m, 2H), 2.50 - 2.39 (m, 2H), 2.33 (s, 3H), 2.18 - 2.12 (m, 2H), 1.84 - 1.80 (m, 2H); **LRMS** (ES) m/z 458.3 (M⁺ + 1).

### Example 80: Synthesis of Compound 80, 3-(4-(5-(difluoromethyl)-1,3,4-oxadiazole-2-yl)benzyl)-5-fluoro-1-(1-(oxetan-3-yl)piperidine-4-yl)-1,3-dihydro-2H-benzo[d]imidazole-2-one

### [Step 1] Synthesis of the compound 80

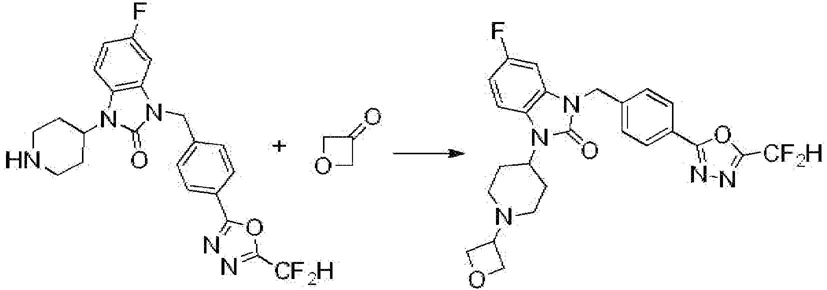

The 3-(4-(5-(difluoromethyl)-1,3,4-oxadiazole-2-yl)benzyl)-5-fluoro-1-(piperidine-4-yl)-1,3-dihydro-2H -benzo[d]imidazole-2-one (0.100 g, 0.226 mmol) prepared in the step 2 of the compound 79, sodium triacetoxyborohydride (0.096 g, 0.451 mmol), acetic acid (0.013 mL, 0.226 mmol) and oxetan-3-one (0.033 g, 0.451 mmol) were dissolved in dichloromethane (2 mL) at room temperature, after which the resulting solution was stirred at the same temperature for 18 hours. Water was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; dichloromethane/methanol = 0 to 5%) and concentrated to obtain a title compound (0.086 g, 76.1%) in a white solid form.

**¹H NMR** (400 MHz, CDCl₃) δ 8.05 (d, *J =* 8.4 Hz, 2H), 7.44 (d, *J =* 8.4 Hz, 2H), 7.23 (dd, *J =* 8.7, 4.3 Hz, 1H), 6.89 (t, *J =* 51.7 Hz, 1H), 6.76 (td, J= 9.1, 2.5 Hz, 1H), 6.58 (dd, J= 8.4, 2.5 Hz, 1H), 5.09 (s, 2H), 4.63 (dt, *J* = 22.9, 6.4 Hz, 4H), 4.45 - 4.37 (m, 1H), 3.55 - 3.48 (m, 1H), 2.89 (d, *J =* 11.5 Hz, 2H), 2.49 - 2.39 (m, 2H), 2.01 (td, *J =* 11.8, 1.9 Hz, 2H), 1.87 - 1.83 (m, 2H); LRMS (ES) m/z 500.6 (M⁺ + 1).

### Example 81: Synthesis of Compound 81, 5,6-dichloro-1-((5-(5-(difluoromethyl)-1,3,4-oxadiazole-2-yl)pyridine-2-yl)methyl)-3-(1-methylpipe ridine-4-yl)-1,3-dihydro-2H-benzo[d]imidazole-2-one

### [Step 1] Synthesis of tert-butyl 4-((2-amino-4,5-dichlorophenyl)amino)piperidine-1-carboxylate

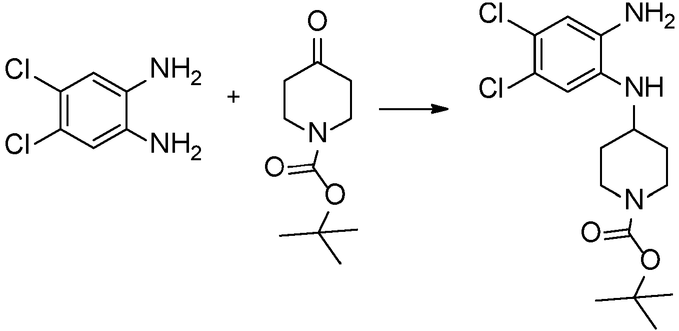

4,5-dichlorobenzene-1,2-diamine (5.000 g, 28.244 mmol), tert-butyl 4-oxopiperidine-1-carboxylate (6.753 g, 33.893 mmol), sodium triacetoxyborohydride (11.972 g, 56.488 mmol) and acetic acid (1.617 mL, 28.244 mmol) were dissolved in dichloromethane (280 mL) at room temperature, after which the resulting solution was stirred at the same temperature for 18 hours. Water was poured into the reaction mixture and an extraction was performed with dichloromethane. An organic layer was washed with saturated sodium chloride aqueous solution, then dehydrated with anhydrous magnesium sulfate, then filtered, and then concentrated under reduced pressure. The resulting concentrate was purified via column chromatography (SiO₂, 120 g cartridge; ethyl acetate/hexane = 0 to 30%) and concentrated to obtain a title compound (4.380 g, 43.0%) in a brown solid form.

### [Step 2] Synthesis of tert-butyl 4-(5,6-dichloro-2-oxo-2,3-dihydro-1H-benzo[d]imidazole-1-yl)piperidine-1-carboxylate

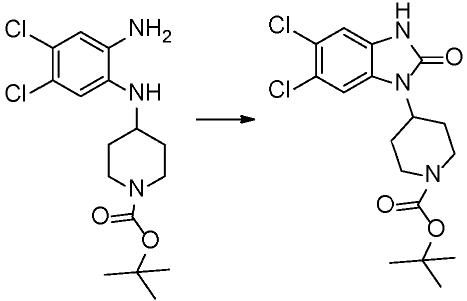

The tert-butyl 4-((2-amino-4,5-dichlorophenyl)amino)piperidine-1-carboxylate (4.380 g, 12.157 mmol) prepared in the step 1 was dissolved in tetrahydrofuran (120 mL), after which 1,1'-carbonyldiimidazole (CDI, 2.760 g, 17.020 mmol) was added into the resulting solution at 0°C and stirred at room temperature for 18 hours. Water was poured into the reaction mixture and an extraction was performed with ethyl acetate. An organic layer was washed with water, then dehydrated with anhydrous sodium sulfate, then filtered, and then concentrated under reduced pressure. The resulting concentrate was purified via column chromatography (SiO₂, 120 g cartridge; ethyl acetate/hexane = 0 to 70%) and concentrated to obtain a title compound (4.110 g, 87.5%) in a gray solid form.

### [Step 3] Synthesis of tert-butyl 4-(5,6-dichloro-3-((5-(5-(difluoromethyl)-1,3,4-oxadiazole-2-yl)pyridine-2-yl)methyl)-2-oxo-2,3-di hydro-1H-benzo[d]imidazole-1-yl)piperidine-1-carboxylate

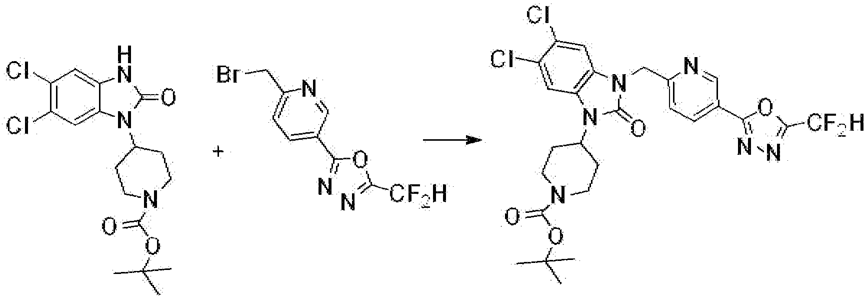

The tert-butyl 4-(5,6-dichloro-2-oxo-2,3-dihydro-1H-benzo[d]imidazole-1-yl)piperidine-1-carboxylate (2.800 g, 7.249 mmol) prepared in the step 2, sodium hydride (60.00%, 0.377 g, 9.423 mmol) and 2-(6-(bromomethyl)pyridine-3-yl)-5-(difluoromethyl)-1,3,4-oxadiazole (2.733 g, 9.423 mmol) were dissolved in N,N-dimethylformamide (70 mL) at room temperature, after which the resulting solution was stirred at the same temperature for 18 hours. Water was poured into the reaction mixture and an extraction was performed with dichloromethane. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; ethyl acetate/hexane = 0 to 30%) and concentrated to obtain a title compound (3.240 g, 75.1%) in a yellow solid form.

### [Step 4] Synthesis of 5,6-dichloro-1-((5-(5-(difluoromethyl)-1,3,4-oxadiazole-2-yl)pyridine-2-yl)methyl)-3-(piperidine-4-yl)-1,3-dihydro-2H-benzo[d]imidazole-2-one

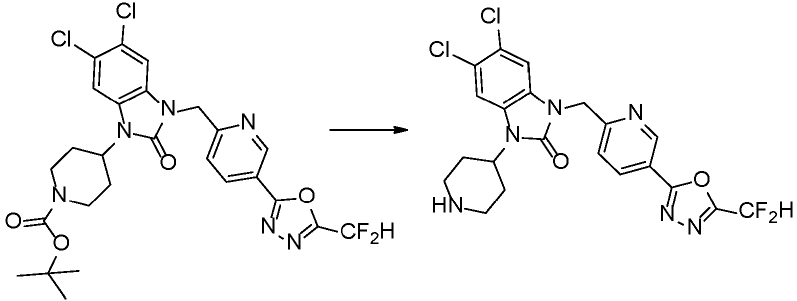

The tert-butyl 4-(5,6-dichloro-3-((5-(5-(difluoromethyl)-1,3,4-oxadiazole-2-yl)pyridine-2-yl)methyl)-2-oxo-2,3-di hydro-1H-benzo[d]imidazole-1-yl)piperidine-1-carboxylate (3.240 g, 5.441 mmol) prepared in the step 3 was dissolved in dichloromethane (50 mL) at 0°C, after which trifluoroacetic acid (8.334 mL, 108.829 mmol) was added into the resulting solution and stirred at room temperature for 5 hours. Saturated sodium hydrogen carbonate aqueous solution was poured into the reaction mixture and an extraction was performed with dichloromethane. An organic layer was washed with saturated sodium chloride aqueous solution, then dehydrated with anhydrous magnesium sulfate, then filtered, and then concentrated under reduced pressure. The resulting concentrate was purified via column chromatography (SiO₂, 80 g cartridge; dichloromethane/methanol = 0 to 30%) and concentrated, after which an obtained product was purified again via chromatography (SiO₂, 80 g cartridge; dichloromethane/methanol = 0 to 30%) and concentrated to obtain a title compound (2.316 g, 85.9%) in a brown solid form.

### [Step 5] Synthesis of the compound 81

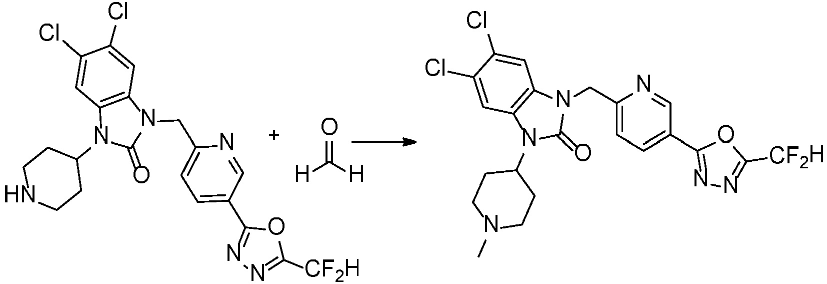

The 5,6-dichloro-1-((5-(5-(difluoromethyl)-1,3,4-oxadiazole-2-yl)pyridine-2-yl)methyl)-3-(piperidine-4-yl)-1,3-dihydro-2H-benzo[d]imidazole-2-one (0.100 g, 0.202 mmol) prepared in the step 4, sodium triacetoxyborohydride (0.086 g, 0.404 mmol), acetic acid (0.012 mL, 0.202 mmol) and formaldehyde (0.012 g, 0.404 mmol) were dissolved in dichloromethane (2 mL) at room temperature, after which the resulting solution was stirred at the same temperature for 18 hours. Water was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; dichloromethane/methanol = 0 to 5%) and concentrated to obtain a title compound (0.051 g, 49.8%) in a white solid form.

**¹H NMR** (400 MHz, CDCl₃) δ 9.26 (d, *J =* 1.6 Hz, 1H), 8.33 (dd, *J* = 8.2, 2.2 Hz, 1H), 7.74 - 7.40 (m, 2H), 7.07 (s, 1H), 6.92 (t, *J =* 51.6 Hz, 1H), 5.21 (s, 2H), 4.41 - 4.32 (m, 1H), 3.02 (d, *J* = 11.6 Hz, 2H), 2.45 - 2.34 (m, 5H), 2.18 - 2.11 (m, 2H), 1.84 - 1.80 (m, 2H); **LRMS** (ES) m/z 511.5 (M⁺+ 2).

### Example 82: Synthesis of Compound 82, 5,6-dichloro-1-((5-(5-(difluoromethyl)-1,3,4-oxadiazole-2-yl)pyridine-2-yl)methyl)-3-(1-(oxetan-3-yl)piperidine-4-yl)-1,3-dihydro-2H-benzo[d]imidazole-2-one

### [Step 1] Synthesis of the compound 82

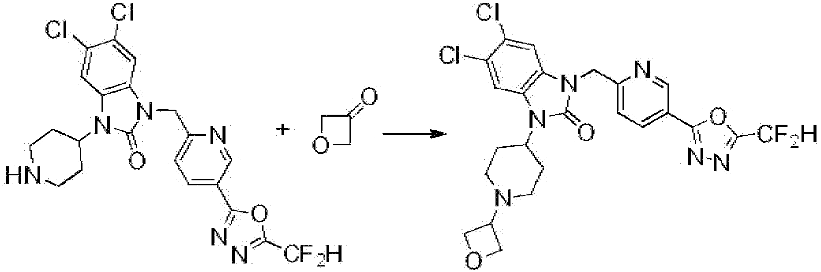

The 5,6-dichloro-1-((5-(5-(difluoromethyl)-1,3,4-oxadiazole-2-yl)pyridine-2-yl)methyl)-3-(piperidine-4-yl)-1,3-dihydro-2H-benzo[d]imidazole-2-one (0.100 g, 0.202 mmol) prepared in the step 4 of the compound 81, sodium triacetoxyborohydride (0.086 g, 0.404 mmol), acetic acid (0.012 mL, 0.202 mmol) and oxetan-3-one (0.029 g, 0.404 mmol) were dissolved in dichloromethane (2 mL) at room temperature, after which the resulting solution was stirred at the same temperature for 18 hours. Water was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; dichloromethane/methanol = 0 to 5%) and concentrated to obtain a title compound (0.091 g, 82.0%) in a yellow solid form.

**¹H NMR** (400 MHz, CDCl₃) δ 9.25 (dd, *J* = 2.2, 0.6 Hz, 1H), 8.33 (dd, *J* = 8.2, 2.2 Hz, 1H), 7.42 (dd, *J* = 8.2, 0.4 Hz, 1H), 7.38 (s, 1H), 7.08 (s, 1H), 6.92 (t, *J* = 51.6 Hz, 1H), 5.20 (s, 2H), 4.65 (dt, *J =* 19.9, 6.4 Hz, 4H), 4.41 - 4.33 (m, 1H), 3.56 - 3.50 (m, 1H), 2.93 - 2.90 (m, 2H), 2.45 - 2.35 (m, 2H), 2.04 - 1.98 (m, 2H), 1.87 - 1.83 (m, 2H); **LRMS** (ES) m/z 553.5 (M⁺ + 2).

### Example 83: Synthesis of Compound 83, 1-((5-(5-(difluoromethyl)-1,3,4-oxadiazole-2-yl)pyridine-2-yl)methyl)-5,6-difluoro-3-(1-methylpiper idine-4-yl)-1,3-dihydro-2H-benzo[d]imidazole-2-one

### [Step 1] Synthesis of tert-butyl 4-((2-amino-4,5-difluorophenyl)amino)piperidine-1-carboxylate

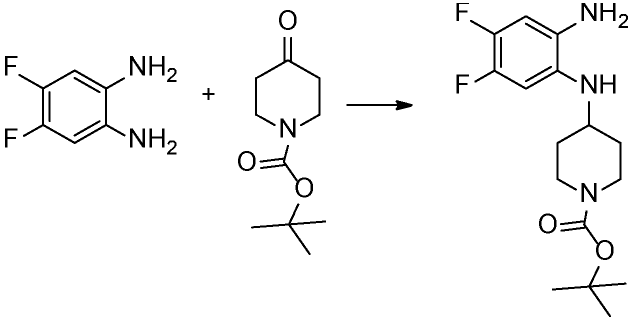

4,5-difluorobenzene-1,2-diamine (4.000 g, 27.755 mmol), tert-butyl 4-oxopiperidine-1-carboxylate (5.807 g, 29.142 mmol) and sodium triacetoxyborohydride (11.765 g, 55.509 mmol) were dissolved in dichloromethane (270 mL) at room temperature, after which the resulting solution was stirred at the same temperature for 18 hours. Water was poured into the reaction mixture and an extraction was performed with dichloromethane. An organic layer was washed with saturated sodium chloride aqueous solution, then dehydrated with anhydrous magnesium sulfate, then filtered, and then concentrated under reduced pressure. The resulting concentrate was purified via column chromatography (SiO₂, 12 g cartridge; ethyl acetate/hexane = 0 to 40%) and concentrated to obtain a title compound (5.970 g, 65.7%) in a brown liquid form.

### [Step 2] Synthesis of tert-butyl 4-(5,6-difluoro-2-oxo-2,3-dihydro-1H-benzo[d]imidazole-1-yl)piperidine-1-carboxylate

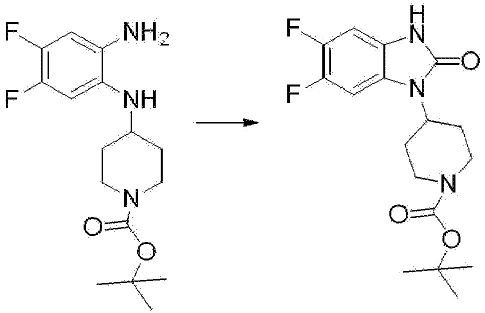

The tert-butyl 4-((2-amino-4,5-difluorophenyl)amino)piperidine-1-carboxylate (5.970 g, 18.236 mmol) prepared in the step 1 was dissolved in tetrahydrofuran (180 mL) at 0°C, after which 1,1'-carbonyldiimidazole (CDI, 3.548 g, 21.883 mmol) was added into the resulting solution and stirred at room temperature for 18 hours. Solvent was removed from the reaction mixture under reduced pressure, after which water was poured into the resulting concentrate, and then an extraction was performed with ethyl acetate. An organic layer was washed with saturated sodium chloride aqueous solution, then dehydrated with anhydrous magnesium sulfate, then filtered, and then concentrated under reduced pressure. The resulting concentrate was purified via column chromatography (SiO₂, 12 g cartridge; ethyl acetate/hexane = 0 to 80%) and concentrated to obtain a title compound (5.060 g, 78.5%) in a brown solid form.

### [Step 3] Synthesis of tert-butyl 4-(3-((5-(5-(difluoromethyl)-1,3,4-oxadiazole-2-yl)pyridine-2-yl)methyl)-5,6-difluoro-2-oxo-2,3-dih ydro-1H-benzo[d]imidazole-1-yl)piperidine-1-carboxylate

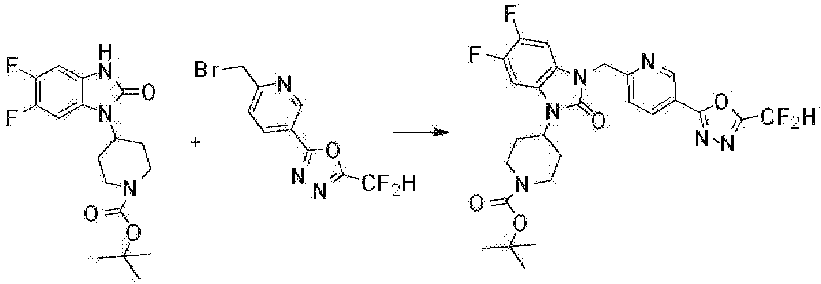

The tert-butyl 4-(5,6-difluoro-2-oxo-2,3-dihydro-1H-benzo[d]imidazole-1-yl)piperidine-1-carboxylate (1.600 g, 4.528 mmol) prepared in the step 2 was dissolved in N,N-dimethylformamide (50 mL) at 0°C, after which sodium hydride (60.00%, 0.272 g, 6.792 mmol) was added into the resulting solution and stirred at the same temperature for 30 minutes. 2-(6-(bromomethyl)pyridine-3-yl)-5-(difluoromethyl)-1,3,4-oxadiazole (1.707 g, 5.886 mmol) was added into the reaction mixture and further stirred at room temperature for 5 hours. Saturated sodium hydrogen carbonate aqueous solution was poured into the reaction mixture and an extraction was performed with ethyl acetate. An organic layer was washed with saturated sodium chloride aqueous solution, then dehydrated with anhydrous magnesium sulfate, then filtered, and then concentrated under reduced pressure. The resulting concentrate was purified via column chromatography (SiO₂, 40 g cartridge; ethyl acetate/hexane = 0 to 70%) and concentrated to obtain a title compound (1.580 g, 62.0%) in a brown solid form.

### [Step 4] Synthesis of 1-((5-(5-(difluoromethyl)-1,3,4-oxadiazole-2-yl)pyridine-2-yl)methyl)-5,6-difluoro-3-(piperidine-4-y I)-1,3-dihydro-2H-benzo[d]imidazole-2-one

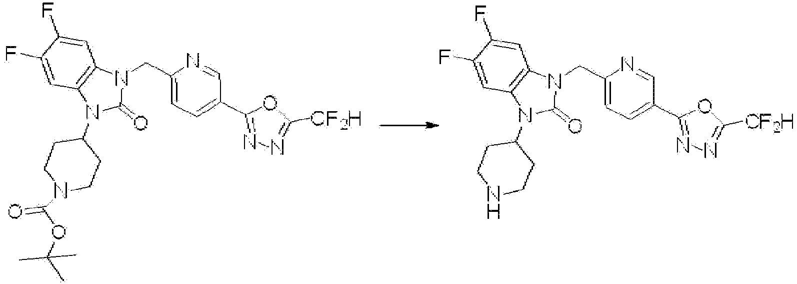

The tert-butyl 4-(3-((5-(5-(difluoromethyl)-1,3,4-oxadiazole-2-yl)pyridine-2-yl)methyl)-5,6-difluoro-2-oxo-2,3-dih ydro-1H-benzo[d]imidazole-1-yl)piperidine-1-carboxylate (1.580 g, 2.809 mmol) prepared in the step 3 was dissolved in dichloromethane (19 mL) at 0°C, after which trifluoroacetic acid (2.151 mL, 28.087 mmol) was added into the resulting solution and stirred at room temperature for 5 hours. Saturated sodium hydrogen carbonate aqueous solution was poured into the reaction mixture and an extraction was performed with dichloromethane. An organic layer was washed with saturated sodium chloride aqueous solution, then dehydrated with anhydrous magnesium sulfate, then filtered, and then concentrated under reduced pressure. The resulting concentrate was purified via column chromatography (SiO₂, 40 g cartridge; ethyl acetate/hexane = 50 to 100%) and concentrated, after which an obtained product was purified again via chromatography (SiO₂, 40 g cartridge; dichloromethane/methanol = 0 to 30%) and concentrated to obtain a title compound (0.513 g, 39.5%) in a brown solid form.

### [Step 5] Synthesis of the compound 83

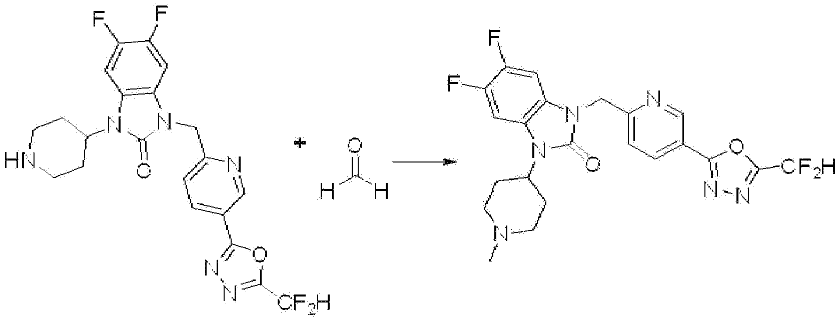

The 1-((5-(5-(difluoromethyl)-1,3,4-oxadiazole-2-yl)pyridine-2-yl)methyl)-5,6-difluoro-3-(piperidine-4-y I)-1,3-dihydro-2H-benzo[d]imidazole-2-one (0.100 g, 0.216 mmol) prepared in the step 4, sodium triacetoxyborohydride (0.092 g, 0.433 mmol), acetic acid (0.012 mL, 0.216 mmol) and formaldehyde (0.013 g, 0.433 mmol) were dissolved in dichloromethane (2 mL) at room temperature, after which the resulting solution was stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; dichloromethane/methanol = 0 to 5%) and concentrated to obtain a title compound (0.070 g, 67.5%) in a yellow solid form.

**¹H NMR** (400 MHz, CDCl₃) δ 9.26 (dd, *J =* 2.2, 0.7 Hz, 1H), 8.33 (dd, *J* = 8.2, 2.2 Hz, 1H), 7.42 (dd, *J* = 8.2, 0.6 Hz, 1H), 7.16 (dd, *J =* 10.4, 6.7 Hz, 1H), 6.91 (t, *J =* 51.6 Hz, 1H), 6.86 (dd, *J* = 9.7, 6.8 Hz, 1H), 5.20 (s, 2H), 4.41 - 4.32 (m, 1H), 3.01 (dd, *J* = 9.8, 1.9 Hz, 2H), 2.44 - 2.33 (m, 5H), 2.14 (td, *J =* 12.0, 2.1 Hz, 2H), 1.84 - 1.80 (m, 2H); **LRMS** (ES) m/z 477.6 (M⁺ + 1).

### Example 84: Synthesis of Compound 84, 1-((5-(5-(difluoromethyl)-1,3,4-oxadiazole-2-yl)pyridine-2-yl)methyl)-5,6-difluoro-3-(1-(oxetan-3-y I)piperidine-4-yl)-1,3-dihydro-2H-benzo[d]imidazole-2-one

### [Step 1] Synthesis of the compound 84

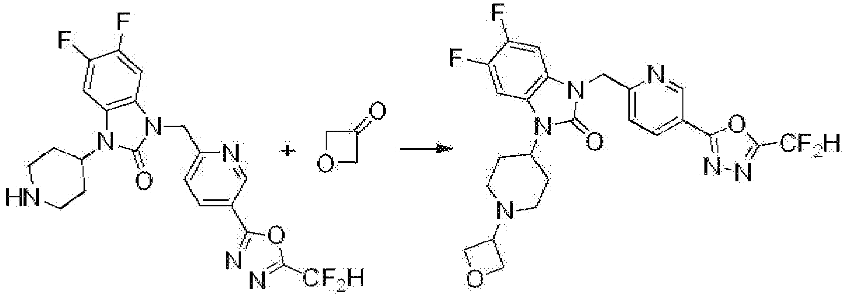

The 1-((5-(5-(difluoromethyl)-1,3,4-oxadiazole-2-yl)pyridine-2-yl)methyl)-5,6-difluoro-3-(piperidine-4-y I)-1,3-dihydro-2H-benzo[d]imidazole-2-one (0.100 g, 0.216 mmol) prepared in the step 4 of the compound 83, sodium triacetoxyborohydride (0.092 g, 0.433 mmol), acetic acid (0.012 mL, 0.216 mmol) and oxetan-3-one (0.031 g, 0.433 mmol) were dissolved in dichloromethane (2 mL) at room temperature, after which the resulting solution was stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; dichloromethane/methanol = 0 to 5%) and concentrated to obtain a title compound (0.071 g, 63.1%) in a yellow solid form.

**¹H NMR** (400 MHz, CDCl₃) δ 9.25 (dd, *J =* 2.2, 0.8 Hz, 1H), 8.33 (dd, *J* = 8.2, 2.2 Hz, 1H), 7.42 (dd, *J =* 8.2, 0.7 Hz, 1H), 7.16 (dd, *J =* 10.3, 6.7 Hz, 1H), 6.91 (t, *J =* 51.6 Hz, 1H), 6.87 (dd, *J = 9.7,* 6.8 Hz, 1H), 5.19 (s, 2H), 4.97 (dt, J = 21.5, 200.2 Hz, 4H), 4.41 - 4.33 (m, 1H), 3.55 - 3.49 (m, 1H), 2.90 (d, *J =* 11.6 Hz, 2H), 2.43 - 2.33 (m, 2H), 2.01 (td, *J =* 11.8, 2.0 Hz, 2H), 1.86 - 1.83 (m, 2H); **LRMS** (ES) m/z 519.6 (M⁺ + 1).

### Example 85: Synthesis of Compound 85, 5,6-dichloro-1-(4-(5-(difluoromethyl)-1,3,4-oxadiazole-2-yl)benzyl)-3-(1-methylpiperidine-4-yl)-1, 3-dihydro-2H-benzo[d]imidazole-2-one

### [Step 1] Synthesis of tert-butyl 4-(5,6-dichloro-3-(4-(5-(difluoromethyl)-1,3,4-oxadiazole-2-yl)benzyl)-2-oxo-2,3-dihydro-1H-benz o[d]imidazole-1-yl)piperidine-1-carboxylate

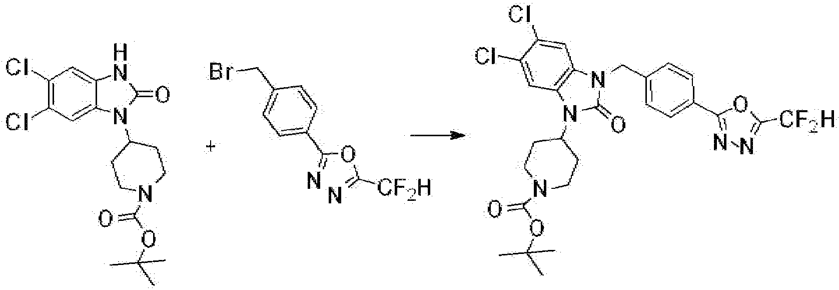

The tert-butyl 4-(5,6-dichloro-2-oxo-2,3-dihydro-1H-benzo[d]imidazole-1-yl)piperidine-1-carboxylate (2.050 g, 5.307 mmol) prepared in the step 2 of the compound 81 was dissolved in N,N-dimethylformamide (50 mL) at 0°C, after which sodium hydride (60.00%, 0.318 g, 7.961 mmol) was added into the resulting solution and stirred at the same temperature for 30 minutes. 2-(4-(bromomethyl)phenyl)-5-(difluoromethyl)-1,3,4-oxadiazole (1.994 g, 6.899 mmol) was added into the reaction mixture and further stirred at room temperature for 5 hours. Water was poured into the reaction mixture and an extraction was performed with ethyl acetate. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; ethyl acetate/hexane = 0 to 30%) and concentrated to obtain a title compound (2.120 g, 67.2%) in a brown solid form.

### [Step 2] Synthesis of tert-butyl 4-(5,6-dichloro-3-(4-(5-(difluoromethyl)-1,3,4-oxadiazole-2-yl)benzyl)-2-oxo-2,3-dihydro-1H-benz o[d]imidazole-1-yl)piperidine-1-carboxylate

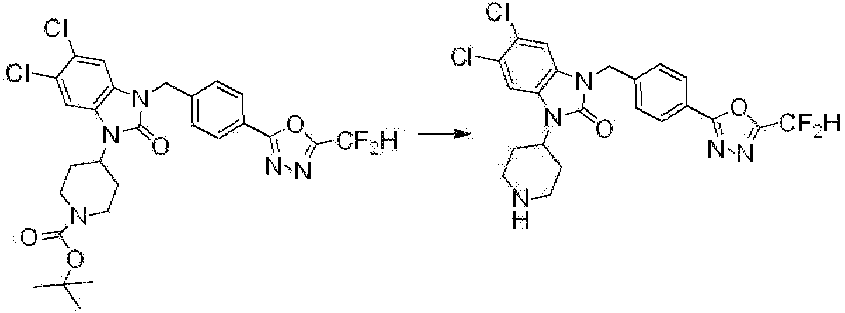

The tert-butyl 4-(5,6-dichloro-3-(4-(5-(difluoromethyl)-1,3,4-oxadiazole-2-yl)benzyl)-2-oxo-2,3-dihydro-1H-benz o[d]imidazole-1-yl)piperidine-1-carboxylate (2.120 g, 3.566 mmol) prepared in the step 1 and trifluoroacetic acid (2.731 mL, 35.664 mmol) were dissolved in dichloromethane (5 mL) at room temperature, after which the resulting solution was stirred at the same temperature for 18 hours. Water was poured into the reaction mixture and an extraction was performed with dichloromethane. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 40 g cartridge; dichloromethane/methanol = 0 to 30%) and concentrated to obtain a title compound (0.195 g, 9.2%) in a white solid form.

### [Step 3] Synthesis of the compound 85

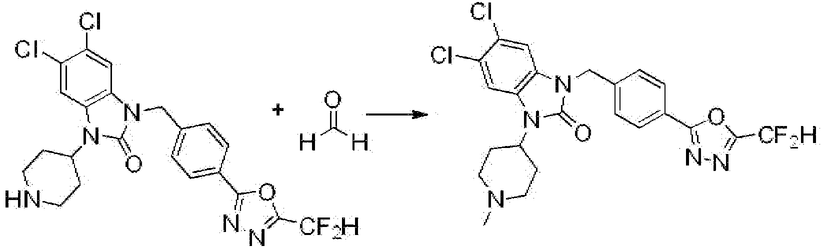

The 5,6-dichloro-1-(4-(5-(difluoromethyl)-1,3,4-oxadiazole-2-yl)benzyl)-3-(piperidine-4-yl)-1,3-dihydro -2H-benzo[d]imidazole-2-one (0.100 g, 0.202 mmol) prepared in the step 2, sodium triacetoxyborohydride (0.086 g, 0.405 mmol), acetic acid (0.012 mL, 0.202 mmol) and formaldehyde (0.012 g, 0.405 mmol) were dissolved in dichloromethane (2 mL) at room temperature, after which the resulting solution was stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; dichloromethane/methanol = 0 to 5%) and concentrated to obtain a title compound (0.065 g, 63.1%) in a white solid form.

**¹H NMR** (400 MHz, CDCl₃) δ 8.07 (d, *J* = 8.4 Hz, 2H), 7.44 - 7.41 (m, 3H), 6.90 (t, *J* = 51.7 Hz, 1H), 6.87 (s, 1H), 5.09 (s, 2H), 4.43 - 4.35 (m, 1H), 3.05 - 3.02 (m, 2H), 2.46 - 2.35 (m, 5H), 2.16 (td, *J* = 12.0, 2.1 Hz, 2H), 1.85 - 1.81 (m, 2H); **LRMS** (ES) m/z 510.5 (M⁺ + 2).

### Example 86: Synthesis of Compound 86, 5,6-dichloro-1-(4-(5-(difluoromethyl)-1,3,4-oxadiazole-2-yl)benzyl)-3-(1-(oxetan-3-yl)piperidine-4 -yl)-1,3-dihydro-2H-benzo[d]imidazole-2-one

### [Step 1] Synthesis of the compound 86

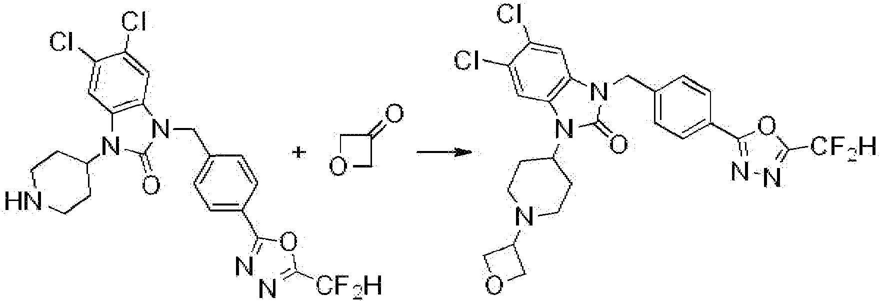

The 5,6-dichloro-1-(4-(5-(difluoromethyl)-1,3,4-oxadiazole-2-yl)benzyl)-3-(piperidine-4-yl)-1,3-dihydro -2H-benzo[d]imidazole-2-one (0.100 g, 0.202 mmol) prepared in the step 2 of the compound 85, sodium triacetoxyborohydride (0.086 g, 0.405 mmol), acetic acid (0.012 mL, 0.202 mmol) and oxetan-3-one (0.029 g, 0.405 mmol) were dissolved in dichloromethane (2 mL) at room temperature, after which the resulting solution was stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; dichloromethane/methanol = 0 to 5%) and concentrated to obtain a title compound (0.056 g, 50.3%) in a white solid form.

**¹H NMR** (400 MHz, CDCl₃) δ 8.08 - 8.06 (m, 2H), 7.43 (d, *J* = 8.5 Hz, 2H), 7.39 (s, 1H), 6.90 (t, *J =* 51.7 Hz, 1H), 6.89 (s, 1H), 5.09 (s, 2H), 4.66 (dt, *J =* 19.4, 6.4 Hz, 4H), 4.43 - 4.35 (m, 1H), 3.57 - 3.51 (m, 1H), 2.92 (d, *J =* 11.6 Hz, 2H), 2.45 - 2.35 (m, 2H), 2.05 - 1.99 (m, 2H), 1.88 - 1.84 (m, 2H); **LRMS** (ES) m/z 552.5 (M⁺ + 2).

### Example 87: Synthesis of Compound 87, 5,6-dichloro-1-(4-(5-(difluoromethyl)-1,3,4-oxadiazole-2-yl)-2-fluorobenzyl)-3-(1-methylpiperidine -4-yl)-1,3-dihydro-2H-benzo[d]imidazole-2-one

### [Step 1] Synthesis of tert-butyl 4-(5,6-dichloro-3-(4-(5-(difluoromethyl)-1,3,4-oxadiazole-2-yl)-2-fluorobenzyl)-2-oxo-2,3-dihydro-1H-benzo[d]imidazole-1-yl)piperidine-1-carboxylate

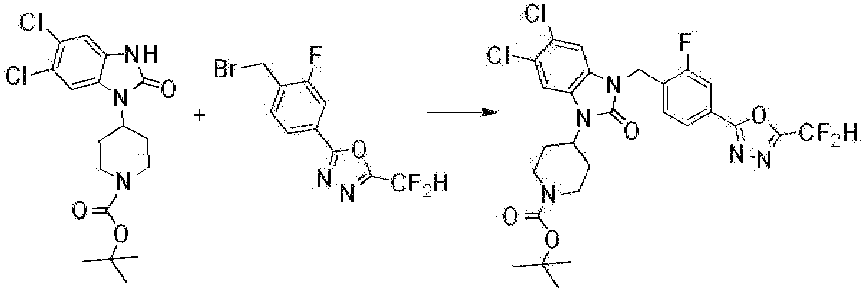

The tert-butyl 4-(5,6-dichloro-2-oxo-2,3-dihydro-1H-benzo[d]imidazole-1-yl)piperidine-1-carboxylate (2.050 g, 5.307 mmol) prepared in the step 2 of the compound 81 was dissolved in N,N-dimethylformamide (50 mL) at 0°C, after which sodium hydride (60.00%, 0.318 g, 7.961 mmol) was added into the resulting solution and stirred at the same temperature for 30 minutes. 2-(4-(bromomethyl)-3-fluorophenyl)-5-(difluoromethyl)-1,3,4-oxadiazole (2.119 g, 6.899 mmol) was added into the reaction mixture and further stirred at room temperature for 5 hours. Water was poured into the reaction mixture and an extraction was performed with ethyl acetate. An organic layer was washed with saturated sodium chloride aqueous solution, then dehydrated with anhydrous magnesium sulfate, then filtered, and then concentrated under reduced pressure. The resulting concentrate was purified via column chromatography (SiO₂, 12 g cartridge; ethyl acetate/hexane = 0 to 70%) and concentrated to obtain a title compound (2.157 g, 66.4%) in a light brown solid form.

### [Step 2] Synthesis of 5,6-dichloro-1-(4-(5-(difluoromethyl)-1,3,4-oxadiazole-2-yl)-2-fluorobenzyl)-3-(piperidine-4-yl)-1, 3-dihydro-2H-benzo[d]imidazole-2-one

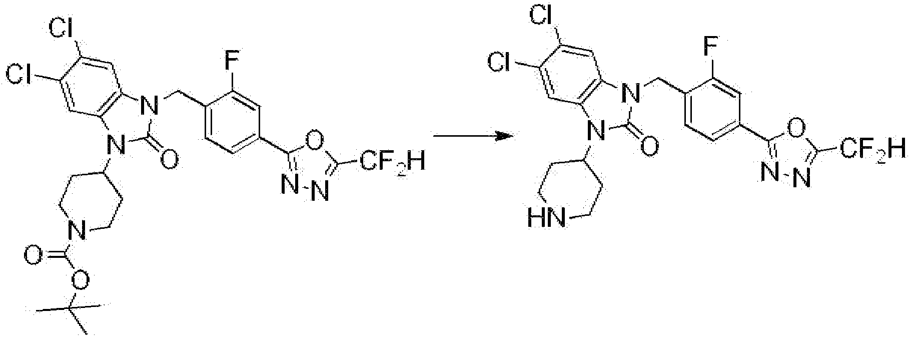

The tert-butyl 4-(5,6-dichloro-3-(4-(5-(difluoromethyl)-1,3,4-oxadiazole-2-yl)-2-fluorobenzyl)-2-oxo-2,3-dihydro-1H-benzo[d]imidazole-1-yl)piperidine-1-carboxylate (2.157 g, 3.522 mmol) prepared in the step 1 and trifluoroacetic acid (2.697 mL, 35.219 mmol) were dissolved in dichloromethane (25 mL) at room temperature, after which the resulting solution was stirred at the same temperature for 5 hours. Saturated sodium hydrogen carbonate aqueous solution was poured into the reaction mixture and an extraction was performed with dichloromethane. An organic layer was washed with saturated sodium chloride aqueous solution, then dehydrated with anhydrous magnesium sulfate, then filtered, and then concentrated under reduced pressure. The resulting concentrate was purified via column chromatography (SiO₂, 40 g cartridge; dichloromethane/methanol = 0 to 50%) and concentrated to obtain a title compound (1.268 g, 70.3%) in a white solid form.

### [Step 3] Synthesis of the compound 87

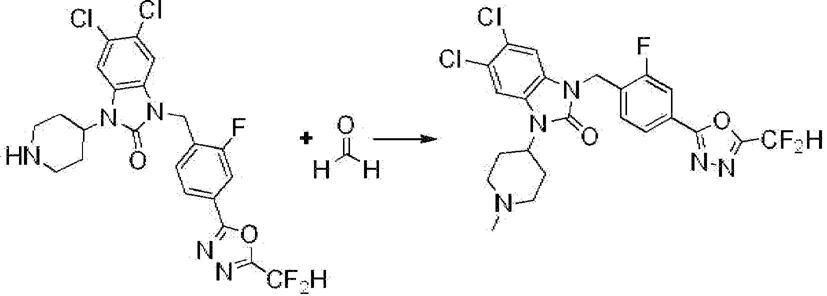

The 5,6-dichloro-1-(4-(5-(difluoromethyl)-1,3,4-oxadiazole-2-yl)-2-fluorobenzyl)-3-(piperidine-4-yl)-1, 3-dihydro-2H-benzo[d]imidazole-2-one (0.100 g, 0.195 mmol) prepared in the step 2, sodium triacetoxyborohydride (0.083 g, 0.390 mmol), acetic acid (0.011 mL, 0.195 mmol) and formaldehyde (0.012 g, 0.390 mmol) were dissolved in dichloromethane (2 mL) at room temperature, after which the resulting solution was stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; dichloromethane/methanol = 0 to 5%) and concentrated to obtain a title compound (0.061 g, 58.9%) in a white solid form.

**¹H NMR** (400 MHz, CDCl₃) δ 7.84 - 7.82 (m, 2H), 7.43 - 7.39 (m, 2H), 6.99 (s, 1H), 6.90 (t, *J = 51.6* Hz, 1H), 5.12 (s, 2H), 4.40 - 4.32 (m, 1H), 3.02 (d, *J* = 11.7 Hz, 2H), 2.44 - 2.31 (m, 5H), 2.17 - 2.11 (m, 2H), 1.81 (dd, *J =* 12.0, 2.2 Hz, 2H); **LRMS** (ES) m/z 526.5 (M⁺).

### Example 88: Synthesis of Compound 88, 5,6-dichloro-1-(4-(5-(difluoromethyl)-1,3,4-oxadiazole-2-yl)-2-fluorobenzyl)-3-(1-(oxetan-3-yl)pip eridine-4-yl)-1,3-dihydro-2H-benzo[d]imidazole-2-one

### [Step 1] Synthesis of the compound 88

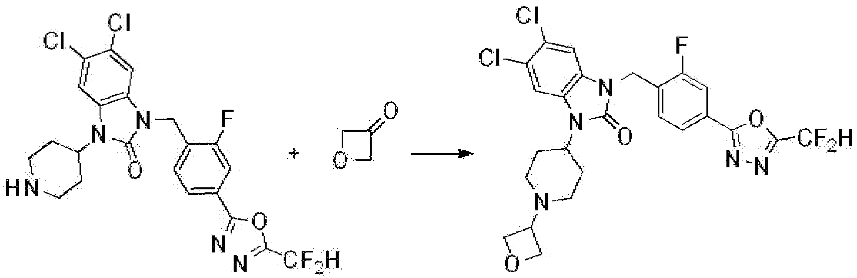

The 5,6-dichloro-1-(4-(5-(difluoromethyl)-1,3,4-oxadiazole-2-yl)-2-fluorobenzyl)-3-(piperidine-4-yl)-1, 3-dihydro-2H-benzo[d]imidazole-2-one (0.100 g, 0.195 mmol) prepared in the step 2 of the compound 87, sodium triacetoxyborohydride (0.083 g, 0.390 mmol), acetic acid (0.011 mL, 0.195 mmol) and oxetan-3-one (0.028 g, 0.390 mmol) were dissolved in dichloromethane (2 mL) at room temperature, after which the resulting solution was stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; dichloromethane/methanol = 0 to 5%) and concentrated to obtain a title compound (0.067 g, 60.3%) in a white solid form.

**¹H NMR** (400 MHz, CDCl₃) δ 7.86 - 7.83 (m, 2H), 7.44 (t, *J* = 6.5 Hz, 1H), 7.38 (s, 1H), 7.01 (s, 1H), 6.90 (t, *J =* 51.7 Hz, 1H), 5.12 (s, 2H), 4.65 (dt, *J =* 19.6, 6.4 Hz, 4H), 4.41 - 4.33 (m, 1H), 3.56 - 3.50 (m, 1H), 2.92 (d, *J =* 11.6 Hz, 2H), 2.45 - 2.34 (m, 2H), 2.04 - 1.99 (m, 2H), 1.85 (dd, *J=* 12.0, 2.2 Hz, 2H); **LRMS** (ES) m/z 568.5 (M⁺).

### Example 89: Synthesis of Compound 89, 3-((5-(5-(difluoromethyl)-1,3,4-oxadiazole-2-yl)pyridine-2-yl)methyl)-1-(1-methylpiperidine-4-yl)-5 -(trifluoromethyl)-1,3-dihydro-2H-benzo[d]imidazole-2-one

### [Step 1] Synthesis of tert-butyl 4-((2-nitro-4-(trifluoromethyl)phenyl)amino)piperidine-1-carboxylate

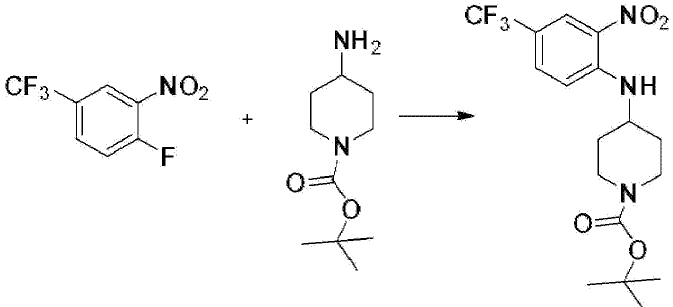

1-fluoro-2-nitro-4-(trifluoromethyl)benzene (5.000 g, 23.912 mmol), tert-butyl 4-aminopiperidine-1-carboxylate (5.029 g, 25.108 mmol), potassium carbonate (6.609 g, 47.824 mmol) and potassium iodide (0.397 g, 2.391 mmol) were dissolved in N,N-dimethylformamide (240 mL) at room temperature, after which the resulting solution was stirred at 80°C for 18 hours, and then a reaction was finished by lowering a temperature to room temperature. Solvent was removed from the reaction mixture under reduced pressure, after which water was poured into the resulting concentrate, and then an extraction was performed with ethyl acetate. An organic layer was washed with saturated sodium chloride aqueous solution, then dehydrated with anhydrous magnesium sulfate, then filtered, and then concentrated under reduced pressure. The resulting concentrate was purified via column chromatography (SiO₂, 120 g cartridge; ethyl acetate/hexane = 0 to 50%) and concentrated to obtain a title compound (7.920 g, 85.1%) in a yellow solid form.

### [Step 2] Synthesis of tert-butyl 4-((2-amino-4-(trifluoromethyl)phenyl)amino)piperidine-1-carboxylate

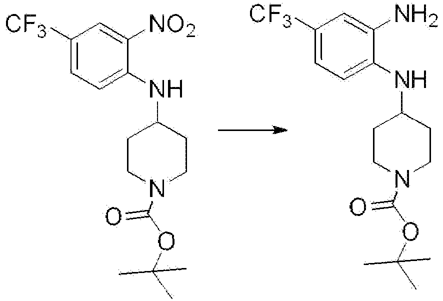

The tert-butyl 4-((2-nitro-4-(trifluoromethyl)phenyl)amino)piperidine-1-carboxylate (7.920 g, 20.340 mmol) prepared in the step 1 and Pd/C (10%, 0.481 g, 2.034 mmol) were dissolved in methanol (200 mL) at room temperature, after which the resulting solution was stirred at the same temperature for 18 hours in the presence of a hydrogen balloon. The reaction mixture was filtered via a celite pad to remove a solid therefrom, after which solvent was removed from the resulting filtrate under reduced pressure, and then a title compound was used without an additional purification process (7.130 g, 97.5%, violet solid).

### [Step 3] Synthesis of tert-butyl 4-(2-oxo-5-(trifluoromethyl)-2,3-dihydro-1H-benzo[d]imidazole-1-yl)piperidine-1-carboxylate

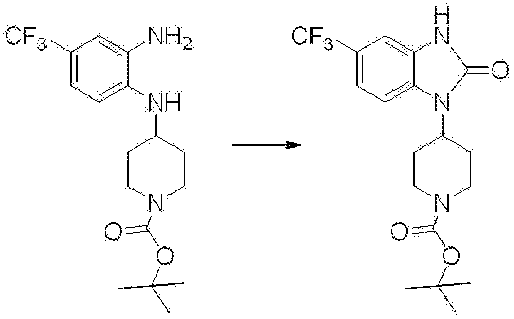

The tert-butyl 4-((2-amino-4-(trifluoromethyl)phenyl)amino)piperidine-1-carboxylate (7.130 g, 19.839 mmol) prepared in the step 2 was dissolved in N,N-dimethylformamide (200 mL), after which 1,1'-carbonyldiimidazole (CDI, 4.504 g, 27.775 mmol) was added into the resulting solution at 0°C and stirred at room temperature for 18 hours. Solvent was removed from the reaction mixture under reduced pressure, after which the resulting concentrate was purified via column chromatography (SiO₂, 120 g cartridge; ethyl acetate/hexane = 0 to 70%) and concentrated to obtain a title compound (7.610 g, 99.5%) in a gray solid form.

### [Step 4] Synthesis of tert-butyl 4-(3-((5-(5-(difluoromethyl)-1,3,4-oxadiazole-2-yl)pyridine-2-yl)methyl)-2-oxo-5-(trifluoromethyl)-2,3-dihydro-1H-benzo[d]imidazole-1-yl)piperidine-1-carboxylate

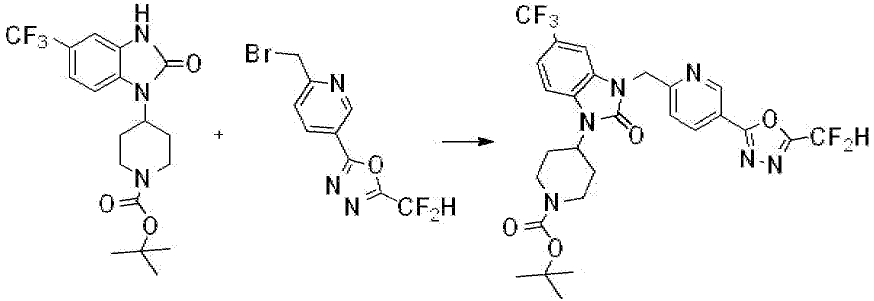

The tert-butyl 4-(2-oxo-5-(trifluoromethyl)-2,3-dihydro-1H-benzo[d]imidazole-1-yl)piperidine-1-carboxylate (2.500 g, 6.487 mmol) prepared in the step 3 was dissolved in N,N-dimethylformamide (65 mL) at 0°C, after which sodium hydride (60.00%, 0.389 g, 9.730 mmol) was added into the resulting solution and stirred at the same temperature for 30 minutes. 2-(6-(bromomethyl)pyridine-3-yl)-5-(difluoromethyl)-1,3,4-oxadiazole (2.446 g, 8.433 mmol) was added into the reaction mixture and further stirred at room temperature for 5 hours. Water was poured into the reaction mixture and an extraction was performed with ethyl acetate. An organic layer was washed with saturated sodium chloride aqueous solution, then dehydrated with anhydrous magnesium sulfate, then filtered, and then concentrated under reduced pressure. The resulting concentrate was purified via column chromatography (SiO₂, 40 g cartridge; ethyl acetate/hexane = 0 to 70%) and concentrated to obtain a title compound (2.516 g, 65.2%) in a brown solid form.

### [Step 5] Synthesis of 3-((5-(5-(difluoromethyl)-1,3,4-oxadiazole-2-yl)pyridine-2-yl)methyl)-1-(piperidine-4-yl)-5-(trifluor omethyl)-1,3-dihydro-2H-benzo[d]imidazole-2-one

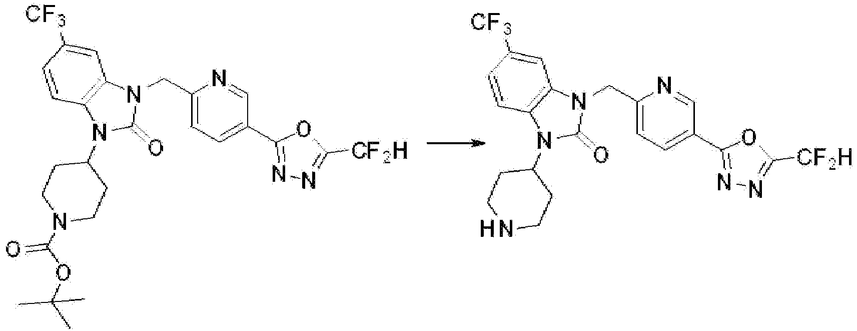

The tert-butyl 4-(3-((5-(5-(difluoromethyl)-1,3,4-oxadiazole-2-yl)pyridine-2-yl)methyl)-2-oxo-5-(trifluoromethyl)-2,3-dihydro-1H-benzo[d]imidazole-1-yl)piperidine-1-carboxylate (2.510 g, 4.222 mmol) prepared in the step 4 and trifluoroacetic acid (3.233 mL, 42.218 mmol) were dissolved in dichloromethane (40 mL) at room temperature, after which the resulting solution was stirred at the same temperature for 18 hours. Saturated sodium hydrogen carbonate aqueous solution was poured into the reaction mixture and an extraction was performed with dichloromethane. An organic layer was washed with saturated sodium chloride aqueous solution, then dehydrated with anhydrous magnesium sulfate, then filtered, and then concentrated under reduced pressure. The resulting concentrate was purified via column chromatography (SiO₂, 40 g cartridge; ethyl acetate/hexane = 50 to 100%) and concentrated, after which an obtained product was purified again via chromatography (SiO₂, 40 g cartridge; dichloromethane/methanol = 0 to 50%) and concentrated to obtain a title compound (1.573 g, 75.4%) in a brown solid form.

### [Step 6] Synthesis of the compound 89

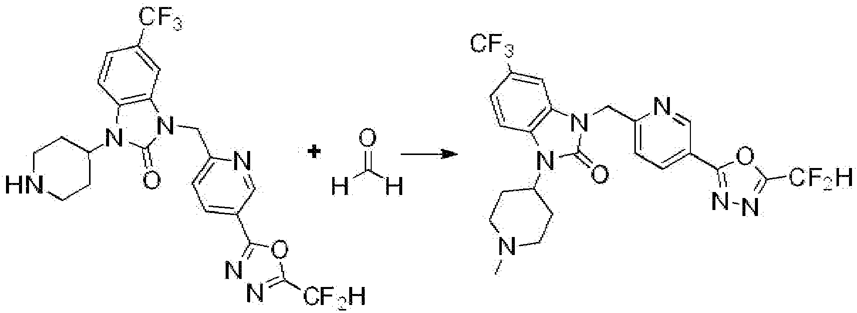

The 3-((5-(5-(difluoromethyl)-1,3,4-oxadiazole-2-yl)pyridine-2-yl)methyl)-1-(piperidine-4-yl)-5-(trifluor omethyl)-1,3-dihydro-2H-benzo[d]imidazole-2-one (0.100 g, 0.202 mmol) prepared in the step 5, sodium triacetoxyborohydride (0.086 g, 0.405 mmol), acetic acid (0.012 mL, 0.202 mmol) and formaldehyde (0.012 g, 0.405 mmol) were dissolved in dichloromethane (2 mL) at room temperature, after which the resulting solution was stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; dichloromethane/methanol = 0 to 5%) and concentrated to obtain a title compound (0.061 g, 59.3%) in a yellow solid form.

**¹H NMR** (400 MHz, CDCl₃) δ 9.26 (d, *J =* 2.1 Hz, 1H), 8.33 (dd, *J* = 8.2, 2.2 Hz, 1H), 7.44 - 7.25 (m, 4H), 6.91 (t, *J* = 51.6 Hz, 1H), 5.29 (s, 2H), 4.48 - 4.39 (m, 1H), 3.03 (d, *J* = 11.7 Hz, 2H), 2.55 - 2.43 (m, 2H), 2.34 (s, 3H), 2.17 (td, *J =* 12.0, 2.0 Hz, 2H), 1.86 - 1.82 (m, 2H); LRMS (ES) m/z 509.3 (M⁺+ 1).

### Example 90: Synthesis of Compound 90, 3-((5-(5-(difluoromethyl)-1,3,4-oxadiazole-2-yl)pyridine-2-yl)methyl)-1-(1-(oxetan-3-yl)piperidine-4-yl)-5-(trifluoromethyl)-1,3-dihydro-2H-benzo[d]imidazole-2-one

### [Step 1] Synthesis of the compound 90

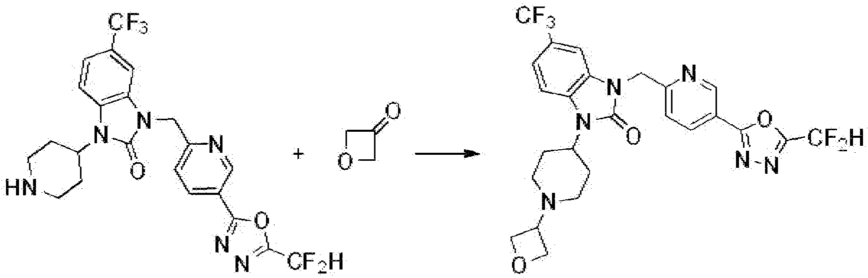

The 3-((5-(5-(difluoromethyl)-1,3,4-oxadiazole-2-yl)pyridine-2-yl)methyl)-1-(piperidine-4-yl)-5-(trifluor omethyl)-1,3-dihydro-2H-benzo[d]imidazole-2-one (0.100 g, 0.202 mmol) prepared in the step 5 of the compound 89, sodium triacetoxyborohydride (0.086 g, 0.405 mmol), acetic acid (0.012 mL, 0.202 mmol) and oxetan-3-one (0.029 g, 0.405 mmol) were dissolved in dichloromethane (2 mL) at room temperature, after which the resulting solution was stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; dichloromethane/methanol = 0 to 5%) and concentrated to obtain a title compound (0.083 g, 74.9%) in a yellow solid form.

**¹H NMR** (400 MHz, CDCl₃) δ 9.26 - 9.25 (m, 1H), 8.33 (dd, *J* = 8.2, 2.2 Hz, 1H), 7.44 - 7.34 (m, 3H), 7.26 (s, 1H), 6.92 (t, *J =* 51.6 Hz, 1H), 5.28 (s, 2H), 4.64 (dt, *J =* 22.9, 6.4 Hz, 4H), 4.49 - 4.40 (m, 1H), 3.56 - 3.50 (m, 1H), 2.91 (d, *J =* 11.5 Hz, 2H), 2.52 - 2.42 (m, 2H), 2.05 - 2.00 (m, 2H), 1.89 - 1.85 (m, 2H); **LRMS** (ES) m/z 551.6 (M⁺ + 1).

### Example 91: Synthesis of Compound 91, 1-((5-(5-(difluoromethyl)-1,3,4-oxadiazole-2-yl)pyridine-2-yl)methyl)-5,6-difluoro-3-(1-methylpiper idine-4-yl)-1,3-dihydro-2H-benzo[d]imidazole-2-one

### [Step 1] Synthesis of tert-butyl 4-(3-(4-(5-(difluoromethyl)-1,3,4-oxadiazole-2-yl)-2-fluorobenzyl)-2-oxo-5-(trifluoromethyl)-2,3-di hydro-1H-benzo[d]imidazole-1-yl)piperidine-1-carboxylate

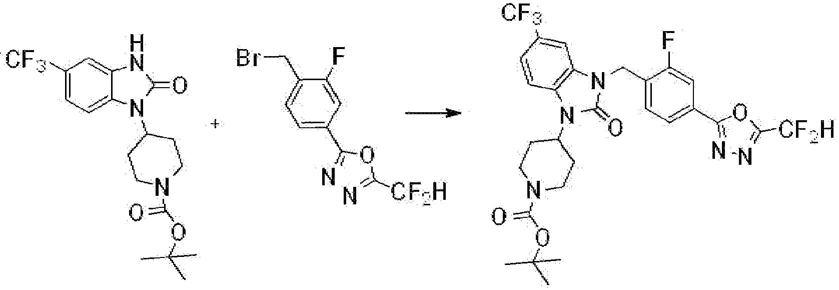

The tert-butyl 4-(2-oxo-5-(trifluoromethyl)-2,3-dihydro-1H-benzo[d]imidazole-1-yl)piperidine-1-carboxylate (2.500 g, 6.487 mmol) prepared in the step 3 of the compound 89 was dissolved in N,N-dimethylformamide (65 mL) at 0°C, after which sodium hydride (60.00%, 0.389 g, 9.730 mmol) was added into the resulting solution and stirred at the same temperature for 30 minutes. 2-(4-(bromomethyl)-3-fluorophenyl)-5-(difluoromethyl)-1,3,4-oxadiazole (2.590 g, 8.433 mmol) was added into the reaction mixture and further stirred at room temperature for 5 hours. Water was poured into the reaction mixture and an extraction was performed with ethyl acetate. An organic layer was washed with saturated sodium chloride aqueous solution, then dehydrated with anhydrous magnesium sulfate, then filtered, and then concentrated under reduced pressure. The resulting concentrate was purified via column chromatography (SiO₂, 40 g cartridge; ethyl acetate/hexane = 0 to 50%) and concentrated to obtain a title compound (2.821 g, 71.1%) in a white solid form.

### [Step 2] Synthesis of 3-(4-(5-(difluoromethyl)-1,3,4-oxadiazole-2-yl)-2-fluorobenzyl)-1-(piperidine-4-yl)-5-(trifluorometh yl)-1,3-dihydro-2H-benzo[d]imidazole-2-one 2,2,2-trifluoroacetate

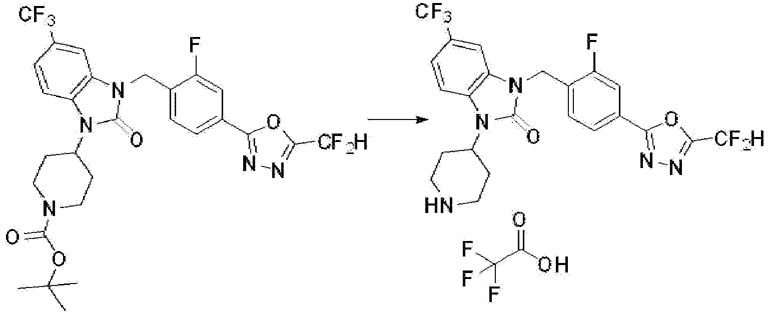

The tert-butyl 4-(3-(4-(5-(difluoromethyl)-1,3,4-oxadiazole-2-yl)-2-fluorobenzyl)-2-oxo-5-(trifluoromethyl)-2,3-di hydro-1H-benzo[d]imidazole-1-yl)piperidine-1-carboxylate (3.500 g, 5.723 mmol) prepared in the step 1 and trifluoroacetic acid (4.383 mL, 57.232 mmol) were dissolved in dichloromethane (50 mL) at room temperature, after which the resulting solution was stirred at the same temperature for 5 hours. Saturated sodium hydrogen carbonate aqueous solution was poured into the reaction mixture and an extraction was performed with dichloromethane. An organic layer was washed with saturated sodium chloride aqueous solution, then dehydrated with anhydrous magnesium sulfate, then filtered, and then concentrated under reduced pressure. A title compound was used without an additional purification process (4.770 g, 133.3%, light yellow solid).

### [Step 3] Synthesis of the compound 91

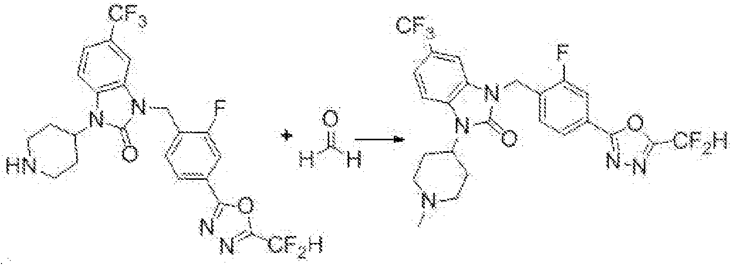

The 1-((5-(5-(difluoromethyl)-1,3,4-oxadiazole-2-yl)pyridine-2-yl)methyl)-5,6-difluoro-3-(piperidine-4-y l)-1,3-dihydro-2H-benzo[d]imidazole-2-one (0.100 g, 0.196 mmol) prepared in the step 2, sodium triacetoxyborohydride (0.083 g, 0.391 mmol), acetic acid (0.011 mL, 0.196 mmol) and formaldehyde (0.012 g, 0.391 mmol) were dissolved in dichloromethane (2 mL) at room temperature, after which the resulting solution was stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; dichloromethane/methanol = 0 to 5%) and concentrated to obtain a title compound (0.079 g, 77.0%) in a white solid form.

**¹H NMR** (400 MHz, CDCl₃) δ 7.85 - 7.83 (m, 2H), 7.46 - 7.32 (m, 3H), 7.19 (s, 1H), 6.89 (t, *J* = 51.7 Hz, 1H), 5.20 (s, 2H), 4.47 - 4.39 (m, 1H), 3.02 (d, *J* = 11.6 Hz, 2H), 2.51 - 2.41 (m, 2H), 2.34 (s, 3H), 2.19 - 2.12 (m, 2H), 1.85 - 1.81 (m, 2H); **LRMS** (ES) m/z 526.1 (M⁺ + 1).

### Example 92: Synthesis of Compound 92, 3-(4-(5-(difluoromethyl)-1,3,4-oxadiazole-2-yl)-2-fluorobenzyl)-1-(1-(oxetan-3-yl)piperidine-4-yl)-5-(trifluoromethyl)-1,3-dihydro-2H-benzo[d]imidazole-2-one

### [Step 1] Synthesis of the compound 92

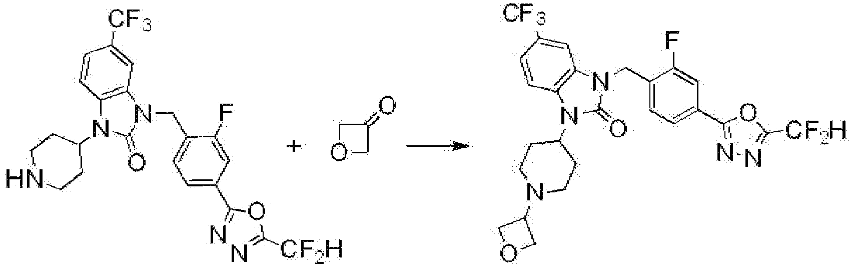

The 3-(4-(5-(difluoromethyl)-1,3,4-oxadiazole-2-yl)-2-fluorobenzyl)-1-(piperidine-4-yl)-5-(trifluorometh yl)-1,3-dihydro-2H-benzo[d]imidazole-2-one (0.100 g, 0.196 mmol) prepared in the step 2 of the compound 91, sodium triacetoxyborohydride (0.083 g, 0.391 mmol), acetic acid (0.011 mL, 0.196 mmol) and oxetan-3-one (0.028 g, 0.391 mmol) were dissolved in dichloromethane (2 mL) at room temperature, after which the resulting solution was stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; dichloromethane/methanol = 0 to 5%) and concentrated to obtain a title compound (0.093 g, 83.7%) in a white solid form.

**¹H NMR** (400 MHz, CDCl₃) δ 7.84 (d, *J* = 9.1 Hz, 1H), 7.83 (s, 1H), 7.47 - 7.34 (m, 3H), 7.21 (s, 1H), 6.90 (t, *J* = 51.7 Hz, 1H), 5.20 (s, 2H), 4.64 (dt, *J* = 23.1, 6.4 Hz, 4H), 4.48 - 4.40 (m, 1H), 3.56 - 3.50 (m, 1H), 2.91 (d, *J =* 11.5 Hz, 2H), 2.51 - 2.41 (m, 2H), 2.05 - 2.00 (m, 2H), 1.88 - 1.85 (m, 2H); **LRMS** (ES) m/z 568.2 (M⁺ + 1).

### Example 93: Synthesis of Compound 93, 1-(4-(5-(difluoromethyl)-1,3,4-oxadiazole-2-yl)-2-fluorobenzyl)-5,6-difluoro-3-(1-methylpiperidine-4-yl)-1,3-dihydro-2H-benzo[d]imidazole-2-one

### [Step 1] Synthesis of tert-butyl 4-(3-(4-(5-(difluoromethyl)-1,3,4-oxadiazole-2-yl)-2-fluorobenzyl)-5,6-difluoro-2-oxo-2,3-dihydro-1H-benzo[d]imidazole-1-yl)piperidine-1-carboxylate

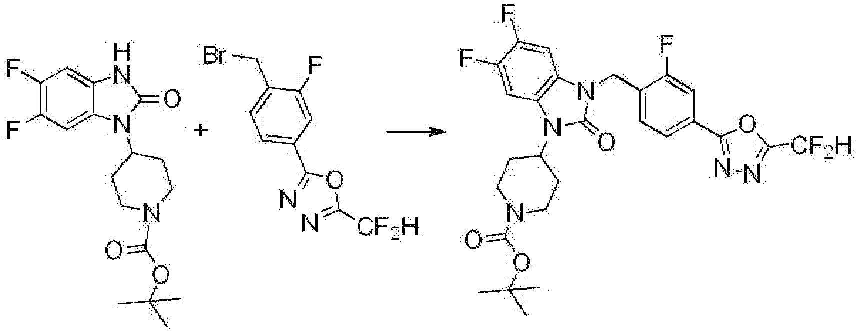

The tert-butyl 4-(5,6-difluoro-2-oxo-2,3-dihydro-1H-benzo[d]imidazole-1-yl)piperidine-1-carboxylate (1.600 g, 4.528 mmol) prepared in the step 2 of the compound 83 was dissolved in N,N-dimethylformamide (50 mL) at 0°C, after which sodium hydride (60.00%, 0.272 g, 6.792 mmol) was added into the resulting solution and stirred at the same temperature for 30 minutes. 2-(4-(bromomethyl)-3-fluorophenyl)-5-(difluoromethyl)-1,3,4-oxadiazole (1.807 g, 5.886 mmol) was added into the reaction mixture and further stirred at room temperature for 5 hours. Water was poured into the reaction mixture and an extraction was performed with ethyl acetate. An organic layer was washed with saturated sodium chloride aqueous solution, then dehydrated with anhydrous magnesium sulfate, then filtered, and then concentrated under reduced pressure. The resulting concentrate was purified via column chromatography (SiO₂, 40 g cartridge; ethyl acetate/hexane = 0 to 70%) and concentrated to obtain a title compound (1.270 g, 48.4%) in a light yellow solid form.

### [Step 2] Synthesis of 1-(4-(5-(difluoromethyl)-1,3,4-oxadiazole-2-yl)-2-fluorobenzyl)-5,6-difluoro-3-(piperidine-4-yl)-1,3 -dihydro-2H-benzo[d]imidazole-2-one

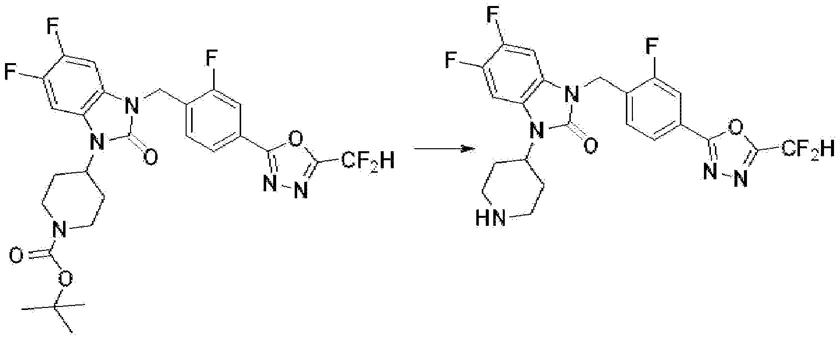

The tert-butyl 4-(3-(4-(5-(difluoromethyl)-1,3,4-oxadiazole-2-yl)-2-fluorobenzyl)-5,6-difluoro-2-oxo-2,3-dihydro-1H-benzo[d]imidazole-1-yl)piperidine-1-carboxylate (1.270 g, 2.191 mmol) prepared in the step 1 and trifluoroacetic acid (1.678 mL, 21.914 mmol) were dissolved in dichloromethane (20 mL) at room temperature, after which the resulting solution was stirred at the same temperature for 18 hours. Saturated sodium hydrogen carbonate aqueous solution was poured into the reaction mixture and an extraction was performed with dichloromethane. An organic layer was washed with saturated sodium chloride aqueous solution, then dehydrated with anhydrous magnesium sulfate, then filtered, and then concentrated under reduced pressure. The resulting concentrate was purified via column chromatography (SiO₂, 40 g cartridge; dichloromethane/methanol = 0 to 50%) and concentrated to obtain a title compound (0.401 g, 38.1%) in a white solid form.

### [Step 3] Synthesis of the compound 93

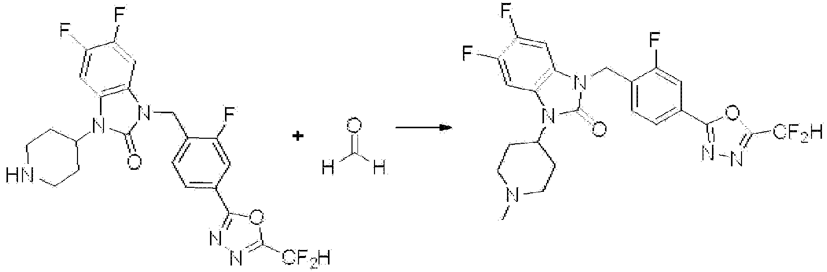

The 1-(4-(5-(difluoromethyl)-1,3,4-oxadiazole-2-yl)-2-fluorobenzyl)-5,6-difluoro-3-(piperidine-4-yl)-1,3 -dihydro-2H-benzo[d]imidazole-2-one (0.100 g, 0.209 mmol) prepared in the step 2, sodium triacetoxyborohydride (0.088 g, 0.417 mmol), acetic acid (0.012 mL, 0.209 mmol) and formaldehyde (0.013 g, 0.417 mmol) were dissolved in dichloromethane (2 mL) at room temperature, after which the resulting solution was stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; dichloromethane/methanol = 0 to 5%) and concentrated to obtain a title compound (0.051 g, 49.1%) in a white solid form.

**¹H NMR** (400 MHz, CDCl₃) δ 7.84 (d, *J* = 8.7 Hz, 2H), 7.44 (t, *J =* 7.6 Hz, 1H), 7.17 (dd, *J* = 10.3, 6.7 Hz, 1H), 7.03 - 6.76 (m, 2H), 5.12 (s, 2H), 4.41 - 4.32 (m, 1H), 3.01 (d, *J* = 11.7 Hz, 2H), 2.43 - 2.33 (m, 5H), 2.17 - 2.11 (m, 2H), 1.83 - 1.79 (m, 2H); **LRMS** (ES) m/z 494.4 (M⁺ + 1).

### Example 94: Synthesis of Compound 94, 1-(4-(5-(difluoromethyl)-1,3,4-oxadiazole-2-yl)-2-fluorobenzyl)-5,6-difluoro-3-(1-(oxetan-3-yl)pipe ridine-4-yl)-1,3-dihydro-2H-benzo[d]imidazole-2-one

### [Step 1] Synthesis of the compound 94

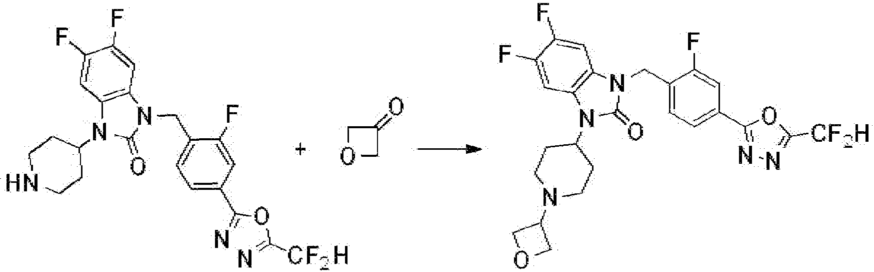

The 1-(4-(5-(difluoromethyl)-1,3,4-oxadiazole-2-yl)-2-fluorobenzyl)-5,6-difluoro-3-(piperidine-4-yl)-1,3 -dihydro-2H-benzo[d]imidazole-2-one (0.100 g, 0.209 mmol) prepared in the step 2 of the compound 93, sodium triacetoxyborohydride (0.088 g, 0.417 mmol), acetic acid (0.012 mL, 0.209 mmol) and oxetan-3-one (0.030 g, 0.417 mmol) were dissolved in dichloromethane (2 mL) at room temperature, after which the resulting solution was stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; dichloromethane/methanol = 0 to 5%) and concentrated to obtain a title compound (0.083 g, 74.7%) in a white solid form.

**¹H NMR** (400 MHz, CDCl₃) δ 7.84 - 7.81 (m, 2H), 7.44 (t, *J* = 7.6 Hz, 1H), 7.16 (dd, *J* = 10.3, 6.7 Hz, 1H), 7.03 - 6.77 (m, 2H), 5.11 (s, 2H), 4.69 (dt, *J* = 21.2, 36.2 Hz, 4H), 4.41 - 4.32 (m, 1H), 3.55 - 3.49 (m, 1H), 2.90 (d, *J* = 11.5 Hz, 2H), 2.43 - 2.33 (m, 2H), 2.03 - 1.98 (m, 2H), 1.85 - 1.82 (m, 2H); **LRMS** (ES) m/z 536.2 (M⁺ + 1).

### Example 95: Synthesis of Compound 95, 1-(4-(5-(difluoromethyl)-1,3,4-oxadiazole-2-yl)benzyl)-5,6-difluoro-3-(1-methylpiperidine-4-yl)-1,3 -dihydro-2H-benzo[d]imidazole-2-one

### [Step 1] Synthesis of tert-butyl 4-(3-(4-(5-(difluoromethyl)-1,3,4-oxadiazole-2-yl)benzyl)-5,6-difluoro-2-oxo-2,3-dihydro-1H-benz o[d]imidazole-1-yl)piperidine-1-carboxylate

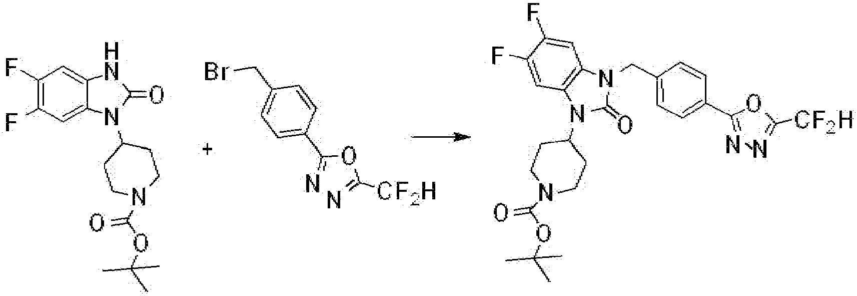

The tert-butyl 4-(5,6-difluoro-2-oxo-2,3-dihydro-1H-benzo[d]imidazole-1-yl)piperidine-1-carboxylate (1.600 g, 4.528 mmol) prepared in the step 2 of the compound 83 was dissolved in N,N-dimethylformamide (45 mL) at 0°C, after which sodium hydride (60.00%, 0.272 g, 6.792 mmol) was added into the resulting solution and stirred at the same temperature for 30 minutes. 2-(4-(bromomethyl)phenyl)-5-(difluoromethyl)-1,3,4-oxadiazole (1.702 g, 5.886 mmol) was added into the reaction mixture and further stirred at room temperature for 5 hours. Water was poured into the reaction mixture and an extraction was performed with ethyl acetate. An organic layer was washed with saturated sodium chloride aqueous solution, then dehydrated with anhydrous magnesium sulfate, then filtered, and then concentrated under reduced pressure. The resulting concentrate was purified via column chromatography (SiO₂, 40 g cartridge; ethyl acetate/hexane = 0 to 50%) and concentrated to obtain a title compound (1.869 g, 73.5%) in a light yellow solid form.

### [Step 2] Synthesis of 1-(4-(5-(difluoromethyl)-1,3,4-oxadiazole-2-yl)benzyl)-5,6-difluoro-3-(piperidine-4-yl)-1,3-dihydro -2H-benzo[d]imidazole-2-one

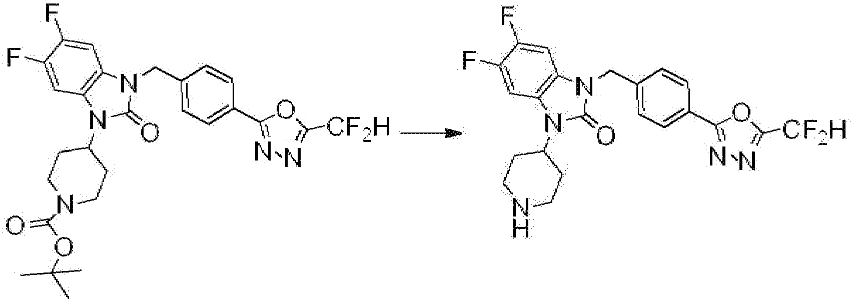

The tert-butyl 4-(3-(4-(5-(difluoromethyl)-1,3,4-oxadiazole-2-yl)benzyl)-5,6-difluoro-2-oxo-2,3-dihydro-1H-benz o[d]imidazole-1-yl)piperidine-1-carboxylate (1.869 g, 3.328 mmol) prepared in the step 1 and trifluoroacetic acid (2.549 mL, 33.283 mmol) were dissolved in dichloromethane (20 mL) at room temperature, after which the resulting solution was stirred at the same temperature for 18 hours. Saturated sodium hydrogen carbonate aqueous solution was poured into the reaction mixture and an extraction was performed with dichloromethane. An organic layer was washed with saturated sodium chloride aqueous solution, then dehydrated with anhydrous magnesium sulfate, then filtered, and then concentrated under reduced pressure. The resulting concentrate was purified via column chromatography (SiO₂, 40 g cartridge; ethyl acetate/hexane = 50 to 100%) and concentrated, after which an obtained product was purified again via chromatography (SiO₂, 40 g cartridge; dichloromethane/1%-methanol aqueous solution = 0 to 50%) and concentrated to obtain a title compound (1.152 g, 75.0%) in a white solid form.

### [Step 3] Synthesis of the compound 95

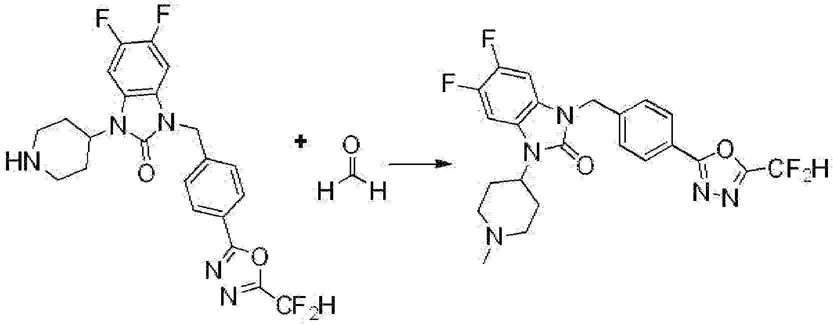

The 1-(4-(5-(difluoromethyl)-1,3,4-oxadiazole-2-yl)benzyl)-5,6-difluoro-3-(piperidine-4-yl)-1,3-dihydro -2H-benzo[d]imidazole-2-one (0.100 g, 0.217 mmol) prepared in the step 2, sodium triacetoxyborohydride (0.092 g, 0.433 mmol), acetic acid (0.012 mL, 0.217 mmol) and formaldehyde (0.013 g, 0.433 mmol) were dissolved in dichloromethane (2 mL) at room temperature, after which the resulting solution was stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; dichloromethane/methanol = 0 to 5%) and concentrated to obtain a title compound (0.061 g, 59.2%) in a white solid form.

**¹H NMR** (400 MHz, CDCl₃) δ 8.05 (d, CDC*J* = 8.4 Hz, 2H), 7.42 (d, *J =* 8.4 Hz, 2H), 7.18 (dd, *J* = 10.5, 6.6 Hz, 1H), 6.89 (t, *J* = 51.7 Hz, 1H), 6.63 (dd, *J* = 9.6, 6.8 Hz, 1H), 5.07 (s, 2H), 4.42 -4.34 (m, 1H), 3.02 (d, *J* = 11.7 Hz, 2H), 2.45 - 2.33 (m, 5H), 2.18 - 2.13 (m, 2H), 1.84 - 1.80 (m, 2H); **LRMS** (ES) m/z 476.3 (M⁺ + 1).

### Example 96: Synthesis of Compound 96, 1-(4-(5-(difluoromethyl)-1,3,4-oxadiazole-2-yl)benzyl)-5,6-difluoro-3-(1-(oxetan-3-yl)piperidine-4-yl)-1,3-dihydro-2H-benzo[d]imidazole-2-one

### [Step 1] Synthesis of the compound 96

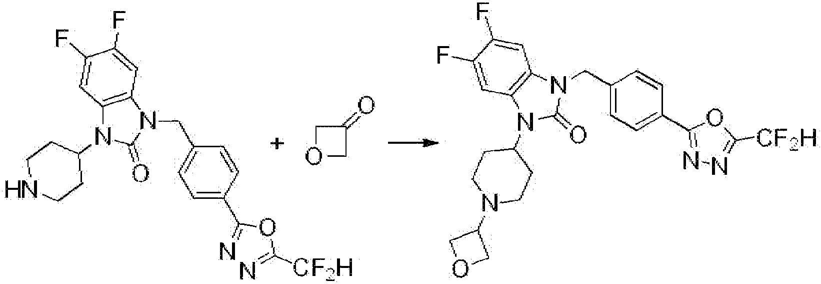

The 1-(4-(5-(difluoromethyl)-1,3,4-oxadiazole-2-yl)benzyl)-5,6-difluoro-3-(piperidine-4-yl)-1,3-dihydro -2H-benzo[d]imidazole-2-one (0.100 g, 0.217 mmol) prepared in the step 2 of the compound 95, sodium triacetoxyborohydride (0.092 g, 0.433 mmol), acetic acid (0.012 mL, 0.217 mmol) and oxetan-3-one (0.031 g, 0.433 mmol) were dissolved in dichloromethane (2 mL) at room temperature, after which the resulting solution was stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; dichloromethane/methanol = 0 to 5%) and concentrated to obtain a title compound (0.099 g, 88.2%) in a white solid form.

**¹H NMR** (400 MHz, CDCl₃) δ 8.09 (dd, *J* = 7.5, 2.7 Hz, 2H), 7.45 (d, *J =* 8.4 Hz, 2H), 7.18 (dd, *J* = 10.3, 6.7 Hz, 1H), 6.90 (t, *J =* 51.7 Hz, 1H), 6.65 (dd, *J* = 9.6, 6.8 Hz, 1H), 5.10 (s, 2H), 4.67 (dt, *J* = 20.8, 6.4 Hz, 4H), 4.45 - 4.37 (m, 1H), 3.58 - 3.53 (m, 1H), 2.92 (d, *J* = 11.4 Hz, 2H), 2.45 - 2.35 (m, 2H), 2.04 (td, *J* = 11.8, 1.9 Hz, 2H), 1.89 - 1.86 (m, 2H); **LRMS** (ES) m/z 518.5 (M⁺ + 1).

### Example 97: Synthesis of Compound 97, 5-chloro-1-((5-(5-(difluoromethyl)-1,3,4-oxadiazole-2-yl)pyridine-2-yl)methyl)-3-(1-methylpiperidi ne-4-yl)-1,3-dihydro-2H-benzo[d]imidazole-2-one

### [Step 1] Synthesis of tert-butyl 4-((5-chloro-2-nitrophenyl)amino)piperidine-1-carboxylate

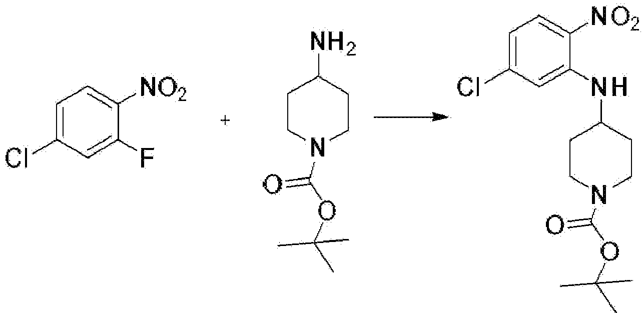

4-chloro-2-fluoro-1-nitrobenzene (5.000 g, 28.484 mmol), tert-butyl 4-aminopiperidine-1-carboxylate (5.990 g, 29.908 mmol), potassium carbonate (7.873 g, 56.967 mmol) and potassium iodide(0.473 g, 2.848 mmol) were dissolved in N,N-dimethylformamide (300 mL) at room temperature, after which the resulting solution was stirred at 80°C for 18 hours, and then a reaction was finished by lowering a temperature to room temperature. The reaction mixture was filtered via a paper filter to remove a solid therefrom, after which solvent was removed from the resulting filtrate under reduced pressure. Then, the resulting concentrate was purified via column chromatography (SiO₂, 120 g cartridge; ethyl acetate/hexane = 0 to 50%) and concentrated to obtain a title compound (9.450 g, 93.2%) in an orange liquid form.

### [Step 2] Synthesis of tert-butyl 4-((2-amino-5-chlorophenyl)amino)piperidine-1-carboxylate

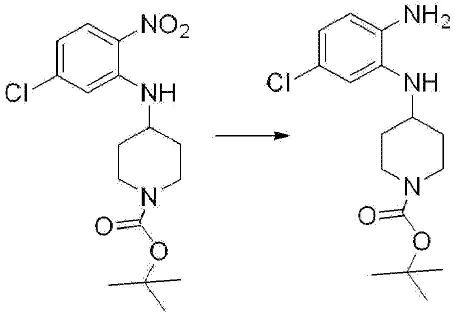

The tert-butyl 4-((5-chloro-2-nitrophenyl)amino)piperidine-1-carboxylate (9.450 g, 26.558 mmol) prepared in the step 1 and Raney Ni 10 wt% were dissolved in methanol (200 mL) at room temperature, after which the resulting solution was stirred at the same temperature for 18 hours in the presence of a hydrogen balloon. The reaction mixture was filtered via a celite pad to remove a solid therefrom, after which solvent was removed from the resulting filtrate under reduced pressure. Then, water was poured into the resulting concentrate and then an extraction was performed with ethyl acetate. An organic layer was washed with saturated sodium chloride aqueous solution, then dehydrated with anhydrous magnesium sulfate, then filtered, and then concentrated under reduced pressure. A title compound was used without an additional purification process (8.270 g, 95.6%, brown solid).

### [Step 3] Synthesis of tert-butyl 4-(6-chloro-2-oxo-2,3-dihydro-1H-benzo[d]imidazole-1-yl)piperidine-1-carboxylate

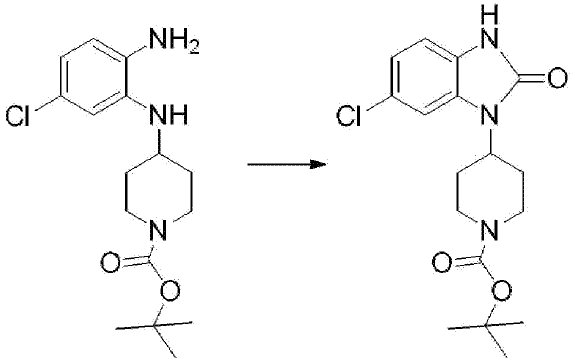

The tert-butyl 4-((2-amino-5-chlorophenyl)amino)piperidine-1-carboxylate (8.270 g, 25.381 mmol) prepared in the step 2 and 1,1'-carbonyldiimidazole (CDI, 5.762 g, 35.533 mmol) were dissolved in N,N-dimethylformamide (250 mL) at room temperature, after which the resulting solution was stirred at the same temperature for 18 hours. Solvent was removed from the reaction mixture under reduced pressure, after which water was poured into the resulting concentrate, and then an extraction was performed with ethyl acetate. An organic layer was washed with saturated sodium chloride aqueous solution, then dehydrated with anhydrous magnesium sulfate, then filtered, and then concentrated under reduced pressure. The resulting concentrate was purified via column chromatography (SiO₂, 120 g cartridge; ethyl acetate/hexane = 0 to 100%) and concentrated to obtain a title compound (6.720 g, 75.3%) in a brown solid form.

### [Step 4] Synthesis of tert-butyl 4-(6-chloro-3-((5-(5-(difluoromethyl)-1,3,4-oxadiazole-2-yl)pyridine-2-yl)methyl)-2-oxo-2,3-dihydr o-1H-benzo[d]imidazole-1-yl)piperidine-1-carboxylate

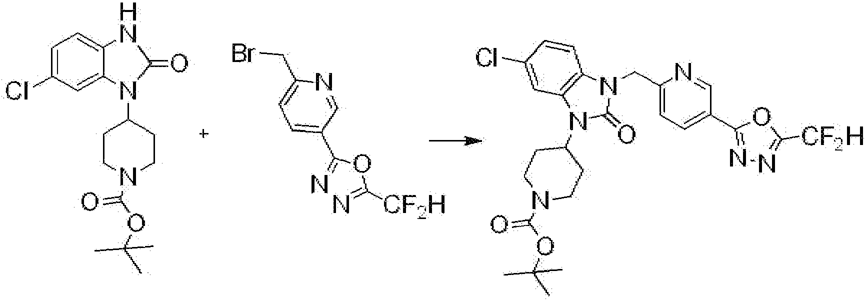

The tert-butyl 4-(6-chloro-2-oxo-2,3-dihydro-1H-benzo[d]imidazole-1-yl)piperidine-1-carboxylate (2.200 g, 6.253 mmol) prepared in the step 3 was dissolved in N,N-dimethylformamide (50 mL) at 0°C, after which sodium hydride (60.00%, 0.375 g, 9.380 mmol) was added into the resulting solution and stirred at the same temperature for 30 minutes. 2-(6-(bromomethyl)pyridine-3-yl)-5-(difluoromethyl)-1,3,4-oxadiazole (2.358 g, 8.129 mmol) was added into the reaction mixture and further stirred at room temperature for 2.5 hours. Water was poured into the reaction mixture and an extraction was performed with ethyl acetate. An organic layer was washed with saturated sodium chloride aqueous solution, then dehydrated with anhydrous magnesium sulfate, then filtered, and then concentrated under reduced pressure. The resulting concentrate was purified via column chromatography (SiO₂, 40 g cartridge; ethyl acetate/hexane = 0 to 100%) and concentrated to obtain a title compound (2.124 g, 60.5%) in a brown solid form.

### [Step 5] Synthesis of 5-chloro-1-((5-(5-(difluoromethyl)-1,3,4-oxadiazole-2-yl)pyridine-2-yl)methyl)-3-(piperidine-4-yl)-1,3-dihydro-2H-benzo[d]imidazole-2-one

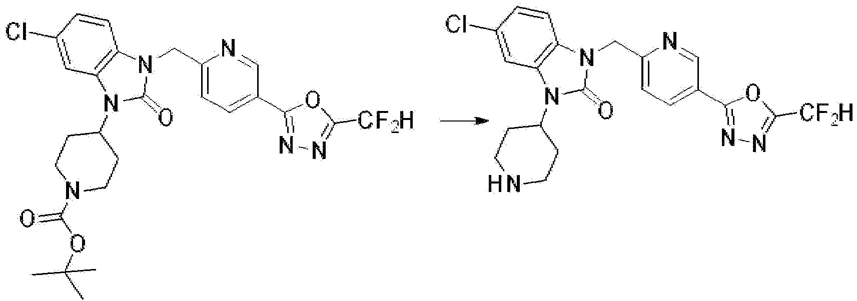

The tert-butyl 4-(6-chloro-3-((5-(5-(difluoromethyl)-1,3,4-oxadiazole-2-yl)pyridine-2-yl)methyl)-2-oxo-2,3-dihydr o-1H-benzo[d]imidazole-1-yl)piperidine-1-carboxylate (2.124 g, 3.786 mmol) prepared in the step 4 and trifluoroacetic acid (2.899 mL, 37.862 mmol) were dissolved in dichloromethane (30 mL) at room temperature, after which the resulting solution was stirred at the same temperature for 4 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 40 g cartridge; ethyl acetate/hexane = 80 to 100%) and concentrated, after which an obtained product was purified again via chromatography (SiO₂, 40 g cartridge; methanol/dichloromethane = 0 to 70%) and concentrated to obtain a title compound (1.210 g, 69.3%) in a brown solid form.

### [Step 6] Synthesis of the compound 97

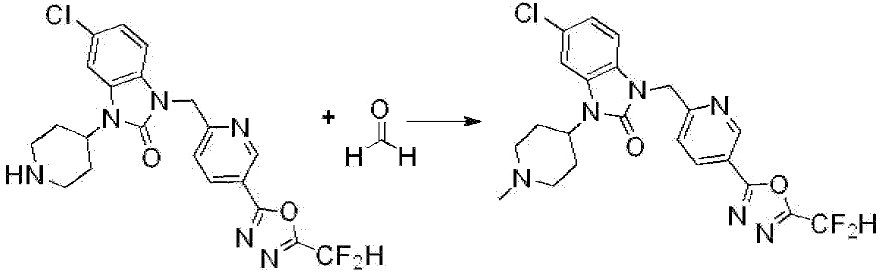

The 5-chloro-1-((5-(5-(difluoromethyl)-1,3,4-oxadiazole-2-yl)pyridine-2-yl)methyl)-3-(piperidine-4-yl)-1,3-dihydro-2H-benzo[d]imidazole-2-one (0.100 g, 0.217 mmol) prepared in the step 5, sodium triacetoxyborohydride (0.092 g, 0.434 mmol) , acetic acid (0.012 mL, 0.217 mmol) and formaldehyde (0.020 g, 0.651 mmol) were dissolved in dichloromethane (2 mL) at room temperature, after which the resulting solution was stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.065 g, 62.8%) in a red solid form.

¹H NMR (400 MHz, CDCl₃) δ 9.24 (dd, *J =* 2.3, 0.4 Hz, 1H), 8.29 (dd, *J* = 8.2, 2.2 Hz, 1H), 7.37 (d, *J* = 7.9 Hz, 1H), 7.32 (d, *J =* 1.8 Hz, 1H), 7.04 - 6.78 (m, 3H), 5.23 (s, 2H), 4.42 - 4.34 (m, 1H), 3.01 (d, *J* = 11.6 Hz, 2H), 2.48 - 2.38 (m, 2H), 2.33 (s, 3H), 2.14 (td, *J* = 12.0, 2.1 Hz, 2H), 1.83 - 1.80 (m, 2H); LRMS (ES) m/z 475.5 (M⁺ + 1).

### Example 98: Synthesis of Compound 98, 5-chloro-1-((5-(5-(difluoromethyl)-1,3,4-oxadiazole-2-yl)pyridine-2-yl)methyl)-3-(1-(oxetan-3-yl)pi peridine-4-yl)-1,3-dihydro-2H-benzo[d]imidazole-2-one

### [Step 1] Synthesis of the compound 98

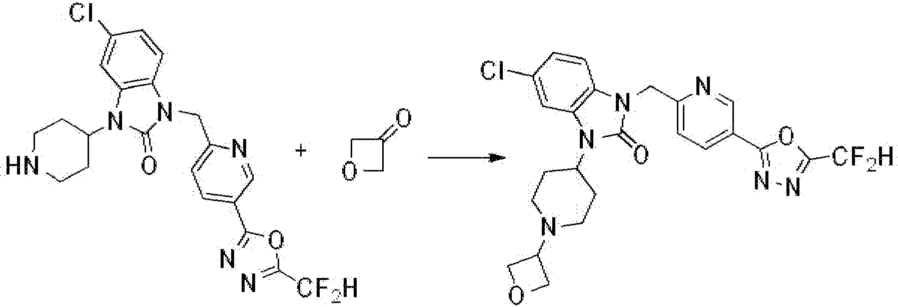

The 5-chloro-1-((5-(5-(difluoromethyl)-1,3,4-oxadiazole-2-yl)pyridine-2-yl)methyl)-3-(piperidine-4-yl)-1,3-dihydro-2H-benzo[d]imidazole-2-one (0.100 g, 0.217 mmol) prepared in the step 5 of the compound 97, sodium triacetoxyborohydride (0.092 g, 0.434 mmol), acetic acid (0.012 mL, 0.217 mmol) and oxetan-3-one (0.047 g, 0.651 mmol) were dissolved in dichloromethane (2 mL) at room temperature, after which the resulting solution was stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.072 g, 64.1%) in a yellow solid form.

**¹H NMR** (400 MHz, CDCl₃) δ 9.23 (dd, *J =* 2.2, 0.7 Hz, 1H), 8.29 (dd, *J* = 8.2, 2.2 Hz, 1H), 7.38 (d, *J* = 8.2 Hz, 1H), 7.30 (d, *J* = 1.8 Hz, 1H), 7.04 - 6.78 (m, 3H), 5.22 (s, 2H), 4.64 (dt, *J =* 16.8, 6.4 Hz, 4H), 4.43 - 4.34 (m, 1H), 3.56 - 3.49 (m, 1H), 2.91 (d, *J* = 11.5 Hz, 2H), 2.48 - 2.37 (m, 2H), 2.04 - 1.98 (m, 2H), 1.86 - 1.83 (m, 2H); LRMS (ES) m/z 517.4 (M⁺+ 1).

### Example 99: Synthesis of Compound 99, 5-chloro-1-(4-(5-(difluoromethyl)-1,3,4-oxadiazole-2-yl)benzyl)-3-(1-methylpiperidine-4-yl)-1,3-di hydro-2H-benzo[d]imidazole-2-one

### [Step 1] Synthesis of tert-butyl 4-(6-chloro-3-(4-(5-(difluoromethyl)-1,3,4-oxadiazole-2-yl)benzyl)-2-oxo-2,3-dihydro-1H-benzo[d] imidazole-1-yl)piperidine-1-carboxylate

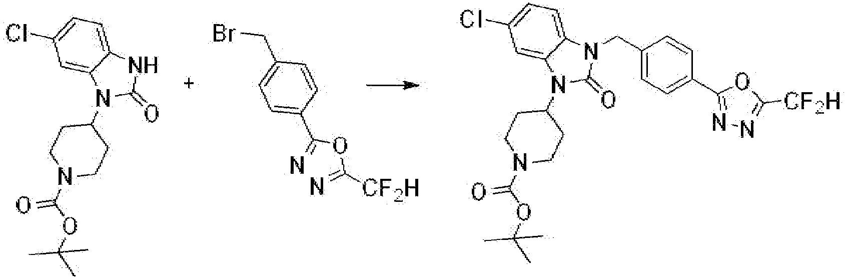

The tert-butyl 4-(6-chloro-2-oxo-2,3-dihydro-1H-benzo[d]imidazole-1-yl)piperidine-1-carboxylate (2.200 g, 6.253 mmol) prepared in the step 3 of the compound 97 was dissolved in N,N-dimethylformamide (60 mL) at 0°C, after which sodium hydride (60.00%, 0.375 g, 9.380 mmol) was added into the resulting solution and stirred at the same temperature for 30 minutes. 2-(4-(bromomethyl)phenyl)-5-(difluoromethyl)-1,3,4-oxadiazole (2.350 g, 8.129 mmol) was added into the reaction mixture and further stirred at room temperature for 4 hours. Water was poured into the reaction mixture and an extraction was performed with ethyl acetate. An organic layer was washed with saturated sodium chloride aqueous solution, then dehydrated with anhydrous magnesium sulfate, then filtered, and then concentrated under reduced pressure. The resulting concentrate was purified via column chromatography (SiO₂, 40 g cartridge; ethyl acetate/hexane = 0 to 30%) and concentrated to obtain a title compound (2.251 g, 64.3%) in a brown solid form.

### [Step 2] Synthesis of 5-chloro-1-(4-(5-(difluoromethyl)-1,3,4-oxadiazole-2-yl)benzyl)-3-(piperidine-4-yl)-1,3-dihydro-2H -benzo[d]imidazole-2-one

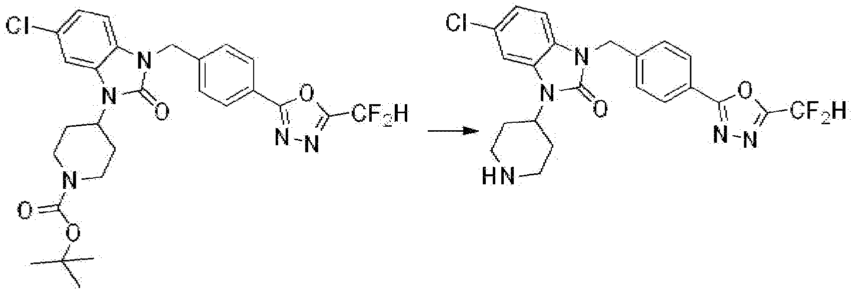

The tert-butyl 4-(6-chloro-3-(4-(5-(difluoromethyl)-1,3,4-oxadiazole-2-yl)benzyl)-2-oxo-2,3-dihydro-1H-benzo[d] imidazole-1-yl)piperidine-1-carboxylate (2.251 g, 4.020 mmol) prepared in the step 1 and trifluoroacetic acid (3.078 mL, 40.196 mmol) were dissolved in dichloromethane (30 mL) at room temperature, after which the resulting solution was stirred at the same temperature for 4 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 24 g cartridge; ethyl acetate/hexane = 80 to 100%) and concentrated, after which an obtained product was purified again via chromatography (SiO₂, 24 g cartridge; methanol/dichloromethane = 0 to 60%) and concentrated to obtain a title compound (1.746 g, 94.5%) in a yellow solid form.

### [Step 3] Synthesis of the compound 99

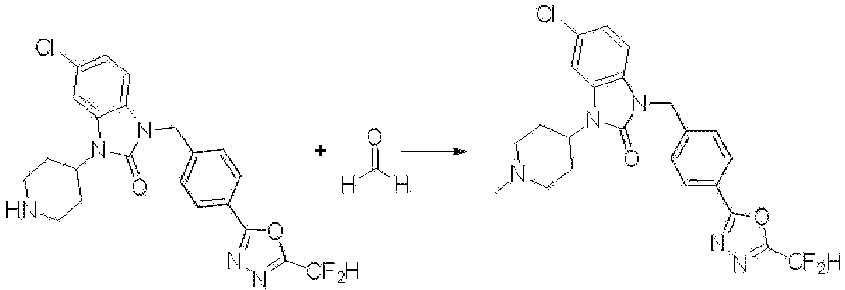

The 5-chloro-1-(4-(5-(difluoromethyl)-1,3,4-oxadiazole-2-yl)benzyl)-3-(piperidine-4-yl)-1,3-dihydro-2H -benzo[d]imidazole-2-one (0.100 g, 0.217 mmol) prepared in the step 2, sodium triacetoxyborohydride (0.092 g, 0.435 mmol), acetic acid (0.012 mL, 0.217 mmol) and formaldehyde (0.020 g, 0.652 mmol) were dissolved in dichloromethane (2 mL) at room temperature, after which the resulting solution was stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.051 g, 49.9%) in a yellow solid form.

**¹H NMR** (400 MHz, CDCl₃) δ 8.04 (d, *J =* 8.4 Hz, 2H), 7.42 (d, *J =* 8.5 Hz, 2H), 7.33 (d, *J* = 1.8 Hz, 1H), 6.94 (dd, *J* = 8.3, 1.9 Hz, 1H), 6.89 (t, *J =* 51.7 Hz, 1H), 6.71 (d, *J* = 8.4 Hz, 1H), 5.11 (s, 2H), 4.44 - 4.36 (m, 1H), 3.03 - 3.01 (m, 2H), 2.49 - 2.38 (m, 2H), 2.34 (s, 3H), 2.15 (td, *J* = 12.0, 2.1 Hz, 2H), 1.89 - 1.79 (m, 2H); **LRMS** (ES) m/z 474.4 (M⁺ + 1).

### Example 100: Synthesis of Compound 100, 5-chloro-1-(4-(5-(difluoromethyl)-1,3,4-oxadiazole-2-yl)benzyl)-3-(1-(oxetan-3-yl)piperidine-4-yl)-1,3-dihydro-2H-benzo[d]imidazole-2-one

### [Step 1] Synthesis of the compound 100

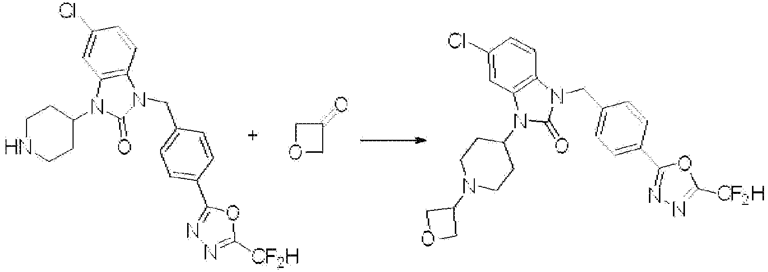

The 5-chloro-1-(4-(5-(difluoromethyl)-1,3,4-oxadiazole-2-yl)benzyl)-3-(piperidine-4-yl)-1,3-dihydro-2H -benzo[d]imidazole-2-one (0.100 g, 0.217 mmol) prepared in the step 2 of the compound 99, sodium triacetoxyborohydride (0.092 g, 0.435 mmol), acetic acid (0.012 mL, 0.217 mmol) and oxetan-3-one (0.047 g, 0.652 mmol) were dissolved in dichloromethane (2 mL) at room temperature, after which the resulting solution was stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.052 g, 46.6%) in a yellow solid form.

**¹H NMR** (400 MHz, CDCl₃) δ 8.05 (dd, *J* = 6.6, 1.7 Hz, 2H), 7.43 (d, *J =* 8.2 Hz, 2H), 7.32 (d, *J* = 1.8 Hz, 1H), 6.97 (dd, *J =* 8.4, 1.9 Hz, 1H), 6.89 (t, *J* = 51.7 Hz, 1H), 6.73 (d, *J* = 8.4 Hz, 1H), 5.11 (s, 2H), 4.66 (dt, *J* = 16.8, 6.4 Hz, 4H), 4.45 - 4.36 (m, 1H), 3.57 - 3.51 (m, 1H), 2.92 (d, *J* = 11.5 Hz, 2H), 2.49 - 2.39 (m, 2H), 2.06 - 2.00 (m, 2H), 1.88 - 1.85 (m, 2H); LRMS (ES) m/z 516.5 (M⁺ + 1).

### Example 101: Synthesis of Compound 101, 5-chloro-1-(4-(5-(difluoromethyl)-1,3,4-oxadiazole-2-yl)-2-fluorobenzyl)-3-(1-methylpiperidine-4-y I)-1,3-dihydro-2H-benzo[d]imidazole-2-one

### [Step 1] Synthesis of tert-butyl 4-(6-chloro-3-(4-(5-(difluoromethyl)-1,3,4-oxadiazole-2-yl)-2-fluorobenzyl)-2-oxo-2,3-dihydro-1H-benzo[d]imidazole-1-yl)piperidine-1-carboxylate

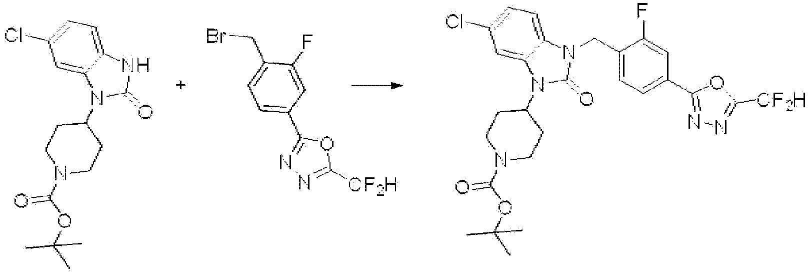

The tert-butyl 4-(6-chloro-2-oxo-2,3-dihydro-1H-benzo[d]imidazole-1-yl)piperidine-1-carboxylate (2.200 g, 6.253 mmol) prepared in the step 3 of the compound 97 was dissolved in N,N-dimethylformamide (60 mL) at 0°C, after which sodium hydride (60.00%, 0.375 g, 9.380 mmol) was added into the resulting solution and stirred at the same temperature for 30 minutes. 2-(4-(bromomethyl)-3-fluorophenyl)-5-(difluoromethyl)-1,3,4-oxadiazole (2.496 g, 8.129 mmol) was added into the reaction mixture and further stirred at room temperature for 4 hours. Water was poured into the reaction mixture and an extraction was performed with ethyl acetate. An organic layer was washed with saturated sodium chloride aqueous solution, then dehydrated with anhydrous magnesium sulfate, then filtered, and then concentrated under reduced pressure. The resulting concentrate was purified via column chromatography (SiO₂, 40 g cartridge; ethyl acetate/hexane = 0 to 70%) and concentrated to obtain a title compound (2.693 g, 74.5%) in a brown solid form.

### [Step 2] Synthesis of 5-chloro-1-(4-(5-(difluoromethyl)-1,3,4-oxadiazole-2-yl)-2-fluorobenzyl)-3-(piperidine-4-yl)-1,3-di hydro-2H-benzo[d]imidazole-2-one

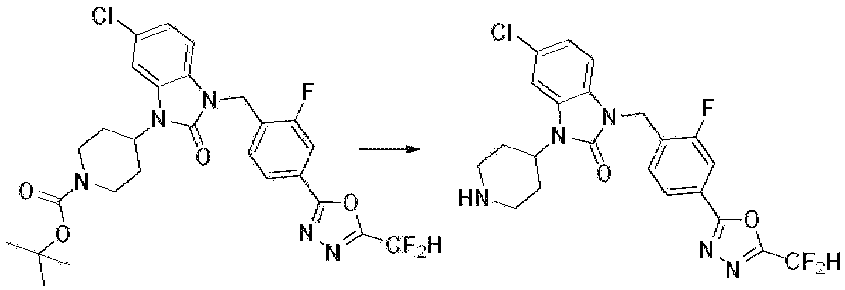

The tert-butyl 4-(6-chloro-3-(4-(5-(difluoromethyl)-1,3,4-oxadiazole-2-yl)-2-fluorobenzyl)-2-oxo-2,3-dihydro-1H-benzo[d]imidazole-1-yl)piperidine-1-carboxylate (2.693 g, 4.659 mmol) prepared in the step 1 and trifluoroacetic acid (3.568 mL, 46.593 mmol) were dissolved in dichloromethane (30 mL) at room temperature, after which the resulting solution was stirred at the same temperature for 4 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 24 g cartridge; ethyl acetate/hexane = 80 to 100%) and concentrated, after which an obtained product was purified again via chromatography (SiO₂, 24 g cartridge; methanol/dichloromethane = 0 to 60%) and concentrated to obtain a title compound (2.155 g, 96.8%) in a yellow solid form.

### [Step 3] Synthesis of the compound 101

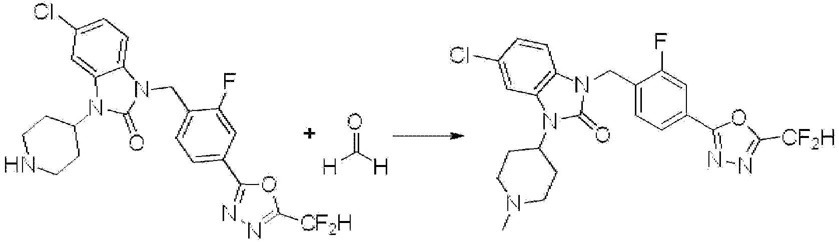

The 5-chloro-1-(4-(5-(difluoromethyl)-1,3,4-oxadiazole-2-yl)-2-fluorobenzyl)-3-(piperidine-4-yl)-1,3-di hydro-2H-benzo[d]imidazole-2-one (0.100 g, 0.209 mmol) prepared in the step 2, sodium triacetoxyborohydride (0.089 g, 0.419 mmol), acetic acid (0.012 mL, 0.209 mmol) and formaldehyde (0.019 g, 0.628 mmol) were dissolved in dichloromethane (2 mL) at room temperature, after which the resulting solution was stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.058 g, 56.2%) in a white solid form.

**¹H NMR** (400 MHz, CDCl₃) δ 7.84 - 7.80 (m, 2H), 7.41 (t, *J* = 7.8 Hz, 1H), 7.33 (d, *J* = 1.8 Hz, 1H), 6.99 (dd, *J =* 8.4, 1.8 Hz, 1H), 6.89 (t, *J =* 51.6 Hz, 1H), 6.83 (d, *J =* 8.4 Hz, 1H), 5.15 (s, 2H), 4.43 - 4.35 (m, 1H), 3.02 (d, *J =* 11.6 Hz, 2H), 2.48 - 2.38 (m, 2H), 2.34 (s, 3H), 2.15 (td, *J* = 11.9, 1.9 Hz, 2H), 1.82 (dd, *J =* 12.0, 2.2 Hz, 2H); LRMS (ES) m/z 492.3 (M⁺+ 1).

### Example 102: Synthesis of Compound 102, 5-chloro-1-(4-(5-(difluoromethyl)-1,3,4-oxadiazole-2-yl)-2-fluorobenzyl)-3-(1-(oxetan-3-yl)piperidi ne-4-yl)-1,3-dihydro-2H-benzo[d]imidazole-2-one

### [Step 1] Synthesis of the compound 102

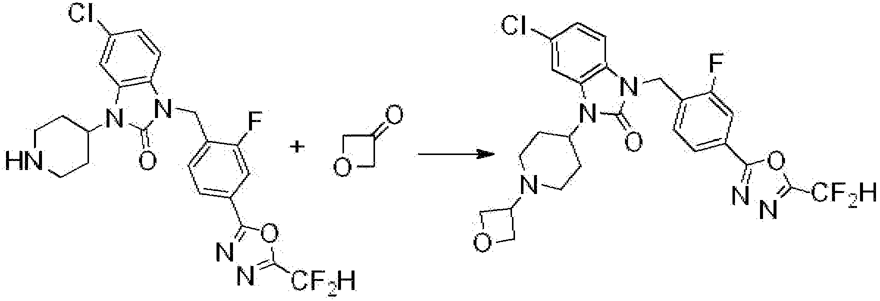

The 5-chloro-1-(4-(5-(difluoromethyl)-1,3,4-oxadiazole-2-yl)-2-fluorobenzyl)-3-(piperidine-4-yl)-1,3-di hydro-2H-benzo[d]imidazole-2-one (0.100 g, 0.209 mmol) prepared in the step 2 of the compound 101, sodium triacetoxyborohydride (0.089 g, 0.419 mmol), acetic acid (0.012 mL, 0.209 mmol) and oxetan-3-one (0.045 g, 0.628 mmol) were dissolved in dichloromethane (2 mL) at room temperature, after which the resulting solution was stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.083 g, 74.2%) in a light yellow solid form.

**¹H NMR** (400 MHz, CDCl₃) δ 7.83 - 7.80 (m, 2H), 7.41 (t, *J* = 7.8 Hz, 1H), 7.31 (d, *J* = 1.8 Hz, 1H), 7.02 - 6.77 (m, 3H), 5.14 (s, 2H), 4.65 (dt, *J* = 17.0, 5.9 Hz, 4H), 4.43 - 4.34 (m, 1H), 3.56 - 3.50 (m, 1H), 2.91 (d, *J =* 11.4 Hz, 2H), 2.47 - 2.38 (m, 2H), 2.04 - 1.99 (m, 2H), 1.85 (dd, *J* = 12.0, 2.2 Hz, 2H); **LRMS** (ES) m/z 536.3 (M⁺ + 1).

### Example 103: Synthesis of Compound 103, 3-(4-(5-(difluoromethyl)-1,3,4-oxadiazole-2-yl)-2-fluorobenzyl)-1-(1-methylpiperidine-4-yl)-1,3-dih ydro-2H-imidazo[4,5-b]pyridine-2-one

### [Step 1] Synthesis of tert-butyl 4-((2-nitropyridine-3-yl)amino)piperidine-1-carboxylate

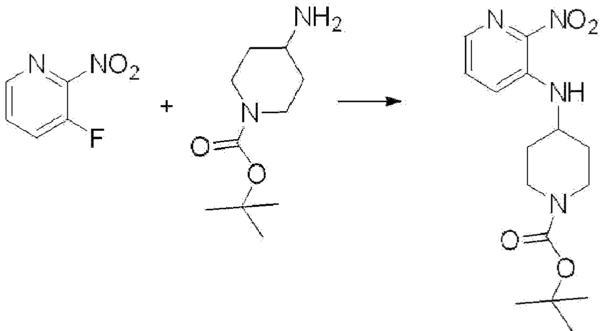

3-fluoro-2-nitropyridine (5.000 g, 35.189 mmol), tert-butyl 4-aminopiperidine-1-carboxylate (7.400 g, 36.948 mmol), potassium carbonate (9.727 g, 70.378 mmol) and potassium iodide (0.584 g, 3.519 mmol) were dissolved in N,N-dimethylformamide (300 mL) at room temperature, after which the resulting solution was stirred at 80°C for 18 hours, and then a reaction was finished by lowering a temperature to room temperature. The reaction mixture was filtered via a pater filter to remove a solid therefrom, after which solvent was removed from the resulting filtrate under reduced pressure. Then, water was poured into the resulting concentrate and then an extraction was performed with ethyl acetate. An organic layer was washed with saturated sodium chloride aqueous solution, then dehydrated with anhydrous magnesium sulfate, then filtered, and then concentrated under reduced pressure. A title compound was used without an additional purification process (11.450 g, 100.9%, orange liquid).

### [Step 2] Synthesis of tert-butyl 4-((2-aminopyridine-3-yl)amino)piperidine-1-carboxylate

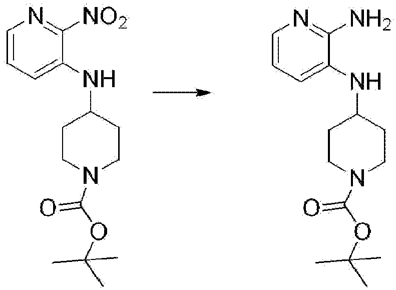

The tert-butyl 4-((2-nitropyridine-3-yl)amino)piperidine-1-carboxylate (11.450 g, 35.518 mmol) prepared in the step 1 and Pd/C (10%, 0.840 g, 3.552 mmol) were dissolved in methanol (250 mL) at room temperature, after which the resulting solution was stirred at the same temperature for 18 hours in the presence of a hydrogen balloon. The reaction mixture was filtered via a celite pad to remove a solid therefrom, after which solvent was removed from the resulting filtrate under reduced pressure, and then a title compound was used without an additional purification process (9.820 g, 94.6%, black solid).

### [Step 3] Synthesis of tert-butyl 4-(2-oxo-2,3-dihydro-1H-imidazo[4,5-b]pyridine-1-yl)piperidine-1-carboxylate

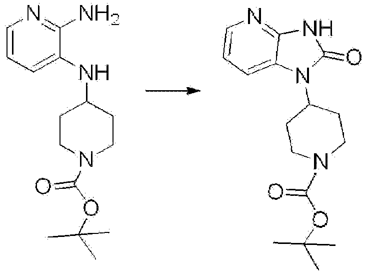

The tert-butyl 4-((2-aminopyridine-3-yl)amino)piperidine-1-carboxylate (9.820 g, 33.586 mmol) prepared in the step 2 and 1,1'-carbonyldiimidazole (CDI, 8.169 g, 50.380 mmol) were dissolved in N,N-dimethylformamide (150 mL) at room temperature, after which the resulting solution was stirred at the same temperature for 18 hours. Solvent was removed from the reaction mixture under reduced pressure, after which water was poured into the resulting concentrate, and then an extraction was performed with ethyl acetate. An organic layer was washed with saturated sodium chloride aqueous solution, then dehydrated with anhydrous magnesium sulfate, then filtered, and then concentrated under reduced pressure. The resulting concentrate was purified via column chromatography (SiO₂, 120 g cartridge; ethyl acetate/hexane = 0 to 100%) and concentrated to obtain a title compound (5.770 g, 54.0%) in a brown solid form.

### [Step 4] Synthesis of tert-butyl 4-(3-(4-(5-(difluoromethyl)-1,3,4-oxadiazole-2-yl)-2-fluorobenzyl)-2-oxo-2,3-dihydro-1H-imidazo[ 4,5-b]pyridine-1-yl)piperidine-1-carboxylate

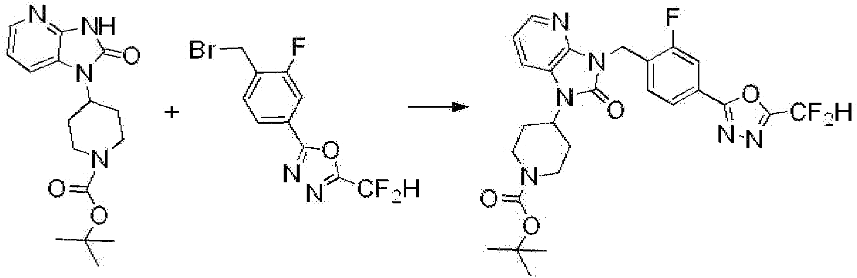

The tert-butyl 4-(2-oxo-2,3-dihydro-1H-imidazo[4,5-b]pyridine-1-yl)piperidine-1-carboxylate (1.900 g, 5.968 mmol) prepared in the step 3 was dissolved in N,N-dimethylformamide (60 mL) at 0°C, after which sodium hydride (60.00%, 0.358 g, 8.952 mmol) was added into the resulting solution and stirred at the same temperature for 30 minutes. 2-(4-(bromomethyl)-3-fluorophenyl)-5-(difluoromethyl)-1,3,4-oxadiazole (2.382 g, 7.758 mmol) was added into the reaction mixture and further stirred at room temperature for 4 hours. Water was poured into the reaction mixture and an extraction was performed with ethyl acetate. An organic layer was washed with saturated sodium chloride aqueous solution, then dehydrated with anhydrous magnesium sulfate, then filtered, and then concentrated under reduced pressure. The resulting concentrate was purified via column chromatography (SiO₂, 40 g cartridge; ethyl acetate/hexane = 0 to 70%) and concentrated to obtain a title compound (0.940 g, 28.9%) in a brown solid form.

### [Step 5] Synthesis of 3-(4-(5-(difluoromethyl)-1,3,4-oxadiazole-2-yl)-2-fluorobenzyl)-1-(piperidine-4-yl)-1,3-dihydro-2H -imidazo[4,5-b]pyridine-2-one

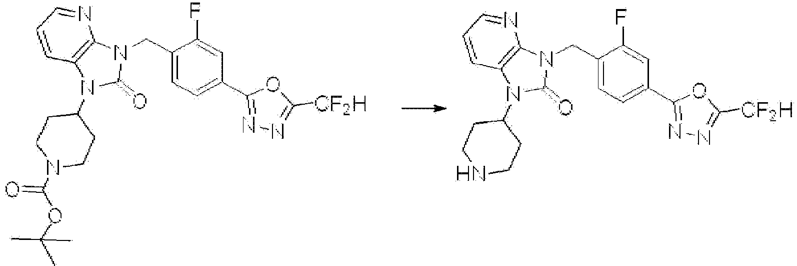

The tert-butyl 4-(3-(4-(5-(difluoromethyl)-1,3,4-oxadiazole-2-yl)-2-fluorobenzyl)-2-oxo-2,3-dihydro-1H-imidazo[ 4,5-b]pyridine-1-yl)piperidine-1-carboxylate (0.940 g, 1.727 mmol) prepared in the step 4 and trifluoroacetic acid (1.322 mL, 17.266 mmol) were dissolved in dichloromethane (15 mL) at room temperature, after which the resulting solution was stirred at the same temperature for 5 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 24 g cartridge; ethyl acetate/hexane = 80 to 100%) and concentrated, after which an obtained product was purified again via chromatography (SiO₂, 24 g cartridge; methanol/dichloromethane = 0 to 80%) and concentrated to obtain a title compound (0.587 g, 76.4%) in a yellow solid form.

### [Step 6] Synthesis of the compound 103

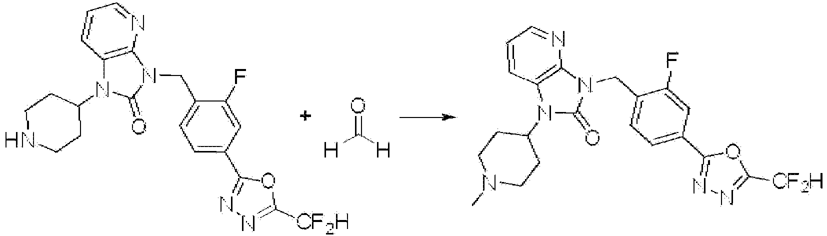

The 3-(4-(5-(difluoromethyl)-1,3,4-oxadiazole-2-yl)-2-fluorobenzyl)-1-(piperidine-4-yl)-1,3-dihydro-2H -imidazo[4,5-b]pyridine-2-one (0.100 g, 0.225 mmol) prepared in the step 5, sodium triacetoxyborohydride (0.095 g, 0.450 mmol) and formaldehyde (0.020 g, 0.675 mmol) were dissolved in dichloromethane (2 mL) at room temperature, after which the resulting solution was stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.048 g, 46.7%) in a white solid form.

**¹H NMR** (400 MHz, CDCl₃) δ 8.00 (dd, *J* = 5.2, 1.2 Hz, 1H), 7.79 (d, *J =* 8.9 Hz, 2H), 7.50 (dd, *J* = 7.9, 1.2 Hz, 1H), 7.41 (t, *J* = 7.6 Hz, 1H), 6.97 (dd, *J* = 7.9, 5.2 Hz, 1H), 6.89 (t, *J* = 51.7 Hz, 1H), 5.29 (s, 2H), 4.46 - 4.40 (m, 1H), 3.00 (d, *J* = 11.7 Hz, 2H), 2.41 - 2.30 (m, 5H), 2.14 (td, *J =* 12.0, 2.0 Hz, 2H), 1.86 - 1.83 (m, 2H); **LRMS** (ES) m/z 459.4 (M⁺ + 1).

### Example 104: Synthesis of Compound 104, 3-(4-(5-(difluoromethyl)-1,3,4-oxadiazole-2-yl)-2-fluorobenzyl)-1-(1-(oxetan-3-yl)piperidine-4-yl)-1,3-dihydro-2H-imidazo[4,5-b]pyridine-2-one

### [Step 1] Synthesis of the compound 104

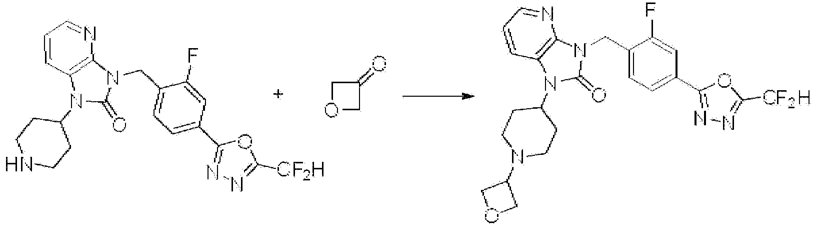

The 3-(4-(5-(difluoromethyl)-1,3,4-oxadiazole-2-yl)-2-fluorobenzyl)-1-(piperidine-4-yl)-1,3-dihydro-2H -imidazo[4,5-b]pyridine-2-one (0.100 g, 0.225 mmol) prepared in the step 5 of the compound 103, sodium triacetoxyborohydride (0.095 g, 0.450 mmol) and oxetan-3-one (0.049 g, 0.675 mmol) were dissolved in dichloromethane (2 mL) at room temperature, after which the resulting solution was stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.066 g, 58.7%) in a white solid form.

**¹H NMR** (400 MHz, CDCl₃) δ 8.01 (dd, *J* = 5.2, 1.0 Hz, 1H), 7.79 - 7.77 (m, 2H), 7.52 (dd, *J* = 7.8, 1.0 Hz, 1H), 7.42 (t, *J* = 7.*7* Hz, 1H), 7.00 (dd, *J =* 7.9, 5.3 Hz, 1H), 6.89 (t, *J* = 51.3 Hz, 1H), 5.28 (s, 2H), 4.62 (dt, *J* = 26.4, 6.4 Hz, 4H), 4.48 - 4.40 (m, 1H), 3.54 - 3.48 (m, 1H), 2.88 (d, *J =* 11.6 Hz, 2H), 2.41 - 2.30 (m, 2H), 2.02 - 1.97 (m, 2H), 1.87 (dd, *J =* 11.8, 2.0 Hz, 2H); **LRMS** (ES) m/z 501.4 (M⁺ + 1).

### Example 105: Synthesis of Compound 105, 3-(4-(5-(difluoromethyl)-1,3,4-oxadiazole-2-yl)benzyl)-1-(1-methylpiperidine-4-yl)-1,3-dihydro-2H -imidazo[4,5-b]pyridine-2-one

### [Step 1] Synthesis of tert-butyl 4-(3-(4-(5-(difluoromethyl)-1,3,4-oxadiazole-2-yl)benzyl)-2-oxo-2,3-dihydro-1H-imidazo[4,5-b]pyr idine-1-yl)piperidine-1-carboxylate

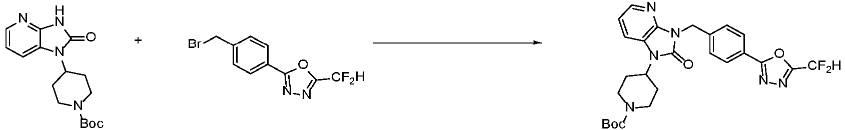

The tert-butyl 4-(2-oxo-2,3-dihydro-1H-imidazo[4,5-b]pyridine-1-yl)piperidine-1-carboxylate (1.900 g, 5.968 mmol) prepared in the step 3 of the compound 103 was dissolved in N,N-dimethylformamide (60 mL) at 0°C, after which sodium hydride (60.00%, 0.358 g, 8.952 mmol) was added into the resulting solution and stirred at the same temperature for 30 minutes. 2-(4-(bromomethyl)phenyl)-5-(difluoromethyl)-1,3,4-oxadiazole (2.243 g, 7.758 mmol) was added into the reaction mixture and further stirred at room temperature for 4 hours. Water was poured into the reaction mixture and an extraction was performed with ethyl acetate. An organic layer was washed with saturated sodium chloride aqueous solution, then dehydrated with anhydrous magnesium sulfate, then filtered, and then concentrated under reduced pressure. The resulting concentrate was purified via column chromatography (SiO₂, 40 g cartridge; ethyl acetate/hexane = 0 to 70%) and concentrated to obtain a title compound (1.125 g, 35.8%) in a brown solid form.

### [Step 2] Synthesis of 3-(4-(5-(difluoromethyl)-1,3,4-oxadiazole-2-yl)benzyl)-1-(piperidine-4-yl)-1,3-dihydro-2H-imidazo [4,5-b]pyridine-2-one

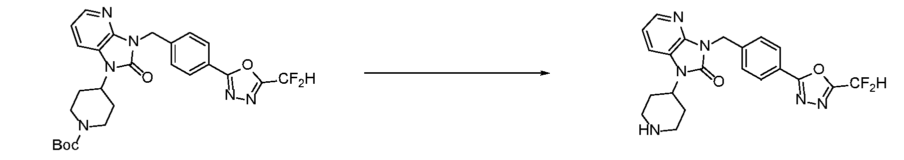

The tert-butyl 4-(3-(4-(5-(difluoromethyl)-1,3,4-oxadiazole-2-yl)benzyl)-2-oxo-2,3-dihydro-1H-imidazo[4,5-b]pyr idine-1-yl)piperidine-1-carboxylate (1.125 g, 2.136 mmol) prepared in the step 1 and trifluoroacetic acid (1.636 mL, 21.362 mmol) were dissolved in dichloromethane (15 mL) at room temperature, after which the resulting solution was stirred at the same temperature for 5 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 24 g cartridge; ethyl acetate/hexane = 80 to 100%) and concentrated, after which an obtained product was purified again via chromatography (SiO₂, 24 g cartridge; methanol/dichloromethane = 0 to 80%) and concentrated to obtain a title compound (0.830 g, 91.1%) in a yellow solid form.

### [Step 3] Synthesis of the compound 105

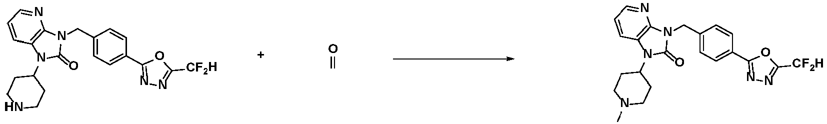

The 3-(4-(5-(difluoromethyl)-1,3,4-oxadiazole-2-yl)benzyl)-1-(piperidine-4-yl)-1,3-dihydro-2H-imidazo [4,5-b]pyridine-2-one (0.100 g, 0.235 mmol) prepared in the step 2, sodium triacetoxyborohydride (0.099 g, 0.469 mmol) and formaldehyde (0.021 g, 0.704 mmol) were dissolved in dichloromethane (2 mL) at room temperature, after which the resulting solution was stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.024 g, 23.1%) in a white solid form.

**¹H NMR** (400 MHz, CDCl₃) δ 8.05 - 8.01 (m, 3H), 7.62 (d, *J* = 8.2 Hz, 2H), 7.48 (dd, *J* = 7.9, 1.0 Hz, 1H), 6.96 (dd, *J* = 7.8, 5.2 Hz, 1H), 6.88 (t, *J =* 51.8 Hz, 1H), 5.22 (s, 2H), 4.47 - 4.39 (m, 1H), 3.00 (d, *J* = 11.8 Hz, 2H), 2.40 - 2.30 (m, 5H), 2.17 - 2.11 (m, 2H), 1.83 (dd, *J* = 11.8, 2.0 Hz, 2H); LRMS (ES) m/z 441.5 (M⁺+ 1).

### Example 106: Synthesis of Compound 106, 3-(4-(5-(difluoromethyl)-1,3,4-oxadiazole-2-yl)benzyl)-1-(1-(oxetan-3-yl)piperidine-4-yl)-1,3-dihyd ro-2H-imidazo[4,5-b]pyridine-2-one

### [Step 1] Synthesis of the compound 106

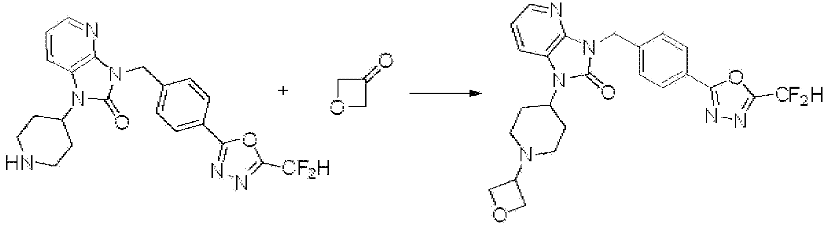

The 3-(4-(5-(difluoromethyl)-1,3,4-oxadiazole-2-yl)benzyl)-1-(piperidine-4-yl)-1,3-dihydro-2H-imidazo [4,5-b]pyridine-2-one (0.100 g, 0.235 mmol) prepared in the step 2 of the compound 105, sodium triacetoxyborohydride (0.099 g, 0.469 mmol) and oxetan-3-one (0.051 g, 0.704 mmol) were dissolved in dichloromethane (2 mL) at room temperature, after which the resulting solution was stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.080 g, 70.6%) in a white solid form.

**¹H NMR** (400 MHz, CDCl₃) δ 8.02 - 8.00 (m, 3H), 7.61 (d, *J* = 8.3 Hz, 2H), 7.49 (dd, *J* = 7.8, 1.1 Hz, 1H), 6.98 (dd, *J =* 7.8, 5.2 Hz, 1H), 6.88 (t, *J =* 51.7 Hz, 1H), 5.19 (s, 2H), 4.61 (dt, *J* = 26.4, 6.4 Hz, 4H), 4.46 - 4.38 (m, 1H), 3.53 - 3.46 (m, 1H), 2.87 (d, *J =* 11.6 Hz, 2H), 2.38 - 2.28 (m, 2H), 2.01 - 1.95 (m, 2H), 1.85 (dd, *J =* 11.9, 2.1 Hz, 2H); **LRMS** (ES) m/z 483.3 (M⁺ + 1).

### Example 107: Synthesis of Compound 107, 3-((5-(5-(difluoromethyl)-1,3,4-oxadiazole-2-yl)pyridine-2-yl)methyl)-1-(1-methylpiperidine-4-yl)-1 ,3-dihydro-2H-imidazo[4,5-b]pyridine-2-one

### [Step 1] Synthesis of tert-butyl 4-(3-((5-(5-(difluoromethyl)-1,3,4-oxadiazole-2-yl)pyridine-2-yl)methyl)-2-oxo-2,3-dihydro-1H-imi dazo[4,5-b]pyridine-1-yl)piperidine-1-carboxylate

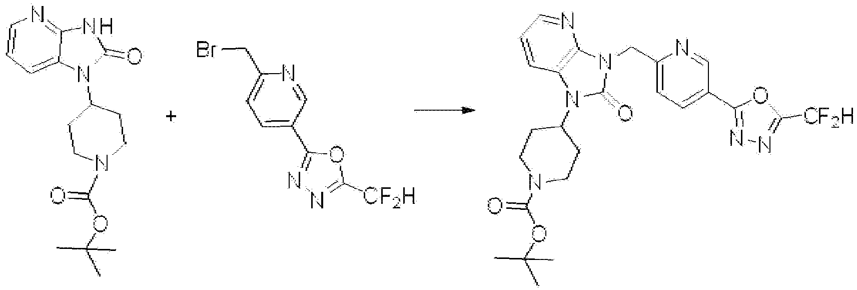

2-(6-(bromomethyl)pyridine-3-yl)-5-(difluoromethyl)-1,3,4-oxadiazole (1.900 g, 6.550 mmol) was dissolved in N,N-dimethylformamide (60 mL) at 0°C, after which sodium hydride (60.00%, 0.393 g, 9.825 mmol) was added into the resulting solution and stirred at the same temperature for 30 minutes. Tert-butyl 4-(2-oxo-2,3-dihydro-1H-imidazo[4,5-b]pyridine-1-yl)piperidine-1-carboxylate (2.711 g, 8.515 mmol) was added into the reaction mixture and further stirred at room temperature for 4 hours. Water was poured into the reaction mixture and an extraction was performed with ethyl acetate. An organic layer was washed with saturated sodium chloride aqueous solution, then dehydrated with anhydrous magnesium sulfate, then filtered, and then concentrated under reduced pressure. The resulting concentrate was purified via column chromatography (SiO₂, 40 g cartridge; ethyl acetate/hexane = 0 to 80%) and concentrated to obtain a title compound (1.814 g, 52.5%) in a brown solid form.

### [Step 2] Synthesis of tert-butyl 4-(3-((5-(5-(difluoromethyl)-1,3,4-oxadiazole-2-yl)pyridine-2-yl)methyl)-2-oxo-2,3-dihydro-1H-imi dazo[4,5-b]pyridine-1-yl)piperidine-1-carboxylate

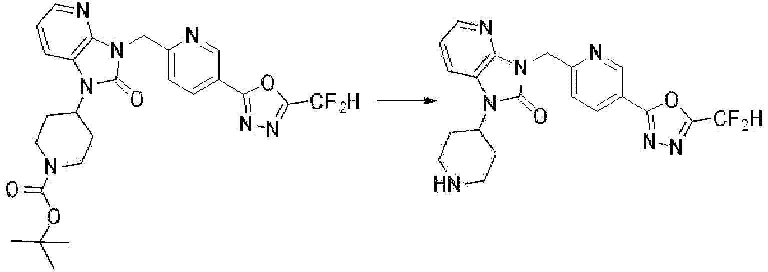

The 3-((5-(5-(difluoromethyl)-1,3,4-oxadiazole-2-yl)pyridine-2-yl)methyl)-1-(piperidine-4-yl)-1,3-dihydr o-2H-imidazo[4,5-b]pyridine-2-one (1.814 g, 4.244 mmol) prepared in the step 1 and trifluoroacetic acid (0.325 mL, 4.244 mmol) were dissolved in dichloromethane (30 mL) at room temperature, after which the resulting solution was stirred at the same temperature for 5 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 24 g cartridge; ethyl acetate/hexane = 80 to 100%) and concentrated, after which an obtained product was purified again via chromatography (SiO₂, 24 g cartridge; methanol/dichloromethane = 0 to 80%) and concentrated to obtain a title compound (1.029 g, 46.0%) in a brown solid form.

### [Step 3] Synthesis of the compound 107

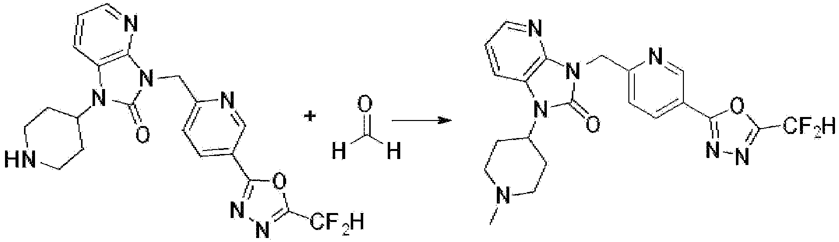

The 3-((5-(5-(difluoromethyl)-1,3,4-oxadiazole-2-yl)pyridine-2-yl)methyl)-1-(piperidine-4-yl)-1,3-dihydr o-2H-imidazo[4,5-b]pyridine-2-one (0.100 g, 0.234 mmol) prepared in the step 2, sodium triacetoxyborohydride (0.099 g, 0.468 mmol) and formaldehyde (0.021 g, 0.702 mmol) were dissolved in dichloromethane (2 mL) at room temperature, after which the resulting solution was stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.031 g, 30.3%) in a white solid form.

**¹H NMR** (400 MHz, CDCl₃) δ 9.22 (d, *J =* 1.7 Hz, 1H), 8.30 (dd, *J* = 8.2, 2.2 Hz, 1H), 7.99 (dd, *J* = 5.2, 1.1 Hz, 1H), 7.56 (dd, *J* = 7.9, 1.1 Hz, 1H), 7.43 (d, *J* = 8.2 Hz, 1H), 6.98 (dd, *J =* 7.9, 5.2 Hz, 1H), 6.91 (t, *J =* 51.7 Hz, 1H), 5.39 (s, 2H), 4.54 - 4.45 (m, 1H), 3.10 (d, *J =* 11.8 Hz, 2H), 2.51 - 2.41 (m, 2H), 2.38 (s, 3H), 2.27 - 2.21 (m, 2H), 1.88 (dd, *J =* 12.3, 2.2 Hz, 2H); **LRMS** (ES) m/z 442.5 (M⁺ + 1).

### Example 108: Synthesis of Compound 108, 3-(1-acetylpiperidine-4-yl)-1-((5-(5-(difluoromethyl)-1,3,4-oxadiazole-2-yl)pyridine-2-yl)methyl)-5-fluoro-1,3-dihydro-2H-benzo[d]imidazole-2-one

### [Step 1] Synthesis of the compound 108

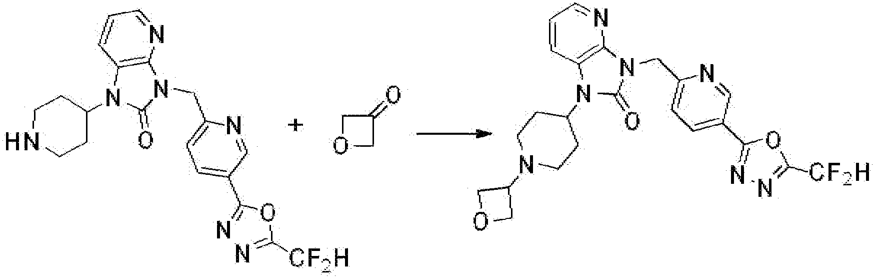

The 1-((5-(5-(difluoromethyl)-1,3,4-oxadiazole-2-yl)pyridine-2-yl)methyl)-5-fluoro-3-(piperidine-4-yl)-1 ,3-dihydro-2H-benzo[d]imidazole-2-one (0.100 g, 0.225 mmol) prepared in the step 2 of the compound 107, acetyl chloride (0.032 mL, 0.450 mmol) and triethylamine (0.063 mL, 0.450 mmol) were dissolved in dichloromethane (1.5 mL) at room temperature, after which the resulting solution was stirred at the same temperature for 18 hours. Water was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 12 g cartridge; ethyl acetate/hexane = 0 to 100%) and concentrated to obtain a title compound (0.030 g, 27.4%) in a brown liquid form.

**¹H NMR** (400 MHz, CDCl₃) δ 9.19 (d, *J =* 1.6 Hz, 1H), 8.28 (dd, *J* = 8.2, 2.2 Hz, 1H), 7.98 (dd, *J* = 5.2, 0.9 Hz, 1H), 7.51 (dd, *J =* 7.8, 0.9 Hz, 1H), 7.42 (d, *J =* 8.2 Hz, 1H), 6.98 (dd, *J =* 7.8, 5.2 Hz, 1H), 6.90 (t, *J =* 51.6 Hz, 1H), 5.36 (s, 2H), 4.62 (dt, *J =* 23.8, 6.3 Hz, 4H), 4.48 - 4.40 (m, 1H), 3.53 - 3.42 (m, 1H), 2.88 (d, *J =* 11.5 Hz, 2H), 2.41 - 2.31 (m, 2H), 2.02 - 1.97 (m, 2H), 1.89 - 1.86 (m, 2H); **LRMS** (ES) m/z 484.4 (M⁺+ 1).

### Example 109: Synthesis of Compound 109, 1-(2-oxaspiro[3.3]heptane-6-yl)-3-((5-(5-(trifluoromethyl)-1,3,4-oxadiazole-2-yl)pyridine-2-yl)met hyl)-1,3-dihydro-2H-benzo[d]imidazole-2-one

### [Step 1] Synthesis of the compound 109

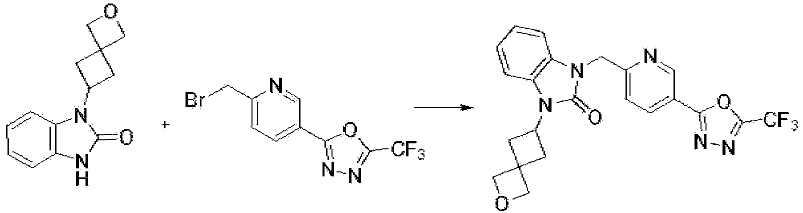

The 1-(2-oxaspiro[3.3]heptane-6-yl)-1,3-dihydro-2H-benzo[d]imidazole-2-one (0.050 g, 0.217 mmol) prepared in the step 2 of the compound 27 and sodium hydride (60.00%, 0.010 g, 0.239 mmol) were dissolved in N,N-dimethylformamide (3 mL) at 0°C, after which 2-(6-(bromomethyl)pyridine-3-yl)-5-(trifluoromethyl)-1,3,4-oxadiazole (0.074 g, 0.239 mmol) was added into the resulting solution and then stirred at the same temperature for 2 hours. Solvent was removed from the reaction mixture under reduced pressure, after which the resulting concentrate was purified via column chromatography (SiO₂, 4 g cartridge; ethyl acetate/hexane = 60 to 100%) and concentrated to obtain a title compound (0.011 g, 11.1%) in a yellow oil form.

**¹H NMR** (400 MHz, CDCl₃) δ 9.30 (dd, *J* = 2.2, 0.8 Hz, 1H), 8.34 (dd, *J* = 8.2, 2.2 Hz, 1H), 7.44 (d, *J* = 8.2 Hz, 2H), 7.14 - 7.04 (m, 4H), 6.98 - 6.97 (m, 1H), 5.29 (s, 2H), 4.87 (s, 2H), 4.82 (s, 2H), 4.75 - 4.69 (m, 1H), 3.20 - 3.15 (m, 2H), 2.85 - 2.80 (m, 2H); **LRMS** (ES) m/z 458.3 (M⁺ + H).

### Example 110: Synthesis of Compound 110, 3-(4-(5-(difluoromethyl)-1,3,4-oxadiazole-2-yl)-2-fluorobenzyl)-1-methyl-5-(pyridine-3-yl)-1,3-dihy dro-2H-benzo[d]imidazole-2-one

### [Step 1] Synthesis of 4-bromo-N-methyl-2-nitroaniline

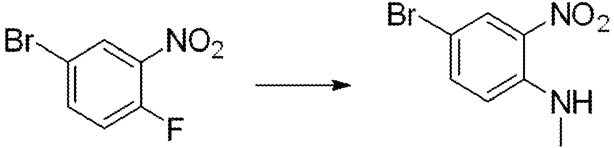

1-fluoro-2-nitro-4-(trifluoromethyl)benzene (5.000 g, 22.727 mmol), methanamine (2.00 M solution, 13.636 mL, 27.273 mmol), potassium carbonate (6.282 g, 45.455 mmol) and potassium iodide (0.377 g, 2.273 mmol) were dissolved in N,N-dimethylformamide (230 mL) at room temperature, after which the resulting solution was stirred at 80°C for 18 hours, and then a reaction was finished by lowering a temperature to room temperature. Solvent was removed from the reaction mixture under reduced pressure, after which water was poured into the resulting concentrate, and then an extraction was performed with ethyl acetate. An organic layer was washed with saturated sodium chloride aqueous solution, then dehydrated with anhydrous magnesium sulfate, then filtered, and then concentrated under reduced pressure. The resulting concentrate was purified via column chromatography (SiO₂, 120 g cartridge; ethyl acetate/hexane = 0 to 50%) and concentrated to obtain a title compound (4.750 g, 90.5%) in an orange solid form.

### [Step 2] 4-bromo-N¹-methylbenzene-1,2-diamine

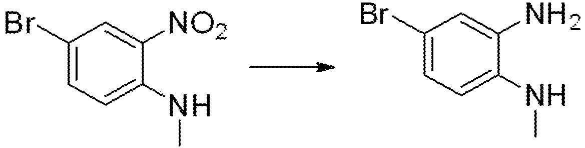

The 4-bromo-N-methyl-2-nitroaniline (4.750 g, 20.558 mmol) prepared in the step 1 and Raney Ni (0.48 g, 10 wt%) were dissolved in methanol (200 mL) at room temperature, after which the resulting solution was stirred at the same temperature for 18 hours in the presence of a hydrogen balloon. The reaction mixture was filtered via a celite pad to remove a solid therefrom, after which solvent was removed from the resulting filtrate under reduced pressure. Then, water was poured into the resulting concentrate and then an extraction was performed with ethyl acetate. An organic layer was washed with saturated sodium chloride aqueous solution, then dehydrated with anhydrous magnesium sulfate, then filtered, and then concentrated under reduced pressure. A title compound was used without an additional purification process (3.980 g, 96.3%, black liquid).

### [Step 3] Synthesis of 5-bromo-1-methyl-1,3-dihydro-2H-benzo[d]imidazole-2-one

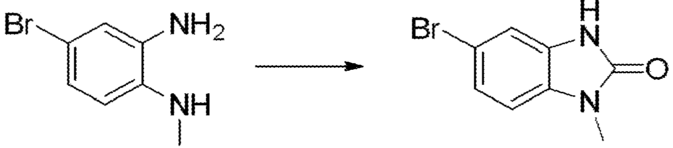

The 4-bromo-N¹-methylbenzene-1,2-diamine (3.980 g, 19.794 mmol) prepared in the step 2 and 1,1'-carbonyldiimidazole (CDI, 4.814 g, 29.691 mmol) were dissolved in N,N-dimethylformamide (150 mL) at room temperature, after which the resulting solution was stirred at the same temperature for 18 hours. Solvent was removed from the reaction mixture under reduced pressure, after which water was poured into the resulting concentrate, and then an extraction was performed with ethyl acetate. An organic layer was washed with saturated sodium chloride aqueous solution, then dehydrated with anhydrous magnesium sulfate, then filtered, and then concentrated under reduced pressure. The resulting concentrate was purified via column chromatography (SiO₂, 80 g cartridge; ethyl acetate/hexane = 0 to 80%) and concentrated to obtain a title compound (0.530 g, 11.8%) in a violet solid form.

### [Step 4] Synthesis of 5-bromo-3-(4-(5-(difluoromethyl)-1,3,4-oxadiazole-2-yl)-2-fluorobenzyl)-1-methyl-1,3-dihydro-2H -benzo[d]imidazole-2-one

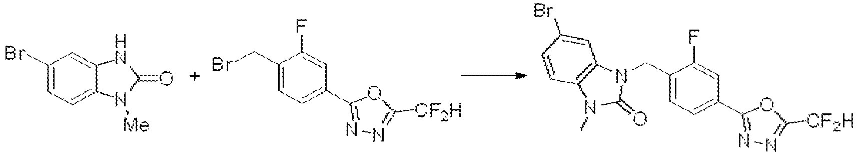

The 5-bromo-1-methyl-1,3-dihydro-2H-benzo[d]imidazole-2-one (0.530 g, 2.334 mmol) prepared in the step 3 was dissolved in N,N-dimethylformamide (20 mL) at 0°C, after which sodium hydride (60.00%, 0.140 g, 3.501 mmol) was added into the resulting solution and stirred at the same temperature for 30 minutes. 2-(4-(bromomethyl)-3-fluorophenyl)-5-(difluoromethyl)-1,3,4-oxadiazole (0.932 g, 3.034 mmol) was added into the reaction mixture and further stirred at room temperature for 5 hours. Water was poured into the reaction mixture and an extraction was performed with ethyl acetate. An organic layer was washed with saturated sodium chloride aqueous solution, then dehydrated with anhydrous magnesium sulfate, then filtered, and then concentrated under reduced pressure. The resulting concentrate was purified via column chromatography (SiO₂, 12 g cartridge; ethyl acetate/hexane = 0 to 70%) and concentrated to obtain a title compound (0.485 g, 45.8%) in a brown solid form.

### [Step 5] Synthesis of the compound 110

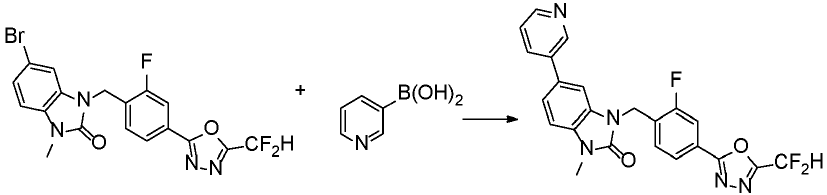

The 5-bromo-3-(4-(5-(difluoromethyl)-1,3,4-oxadiazole-2-yl)-2-fluorobenzyl)-1-methyl-1,3-dihydro-2H -benzo[d]imidazole-2-one (0.200 g, 0.441 mmol) prepared in the step 4, pyridine-3-ylboronic acid (0.108 g, 0.883 mmol), sodium carbonate (0.234 g, 2.206 mmol) and [1,1'-bis(diphenylphosphino)ferrocene]dichloropalladium dichloromethane complex (0.018 g, 0.022 mmol) were mixed in 1,2-dimethoxyethane (2.3 mL)/water (0.7 mL) at room temperature, after which the resulting mixture was irradiated with microwave, then heated at 100°C for 30 minutes, and then a reaction was finished by lowering a temperature to room temperature. Water was poured into the reaction mixture and an extraction was performed with ethyl acetate. An organic layer was washed with saturated sodium chloride aqueous solution, then dehydrated with anhydrous magnesium sulfate, then filtered, and then concentrated under reduced pressure. The resulting concentrate was purified via column chromatography (SiO₂, 4 g cartridge; methanol/dichloromethane = 0 to 3%) and concentrated to obtain a title compound (0.067 g, 33.8%) in a brown solid form.

**¹H NMR** (400 MHz, CDCl₃) δ 8.75 (d, *J* = 1.8 Hz, 1H), 8.53 (dd, *J =* 4.8, 1.6 Hz, 1H), 7.84 - 7.76 (m, 3H), 7.49 (t, *J =* 7.8 Hz, 1H), 7.33 - 7.30 (m, 2H), 7.13 (d, *J* = 1.4 Hz, 1H), 7.09 (d, *J =* 8.1 Hz, 1H), 6.89 (t, *J =* 51.7 Hz, 1H), 5.24 (s, 2H), 3.49 (s, 3H); **LRMS** (ES) m/z 451.9 (M⁺+ 1).

### Example 111: Synthesis of Compound 111, 3-(4-(5-(difluoromethyl)-1,3,4-oxadiazole-2-yl)-2-fluorobenzyl)-1-methyl-5-(pyridine-4-yl)-1,3-dihy dro-2H-benzo[d]imidazole-2-one

### [Step 1] Synthesis of the compound 111

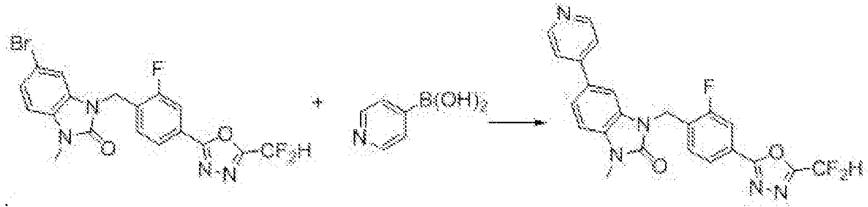

The 5-bromo-3-(4-(5-(difluoromethyl)-1,3,4-oxadiazole-2-yl)-2-fluorobenzyl)-1-methyl-1,3-dihydro-2H -benzo[d]imidazole-2-one (0.200 g, 0.441 mmol) prepared in the step 4 of the compound 110, pyridine-4-ylboronic acid (0.108 g, 0.883 mmol), sodium carbonate (0.234 g, 2.206 mmol) and [1,1'-bis(diphenylphosphino)ferrocene]dichloropalladium dichloromethane complex (0.018 g, 0.022 mmol) were mixed in 1,4-dioxane (2.3 mL)/water (0.7 mL) at room temperature, after which the resulting mixture was irradiated with microwave, then heated at 100°C for 30 minutes, and then a reaction was finished by lowering a temperature to room temperature. Solvent was removed from the reaction mixture under reduced pressure, after which the resulting concentrate was purified via column chromatography (SiO₂, 4 g cartridge; methanol/dichloromethane = 0 to 3%) and concentrated to obtain a title compound (0.019 g, 9.3%) in a brown solid form.

**¹H NMR** (400 MHz, CDCl₃) δ 8.63 (s, 2H), 7.88 - 7.83 (m, 2H), 7.52 (t, *J* = 7.8 Hz, 1H), 7.45 - 7.41 (m, 3H), 7.22 (d, *J* = 1.3 Hz, 1H), 7.11 (d, *J* = 8.2 Hz, 1H), 6.89 (t, *J =* 51.6 Hz, 1H), 5.26 (s, 2H), 3.51 (s, 3H); **LRMS** (ES) m/z 452.0 (M⁺ + 1).

### Example 112: Synthesis of Compound 112, 3-(4-(5-(difluoromethyl)-1,3,4-oxadiazole-2-yl)-2-fluorobenzyl)-5-(3-fluorophenyl)-1-methyl-1,3-di hydro-2H-benzo[d]imidazole-2-one

### [Step 1] Synthesis of the compound 112

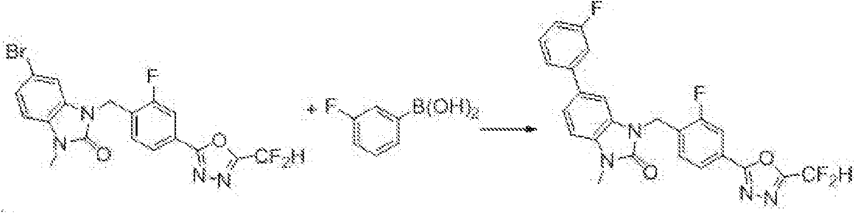

The 5-bromo-3-(4-(5-(difluoromethyl)-1,3,4-oxadiazole-2-yl)-2-fluorobenzyl)-1-methyl-1,3-dihydro-2H -benzo[d]imidazole-2-one (0.200 g, 0.441 mmol) prepared in the step 4 of the compound 110, (3-fluorophenyl)boronic acid (0.074 g, 0.530 mmol), [1,1'-bis(di-tert-butylphosphino)ferrocene]palladium(II) dichloride (Pd(dtbpf)Cl₂, 0.014 g, 0.022 mmol) and cesium carbonate (0.288 g, 0.883 mmol) were mixed in 1,4-dioxane (2.25 mL)/water (0.75 mL) at room temperature, after which the resulting mixture was irradiated with microwave, then heated at 100°C for 30 minutes, and then a reaction was finished by lowering a temperature to room temperature. Solvent was removed from the reaction mixture under reduced pressure, after which water was poured into the resulting concentrate, and then an extraction was performed with dichloromethane. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 4 g cartridge; ethyl acetate/hexane = 20 to 50%) and concentrated to obtain a title compound (0.035 g, 16.7%) in a white solid form.

**¹H NMR** (400 MHz, CDCl₃) δ 8.63 (s, 2H), 7.88 - 7.83 (m, 2H), 7.52 (t, *J* = 7.8 Hz, 1H), 7.45 - 7.41 (m, 3H), 7.22 (d, *J* = 1.3 Hz, 1H), 7.11 (d, *J* = 8.2 Hz, 1H), 6.89 (t, *J* = 51.6 Hz, 1H), 5.26 (s, 2H), 3.51 (s, 3H); **LRMS** (ES) m/z 469.2 (M⁺ + 1).

### Example 113: Synthesis of Compound 113, 5-bromo-1-(4-(5-(difluoromethyl)-1,3,4-oxadiazole-2-yl)-2-fluorobenzyl)-6-fluoro-3-(1-methylpiper idine-4-yl)-1,3-dihydro-2H-benzo[d]imidazole-2-one

### [Step 1] Synthesis of tert-butyl 4-((5-bromo-4-fluoro-2-nitrophenyl)amino)piperidine-1-carboxylate

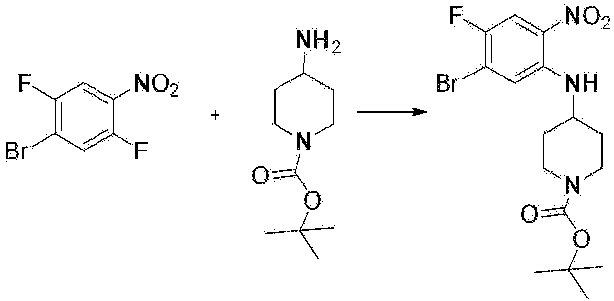

1-bromo-2,5-difluoro-4-nitrobenzene (5.000 g, 21.009 mmol), tert-butyl 4-aminopiperidine-1-carboxylate (5.049 g, 25.211 mmol) and potassium carbonate (5.807 g, 42.019 mmol) were dissolved in N,N-dimethylformamide (30 mL) at 80°C, after which the resulting solution was stirred at the same temperature for 18 hours, and then a reaction was finished by lowering a temperature to room temperature. Water was poured into the reaction mixture and an extraction was performed with ethyl acetate. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 80 g cartridge; ethyl acetate/hexane = 0 to 30%) and concentrated to obtain a title compound (6.000 g, 68.3%) in a red solid form.

### [Step 2] Synthesis of tert-butyl 4-((2-amino-5-bromo-4-fluorophenyl)amino)piperidine-1-carboxylate

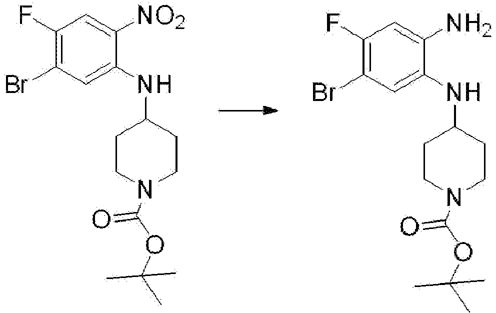

The tert-butyl 4-((5-bromo-4-fluoro-2-nitrophenyl)amino)piperidine-1-carboxylate (6.000 g, 14.345 mmol) prepared in the step 1 was dissolved in methanol (50 mL) at room temperature, after which Raney Ni (30 mg) was slowly added into the resulting solution and stirred at the same temperature for 18 hours in the presence of a hydrogen balloon attached thereto. The reaction mixture was filtered via a celite pad to remove a solid therefrom, after which solvent was removed from a resulting filtrate under reduced pressure, and then a title compound was used without an additional purification process (5.560 g, 99.8%, black oil).

### [Step 3] Synthesis of tert-butyl 4-(6-bromo-5-fluoro-2-oxo-2,3-dihydro-1H-benzo[d]imidazole-1-yl)piperidine-1-carboxylate

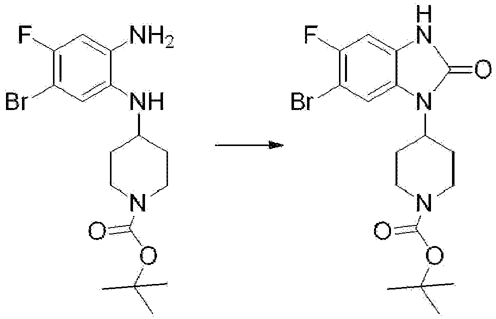

The tert-butyl 4-((2-amino-5-bromo-4-fluorophenyl)amino)piperidine-1-carboxylate (5.560 g, 14.320 mmol) prepared in the step 2 and 1,1'-carbonyldiimidazole (CDI, 3.483 g, 21.479 mmol) were dissolved in N,N-dimethylformamide (50 mL) at room temperature, after which the resulting solution was stirred at the same temperature for 18 hours. Solvent was removed from the reaction mixture under reduced pressure, after which the resulting concentrate was purified via column chromatography (SiO₂, 40 g cartridge; ethyl acetate/hexane = 0 to 50%) and concentrated to obtain a title compound (3.800 g, 64.1%) in a white solid form.

### [Step 4] Synthesis of tert-butyl 4-(6-bromo-3-(4-(5-(difluoromethyl)-1,3,4-oxadiazole-2-yl)-2-fluorobenzyl)-5-fluoro-2-oxo-2,3-dih ydro-1H-benzo[d]imidazole-1-yl)piperidine-1-carboxylate

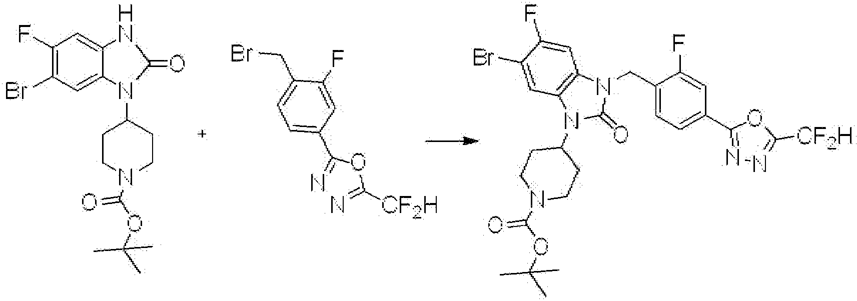

The tert-butyl 4-(6-bromo-5-fluoro-2-oxo-2,3-dihydro-1H-benzo[d]imidazole-1-yl)piperidine-1-carboxylate (2.000 g, 4.828 mmol) prepared in the step 3 was dissolved in N,N-dimethylformamide (30 mL) at 0°C, after which sodium hydride (60.00%, 0.290 g, 7.241 mmol) was added into the resulting solution and stirred at the same temperature for 30 minutes. 2-(4-(bromomethyl)-3-fluorophenyl)-5-(difluoromethyl)-1,3,4-oxadiazole (1.482 g, 4.828 mmol) was added into the reaction mixture and further stirred at room temperature for 3 hours. Water was poured into the reaction mixture and an extraction was performed with ethyl acetate. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 80 g cartridge; ethyl acetate/hexane = 0 to 70%) and concentrated to obtain a title compound (1.800 g, 58.2%) in a brown oil form.

### [Step 5] Synthesis of 5-bromo-1-(4-(5-(difluoromethyl)-1,3,4-oxadiazole-2-yl)-2-fluorobenzyl)-6-fluoro-3-(piperidine-4-y I)-1,3-dihydro-2H-benzo[d]imidazole-2-one

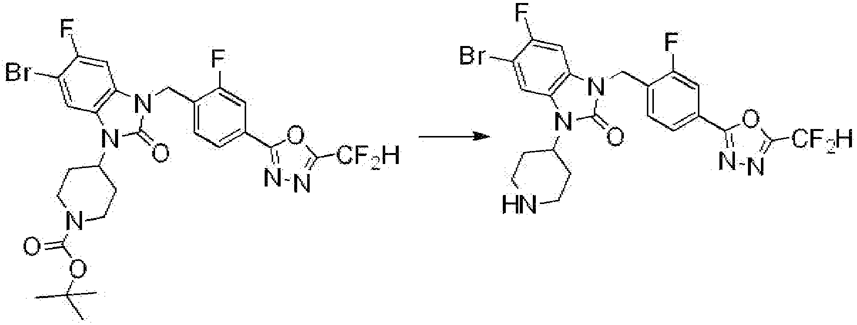

The tert-butyl 4-(6-bromo-3-(4-(5-(difluoromethyl)-1,3,4-oxadiazole-2-yl)-2-fluorobenzyl)-5-fluoro-2-oxo-2,3-dih ydro-1H-benzo[d]imidazole-1-yl)piperidine-1-carboxylate (1.700 g, 2.654 mmol) prepared in the step 4 and trifluoroacetic acid (2.033 mL, 26.545 mmol) were dissolved in dichloromethane (50 mL) at room temperature, after which the resulting solution was stirred at the same temperature for 18 hours. Saturated sodium hydrogen carbonate aqueous solution was poured into the reaction mixture and an extraction was performed with dichloromethane. An organic layer was washed with saturated sodium chloride aqueous solution, then dehydrated with anhydrous sodium sulfate, then filtered, and then concentrated under reduced pressure. A title compound was used without an additional purification process (1.000 g, 69.7%, brown oil).

### [Step 6] Synthesis of the compound 113

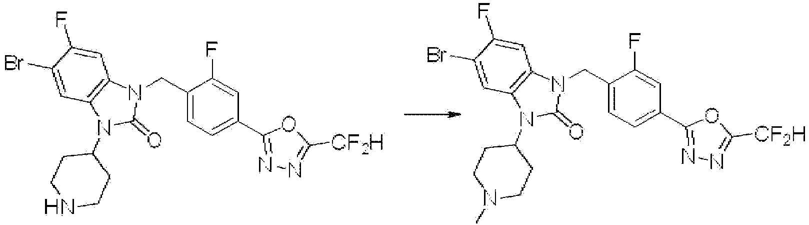

The 5-bromo-1-(4-(5-(difluoromethyl)-1,3,4-oxadiazole-2-yl)-2-fluorobenzyl)-6-fluoro-3-(piperidine-4-y I)-1,3-dihydro-2H-benzo[d]imidazole-2-one (1.000 g, 1.851 mmol) prepared in the step 5, formaldehyde (0.111 g, 3.702 mmol), acetic acid (0.212 mL, 3.702 mmol) and sodium triacetoxyborohydride (0.784 g, 3.702 mmol) were dissolved in dichloromethane (30 mL) at room temperature, after which the resulting solution was stirred at the same temperature for 12 hours. Water was poured into the reaction mixture and an extraction was performed with dichloromethane. An organic layer was washed with saturated sodium chloride aqueous solution, then dehydrated with anhydrous sodium sulfate, then filtered, and then concentrated under reduced pressure. Ethyl acetate (10 mL) and hexane (30 mL) were inserted into the resulting concentrate and stirred to filter out a precipitated solid, then washed with hexane, and then dried to obtain a title compound (0.830 g, 80.9%) in a white solid form.

**¹H NMR** (400 MHz, DMSO-d₆) δ 7.99 (d, *J* = 5.8 Hz, 1H), 7.91 - 7.84 (m, 2H), 7.69 (s, 0.25H), 7.56 (s, 0.5H), 7.45 - 7.40 (m, 2.25H), 5.22 (s, 2H), 4.59 - 4.58 (m, 1H), 3.52 - 3.49 (m, 2H), 3.18 - 3.12 (m, 2H), 2.80 - 2.73 (m, 5H), 1.95 - 1.91 (m, 2H).

### Example 114: Synthesis of Compound 114, 1-(4-(5-(difluoromethyl)-1,3,4-oxadiazole-2-yl)-2-fluorobenzyl)-6-fluoro-5-(furan-2-yl)-3-(1-methyl piperidine-4-yl)-1,3-dihydro-2H-benzo[d]imidazole-2-one

### [Step 1] Synthesis of the compound 114

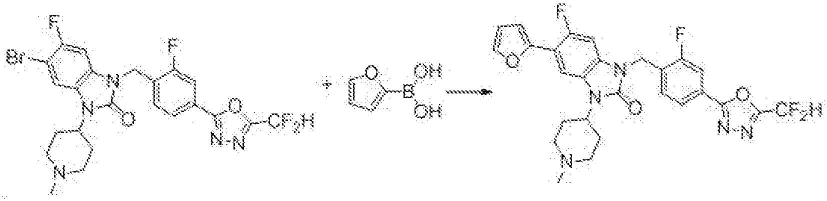

5-bromo-1-(4-(5-(difluoromethyl)-1,3,4-oxadiazole-2-yl)-2-fluorobenzyl)-6-fluoro-3-(1-me thylpiperidine-4-yl)-1,3-dihydro-2H-benzo[d]imidazole-2-one (0.150 g, 0.271 mmol), furan-2-ylboronic acid (0.061 g, 0.541 mmol), [1,1'-bis(di-tert-butylphosphino)ferrocene]palladium(II) dichloride (0.018 g, 0.027 mmol) and cesium carbonate (0.132 g, 0.406 mmol) were mixed in 1,4-dioxane (6 mL)/water (2 mL), after which the resulting mixture was irradiated with microwave, then heated at 100°C for 20 minutes, and then a reaction was finished by lowering a temperature to room temperature. Water was poured into the reaction mixture and an extraction was performed with ethyl acetate. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; methanol/dichloromethane = 0 to 10%) and concentrated to obtain a title compound (0.120 g, 81.9%) in a brown solid form.

**¹H NMR** (400 MHz, CDCl₃) δ 7.89 - 7.85 (m, 2H), 7.68 (d, *J* = 6.1 Hz, 1H), 7.50 - 7.46 (m, 2H), 7.05 (s, 0.25H), 6.92 (s, 0.5H), 6.79 (s, 0.25H), 6.78 - 6.73 (m, 2H), 6.41 - 6.50 (m, 1H), 5.17 (s, 2H), 4.46 - 4.40 (m, 1H), 3.09 - 3.06 (m, 2H), 2.56 - 2.49 (m, 2H), 2.39 (s, 3H), 2.23 - 2.17 (m, 2H), 1.89 - 1.85 (m, 2H); **LRMS** (ES) m/z 542.3 (M⁺ + 1).

### Example 115: Synthesis of Compound 115, 1-(4-(5-(difluoromethyl)-1,3,4-oxadiazole-2-yl)-2-fluorobenzyl)-6-fluoro-5-(furan-3-yl)-3-(1-methyl piperidine-4-yl)-1,3-dihydro-2H-benzo[d]imidazole-2-one

### [Step 1] Synthesis of the compound 115

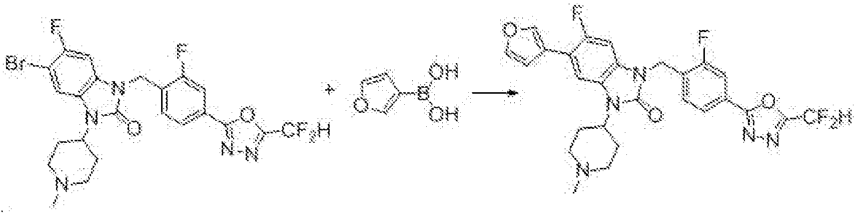

5-bromo-1-(4-(5-(difluoromethyl)-1,3,4-oxadiazole-2-yl)-2-fluorobenzyl)-6-fluoro-3-(1-me thylpiperidine-4-yl)-1,3-dihydro-2H-benzo[d]imidazole-2-one (0.150 g, 0.271 mmol), furan-3-ylboronic acid (0.061 g, 0.541 mmol), [1,1'-bis(di-tert-butylphosphino)ferrocene]palladium(II) dichloride (0.018 g, 0.027 mmol) and cesium carbonate (0.132 g, 0.406 mmol) were mixed in 1,4-dioxane (6 mL)/water (2 mL), after which the resulting mixture was irradiated with microwave, then heated at 100°C for 20 minutes, and then a reaction was finished by lowering a temperature to room temperature. Water was poured into the reaction mixture and an extraction was performed with ethyl acetate. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; methanol/dichloromethane = 0 to 10%) and concentrated to obtain a title compound (0.130 g, 88.7%) in a brown solid form.

**¹H NMR** (400 MHz, CDCl₃) δ 7.89 - 7.85 (m, 2H), 7.82 - 7.81 (m, 1H), 7.50 - 7.45 (m, 2H), 7.34 (d, *J* = 6.0 Hz, 1H), 7.05 (s, 0.25H), 6.92 (s, 0.5H), 6.79 (s, 0.25H), 6.79 - 6.77 (m, 2H), 5.31 (s, 2H), 4.47 - 4.39 (m, 1H), 3.07 - 3.04 (m, 2H), 2.55 - 2.45 (m, 2H), 2.37 (s, 3H), 2.23 - 2.18 (m, 2H), 1.89 - 1.86 (m, 2H); **LRMS** (ES) m/z 542.3 (M⁺ + 1).

### Example 116: Synthesis of Compound 116, 1-(4-(5-(difluoromethyl)-1,3,4-oxadiazole-2-yl)-2-fluorobenzyl)-6-fluoro-5-(2-fluorophenyl)-3-(1-m ethylpiperidine-4-yl)-1,3-dihydro-2H-benzo[d]imidazole-2-one

### [Step 1] Synthesis of the compound 116

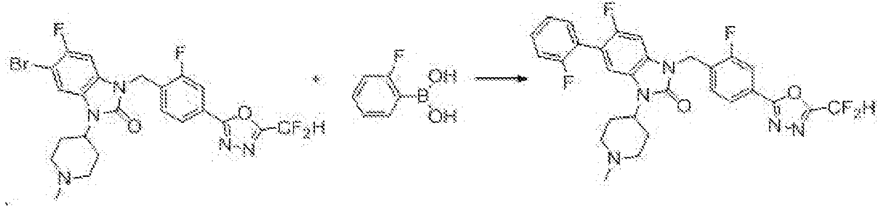

5-bromo-1-(4-(5-(difluoromethyl)-1,3,4-oxadiazole-2-yl)-2-fluorobenzyl)-6-fluoro-3-(1-me thylpiperidine-4-yl)-1,3-dihydro-2H-benzo[d]imidazole-2-one (0.150 g, 0.271 mmol), (2-fluorophenyl)boronic acid (0.076 g, 0.541 mmol), [1,1'-bis(di-tert-butylphosphino)ferrocene]palladium(II) dichloride (0.018 g, 0.027 mmol) and cesium carbonate (0.132 g, 0.406 mmol) were mixed in 1,4-dioxane (6 mL)/water (2 mL), after which the resulting mixture was irradiated with microwave, then heated at 100°C for 20 minutes, and then a reaction was finished by lowering a temperature to room temperature. Water was poured into the reaction mixture and an extraction was performed with ethyl acetate. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; methanol/dichloromethane = 0 to 10%) and concentrated to obtain a title compound (0.110 g, 71.4%) in a brown oil form.

**¹H NMR** (400 MHz, CDCl₃) δ 7.89 (d, *J* = 9.1 Hz, 1H), 7.53 (t, *J* = 7.7 Hz, 1H), 7.40 - 7.33 (m, 2H), 7.28 - 7.27 (m, 1H), 7.23 - 7.13 (m, 1H), 7.05 (s, 0.25H), 6.92 (s, 0.5H), 6.83 (d, *J =* 9.2 Hz, 1H), 6.79 (s, 0.25H), 5.20 (s, 2H), 4.48 - 4.42 (m, 1H), 3.04 (d, *J =* 11.7 Hz, 1H), 2.51 - 2.41 (m, 2H), 2.34 (s, 3H), 2.21 - 2.15 (m, 2H), 1.89 - 1.86 (m, 2H); **LRMS** (ES) m/z 570.3 (M⁺+ 1).

### Example 117: Synthesis of Compound 117, 1-(4-(5-(difluoromethyl)-1,3,4-oxadiazole-2-yl)-2-fluorobenzyl)-6-fluoro-3-(1-methylpiperidine-4-yl )-5-(pyridine-3-yl)-1,3-dihydro-2H-benzo[d]imidazole-2-one

### [Step 1] Synthesis of the compound 117

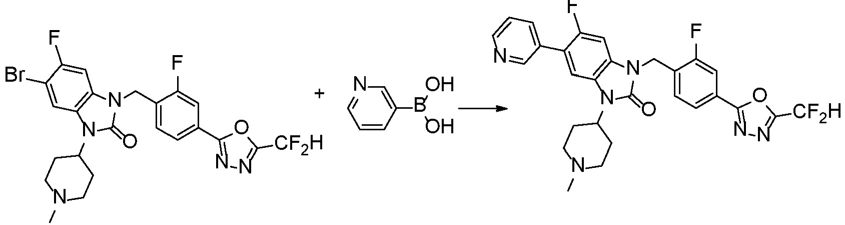

5-bromo-1-(4-(5-(difluoromethyl)-1,3,4-oxadiazole-2-yl)-2-fluorobenzyl)-6-fluoro-3-(1-me thylpiperidine-4-yl)-1,3-dihydro-2H-benzo[d]imidazole-2-one (0.150 g, 0.271 mmol), pyridine-3-ylboronic acid (0.067 g, 0.541 mmol), [1,1'-bis(di-tert-butylphosphino)ferrocene]palladium(II) dichloride (0.018 g, 0.027 mmol) and cesium carbonate (0.132 g, 0.406 mmol) were mixed in 1,4-dioxane (6 mL)/water (2 mL), after which the resulting mixture was irradiated with microwave, then heated at 100°C for 20 minutes, and then a reaction was finished by lowering a temperature to room temperature. Water was poured into the reaction mixture and an extraction was performed with ethyl acetate. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; methanol/dichloromethane = 0 to 10%) and concentrated to obtain a title compound (0.090 g, 60.2%) in a brown oil form.

**¹H NMR** (400 MHz, CDCl₃) δ 8.74 (s, 1H), 8.60 (dd, *J =* 4.8, 1.5 Hz, 1H), 7.88 (d, *J* = 8.9 Hz, 2H), 7.82 (dd, *J* = 8.0, 1.7 Hz, 1H), 7.52 (t, *J* = 7.6 Hz, 1H), 7.37 (dd, *J* = 7.9, 4.9 Hz, 1H), 7.32 (d, *J* = 6.2 Hz, 1H), 7.05 (s, 0.25H), 6.93 (s, 0.5H), 6.85 (d, *J* = 9.7 Hz, 1H), 6.79 (s, 0.25H), 5.20 (s, 2H), 4.49 - 4.41 (m, 1H), 3.05 - 3.02 (m, 2H), 2.54 - 2.43 (m, 2H), 2.35 (s, 3H), 2.22 - 2.16 (m, 2H), 1.89 - 1.86 (m, 2H); **LRMS** (ES) m/z 553.4 (M⁺ + 1).

### Example 118: Synthesis of Compound 118, 1-(4-(5-(difluoromethyl)-1,3,4-oxadiazole-2-yl)-2-fluorobenzyl)-6-fluoro-3-(1-methylpiperidine-4-yl )-5-(pyridine-4-yl)-1,3-dihydro-2H-benzo[d]imidazole-2-one

### [Step 1] Synthesis of the compound 118

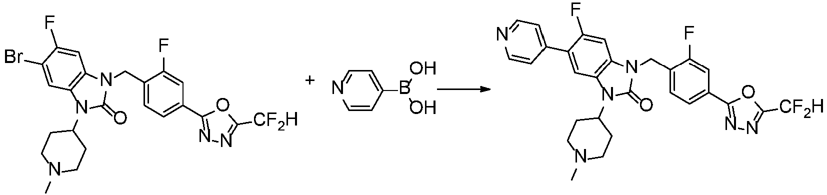

5-bromo-1-(4-(5-(difluoromethyl)-1,3,4-oxadiazole-2-yl)-2-fluorobenzyl)-6-fluoro-3-(1-me thylpiperidine-4-yl)-1,3-dihydro-2H-benzo[d]imidazole-2-one (0.150 g, 0.271 mmol), pyridine-4-ylboronic acid (0.067 g, 0.541 mmol), [1,1'-bis(di-tert-butylphosphino)ferrocene]palladium(II) dichloride (0.018 g, 0.027 mmol) and cesium carbonate (0.132 g, 0.406 mmol) were mixed in 1,4-dioxane (6 mL)/water (2 mL), after which the resulting mixture was irradiated with microwave, then heated at 100°C for 20 minutes, and then a reaction was finished by lowering a temperature to room temperature. Water was poured into the reaction mixture and an extraction was performed with ethyl acetate. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; methanol/dichloromethane = 0 to 10%) and concentrated to obtain a title compound (0.100 g, 66.9%) in a brown oil form.

**¹H NMR** (400 MHz, CDCl₃) δ 8.66 (d, *J =* 5.6 Hz, 1H), 7.88 (d, *J =* 8.9 Hz, 2H), 7.52 (t, *J* = 7.7 Hz, 1H), 7.44 (d, *J =* 4.6 Hz, 2H), 7.34 (d, *J =* 6.2 Hz, 1H), 7.05 (s, 0.25H), 6.92 (s, 0.5H), 6.85 (d, *J =* 10.0 Hz, 1H), 6.79 (s, 0.25H), 5.20 (s, 2H), 4.49 - 4.41 (m, 1H), 3.06 - 3.04 (m, 2H), 2.53 - 2.43 (m, 2H), 2.36 (s, 3H), 2.23 - 2.17 (m, 2H), 1.89 - 1.86 (m, 2H); **LRMS** (ES) m/z 553.3 (M⁺ + 1).

### Example 119: Synthesis of Compound 119, 5-bromo-3-(4-(5-(difluoromethyl)-1,3,4-oxadiazole-2-yl)-2-fluorobenzyl)-1-(1-methylpiperidine-4-yl)-1,3-dihydro-2H-benzo[d]imidazole-2-one

### [Step 1] Synthesis of tert-butyl 4-((4-bromo-2-nitrophenyl)amino)piperidine-1-carboxylate

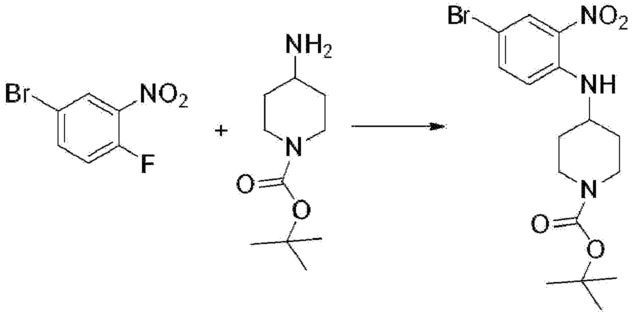

4-bromo-1-fluoro-2-nitrobenzene (5.000 g, 22.727 mmol), tert-butyl 4-aminopiperidine-1-carboxylate (5.007 g, 25.000 mmol), potassium carbonate (6.282 g, 45.455 mmol) and potassium iodide (0.377 g, 2.273 mmol) were dissolved in N,N-dimethylformamide (300 mL) at room temperature, after which the resulting solution was stirred at 80°C for 18 hours, and then a reaction was finished by lowering a temperature to room temperature. The reaction mixture was filtered via a glass filter to remove a solid therefrom, after which solvent was removed from the resulting filtrate under reduced pressure, and then a title compound was used without an additional purification process (9.000 g, 98.9%, orange solid).

### [Step 2] Synthesis of tert-butyl 4-((2-amino-4-bromophenyl)amino)piperidine-1-carboxylate

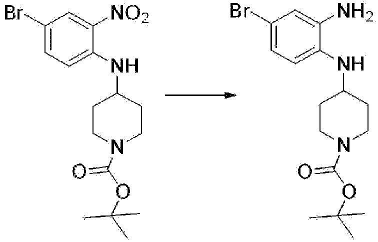

The tert-butyl 4-((4-bromo-2-nitrophenyl)amino)piperidine-1-carboxylate (11.000 g, 27.481 mmol) prepared in the step 1 and Raney nickel (1.1 g, 10 wt%) were dissolved in methanol (250 mL) at room temperature, after which the resulting solution was stirred at the same temperature for 18 hours in the presence of a hydrogen balloon. The reaction mixture was filtered via a celite pad to remove a solid therefrom, after which solvent was removed from the resulting filtrate under reduced pressure, and then a title compound was used without an additional purification process (9.580 g, 94.1%, red solid).

### [Step 3] Synthesis of tert-butyl 4-(5-bromo-2-oxo-2,3-dihydro-1H-benzo[d]imidazole-1-yl)piperidine-1-carboxylate

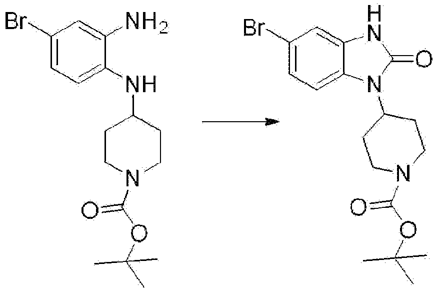

The tert-butyl 4-((2-amino-4-bromophenyl)amino)piperidine-1-carboxylate (9.580 g, 25.872 mmol) prepared in the step 2 and 1,1'-carbonyldiimidazole (CDI, 5.873 g, 36.220 mmol) were dissolved in dichloromethane (200 mL) at room temperature, after which the resulting solution was stirred at 65°C for 3 hours, and then a reaction was finished by lowering a temperature to room temperature. Solvent was removed from the reaction mixture under reduced pressure, after which the resulting concentrate was purified via column chromatography (SiO₂, 120 g cartridge; ethyl acetate/hexane = 0 to 80%) and concentrated to obtain a title compound (7.090 g, 69.2%) in a pink solid form.

### [Step 4] Synthesis of tert-butyl 4-(5-bromo-3-(4-(5-(difluoromethyl)-1,3,4-oxadiazole-2-yl)-2-fluorobenzyl)-2-oxo-2,3-dihydro-1H-benzo[d]imidazole-1-yl)piperidine-1-carboxylate

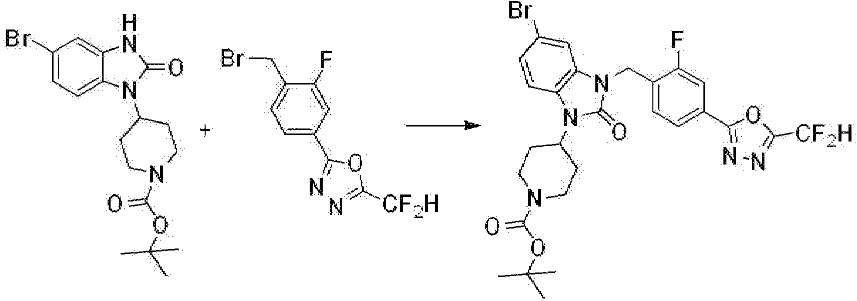

The tert-butyl 4-(5-bromo-2-oxo-2,3-dihydro-1H-benzo[d]imidazole-1-yl)piperidine-1-carboxylate (2.300 g, 5.804 mmol) prepared in the step 3 was dissolved in N,N-dimethylformamide (50 mL) at 0°C, after which sodium hydride (60.00%, 0.348 g, 8.706 mmol) was added into the resulting solution and stirred at the same temperature for 30 minutes. 2-(4-(bromomethyl)-3-fluorophenyl)-5-(difluoromethyl)-1,3,4-oxadiazole (2.317 g, 7.545 mmol) was added into the reaction mixture and further stirred at room temperature for 4 hours. Water was poured into the reaction mixture and an extraction was performed with ethyl acetate. An organic layer was washed with saturated sodium chloride aqueous solution, then dehydrated with anhydrous magnesium sulfate, then filtered, and then concentrated under reduced pressure. The resulting concentrate was purified via column chromatography (SiO₂, 40 g cartridge; ethyl acetate/hexane = 0 to 100%) and concentrated to obtain a title compound (3.415 g, 94.5%) in a brown solid form.

### [Step 5] Synthesis of the compound 119

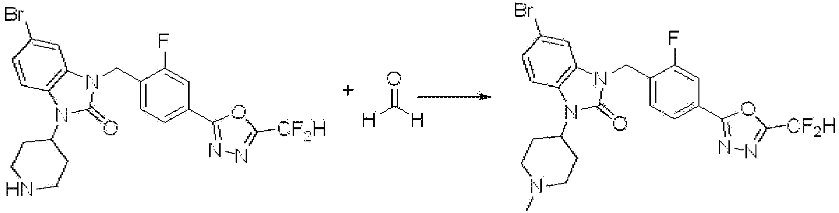

The tert-butyl 4-(5-bromo-3-(4-(5-(difluoromethyl)-1,3,4-oxadiazole-2-yl)-2-fluorobenzyl)-2-oxo-2,3-dihydro-1H-benzo[d]imidazole-1-yl)piperidine-1-carboxylate prepared in the step 4 was used to perform a deprotection reaction in substantially the same manner as shown in the step 5 of Example 113 so as to obtain 5-bromo-3-(4-(5-(difluoromethyl)-1,3,4-oxadiazole-2-yl)-2-fluorobenzyl)-1-(piperidine-4-yl)-1,3-di hydro-2H-benzo[d]imidazole-2-one.

Then, 5-bromo-3-(4-(5-(difluoromethyl)-1,3,4-oxadiazole-2-yl)-2-fluorobenzyl)-1-(piperidine-4-yl)-1,3-di hydro-2H-benzo[d]imidazole-2-one (2.072 g, 3.966 mmol), sodium triacetoxyborohydride (1.681 g, 7.932 mmol) and formaldehyde (0.357 g, 11.898 mmol) were dissolved in dichloromethane (40 mL) at room temperature, after which the resulting solution was stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 24 g cartridge; methanol/dichloromethane = 0 to 5%) and concentrated to obtain a title compound (0.362 g, 17.0%) in a white solid form.

**¹H NMR** (400 MHz, CDCl₃) δ 7.87 - 7.83 (m, 2H), 7.42 (t, *J* = 7.7 Hz, 1H), 7.18 (s, 2H), 7.07 (s, 1H), 6.90 (t, *J =* 51.6 Hz, 1H), 5.15 (s, 2H), 4.45 - 4.36 (m, 1H), 3.02 (d, *J =* 11.8 Hz, 2H), 2.48 - 2.38 (m, 2H), 2.34 (s, 3H), 2.15 (td, *J =* 11.9, 2.0 Hz, 2H), 1.90 - 1.80 (m, 2H); **LRMS** (ES) m/z 538.1 (M⁺ + 1).

### Example 120: Synthesis of Compound 120, 3-(4-(5-(difluoromethyl)-1,3,4-oxadiazole-2-yl)-2-fluorobenzyl)-5-(3-fluorophenyl)-1-(1-methylpip eridine-4-yl)-1,3-dihydro-2H-benzo[d]imidazole-2-one

### [Step 1] Synthesis of the compound 120

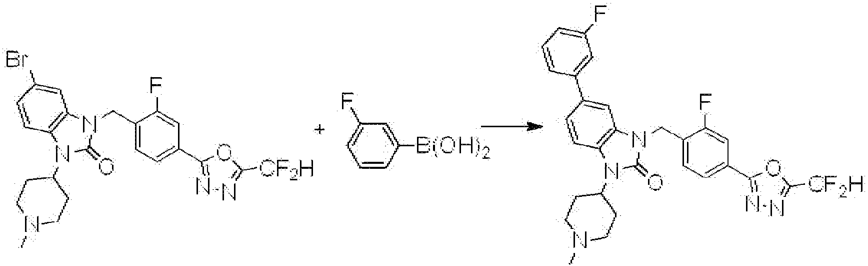

The 5-bromo-3-(4-(5-(difluoromethyl)-1,3,4-oxadiazole-2-yl)-2-fluorobenzyl)-1-(1-methylpiperidine-4-yl)-1,3-dihydro-2H-benzo[d]imidazole-2-one (0.150 g, 0.280 mmol) prepared in the step 5 of the compound 119, (3-fluorophenyl)boronic acid (0.047 g, 0.336 mmol), [1,1'-bis(di-tert-butylphosphino)ferrocene]palladium dichloride (0.009 g, 0.014 mmol) and cesium carbonate (0.182 g, 0.559 mmol) were mixed in 1,4-dioxane (2.3 mL)/water (0.7 mL) at room temperature, after which the resulting mixture was irradiated with microwave, then heated at 100°C for 30 minutes, and then a reaction was finished by lowering a temperature to room temperature. Solvent was removed from the reaction mixture under reduced pressure, after which water was poured into a resulting concentrate, and an extraction was performed with dichloromethane, then filtered via a plastic filter to remove a solid residue and an aqueous solution layer therefrom, and then concentrated under reduced pressure. The resulting concentrate was purified via column chromatography (SiO₂, 12 g cartridge; methanol/dichloromethane = 0 to 10%) and concentrated to obtain a title compound (0.074 g, 48.2%) in a brown solid form.

**¹H NMR** (400 MHz, CDCl₃) δ 7.87 - 7.82 (m, 2H), 7.48 (t, *J* = 7.8 Hz, 1H), 7.40 - 7.33 (m, 2H), 7.28 - 7.26 (m, 2H), 7.19 (dt, *J =* 10.2, 2.1 Hz, 1H), 7.15 (d, *J=* 1.6 Hz, 1H), 7.01 - 6.97 (m, 1H), 6.90 (t, *J =* 51.7 Hz, 1H), 5.24 (s, 2H), 4.50 - 4.42 (m, 1H), 3.06 (d, *J* = 11.7 Hz, 2H), 2.59 - 2.48 (m, 2H), 2.39 (s, 3H), 2.23 (t, *J =* 11.5 Hz, 2H), 1.87 (dd, *J =* 11.9, 2.5 Hz, 2H); **LRMS** (ES) m/z 551.9 (M⁺ + 1).

### Example 121: Synthesis of Compound 121, 3-(4-(5-(difluoromethyl)-1,3,4-oxadiazole-2-yl)-2-fluorobenzyl)-5-(2,5-difluorophenyl)-1-(1-methyl piperidine-4-yl)-1,3-dihydro-2H-benzo[d]imidazole-2-one

### [Step 1] Synthesis of the compound 121

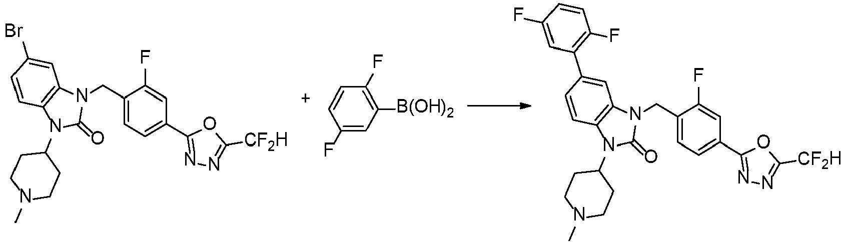

The 5-bromo-3-(4-(5-(difluoromethyl)-1,3,4-oxadiazole-2-yl)-2-fluorobenzyl)-1-(1-methylpiperidine-4-yl)-1,3-dihydro-2H-benzo[d]imidazole-2-one (0.120 g, 0.224 mmol) prepared in the step 5 of the compound 119, (2,5-difluorophenyl)boronic acid (0.042 g, 0.268 mmol), [1,1'-bis(di-tert-butylphosphino)ferrocene]palladium dichloride (0.007 g, 0.011 mmol) and cesium carbonate (0.146 g, 0.447 mmol) were mixed in 1,4-dioxane (1.7 mL)/water (0.6 mL) at room temperature, after which the resulting mixture was irradiated with microwave, then heated at 100°C for 30 minutes, and then a reaction was finished by lowering a temperature to room temperature. Solvent was removed from the reaction mixture under reduced pressure, after which saturated sodium hydrogen carbonate aqueous solution was poured into a resulting concentrate, and an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.032 g, 24.9%) in a brown solid form.

**¹H NMR** (400 MHz, CDCl₃) δ 7.86 - 7.82 (m, 2H), 7.47 (t, *J =* 7.8 Hz, 1H), 7.40 (d, *J =* 8.3 Hz, 1H), 7.22 (d, *J =* 8.3 Hz, 1H), 7.14 (s, 1H), 7.10 - 7.05 (m, 2H), 6.98 - 6.93 (m, 1H), 6.89 (t, *J* = 51.7 Hz, 1H), 5.23 (s, 2H), 4.51 - 4.42 (m, 1H), 3.05 (d, *J =* 11.6 Hz, 2H), 2.56 - 2.46 (m, 2H), 2.37 (s, 3H), 2.19 (t, *J* = 11.2 Hz, 2H), 1.87 (dd, *J* = 12.0, 2.2 Hz, 2H); **LRMS** (ES) m/z 570.0 (M⁺ + 1).

### Example 122: Synthesis of Compound 122, 3-(4-(5-(difluoromethyl)-1,3,4-oxadiazole-2-yl)-2-fluorobenzyl)-1-(1-methylpiperidine-4-yl)-5-(4-(tr ifluoromethyl)phenyl)-1,3-dihydro-2H-benzo[d]imidazole-2-one

### [Step 1] Synthesis of the compound 122

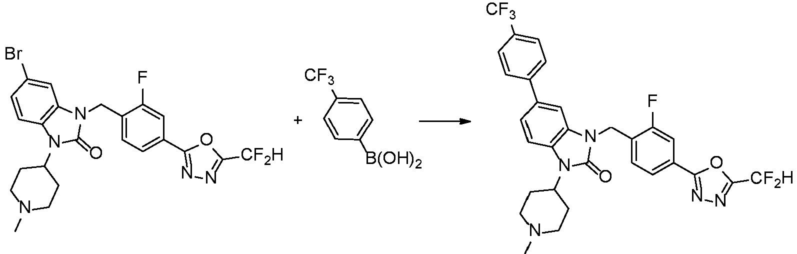

The 5-bromo-3-(4-(5-(difluoromethyl)-1,3,4-oxadiazole-2-yl)-2-fluorobenzyl)-1-(1-methylpiperidine-4-yl)-1,3-dihydro-2H-benzo[d]imidazole-2-one (0.120 g, 0.224 mmol) prepared in the step 5 of the compound 119, (4-(trifluoromethyl)phenyl)boronic acid (0.051 g, 0.268 mmol), [1,1'-bis(di-tert-butylphosphino)ferrocene]palladium dichloride (0.007 g, 0.011 mmol) and cesium carbonate (0.146 g, 0.447 mmol) were mixed in 1,4-dioxane (1.7 mL)/water (0.6 mL) at room temperature, after which the resulting mixture was irradiated with microwave, then heated at 100°C for 30 minutes, and then a reaction was finished by lowering a temperature to room temperature. Solvent was removed from the reaction mixture under reduced pressure, after which saturated sodium hydrogen carbonate aqueous solution was poured into a resulting concentrate, and an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.033 g, 24.6%) in a brown solid form.

**¹H NMR** (400 MHz, CDCl₃) δ 8.14 (d, *J =* 7.6 Hz, 1H), 7.87 - 7.84 (m, 2H), 7.67 - 7.58 (m, 4H), 7.50 (t, *J = 7.8* Hz, 1H), 7.30 (dd, *J* = 8.3, 1.3 Hz, 1H), 7.18 (s, 1H), 6.89 (t, *J* = 51.7 Hz, 1H), 5.24 (s, 2H), 4.48 - 4.41 (m, 1H), 3.12 (d, *J* = 11.3 Hz, 2H), 2.64 - 2.61 (m, 2H), 2.61 - 2.20 (m, 5H), 1.89 (d, *J =* 10.2 Hz, 2H); **LRMS** (ES) m/z 602.1 (M⁺ + 1).

### Example 123: Synthesis of Compound 123, 3-(4-(5-(difluoromethyl)-1,3,4-oxadiazole-2-yl)-2-fluorobenzyl)-5-(2,5-difluorophenyl)-1-(1-methyl piperidine-4-yl)-1,3-dihydro-2H-benzo[d]imidazole-2-one

### [Step 1] Synthesis of the compound 123

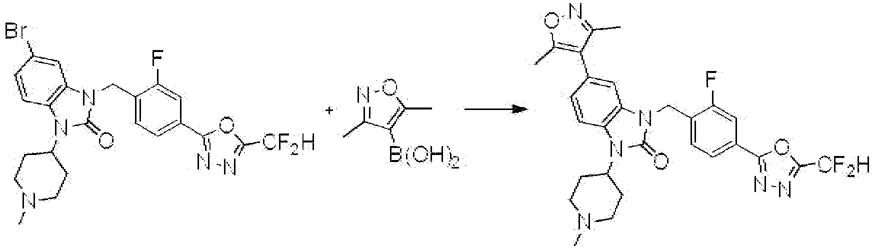

The 5-bromo-3-(4-(5-(difluoromethyl)-1,3,4-oxadiazole-2-yl)-2-fluorobenzyl)-1-(1-methylpiperidine-4-yl)-1,3-dihydro-2H-benzo[d]imidazole-2-one (0.120 g, 0.224 mmol) prepared in the step 5 of the compound 119, (2,5-difluorophenyl)boronic acid (0.042 g, 0.268 mmol), [1,1'-bis(di-tert-butylphosphino)ferrocene]palladium dichloride (0.007 g, 0.011 mmol) and cesium carbonate (0.146 g, 0.447 mmol) were mixed in 1,4-dioxane (1.7 mL)/water (0.6 mL) at room temperature, after which the resulting mixture was irradiated with microwave, then heated at 100°C for 30 minutes, and then a reaction was finished by lowering a temperature to room temperature. Solvent was removed from the reaction mixture under reduced pressure, after which saturated sodium hydrogen carbonate aqueous solution was poured into a resulting concentrate, and an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.032 g, 24.9%) in a brown solid form.

**¹H NMR** (400 MHz, CDCl₃) δ 7.86 - 7.82 (m, 2H), 7.55 (t, *J =* 7.7 Hz, 1H), 7.37 (d, *J =* 8.2 Hz, 1H), 6.92 (dd, *J =* 8.2, 1.6 Hz, 1H), 6.90 (t, *J* = 51.6 Hz, 1H), 6.85 (d, *J* = 1.3 Hz, 1H), 5.19 (s, 2H), 4.50 - 4.41 (m, 1H), 3.05 (d, *J =* 11.6 Hz, 2H), 2.54 - 2.46 (m, 2H), 2.36 (s, 3H), 2.33 (s, 3H), 2.22 - 2.16 (m, 5H), 1.87 (dd, *J =* 12.0, 2.2 Hz, 2H); **LRMS** (ES) m/z 554.2 (M⁺ + 2).

### Example 124: Synthesis of Compound 124, 3-(4-(5-(difluoromethyl)-1,3,4-oxadiazole-2-yl)-2-fluorobenzyl)-5-(5-methylfuran-2-yl)-1-(1-methyl piperidine-4-yl)-1,3-dihydro-2H-benzo[d]imidazole-2-one

### [Step 1] Synthesis of the compound 124

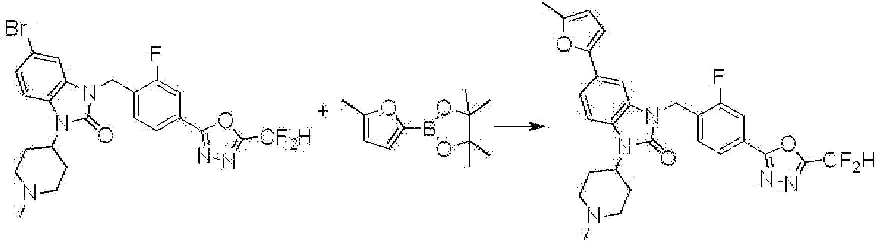

The 5-bromo-3-(4-(5-(difluoromethyl)-1,3,4-oxadiazole-2-yl)-2-fluorobenzyl)-1-(1-methylpiperidine-4-yl)-1,3-dihydro-2H-benzo[d]imidazole-2-one (0.120 g, 0.224 mmol) prepared in the step 5 of the compound 119, 4,4,5,5-tetramethyl-2-(5-methylfuran-2-yl)-1,3,2-dioxaborolane (0.056 g, 0.268 mmol), [1,1'-bis(di-tert-butylphosphino)ferrocene]palladium dichloride (0.007 g, 0.011 mmol) and cesium carbonate (0.146 g, 0.447 mmol) were mixed in 1,4-dioxane (1.7 mL)/water (0.6 mL) at room temperature, after which the resulting mixture was irradiated with microwave, then heated at 100°C for 30 minutes, and then a reaction was finished by lowering a temperature to room temperature. Solvent was removed from the reaction mixture under reduced pressure, after which saturated sodium hydrogen carbonate aqueous solution was poured into a resulting concentrate, and an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.030 g, 25.2%) in a brown solid form.

**¹H NMR** (400 MHz, CDCl₃) δ 7.87 - 7.80 (m, 2H), 7.41 (t, *J =* 7.6 Hz, 1H), 7.34 - 7.28 (m, 2H), 7.19 (d, *J =* 1.0 Hz, 1H), 6.89 (t, *J =* 51.7 Hz, 1H), 6.41 (d, *J =* 3.2 Hz, 1H), 6.01 - 6.00 (m, 1H), 5.28 (s, 2H), 4.47 - 4.39 (m, 1H), 3.04 (d, *J =* 11.7 Hz, 2H), 2.54 - 2.43 (m, 2H), 2.36 (s, 3H), 2.34 (s, 3H), 2.20 - 2.15 (m, 2H), 1.86 (dd, *J =* 12.1, 2.2 Hz, 2H); **LRMS** (ES) m/z 538.0 (M⁺ + 1).

### Example 125: Synthesis of Compound 125, 3-(4-(5-(difluoromethyl)-1,3,4-oxadiazole-2-yl)-2-fluorobenzyl)-1-(1-methylpiperidine-4-yl)-5-(pyri dine-3-yl)-1,3-dihydro-2H-benzo[d]imidazole-2-one

### [Step 1] Synthesis of the compound 125

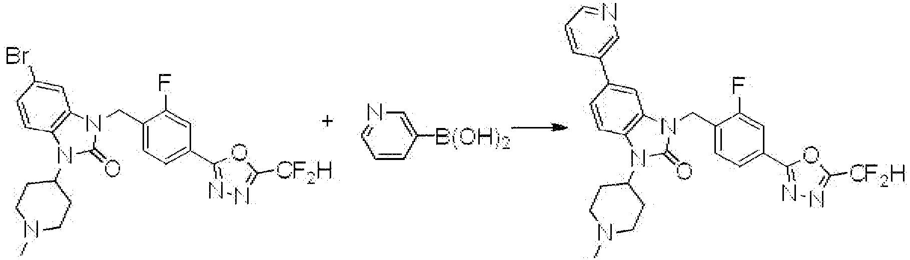

The 5-bromo-3-(4-(5-(difluoromethyl)-1,3,4-oxadiazole-2-yl)-2-fluorobenzyl)-1-(1-methylpiperidine-4-yl)-1,3-dihydro-2H-benzo[d]imidazole-2-one (0.150 g, 0.280 mmol) prepared in the step 5 of the compound 119, pyridine-3-ylboronic acid (0.041 g, 0.336 mmol), [1,1'-bis(di-tert-butylphosphino)ferrocene]palladium dichloride (0.009 g, 0.014 mmol) and cesium carbonate (0.182 g, 0.559 mmol) were mixed in 1,4-dioxane (2.3 mL)/water (0.7 mL) at room temperature, after which the resulting mixture was irradiated with microwave, then heated at 100°C for 30 minutes, and then a reaction was finished by lowering a temperature to room temperature. Solvent was removed from the reaction mixture under reduced pressure, after which water was poured into a resulting concentrate, and an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 10%) and concentrated to obtain a title compound (0.077 g, 51.6%) in a brown solid form.

**¹H NMR** (400 MHz, CDCl₃) δ 8.75 - 8.74 (m, 1H), 8.53 (dd, *J* = 4.8, 1.6 Hz, 1H), 7.85 - 7.81 (m, 2H), 7.77 (dt, *J* = 8.0, 1.6 Hz, 1H), 7.48 (t, *J =* 7.8 Hz, 1H), 7.42 (d, *J =* 8.3 Hz, 1H), 7.32 (ddd, *J =* 7.9, 4.8, 0.6 Hz, 1H), 7.27 - 7.14 (m, 1H), 7.14 (d, *J* = 1.6 Hz, 1H), 6.89 (t, *J =* 51.7 Hz, 1H), 5.23 (s, 2H), 4.49 - 4.41 (m, 1H), 3.03 (d, *J* = 11.6 Hz, 2H), 2.55 - 2.45 (m, 2H), 2.35 (s, 3H), 2.20 - 2.15 (m, 2H), 1.86 (dd, *J =* 12.0, 2.3 Hz, 2H); **LRMS** (ES) m/z 535.3 (M⁺ + 1).

### Example 126: Synthesis of Compound 126, 5-bromo-3-(4-(5-(difluoromethyl)-1,3,4-oxadiazole-2-yl)benzyl)-1-(1-methylpiperidine-4-yl)-1,3-di hydro-2H-benzo[d]imidazole-2-one

### [Step 1] Synthesis of tert-butyl 4-(5-bromo-3-(4-(5-(difluoromethyl)-1,3,4-oxadiazole-2-yl)benzyl)-2-oxo-2,3-dihydro-1H-benzo[d ]imidazole-1-yl)piperidine-1-carboxylate

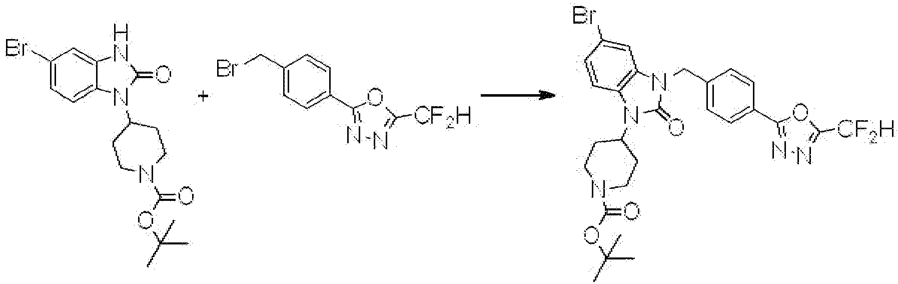

The tert-butyl 4-(5-bromo-2-oxo-2,3-dihydro-1H-benzo[d]imidazole-1-yl)piperidine-1-carboxylate (2.300 g, 5.804 mmol) prepared in the step 3 of the compound 119 was dissolved in N,N-dimethylformamide (50 mL) at 0°C, after which sodium hydride (60.00%, 0.348 g, 8.706 mmol) was added into the resulting solution and stirred at the same temperature for 30 minutes. 2-(4-(bromomethyl)phenyl)-5-(difluoromethyl)-1,3,4-oxadiazole (2.181 g, 7.545 mmol) was added into the reaction mixture and further stirred at room temperature for 4 hours. Water was poured into the reaction mixture and an extraction was performed with ethyl acetate. An organic layer was washed with saturated sodium chloride aqueous solution, then dehydrated with anhydrous magnesium sulfate, then filtered, and then concentrated under reduced pressure. The resulting concentrate was purified via column chromatography (SiO₂, 40 g cartridge; ethyl acetate/hexane = 0 to 100%) and concentrated to obtain a title compound (3.390 g, 96.6%) in a brown solid form.

### [Step 2] Synthesis of 5-bromo-3-(4-(5-(difluoromethyl)-1,3,4-oxadiazole-2-yl)benzyl)-1-(piperidine-4-yl)-1,3-dihydro-2 H-benzo[d]imidazole-2-one

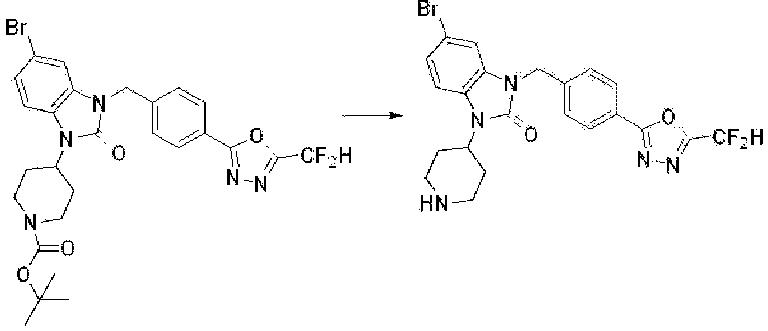

The tert-butyl 4-(5-bromo-3-(4-(5-(difluoromethyl)-1,3,4-oxadiazole-2-yl)benzyl)-2-oxo-2,3-dihydro-1H-benzo[d ]imidazole-1-yl)piperidine-1-carboxylate (3.390 g, 5.608 mmol) prepared in the step 1 and trifluoroacetic acid (4.295 mL, 56.084 mmol) were dissolved in dichloromethane (5 mL) at room temperature, after which the resulting solution was stirred at the same temperature for 4 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 24 g cartridge; ethyl acetate/hexane = 80 to 100%) and concentrated, after which an obtained product was purified again via chromatography (SiO₂, 24 g cartridge; methanol/dichloromethane = 0 to 80%) and concentrated to obtain a title compound (2.154 g, 76.2%) in a brown solid form.

### [Step 3] Synthesis of the compound 126

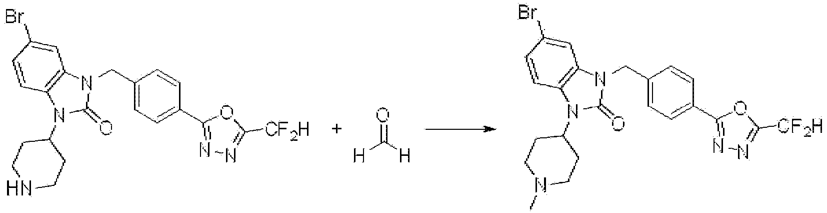

The 5-bromo-3-(4-(5-(difluoromethyl)-1,3,4-oxadiazole-2-yl)benzyl)-1-(piperidine-4-yl)-1,3-dihydro-2 H-benzo[d]imidazole-2-one (1.320 g, 2.616 mmol) prepared in the step 2, sodium triacetoxyborohydride (1.109 g, 5.233 mmol) and formaldehyde (0.236 g, 7.849 mmol) were dissolved in dichloromethane (25 mL) at room temperature, after which the resulting solution was stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 12 g cartridge; methanol/dichloromethane = 0 to 5%) and concentrated to obtain a title compound (0.760 g, 56.0%) in a white solid form.

**¹H NMR** (400 MHz, CDCl₃) δ 8.09 (d, *J* = 8.4 Hz, 2H), 7.45 (d, *J* = 8.4 Hz, 2H), 7.02 - 7.15 (m, 2H), 6.96 (d, *J* = 1.4 Hz, 1H), 6.90 (t, *J* = 51.8 Hz, 1H), 5.11 (s, 2H), 4.46 - 4.38 (m, 1H), 3.02 (d, *J =* 11.8 Hz, 2H), 2.49 - 2.39 (m, 2H), 2.35 (s, 3H), 2.19 - 2.13 (m, 2H), 1.84 (dd, *J =* 11.9, 2.4 Hz, 2H); **LRMS** (ES) m/z 518.2 (M⁺ + 1).

### Example 127: Synthesis of Compound 127, 3-(4-(5-(difluoromethyl)-1,3,4-oxadiazole-2-yl)benzyl)-1-(1-methylpiperidine-4-yl)-5-(pyridine-3-yl )-1,3-dihydro-2H-benzo[d]imidazole-2-one

### [Step 1] Synthesis of the compound 127

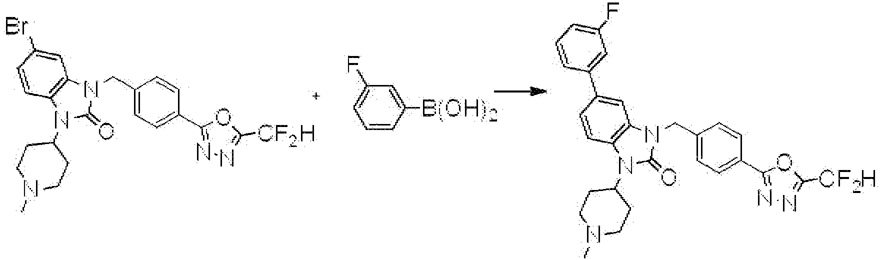

The 5-bromo-3-(4-(5-(difluoromethyl)-1,3,4-oxadiazole-2-yl)benzyl)-1-(1-methylpiperidine-4-yl)-1,3-di hydro-2H-benzo[d]imidazole-2-one (0.120 g, 0.231 mmol) prepared in the step 3 of the compound 126, pyridine-3-ylboronic acid (0.034 g, 0.278 mmol), [1,1'-bis(di-tert-butylphosphino)ferrocene]palladium dichloride (0.008 g, 0.012 mmol) and cesium carbonate (0.151 g, 0.463 mmol) were mixed in 1,4-dioxane (1.7 mL) at room temperature, after which the resulting mixture was irradiated with microwave, then heated at 100°C for 30 minutes, and then a reaction was finished by lowering a temperature to room temperature. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 10%) and concentrated to obtain a title compound (0.060 g, 50.4%) in a brown solid form.

**¹H NMR** (400 MHz, CDCl₃) δ 8.06 (dd, *J* = 6.7, 1.8 Hz, 2H), 7.49 (d, *J =* 8.5 Hz, 2H), 7.40 - 7.12 (m, 5H), 7.01 (d, *J = 1.7* Hz, 1H), 7.00 - 6.95 (m, 1H), 6.88 (t, *J* = 51.5 Hz, 1H), 5.19 (s, 2H), 4.51 - 4.43 (m, 1H), 3.05 (d, *J =* 11.6 Hz, 2H), 2.58 - 2.48 (m, 2H), 2.38 (s, 3H), 2.22 (t, *J =* 11.4 Hz, 2H), 1.88 (dd, *J =* 11.9, 2.2 Hz, 2H); **LRMS** (ES) m/z 535.3 (M⁺ + 1).

### Example 128: Synthesis of Compound 128, 3-(4-(5-(difluoromethyl)-1,3,4-oxadiazole-2-yl)benzyl)-5-(3-fluorophenyl)-1-(1-methylpiperidine-4 -yl)-1,3-dihydro-2H-benzo[d]imidazole-2-one

### [Step 1] Synthesis of the compound 128

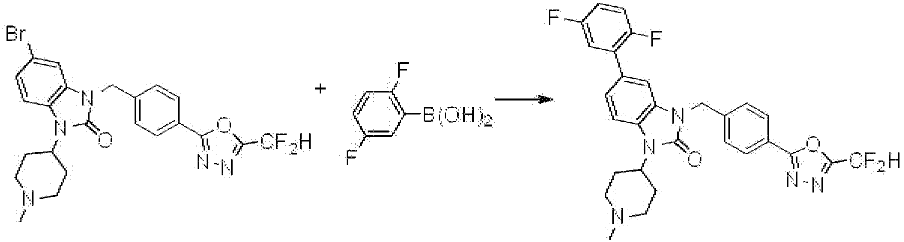

The 5-bromo-3-(4-(5-(difluoromethyl)-1,3,4-oxadiazole-2-yl)benzyl)-1-(1-methylpiperidine-4-yl)-1,3-di hydro-2H-benzo[d]imidazole-2-one (0.120 g, 0.231 mmol) prepared in the step 3 of the compound 126, (3-fluorophenyl)boronic acid (0.039 g, 0.278 mmol), [1,1'-bis(di-tert-butylphosphino)ferrocene]palladium dichloride (0.008 g, 0.012 mmol) and cesium carbonate (0.151 g, 0.463 mmol) were mixed in 1,4-dioxane (1.7 mL) at room temperature, after which the resulting mixture was irradiated with microwave, then heated at 100°C for 30 minutes, and then a reaction was finished by lowering a temperature to room temperature. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.081 g, 65.4%) in a brown solid form.

**¹H NMR** (400 MHz, CDCl₃) δ 8.05 (dd, *J* = 6.7, 1.8 Hz, 2H), 7.48 (d, *J =* 8.5 Hz, 2H), 7.39 (d, *J =* 8.3 Hz, 1H), 7.19 (dt, *J =* 8.3, 1.3 Hz, 1H), 7.07 - 6.75 (m, 5H), 5.17 (s, 2H), 4.50 - 4.42 (m, 1H), 3.03 (d, *J =* 11.6 Hz, 2H), 2.56 - 2.45 (m, 2H), 2.35 (s, 3H), 2.20 - 2.14 (m, 2H), 1.87 (dd, *J* = 11.9, 2.3 Hz, 2H); **LRMS** (ES) m/z 552.1 (M⁺ + 1).

### Example 129: Synthesis of Compound 129, 3-(4-(5-(difluoromethyl)-1,3,4-oxadiazole-2-yl)benzyl)-5-(2,5-difluorophenyl)-1-(1-methylpiperidin e-4-yl)-1,3-dihydro-2H-benzo[d]imidazole-2-one

### [Step 1] Synthesis of the compound 129

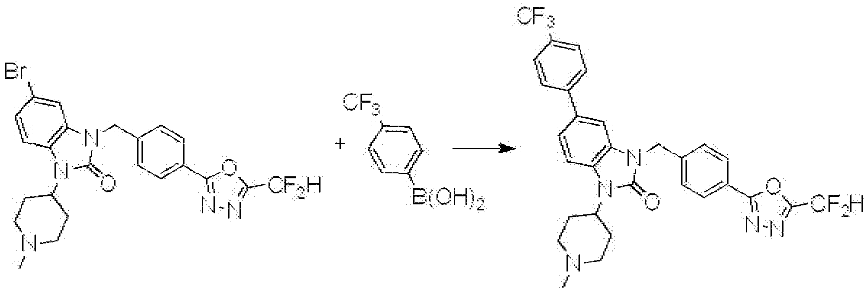

The 5-bromo-3-(4-(5-(difluoromethyl)-1,3,4-oxadiazole-2-yl)benzyl)-1-(1-methylpiperidine-4-yl)-1,3-di hydro-2H-benzo[d]imidazole-2-one (0.120 g, 0.231 mmol) prepared in the step 3 of the compound 126, (2,5-difluorophenyl)boronic acid (0.044 g, 0.278 mmol), [1,1'-bis(di-tert-butylphosphino)ferrocene]palladium dichloride (0.008 g, 0.012 mmol) and cesium carbonate (0.151 g, 0.463 mmol) were mixed in 1,4-dioxane (1.7 mL) at room temperature, after which the resulting mixture was irradiated with microwave, then heated at 100°C for 30 minutes, and then a reaction was finished by lowering a temperature to room temperature. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.096 g, 75.4%) in a brown solid form.

**¹H NMR** (400 MHz, CDCl₃) δ 8.06 (d, *J =* 8.4 Hz, 2H), 7.63 (d, *J =* 8.2 Hz, 2H), 7.54 (d, *J* = 8.1 Hz, 2H), 7.49 (d, *J =* 8.4 Hz, 2H), 7.42 (d, *J* = 8.3 Hz, 1H), 7.27 (dd, *J* = 8.6, 2.0 Hz, 1H), 7.03 (d, *J =* 1.6 Hz, 1H), 6.88 (t, *J =* 51.7 Hz, 1H), 5.20 (s, 2H), 4.52 - 4.44 (m, 1H), 3.07 (d, *J =* 11.7 Hz, 2H), 2.61 - 2.51 (m, 2H), 2.40 (s, 3H), 2.26 (t, *J* = 11.7 Hz, 2H), 1.90 - 1.87 (m, 2H); LRMS (ES) m/z 584.0 (M⁺ + 1).

### Example 130: Synthesis of Compound 130, 3-(4-(5-(difluoromethyl)-1,3,4-oxadiazole-2-yl)benzyl)-1-(1-methylpiperidine-4-yl)-5-(4-(trifluorom ethyl)phenyl)-1,3-dihydro-2H-benzo[d]imidazole-2-one

### [Step 1] Synthesis of the compound 130

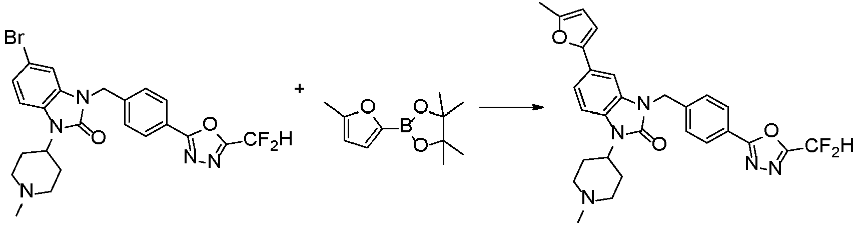

The 5-bromo-3-(4-(5-(difluoromethyl)-1,3,4-oxadiazole-2-yl)benzyl)-1-(1-methylpiperidine-4-yl)-1,3-di hydro-2H-benzo[d]imidazole-2-one (0.120 g, 0.231 mmol) prepared in the step 3 of the compound 126, (4-(trifluoromethyl)phenyl)boronic acid (0.053 g, 0.278 mmol), [1,1'-bis(di-tert-butylphosphino)ferrocene]palladium dichloride (0.008 g, 0.012 mmol) and cesium carbonate (0.151 g, 0.463 mmol) were mixed in 1,4-dioxane (1.7 mL) at room temperature, after which the resulting mixture was irradiated with microwave, then heated at 100°C for 30 minutes, and then a reaction was finished by lowering a temperature to room temperature. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.081 g, 60.0%) in a brown solid form.

**¹H NMR** (400 MHz, CDCl₃) δ 8.05 (d, *J* = 8.4 Hz, 2H), 7.47 (d, *J* = 8.4 Hz, 2H), 7.32 - 7.26 (m, 2H), 7.09 (s, 1H), 6.88 (t, *J =* 51.7 Hz, 1H), 6.37 (d, *J =* 3.2 Hz, 1H), 5.98 (dd, *J* = 3.2, 1.0 Hz, 1H), 5.16 (s, 2H), 4.46 - 4.38 (m, 1H), 3.02 (d, *J =* 11.6 Hz, 2H), 2.53 - 2.43 (m, 2H), 2.34 (s, 3H), 2.31 (s, 3H), 2.16 - 2.13 (m, 2H), 1.85 (dd, *J =* 12.0, 2.2 Hz, 2H); **LRMS** (ES) m/z 519.9 (M⁺ + 1).

### Example 131: Synthesis of Compound 131, 3-(4-(5-(difluoromethyl)-1,3,4-oxadiazole-2-yl)benzyl)-5-(3,5-dimethylisooxazole-4-yl)-1-(1-meth ylpiperidine-4-yl)-1,3-dihydro-2H-benzo[d]imidazole-2-one

### [Step 1] Synthesis of the compound 131

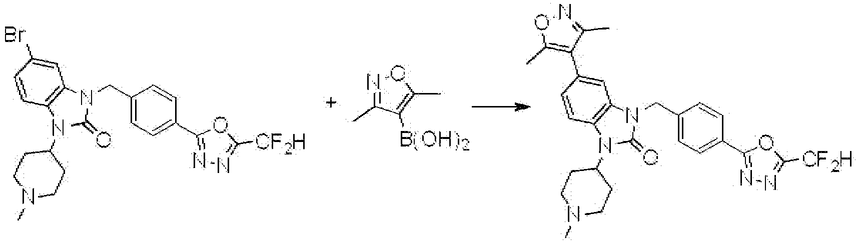

The 5-bromo-3-(4-(5-(difluoromethyl)-1,3,4-oxadiazole-2-yl)benzyl)-1-(1-methylpiperidine-4-yl)-1,3-di hydro-2H-benzo[d]imidazole-2-one (0.120 g, 0.231 mmol) prepared in the step 3 of the compound 126, (3,5-dimethylisooxazole-4-yl)boronic acid (0.039 g, 0.278 mmol), [1,1'-bis(di-tert-butylphosphino)ferrocene]palladium dichloride (0.008 g, 0.012 mmol) and cesium carbonate (0.151 g, 0.463 mmol) were mixed in 1,4-dioxane (1.7 mL) at room temperature, after which the resulting mixture was irradiated with microwave, then heated at 100°C for 30 minutes, and then a reaction was finished by lowering a temperature to room temperature. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.067 g, 54.5%) in a brown solid form.

**¹H NMR** (400 MHz, CDCl₃) δ 8.05 (d, *J =* 8.4 Hz, 2H), 7.47 (d, *J =* 8.4 Hz, 2H), 7.36 (d, *J* = 8.2 Hz, 1H), 7.02 - 6.76 (m, 2H), 6.66 (d, *J =* 8.7 Hz, 1H), 5.15 (s, 2H), 4.49 - 4.41 (m, 1H), 3.03 (d, *J* = 11.5 Hz, 2H), 2.54 - 2.44 (m, 2H), 2.35 (s, 3H), 2.25 (s, 3H), 2.20 - 2.14 (m, 2H), 2.11 (s, 3H), 1.87 (dd, *J =* 11.9, 2.2 Hz, 2H); **LRMS** (ES) m/z 536.3 (M⁺ + 1).

### Example 132: Synthesis of Compound 132, 3-(4-(5-(difluoromethyl)-1,3,4-oxadiazole-2-yl)benzyl)-1-(1-methylpiperidine-4-yl)-5-(pyridine-3-yl )-1,3-dihydro-2H-benzo[d]imidazole-2-one

### [Step 1] Synthesis of the compound 132

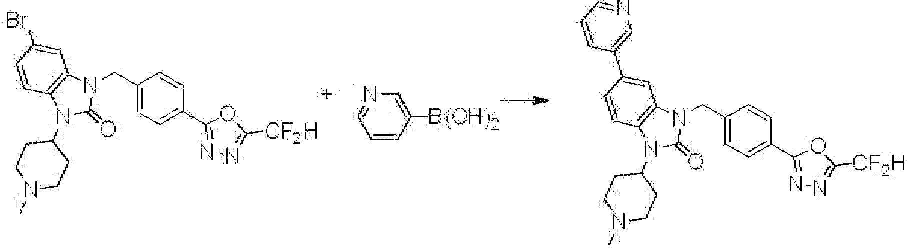

The 5-bromo-3-(4-(5-(difluoromethyl)-1,3,4-oxadiazole-2-yl)benzyl)-1-(1-methylpiperidine-4-yl)-1,3-di hydro-2H-benzo[d]imidazole-2-one (0.120 g, 0.231 mmol) prepared in the step 3 of the compound 126, pyridine-3-ylboronic acid (0.034 g, 0.278 mmol), [1,1'-bis(di-tert-butylphosphino)ferrocene]palladium dichloride (0.008 g, 0.012 mmol) and cesium carbonate (0.151 g, 0.463 mmol) were mixed in 1,4-dioxane (1.7 mL) at room temperature, after which the resulting mixture was irradiated with microwave, then heated at 100°C for 30 minutes, and then a reaction was finished by lowering a temperature to room temperature. Water was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 10%) and concentrated to obtain a title compound (0.060 g, 50.4%) in a brown solid form.

**¹H NMR** (400 MHz, CDCl₃) δ 8.70 (d, *J =* 1.8 Hz, 1H), 8.52 (dd, *J* = 4.8, 1.6 Hz, 1H), 8.08 - 8.05 (m, 2H), 7.74 - 7.71 (m, 1H), 7.48 (d, *J =* 8.6 Hz, 2H), 7.42 (d, *J =* 8.2 Hz, 1H), 7.31 - 7.26 (m, 1H), 7.26 - 7.23 (m, 1H), 7.00 (d, *J* = 1.6 Hz, 1H), 6.88 (t, *J =* 51.7 Hz, 1H), 5.20 (s, 2H), 4.51 - 4.43 (m, 1H), 3.04 (d, *J* = 11.6 Hz, 2H), 2.56 - 2.46 (m, 2H), 2.36 (s, 3H), 2.21 - 2.16 (m, 2H), 1.88 (dd, *J* = 11.9, 2.3 Hz, 2H); **LRMS** (ES) m/z 517.3(M⁺ + 1).

### Example 133: Synthesis of Compound 133, 1-((5-(5-(difluoromethyl)-1,3,4-oxadiazole-2-yl)pyridine-2-yl)methyl)-5-(3-fluorophenyl)-3-(1-meth ylpiperidine-4-yl)-1,3-dihydro-2H-benzo[d]imidazole-2-one

### [Step 1] Synthesis of tert-butyl 4-((5-bromo-2-nitrophenyl)amino)piperidine-1-carboxylate

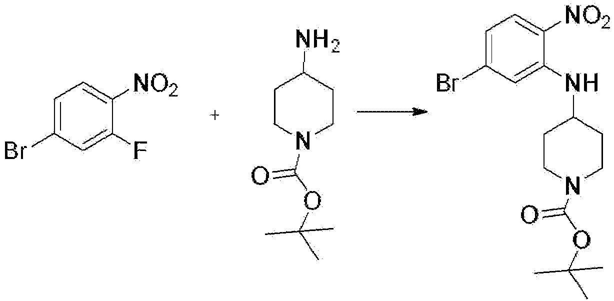

4-bromo-2-fluoro-1-nitrobenzene (5.000 g, 22.727 mmol), tert-butyl 4-aminopiperidine-1-carboxylate (4.552 g, 22.727 mmol) and potassium carbonate (6.282 g, 45.455 mmol) were dissolved in N,N-dimethylformamide (150 mL) at room temperature, after which the resulting solution was stirred at 80°C for 4 hours, and then a reaction was finished by lowering a temperature to room temperature. Solvent was removed from the reaction mixture under reduced pressure, after which saturated sodium hydrogen carbonate aqueous solution was poured into the resulting concentrate, and then an extraction was performed with ethyl acetate. An organic layer was washed with saturated sodium chloride aqueous solution, then dehydrated with anhydrous magnesium sulfate, then filtered, and then concentrated under reduced pressure. The resulting concentrate was purified via column chromatography (SiO₂, 80 g cartridge; ethyl acetate/hexane = 0 to 20%) and concentrated to obtain a title compound (8.800 g, 96.7%) in a yellow solid form.

### [Step 2] Synthesis of tert-butyl 4-((2-amino-5-bromophenyl)amino)piperidine-1-carboxylate

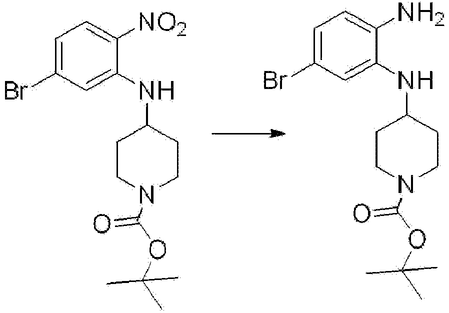

The tert-butyl 4-((5-bromo-2-nitrophenyl)amino)piperidine-1-carboxylate (8.800 g, 21.985 mmol) prepared in the step 1 was dissolved in ethanol (200 mL) and stirred at room temperature, after which Raney nickel (800 mg) was slowly added into the resulting solution at the same temperature and stirred at the same temperature for 18 hours in the presence of a hydrogen balloon attached thereto. The reaction mixture was filtered via a celite pad to remove a solid therefrom, after which solvent was removed from the resulting filtrate under reduced pressure, and then a title compound was used without an additional purification process (7.440 g, 91.4%, brown solid).

### [Step 3] Synthesis of tert-butyl 4-(6-bromo-2-oxo-2,3-dihydro-1H-benzo[d]imidazole-1-yl)piperidine-1-carboxylate

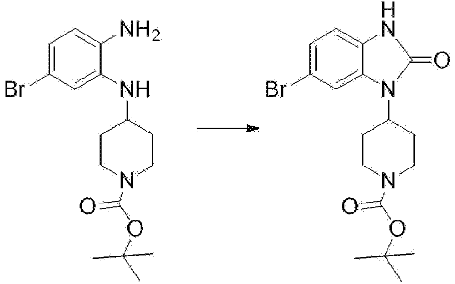

The tert-butyl 4-((2-amino-5-bromophenyl)amino)piperidine-1-carboxylate (7.440 g, 20.092 mmol) prepared in the step 2 and 1,1'-carbonyldiimidazole (CDI, 3.584 g, 22.102 mmol) were mixed in dichloromethane (200 mL) at room temperature, after which the resulting solution was stirred at the same temperature for 18 hours. Water was poured into the reaction mixture and an extraction was performed with dichloromethane. An organic layer was washed with saturated sodium chloride aqueous solution, then dehydrated with anhydrous magnesium sulfate, then filtered, and then concentrated under reduced pressure. The resulting concentrate was purified via column chromatography (SiO₂, 80 g cartridge; ethyl acetate/hexane = 0 to 40%) and concentrated to obtain a title compound (5.460 g, 68.6%) in a light brown solid form.

### [Step 4] Synthesis of tert-butyl 4-(6-bromo-3-((5-(5-(difluoromethyl)-1,3,4-oxadiazole-2-yl)pyridine-2-yl)methyl)-2-oxo-2,3-dihydr o-1H-benzo[d]imidazole-1-yl)piperidine-1-carboxylate

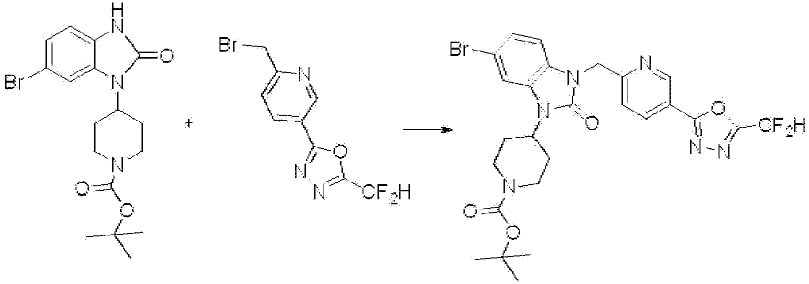

The tert-butyl 4-(6-bromo-2-oxo-2,3-dihydro-1H-benzo[d]imidazole-1-yl)piperidine-1-carboxylate (3.000 g, 7.570 mmol) prepared in the step 3 and sodium hydride (60.00%, 0.333 g, 8.327 mmol) were dissolved in N,N-dimethylformamide (100 mL) at 0°C, after which 2-(6-(bromomethyl)pyridine-3-yl)-5-(difluoromethyl)-1,3,4-oxadiazole (2.415 g, 8.327 mmol) was added into the resulting solution and stirred at the same temperature for 2 hours. Solvent was removed from the reaction mixture under reduced pressure, after which water was poured into the resulting concentrate, and then an extraction was performed with ethyl acetate. An organic layer was washed with saturated sodium chloride aqueous solution, then dehydrated with anhydrous magnesium sulfate, then filtered, and then concentrated under reduced pressure. The resulting concentrate was purified via column chromatography (SiO₂, 40 g cartridge; ethyl acetate/hexane = 0 to 30%) and concentrated to obtain a title compound (3.450 g, 75.3%) in a white solid form.

### [Step 5] Synthesis of 5-bromo-1-((5-(5-(difluoromethyl)-1,3,4-oxadiazole-2-yl)pyridine-2-yl)methyl)-3-(piperidine-4-yl)-1,3-dihydro-2H-benzo[d]imidazole-2-one

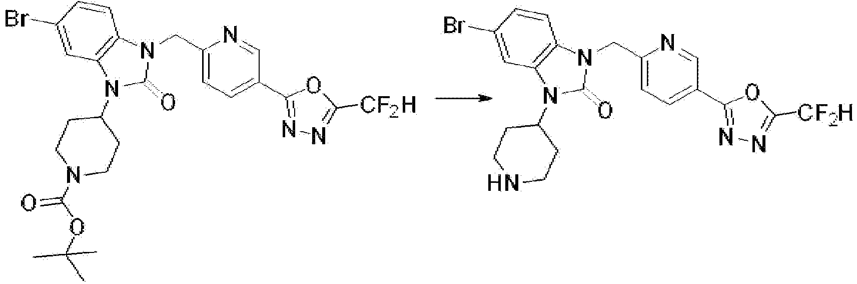

The tert-butyl 4-(6-bromo-3-((5-(5-(difluoromethyl)-1,3,4-oxadiazole-2-yl)pyridine-2-yl)methyl)-2-oxo-2,3-dihydr o-1H-benzo[d]imidazole-1-yl)piperidine-1-carboxylate (3.450 g, 5.698 mmol) prepared in the step 4 and trifluoroacetic acid (2.618 mL, 34.190 mmol) were dissolved in dichloromethane (40 mL) at room temperature, after which the resulting solution was stirred at the same temperature for 7 hours. Saturated sodium hydrogen carbonate aqueous solution was poured into the reaction mixture and an extraction was performed with dichloromethane. An organic layer was washed with saturated sodium chloride aqueous solution, then dehydrated with anhydrous magnesium sulfate, then filtered, and then concentrated under reduced pressure. A title compound was used without an additional purification process (2.300 g, 79.9%, light yellow solid).

### [Step 6] Synthesis of 5-bromo-1-((5-(5-(difluoromethyl)-1,3,4-oxadiazole-2-yl)pyridine-2-yl)methyl)-3-(1-methylpiperidi ne-4-yl)-1,3-dihydro-2H-benzo[d]imidazole-2-one

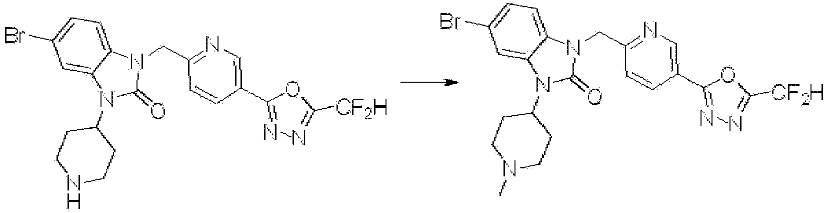

The 5-bromo-1-((5-(5-(difluoromethyl)-1,3,4-oxadiazole-2-yl)pyridine-2-yl)methyl)-3-(piperidine-4-yl)-1,3-dihydro-2H-benzo[d]imidazole-2-one (2.300 g, 4.552 mmol) prepared in the step 5 and sodium triacetoxyborohydride (1.929 g, 9.103 mmol) were dissolved in dichloromethane (100 mL) at room temperature, after which formaldehyde (37.00% solution, 0.513 mL, 6.827 mmol) was added into the resulting solution and stirred at the same temperature for 18 hours. Saturated sodium hydrogen carbonate aqueous solution was poured into the reaction mixture and an extraction was performed with dichloromethane. An organic layer was washed with saturated sodium chloride aqueous solution, then dehydrated with anhydrous magnesium sulfate, then filtered, and then concentrated under reduced pressure. The resulting concentrate was purified via column chromatography (SiO₂, 40 g cartridge; methanol/dichloromethane = 0 to 5%) and concentrated to obtain a title compound (2.000 g, 84.6%) in a light yellow solid form.

### [Step 7] Synthesis of the compound 133

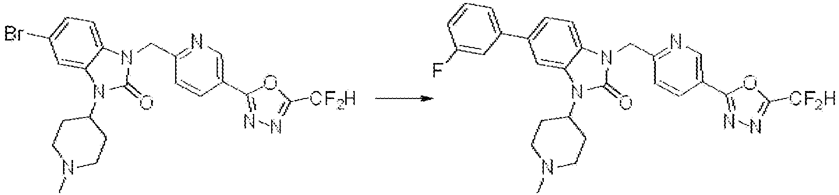

The 5-bromo-1-((5-(5-(difluoromethyl)-1,3,4-oxadiazole-2-yl)pyridine-2-yl)methyl)-3-(1-methylpiperidi ne-4-yl)-1,3-dihydro-2H-benzo[d]imidazole-2-one (0.150 g, 0.289 mmol) prepared in the step 6, (3-fluorophenyl)boronic acid (0.048 g, 0.347 mmol), [1,1'-bis(di-tert-butylphosphino)ferrocene]palladium(II) dichloride (Pd(dtbpf)Cl₂, 0.009 g, 0.014 mmol) and cesium carbonate (0.282 g, 0.866 mmol) were mixed in 1,4-dioxane (3 mL)/water (1 mL) at room temperature, after which the resulting mixture was irradiated with microwave, then heated at 100°C for 20 minutes, and then a reaction was finished by lowering a temperature to room temperature. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 10%) and concentrated to obtain a title compound (0.018 g, 11.7%) in a brown solid form.

**¹H NMR** (400 MHz, CDCl₃) δ 9.32 (d, *J* = 2.1 Hz, 1H), 8.35 (dd, *J* = 8.2, 2.2 Hz, 1H), 7.51 (s, 1H), 7.45 (d, *J =* 8.3 Hz, 1H), 7.43 - 7.27 (m, 1H), 7.34 (d, *J =* 7.6 Hz, 1H), 7.25 - 7.23 (m, 2H), 7.08 - 6.82 (m, 3H), 5.34 (s, 2H), 4.54 - 4.48 (m, 1H), 3.10 (d, *J* = 11.1 Hz, 2H), 2.61 - 2.58 (m, 2H), 2.40 (s, 3H), 2.25 (t, *J =* 10.6 Hz, 2H), 1.92 (d, *J =* 10.3 Hz, 2H); **LRMS** (ES) m/z 535.3 (M⁺ + H).

### Example 134: Synthesis of Compound 134, 1-((5-(5-(difluoromethyl)-1,3,4-oxadiazole-2-yl)pyridine-2-yl)methyl)-5-(2-fluorophenyl)-3-(1-meth ylpiperidine-4-yl)-1,3-dihydro-2H-benzo[d]imidazole-2-one

### [Step 1] Synthesis of the compound 134

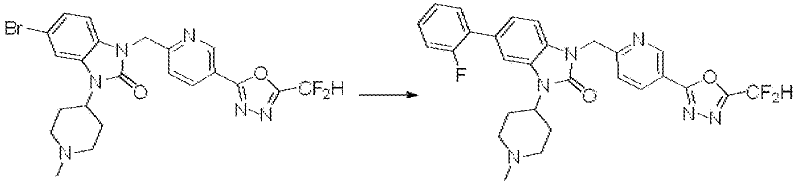

The 5-bromo-1-((5-(5-(difluoromethyl)-1,3,4-oxadiazole-2-yl)pyridine-2-yl)methyl)-3-(1-methylpiperidi ne-4-yl)-1,3-dihydro-2H-benzo[d]imidazole-2-one (0.150 g, 0.289 mmol) prepared in the step 6 of the compound 133, (2-fluorophenyl)boronic acid (0.048 g, 0.347 mmol), [1,1'-bis(di-tert-butylphosphino)ferrocene]palladium(II) dichloride (Pd(dtbpf)Cl₂, 0.009 g, 0.014 mmol) and cesium carbonate (0.282 g, 0.866 mmol) were mixed in 1,4-dioxane (3 mL)/water (1 mL) at room temperature, after which the resulting mixture was irradiated with microwave, then heated at 100°C for 20 minutes, and then a reaction was finished by lowering a temperature to room temperature. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 12 g cartridge; methanol/dichloromethane = 0 to 10%) and concentrated to obtain a title compound (0.026 g, 16.8%) in a brown solid form.

**¹H NMR** (400 MHz, CDCl₃) δ 9.32 (d, *J =* 2.1 Hz, 1H), 8.35 (dd, *J* = 8.2, 2.2 Hz, 1H), 7.50 (s, 1H), 7.45 (d, *J =* 8.2 Hz, 1H), 7.40 (td, *J =* 7.7, 1.7 Hz, 1H), 7.33 - 7.29 (m, 1H), 7.23 - 7.20 (m, 2H), 7.17 - 7.13 (m, 1H), 7.08 - 6.82 (m, 2H), 5.34 (s, 2H), 4.53 - 4.46 (m, 1H), 3.07 (d, *J =* 11.7 Hz, 2H), 2.57 - 2.50 (m, 2H), 2.37 (s, 3H), 2.24 - 2.18 (m, 2H), 1.91 (d, *J =* 10.1 Hz, 2H); **LRMS** (ES) m/z 535.4 (M⁺ + H).

### Example 135: Synthesis of Compound 135, 1-((5-(5-(difluoromethyl)-1,3,4-oxadiazole-2-yl)pyridine-2-yl)methyl)-3-(1-methylpiperidine-4-yl)-5 -(pyridine-4-yl)-1,3-dihydro-2H-benzo[d]imidazole-2-one

### [Step 1] Synthesis of the compound 135

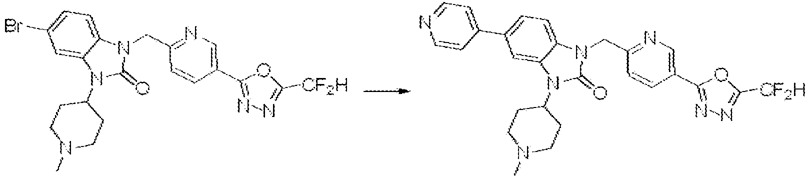

The 5-bromo-1-((5-(5-(difluoromethyl)-1,3,4-oxadiazole-2-yl)pyridine-2-yl)methyl)-3-(1-methylpiperidi ne-4-yl)-1,3-dihydro-2H-benzo[d]imidazole-2-one (0.150 g, 0.289 mmol) prepared in the step 6 of the compound 133, pyridine-4-ylboronic acid (0.043 g, 0.347 mmol), [1,1'-bis(di-tert-butylphosphino)ferrocene]palladium(II) dichloride (Pd(dtbpf)Cl₂, 0.009 g, 0.014 mmol) and cesium carbonate (0.282 g, 0.866 mmol) were mixed in 1,4-dioxane (3 mL)/water (1 mL) at room temperature, after which the resulting mixture was irradiated with microwave, then heated at 100°C for 20 minutes, and then a reaction was finished by lowering a temperature to room temperature. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 12 g cartridge; methanol/dichloromethane = 0 to 10%) and concentrated to obtain a title compound (0.011 g, 7.4%) in a brown solid form.

**¹H NMR** (400 MHz, CDCl₃) δ 9.32 (d, *J =* 2.2 Hz, 1H), 8.66 (d, *J =* 6.0 Hz, 2H), 8.36 (dd, *J* = 8.2, 2.2 Hz, 1H), 7.60 (brs, 1H), 7.51 - 7.46 (m, 3H), 7.34 (dd, *J* = 8.2, 1.4 Hz, 1H), 7.13 - 6.82 (m, 2H), 5.34 (s, 2H), 4.54 - 4.53 (m, 1H), 3.13 (d, *J =* 9.4 Hz, 2H), 2.62 (brs, 2H), 2.43 (s, 3H), 2.30 - 2.29 (m, 2H), 1.94 (d, *J =* 10.6 Hz, 2H); **LRMS** (ES) m/z 518.3 (M⁺ + H).

### Example 136: Synthesis of Compound 136, 1-((5-(5-(difluoromethyl)-1,3,4-oxadiazole-2-yl)pyridine-2-yl)methyl)-3-(1-methylpiperidine-4-yl)-5 -(pyridine-3-yl)-1,3-dihydro-2H-benzo[d]imidazole-2-one

### [Step 1] Synthesis of the compound 136

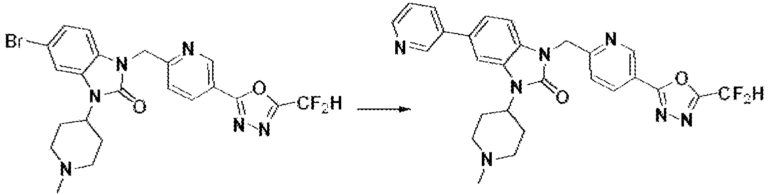

The 5-bromo-1-((5-(5-(difluoromethyl)-1,3,4-oxadiazole-2-yl)pyridine-2-yl)methyl)-3-(1-methylpiperidi ne-4-yl)-1,3-dihydro-2H-benzo[d]imidazole-2-one (0.150 g, 0.289 mmol) prepared in the step 6 of the compound 133, pyridine-3-ylboronic acid (0.043 g, 0.347 mmol), [1,1'-bis(di-tert-butylphosphino)ferrocene]palladium(II) dichloride (Pd(dtbpf)Cl₂, 0.009 g, 0.014 mmol) and cesium carbonate (0.282 g, 0.866 mmol) were mixed in 1,4-dioxane (3 mL)/water (1 mL) at room temperature, after which the resulting mixture was irradiated with microwave, then heated at 100°C for 20 minutes, and then a reaction was finished by lowering a temperature to room temperature. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 12 g cartridge; methanol/dichloromethane = 0 to 10%) and concentrated to obtain a title compound (0.010 g, 6.7%) in a brown solid form.

**¹H NMR** (400 MHz, CDCl₃) δ 9.32 (d, *J =* 1.5 Hz, 1H), 8.82 (d, *J =* 1.9 Hz, 1H), 8.60 (dd, *J* = 4.8, 1.4 Hz, 1H), 8.36 (dd, *J* = 8.2, 2.2 Hz, 1H), 7.89 (brs, 1H), 7.54 (brs, 1H), 7.46 (d, *J =* 8.2 Hz, 1H), 7.39 - 7.36 (m, 1H), 7.26 (d, *J* = 8.1 Hz, 1H), 7.11 - 6.82 (m, 2H), 5.35 (s, 2H), 4.54 - 4.53 (m, 1H), 3.12 - 3.11 (m, 2H), 2.62 (brs, 2H), 2.42 (s, 3H), 2.29 - 2.28 (m, 2H), 1.94 (d, *J* = 10.5 Hz, 2H); LRMS (ES) m/z 518.4 (M⁺ + H).

### Example 137: Synthesis of Compound 137, 1-((5-(5-(difluoromethyl)-1,3,4-oxadiazole-2-yl)pyridine-2-yl)methyl)-5-(furan-3-yl)-3-(1-methylpip eridine-4-yl)-1,3-dihydro-2H-benzo[d]imidazole-2-one

### [Step 1] Synthesis of the compound 137

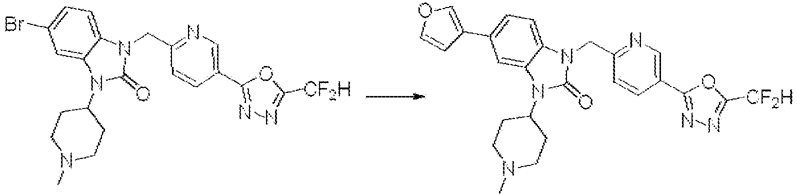

The 5-bromo-1-((5-(5-(difluoromethyl)-1,3,4-oxadiazole-2-yl)pyridine-2-yl)methyl)-3-(1-methylpiperidi ne-4-yl)-1,3-dihydro-2H-benzo[d]imidazole-2-one (0.150 g, 0.289 mmol) prepared in the step 6 of the compound 133, furan-3-ylboronic acid (0.039 g, 0.347 mmol), [1,1'-bis(di-tert-butylphosphino)ferrocene]palladium(II) dichloride (Pd(dtbpf)Cl₂, 0.009 g, 0.014 mmol) and cesium carbonate (0.282 g, 0.866 mmol) were mixed in 1,4-dioxane (3 mL)/water (1 mL) at room temperature, after which the resulting mixture was irradiated with microwave, then heated at 100°C for 20 minutes, and then a reaction was finished by lowering a temperature to room temperature. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 12 g cartridge; methanol/dichloromethane = 0 to 10%) and concentrated to obtain a title compound (0.016 g, 10.9%) in a brown solid form.

**¹H NMR** (400 MHz, CDCl₃) δ 9.31 (d, *J =* 2.1 Hz, 1H), 8.34 (dd, *J* = 8.2, 2.2 Hz, 1H), 7.72 (s, 1H), 7.49 (t, *J =* 1.7 Hz, 1H), 7.43 - 7.41 (m, 2H), 7.17 (dd, *J* = 8.1, 1.4 Hz, 1H), 7.08 - 6.82 (m, 2H), 6.71 (s, 1H), 5.32 (s, 2H), 4.49 - 4.46 (m, 1H), 3.10 (d, *J* = 10.6 Hz, 2H), 2.60 - 2.58 (m, 2H), 2.41 (s, 3H), 2.25 (t, *J =* 10.6 Hz, 2H), 1.91 (d, *J =* 10.2 Hz, 2H); LRMS (ES) m/z 507.3 (M⁺ + H).

### Example 138: Synthesis of Compound 138, 1-((5-(5-(difluoromethyl)-1,3,4-oxadiazole-2-yl)pyridine-2-yl)methyl)-5-(furan-2-yl)-3-(1-methylpip eridine-4-yl)-1,3-dihydro-2H-benzo[d]imidazole-2-one

### [Step 1] Synthesis of the compound 138

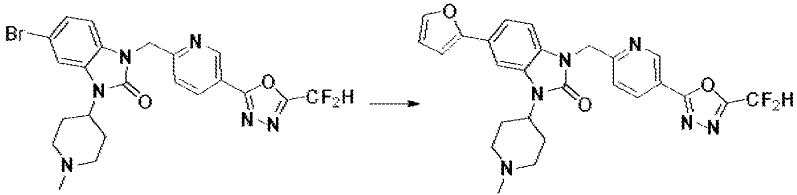

The 5-bromo-1-((5-(5-(difluoromethyl)-1,3,4-oxadiazole-2-yl)pyridine-2-yl)methyl)-3-(1-methylpiperidi ne-4-yl)-1,3-dihydro-2H-benzo[d]imidazole-2-one (0.150 g, 0.289 mmol) prepared in the step 6 of the compound 133, furan-2-ylboronic acid (0.039 g, 0.347 mmol), [1,1'-bis(di-tert-butylphosphino)ferrocene]palladium(II) dichloride (Pd(dtbpf)Cl₂, 0.009 g, 0.014 mmol) and cesium carbonate (0.282 g, 0.866 mmol) were mixed in 1,4-dioxane (3 mL)/water (1 mL) at room temperature, after which the resulting mixture was irradiated with microwave, then heated at 100°C for 20 minutes, and then a reaction was finished by lowering a temperature to room temperature. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 12 g cartridge; methanol/dichloromethane = 0 to 10%) and concentrated to obtain a title compound (0.008 g, 5.5%) in a brown solid form.

**¹H NMR** (400 MHz, CDCl₃) δ 79.32 (d, *J* = 1.5 Hz, 1H), 8.34 (dd, *J* = 8.2, 2.2 Hz, 1H), 7.66 (brs, 1H), 7.46 (d, *J =* 1.2 Hz, 1H), 7.42 (d, *J =* 8.2 Hz, 1H), 7.37 (d, *J =* 8.2 Hz, 1H), 7.08 - 6.82 (m, 2H), 6.64 (brs, 1H), 6.49 - 6.48 (m, 1H), 5.32 (s, 2H), 4.50 - 4.49 (m, 1H), 3.12 - 3.11 (m, 2H), 2.61 (brs, 2H), 2.43 (s, 3H), 2.26 - 2.25 (m, 2H), 1.92 (d, *J =* 11.2 Hz, 2H); LRMS (ES) m/z 507.2 (M⁺ + H).

### Example 139: Synthesis of Compound 139, 1-(4-(5-(difluoromethyl)-1,3,4-oxadiazole-2-yl)-2-fluorobenzyl)-3-(1-methylpiperidine-4-yl)-5-(pyri dine-3-yl)-1,3-dihydro-2H-benzo[d]imidazole-2-one

### [Step 1] Synthesis of tert-butyl 4-(6-bromo-3-(4-(5-(difluoromethyl)-1,3,4-oxadiazole-2-yl)-2-fluorobenzyl)-2-oxo-2,3-dihydro-1H-benzo[d]imidazole-1-yl)piperidine-1-carboxylate

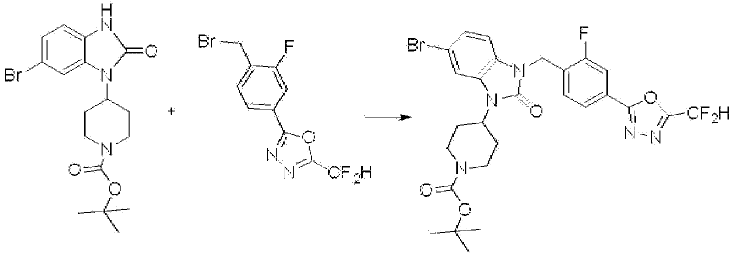

The tert-butyl 4-(6-bromo-2-oxo-2,3-dihydro-1H-benzo[d]imidazole-1-yl)piperidine-1-carboxylate (2.460 g, 6.208 mmol) prepared in the step 3 of the compound 133 and sodium hydride (60.00%, 0.273 g, 6.828 mmol) were dissolved in N,N-dimethylformamide (100 mL) at 0°C, after which 2-(4-(bromomethyl)-3-fluorophenyl)-5-(difluoromethyl)-1,3,4-oxadiazole (2.097 g, 6.828 mmol) was added into the resulting solution and stirred at the same temperature for 2 hours. Solvent was removed from the reaction mixture under reduced pressure, after which water was poured into the resulting concentrate, and then an extraction was performed with ethyl acetate. An organic layer was washed with saturated sodium chloride aqueous solution, then dehydrated with anhydrous magnesium sulfate, then filtered, and then concentrated under reduced pressure. The resulting concentrate was purified via column chromatography (SiO₂, 40 g cartridge; ethyl acetate/hexane = 0 to 30%) and concentrated to obtain a title compound (3.900 g, 100.9%) in a white solid form.

### [Step 2] Synthesis of 5-bromo-1-(4-(5-(difluoromethyl)-1,3,4-oxadiazole-2-yl)-2-fluorobenzyl)-3-(piperidine-4-yl)-1,3-di hydro-2H-benzo[d]imidazole-2-one

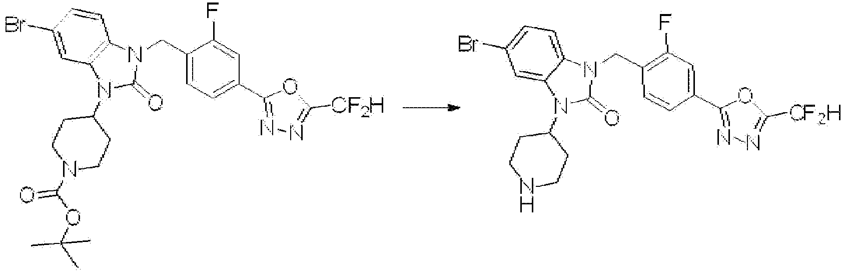

The tert-butyl 4-(6-bromo-3-(4-(5-(difluoromethyl)-1,3,4-oxadiazole-2-yl)-2-fluorobenzyl)-2-oxo-2,3-dihydro-1H-benzo[d]imidazole-1-yl)piperidine-1-carboxylate (3.900 g, 6.266 mmol) prepared in the step 1 and trifluoroacetic acid (2.399 mL, 31.328 mmol) were dissolved in dichloromethane (50 mL) at room temperature, after which the resulting solution was stirred at the same temperature for 6 hours. Saturated sodium hydrogen carbonate aqueous solution was poured into the reaction mixture and an extraction was performed with dichloromethane. An organic layer was washed with saturated sodium chloride aqueous solution, then dehydrated with anhydrous magnesium sulfate, then filtered, and then concentrated under reduced pressure. A title compound was used without an additional purification process (3.080 g, 94.1%, light yellow solid).

### [Step 3] Synthesis of 5-bromo-1-(4-(5-(difluoromethyl)-1,3,4-oxadiazole-2-yl)-2-fluorobenzyl)-3-(1-methylpiperidine-4-yl)-1,3-dihydro-2H-benzo[d]imidazole-2-one

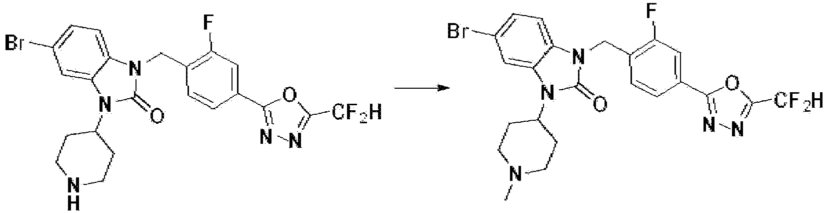

The 5-bromo-1-(4-(5-(difluoromethyl)-1,3,4-oxadiazole-2-yl)-2-fluorobenzyl)-3-(piperidine-4-yl)-1,3-di hydro-2H-benzo[d]imidazole-2-one (3.080 g, 5.897 mmol) prepared in the step 2 and sodium triacetoxyborohydride (2.499 g, 11.793 mmol) were dissolved in dichloromethane (100 mL) at room temperature, after which formaldehyde (37.00% solution, 0.665 mL, 8.845 mmol) was added into the resulting solution and stirred at the same temperature for 18 hours. Saturated sodium hydrogen carbonate aqueous solution was poured into the reaction mixture and an extraction was performed with dichloromethane. An organic layer was washed with saturated sodium chloride aqueous solution, then dehydrated with anhydrous magnesium sulfate, then filtered, and then concentrated under reduced pressure. The resulting concentrate was purified via column chromatography (SiO₂, 40 g cartridge; methanol/dichloromethane = 0 to 5%) and concentrated to obtain a title compound (2.840 g, 89.8%) in a light yellow solid form.

### [Step 4] Synthesis of the compound 139

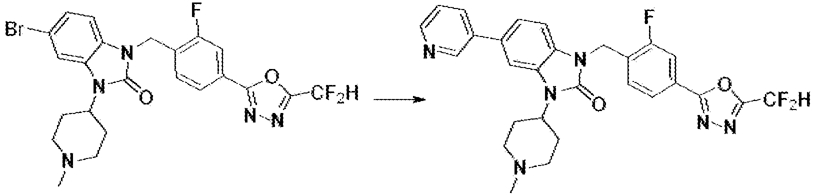

The 5-bromo-1-(4-(5-(difluoromethyl)-1,3,4-oxadiazole-2-yl)-2-fluorobenzyl)-3-(1-methylpiperidine-4-yl)-1,3-dihydro-2H-benzo[d]imidazole-2-one (0.150 g, 0.280 mmol) prepared in the step 3, pyridine-3-ylboronic acid (0.047 g, 0.336 mmol), [1,1'-bis(di-tert-butylphosphino)ferrocene]palladium(II) dichloride (Pd(dtbpf)Cl₂, 0.009 g, 0.014 mmol) and cesium carbonate (0.273 g, 0.839 mmol) were mixed in 1,4-dioxane (3 mL)/water (1 mL) at room temperature, after which the resulting mixture was irradiated with microwave, then heated at 100°C for 20 minutes, and then a reaction was finished by lowering a temperature to room temperature. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 10%) and concentrated to obtain a title compound (0.091 g, 60.9%) in a brown solid form.

**¹H NMR** (400 MHz, CDCl₃) δ 8.81 (d, *J =* 1.8 Hz, 1H), 8.60 (dd, *J* = 4.7, 1.3 Hz, 1H), 7.90 - 7.86 (m, 3H), 7.52 - 7.48 (m, 2H), 7.39 - 7.36 (m, 1H), 7.28 - 7.26 (m, 1H), 7.08 - 6.80 (m, 2H), 5.26 (s, 2H), 4.54 - 4.48 (m, 1H), 3.09 (d, *J =* 9.8 Hz, 2H), 2.59 - 2.57 (m, 2H), 2.40 (s, 3H), 2.26 - 2.24 (m, 2H), 1.91 (d, *J =* 12.0 Hz, 2H); **LRMS** (ES) m/z 535.3 (M⁺ + H).

### Example 140: Synthesis of Compound 140, 1-(4-(5-(difluoromethyl)-1,3,4-oxadiazole-2-yl)-2-fluorobenzyl)-5-(furan-2-yl)-3-(1-methylpiperidin e-4-yl)-1,3-dihydro-2H-benzo[d]imidazole-2-one

### [Step 1] Synthesis of the compound 140

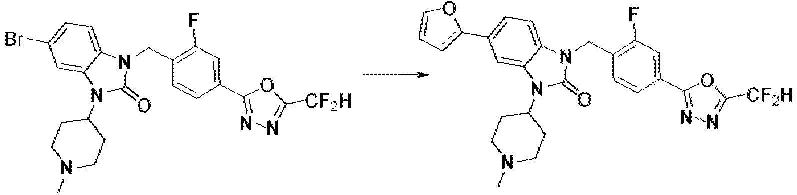

The 5-bromo-1-(4-(5-(difluoromethyl)-1,3,4-oxadiazole-2-yl)-2-fluorobenzyl)-3-(1-methylpiperidine-4-yl)-1,3-dihydro-2H-benzo[d]imidazole-2-one (0.150 g, 0.280 mmol) prepared in the step 3 of the compound 139, furan-2-ylboronic acid (0.038 g, 0.336 mmol), [1,1'-bis(di-tert-butylphosphino)ferrocene]palladium(II) dichloride (Pd(dtbpf)Cl₂, 0.009 g, 0.014 mmol) and cesium carbonate (0.273 g, 0.839 mmol) were mixed in 1,4-dioxane (3 mL)/water (1 mL) at room temperature, after which the resulting mixture was irradiated with microwave, then heated at 100°C for 20 minutes, and then a reaction was finished by lowering a temperature to room temperature. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 10%) and concentrated to obtain a title compound (0.106 g, 72.4%) in a brown solid form.

**¹H NMR** (400 MHz, CDCl₃) δ 7.88 - 7.84 (m, 2H), 7.71 (s, 1H), 7.49 - 7.45 (m, 2H), 7.40 (s, 1H), 7.18 (dd, *J* = 8.1, 1.5 Hz, 1H), 7.05 - 6.79 (m, 2H), 6.69 (s, 1H), 5.22 (s, 2H), 4.51 - 4.43 (m, 1H), 3.08 (d, *J* = 11.4 Hz, 2H), 2.57 - 2.51 (m, 2H), 2.39 (s, 3H), 2.22 (t, *J* = 11.5 Hz, 2H), 1.89 (d, *J =* 10.0 Hz, 2H); **LRMS** (ES) m/z 524.2 (M⁺ + H).

### Example 141: Synthesis of Compound 141, 5-bromo-3-((5-(5-(difluoromethyl)-1,3,4-oxadiazole-2-yl)pyridine-2-yl)methyl)-1-(1-methylpiperidi ne-4-yl)-1,3-dihydro-2H-benzo[d]imidazole-2-one

### [Step 1] Synthesis of methyl 6-((6-bromo-3-(1-(tert-butoxycarbonyl)piperidine-4-yl)-2-oxo-2,3-dihydro-1H-benzo[d]imidazole-1-yl)methyl)nicotinate

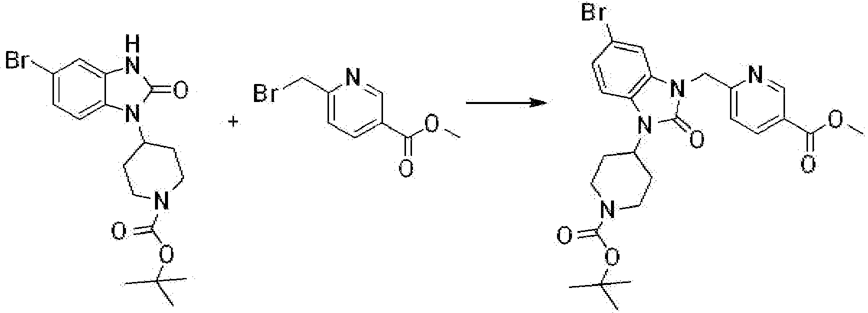

The tert-butyl 4-(5-bromo-2-oxo-2,3-dihydro-1H-benzo[d]imidazole-1-yl)piperidine-1-carboxylate (2.300 g, 5.804 mmol) prepared in the step 3 of the compound 119 was dissolved in N,N-dimethylformamide (60 mL) at 0°C, after which sodium hydride (60.00%, 0.348 g, 8.706 mmol) was added into the resulting solution and stirred at the same temperature for 30 minutes. Methyl 6-(bromomethyl)nicotinate (1.736 g, 7.545 mmol) was added into the reaction mixture and further stirred at room temperature for 5 hours. Water was poured into the reaction mixture and an extraction was performed with ethyl acetate. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 40 g cartridge; ethyl acetate/hexane = 0 to 80%) and concentrated to obtain a title compound (2.568 g, 81.1%) in a brown solid form.

### [Step 2] Synthesis of tert-butyl 4-(5-bromo-3-((5-(hydrazinecarbonyl)pyridine-2-yl)methyl)-2-oxo-2,3-dihydro-1H-benzo[d]imidaz ole-1-yl)piperidine-1-carboxylate

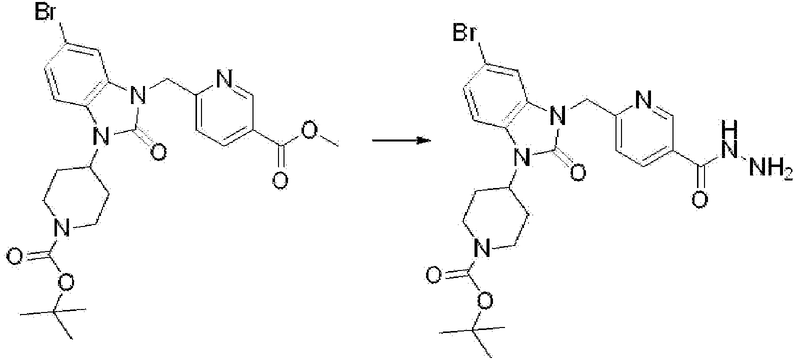

The methyl 6-((6-bromo-3-(1-(tert-butoxycarbonyl)piperidine-4-yl)-2-oxo-2,3-dihydro-1H-benzo[d]imidazole-1-yl)methyl)nicotinate (2.568 g, 4.708 mmol) prepared in the step 1 and hydrazine monohydrate (6.364 g, 127.122 mmol) were mixed in ethanol (25 mL) at room temperature, after which the resulting suspension was stirred at 80°C for 18 hours, and then a reaction was finished by lowering a temperature to room temperature. A precipitated solid was filtered, then washed with ethanol, and then dried to obtain a title compound (1.680 g, 65.4%) in a white solid form.

### [Step 3] Synthesis of tert-butyl 4-(5-bromo-3-((5-(5-(difluoromethyl)-1,3,4-oxadiazole-2-yl)pyridine-2-yl)methyl)-2-oxo-2,3-dihydr o-1H-benzo[d]imidazole-1-yl)piperidine-1-carboxylate

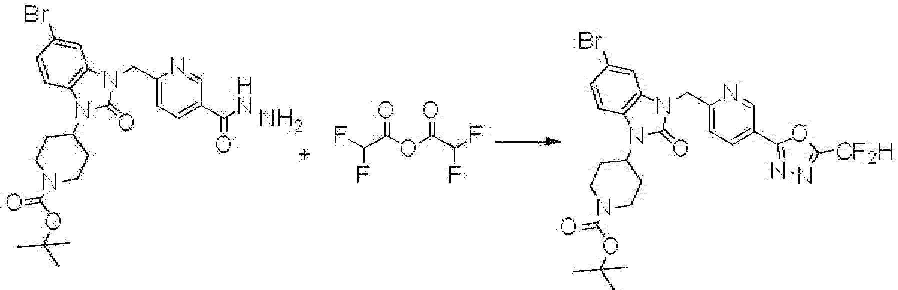

The tert-butyl 4-(5-bromo-3-((5-(hydrazinecarbonyl)pyridine-2-yl)methyl)-2-oxo-2,3-dihydro-1H-benzo[d]imidaz ole-1-yl)piperidine-1-carboxylate (1.680 g, 3.080 mmol) prepared in the step 2, 2,2-difluoroacetic anhydride (2.489 mL, 20.021 mmol) and imidazole (0.629 g, 9.240 mmol) were dissolved in dichloromethane (17 mL) at room temperature, after which the resulting solution was stirred at 60°C for 18 hours, and then a reaction was finished by lowering a temperature to room temperature. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 24 g cartridge; methanol/dichloromethane = 0 to 5%) and concentrated to obtain a title compound (1.600 g, 85.8%) in a yellow solid form.

### [Step 4] Synthesis of 5-bromo-3-((5-(5-(difluoromethyl)-1,3,4-oxadiazole-2-yl)pyridine-2-yl)methyl)-1-(piperidine-4-yl)-1,3-dihydro-2H-benzo[d]imidazole-2-one

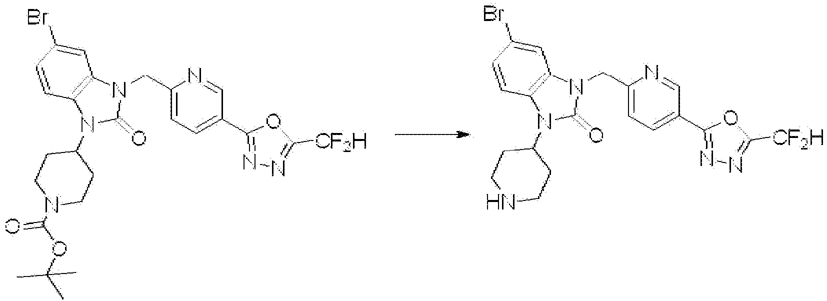

The tert-butyl 4-(5-bromo-3-((5-(5-(difluoromethyl)-1,3,4-oxadiazole-2-yl)pyridine-2-yl)methyl)-2-oxo-2,3-dihydr o-1H-benzo[d]imidazole-1-yl)piperidine-1-carboxylate (1.600 g, 2.643 mmol) prepared in the step 3 and trifluoroacetic acid (2.024 mL, 26.427 mmol) were dissolved in dichloromethane (20 mL) at room temperature, after which the resulting solution was stirred at the same temperature for 4 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 24 g cartridge; ethyl acetate/hexane = 80 to 100%) and concentrated, after which an obtained product was purified again via chromatography (SiO₂, 24 g cartridge; methanol/dichloromethane = 0 to 80%) and concentrated to obtain a title compound (1.166 g, 87.3%) in a yellow solid form.

### [Step 5] Synthesis of the compound 141

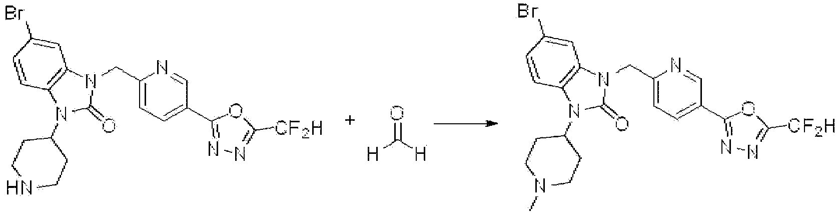

The 5-bromo-3-((5-(5-(difluoromethyl)-1,3,4-oxadiazole-2-yl)pyridine-2-yl)methyl)-1-(piperidine-4-yl)-1,3-dihydro-2H-benzo[d]imidazole-2-one (1.166 g, 2.307 mmol) prepared in the step 4, formaldehyde (37.00%, 0.562 g, 6.922 mmol) and sodium triacetoxyborohydride (0.978 g, 4.615 mmol) were dissolved in dichloromethane (25 mL) at room temperature, after which the resulting solution was stirred at the same temperature for 18 hours. Water was poured into the reaction mixture and an extraction was performed with dichloromethane. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; ethyl acetate/hexane = 0 to 30%) and concentrated to obtain a title compound (1.018 g, 85.0%) in a yellow solid form.

**¹H NMR** (400 MHz, CDCl₃) δ 9.29 (d, *J =* 1.7 Hz, 1H), 8.34 (dd, *J* = 8.2, 2.2 Hz, 1H), 7.40 (d, *J = 8.3* Hz, 1H), 7.18 (s, 2H), 7.13 (s, 1H), 6.93 (t, *J* = 51.7 Hz, 1H), 5.24 (s, 2H), 4.44 - 4.38 (m, 1H), 3.02 (d, *J* = 11.8 Hz, 2H), 2.55 - 2.35 (m, 2H), 2.35 (s, 3H), 2.15 (t, *J* = 11.0 Hz, 2H), 1.84 (dd, *J* = 11.8, 2.2 Hz, 2H); **LRMS** (ES) m/z 519.2 (M⁺ + 1).

### Example 142: Synthesis of Compound 142, 3-((5-(5-(difluoromethyl)-1,3,4-oxadiazole-2-yl)pyridine-2-yl)methyl)-5-fluoro-1-methyl-1,3-dihydr o-2H-benzo[d]imidazole-2-one

### [Step 1] Synthesis of 4-fluoro-N-methyl-2-nitroaniline

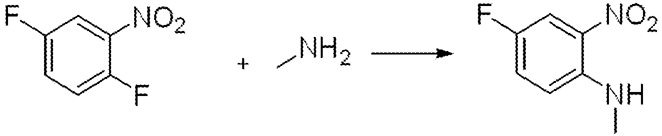

1,4-difluoro-2-nitrobenzene (3.000 g, 18.857 mmol), methanamine (33.00% solution, 3.915 mL, 56.572 mmol), potassium carbonate (5.212 g, 37.715 mmol) and potassium iodide (0.313 g, 1.886 mmol) were dissolved in N,N-dimethylformamide (90 mL) at room temperature, after which the resulting solution was stirred at 80°C for 6 hours, and then a reaction was finished by lowering a temperature to room temperature. Solvent was removed from the reaction mixture under reduced pressure, after which the resulting concentrate was purified via column chromatography (SiO₂, 80 g cartridge; ethyl acetate/hexane = 0 to 10%) and concentrated to obtain a title compound (2.690 g, 83.8%) in a red solid form.

### [Step 2] Synthesis of 4-fluoro-N¹-methylbenzene-1,2-diamine

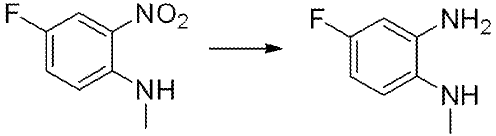

The 4-fluoro-N-methyl-2-nitroaniline (2.690 g, 15.811 mmol) prepared in the step 1 and Pd/C (45.00%, 0.374 g, 1.581 mmol) were dissolved in methanol (100 mL) at room temperature, after which the resulting solution was stirred at the same temperature for 18 hours in the presence of a hydrogen balloon. The reaction mixture was filtered via a celite pad to remove a solid therefrom, after which solvent was removed from the resulting filtrate under reduced pressure, and then a title compound was used without an additional purification process (2.070 g, 93.4%, brown liquid).

### [Step 3] Synthesis of 5-fluoro-1-methyl-1,3-dihydro-2H-benzo[d]imidazole-2-one

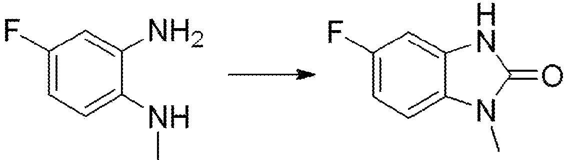

The 4-fluoro-N¹-methylbenzene-1,2-diamine (2.070 g, 14.769 mmol) prepared in the step 2 and 1,1'-carbonyldiimidazole (CDI, 2.874 g, 17.723 mmol) were dissolved in tetrahydrofuran (70 mL) at room temperature, after which the resulting solution was stirred at the same temperature for 18 hours. A precipitated solid was filtered, then washed with dichloromethane, and then dried to obtain a title compound (0.600 g, 24.4%) in a white solid form.

### [Step 4] Synthesis of the compound 142

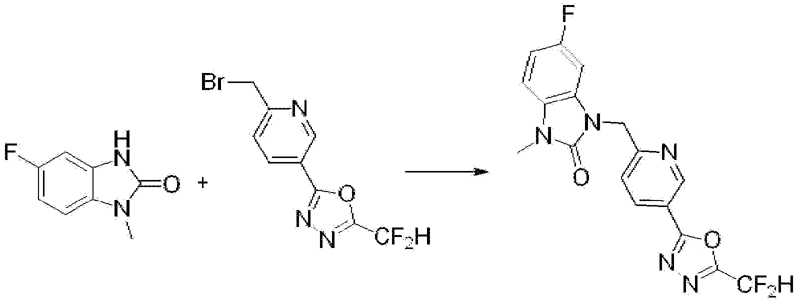

The 5-fluoro-1-methyl-1,3-dihydro-2H-benzo[d]imidazole-2-one (0.200 g, 1.204 mmol) prepared in the step 3 was dissolved in N,N-dimethylformamide (10 mL) at 0°C, after which sodium hydride (60.00%, 0.072 g, 1.805 mmol) was added into the resulting solution and stirred at the same temperature for 20 minutes. 2-(6-(bromomethyl)pyridine-3-yl)-5-(difluoromethyl)-1,3,4-oxadiazole (0.454 g, 1.565 mmol) was added into the reaction mixture and further stirred at room temperature for 4 hours. Solvent was removed from the reaction mixture under reduced pressure, after which the resulting concentrate was purified via column chromatography (SiO₂, 4 g cartridge; ethyl acetate/hexane = 0 to 50%) and concentrated to obtain a title compound (0.195 g, 43.1%) in a white solid form.

**¹H NMR** (400 MHz, CDCl₃) δ 9.26 (d, *J =* 2.1 Hz, 1H), 8.32 (dd, *J* = 8.2, 2.2 Hz, 1H), 7.42 (d, *J =* 8.3 Hz, 1H), 7.05 - 6.72 (m, 4H), 5.24 (s, 2H), 3.45 (s, 3H); **LRMS** (ES) m/z 376.0 (M⁺ + 1).

### Example 143: Synthesis of Compound 143, 3-(4-(5-(difluoromethyl)-1,3,4-oxadiazole-2-yl)-2-fluorobenzyl)-5-fluoro-1-methyl-1,3-dihydro-2H-benzo[d]imidazole-2-one

### [Step 1] Synthesis of the compound 143

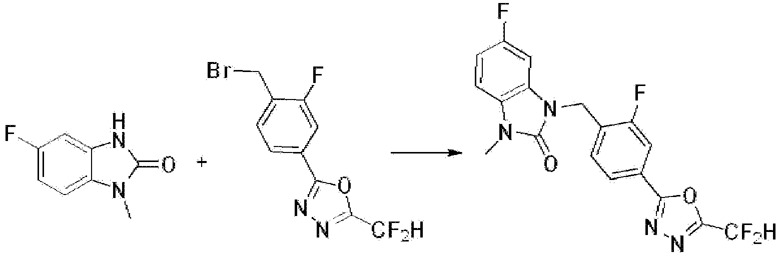

The 5-fluoro-1-methyl-1,3-dihydro-2H-benzo[d]imidazole-2-one (0.200 g, 1.204 mmol) prepared in the step 3 of the compound 142 was dissolved in N,N-dimethylformamide (10 mL) at 0°C, after which sodium hydride (60.00%, 0.072 g, 1.805 mmol) was added into the resulting solution and stirred at the same temperature for 20 minutes. 2-(4-(bromomethyl)-3-fluorophenyl)-5-(difluoromethyl)-1,3,4-oxadiazole (0.480 g, 1.565 mmol) was added into the reaction mixture and further stirred at room temperature for 4 hours. Solvent was removed from the reaction mixture under reduced pressure, after which the resulting concentrate was purified via column chromatography (SiO₂, 4 g cartridge; ethyl acetate/hexane = 0 to 30%) and concentrated to obtain a title compound (0.302 g, 63.9%) in a white solid form.

**¹H NMR** (400 MHz, CDCl₃) δ 7.86 - 7.82 (m, 2H), 7.47 (t, *J =* 7.8 Hz, 1H), 7.03 - 6.70 (m, 4H), 5.16 (s, 2H), 3.45 (s, 3H); **LRMS** (ES) m/z 393.2 (M⁺ + 1).

### Example 144: Synthesis of Compound 144, 3-(4-(5-(difluoromethyl)-1,3,4-oxadiazole-2-yl)benzyl)-5-fluoro-1-methyl-1,3-dihydro-2H-benzo[d] imidazole-2-one

### [Step 1] Synthesis of the compound 144

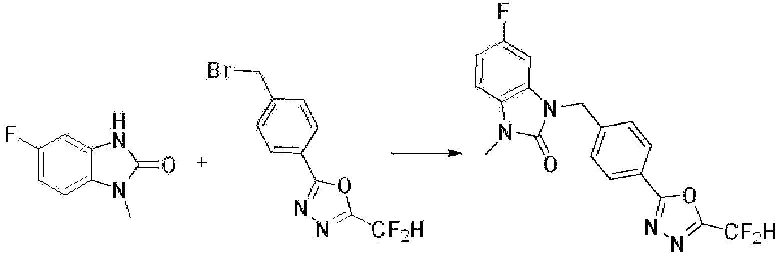

The 5-fluoro-1-methyl-1,3-dihydro-2H-benzo[d]imidazole-2-one (0.200 g, 1.204 mmol) prepared in the step 3 of the compound 142 was dissolved in N,N-dimethylformamide (12 mL) at 0°C, after which sodium hydride (60.00%, 0.072 g, 1.805 mmol) was added into the resulting solution and stirred at the same temperature for 20 minutes. 2-(4-(bromomethyl)phenyl)-5-(difluoromethyl)-1,3,4-oxadiazole (0.452 g, 1.565 mmol) was added into the reaction mixture and further stirred at room temperature for 4 hours. Solvent was removed from the reaction mixture under reduced pressure, after which the resulting concentrate was purified via column chromatography (SiO₂, 4 g cartridge; ethyl acetate/hexane = 0 to 30%) and concentrated to obtain a title compound (0.362 g, 80.4%) in a white solid form.

**¹H NMR** (400 MHz, CDCl₃) δ 8.02 - 7.96 (m, 2H), 7.42 (d, *J =* 8.6 Hz, 2H), 7.02 - 6.72 (m, 3H), 6.55 (dd, *J* = 8.4, 2.4 Hz, 1H), 5.07 (s, 2H), 3.41 (s, 3H); **LRMS** (ES) m/z 374.9 (M⁺ + 1).

### Example 145: Synthesis of Compound 145, 3-((5-(5-(difluoromethyl)-1,3,4-oxadiazole-2-yl)pyridine-2-yl)methyl)-1-(1-methylpiperidine-4-yl)-5 -(pyridine-3-yl)-1,3-dihydro-2H-benzo[d]imidazole-2-one

### [Step 1] Synthesis of the compound 145

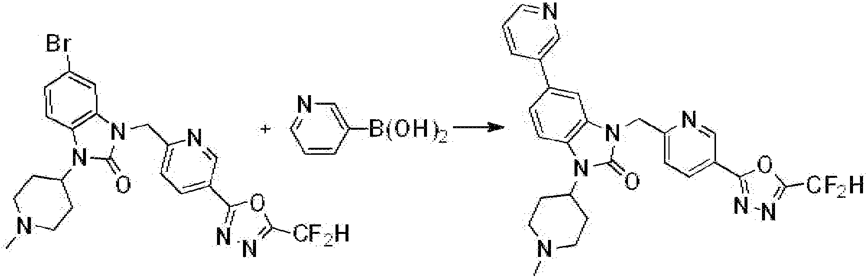

The 5-bromo-3-((5-(5-(difluoromethyl)-1,3,4-oxadiazole-2-yl)pyridine-2-yl)methyl)-1-(1-methylpiperidi ne-4-yl)-1,3-dihydro-2H-benzo[d]imidazole-2-one (0.120 g, 0.231 mmol) prepared in the step 5 of the compound 141, pyridine-3-ylboronic acid (0.034 g, 0.277 mmol), [1,1'-bis(di-tert-butylphosphino)ferrocene]palladium dichloride (0.008 g, 0.012 mmol) and cesium carbonate (0.151 g, 0.462 mmol) were mixed in 1,4-dioxane (1.7 mL) at room temperature, after which the resulting mixture was irradiated with microwave, then heated at 100°C for 30 minutes, and then a reaction was finished by lowering a temperature to room temperature. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 10%) and concentrated to obtain a title compound (0.018 g, 15.3%) in a brown solid form.

**¹H NMR** (400 MHz, CDCl₃) δ 9.27 (dd, *J* = 2.2, 0.6 Hz, 1H), 8.74 (s, 1H), 8.54 (s, 1H), 8.32 (dd, *J* = 8.2, 2.2 Hz, 1H), 7.78 (d, *J* = 7.7 Hz, 1H), 7.45 - 7.42 (m, 2H), 7.32 - 7.18 (m, 3H), 6.91 (t, *J =* 51.6 Hz, 1H), 5.33 (s, 2H), 4.52 - 4.44 (m, 1H), 3.06 (d, *J* = 11.8 Hz, 2H), 2.57 - 2.48 (m, 2H), 2.37 (s, 3H), 2.23 - 2.16 (m, 2H), 1.89 (dd, *J =* 12.1, 2.2 Hz, 2H); **LRMS** (ES) m/z 518.3 (M⁺ + 1).

### Example 146: Synthesis of Compound 146, 3-((5-(5-(difluoromethyl)-1,3,4-oxadiazole-2-yl)pyridine-2-yl)methyl)-5-(5-methylfuran-2-yl)-1-(1-methylpiperidine-4-yl)-1,3-dihydro-2H-benzo[d]imidazole-2-one

### [Step 1] Synthesis of the compound 146

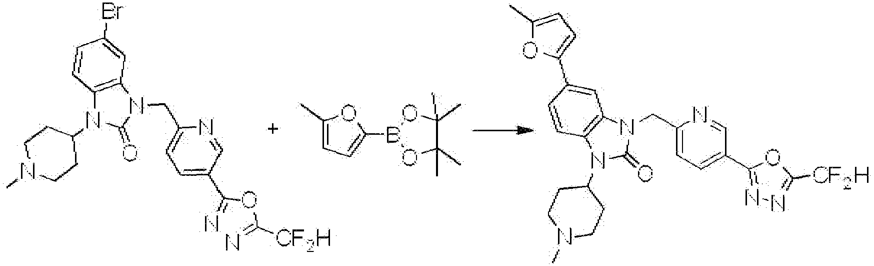

The 5-bromo-3-((5-(5-(difluoromethyl)-1,3,4-oxadiazole-2-yl)pyridine-2-yl)methyl)-1-(1-methylpiperidi ne-4-yl)-1,3-dihydro-2H-benzo[d]imidazole-2-one (0.120 g, 0.231 mmol) prepared in the step 5 of the compound 141, 4,4,5,5-tetramethyl-2-(5-methylfuran-2-yl)-1,3,2-dioxaborolane (0.058 g, 0.277 mmol), [1,1'-bis(di-tert-butylphosphino)ferrocene]palladium dichloride (0.008 g, 0.012 mmol) and cesium carbonate (0.151 g, 0.462 mmol) were mixed in 1,4-dioxane (1.7 mL) at room temperature, after which the resulting mixture was irradiated with microwave, then heated at 100°C for 30 minutes, and then a reaction was finished by lowering a temperature to room temperature. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.022 g, 18.2%) in a brown solid form.

**¹H NMR** (400 MHz, CDCl₃) δ 9.30 - 9.29 (m, 1H), 8.30 (dd, *J* = 8.2, 2.2 Hz, 1H), 7.37 (d, *J* = 8.2 Hz, 1H), 7.32 - 7.26 (m, 2H), 7.19 (s, 1H), 6.91 (t, *J =* 51.6 Hz, 1H), 6.40 (d, *J* = 3.2 Hz, 1H), 5.99 (dd, *J* = 3.1, 1.0 Hz, 1H), 5.31 (s, 2H), 4.49 - 4.40 (m, 1H), 3.04 (d, *J* = 11.6 Hz, 2H), 2.53 - 2.44 (m, 2H), 2.36 (s, 3H), 2.32 (s, 3H), 2.16 (t, *J =* 5.4 Hz, 2H), 1.87 (dd, *J =* 12.1, 2.1 Hz, 2H); **LRMS** (ES) m/z 520.9 (M⁺ + 1).

### Example 147: Synthesis of Compound 147, 5-bromo-1-((5-(5-(difluoromethyl)-1,3,4-oxadiazole-2-yl)pyridine-2-yl)methyl)-6-fluoro-3-methyl-1 ,3-dihydro-2H-benzo[d]imidazole-2-one

### [Step 1] Synthesis of 5-bromo-4-fluoro-N-methyl-2-nitroaniline

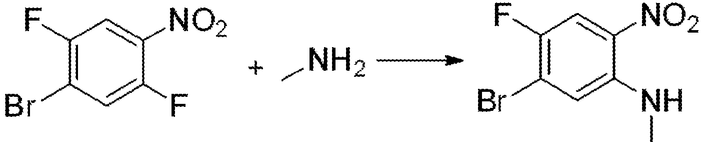

1-bromo-2,5-difluoro-4-nitrobenzene (10.000 g, 42.019 mmol), methanamine (33.00% solution, 5.754 mL, 46.220 mmol), potassium carbonate (11.614 g, 84.037 mmol) and potassium iodide (0.698 g, 4.202 mmol) were dissolved in N,N-dimethylformamide (250 mL) at room temperature, after which the resulting solution was stirred at 80°C for 3 hours, and then a reaction was finished by lowering a temperature to room temperature. Water (250 mL) was put into the reaction mixture and stirred, after which a precipitated solid was filtered, then washed with water, and then dried to obtain a title compound (10.130 g, 96.8%) in a red solid form.

### [Step 2] Synthesis of 5-bromo-4-fluoro-N¹-methylbenzene-1,2-diamine

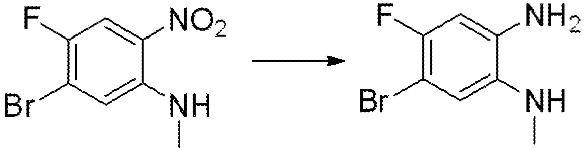

The 5-bromo-4-fluoro-N-methyl-2-nitroaniline (12.480 g, 50.112 mmol) prepared in the step 1 and Raney nickel (1.2g, 10 wt%) were dissolved in methanol (250 mL) at room temperature, after which the resulting solution was stirred at the same temperature for 18 hours in the presence of a hydrogen balloon. The reaction mixture was filtered via a celite pad to remove a solid therefrom, after which solvent was removed from the resulting filtrate under reduced pressure, and then a title compound was used without an additional purification process (10.110 g, 92.1%, brown liquid).

### [Step 3] Synthesis of 6-bromo-5-fluoro-1-methyl-1,3-dihydro-2H-benzo[d]imidazole-2-one

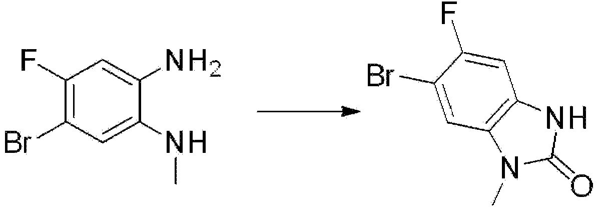

The 5-bromo-4-fluoro-N¹-methylbenzene-1,2-diamine (10.110 g, 46.152 mmol) prepared in the step 2, 1,1'-carbonyldiimidazole (CDI, 9.729 g, 59.997 mmol) and triethylamine (7.076 mL, 50.767 mmol) were dissolved in tetrahydrofuran (200 mL) at room temperature, after which the resulting solution was heated under reflux for 18 hours, and then a reaction was finished by lowering a temperature to room temperature. A precipitated solid was filtered and dried to obtain a title compound (8.486 g, 75.0%) in a violet solid form.

### [Step 4] Synthesis of the compound 147

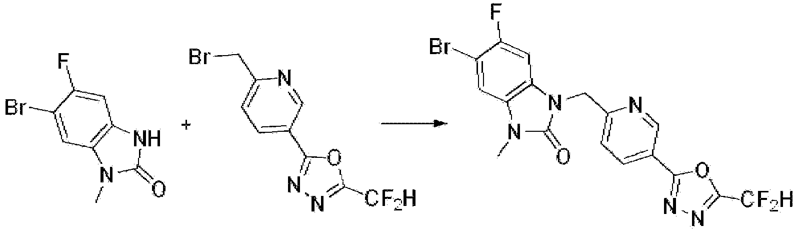

The 6-bromo-5-fluoro-1-methyl-1,3-dihydro-2H-benzo[d]imidazole-2-one (1.489 g, 6.076 mmol) prepared in the step 3 was dissolved in N,N-dimethylformamide (60 mL) at 0°C, after which sodium hydride (60.00%, 0.365 g, 9.114 mmol) was added into the resulting solution and stirred at the same temperature for 30 minutes. 2-(6-(bromomethyl)pyridine-3-yl)-5-(difluoromethyl)-1,3,4-oxadiazole (1.939 g, 6.684 mmol) was added into the reaction mixture and further stirred at room temperature for 5 hours. Water was poured into the reaction mixture and an extraction was performed with ethyl acetate. An organic layer was washed with saturated sodium chloride aqueous solution, then dehydrated with anhydrous magnesium sulfate, then filtered, and then concentrated under reduced pressure. The resulting concentrate was purified via column chromatography (SiO₂, 24 g cartridge; ethyl acetate/hexane = 30 to 70%) and concentrated to obtain a title compound (1.145 g, 41.5%) in a yellow solid form.

**¹H NMR** (400 MHz, CDCl₃) δ 9.27 (dd, *J* = 2.2, 0.7 Hz, 1H), 8.35 (dd, *J* = 8.2, 2.2 Hz, 1H), 7.46 (d, *J = 8.2* Hz, 1H), 7.13 (d, *J =* 5.6 Hz, 1H), 6.93 (t, *J =* 51.6 Hz, 1H), 6.86 (d, *J =* 8.1 Hz, 1H), 5.24 (s, 2H), 3.45 (s, 3H); LRMS (ES) m/z 454.1 (M⁺ + 1).

### Example 148: Synthesis of Compound 148, 5-bromo-1-(4-(5-(difluoromethyl)-1,3,4-oxadiazole-2-yl)-2-fluorobenzyl)-6-fluoro-3-methyl-1,3-dih ydro-2H-benzo[d]imidazole-2-one

### [Step 1] Synthesis of the compound 148

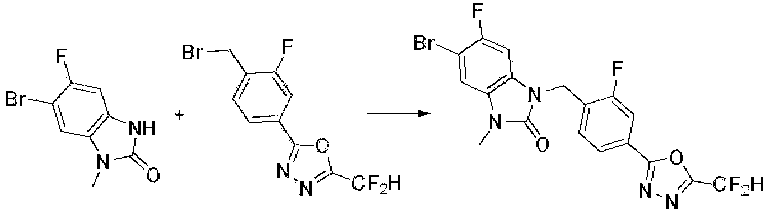

The 6-bromo-5-fluoro-1-methyl-1,3-dihydro-2H-benzo[d]imidazole-2-one (0.300 g, 1.224 mmol) prepared in the step 3 of the compound 147 was dissolved in N,N-dimethylformamide (12 mL) at 0°C, after which sodium hydride (60.00%, 0.073 g, 1.836 mmol) was added into the resulting solution and stirred at the same temperature for 30 minutes. 2-(4-(bromomethyl)-3-fluorophenyl)-5-(difluoromethyl)-1,3,4-oxadiazole (0.414 g, 1.347 mmol) was added into the reaction mixture and further stirred at room temperature for 5 hours. Solvent was removed from the reaction mixture under reduced pressure, after which the resulting concentrate was purified via column chromatography (SiO₂, 40 g cartridge; ethyl acetate/hexane = 0 to 60%) and concentrated to obtain a title compound (0.384 g, 66.6%) in a brown solid form.

**¹H NMR** (400 MHz, DMSO-d₆) δ 7.89 (dd, *J =* 10.3, 1.6 Hz, 1H), 7.84 (dd, *J* = 8.0, 1.7 Hz, 1H), 7.59 (d, *J =* 6.0 Hz, 1H), 7.54 (t, *J = 51.3* Hz, 1H), 7.41 (t, *J =* 7.8 Hz, 1H), 7.36 (d, *J* = 9.0 Hz, 1H), 5.21 (s, 2H), 3.35 (s, 3H); **LRMS** (ES) m/z 473.2 (M⁺ + 1).

### Example 149: Synthesis of Compound 149, 5-bromo-1-((5-(5-(difluoromethyl)-1,3,4-oxadiazole-2-yl)pyridine-2-yl)methyl)-6-fluoro-3-(1-methy lpiperidine-4-yl)-1,3-dihydro-2H-benzo[d]imidazole-2-one

### [Step 1] Synthesis of tert-butyl 4-(6-bromo-3-((5-(5-(difluoromethyl)-1,3,4-oxadiazole-2-yl)pyridine-2-yl)methyl)-5-fluoro-2-oxo-2 ,3-dihydro-1H-benzo[d]imidazole-1-yl)piperidine-1-carboxylate

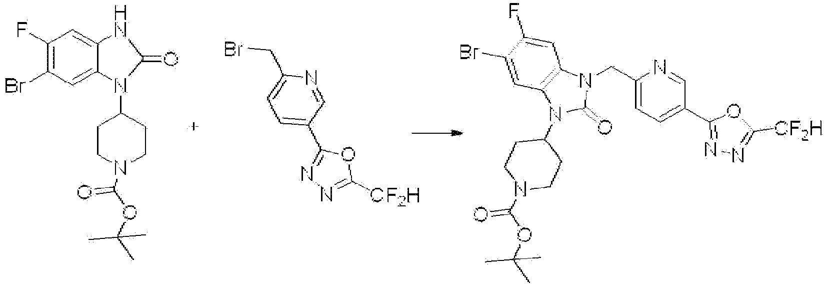

Tert-butyl 4-(6-bromo-5-fluoro-2-oxo-2,3-dihydro-1H-benzo[d]imidazole-1-yl) piperidine-1-carboxylate (1.500 g, 3.621 mmol) was dissolved in N,N-dimethylformamide (20 mL) at 0°C, after which sodium hydride (60.00%, 0.174 g, 4.345 mmol) was added into the resulting solution and stirred at the same temperature for 30 minutes. 2-(6-(bromomethyl)pyridine-3-yl)-5-(difluoromethyl)-1,3,4-oxadiazole (1.050 g, 3.621 mmol) was added into the reaction mixture and further stirred at room temperature for 2 hours. Water was poured into the reaction mixture and an extraction was performed with ethyl acetate. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 40 g cartridge; ethyl acetate/hexane = 0 to 50%) and concentrated to obtain a title compound (1.500 g, 66.5%) in a colorless oil form.

### [Step 2] Synthesis of 5-bromo-1-((5-(5-(difluoromethyl)-1,3,4-oxadiazole-2-yl)pyridine-2-yl)methyl)-6-fluoro-3-(piperidi ne-4-yl)-1,3-dihydro-2H-benzo[d]imidazole-2-one 2,2,2-trifluoroacetate

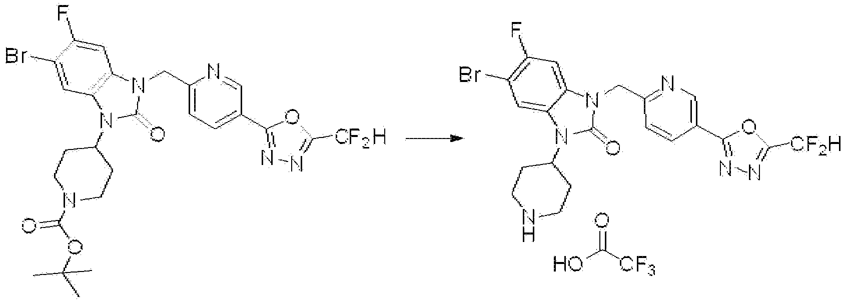

The tert-butyl 4-(6-bromo-3-((5-(5-(difluoromethyl)-1,3,4-oxadiazole-2-yl)pyridine-2-yl)methyl)-5-fluoro-2-oxo-2 ,3-dihydro-1H-benzo[d]imidazole-1-yl)piperidine-1-carboxylate (1.500 g, 2.406 mmol) prepared in the step 1 and trifluoroacetic acid (1.842 mL, 24.060 mmol) were dissolved in dichloromethane (30 mL) at room temperature, after which the resulting solution was stirred at the same temperature for 12 hours. Solvent was removed from the reaction mixture under reduced pressure, after which a title compound was used without an additional purification process (1.400 g, 91.3%, yellow solid).

### [Step 3] Synthesis of the compound 149

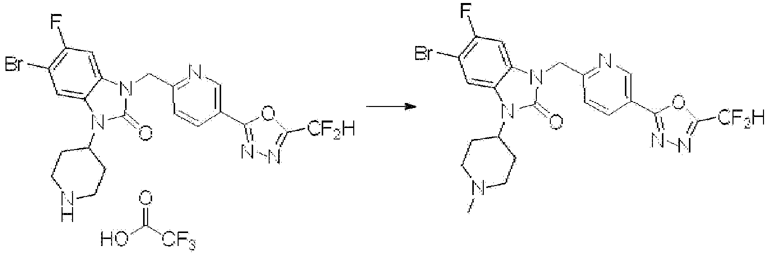

The 5-bromo-1-((5-(5-(difluoromethyl)-1,3,4-oxadiazole-2-yl)pyridine-2-yl)methyl)-6-fluoro-3-(piperidi ne-4-yl)-1,3-dihydro-2H-benzo[d]imidazole-2-one 2,2,2-trifluoroacetate (1.400 g, 2.197 mmol) prepared in the step 2, formaldehyde (0.132 g, 4.393 mmol), N,N-diisopropylethylamine (0.383 mL, 2.197 mmol) and sodium triacetoxyborohydride (0.931 g, 4.393 mmol) were dissolved in dichloromethane (20 mL) at room temperature, after which the resulting solution was stirred at the same temperature for 12 hours. Water was poured into the reaction mixture and an extraction was performed with dichloromethane. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; methanol/dichloromethane = 0 to 10%) and concentrated to obtain a title compound (1.300 g, 110.1%) in a white foam solid form.

**LRMS** (ES) m/z 538.4 (M⁺ + 1).

### Example 150: Synthesis of Compound 150, 1-((5-(5-(difluoromethyl)-1,3,4-oxadiazole-2-yl)pyridine-2-yl)methyl)-6-fluoro-5-(furan-3-yl)-3-(1-methylpiperidine-4-yl)-1,3-dihydro-2H-benzo[d]imidazole-2-one

### [Step 1] Synthesis of the compound 150

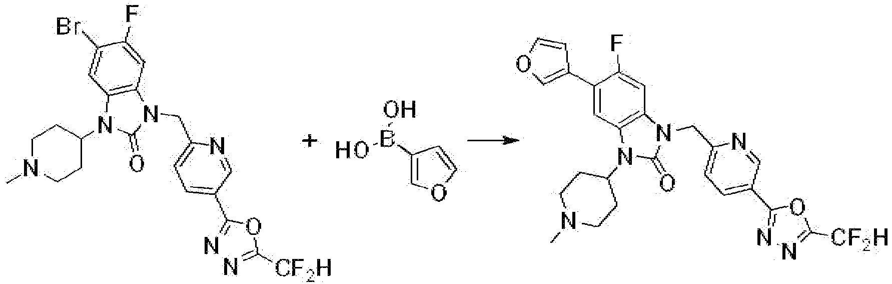

5-bromo-1-((5-(5-(difluoromethyl)-1,3,4-oxadiazole-2-yl)pyridine-2-yl)methyl)-6-fluoro-3-( 1-methylpiperidine-4-yl)-1,3-dihydro-2H-benzo[d]imidazole-2-one (0.150 g, 0.279 mmol), furan-3-ylboronic acid (0.047 g, 0.419 mmol), [1,1' -bis(diphenylphosphino)ferrocene]dichloropalladium (0.018 g, 0.028 mmol) and cesium carbonate (0.136 g, 0.419 mmol) were mixed in 1,4-dioxane (6 mL)/water (2 mL), after which the resulting mixture was irradiated with microwave, then heated at 100°C for 20 minutes, and then a reaction was finished by lowering a temperature to room temperature. Solvent was removed from the reaction mixture under reduced pressure, after which water was poured into the resulting concentrate, and then an extraction was performed with dichloromethane. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; methanol/dichloromethane = 0 to 10%) and concentrated to obtain a title compound (0.110 g, 75.1%) in a brown oil form.

**¹H NMR** (400 MHz, CDCl₃) δ 9.31 (d, *J =* 1.6 Hz, 1H), 8.36 (dd, *J* = 8.2, 2.2 Hz, 1H), 7.83 (s, 1H), 7.51 (t, *J* = 1.7 Hz, 1H), 7.45 (d, *J* = 8.2 Hz, 1H), 7.38 (d, *J =* 5.9 Hz, 1H), 7.07 (s, 0.25H), 6.94 (s, 0.5H), 6.83 (s, 0.25H), 6.85 - 6.82 (m, 2H), 5.27 (s, 2H), 4.52 - 4.51 (m, 1H), 3.50 (s, 3H), 3.10 - 3.08 (m, 2H), 2.60 - 2.10 (m, 4H), 1.91 - 1.88 (m, 2H).

### Example 151: Synthesis of Compound 151, 1-((5-(5-(difluoromethyl)-1,3,4-oxadiazole-2-yl)pyridine-2-yl)methyl)-6-fluoro-5-(furan-2-yl)-3-(1-methylpiperidine-4-yl)-1,3-dihydro-2H-benzo[d]imidazole-2-one

### [Step 1] Synthesis of the compound 151

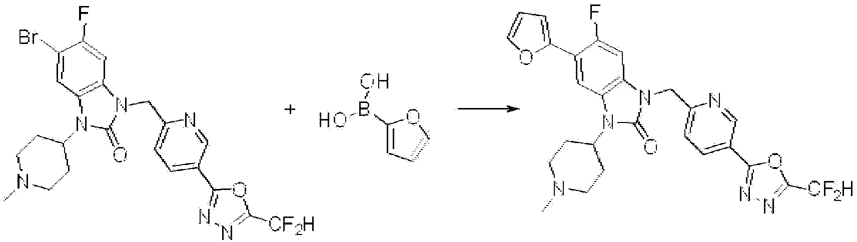

5-bromo-1-((5-(5-(difluoromethyl)-1,3,4-oxadiazole-2-yl)pyridine-2-yl)methyl)-6-fluoro-3-( 1-methylpiperidine-4-yl)-1,3-dihydro-2H-benzo[d]imidazole-2-one (0.150 g, 0.279 mmol), furan-2-ylboronic acid (0.047 g, 0.419 mmol), [1,1'-bis(diphenylphosphino)ferrocene]dichloropalladium (0.018 g, 0.028 mmol) and cesium carbonate (0.136 g, 0.419 mmol) were mixed in 1,4-dioxane (6 mL)/water (2 mL), after which the resulting mixture was irradiated with microwave, then heated at 100°C for 20 minutes, and then a reaction was finished by lowering a temperature to room temperature. Solvent was removed from the reaction mixture under reduced pressure, after which water was poured into the resulting concentrate, and then an extraction was performed with dichloromethane. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; methanol/dichloromethane = 0 to 10%) and concentrated to obtain a title compound (0.080 g, 54.6%) in a brown oil form.

**¹H NMR** (400 MHz, CDCl₃) δ 9.31 (d, *J =* 2.1 Hz, 1H), 8.37 (dd, *J* = 8.2, 2.2 Hz, 1H), 7.70 (d, *J* = 6.0 Hz, 1H), 7.50 (d, *J =* 1.6 Hz, 1H), 4.23 (s, 1H), 7.07 (s, 0.25H), 6.94 (s, 0.0.5H), 6.84 (d, *J =* 10.4 Hz, 1H), 6.81 (s, 0.25H), 6.76 (t, *J =* 6.2 Hz, 1H), 6.52 - 6.51 (m, 1H), 5.27 (s, 2H), 4.50 - 4.40 (m, 1H), 3.10 - 3.07 (m, 2H), 2.58 - 2.54 (m, 2H), 2.40 (s, 3H), 2.22 - 2.18 (m, 2H), 1.90 - 1.87 (m, 2H).

### Example 152: Synthesis of Compound 152, 1-((5-(5-(difluoromethyl)-1,3,4-oxadiazole-2-yl)pyridine-2-yl)methyl)-6-fluoro-5-(2-fluorophenyl)-3 -(1-methylpiperidine-4-yl)-1,3-dihydro-2H-benzo[d]imidazole-2-one

### [Step 1] Synthesis of the compound 152

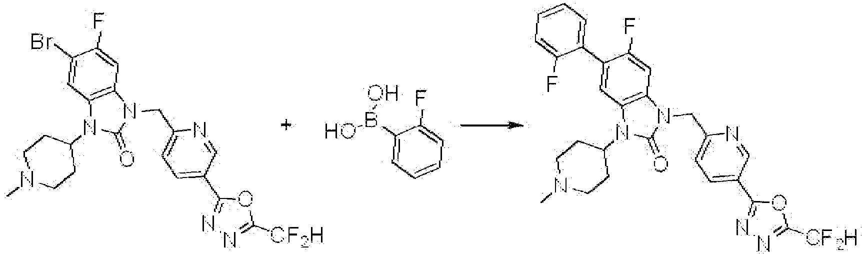

5-bromo-1-((5-(5-(difluoromethyl)-1,3,4-oxadiazole-2-yl)pyridine-2-yl)methyl)-6-fluoro-3-( 1-methylpiperidine-4-yl)-1,3-dihydro-2H-benzo[d]imidazole-2-one (0.150 g, 0.279 mmol), (2-fluorophenyl)boronic acid (0.059 g, 0.419 mmol), [1,1'-bis(diphenylphosphino)ferrocene]dichloropalladium (0.018 g, 0.028 mmol) and cesium carbonate (0.136 g, 0.419 mmol) were mixed in 1,4-dioxane (6 mL)/water (2 mL), after which the resulting mixture was irradiated with microwave, then heated at 100°C for 20 minutes, and then a reaction was finished by lowering a temperature to room temperature. Solvent was removed from the reaction mixture under reduced pressure, after which water was poured into the resulting concentrate, and then an extraction was performed with dichloromethane. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; methanol/dichloromethane = 0 to 10%) and concentrated to obtain a title compound (0.030 g, 19.5%) in a brown oil form.

**¹H NMR** (400 MHz, CDCl₃) 400 MHz, CDJ = 1.7 Hz, 1H), 8.39 (dd, *J* = 8.3, 2.1 Hz, 1H), 7.49 (d, *J = 8.3* Hz, 1H), 7.41 - 7.34 (m, 2H), 7.28 - 7.27 (m, 1H), 7.24 - 7.14 (m, 2H), 7.08 (s, 0.25H), 6.95 (s, 0.5H), 6.90 (d, *J =* 9.2 Hz, 1H), 6.82 (s, 0.25H), 5.30 (s, 2H), 4.50 - 4.44 (m, 1H), 3.05 - 3.02 (m, 2H), 2.51 - 2.42 (m, 2H), 2.35 (s, 3H), 2.23 - 2.19 (m, 2H), 1.91 - 1.88 (m, 2H).

### Example 153: Synthesis of Compound 153, 1-((5-(5-(difluoromethyl)-1,3,4-oxadiazole-2-yl)pyridine-2-yl)methyl)-6-fluoro-3-(1-methylpiperidin e-4-yl)-5-(pyridine-4-yl)-1,3-dihydro-2H-benzo[d]imidazole-2-one

### [Step 1] Synthesis of the compound 153

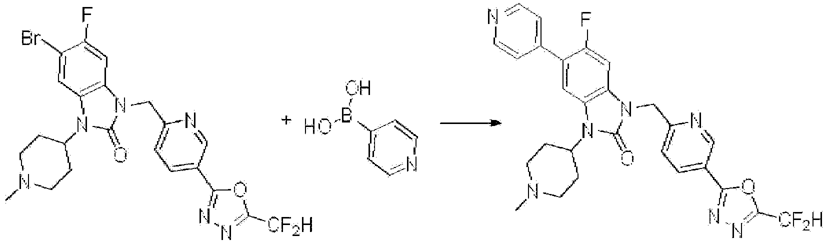

5-bromo-1-((5-(5-(difluoromethyl)-1,3,4-oxadiazole-2-yl)pyridine-2-yl)methyl)-6-fluoro-3-( 1-methylpiperidine-4-yl)-1,3-dihydro-2H-benzo[d]imidazole-2-one (0.150 g, 0.279 mmol), pyridine-4-ylboronic acid (0.051 g, 0.419 mmol), [1,1' -bis(diphenylphosphino)ferrocene]dichloropalladium (0.018 g, 0.028 mmol) and cesium carbonate (0.136 g, 0.419 mmol) were mixed in 1,4-dioxane (6 mL)/water (2 mL), after which the resulting mixture was irradiated with microwave, then heated at 100°C for 20 minutes, and then a reaction was finished by lowering a temperature to room temperature. Solvent was removed from the reaction mixture under reduced pressure, after which water was poured into the resulting concentrate, and then an extraction was performed with dichloromethane. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; methanol/dichloromethane = 0 to 10%) and concentrated to obtain a title compound (0.060 g, 40.1%) in a brown oil form.

**¹H NMR** (400 MHz, CDCl₃) δ 9.32 (dd, *J =* 2.2, 0.7 Hz, 1H), 8.68 - 8.67 (m, 1H), 8.39 (dd, *J* = 8.2, 2.2 Hz, 1H), 7.50 (dd, *J* = 8.3, 0.6 Hz, 1H), 7.45 (d, *J = 4.6* Hz, 1H), 7.33 (d, *J* = 5.9 Hz, 1H), 7.08 (s, 0.25H), 6.95 (s, 0.5H), 6.94 - 6.92 (m, 1H), 6.82 (s, 0.25H), 5.30 (s, 2H), 4.50 - 4.44 (m, 1H), 3.08 - 3.05 (m, 2H), 2.54 - 2.44 (m, 2H), 2.38 (s, 3H), 2.24 - 2.19 (m, 2H), 1.92 - 1.89 (m, 2H).

### Example 154: Synthesis of Compound 154, 1-((5-(5-(difluoromethyl)-1,3,4-oxadiazole-2-yl)pyridine-2-yl)methyl)-6-fluoro-5-(3-fluorophenyl)-3 -(1-methylpiperidine-4-yl)-1,3-dihydro-2H-benzo[d]imidazole-2-one

### [Step 1] Synthesis of the compound 154

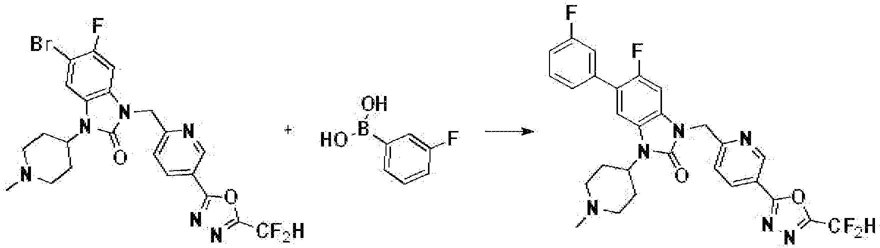

5-bromo-1-((5-(5-(difluoromethyl)-1,3,4-oxadiazole-2-yl)pyridine-2-yl)methyl)-6-fluoro-3-( 1-methylpiperidine-4-yl)-1,3-dihydro-2H-benzo[d]imidazole-2-one (0.150 g, 0.279 mmol), (3-fluorophenyl)boronic acid (0.059 g, 0.419 mmol), [1,1'-bis(diphenylphosphino)ferrocene]dichloropalladium (0.018 g, 0.028 mmol) and cesium carbonate (0.136 g, 0.419 mmol) were mixed in 1,4-dioxane (6 mL)/water (2 mL), after which the resulting mixture was irradiated with microwave, then heated at 100°C for 20 minutes, and then a reaction was finished by lowering a temperature to room temperature. Solvent was removed from the reaction mixture under reduced pressure, after which water was poured into the resulting concentrate, and then an extraction was performed with dichloromethane. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; methanol/dichloromethane = 0 to 10%) and concentrated to obtain a title compound (0.040 g, 25.9%) in a brown oil form.

**¹H NMR** (400 MHz, CDCl₃) δ 9.33 (dd, *J* = 2.2, 0.8 Hz, 1H), 8.39 (dd, *J* = 8.2, 2.2 Hz, 1H), 7.49 - 7.47 (m, 1H), 7.43 - 7.38 (m, 2H), 7.28 - 7.27 (m, 1H), 7.22 - 7.20 (m, 1H), 7.10 - 7.05 (m, 1H), 7.07 (s, 0.25H), 6.95 (s, 0.5H), 6.90 (d, *J* = 9.8 Hz, 1H), 6.82 (s, 0.25H), 5.29 (s, 2H), 4.53 - 4.41 (m, 1H), 3.09 - 3.07 (m, 2H), 2.42 - 2.40 (m, 2H), 2.40 (s, 3H), 2.39 - 0.20 (m, 2H), 1.91 - 1.88 (m, 2H).

### Example 155: Synthesis of Compound 155, 1-((5-(5-(difluoromethyl)-1,3,4-oxadiazole-2-yl)pyridine-2-yl)methyl)-6-fluoro-3-(1-methylpiperidin e-4-yl)-5-(pyridine-3-yl)-1,3-dihydro-2H-benzo[d]imidazole-2-one

### [Step 1] Synthesis of the compound 155

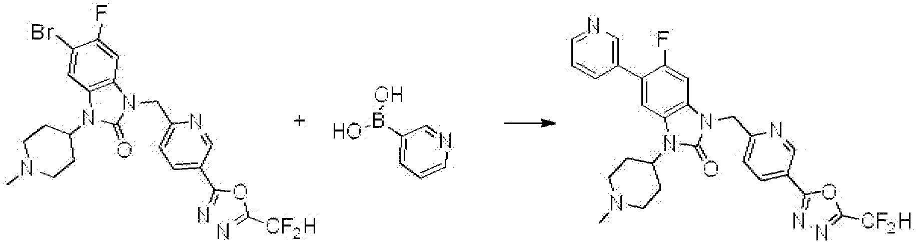

5-bromo-1-((5-(5-(difluoromethyl)-1,3,4-oxadiazole-2-yl)pyridine-2-yl)methyl)-6-fluoro-3-( 1-methylpiperidine-4-yl)-1,3-dihydro-2H-benzo[d]imidazole-2-one (0.150 g, 0.279 mmol), pyridine-3-ylboronic acid (0.051 g, 0.419 mmol), [1,1'-bis(diphenylphosphino)ferrocene]dichloropalladium (0.018 g, 0.028 mmol) and cesium carbonate (0.136 g, 0.419 mmol) were mixed in 1,4-dioxane (6 mL)/water (2 mL), after which the resulting mixture was irradiated with microwave, then heated at 100°C for 20 minutes, and then a reaction was finished by lowering a temperature to room temperature. Solvent was removed from the reaction mixture under reduced pressure, after which water was poured into the resulting concentrate, and then an extraction was performed with dichloromethane. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; methanol/dichloromethane = 0 to 10%) and concentrated to obtain a title compound (0.030 g, 20.1%) in a brown oil form.

**¹H NMR** (400 MHz, CDCl₃) δ 9.33 - 9.32 (m, 1H), 8.75 (s, 1H), 8.62 (d, *J* = 3.7 Hz, 1H), 8.39 (dd, *J =* 8.2, 2.2 Hz, 1H), 7.82 (d, *J = 4.0* Hz, 1H), 7.49 (d, *J = 8.0* Hz, 1H), 7.38 (dd, *J =* 7.9, 4.8 Hz, 1H), 7.30 - 7.28 (m, 1H), 7.08 (s, 1H), 6.95 (s, 1H), 6.93 (d, *J =* 9.7 Hz, 1H), 6.82 (s, 1H), 5.31 (s, 2H), 4.49 - 4.43 (m, 1H), 3.07 - 3.04 (m, 2H), 2.53 - 2.36 (m, 2H), 2.23 (s, 3H), 2.20 - 2.17 (m, 2H), 1.91 - 1.88 (m, 2H).

### Example 156: Synthesis of Compound 156, 1-(4-(5-(difluoromethyl)-1,3,4-oxadiazole-2-yl)-2-fluorobenzyl)-3,5-bis(1-methylpiperidine-4-yl)-1, 3-dihydro-2H-benzo[d]imidazole-2-one

### [Step 1] Synthesis of tert-butyl 4-(1-(4-(5-(difluoromethyl)-1,3,4-oxadiazole-2-yl)-2-fluorobenzyl)-3-(1-methylpiperidine-4-yl)-2-o xo-2,3-dihydro-1H-benzo[d]imidazole-5-yl)-3,6-dihydropyridine-1(2H)-carboxylate

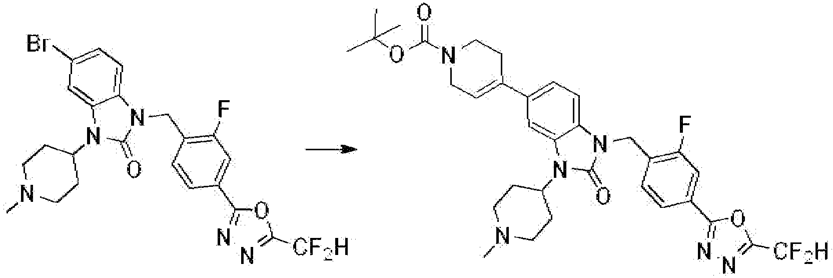

The 5-bromo-1-(4-(5-(difluoromethyl)-1,3,4-oxadiazole-2-yl)-2-fluorobenzyl)-3-(1-methylpiperidine-4-yl)-1,3-dihydro-2H-benzo[d]imidazole-2-one (1.000 g, 1.864 mmol) prepared in the step 3 of the compound 139, tert-butyl 4-(4,4,5,5-tetramethyl-1,3,2-dioxaborolane-2-yl)-3,6-dihydropyridine-1(2H)-carboxylate (0.692 g, 2.237 mmol), [1,1'-bis(di-tert-butylphosphino)ferrocene]palladium(II) dichloride (Pd(dtbpf)Cl₂, 0.061 g, 0.093 mmol) and cesium carbonate (1.822 g, 5.593 mmol) were mixed in 1,4-dioxane (3 mL)/water (1 mL) at room temperature, after which the resulting mixture was irradiated with microwave, then heated at 100°C for 20 minutes, and then a reaction was finished by lowering a temperature to room temperature. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 10%) and concentrated to obtain a title compound (1.120 g, 94.1%) in a brown oil form.

### [Step 2] Synthesis of tert-butyl 4-(1-(4-(5-(difluoromethyl)-1,3,4-oxadiazole-2-yl)-2-fluorobenzyl)-3-(1-methylpiperidine-4-yl)-2-o xo-2,3-dihydro-1H-benzo[d]imidazole-5-yl)piperidine-1-carboxylate

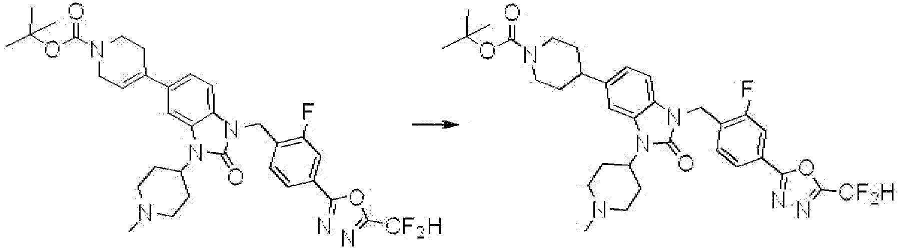

The tert-butyl 4-(1-(4-(5-(difluoromethyl)-1,3,4-oxadiazole-2-yl)-2-fluorobenzyl)-3-(1-methylpiperidine-4-yl)-2-o xo-2,3-dihydro-1H-benzo[d]imidazole-5-yl)-3,6-dihydropyridine-1(2H)-carboxylate (1.120 g, 1.754 mmol) prepared in the step 1 was dissolved in methanol (30 mL, 0.058 M) and subjected to a reaction by using H-cube (10%-Pd/C, 40°C, 2 bar H₂, 0.5 mL/min flow rate). Solvent was removed from the reaction mixture under reduced pressure, after which a title compound was used without an additional purification process (0.886 g, 78.9%, brown oil).

### [Step 3] Synthesis of 1-(4-(5-(difluoromethyl)-1,3,4-oxadiazole-2-yl)-2-fluorobenzyl)-3-(1-methylpiperidine-4-yl)-5-(pip eridine-4-yl)-1,3-dihydro-2H-benzo[d]imidazole-2-one

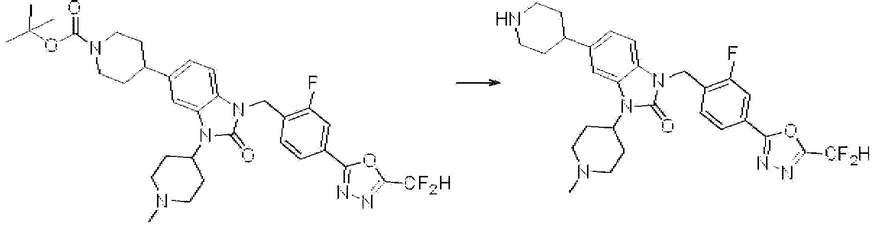

The tert-butyl 4-(1-(4-(5-(difluoromethyl)-1,3,4-oxadiazole-2-yl)-2-fluorobenzyl)-3-(1-methylpiperidine-4-yl)-2-o xo-2,3-dihydro-1H-benzo[d]imidazole-5-yl)piperidine-1-carboxylate (0.886 g, 1.383 mmol) prepared in the step 2 and trifluoroacetic acid (0.794 mL, 10.371 mmol) were dissolved in dichloromethane (7 mL) at room temperature, after which the resulting solution was stirred at the same temperature for 5 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. A title compound was used without an additional purification process (0.668 g, 89.4%, brown oil).

### [Step 4] Synthesis of the compound 156

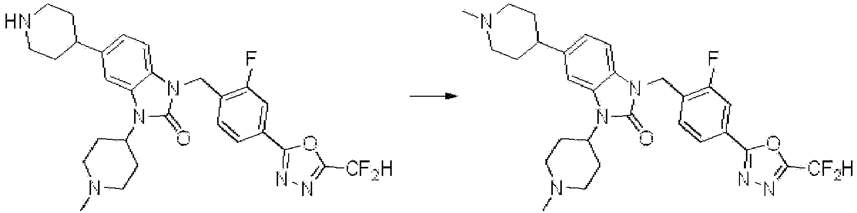

The 1-(4-(5-(difluoromethyl)-1,3,4-oxadiazole-2-yl)-2-fluorobenzyl)-3-(1-methylpiperidine-4-yl)-5-(pip eridine-4-yl)-1,3-dihydro-2H-benzo[d]imidazole-2-one (0.100 g, 0.185 mmol) prepared in the step 3, formaldehyde (38.00%, 0.022 g, 0.277 mmol) and sodium triacetoxyborohydride (0.078 g, 0.370 mmol) were dissolved in dichloromethane (4 mL) at room temperature, after which the resulting solution was stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 15%) and concentrated to obtain a title compound (0.042 g, 40.9%) in a yellow oil form.

**¹H NMR** (400 MHz, CDCl₃) δ 7.87 - 7.82 (m, 2H), 7.47 (t, *J* = 7.8 Hz, 1H), 7.24 (s, 1H), 7.05 - 6.79 (m, 3H), 5.19 (s, 2H), 4.48 - 4.42 (m, 1H), 3.05 (d, *J* = 11.2 Hz, 4H), 2.57 - 2.48 (m, 3H), 2.40 - 2.39 (m, 6H), 2.23 - 2.14 (m, 4H), 1.92 - 1.85 (m, 6H); LRMS (ES) m/z 555.3 (M⁺ + H).

### Example 157: Synthesis of Compound 157, 5-(1-acetylpiperidine-4-yl)-1-(4-(5-(difluoromethyl)-1,3,4-oxadiazole-2-yl)-2-fluorobenzyl)-3-(1-m ethylpiperidine-4-yl)-1,3-dihydro-2H-benzo[d]imidazole-2-one

### [Step 1] Synthesis of the compound 157

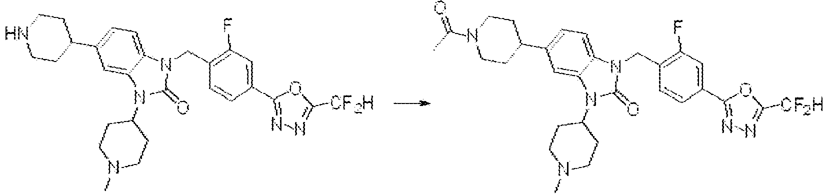

The 1-(4-(5-(difluoromethyl)-1,3,4-oxadiazole-2-yl)-2-fluorobenzyl)-3-(1-methylpiperidine-4-yl)-5-(pip eridine-4-yl)-1,3-dihydro-2H-benzo[d]imidazole-2-one (0.100 g, 0.185 mmol) prepared in the step 3 of the compound 156 and triethylamine (0.052 mL, 0.370 mmol) were dissolved in dichloromethane (4 mL) at room temperature, after which acetyl chloride (0.020 mL, 0.277 mmol) was added into the resulting solution and stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.053 g, 49.2%) in a light yellow solid form.

**¹H NMR** (400 MHz, CDCl₃) δ 7.87 - 7.83 (m, 2H), 7.47 (t, *J* = 7.8 Hz, 1H), 7.19 (brs, 1H), 7.05 - 6.79 (m, 3H), 5.19 (s, 2H), 4.83 - 4.78 (m, 1H), 4.47 - 4.46 (m, 1H), 3.97 - 3.93 (m, 1H), 3.21 - 3.07 (m, 3H), 2.80 - 2.74 (m, 1H), 2.65 - 2.52 (m, 3H), 2.41 (s, 3H), 2.25 - 2.20 (m, 2H), 2.16 (s, 3H), 1.92 - 1.86 (m, 4H), 1.70 - 1.59 (m, 2H); **LRMS** (ES) m/z 583.3 (M⁺ + H).

### Example 158: Synthesis of Compound 158, 1-(4-(5-(difluoromethyl)-1,3,4-oxadiazole-2-yl)-2-fluorobenzyl)-3-(1-methylpiperidine-4-yl)-5-(1-( methylsulfonyl)piperidine-4-yl)-1,3-dihydro-2H-benzo[d]imidazole-2-one

### [Step 1] Synthesis of the compound 158

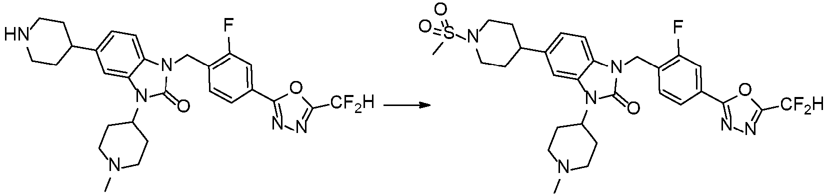

The 1-(4-(5-(difluoromethyl)-1,3,4-oxadiazole-2-yl)-2-fluorobenzyl)-3-(1-methylpiperidine-4-yl)-5-(pip eridine-4-yl)-1,3-dihydro-2H-benzo[d]imidazole-2-one (0.100 g, 0.185 mmol) prepared in the step 3 of the compound 156 and triethylamine (0.052 mL, 0.370 mmol) were dissolved in dichloromethane (4 mL) at room temperature, after which methanesulfonyl chloride (0.021 mL, 0.277 mmol) was added into the resulting solution and stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.039 g, 34.1%) in a light brown solid form.

**¹H NMR** (400 MHz, CDCl₃) δ 7.87 - 7.83 (m, 2H), 7.47 (t, *J =* 7.8 Hz, 1H), 7.21 (brs, 1H), 7.05 - 6.79 (m, 3H), 5.19 (s, 2H), 4.50 - 4.44 (m, 1H), 3.96 (d, *J* = 11.8 Hz, 2H), 3.08 (d, *J =* 9.4 Hz, 2H), 3.02 (brs, 1H), 2.85 (s, 3H), 2.80 - 2.74 (m, 2H), 2.70 - 2.66 (m, 1H), 2.65 - 2.61 (m, 2H), 2.54 (s, 3H), 2.23 (t, *J =* 10.4 Hz, 2H), 1.96 (d, *J =* 10.8 Hz, 2H), 1.90 - 1.84 (m, 3H); **LRMS** (ES) m/z 619.3 (M⁺ + H).

### Example 159: Synthesis of Compound 159, 1-(4-(5-(difluoromethyl)-1,3,4-oxadiazole-2-yl)-2-fluorobenzyl)-5-(3-fluorophenyl)-3-(1-methylpip eridine-4-yl)-1,3-dihydro-2H-benzo[d]imidazole-2-one

### [Step 1] Synthesis of the compound 159

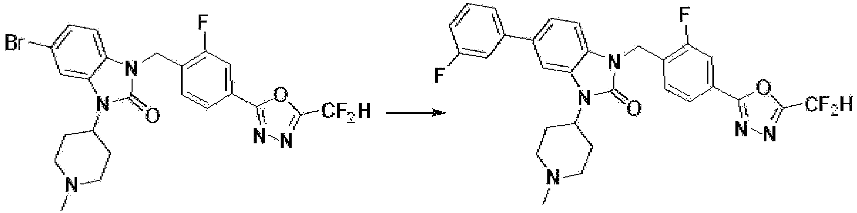

The 5-bromo-1-(4-(5-(difluoromethyl)-1,3,4-oxadiazole-2-yl)-2-fluorobenzyl)-3-(1-methylpiperidine-4-yl)-1,3-dihydro-2H-benzo[d]imidazole-2-one (0.100 g, 0.186 mmol) prepared in the step 3 of the compound 139, (3-fluorophenyl)boronic acid (0.031 g, 0.224 mmol), [1,1'-bis(di-tert-butylphosphino)ferrocene]palladium(II) dichloride (Pd(dtbpf)Cl₂, 0.006 g, 0.009 mmol) and cesium carbonate (0.182 g, 0.559 mmol) were mixed in 1,4-dioxane (3 mL)/water (1 mL) at room temperature, after which the resulting mixture was irradiated with microwave, then heated at 100°C for 20 minutes, and then a reaction was finished by lowering a temperature to room temperature. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.047 g, 4.5%) in a brown solid form.

**¹H NMR** (400 MHz, CDCl₃) δ 7.89 - 7.85 (m, 2H), 7.51 - 7.48 (m, 2H), 7.42 - 7.37 (m, 1H), 7.33 (d, *J =* 7.8 Hz, 1H), 7.27 - 7.22 (m, 2H), 7.06 - 6.80 (m, 3H), 5.24 (s, 2H), 4.53 - 4.47 (m, 1H), 3.08 (d, *J =* 11.5 Hz, 2H), 2.57 (q, *J =* 11.1 Hz, 2H), 2.39 (s, 3H), 2.23 (t, *J =* 11.6 Hz, 2H), 1.91 (d, *J =* 10.1 Hz, 2H); **LRMS** (ES) m/z 552.3 (M⁺ + H).

### Example 160: Synthesis of Compound 160, 1-(4-(5-(difluoromethyl)-1,3,4-oxadiazole-2-yl)-2-fluorobenzyl)-5-(2-fluorophenyl)-3-(1-methylpip eridine-4-yl)-1,3-dihydro-2H-benzo[d]imidazole-2-one

### [Step 1] Synthesis of the compound 160

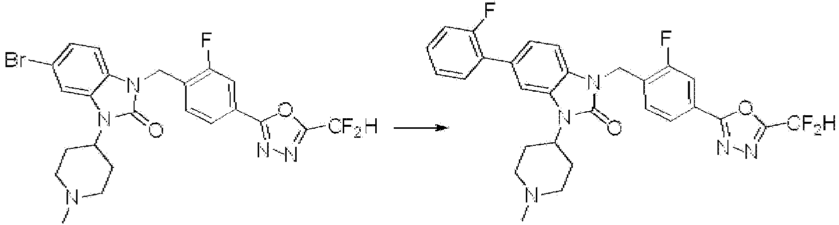

The 5-bromo-1-(4-(5-(difluoromethyl)-1,3,4-oxadiazole-2-yl)-2-fluorobenzyl)-3-(1-methylpiperidine-4-yl)-1,3-dihydro-2H-benzo[d]imidazole-2-one (0.100 g, 0.186 mmol) prepared in the step 3 of the compound 139, (2-fluorophenyl)boronic acid (0.031 g, 0.224 mmol), [1,1'-bis(di-tert-butylphosphino)ferrocene]palladium(II) dichloride (Pd(dtbpf)Cl₂, 0.006 g, 0.009 mmol) and cesium carbonate (0.182 g, 0.559 mmol) were mixed in 1,4-dioxane (3 mL)/water (1 mL) at room temperature, after which the resulting mixture was irradiated with microwave, then heated at 100°C for 20 minutes, and then a reaction was finished by lowering a temperature to room temperature. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.054 g, 52.5%) in a brown solid form.

**¹H NMR** (400 MHz, CDCl₃) δ 7.89 - 7.85 (m, 2H), 7.52 - 7.49 (m, 2H), 7.41 (td, *J* = 7.7, 1.7 Hz, 1H), 7.35 7.30 (m, 1H), 7.24 - 7.12 (m, 3H), 7.05 - 6.80 (m, 2H), 5.24 (s, 2H), 4.52 - 4.45 (m, 1H), 3.06 (d, *J* = 11.5 Hz, 2H), 2.59 - 2.51 (m, 2H), 2.37 (s, 3H), 2.21 (t, *J* = 11.4 Hz, 2H), 1.90 (d, *J =* 10.2 Hz, 2H); **LRMS** (ES) m/z 552.2 (M⁺ + H).

### Example 161: Synthesis of Compound 161, 1-(4-(5-(difluoromethyl)-1,3,4-oxadiazole-2-yl)-2-fluorobenzyl)-3-(1-methylpiperidine-4-yl)-5-(pyri dine-4-yl)-1,3-dihydro-2H-benzo[d]imidazole-2-one

### [Step 1] Synthesis of the compound 161

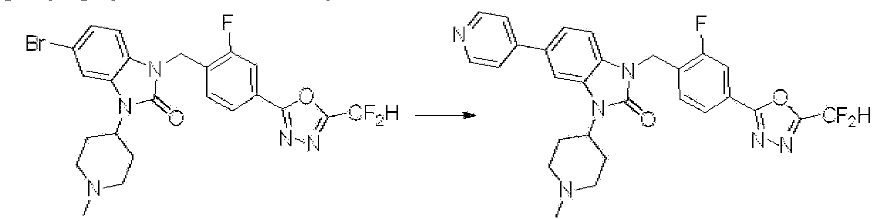

The 5-bromo-1-(4-(5-(difluoromethyl)-1,3,4-oxadiazole-2-yl)-2-fluorobenzyl)-3-(1-methylpiperidine-4-yl)-1,3-dihydro-2H-benzo[d]imidazole-2-one (0.150 g, 0.280 mmol) prepared in the step 3 of the compound 139, pyridine-4-ylboronic acid (0.041 g, 0.336 mmol), [1,1'-bis(di-tert-butylphosphino)ferrocene]palladium(II) dichloride (Pd(dtbpf)Cl₂, 0.009 g, 0.014 mmol) and cesium carbonate (0.273 g, 0.839 mmol) were mixed in 1,4-dioxane (3 mL)/water (1 mL) at room temperature, after which the resulting mixture was irradiated with microwave, then heated at 100°C for 20 minutes, and then a reaction was finished by lowering a temperature to room temperature. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.034 g, 22.7%) in a brown solid form.

**¹H NMR** (400 MHz, CDCl₃) δ 8.64 (d, *J* = 3.0 Hz, 2H), 7.88 - 7.84 (m, 2H), 7.56 (s, 1H), 7.51 - 7.47 (m, 3H), 7.33 (dd, *J* = 8.2, 1.4 Hz, 1H), 7.08 - 6.79 (m, 2H), 5.30 (s, 2H), 4.51 - 4.47 (m, 1H), 3.08 (d, *J* = 11.1 Hz, 2H), 2.58 - 2.56 (m, 2H), 2.39 (s, 3H), 2.23 (t, *J* = 11.4 Hz, 2H), 1.91 (d, *J =* 10.1 Hz, 2H); **LRMS** (ES) m/z 535.3 (M⁺ + H).

### Example 162: Synthesis of Compound 162, 1-(4-(5-(difluoromethyl)-1,3,4-oxadiazole-2-yl)-2-fluorobenzyl)-5-(furan-3-yl)-3-(1-methylpiperidin e-4-yl)-1,3-dihydro-2H-benzo[d]imidazole-2-one

### [Step 1] Synthesis of the compound 162

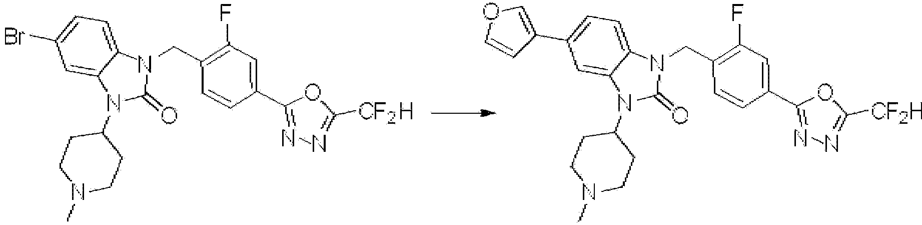

The 5-bromo-1-(4-(5-(difluoromethyl)-1,3,4-oxadiazole-2-yl)-2-fluorobenzyl)-3-(1-methylpiperidine-4-yl)-1,3-dihydro-2H-benzo[d]imidazole-2-one (0.100 g, 0.186 mmol) prepared in the step 3 of the compound 139, furan-3-ylboronic acid (0.025 g, 0.224 mmol), [1,1'-bis(di-tert-butylphosphino)ferrocene]palladium(II) dichloride (Pd(dtbpf)Cl₂, 0.006 g, 0.009 mmol) and cesium carbonate (0.182 g, 0.559 mmol) were mixed in 1,4-dioxane (3 mL)/water (1 mL) at room temperature, after which the resulting mixture was irradiated with microwave, then heated at 100°C for 20 minutes, and then a reaction was finished by lowering a temperature to room temperature. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.042 g, 43.0%) in a light brown solid form.

**¹H NMR** (400 MHz, CDCl₃) δ 7.95 - 7.88 (m, 3H), 7.69 (d, *J =* 1.3 Hz, 1H), 7.56 (t, *J =* 1.7 Hz, 1H), 7.46 (t, *J =* 7.8 Hz, 1H), 7.35 - 7.09 (m, 3H), 6.90 (d, *J* = 1.0 Hz, 1H), 5.28 (s, 2H), 4.50 - 4.44 (m, 1H), 3.10 (d, *J =* 11.8 Hz, 2H), 2.69 - 2.59 (m, 2H), 2.42 (s, 3H), 2.30 (td, *J =* 12.2, 2.1 Hz, 2H), 1.86 (dd, *J =* 12.5, 2.5 Hz, 2H); **LRMS** (ES) m/z 524.2 (M⁺ + H).

### Example 163: Synthesis of Compound 163, 1-(4-(5-(difluoromethyl)-1,3,4-oxadiazole-2-yl)-2-fluorobenzyl)-5-(5-methylfuran-2-yl)-3-(1-methyl piperidine-4-yl)-1,3-dihydro-2H-benzo[d]imidazole-2-one

### [Step 1] Synthesis of the compound 163

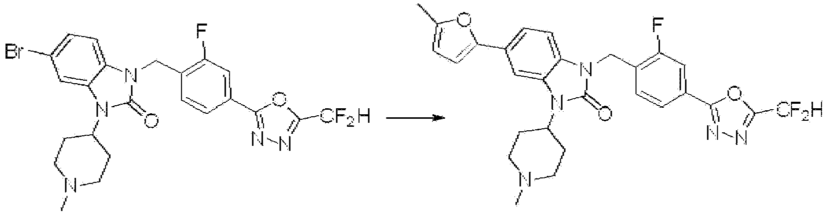

The 5-bromo-1-(4-(5-(difluoromethyl)-1,3,4-oxadiazole-2-yl)-2-fluorobenzyl)-3-(1-methylpiperidine-4-yl)-1,3-dihydro-2H-benzo[d]imidazole-2-one (0.100 g, 0.186 mmol) prepared in the step 3 of the compound 139, 4,4,5,5-tetramethyl-2-(5-methylfuran-2-yl)-1,3,2-dioxaborolane (0.047 g, 0.224 mmol), [1,1'-bis(di-tert-butylphosphino)ferrocene]palladium(II) dichloride (Pd(dtbpf)Cl₂, 0.006 g, 0.009 mmol) and cesium carbonate (0.182 g, 0.559 mmol) were mixed in 1,4-dioxane (3 mL)/water (1 mL) at room temperature, after which the resulting mixture was irradiated with microwave, then heated at 100°C for 20 minutes, and then a reaction was finished by lowering a temperature to room temperature. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.043 g, 42.9%) in a light brown solid form.

**¹H NMR** (400 MHz, CDCl₃) δ 7.87 - 7.82 (m, 2H), 7.56 (s, 1H), 7.45 (t, *J* = 7.8 Hz, 1H), 7.32 (dd, *J* = 8.2, 1.4 Hz, 1H), 7.05 - 6.79 (m, 2H), 6.51 - 6.50 (m, 1H), 6.05 - 6.04 (m, 1H), 5.20 (s, 2H), 4.49 - 4.43 (m, 1H), 3.10 (d, *J* = 11.2 Hz, 2H), 2.61 - 2.56 (m, 2H), 2.41 - 2.38 (m, 6H), 2.25 (t, *J =* 11.4 Hz, 2H), 1.89 (d, *J* = 10.4 Hz, 2H); **LRMS** (ES) m/z 538.2 (M⁺ + H).

### Example 164: Synthesis of Compound 164, 1-(4-(5-(difluoromethyl)-1,3,4-oxadiazole-2-yl)-2-fluorobenzyl)-5-(6-methoxypyridine-3-yl)-3-(1-m ethylpiperidine-4-yl)-1,3-dihydro-2H-benzo[d]imidazole-2-one

### [Step 1] Synthesis of the compound 164

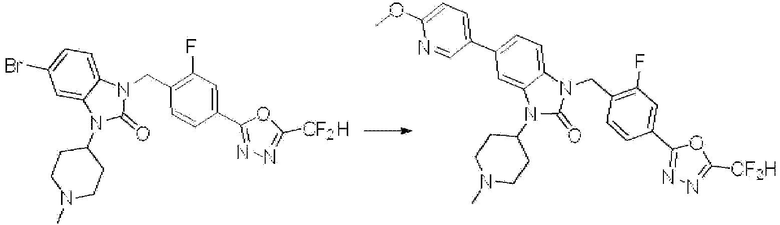

The 5-bromo-1-(4-(5-(difluoromethyl)-1,3,4-oxadiazole-2-yl)-2-fluorobenzyl)-3-(1-methylpiperidine-4-yl)-1,3-dihydro-2H-benzo[d]imidazole-2-one (0.100 g, 0.186 mmol) prepared in the step 3 of the compound 139, (6-methoxypyridine-3-yl)boronic acid (0.034 g, 0.224 mmol), [1,1'-bis(di-tert-butylphosphino)ferrocene]palladium(II) dichloride (Pd(dtbpf)Cl₂, 0.006 g, 0.009 mmol) and cesium carbonate (0.182 g, 0.559 mmol) were mixed in 1,4-dioxane (3 mL)/water (1 mL) at room temperature, after which the resulting mixture was irradiated with microwave, then heated at 100°C for 20 minutes, and then a reaction was finished by lowering a temperature to room temperature. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.013 g, 12.4%) in a light brown solid form.

**¹H NMR** (400 MHz, CDCl₃) δ 8.32 (d, *J =* 2.5 Hz, 1H), 7.87 - 7.83 (m, 2H), 7.75 (dd, *J* = 8.6, 2.5 Hz, 1H), 7.48 (t, *J = 7.8* Hz, 1H), 7.43 (s, 1H), 7.18 (dd, *J =* 8.1, 1.5 Hz, 1H), 7.05 - 6.79 (m, 3H), 5.23 (s, 2H), 4.51 - 4.44 (m, 1H), 3.96 (s, 3H), 3.05 (d, *J =* 11.5 Hz, 2H), 2.59 - 2.51 (m, 2H), 2.37 (s, 3H), 2.20 (t, *J =* 11.4 Hz, 2H), 1.89 (d, *J =* 11.9 Hz, 2H); **LRMS** (ES) m/z 565.2 (M⁺ + H).

### Example 165: Synthesis of Compound 165, 1-((5-(5-(difluoromethyl)-1,3,4-oxadiazole-2-yl)pyridine-2-yl)methyl)-6-fluoro-3-methyl-5-(pyridine -3-yl)-1,3-dihydro-2H-benzo[d]imidazole-2-one

### [Step 1] Synthesis of the compound 165

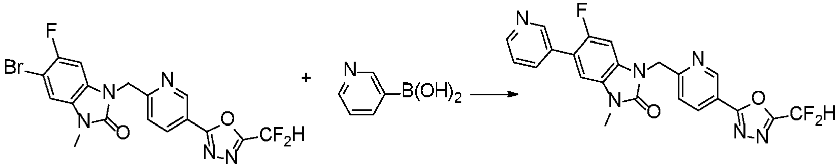

The 5-bromo-1-((5-(5-(difluoromethyl)-1,3,4-oxadiazole-2-yl)pyridine-2-yl)methyl)-6-fluoro-3-methyl-1 ,3-dihydro-2H-benzo[d]imidazole-2-one (0.100 g, 0.220 mmol) prepared in the step 4 of the compound 147, pyridine-3-ylboronic acid (0.032 g, 0.264 mmol), [1,1'-bis(di-tert-butylphosphino)ferrocene]palladium dichloride (0.007 g, 0.011 mmol) and cesium carbonate (0.143 g, 0.440 mmol) were mixed in 1,4-dioxane (1.7 mL)/water (0.6 mL) at room temperature, after which the resulting mixture was irradiated with microwave, then heated at 100°C for 30 minutes, and then a reaction was finished by lowering a temperature to room temperature. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 10%) and concentrated to obtain a title compound (0.007 g, 7.4%) in a brown solid form.

**¹H NMR** (400 MHz, CDCl₃) δ 9.30 (d, *J =* 1.5 Hz, 1H), 8.77 (s, 1H), 8.60 (d, *J =* 3.5 Hz, 1H), 8.37 (dd, *J* = 8.2, 2.2 Hz, 1H), 7.85 (d, *J = 8.3* Hz, 1H), 7.49 (d, *J = 8.4* Hz, 1H), 7.37 (dd, *J* = 7.7, 4.7 Hz, 1H), 7.06 - 6.80 (m, 3H), 5.30 (s, 2H), 3.51 (s, 3H); LRMS (ES) m/z 453.3 (M⁺ + 1).

### Example 166: Synthesis of Compound 166, 1-((5-(5-(difluoromethyl)-1,3,4-oxadiazole-2-yl)pyridine-2-yl)methyl)-6-fluoro-5-(3-fluorophenyl)-3 -methyl-1,3-dihydro-2H-benzo[d]imidazole-2-one

### [Step 1] Synthesis of the compound 166

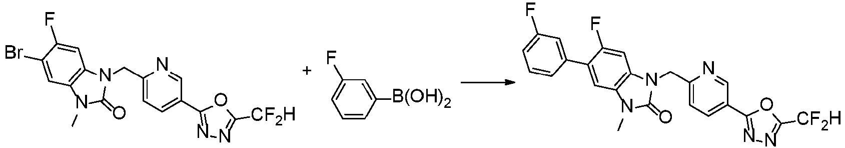

The 5-bromo-1-((5-(5-(difluoromethyl)-1,3,4-oxadiazole-2-yl)pyridine-2-yl)methyl)-6-fluoro-3-methyl-1 ,3-dihydro-2H-benzo[d]imidazole-2-one (0.100 g, 0.220 mmol) prepared in the step 4 of the compound 147, (3-fluorophenyl)boronic acid (0.037 g, 0.264 mmol), [1,1'-bis(di-tert-butylphosphino)ferrocene]palladium dichloride (0.007 g, 0.011 mmol) and cesium carbonate (0.143 g, 0.440 mmol) were mixed in 1,4-dioxane (1.7 mL)/water (0.6 mL) at room temperature, after which the resulting mixture was irradiated with microwave, then heated at 100°C for 30 minutes, and then a reaction was finished by lowering a temperature to room temperature. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; ethyl acetate/hexane = 50 to 100%) and concentrated to obtain a title compound (0.018 g, 17.4%) in a brown solid form.

**¹H NMR** (400 MHz, CDCl₃) δ 9.30 (d, *J =* 2.2 Hz, 1H), 8.36 (dd, *J =* 8.2, 2.2 Hz, 1H), 7.48 (d, *J = 8.4* Hz, 1H), 7.42 - 7.36 (m, 1H), 7.30 - 7.20 (m, 2H), 7.07 - 6.80 (m, 4H), 5.28 (s, 2H), 3.49 (s, 3H); **LRMS** (ES) m/z 470.1 (M⁺+ 1).

### Example 167: Synthesis of Compound 167, 1-((5-(5-(difluoromethyl)-1,3,4-oxadiazole-2-yl)pyridine-2-yl)methyl)-6-fluoro-3-methyl-5-(4-(triflu oromethyl)phenyl)-1,3-dihydro-2H-benzo[d]imidazole-2-one

### [Step 1] Synthesis of the compound 167

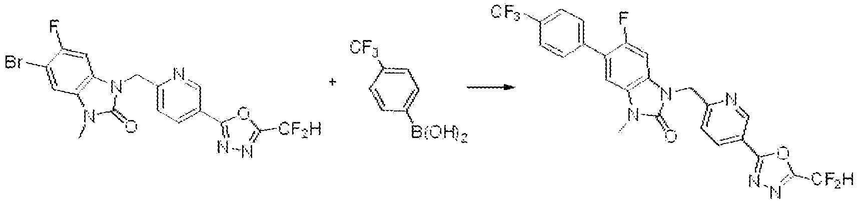

The 5-bromo-1-((5-(5-(difluoromethyl)-1,3,4-oxadiazole-2-yl)pyridine-2-yl)methyl)-6-fluoro-3-methyl-1 ,3-dihydro-2H-benzo[d]imidazole-2-one (0.100 g, 0.220 mmol) prepared in the step 4 of the compound 147, (4-(trifluoromethyl)phenyl)boronic acid (0.050 g, 0.264 mmol), [1,1'-bis(di-tert-butylphosphino)ferrocene]palladium dichloride (0.007 g, 0.011 mmol) and cesium carbonate (0.143 g, 0.440 mmol) were mixed in 1,4-dioxane (1.7 mL)/water (0.6 mL) at room temperature, after which the resulting mixture was irradiated with microwave, then heated at 100°C for 30 minutes, and then a reaction was finished by lowering a temperature to room temperature. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; ethyl acetate/hexane = 50 to 100%) and concentrated to obtain a title compound (0.019 g, 16.6%) in a brown solid form.

**¹H NMR** (400 MHz, CDCl₃) δ 9.30 (dd, *J* = 2.2, 0.7 Hz, 1H), 8.36 (dd, *J* = 8.2, 2.2 Hz, 1H), 7.69 (d, *J = 8.3* Hz, 2H), 7.62 (d, *J =* 8.0 Hz, 2H), 7.49 (dd, *J* = 8.2, 0.6 Hz, 1H), 7.05 - 6.80 (m, 3H), 5.29 (s, 2H), 3.50 (s, 3H); **LRMS** (ES) m/z 520.1 (M⁺ + 1).

### Example 168: Synthesis of Compound 168, 1-((5-(5-(difluoromethyl)-1,3,4-oxadiazole-2-yl)pyridine-2-yl)methyl)-6-fluoro-5-(furan-3-yl)-3-met hyl-1,3-dihydro-2H-benzo[d]imidazole-2-one

### [Step 1] Synthesis of the compound 168

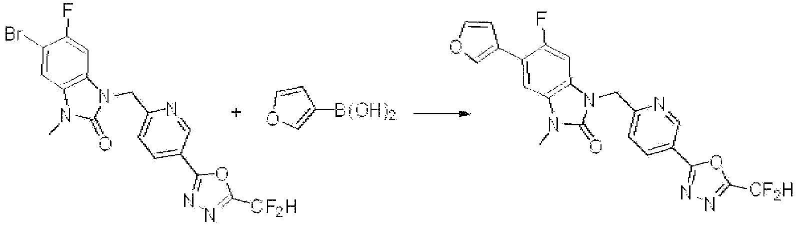

The 5-bromo-1-((5-(5-(difluoromethyl)-1,3,4-oxadiazole-2-yl)pyridine-2-yl)methyl)-6-fluoro-3-methyl-1 ,3-dihydro-2H-benzo[d]imidazole-2-one (0.100 g, 0.220 mmol) prepared in the step 4 of the compound 147, furan-3-ylboronic acid (0.030 g, 0.264 mmol), [1,1'-bis(di-tert-butylphosphino)ferrocene]palladium dichloride (0.007 g, 0.011 mmol) and cesium carbonate (0.143 g, 0.440 mmol) were mixed in 1,4-dioxane (1.7 mL) at room temperature, after which the resulting mixture was irradiated with microwave, then heated at 100°C for 30 minutes, and then a reaction was finished by lowering a temperature to room temperature. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; hexane/ethyl acetate = 50 to 100%) and concentrated to obtain a title compound (0.033 g, 33.7%) in a brown solid form.

**¹H NMR** (400 MHz, CDCl₃) δ 9.29 (dd, *J =* 2.2, 0.7 Hz, 1H), 8.34 (dd, *J* = 8.2, 2.2 Hz, 1H), 7.83 - 7.82 (m, 1H), 7.50 - 7.49 (m, 1H), 7.45 (d, *J* = 8.2 Hz, 1H), 7.05 - 6.74 (m, 4H), 5.26 (s, 2H), 3.50 (s, 3H); LRMS (ES) m/z 442.0 (M⁺+ 1).

### Example 169: Synthesis of Compound 169, 1-(4-(5-(difluoromethyl)-1,3,4-oxadiazole-2-yl)-2-fluorobenzyl)-6-fluoro-3-methyl-5-(pyridine-3-yl) -1,3-dihydro-2H-benzo[d]imidazole-2-one

### [Step 1] Synthesis of the compound 169

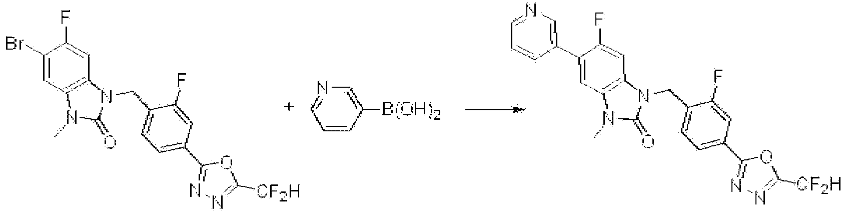

The 5-bromo-1-(4-(5-(difluoromethyl)-1,3,4-oxadiazole-2-yl)-2-fluorobenzyl)-6-fluoro-3-methyl-1,3-dih ydro-2H-benzo[d]imidazole-2-one (0.150 g, 0.318 mmol) prepared in the step 1 of the compound 148, pyridine-3-ylboronic acid (0.047 g, 0.382 mmol), [1,1'-bis(di-tert-butylphosphino)ferrocene]palladium dichloride (0.010 g, 0.016 mmol) and cesium carbonate (0.207 g, 0.637 mmol) were mixed in 1,4-dioxane (1.7 mL)/water (0.6 mL) at room temperature, after which the resulting mixture was irradiated with microwave, then heated at 100°C for 30 minutes, and then a reaction was finished by lowering a temperature to room temperature. Saturated sodium hydrogen carbonate aqueous solution was poured into the reaction mixture and an extraction was performed with dichloromethane. An organic layer was washed with saturated sodium chloride aqueous solution, then dehydrated with anhydrous magnesium sulfate, then filtered, and then concentrated under reduced pressure. The resulting concentrate was purified via column chromatography (SiO₂, 4 g cartridge; methanol/dichloromethane = 0 to 5%) and concentrated to obtain a title compound (0.107 g, 71.3%) in a brown solid form.

**¹H NMR** (400 MHz, CDCl₃) δ 8.75 (s, 1H), 8.58 (dd, *J* = 4.8, 1.5 Hz, 1H), 7.87 - 7.81 (m, 3H), 7.52 (t, *J = 7.5* Hz, 1H), 7.37 - 7.34 (m, 1H), 6.99 (d, *J = 6.2* Hz, 1H), 6.89 (t, *J* = 51.7 Hz, 1H), 6.84 (d, *J* = 9.8 Hz, 1H), 5.20 (s, 2H), 3.49 (s, 3H); LRMS (ES) m/z 469.8 (M⁺ + 1).

### Example 170: Synthesis of Compound 170, 1-(4-(5-(difluoromethyl)-1,3,4-oxadiazole-2-yl)-2-fluorobenzyl)-6-fluoro-3-methyl-5-(5-methylfura n-2-yl)-1,3-dihydro-2H-benzo[d]imidazole-2-one

### [Step 1] Synthesis of the compound 170

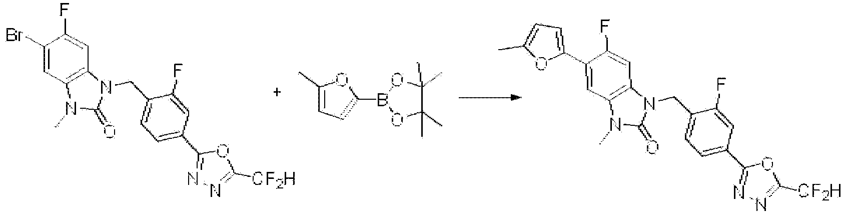

The 5-bromo-1-(4-(5-(difluoromethyl)-1,3,4-oxadiazole-2-yl)-2-fluorobenzyl)-6-fluoro-3-methyl-1,3-dih ydro-2H-benzo[d]imidazole-2-one (0.150 g, 0.318 mmol) prepared in the step 1 of the compound 148, 4,4,5,5-tetramethyl-2-(5-methylfuran-2-yl)-1,3,2-dioxaborolane (0.079 g, 0.382 mmol), [1,1'-bis(di-tert-butylphosphino)ferrocene]palladium dichloride (0.010 g, 0.016 mmol) and cesium carbonate (0.207 g, 0.637 mmol) were mixed in 1,4-dioxane (1.7 mL)/water (0.6 mL) at room temperature, after which the resulting mixture was irradiated with microwave, then heated at 100°C for 30 minutes, and then a reaction was finished by lowering a temperature to room temperature. Saturated sodium hydrogen carbonate aqueous solution was poured into the reaction mixture and an extraction was performed with dichloromethane. An organic layer was washed with saturated sodium chloride aqueous solution, then dehydrated with anhydrous magnesium sulfate, then filtered, and then concentrated under reduced pressure. The resulting concentrate was purified via column chromatography (SiO₂, 4 g cartridge; ethyl acetate/hexane = 30 to 100%) and concentrated to obtain a title compound (0.098 g, 65.3%) in a white solid form.

**¹H NMR** (400 MHz, CDCl₃) δ 7.88 - 7.84 (m, 2H), 7.51 - 7.47 (m, 1H), 7.36 (d, *J* = 6.1 Hz, 1H), 7.03 - 6.73 (m, 2H), 6.64 (t, *J =* 3.6 Hz, 1H), 6.10 - 6.09 (m, 1H), 5.16 (s, 2H), 3.51 (s, 3H), 2.39 (d, *J* = 0.5 Hz, 3H); LRMS (ES) m/z 473.2 (M⁺ + 1).

### Example 171: Synthesis of Compound 171, 1-(1-acetylpiperidine-4-yl)-3-((5-(5-(difluoromethyl)-1,3,4-oxadiazole-2-yl)pyridine-2-yl)methyl)-4-fluoro-1,3-dihydro-2H-benzo[d]imidazole-2-one

### [Step 1] Synthesis of the compound 171

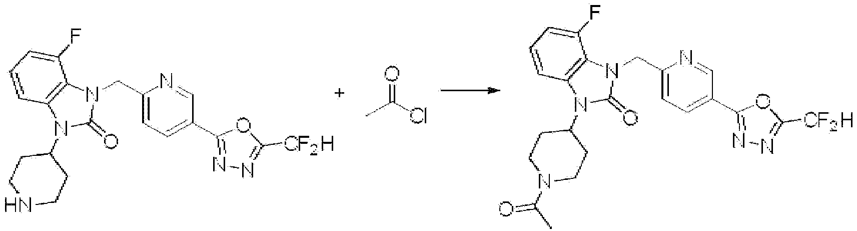

The 3-((5-(5-(difluoromethyl)-1,3,4-oxadiazole-2-yl)pyridine-2-yl)methyl)-4-fluoro-1-(piperidine-4-yl)-1 ,3-dihydro-2H-benzo[d]imidazole-2-one (0.100 g, 0.225 mmol) prepared in the step 5 of the compound 76, triethylamine (0.063 mL, 0.450 mmol) and acetyl chloride (0.032 mL, 0.450 mmol) were dissolved in dichloromethane (2 mL) at room temperature, after which the resulting solution was stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.060 g, 54.8%) in a yellow solid form.

**¹H NMR** (400 MHz, CDCl₃) δ 9.22 (d, *J =* 1.6 Hz, 1H), 8.32 (dd, *J* = 8.2, 2.2 Hz, 1H), 7.33 (d, *J = 8.3* Hz, 1H), 7.04 - 6.73 (m, 4H), 5.41 (s, 2H), 4.88 - 4.85 (m, 1H), 4.60 - 4.52 (m, 1H), 4.02 - 3.99 (m, 1H), 3.26 - 3.20 (m, 1H), 2.70 - 2.63 (m, 1H), 2.41 - 2.26 (m, 2H), 2.16 (s, 3H), 1.97 - 1.90 (m, 2H); **LRMS** (ES) m/z 487.0 (M⁺ + 1).

### Example 172: Synthesis of Compound 172, 1-(1-acetylpiperidine-4-yl)-3-(4-(5-(difluoromethyl)-1,3,4-oxadiazole-2-yl)benzyl)-5-fluoro-1,3-dih ydro-2H-benzo[d]imidazole-2-one

### [Step 1] Synthesis of the compound 172

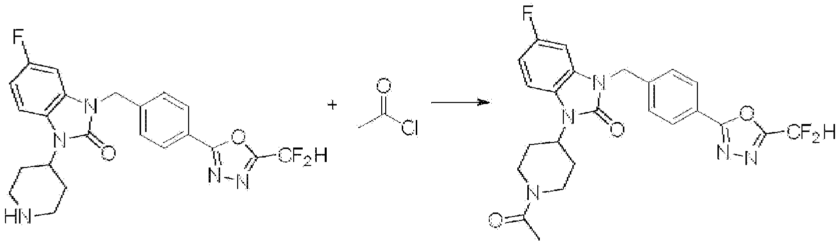

The 3-(4-(5-(difluoromethyl)-1,3,4-oxadiazole-2-yl)benzyl)-5-fluoro-1-(piperidine-4-yl)-1,3-dihydro-2H -benzo[d]imidazole-2-one (0.100 g, 0.226 mmol) prepared in the step 2 of the compound 79, triethylamine (0.063 mL, 0.451 mmol) and acetyl chloride (0.032 mL, 0.451 mmol) were dissolved in dichloromethane (2 mL) at room temperature, after which the resulting solution was stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.097 g, 88.5%) in a yellow solid form.

**¹H NMR** (400 MHz, CDCl₃) δ 8.05 (dd, *J* = 6.7, 1.7 Hz, 2H), 7.45 (d, *J =* 8.4 Hz, 2H), 7.02 - 6.72 (m, 3H), 6.59 (dd, *J* = 8.3, 2.4 Hz, 1H), 5.27 (s, 2H), 4.87 - 4.84 (m, 1H), 4.58 - 4.50 (m, 1H), 4.00 (d, *J =* 13.6 Hz, 1H), 3.26 - 3.19 (m, 1H), 2.69 - 2.63 (m, 1H), 2.37 - 2.22 (m, 2H), 2.15 (s, 3H), 1.95 - 1.88 (m, 2H); **LRMS** (ES) m/z 486.1 (M⁺+ 1).

### Example 173: Synthesis of Compound 173, 1-(1-acetylpiperidine-4-yl)-3-((5-(5-(difluoromethyl)-1,3,4-oxadiazole-2-yl)pyridine-2-yl)methyl)-5, 6-difluoro-1,3-dihydro-2H-benzo[d]imidazole-2-one

### [Step 1] Synthesis of the compound 173

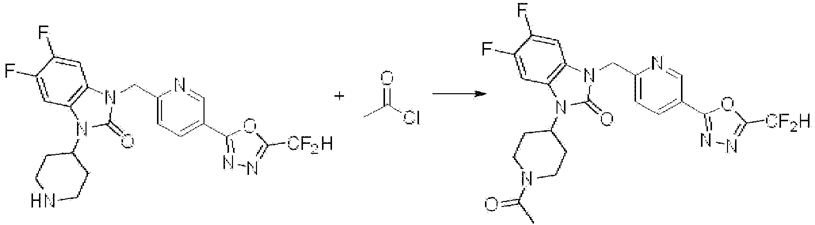

The 1-((5-(5-(difluoromethyl)-1,3,4-oxadiazole-2-yl)pyridine-2-yl)methyl)-5,6-difluoro-3-(piperidine-4-y I)-1,3-dihydro-2H-benzo[d]imidazole-2-one (0.100 g, 0.216 mmol) prepared in the step 4 of the compound 83, triethylamine (0.060 mL, 0.433 mmol) and acetyl chloride (0.031 mL, 0.433 mmol) were dissolved in dichloromethane (2 mL) at room temperature, after which the resulting solution was stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.052 g, 48.0%) in a white solid form.

**¹H NMR** (400 MHz, CDCl₃) δ 9.25 (dd, *J =* 2.2, 0.7 Hz, 1H), 8.34 (dd, *J* = 8.2, 2.2 Hz, 1H), 7.44 (d, *J =* 8.2 Hz, 1H), 7.05 - 6.79 (m, 3H), 5.19 (d, *J =* 1.9 Hz, 2H), 4.88 - 4.84 (m, 1H), 4.51 - 4.44 (m, 1H), 4.01 (dd, *J =* 13.8, 2.1 Hz, 1H), 3.26 - 3.19 (m, 1H), 2.69 - 2.62 (m, 1H), 2.34 - 2.18 (m, 2H), 2.15 (s, 3H), 1.95 - 1.87 (m, 2H); **LRMS** (ES) m/z 505.0 (M⁺+ 1).

### Example 174: Synthesis of Compound 174, 1-(1-acetylpiperidine-4-yl)-3-(4-(5-(difluoromethyl)-1,3,4-oxadiazole-2-yl)-2-fluorobenzyl)-5,6-difl uoro-1,3-dihydro-2H-benzo[d]imidazole-2-one

### [Step 1] Synthesis of the compound 174

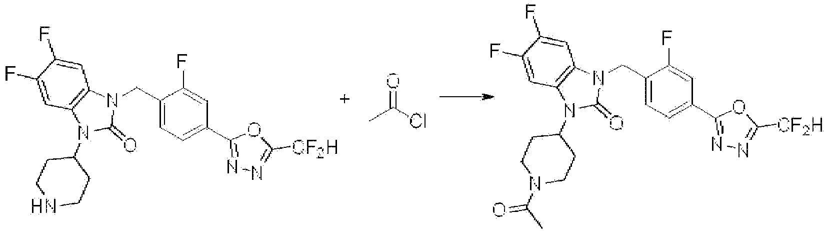

The 1-(4-(5-(difluoromethyl)-1,3,4-oxadiazole-2-yl)-2-fluorobenzyl)-5,6-difluoro-3-(piperidine-4-yl)-1,3 -dihydro-2H-benzo[d]imidazole-2-one (0.100 g, 0.209 mmol) prepared in the step 2 of the compound 93, triethylamine (0.058 mL, 0.417 mmol) and acetyl chloride (0.030 mL, 0.417 mmol) were dissolved in dichloromethane (2 mL) at room temperature, after which the resulting solution was stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.099 g, 91.1%) in a yellow solid form.

**¹H NMR** (400 MHz, CDCl₃) δ 7.85 - 7.81 (m, 2H), 7.45 (t, *J* = 7.7 Hz, 1H), 7.03 - 6.77 (m, 3H), 5.10 (s, 2H), 4.87 - 4.84 (m, 1H), 4.51 - 4.43 (m, 1H), 4.02 - 3.99 (m, 1H), 3.24 - 3.18 (m, 1H), 2.67 - 2.61 (m, 1H), 2.35 - 2.17 (m, 2H), 2.14 (s, 3H), 1.93 - 1.86 (m, 2H); **LRMS** (ES) m/z 521.9 (M⁺ + 1).

### Example 175: Synthesis of Compound 175, 1-(1-acetylpiperidine-4-yl)-3-(4-(5-(difluoromethyl)-1,3,4-oxadiazole-2-yl)benzyl)-5,6-difluoro-1,3-dihydro-2H-benzo[d]imidazole-2-one

### [Step 1] Synthesis of the compound 175

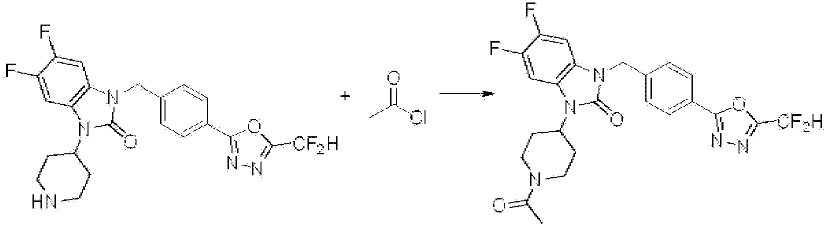

The 1-(4-(5-(difluoromethyl)-1,3,4-oxadiazole-2-yl)benzyl)-5,6-difluoro-3-(piperidine-4-yl)-1,3-dihydro -2H-benzo[d]imidazole-2-one (0.100 g, 0.217 mmol) prepared in the step 2 of the compound 95, triethylamine (0.060 mL, 0.433 mmol) and acetyl chloride (0.031 mL, 0.433 mmol) were dissolved in dichloromethane (2 mL) at room temperature, after which the resulting solution was stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.079 g, 72.4%) in a white solid form.

**¹H NMR** (400 MHz, CDCl₃) δ 8.07 (d, *J =* 8.3 Hz, 2H), 7.43 (d, *J =* 8.3 Hz, 2H), 6.96 (dd, *J =* 10.1, 6.6 Hz, 1H), 6.90 (t, *J =* 51.7 Hz, 1H), 6.66 (dd, *J* = 9.5, 6.8 Hz, 1H), 5.05 (s, 2H), 4.87 (d, *J = 13.6* Hz, 1H), 4.54 - 4.45 (m, 1H), 4.01 (d, *J* = 13.8 Hz, 1H), 3.26 - 3.19 (m, 1H), 2.69 - 2.63 (m, 1H), 2.35 - 2.19 (m, 2H), 2.16 (s, 3H), 1.95 - 1.88 (m, 2H); **LRMS** (ES) m/z 504.1 (M⁺+ 1).

### Example 176: Synthesis of Compound 176, 1-(1-acetylpiperidine-4-yl)-3-((5-(5-(difluoromethyl)-1,3,4-oxadiazole-2-yl)pyridine-2-yl)methyl)-5-(trifluoromethyl)-1,3-dihydro-2H-benzo[d]imidazole-2-one

### [Step 1] Synthesis of the compound 176

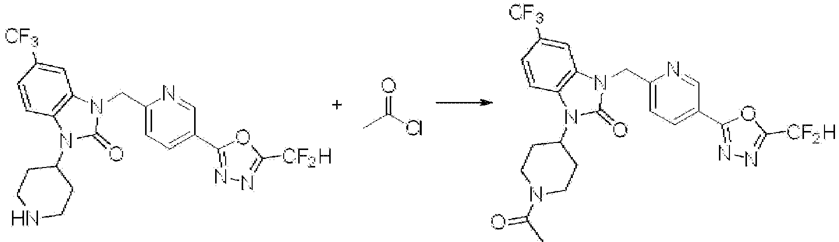

The 3-((5-(5-(difluoromethyl)-1,3,4-oxadiazole-2-yl)pyridine-2-yl)methyl)-1-(piperidine-4-yl)-5-(trifluor omethyl)-1,3-dihydro-2H-benzo[d]imidazole-2-one (0.100 g, 0.202 mmol) prepared in the step 5 of the compound 89, triethylamine (0.056 mL, 0.405 mmol) and acetyl chloride (0.029 mL, 0.405 mmol) were dissolved in dichloromethane (2 mL) at room temperature, after which the resulting solution was stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.083 g, 76.1%) in a white solid form.

**¹H NMR** (400 MHz, CDCl₃) δ 9.27 (dd, *J =* 2.2, 0.7 Hz, 1H), 8.35 (dd, *J* = 8.2, 2.2 Hz, 1H), 7.45 (dd, *J =* 6.0, 6.5 Hz, 1H), 7.36 (dd, *J* = 8.3, 0.8 Hz, 1H), 7.29 (d, *J = 1.3* Hz, 1H), 7.19 (d, *J =* 8.3 Hz, 1H), 6.92 (t, *J* = 51.6 Hz, 1H), 5.29 (s, 2H), 4.89 (dd, *J =* 11.5, 2.0 Hz, 1H), 4.62 - 4.54 (m, 1H), 4.03 (dd, *J* = 12.0, 2.0 Hz, 1H), 3.28 - 3.21 (m, 1H), 2.68 (td, *J* = 13.1, 2.0 Hz, 1H), 2.41 - 2.26 (m, 2H), 2.17 (s, 3H), 1.97 - 1.91 (m, 2H); **LRMS** (ES) m/z 537.1 (M⁺+ 1).

### Example 177: Synthesis of Compound 177, 1-((5-(5-(difluoromethyl)-1,3,4-oxadiazole-2-yl)pyridine-2-yl)methyl)-6-fluoro-3-methyl-5-(5-meth ylfuran-2-yl)-1,3-dihydro-2H-benzo[d]imidazole-2-one

### [Step 1] Synthesis of the compound 177

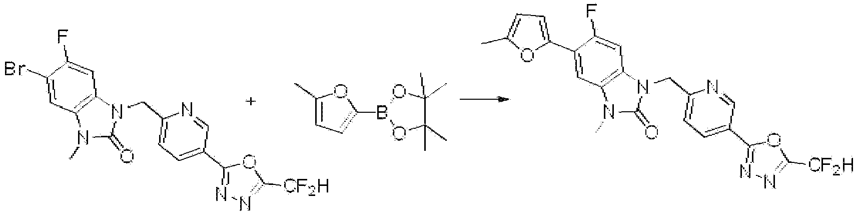

The 5-bromo-1-((5-(5-(difluoromethyl)-1,3,4-oxadiazole-2-yl)pyridine-2-yl)methyl)-6-fluoro-3-methyl-1 ,3-dihydro-2H-benzo[d]imidazole-2-one (0.120 g, 0.264 mmol) prepared in the step 4 of the compound 147, 4,4,5,5-tetramethyl-2-(5-methylfuran-2-yl)-1,3,2-dioxaborolane (0.066 g, 0.317 mmol), [1,1'-bis(di-tert-butylphosphino)ferrocene]palladium dichloride (0.009 g, 0.013 mmol) and cesium carbonate (0.172 g, 0.528 mmol) were mixed in 1,4-dioxane (2.3 mL)/water (0.6 mL) at room temperature, after which the resulting mixture was irradiated with microwave, then heated at 100°C for 30 minutes, and then a reaction was finished by lowering a temperature to room temperature. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; ethyl acetate/hexane = 30 to 100%) and concentrated to obtain a title compound (0.055 g, 45.8%) in a yellow solid form.

**¹H NMR** (400 MHz, CDCl₃) δ 9.27 (d, *J =* 1.6 Hz, 1H), 8.32 (dd, *J* = 8.2, 2.2 Hz, 1H), 7.44 (d, *J* = 8.2 Hz, 1H), 7.34 (d, *J = 6.1* Hz, 1H), 7.05 - 6.76 (m, 2H), 6.61 (t, *J =* 3.6 Hz, 1H), 6.08 - 6.07 (m, 1H), 5.23 (s, 2H), 3.50 (s, 3H), 2.38 (s, 3H); LRMS (ES) m/z 457.1 (M⁺ + 1).

### Example 178: Synthesis of Compound 178, 1-((5-(5-(difluoromethyl)-1,3,4-oxadiazole-2-yl)pyridine-2-yl)methyl)-5-(3,5-dimethylisoxazole-4-y l)-6-fluoro-3-methyl-1,3-dihydro-2H-benzo[d]imidazole-2-one

### [Step 1] Synthesis of the compound 178

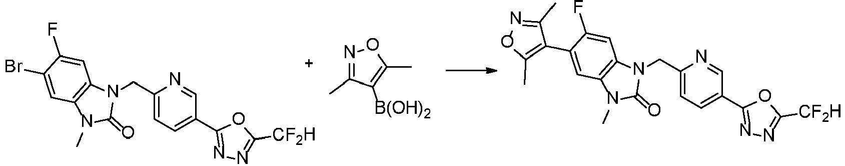

The 5-bromo-1-((5-(5-(difluoromethyl)-1,3,4-oxadiazole-2-yl)pyridine-2-yl)methyl)-6-fluoro-3-methyl-1 ,3-dihydro-2H-benzo[d]imidazole-2-one (0.120 g, 0.264 mmol) prepared in the step 4 of the compound 147, (3,5-dimethylisooxazole-4-yl)boronic acid (0.045 g, 0.317 mmol), [1,1'-bis(di-tert-butylphosphino)ferrocene]palladium dichloride (0.009 g, 0.013 mmol) and cesium carbonate (0.172 g, 0.528 mmol) were mixed in 1,4-dioxane (2.3 mL)/water (0.6 mL) at room temperature, after which the resulting mixture was irradiated with microwave, then heated at 100°C for 30 minutes, and then a reaction was finished by lowering a temperature to room temperature. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; ethyl acetate/hexane = 30 to 100%) and concentrated to obtain a title compound (0.044 g, 35.2%) in a white solid form.

**¹H NMR** (400 MHz, CDCl₃) δ 9.29 (d, *J =* 1.7 Hz, 1H), 8.36 (dd, *J* = 8.2, 2.2 Hz, 1H), 7.51 (d, *J =* 8.2 Hz, 1H), 7.05 - 6.79 (m, 2H), 6.74 (d, *J* = 5.8 Hz, 1H), 5.27 (s, 2H), 3.47 (s, 3H), 2.31 (s, 3H), 2.17 (s, 3H); **LRMS** (ES) m/z 471.2 (M⁺+ 1).

### Example 179: Synthesis of Compound 179, 1-(4-(5-(difluoromethyl)-1,3,4-oxadiazole-2-yl)-2-fluorobenzyl)-5-(1-(2-hydroxyacetyl)piperidine-4 -yl)-3-(1-methylpiperidine-4-yl)-1,3-dihydro-2H-benzo[d]imidazole-2-one

### [Step 1] Synthesis of the compound 179

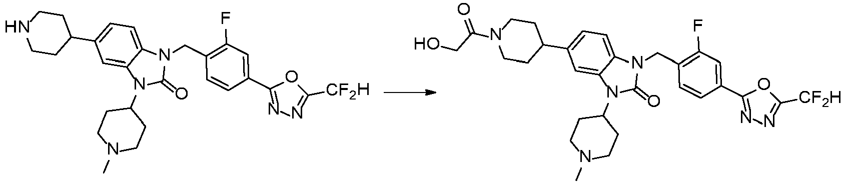

The 1-(4-(5-(difluoromethyl)-1,3,4-oxadiazole-2-yl)-2-fluorobenzyl)-3-(1-methylpiperidine-4-yl)-5-(pip eridine-4-yl)-1,3-dihydro-2H-benzo[d]imidazole-2-one (0.200 g, 0.370 mmol) prepared in the step 3 of the compound 156, 2-hydroxyacetic acid (0.034 g, 0.444 mmol), 1-[bis(dimethylamino)methylene]-1H-1,2,3-triazolo[4,5-b]pyridinium 3-oxide hexafluorophosphate (HATU, 0.281 g, 0.740 mmol) and N,N-diisopropylethylamine (0.322 mL, 1.850 mmol) were dissolved in N,N-dimethylformamide (5 mL) at room temperature, after which the resulting solution was stirred at the same temperature for 18 hours. Solvent was removed from the reaction mixture under reduced pressure, after which the resulting concentrate was purified via column chromatography (SiO₂, 4 g cartridge; methanol/dichloromethane = 0 to 10%) and concentrated to obtain a product. Then, the resulting product was purified again via chromatography (SiO₂, 4 g cartridge; methanol/dichloromethane = 0 to 10%) and concentrated to obtain a title compound (0.034 g, 15.4%) in a yellow oil form.

**¹H NMR** (400 MHz, CDCl₃) δ 7.86 - 7.82 (m, 2H), 7.46 (t, *J =* 7.8 Hz, 1H), 7.28 (brs, 1H), 7.05 - 6.79 (m, 3H), 5.18 (s, 2H), 4.76 (d, *J =* 13.3 Hz, 1H), 4.50 - 4.44 (m, 1H), 4.22 (q, *J* = 14.2 Hz, 2H), 3.63 (d, *J* = 13.5 Hz, 1H), 3.14 - 3.07 (m, 3H), 2.83 - 2.68 (m, 2H), 2.54 - 2.51 (m, 2H), 2.40 (s, 3H), 2.23 (t, *J =* 10.5 Hz, 2H), 1.93 - 1.85 (m, 4H), 1.72 - 1.61 (m, 2H); LRMS (ES) m/z 599.3 (M⁺ + H).

### Example 180: Synthesis of Compound 180, 1-(1-acetylpiperidine-4-yl)-3-(4-(5-(difluoromethyl)-1,3,4-oxadiazole-2-yl)-2-fluorobenzyl)-5-(triflu oromethyl)-1,3-dihydro-2H-benzo[d]imidazole-2-one

### [Step 1] Synthesis of the compound 180

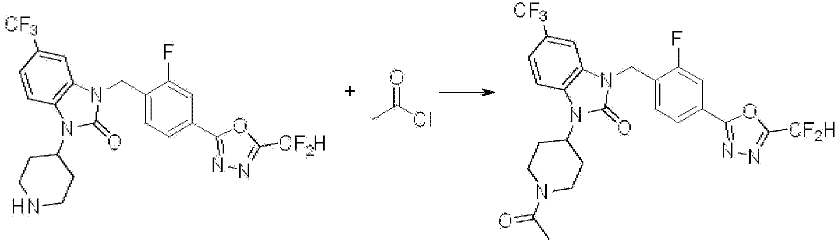

The 3-(4-(5-(difluoromethyl)-1,3,4-oxadiazole-2-yl)-2-fluorobenzyl)-1-(piperidine-4-yl)-5-(trifluorometh yl)-1,3-dihydro-2H-benzo[d]imidazole-2-one (0.100 g, 0.196 mmol) prepared in the step 2 of the compound 91, triethylamine (0.055 mL, 0.391 mmol) and acetyl chloride (0.028 mL, 0.391 mmol) were dissolved in dichloromethane (2 mL) at room temperature, after which the resulting solution was stirred at the same temperature for 18 hours. Saturated aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.093 g, 85.8%) in a white solid form.

¹H NMR (400 MHz, CDCl₃) δ 7.85 - 7.82 (m, 2H), 7.46 (t, *J* = 7.7 Hz, 1H), 7.35 (dd, *J* = 8.3, 0.8 Hz, 1H), 7.22 - 7.18 (m, 2H), 6.90 (t, *J =* 51.7 Hz, 1H), 5.19 (s, 2H), 4.89 - 4.85 (m, 1H), 4.60 - 4.52 (m, 1H), 4.03 - 4.00 (m, 1H), 3.27 - 3.20 (m, 1H), 2.67 (td, *J =* 13.9, 3.3 Hz, 1H), 2.40 - 2.24 (m, 2H), 2.15 (s, 3H), 1.92 (t, *J =* 14.5 Hz, 2H); LRMS (ES) m/z 554.2 (M⁺ + 1).

### Example 181: Synthesis of Compound 181, 3-(1-acetylpiperidine-4-yl)-5-chloro-1-((5-(5-(difluoromethyl)-1,3,4-oxadiazole-2-yl)pyridine-2-yl) methyl)-1,3-dihydro-2H-benzo[d]imidazole-2-one

### [Step 1] Synthesis of the compound 181

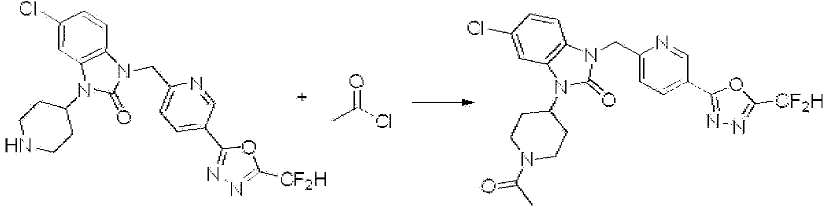

The 5-chloro-1-((5-(5-(difluoromethyl)-1,3,4-oxadiazole-2-yl)pyridine-2-yl)methyl)-3-(piperidine-4-yl)-1,3-dihydro-2H-benzo[d]imidazole-2-one (0.100 g, 0.217 mmol) prepared in the step 5 of the compound 97, triethylamine (0.060 mL, 0.434 mmol) and acetyl chloride (0.031 mL, 0.434 mmol) were dissolved in dichloromethane (2 mL) at room temperature, after which the resulting solution was stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.081 g, 73.8%) in a yellow solid form.

**¹H NMR** (400 MHz, CDCl₃) δ 9.23 (dd, *J =* 2.2, 0.7 Hz, 1H), 8.31 (dd, *J* = 8.2, 2.2 Hz, 1H), 7.39 (d, *J = 8.2* Hz, 1H), 7.09 (d, *J =* 1.8 Hz, 1H), 7.05 - 6.79 (m, 3H), 5.22 (d, *J* = 2.0 Hz, 2H), 4.86 (dd, *J* = 11.5, 2.1 Hz, 1H), 4.54 - 4.46 (m, 1H), 4.02 - 3.99 (m, 1H), 3.26 - 3.18 (m, 1H), 2.69 - 2.62 (m, 1H), 2.39 - 2.23 (m, 2H), 2.15 (s, 3H), 1.94 - 1.87 (m, 2H); LRMS (ES) m/z 503.1 (M⁺+ 1).

### Example 182: Synthesis of Compound 182, 3-(1-acetylpiperidine-4-yl)-5-chloro-1-(4-(5-(difluoromethyl)-1,3,4-oxadiazole-2-yl)-2-fluorobenzyl )-1,3-dihydro-2H-benzo[d]imidazole-2-one

### [Step 1] Synthesis of the compound 182

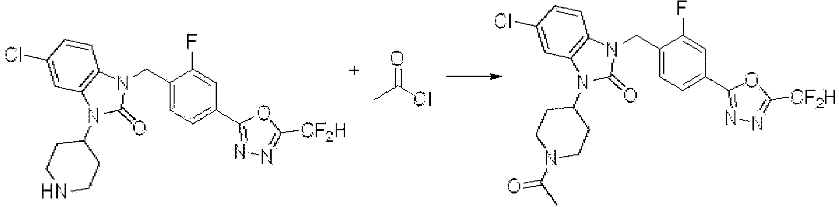

The 5-chloro-1-(4-(5-(difluoromethyl)-1,3,4-oxadiazole-2-yl)-2-fluorobenzyl)-3-(piperidine-4-yl)-1,3-di hydro-2H-benzo[d]imidazole-2-one (0.100 g, 0.209 mmol) prepared in the step 2 of the compound 101, triethylamine (0.058 mL, 0.419 mmol) and acetyl chloride (0.030 mL, 0.419 mmol) were dissolved in dichloromethane (2 mL) at room temperature, after which the resulting solution was stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.049 g, 44.6%) in a white solid form.

**¹H NMR** (400 MHz, CDCl₃) δ 7.82 (d, *J =* 8.6 Hz, 2H), 7.43 (t, *J = 7.5* Hz, 1H), 7.09 (d, *J* = 1.8 Hz, 1H), 7.03 - 6.77 (m, 3H), 5.14 (s, 2H), 4.90 - 4.85 (m, 1H), 4.54 - 4.46 (m, 1H), 4.03 - 4.00 (m, 1H), 3.23 (td, *J* = 13.2, 2.3 Hz, 1H), 2.70 - 2.63 (m, 1H), 2.39 - 2.22 (m, 2H), 2.17 (s, 3H), 1.94 - 1.87 (m, 2H); **LRMS** (ES) m/z 520.1 (M⁺ + 1).

### Example 183: Synthesis of Compound 183, 3-(1-acetylpiperidine-4-yl)-5-chloro-1-(4-(5-(difluoromethyl)-1,3,4-oxadiazole-2-yl)benzyl)-1,3-dih ydro-2H-benzo[d]imidazole-2-one

### [Step 1] Synthesis of the compound 183

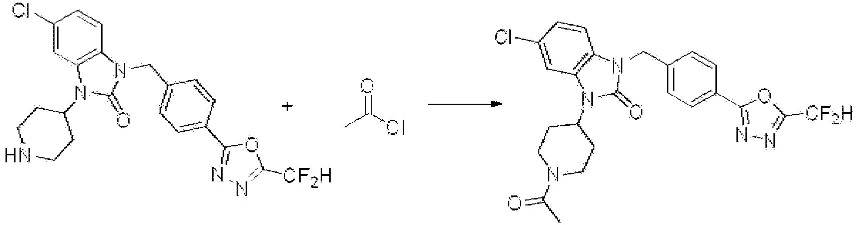

The 5-chloro-1-(4-(5-(difluoromethyl)-1,3,4-oxadiazole-2-yl)benzyl)-3-(piperidine-4-yl)-1,3-dihydro-2H -benzo[d]imidazole-2-one (0.100 g, 0.217 mmol) prepared in the step 2 of the compound 99, triethylamine (0.061 mL, 0.435 mmol) and acetyl chloride (0.031 mL, 0.435 mmol) were dissolved in dichloromethane (2 mL) at room temperature, after which the resulting solution was stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methaol/dichloromethane = 0 to 5%) and concentrated to obtain a title compound (0.084 g, 76.6%) in a white solid form.

**¹H NMR** (400 MHz, CDCl₃) δ 8.07 - 8.04 (m, 2H), 7.43 (d, *J* = 8.5 Hz, 2H), 7.10 (d, *J* = 1.8 Hz, 1H), 6.97 (dd, *J* = 8.4, 1.8 Hz, 1H), 6.89 (t, *J =* 51.7 Hz, 1H), 6.74 (d, *J =* 8.4 Hz, 1H), 5.10 (s, 2H), 4.90 - 4.86 (m, 1H), 4.55 - 4.48 (m, 1H), 4.04 - 4.00 (m, 1H), 3.23 (td, *J* = 13.2, 2.1 Hz, 1H), 2.70 - 2.64 (m, 1H), 2.40 - 2.23 (m, 2H), 2.17 (s, 3H), 1.96 - 1.88 (m, 2H); **LRMS** (ES) m/z 502.2 (M⁺+ 1).

### Example 184: Synthesis of Compound 184, 1-(1-acetylpiperidine-4-yl)-3-((5-(5-(difluoromethyl)-1,3,4-oxadiazole-2-yl)pyridine-2-yl)methyl)-1, 3-dihydro-2H-imidazo[4,5-b]pyridine-2-one

### [Step 1] Synthesis of the compound 184

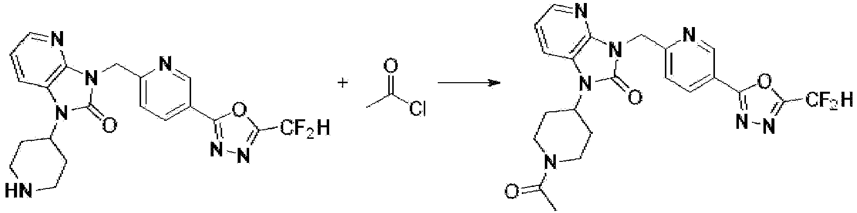

The 3-((5-(5-(difluoromethyl)-1,3,4-oxadiazole-2-yl)pyridine-2-yl)methyl)-1-(piperidine-4-yl)-1,3-dihydr o-2H-imidazo[4,5-b]pyridine-2-one (0.100 g, 0.234 mmol) prepared in the step 2 of the compound 107, triethylamine (0.065 mL, 0.468 mmol) and acetyl chloride (0.033 mL, 0.468 mmol) were dissolved in dichloromethane (2 mL) at room temperature, after which the resulting solution was stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.089 g, 81.3%) in a white solid form.

**¹H NMR** (400 MHz, CDCl₃) δ 9.19 (d, *J =* 2.2 Hz, 1H), 8.29 (dd, *J* = 8.2, 2.2 Hz, 1H), 7.98 (dd, *J* = 5.2, 1.2 Hz, 1H), 7.43 (d, *J* = 8.2 Hz, 1H), 7.31 (dd, *J* = 7.9, 1.3 Hz, 1H), 6.97 (dd, *J* = 7.8, 5.2 Hz, 1H), 6.91 (t, *J =* 51.6 Hz, 1H), 5.36 (s, 2H), 4.86 - 4.82 (m, 1H), 4.61 - 4.55 (m, 1H), 4.00 - 3.97 (m, 1H), 3.25 - 3.18 (m, 1H), 2.64 (td, *J =* 13.0, 2.2 Hz, 1H), 2.25 - 2.18 (m, 2H), 2.13 (s, 3H), 1.99 - 1.90 (m, 2H); **LRMS** (ES) m/z 471.4 (M⁺ + 2).

### Example 185: Synthesis of Compound 185, 1-(1-acetylpiperidine-4-yl)-3-(4-(5-(difluoromethyl)-1,3,4-oxadiazole-2-yl)-2-fluorobenzyl)-1,3-dih ydro-2H-imidazo[4,5-b]pyridine-2-one

### [Step 1] Synthesis of the compound 185

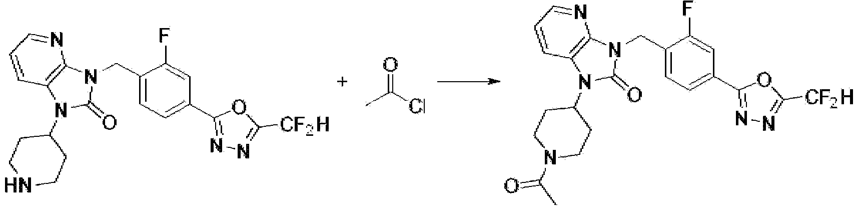

The 3-(4-(5-(difluoromethyl)-1,3,4-oxadiazole-2-yl)-2-fluorobenzyl)-1-(piperidine-4-yl)-1,3-dihydro-2H -imidazo[4,5-b]pyridine-2-one (0.100 g, 0.225 mmol) prepared in the step 5 of the compound 101, triethylamine (0.063 mL, 0.450 mmol) and acetyl chloride (0.032 mL, 0.450 mmol) were dissolved in dichloromethane (2 mL) at room temperature, after which the resulting solution was stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.082 g, 75.3%) in a white solid form.

¹H NMR (400 MHz, CDCl₃) δ 8.01 (dd, *J* = 5.2, 1.3 Hz, 1H), 7.80 - 7.76 (m, 2H), 7.43 (t, *J* = 7.7 Hz, 1H), 7.31 (dd, *J* = 7.9, 1.3 Hz, 1H), 6.99 (dd, *J* = 7.8, 5.2 Hz, 1H), 6.89 (t, *J =* 51.7 Hz, 1H), 5.27 (d, *J =* 3.9 Hz, 2H), 4.86 - 4.82 (m, 1H), 4.61 - 4.53 (m, 1H), 3.99 (dd, *J =* 12.0, 1.9 Hz, 1H), 3.25 - 3.18 (m, 1H), 2.64 (td, *J =* 13.0, 2.1 Hz, 1H), 2.27 - 2.17 (m, 2H), 2.14 (s, 3H), 1.97 - 1.89 (m, 2H); **LRMS** (ES) m/z 487.0 (M⁺+ 1).

### Example 186: Synthesis of Compound 186, 1-(1-acetylpiperidine-4-yl)-3-(4-(5-(difluoromethyl)-1,3,4-oxadiazole-2-yl)benzyl)-1,3-dihydro-2H-imidazo[4,5-b]pyridine-2-one

### [Step 1] Synthesis of the compound 186

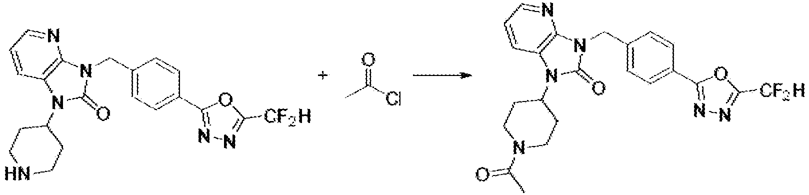

The 3-(4-(5-(difluoromethyl)-1,3,4-oxadiazole-2-yl)benzyl)-1-(piperidine-4-yl)-1,3-dihydro-2H-imidazo [4,5-b]pyridine-2-one (0.100 g, 0.235 mmol) prepared in the step 2 of the compound 105, triethylamine (0.065 mL, 0.469 mmol) and acetyl chloride (0.033 mL, 0.469 mmol) were dissolved in dichloromethane (2 mL) at room temperature, after which the resulting solution was stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.093 g, 85.0%) in a white solid form.

**¹H NMR** (400 MHz, CDCl₃) δ 8.01 - 7.98 (m, 3H), 7.59 (d, *J =* 8.4 Hz, 2H), 7.28 (dd, *J* = 7.8, 1.3 Hz, 1H), 6.95 (dd, *J* = 7.8, 5.2 Hz, 1H), 6.87 (t, *J =* 51.7 Hz, 1H), 5.17 (s, 2H), 4.81 (dd, *J* = 11.5, 2.1 Hz, 1H), 4.58 - 4.50 (m, 1H), 3.98 - 3.95 (m, 1H), 3.23 - 3.16 (m, 1H), 2.62 (td, *J* = 13.0, 2.1 Hz, 1H), 2.25 - 2.11 (m, 5H), 1.93 - 1.85 (m, 2H); **LRMS** (ES) m/z 469.2 (M⁺+ 1).

### Example 187: Synthesis of Compound 187, 1-(1-acetylpiperidine-4-yl)-3-((5-(5-(difluoromethyl)-1,3,4-oxadiazole-2-yl)pyridine-2-yl)methyl)-1, 3-dihydro-2H-benzo[d]imidazole-2-one

### [Step 1] Synthesis of the compound 187

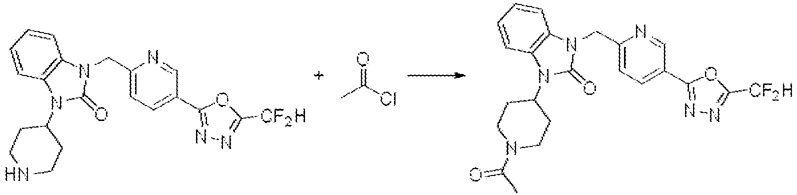

1-((5-(5-(difluoromethyl)-1,3,4-oxadiazole-2-yl)pyridine-2-yl)methyl)-3-(piperidine-4-yl)-1, 3-dihydro-2H-benzo[d]imidazole-2-one (0.100 g, 0.235 mmol), triethylamine (0.065 mL, 0.469 mmol) and acetyl chloride (0.033 mL, 0.469 mmol) were dissolved in dichloromethane (2 mL) at room temperature, after which the resulting solution was stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.085 g, 77.6%) in a white solid form.

**¹H NMR** (400 MHz, CDCl₃) δ 9.23 (d, *J =* 2.1 Hz, 1H), 8.27 (dd, *J* = 8.2, 2.2 Hz, 1H), 7.37 (d, *J* = 8.2 Hz, 1H), 7.11 - 6.78 (m, 5H), 5.24 (d, *J =* 1.9 Hz, 2H), 4.83 (dd, *J =* 11.5, 2.0 Hz, 1H), 4.60 - 4.51 (m, 1H), 3.99 (dd, *J =* 11.9, 1.9 Hz, 1H), 3.22 (td, *J =* 13.2, 2.1 Hz, 1H), 2.65 (td, *J =* 13.0, 2.2 Hz, 1H), 2.41 - 2.25 (m, 2H), 2.13 (s, 3H), 1.95 - 1.87 (m, 2H); **LRMS** (ES) m/z 468.9 (M⁺+ 1).

### Example 188: Synthesis of Compound 188, 3-((5-(5-(difluoromethyl)-1,3,4-oxadiazole-2-yl)pyridine-2-yl)methyl)-1-(1,1-dioxydotetrahydro-2H -thiopyran-4-yl)-5-fluoro-1,3-dihydro-2H-benzo[d]imidazole-2-one

### [Step 1] Synthesis of 4-((4-fluoro-2-nitrophenyl)amino)tetrahydro-2H-thiopyran 1,1-dioxide

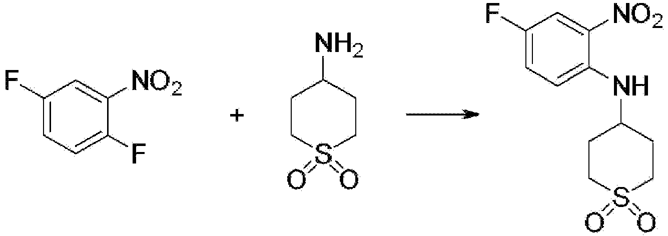

1,4-difluoro-2-nitrobenzene (0.500 g, 3.143 mmol), 4-aminotetrahydro-2H-thiopyran 1,1-dioxide (0.563 g, 3.771 mmol), potassium carbonate (0.869 g, 6.286 mmol) and potassium iodide (0.052 g, 0.314 mmol) were dissolved in N,N-dimethylformamide (10 mL) at room temperature, after which the resulting solution was stirred at 80°C for 18 hours, and then a reaction was finished by lowering a temperature to room temperature. Water was poured into the reaction mixture and an extraction was performed with dichloromethane. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; ethyl acetate/hexane = 30 to 100%) and concentrated to obtain a title compound (0.536 g, 59.2%) in a yellow solid form.

### [Step 2] Synthesis of 4-((2-amino-4-fluorophenyl)amino)tetrahydro-2H-thiopyran 1,1-dioxide

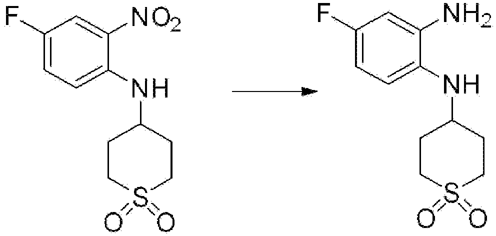

The 4-((4-fluoro-2-nitrophenyl)amino)tetrahydro-2H-thiopyran 1,1-dioxide (0.536 g, 1.859 mmol) prepared in the step 1 and palladium carbide (45.00%, 0.044 g, 0.186 mmol) were dissolved in ethanol (15 mL) at room temperature, after which the resulting solution was stirred at the same temperature for 18 hours in the presence of a hydrogen balloon. The reaction mixture was filtered via a celite pad to remove a solid therefrom, after which solvent was removed from the resulting filtrate under reduced pressure, and then a title compound was used without an additional purification process (0.477 g, 99.3%, brown solid).

### [Step 3] Synthesis of 1-(1,1-dioxydotetrahydro-2H-thiopyran-4-yl)-5-fluoro-1,3-dihydro-2H-benzo[d]imidazole-2-one

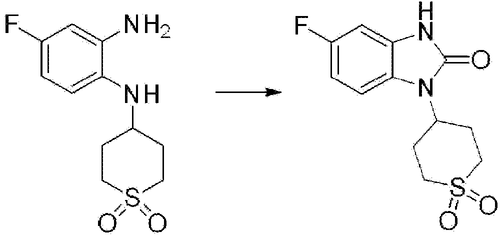

The 4-((2-amino-4-fluorophenyl)amino)tetrahydro-2H-thiopyran 1,1-dioxide (0.500 g, 1.936 mmol) prepared in the step 2, 1,1'-carbonyldiimidazole (CDI, 0.408 g, 2.516 mmol) and triethylamine (0.270 mL, 1.936 mmol) were dissolved in tetrahydrofuran (12 mL) at room temperature, after which the resulting solution was heated under reflux for 18 hours, and then a reaction was finished by lowering a temperature to room temperature. A precipitated solid was filtered and dried to obtain a title compound (0.383 g, 69.6%) in a gray solid form.

### [Step 4] Synthesis of the compound 188

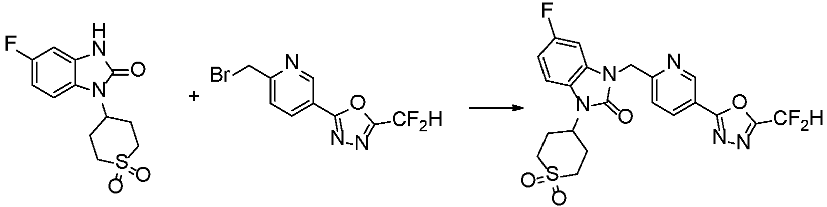

The 1-(1,1-dioxydotetrahydro-2H-thiopyran-4-yl)-5-fluoro-1,3-dihydro-2H-benzo[d]imidazole-2-one (0.126 g, 0.443 mmol) prepared in the step 3 was dissolved in N,N-dimethylformamide (2 mL) at 0°C, after which sodium hydride (60.00%, 0.023 g, 0.576 mmol) was added into the resulting solution and stirred at the same temperature for 30 minutes. 2-(6-(bromomethyl)pyridine-3-yl)-5-(difluoromethyl)-1,3,4-oxadiazole (0.141 g, 0.487 mmol) was added into the reaction mixture and further stirred at room temperature for 3 hours. Solvent was removed from the reaction mixture under reduced pressure, after which the resulting concentrate was purified via column chromatography (SiO₂, 12 g cartridge; methanol/dichloromethane = 0 to 3%) and concentrated to obtain a title compound (0.120 g, 54.9%) in a yellow solid form.

¹H NMR (400 MHz, CDCl₃) δ 9.28 (dd, *J =* 2.2, 0.8 Hz, 1H), 8.36 (dd, *J* = 8.2, 2.2 Hz, 1H), 7.45 (dd, *J* = 8.2, 0.7 Hz, 1H), 7.17 (dd, *J* = 9.4, 4.2 Hz, 1H), 7.06 - 6.80 (m, 3H), 5.24 (s, 2H), 4.76 - 4.68 (m, 1H), 3.31 - 3.18 (m, 4H), 3.09 - 2.98 (m, 2H), 2.27 - 2.23 (m, 2H); **LRMS** (ES) m/z 493.2 (M⁺+ 1).

### Example 189: Synthesis of Compound 189, 3-(4-(5-(difluoromethyl)-1,3,4-oxadiazole-2-yl)-2-fluorobenzyl)-1-(1,1-dioxydotetrahydro-2H-thiop yran-4-yl)-5-fluoro-1,3-dihydro-2H-benzo[d]imidazole-2-one

### [Step 1] Synthesis of the compound 189

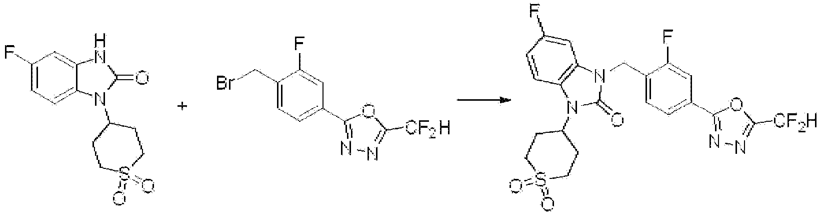

The 1-(1,1-dioxydotetrahydro-2H-thiopyran-4-yl)-5-fluoro-1,3-dihydro-2H-benzo[d]imidazole-2-one (0.126 g, 0.443 mmol) prepared in the step 3 of the compound 188 was dissolved in N,N-dimethylformamide (2 mL) at 0°C, after which sodium hydride (60.00%, 0.023 g, 0.576 mmol) was added into the resulting solution and stirred at the same temperature for 30 minutes. 2-(4-(bromomethyl)-3-fluorophenyl)-5-(difluoromethyl)-1,3,4-oxadiazole (0.150 g, 0.487 mmol) was added into the reaction mixture and further stirred at room temperature for 3 hours. Solvent was removed from the reaction mixture under reduced pressure, after which the resulting concentrate was purified via column chromatography (SiO₂, 12 g cartridge; methanol/dichloromethane = 0 to 3%) and concentrated to obtain a title compound (0.132 g, 58.3%) in a white solid form.

**¹H NMR** (400 MHz, CDCl₃) δ 7.85 - 7.81 (m, 2H), 7.45 (t, *J* = 7.7 Hz, 1H), 7.14 (dd, *J* = 8.7, 4.2 Hz, 1H), 7.03 - 6.73 (m, 3H), 5.12 (s, 2H), 4.73 - 4.65 (m, 1H), 3.31 - 3.15 (m, 4H), 3.06 - 2.94 (m, 2H), 2.23 - 2.19 (m, 2H); **LRMS** (ES) m/z 511.3 (M⁺ + 1).

### Example 190: Synthesis of Compound 190, 3-(4-(5-(difluoromethyl)-1,3,4-oxadiazole-2-yl)benzyl)-1-(1,1-dioxydotetrahydro-2H-thiopyran-4-y I)-5-fluoro-1,3-dihydro-2H-benzo[d]imidazole-2-one

### [Step 1] Synthesis of the compound 190

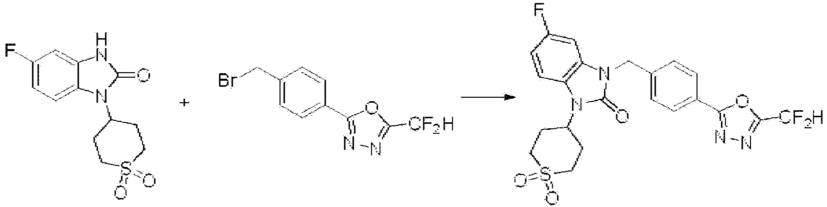

The 1-(1,1-dioxydotetrahydro-2H-thiopyran-4-yl)-5-fluoro-1,3-dihydro-2H-benzo[d]imidazole-2-one (0.126 g, 0.443 mmol) prepared in the step 3 of the compound 188 was dissolved in N,N-dimethylformamide (2 mL) at 0°C, after which sodium hydride (60.00%, 0.023 g, 0.576 mmol) was added into the resulting solution and stirred at the same temperature for 30 minutes. 2-(4-(bromomethyl)phenyl)-5-(difluoromethyl)-1,3,4-oxadiazole (0.141 g, 0.487 mmol) was added into the reaction mixture and further stirred at room temperature for 4 hours. Solvent was removed from the reaction mixture under reduced pressure, after which the resulting concentrate was purified via column chromatography (SiO₂, 12 g cartridge; methanol/dichloromethane = 0 to 3%) and concentrated to obtain a title compound (0.130 g, 59.6%) in a white solid form.

**¹H NMR** (400 MHz, CDCl₃) δ 8.06 (dd, *J* = 8.4, 1.8 Hz, 2H), 7.44 (d, *J =* 8.6 Hz, 2H), 7.15 (dd, *J* = 8.7, 4.2 Hz, 1H), 6.90 (t, *J =* 51.7 Hz, 1H), 6.82 - 6.77 (m, 1H), 6.62 (dd, *J =* 8.2, 2.4 Hz, 1H), 5.09 (s, 2H), 4.76 - 4.68 (m, 1H), 3.32 - 3.16 (m, 4H), 3.07 - 2.96 (m, 2H), 2.25 - 2.21 (m, 2H); **LRMS** (ES) m/z 494.1 (M⁺+ 1).

### Example 191: Synthesis of Compound 191, 3-((5-(5-(difluoromethyl)-1,3,4-oxadiazole-2-yl)pyridine-2-yl)methyl)-4-fluoro-1-(1-(methylsulfonyl )piperidine-4-yl)-1,3-dihydro-2H-benzo[d]imidazole-2-one

### [Step 1] Synthesis of the compound 191

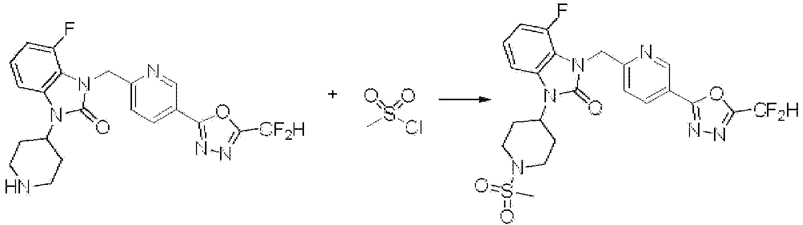

The 3-((5-(5-(difluoromethyl)-1,3,4-oxadiazole-2-yl)pyridine-2-yl)methyl)-4-fluoro-1-(piperidine-4-yl)-1 ,3-dihydro-2H-benzo[d]imidazole-2-one (0.100 g, 0.225 mmol) prepared in the step 5 of the compound 76, triethylamine (0.063 mL, 0.450 mmol) and methanesulfonyl chloride (0.035 mL, 0.450 mmol) were dissolved in dichloromethane (2 mL) at room temperature, after which the resulting solution was stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.079 g, 67.5%) in a white solid form.

**¹H NMR** (400 MHz, CDCl₃) δ 9.23 (d, *J =* 1.8 Hz, 1H), 8.33 (dd, *J* = 8.2, 2.0 Hz, 1H), 7.34 (d, *J = 8.2* Hz, 1H), 7.05 - 6.76 (m, 4H), 5.42 (s, 2H), 4.58 - 4.50 (m, 1H), 4.03 (d, *J* = 12.3 Hz, 2H), 2.92 - 2.86 (m, 5H), 2.60 - 2.50 (m, 2H), 1.99 (dd, *J* = 12.3, 2.2 Hz, 2H); **LRMS** (ES) m/z 523.0 (M⁺ + 1).

### Example 192: Synthesis of Compound 192, 3-(4-(5-(difluoromethyl)-1,3,4-oxadiazole-2-yl)benzyl)-5-fluoro-1-(1-(methylsulfonyl)piperidine-4-yl)-1,3-dihydro-2H-benzo[d]imidazole-2-one

### [Step 1] Synthesis of the compound 192

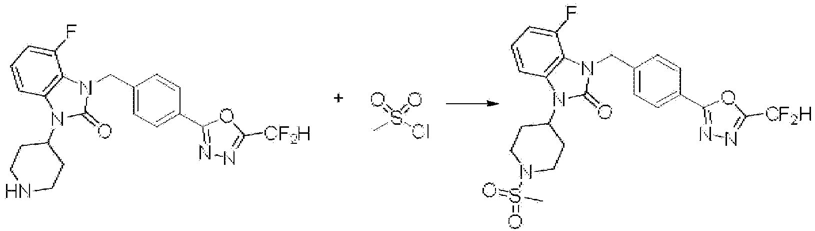

The 3-(4-(5-(difluoromethyl)-1,3,4-oxadiazole-2-yl)benzyl)-5-fluoro-1-(piperidine-4-yl)-1,3-dihydro-2H -benzo[d]imidazole-2-one (0.100 g, 0.226 mmol) prepared in the step 2 of the compound 79, triethylamine (0.063 mL, 0.451 mmol) and methanesulfonyl chloride (0.035 mL, 0.451 mmol) were dissolved in dichloromethane (2 mL) at room temperature, after which the resulting solution was stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.087 g, 74.1%) in a white solid form.

¹H NMR (400 MHz, CDCl₃) δ 8.04 (d, *J =* 8.4 Hz, 2H), 7.66 - 7.05 (m, 3H), 7.34 (dd, *J* = 8.7, 4.5 Hz, 1H), 7.16 (dd, *J* = 9.0, 2.5 Hz, 1H), 6.91 - 6.86 (m, 1H), 5.16 (s, 2H), 4.44 - 4.37 (m, 1H), 3.73 (d, *J =* 11.8 Hz, 2H), 2.97 - 2.91 (m, 5H), 2.45 - 2.33 (m, 2H), 1.86 (d, *J =* 10.0 Hz, 2H); **LRMS** (ES) m/z 522.2 (M⁺ + 1).

### Example 193: Synthesis of Compound 193, 1-((5-(5-(difluoromethyl)-1,3,4-oxadiazole-2-yl)pyridine-2-yl)methyl)-5,6-difluoro-3-(1-(methylsulf onyl)piperidine-4-yl)-1,3-dihydro-2H-benzo[d]imidazole-2-one

### [Step 1] Synthesis of the compound 193

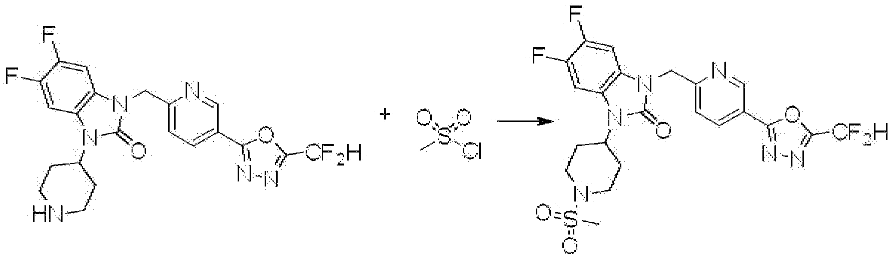

The 1-((5-(5-(difluoromethyl)-1,3,4-oxadiazole-2-yl)pyridine-2-yl)methyl)-5,6-difluoro-3-(piperidine-4-y I)-1,3-dihydro-2H-benzo[d]imidazole-2-one (0.100 g, 0.216 mmol) prepared in the step 4 of the compound 83, triethylamine (0.060 mL, 0.433 mmol) and methanesulfonyl chloride (0.033 mL, 0.433 mmol) were dissolved in dichloromethane (2 mL) at room temperature, after which the resulting solution was stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.073 g, 62.6%) in a light yellow solid form.

¹H NMR (400 MHz, CDCl₃) δ 9.28 (d, *J =* 2.2 Hz, 1H), 8.36 (dd, *J* = 8.2, 2.2 Hz, 1H), 7.45 (d, *J = 8.2* Hz, 1H), 7.07 - 6.80 (m, 3H), 5.21 (s, 2H), 4.50 - 4.44 (m, 1H), 4.04 (dd, *J =* 10.2, 2.2 Hz, 2H), 2.91 - 2.85 (m, 5H), 2.53 - 2.42 (m, 2H), 1.97 (dd, *J =* 12.2, 2.4 Hz, 2H); LRMS (ES) m/z 541.2 (M⁺+ 1).

### Example 194: Synthesis of Compound 194, 1-(4-(5-(difluoromethyl)-1,3,4-oxadiazole-2-yl)benzyl)-5,6-difluoro-3-(1-(methylsulfonyl)piperidine -4-yl)-1,3-dihydro-2H-benzo[d]imidazole-2-one

### [Step 1] Synthesis of the compound 194

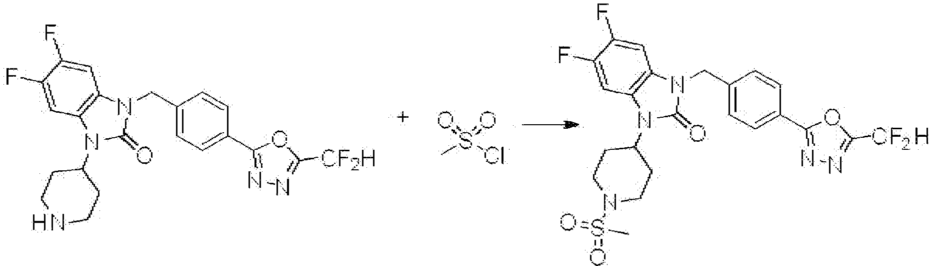

The 1-(4-(5-(difluoromethyl)-1,3,4-oxadiazole-2-yl)benzyl)-5,6-difluoro-3-(piperidine-4-yl)-1,3-dihydro -2H-benzo[d]imidazole-2-one (0.100 g, 0.217 mmol) prepared in the step 2 of the compound 95, triethylamine (0.060 mL, 0.433 mmol) and methanesulfonyl chloride (0.034 mL, 0.433 mmol) were dissolved in dichloromethane (2 mL) at room temperature, after which the resulting solution was stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.087 g, 74.3%) in a white solid form.

**¹H NMR** (400 MHz, CDCl₃) δ 8.10 (d, *J* = 8.4 Hz, 2H), 7.45 (d, *J* = 8.5 Hz, 2H), 7.08 - 7.03 (m, 2H), 6.67 (dd, *J* = 9.5, 6.7 Hz, 1H), 5.09 (s, 2H), 4.53 - 4.45 (m, 1H), 4.05 (dd, *J* = 10.3, 2.1 Hz, 2H), 2.92 - 2.85 (m, 5H), 2.53 - 2.43 (m, 2H), 1.98 (dd, *J* = 12.2, 2.5 Hz, 2H); **LRMS** (ES) m/z 540.3 (M⁺ + 1).

### Example 195: Synthesis of Compound 195, 3-((5-(5-(difluoromethyl)-1,3,4-oxadiazole-2-yl)pyridine-2-yl)methyl)-1-(1-(methylsulfonyl)piperidi ne-4-yl)-5-(trifluoromethyl)-1,3-dihydro-2H-benzo[d]imidazole-2-one

### [Step 1] Synthesis of the compound 195

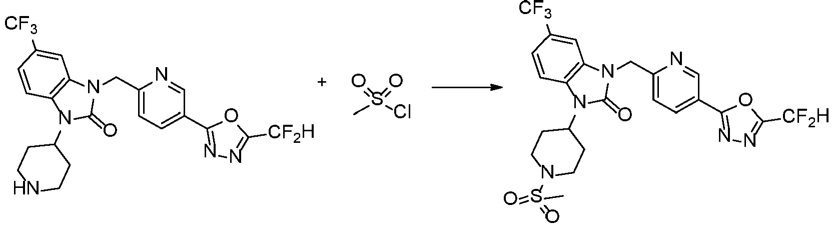

The 3-((5-(5-(difluoromethyl)-1,3,4-oxadiazole-2-yl)pyridine-2-yl)methyl)-1-(piperidine-4-yl)-5-(trifluor omethyl)-1,3-dihydro-2H-benzo[d]imidazole-2-one (0.100 g, 0.202 mmol) prepared in the step 5 of the compound 89, triethylamine (0.056 mL, 0.405 mmol) and methanesulfonyl chloride (0.031 mL, 0.405 mmol) were dissolved in dichloromethane (2 mL) at room temperature, after which the resulting solution was stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.074 g, 63.5%) in a yellow solid form.

**¹H NMR** (400 MHz, CDCl₃) δ 9.25 (dd, *J =* 2.2, 0.7 Hz, 1H), 8.35 (dd, *J* = 8.2, 2.2 Hz, 1H), 7.44 (dd, *J* = 8.2, 0.7 Hz, 1H), 7.37 (dd, *J =* 8.4, 0.8 Hz, 1H), 7.29 - 7.26 (m, 2H), 6.92 (t, *J =* 51.6 Hz, 1H), 5.29 (s, 2H), 4.58 - 4.50 (m, 1H), 4.03 (dd, *J* = 10.3, 2.0 Hz, 2H), 2.92 - 2.86 (m, 5H), 2.59 - 2.48 (m, 2H), 1.98 (dd, *J =* 12.3, 2.4 Hz, 2H); **LRMS** (ES) m/z 573.3 (M⁺+ 1).

### Example 196: Synthesis of Compound 196, 3-(4-(5-(difluoromethyl)-1,3,4-oxadiazole-2-yl)-2-fluorobenzyl)-1-(1-(methylsulfonyl)piperidine-4-yl)-5-(trifluoromethyl)-1,3-dihydro-2H-benzo[d]imidazole-2-one

### [Step 1] Synthesis of the compound 196

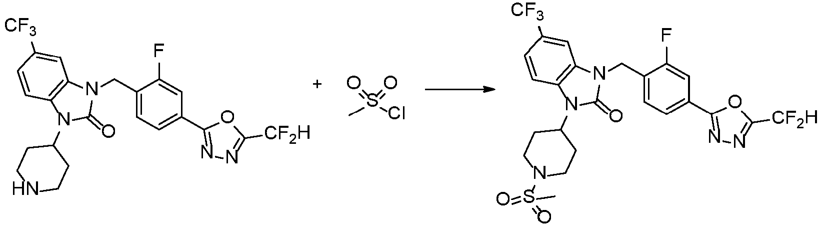

The 3-(4-(5-(difluoromethyl)-1,3,4-oxadiazole-2-yl)-2-fluorobenzyl)-1-(piperidine-4-yl)-5-(trifluorometh yl)-1,3-dihydro-2H-benzo[d]imidazole-2-one (0.100 g, 0.196 mmol) prepared in the step 2 of the compound 91, triethylamine (0.055 mL, 0.391 mmol) and methanesulfonyl chloride (0.030 mL, 0.391 mmol) were dissolved in dichloromethane (2 mL) at room temperature, after which the resulting solution was stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.091 g, 78.8%) in a white solid form.

**¹H NMR** (400 MHz, CDCl₃) δ 7.85 (d, *J =* 8.8 Hz, 2H), 7.46 (t, *J* = 7.6 Hz, 1H), 7.39 (d, *J* = 8.3 Hz, 1H), 7.29 (d, *J = 8.4* Hz, 1H), 7.23 (s, 1H), 6.90 (t, *J =* 51.7 Hz, 1H), 5.21 (s, 2H), 4.58 - 4.50 (m, 1H), 4.03 (d, *J =* 12.2 Hz, 2H), 2.92 - 2.87 (m, 5H), 2.59 - 2.48 (m, 2H), 1.99 - 1.96 (m, 2H); **LRMS** (ES) m/z 554.2 (M⁺+ 1).

### Example 197: Synthesis of Compound 197, 5-(1-acetylpiperidine-4-yl)-1-((5-(5-(difluoromethyl)-1,3,4-oxadiazole-2-yl)pyridine-2-yl)methyl)-3-(1-methylpiperidine-4-yl)-1,3-dihydro-2H-benzo[d]imidazole-2-one

### [Step 1] Synthesis of tert-butyl 4-(1-(4-(5-(difluoromethyl)-1,3,4-oxadiazole-2-yl)-2-fluorobenzyl)-3-(1-methylpiperidine-4-yl)-2-o xo-2,3-dihydro-1H-benzo[d]imidazole-5-yl)-3,6-dihydropyridine-1(2H)-carboxylate

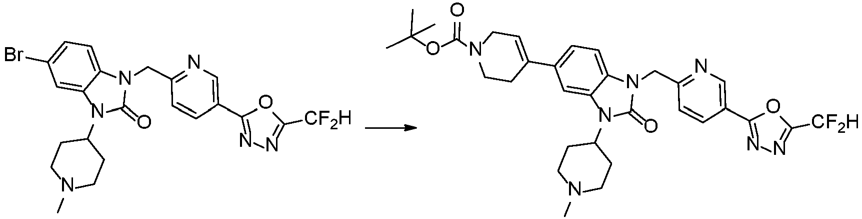

The 5-bromo-1-((5-(5-(difluoromethyl)-1,3,4-oxadiazole-2-yl)pyridine-2-yl)methyl)-3-(1-methylpiperidi ne-4-yl)-1,3-dihydro-2H-benzo[d]imidazole-2-one (1.000 g, 1.925 mmol) prepared in the step 6 of the compound 133, tert-butyl 4-(4,4,5,5-tetramethyl-1,3,2-dioxaborolane-2-yl)-3,6-dihydropyridine-1(2H)-carboxylate (0.714 g, 2.311 mmol), [1,1'-bis(di-tert-butylphosphino)ferrocene]palladium(II) dichloride (Pd(dtbpf)Cl₂, 0.063 g, 0.096 mmol) and cesium carbonate (1.882 g, 5.776 mmol) were mixed in 1,4-dioxane (3 mL)/water (1 mL) at room temperature, after which the resulting mixture was irradiated with microwave, then heated at 100°C for 20 minutes, and then a reaction was finished by lowering a temperature to room temperature. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 12 g cartridge; methanol/dichloromethane = 0 to 5%) and concentrated to obtain a title compound (0.552 g, 46.1%) in an orange solid form.

### [Step 2] Synthesis of tert-butyl 4-(1-(4-(5-(difluoromethyl)-1,3,4-oxadiazole-2-yl)-2-fluorobenzyl)-3-(1-methylpiperidine-4-yl)-2-o xo-2,3-dihydro-1H-benzo[d]imidazole-5-yl)piperidine-1-carboxylate

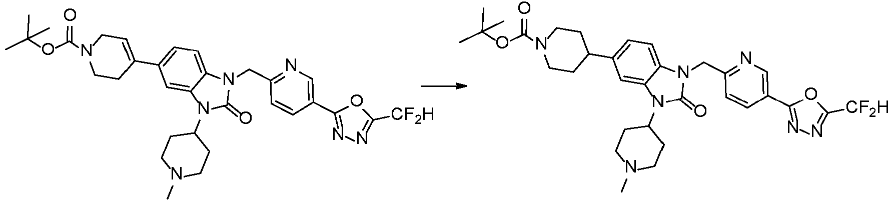

The tert-butyl 4-(1-(4-(5-(difluoromethyl)-1,3,4-oxadiazole-2-yl)-2-fluorobenzyl)-3-(1-methylpiperidine-4-yl)-2-o xo-2,3-dihydro-1H-benzo[d]imidazole-5-yl)-3,6-dihydropyridine-1(2H)-carboxylate (0.621 g, 0.972 mmol) prepared in the step 1 was dissolved in methanol (20 mL) and subjected to a reaction by using H-cube (10%-Pd/C, 2 atm, 40°C, 0.5 mL/min). Solvent was removed from the reaction mixture under reduced pressure, after which a title compound was used without an additional purification process (0.476 g, 76.4%, yellow oil).

### [Step 3] Synthesis of 1-((5-(5-(difluoromethyl)-1,3,4-oxadiazole-2-yl)pyridine-2-yl)methyl)-3-(1-methylpiperidine-4-yl)-5 -(piperidine-4-yl)-1,3-dihydro-2H-benzo[d]imidazole-2-one

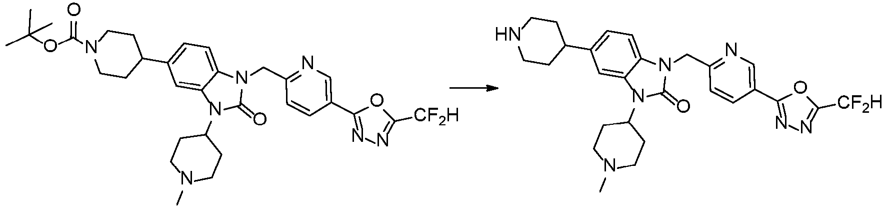

The tert-butyl 4-(1-(4-(5-(difluoromethyl)-1,3,4-oxadiazole-2-yl)-2-fluorobenzyl)-3-(1-methylpiperidine-4-yl)-2-o xo-2,3-dihydro-1H-benzo[d]imidazole-5-yl)piperidine-1-carboxylate (0.476 g, 0.743 mmol) prepared in the step 2 and trifluoroacetic acid (0.284 mL, 3.715 mmol) were dissolved in dichloromethane (6 mL) at room temperature, after which the resulting solution was stirred at the same temperature for 6 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. A title compound was used without an additional purification process (0.374 g, 93.1%, orange oil).

### [Step 4] Synthesis of the compound 197

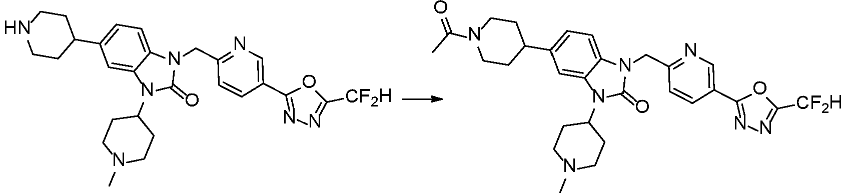

The 1-((5-(5-(difluoromethyl)-1,3,4-oxadiazole-2-yl)pyridine-2-yl)methyl)-3-(1-methylpiperidine-4-yl)-5 -(piperidine-4-yl)-1,3-dihydro-2H-benzo[d]imidazole-2-one (0.100 g, 0.191 mmol) prepared in the step 3 and triethylamine (0.053 mL, 0.382 mmol) were dissolved in dichloromethane (3 mL) at room temperature, after which acetyl chloride (0.022 g, 0.286 mmol) was added into the resulting solution and stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 10%) and concentrated to obtain a title compound (0.056 g, 51.8%) in a yellow oil form.

**¹H NMR** (400 MHz, CDCl₃) δ 7.87 ~ 7.83 (m, 2H), 7.47 (t, *J = 7.8* Hz, 1H), 7.19 (brs, 1H), 7.05 ~ 6.79 (m, 3H), 5.19 (s, 2H), 4.83 ~ 4.78 (m, 1H), 4.47 ~ 4.46 (m, 1H), 3.97 ~ 3.93 (m, 1H), 3.21 ~ 3.07 (m, 3H), 2.80 ~ 2.74 (m, 1H), 2.65 ~ 2.52 (m, 3H), 2.41 (s, 3H), 2.25 ~ 2.20 (m, 2H), 2.16 (s, 3H), 1.92 ~ 1.86 (m, 4H), 1.70 ~ 1.59 (m, 2H); **LRMS** (ES) m/z 583.3 (M⁺+ H).

### Example 198: Synthesis of Compound 198, 1-((5-(5-(difluoromethyl)-1,3,4-oxadiazole-2-yl)pyridine-2-yl)methyl)-3-(1-methylpiperidine-4-yl)-5 -(1-(methylsulfonyl)piperidine-4-yl)-1,3-dihydro-2H-benzo[d]imidazole-2-one

### [Step 1] Synthesis of the compound 198

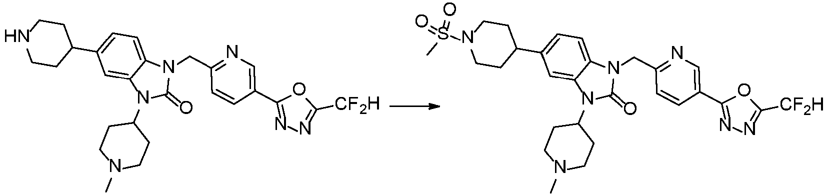

The 1-((5-(5-(difluoromethyl)-1,3,4-oxadiazole-2-yl)pyridine-2-yl)methyl)-3-(1-methylpiperidine-4-yl)-5 -(piperidine-4-yl)-1,3-dihydro-2H-benzo[d]imidazole-2-one (0.100 g, 0.191 mmol) prepared in the step 3 of the compound 197 and triethylamine (0.053 mL, 0.382 mmol) were dissolved in dichloromethane (3 mL) at room temperature, after which methanesulfonyl chloride (0.022 mL, 0.286 mmol) was added into the resulting solution and stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 10%) and concentrated to obtain a title compound (0.039 g, 33.9%) in a yellow oil form.

¹H NMR (400 MHz, CDCl₃) δ 7.87 ~ 7.83 (m, 2H), 7.47 (t, *J =* 7.8 Hz, 1H), 7.21 (brs, 1H), 7.05 ~ 6.79 (m, 3H), 5.19 (s, 1H), 4.50 ~ 4.44 (m, 1H), 3.96 (d, *J =* 11.8 Hz, 2H), 3.08 (d, *J = 9.4* Hz, 2H), 3.02 (brs, 1H), 2.85 (s, 3H), 2.80 ~ 2.74 (m, 2H), 2.70 ~ 2.66 (m, 1H), 2.65 ~ 2.61 (m, 2H), 2.54 (s, 3H), 2.23 (t, *J =* 10.4 Hz, 2H), 1.96 (d, *J =* 10.8 Hz, 2H), 1.90 ~ 1.84 (m, 3H); LRMS (ES) m/z 619.3 (M⁺+ H).

### Example 199: Synthesis of Compound 199, 1-((5-(5-(difluoromethyl)-1,3,4-oxadiazole-2-yl)pyridine-2-yl)methyl)-3,5-bis(1-methylpiperidine-4 -yl)-1,3-dihydro-2H-benzo[d]imidazole-2-one

### [Step 1] Synthesis of the compound 199

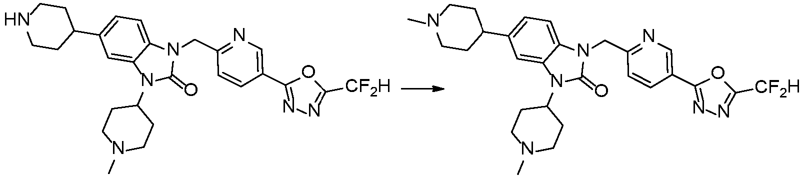

The 1-((5-(5-(difluoromethyl)-1,3,4-oxadiazole-2-yl)pyridine-2-yl)methyl)-3-(1-methylpiperidine-4-yl)-5 -(piperidine-4-yl)-1,3-dihydro-2H-benzo[d]imidazole-2-one (0.100 g, 0.191 mmol) prepared in the step 3 of the compound 197 and formaldehyde (37.00%, 0.023 g, 0.286 mmol) were dissolved in dichloromethane (3 mL) at room temperature, after which sodium triacetoxyborohydride (0.081 g, 0.382 mmol) was added into the resulting solution and stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 30%) and concentrated to obtain a title compound (0.025 g, 24.3%) in a yellow oil form.

**¹H NMR** (400 MHz, CDCl₃) δ 9.30 - 9.29 (m, 1H), 8.32 (dd, *J* = 8.2, 2.2 Hz, 1H), 7.41 (d, *J* = 8.2 Hz, 1H), 7.23 (s, 1H), 7.07 - 6.81 (m, 3H), 5.28 (s, 2H), 4.48 - 4.41 (m, 1H), 3.03 (t, *J =* 11.3 Hz, 4H), 2.57 - 2.47 (m, 3H), 2.38 (s, 3H), 2.36 (s, 3H), 2.19 (t, *J* = 11.2 Hz, 2H), 2.11 - 2.06 (m, 2H), 1.87 - 1.83 (m, 6H); **LRMS** (ES) m/z 538.4 (M⁺ + H).

### Example 200: Synthesis of Compound 200, 5-chloro-1-((5-(5-(difluoromethyl)-1,3,4-oxadiazole-2-yl)pyridine-2-yl)methyl)-3-(1-(methylsulfony I)piperidine-4-yl)-1,3-dihydro-2H-benzo[d]imidazole-2-one

### [Step 1] Synthesis of the compound 200

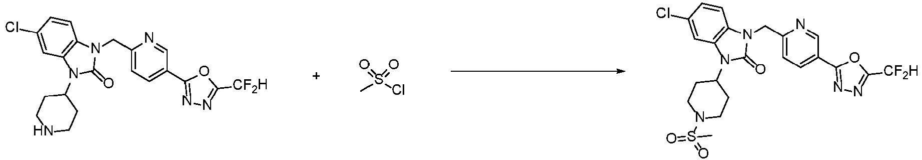

The 5-chloro-1-((5-(5-(difluoromethyl)-1,3,4-oxadiazole-2-yl)pyridine-2-yl)methyl)-3-(piperidine-4-yl)-1,3-dihydro-2H-benzo[d]imidazole-2-one (0.100 g, 0.217 mmol) prepared in the step 5 of the compound 97, triethylamine (0.060 mL, 0.434 mmol) and methanesulfonyl chloride (0.034 mL, 0.434 mmol) were dissolved in dichloromethane (2 mL) at room temperature, after which the resulting solution was stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.086 g, 73.8%) in a yellow solid form.

¹H NMR (400 MHz, CDCl₃) δ 9.26 (d, *J =* 2.0 Hz, 1H), 8.33 (dd, *J* = 8.2, 2.2 Hz, 1H), 7.40 (d, *J =* 8.2 Hz, 1H), 7.18 (d, *J =* 1.8 Hz, 1H), 7.06 -6.80 (m, 3H), 5.24 (s, 2H), 4.52 - 4.43 (m, 1H), 4.03 (dd, *J =* 10.3, 2.0 Hz, 2H), 2.91 - 2.85 (m, 5H), 2.58 - 2.47 (m, 2H), 1.98 (dd, *J =* 12.3, 2.2 Hz, 2H); LRMS (ES) m/z 539.2 (M⁺+ 1).

### Example 201: Synthesis of Compound 201, 5-chloro-1-(4-(5-(difluoromethyl)-1,3,4-oxadiazole-2-yl)-2-fluorobenzyl)-3-(1-(methylsulfonyl)pipe ridine-4-yl)-1,3-dihydro-2H-benzo[d]imidazole-2-one

### [Step 1] Synthesis of the compound 201

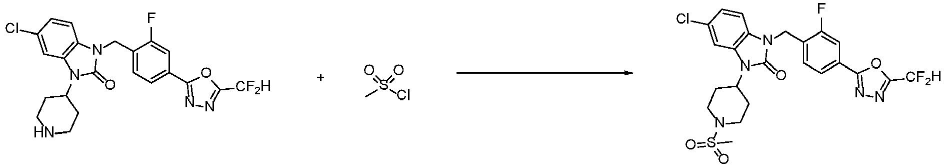

The 5-chloro-1-(4-(5-(difluoromethyl)-1,3,4-oxadiazole-2-yl)-2-fluorobenzyl)-3-(piperidine-4-yl)-1,3-di hydro-2H-benzo[d]imidazole-2-one (0.100 g, 0.209 mmol) prepared in the step 2 of the compound 101, triethylamine (0.058 mL, 0.419 mmol) and methanesulfonyl chloride (0.032 mL, 0.419 mmol) were dissolved in dichloromethane (2 mL) at room temperature, after which the resulting solution was stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.086 g, 73.7%) in a white solid form.

**¹H NMR** (400 MHz, CDCl₃) δ 7.82 (d, *J =* 8.8 Hz, 2H), 7.42 (t, *J = 7.5* Hz, 1H), 7.18 (d, *J* = 1.8 Hz, 1H), 7.03 - 6.78 (m, 3H), 5.15 (s, 2H), 4.50 - 4.42 (m, 1H), 4.02 (dd, *J* = 10.2, 2.0 Hz, 2H), 2.90 - 2.84 (m, 5H), 2.56 - 2.46 (m, 2H), 1.96 (dd, *J* = 12.3, 2.2 Hz, 2H); LRMS (ES) m/z 556.2 (M⁺+ 1).

### Example 202: Synthesis of Compound 202, 5-chloro-1-(4-(5-(difluoromethyl)-1,3,4-oxadiazole-2-yl)benzyl)-3-(1-(methylsulfonyl)piperidine-4-yl)-1,3-dihydro-2H-benzo[d]imidazole-2-one

### [Step 1] Synthesis of the compound 202

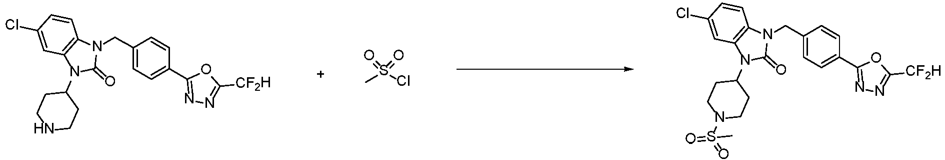

The 5-chloro-1-(4-(5-(difluoromethyl)-1,3,4-oxadiazole-2-yl)benzyl)-3-(piperidine-4-yl)-1,3-dihydro-2H -benzo[d]imidazole-2-one (0.100 g, 0.217 mmol) prepared in the step 2 of the compound 99, triethylamine (0.061 mL, 0.435 mmol) and methanesulfonyl chloride (0.034 mL, 0.435 mmol) were dissolved in dichloromethane (2 mL) at room temperature, after which the resulting solution was stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.100 g, 85.2%) in a white solid form.

**¹H NMR** (400 MHz, CDCl₃) δ 8.04 (dd, *J* = 6.7, 1.8 Hz, 2H), 7.42 (d, *J =* 8.5 Hz, 2H), 7.17 (d, *J = 1.8* Hz, 1H), 7.03 - 6.74 (m, 3H), 5.10 (s, 2H), 4.51 - 4.43 (m, 1H), 4.02 (dd, *J = 10.2,* 2.0 Hz, 2H), 2.88 - 2.85 (m, 5H), 2.57 - 2.46 (m, 2H), 1.96 (dd, *J* = 12.3, 2.3 Hz, 2H); LRMS (ES) m/z 538.1 (M⁺ + 1).

### Example 203: Synthesis of Compound 203, 3-((5-(5-(difluoromethyl)-1,3,4-oxadiazole-2-yl)pyridine-2-yl)methyl)-1-(1-(methylsulfonyl)piperidi ne-4-yl)-1,3-dihydro-2H-imidazo[4,5-b]pyridine-2-one

### [Step 1] Synthesis of the compound 203

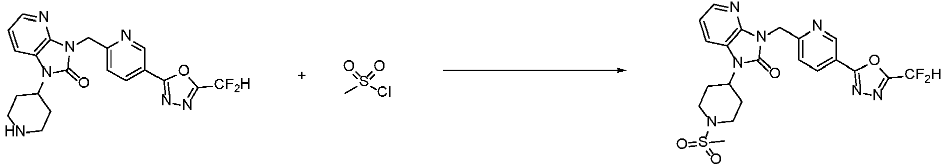

The 3-((5-(5-(difluoromethyl)-1,3,4-oxadiazole-2-yl)pyridine-2-yl)methyl)-1-(piperidine-4-yl)-1,3-dihydr o-2H-imidazo[4,5-b]pyridine-2-one (0.100 g, 0.234 mmol) prepared in the step 2 of the compound 107, triethylamine (0.065 mL, 0.468 mmol) and methanesulfonyl chloride (0.036 mL, 0.468 mmol) were dissolved in dichloromethane (2 mL) at room temperature, after which the resulting solution was stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.041 g, 34.8%) in a white solid form.

**¹H NMR** (400 MHz, CDCl₃) δ 9.23 (d, *J =* 1.7 Hz, 1H), 8.33 (dd, *J =* 8.2, 2.2 Hz, 1H), 8.04 (dd, *J* = 5.2, 1.0 Hz, 1H), 7.48 - 7.42 (m, 2H), 7.05 - 6.78 (m, 2H), 5.40 (s, 2H), 4.63 - 4.54 (m, 1H), 4.04 (dd, *J* = 10.3, 2.0 Hz, 2H), 2.91 - 2.85 (m, 5H), 2.50 - 2.40 (m, 2H), 2.02 (dd, *J* = 12.4, 2.4 Hz, 2H); **LRMS** (ES) m/z 506.2 (M⁺ + 1).

### Example 204: Synthesis of Compound 204, 3-(4-(5-(difluoromethyl)-1,3,4-oxadiazole-2-yl)-2-fluorobenzyl)-1-(1-(methylsulfonyl)piperidine-4-yl)-1,3-dihydro-2H-imidazo[4,5-b]pyridine-2-one

### [Step 1] Synthesis of the compound 204

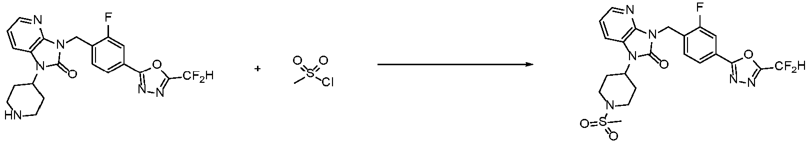

The 3-(4-(5-(difluoromethyl)-1,3,4-oxadiazole-2-yl)-2-fluorobenzyl)-1-(piperidine-4-yl)-1,3-dihydro-2H -imidazo[4,5-b]pyridine-2-one (0.100 g, 0.225 mmol) prepared in the step 5 of the compound 103, triethylamine (0.063 mL, 0.450 mmol) and methanesulfonyl chloride (0.035 mL, 0.450 mmol) were dissolved in dichloromethane (2 mL) at room temperature, after which the resulting solution was stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.103 g, 87.3%) in a white solid form.

**¹H NMR** (400 MHz, CDCl₃) δ 8.03 (dd, *J* = 5.2, 1.0 Hz, 1H), 7.80 - 7.77 (m, 2H), 7.45 - 7.41 (m, 2H), 7.03 - 6.77 (m, 2H), 5.28 (s, 2H), 4.59 - 4.50 (m, 1H), 4.00 (dd, *J* = 10.4, 1.9 Hz, 2H), 2.89 - 2.83 (m, 5H), 2.47 - 2.37 (m, 2H), 1.98 (dd, *J* = 12.3, 2.3 Hz, 2H); **LRMS** (ES) m/z 523.1 (M⁺ + 1).

### Example 205: Synthesis of Compound 205, 3-(4-(5-(difluoromethyl)-1,3,4-oxadiazole-2-yl)benzyl)-1-(1-(methylsulfonyl)piperidine-4-yl)-1,3-di hydro-2H-imidazo[4,5-b]pyridine-2-one

### [Step 1] Synthesis of the compound 205

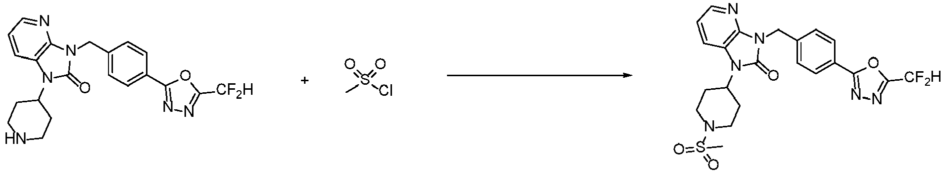

The 3-(4-(5-(difluoromethyl)-1,3,4-oxadiazole-2-yl)benzyl)-1-(piperidine-4-yl)-1,3-dihydro-2H-imidazo [4,5-b]pyridine-2-one (0.100 g, 0.235 mmol) prepared in the step 2 of the compound 105, triethylamine (0.065 mL, 0.469 mmol) and methanesulfonyl chloride (0.036 mL, 0.469 mmol) were dissolved in dichloromethane (2 mL) at room temperature, after which the resulting solution was stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.066 g, 55.9%) in a white solid form.

**¹H NMR** (400 MHz, CDCl₃) δ 8.06 - 8.03 (m, 3H), 7.63 (d, *J* = 8.3 Hz, 2H), 7.40 (dd, *J* = 7.9, 1.2 Hz, 1H), 7.03 - 6.76 (m, 2H), 5.22 (s, 2H), 4.59 - 4.51 (m, 1H), 4.01 (dd, *J* = 10.3, 2.1 Hz, 2H), 2.89 - 2.83 (m, 5H), 2.46 - 2.36 (m, 2H), 1.97 (dd, *J* = 12.3, 2.3 Hz, 2H); LRMS (ES) m/z 505.1 (M⁺ + 1).

### Example 206: Synthesis of Compound 206, 1-((5-(5-(difluoromethyl)-1,3,4-oxadiazole-2-yl)pyridine-2-yl)methyl)-3-(1-(methylsulfonyl)piperidi ne-4-yl)-1,3-dihydro-2H-benzo[d]imidazole-2-one

### [Step 1] Synthesis of the compound 206

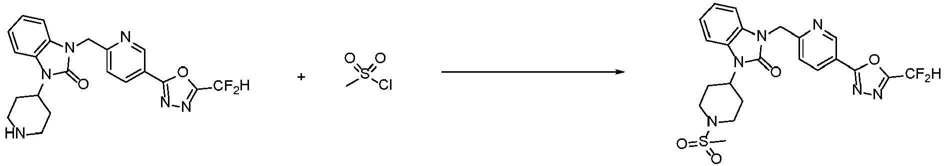

1-((5-(5-(difluoromethyl)-1,3,4-oxadiazole-2-yl)pyridine-2-yl)methyl)-3-(piperidine-4-yl)-1, 3-dihydro-2H-benzo[d]imidazole-2-one (0.100 g, 0.235 mmol), triethylamine (0.065 mL, 0.469 mmol) and methanesulfonyl chloride (0.036 mL, 0.469 mmol) were dissolved in dichloromethane (2 mL) at room temperature, after which the resulting solution was stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.048 g, 40.9%) in a white solid form.

**¹H NMR** (400 MHz, CDCl₃) δ 9.28 (d, *J =* 1.8 Hz, 1H), 8.31 (dd, *J* = 8.2, 2.2 Hz, 1H), 7.41 (d, *J = 23.8* Hz, 1H), 7.22 (d, *J* = 7.5 Hz, 1H), 7.12 - 6.79 (m, 4H), 5.28 (s, 2H), 4.59 - 4.51 (m, 1H), 4.03 (dd, *J* = 10.1, 2.1 Hz, 2H), 2.92 - 2.85 (m, 5H), 2.62 - 2.52 (m, 2H), 1.98 (dd, *J* = 12.3, 2.3 Hz, 2H); **LRMS** (ES) m/z 505.0 (M⁺ + 1).

### Example 207: Synthesis of Compound 207, N-(3-benzyl-1-((5-(5-(difluoromethyl)-1,3,4-oxadiazole-2-yl)pyridine-2-yl)methyl)-2-oxo-2,3-dihyd ro-1H-benzo[d]imidazole-5-yl)pivalamide

### [Step 1] Synthesis of N-(4-bromo-3-chlorophenyl)pivalamide

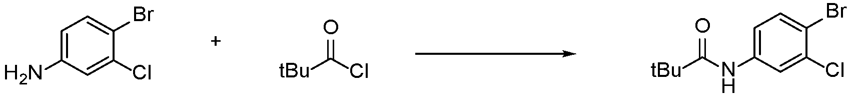

4-bromo-3-chloroaniline (0.500 g, 2.422 mmol), triethylamine (0.405 mL, 2.906 mmol) and trimethylacetyl chloride (0.328 mL, 2.664 mmol) were dissolved in dichloromethane (5 mL) at room temperature, after which the resulting solution was stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 24 g cartridge; ethyl acetate/hexane = 0 to 30%) and concentrated to obtain a title compound (0.684 g, 97.2%) in a white solid form.

### [Step 2] Synthesis of N-(3-benzyl-2-oxo-2,3-dihydro-1H-benzo[d]imidazole-5-yl)pivalamide

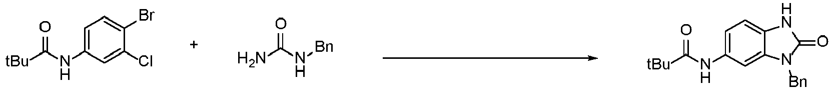

The N-(4-bromo-3-chlorophenyl)pivalamide (0.150 g, 0.516 mmol) prepared in the step 1, 1-benzylurea (0.310 g, 2.065 mmol), BrettPhos palladium G3 (0.023 g, 0.026 mmol) and potassium phosphate (0.263 g, 1.239 mmol) were dissolved in tert-butanol (2 mL) at room temperature, after which the resulting solution was stirred at 110°C for 18 hours, and then a reaction was finished by lowering a temperature to room temperature. The reaction mixture was filtered via a celite pad to remove a solid therefrom, after which solvent was removed from the resulting filtrate under reduced pressure. Then, the resulting concentrate was purified via column chromatography (SiO₂, 4 g cartridge; ethyl acetate/hexane = 0 to 30%) and concentrated, after which an obtained product was purified again via chromatography (SiO₂, 4 g cartridge; methanol/dichloromethane = 0 to 5%) and concentrated to obtain a title compound (0.110 g, 65.9%) in a yellow solid form.

### [Step 3] Synthesis of the compound 207

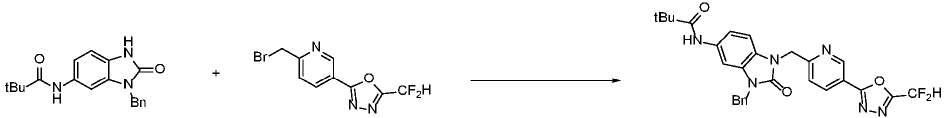

The N-(3-benzyl-2-oxo-2,3-dihydro-1H-benzo[d]imidazole-5-yl)pivalamide (0.110 g, 0.340 mmol) prepared in the step 2 was dissolved in N,N-dimethylformamide (3 mL) at 0°C, after which sodium hydride (60.00%, 0.020 g, 0.510 mmol) was added into the resulting solution and stirred at the same temperature for 30 minutes. 2-(6-(bromomethyl)pyridine-3-yl)-5-(difluoromethyl)-1,3,4-oxadiazole (0.109 g, 0.374 mmol) was added into the reaction mixture and further stirred at room temperature for 3 hours. Water was poured into the reaction mixture and an extraction was performed with ethyl acetate. An organic layer was washed with saturated sodium chloride aqueous solution, then dehydrated with anhydrous magnesium sulfate, then filtered, and then concentrated under reduced pressure. The resulting concentrate was purified via column chromatography (SiO₂, 4 g cartridge; ethyl acetate/hexane = 0 to 30%) and concentrated, after which an obtained product was purified again via chromatography (SiO₂, 4 g cartridge; methanol/dichloromethane = 0 to 2%) and concentrated to obtain a title compound (0.065 g, 35.9%) in a yellow solid form.

**¹H NMR** (400 MHz, CDCl₃) δ 9.23 (d, *J =* 1.7 Hz, 1H), 8.26 (dd, *J* = 8.2, 2.2 Hz, 1H), 7.55 (d, *J = 1.8* Hz, 1H), 7.44 (s, 1H), 7.34 - 7.20 (m, 6H), 6.91 - 6.78 (m, 3H), 5.26 (s, 2H), 5.05 (s, 2H), 1.25 (s, 9H); **LRMS** (ES) m/z 533.1 (M⁺+ 1).

### Example 208: Synthesis of Compound 208, 1-((5-(5-(difluoromethyl)-1,3,4-oxadiazole-2-yl)pyridine-2-yl)methyl)-3-methyl-5-(pyridine-3-yl)-1, 3-dihydro-2H-benzo[d]imidazole-2-one

### [Step 1] Synthesis of 5-bromo-N-methyl-2-nitroaniline

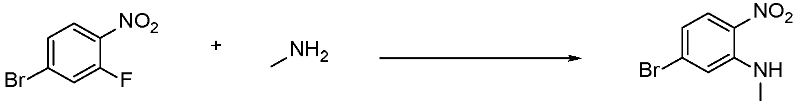

4-bromo-2-fluoro-1-nitrobenzene (5.000 g, 22.727 mmol), methanamine (0.777 g, 25.000 mmol), potassium carbonate (6.282 g, 45.455 mmol) and potassium iodide (0.377 g, 2.273 mmol) were dissolved in dichloromethane (100 mL) at room temperature, after which the resulting solution was stirred at 80°C for 18 hours, and then a reaction was finished by lowering a temperature to room temperature. Hexane was put into the reaction mixture and stirred, after which a precipitated solid was filtered and dried to obtain a title compound (4.760 g, 90.6%) in a yellow solid form.

### [Step 2] Synthesis of 5-bromo-N¹-methylbenzene-1,2-diamine

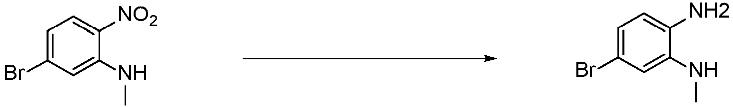

The 5-bromo-N-methyl-2-nitroaniline (4.760 g, 20.602 mmol) prepared in the step 1 and Raney nickel (400 mg, 10 wt%) were dissolved in methanol (100 mL) at room temperature, after which the resulting solution was stirred at the same temperature for 18 hours in the presence of a hydrogen balloon. The reaction mixture was filtered via a celite pad to remove a solid therefrom, after which solvent was removed from the resulting filtrate under reduced pressure, and then a title compound was used without an additional purification process (4.000 g, 96.6%, black liquid).

### [Step 3] Synthesis of 6-bromo-1-methyl-1,3-dihydro-2H-benzo[d]imidazole-2-one

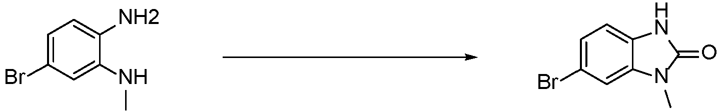

The 5-bromo-N¹-methylbenzene-1,2-diamine (4.000 g, 19.894 mmol) prepared in the step 2, triethylamine (2.773 mL, 19.894 mmol) and 1,1'-carbonyldiimidazole (CDI, 3.871 g, 23.872 mmol) were dissolved in dichloromethane (60 mL) at room temperature, after which the resulting solution was heated under reflux for 18 hours, and then a reaction was finished by lowering a temperature to room temperature. A precipitated solid was filtered, then washed with hexane, and then dried to obtain a title compound (3.935 g, 87.1%) in a brown solid form.

### [Step 4] Synthesis of 5-bromo-1-((5-(5-(difluoromethyl)-1,3,4-oxadiazole-2-yl)pyridine-2-yl)methyl)-3-methyl-1,3-dihyd ro-2H-benzo[d]imidazole-2-one

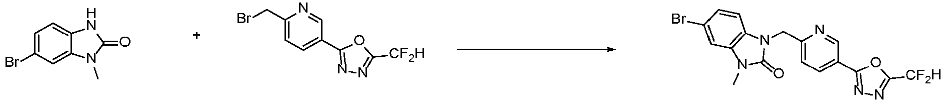

The 6-bromo-1-methyl-1,3-dihydro-2H-benzo[d]imidazole-2-one (1.930 g, 8.500 mmol) prepared in the step 3 was dissolved in N,N-dimethylformamide (40 mL) at 0°C, after which sodium hydride (60.00%, 0.510 g, 12.750 mmol) was added into the resulting solution and stirred at the same temperature for 30 minutes. 2-(6-(bromomethyl)pyridine-3-yl)-5-(difluoromethyl)-1,3,4-oxadiazole (2.712 g, 9.350 mmol) was added into the reaction mixture and further stirred at room temperature for 4 hours. Water was poured into the reaction mixture and an extraction was performed with ethyl acetate. An organic layer was washed with saturated sodium chloride aqueous solution, then dehydrated with anhydrous magnesium sulfate, then filtered, and then concentrated under reduced pressure. The resulting concentrate was purified via column chromatography (SiO₂, 40 g cartridge; ethyl acetate/hexane = 20 to 50%) and concentrated to obtain a title compound (2.060 g, 55.6%) in a light brown solid form.

### [Step 5] Synthesis of the compound 208

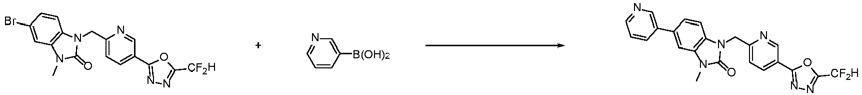

The 5-bromo-1-((5-(5-(difluoromethyl)-1,3,4-oxadiazole-2-yl)pyridine-2-yl)methyl)-3-methyl-1,3-dihyd ro-2H-benzo[d]imidazole-2-one (0.150 g, 0.344 mmol) prepared in the step 4, pyridine-3-ylboronic acid (0.051 g, 0.413 mmol), [1,1'-bis(di-tert-butylphosphino)ferrocene]palladium dichloride (0.011 g, 0.017 mmol) and cesium carbonate (0.224 g, 0.688 mmol) were mixed in 1,4-dioxane (2.3 mL)/water (0.6 mL) at room temperature, after which the resulting mixture was irradiated with microwave, then heated at 100°C for 30 minutes, and then a reaction was finished by lowering a temperature to room temperature. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.081 g, 54.4%) in a brown solid form.

**¹H NMR** (400 MHz, CDCl₃) δ 9.24 (d, *J* = 2.1 Hz, 1H), 8.83 (s, 1H), 8.57 (s, 1H), 8.29 (dd, *J* = 8.2, 2.2 Hz, 1H), 7.81 (d, *J* = 7.9 Hz, 1H), 7.42 (d, *J* = 8.2 Hz, 1H), 7.33 (dd, *J* = 7.4, 4.7 Hz, 1H), 7.21 (dd, *J* = 8.1, 1.6 Hz, 1H), 7.16 (d, *J* = 1.4 Hz, 1H), 7.04 - 6.78 (m, 2H), 5.28 (s, 2H), 3.50 (s, 3H); **LRMS** (ES) m/z 533.1 (M⁺ + 1).

### Example 209: Synthesis of Compound 209, 1-((5-(5-(difluoromethyl)-1,3,4-oxadiazole-2-yl)pyridine-2-yl)methyl)-3-methyl-5-(pyridine-4-yl)-1, 3-dihydro-2H-benzo[d]imidazole-2-one

### [Step 1] Synthesis of the compound 209

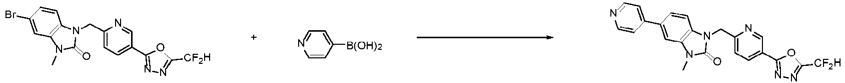

The 5-bromo-1-((5-(5-(difluoromethyl)-1,3,4-oxadiazole-2-yl)pyridine-2-yl)methyl)-3-methyl-1,3-dihyd ro-2H-benzo[d]imidazole-2-one (0.150 g, 0.344 mmol) prepared in the step 4 of the compound 208, pyridine-4-ylboronic acid (0.051 g, 0.413 mmol), [1,1'-bis(di-tert-butylphosphino)ferrocene]palladium dichloride (0.011 g, 0.017 mmol) and cesium carbonate (0.224 g, 0.688 mmol) were mixed in 1,4-dioxane (2.3 mL)/water (0.6 mL) at room temperature, after which the resulting mixture was irradiated with microwave, then heated at 100°C for 30 minutes, and then a reaction was finished by lowering a temperature to room temperature. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.034 g, 22.8%) in a brown solid form.

¹H NMR (400 MHz, CDCl₃) δ 9.27 (dd, *J =* 2.1, 0.6 Hz, 1H), 8.64 (s, 2H), 8.32 (dd, *J =* 8.2, 2.2 Hz, 1H), 7.49 - 7.44 (m, 3H), 7.33 (dd, *J* = 8.1, 1.6 Hz, 1H), 7.25 (d, *J* = 1.5 Hz, 1H), 7.07 - 6.79 (m, 2H), 5.31 (s, 2H), 3.53 (s, 3H); LRMS (ES) m/z 436.1 (M⁺ + 1).

### Example 210: Synthesis of Compound 210, 1-((5-(5-(difluoromethyl)-1,3,4-oxadiazole-2-yl)pyridine-2-yl)methyl)-5-(3,5-dimethylisoxazole-4-y I)-3-(1-methylpiperidine-4-yl)-1,3-dihydro-2H-benzo[d]imidazole-2-one

### [Step 1] Synthesis of the compound 210

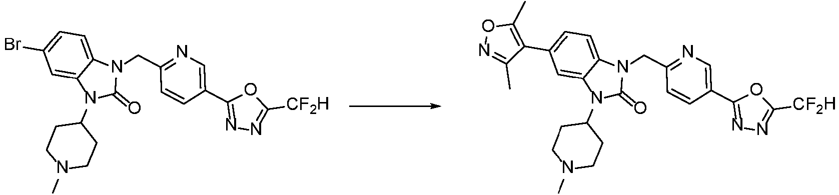

5-bromo-1-((5-(5-(difluoromethyl)-1,3,4-oxadiazole-2-yl)pyridine-2-yl)methyl)-3-(1-methy lpiperidine-4-yl)-1,3-dihydro-2H-benzo[d]imidazole-2-one (0.200 g, 0.385 mmol), (3,5-dimethylisooxazole-4-yl)boronic acid (0.065 g, 0.462 mmol), [1,1'-bis(di-tert-butylphosphino)ferrocene]palladium(II) dichloride (Pd(dtbpf)Cl₂, 0.013 g, 0.019 mmol) and cesium carbonate (0.376 g, 1.155 mmol) were mixed in 1,4-dioxane (1.5 mL)/water (0.5 mL) at room temperature, after which the resulting mixture was irradiated with microwave, then heated at 100°C for 20 minutes, and then a reaction was finished by lowering a temperature to room temperature. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 10%) and concentrated to obtain a title compound (0.100 g, 48.5%) in a brown solid form.

**¹H NMR** (400 MHz, CDCl₃) δ 9.32 (s, 1H), 8.37 (d, *J* = 8.2 Hz, 1H), 7.50 (d, *J* = 8.2 Hz, 1H), 7.28 (s, 1H), 7.25 (brs, 1H), 7.08 - 6.82 (m, 3H), 5.33 (s, 2H), 4.50 - 4.51 (m, 1H), 3.08 - 3.09 (m, 2H), 2.55 - 2.56 (m, 2H), 2.40 (s, 6H), 2.25 - 2.26 (m, 5H), 1.92 (d, *J* = 11.2 Hz, 2H); **LRMS** (ES) m/z 536.3 (M+ + 1).

### Example 211: Synthesis of Compound 211, 1-((5-(5-(difluoromethyl)-1,3,4-oxadiazole-2-yl)pyridine-2-yl)methyl)-5-(isoxazole-4-yl)-3-(1-meth ylpiperidine-4-yl)-1,3-dihydro-2H-benzo[d]imidazole-2-one

### [Step 1] Synthesis of the compound 211

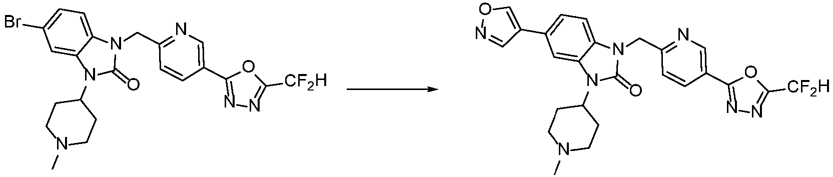

5-bromo-1-((5-(5-(difluoromethyl)-1,3,4-oxadiazole-2-yl)pyridine-2-yl)methyl)-3-(1-methy lpiperidine-4-yl)-1,3-dihydro-2H-benzo[d]imidazole-2-one (0.200 g, 0.385 mmol), benzofuran-2-ylboronic acid (0.057 g, 0.462 mmol), [1,1'-bis(di-tert-butylphosphino)ferrocene]palladium(II) dichloride (Pd(dtbpf)Cl₂, 0.013 g, 0.019 mmol) and cesium carbonate (0.376 g, 1.155 mmol) were mixed in 1,4-dioxane (1.5 mL)/water (0.5 mL) at room temperature, after which the resulting mixture was irradiated with microwave, then heated at 100°C for 20 minutes, and then a reaction was finished by lowering a temperature to room temperature. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 10%) and concentrated to obtain a title compound (0.008 g, 4.1%) in a brown oil form.

**¹H NMR** (400 MHz, CDCl₃) δ 9.31 (d, *J =* 2.0 Hz, 1H), 8.80 (brs, 1H), 8.61 (s, 1H), 8.35 (dd, J = 8.2, 2.2 Hz, 1H), 7.54 (brs, 1H), 7.45 (d, *J* = 8.2 Hz, 1H), 7.17 (d, *J =* 8.1 Hz, 1H), 7.08 - 6.82 (m, 2H), 5.32 (s, 2H), 4.54 - 4.55 (m, 1H), 3.16 - 3.17 (m, 2H), 2.69 (brs, 2H), 2.48 (s, 3H), 2.39 - 2.35 (m, 2H), 1.94 (d, *J* = 10.6 Hz, 2H); **LRMS** (ES) m/z 508.4 (M⁺ + 1).

### Example 212: Synthesis of Compound 212, 1-((5-(5-(difluoromethyl)-1,3,4-oxadiazole-2-yl)pyridine-2-yl)methyl)-5-(1H-indole-4-yl)-3-methyl-1,3-dihydro-2H-benzo[d]imidazole-2-one

### [Step 1] Synthesis of the compound 212

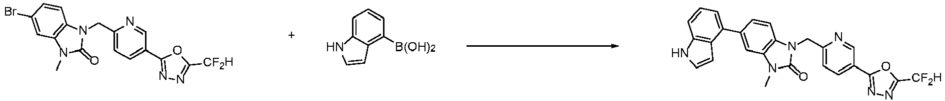

The 5-bromo-1-((5-(5-(difluoromethyl)-1,3,4-oxadiazole-2-yl)pyridine-2-yl)methyl)-3-methyl-1,3-dihyd ro-2H-benzo[d]imidazole-2-one (0.150 g, 0.344 mmol) prepared in the step 4 of the compound 208, (1H-indole-4-yl)boronic acid (0.066 g, 0.413 mmol), [1,1'-bis(di-tert-butylphosphino)ferrocene]palladium dichloride (0.011 g, 0.017 mmol) and cesium carbonate (0.224 g, 0.688 mmol) were mixed in 1,4-dioxane (2.3 mL)/water (0.6 mL) at room temperature, after which the resulting mixture was irradiated with microwave, then heated at 100°C for 30 minutes, and then a reaction was finished by lowering a temperature to room temperature. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.049 g, 30.2%) in a brown solid form.

**¹H NMR** (400 MHz, CDCl₃) δ 9.28 (d, *J =* 1.6 Hz, 1H), 8.65 (s, 1H), 8.28 (dd, *J* = 8.2, 2.2 Hz, 1H), 7.44 - 7.34 (m, 4H), 7.24 - 7.22 (m, 2H), 7.15 (dd, *J* = 7.3, 0.7 Hz, 1H), 7.03 (d, *J = 7.9* Hz, 1H), 6.91 (t, *J =* 52.0 Hz, 1H), 6.68 (t, *J = 2.1* Hz, 1H), 5.34 (s, 2H), 3.52 (s, 3H); LRMS (ES) m/z 473.3 (M⁺ + 1).

### Example 213: Synthesis of Compound 213, 5-chloro-3-((5-(5-(difluoromethyl)-1,3,4-oxadiazole-2-yl)pyridine-2-yl)methyl)-1-(1-methylpiperidi ne-4-yl)-1,3-dihydro-2H-benzo[d]imidazole-2-one

### [Step 1] Synthesis of tert-butyl 4-((4-chloro-2-nitrophenyl)amino)piperidine-1-carboxylate

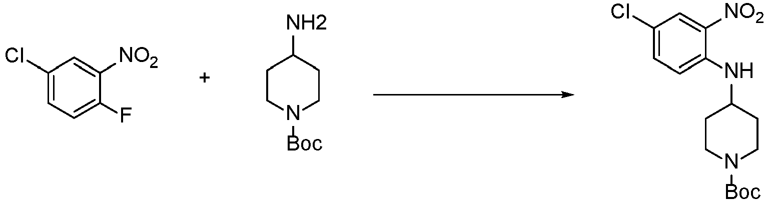

4-chloro-1-fluoro-2-nitrobenzene (5.000 g, 28.484 mmol), tert-butyl 4-aminopiperidine-1-carboxylate (6.275 g, 31.332 mmol), potassium carbonate (7.873 g, 56.967 mmol) and potassium iodide (0.473 g, 2.848 mmol) were dissolved in N,N-dimethylformamide (140 mL) at room temperature, after which the resulting solution was stirred at the same temperature for 18 hours. Water (200 mL) was put into the reaction mixture and stirred, after which a precipitated solid was filtered, then washed with hexane, and then dried to obtain a title compound (10.100 g, 99.7%) in an orange solid form.

### [Step 2] Synthesis of tert-butyl 4-((2-amino-4-chlorophenyl)amino)piperidine-1-carboxylate

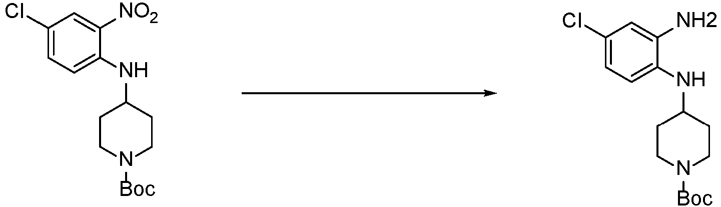

The tert-butyl 4-((4-chloro-2-nitrophenyl)amino)piperidine-1-carboxylate (10.100 g, 28.385 mmol) prepared in the step 1 and Raney nickel (1 g, 10 wt%) were dissolved in methanol (140 mL) at room temperature, after which the resulting solution was stirred at the same temperature for 18 hours in the presence of a hydrogen balloon. The reaction mixture was filtered via a celite pad to remove a solid therefrom, after which solvent was removed from the resulting filtrate under reduced pressure, and then a title compound was used without an additional purification process (9.190 g, 99.4%, black liquid).

### [Step 3] Synthesis of tert-butyl 4-(5-chloro-2-oxo-2,3-dihydro-1H-benzo[d]imidazole-1-yl)piperidine-1-carboxylate

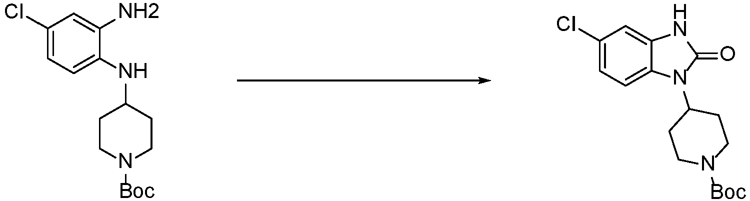

The tert-butyl 4-((2-amino-4-chlorophenyl)amino)piperidine-1-carboxylate (9.190 g, 28.204 mmol) prepared in the step 2, triethylamine (3.931 mL, 28.204 mmol) and 1,1'-carbonyldiimidazole (CDI, 5.488 g, 33.845 mmol) were dissolved in dichloromethane (80 mL) at room temperature, after which the resulting solution was heated under reflux for 18 hours, and then a reaction was finished by lowering a temperature to room temperature. Solvent was removed from the reaction mixture under reduced pressure, after which the resulting concentrate was purified via column chromatography (SiO₂, 80 g cartridge; ethyl acetate/hexane = 30 to 70%) and concentrated to obtain a title compound (5.660 g, 57.0%) in a red solid form.

### [Step 4] Synthesis of tert-butyl 4-(5-chloro-3-((5-(5-(difluoromethyl)-1,3,4-oxadiazole-2-yl)pyridine-2-yl)methyl)-2-oxo-2,3-dihydr o-1H-benzo[d]imidazole-1-yl)piperidine-1-carboxylate

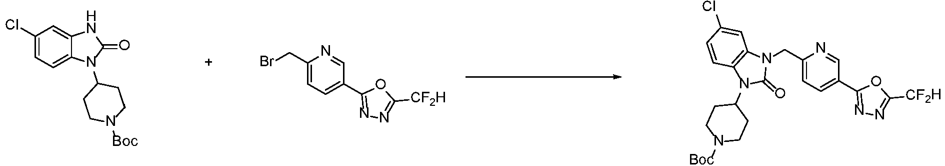

The tert-butyl 4-(5-chloro-2-oxo-2,3-dihydro-1H-benzo[d]imidazole-1-yl)piperidine-1-carboxylate (3.000 g, 8.527 mmol) prepared in the step 3 was dissolved in N,N-dimethylformamide (45 mL) at 0°C, after which sodium hydride (60.00%, 0.512 g, 12.790 mmol) was added into the resulting solution and stirred at the same temperature for 30 minutes. 2-(6-(bromomethyl)pyridine-3-yl)-5-(difluoromethyl)-1,3,4-oxadiazole (2.721 g, 9.380 mmol) was added into the reaction mixture and further stirred at room temperature for 3 hours. Water was poured into the reaction mixture and an extraction was performed with ethyl acetate. An organic layer was washed with saturated sodium chloride aqueous solution, then dehydrated with anhydrous magnesium sulfate, then filtered, and then concentrated under reduced pressure. The resulting concentrate was purified via column chromatography (SiO₂, 40 g cartridge; ethyl acetate/hexane = 30 to 60%) and concentrated to obtain a title compound (3.270 g, 68.4%) in a light yellow solid form.

### [Step 5] Synthesis of 5-chloro-3-((5-(5-(difluoromethyl)-1,3,4-oxadiazole-2-yl)pyridine-2-yl)methyl)-1-(piperidine-4-yl)-1,3-dihydro-2H-benzo[d]imidazole-2-one

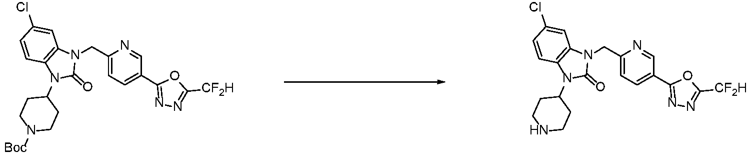

The tert-butyl 4-(5-chloro-3-((5-(5-(difluoromethyl)-1,3,4-oxadiazole-2-yl)pyridine-2-yl)methyl)-2-oxo-2,3-dihydr o-1H-benzo[d]imidazole-1-yl)piperidine-1-carboxylate (3.270 g, 5.829 mmol) prepared in the step 4 and trifluoroacetic acid (4.464 mL, 58.290 mmol) were dissolved in dichloromethane (30 mL) at room temperature, after which the resulting solution was stirred at the same temperature for 5 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 40 g cartridge; ethyl acetate/hexane = 80 to 100%) and concentrated, after which an obtained product was purified again via chromatography (SiO₂, 40 g cartridge; methanol/dichloromethane = 0 to 70%) and concentrated to obtain a title compound (2.100 g, 78.2%) in a light yellow solid form.

### [Step 6] Synthesis of the compound 213

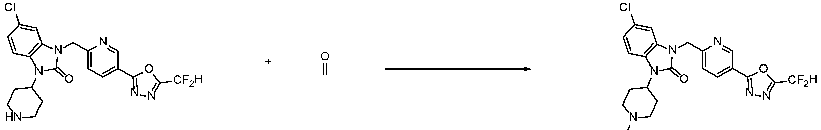

The 5-chloro-3-((5-(5-(difluoromethyl)-1,3,4-oxadiazole-2-yl)pyridine-2-yl)methyl)-1-(piperidine-4-yl)-1,3-dihydro-2H-benzo[d]imidazole-2-one (0.100 g, 0.217 mmol) prepared in the step 5, formaldehyde (0.020 g, 0.651 mmol) and sodium triacetoxyborohydride (0.092 g, 0.434 mmol) were dissolved in dichloromethane (2 mL) at room temperature, after which the resulting solution was stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.079 g, 77.1%) in a white solid form.

**¹H NMR** (400 MHz, CDCl₃) δ 9.26 (s, 1H), 8.31 (dd, *J* = 8.2, 1.9 Hz, 1H), 7.38 (d, *J* = 8.2 Hz, 1H), 7.20 (d, *J =* 8.4 Hz, 1H), 7.04 - 6.79 (m, 3H), 5.22 (s, 2H), 4.42 - 4.35 (m, 1H), 3.00 (d, *J* = 11.5 Hz, 2H), 2.47 - 2.37 (m, 2H), 2.32 (s, 3H), 2.14 (t, *J* = 11.8 Hz, 2H), 1.82 (d, *J =* 11.8 Hz, 2H); **LRMS** (ES) m/z 477.2 (M⁺ + 1).

### Example 214: Synthesis of Compound 214, 5-chloro-3-((5-(5-(difluoromethyl)-1,3,4-oxadiazole-2-yl)pyridine-2-yl)methyl)-1-(1-isopropylpiperi dine-4-yl)-1,3-dihydro-2H-benzo[d]imidazole-2-one

### [Step 1] Synthesis of the compound 214

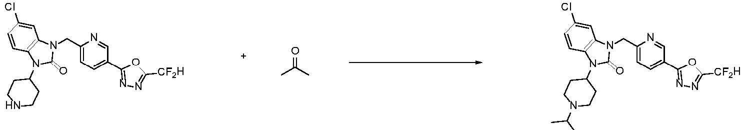

The 5-chloro-3-((5-(5-(difluoromethyl)-1,3,4-oxadiazole-2-yl)pyridine-2-yl)methyl)-1-(piperidine-4-yl)-1,3-dihydro-2H-benzo[d]imidazole-2-one (0.100 g, 0.217 mmol) prepared in the step 5 of the compound 213, propane-2-one (0.038 g, 0.651 mmol) and sodium triacetoxyborohydride (0.092 g, 0.434 mmol) were dissolved in dichloromethane (2 mL) at room temperature, after which the resulting solution was stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.075 g, 68.7%) in a white solid form.

**¹H NMR** (400 MHz, CDCl₃) δ 9.28 (d, *J =* 2.1 Hz, 1H), 8.33 (dd, *J* = 8.2, 2.2 Hz, 1H), 7.40 (d, *J =* 8.2 Hz, 1H), 7.26 (t, *J* = 4.2 Hz, 1H), 7.05 - 6.79 (m, 3H), 5.24 (s, 2H), 4.41 - 4.35 (m, 1H), 3.03 (d, *J* = 7.9 Hz, 2H), 2.85 - 2.79 (m, 1H), 2.43 - 2.31 (m, 4H), 1.86 (d, *J =* 9.0 Hz, 2H), 1.07 (d, *J* = 6.6 Hz, 6H); **LRMS** (ES) m/z 503.1 (M⁺ + 1).

### Example 215: Synthesis of Compound 215, 1-(1-acetylpiperidine-4-yl)-5-chloro-3-((5-(5-(difluoromethyl)-1,3,4-oxadiazole-2-yl)pyridine-2-yl) methyl)-1,3-dihydro-2H-benzo[d]imidazole-2-one

### [Step 1] Synthesis of the compound 215

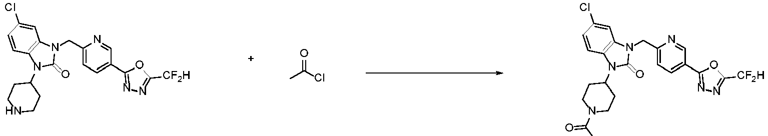

The 5-chloro-3-((5-(5-(difluoromethyl)-1,3,4-oxadiazole-2-yl)pyridine-2-yl)methyl)-1-(piperidine-4-yl)-1,3-dihydro-2H-benzo[d]imidazole-2-one (0.100 g, 0.217 mmol) prepared in the step 5 of the compound 213, acetyl chloride (0.031 mL, 0.434 mmol) and triethylamine (0.060 mL, 0.434 mmol) were dissolved in dichloromethane (2 mL) at room temperature, after which the resulting solution was stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.096 g, 88.0%) in a white solid form.

**¹H NMR** (400 MHz, CDCl₃) δ 9.27 (dd, *J* = 2.1, 0.6 Hz, 1H), 8.34 (dd, *J* = 8.2, 2.2 Hz, 1H), 7.42 (d, *J = 8.2* Hz, 1H), 7.05 - 7.00 (m, 3H), 6.93 (t, *J* = 51.6 Hz, 1H), 5.23 (d, *J* = 1.9 Hz, 2H), 4.87 (dd, *J* = 11.6, 1.9 Hz, 1H), 4.58 - 4.50 (m, 1H), 4.01 (d, *J* = 13.8 Hz, 1H), 3.26 - 3.20 (m, 1H), 2.70 - 2.63 (m, 1H), 2.40 - 2.22 (m, 2H), 2.16 (s, 3H), 1.92 (t, *J* = 14.0 Hz, 2H); **LRMS** (ES) m/z 503.1 (M⁺ + 1).

### Example 216: Synthesis of Compound 216, 5-chloro-3-((5-(5-(difluoromethyl)-1,3,4-oxadiazole-2-yl)pyridine-2-yl)methyl)-1-(1-(methylsulfony I)piperidine-4-yl)-1,3-dihydro-2H-benzo[d]imidazole-2-one

### [Step 1] Synthesis of the compound 216

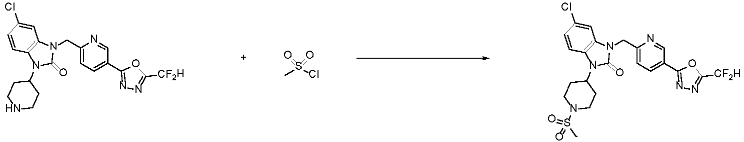

The 5-chloro-3-((5-(5-(difluoromethyl)-1,3,4-oxadiazole-2-yl)pyridine-2-yl)methyl)-1-(piperidine-4-yl)-1,3-dihydro-2H-benzo[d]imidazole-2-one (0.100 g, 0.217 mmol) prepared in the step 5 of the compound 213, methanesulfonyl chloride (0.034 mL, 0.434 mmol) and triethylamine (0.060 mL, 0.434 mmol) were dissolved in dichloromethane (2 mL) at room temperature, after which the resulting solution was stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.079 g, 67.6%) in a white solid form.

**¹H NMR** (400 MHz, CDCl₃) δ 9.28 (d, *J =* 1.4 Hz, 1H), 8.35 (dd, *J* = 8.2, 2.0 Hz, 1H), 7.42 (d, *J* = 8.2 Hz, 1H), 7.13 - 6.79 (m, 4H), 5.24 (s, 2H), 4.56 - 4.47 (m, 1H), 4.03 (d, *J =* 12.3 Hz, 2H), 2.90 - 2.85 (m, 5H), 2.56 - 2.45 (m, 2H), 1.97 (d, *J =* 10.7 Hz, 2H); **LRMS** (ES) m/z 539.3 (M⁺ + 1).

### Example 217: Synthesis of Compound 217, 1-((5-(5-(difluoromethyl)-1,3,4-oxadiazole-2-yl)pyridine-2-yl)methyl)-6-fluoro-5-(1H-indole-4-yl)-3 -(1-methylpiperidine-4-yl)-1,3-dihydro-2H-benzo[d]imidazole-2-one

### [Step 1] Synthesis of the compound 217

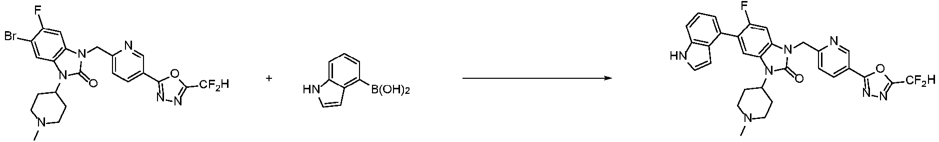

The 5-bromo-1-((5-(5-(difluoromethyl)-1,3,4-oxadiazole-2-yl)pyridine-2-yl)methyl)-6-fluoro-3-(1-methy lpiperidine-4-yl)-1,3-dihydro-2H-benzo[d]imidazole-2-one (0.150 g, 0.279 mmol) prepared in the step 3 of the compound 149, (1H-indole-4-yl)boronic acid (0.054 g, 0.335 mmol), [1,1'-bis(di-tert-butylphosphino)ferrocene]palladium dichloride (0.009 g, 0.014 mmol) and cesium carbonate (0.182 g, 0.558 mmol) were mixed in 1,4-dioxane (2.3 mL)/water (0.6 mL) at room temperature, after which the resulting mixture was irradiated with microwave, then heated at 100°C for 30 minutes, and then a reaction was finished by lowering a temperature to room temperature. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.026 g, 16.2%) in a brown solid form.

**¹H NMR** (400 MHz, CDCl₃) δ 9.31 (d, *J =* 1.8 Hz, 1H), 8.62 (s, 1H), 8.35 (dd, *J* = 8.2, 2.2 Hz, 1H), 7.47 (d, *J* = 8.2 Hz, 1H), 7.42 - 7.39 (m, 2H), 7.26 - 7.20 (m, 2H), 7.10 (d, *J* = 7.2 Hz, 1H), 7.08 (t, *J =* 146.7 Hz, 1H), 6.89 (d, *J* = 9.3 Hz, 1H), 6.40 (d, *J* = 1.6 Hz, 1H), 5.30 (s, 2H), 4.47 - 4.39 (m, 1H), 2.99 (d, *J =* 11.6 Hz, 2H), 2.52 - 2.42 (m, 2H), 2.30 (s, 3H), 2.14 (t, *J =* 11.1 Hz, 2H), 1.87 (d, *J =* 10.1 Hz, 2H); **LRMS** (ES) m/z 574.2 (M⁺ + 1).

### Example 218: Synthesis of Compound 218, 1-((5-(5-(difluoromethyl)-1,3,4-oxadiazole-2-yl)pyridine-2-yl)methyl)-3-methyl-5-(5-methylfuran-2 -yl)-1,3-dihydro-2H-benzo[d]imidazole-2-one

### [Step 1] Synthesis of the compound 218

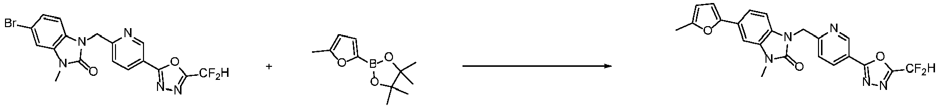

The 5-bromo-1-((5-(5-(difluoromethyl)-1,3,4-oxadiazole-2-yl)pyridine-2-yl)methyl)-3-methyl-1,3-dihyd ro-2H-benzo[d]imidazole-2-one (0.150 g, 0.344 mmol) prepared in the step 4 of the compound 208, 4,4,5,5-tetramethyl-2-(5-methylfuran-2-yl)-1,3,2-dioxaborolane (0.086 g, 0.413 mmol), [1,1'-bis(di-tert-butylphosphino)ferrocene]palladium dichloride (0.011 g, 0.017 mmol) and cesium carbonate (0.224 g, 0.688 mmol) were mixed in 1,4-dioxane (2.3 mL)/water (0.6 mL) at room temperature, after which the resulting mixture was irradiated with microwave, then heated at 100°C for 30 minutes, and then a reaction was finished by lowering a temperature to room temperature. Saturated sodium hydrogen carbonate aqueous solution was poured into the reaction mixture and an extraction was performed with ethyl acetate. An organic layer was washed with saturated sodium chloride aqueous solution, then dehydrated with anhydrous magnesium sulfate, then filtered, and then concentrated under reduced pressure. The resulting concentrate was purified via column chromatography (SiO₂, 4 g cartridge; methanol/dichloromethane = 0 to 2%) and concentrated to obtain a title compound (0.073 g, 48.5%) in a brown solid form.

¹H NMR (400 MHz, CDCl₃) δ 9.26 (d, *J =* 1.6 Hz, 1H), 8.29 (dd, *J* = 8.2, 2.2 Hz, 1H), 7.40 (d, *J* = 8.2 Hz, 1H), 7.29 (dd, *J* = 8.2, 1.5 Hz, 1H), 7.26 (d, *J* = 1.3 Hz, 1H), 7.05 - 6.79 (m, 2H), 6.44 (d, *J =* 3.2 Hz, 1H), 6.03 - 6.02 (m, 1H), 5.27 (s, 2H), 3.50 (s, 3H), 2.36 (d, *J* = 0.2 Hz, 3H); **LRMS** (ES) m/z 437.9 (M⁺ + 1).

### Example 219: Synthesis of Compound 219, 1-((5-(5-(difluoromethyl)-1,3,4-oxadiazole-2-yl)pyridine-2-yl)methyl)-3-methyl-5-(thiophene-3-yl)-1,3-dihydro-2H-benzo[d]imidazole-2-one

### [Step 1] Synthesis of the compound 219

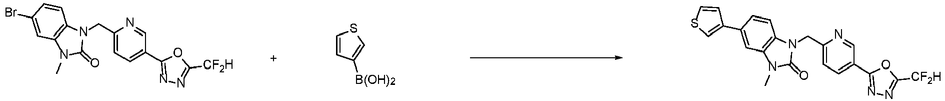

The 5-bromo-1-((5-(5-(difluoromethyl)-1,3,4-oxadiazole-2-yl)pyridine-2-yl)methyl)-3-methyl-1,3-dihyd ro-2H-benzo[d]imidazole-2-one (0.150 g, 0.344 mmol) prepared in the step 4 of the compound 208, thiophene-3-ylboronic acid (0.053 g, 0.413 mmol), [1,1'-bis(di-tert-butylphosphino)ferrocene]palladium dichloride (0.011 g, 0.017 mmol) and cesium carbonate (0.224 g, 0.688 mmol) were mixed in 1,4-dioxane (2.3 mL)/water (0.6 mL) at room temperature, after which the resulting mixture was irradiated with microwave, then heated at 100°C for 30 minutes, and then a reaction was finished by lowering a temperature to room temperature. Saturated sodium hydrogen carbonate aqueous solution was poured into the reaction mixture and an extraction was performed with ethyl acetate. An organic layer was washed with saturated sodium chloride aqueous solution, then dehydrated with anhydrous magnesium sulfate, then filtered, and then concentrated under reduced pressure. The resulting concentrate was purified via column chromatography (SiO₂, 4 g cartridge; methanol/dichloromethane = 0 to 2%) and concentrated to obtain a title compound (0.065 g, 43.0%) in a brown solid form.

**¹H NMR** (400 MHz, CDCl₃) δ 9.17 (d, *J =* 1.8 Hz, 1H), 8.20 (dd, *J* = 8.2, 2.2 Hz, 1H), 7.31 (d, *J* = 8.2 Hz, 1H), 7.28 - 7.22 (m, 3H), 7.16 (dd, *J* = 8.1, 1.6 Hz, 1H), 7.09 (d, *J =* 1.4 Hz, 1H), 6.85 (d, *J* = 8.1 Hz, 1H), 6.81 (t, *J* = 51.6 Hz, 1H), 5.19 (s, 2H), 3.41 (s, 3H); **LRMS** (ES) m/z 440.1 (M⁺+ 1).

### Example 220: Synthesis of Compound 220, 1-((5-(5-(difluoromethyl)-1,3,4-oxadiazole-2-yl)pyridine-2-yl)methyl)-3-methyl-5-(4-(methylsulfon yl)phenyl)-1,3-dihydro-2H-benzo[d]imidazole-2-one

**[Step 1] Synthesis of the compound 220**

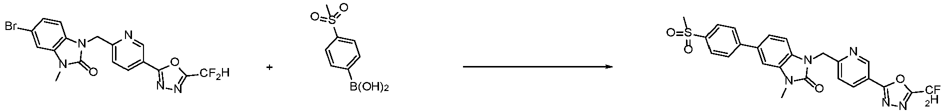

The 5-bromo-1-((5-(5-(difluoromethyl)-1,3,4-oxadiazole-2-yl)pyridine-2-yl)methyl)-3-methyl-1,3-dihyd ro-2H-benzo[d]imidazole-2-one (0.150 g, 0.344 mmol) prepared in the step 4 of the compound 208, (4-(methylsulfonyl)phenyl)boronic acid (0.083 g, 0.413 mmol), [1,1'-bis(di-tert-butylphosphino)ferrocene]palladium dichloride (0.011 g, 0.017 mmol) and cesium carbonate (0.224 g, 0.688 mmol) were mixed in 1,4-dioxane (2.3 mL)/water (0.6 mL) at room temperature, after which the resulting mixture was irradiated with microwave, then heated at 100°C for 30 minutes, and then a reaction was finished by lowering a temperature to room temperature. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 2%) and concentrated to obtain a title compound (0.096 g, 54.4%) in a white solid form.

**¹H NMR** (400 MHz, CDCl₃) δ 9.24 (d, *J =* 2.0 Hz, 1H), 8.30 (dd, *J* = 8.2, 2.2 Hz, 1H), 7.95 (d, *J* = 8.4 Hz, 2H), 7.72 (dd, *J* = 6.8, 1.7 Hz, 2H), 7.44 (d, *J =* 8.2 Hz, 1H), 7.27 (dd, *J* = 8.2, 1.5 Hz, 1H), 7.22 (d, *J =* 1.5 Hz, 1H), 7.05 (d, *J =* 8.2 Hz, 1H), 6.92 (t, *J =* 51.6 Hz, 1H), 5.30 (s, 2H), 3.52 (s, 3H), 3.06 (s, 3H); **LRMS** (ES) m/z 512.3 (M⁺ + 1).

### Example 221: Synthesis of Compound 221, 1-((5-(5-(difluoromethyl)-1,3,4-oxadiazole-2-yl)pyridine-2-yl)methyl)-3-methyl-5-(1H-pyrazole-5-y I)-1,3-dihydro-2H-benzo[d]imidazole-2-one

### [Step 1] Synthesis of the compound 221

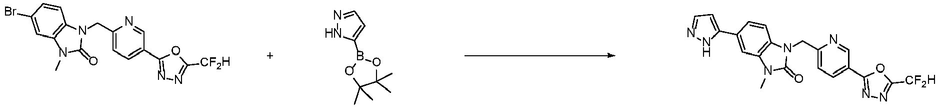

The 5-bromo-1-((5-(5-(difluoromethyl)-1,3,4-oxadiazole-2-yl)pyridine-2-yl)methyl)-3-methyl-1,3-dihyd ro-2H-benzo[d]imidazole-2-one (0.150 g, 0.344 mmol) prepared in the step 4 of the compound 208, 5-(4,4,5,5-tetramethyl-1,3,2-dioxaborolane-2-yl)-1H-pyrazole (0.080 g, 0.413 mmol), [1,1'-bis(di-tert-butylphosphino)ferrocene]palladium dichloride (0.011 g, 0.017 mmol) and cesium carbonate (0.224 g, 0.688 mmol) were mixed in 1,4-dioxane (2.3 mL)/water (0.6 mL) at room temperature, after which the resulting mixture was irradiated with microwave, then heated at 100°C for 30 minutes, and then a reaction was finished by lowering a temperature to room temperature. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.022 g, 15.1%) in a brown solid form.

¹H NMR (400 MHz, CDCl₃) δ 9.26 (d, *J =* 1.7 Hz, 1H), 8.29 (dd, *J* = 8.2, 2.2 Hz, 1H), 7.59 (d, *J =* 2.2 Hz, 1H), 7.46 - 7.39 (m, 3H), 6.95 (d, *J =* 8.1 Hz, 1H), 6.92 (t, *J =* 51.6 Hz, 1H), 6.57 (d, *J = 1.0* Hz, 1H), 5.29 (s, 2H), 3.47 (s, 3H); LRMS (ES) m/z 424.3 (M⁺ + 1).

### Example 222: Synthesis of Compound 222, 1-((5-(5-(difluoromethyl)-1,3,4-oxadiazole-2-yl)pyridine-2-yl)methyl)-3-methyl-5-(1-methyl-1H-pyr azole-5-yl)-1,3-dihydro-2H-benzo[d]imidazole-2-one

### [Step 1] Synthesis of the compound 222

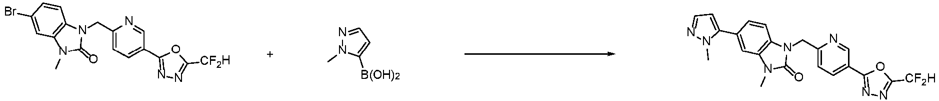

The 5-bromo-1-((5-(5-(difluoromethyl)-1,3,4-oxadiazole-2-yl)pyridine-2-yl)methyl)-3-methyl-1,3-dihyd ro-2H-benzo[d]imidazole-2-one (0.150 g, 0.344 mmol) prepared in the step 4 of the compound 208, (1-methyl-1H-pyrazole-5-yl)boronic acid (0.052 g, 0.413 mmol), [1,1'-bis(di-tert-butylphosphino)ferrocene]palladium dichloride (0.011 g, 0.017 mmol) and cesium carbonate (0.224 g, 0.688 mmol) were mixed in 1,4-dioxane (2.3 mL)/water (0.6 mL) at room temperature, after which the resulting mixture was irradiated with microwave, then heated at 100°C for 30 minutes, and then a reaction was finished by lowering a temperature to room temperature. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 2%) and concentrated to obtain a title compound (0.018 g, 11.8%) in a brown solid form.

¹H NMR (400 MHz, CDCl₃) δ 9.29 (d, *J =* 1.8 Hz, 1H), 8.34 (dd, *J* = 8.2, 2.2 Hz, 1H), 7.50 (d, *J =* 1.9 Hz, 1H), 7.47 (d, *J = 8.2* Hz, 1H), 7.09 - 6.79 (m, 4H), 6.27 (d, *J =* 1.9 Hz, 1H), 5.32 (s, 2H), 3.86 (s, 3H), 3.51 (s, 3H); **LRMS** (ES) m/z 438.0 (M⁺+ 1).

### Example 223: Synthesis of Compound 223, 1-((5-(5-(difluoromethyl)-1,3,4-oxadiazole-2-yl)pyridine-2-yl)methyl)-5-(2,4-difluorophenyl)-6-fluo ro-3-(1-methylpiperidine-4-yl)-1,3-dihydro-2H-benzo[d]imidazole-2-one

### [Step 1] Synthesis of the compound 223

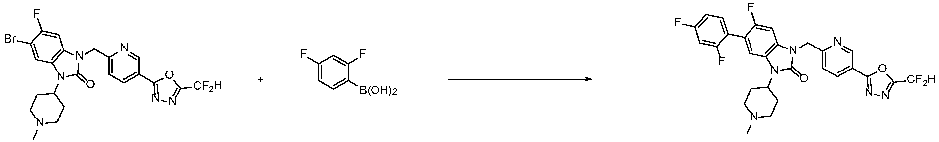

The 5-bromo-1-((5-(5-(difluoromethyl)-1,3,4-oxadiazole-2-yl)pyridine-2-yl)methyl)-6-fluoro-3-(1-methy lpiperidine-4-yl)-1,3-dihydro-2H-benzo[d]imidazole-2-one (0.150 g, 0.279 mmol) prepared in the step 3 of the compound 149, (2,4-difluorophenyl)boronic acid (0.053 g, 0.335 mmol), [1,1'-bis(di-tert-butylphosphino)ferrocene]palladium dichloride (0.009 g, 0.014 mmol) and cesium carbonate (0.182 g, 0.558 mmol) were mixed in 1,4-dioxane (2.3 mL)/water (0.6 mL) at room temperature, after which the resulting mixture was irradiated with microwave, then heated at 100°C for 30 minutes, and then a reaction was finished by lowering a temperature to room temperature. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.042 g, 26.4%) in a brown solid form.

**¹H NMR** (400 MHz, CDCl₃) δ 9.29 (d, *J =* 1.6 Hz, 1H), 8.35 (dd, *J* = 8.2, 2.2 Hz, 1H), 7.46 (d, *J* = 8.2 Hz, 1H), 7.32 - 7.26 (m, 1H), 7.21 (d, *J =* 5.8 Hz, 1H), 7.05 - 6.79 (m, 4H), 5.26 (s, 2H), 4.47 - 4.39 (m, 1H), 3.01 (d, *J* = 11.6 Hz, 2H), 2.49 - 2.38 (m, 2H), 2.33 (s, 3H), 2.16 (t, *J* = 11.1 Hz, 2H), 1.86 (dd, *J =* 11.9, 2.0 Hz, 2H); **LRMS** (ES) m/z 571.1 (M⁺ + 1).

### Example 224: Synthesis of Compound 224, 1-((5-(5-(difluoromethyl)-1,3,4-oxadiazole-2-yl)pyridine-2-yl)methyl)-6-fluoro-5-(1H-indole-4-yl)-3 -methyl-1,3-dihydro-2H-benzo[d]imidazole-2-one

### [Step 1] Synthesis of the compound 224

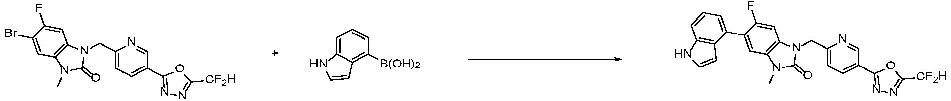

The 5-bromo-1-((5-(5-(difluoromethyl)-1,3,4-oxadiazole-2-yl)pyridine-2-yl)methyl)-6-fluoro-3-methyl-1 ,3-dihydro-2H-benzo[d]imidazole-2-one (0.150 g, 0.330 mmol) prepared in the step 4 of the compound 147, (1H-indole-4-yl)boronic acid (0.064 g, 0.396 mmol), [1,1'-bis(di-tert-butylphosphino)ferrocene]palladium dichloride (0.011 g, 0.017 mmol) and cesium carbonate (0.215 g, 0.660 mmol) were mixed in 1,4-dioxane (2.3 mL)/water (0.6 mL) at room temperature, after which the resulting mixture was irradiated with microwave, then heated at 100°C for 30 minutes, and then a reaction was finished by lowering a temperature to room temperature. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.054 g, 33.6%) in a yellow solid form.

**¹H NMR** (400 MHz, CDCl₃) δ 9.29 (d, *J* = 1.6 Hz, 1H), 8.59 (s, 1H), 8.31 (dd, *J* = 8.2, 2.2 Hz, 1H), 7.45 (d, *J* = 8.2 Hz, 1H), 7.38 (d, *J =* 8.1 Hz, 1H), 7.26 - 7.20 (m, 2H), 7.13 (d, *J* = 6.1 Hz, 2H), 6.92 (t, *J* = 51.7 Hz, 1H), 6.87 (d, *J* = 9.4 Hz, 1H), 6.44 (d, *J* = 2.0 Hz, 1H), 5.30 (s, 2H), 3.46 (s, 3H); **LRMS** (ES) m/z 491.3 (M⁺ + 1).

### Example 225: Synthesis of Compound 225, 1-((5-(5-(difluoromethyl)-1,3,4-oxadiazole-2-yl)pyridine-2-yl)methyl)-6-fluoro-3-methyl-5-(4-(meth ylsulfonyl)phenyl)-1,3-dihydro-2H-benzo[d]imidazole-2-one

### [Step 1] Synthesis of the compound 225

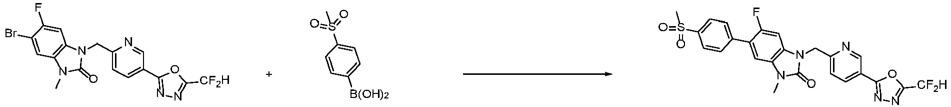

The 5-bromo-1-((5-(5-(difluoromethyl)-1,3,4-oxadiazole-2-yl)pyridine-2-yl)methyl)-6-fluoro-3-methyl-1 ,3-dihydro-2H-benzo[d]imidazole-2-one (0.150 g, 0.330 mmol) prepared in the step 4 of the compound 147, (4-(methylsulfonyl)phenyl)boronic acid (0.079 g, 0.396 mmol), [1,1'-bis(di-tert-butylphosphino)ferrocene]palladium dichloride (0.011 g, 0.017 mmol) and cesium carbonate (0.215 g, 0.660 mmol) were mixed in 1,4-dioxane (2.3 mL)/water (0.6 mL) at room temperature, after which the resulting mixture was irradiated with microwave, then heated at 100°C for 30 minutes, and then a reaction was finished by lowering a temperature to room temperature. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 2%) and concentrated to obtain a title compound (0.073 g, 41.5%) in a yellow solid form.

**¹H NMR** (400 MHz, CDCl₃) δ 9.26 (d, *J* = 1.8 Hz, 1H), 8.34 (dd, *J* = 8.2, 2.2 Hz, 1H), 7.96 (d, *J* = 8.4 Hz, 2H), 7.69 (dd, *J* = 8.3, 1.3 Hz, 2H), 7.61 (d, *J* = 110.4 Hz, 1H), 7.00 (d, *J* = 6.2 Hz, 1H), 6.93 (t, *J =* 51.6 Hz, 1H), 6.89 (d, *J* = 10.0 Hz, 1H), 5.27 (s, 2H), 3.49 (s, 3H), 3.07 (s, 3H); **LRMS** (ES) m/z 530.3 (M⁺+ 1).

### Example 226: Synthesis of Compound 226, 1-((5-(5-(difluoromethyl)-1,3,4-oxadiazole-2-yl)pyridine-2-yl)methyl)-6-fluoro-3-methyl-5-(1-meth yl-1H-pyrazole-5-yl)-1,3-dihydro-2H-benzo[d]imidazole-2-one

### [Step 1] Synthesis of the compound 226

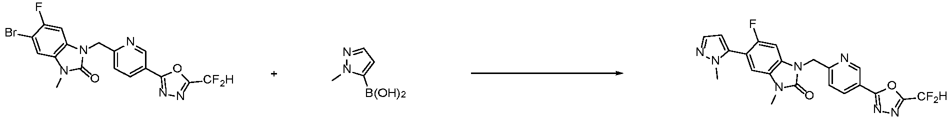

The 5-bromo-1-((5-(5-(difluoromethyl)-1,3,4-oxadiazole-2-yl)pyridine-2-yl)methyl)-6-fluoro-3-methyl-1 ,3-dihydro-2H-benzo[d]imidazole-2-one (0.150 g, 0.330 mmol) prepared in the step 4 of the compound 147, (1-methyl-1H-pyrazole-5-yl)boronic acid (0.050 g, 0.396 mmol), [1,1'-bis(di-tert-butylphosphino)ferrocene]palladium dichloride (0.011 g, 0.017 mmol) and cesium carbonate (0.215 g, 0.660 mmol) were mixed in 1,4-dioxane (2.3 mL)/water (0.6 mL) at room temperature, after which the resulting mixture was irradiated with microwave, then heated at 100°C for 30 minutes, and then a reaction was finished by lowering a temperature to room temperature. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 2%) and concentrated to obtain a title compound (0.014 g, 9.4%) in a brown solid form.

**¹H NMR** (400 MHz, CDCl₃) δ 9.30 (d, *J* = 1.6 Hz, 1H), 8.37 (dd, *J* = 8.2, 2.2 Hz, 1H), 7.54 (d, *J =* 1.9 Hz, 1H), 7.51 (d, *J = 8.2* Hz, 1H), 7.06 - 6.80 (m, 3H), 6.29 (d, *J* = 1.9 Hz, 1H), 5.29 (s, 2H), 3.78 (d, *J* = 1.5 Hz, 3H), 3.48 (s, 3H); **LRMS** (ES) m/z 456.22 (M⁺+ 1).

### Example 227: Synthesis of Compound 227, 1-((5-(5-(difluoromethyl)-1,3,4-oxadiazole-2-yl)pyridine-2-yl)methyl)-6-fluoro-3-methyl-5-(1-meth yl-1H-indole-5-yl)-1,3-dihydro-2H-benzo[d]imidazole-2-one

### [Step 1] Synthesis of the compound 227

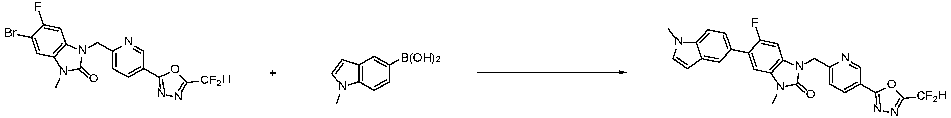

The 5-bromo-1-((5-(5-(difluoromethyl)-1,3,4-oxadiazole-2-yl)pyridine-2-yl)methyl)-6-fluoro-3-methyl-1 ,3-dihydro-2H-benzo[d]imidazole-2-one (0.150 g, 0.330 mmol) prepared in the step 4 of the compound 147, (1-methyl-1H-indole-5-yl)boronic acid (0.069 g, 0.396 mmol), [1,1'-bis(di-tert-butylphosphino)ferrocene]palladium dichloride (0.011 g, 0.017 mmol) and cesium carbonate (0.215 g, 0.660 mmol) were mixed in 1,4-dioxane (2.3 mL)/water (0.6 mL) at room temperature, after which the resulting mixture was irradiated with microwave, then heated at 100°C for 30 minutes, and then a reaction was finished by lowering a temperature to room temperature. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 2%) and concentrated to obtain a title compound (0.047 g, 28.3%) in a yellow solid form.

**¹H NMR** (400 MHz, CDCl₃) δ 9.30 (d, *J* = 1.6 Hz, 1H), 8.33 (dd, *J* = 8.2, 2.2 Hz, 1H), 7.74 (d, *J* = 1.1 Hz, 1H), 7.45 (d, *J* = 8.2 Hz, 1H), 7.37 (s, 2H), 7.09 - 6.80 (m, 4H), 6.52 (d, *J* = 3.1 Hz, 1H), 5.29 (s, 2H), 3.82 (s, 3H), 3.50 (s, 3H); **LRMS** (ES) m/z 505.4 (M⁺+ 1).

### Example 228: Synthesis of Compound 228, 5-bromo-6-chloro-3-((5-(5-(difluoromethyl)-1,3,4-oxadiazole-2-yl)pyridine-2-yl)methyl)benzo[d]ox azole-2(3H)-one

### [Step 1] Synthesis of 5-bromo-6-chlorobenzo[d]oxazole-2(3H)-one

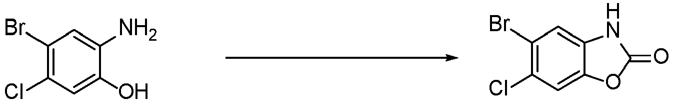

2-amino-4-bromo-5-chlorophenol (0.200 g, 0.899 mmol) and 1,1'-carbonyldiimidazole (CDI, 0.219 g, 1.348 mmol) were dissolved in tetrahydrofuran (10 mL) at room temperature, after which the resulting solution was stirred at the same temperature for 18 hours. Water was poured into the reaction mixture and an extraction was performed with ethyl acetate. An organic layer was washed with saturated sodium chloride aqueous solution, then dehydrated with anhydrous sodium sulfate, then filtered, and then concentrated under reduced pressure. The resulting concentrate was purified via column chromatography (SiO2, 12 g cartridge; ethyl acetate/hexane = 0 to 30%) and concentrated to obtain a title compound (0.190 g, 85.1%) in a white solid form.

### [Step 2] Synthesis of the compound 228

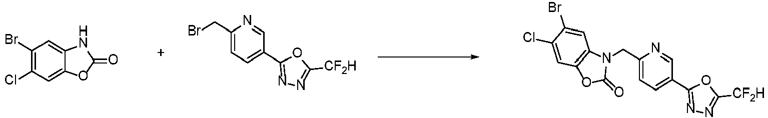

The 5-bromo-6-chlorobenzo[d]oxazole-2(3H)-one (0.190 g, 0.765 mmol) prepared in the step 1 was dissolved in N,N-dimethylformamide (10 mL) at 0°C, after which sodium hydride (60.00%, 0.046 g, 1.147 mmol) was added into the resulting solution and stirred at the same temperature for 30 minutes. 2-(6-(bromomethyl)pyridine-3-yl)-5-(difluoromethyl)-1,3,4-oxadiazole (0.244 g, 0.841 mmol) was added into the reaction mixture and further stirred at room temperature for 3 hours. Water was poured into the reaction mixture and an extraction was performed with ethyl acetate. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; ethyl acetate/hexane = 0 to 50%) and concentrated to obtain a title compound (0.253 g, 72.3%) in a white solid form.

**¹H NMR** (400 MHz, CDCl₃) δ 9.31 (dd, *J* = 2.2, 0.8 Hz, 1H), 8.45 (dd, *J* = 8.2, 2.2 Hz, 1H), 7.57 (d, *J = 8.2* Hz, 1H), 7.39 (s, 1H), 7.33 (s, 1H), 7.08 (s, 0.25H), 6.95 (s, 0.5H), 6.83 (s, 0.25H), 5.20 (s, 2H); **LRMS** (ES) m/z 459.1 (M⁺+ 1).

### Example 229: Synthesis of Compound 229, 6-chloro-3-((5-(5-(difluoromethyl)-1,3,4-oxadiazole-2-yl)pyridine-2-yl)methyl)-5-(pyridine-3-yl)ben zo[d]oxazole-2(3H)-one

### [Step 1] Synthesis of the compound 229

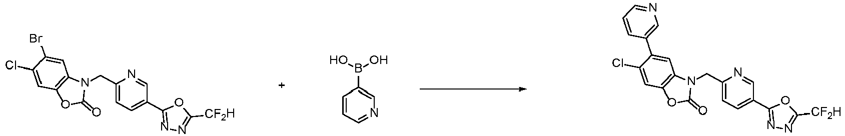

5-bromo-6-chloro-3-((5-(5-(difluoromethyl)-1,3,4-oxadiazole-2-yl)pyridine-2-yl)methyl)be nzo[d]oxazole-2(3H)-one (0.200 g, 0.437 mmol), pyridine-3-ylboronic acid (0.054 g, 0.437 mmol), [1,1'-bis(di-tert-butylphosphino)ferrocene]palladium(II) dichloride (Pd(dtbpf)Cl₂, 0.028 g, 0.044 mmol) and cesium carbonate (0.214 g, 0.656 mmol) were mixed in 1,4-dioxane (10 mL)/water (3 mL), after which the resulting mixture was irradiated with microwave, then heated at 100°C for 20 minutes, and then a reaction was finished by lowering a temperature to room temperature. Water was poured into the reaction mixture and an extraction was performed with ethyl acetate. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; ethyl acetate/hexane = 0 to 70%) and concentrated to obtain a title compound (0.100 g, 50.2%) in a white solid form.

**¹H NMR** (400 MHz, CDCl₃) δ 9.27 (dd, *J* = 2.2, 0.8 Hz, 1H), 8.65 (dd, *J* = 4.9, 1.6 Hz, 1H), 8.62 (d, *J* = 1.6 Hz, 1H), 8.43 (dd, *J* = 8.2, 2.2 Hz, 1H), 7.77 ~ 7.74 (m, 1H), 7.59 (dd, *J* = 8.2, 0.7 Hz, 1H), 7.44 (s, 1H), 7.41 ~ 7.36 (m, 1H), 7.07 (s, 0.25H), 7.03 (s, 1H), 6.95 (s, 0.5H), 6.82 (s, 0.25H), 5.23 (s, 2H); **LRMS** (ES) m/z 456.3 (M⁺+ 1).

### Example 230: Synthesis of Compound 230, 6-bromo-3-((5-(5-(difluoromethyl)-1,3,4-oxadiazole-2-yl)pyridine-2-yl)methyl)benzo[d]thiazole-2( 3H)-one

### [Step 1] Synthesis of the compound 230

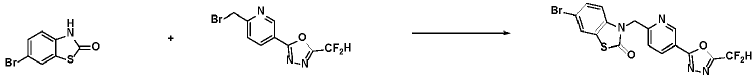

6-bromobenzo[d]thiazole-2(3H)-one (0.300 g, 1.304 mmol) was dissolved in N,N-dimethylformamide (10 mL) at 0°C, after which sodium hydride (60.00%, 0.078 g, 1.956 mmol) was added into the resulting solution and stirred at the same temperature for 30 minutes. 2-(6-(bromomethyl)pyridine-3-yl)-5-(difluoromethyl)-1,3,4-oxadiazole (0.378 g, 1.304 mmol) was added into the reaction mixture and further stirred at room temperature for 3 hours. Water was poured into the reaction mixture and an extraction was performed with ethyl acetate. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; ethyl acetate/hexane = 0 to 50%) and concentrated to obtain a title compound (0.309 g, 54.0%) in a yellow solid form.

**¹H NMR** (400 MHz, CDCl₃) δ 9.29 (dd, *J* = 2.2, 0.8 Hz, 1H), 8.37 (dd, *J* = 8.2, 2.2 Hz, 1H), 7.59 (d, *J* = 1.9 Hz, 1H), 7.46 (dd, *J* = 8.2, 0.8 Hz, 1H), 7.37 (dd, *J* = 8.6, 2.0 Hz, 1H), 7.07 (s, 0.25H), 7.02 (d, *J =* 8.6 Hz, 1H), 6.95 (s, 0.5H), 6.82 (s, 0.25H), 5.34 (s, 2H).

### Example 231: Synthesis of Compound 231, 3-((5-(5-(difluoromethyl)-1,3,4-oxadiazole-2-yl)pyridine-2-yl)methyl)-6-(pyridine-3-yl)benzo[d]thia zole-2(3H)-one

### [Step 1] Synthesis of the compound 231

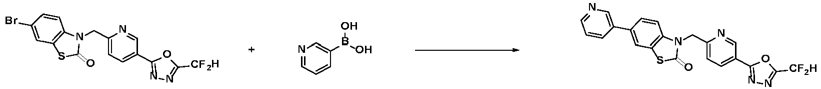

6-bromo-3-((5-(5-(difluoromethyl)-1,3,4-oxadiazole-2-yl)pyridine-2-yl)methyl)benzo[d]thi azole-2(3H)-one (0.170 g, 0.387 mmol), pyridine-3-ylboronic acid (0.062 g, 0.503 mmol), [1,1'-bis(di-tert-butylphosphino)ferrocene]palladium(II) dichloride (Pd(dtbpf)Cl₂, 0.025 g, 0.039 mmol) and cesium carbonate (0.189 g, 0.581 mmol) were mixed in 1,4-dioxane (9 mL)/water (3 mL), after which the resulting mixture was irradiated with microwave, then heated at 100°C for 25 hours, and then a reaction was finished by lowering a temperature to room temperature. Solvent was removed from the reaction mixture under reduced pressure, after which water was poured into the resulting concentrate, and then an extraction was performed with dichloromethane. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; ethyl acetate/hexane = 0 to 50%) and concentrated to obtain a title compound (0.110 g, 65.0%) in a white solid form.

**¹H NMR** (400 MHz, CDCl₃) δ d 9.33 (d, *J* = 1.6 Hz, 1H), 8.82 (s, 1H), 8.65 ~ 8.60 (m, 1H), 8.39 (dd, *J* = 9.0, 1.4 Hz, 1H), 7.85 ~ 7.80 (m, 1H), 7.69 (d, *J =* 1.6 Hz, 1H), 7.51 ~ 7.46 (m, 2H), 7.45 ~ 7.40 (m, 1H), 7.24 (d, *J =* 8.4 Hz, 1H), 7.07 (s, 0.25H), 6.94 (s, 0.5H), 6.81 (s, 0.25H), 5.42 (s, 2H); **LRMS** (ES) m/z 438.2 (M⁺+ 1).

### Example 232: Synthesis of Compound 232, 5-chloro-3-((5-(5-(difluoromethyl)-1,3,4-oxadiazole-2-yl)pyridine-2-yl)methyl)-1-(1-(2,2,3,3,4,4,4-heptafluorobutyl)piperidine-4-yl)-1,3-dihydro-2H-benzo[d]imidazole-2-one

### [Step 1] Synthesis of the compound 232

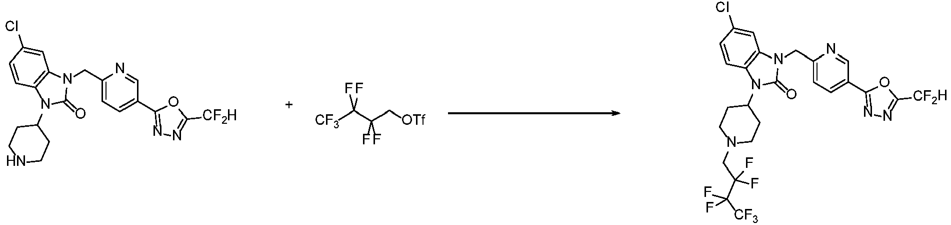

The 5-chloro-3-((5-(5-(difluoromethyl)-1,3,4-oxadiazole-2-yl)pyridine-2-yl)methyl)-1-(piperidine-4-yl)-1,3-dihydro-2H-benzo[d]imidazole-2-one (0.100 g, 0.217 mmol) prepared in the step 5 of the compound 213, potassium carbonate (0.060 g, 0.434 mmol) and 2,2,3,3,4,4,4-heptafluorobutyl trifluoromethanesulfonate (0.079 g, 0.239 mmol) were dissolved in acetonitrile (2 mL) at room temperature, after which the resulting solution was stirred at the same temperature for 18 hours. Solvent was removed from the reaction mixture under reduced pressure, after which water was poured into a resulting concentrate, and an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.051 g, 36.5%) in a white solid form.

**¹H NMR** (400 MHz, CDCl₃) δ 9.28 (d, *J* = 1.5 Hz, 1H), 8.34 (dd, *J* = 8.2, 2.2 Hz, 1H), 7.41 (d, *J = 8.2* Hz, 1H), 7.16 - 6.79 (m, 4H), 5.23 (s, 2H), 4.42 - 4.34 (m, 1H), 3.15 - 3.07 (m, 4H), 2.61 (t, *J =* 11.2 Hz, 2H), 2.50 - 2.40 (m, 2H), 1.82 (dd, *J* = 11.9, 2.2 Hz, 2H); **LRMS** (ES) m/z 644.9 (M⁺+ 1).

### Example 233: Synthesis of Compound 233, 5-chloro-1-(1-(2,2-difluorobutyl)piperidine-4-yl)-3-((5-(5-(difluoromethyl)-1,3,4-oxadiazole-2-yl)py ridine-2-yl)methyl)-1,3-dihydro-2H-benzo[d]imidazole-2-one

### [Step 1] Synthesis of the compound 233

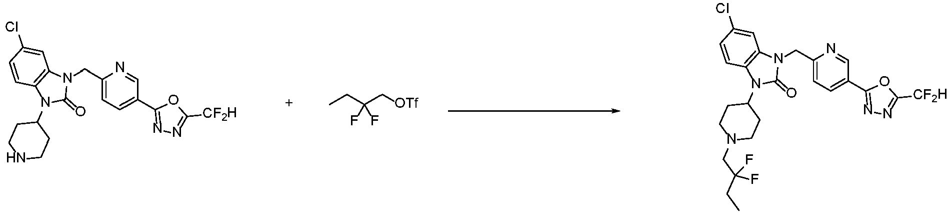

The 5-chloro-3-((5-(5-(difluoromethyl)-1,3,4-oxadiazole-2-yl)pyridine-2-yl)methyl)-1-(piperidine-4-yl)-1,3-dihydro-2H-benzo[d]imidazole-2-one (0.100 g, 0.217 mmol) prepared in the step 5 of the compound 213, potassium carbonate (0.060 g, 0.434 mmol) and 2,2-difluorobutyl trifluoromethanesulfonate (0.058 g, 0.239 mmol) were dissolved in acetonitrile (2 mL) at room temperature, after which the resulting solution was stirred at the same temperature for 18 hours. Solvent was removed from the reaction mixture under reduced pressure, after which water was poured into a resulting concentrate, and an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.079 g, 65.7%) in a white solid form.

**¹H NMR** (400 MHz, CDCl₃) δ 9.27 (d, *J* = 1.5 Hz, 1H), 8.33 (dd, *J* = 8.2, 2.2 Hz, 1H), 7.41 (d, *J* = 8.2 Hz, 1H), 7.15 - 6.79 (m, 4H), 5.23 (s, 2H), 4.35 - 4.29 (m, 1H), 3.14 - 3.09 (m, 2H), 2.73 (t, *J =* 13.8 Hz, 2H), 2.49 - 2.39 (m, 4H), 2.05 - 1.91 (m, 2H), 1.81 - 1.80 (m, 2H), 1.03 (t, *J*= 7.5 Hz, 3H); **LRMS** (ES) m/z 552.9 (M⁺+ 1).

### Example 234: Synthesis of Compound 234, 5-chloro-3-((5-(5-(difluoromethyl)-1,3,4-oxadiazole-2-yl)pyridine-2-yl)methyl)-1-(1-(2,2-difluoropr opyl)piperidine-4-yl)-1,3-dihydro-2H-benzo[d]imidazole-2-one

### [Step 1] Synthesis of the compound 234

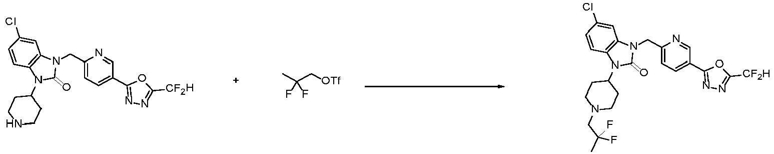

The 5-chloro-3-((5-(5-(difluoromethyl)-1,3,4-oxadiazole-2-yl)pyridine-2-yl)methyl)-1-(piperidine-4-yl)-1,3-dihydro-2H-benzo[d]imidazole-2-one (0.100 g, 0.217 mmol) prepared in the step 5 of the compound 213, potassium carbonate (0.060 g, 0.434 mmol) and 2,2-difluoropropyl trifluoromethanesulfonate (0.054 g, 0.239 mmol) were dissolved in acetonitrile (2 mL) at room temperature, after which the resulting solution was stirred at the same temperature for 18 hours. Solvent was removed from the reaction mixture under reduced pressure, after which water was poured into a resulting concentrate, and an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.082 g, 70.2%) in a white solid form.

**¹H NMR** (400 MHz, CDCl₃) δ 9.27 (dd, *J* = 2.2, 0.7 Hz, 1H), 8.33 (dd, *J* = 8.2, 2.2 Hz, 1H), 7.41 (dd, *J* = 8.2, 0.6 Hz, 1H), 7.15 (d, *J =* 8.4 Hz, 1H), 7.05 - 6.79 (m, 3H), 5.22 (s, 2H), 4.37 - 4.30 (m, 1H), 3.13 - 3.07 (m, 2H), 2.73 (t, *J* = 13.5 Hz, 2H), 2.49 - 2.38 (m, 4H), 1.82 - 1.78 (m, 2H), 1.67 (t, *J* = 18.7 Hz, 3H); **LRMS** (ES) m/z 540.9 (M⁺+ 1).

### Example 235: Synthesis of Compound 235, 5-chloro-3-((5-(5-(difluoromethyl)-1,3,4-oxadiazole-2-yl)pyridine-2-yl)methyl)-1-(1-(2,2,3,3-tetrafl uoropropyl)piperidine-4-yl)-1,3-dihydro-2H-benzo[d]imidazole-2-one

### [Step 1] Synthesis of the compound 235

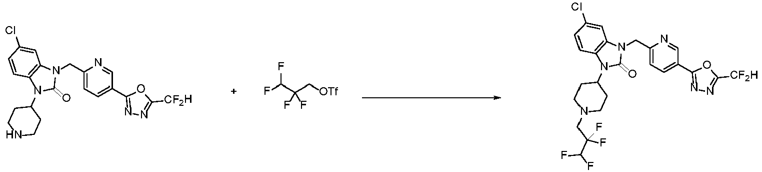

The 5-chloro-3-((5-(5-(difluoromethyl)-1,3,4-oxadiazole-2-yl)pyridine-2-yl)methyl)-1-(piperidine-4-yl)-1,3-dihydro-2H-benzo[d]imidazole-2-one (0.100 g, 0.217 mmol) prepared in the step 5 of the compound 213, potassium carbonate (0.060 g, 0.434 mmol) and 2,2,3,3-tetrafluoropropyl trifluoromethanesulfonate (0.063 g, 0.239 mmol) were dissolved in acetonitrile (2 mL) at room temperature, after which the resulting solution was stirred at the same temperature for 18 hours. Solvent was removed from the reaction mixture under reduced pressure, after which water was poured into a resulting concentrate, and an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.101 g, 80.6%) in a white solid form.

**¹H NMR** (400 MHz, CDCl₃) δ 9.27 (dd, *J* = 2.1, 0.7 Hz, 1H), 8.33 (dd, *J* = 8.2, 2.2 Hz, 1H), 7.41 (dd, *J* = 8.2, 0.5 Hz, 1H), 7.12 - 6.92 (m, 4H), 6.20 - 5.90 (m, 1H), 5.22 (s, 2H), 4.37 - 4.29 (m, 1H), 3.11 (d, *J* = 10.8 Hz, 2H), 2.96 (t, *J* = 14.1 Hz, 2H), 2.55 - 2.39 (m, 4H), 1.82 (dd, *J* = 11.1, 3.1 Hz, 2H); **LRMS** (ES) m/z 576.9 (M⁺ + 1).

### Example 236: Synthesis of Compound 236, 5-chloro-3-((5-(5-(difluoromethyl)-1,3,4-oxadiazole-2-yl)pyridine-2-yl)methyl)-1-(1-(2,2,2-trifluoro ethyl)piperidine-4-yl)-1,3-dihydro-2H-benzo[d]imidazole-2-one

### [Step 1] Synthesis of the compound 236

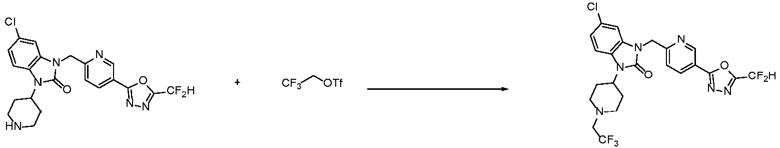

The 5-chloro-3-((5-(5-(difluoromethyl)-1,3,4-oxadiazole-2-yl)pyridine-2-yl)methyl)-1-(piperidine-4-yl)-1,3-dihydro-2H-benzo[d]imidazole-2-one (0.100 g, 0.217 mmol) prepared in the step 5 of the compound 213, potassium carbonate (0.060 g, 0.434 mmol) and 2,2,2-trifluoroethyl trifluoromethanesulfonate (0.055 g, 0.239 mmol) were dissolved in acetonitrile (2 mL) at room temperature, after which the resulting solution was stirred at the same temperature for 18 hours. Solvent was removed from the reaction mixture under reduced pressure, after which water was poured into a resulting concentrate, and an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.087 g, 73.8%) in a white solid form.

**¹H NMR** (400 MHz, CDCl₃) δ 9.27 (d, *J* = 2.1 Hz, 1H), 8.33 (dd, *J* = 8.2, 2.2 Hz, 1H), 7.41 (d, *J* = 8.2 Hz, 1H), 7.18 (d, *J* = 8.4 Hz, 1H), 7.05 -6.79 (m, 3H), 5.23 (s, 2H), 4.42 -4.34 (m, 1H), 3.12 (d, *J* = 11.5 Hz, 2H), 3.05 (q, *J* = 9.6 Hz, 2H), 2.59 (t, *J =* 11.3 Hz, 2H), 2.50 - 2.40 (m, 2H), 1.82 (dd, *J =* 11.9, 2.2 Hz, 2H); **LRMS** (ES) m/z 545.2 (M⁺+ 1).

### Example 237: Synthesis of Compound 237, tert-butyl 5-(3-((5-(5-(difluoromethyl)-1,3,4-oxadiazole-2-yl)pyridine-2-yl)methyl)-2-oxo-2,3-dihydrobenzo[d ]thiazole-6-yl)-1H-indole-1-carboxylate

### [Step 1] Synthesis of the compound 237

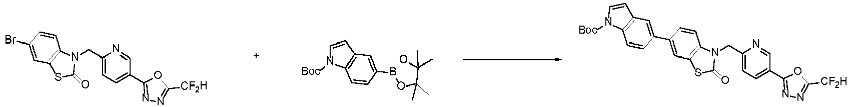

6-bromo-3-((5-(5-(difluoromethyl)-1,3,4-oxadiazole-2-yl)pyridine-2-yl)methyl)benzo[d]thi azole-2(3H)-one (0.150 g, 0.342 mmol), tert-butyl 5-(4,4,5,5-tetramethyl-1,3,2-dioxaborolane-2-yl)-1H-indole-1-carboxylate (0.141 g, 0.410 mmol), [1,1'-bis(di-tert-butylphosphino)ferrocene]palladium(II) dichloride (Pd(dtbpf)Cl₂, 0.022 g, 0.034 mmol) and cesium carbonate (0.167 g, 0.512 mmol) were mixed in 1,4-dioxane (9 mL)/water (3 mL), after which the resulting mixture was irradiated with microwave, then heated at 100°C for 20 minutes, and then a reaction was finished by lowering a temperature to room temperature. Water was poured into the reaction mixture and an extraction was performed with ethyl acetate. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; ethyl acetate/hexane = 0 to 50%) and concentrated to obtain a title compound (0.013 g, 6.6%) in a white solid form.

**¹H NMR** (400 MHz, CDCl₃) δ 9.34 (dd, *J* = 2.2, 0.7 Hz, 1H), 8.38 (dd, *J* = 8.2, 2.2 Hz, 1H), 8.21 (d, *J* = 8.4 Hz, 1H), 7.74 ~ 7.71 (m, 2H), 7.65 (d, *J =* 3.6 Hz, 1H), 7.54 ~ 7.48 (m, 3H), 7.17 (d, *J* = 8.4 Hz, 1H), 7.07 (s, 0.25H), 6.95 (s, 0.5H), 6.82 (s, 0.25H), 6.62 (dd, J = 3.7, 0.5 Hz, 1H), 5.42 (s, 2H), 1.70 (s, 9H).

### Example 238: Synthesis of Compound 238, tert-butyl 4-(3-((5-(5-(difluoromethyl)-1,3,4-oxadiazole-2-yl)pyridine-2-yl)methyl)-2-oxo-2,3-dihydrobenzo[d ]thiazole-6-yl)-3,6-dihydropyridine-1(2H)-carboxylate

### [Step 1] Synthesis of the compound 238

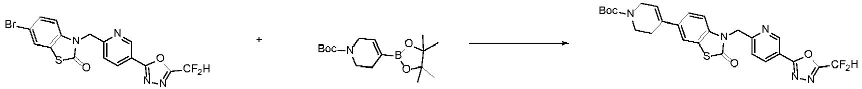

6-bromo-3-((5-(5-(difluoromethyl)-1,3,4-oxadiazole-2-yl)pyridine-2-yl)methyl)benzo[d]thi azole-2(3H)-one (0.700 g, 1.594 mmol), tert-butyl 4-(4,4,5,5-tetramethyl-1,3,2-dioxaborolane-2-yl)-3,6-dihydropyridine-1(2H)-carboxylate (0.591 g, 1.912 mmol), [1,1'-bis(di-tert-butylphosphino)ferrocene]palladium(II) dichloride (Pd(dtbpf)Cl₂, 0.104 g, 0.159 mmol) and cesium carbonate (0.779 g, 2.391 mmol) were mixed in 1,4-dioxane (9 mL)/water (3 mL), after which the resulting mixture was irradiated with microwave, then heated at 100°C for 20 minutes, and then a reaction was finished by lowering a temperature to room temperature. Water was poured into the reaction mixture and an extraction was performed with ethyl acetate. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; ethyl acetate/hexane = 0 to 50%) and concentrated to obtain a title compound (0.442 g, 51.2%) in a white foam solid form.

**¹H NMR** (400 MHz, CDCl₃) δ 9.31 ~ 9.30 (m, 1H), 8.36 (dd, *J* = 8.2, 2.2 Hz, 1H), 7.48 ~ 7.44 (m, 2H), 7.28 ~ 7.25 (m, 1H), 7.07 (s, 0.25H), 7.06 (d, *J* = 8.6 Hz, 1H), 6.95 (s, 0.5H), 6.82 (s, 0.25H), 6.00 (s, 1H), 5.37 (s, 2H), 4.05 ~ 4.00 (m, 2H), 3.64 (t, *J* = 5.6 Hz, 2H), 2.50 ~ 2.49 (m, 2H), 1.52 (s, 9H).

### Example 239: Synthesis of Compound 239, 1-((5-(5-(difluoromethyl)-1,3,4-oxadiazole-2-yl)pyridine-2-yl)methyl)-6-fluoro-5-(1H-indole-4-yl)-3 -(1-(2,2,2-trifluoroethyl)piperidine-4-yl)-1,3-dihydro-2H-benzo[d]imidazole-2-one

### [Step 1] Synthesis of 5-bromo-1-((5-(5-(difluoromethyl)-1,3,4-oxadiazole-2-yl)pyridine-2-yl)methyl)-6-fluoro-3-(piperidi ne-4-yl)-1,3-dihydro-2H-benzo[d]imidazole-2-one

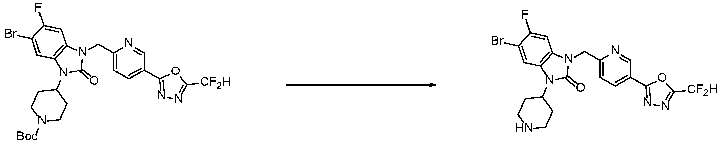

The tert-butyl 4-(6-bromo-3-((5-(5-(difluoromethyl)-1,3,4-oxadiazole-2-yl)pyridine-2-yl)methyl)-5-fluoro-2-oxo-2 ,3-dihydro-1H-benzo[d]imidazole-1-yl)piperidine-1-carboxylate (2.750 g, 4.411 mmol) prepared in the step 1 of the compound 149 and trifluoroacetic acid (3.378 mL, 44.111 mmol) were dissolved in dichloromethane (20 mL) at room temperature, after which the resulting solution was stirred at the same temperature for 4 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 40 g cartridge; ethyl acetate/hexane = 50 to 100%) and concentrated, after which an obtained product was purified again via chromatography (SiO₂, 40 g cartridge; methanol/dichloromethane = 0 to 50%) and concentrated to obtain a title compound (2.100 g, 91.0%) in a brown solid form.

### [Step 2] Synthesis of 5-bromo-1-((5-(5-(difluoromethyl)-1,3,4-oxadiazole-2-yl)pyridine-2-yl)methyl)-6-fluoro-3-(1-(2,2,2 -trifluoroethyl)piperidine-4-yl)-1,3-dihydro-2H-benzo[d]imidazole-2-one

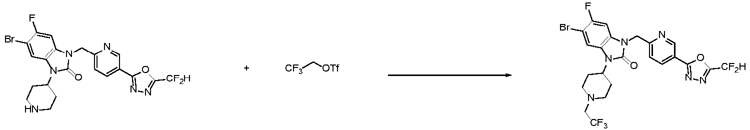

The 5-bromo-1-((5-(5-(difluoromethyl)-1,3,4-oxadiazole-2-yl)pyridine-2-yl)methyl)-6-fluoro-3-(piperidi ne-4-yl)-1,3-dihydro-2H-benzo[d]imidazole-2-one (0.400 g, 0.764 mmol) prepared in the step 1, potassium carbonate (0.211 g, 1.529 mmol) and 2,2,2-trifluoroethyl trifluoromethanesulfonate (0.177 g, 0.764 mmol) were dissolved in acetonitrile (5 mL) at room temperature, after which the resulting solution was stirred at the same temperature for 18 hours. Solvent was removed from the reaction mixture under reduced pressure, after which water was poured into a resulting concentrate, and an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 2%) and concentrated to obtain a title compound (0.380 g, 82.1%) in a yellow solid form.

### [Step 3] Synthesis of the compound 239

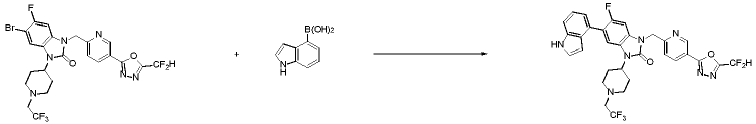

The 5-bromo-1-((5-(5-(difluoromethyl)-1,3,4-oxadiazole-2-yl)pyridine-2-yl)methyl)-6-fluoro-3-(1-(2,2,2 -trifluoroethyl)piperidine-4-yl)-1,3-dihydro-2H-benzo[d]imidazole-2-one (0.150 g, 0.248 mmol) prepared in the step 2, (1H-indole-4-yl)boronic acid (0.048 g, 0.297 mmol), cesium carbonate (0.161 g, 0.496 mmol) and [1,1'-bis(di-tert-butylphosphino)ferrocene]palladium dichloride (0.008 g, 0.012 mmol) were mixed in 1,4-dioxane (2.1 mL)/water (0.7 mL) at room temperature, after which the resulting mixture was irradiated with microwave, then heated at 100°C for 30 minutes, and then a reaction was finished by lowering a temperature to room temperature. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.045 g, 28.3%) in a yellow solid form.

**¹H NMR** (400 MHz, CDCl₃) δ 9.32 (dd, *J* = 2.2, 0.7 Hz, 1H), 8.51 (s, 1H), 8.36 (dd, *J* = 8.2, 2.2 Hz, 1H), 7.48 (dd, *J* = 8.2, 0.5 Hz, 1H), 7.42 (d, *J* = 8.1 Hz, 1H), 7.39 (d, *J* = 5.9 Hz, 1H), 7.30 - 7.23 (m, 2H), 7.16 (d, *J* = 7.2 Hz, 1H), 6.93 (t, *J* = 51.6 Hz, 1H), 6.92 (d, *J =* 10.0 Hz, 1H), 6.44 (d, *J = 2.2* Hz, 1H), 5.29 (s, 2H), 4.44 - 4.36 (m, 1H), 3.09 (d, *J* = 11.3 Hz, 2H), 3.01 (q, *J* = 9.6 Hz, 2H), 2.61 - 2.43 (m, 4H), 1.86 (dd, *J* = 11.7, 2.1 Hz, 2H); **LRMS** (ES) m/z 642.0 (M⁺+ 1).

### Example 240: Synthesis of Compound 240, 1-((5-(5-(difluoromethyl)-1,3,4-oxadiazole-2-yl)pyridine-2-yl)methyl)-6-fluoro-5-(pyridine-3-yl)-3-( 1-(2,2,2-trifluoroethyl)piperidine-4-yl)-1,3-dihydro-2H-benzo[d]imidazole-2-one

### [Step 1] Synthesis of the compound 240

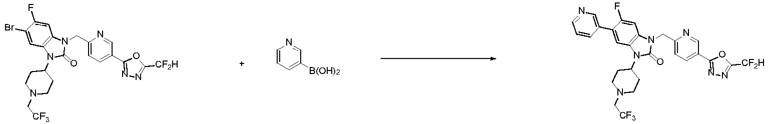

The 5-bromo-1-((5-(5-(difluoromethyl)-1,3,4-oxadiazole-2-yl)pyridine-2-yl)methyl)-6-fluoro-3-(1-(2,2,2 -trifluoroethyl)piperidine-4-yl)-1,3-dihydro-2H-benzo[d]imidazole-2-one (0.150 g, 0.248 mmol) prepared in the step 2 of the compound 239, pyridine-3-ylboronic acid (0.037 g, 0.297 mmol), cesium carbonate (0.161 g, 0.496 mmol) and [1,1'-bis(di-tert-butylphosphino)ferrocene]palladium dichloride (0.008 g, 0.012 mmol) were mixed in 1,4-dioxane (2.1 mL)/water (0.7 mL) at room temperature, after which the resulting mixture was irradiated with microwave, then heated at 100°C for 30 minutes, and then a reaction was finished by lowering a temperature to room temperature. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.123 g, 82.0%) in a brown solid form.

**¹H NMR** (400 MHz, CDCl₃) δ 9.29 (dd, *J =* 2.1, 0.6 Hz, 1H), 8.74 (s, 1H), 8.59 (d, *J =* 4.0 Hz, 1H), 8.36 (dd, *J* = 8.2, 2.2 Hz, 1H), 7.83 (dd, *J* = 7.9, 1.4 Hz, 1H), 7.48 (d, *J* = 8.2 Hz, 1H), 7.38 (dd, *J* = 7.8, 4.8 Hz, 1H), 7.21 (d, *J* = 6.1 Hz, 1H), 6.92 (t, *J* = 51.6 Hz, 1H), 6.91 (d, *J =* 9.0 Hz, 1H), 5.26 (s, 2H), 4.43 - 4.35 (m, 1H), 3.13 (d, *J =* 11.5 Hz, 2H), 3.05 (q, *J =* 9.6 Hz, 2H), 2.62 (t, *J* = 11.4 Hz, 2H), 2.52 - 2.24 (m, 2H), 1.86 (dd, *J* = 11.8, 2.0 Hz, 2H); **LRMS** (ES) m/z 604.1 (M⁺ + 1).

### Example 241: Synthesis of Compound 241, 1-((5-(5-(difluoromethyl)-1,3,4-oxadiazole-2-yl)pyridine-2-yl)methyl)-6-fluoro-5-(1H-indole-4-yl)-3 -(1-(2,2,3,3-tetrafluoropropyl)piperidine-4-yl)-1,3-dihydro-2H-benzo[d]imidazole-2-one

### [Step 1] Synthesis of 5-bromo-1-((5-(5-(difluoromethyl)-1,3,4-oxadiazole-2-yl)pyridine-2-yl)methyl)-6-fluoro-3-(1-(2,2,3 ,3-tetrafluoropropyl)piperidine-4-yl)-1,3-dihydro-2H-benzo[d]imidazole-2-one

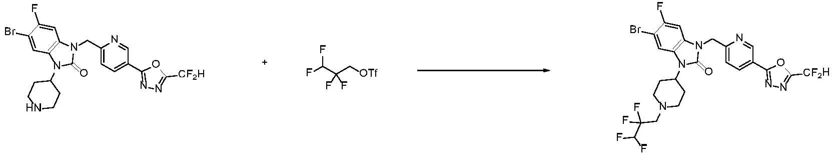

The 5-bromo-1-((5-(5-(difluoromethyl)-1,3,4-oxadiazole-2-yl)pyridine-2-yl)methyl)-6-fluoro-3-(piperidi ne-4-yl)-1,3-dihydro-2H-benzo[d]imidazole-2-one (0.400 g, 0.764 mmol) prepared in the step 1 of the compound 239, potassium carbonate (0.211 g, 1.529 mmol) and 2,2,3,3-tetrafluoropropyl trifluoromethanesulfonate (0.202 g, 0.764 mmol) were dissolved in acetonitrile (5 mL) at room temperature, after which the resulting solution was stirred at the same temperature for 18 hours. Solvent was removed from the reaction mixture under reduced pressure, after which water was poured into a resulting concentrate, and an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 2%) and concentrated to obtain a title compound (0.350 g, 71.8%) in a yellow solid form.

### [Step 2] Synthesis of the compound 241

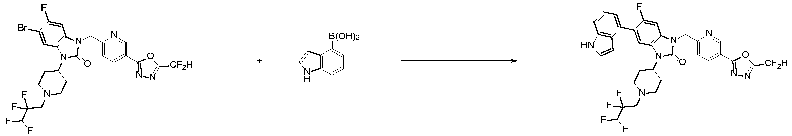

The 5-bromo-1-((5-(5-(difluoromethyl)-1,3,4-oxadiazole-2-yl)pyridine-2-yl)methyl)-6-fluoro-3-(1-(2,2,3 ,3-tetrafluoropropyl)piperidine-4-yl)-1,3-dihydro-2H-benzo[d]imidazole-2-one (0.150 g, 0.235 mmol) prepared in the step 1, (1H-indole-4-yl)boronic acid (0.045 g, 0.282 mmol), cesium carbonate (0.153 g, 0.471 mmol) and [1,1'-bis(di-tert-butylphosphino)ferrocene]palladium dichloride (0.008 g, 0.012 mmol) were mixed in 1,4-dioxane (2.1 mL)/water (0.7 mL) at room temperature, after which the resulting mixture was irradiated with microwave, then heated at 100°C for 30 minutes, and then a reaction was finished by lowering a temperature to room temperature. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.112 g, 70.3%) in a brown solid form.

**¹H NMR** (400 MHz, CDCl₃) δ 9.31 (d, *J =* 1.7 Hz, 1H), 8.54 (s, 1H), 8.36 (dd, *J* = 8.2, 2.2 Hz, 1H), 7.48 (d, *J* = 8.2 Hz, 1H), 7.43 (d, *J =* 13.6 Hz, 1H), 7.37 (d, *J* = 5.9 Hz, 1H), 7.29 (d, *J =* 7.4 Hz, 1H), 7.26 - 7.24 (m, 1H), 7.17 (d, *J =* 12.0 Hz, 1H), 7.05 -6.80 (m, 2H), 6.47 (d, *J* = 1.8 Hz, 1H), 6.13 - 5.84 (m, 1H), 5.29 (s, 2H), 4.40 - 4.36 (m, 1H), 3.09 - 3.07 (m, 2H), 2.92 (t, *J =* 13.9 Hz, 2H), 2.52 - 2.41 (m, 4H), 1.86 - 1.85 (m, 2H); **LRMS** (ES) m/z 673.9 (M⁺ + 1).

### Example 242: Synthesis of Compound 242, 1-((5-(5-(difluoromethyl)-1,3,4-oxadiazole-2-yl)pyridine-2-yl)methyl)-6-fluoro-5-(pyridine-3-yl)-3-( 1-(2,2,3,3-tetrafluoropropyl)piperidine-4-yl)-1,3-dihydro-2H-benzo[d]imidazole-2-one

### [Step 1] Synthesis of the compound 242

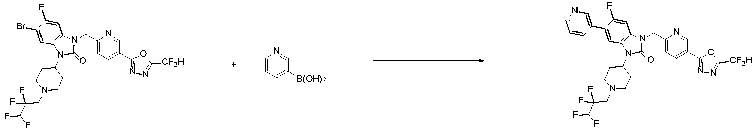

The 5-bromo-1-((5-(5-(difluoromethyl)-1,3,4-oxadiazole-2-yl)pyridine-2-yl)methyl)-6-fluoro-3-(1-(2,2,3 ,3-tetrafluoropropyl)piperidine-4-yl)-1,3-dihydro-2H-benzo[d]imidazole-2-one (0.150 g, 0.235 mmol) prepared in the step 1 of the compound 241, pyridine-3-ylboronic acid (0.035 g, 0.282 mmol), cesium carbonate (0.153 g, 0.471 mmol) and [1,1'-bis(di-tert-butylphosphino)ferrocene]palladium dichloride (0.008 g, 0.012 mmol) were mixed in 1,4-dioxane (2.1 mL)/water (0.7 mL) at room temperature, after which the resulting mixture was irradiated with microwave, then heated at 100°C for 30 minutes, and then a reaction was finished by lowering a temperature to room temperature. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.095 g, 63.3%) in a brown solid form.

**¹H NMR** (400 MHz, CDCl₃) δ 9.26 (dd, *J =* 2.1, 0.6 Hz, 1H), 8.71 (s, 1H), 8.56 (d, *J =* 3.7 Hz, 1H), 8.34 (dd, *J* = 8.2, 2.2 Hz, 1H), 7.80 (dd, *J* = 8.0, 1.8 Hz, 1H), 7.46 (d, *J* = 8.2 Hz, 1H), 7.35 (dd, *J* = 7.8, 4.9 Hz, 1H), 7.13 (d, *J* = 6.1 Hz, 1H), 6.92 (t, *J =* 51.6 Hz, 1H), 6.91 (d, *J* = 9.7 Hz, 1H), 6.16 - 5.86 (m, 1H), 5.24 (s, 2H), 4.34 - 4.27 (m, 1H), 3.10 (d, *J =* 8.8 Hz, 2H), 2.94 (t, *J* = 14.1 Hz, 2H), 2.54 - 2.41 (m, 4H), 1.84 (d, *J =* 9.8 Hz, 2H); LRMS (ES) m/z 636.2 (M⁺ + 1).

### Example 243: Synthesis of Compound 243, 5-chloro-3-((5-(5-(difluoromethyl)-1,3,4-oxadiazole-2-yl)pyridine-2-yl)methyl)-1-(1-(2-hydroxyace tyl)piperidine-4-yl)-1,3-dihydro-2H-benzo[d]imidazole-2-one

### [Step 1] Synthesis of the compound 243

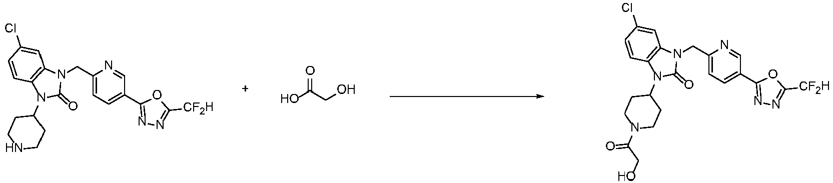

The 5-chloro-3-((5-(5-(difluoromethyl)-1,3,4-oxadiazole-2-yl)pyridine-2-yl)methyl)-1-(piperidine-4-yl)-1,3-dihydro-2H-benzo[d]imidazole-2-one (0.100 g, 0.217 mmol) prepared in the step 5 of the compound 213, 2-hydroxyacetic acid (0.018 g, 0.239 mmol), 1-ethyl-3-(3-dimethylaminopropyl)carbodiimide (EDC, 0.067 g, 0.434 mmol), 1H-benzo[d][1,2,3]triazole-1-ol (HOBt, 0.059 g, 0.434 mmol) and triethylamine (0.091 mL, 0.651 mmol) were dissolved in dichloromethane (2 mL) at room temperature, after which the resulting solution was stirred at the same temperature for 18 hours. Water was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.084 g, 74.4%) in a white solid form.

**¹H NMR** (400 MHz, CDCl₃) δ 9.24 (d, *J* = 1.6 Hz, 1H), 8.32 (dd, *J* = 8.2, 2.2 Hz, 1H), 7.41 (d, *J =* 8.2 Hz, 1H), 7.03 - 6.98 (m, 3H), 6.92 (t, *J* = 51.6 Hz, 1H), 5.21 (s, 2H), 4.83 - 4.82 (m, 1H), 4.58 -4.50 (m, 1H), 4.21 (q, *J* = 13.9 Hz, 2H), 3.68 (d, *J* = 13.7 Hz, 1H), 3.19 - 3.12 (m, 1H), 2.81 (t, *J* = 12.1 Hz, 1H), 2.37 - 2.26 (m, 2H), 1.95 (d, *J* = 12.1 Hz, 2H); **LRMS** (ES) m/z 519.4 (M⁺+ 1).

### Example 244: Synthesis of Compound 244, methyl 6-(1-((5-(5-(difluoromethyl)-1,3,4-oxadiazole-2-yl)pyridine-2-yl)methyl)-3-methyl-2-oxo-2,3-dihyd ro-1H-benzo[d]imidazole-5-yl)-1H-indole-2-carboxylate

### [Step 1] Synthesis of 1-(tert-butyl) 2-methyl 6-(1-((5-(5-(difluoromethyl)-1,3,4-oxadiazole-2-yl)pyridine-2-yl)methyl)-6-fluoro-3-methyl-2-oxo-2 ,3-dihydro-1H-benzo[d]imidazole-5-yl)-1H-indole-1,2-dicarboxylate

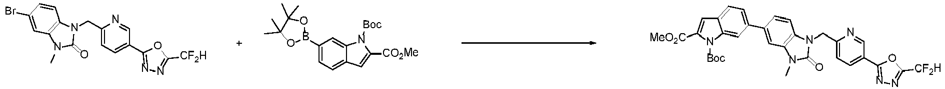

The 5-bromo-1-((5-(5-(difluoromethyl)-1,3,4-oxadiazole-2-yl)pyridine-2-yl)methyl)-6-fluoro-3-methyl-1 ,3-dihydro-2H-benzo[d]imidazole-2-one (0.200 g, 0.458 mmol) prepared in the step 4 of the compound 208, 1-(tert-butyl) 2-methyl 6-(4,4,5,5-tetramethyl-1,3,2-dioxaborolane-2-yl)-1H-indole-1,2-dicarboxylate (0.221 g, 0.550 mmol), cesium carbonate (0.299 g, 0.917 mmol) and [1,1'-bis(di-tert-butylphosphino)ferrocene]palladium dichloride (0.015 g, 0.023 mmol) were mixed in 1,4-dioxane (2.1 mL)/water (0.7 mL) at room temperature, after which the resulting mixture was irradiated with microwave, then heated at 100°C for 30 minutes, and then a reaction was finished by lowering a temperature to room temperature. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.150 g, 50.4%) in a brown solid form.

### [Step 2] Synthesis of the compound 244

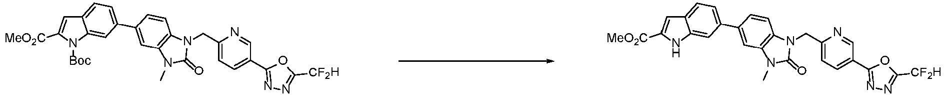

The 1-(tert-butyl) 2-methyl 6-(1-((5-(5-(difluoromethyl)-1,3,4-oxadiazole-2-yl)pyridine-2-yl)methyl)-3-methyl-2-oxo-2,3-dihyd ro-1H-benzo[d]imidazole-5-yl)-1H-indole-1,2-dicarboxylate (0.150 g, 0.238 mmol) prepared in the step 1 and trifluoroacetic acid (0.182 mL, 2.379 mmol) were dissolved in dichloromethane (2 mL) at room temperature, after which the resulting solution was stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. Dichloromethane (10 mL) was put into the resulting concentrate and stirred, after which a precipitated solid was filtered, then washed with dichloromethane, and then dried to obtain a title compound (0.075 g, 59.4%) in a brown solid form.

**¹H NMR** (400 MHz, CDCl₃) δ 9.29 (d, *J =* 1.6 Hz, 1H), 9.04 (s, 1H), 8.32 (dd, *J* = 8.2, 2.2 Hz, 1H), 7.73 (d, *J = 8.4* Hz, 1H), 7.56 (s, 1H), 7.44 (d, *J* = 8.2 Hz, 1H), 7.38 (dd, *J* = 8.4, 1.5 Hz, 1H), 7.31 (dd, *J* = 8.1, 1.6 Hz, 1H), 7.26 - 7.22 (m, 2H), 7.01 (d, *J* = 3.3 Hz, 1H), 6.92 (t, *J* = 51.6 Hz, 1H), 5.33 (s, 2H), 3.95 (s, 3H), 3.54 (s, 3H); **LRMS** (ES) m/z 531.4 (M⁺+ 1).

### Example 245: Synthesis of the compound 245, 3-(4-(5-(difluoromethyl)-1,3,4-oxadiazole-2-yl)-2-fluorobenzyl)benzo[d]oxazole-2(3H)-one

### [Step 1] Synthesis of the compound 245

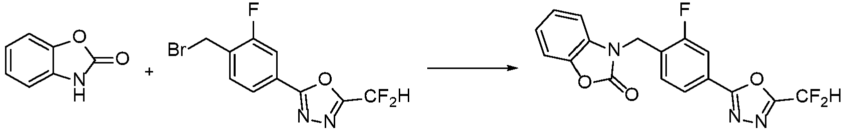

Benzo[d]oxazole-2(3H)-one (0.100 g, 0.740 mmol), 2-(4-(bromomethyl)-3-fluorophenyl)-5-(difluoromethyl)-1,3,4-oxadiazole (0.250 g, 0.814 mmol), potassium carbonate (0.153 g, 1.110 mmol) and potassium iodide (0.012 g, 0.074 mmol) were dissolved in N,N-dimethylformamide (4 mL) at room temperature, after which the resulting solution was stirred at 80°C for 18 hours, and then a reaction was finished by lowering a temperature to room temperature. Saturated sodium hydrogen carbonate aqueous solution was poured into the reaction mixture and an extraction was performed with ethyl acetate. An organic layer was washed with saturated sodium chloride aqueous solution, then dehydrated with anhydrous magnesium sulfate, then filtered, and then concentrated under reduced pressure. Ethyl acetate was put into the resulting concentrate and stirred, after which a precipitated solid was filtered, then washed with ethyl acetate, and then dried to obtain a title compound (0.191 g, 71.4%) in a light yellow solid form.

**¹H NMR** (400 MHz, CDCl₃) δ 7.93 - 7.89 (m, 2H), 7.67 (d, *J* = 7.4 Hz, 1H), 7.64 - 7.43 (m, 1H), 7.41 - 7.39 (m, 1H), 7.23 - 7.14 (m, 3H), 5.23 (s, 2H); **LRMS** (ES) m/z 362.3 (M⁺ + 1).

### Example 246: Synthesis of Compound 246, 3-((5-(5-(difluoromethyl)-1,3,4-oxadiazole-2-yl)pyridine-2-yl)methyl)benzo[d]oxazole-2(3H)-one

### [Step 1] Synthesis of the compound 246

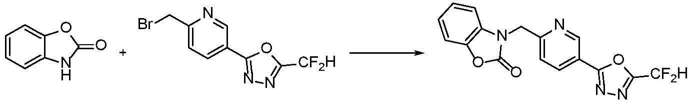

Benzo[d]oxazole-2(3H)-one (0.115 g, 0.851 mmol), 2-(6-(bromomethyl)pyridine-3-yl)-5-(difluoromethyl)-1,3,4-oxadiazole (0.247 g, 0.851 mmol), potassium carbonate (0.176 g, 1.277 mmol) and potassium iodide (0.014 g, 0.085 mmol) were dissolved in N,N-dimethylformamide (4 mL) at room temperature, after which the resulting solution was stirred at 80°C for 18 hours, and then a reaction was finished by lowering a temperature to room temperature. Solvent was removed from the reaction mixture under reduced pressure, after which saturated sodium hydrogen carbonate aqueous solution was poured into the resulting concentrate, and then an extraction was performed with ethyl acetate. An organic layer was washed with saturated sodium chloride aqueous solution, then dehydrated with anhydrous magnesium sulfate, then filtered, and then concentrated under reduced pressure. Ethyl acetate was put into the resulting concentrate and stirred, after which a precipitated solid was filtered, then washed with ethyl acetate, and then dried to obtain a title compound (0.160 g, 54.6%) in a light orange solid form.

**¹H NMR** (400 MHz, CDCl₃) δ 9.14 (d, *J* = 1.6 Hz, 1H), 8.46 (dd, *J* = 8.2, 2.2 Hz, 1H), 7.72 (d, *J* = 8.3 Hz, 1H), 7.70 - 7.45 (m, 1H), 7.41 - 7.39 (m, 1H), 7.19 - 7.14 (m, 3H), 5.32 (s, 2H); **LRMS** (ES) m/z 345.3 (M⁺ + 1).

### Example 247: Synthesis of Compound 247, 1-((5-(5-(difluoromethyl)-1,3,4-oxadiazole-2-yl) pyridine-2-yl)methyl)-3-methyl-5-(1H-pyrazole-4-y I)-1,3-dihydro-2H-benzo[d]imidazole-2-one

### [Step 1] Synthesis of the compound 247

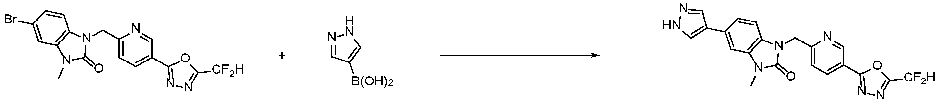

The 5-bromo-1-((5-(5-(difluoromethyl)-1,3,4-oxadiazole-2-yl)pyridine-2-yl)methyl)-3-methyl-1,3-dihyd ro-2H-benzo[d]imidazole-2-one (0.150 g, 0.344 mmol) prepared in the step 4 of the compound 208, (1H-pyrazole-4-yl)boronic acid (0.050 g, 0.447 mmol), cesium carbonate (0.224 g, 0.688 mmol) and [1,1'-bis(di-tert-butylphosphino)ferrocene]palladium dichloride (0.022 g, 0.034 mmol) were mixed in 1,4-dioxane (2.1 mL)/water (0.7 mL) at room temperature, after which the resulting mixture was irradiated with microwave, then heated at 100°C for 30 minutes, and then a reaction was finished by lowering a temperature to room temperature. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.064 g, 43.8%) in a white solid form.

**¹H NMR** (400 MHz, CDCl₃) δ 9.28 (d, *J* = 1.6 Hz, 1H), 8.31 (dd, *J* = 8.2, 2.2 Hz, 1H), 7.90 - 7.75 (m, 2H), 7.42 (d, *J* = 8.3 Hz, 1H), 7.17 - 7.10 (m, 2H), 6.94 (d, *J* = 8.1 Hz, 1H), 6.92 (t, *J* = 51.6 Hz, 1H), 5.29 (s, 2H), 3.51 (s, 3H); **LRMS** (ES) m/z 424.0 (M⁺ + 1).

### Example 248: Synthesis of Compound 248, 1-((5-(5-(difluoromethyl)-1,3,4-oxadiazole-2-yl)pyridine-2-yl)methyl)-6-fluoro-3-(1-methylpiperidin e-4-yl)-5-(1H-pyrazole-4-yl)-1,3-dihydro-2H-benzo[d]imidazole-2-one

### [Step 1] Synthesis of the compound 248

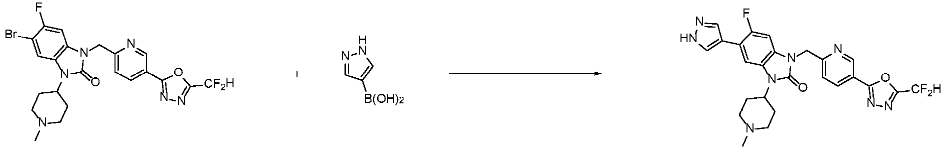

The 5-bromo-1-((5-(5-(difluoromethyl)-1,3,4-oxadiazole-2-yl)pyridine-2-yl)methyl)-6-fluoro-3-(1-methy lpiperidine-4-yl)-1,3-dihydro-2H-benzo[d]imidazole-2-one (0.150 g, 0.279 mmol) prepared in the step 3 of the compound 149, (1H-pyrazole-4-yl)boronic acid (0.037 g, 0.335 mmol), cesium carbonate (0.182 g, 0.558 mmol) and [1,1'-bis(di-tert-butylphosphino)ferrocene]palladium dichloride (0.009 g, 0.014 mmol) were mixed in 1,4-dioxane (2.3 mL)/water (0.6 mL) at room temperature, after which the resulting mixture was irradiated with microwave, then heated at 100°C for 30 minutes, and then a reaction was finished by lowering a temperature to room temperature. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 10%) and concentrated to obtain a title compound (0.041 g, 28.0%) in a brown solid form.

**¹H NMR** (400 MHz, CDCl₃) δ 9.27 (d, *J* = 2.0 Hz, 1H), 8.32 (dd, *J* = 8.2, 2.1 Hz, 1H), 7.80 (d, *J* = 1.1 Hz, 2H), 7.75 (d, *J =* 6.1 Hz, 1H), 7.41 (d, *J =* 8.2 Hz, 1H), 6.92 (t, *J =* 51.6 Hz, 1H), 6.78 (d, *J* = 9.9 Hz, 1H), 5.24 (s, 2H), 4.60 - 4.52 (m, 1H), 3.18 (d, *J* = 11.4 Hz, 2H), 2.82 - 2.73 (m, 2H), 2.46 (s, 3H), 2.35 (t, *J* = 11.4 Hz, 2H), 1.90 (d, *J* = 10.7 Hz, 2H); **LRMS** (ES) m/z 525.6 (M⁺ + 1).

### Example 249: Synthesis of Compound 249, 1-((5-(5-(difluoromethyl)-1,3,4-oxadiazole-2-yl)pyridine-2-yl)methyl)-6-fluoro-5-(1-methyl-1H-pyr azole-5-yl)-3-(1-methylpiperidine-4-yl)-1,3-dihydro-2H-benzo[d]imidazole-2-one

### [Step 1] Synthesis of the compound 249

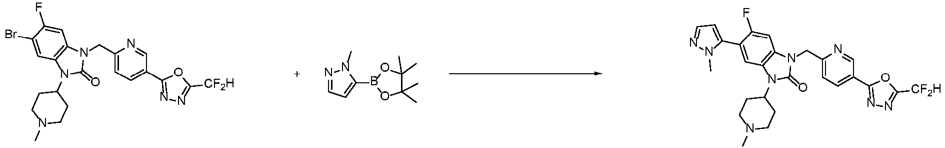

The 5-bromo-1-((5-(5-(difluoromethyl)-1,3,4-oxadiazole-2-yl)pyridine-2-yl)methyl)-6-fluoro-3-(1-methy lpiperidine-4-yl)-1,3-dihydro-2H-benzo[d]imidazole-2-one (0.150 g, 0.279 mmol) prepared in the step 3 of the compound 149, 1-methyl-5-(4,4,5,5-tetramethyl-1,3,2-dioxaborolane-2-yl)-1H-pyrazole (0.070 g, 0.335 mmol), cesium carbonate (0.182 g, 0.558 mmol) and [1,1'-bis(di-tert-butylphosphino)ferrocene]palladium dichloride (0.009 g, 0.014 mmol) were mixed in 1,4-dioxane (2.3 mL)/water (0.6 mL) at room temperature, after which the resulting mixture was irradiated with microwave, then heated at 100°C for 30 minutes, and then a reaction was finished by lowering a temperature to room temperature. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 10%) and concentrated to obtain a title compound (0.041 g, 27.5%) in a brown solid form.

**¹H NMR** (400 MHz, CDCl₃) δ 9.27 (d, *J* = 1.8 Hz, 1H), 8.35 (dd, *J* = 8.2, 2.2 Hz, 1H), 7.50 - 7.47 (m, 2H), 7.19 (d, *J* = 5.8 Hz, 1H), 6.92 (t, *J* = 51.6 Hz, 1H), 6.90 (d, *J* = 9.2 Hz, 1H), 6.24 (d, *J* = 1.8 Hz, 1H), 5.25 (s, 2H), 4.44 - 4.35 (m, 1H), 3.74 (d, *J* = 1.2 Hz, 3H), 3.00 (d, *J =* 11.6 Hz, 2H), 2.47 - 2.37 (m, 2H), 2.31 (s, 3H), 2.14 (t, *J =* 11.1 Hz, 2H), 1.84 (dd, *J* = 11.8, 2.0 Hz, 2H); **LRMS** (ES) m/z 539.5 (M⁺ + 1).

### Example 250: Synthesis of Compound 250, 1-((5-(5-(difluoromethyl)-1,3,4-oxadiazole-2-yl)pyridine-2-yl)methyl)-6-fluoro-5-(1-methyl-1H-indo le-5-yl)-3-(1-methylpiperidine-4-yl)-1,3-dihydro-2H-benzo[d]imidazole-2-one

### [Step 1] Synthesis of the compound 250

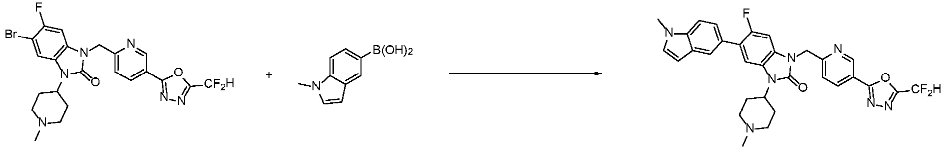

The 5-bromo-1-((5-(5-(difluoromethyl)-1,3,4-oxadiazole-2-yl)pyridine-2-yl)methyl)-6-fluoro-3-(1-methy lpiperidine-4-yl)-1,3-dihydro-2H-benzo[d]imidazole-2-one (0.150 g, 0.279 mmol) prepared in the step 3 of the compound 149, (1-methyl-1H-indole-5-yl)boronic acid (0.059 g, 0.335 mmol), cesium carbonate (0.182 g, 0.558 mmol) and [1,1'-bis(di-tert-butylphosphino)ferrocene]palladium dichloride (0.009 g, 0.014 mmol) were mixed in 1,4-dioxane (2.3 mL)/water (0.6 mL) at room temperature, after which the resulting mixture was irradiated with microwave, then heated at 100°C for 30 minutes, and then a reaction was finished by lowering a temperature to room temperature. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 10%) and concentrated to obtain a title compound (0.101 g, 61.6%) in a brown solid form.

**¹H NMR** (400 MHz, CDCl₃) δ 9.27 (d, *J* = 1.6 Hz, 1H), 8.31 (dd, *J* = 8.2, 2.2 Hz, 1H), 7.70 (d, *J* = 1.0 Hz, 1H), 7.42 (d, *J = 8.2* Hz, 1H), 7.37 (d, *J =* 6.3 Hz, 1H), 7.33 (s, 2H), 7.06 - 6.79 (m, 3H), 6.50 (d, *J =* 3.1 Hz, 1H), 5.26 (s, 2H), 4.47 - 4.39 (m, 1H), 3.78 (s, 3H), 3.01 (d, *J =* 11.6 Hz, 2H), 2.57 - 2.47 (m, 2H), 2.32 (s, 3H), 2.16 (t, *J* = 11.1 Hz, 2H), 1.87 (d, *J =* 10.1 Hz, 2H); **LRMS** (ES) m/z 588.5 (M⁺ + 1).

### Example 251: Synthesis of Compound 251, tert-butyl 5-(1-((5-(5-(difluoromethyl)-1,3,4-oxadiazole-2-yl)pyridine-2-yl)methyl)-6-fluoro-3-(1-methylpiperi dine-4-yl)-2-oxo-2,3-dihydro-1H-benzo[d]imidazole-5-yl)-1H-indole-1-carboxylate

### [Step 1] Synthesis of the compound 251

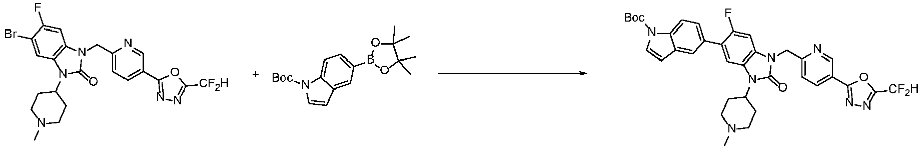

The 5-bromo-1-((5-(5-(difluoromethyl)-1,3,4-oxadiazole-2-yl)pyridine-2-yl)methyl)-6-fluoro-3-(1-methy lpiperidine-4-yl)-1,3-dihydro-2H-benzo[d]imidazole-2-one (0.150 g, 0.279 mmol) prepared in the step 3 of the compound 149, tert-butyl 5-(4,4,5,5-tetramethyl-1,3,2-dioxaborolane-2-yl)-1H-indole-1-carboxylate (0.115 g, 0.335 mmol), cesium carbonate (0.182 g, 0.558 mmol) and [1,1'-bis(di-tert-butylphosphino)ferrocene]palladium dichloride (0.009 g, 0.014 mmol) were mixed in 1,4-dioxane (2.1 mL)/water (0.5 mL) at room temperature, after which the resulting mixture was irradiated with microwave, then heated at 100°C for 30 minutes, and then a reaction was finished by lowering a temperature to room temperature. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.124 g, 65.9%) in a red solid form.

**¹H NMR** (400 MHz, CDCl₃) δ 9.29 (d, *J* = 1.6 Hz, 1H), 8.34 (dd, *J* = 8.2, 2.2 Hz, 1H), 8.15 (d, *J = 8.4* Hz, 1H), 7.64 - 7.62 (m, 2H), 7.45 - 7.40 (m, 2H), 7.34 (d, *J = 6.3* Hz, 1H), 6.92 (t, *J =* 51.6 Hz, 1H), 6.85 (d, *J* = 9.8 Hz, 1H), 6.59 (d, *J* = 3.6 Hz, 1H), 5.27 (s, 2H), 4.48 - 4.40 (m, 1H), 3.02 (d, *J =* 11.6 Hz, 2H), 2.55 - 2.45 (m, 2H), 2.33 (s, 3H), 2.20 - 2.15 (m, 2H), 1.89 - 1.86 (m, 2H), 1.68 (s, 9H); **LRMS** (ES) m/z 674.19 (M⁺+ 1).

### Example 252: Synthesis of Compound 252, tert-butyl 5-(1-((5-(5-(difluoromethyl)-1,3,4-oxadiazole-2-yl)pyridine-2-yl)methyl)-6-fluoro-3-(1-methylpiperi dine-4-yl)-2-oxo-2,3-dihydro-1H-benzo[d]imidazole-5-yl)-2-methyl-1H-indole-1-carboxylate

### [Step 1] Synthesis of the compound 252

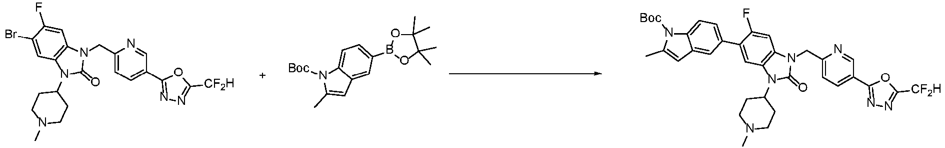

The 5-bromo-1-((5-(5-(difluoromethyl)-1,3,4-oxadiazole-2-yl)pyridine-2-yl)methyl)-6-fluoro-3-(1-methy lpiperidine-4-yl)-1,3-dihydro-2H-benzo[d]imidazole-2-one (0.150 g, 0.279 mmol) prepared in the step 3 of the compound 149, tert-butyl 2-methyl-5-(4,4,5,5-tetramethyl-1,3,2-dioxaborolane-2-yl)-1H-indole-1-carboxylate (0.120 g, 0.335 mmol), cesium carbonate (0.182 g, 0.558 mmol) and [1,1'-bis(di-tert-butylphosphino)ferrocene]palladium dichloride (0.009 g, 0.014 mmol) were mixed in 1,4-dioxane (2.1 mL)/water (0.5 mL) at room temperature, after which the resulting mixture was irradiated with microwave, then heated at 100°C for 30 minutes, and then a reaction was finished by lowering a temperature to room temperature. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.112 g, 58.5%) in a brown solid form.

**¹H NMR** (400 MHz, CDCl₃) δ 9.28 (d, *J* = 1.8 Hz, 1H), 8.34 (dd, *J* = 17.7, 11.7 Hz, 1H), 8.10 (d, *J* = 8.7 Hz, 1H), 7.51 (s, 1H), 7.43 (d, *J = 8.2* Hz, 1H), 7.33 (t, *J* = 7.9 Hz, 2H), 6.92 (t, *J* = 51.7 Hz, 1H), 6.83 (d, *J =* 9.7 Hz, 1H), 6.33 (s, 1H), 5.26 (s, 2H), 4.47 - 4.39 (m, 1H), 3.03 - 3.01 (m, 2H), 2.60 (s, 3H), 2.55 - 2.43 (m, 2H), 2.20 (s, 3H), 2.20 - 2.14 (m, 2H), 1.88 - 1.86 (m, 2H), 1.68 (s, 9H); **LRMS** (ES) m/z 688.48 (M⁺ + 1).

### Example 253: Synthesis of Compound 253, tert-butyl 2-(1-((5-(5-(difluoromethyl)-1,3,4-oxadiazole-2-yl)pyridine-2-yl)methyl)-6-fluoro-3-(1-methylpiperi dine-4-yl)-2-oxo-2,3-dihydro-1H-benzo[d]imidazole-5-yl)-1H-indole-1-carboxylate

### [Step 1] Synthesis of the compound 253

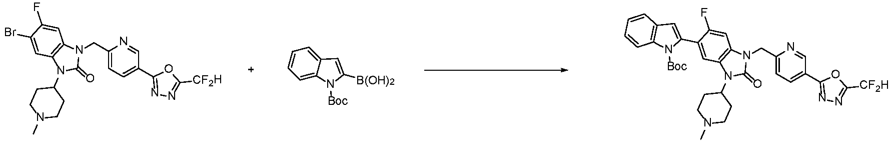

The 5-bromo-1-((5-(5-(difluoromethyl)-1,3,4-oxadiazole-2-yl)pyridine-2-yl)methyl)-6-fluoro-3-(1-methy lpiperidine-4-yl)-1,3-dihydro-2H-benzo[d]imidazole-2-one (0.150 g, 0.279 mmol) prepared in the step 3 of the compound 149, (1-(tert-butoxycarbonyl)-1H-indole-2-yl)boronic acid (0.087 g, 0.335 mmol), cesium carbonate (0.182 g, 0.558 mmol) and [1,1'-bis(di-tert-butylphosphino)ferrocene]palladium dichloride (0.009 g, 0.014 mmol) were mixed in 1,4-dioxane (2.1 mL)/water (0.5 mL) at room temperature, after which the resulting mixture was irradiated with microwave, then heated at 100°C for 30 minutes, and then a reaction was finished by lowering a temperature to room temperature. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.139 g, 73.8%) in a brown solid form.

**¹H NMR** (400 MHz, CDCl₃) δ 9.25 (d, *J=* 2.0 Hz, 1H), 8.32 (dd, *J* = 8.2, 2.2 Hz, 1H), 8.13 (d, *J* = 8.3 Hz, 1H), 7.52 (d, *J* = 7.6 Hz, 1H), 7.43 (d, *J =* 8.2 Hz, 1H), 7.34 (d, *J* = 5.8 Hz, 1H), 7.30 (td, *J* = 7.8, 1.1 Hz, 1H), 7.21 (t, *J =* 7.5 Hz, 1H), 6.92 (t, *J = 51.6* Hz, 1H), 6.82 (d, *J* = 9.2 Hz, 1H), 6.56 (s, 1H), 5.25 (s, 2H), 4.44 - 4.36 (m, 1H), 3.00 - 2.98 (m, 2H), 2.53 - 2.43 (m, 2H), 2.30 (s, 3H), 2.17 - 2.11 (m, 2H), 1.87 - 1.84 (m, 2H), 1.37 (s, 9H); LRMS (ES) m/z 674.19 (M⁺ + 1).

### Example 254: Synthesis of Compound 254, tert-butyl 2-(1-((5-(5-(difluoromethyl)-1,3,4-oxadiazole-2-yl)pyridine-2-yl)methyl)-6-fluoro-3-(1-methylpiperi dine-4-yl)-2-oxo-2,3-dihydro-1H-benzo[d]imidazole-5-yl)-1H-indole-1-carboxylate

### [Step 1] Synthesis of the compound 254

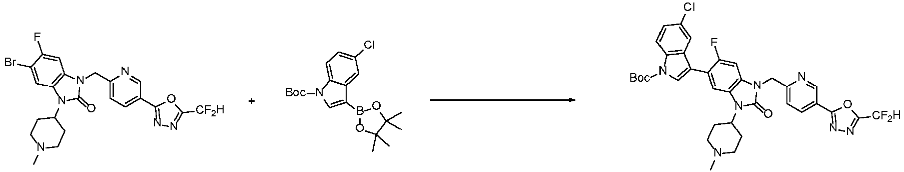

The 5-bromo-1-((5-(5-(difluoromethyl)-1,3,4-oxadiazole-2-yl)pyridine-2-yl)methyl)-6-fluoro-3-(1-methy lpiperidine-4-yl)-1,3-dihydro-2H-benzo[d]imidazole-2-one (0.150 g, 0.279 mmol) prepared in the step 3 of the compound 149, tert-butyl 5-chloro-3-(4,4,5,5-tetramethyl-1,3,2-dioxaborolane-2-yl)-1H-indole-1-carboxylate (0.127 g, 0.335 mmol), cesium carbonate (0.182 g, 0.558 mmol) and [1,1'-bis(di-tert-butylphosphino)ferrocene]palladium dichloride (0.009 g, 0.014 mmol) were mixed in 1,4-dioxane (2.1 mL)/water (0.5 mL) at room temperature, after which the resulting mixture was irradiated with microwave, then heated at 100°C for 30 minutes, and then a reaction was finished by lowering a temperature to room temperature. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.134 g, 68.0%) in a brown solid form.

**¹H NMR** (400 MHz, CDCl₃) δ 9.28 (d, *J* = 1.6 Hz, 1H), 8.34 (dd, *J* = 8.2, 2.2 Hz, 1H), 8.10 (d, *J* = 8.8 Hz, 1H), 7.66 (s, 1H), 7.46 - 7.42 (m, 2H), 7.33 (d, *J* = 5.8 Hz, 1H), 7.26 (dd, *J* = 8.8, 2.1 Hz, 1H), 6.92 (t, *J =* 51.6 Hz, 1H), 6.90 (d, *J* = 9.3 Hz, 1H), 5.27 (s, 2H), 4.47 - 4.38 (m, 1H), 3.02 - 2.99 (m, 2H), 2.52 - 2.43 (m, 2H), 2.31 (s, 3H), 2.19 - 2.13 (m, 2H), 1.88 - 1.86 (m, 2H), 1.67 (s, 9H); **LRMS** (ES) m/z 708.40 (M⁺ + 1).

### Example 255: Synthesis of Compound 255, tert-butyl 5-(1-((5-(5-(difluoromethyl)-1,3,4-oxadiazole-2-yl)pyridine-2-yl)methyl)-6-fluoro-3-(1-methylpiperi dine-4-yl)-2-oxo-2,3-dihydro-1H-benzo[d]imidazole-5-yl)-1H-indazole-1-carboxylate

### [Step 1] Synthesis of the compound 255

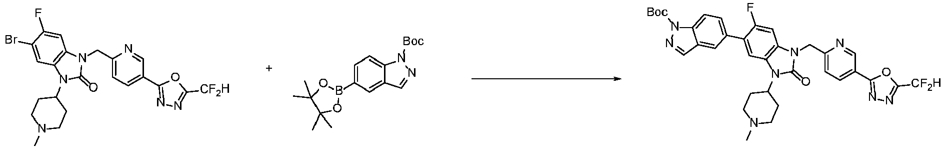

The 5-bromo-1-((5-(5-(difluoromethyl)-1,3,4-oxadiazole-2-yl)pyridine-2-yl)methyl)-6-fluoro-3-(1-methy lpiperidine-4-yl)-1,3-dihydro-2H-benzo[d]imidazole-2-one (0.150 g, 0.279 mmol) prepared in the step 3 of the compound 149, tert-butyl 5-(4,4,5,5-tetramethyl-1,3,2-dioxaborolane-2-yl)-1H-indazole-1-carboxylate (0.115 g, 0.335 mmol), cesium carbonate (0.182 g, 0.558 mmol) and [1,1'-bis(di-tert-butylphosphino)ferrocene]palladium dichloride (0.009 g, 0.014 mmol) were mixed in 1,4-dioxane (2.1 mL)/water (0.5 mL) at room temperature, after which the resulting mixture was irradiated with microwave, then heated at 100°C for 30 minutes, and then a reaction was finished by lowering a temperature to room temperature. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.067 g, 35.5%) in a brown solid form.

**¹H NMR** (400 MHz, CDCl₃) δ 9.28 (s, 1H), 8.34 (dd, *J* = 8.2, 1.7 Hz, 1H), 8.20 - 8.18 (m, 2H), 7.81 (s, 1H), 7.65 (d, *J =* 8.7 Hz, 1H), 7.46 (d, *J = 8.2* Hz, 1H), 7.34 (d, *J* = 6.2 Hz, 1H), 6.92 (t, *J = 51.6* Hz, 1H), 6.88 (d, *J* = 9.8 Hz, 1H), 5.27 (s, 2H), 4.47 - 4.41 (m, 1H), 3.02 (d, *J* = 11.2 Hz, 2H), 2.53 - 2.45 (m, 2H), 2.32 (s, 3H), 2.17 (t, *J* = 11.4 Hz, 2H), 1.87 (d, *J* = 10.3 Hz, 2H), 1.72 (s, 9H); **LRMS** (ES) m/z 675.04 (M⁺ + 1).

### Example 256: Synthesis of Compound 256, 1-((5-(5-(difluoromethyl)-1,3,4-oxadiazole-2-yl)pyridine-2-yl)methyl)-6-fluoro-5-(1H-indazole-5-yl) -3-(1-methylpiperidine-4-yl)-1,3-dihydro-2H-benzo[d]imidazole-2-one

### [Step 1] Synthesis of the compound 256

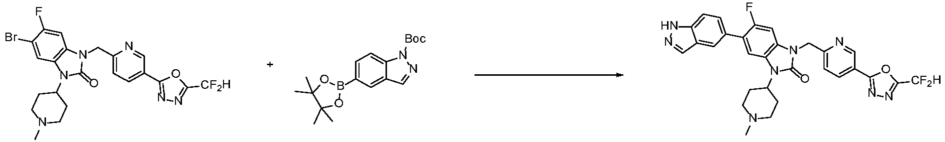

The 5-bromo-1-((5-(5-(difluoromethyl)-1,3,4-oxadiazole-2-yl)pyridine-2-yl)methyl)-6-fluoro-3-(1-methy lpiperidine-4-yl)-1,3-dihydro-2H-benzo[d]imidazole-2-one (0.150 g, 0.279 mmol) prepared in the step 3 of the compound 149, tert-butyl 5-(4,4,5,5-tetramethyl-1,3,2-dioxaborolane-2-yl)-1H-indazole-1-carboxylate (0.115 g, 0.335 mmol), cesium carbonate (0.182 g, 0.558 mmol) and [1,1'-bis(di-tert-butylphosphino)ferrocene]palladium dichloride (0.009 g, 0.014 mmol) were mixed in 1,4-dioxane (2.1 mL)/water (0.5 mL) at room temperature, after which the resulting mixture was irradiated with microwave, then heated at 100°C for 30 minutes, and then a reaction was finished by lowering a temperature to room temperature. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.036 g, 22.5%) in a brown solid form.

**¹H NMR** (400 MHz, CDCl₃) δ 12.37 (s, 1H), 9.29 (d, *J* = 1.4 Hz, 1H), 8.35 (dd, *J* = 8.2, 2.0 Hz, 1H), 7.77 (s, 1H), 7.49 - 7.41 (m, 5H), 6.93 (t, *J =* 51.6 Hz, 1H), 6.84 (d, *J* = 9.8 Hz, 1H), 5.28 (s, 2H), 4.61 -4.55 (m, 1H), 3.17 (d, *J* = 10.9 Hz, 2H), 2.81 - 2.72 (m, 2H), 2.42 (s, 3H), 2.31 (t, *J* = 12.0 Hz, 2H), 1.95 (d, *J* = 10.8 Hz, 2H); **LRMS** (ES) m/z 575.02 (M⁺+ 1).

### Example 257: Synthesis of Compound 257, 1-((5-(5-(difluoromethyl)-1,3,4-oxadiazole-2-yl)pyridine-2-yl)methyl)-6-fluoro-5-(1H-indole-4-yl)-3 -(1-methylazetidine-3-yl)-1,3-dihydro-2H-benzo[d]imidazole-2-one

### [Step 1] Synthesis of tert-butyl 3-((5-bromo-4-fluoro-2-nitrophenyl)amino)azetidine-1-carboxylate

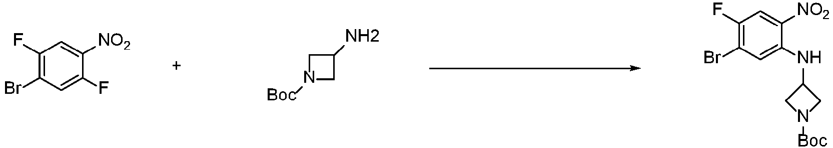

1-bromo-2,5-difluoro-4-nitrobenzene (5.000 g, 21.009 mmol), tert-butyl 3-aminoazetidine-1-carboxylate (4.704 g, 27.312 mmol), potassium carbonate (5.807 g, 42.019 mmol) and potassium iodide (0.349 g, 2.101 mmol) were dissolved in N,N-dimethylformamide (100 mL) at room temperature, after which the resulting solution was stirred at 80°C for 5 hours, and then a reaction was finished by lowering a temperature to room temperature. Water was poured into the reaction mixture and an extraction was performed with ethyl acetate. An organic layer was washed with saturated sodium chloride aqueous solution, then dehydrated with anhydrous magnesium sulfate, then filtered, and then concentrated under reduced pressure. The resulting concentrate was purified via column chromatography (SiO₂, 80 g cartridge; ethyl acetate/hexane = 0 to 30%) and concentrated to obtain a title compound (7.450 g, 90.9%) in a yellow solid form.

### [Step 2] Synthesis of tert-butyl 3-((2-amino-5-bromo-4-fluorophenyl)amino)azetidine-1-carboxylate

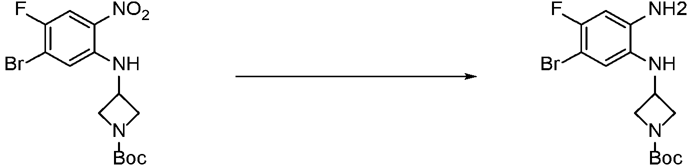

The tert-butyl 3-((5-bromo-4-fluoro-2-nitrophenyl)amino)azetidine-1-carboxylate (7.450 g, 19.092 mmol) prepared in the step 1 was dissolved in methanol (100 mL) and stirred at room temperature, after which Raney nickel (750 mg) was slowly added into the resulting solution at the same temperature and stirred at 60°C for 18 hours in the presence of a hydrogen balloon attached thereto, and then a reaction was finished by lowering a temperature to room temperature. The reaction mixture was filtered via a celite pad to remove a solid therefrom, after which solvent was removed from the resulting filtrate under reduced pressure, and then a title compound was used without an additional purification process (6.830 g, 99.3%, black solid).

### [Step 3] Synthesis of tert-butyl 3-(6-bromo-5-fluoro-2-oxo-2,3-dihydro-1H-benzo[d]imidazole-1-yl)azetidine-1-carboxylate

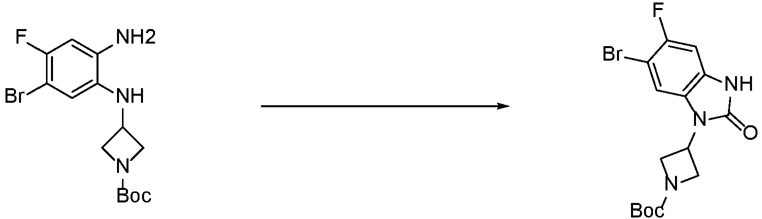

The tert-butyl 3-((2-amino-5-bromo-4-fluorophenyl)amino)azetidine-1-carboxylate (7.450 g, 20.681 mmol) prepared in the step 2, triethylamine (2.883 mL, 20.681 mmol) and 1,1'-carbonyldiimidazole (CDI, 4.360 g, 26.886 mmol) were dissolved in dichloromethane (100 mL) at room temperature, after which the resulting solution was heated under reflux for 18 hours, and then a reaction was finished by lowering a temperature to room temperature. Solvent was removed from the reaction mixture under reduced pressure, after which the resulting concentrate was purified via column chromatography (SiO₂, 80 g cartridge; methanol/dichloromethane = 0 to 3%) and concentrated to obtain a title compound (2.820 g, 35.3%) in a brown solid form.

### [Step 4] Synthesis of tert-butyl 3-(6-bromo-3-((5-(5-(difluoromethyl)-1,3,4-oxadiazole-2-yl)pyridine-2-yl)methyl)-5-fluoro-2-oxo-2 ,3-dihydro-1H-benzo[d]imidazole-1-yl)azetidine-1-carboxylate

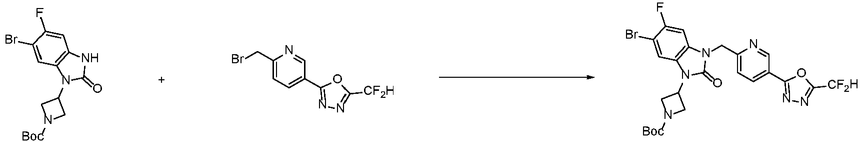

The tert-butyl 3-(6-bromo-5-fluoro-2-oxo-2,3-dihydro-1H-benzo[d]imidazole-1-yl)azetidine-1-carboxylate (2.810 g, 7.276 mmol) prepared in the step 3 was dissolved in N,N-dimethylformamide (40 mL) at 0°C, after which sodium hydride (60.00%, 0.436 g, 10.913 mmol) was added into the resulting solution and stirred at the same temperature for 30 minutes. 2-(6-(bromomethyl)pyridine-3-yl)-5-(difluoromethyl)-1,3,4-oxadiazole (2.321 g, 8.003 mmol) was added into the reaction mixture and further stirred at room temperature for 4 hours. Water was poured into the reaction mixture and an extraction was performed with ethyl acetate. An organic layer was washed with saturated sodium chloride aqueous solution, then dehydrated with anhydrous magnesium sulfate, then filtered, and then concentrated under reduced pressure. The resulting concentrate was purified via column chromatography (SiO₂, 40 g cartridge; ethyl acetate/hexane = 0 to 60%) and concentrated to obtain a title compound (2.950 g, 68.1%) in a brown solid form.

### [Step 5] Synthesis of 1-(azetidine-3-yl)-6-bromo-3-((5-(5-(difluoromethyl)-1,3,4-oxadiazole-2-yl)pyridine-2-yl)methyl)-5 -fluoro-1,3-dihydro-2H-benzo[d]imidazole-2-one

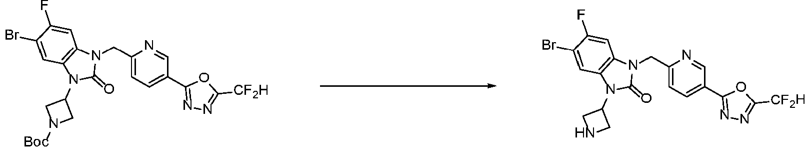

The tert-butyl 3-(6-bromo-3-((5-(5-(difluoromethyl)-1,3,4-oxadiazole-2-yl)pyridine-2-yl)methyl)-5-fluoro-2-oxo-2 ,3-dihydro-1H-benzo[d]imidazole-1-yl)azetidine-1-carboxylate (2.900 g, 4.871 mmol) prepared in the step 4 and trifluoroacetic acid (3.730 mL, 48.708 mmol) were dissolved in dichloromethane (25 mL) at room temperature, after which the resulting solution was stirred at the same temperature for 5 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 40 g cartridge; ethyl acetate/hexane = 80 to 100%) and concentrated, after which an obtained product was purified again via chromatography (SiO₂, 40 g cartridge; methanol/dichloromethane = 0 to 60%) and concentrated to obtain a title compound (2.290 g, 94.9%) in a brown solid form.

### [Step 6] Synthesis of 5-bromo-1-((5-(5-(difluoromethyl)-1,3,4-oxadiazole-2-yl)pyridine-2-yl)methyl)-6-fluoro-3-(1-methy lazetidine-3-yl)-1,3-dihydro-2H-benzo[d]imidazole-2-one

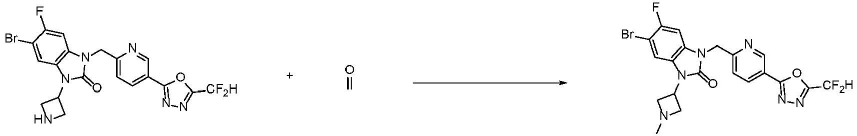

The 1-(azetidine-3-yl)-6-bromo-3-((5-(5-(difluoromethyl)-1,3,4-oxadiazole-2-yl)pyridine-2-yl)methyl)-5 -fluoro-1,3-dihydro-2H-benzo[d]imidazole-2-one (1.540 g, 3.109 mmol) prepared in the step 5, sodium triacetoxyborohydride (1.318 g, 6.219 mmol) and formaldehyde (37.00% solution in water, 0.701 mL, 9.328 mmol) were dissolved in dichloromethane (15 mL) at room temperature, after which the resulting solution was stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 24 g cartridge; methanol/dichloromethane = 0 to 5%) and concentrated to obtain a title compound (0.570 g, 36.0%) in a brown solid form.

### [Step 7] Synthesis of the compound 257

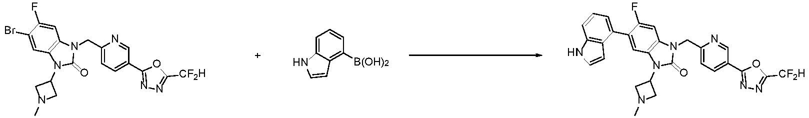

The 5-bromo-1-((5-(5-(difluoromethyl)-1,3,4-oxadiazole-2-yl)pyridine-2-yl)methyl)-6-fluoro-3-(1-methy lazetidine-3-yl)-1,3-dihydro-2H-benzo[d]imidazole-2-one (0.140 g, 0.275 mmol) prepared in the step 6, (1H-indole-4-yl)boronic acid (0.053 g, 0.330 mmol), cesium carbonate (0.179 g, 0.550 mmol) and [1,1'-bis(di-tert-butylphosphino)ferrocene]palladium dichloride (0.009 g, 0.014 mmol) were mixed in 1,4-dioxane (2.1 mL)/water (0.5 mL) at room temperature, after which the resulting mixture was irradiated with microwave, then heated at 100°C for 30 minutes, and then a reaction was finished by lowering a temperature to room temperature. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.036 g, 23.7%) in a brown solid form.

**¹H NMR** (400 MHz, CDCl₃) δ 9.31 (d, *J* = 1.7 Hz, 1H), 8.51 (s, 1H), 8.37 (dd, *J* = 8.2, 2.2 Hz, 1H), 7.77 (d, *J* = 6.1 Hz, 1H), 7.50 (d, *J =* 8.2 Hz, 1H), 7.42 (d, *J =* 8.1 Hz, 1H), 7.29 - 7.24 (m, 2H), 7.18 (d, *J =* 7.3 Hz, 1H), 6.93 (t, *J =* 51.6 Hz, 1H), 6.91 (d, *J =* 9.4 Hz, 1H), 6.49 (d, *J* = 1.9 Hz, 1H), 5.28 (s, 2H), 5.07 - 5.00 (m, 1H), 3.84 - 3.78 (m, 4H), 2.43 (s, 3H); **LRMS** (ES) m/z 545.98 (M⁺ + 1).

### Example 258: Synthesis of Compound 258, 1-((5-(5-(difluoromethyl)-1,3,4-oxadiazole-2-yl)pyridine-2-yl)methyl)-6-fluoro-3-(1-methylazetidin e-3-yl)-5-(pyridine-3-yl)-1,3-dihydro-2H-benzo[d]imidazole-2-one

### [Step 1] Synthesis of the compound 258

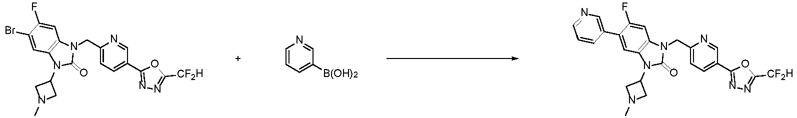

The 5-bromo-1-((5-(5-(difluoromethyl)-1,3,4-oxadiazole-2-yl)pyridine-2-yl)methyl)-6-fluoro-3-(1-methy lazetidine-3-yl)-1,3-dihydro-2H-benzo[d]imidazole-2-one (0.140 g, 0.275 mmol) prepared in the step 6 of the compound 257, pyridine-3-ylboronic acid (0.041 g, 0.330 mmol), cesium carbonate (0.179 g, 0.550 mmol) and [1,1'-bis(di-tert-butylphosphino)ferrocene]palladium dichloride (0.009 g, 0.014 mmol) were mixed in 1,4-dioxane (2.1 mL)/water (0.5 mL) at room temperature, after which the resulting mixture was irradiated with microwave, then heated at 100°C for 30 minutes, and then a reaction was finished by lowering a temperature to room temperature. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 10%) and concentrated to obtain a title compound (0.051 g, 36.6%) in a brown solid form.

**¹H NMR** (400 MHz, CDCl₃) δ 9.27 (d, *J =* 1.8 Hz, 1H), 8.75 (s, 1H), 8.56 (d, *J =* 3.8 Hz, 1H), 8.35 (dd, *J* = 8.2, 2.2 Hz, 1H), 7.83 (dd, *J* = 7.9, 1.5 Hz, 1H), 7.75 (d, *J* = 6.3 Hz, 1H), 7.48 (d, *J =* 8.2 Hz, 1H), 7.35 (dd, *J* = 7.8, 4.8 Hz, 1H), 6.92 (t, *J* = 51.6 Hz, 1H), 6.90 (d, *J* = 9.8 Hz, 1H), 5.24 (s, 2H), 5.12 - 5.05 (m, 1H), 3.91 - 5.05 (m, 4H), 2.51 (s, 3H); **LRMS** (ES) m/z 508.20 (M⁺+ 1).

### Example 259: Synthesis of Compound 259, 1-((5-(5-(difluoromethyl)-1,3,4-oxadiazole-2-yl)pyridine-2-yl)methyl)-6-fluoro-3-(1-methylazetidin e-3-yl)-5-(pyridine-4-yl)-1,3-dihydro-2H-benzo[d]imidazole-2-one

### [Step 1] Synthesis of the compound 259

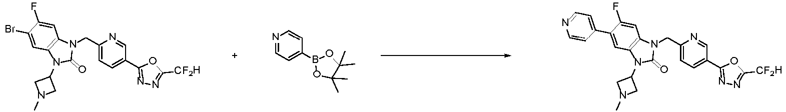

The 5-bromo-1-((5-(5-(difluoromethyl)-1,3,4-oxadiazole-2-yl)pyridine-2-yl)methyl)-6-fluoro-3-(1-methy lazetidine-3-yl)-1,3-dihydro-2H-benzo[d]imidazole-2-one (0.140 g, 0.275 mmol) prepared in the step 6 of the compound 257, 4-(4,4,5,5-tetramethyl-1,3,2-dioxaborolane-2-yl)pyridine (0.068 g, 0.330 mmol), cesium carbonate (0.179 g, 0.550 mmol) and [1,1'-bis(di-tert-butylphosphino)ferrocene]palladium dichloride (0.009 g, 0.014 mmol) were mixed in 1,4-dioxane (2.1 mL)/water (0.5 mL) at room temperature, after which the resulting mixture was irradiated with microwave, then heated at 100°C for 30 minutes, and then a reaction was finished by lowering a temperature to room temperature. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 10%) and concentrated to obtain a title compound (0.021 g, 14.9%) in a brown solid form.

**¹H NMR** (400 MHz, CDCl₃) δ 9.28 (d, *J =* 1.7 Hz, 1H), 8.65 (d, *J =* 5.8 Hz, 1H), 8.36 (dd, *J* = 8.2, 2.2 Hz, 1H), 7.81 (d, *J = 6.3* Hz, 1H), 7.49 (d, *J =* 8.2 Hz, 1H), 7.45 (d, *J* = 4.6 Hz, 1H), 7.05 - 6.79 (m, 2H), 5.25 (s, 2H), 5.14 - 5.07 (m, 1H), 3.94 - 3.87 (m, 4H), 2.55 (s, 3H); **LRMS** (ES) m/z 508.6 (M⁺ + 1).

### Example 260: Synthesis of Compound 260, 1-((5-(5-(difluoromethyl)-1,3,4-oxadiazole-2-yl)pyridine-2-yl)methyl)-6-fluoro-3-methyl-5-(4-meth ylpiperazine-1-yl)-1,3-dihydro-2H-benzo[d]imidazole-2-one

### [Step 1] Synthesis of tert-butyl 4-(2,5-difluoro-4-nitrophenyl)piperazine-1-carboxylate

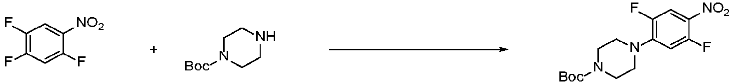

1,2,4-trifluoro-5-nitrobenzene (3.660 g, 20.669 mmol), potassium carbonate (2.999 g, 21.702 mmol) and tert-butyl piperazine-1-carboxylate (4.042 g, 21.702 mmol) were dissolved in N,N-dimethylformamide (80 mL) at room temperature, after which the resulting solution was stirred at the same temperature for 2 hours. Water was poured into the reaction mixture and an extraction was performed with ethyl acetate. An organic layer was washed with saturated sodium chloride aqueous solution, then dehydrated with anhydrous magnesium sulfate, then filtered, and then concentrated under reduced pressure. The resulting concentrate was purified via column chromatography (SiO₂, 40 g cartridge; ethyl acetate/hexane = 0 to 20%) and concentrated to obtain a title compound (5.660 g, 79.8%) in a yellow solid form.

### [Step 2] Synthesis of tert-butyl 4-(2-fluoro-5-(methylamino)-4-nitrophenyl)piperazine-1-carboxylate

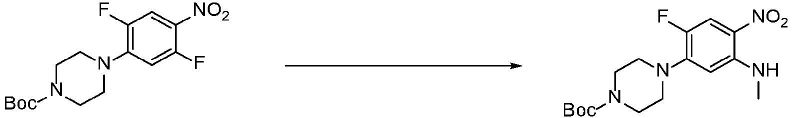

The tert-butyl 4-(2,5-difluoro-4-nitrophenyl)piperazine-1-carboxylate (5.660 g, 16.486 mmol) prepared in the step 1, methylamine (33.00% solution in EtOH, 1.368 mL, 19.783 mmol), potassium carbonate (4.557 g, 32.971 mmol) and potassium iodide (0.274 g, 1.649 mmol) were dissolved in N,N-dimethylformamide (80 mL) at room temperature, after which the resulting solution was stirred at 80°C for 18 hours, and then a reaction was finished by lowering a temperature to room temperature. Water was poured into the reaction mixture and an extraction was performed with ethyl acetate. An organic layer was washed with saturated sodium chloride aqueous solution, then dehydrated with anhydrous magnesium sulfate, then filtered, and then concentrated under reduced pressure. The resulting concentrate was purified via column chromatography (SiO₂, 80 g cartridge; ethyl acetate/hexane = 0 to 30%) and concentrated to obtain a title compound (3.470 g, 59.4%) in an orange solid form.

### [Step 3] Synthesis of tert-butyl 4-(4-amino-2-fluoro-5-(methylamino)phenyl)piperazine-1-carboxylate

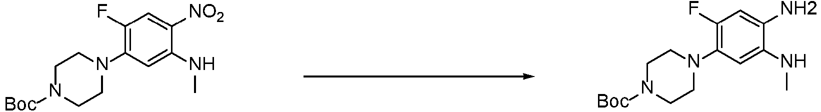

The tert-butyl 4-(2-fluoro-5-(methylamino)-4-nitrophenyl)piperazine-1-carboxylate (3.460 g, 9.764 mmol) prepared in the step 2 was dissolved in methanol (40 mL) and stirred at room temperature, after which 10%-Pd/C (350 mg) was slowly added into the resulting solution at the same temperature and stirred at the same temperature for 18 hours in the presence of a hydrogen balloon attached thereto. The reaction mixture was filtered via a celite pad to remove a solid therefrom, after which solvent was removed from the resulting filtrate without the solid under reduced pressure. Then, the resulting concentrate was purified via column chromatography (SiO₂, 40 g cartridge; ethyl acetate/hexane = 0 to 50%) and concentrated to obtain a title compound (3.020 g, 95.3%) in a violet solid form.

### [Step 4] Synthesis of tert-butyl 4-(6-fluoro-3-methyl-2-oxo-2,3-dihydro-1H-benzo[d]imidazole-5-yl)piperazine-1-carboxylate

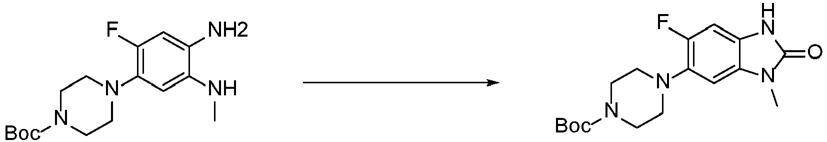

The tert-butyl 4-(4-amino-2-fluoro-5-(methylamino)phenyl)piperazine-1-carboxylate (3.020 g, 9.309 mmol) prepared in the step 3, triethylamine (1.298 mL, 9.309 mmol) and 1,1'-carbonyldiimidazole (CDI, 1.811 g, 11.171 mmol) were dissolved in dichloromethane (30 mL) at room temperature, after which the resulting solution was heated under reflux for 18 hours, and then a reaction was finished by lowering a temperature to room temperature. Solvent was removed from the reaction mixture under reduced pressure, after which the resulting concentrate was purified via column chromatography (SiO₂, 40 g cartridge; methanol/dichloromethane = 0 to 5%) and concentrated to obtain a title compound (3.000 g, 92.0%) in a brown solid form.

### [Step 5] Synthesis of tert-butyl 4-(1-((5-(5-(difluoromethyl)-1,3,4-oxadiazole-2-yl)pyridine-2-yl)methyl)-6-fluoro-3-methyl-2-oxo-2 ,3-dihydro-1H-benzo[d]imidazole-5-yl)piperazine-1-carboxylate

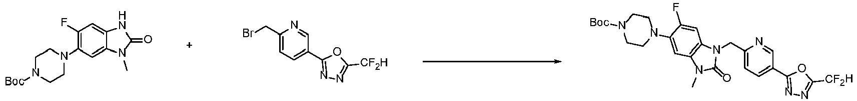

The tert-butyl 4-(6-fluoro-3-methyl-2-oxo-2,3-dihydro-1H-benzo[d]imidazole-5-yl)piperazine-1-carboxylate (3.000 g, 8.562 mmol) prepared in the step 4 was dissolved in N,N-dimethylformamide (40 mL) at 0°C, after which sodium hydride (60.00%, 0.514 g, 12.843 mmol) was added into the resulting solution and stirred at the same temperature for 30 minutes. 2-(6-(bromomethyl)pyridine-3-yl)-5-(difluoromethyl)-1,3,4-oxadiazole (2.732 g, 9.418 mmol) was added into the reaction mixture and further stirred at room temperature for 4 hours. Water was poured into the reaction mixture and an extraction was performed with ethyl acetate. An organic layer was washed with saturated sodium chloride aqueous solution, then dehydrated with anhydrous magnesium sulfate, then filtered, and then concentrated under reduced pressure. The resulting concentrate was purified via column chromatography (SiO₂, 40 g cartridge; ethyl acetate/hexane = 40 to 80%) and concentrated to obtain a title compound (2.950 g, 61.6%) in a brown solid form.

### [Step 6] Synthesis of 1-((5-(5-(difluoromethyl)-1,3,4-oxadiazole-2-yl)pyridine-2-yl)methyl)-6-fluoro-3-methyl-5-(piperazi ne-1-yl)-1,3-dihydro-2H-benzo[d]imidazole-2-one

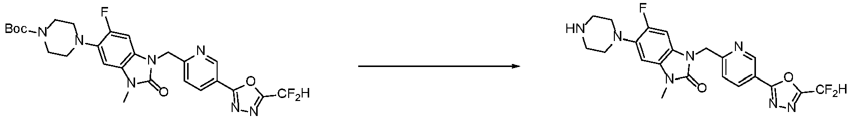

The tert-butyl 4-(1-((5-(5-(difluoromethyl)-1,3,4-oxadiazole-2-yl)pyridine-2-yl)methyl)-6-fluoro-3-methyl-2-oxo-2 ,3-dihydro-1H-benzo[d]imidazole-5-yl)piperazine-1-carboxylate (3.000 g, 5.361 mmol) prepared in the step 5 and trifluoroacetic acid (4.106 mL, 53.615 mmol) were dissolved in dichloromethane (30 mL) at room temperature, after which the resulting solution was stirred at the same temperature for 4 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 40 g cartridge; ethyl acetate/hexane = 50 to 100%) and concentrated to obtain a title compound (2.150 g, 87.3%) in a red solid form.

### [Step 7] Synthesis of the compound 260

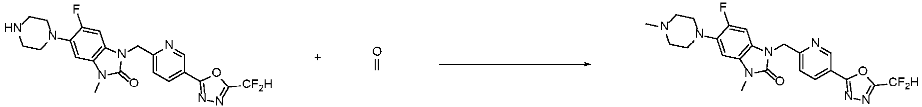

The 1-((5-(5-(difluoromethyl)-1,3,4-oxadiazole-2-yl)pyridine-2-yl)methyl)-6-fluoro-3-methyl-5-(piperazi ne-1-yl)-1,3-dihydro-2H-benzo[d]imidazole-2-one (0.100 g, 0.218 mmol) prepared in the step 6, sodium triacetoxyborohydride (0.092 g, 0.435 mmol) and formaldehyde (0.020 g, 0.653 mmol) were dissolved in dichloromethane (2 mL) at room temperature, after which the resulting solution was stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 10%) and concentrated to obtain a title compound (0.071 g, 68.5%) in a yellow solid form.

**¹H NMR** (400 MHz, CDCl₃) δ 9.22 (d, *J* = 1.6 Hz, 1H), 8.27 (dd, *J* = 8.2, 2.2 Hz, 1H), 7.37 (d, *J* = 8.2 Hz, 1H), 6.91 (t, *J = 51.6* Hz, 1H), 6.70 (d, *J =* 11.2 Hz, 1H), 6.61 (d, *J =* 7.0 Hz, 1H), 5.18 (s, 2H), 3.41 (s, 3H), 3.03 (t, *J* = 4.2 Hz, 4H), 2.26 - 2.26 (m, 4H), 2.31 (s, 3H); **LRMS** (ES) m/z 474.27 (M⁺ + 1).

### Example 261: Synthesis of Compound 261, 1-((5-(5-(difluoromethyl)-1,3,4-oxadiazole-2-yl)pyridine-2-yl)methyl)-6-fluoro-5-(4-isopropylpipera zine-1-yl)-3-methyl-1,3-dihydro-2H-benzo[d]imidazole-2-one

### [Step 1] Synthesis of the compound 261

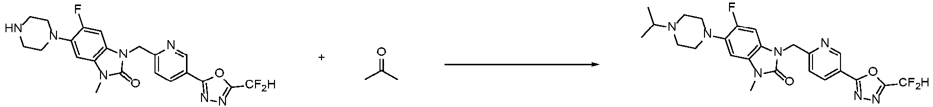

The 1-((5-(5-(difluoromethyl)-1,3,4-oxadiazole-2-yl)pyridine-2-yl)methyl)-6-fluoro-3-methyl-5-(piperazi ne-1-yl)-1,3-dihydro-2H-benzo[d]imidazole-2-one (0.100 g, 0.218 mmol) prepared in the step 6 of the compound 260, sodium triacetoxyborohydride (0.092 g, 0.435 mmol) and propane-2-one (0.038 g, 0.653 mmol) were dissolved in dichloromethane (2 mL) at room temperature, after which the resulting solution was stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 10%) and concentrated to obtain a title compound (0.107 g, 97.9%) in a yellow solid form.

**¹H NMR** (400 MHz, CDCl₃) δ 9.23 (d, *J* = 1.6 Hz, 1H), 8.28 (dd, *J* = 8.2, 2.2 Hz, 1H), 7.38 (d, *J* = 8.2 Hz, 1H), 6.91 (t, *J = 51.6* Hz, 1H), 6.71 (d, *J =* 11.2 Hz, 1H), 6.62 (d, *J* = 7.0 Hz, 1H), 5.18 (s, 2H), 3.41 (s, 3H), 3.05 - 3.03 (m, 4H), 2.74 - 2.67 (m, 5H), 1.06 (d, *J* = 6.5 Hz, 6H); LRMS (ES) m/z 502.05 (M⁺ + 1).

### Example 262: Synthesis of Compound 262, 1-((5-(5-(difluoromethyl)-1,3,4-oxadiazole-2-yl)pyridine-2-yl)methyl)-6-fluoro-3-methyl-5-(4-(2,2,3 ,3-tetrafluoropropyl)piperazine-1-yl)-1,3-dihydro-2H-benzo[d]imidazole-2-one

### [Step 1] Synthesis of the compound 262

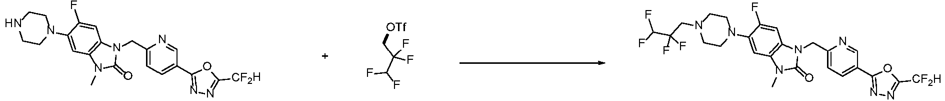

The 1-((5-(5-(difluoromethyl)-1,3,4-oxadiazole-2-yl)pyridine-2-yl)methyl)-6-fluoro-3-methyl-5-(piperazi ne-1-yl)-1,3-dihydro-2H-benzo[d]imidazole-2-one (0.100 g, 0.218 mmol) prepared in the step 6 of the compound 260, potassium carbonate (0.060 g, 0.435 mmol) and 2,2,3,3-tetrafluoropropyl trifluoromethanesulfonate (0.063 g, 0.239 mmol) were dissolved in acetonitrile (2 mL) at room temperature, after which the resulting solution was stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 3%) and concentrated to obtain a title compound (0.095 g, 75.8%) in a yellow solid form.

**¹H NMR** (400 MHz, CDCl₃) δ 9.25 (d, *J* = 1.7 Hz, 1H), 8.30 (dd, *J* = 8.2, 2.2 Hz, 1H), 7.40 (d, *J =* 8.2 Hz, 1H), 6.92 (t, *J = 51.6* Hz, 1H), 6.74 (d, *J* = 11.2 Hz, 1H), 6.63 (d, *J* = 6.9 Hz, 1H), 6.16 - 5.86 (m, 1H), 5.20 (s, 2H), 3.42 (s, 3H), 3.03 (t, *J =* 4.6 Hz, 4H), 2.95 (t, *J* = 14.1 Hz, 2H), 2.80 (t, *J =* 4.6 Hz, 4H); LRMS (ES) m/z 573.96 (M⁺ + 1).

### Example 263: Synthesis of Compound 263, 1-((5-(5-(difluoromethyl)-1,3,4-oxadiazole-2-yl)pyridine-2-yl)methyl)-6-fluoro-3-methyl-5-(4-(2,2,2 -trifluoroethyl)piperazine-1-yl)-1,3-dihydro-2H-benzo[d]imidazole-2-one

### [Step 1] Synthesis of the compound 263

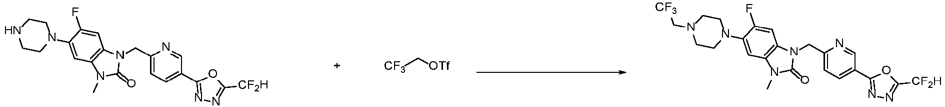

The 1-((5-(5-(difluoromethyl)-1,3,4-oxadiazole-2-yl)pyridine-2-yl)methyl)-6-fluoro-3-methyl-5-(piperazi ne-1-yl)-1,3-dihydro-2H-benzo[d]imidazole-2-one (0.100 g, 0.218 mmol) prepared in the step 6 of the compound 260, potassium carbonate (0.060 g, 0.435 mmol) and 2,2,2-trifluoroethyl trifluoromethanesulfonate (0.056 g, 0.239 mmol) were dissolved in dichloromethane (2 mL) at room temperature, after which the resulting solution was stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 10%) and concentrated to obtain a title compound (0.092 g, 78.2%) in a yellow solid form.

**¹H NMR** (400 MHz, CDCl₃) δ 9.24 (d, *J* = 1.7 Hz, 1H), 8.29 (dd, *J* = 8.2, 2.2 Hz, 1H), 7.40 (d, *J* = 8.2 Hz, 1H), 6.92 (t, *J = 51.6* Hz, 1H), 6.73 (d, *J =* 11.2 Hz, 1H), 6.63 (d, *J =* 6.9 Hz, 1H), 5.19 (s, 2H), 3.42 (s, 3H), 3.06 - 2.98 (m, 6H), 2.84 (t, *J* = 4.7 Hz, 4H); **LRMS** (ES) m/z 541.94 (M⁺ + 1).

### Example 264: Synthesis of Compound 264, 1-((5-(5-(difluoromethyl)-1,3,4-oxadiazole-2-yl)pyridine-2-yl)methyl)-6-fluoro-3-methyl-5-(4-(oxet an-3-yl)piperazine-1-yl)-1,3-dihydro-2H-benzo[d]imidazole-2-one

### [Step 1] Synthesis of the compound 264

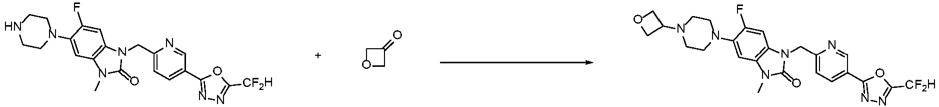

The 1-((5-(5-(difluoromethyl)-1,3,4-oxadiazole-2-yl)pyridine-2-yl)methyl)-6-fluoro-3-methyl-5-(piperazi ne-1-yl)-1,3-dihydro-2H-benzo[d]imidazole-2-one (0.100 g, 0.218 mmol) prepared in the step 6 of the compound 260, sodium triacetoxyborohydride (0.092 g, 0.435 mmol) and oxetan-3-one (0.047 g, 0.653 mmol) were dissolved in dichloromethane (2 mL) at room temperature, after which the resulting solution was stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 10%) and concentrated to obtain a title compound (0.055 g, 48.7%) in a brown solid form.

**¹H NMR** (400 MHz, CDCl₃) δ 9.24 (s, 1H), 8.29 (dd, *J* = 8.2, 2.0 Hz, 1H), 7.39 (d, *J* = 8.2 Hz, 1H), 6.92 (t, *J* = 51.6 Hz, 1H), 6.73 (d, *J* = 11.2 Hz, 1H), 6.63 (d, *J* = 6.9 Hz, 1H), 5.19 (s, 2H), 4.64 (td, *J* = 12.4, 6.1 Hz, 4H), 3.59 - 3.52 (m, 1H), 3.42 (s, 3H), 3.10 - 3.00 (m, 4H), 2.60 - 2.45 (m, 4H); **LRMS** (ES) m/z 516.08 (M⁺+ 1).

### Example 265: Synthesis of Compound 265, 6-chloro-3-((5-(5-(difluoromethyl)-1,3,4-oxadiazole-2-yl)pyridine-2-yl)methyl)benzo[d]oxazole-2( 3H)-one

### [Step 1] Synthesis of the compound 265

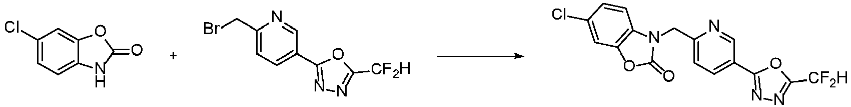

6-chlorobenzo[d]oxazole-2(3H)-one (0.113 g, 0.666 mmol), 2-(6-(bromomethyl)pyridine-3-yl)-5-(difluoromethyl)-1,3,4-oxadiazole (0.213 g, 0.733 mmol), potassium carbonate (0.138 g, 1.000 mmol) and potassium iodide (0.011 g, 0.067 mmol) were dissolved in N,N-dimethylformamide (4 mL) at room temperature, after which the resulting solution was stirred at 100°C for 18 hours, and then a reaction was finished by lowering a temperature to room temperature. Solvent was removed from the reaction mixture under reduced pressure, after which dichloromethane was put into the resulting concentrate and stirred to filter out a precipitated solid, then washed with dichloromethane, and then dried to obtain a title compound (0.124 g, 49.1%) in a white solid form.

**¹H NMR** (400 MHz, CDCl₃) δ 9.13 (d, *J* = 2.1 Hz, 1H), 8.46 (dd, *J* = 8.2, 2.2 Hz, 1H), 7.73 (d, *J* = 8.2 Hz, 1H), 7.70 - 7.44 (m, 2H), 7.27 (dd, *J* = 8.3, 1.8 Hz, 1H), 7.21 (d, *J* = 8.4 Hz, 1H), 5.33 (s, 2H); LRMS (ES) m/z 379.3 (M⁺ + 1).

### Example 266: Synthesis of Compound 266, 1-((5-(5-(difluoromethyl)-1,3,4-oxadiazole-2-yl)pyridine-2-yl)methyl)-6-fluoro-5-(4-(2-hydroxyacet yl)piperazine-1-yl)-3-methyl-1,3-dihydro-2H-benzo[d]imidazole-2-one

### [Step 1] Synthesis of the compound 266

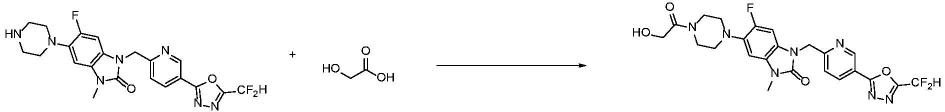

The 1-((5-(5-(difluoromethyl)-1,3,4-oxadiazole-2-yl)pyridine-2-yl)methyl)-6-fluoro-3-methyl-5-(piperazi ne-1-yl)-1,3-dihydro-2H-benzo[d]imidazole-2-one (0.100 g, 0.218 mmol) prepared in the step 6 of the compound 260, 2-hydroxyacetic acid (0.018 g, 0.239 mmol), 1-ethyl-3-(3-dimethylaminopropyl) carbodiimide (EDC, 0.068 g, 0.435 mmol), 1H-benzo[d][1,2,3]triazole-1-ol (HOBt, 0.059 g, 0.435 mmol) and triethylamine (0.061 mL, 0.435 mmol) were dissolved in dichloromethane (2 mL) at room temperature, after which the resulting solution was stirred at the same temperature for 18 hours. Water was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.061 g, 54.5%) in a yellow solid form.

**¹H NMR** (400 MHz, CDCl₃) δ 9.24 (d, *J* = 1.6 Hz, 1H), 8.31 (dd, *J* = 8.2, 2.2 Hz, 1H), 7.41 (d, *J* = 8.2 Hz, 1H), 6.92 (t, *J = 51.6* Hz, 1H), 6.77 (d, *J =* 11.0 Hz, 1H), 6.61 (d, *J =* 6.9 Hz, 1H), 5.20 (s, 2H), 4.18 (s, 2H), 3.81 (t, *J* = 4.9 Hz, 2H), 3.62 (s, 1H), 3.45 - 3.35 (m, 5H), 3.05 - 2.95 (m, 4H); **LRMS** (ES) m/z 518.00 (M⁺ + 1).

### Example 267: Synthesis of Compound 267, 1-((5-(5-(difluoromethyl)-1,3,4-oxadiazole-2-yl)pyridine-2-yl)methyl)-6-fluoro-3-(2-morpholinoeth yl)-5-(pyridine-3-yl)-1,3-dihydro-2H-benzo[d]imidazole-2-one

### [Step 1] Synthesis of 5-bromo-4-fluoro-N-(2-morpholinoethyl)-2-nitroaniline

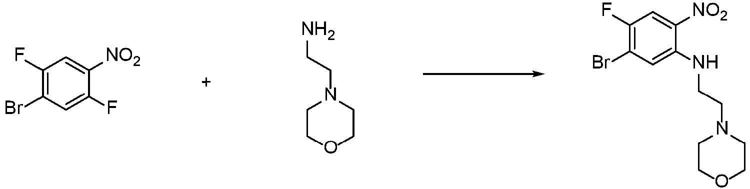

1-bromo-2,5-difluoro-4-nitrobenzene (2.000 g, 8.404 mmol), 2-morpholinoethane-1-amine (1.313 g, 10.084 mmol) and N,N-diisopropylethylamine (2.196 mL, 12.606 mmol) were dissolved in tetrahydrofuran (5 mL) at 65°C, after which the resulting solution was stirred at the same temperature for 18 hours, and then a reaction was finished by lowering a temperature to room temperature. Solvent was removed from the reaction mixture under reduced pressure, after which ethyl acetate (20 mL) and hexane (10 mL) were inserted into the resulting concentrate and stirred to filter out a precipitated solid, then washed with hexane, and then dried to obtain a title compound (2.500 g, 85.4%) in a yellow solid form.

### [Step 2] Synthesis of 5-bromo-4-fluoro-N'-(2-morpholinoethyl)benzene-1,2-diamine

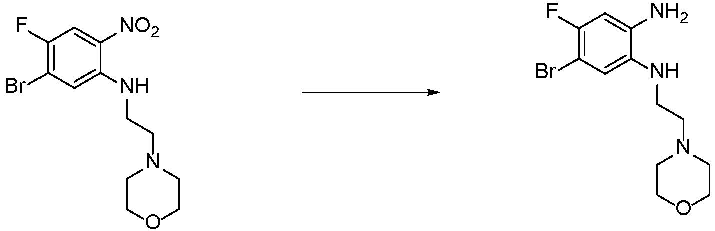

The 5-bromo-4-fluoro-N-(2-morpholinoethyl)-2-nitroaniline (2.500 g, 7.180 mmol) prepared in the step 1 was dissolved in ethanol (50 mL) at room temperature, after which Raney nickel was slowly added thereinto and stirred at the same temperature for 18 hours in the presence of a hydrogen balloon attached thereto. The reaction mixture was filtered via a celite pad to remove a solid therefrom, after which solvent was removed from the resulting filtrate under reduced pressure, and then a title compound was used without an additional purification process (1.800 g, 78.8%, black solid).

### [Step 3] Synthesis of 6-bromo-5-fluoro-1-(2-morpholinoethyl)-1,3-dihydro-2H-benzo[d]imidazole-2-one

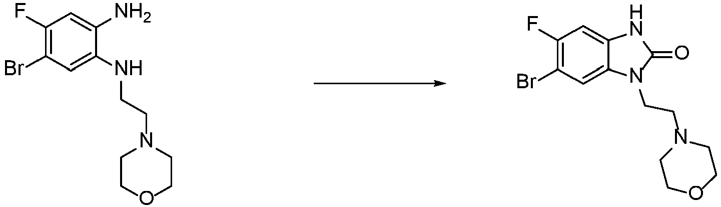

The 5-bromo-4-fluoro-N'-(2-morpholinoethyl)benzene-1,2-diamine (1.800 g, 5.657 mmol) prepared in the step 2 and 1,1'-carbonyldiimidazole (CDI, 1.376 g, 8.485 mmol) were dissolved in tetrahydrofuran (50 mL) at room temperature, after which the resulting solution was stirred at the same temperature for 18 hours. Water was poured into the reaction mixture and an extraction was performed with ethyl acetate. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 40 g cartridge; methanol/dichloromethane = 0 to 10%) and concentrated to obtain a title compound (1.230 g, 63.2%) in a red solid form.

### [Step 4] Synthesis of methyl 6-((5-bromo-6-fluoro-3-(2-morpholinoethyl)-2-oxo-2,3-dihydro-1H-benzo[d]imidazole-1-yl)methyl )nicotinate

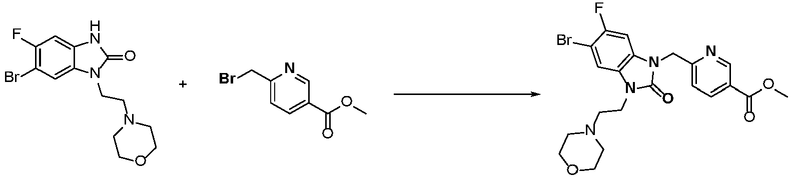

The 6-bromo-5-fluoro-1-(2-morpholinoethyl)-1,3-dihydro-2H-benzo[d]imidazole-2-one (2.000 g, 5.811 mmol) prepared in the step 3 was dissolved in N,N-dimethylformamide (30 mL) at 0°C, after which sodium hydride (60.00%, 0.349 g, 8.716 mmol) was added into the resulting solution and stirred at the same temperature for 30 minutes. Methyl 6-(bromomethyl)nicotinate (1.337 g, 5.811 mmol) was added into the reaction mixture and further stirred at room temperature for 18 hours. Water was poured into the reaction mixture and an extraction was performed with ethyl acetate. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 40 g cartridge; methanol/dichloromethane = 0 to 10%) and concentrated to obtain a title compound (1.160 g, 40.5%) in a colorless oil form.

### [Step 5] Synthesis of 6-((5-bromo-6-fluoro-3-(2-morpholinoethyl)-2-oxo-2,3-dihydro-1H-benzo[d]imidazole-1-yl)methyl )nicotinohydrazide

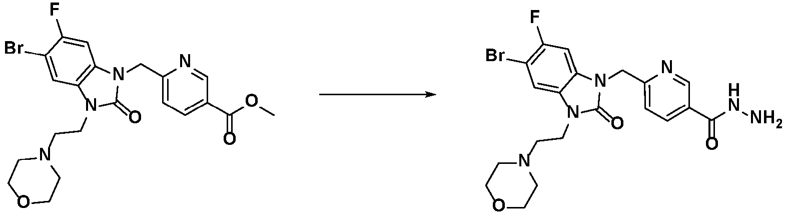

The methyl 6-((5-bromo-6-fluoro-3-(2-morpholinoethyl)-2-oxo-2,3-dihydro-1H-benzo[d]imidazole-1-yl)methyl )nicotinate (1.160 g, 2.351 mmol) prepared in the step 4 and hydrazine monohydrate (2.286 mL, 47.027 mmol) were dissolved in ethanol (10 mL) at 80°C, after which the resulting solution was stirred at the same temperature for 18 hours, and then a reaction was finished by lowering a temperature to room temperature. Solvent was removed from the reaction mixture under reduced pressure, after which the resulting concentrate was purified via column chromatography (SiO₂, 12 g cartridge; ethyl acetate/hexane = 0 to 30%) and concentrated to obtain a title compound (0.652 g, 56.2%) in a white solid form.

### [Step 6] Synthesis of 5-bromo-1-((5-(5-(difluoromethyl)-1,3,4-oxadiazole-2-yl)pyridine-2-yl)methyl)-6-fluoro-3-(2-morp holinoethyl)-1,3-dihydro-2H-benzo[d]imidazole-2-one

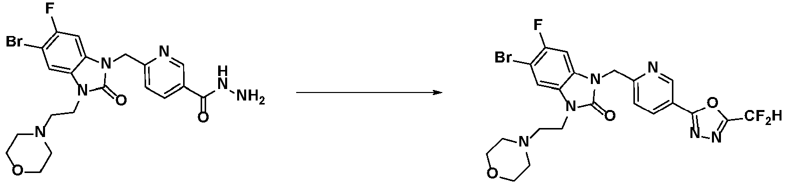

The 6-((5-bromo-6-fluoro-3-(2-morpholinoethyl)-2-oxo-2,3-dihydro-1H-benzo[d]imidazole-1-yl)methyl )nicotinohydrazide (0.652 g, 1.322 mmol) prepared in the step 5, 2,2-difluoroacetic anhydride (0.493 mL, 3.965 mmol) and imidazole (0.270 g, 3.965 mmol) were dissolved in dichloromethane (30 mL) at 45°C, after which the resulting solution was stirred at the same temperature for 18 hours, and then a reaction was finished by lowering a temperature to room temperature. Water was poured into the reaction mixture and an extraction was performed with dichloromethane. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; ethyl acetate/hexane = 0 to 70%) and concentrated to obtain a title compound (0.600 g, 82.0%) in a white foam solid form.

### [Step 7] Synthesis of the compound 267

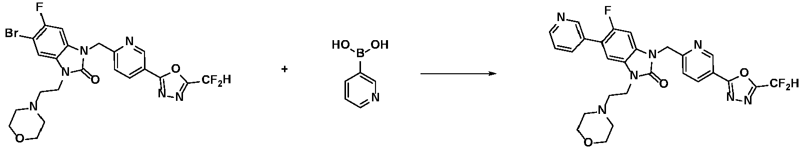

The 5-bromo-1-((5-(5-(difluoromethyl)-1,3,4-oxadiazole-2-yl)pyridine-2-yl)methyl)-6-fluoro-3-(2-morp holinoethyl)-1,3-dihydro-2H-benzo[d]imidazole-2-one (0.100 g, 0.181 mmol) prepared in the step 6, pyridine-3-ylboronic acid (0.033 g, 0.271 mmol), [1,1'-bis(di-tert-butylphosphino)ferrocene]palladium(II) dichloride (Pd(dtbpf)Cl₂, 0.012 g, 0.018 mmol) and cesium carbonate (0.118 g, 0.361 mmol) were mixed in 1,4-dioxane (9 mL)/water (3 mL), after which the resulting mixture was irradiated with microwave, then heated at 100°C for 20 minutes, and then a reaction was finished by lowering a temperature to room temperature. Water was poured into the reaction mixture and an extraction was performed with ethyl acetate. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; methanol/dichloromethane = 0 to 10%) and concentrated to obtain a title compound (0.060 g, 60.2%) in a white foam solid form.

**¹H NMR** (400 MHz, CDCl₃) δ 9.33 (d, *J* = 1.5 Hz, 1H), 8.76 (s, 1H), 8.62 ~ 8.61 (m, 1H), 8.39 (dd, *J* = 8.2, 2.2 Hz, 1H), 7.87 (dd, *J* = 7.9, 1.9 Hz, 1H), 7.51 (d, *J =* 8.4 Hz, 1H), 7.40 (dd, *J* = 7.9, 4.6 Hz, 1H), 7.08 (s, 0.25H), 7.07 (d, *J* = 6.2 Hz, 1H), 6.95 (s, 0.5H), 6.92 (d, *J* = 9.8 Hz, 1H), 6.80 (s, 0.25H), 4.70 (s, 2H), 4.16 ~ 4.09 (m, 2H), 3.69 (t, *J* = 4.6 Hz, 4H), 2.76 (t, *J* = 6.7 Hz, 2H), 2.58 (t, *J* = 4.5 Hz, 4H).

### Example 268: Synthesis of Compound 268, (S)-1-((5-(5-(difluoromethyl)-1,3,4-oxadiazole-2-yl)pyridine-2-yl)methyl)-6-fluoro-3-(1-methylpyrr olidine-3-yl)-5-(pyridine-3-yl)-1,3-dihydro-2H-benzo[d]imidazole-2-one

### [Step 1] Synthesis of tert-butyl (S)-3-((5-bromo-4-fluoro-2-nitrophenyl)amino)pyrrolidine-1-carboxylate

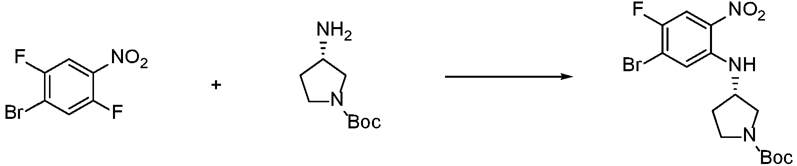

1-bromo-2,5-difluoro-4-nitrobenzene (2.000 g, 8.404 mmol), tert-butyl (S)-3-aminopyrrolidine-1-carboxylate (1.878 g, 10.084 mmol) and N,N-diisopropylethylamine (2.196 mL, 12.606 mmol) were dissolved in tetrahydrofuran (5 mL) at 65°C, after which the resulting solution was stirred at the same temperature for 18 hours, and then a reaction was finished by lowering a temperature to room temperature. Solvent was removed from the reaction mixture under reduced pressure, after which ethyl acetate (20 mL) and hexane (10 mL) were inserted into the resulting concentrate and stirred to filter out a precipitated solid, then washed with hexane, and then dried to obtain a title compound (1.450 g, 42.7%) in a yellow solid form.

### [Step 2] Synthesis of tert-butyl (S)-3-((2-amino-5-bromo-4-fluorophenyl)amino)pyrrolidine-1-carboxylate

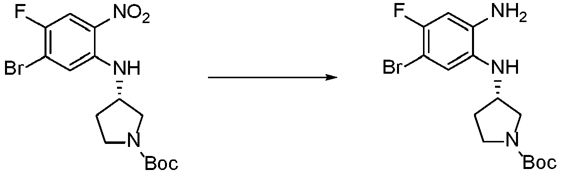

The tert-butyl (S)-3-((5-bromo-4-fluoro-2-nitrophenyl)amino)pyrrolidine-1-carboxylate (1.45 g, 3.587 mmol) prepared in the step 1 was dissolved in ethanol (50 mL) at room temperature, after which Raney nickel was slowly added into the resulting solution and stirred at the same temperature for 18 hours in the presence of a hydrogen balloon attached thereto. The reaction mixture was filtered via a celite pad to remove a solid therefrom, after which solvent was removed from the resulting filtrate under reduced pressure, and then a title compound was used without an additional purification process (1.23 g, 91.6%, black solid).

### [Step 3] Synthesis of tert-butyl (S)-3-(6-bromo-5-fluoro-2-oxo-2,3-dihydro-1H-benzo[d]imidazole-1-yl)pyrrolidine-1-carboxylate

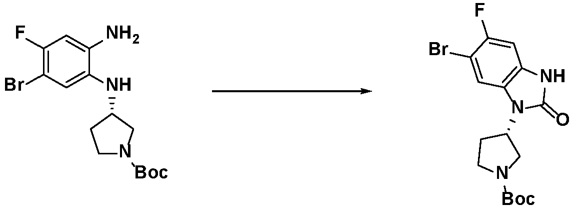

The tert-butyl (S)-3-((2-amino-5-bromo-4-fluorophenyl)amino)pyrrolidine-1-carboxylate (1.230 g, 3.287 mmol) prepared in the step 2 and 1,1'-carbonyldiimidazole (CDI, 0.799 g, 4.930 mmol) were dissolved in tetrahydrofuran (30 mL) at room temperature, after which the resulting solution was stirred at the same temperature for 18 hours. Water was poured into the reaction mixture and an extraction was performed with ethyl acetate. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; ethyl acetate/hexane = 0 to 50%) and concentrated to obtain a title compound (0.830 g, 63.1%) in a white solid form.

### [Step 4] Synthesis of methyl (S)-6-((5-bromo-3-(1-(tert-butoxycarbonyl)pyrrolidine-3-yl)-6-fluoro-2-oxo-2,3-dihydro-1H-benzo[ d]imidazole-1-yl)methyl)nicotinate

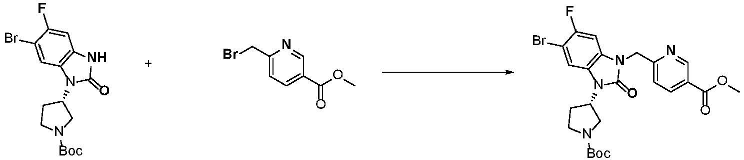

The tert-butyl (S)-3-(6-bromo-5-fluoro-2-oxo-2,3-dihydro-1H-benzo[d]imidazole-1-yl)pyrrolidine-1-carboxylate (0.830 g, 2.074 mmol) prepared in the step 3 was dissolved in N,N-dimethylformamide (30 mL) at 0°C, after which sodium hydride (60.00%, 0.124 g, 3.111 mmol) was added into the resulting solution and stirred at the same temperature for 30 minutes. Methyl 6-(bromomethyl)nicotinate (0.525 g, 2.281 mmol) was added into the reaction mixture and further stirred at room temperature for 18 hours. Water was poured into the reaction mixture and an extraction was performed with ethyl acetate. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 40 g cartridge; ethyl acetate/hexane = 0 to 50%) and concentrated to obtain a title compound (0.634 g, 55.6%) in a white foam solid form.

### [Step 5] Synthesis of tert-butyl (S)-3-(6-bromo-5-fluoro-3-((5-(hydrazinecarbonyl)pyridine-2-yl)methyl)-2-oxo-2,3-dihydro-1H-be nzo[d]imidazole-1-yl)pyrrolidine-1-carboxylate

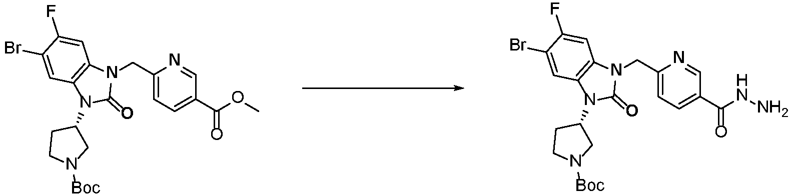

The methyl (S)-6-((5-bromo-3-(1-(tert-butoxycarbonyl)pyrrolidine-3-yl)-6-fluoro-2-oxo-2,3-dihydro-1H-benzo[ d]imidazole-1-yl)methyl)nicotinate (0.634 g, 1.154 mmol) prepared in the step 4 and hydrazine monohydrate (1.122 mL, 23.080 mmol) were dissolved in ethanol (10 mL) at 80°C, after which the resulting solution was stirred at the same temperature for 18 hours, and then a reaction was finished by lowering a temperature to room temperature. Solvent was removed from the reaction mixture under reduced pressure, after which a title compound was used without an additional purification process (0.634 g, 100.0%, white solid).

### [Step 6] Synthesis of tert-butyl (S)-3-(6-bromo-3-((5-(5-(difluoromethyl)-1,3,4-oxadiazole-2-yl)pyridine-2-yl)methyl)-5-fluoro-2-ox o-2,3-dihydro-1H-benzo[d]imidazole-1-yl)pyrrolidine-1-carboxylate

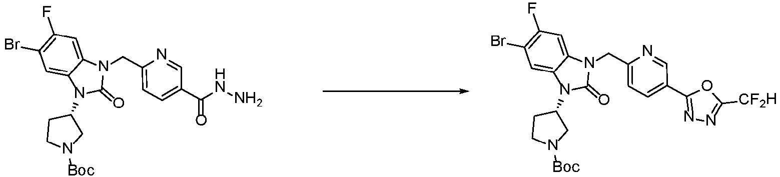

The tert-butyl (S)-3-(6-bromo-5-fluoro-3-((5-(hydrazinecarbonyl)pyridine-2-yl)methyl)-2-oxo-2,3-dihydro-1H-be nzo[d]imidazole-1-yl)pyrrolidine-1-carboxylate (0.634 g, 1.154 mmol) prepared in the step 5, 2,2-difluoroacetic anhydride (0.430 mL, 3.462 mmol) and imidazole (0.236 g, 3.462 mmol) were dissolved in dichloromethane (30 mL) at 45°C, after which the resulting solution was stirred at the same temperature for 18 hours, and then a reaction was finished by lowering a temperature to room temperature. Water was poured into the reaction mixture and an extraction was performed with dichloromethane. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; ethyl acetate/hexane = 0 to 70%) and concentrated to obtain a title compound (0.500 g, 71.1%) in a colorless oil form.

### [Step 7] Synthesis of (S)-5-bromo-1-((5-(5-(difluoromethyl)-1,3,4-oxadiazole-2-yl)pyridine-2-yl)methyl)-6-fluoro-3-(pyrr olidine-3-yl)-1,3-dihydro-2H-benzo[d]imidazole-2-one

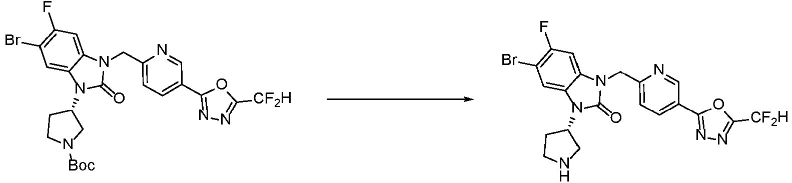

The tert-butyl (S)-3-(6-bromo-3-((5-(5-(difluoromethyl)-1,3,4-oxadiazole-2-yl)pyridine-2-yl)methyl)-5-fluoro-2-ox o-2,3-dihydro-1H-benzo[d]imidazole-1-yl)pyrrolidine-1-carboxylate (0.500 g, 0.820 mmol) prepared in the step 6 and trifluoroacetic acid (0.628 mL, 8.205 mmol) were dissolved in dichloromethane (10 mL) at room temperature, after which the resulting solution was stirred at the same temperature for 18 hours. Solvent was removed from the reaction mixture under reduced pressure, after which saturated sodium hydrogen carbonate aqueous solution was poured into the resulting concentrate, and then an extraction was performed with dichloromethane. An organic layer was washed with saturated sodium chloride aqueous solution, then dehydrated with anhydrous sodium sulfate, then filtered, and then concentrated under reduced pressure. A title compound was used without an additional purification process (0.349 g, 83.5%, yellow oil).

### [Step 8] Synthesis of (S)-5-bromo-1-((5-(5-(difluoromethyl)-1,3,4-oxadiazole-2-yl)pyridine-2-yl)methyl)-6-fluoro-3-(1-m ethylpyrrolidine-3-yl)-1,3-dihydro-2H-benzo[d]imidazole-2-one

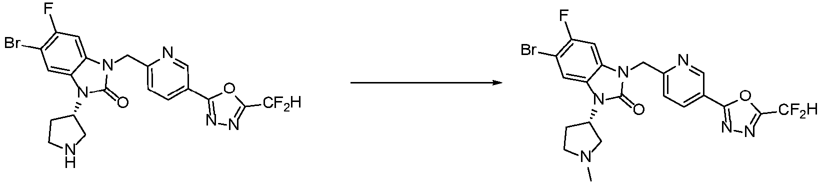

The (S)-5-bromo-1-((5-(5-(difluoromethyl)-1,3,4-oxadiazole-2-yl)pyridine-2-yl)methyl)-6-fluoro-3-(pyrr olidine-3-yl)-1,3-dihydro-2H-benzo[d]imidazole-2-one (0.349 g, 0.685 mmol) prepared in the step 7, formaldehyde (0.041 g, 1.371 mmol) and sodium triacetoxyborohydride (0.290 g, 1.371 mmol) were dissolved in dichloromethane (10 mL) at room temperature, after which the resulting solution was stirred at the same temperature for 18 hours. Water was poured into the reaction mixture and an extraction was performed with dichloromethane. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; methanol/dichloromethane = 0 to 10%) and concentrated to obtain a title compound (0.300 g, 83.7%) in a white foam solid form.

### [Step 9] Synthesis of the compound 268

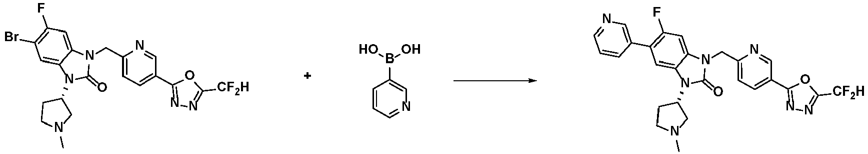

(S)-5-bromo-1-((5-(5-(difluoromethyl)-1,3,4-oxadiazole-2-yl)pyridine-2-yl)methyl)-6-fluoro-3-(1-m ethylpyrrolidine-3-yl)-1,3-dihydro-2H-benzo[d]imidazole-2-one (0.100 g, 0.191 mmol), pyridine-3-ylboronic acid (0.035 g, 0.287 mmol), [1,1'-bis(di-tert-butylphosphino)ferrocene]palladium(II) dichloride (Pd(dtbpf)Cl₂, 0.012 g, 0.019 mmol) and cesium carbonate (0.125 g, 0.382 mmol) were mixed in 1,4-dioxane (9 mL)/water (3 mL), after which the resulting mixture was irradiated with microwave, then heated at 100°C for 20 minutes, and then a reaction was finished by lowering a temperature to room temperature. Water was poured into the reaction mixture and an extraction was performed with ethyl acetate. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; methanol/dichloromethane = 0 to 10%) and concentrated to obtain a title compound (0.010 g, 10.0%) in a colorless oil form.

**¹H NMR** (400 MHz, CDCl₃) δ 9.33 (d, *J =* 1.6 Hz, 1H), 8.78 (s, 1H), 8.61 (d, *J =* 3.5 Hz, 1H), 8.39 (dd, *J* = 8.2, 2.2 Hz, 1H), 7.97 (d, *J = 6.3* Hz, 1H), 7.88 (dd, *J* = 7.8, 1.6 Hz, 1H), 7.50 (d, *J* = 8.2 Hz, 1H), 7.41 ~ 7.38 (m, 1H), 7.08 (s, 0.25H), 6.95 (s, 0.5H), 6.91 (d, *J* = 10.0 Hz, 1H), 6.82 (s, 0.25H), 5.25 (s, 2H), 3.17 ~ 3.15 (m, 2H), 1.70 ~ 1.60 (m, 1H), 2.45 ~ 2.38 (m, 5H), 2.35 ~ 2.20 (m, 2H).

### Example 269: Synthesis of Compound 269, 1-((5-(5-(difluoromethyl)-1,3,4-oxadiazole-2-yl)pyridine-2-yl)methyl)-6-fluoro-5-(1H-indole-4-yl)-3 -(2-morpholinoethyl)-1,3-dihydro-2H-benzo[d]imidazole-2-one

### [Step 1] Synthesis of the compound 269

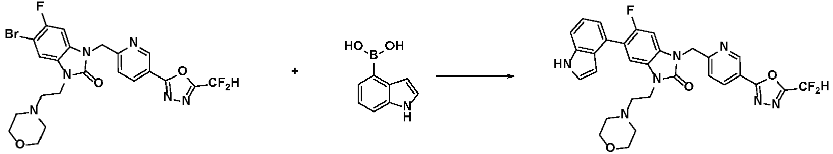

5-bromo-1-((5-(5-(difluoromethyl)-1,3,4-oxadiazole-2-yl)pyridine-2-yl)methyl)-6-fluoro-3-( 2-morpholinoethyl)-1,3-dihydro-2H-benzo[d]imidazole-2-one (0.200 g, 0.361 mmol), (1H-indole-4-yl)boronic acid (0.087 g, 0.542 mmol), [1,1'-bis(di-tert-butylphosphino)ferrocene]palladium(II) dichloride (Pd(dtbpf)Cl₂, 0.024 g, 0.036 mmol) and cesium carbonate (0.236 g, 0.723 mmol) were mixed in 1,4-dioxane (9 mL)/water (3 mL), after which the resulting mixture was irradiated with microwave, then heated at 100°C for 20 minutes, and then a reaction was finished by lowering a temperature to room temperature. Water was poured into the reaction mixture and an extraction was performed with ethyl acetate. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; methanol/dichloromethane = 0 to 10%) and concentrated to obtain a title compound (0.100 g, 46.9%) in a white foam solid form.

**¹H NMR** (400 MHz, CDCl₃) δ 9.35 (d, *J* = 1.6 Hz, 1H), 8.41 ~ 8.38 (m, 2H), 7.52 (d, *J =* 8.2 Hz, 1H), 7.45 (d, *J* = 8.1 Hz, 1H), 7.31 ~ 7.26 (m, 3H), 7.22 (d, *J* = 5.9 Hz, 1H), 7.17 (d, *J =* 7.3 Hz, 1H), 7.08 (s, 0.25H), 6.95 (s, 0.5H), 6.91 (d, *J =* 9.4 Hz, 1H), 6.82 (s, 0.25H), 6.48 (d, *J =* 2.1 Hz, 1H), 5.32 (s, 2H), 4.17 ~ 4.08 (m, 2H), 3.70 (t, *J* = 4.5 Hz, 4H), 2.76 (t, *J* = 6.7 Hz, 2H), 2.60 ~ 2.58 (m, 4H).

### Example 270: Synthesis of Compound 270, (S)-1-((5-(5-(difluoromethyl)-1,3,4-oxadiazole-2-yl)pyridine-2-yl)methyl)-6-fluoro-5-(1H-indole-4-y I)-3-(1-methylpyrrolidine-3-yl)-1,3-dihydro-2H-benzo[d]imidazole-2-one

### [Step 1] Synthesis of the compound 270

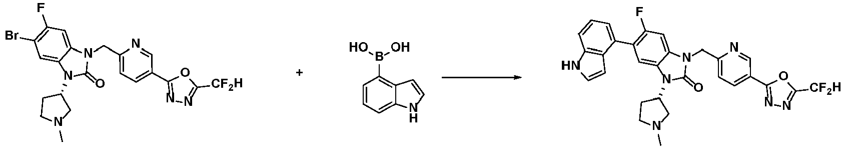

(S)-5-bromo-1-((5-(5-(difluoromethyl)-1,3,4-oxadiazole-2-yl)pyridine-2-yl)methyl)-6-fluor o-3-(1-methylpyrrolidine-3-yl)-1,3-dihydro-2H-benzo[d]imidazole-2-one (0.100 g, 0.191 mmol), (1H-indole-4-yl)boronic acid (0.046 g, 0.287 mmol), [1,1'-bis(di-tert-butylphosphino)ferrocene]palladium(II) dichloride (Pd(dtbpf)Cl₂, 0.012 g, 0.019 mmol) and cesium carbonate (0.125 g, 0.382 mmol) were mixed in 1,4-dioxane (9 mL)/water (3 mL), after which the resulting mixture was irradiated with microwave, then heated at 100°C for 20 minutes, and then a reaction was finished by lowering a temperature to room temperature. Water was poured into the reaction mixture and an extraction was performed with ethyl acetate. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; methanol/dichloromethane = 0 to 10%) and concentrated to obtain a title compound (0.030 g, 28.1%) in a white foam solid form.

**¹H NMR** (400 MHz, CDCl₃) δ 9.34 ~ 9.34 (m, 1H), 8.67 (s, 1H), 8.40 (dd, *J* = 8.2, 2.2 Hz, 1H), 7.99 (d, *J* = 6.2 Hz, 1H), 7.52 (d, *J =* 8.0 Hz, 1H), 7.46 (d, *J* = 8.1 Hz, 1H), 7.30 ~ 7.26 (m, 2H), 7.21 (d, *J* = 7.3 Hz, 1H), 7.08 (s, 0.25H), 6.95 (s, 0.5H), 6.91 (d, *J* = 9.6 Hz, 1H), 6.82 (s, 0.25H), 6.53 ~ 6.52 (m, 1H), 5.31 (s, 2H), 3.10 ~ 2.95 (m, 2H), 1.85 ~ 1.70 (m, 1H), 2.50 ~ 2.40 (m, 6H), 2.15 ~ 2.10 (m, 1H); **LRMS** (ES) m/z 560.6 (M⁺+ 1).

### Example 271: Synthesis of Compound 271, 5-chloro-3-((5-(5-(difluoromethyl)-1,3,4-oxadiazole-2-yl)pyridine-2-yl)methyl)-1-(1-(oxetan-3-yl)pi peridine-4-yl)-1,3-dihydro-2H-benzo[d]imidazole-2-one

### [Step 1] Synthesis of the compound 271

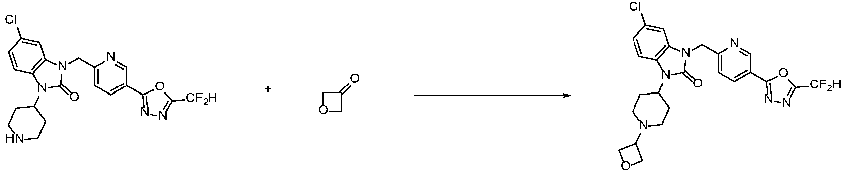

The 5-chloro-3-((5-(5-(difluoromethyl)-1,3,4-oxadiazole-2-yl)pyridine-2-yl)methyl)-1-(piperidine-4-yl)-1,3-dihydro-2H-benzo[d]imidazole-2-one (0.100 g, 0.217 mmol) prepared in the step 5 of the compound 213, oxetan-3-one (0.047 g, 0.651 mmol) and sodium triacetoxyborohydride (0.092 g, 0.434 mmol) were dissolved in dichloromethane (2 mL) at room temperature, after which the resulting solution was stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.071 g, 63.3%) in a yellow solid form.

**¹H NMR** (400 MHz, CDCl₃) δ 9.26 (d, *J* = 1.6 Hz, 1H), 8.32 (dd, *J* = 8.2, 2.2 Hz, 1H), 7.22 (d, *J =* 135.8 Hz, 1H), 7.23 (d, *J* = 8.5 Hz, 1H), 7.05 - 6.79 (m, 3H), 5.22 (s, 2H), 4.64 (td, *J =* 14.4, 7.5 Hz, 4H), 4.45 - 4.36 (m, 1H), 3.55 - 3.49 (m, 1H), 2.89 (d, *J* = 11.5 Hz, 2H), 2.49 - 2.38 (m, 2H), 2.04 - 1.98 (m, 2H), 1.85 (dd, *J* = 12.0, 2.2 Hz, 2H); LRMS (ES) m/z 519.2 (M⁺+ 1).

### Example 272: Synthesis of Compound 272, (R)-1-((5-(5-(difluoromethyl)-1,3,4-oxadiazole-2-yl)pyridine-2-yl)methyl)-6-fluoro-3-(1-methylpyrr olidine-3-yl)-5-(pyridine-4-yl)-1,3-dihydro-2H-benzo[d]imidazole-2-one

### [Step 1] Synthesis of tert-butyl (R)-3-((5-bromo-4-fluoro-2-nitrophenyl)amino)pyrrolidine-1-carboxylate

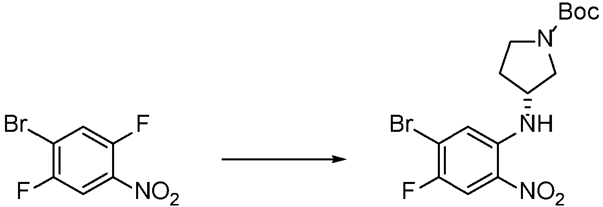

1-bromo-2,5-difluoro-4-nitrobenzene (2.000 g, 8.404 mmol), tert-butyl (R)-3-aminopyrrolidine-1-carboxylate (1.722 g, 9.244 mmol) and N,N-diisopropylethylamine (2.196 mL, 12.606 mmol) were dissolved in tetrahydrofuran (50 mL) at room temperature, after which the resulting solution was stirred at 60°C for 18 hours, and then a reaction was finished by lowering a temperature to room temperature. Water was poured into the reaction mixture and an extraction was performed with dichloromethane. An organic layer was washed with saturated sodium chloride aqueous solution, then dehydrated with anhydrous magnesium sulfate, then filtered, and then concentrated under reduced pressure. A title compound was used without an additional purification process (3.370 g, 99.2%, orange solid).

### [Step 2] Synthesis of tert-butyl (R)-3-((2-amino-5-bromo-4-fluorophenyl)amino)pyrrolidine-1-carboxylate

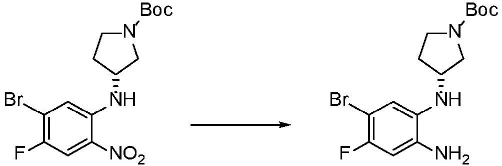

The tert-butyl (R)-3-((5-bromo-4-fluoro-2-nitrophenyl)amino)pyrrolidine-1-carboxylate (3.370 g, 8.337 mmol) prepared in the step 1 was dissolved in methanol (100 mL) and stirred at room temperature, after which Raney nickel (350 mg) was slowly added into the resulting solution at the same temperature and stirred at the same temperature for 18 hours in the presence of a hydrogen balloon attached thereto. The reaction mixture was filtered via a celite pad to remove a solid therefrom, after which solvent was removed from a resulting filtrate under reduced pressure, and then a title compound was used without an additional purification process (3.110 g, 99.7%, brown oil).

### [Step 3] Synthesis of tert-butyl (R)-3-(6-bromo-5-fluoro-2-oxo-2,3-dihydro-1H-benzo[d]imidazole-1-yl)pyrrolidine-1-carboxylate

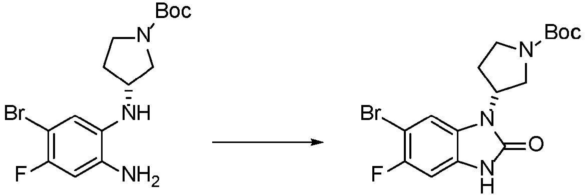

The tert-butyl (R)-3-((2-amino-5-bromo-4-fluorophenyl)amino)pyrrolidine-1-carboxylate (3.110 g, 8.310 mmol) prepared in the step 2 and 1,1'-carbonyldiimidazole (CDI, 1.347 g, 8.310 mmol) were dissolved in dichloromethane (100 mL) at room temperature, after which the resulting solution was heated under reflux for 18 hours, and then a reaction was finished by lowering a temperature to room temperature. Water was poured into the reaction mixture and an extraction was performed with dichloromethane. An organic layer was washed with saturated sodium chloride aqueous solution, then dehydrated with anhydrous sodium sulfate, then filtered, and then concentrated under reduced pressure. A precipitated solid was filtered, then washed with dichloromethane, and then dried to obtain a title compound (2.050 g, 61.6%) in a violet solid form.

### [Step 4] Synthesis of tert-butyl (R)-3-(6-bromo-3-((5-(5-(difluoromethyl)-1,3,4-oxadiazole-2-yl)pyridine-2-yl)methyl)-5-fluoro-2-o xo-2,3-dihydro-1H-benzo[d]imidazole-1-yl)pyrrolidine-1-carboxylate

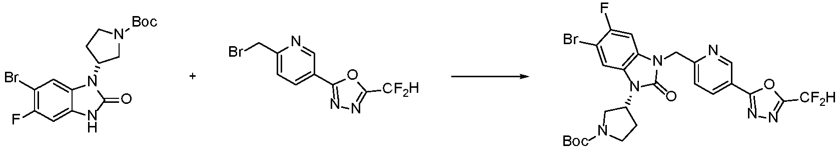

The tert-butyl (R)-3-(6-bromo-5-fluoro-2-oxo-2,3-dihydro-1H-benzo[d]imidazole-1-yl)pyrrolidine-1-carboxylate (2.050 g, 5.122 mmol) prepared in the step 3, 2-(6-(bromomethyl)pyridine-3-yl)-5-(difluoromethyl)-1,3,4-oxadiazole (1.634 g, 5.634 mmol), potassium carbonate (1.062 g, 7.683 mmol) and potassium iodide (0.085 g, 0.512 mmol) were dissolved in N,N-dimethylformamide (20 mL) at room temperature, after which the resulting solution was stirred at 100°C for 18 hours, and then a reaction was finished by lowering a temperature to room temperature. Solvent was removed from the reaction mixture under reduced pressure, after which saturated sodium hydrogen carbonate aqueous solution was poured into the resulting concentrate, and then an extraction was performed with dichloromethane. An organic layer was washed with saturated sodium chloride aqueous solution, then dehydrated with anhydrous magnesium sulfate, then filtered, and then concentrated under reduced pressure. The resulting concentrate was purified via column chromatography (SiO₂, 40 g cartridge; ethyl acetate/hexane = 10 to 50%) and concentrated to obtain a title compound (1.400 g, 44.9%) in a light yellow solid form.

### [Step 5] Synthesis of (R)-5-bromo-1-((5-(5-(difluoromethyl)-1,3,4-oxadiazole-2-yl)pyridine-2-yl)methyl)-6-fluoro-3-(pyrr olidine-3-yl)-1,3-dihydro-2H-benzo[d]imidazole-2-one

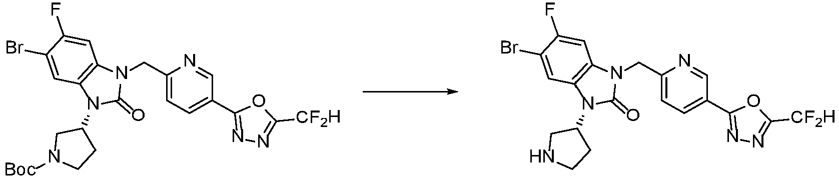

The tert-butyl (R)-3-(6-bromo-3-((5-(5-(difluoromethyl)-1,3,4-oxadiazole-2-yl)pyridine-2-yl)methyl)-5-fluoro-2-o xo-2,3-dihydro-1H-benzo[d]imidazole-1-yl)pyrrolidine-1-carboxylate (1.400 g, 2.297 mmol) prepared in the step 4 and trifluoroacetic acid (1.056 mL, 13.784 mmol) were dissolved in dichloromethane (15 mL) at room temperature, after which the resulting solution was stirred at the same temperature for 5 hours. Saturated sodium hydrogen carbonate aqueous solution was poured into the reaction mixture and an extraction was performed with dichloromethane. An organic layer was washed with saturated sodium chloride aqueous solution, then dehydrated with anhydrous magnesium sulfate, then filtered, and then concentrated under reduced pressure. A title compound was used without an additional purification process (1.030 g, 88.0%, orange solid).

### [Step 6] Synthesis of (R)-5-bromo-1-((5-(5-(difluoromethyl)-1,3,4-oxadiazole-2-yl)pyridine-2-yl)methyl)-6-fluoro-3-(1-m ethylpyrrolidine-3-yl)-1,3-dihydro-2H-benzo[d]imidazole-2-one

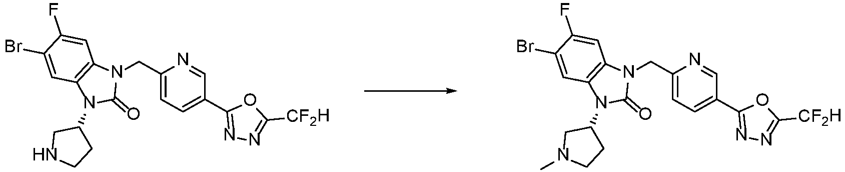

The (R)-5-bromo-1-((5-(5-(difluoromethyl)-1,3,4-oxadiazole-2-yl)pyridine-2-yl)methyl)-6-fluoro-3-(pyrr olidine-3-yl)-1,3-dihydro-2H-benzo[d]imidazole-2-one (0.500 g, 0.982 mmol) prepared in the step 5 and formaldehyde (38.00% solution, 0.108 mL, 1.473 mmol) were dissolved in dichloromethane (10 mL) at room temperature, after which sodium triacetoxyborohydride (0.416 g, 1.964 mmol) was added into the resulting solution and stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 12 g cartridge; methanol/dichloromethane = 0 to 5%) and concentrated to obtain a title compound (0.440 g, 85.6%) in a white solid form.

### [Step 7] Synthesis of the compound 272

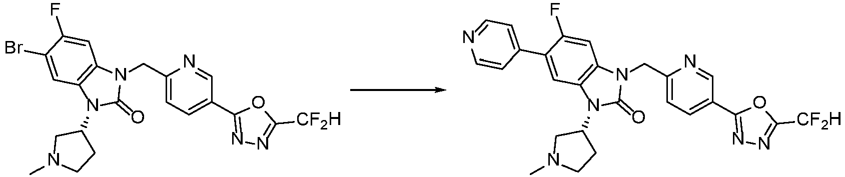

The (R)-5-bromo-1-((5-(5-(difluoromethyl)-1,3,4-oxadiazole-2-yl)pyridine-2-yl)methyl)-6-fluoro-3-(1-m ethylpyrrolidine-3-yl)-1,3-dihydro-2H-benzo[d]imidazole-2-one (0.100 g, 0.191 mmol) prepared in the step 6, pyridine-4-ylboronic acid (0.028 g, 0.229 mmol), [1,1'-bis(di-tert-butylphosphino)ferrocene]palladium(II) dichloride (Pd(dtbpf)Cl₂, 0.006 g, 0.010 mmol) and cesium carbonate (0.187 g, 0.573 mmol) were mixed in 1,4-dioxane (1.5 mL)/water (0.5 mL) at room temperature, after which the resulting mixture was irradiated with microwave, then heated at 100°C for 30 minutes, and then a reaction was finished by lowering a temperature to room temperature. Solvent was removed from the reaction mixture under reduced pressure, after which saturated sodium hydrogen carbonate aqueous solution was poured into the resulting concentrate, and then an extraction was performed with dichloromethane. An organic layer was washed with saturated sodium chloride aqueous solution, then dehydrated with anhydrous magnesium sulfate, then filtered, and then concentrated under reduced pressure. The resulting concentrate was purified via column chromatography (SiO₂, 4 g cartridge; methanol/dichloromethane = 0 to 10%) and concentrated to obtain a title compound (0.005 g, 5.0%) in a brown solid form.

**¹H NMR** (400 MHz, CDCl₃) δ 9.32 (d, *J =* 1.6 Hz, 1H), 8.68 (d, *J =* 5.8 Hz, 2H), 8.39 (dd, *J* = 8.2, 2.2 Hz, 1H), 8.09 (d, *J =* 6.6 Hz, 1H), 7.52 - 7.48 (m, 3H), 7.08 - 6.82 (m, 2H), 5.33 - 5.24 (m, 3H), 3.19 - 3.13 (m, 2H), 2.65 (t, *J =* 9.6 Hz, 1H), 2.46 (s, 3H), 2.43 - 2.35 (m, 2H), 2.19 - 2.16 (m, 1H); **LRMS** (ES) m/z 522.5 (M⁺ + 1).

### Example 273: Synthesis of Compound 273, (R)-1-((5-(5-(difluoromethyl)-1,3,4-oxadiazole-2-yl)pyridine-2-yl)methyl)-6-fluoro-5-(1H-indole-4-yl)-3-(1-methylpyrrolidine-3-yl)-1,3-dihydro-2H-benzo[d]imidazole-2-one

### [Step 1] Synthesis of the compound 273

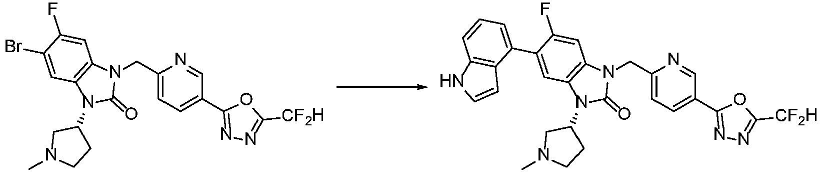

(R)-5-bromo-1-((5-(5-(difluoromethyl)-1,3,4-oxadiazole-2-yl)pyridine-2-yl)methyl)-6-fluor o-3-(1-methylpyrrolidine-3-yl)-1,3-dihydro-2H-benzo[d]imidazole-2-one (0.100 g, 0.191 mmol), (1H-indole-4-yl)boronic acid (0.037 g, 0.229 mmol), [1,1'-bis(di-tert-butylphosphino)ferrocene]palladium(II) dichloride (Pd(dtbpf)Cl₂, 0.006 g, 0.010 mmol) and cesium carbonate (0.187 g, 0.573 mmol) were mixed in 1,4-dioxane (1.5 mL)/water (0.5 mL) at room temperature, after which the resulting mixture was irradiated with microwave, then heated at 100°C for 30 minutes, and then a reaction was finished by lowering a temperature to room temperature. Solvent was removed from the reaction mixture under reduced pressure, after which saturated sodium hydrogen carbonate aqueous solution was poured into the resulting concentrate, and then an extraction was performed with dichloromethane. An organic layer was washed with saturated sodium chloride aqueous solution, then dehydrated with anhydrous magnesium sulfate, then filtered, and then concentrated under reduced pressure. The resulting concentrate was purified via column chromatography (SiO₂, 4 g cartridge; methanol/dichloromethane = 0 to 10%) and concentrated to obtain a title compound (0.015 g, 14.0%) in a brown solid form.

**¹H NMR** (400 MHz, CDCl₃) δ 9.34 (d, *J =* 1.6 Hz, 1H), 8.51 (s, 1H), 8.39 (dd, *J* = 8.2, 2.2 Hz, 1H), 8.03 (d, *J =* 6.3 Hz, 1H), 7.51 (d, *J =* 8.2 Hz, 1H), 7.45 (d, *J =* 8.0 Hz, 1H), 7.31 - 7.27 (m, 3H), 7.22 (d, *J =* 7.3 Hz, 1H), 7.08 - 6.82 (m, 2H), 6.55 (s, 1H), 5.31 - 5.26 (m, 3H), 3.11 (dd, *J* = 10.2, 4.0 Hz, 1H), 3.00 -2.98 (m, 1H), 2.71 (t, *J =* 9.5 Hz, 1H), 2.40 -2.33 (m, 5H), 2.25 -2.24 (m, 1H); **LRMS** (ES) m/z 560.5 (M⁺ + 1).

### Example 274: Synthesis of Compound 274, (R)-1-((5-(5-(difluoromethyl)-1,3,4-oxadiazole-2-yl)pyridine-2-yl)methyl)-6-fluoro-3-(1-methylpipe ridine-3-yl)-5-(pyridine-3-yl)-1,3-dihydro-2H-benzo[d]imidazole-2-one

### [Step 1] Synthesis of tert-butyl (R)-3-((5-bromo-4-fluoro-2-nitrophenyl)amino)piperidine-1-carboxylate

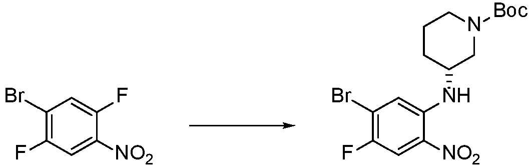

1-bromo-2,5-difluoro-4-nitrobenzene (2.000 g, 8.404 mmol), tert-butyl (R)-3-aminopiperidine-1-carboxylate (1.851 g, 9.244 mmol) and N,N-diisopropylethylamine (2.196 mL, 12.606 mmol) were dissolved in tetrahydrofuran (50 mL) at room temperature, after which the resulting solution was stirred at 60°C for 18 hours, and then a reaction was finished by lowering a temperature to room temperature. Water was poured into the reaction mixture and an extraction was performed with dichloromethane. An organic layer was washed with saturated sodium chloride aqueous solution, then dehydrated with anhydrous magnesium sulfate, then filtered, and then concentrated under reduced pressure. A title compound was used without an additional purification process (3.290 g, 93.6%, orange solid).

### [Step 2] Synthesis of tert-butyl (R)-3-((2-amino-5-bromo-4-fluorophenyl)amino)piperidine-1-carboxylate

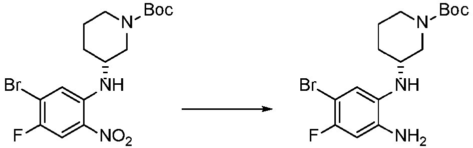

The tert-butyl (R)-3-((5-bromo-4-fluoro-2-nitrophenyl)amino)piperidine-1-carboxylate (3.290 g, 7.866 mmol) prepared in the step 1 was dissolved in methanol (100 mL) and stirred at room temperature, after which Raney nickel (350 mg) was slowly added into the resulting solution at the same temperature and stirred at the same temperature for 18 hours in the presence of a hydrogen balloon attached thereto. The reaction mixture was filtered via a celite pad to remove a solid therefrom, after which solvent was removed from a resulting filtrate under reduced pressure, and then a title compound was used without an additional purification process (2.990 g, 97.9%, brown oil).

### [Step 3] Synthesis of tert-butyl (R)-3-(6-bromo-5-fluoro-2-oxo-2,3-dihydro-1H-benzo[d]imidazole-1-yl)piperidine-1-carboxylate

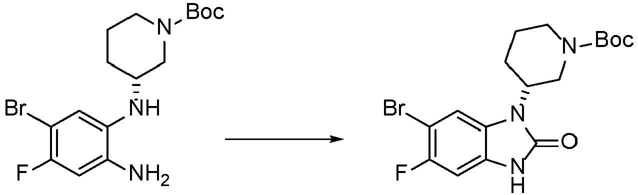

The tert-butyl (R)-3-((2-amino-5-bromo-4-fluorophenyl)amino)piperidine-1-carboxylate (2.990 g, 7.701 mmol) prepared in the step 2 and 1,1'-carbonyldiimidazole (1.249 g, 7.701 mmol) were dissolved in dichloromethane (100 mL) at room temperature, after which the resulting solution was heated under reflux for 18 hours, and then a reaction was finished by lowering a temperature to room temperature. Solvent was removed from the reaction mixture under reduced pressure, after which the resulting concentrate was purified via column chromatography (SiO₂, 40 g cartridge; ethyl acetate/hexane = 5 to 40%) and concentrated to obtain a product. Then, diethyl ether was inserted into the resulting product and stirred to filter out a precipitated solid, then washed with diethyl ether, and then dried to obtain a title compound (1.440 g, 45.1%) in a light brown solid form.

### [Step 4] Synthesis of tert-butyl (R)-3-(6-bromo-3-((5-(5-(difluoromethyl)-1,3,4-oxadiazole-2-yl)pyridine-2-yl)methyl)-5-fluoro-2-o xo-2,3-dihydro-1H-benzo[d]imidazole-1-yl)piperidine-1-carboxylate

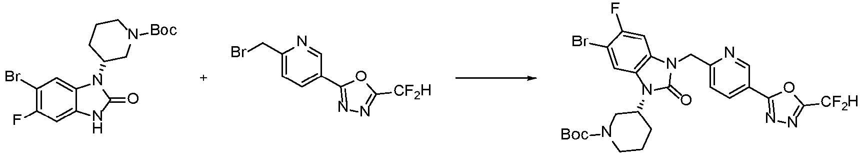

The tert-butyl (R)-3-(6-bromo-5-fluoro-2-oxo-2,3-dihydro-1H-benzo[d]imidazole-1-yl)piperidine-1-carboxylate (1.440 g, 3.476 mmol) prepared in the step 3, 2-(6-(bromomethyl)pyridine-3-yl)-5-(difluoromethyl)-1,3,4-oxadiazole (1.109 g, 3.824 mmol), potassium carbonate (0.721 g, 5.214 mmol) and potassium iodide (0.058 g, 0.348 mmol) were dissolved in N,N-dimethylformamide (20 mL) at room temperature, after which the resulting solution was stirred at 100°C for 18 hours, and then a reaction was finished by lowering a temperature to room temperature. Solvent was removed from the reaction mixture under reduced pressure, after which saturated sodium hydrogen carbonate aqueous solution was poured into the resulting concentrate, and then an extraction was performed with dichloromethane. An organic layer was washed with saturated sodium chloride aqueous solution, then dehydrated with anhydrous magnesium sulfate, then filtered, and then concentrated under reduced pressure. The resulting concentrate was purified via column chromatography (SiO₂, 40 g cartridge; ethyl acetate/hexane = 10 to 50%) and concentrated to obtain a title compound (1.680 g, 77.5%) in a light yellow solid form.

### [Step 5] Synthesis of (R)-5-bromo-1-((5-(5-(difluoromethyl)-1,3,4-oxadiazole-2-yl)pyridine-2-yl)methyl)-6-fluoro-3-(pipe ridine-3-yl)-1,3-dihydro-2H-benzo[d]imidazole-2-one

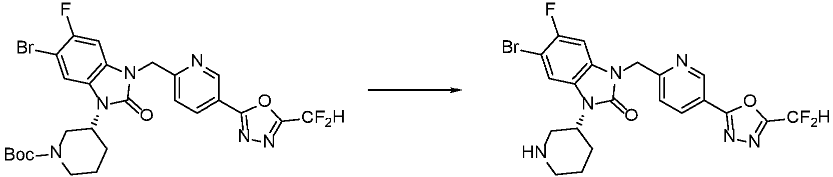

The tert-butyl (R)-3-(6-bromo-3-((5-(5-(difluoromethyl)-1,3,4-oxadiazole-2-yl)pyridine-2-yl)methyl)-5-fluoro-2-o xo-2,3-dihydro-1H-benzo[d]imidazole-1-yl)piperidine-1-carboxylate (1.680 g, 2.695 mmol) prepared in the step 4 and trifluoroacetic acid (1.238 mL, 16.169 mmol) were dissolved in dichloromethane (15 mL) at room temperature, after which the resulting solution was stirred at the same temperature for 5 hours. Saturated sodium hydrogen carbonate aqueous solution was poured into the reaction mixture and an extraction was performed with dichloromethane. An organic layer was washed with saturated sodium chloride aqueous solution, then dehydrated with anhydrous magnesium sulfate, then filtered, and then concentrated under reduced pressure. A title compound was used without an additional purification process (1.290 g, 91.5%, orange solid).

### [Step 6] Synthesis of (R)-5-bromo-1-((5-(5-(difluoromethyl)-1,3,4-oxadiazole-2-yl)pyridine-2-yl)methyl)-6-fluoro-3-(1-m ethylpiperidine-3-yl)-1,3-dihydro-2H-benzo[d]imidazole-2-one

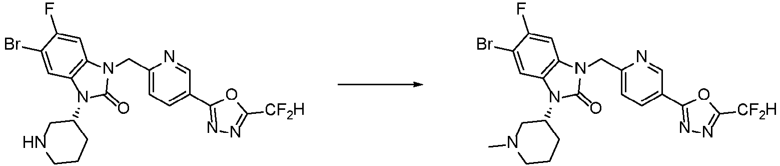

The (R)-5-bromo-1-((5-(5-(difluoromethyl)-1,3,4-oxadiazole-2-yl)pyridine-2-yl)methyl)-6-fluoro-3-(pipe ridine-3-yl)-1,3-dihydro-2H-benzo[d]imidazole-2-one (0.600 g, 1.147 mmol) prepared in the step 5 and sodium triacetoxyborohydride (0.486 g, 2.293 mmol) were dissolved in dichloromethane (10 mL) at room temperature, after which formaldehyde (38.00% solution, 0.126 mL, 1.720 mmol) was added into the resulting solution and stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 12 g cartridge; methanol/dichloromethane = 0 to 5%) and concentrated to obtain a title compound (0.360 g, 58.4%) in a light yellow solid form.

### [Step 7] Synthesis of the compound 274

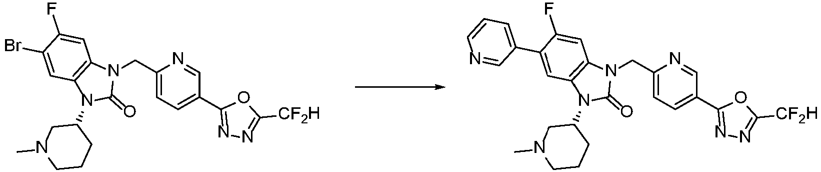

The (R)-5-bromo-1-((5-(5-(difluoromethyl)-1,3,4-oxadiazole-2-yl)pyridine-2-yl)methyl)-6-fluoro-3-(1-m ethylpiperidine-3-yl)-1,3-dihydro-2H-benzo[d]imidazole-2-one (0.100 g, 0.186 mmol) prepared in the step 6, pyridine-3-ylboronic acid (0.027 g, 0.223 mmol), [1,1'-bis(di-tert-butylphosphino)ferrocene]palladium(II) dichloride (Pd(dtbpf)Cl₂, 0.006 g, 0.009 mmol) and cesium carbonate (0.182 g, 0.558 mmol) were mixed in 1,4-dioxane (1.5 mL)/water (0.5 mL) at room temperature, after which the resulting mixture was irradiated with microwave, then heated at 100°C for 20 minutes, and then a reaction was finished by lowering a temperature to room temperature. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.054 g, 54.2%) in a brown solid form.

**¹H NMR** (400 MHz, CDCl₃) δ 9.31 (d, *J =* 1.7 Hz, 1H), 8.76 (s, 1H), 8.61 (dd, *J* = 4.8, 1.4 Hz, 1H), 8.38 (dd, *J* = 8.2, 2.2 Hz, 1H), 7.84 (dd, *J* = 7.9, 1.8 Hz, 1H), 7.49 (d, *J* = 8.2 Hz, 1H), 7.41 -7.37 (m, 1H), 7.18 (d, *J =* 6.2 Hz, 1H), 7.08 -6.82 (m, 2H), 5.27 (s, 2H), 4.50 -4.43 (m, 1H), 3.00 (dd, *J =* 10.5, 3.9 Hz, 1H), 2.89 (d, *J =* 11.2 Hz, 1H), 2.73 (t, *J =* 10.9 Hz, 1H), 2.37 (s, 3H), 2.30 - 2.26 (m, 1H), 2.08 - 2.05 (m, 1H), 1.99 - 1.96 (m, 1H), 1.92 - 1.88 (m, 1H), 1.83 - 1.80 (m, 1H); **LRMS** (ES) m/z 536.5 (M⁺ + 1).

### Example 275: Synthesis of Compound 275, (R)-1-((5-(5-(difluoromethyl)-1,3,4-oxadiazole-2-yl)pyridine-2-yl)methyl)-6-fluoro-3-(1-methylpipe ridine-3-yl)-5-(pyridine-4-yl)-1,3-dihydro-2H-benzo[d]imidazole-2-one

### [Step 1] Synthesis of the compound 275

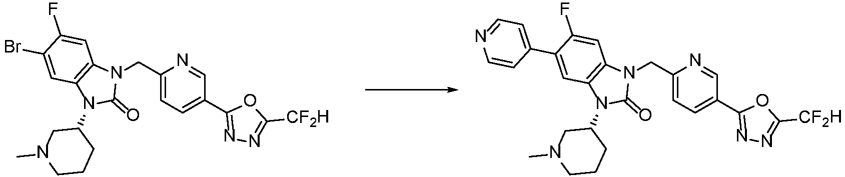

(R)-5-bromo-1-((5-(5-(difluoromethyl)-1,3,4-oxadiazole-2-yl)pyridine-2-yl)methyl)-6-fluor o-3-(1-methylpiperidine-3-yl)-1,3-dihydro-2H-benzo[d]imidazole-2-one (0.100 g, 0.186 mmol), pyridine-4-ylboronic acid (0.027 g, 0.223 mmol), [1,1'-bis(di-tert-butylphosphino)ferrocene]palladium(II) dichloride (Pd(dtbpf)Cl₂, 0.006 g, 0.009 mmol) and cesium carbonate (0.182 g, 0.558 mmol) were mixed in 1,4-dioxane (1.5 mL)/water (0.5 mL) at room temperature, after which the resulting mixture was irradiated with microwave, then heated at 100°C for 20 minutes, and then a reaction was finished by lowering a temperature to room temperature. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.054 g, 54.2%) in a brown solid form.

**¹H NMR** (400 MHz, CDCl₃) δ 9.30 (d, *J =* 1.8 Hz, 1H), 8.67 (d, *J =* 6.0 Hz, 2H), 8.38 (dd, *J* = 8.2, 2.2 Hz, 1H), 7.50 - 7.45 (m, 3H), 7.22 (d, *J* = 6.1 Hz, 1H), 7.08 - 6.82 (m, 2H), 5.26 (s, 2H), 4.49 - 4.43 (m, 1H), 3.72 - 3.70 (m, 1H), 3.04 (d, *J = 10.5* Hz, 1H), 2.79 (t, *J =* 10.9 Hz, 1H), 2.40 - 2.31 (m, 4H), 2.14 -2.08 (m, 1H), 1.85 - 1.79 (m, 2H), 1.28 - 1.27 (m, 1H); **LRMS** (ES) m/z 536.5 (M⁺ + 1).

### Example 276: Synthesis of Compound 276, (R)-1-((5-(5-(difluoromethyl)-1,3,4-oxadiazole-2-yl)pyridine-2-yl)methyl)-6-fluoro-5-(1H-indole-4-yl)-3-(1-methylpiperidine-3-yl)-1,3-dihydro-2H-benzo[d]imidazole-2-one

### [Step 1] Synthesis of the compound 276

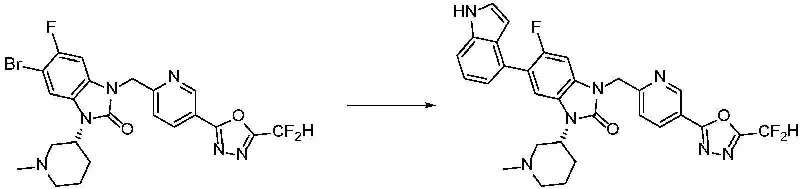

(R)-5-bromo-1-((5-(5-(difluoromethyl)-1,3,4-oxadiazole-2-yl)pyridine-2-yl)methyl)-6-fluor o-3-(1-methylpiperidine-3-yl)-1,3-dihydro-2H-benzo[d]imidazole-2-one (0.100 g, 0.186 mmol), (1H-indole-4-yl)boronic acid (0.036 g, 0.223 mmol), [1,1'-bis(di-tert-butylphosphino)ferrocene]palladium(II) dichloride (Pd(dtbpf)Cl₂, 0.006 g, 0.009 mmol) and cesium carbonate (0.182 g, 0.558 mmol) were mixed in 1,4-dioxane (1.5 mL)/water (0.5 mL) at room temperature, after which the resulting mixture was irradiated with microwave, then heated at 100°C for 20 minutes, and then a reaction was finished by lowering a temperature to room temperature. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.080 g, 74.9%) in a brown solid form.

**¹H NMR** (400 MHz, CDCl₃) δ 9.34 (d, *J* = 1.6 Hz, 1H), 8.51 (brs, 1H), 8.39 (dd, *J* = 8.2, 2.2 Hz, 1H), 7.51 (d, *J =* 8.2 Hz, 1H), 7.45 (d, *J =* 8.1 Hz, 1H), 7.36 (d, *J =* 5.9 Hz, 1H), 7.32 - 7.27 (m, 2H), 7.18 (d, *J =* 7.3 Hz, 1H), 7.08 - 6.82 (m, 2H), 6.49 - 6.48 (m, 1H), 5.32 - 5.26 (m, 2H), 4.55 -4.48 (m, 1H), 3.03 (dd, *J =* 10.5, 3.8 Hz, 1H), 2.88 (d, *J =* 11.2 Hz, 1H), 2.75 (t, *J =* 11.0 Hz, 1H), 2.36 (s, 3H), 2.29 - 2.20 (m, 1H), 2.07 - 1.97 (m, 1H), 1.89 - 1.81 (m, 3H); **LRMS** (ES) m/z 574.6 (M⁺ + 1).

### Example 277: Synthesis of Compound 277, 3-((5-(5-(difluoromethyl)-1,3,4-oxadiazole-2-yl)pyridine-2-yl)methyl)-5-fluoro-6-(pyridine-3-yl)ben zo[d]oxazole-2(3H)-one

### [Step 1] Synthesis of 6-bromo-3-((5-(5-(difluoromethyl)-1,3,4-oxadiazole-2-yl)pyridine-2-yl)methyl)-5-fluorobenzo[d]ox azole-2(3H)-one

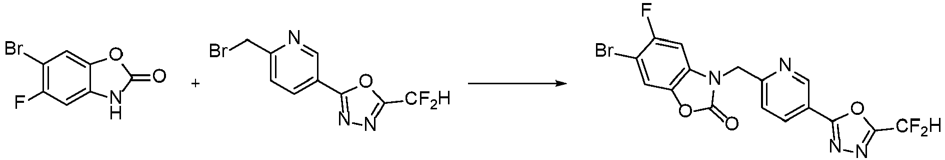

6-bromo-5-fluorobenzo[d]oxazole-2(3H)-one (3.000 g, 12.930 mmol), 2-(6-(bromomethyl)pyridine-3-yl)-5-(difluoromethyl)-1,3,4-oxadiazole (3.938 g, 13.577 mmol), potassium carbonate (2.681 g, 19.396 mmol) and potassium iodide (0.215 g, 1.293 mmol) were dissolved in N,N-dimethylformamide (150 mL) at room temperature, after which the resulting solution was stirred at the same temperature for 18 hours. Solvent was removed from the reaction mixture under reduced pressure, after which saturated sodium hydrogen carbonate aqueous solution was poured into the resulting concentrate, and then an extraction was performed with dichloromethane. An organic layer was washed with saturated sodium chloride aqueous solution, then dehydrated with anhydrous magnesium sulfate, then filtered, and then concentrated under reduced pressure. Ethyl acetate was put into the resulting concentrate and stirred, after which a precipitated solid was filtered, then washed with ethyl acetate, and then dried to obtain a title compound. The resulting filtrate was further concentrated under reduced pressure, after which methanol was put into the resulting concentrate and stirred to filter out a precipitated solid, then washed with methanol, and then dried to obtain a title compound (4.650 g, 81.5%) in an orange solid form.

### [Step 2] Synthesis of the compound 277

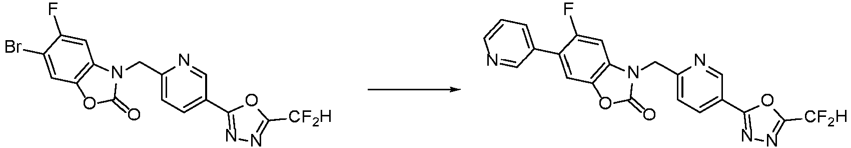

The 6-bromo-3-((5-(5-(difluoromethyl)-1,3,4-oxadiazole-2-yl)pyridine-2-yl)methyl)-5-fluorobenzo[d]ox azole-2(3H)-one (0.100 g, 0.227 mmol) prepared in the step 1, pyridine-3-ylboronic acid (0.033 g, 0.272 mmol), [1,1'-bis(di-tert-butylphosphino)ferrocene]palladium(II) dichloride (Pd(dtbpf)Cl₂, 0.007 g, 0.011 mmol) and cesium carbonate (0.222 g, 0.680 mmol) were mixed in 1,4-dioxane (1.5 mL)/water (0.5 mL) at room temperature, after which the resulting mixture was irradiated with microwave, then heated at 100°C for 20 minutes, and then a reaction was finished by lowering a temperature to room temperature. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; ethyl acetate/hexane = 20 to 60%) and concentrated to obtain a title compound (0.061 g, 61.3%) in an orange solid form.

**¹H NMR** (400 MHz, CDCl₃) δ 9.34 (d, *J =* 2.1 Hz, 1H), 8.76 (s, 1H), 8.64 (dd, *J =* 5.2, 1.2 Hz, 1H), 8.46 (dd, *J* = 8.2, 2.2 Hz, 1H), 7.85 - 7.82 (m, 1H), 7.62 (d, *J = 7.7* Hz, 1H), 7.42 - 7.39 (m, 1H), 7.32 (d, *J* = 6.0 Hz, 1H), 7.09 - 6.83 (m, 2H), 5.25 (s, 2H); **LRMS** (ES) m/z 440.4 (M⁺ + 1).

### Example 278: Synthesis of Compound 278, 3-((5-(5-(difluoromethyl)-1,3,4-oxadiazole-2-yl)pyridine-2-yl)methyl)-5-fluoro-6-(pyridine-4-yl)ben zo[d]oxazole-2(3H)-one

### [Step 1] Synthesis of the compound 278

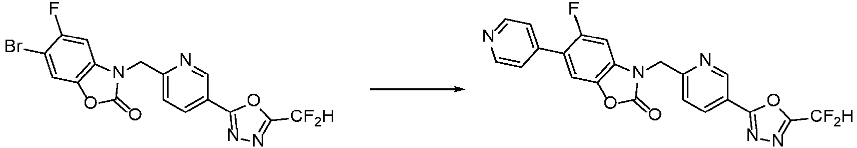

6-bromo-3-((5-(5-(difluoromethyl)-1,3,4-oxadiazole-2-yl)pyridine-2-yl)methyl)-5-fluorobe nzo[d]oxazole-2(3H)-one (0.100 g, 0.227 mmol), pyridine-4-ylboronic acid (0.033 g, 0.272 mmol), [1,1'-bis(di-tert-butylphosphino)ferrocene]palladium(II) dichloride (Pd(dtbpf)Cl₂, 0.007 g, 0.011 mmol) and cesium carbonate (0.222 g, 0.680 mmol) were mixed in 1,4-dioxane (1.5 mL)/water (0.5 mL) at room temperature, after which the resulting mixture was irradiated with microwave, then heated at 100°C for 20 minutes, and then a reaction was finished by lowering a temperature to room temperature. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; ethyl acetate/hexane = 10 to 50%) and concentrated to obtain a title compound (0.067 g, 67.3%) in a yellow solid form.

**¹H NMR** (400 MHz, CDCl₃) δ 9.33 (d, *J* = 1.6 Hz, 1H), 8.71 - 8.69 (m, 2H), 8.46 (dd, *J* = 8.2, 2.2 Hz, 1H), 7.62 (d, *J =* 8.2 Hz, 1H), 7.46 - 7.44 (m, 2H), 7.35 (d, *J =* 5.9 Hz, 1H), 7.09 - 6.83 (m, 2H), 5.25 (s, 2H); LRMS (ES) m/z 440.4 (M⁺ + 1).

### Example 279: Synthesis of Compound 279, 3-((5-(5-(difluoromethyl)-1,3,4-oxadiazole-2-yl)pyridine-2-yl)methyl)-5-fluoro-6-(1H-indole-4-yl)be nzo[d]oxazole-2(3H)-one

### [Step 1] Synthesis of the compound 279

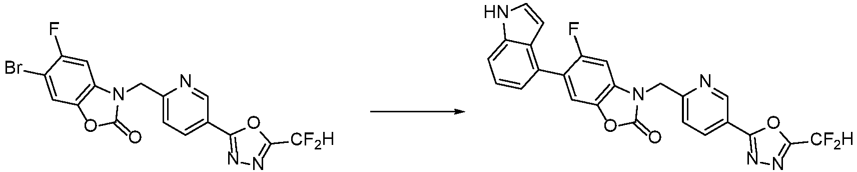

6-bromo-3-((5-(5-(difluoromethyl)-1,3,4-oxadiazole-2-yl)pyridine-2-yl)methyl)-5-fluorobe nzo[d]oxazole-2(3H)-one (0.100 g, 0.227 mmol), (1H-indole-4-yl)boronic acid (0.044 g, 0.272 mmol), [1,1'-bis(di-tert-butylphosphino)ferrocene]palladium(II) dichloride (Pd(dtbpf)Cl₂, 0.007 g, 0.011 mmol) and cesium carbonate (0.222 g, 0.680 mmol) were mixed in 1,4-dioxane (1.5 mL)/water (0.5 mL) at room temperature, after which the resulting mixture was irradiated with microwave, then heated at 100°C for 20 minutes, and then a reaction was finished by lowering a temperature to room temperature. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; ethyl acetate/hexane = 10 to 50%) and concentrated to obtain a product. Then, diethyl ether and hexane were inserted into the resulting product and stirred to filter out a precipitated solid, then washed with hexane, and then dried to obtain a title compound (0.021 g, 19.4%) in a yellow solid form.

**¹H NMR** (400 MHz, CDCl₃) δ 9.36 (d, *J =* 2.1 Hz, 1H), 8.46 (dd, *J* = 8.2, 2.2 Hz, 1H), 8.32 brs, 1H), 7.62 (d, *J* = 8.2 Hz, 1H), 7.48 - 7.44 (m, 2H), 7.31 - 7.27 (m, 2H), 7.16 (d, *J* = 6.3 Hz, 1H), 7.09 - 6.83 (m, 2H), 6.48 - 6.47 (m, 1H), 5.27 (s, 2H); LRMS (ES) m/z 478.4 (M⁺ + 1).

### Example 280: Synthesis of Compound 280, 1-((5-(5-(difluoromethyl)-1,3,4-oxadiazole-2-yl)pyridine-2-yl)methyl)-3-(1-(oxetan-3-yl)piperidine-4-yl)-5-(pyridine-3-yl)-1,3-dihydro-2H-benzo[d]imidazole-2-one

### [Step 1] Synthesis of the compound 280

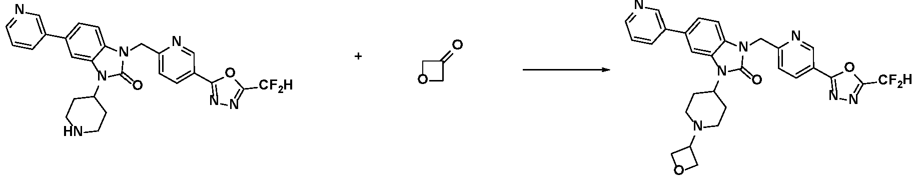

1-((5-(5-(difluoromethyl)-1,3,4-oxadiazole-2-yl)pyridine-2-yl)methyl)-3-(piperidine-4-yl)-5 -(pyridine-3-yl)-1,3-dihydro-2H-benzo[d]imidazole-2-one (0.162 g, 0.322 mmol), oxetan-3-one (0.046 g, 0.643 mmol) and sodium triacetoxyborohydride (0.136 g, 0.643 mmol) were dissolved in dichloromethane (10 mL) at room temperature, after which the resulting solution was stirred at the same temperature for 18 hours. Water was poured into the reaction mixture and an extraction was performed with dichloromethane. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; methanol/dichloromethane = 0 to 10%) and concentrated to obtain a title compound (0.100 g, 55.5%) in a white foam solid form.

**¹H NMR** (400 MHz, CDCl₃) δ 9.31 - 9.30 (m, 1H), 8.84 (d, *J =* 1.9 Hz, 1H), 8.60 (dd, *J* = 4.8, 1.4 Hz, 1H), 8.35 (dd, *J* = 8.2, 2.2 Hz, 1H), 7.91 ~ 7.87 (m, 1H), 7.53 (d, *J =* 1.4 Hz, 1H), 7.47 ~7.44 (m, 1H), 7.41 ~7.38 (m, 1H), 7.25 (dd, *J* = 8.1, 1.5 Hz, 1H), 7.11 (d, *J =* 8.2 Hz, 1H), 7.07 (s, 0.25H), 6.95 (s, 0.5H), 6.82 (s, 0.25H), 5.33 (s, 2H), 4.72 ~ 4.66 (m, 4H), 4.53 ~ 4.47 (m, 1H), 3.59 ~ 3.56 (m, 1H), 2.98 ~ 2.95 (m, 2H), 2.61 ~ 2.52 (m, 2H), 2.17 ~ 2.06 (m, 2H), 1.95 ~ 1.92 (m, 2H).

### Example 281: Synthesis of Compound 281, 1-((5-(5-(difluoromethyl)-1,3,4-oxadiazole-2-yl)pyridine-2-yl)methyl)-3-(1-ethylpiperidine-4-yl)-5-( pyridine-3-yl)-1,3-dihydro-2H-benzo[d]imidazole-2-one

### [Step 1] Synthesis of the compound 281

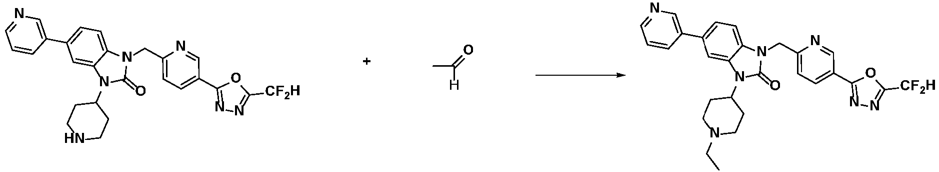

1-((5-(5-(difluoromethyl)-1,3,4-oxadiazole-2-yl)pyridine-2-yl)methyl)-3-(piperidine-4-yl)-5 -(pyridine-3-yl)-1,3-dihydro-2H-benzo[d]imidazole-2-one (0.143 g, 0.284 mmol), acetaldehyde (0.025 g, 0.568 mmol) and sodium triacetoxyborohydride (0.120 g, 0.568 mmol) were dissolved in dichloromethane (10 mL) at room temperature, after which the resulting solution was stirred at the same temperature for 18 hours. Water was poured into the reaction mixture and an extraction was performed with dichloromethane. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; methanol/dichloromethane = 0 to 10%) and concentrated to obtain a title compound (0.080 g, 53.0%) in a white foam solid form.

**¹H NMR** (400 MHz, CDCl₃) δ 9.30 (d, *J =* 1.6 Hz, 1H), 8.82 (d, *J =* 1.3 Hz, 1H), 8.57 (d, *J* = 3.9 Hz, 1H), 8.35 (dd, *J* = 8.2, 2.2 Hz, 1H), 7.92 (dt, *J* = 8.0, 1.9 Hz, 1H), 7.61 (d, *J* = 1.2 Hz, 1H), 7.45 (d, *J =* 8.2 Hz, 1H), 7.37 (dd, *J* = 7.8, 4.8 Hz, 1H), 7.26 (dd, *J* = 8.2, 1.4 Hz, 1H), 7.09 (d, *J =* 8.2 Hz, 1H), 7.08 (s, 0.25H), 6.95 (s, 0.5H), 6.81 (s, 0.25H), 5.33 (s, 2H), 4.63 ~ 4.54 (m, 1H), 3.33 ~ 3.31 (m, 2H), 2.76 ~ 2.64 (m, 4H), 2.37 ~ 2.32 (m, 2H), 1.97 ~ 1.94 (m, 2H), 1.25 ~ 1.19 (m, 3H).

### Example 282: Synthesis of Compound 282, 1-((5-(5-(difluoromethyl)-1,3,4-oxadiazole-2-yl)pyridine-2-yl)methyl)-3-(1-(oxetan-3-yl)piperidine-4-yl)-5-(pyridine-4-yl)-1,3-dihydro-2H-benzo[d]imidazole-2-one

### [Step 1] Synthesis of the compound 282

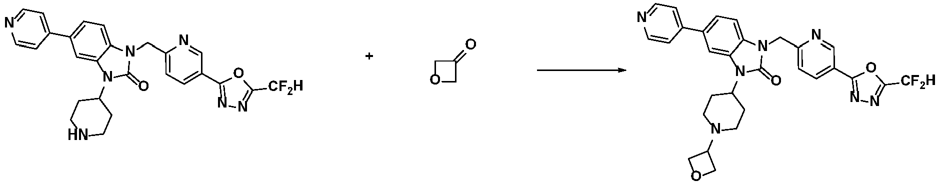

1-((5-(5-(difluoromethyl)-1,3,4-oxadiazole-2-yl)pyridine-2-yl)methyl)-3-(piperidine-4-yl)-5 -(pyridine-4-yl)-1,3-dihydro-2H-benzo[d]imidazole-2-one (0.100 g, 0.199 mmol), oxetan-3-one (0.029 g, 0.397 mmol) and sodium triacetoxyborohydride (0.084 g, 0.397 mmol) were dissolved in dichloromethane (10 mL) at room temperature, after which the resulting solution was stirred at the same temperature for 18 hours. Water was poured into the reaction mixture and an extraction was performed with dichloromethane. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; methanol/dichloromethane = 0 to 10%) and concentrated to obtain a title compound (0.065 g, 58.5%) in a white foam solid form.

**¹H NMR** (400 MHz, CDCl₃) δ 9.30 (dd, *J* = 2.2, 0.7 Hz, 1H), 8.66 (dd, *J* = 4.6, 1.5 Hz, 2H), 8.35 (dd, *J* = 8.2, 2.2 Hz, 1H), 7.58 (d, *J =* 1.4 Hz, 1H), 7.54 (dd, *J* = 4.6, 1.6 Hz, 2H), 7.46 (dd, *J* = 8.2, 0.5 Hz, 1H), 7.34 (dd, *J* = 8.2, 1.5 Hz, 1H), 7.12 (d, *J =* 8.2 Hz, 1H), 7.07 (s, 0.25H), 6.95 (s, 0.5H), 6.82 (s, 0.25H), 5.32 (s, 2H), 4.73 ~ 4.66 (m, 4H), 4.60 ~ 4.40 (m, 1H), 3.60 ~ 3.56 (m, 1H), 3.30 ~ 2.99 (m, 2H), 2.60 ~ 2.56 (m, 2H), 2.12 ~ 2.05 (m, 2H), 1.95 ~ 1.92 (m, 2H).

### Example 283: Synthesis of Compound 283, 1-((5-(5-(difluoromethyl)-1,3,4-oxadiazole-2-yl)pyridine-2-yl)methyl)-3-(1-ethylpiperidine-4-yl)-5-( pyridine-4-yl)-1,3-dihydro-2H-benzo[d]imidazole-2-one

### [Step 1] Synthesis of the compound 283

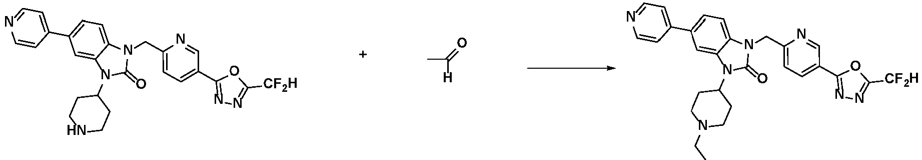

1-((5-(5-(difluoromethyl)-1,3,4-oxadiazole-2-yl)pyridine-2-yl)methyl)-3-(piperidine-4-yl)-5 -(pyridine-4-yl)-1,3-dihydro-2H-benzo[d]imidazole-2-one (0.100 g, 0.199 mmol), acetaldehyde (0.017 g, 0.397 mmol) and sodium triacetoxyborohydride (0.084 g, 0.397 mmol) were dissolved in dichloromethane (10 mL) at room temperature, after which the resulting solution was stirred at the same temperature for 18 hours. Water was poured into the reaction mixture and an extraction was performed with dichloromethane. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; methanol/dichloromethane = 0 to 10%) and concentrated to obtain a title compound (0.050 g, 47.4%) in a colorless oil form.

**¹H NMR** (400 MHz, CDCl₃) δ 9.31 (dd, *J =* 2.2, 0.7 Hz, 1H), 8.65 (dd, *J* = 4.6, 1.6 Hz, 2H), 8.36 (dd, *J* = 8.2, 2.2 Hz, 1H), 7.70 (d, *J =* 1.2 Hz, 1H), 7.56 (dd, *J* = 4.6, 1.6 Hz, 2H), 7.47 (dd, *J* = 8.3, 0.6 Hz, 1H), 7.35 (dd, *J* = 8.2, 1.6 Hz, 1H), 7.12 (d, *J =* 8.2 Hz, 1H), 7.08 (s, 0.25H), 6.95 (s, 0.5H), 6.82 (s, 0.25H), 5.34 (s, 2H), 4.65 ~ 4.60 (m, 1H), 3.37 ~ 3.34 (m, 2H), 2.76 ~ 2.66 (m, 4H), 2.39 ~ 2.33(m, 2H), 1.99 ~ 1.94 (m, 2H), 1.22 (t, *J =* 7.2 Hz, 3H).

### Example 284: Synthesis of Compound 284, 5-(4-((1H-indole-4-yl)methyl)piperazine-1-yl)-1-((5-(5-(difluoromethyl)-1,3,4-oxadiazole-2-yl)pyrid ine-2-yl)methyl)-6-fluoro-3-methyl-1,3-dihydro-2H-benzo[d]imidazole-2-one

### [Step 1] Synthesis of the compound 284

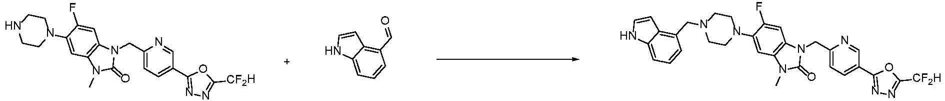

The 1-((5-(5-(difluoromethyl)-1,3,4-oxadiazole-2-yl)pyridine-2-yl)methyl)-6-fluoro-3-methyl-5-(piperazi ne-1-yl)-1,3-dihydro-2H-benzo[d]imidazole-2-one (0.100 g, 0.218 mmol) prepared in the step 6 of the compound 260, 1H-indole-4-carbaldehyde (0.063 g, 0.435 mmol) and sodium triacetoxyborohydride (0.092 g, 0.435 mmol) were dissolved in dichloromethane (2 mL) at room temperature, after which the resulting solution was stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.099 g, 77.0%) in a yellow solid form.

**¹H NMR** (400 MHz, CDCl₃) δ 9.24 (d, *J =* 2.0 Hz, 1H), 8.71 (s, 1H), 8.26 (dd, *J =* 8.2, 2.2 Hz, 1H), 7.36 (d, *J* = 8.2 Hz, 1H), 7.27 (d, *J* = 7.6 Hz, 1H), 7.17 (t, *J =* 2.8 Hz, 1H), 7.14 - 7.07 (m, 2H), 6.92 (t, *J =* 51.6 Hz, 1H), 6.74 - 6.72 (m, 2H), 6.64 (d, *J =* 7.0 Hz, 1H), 5.20 (s, 2H), 3.86 (s, 2H), 3.42 (s, 3H), 3.06 - 3.05 (m, 4H), 2.80 - 2.65 (m, 4H); **LRMS** (ES) m/z 589.2 (M⁺+ 1).

### Example 285: Synthesis of Compound 285, 5-(4-((1H-pyrazole-4-yl)methyl)piperazine-1-yl)-1-((5-(5-(difluoromethyl)-1,3,4-oxadiazole-2-yl)py ridine-2-yl)methyl)-6-fluoro-3-methyl-1,3-dihydro-2H-benzo[d]imidazole-2-one

### [Step 1] Synthesis of the compound 285

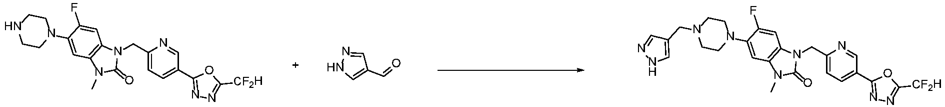

The 1-((5-(5-(difluoromethyl)-1,3,4-oxadiazole-2-yl)pyridine-2-yl)methyl)-6-fluoro-3-methyl-5-(piperazi ne-1-yl)-1,3-dihydro-2H-benzo[d]imidazole-2-one (0.100 g, 0.218 mmol) prepared in the step 6 of the compound 260, 1H-pyrazole-4-carbaldehyde (0.042 g, 0.435 mmol) and sodium triacetoxyborohydride (0.092 g, 0.435 mmol) were dissolved in dichloromethane (2 mL) at room temperature, after which the resulting solution was stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.079 g, 67.1%) in a yellow solid form.

**¹H NMR** (400 MHz, CDCl₃) δ 9.24 (dd, *J* = 2.2, 0.7 Hz, 1H), 8.29 (dd, *J* = 8.2, 2.2 Hz, 1H), 7.52 (s, 2H), 7.39 (dd, *J* = 8.2, 0.5 Hz, 1H), 6.92 (t, *J =* 51.6 Hz, 1H), 6.72 (d, *J* = 11.2 Hz, 1H), 6.62 (d, *J =* 7.0 Hz, 1H), 5.20 (s, 2H), 3.53 (s, 2H), 3.41 (s, 3H), 3.10 - 2.95 (m, 4H), 2.70 - 2.55 (m, 4H); **LRMS** (ES) m/z 540.2 (M⁺ + 1).

### Example 286: Synthesis of Compound 286, (R)-1-((5-(5-(difluoromethyl)-1,3,4-oxadiazole-2-yl)pyridine-2-yl)methyl)-6-fluoro-3-(1-(oxetan-3-y I)pyrrolidine-3-yl)-5-(pyridine-3-yl)-1,3-dihydro-2H-benzo[d]imidazole-2-one

### [Step 1] Synthesis of (R)-5-bromo-1-((5-(5-(difluoromethyl)-1,3,4-oxadiazole-2-yl)pyridine-2-yl)methyl)-6-fluoro-3-(1-(o xetan-3-yl)pyrrolidine-3-yl)-1,3-dihydro-2H-benzo[d]imidazole-2-one

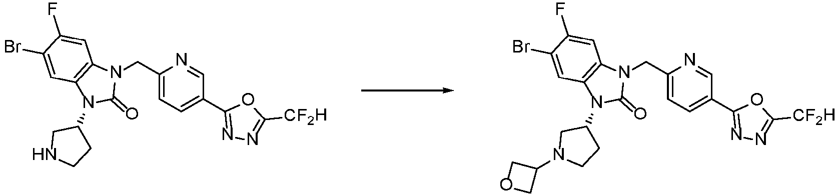

(R)-5-bromo-1-((5-(5-(difluoromethyl)-1,3,4-oxadiazole-2-yl)pyridine-2-yl)methyl)-6-fluor o-3-(pyrrolidine-3-yl)-1,3-dihydro-2H-benzo[d]imidazole-2-one (0.500 g, 0.982 mmol) and oxetan-3-one (0.094 mL, 1.473 mmol) were dissolved in dichloromethane (10 mL) at room temperature, after which sodium triacetoxyborohydride (0.416 g, 1.964 mmol) was added into the resulting solution and stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 12 g cartridge; methanol/dichloromethane = 0 to 3%) and concentrated to obtain a title compound (0.300 g, 54.1%) in a white solid form.

### [Step 2] Synthesis of the compound 286

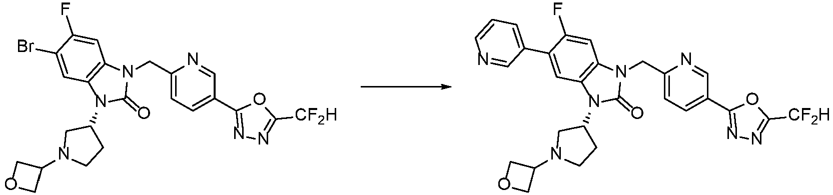

The (R)-5-bromo-1-((5-(5-(difluoromethyl)-1,3,4-oxadiazole-2-yl)pyridine-2-yl)methyl)-6-fluoro-3-(1-(o xetan-3-yl)pyrrolidine-3-yl)-1,3-dihydro-2H-benzo[d]imidazole-2-one (0.100 g, 0.177 mmol) prepared in the step 1, pyridine-3-ylboronic acid (0.026 g, 0.212 mmol), [1,1'-bis(di-tert-butylphosphino)ferrocene]palladium(II) dichloride (Pd(dtbpf)Cl₂, 0.006 g, 0.009 mmol) and cesium carbonate (0.173 g, 0.531 mmol) were mixed in 1,4-dioxane (1.5 mL)/water (0.5 mL) at room temperature, after which the resulting mixture was irradiated with microwave, then heated at 100°C for 20 minutes, and then a reaction was finished by lowering a temperature to room temperature. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.024 g, 24.1%) in a light brown solid form.

**¹H NMR** (400 MHz, CDCl₃) δ 9.31 (d, *J* = 1.8 Hz, 1H), 8.84 (s, 1H), 8.59 (d, *J* = 4.0 Hz, 1H), 8.39 (dd, *J =* 8.2, 2.2 Hz, 1H), 8.15 (d, *J* = 6.6 Hz, 1H), 7.93 (dd, *J =* 8.0, 1.7 Hz, 1H), 7.51 (d, *J* = 8.2 Hz, 1H), 7.41 - 7.38 (m, 1H), 7.08 - 6.82 (m, 2H), 5.33 - 5.24 (m, 3H), 4.79 - 4.73 (m, 2H), 4.68 (t, *J =* 5.9 Hz, 1H), 4.61 (t, *J =* 6.0 Hz, 1H), 3.70 - 3.66 (m, 1H), 3.17 - 3.12 (m, 2H), 2.64 (t, *J* = 9.6 Hz, 1H), 2.66 - 2.64 (m, 1H), 2.32 - 2.20 (m, 2H); **LRMS** (ES) m/z 564.5 (M⁺ + 1).

### Example 287: Synthesis of Compound 287, (R)-1-((5-(5-(difluoromethyl)-1,3,4-oxadiazole-2-yl)pyridine-2-yl)methyl)-6-fluoro-3-(1-(oxetan-3-y I)pyrrolidine-3-yl)-5-(pyridine-4-yl)-1,3-dihydro-2H-benzo[d]imidazole-2-one

### [Step 1] Synthesis of the compound 287

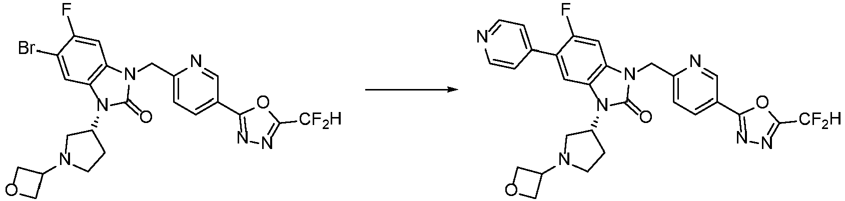

(R)-5-bromo-1-((5-(5-(difluoromethyl)-1,3,4-oxadiazole-2-yl)pyridine-2-yl)methyl)-6-fluor o-3-(1-(oxetan-3-yl)pyrrolidine-3-yl)-1,3-dihydro-2H-benzo[d]imidazole-2-one (0.100 g, 0.177 mmol), pyridine-4-ylboronic acid (0.026 g, 0.212 mmol), [1,1'-bis(di-tert-butylphosphino)ferrocene]palladium(II) dichloride (Pd(dtbpf)Cl₂, 0.006 g, 0.009 mmol) and cesium carbonate (0.173 g, 0.531 mmol) were mixed in 1,4-dioxane (1.5 mL)/water (0.5 mL) at room temperature, after which the resulting mixture was irradiated with microwave, then heated at 100°C for 20 minutes, and then a reaction was finished by lowering a temperature to room temperature. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 12 g cartridge; methanol/dichloromethane = 0 to 5%) and concentrated to obtain a title compound (0.049 g, 49.2%) in a brown solid form.

**¹H NMR** (400 MHz, CDCl₃) δ 9.32 (d, *J =* 1.6 Hz, 1H), 8.68 (d, *J =* 5.6 Hz, 2H), 8.39 (dd, *J* = 8.2, 2.2 Hz, 1H), 8.33 (d, *J = 6.6* Hz, 1H), 7.57 (d, *J = 4.7* Hz, 2H), 7.51 (d, *J* = 8.2 Hz, 2H), 7.08 - 6.82 (m, 2H), 5.33 - 5.24 (m, 3H), 4.83 - 4.76 (m, 2H), 4.70 (t, *J = 5.9* Hz, 1H), 4.63 (t, *J =* 5.9 Hz, 1H), 3.68 - 3.65 (m, 1H), 3.19 - 3.15 (m, 2H), 2.60 (t, *J =* 9.6 Hz, 1H), 2.43 -2.40 (m, 1H), 2.30 -2.20 (m, 2H); **LRMS** (ES) m/z 564.5 (M⁺ + 1).

### Example 288: Synthesis of Compound 288, (R)-1-((5-(5-(difluoromethyl)-1,3,4-oxadiazole-2-yl)pyridine-2-yl)methyl)-6-fluoro-5-(1H-indole-4-yl)-3-(1-(oxetan-3-yl)pyrrolidine-3-yl)-1,3-dihydro-2H-benzo[d]imidazole-2-one

### [Step 1] Synthesis of the compound 288

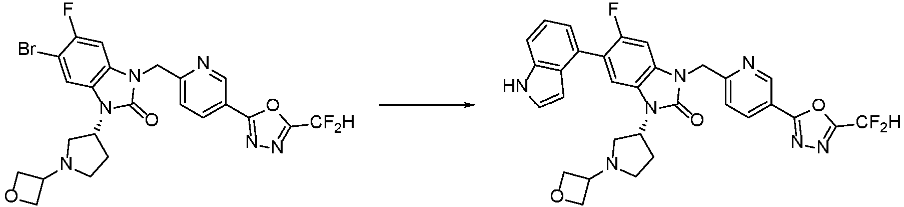

(R)-5-bromo-1-((5-(5-(difluoromethyl)-1,3,4-oxadiazole-2-yl)pyridine-2-yl)methyl)-6-fluor o-3-(1-(oxetan-3-yl)pyrrolidine-3-yl)-1,3-dihydro-2H-benzo[d]imidazole-2-one (0.100 g, 0.177 mmol), (1H-indole-4-yl)boronic acid (0.006 g, 0.035 mmol), [1,1'-bis(di-tert-butylphosphino)ferrocene]palladium(II) dichloride (Pd(dtbpf)Cl₂, 0.006 g, 0.009 mmol) and cesium carbonate (0.173 g, 0.531 mmol) were mixed in 1,4-dioxane (1.5 mL)/water (0.5 mL) at room temperature, after which the resulting mixture was irradiated with microwave, then heated at 100°C for 20 minutes, and then a reaction was finished by lowering a temperature to room temperature. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 12 g cartridge; methanol/dichloromethane = 0 to 5%) and concentrated to obtain a title compound (0.032 g, 30.1%) in a brown solid form.

**¹H NMR** (400 MHz, CDCl₃) δ 9.35 (d, *J* = 1.7 Hz, 1H), 8.41 - 8.39 (m, 2H), 8.03 (d, *J* = 6.2 Hz, 1H), 7.53 (d, *J = 8.2* Hz, 1H), 7.45 (d, *J =* 8.0 Hz, 1H), 7.33 - 7.24 (m, 4H), 7.08 - 6.83 (m, 2H), 6.58 (s, 1H), 5.32 - 5.27 (m, 3H), 4.68 - 4.62 (m, 4H), 3.74 - 3.71 (m, 1H), 3.13 (dd, *J =* 10.0, 3.9 Hz, 1H), 3.02 (t, *J =* 6.6 Hz, 1H), 2.74 (t, *J =* 9.5 Hz, 1H), 2.39 - 2.26 (m, 3H); **LRMS** (ES) m/z 602.5 (M⁺ + 1).

### Example 289: Synthesis of Compound 289, 1-((5-(5-(difluoromethyl)-1,3,4-oxadiazole-2-yl)pyridine-2-yl)methyl)-6-fluoro-5-(1H-indole-4-yl)-3 -((1-(oxetan-3-yl)piperidine-4-yl)methyl)-1,3-dihydro-2H-benzo[d]imidazole-2-one

### [Step 1] Synthesis of tert-butyl 4-(((5-bromo-4-fluoro-2-nitrophenyl)amino)methyl)piperidine-1-carboxylate

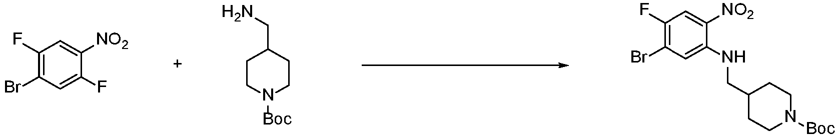

1-bromo-2,5-difluoro-4-nitrobenzene (5.000 g, 21.009 mmol), tert-butyl 4-(aminomethyl)piperidine-1-carboxylate (5.853 g, 27.312 mmol), potassium carbonate (5.807 g, 42.019 mmol) and potassium iodide (0.349 g, 2.101 mmol) were dissolved in N,N-dimethylformamide (100 mL) at room temperature, after which the resulting solution was stirred at 80°C for 5 hours, and then a reaction was finished by lowering a temperature to room temperature. Water was poured into the reaction mixture and an extraction was performed with ethyl acetate. An organic layer was washed with saturated sodium chloride aqueous solution, then dehydrated with anhydrous magnesium sulfate, then filtered, and then concentrated under reduced pressure. The resulting concentrate was purified via column chromatography (SiO₂, 80 g cartridge; ethyl acetate/hexane = 0 to 30%) and concentrated to obtain a title compound (7.910 g, 87.1%) in an orange solid form.

### [Step 2] Synthesis of tert-butyl 4-(((2-amino-5-bromo-4-fluorophenyl)amino)methyl)piperidine-1-carboxylate

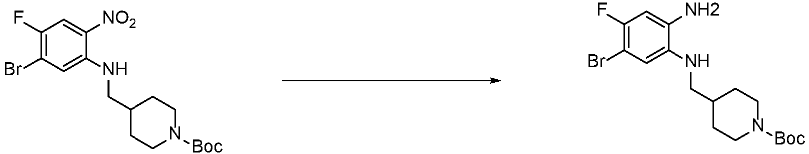

The tert-butyl 4-(((5-bromo-4-fluoro-2-nitrophenyl)amino)methyl)piperidine-1-carboxylate (7.910 g, 18.298 mmol) prepared in the step 1 was dissolved in methanol (100 mL) and stirred at room temperature, after which Raney nickel (800 mg) was slowly added into the resulting solution at the same temperature and stirred at the same temperature for 18 hours in the presence of a hydrogen balloon attached thereto. The reaction mixture was filtered via a celite pad to remove a solid therefrom, after which solvent was removed from the resulting filtrate under reduced pressure, and then a title compound was used without an additional purification process (7.340 g, 99.7%, brown solid).

### [Step 3] Synthesis of tert-butyl 4-((6-bromo-5-fluoro-2-oxo-2,3-dihydro-1H-benzo[d]imidazole-1-yl)methyl)piperidine-1-carboxyl ate F

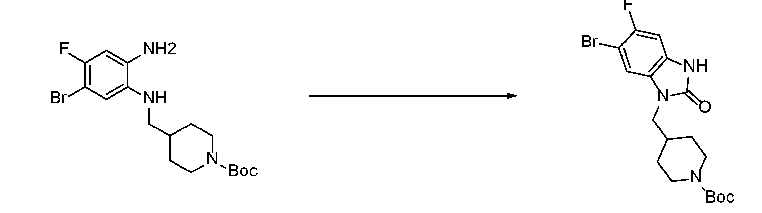

The tert-butyl 4-(((2-amino-5-bromo-4-fluorophenyl)amino)methyl)piperidine-1-carboxylate (7.340 g, 18.245 mmol) prepared in the step 2, triethylamine (2.543 mL, 18.245 mmol) and 1,1'-carbonyldiimidazole (CDI, 3.550 g, 21.894 mmol) were dissolved in dichloromethane (100 mL) at room temperature, after which the resulting solution was heated under reflux for 18 hours, and then a reaction was finished by lowering a temperature to room temperature. Solvent was removed from the reaction mixture under reduced pressure, after which the resulting concentrate was purified via column chromatography (SiO₂, 80 g cartridge; ethyl acetate/hexane = 20 to 80%) and concentrated to obtain a title compound (6.670 g, 85.4%) in a violet solid form.

### [Step 4] Synthesis of tert-butyl 4-((6-bromo-3-((5-(5-(difluoromethyl)-1,3,4-oxadiazole-2-yl)pyridine-2-yl)methyl)-5-fluoro-2-oxo-2,3-dihydro-1H-benzo[d]imidazole-1-yl)methyl)piperidine-1-carboxylate

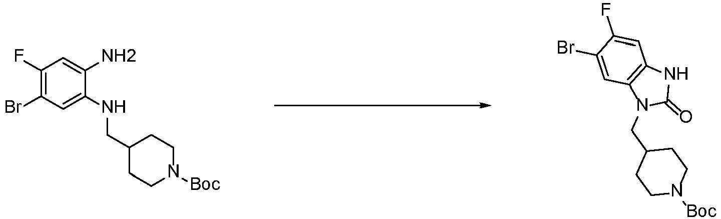

The tert-butyl 4-((6-bromo-5-fluoro-2-oxo-2,3-dihydro-1H-benzo[d]imidazole-1-yl)methyl)piperidine-1-carboxyl ate (4.270 g, 9.970 mmol) prepared in the step 3, sodium hydride (60.00%, 0.598 g, 14.954 mmol) and 2-(6-(bromomethyl)pyridine-3-yl)-5-(difluoromethyl)-1,3,4-oxadiazole (3.181 g, 10.967 mmol) were dissolved in N,N-dimethylformamide (50 mL), after which the resulting solution was stirred at 0°C for 30 minutes and further stirred at room temperature for 4 hours. Water was poured into the reaction mixture and an extraction was performed with ethyl acetate. An organic layer was washed with saturated sodium chloride aqueous solution, then dehydrated with anhydrous magnesium sulfate, then filtered, and then concentrated under reduced pressure. The resulting concentrate was purified via column chromatography (SiO₂, 80 g cartridge; ethyl acetate/hexane = 25 to 60%) and concentrated to obtain a title compound (4.100 g, 64.5%) in a yellow solid form.

### [Step 5] Synthesis of 5-bromo-1-((5-(5-(difluoromethyl)-1,3,4-oxadiazole-2-yl)pyridine-2-yl)methyl)-6-fluoro-3-(piperidi ne-4-ylmethyl)-1,3-dihydro-2H-benzo[d]imidazole-2-one

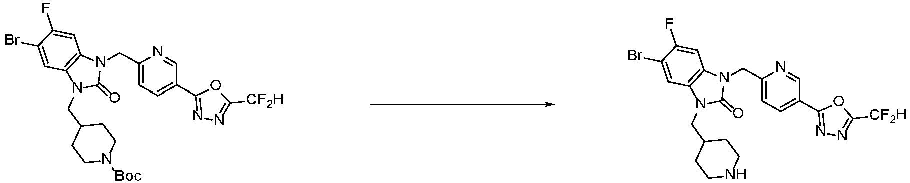

The tert-butyl 4-((6-bromo-3-((5-(5-(difluoromethyl)-1,3,4-oxadiazole-2-yl)pyridine-2-yl)methyl)-5-fluoro-2-oxo-2,3-dihydro-1H-benzo[d]imidazole-1-yl)methyl)piperidine-1-carboxylate (4.100 g, 6.432 mmol) prepared in the step 4 and trifluoroacetic acid (4.925 mL, 64.318 mmol) were dissolved in dichloromethane (30 mL) at room temperature, after which the resulting solution was stirred at the same temperature for 5 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. A title compound was used without an additional purification process (3.640 g, 105.3%, brown solid).

### [Step 6] Synthesis of 5-bromo-1-((5-(5-(difluoromethyl)-1,3,4-oxadiazole-2-yl)pyridine-2-yl)methyl)-6-fluoro-3-((1-(oxet an-3-yl)piperidine-4-yl)methyl)-1,3-dihydro-2H-benzo[d]imidazole-2-one

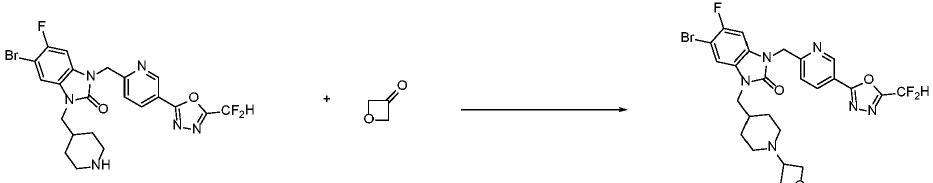

The 5-bromo-1-((5-(5-(difluoromethyl)-1,3,4-oxadiazole-2-yl)pyridine-2-yl)methyl)-6-fluoro-3-(piperidi ne-4-ylmethyl)-1,3-dihydro-2H-benzo[d]imidazole-2-one (3.450 g, 6.421 mmol) prepared in the step 5, sodium triacetoxyborohydride (2.722 g, 12.841 mmol) and oxetan-3-one (1.388 g, 19.262 mmol) were dissolved in dichloromethane (30 mL) at room temperature, after which the resulting solution was stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 40 g cartridge; methanol/dichloromethane = 0 to 5%) and concentrated to obtain a title compound (3.210 g, 84.3%) in a brown solid form.

### [Step 7] Synthesis of the compound 289

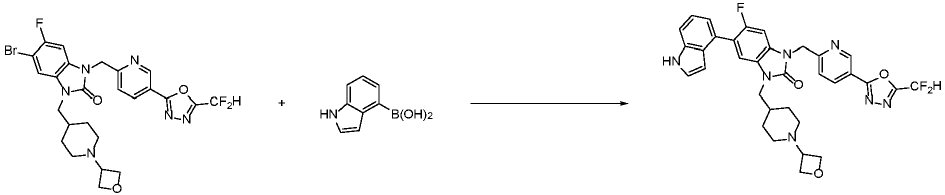

The 5-bromo-1-((5-(5-(difluoromethyl)-1,3,4-oxadiazole-2-yl)pyridine-2-yl)methyl)-6-fluoro-3-((1-(oxet an-3-yl)piperidine-4-yl)methyl)-1,3-dihydro-2H-benzo[d]imidazole-2-one (0.150 g, 0.253 mmol) prepared in the step 6, (1H-indole-4-yl)boronic acid (0.049 g, 0.303 mmol), cesium carbonate (0.165 g, 0.506 mmol) and [1,1'-bis(di-tert-butylphosphino)ferrocene]palladium dichloride (0.008 g, 0.013 mmol) were mixed in 1,4-dioxane (2.1 mL)/water (0.7 mL) at room temperature, after which the resulting mixture was irradiated with microwave, then heated at 100°C for 20 minutes, and then a reaction was finished by lowering a temperature to room temperature. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.081 g, 50.9%) in a brown solid form.

**¹H NMR** (400 MHz, CDCl₃) δ 9.27 (d, *J =* 1.9 Hz, 1H), 8.87 (s, 1H), 8.32 (dd, *J =* 8.2, 2.2 Hz, 1H), 7.44 (d, *J = 8.2* Hz, 1H), 7.38 (d, *J* = 8.1 Hz, 1H), 7.26 - 7.20 (m, 2H), 7.14 - 7.10 (m, 2H), 6.91 (t, *J = 51.7* Hz, 1H), 6.88 (d, *J = 9.4* Hz, 1H), 6.41 (d, *J =* 1.9 Hz, 1H), 5.29 (s, 2H), 4.59 (td, *J* = 12.1, 5.9 Hz, 4H), 3.82 (d, *J = 7.0* Hz, 2H), 3.44 - 3.37 (m, 1H), 2.72 (d, *J =* 11.4 Hz, 2H), 1.94 - 1.90 (m, 1H), 1.78 - 1.70 (m, 4H), 1.51 - 1.41 (m, 2H); **LRMS** (ES) m/z 631.5 (M⁺+ 1).

### Example 290: Synthesis of Compound 290, 1-((5-(5-(difluoromethyl)-1,3,4-oxadiazole-2-yl)pyridine-2-yl)methyl)-6-fluoro-3-((1-(oxetan-3-yl)pi peridine-4-yl)methyl)-5-(pyridine-3-yl)-1,3-dihydro-2H-benzo[d]imidazole-2-one

### [Step 1] Synthesis of the compound 290

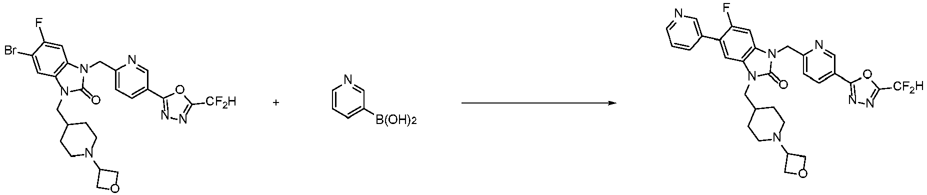

The 5-bromo-1-((5-(5-(difluoromethyl)-1,3,4-oxadiazole-2-yl)pyridine-2-yl)methyl)-6-fluoro-3-((1-(oxet an-3-yl)piperidine-4-yl)methyl)-1,3-dihydro-2H-benzo[d]imidazole-2-one (0.150 g, 0.253 mmol) prepared in the step 6 of the compound 289, pyridine-3-ylboronic acid (0.037 g, 0.303 mmol), cesium carbonate (0.165 g, 0.506 mmol) and [1,1'-bis(di-tert-butylphosphino)ferrocene]palladium dichloride (0.008 g, 0.013 mmol) were mixed in 1,4-dioxane (2.1 mL)/water (0.7 mL) at room temperature, after which the resulting mixture was irradiated with microwave, then heated at 100°C for 20 minutes, and then a reaction was finished by lowering a temperature to room temperature. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 10%) and concentrated to obtain a title compound (0.035 g, 23.7%) in a brown solid form.

**¹H NMR** (400 MHz, CDCl₃) δ 9.27 (d, *J =* 1.6 Hz, 1H), 8.72 (s, 1H), 8.58 (s, 1H), 8.35 (dd, *J* = 8.2, 2.2 Hz, 1H), 7.82 (dd, *J* = 7.9, 1.6 Hz, 1H), 7.47 (d, *J = 8.2* Hz, 1H), 7.36 (dd, *J* = 7.8, 4.8 Hz, 1H), 6.96 (d, *J =* 6.1 Hz, 1H), 6.92 (t, *J =* 51.6 Hz, 1H), 6.89 (d, *J =* 9.8 Hz, 1H), 5.27 (s, 2H), 4.59 (td, *J* = 12.2, 6.0 Hz, 4H), 3.84 (d, *J = 7.1* Hz, 2H), 3.46 - 3.39 (m, 1H), 2.74 (d, *J =* 11.3 Hz, 2H), 1.98 - 1.90 (m, 1H), 1.82 - 1.71 (m, 4H), 1.52 - 1.42 (m, 2H); LRMS (ES) m/z 592.5 (M⁺ + 1).

### Example 291: Synthesis of Compound 291, 1-((5-(5-(difluoromethyl)-1,3,4-oxadiazole-2-yl)pyridine-2-yl)methyl)-6-fluoro-3-((1-(oxetan-3-yl)pi peridine-4-yl)methyl)-5-(pyridine-4-yl)-1,3-dihydro-2H-benzo[d]imidazole-2-one

### [Step 1] Synthesis of the compound 291

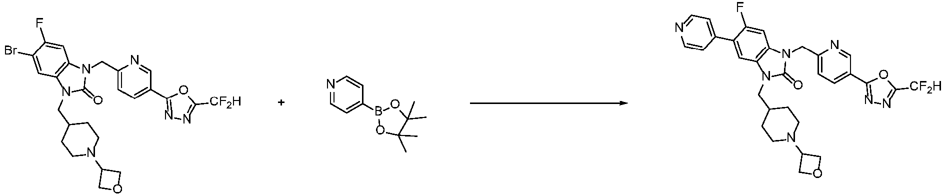

The 5-bromo-1-((5-(5-(difluoromethyl)-1,3,4-oxadiazole-2-yl)pyridine-2-yl)methyl)-6-fluoro-3-((1-(oxet an-3-yl)piperidine-4-yl)methyl)-1,3-dihydro-2H-benzo[d]imidazole-2-one (0.150 g, 0.253 mmol) prepared in the step 6 of the compound 289, 4-(4,4,5,5-tetramethyl-1,3,2-dioxaborolane-2-yl)pyridine (0.062 g, 0.303 mmol), cesium carbonate (0.165 g, 0.506 mmol) and [1,1'-bis(di-tert-butylphosphino)ferrocene]palladium dichloride (0.008 g, 0.013 mmol) were mixed in 1,4-dioxane (2.1 mL)/water (0.7 mL) at room temperature, after which the resulting mixture was irradiated with microwave, then heated at 100°C for 20 minutes, and then a reaction was finished by lowering a temperature to room temperature. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 10%) and concentrated to obtain a title compound (0.100 g, 66.5%) in a brown solid form.

**¹H NMR** (400 MHz, CDCl₃) δ 9.25 (d, *J =* 1.6 Hz, 1H), 8.63 (d, *J =* 4.9 Hz, 2H), 8.34 (dd, *J* = 8.2, 2.1 Hz, 1H), 7.46 (d, *J* = 8.2 Hz, 1H), 7.41 (d, *J* = 4.6 Hz, 2H), 6.98 (d, *J* = 6.0 Hz, 1H), 6.92 (t, *J =* 51.6 Hz, 1H), 6.88 (d, *J =* 10.1 Hz, 1H), 5.26 (s, 2H), 4.58 (td, *J =* 13.1, 6.6 Hz, 4H), 3.83 (d, *J =* 7.1 Hz, 2H), 3.44 - 3.38 (m, 1H), 2.73 (d, *J =* 11.2 Hz, 2H), 1.96 - 1.90 (m, 1H), 1.81 - 1.70 (m, 4H), 1.48 - 1.41 (m, 2H); **LRMS** (ES) m/z 592.6 (M⁺+ 1).

### Example 292: Synthesis of Compound 292, 1-((5-(5-(difluoromethyl)-1,3,4-oxadiazole-2-yl)pyridine-2-yl)methyl)-6-fluoro-3-((1-(oxetan-3-yl)pi peridine-4-yl)methyl)-5-(1H-pyrazole-4-yl)-1,3-dihydro-2H-benzo[d]imidazole-2-one

### [Step 1] Synthesis of the compound 292

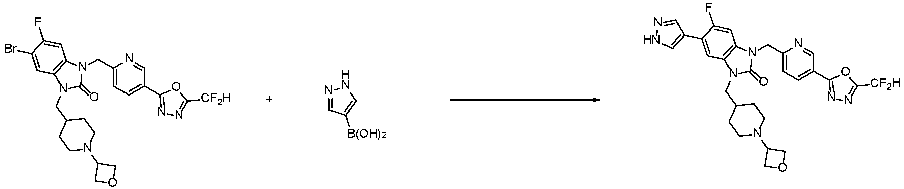

The 5-bromo-1-((5-(5-(difluoromethyl)-1,3,4-oxadiazole-2-yl)pyridine-2-yl)methyl)-6-fluoro-3-((1-(oxet an-3-yl)piperidine-4-yl)methyl)-1,3-dihydro-2H-benzo[d]imidazole-2-one (0.150 g, 0.253 mmol) prepared in the step 6 of the compound 289, (1H-pyrazole-4-yl)boronic acid (0.034 g, 0.303 mmol), cesium carbonate (0.165 g, 0.506 mmol) and [1,1'-bis(di-tert-butylphosphino)ferrocene]palladium dichloride (0.008 g, 0.013 mmol) were mixed in 1,4-dioxane (2.1 mL)/water (0.7 mL) at room temperature, after which the resulting mixture was irradiated with microwave, then heated at 100°C for 20 minutes, and then a reaction was finished by lowering a temperature to room temperature. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 10%) and concentrated to obtain a title compound (0.072 g, 48.9%) in a brown solid form.

**¹H NMR** (400 MHz, CDCl₃) δ 9.27 (d, *J =* 1.6 Hz, 1H), 8.34 (dd, *J* = 8.2, 2.2 Hz, 1H), 7.94 (s, 2H), 7.43 (d, *J =* 8.2 Hz, 1H), 7.14 (d, *J* = 6.0 Hz, 1H), 7.05 - 6.79 (m, 2H), 5.25 (s, 2H), 4.63 (td, *J* = 14.8, 7.7 Hz, 4H), 3.86 (d, *J = 7.2* Hz, 2H), 3.48 - 3.42 (m, 1H), 2.78 (d, *J =* 11.1 Hz, 2H), 2.01 - 1.97 (m, 1H), 1.85 - 1.73 (m, 4H), 1.61 - 1.52 (m, 2H); LRMS (ES) m/z 581.3 (M⁺ + 1).

### Example 293: Synthesis of Compound 293, (R)-1-((5-(5-(difluoromethyl)-1,3,4-oxadiazole-2-yl)pyridine-2-yl)methyl)-6-fluoro-3-(1-(oxetan-3-y I)piperidine-3-yl)-5-(pyridine-3-yl)-1,3-dihydro-2H-benzo[d]imidazole-2-one

### [Step 1] Synthesis of the compound 293

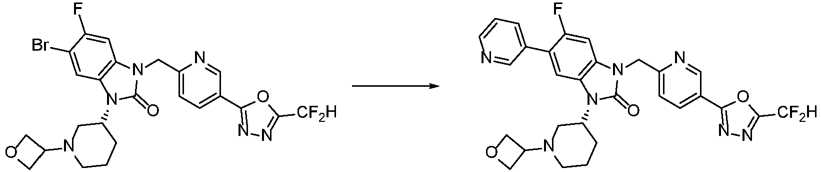

(R)-5-bromo-1-((5-(5-(difluoromethyl)-1,3,4-oxadiazole-2-yl)pyridine-2-yl)methyl)-6-fluor o-3-(1-(oxetan-3-yl)piperidine-3-yl)-1,3-dihydro-2H-benzo[d]imidazole-2-one (0.100 g, 0.173 mmol), pyridine-3-ylboronic acid (0.025 g, 0.207 mmol), [1,1'-bis(di-tert-butylphosphino)ferrocene]palladium(II) dichloride (Pd(dtbpf)Cl₂, 0.006 g, 0.009 mmol) and cesium carbonate (0.169 g, 0.518 mmol) were mixed in 1,4-dioxane (1.5 mL)/water (0.5 mL) at room temperature, after which the resulting mixture was irradiated with microwave, then heated at 100°C for 20 minutes, and then a reaction was finished by lowering a temperature to room temperature. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 12 g cartridge; methanol/dichloromethane = 0 to 5%) and concentrated to obtain a title compound (0.030 g, 30.1%) in a light brown solid form.

**¹H NMR** (400 MHz, CDCl₃) δ 9.32 (d, *J* = 1.7 Hz, 1H), 8.77 (s, 1H), 8.62 (d, *J* = 4.8 Hz, 1H), 8.39 (dd, *J* = 8.2, 2.2 Hz, 1H), 7.85 (dd, *J* = 7.9, 1.6 Hz, 1H), 7.50 (d, *J* = 8.2 Hz, 1H), 7.42 - 7.39 (m, 1H), 7.16 (d, *J =* 6.1 Hz, 1H), 7.08 - 6.82 (m, 2H), 5.27 - 5.26 (m, 2H), 4.70 (t, *J =* 6.5 Hz, 1H), 4.68 - 4.63 (m, 3H), 4.50 - 4.44 (m, 1H), 3.63 - 3.60 (m, 1H), 2.91 (d, *J =* 10.3 Hz, 1H), 2.80 (d, *J* = 10.6 Hz, 1H), 2.70 (t, *J =* 10.8 Hz, 1H), 2.33 - 2.29 (m, 1H), 2.06 - 1.99 (m, 2H), 1.94 - 1.92 (m, 2H); **LRMS** (ES) m/z 578.6 (M⁺ + 1).

### Example 294: Synthesis of Compound 294, (R)-1-((5-(5-(difluoromethyl)-1,3,4-oxadiazole-2-yl)pyridine-2-yl)methyl)-6-fluoro-3-(1-(oxetan-3-y l)piperidine-3-yl)-5-(pyridine-4-yl)-1,3-dihydro-2H-benzo[d]imidazole-2-one

### [Step 1] Synthesis of the compound 294

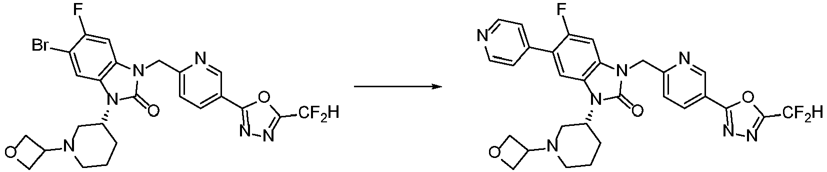

(R)-5-bromo-1-((5-(5-(difluoromethyl)-1,3,4-oxadiazole-2-yl)pyridine-2-yl)methyl)-6-fluor o-3-(1-(oxetan-3-yl)piperidine-3-yl)-1,3-dihydro-2H-benzo[d]imidazole-2-one (0.100 g, 0.173 mmol), pyridine-4-ylboronic acid (0.025 g, 0.207 mmol), [1,1'-bis(di-tert-butylphosphino)ferrocene]palladium(II) dichloride (Pd(dtbpf)Cl₂, 0.006 g, 0.009 mmol) and cesium carbonate (0.169 g, 0.518 mmol) were mixed in 1,4-dioxane (1.5 mL)/water (0.5 mL) at room temperature, after which the resulting mixture was irradiated with microwave, then heated at 100°C for 20 minutes, and then a reaction was finished by lowering a temperature to room temperature. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 12 g cartridge; methanol/dichloromethane = 0 to 5%) and concentrated to obtain a title compound (0.008 g, 8.0%) in a brown solid form.

**¹H NMR** (400 MHz, CDCl₃) δ 9.32 (d, *J =* 2.0 Hz, 1H), 8.70 (d, *J =* 5.7 Hz, 2H), 8.40 (dd, *J* = 8.2, 2.2 Hz, 1H), 7.51 (d, *J = 8.2* Hz, 1H), 7.47 (d, *J = 4.7* Hz, 2H), 7.20 (d, *J* = 6.0 Hz, 1H), 7.08 - 6.82 (m, 2H), 5.31 - 5.22 (m, 2H), 4.73 - 4.69 (m, 1H), 4.66 - 4.64 (m, 3H), 4.46 - 4.42 (m, 1H), 3.64 - 3.61 (m, 1H), 2.91 - 2.89 (m, 1H), 2.81 (d, *J =*11.6 Hz, 1H), 2.72 (t, *J =* 10.8 Hz, 1H), 2.36 -2.70 (m, 1H), 2.07 - 2.04 (m, 1H), 1.97 - 1.93 (m, 2H), 1.84 - 1.80 (m, 1H); **LRMS** (ES) m/z 578.6 (M⁺ + 1).

### Example 295: Synthesis of Compound 295, (R)-1-((5-(5-(difluoromethyl)-1,3,4-oxadiazole-2-yl)pyridine-2-yl)methyl)-6-fluoro-5-(1H-indole-4-yl)-3-(1-(oxetan-3-yl)piperidine-3-yl)-1,3-dihydro-2H-benzo[d]imidazole-2-one

### [Step 1] Synthesis of the compound 295

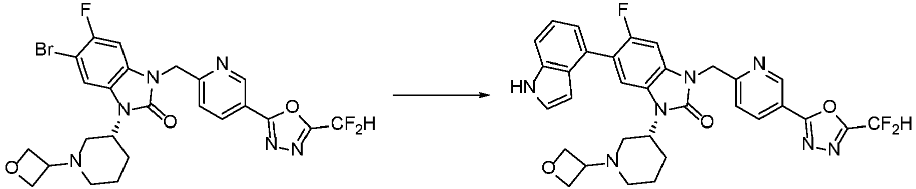

(R)-5-bromo-1-((5-(5-(difluoromethyl)-1,3,4-oxadiazole-2-yl)pyridine-2-yl)methyl)-6-fluor o-3-(1-(oxetan-3-yl)piperidine-3-yl)-1,3-dihydro-2H-benzo[d]imidazole-2-one (0.100 g, 0.173 mmol), (1H-indole-4-yl)boronic acid (0.033 g, 0.207 mmol), [1,1'-bis(di-tert-butylphosphino)ferrocene]palladium(II) dichloride (Pd(dtbpf)Cl₂, 0.006 g, 0.009 mmol) and cesium carbonate (0.169 g, 0.518 mmol) were mixed in 1,4-dioxane (1.5 mL)/water (0.5 mL) at room temperature, after which the resulting mixture was irradiated with microwave, then heated at 100°C for 20 minutes, and then a reaction was finished by lowering a temperature to room temperature. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 12 g cartridge; methanol/dichloromethane = 0 to 5%) and concentrated to obtain a title compound (0.013 g, 12.2%) in a light brown solid form.

**¹H NMR** (400 MHz, CDCl₃) δ 9.34 (dd, *J =* 2.1, 0.7 Hz, 1H), 8.43 - 8.39 (m, 2H), 7.52 (d, *J* = 8.2 Hz, 1H), 7.47 (d, *J* = 8.2 Hz, 1H), 7.34 - 7.29 (m, 2H), 7.18 (d, *J = 7.3* Hz, 1H), 7.08 - 6.83 (m, 2H), 6.48 - 6.47 (m, 1H), 5.34 - 5.25 (m, 2H), 4.70 - 4.63 (m, 4H), 4.54 - 4.48 (m, 1H), 3.61 - 3.57 (m, 1H), 2.91 (dd, *J =* 10.2, 3.9 Hz, 1H), 2.77 (d, *J =* 9.9 Hz, 1H), 2.68 (t, *J =* 10.8 Hz, 1H), 2.31-2.27 (m, 1H), 2.04 (d, *J =* 10.9 Hz, 1H), 1.90 - 1.87 (m, 2H), 1.80 - 1.76 (m, 1H); **LRMS** (ES) m/z 616.2 (M⁺ + 1).

### Example 296: Synthesis of Compound 296, (R)-1-((5-(5-(difluoromethyl)-1,3,4-oxadiazole-2-yl)pyridine-2-yl)methyl)-6-fluoro-3-(1-(oxetan-3-y l)piperidine-3-yl)-5-(1H-pyrazole-4-yl)-1,3-dihydro-2H-benzo[d]imidazole-2-one

### [Step 1] Synthesis of the compound 296

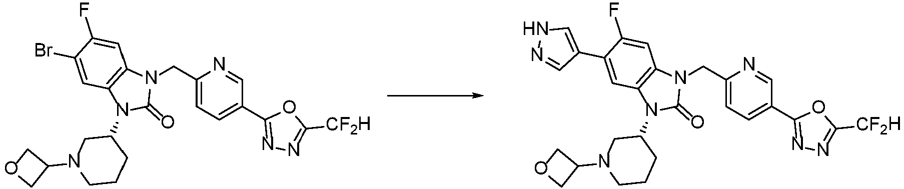

(R)-5-bromo-1-((5-(5-(difluoromethyl)-1,3,4-oxadiazole-2-yl)pyridine-2-yl)methyl)-6-fluor o-3-(1-(oxetan-3-yl)piperidine-3-yl)-1,3-dihydro-2H-benzo[d]imidazole-2-one (0.100 g, 0.173 mmol), (1H-pyrazole-4-yl)boronic acid (0.023 g, 0.207 mmol), [1,1'-bis(di-tert-butylphosphino)ferrocene]palladium(II) dichloride (Pd(dtbpf)Cl₂, 0.006 g, 0.009 mmol) and cesium carbonate (0.169 g, 0.518 mmol) were mixed in 1,4-dioxane (1.5 mL)/water (0.5 mL) at room temperature, after which the resulting mixture was irradiated with microwave, then heated at 100°C for 20 minutes, and then a reaction was finished by lowering a temperature to room temperature. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 12 g cartridge; methanol/dichloromethane = 0 to 5%) and concentrated to obtain a title compound (0.008 g, 8.2%) in a light brown solid form.

**¹H NMR** (400 MHz, CDCl₃) δ 9.31 (d, *J =* 1.5 Hz, 1H), 8.37 (dd, *J* = 8.2, 2.2 Hz, 1H), 7.98 (s, 2H), 7.46 (d, *J* = 8.2 Hz, 1H), 7.32 (d, *J =* 6.0 Hz, 1H), 7.08 - 6.82 (m, 2H), 5.24 - 5.24 (m, 2H), 4.75 (t, *J =* 6.5 Hz, 1H), 4.70 - 4.63 (m, 3H), 4.55 - 4.50 (m, 1H), 3.64 - 3.61 (m, 1H), 2.92 - 2.85 (m, 2H), 2.76 (t, *J =* 10.8 Hz, 1H), 2.45 - 2.40 (m, 1H), 2.07 - 2.00 (m, 3H), 1.90 - 1.87 (m, 1H); **LRMS** (ES) m/z 567.5 (M⁺ + 1).

### Example 297: Synthesis of Compound 297, (S)-1-((5-(5-(difluoromethyl)-1,3,4-oxadiazole-2-yl)pyridine-2-yl)methyl)-6-fluoro-3-(1-(oxetan-3-y I)piperidine-3-yl)-5-(pyridine-3-yl)-1,3-dihydro-2H-benzo[d]imidazole-2-one

### [Step 1] Synthesis of tert-butyl (S)-3-((5-bromo-4-fluoro-2-nitrophenyl)amino)piperidine-1-carboxylate

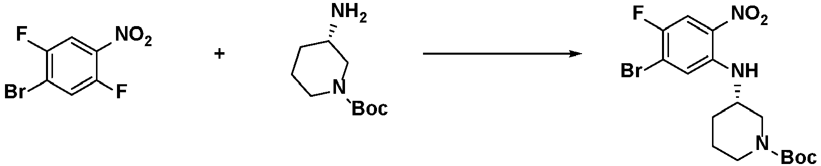

1-bromo-2,5-difluoro-4-nitrobenzene (4.000 g, 16.807 mmol), tert-butyl (S)-3-aminopyrrolidine-1-carboxylate (3.757 g, 20.169 mmol) and N,N-diisopropylethylamine (3.513 mL, 20.169 mmol) were dissolved in tetrahydrofuran (30 mL) at 65°C, after which the resulting solution was stirred at the same temperature for 18 hours, and then a reaction was finished by lowering a temperature to room temperature. Water was poured into the reaction mixture and an extraction was performed with dichloromethane. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 40 g cartridge; ethyl acetate/hexane = 0 to 30%) and concentrated to obtain a title compound (5.140 g, 73.1%) in a yellow solid form.

### [Step 2] Synthesis of tert-butyl (S)-3-((2-amino-5-bromo-4-fluorophenyl)amino)piperidine-1-carboxylate

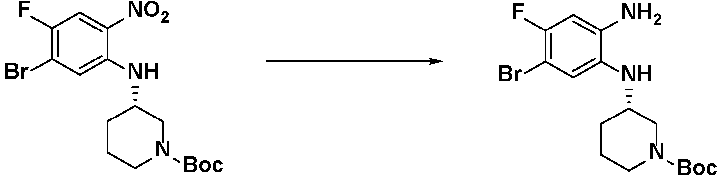

The tert-butyl (S)-3-((5-bromo-4-fluoro-2-nitrophenyl)amino)piperidine-1-carboxylate (5.140 g, 12.289 mmol) prepared in the step 1 was dissolved in ethanol (50 mL) at room temperature, after which Raney nickel was slowly added into the resulting solution and stirred at the same temperature for 18 hours in the presence of a hydrogen balloon attached thereto. The reaction mixture was filtered via a celite pad to remove a solid therefrom, after which solvent was removed from the resulting filtrate under reduced pressure, and then a title compound was used without an additional purification process (4.500 g, 94.3%, yellow solid).

### [Step 3] Synthesis of tert-butyl (S)-3-(6-bromo-5-fluoro-2-oxo-2,3-dihydro-1H-benzo[d]imidazole-1-yl)piperidine-1-carboxylate

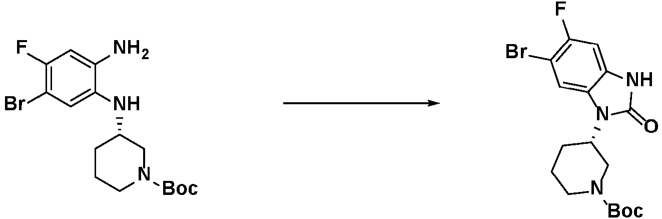

The tert-butyl (S)-3-((2-amino-5-bromo-4-fluorophenyl)amino)piperidine-1-carboxylate (5.140 g, 13.238 mmol) prepared in the step 2 and 1,1'-carbonyldiimidazole (CDI, 3.220 g, 19.857 mmol) were dissolved in tetrahydrofuran (50 mL) at room temperature, after which the resulting solution was stirred at the same temperature for 18 hours. Water was poured into the reaction mixture and an extraction was performed with ethyl acetate. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 40 g cartridge; ethyl acetate/hexane = 0 to 50%) and concentrated to obtain a title compound (3.190 g, 58.2%) in a white solid form.

### [Step 4] Synthesis of methyl (S)-6-((5-bromo-3-(1-(tert-butoxycarbonyl)piperidine-3-yl)-6-fluoro-2-oxo-2,3-dihydro-1H-benzo[ d]imidazole-1-yl)methyl)nicotinate

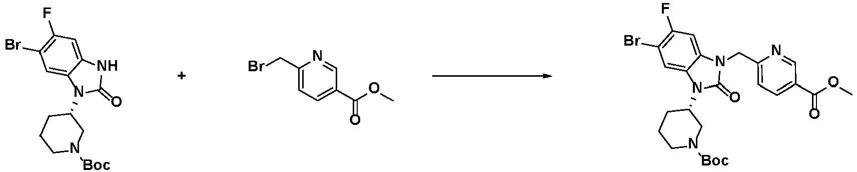

The tert-butyl (S)-3-(6-bromo-5-fluoro-2-oxo-2,3-dihydro-1H-benzo[d]imidazole-1-yl)piperidine-1-carboxylate (3.190 g, 7.700 mmol) prepared in the step 3 was dissolved in N,N-dimethylformamide (30 mL) at 0°C, after which sodium hydride (60.00%, 0.462 g, 11.550 mmol) was added into the resulting solution and stirred at the same temperature for 30 minutes. Methyl 6-(bromomethyl)nicotinate (2.126 g, 9.240 mmol) was added into the reaction mixture and further stirred at room temperature for 18 hours. Water was poured into the reaction mixture and an extraction was performed with ethyl acetate. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 80 g cartridge; ethyl acetate/hexane = 0 to 50%) and concentrated to obtain a title compound (2.570 g, 59.2%) in a white foam solid form.

### [Step 5] Synthesis of tert-butyl (S)-3-(6-bromo-5-fluoro-3-((5-(hydrazinecarbonyl)pyridine-2-yl)methyl)-2-oxo-2,3-dihydro-1H-be nzo[d]imidazole-1-yl)piperidine-1-carboxylate

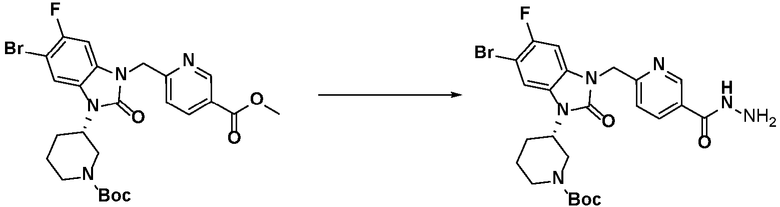

The methyl (S)-6-((5-bromo-3-(1-(tert-butoxycarbonyl)piperidine-3-yl)-6-fluoro-2-oxo-2,3-dihydro-1H-benzo[ d]imidazole-1-yl)methyl)nicotinate (2.570 g, 4.561 mmol) prepared in the step 4 and hydrazine monohydrate (4.434 mL, 91.229 mmol) were dissolved in ethanol (20 mL) at 80°C, after which the resulting solution was stirred at the same temperature for 18 hours, and then a reaction was finished by lowering a temperature to room temperature. Solvent was removed from the reaction mixture under reduced pressure, after which a title compound was used without an additional purification process (2.570 g, 100.0%, white solid).

### [Step 6] Synthesis of tert-butyl (S)-3-(6-bromo-3-((5-(5-(difluoromethyl)-1,3,4-oxadiazole-2-yl)pyridine-2-yl)methyl)-5-fluoro-2-ox o-2,3-dihydro-1H-benzo[d]imidazole-1-yl)piperidine-1-carboxylate

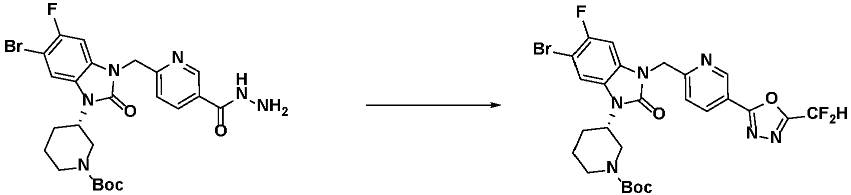

The tert-butyl (S)-3-(6-bromo-5-fluoro-3-((5-(hydrazinecarbonyl)pyridine-2-yl)methyl)-2-oxo-2,3-dihydro-1H-be nzo[d]imidazole-1-yl)piperidine-1-carboxylate (2.700 g, 4.792 mmol) prepared in the step 5, 2,2-difluoroacetic anhydride (1.787 mL, 14.376 mmol) and imidazole (0.979 g, 14.376 mmol) were dissolved in dichloromethane (30 mL) at 45°C, after which the resulting solution was stirred at the same temperature for 18 hours, and then a reaction was finished by lowering a temperature to room temperature. Water was poured into the reaction mixture and an extraction was performed with dichloromethane. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 40 g cartridge; ethyl acetate/hexane = 0 to 50%) and concentrated to obtain a title compound (2.400 g, 80.3%) in a white foam solid form.

### [Step 7] Synthesis of tert-butyl (S)-3-(3-((5-(5-(difluoromethyl)-1,3,4-oxadiazole-2-yl)pyridine-2-yl)methyl)-5-fluoro-2-oxo-6-(pyri dine-3-yl)-2,3-dihydro-1H-benzo[d]imidazole-1-yl)piperidine-1-carboxylate

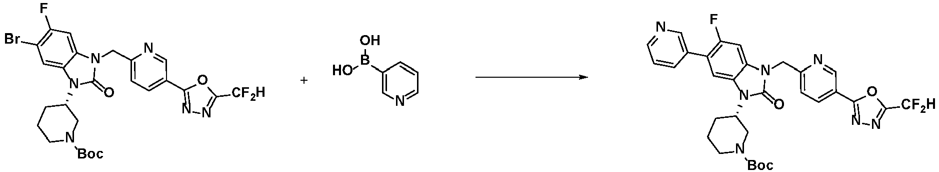

The tert-butyl (S)-3-(6-bromo-3-((5-(5-(difluoromethyl)-1,3,4-oxadiazole-2-yl)pyridine-2-yl)methyl)-5-fluoro-2-ox o-2,3-dihydro-1H-benzo[d]imidazole-1-yl)piperidine-1-carboxylate (0.600 g, 0.962 mmol) prepared in the step 6, pyridine-3-ylboronic acid (0.177 g, 1.444 mmol), [1,1'-bis(diphenylphosphino)ferrocene]dichloropalladium(II) (Pd(dppf)Cl₂, 0.070 g, 0.096 mmol) and cesium carbonate (0.627 g, 1.925 mmol) were mixed in 1,4-dioxane (9 mL)/water (3 mL), after which the resulting mixture was irradiated with microwave, then heated at 100°C for 20 minutes, and then a reaction was finished by lowering a temperature to room temperature. Water was poured into the reaction mixture and an extraction was performed with ethyl acetate. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; ethyl acetate/hexane = 0 to 100%) and concentrated to obtain a title compound (0.410 g, 68.5%) in a brown oil form.

### [Step 8] Synthesis of (S)-1-((5-(5-(difluoromethyl)-1,3,4-oxadiazole-2-yl)pyridine-2-yl)methyl)-6-fluoro-3-(piperidine-3-yl)-5-(pyridine-3-yl)-1,3-dihydro-2H-benzo[d]imidazole-2-one

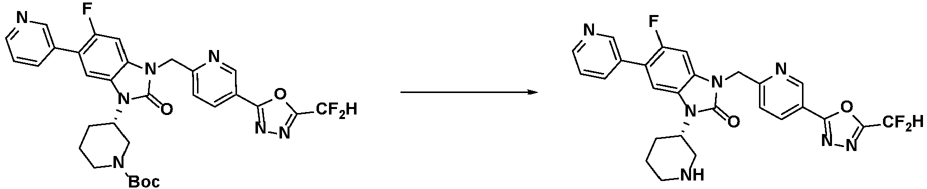

The tert-butyl (S)-3-(3-((5-(5-(difluoromethyl)-1,3,4-oxadiazole-2-yl)pyridine-2-yl)methyl)-5-fluoro-2-oxo-6-(pyri dine-3-yl)-2,3-dihydro-1H-benzo[d]imidazole-1-yl)piperidine-1-carboxylate (0.410 g, 0.660 mmol) prepared in the step 7 and trifluoroacetic acid (0.505 mL, 6.596 mmol) were dissolved in dichloromethane (10 mL) at room temperature, after which the resulting solution was stirred at the same temperature for 18 hours. Solvent was removed from the reaction mixture under reduced pressure, after which saturated sodium hydrogen carbonate aqueous solution was poured into the resulting concentrate, and then an extraction was performed with dichloromethane. An organic layer was washed with saturated sodium chloride aqueous solution, then dehydrated with anhydrous sodium sulfate, then filtered, and then concentrated under reduced pressure. A title compound was used without an additional purification process (0.360 g, 104.7%, yellow oil).

### [Step 9] Synthesis of the compound 297

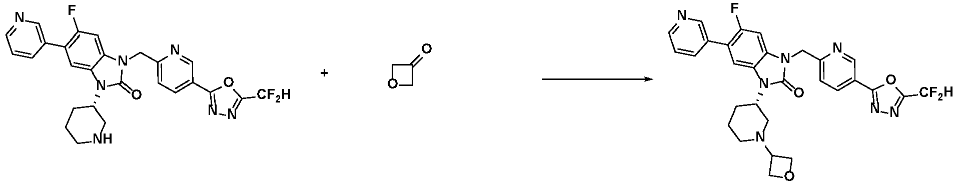

The (S)-1-((5-(5-(difluoromethyl)-1,3,4-oxadiazole-2-yl)pyridine-2-yl)methyl)-6-fluoro-3-(piperidine-3-yl)-5-(pyridine-3-yl)-1,3-dihydro-2H-benzo[d]imidazole-2-one (0.144 g, 0.276 mmol) prepared in the step 8, oxetan-3-one (0.040 g, 0.552 mmol) and sodium triacetoxyborohydride (0.117 g, 0.552 mmol) were dissolved in dichloromethane (10 mL) at room temperature, after which the resulting solution was stirred at the same temperature for 18 hours. Water was poured into the reaction mixture and an extraction was performed with dichloromethane. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; methanol/dichloromethane = 0 to 10%) and concentrated to obtain a title compound (0.100 g, 62.7%) in a white foam solid form.

**¹H NMR** (400 MHz, CDCl₃) δ 9.30 (dd, *J =* 2.1, 0.6 Hz, 1H), 8.76 (s, 1H), 8.60 (d, *J =* 3.6 Hz, 1H), 8.38 (dd, *J* = 8.2, 2.2 Hz, 1H), 7.87 ~ 7.85 (m, 1H), 7.49 (d, *J =* 7.8 Hz, 1H), 7.40 (dd, *J =* 7.7, 5.2 Hz, 1H), 7.18 (d, *J =* 6.2 Hz, 1H), 7.08 (s, 0.25H), 6.95 (s, 0.5H), 6.92 (d, *J =* 9.8 Hz, 1H), 6.82 (s, 0.25H), 5.30 (s, 2H), 4.70 ~ 4.62 (m, 4H), 4.50 ~ 4.47 (m, 1H), 3.62 ~ 3.59 (m, 1H), 2.92 ~ 2.78 (m, 2H), 2.72 ~ 2.67 (m, 1H), 2.33 ~ 2.29 (m, 1H), 2.04 ~ 1.81 (m, 4H).

### Example 298: Synthesis of Compound 298, (S)-1-((5-(5-(difluoromethyl)-1,3,4-oxadiazole-2-yl)pyridine-2-yl)methyl)-3-(1-ethylpiperidine-3-yl) -6-fluoro-5-(pyridine-3-yl)-1,3-dihydro-2H-benzo[d]imidazole-2-one

### [Step 1] Synthesis of the compound 298

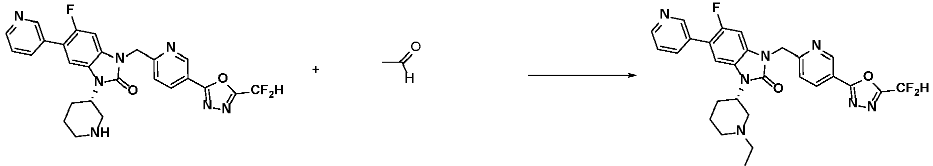

(S)-1-((5-(5-(difluoromethyl)-1,3,4-oxadiazole-2-yl)pyridine-2-yl)methyl)-6-fluoro-3-(piper idine-3-yl)-5-(pyridine-3-yl)-1,3-dihydro-2H-benzo[d]imidazole-2-one (0.139 g, 0.267 mmol), acetaldehyde (0.655 mL, 11.741 mmol) and sodium triacetoxyborohydride (0.113 g, 0.533 mmol) were dissolved in dichloromethane (10 mL) at room temperature, after which the resulting solution was stirred at the same temperature for 18 hours. Water was poured into the reaction mixture and an extraction was performed with dichloromethane. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; methanol/dichloromethane = 0 to 10%) and concentrated to obtain a title compound (0.100 g, 68.3%) in a white foam solid form.

**¹H NMR** (400 MHz, CDCl₃) δ 9.31 (d, *J =* 1.6 Hz, 1H), 8.78 (s, 1H), 8.59 (dd, *J* = 4.8, 1.6 Hz, 1H), 8.38 (dd, *J* = 8.2, 2.2 Hz, 1H), 7.90 ~ 7.87 (m, 1H), 7.49 (d, *J =* 8.2 Hz, 1H), 7.40 (dd, *J =* 4.9, 0.4 Hz, 1H), 7.28 ~ 7.25 (m, 1H), 7.08 (s, 0.25H), 6.95 (s, 0.5H), 6.90 (d, *J* = 9.8 Hz, 1H), 6.82 (s, 0.25H), 5.30 (s, 2H), 4.61 ~ 4.54 (m, 1H), 3.22 ~ 3.07 (m, 2H), 2.88 ~ 2.82 (m, 1H), 2.64 ~ 2.57 (m, 2H), 2.41 ~ 2.37 (m, 1H), 2.14 ~ 2.03 (m, 1H), 1.98 ~ 1.86 (m, 3H), 1.15 (t, *J =* 7.2 Hz, 3H).

### Example 299: Synthesis of Compound 299, (S)-1-((5-(5-(difluoromethyl)-1,3,4-oxadiazole-2-yl)pyridine-2-yl)methyl)-6-fluoro-3-(1-(oxetan-3-y l)piperidine-3-yl)-5-(pyridine-4-yl)-1,3-dihydro-2H-benzo[d]imidazole-2-one

### [Step 1] Synthesis of tert-butyl (S)-3-(3-((5-(5-(difluoromethyl)-1,3,4-oxadiazole-2-yl)pyridine-2-yl)methyl)-5-fluoro-2-oxo-6-(pyri dine-4-yl)-2,3-dihydro-1H-benzo[d]imidazole-1-yl)piperidine-1-carboxylate

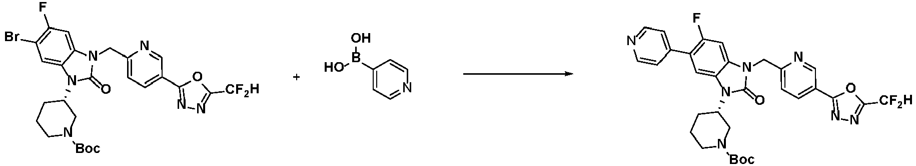

Tert-butyl (S)-3-(6-bromo-3-((5-(5-(difluoromethyl)-1,3,4-oxadiazole-2-yl)pyridine-2-yl)methyl)-5-fluoro-2-ox o-2,3-dihydro-1H-benzo[d]imidazole-1-yl)piperidine-1-carboxylate (0.800 g, 1.283 mmol), pyridine-4-ylboronic acid (0.237 g, 1.925 mmol), [1,1'-bis(diphenylphosphino)ferrocene]dichloropalladium(II) (Pd(dppf)Cl₂, 0.094 g, 0.128 mmol) and cesium carbonate (0.836 g, 2.566 mmol) were mixed in 1,4-dioxane (9 mL)/water (3 mL), after which the resulting mixture was irradiated with microwave, then heated at 100°C for 20 minutes, and then a reaction was finished by lowering a temperature to room temperature. Water was poured into the reaction mixture and an extraction was performed with ethyl acetate. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; ethyl acetate/hexane = 0 to 100%) and concentrated to obtain a title compound (0.514 g, 64.4%) in a brown oil form.

### [Step 2] Synthesis of (S)-1-((5-(5-(difluoromethyl)-1,3,4-oxadiazole-2-yl)pyridine-2-yl)methyl)-6-fluoro-3-(piperidine-3-yl)-5-(pyridine-4-yl)-1,3-dihydro-2H-benzo[d]imidazole-2-one

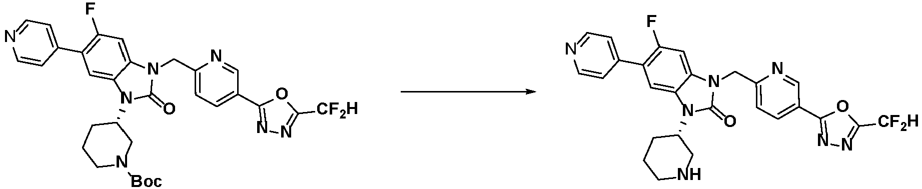

The tert-butyl (S)-3-(3-((5-(5-(difluoromethyl)-1,3,4-oxadiazole-2-yl)pyridine-2-yl)methyl)-5-fluoro-2-oxo-6-(pyri dine-4-yl)-2,3-dihydro-1H-benzo[d]imidazole-1-yl)piperidine-1-carboxylate (0.514 g, 0.827 mmol) prepared in the step 1 and trifluoroacetic acid (0.633 mL, 8.269 mmol) were dissolved in dichloromethane (10 mL) at room temperature, after which the resulting solution was stirred at the same temperature for 18 hours. Solvent was removed from the reaction mixture under reduced pressure, after which saturated sodium hydrogen carbonate aqueous solution was poured into the resulting concentrate, and then an extraction was performed with dichloromethane. An organic layer was washed with saturated sodium chloride aqueous solution, then dehydrated with anhydrous sodium sulfate, then filtered, and then concentrated under reduced pressure. A title compound was used without an additional purification process (0.450 g, 104.4%, yellow oil).

### [Step 3] Synthesis of the compound 299

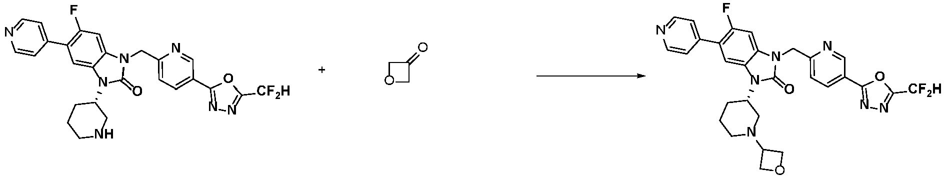

The (S)-1-((5-(5-(difluoromethyl)-1,3,4-oxadiazole-2-yl)pyridine-2-yl)methyl)-6-fluoro-3-(piperidine-3-yl)-5-(pyridine-4-yl)-1,3-dihydro-2H-benzo[d]imidazole-2-one (0.130 g, 0.249 mmol) prepared in the step 2, oxetan-3-one (0.036 g, 0.499 mmol) and sodium triacetoxyborohydride (0.106 g, 0.499 mmol) were dissolved in dichloromethane (10 mL) at room temperature, after which the resulting solution was stirred at the same temperature for 18 hours. Water was poured into the reaction mixture and an extraction was performed with dichloromethane. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; methanol/dichloromethane = 0 to 10%) and concentrated to obtain a title compound (0.080 g, 55.6%) in a white foam solid form.

¹H NMR (400 MHz, CDCl₃) δ 9.31 (dd, *J =* 2.1*,* 0.7 Hz, 1H), 8.68 (d, *J =* 5.6 Hz, 2H), 8.39 (dd, *J* = 8.2, 2.2 Hz, 1H), 7.51 ~ 7.47 (m, 3H), 7.21 (d, *J* = 6.1 Hz, 1H), 7.08 (s, 0.25H), 6.95 (s, 0.5H), 6.93 (d, *J =* 10.1 Hz, 1H), 6.82 (s, 0.25H), 5.31 (s, 2H), 4.68 ~ 4.58 (m, 4H), 4.48 ~ 4.42 (m, 1H), 3.63 ~ 3.60 (m, 1H), 2.92 ~ 2.88 (m, 1H), 2.82 ~ 2.79 (m, 1H), 2.79 ~ 2.69 (m, 1H), 2.35 ~ 2.31 (m, 1H), 2.05 ~ 1.92 (m, 3H), 1.81 ~ 1.79 (m, 1H).

### Example 300: Synthesis of Compound 300, (S)-1-((5-(5-(difluoromethyl)-1,3,4-oxadiazole-2-yl)pyridine-2-yl)methyl)-3-(1-ethylpiperidine-3-yl) -6-fluoro-5-(pyridine-4-yl)-1,3-dihydro-2H-benzo[d]imidazole-2-one

### [Step 1] Synthesis of the compound 300

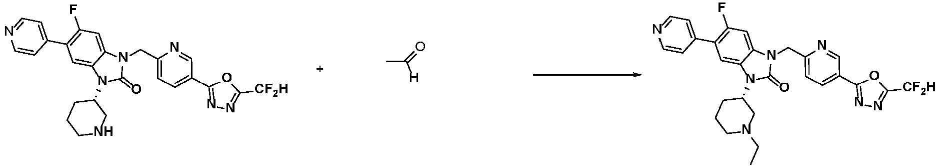

(S)-1-((5-(5-(difluoromethyl)-1,3,4-oxadiazole-2-yl)pyridine-2-yl)methyl)-6-fluoro-3-(piper idine-3-yl)-5-(pyridine-4-yl)-1,3-dihydro-2H-benzo[d]imidazole-2-one (0.168 g, 0.322 mmol), acetaldehyde (0.791 mL, 14.191 mmol) and sodium triacetoxyborohydride (0.137 g, 0.644 mmol) were dissolved in dichloromethane (10 mL) at room temperature, after which the resulting solution was stirred at the same temperature for 18 hours. Water was poured into the reaction mixture and an extraction was performed with dichloromethane. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; methanol/dichloromethane = 0 to 10%) and concentrated to obtain a title compound (0.110 g, 62.1%) in a white foam solid form.

**¹H NMR** (400 MHz, CDCl₃) δ 9.29 (dd, *J =* 2.2, 0.7 Hz, 1H), 8.65 ~ 8.64 (m, 2H), 8.38 (dd, *J* = 8.2, 2.2 Hz, 1H), 7.52 ~ 7.47 (m, 3H), 7.34 (d, *J = 6.2* Hz, 1H), 7.80 (s, 0.25H), 6.95 (s, 0.5H), 6.90 (d, *J =* 10.2 Hz, 1H), 6.82 (s, 0.25H), 5.25 (s, 2H), 4.61 ~ 4.55 (m, 1H), 3.25 ~ 3.21 (m, 1H), 3.12 ~ 3.09 (m, 1H), 2.89 (t, *J =* 11.0 Hz, 1H), 2.67 ~ 2.60 (m, 2H), 2.45 ~ 2.40 (m, 1H), 2.21 ~ 2.15 (m, 1H), 2.18 ~ 1.87 (m, 3H), 1.20 ~ 1.15 (m, 3H).

### Example 301: Synthesis of Compound 301, 3-((5-(5-(difluoromethyl)-1,3,4-oxadiazole-2-yl)pyridine-2-yl)methyl)-5-fluoro-1-((1-(oxetan-3-yl)pi peridine-4-yl)methyl)-1,3-dihydro-2H-benzo[d]imidazole-2-one

### [Step 1] Synthesis of tert-butyl 4-(((4-fluoro-2-nitrophenyl)amino)methyl)piperidine-1-carboxylate

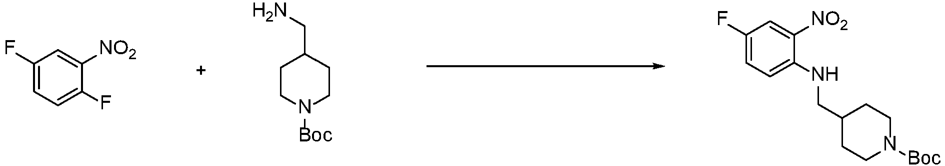

1,4-difluoro-2-nitrobenzene (0.300 g, 1.886 mmol), tert-butyl 4-(aminomethyl)piperidine-1-carboxylate (0.525 g, 2.451 mmol), potassium carbonate (0.521 g, 3.771 mmol) and potassium iodide (0.031 g, 0.189 mmol) were dissolved in N,N-dimethylformamide (7 mL) at room temperature, after which the resulting solution was stirred at 80°C for 18 hours, and then a reaction was finished by lowering a temperature to room temperature. Water was poured into the reaction mixture and an extraction was performed with ethyl acetate. An organic layer was washed with saturated sodium chloride aqueous solution, then dehydrated with anhydrous magnesium sulfate, then filtered, and then concentrated under reduced pressure. The resulting concentrate was purified via column chromatography (SiO₂, 12 g cartridge; ethyl acetate/hexane = 0 to 30%) and concentrated to obtain a title compound (0.484 g, 72.6%) in an orange solid form.

### [Step 2] Synthesis of tert-butyl 4-(((2-amino-4-fluorophenyl)amino)methyl)piperidine-1-carboxylate

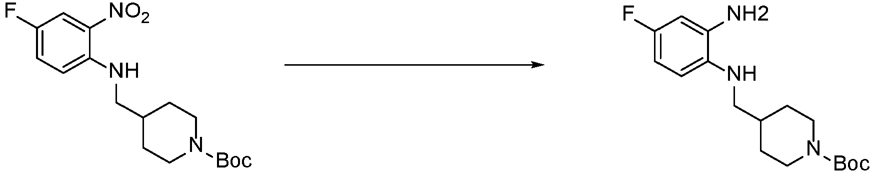

The tert-butyl 4-(((4-fluoro-2-nitrophenyl)amino)methyl)piperidine-1-carboxylate (0.484 g, 1.370 mmol) prepared in the step 1 was dissolved in methanol (13 mL) and stirred at room temperature, after which 10%-Pd/C (50 mg) was slowly added into the resulting solution at the same temperature and stirred at the same temperature for 18 hours in the presence of a hydrogen balloon attached thereto. The reaction mixture was filtered via a celite pad to remove a solid therefrom, after which solvent was removed from the resulting filtrate under reduced pressure, and then a title compound was used without an additional purification process (0.426 g, 96.2%, brown solid).

### [Step 3] Synthesis of tert-butyl 4-((5-fluoro-2-oxo-2,3-dihydro-1H-benzo[d]imidazole-1-yl)methyl)piperidine-1-carboxylate

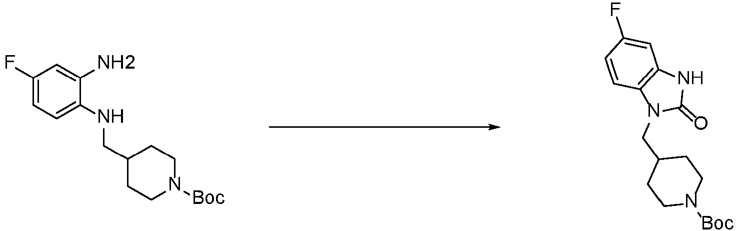

The tert-butyl 4-(((2-amino-4-fluorophenyl)amino)methyl)piperidine-1-carboxylate (0.426 g, 1.317 mmol) prepared in the step 2, triethylamine (0.184 mL, 1.317 mmol) and 1,1'-carbonyldiimidazole (CDI, 0.256 g, 1.581 mmol) were dissolved in dichloromethane (7 mL) at room temperature, after which the resulting solution was heated under reflux for 18 hours, and then a reaction was finished by lowering a temperature to room temperature. Solvent was removed from the reaction mixture under reduced pressure, after which the resulting concentrate was purified via column chromatography (SiO₂, 12 g cartridge; ethyl acetate/hexane = 0 to 50%) and concentrated to obtain a title compound (0.154 g, 33.5%) in a brown solid form.

### [Step 4] Synthesis of tert-butyl 4-((3-((5-(5-(difluoromethyl)-1,3,4-oxadiazole-2-yl)pyridine-2-yl)methyl)-5-fluoro-2-oxo-2,3-dihydr o-1H-benzo[d]imidazole-1-yl)methyl)piperidine-1-carboxylate

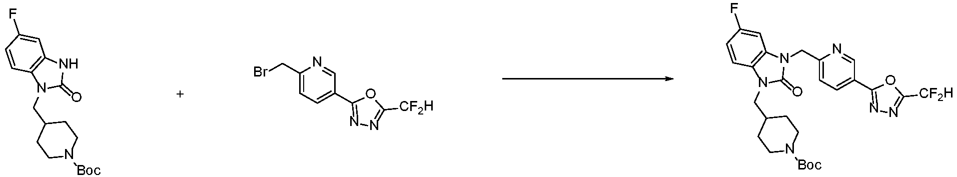

The tert-butyl 4-((5-fluoro-2-oxo-2,3-dihydro-1H-benzo[d]imidazole-1-yl)methyl)piperidine-1-carboxylate (0.154 g, 0.441 mmol) prepared in the step 3, sodium hydride (60.00%, 0.026 g, 0.661 mmol) and 2-(6-(bromomethyl)pyridine-3-yl)-5-(difluoromethyl)-1,3,4-oxadiazole (0.141 g, 0.485 mmol) were dissolved in N,N-dimethylformamide (3 mL), after which the resulting solution was stirred at 0°C for 10 minutes and further stirred at room temperature for 3 hours. Solvent was removed from the reaction mixture under reduced pressure, after which the resulting concentrate was purified via column chromatography (SiO₂, 4 g cartridge; ethyl acetate/hexane = 0 to 50%) and concentrated to obtain a title compound (0.150 g, 60.9%) in a white solid form.

### [Step 5] Synthesis of 3-((5-(5-(difluoromethyl)-1,3,4-oxadiazole-2-yl)pyridine-2-yl)methyl)-5-fluoro-1-(piperidine-4-ylme thyl)-1,3-dihydro-2H-benzo[d]imidazole-2-one

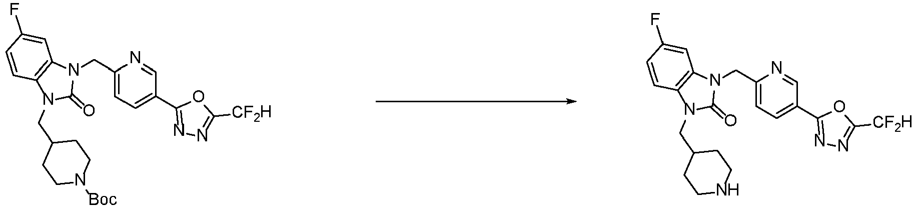

The tert-butyl 4-((3-((5-(5-(difluoromethyl)-1,3,4-oxadiazole-2-yl)pyridine-2-yl)methyl)-5-fluoro-2-oxo-2,3-dihydr o-1H-benzo[d]imidazole-1-yl)methyl)piperidine-1-carboxylate (0.150 g, 0.269 mmol) prepared in the step 4 and trifluoroacetic acid (0.206 mL, 2.685 mmol) were dissolved in dichloromethane (2 mL) at room temperature, after which the resulting solution was stirred at the same temperature for 3 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. A title compound was used without an additional purification process (0.120 g, 97.5%, brown solid).

### [Step 6] Synthesis of the compound 301

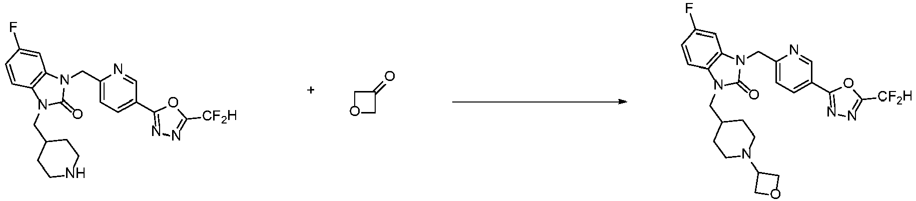

The 3-((5-(5-(difluoromethyl)-1,3,4-oxadiazole-2-yl)pyridine-2-yl)methyl)-5-fluoro-1-(piperidine-4-ylme thyl)-1,3-dihydro-2H-benzo[d]imidazole-2-one (0.120 g, 0.262 mmol) prepared in the step 5, oxetan-3-one (0.057 g, 0.785 mmol) and sodium triacetoxyborohydride (0.111 g, 0.524 mmol) were dissolved in dichloromethane (2 mL) at room temperature, after which the resulting solution was stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.087 g, 64.5%) in a brown solid form.

**¹H NMR** (400 MHz, CDCl₃) δ 9.23 (dd, *J* = 2.1, 0.6 Hz, 1H), 8.31 (dd, *J* = 8.2, 2.2 Hz, 1H), 7.40 (d, *J = 8.2* Hz, 1H), 7.04 - 6.71 (m, 4H), 5.22 (s, 2H), 4.58 (td, *J* = 12.7, 6.3 Hz, 4H), 3.44 - 3.37 (m, 1H), 2.72 (d, *J* = 11.4 Hz, 2H), 1.93 - 1.86 (m, 1H), 1.79 - 1.68 (m, 4H), 1.49 - 1.39 (m, 2H); **LRMS** (ES) m/z 515.5 (M⁺ + 1).

### Example 302: Synthesis of Compound 302, 3-((5-(5-(difluoromethyl)-1,3,4-oxadiazole-2-yl)pyridine-2-yl)methyl)-5-fluoro-1-(1-(oxetan-3-yl)az etidine-3-yl)-1,3-dihydro-2H-benzo[d]imidazole-2-one

### [Step 1] Synthesis of tert-butyl 3-((4-fluoro-2-nitrophenyl)amino)azetidine-1-carboxylate

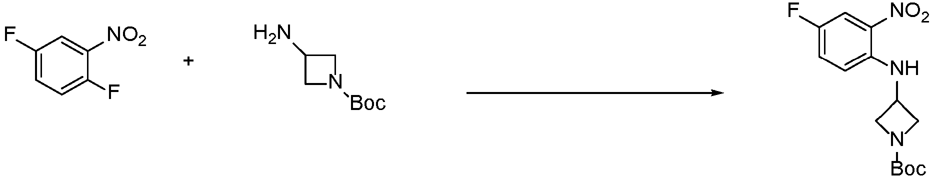

1,4-difluoro-2-nitrobenzene (0.300 g, 1.886 mmol), tert-butyl 3-aminoazetidine-1-carboxylate (0.422 g, 2.451 mmol), potassium carbonate (0.521 g, 3.771 mmol) and potassium iodide (0.031 g, 0.189 mmol) were dissolved in N,N-dimethylformamide (7 mL) at room temperature, after which the resulting solution was stirred at 80°C for 18 hours, and then a reaction was finished by lowering a temperature to room temperature. Water was poured into the reaction mixture and an extraction was performed with ethyl acetate. An organic layer was washed with saturated sodium chloride aqueous solution, then dehydrated with anhydrous magnesium sulfate, then filtered, and then concentrated under reduced pressure. The resulting concentrate was purified via column chromatography (SiO₂, 12 g cartridge; ethyl acetate/hexane = 0 to 30%) and concentrated to obtain a title compound (0.571 g, 97.3%) in an orange solid form.

### [Step 2] Synthesis of tert-butyl 3-((2-amino-4-fluorophenyl)amino)azetidine-1-carboxylate

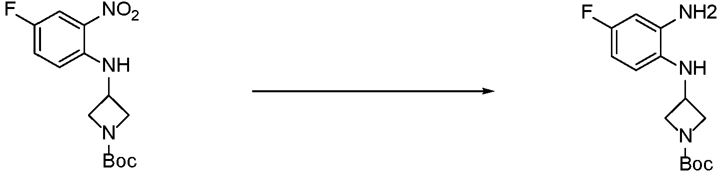

The tert-butyl 3-((4-fluoro-2-nitrophenyl)amino)azetidine-1-carboxylate (0.571 g, 1.834 mmol) prepared in the step 1 was dissolved in methanol (16 mL) and stirred at room temperature, after which 10%-Pd/C (50 mg) was slowly added into the resulting solution at the same temperature and stirred at the same temperature for 18 hours in the presence of a hydrogen balloon attached thereto. The reaction mixture was filtered via a celite pad to remove a solid therefrom, after which solvent was removed from the resulting filtrate under reduced pressure, and then a title compound was used without an additional purification process (0.532 g, 103.2%, brown solid).

### [Step 3] Synthesis of tert-butyl 3-(5-fluoro-2-oxo-2,3-dihydro-1H-benzo[d]imidazole-1-yl)azetidine-1-carboxylate

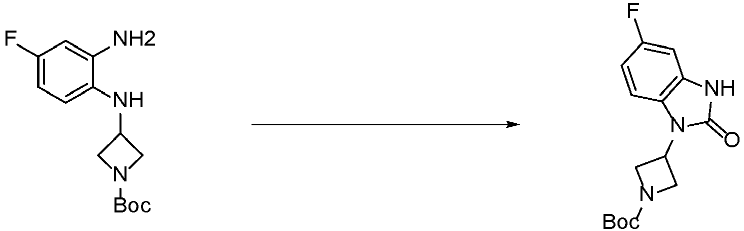

The tert-butyl 3-((2-amino-4-fluorophenyl)amino)azetidine-1-carboxylate (0.532 g, 1.891 mmol) prepared in the step 2, triethylamine (0.264 mL, 1.891 mmol) and 1,1'-carbonyldiimidazole (CDI, 0.368 g, 2.269 mmol) were dissolved in dichloromethane (8 mL) at room temperature, after which the resulting solution was heated under reflux for 18 hours, and then a reaction was finished by lowering a temperature to room temperature. Solvent was removed from the reaction mixture under reduced pressure, after which the resulting concentrate was purified via column chromatography (SiO₂, 12 g cartridge; ethyl acetate/hexane = 0 to 50%) and concentrated to obtain a title compound (0.222 g, 38.3%) in a brown solid form.

### [Step 4] Synthesis 1-(azetidine-3-yl)-3-((5-(5-(difluoromethyl)-1,3,4-oxadiazole-2-yl)pyridine-2-yl)methyl)-5-fluoro-1, 3-dihydro-2H-benzo[d]imidazole-2-one

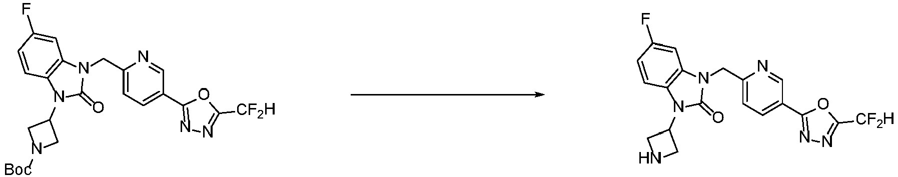

The tert-butyl 3-(3-((5-(5-(difluoromethyl)-1,3,4-oxadiazole-2-yl)pyridine-2-yl)methyl)-5-fluoro-2-oxo-2,3-dihydr o-1H-benzo[d]imidazole-1-yl)azetidine-1-carboxylate (0.320 g, 0.620 mmol) prepared in the step 3 and trifluoroacetic acid (0.474 mL, 6.196 mmol) were dissolved in dichloromethane (3 mL) at room temperature, after which the resulting solution was stirred at the same temperature for 3 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. A title compound was used without an additional purification process (0.250 g, 96.9%, brown solid).

### [Step 5] Synthesis 1-(azetidine-3-yl)-3-((5-(5-(difluoromethyl)-1,3,4-oxadiazole-2-yl)pyridine-2-yl)methyl)-5-fluoro-1, 3-dihydro-2H-benzo[d]imidazole-2-one

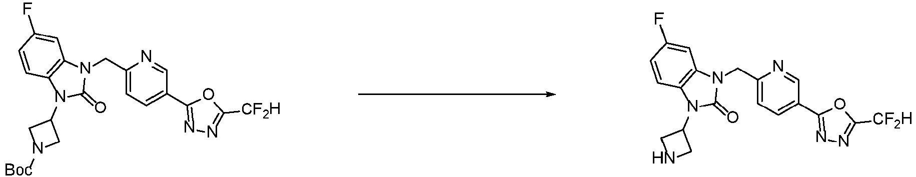

The tert-butyl 3-(3-((5-(5-(difluoromethyl)-1,3,4-oxadiazole-2-yl)pyridine-2-yl)methyl)-5-fluoro-2-oxo-2,3-dihydr o-1H-benzo[d]imidazole-1-yl)azetidine-1-carboxylate (0.320 g, 0.620 mmol) prepared in the step 4 and trifluoroacetic acid (0.474 mL, 6.196 mmol) were dissolved in dichloromethane (3 mL) at room temperature, after which the resulting solution was stirred at the same temperature for 3 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. A title compound was used without an additional purification process (0.250 g, 96.9%, brown solid).

### [Step 6] Synthesis of the compound 302

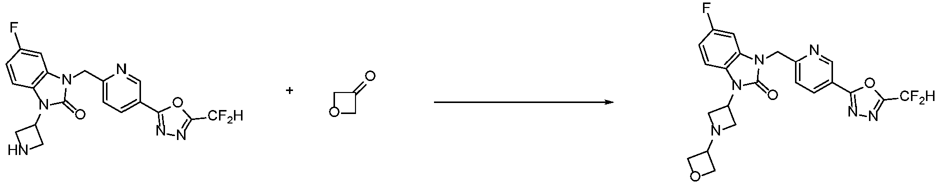

The 1-(azetidine-3-yl)-3-((5-(5-(difluoromethyl)-1,3,4-oxadiazole-2-yl)pyridine-2-yl)methyl)-5-fluoro-1, 3-dihydro-2H-benzo[d]imidazole-2-one (0.250 g, 0.600 mmol) prepared in the step 5, oxetan-3-one (0.130 g, 1.801 mmol) and sodium triacetoxyborohydride (0.255 g, 1.201 mmol) were dissolved in dichloromethane (3 mL) at room temperature, after which the resulting solution was stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.038 g, 13.4%) in a brown solid form.

**¹H NMR** (400 MHz, CDCl₃) δ 9.26 (d, *J =* 1.6 Hz, 1H), 8.33 (dd, *J* = 8.2, 2.2 Hz, 1H), 7.60 (dd, *J* = 8.7, 4.4 Hz, 1H), 7.43 (d, *J =* 8.2 Hz, 1H), 7.05 - 6.76 (m, 3H), 5.22 (s, 2H), 5.14 - 5.07 (m, 1H), 4.76 (t, *J =* 6.7 Hz, 2H), 4.60 (dd, *J* = 6.6, 5.3 Hz, 2H), 3.98 - 3.90 (m, 3H), 3.82 (t, *J =* 8.3 Hz, 2H); **LRMS** (ES) m/z 473.5 (M⁺+ 1).

### Example 303: Synthesis of Compound 303, 1-((5-(5-(difluoromethyl)-1,3,4-oxadiazole-2-yl)pyridine-2-yl)methyl)-6-fluoro-3-methyl-5-(4-(pyrid ine-3-ylmethyl)piperazine-1-yl)-1,3-dihydro-2H-benzo[d]imidazole-2-one

### [Step 1] Synthesis of the compound 303

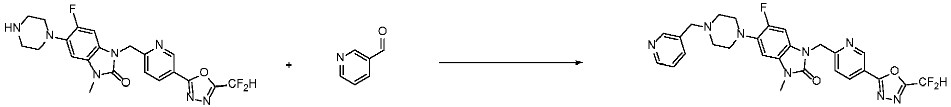

The 1-((5-(5-(difluoromethyl)-1,3,4-oxadiazole-2-yl)pyridine-2-yl)methyl)-6-fluoro-3-methyl-5-(piperazi ne-1-yl)-1,3-dihydro-2H-benzo[d]imidazole-2-one (0.100 g, 0.218 mmol) prepared in the step 4 of the compound 147, nicotinaldehyde (0.070 g, 0.653 mmol) and sodium triacetoxyborohydride(0.092 g, 0.435 mmol) were dissolved in dichloromethane (2 mL) at room temperature, after which the resulting solution was stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.062 g, 51.5%) in a yellow solid form.

**¹H NMR** (400 MHz, CDCl₃) δ 9.23 (dd, *J =* 2.1*,* 0.7 Hz, 1H), 8.54 (d, *J =* 1.6 Hz, 1H), 8.47 (dd, *J* = 4.8, 1.6 Hz, 1H), 8.28 (dd, *J* = 8.2, 2.2 Hz, 1H), 7.66 (td, *J* = 4.8, 2.6 Hz, 1H), 7.38 (dd, *J* = 8.2, 0.4 Hz, 1H), 7.26 - 7.22 (m, 1H), 6.91 (t, *J =* 51.6 Hz, 1H), 6.72 (d, *J =* 11.2 Hz, 1H), 6.62 (d, *J* = 7.0 Hz, 1H), 5.18 (s, 2H), 3.56 (s, 2H), 3.41 (s, 3H), 3.03 (t, *J* = 4.4 Hz, 4H), 2.70 - 2.50 (m, 4H); **LRMS** (ES) m/z 551.4 (M⁺ + 1).

### Example 304: Synthesis of Compound 304, 3-((5-(5-(difluoromethyl)-1,3,4-oxadiazole-2-yl)pyridine-2-yl)methyl)-6-(pyridine-3-yl)benzo[d]oxa zole-2(3H)-one

### [Step 1] Synthesis of 6-bromo-3-((5-(5-(difluoromethyl)-1,3,4-oxadiazole-2-yl)pyridine-2-yl)methyl)benzo[d]oxazole-2( 3H)-one

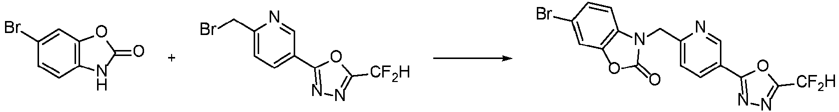

6-bromobenzo[d]oxazole-2(3H)-one (1.000 g, 4.672 mmol), 2-(6-(bromomethyl)pyridine-3-yl)-5-(difluoromethyl)-1,3,4-oxadiazole (1.491 g, 5.140 mmol), potassium carbonate (0.969 g, 7.009 mmol) and potassium iodide (0.078 g, 0.467 mmol) were dissolved in N,N-dimethylformamide (30 mL) at room temperature, after which the resulting solution was stirred at 100°C for 18 hours, and then a reaction was finished by lowering a temperature to room temperature. Solvent was removed from the reaction mixture under reduced pressure, after which saturated sodium hydrogen carbonate aqueous solution was poured into the resulting concentrate, and then an extraction was performed with dichloromethane. An organic layer was washed with saturated sodium chloride aqueous solution, then dehydrated with anhydrous magnesium sulfate, then filtered, and then concentrated under reduced pressure. Methanol was put into the resulting concentrate and stirred, after which a precipitated solid was filtered, then washed with methanol, and then dried to obtain a title compound (1.250 g, 63.2%) in a light yellow solid form.

### [Step 2] Synthesis of the compound 304

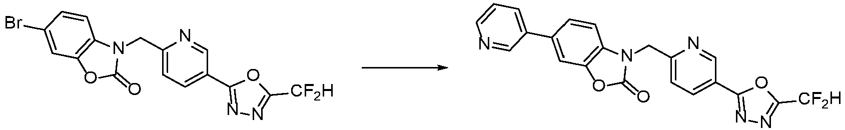

The 6-bromo-3-((5-(5-(difluoromethyl)-1,3,4-oxadiazole-2-yl)pyridine-2-yl)methyl)benzo[d]oxazole-2( 3H)-one (0.100 g, 0.236 mmol) prepared in the step 1, pyridine-3-ylboronic acid (0.035 g, 0.284 mmol), [1,1'-bis(di-tert-butylphosphino)ferrocene]palladium(II) dichloride (Pd(dtbpf)Cl₂, 0.008 g, 0.012 mmol) and cesium carbonate (0.231 g, 0.709 mmol) were mixed in 1,4-dioxane (1.5 mL)/water (0.5 mL) at room temperature, after which the resulting mixture was irradiated with microwave, then heated at 100°C for 20 minutes, and then a reaction was finished by lowering a temperature to room temperature. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; ethyl acetate/hexane = 10 to 40%) and concentrated to obtain a title compound (0.008 g, 8.0%) in a light brown solid form.

**¹H NMR** (400 MHz, CDCl₃) δ 9.33 (d, *J =* 1.4 Hz, 1H), 8.82 (d, *J =* 1.6 Hz, 1H), 8.72 (dd, *J =* 75.5, 1.1 Hz, 1H), 8.43 (dd, *J* = 8.2, 2.2 Hz, 1H), 7.86 - 7.83 (m, 1H), 7.59 (d, *J =* 8.2 Hz, 1H), 7.49 (d, *J =* 1.4 Hz, 1H), 7.41 -7.38 (m, 2H), 7.14 (d, *J =* 8.1 Hz, 1H), 7.08 -6.83 (m, 1H), 5.19 (s, 2H); **LRMS** (ES) m/z 422.4 (M⁺ + 1).

### Example 305: Synthesis of Compound 305, 3-((5-(5-(difluoromethyl)-1,3,4-oxadiazole-2-yl)pyridine-2-yl)methyl)-6-(pyridine-4-yl)benzo[d]oxa zole-2(3H)-one

### [Step 1] Synthesis of the compound 305

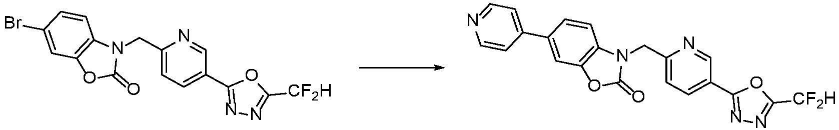

6-bromo-3-((5-(5-(difluoromethyl)-1,3,4-oxadiazole-2-yl)pyridine-2-yl)methyl)benzo[d]ox azole-2(3H)-one (0.100 g, 0.236 mmol), pyridine-4-ylboronic acid (0.035 g, 0.284 mmol), [1,1'-bis(di-tert-butylphosphino)ferrocene]palladium(II) dichloride (Pd(dtbpf)Cl₂, 0.008 g, 0.012 mmol) and cesium carbonate (0.231 g, 0.709 mmol) were mixed in 1,4-dioxane (1.5 mL)/water (0.5 mL) at room temperature, after which the resulting mixture was irradiated with microwave, then heated at 100°C for 20 minutes, and then a reaction was finished by lowering a temperature to room temperature. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; ethyl acetate/hexane = 10 to 40%) and concentrated to obtain a title compound (0.012 g, 12.1%) in a brown solid form.

**¹H NMR** (400 MHz, CDCl₃) δ 9.33 (d, *J =* 2.1 Hz, 1H), 8.68 (d, *J =* 6.2 Hz, 2H), 8.43 (dd, *J* = 8.2, 2.2 Hz, 1H), 7.60 (d, *J* = 8.2 Hz, 1H), 7.55 (d, *J =* 1.4 Hz, 1H), 7.49 - 7.46 (m, 3H), 7.16 (d, *J* = 8.2 Hz, 1H), 7.08 - 6.83 (m, 1H), 5.28 (s, 2H); **LRMS** (ES) m/z 422.4 (M⁺ + 1).

### Example 306: Synthesis of Compound 306, 3-((5-(5-(difluoromethyl)-1,3,4-oxadiazole-2-yl)pyridine-2-yl)methyl)-6-(1H-indole-4-yl)benzo[d]o xazole-2(3H)-one

### [Step 1] Synthesis of the compound 306

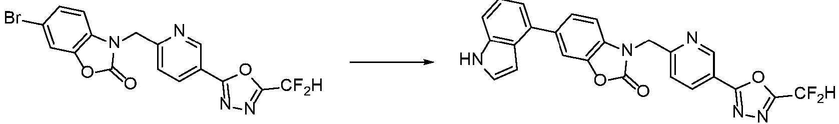

6-bromo-3-((5-(5-(difluoromethyl)-1,3,4-oxadiazole-2-yl)pyridine-2-yl)methyl)benzo[d]ox azole-2(3H)-one (0.100 g, 0.236 mmol), (1H-indole-4-yl)boronic acid (0.046 g, 0.284 mmol), [1,1'-bis(di-tert-butylphosphino)ferrocene]palladium(II) dichloride (Pd(dtbpf)Cl₂, 0.008 g, 0.012 mmol) and cesium carbonate (0.231 g, 0.709 mmol) were mixed in 1,4-dioxane (1.5 mL)/water (0.5 mL) at room temperature, after which the resulting mixture was irradiated with microwave, then heated at 100°C for 20 minutes, and then a reaction was finished by lowering a temperature to room temperature. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; ethyl acetate/hexane = 5 to 20%) and concentrated to obtain a title compound (0.007 g, 6.4%) in a brown solid form.

**¹H NMR** (400 MHz, CDCl₃) δ 9.35 (d, *J =* 1.6 Hz, 1H), 8.43 (dd, *J* = 8.2, 2.2 Hz, 1H), 4.38 (brs, 1H), 7.62 (d, *J =* 1.4 Hz, 1H), 7.59 (d, *J =* 8.2 Hz, 1H), 7.51 (dd, *J =* 8.1, 1.5 Hz, 1H), 7.43 (d, *J =* 8.1 Hz, 1H), 7.31 - 7.27 (m, 3H), 7.16 (d, *J = 7.3* Hz, 1H), 7.11 - 6.83 (m, 2H), 6.70 - 6.68 (m, 1H), 5.30 (s, 2H); **LRMS** (ES) m/z 460.5 (M⁺ + 1).

### Example 307: Synthesis of Compound 307, tert-butyl 4-(3-((5-(5-(difluoromethyl)-1,3,4-oxadiazole-2-yl)pyridine-2-yl)methyl)-2-oxo-2,3-dihydrobenzo[d ]oxazole-6-yl)piperidine-1-carboxylate

### [Step 1] Synthesis of tert-butyl 4-(2-oxo-2,3-dihydrobenzo[d]oxazole-6-yl)piperidine-1-carboxylate

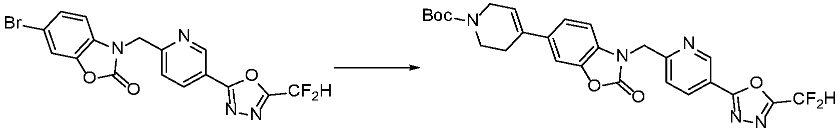

6-bromo-3-((5-(5-(difluoromethyl)-1,3,4-oxadiazole-2-yl)pyridine-2-yl)methyl)benzo[d]ox azole-2(3H)-one (0.800 g, 1.890 mmol), tert-butyl 4-(4,4,5,5-tetramethyl-1,3,2-dioxaborolane-2-yl)-3,6-dihydropyridine-1(2H)-carboxylate (0.701 g, 2.269 mmol), [1,1'-bis(di-tert-butylphosphino)ferrocene]palladium(II) dichloride (Pd(dtbpf)Cl₂, 0.062 g, 0.095 mmol) and cesium carbonate (1.848 g, 5.671 mmol) were mixed in 1,4-dioxane (9 mL)/water (3 mL) at room temperature, after which the resulting mixture was irradiated with microwave, then heated at 100°C for 20 minutes, and then a reaction was finished by lowering a temperature to room temperature. Saturated sodium hydrogen carbonate aqueous solution was poured into the reaction mixture and an extraction was performed with dichloromethane. An organic layer was washed with saturated sodium chloride aqueous solution, then dehydrated with anhydrous magnesium sulfate, then filtered, and then concentrated under reduced pressure. The resulting concentrate was purified via column chromatography (SiO₂, 12 g cartridge; ethyl acetate/hexane = 10 to 30%) and concentrated to obtain a title compound (0.286 g, 28.8%) in a yellow solid form.

### [Step 2] Synthesis of the compound 307

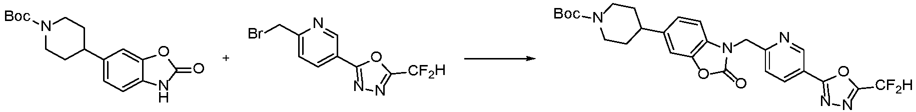

The tert-butyl 4-(2-oxo-2,3-dihydrobenzo[d]oxazole-6-yl)piperidine-1-carboxylate (0.390 g, 1.225 mmol) prepared in the step 1, 2-(6-(bromomethyl)pyridine-3-yl)-5-(difluoromethyl)-1,3,4-oxadiazole (0.391 g, 1.347 mmol), potassium carbonate (0.254 g, 1.837 mmol) and potassium iodide (0.020 g, 0.122 mmol) were dissolved in N,N-dimethylformamide (8 mL) at room temperature, after which the resulting solution was stirred at 100°C for 18 hours, and then a reaction was finished by lowering a temperature to room temperature. Solvent was removed from the reaction mixture under reduced pressure, after which saturated sodium hydrogen carbonate aqueous solution was poured into the resulting concentrate, and then an extraction was performed with ethyl acetate. An organic layer was washed with saturated sodium chloride aqueous solution, then dehydrated with anhydrous magnesium sulfate, then filtered, and then concentrated under reduced pressure. The resulting concentrate was purified via column chromatography (SiO₂, 12 g cartridge; ethyl acetate/hexane = 10 to 40%) and concentrated to obtain a title compound (0.116 g, 18.0%) in a yellow solid form.

**¹H NMR** (400 MHz, CDCl₃) δ 9.32 (d, *J =* 2.2 Hz, 1H), 8.40 (dd, *J =* 8.2, 2.2 Hz, 1H), 7.54 (d, *J = 8.2* Hz, 1H), 7.12 (d, *J =* 1.4 Hz, 1H), 7.08 - 6.82 (m, 3H), 5.22 (s, 2H), 4.26 - 4.25 (m, 2H), 2.81 (t, *J =* 12.0 Hz, 2H), 2.72 - 2.65 (m, 1H), 1.82 (d, *J =* 12.9 Hz, 2H), 1.65 - 1.58 (m, 2H), 1.50 (s, 9H); **LRMS** (ES) m/z 428.4 (M⁺ + 1).

### Example 308: Synthesis of Compound 308, 3-((5-(5-(difluoromethyl)-1,3,4-oxadiazole-2-yl)pyridine-2-yl)methyl)-6-(piperidine-4-yl)benzo[d]o xazole-2(3H)-one

### [Step 1] Synthesis of the compound 308

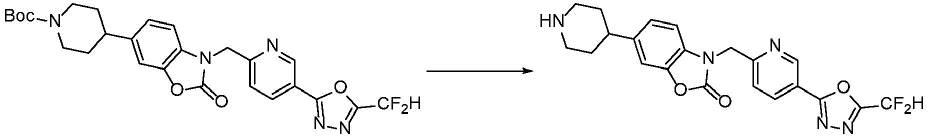

Tert-butyl 4-(3-((5-(5-(difluoromethyl)-1,3,4-oxadiazole-2-yl)pyridine-2-yl)methyl)-2-oxo-2,3-dihydrobenzo[d ]oxazole-6-yl)piperidine-1-carboxylate (0.116 g, 0.220 mmol) and trifluoroacetic acid (0.118 mL, 1.539 mmol) were dissolved in dichloromethane (3 mL) at room temperature, after which the resulting solution was stirred at the same temperature for 2 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. A title compound was obtained without an additional purification process (0.093 g, 99.0%, yellow solid).

**¹H NMR** (400 MHz, CDCl₃) δ 9.31 (d, *J =* 2.3 Hz, 1H), 8.39 (dd, *J* = 8.2, 2.2 Hz, 1H), 7.53 (d, *J =* 8.2 Hz, 1H), 7.15 (d, *J =* 1.4 Hz, 1H), 7.08 -6.82 (m, 3H), 5.22 (s, 2H), 3.24 (d, *J =* 12.0 Hz, 2H), 2.78 (td, *J* = 12.2, 2.3 Hz, 2H), 2.70 - 2.6388 (m, 1H), 1.84 - 1.83 (m, 2H), 1.72 - 1.64 (m, 2H); **LRMS** (ES) m/z 428.5 (M⁺ + 1).

### Example 309: Synthesis of Compound 309, 3-((5-(5-(difluoromethyl)-1,3,4-oxadiazole-2-yl)pyridine-2-yl)methyl)-6-(1-methylpiperidine-4-yl)b enzo[d]oxazole-2(3H)-one

### [Step 1] Synthesis of the compound 309

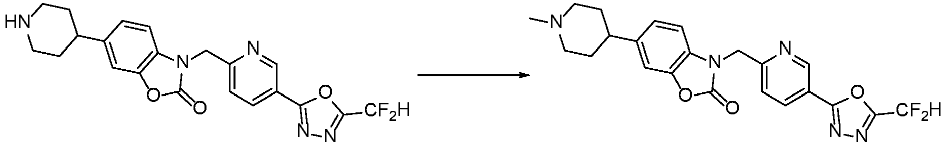

3-((5-(5-(difluoromethyl)-1,3,4-oxadiazole-2-yl)pyridine-2-yl)methyl)-6-(piperidine-4-yl)be nzo[d]oxazole-2(3H)-one (0.090 g, 0.211 mmol) and formaldehyde (37.00% solution, 0.024 mL, 0.316 mmol) were dissolved in dichloromethane (5 mL) at room temperature, after which sodium triacetoxyborohydride (0.089 g, 0.421 mmol) was added into the resulting solution and stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.026 g, 28.0%) in a light yellow oil form.

**¹H NMR** (400 MHz, CDCl₃) δ 9.30 (d, *J =* 1.4 Hz, 1H), 8.38 (dd, *J* = 8.2, 2.2 Hz, 1H), 7.52 (d, *J =* 8.2 Hz, 1H), 7.14 (d, *J =* 1.4 Hz, 1H), 7.08 -6.82 (m, 3H), 5.21 (s, 2H), 2.98 (d, *J =* 11.6 Hz, 2H), 2.53 - 2.48 (m, 1H), 2.32 (s, 3H), 1.84 - 1.75 (m, 2H), 1.47 - 1.25 (m, 4H); **LRMS** (ES) m/z 442.3 (M⁺ + 1).

### Example 310: Synthesis of Compound 310, 1-((5-(5-(difluoromethyl)-1,3,4-oxadiazole-2-yl)pyridine-2-yl)methyl)-6-fluoro-5-(1H-indole-4-yl)-3 -(1-(oxetan-3-yl)azetidine-3-yl)-1,3-dihydro-2H-benzo[d]imidazole-2-one

### [Step 1] Synthesis of 5-bromo-1-((5-(5-(difluoromethyl)-1,3,4-oxadiazole-2-yl)pyridine-2-yl)methyl)-6-fluoro-3-(1-(oxet an-3-yl)azetidine-3-yl)-1,3-dihydro-2H-benzo[d]imidazole-2-one

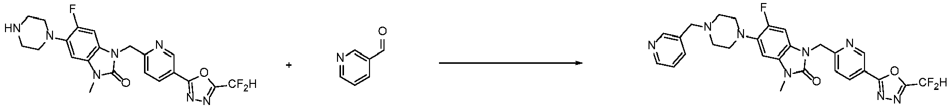

The 1-(azetidine-3-yl)-6-bromo-3-((5-(5-(difluoromethyl)-1,3,4-oxadiazole-2-yl)pyridine-2-yl)methyl)-5 -fluoro-1,3-dihydro-2H-benzo[d]imidazole-2-one (2.390 g, 4.826 mmol) prepared in the step 5 of the compound 257, oxetan-3-one (0.695 g, 9.651 mmol) and sodium triacetoxyborohydride (2.046 g, 9.651 mmol) were dissolved in dichloromethane (45 mL) at room temperature, after which the resulting solution was stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 24 g cartridge; methanol/dichloromethane = 0 to 5%) and concentrated to obtain a title compound (0.530 g, 19.9%) in a white solid form.

### [Step 2] Synthesis of the compound 310

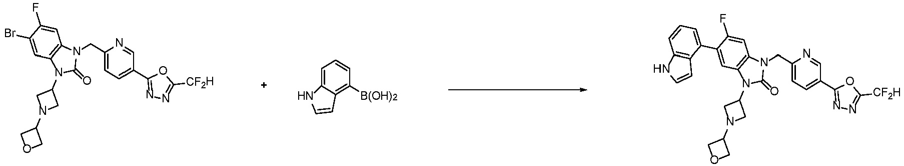

The 5-bromo-1-((5-(5-(difluoromethyl)-1,3,4-oxadiazole-2-yl)pyridine-2-yl)methyl)-6-fluoro-3-(1-(oxet an-3-yl)azetidine-3-yl)-1,3-dihydro-2H-benzo[d]imidazole-2-one (0.150 g, 0.272 mmol) prepared in the step 1, (1H-indole-4-yl)boronic acid (0.053 g, 0.326 mmol), [1,1'-bis(di-tert-butylphosphino)ferrocene]palladium dichloride (0.009 g, 0.014 mmol) and cesium carbonate (0.177 g, 0.544 mmol) were mixed in 1,4-dioxane (2 mL)/water (0.5 mL) at room temperature, after which the resulting mixture was irradiated with microwave, then heated at 100°C for 20 minutes, and then a reaction was finished by lowering a temperature to room temperature. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.115 g, 71.7%) in a brown solid form.

**¹H NMR** (400 MHz, CDCl₃) δ 9.29 (d, *J =* 1.7 Hz, 1H), 8.78 (s, 1H), 8.34 (dd, *J* = 8.2, 2.2 Hz, 1H), 7.79 (d, *J =* 6.0 Hz, 1H), 7.47 (d, *J =* 8.2 Hz, 1H), 7.38 (d, *J =* 8.0 Hz, 1H), 7.26 - 7.16 (m, 3H), 6.92 (t, *J =* 51.6 Hz, 1H), 6.91 (d, *J* = 8.8 Hz, 1H), 6.46 (s, 1H), 5.27 (s, 2H), 5.14 - 5.07 (m, 1H), 4.67 (t, *J =* 6.7 Hz, 2H), 4.56 (t, *J =* 6.0 Hz, 2H), 3.93 - 3.87 (m, 3H), 3.82 (t, *J =* 8.0 Hz, 2H); **LRMS** (ES) m/z 588.7 (M⁺ + 1).

### Example 311: Synthesis of Compound 311, 1-((5-(5-(difluoromethyl)-1,3,4-oxadiazole-2-yl)pyridine-2-yl)methyl)-6-fluoro-3-(1-(oxetan-3-yl)az etidine-3-yl)-5-(pyridine-3-yl)-1,3-dihydro-2H-benzo[d]imidazole-2-one

### [Step 1] Synthesis of the compound 311

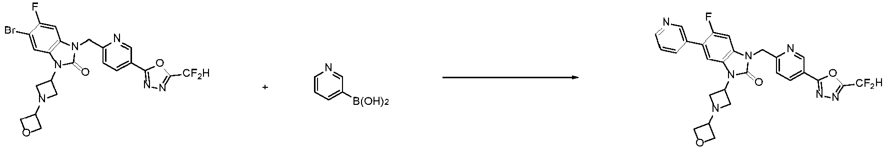

The 5-bromo-1-((5-(5-(difluoromethyl)-1,3,4-oxadiazole-2-yl)pyridine-2-yl)methyl)-6-fluoro-3-(1-(oxet an-3-yl)azetidine-3-yl)-1,3-dihydro-2H-benzo[d]imidazole-2-one (0.150 g, 0.272 mmol) prepared in the step 1 of the compound 310, pyridine-3-ylboronic acid (0.040 g, 0.326 mmol), [1,1'-bis(di-tert-butylphosphino)ferrocene]palladium dichloride (0.007 g, 0.014 mmol) and cesium carbonate (0.177 g, 0.544 mmol) were mixed in 1,4-dioxane (2 mL)/water (0.5 mL) at room temperature, after which the resulting mixture was irradiated with microwave, then heated at 100°C for 20 minutes, and then a reaction was finished by lowering a temperature to room temperature. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 10%) and concentrated to obtain a title compound (0.087 g, 58.4%) in a brown solid form.

**¹H NMR** (400 MHz, CDCl₃) δ 9.25 (d, *J =* 1.6 Hz, 1H), 8.73 (s, 1H), 8.54 (dd, *J* = 4.7, 1.2 Hz, 1H), 8.34 (dd, *J* = 8.2, 2.2 Hz, 1H), 7.82 (dd, *J* = 7.9, 1.6 Hz, 1H), 7.71 (d, *J* = 6.3 Hz, 1H), 7.47 (d, *J =* 8.2 Hz, 1H), 7.34 (dd, *J* = 7.8, 4.9 Hz, 1H), 6.92 (t, *J =* 51.6 Hz, 1H), 6.90 (d, *J =* 10.3 Hz, 1H), 5.23 (s, 2H), 5.12 - 5.05 (m, 1H), 4.71 (t, *J* = 6.7 Hz, 2H), 4.56 (t, *J =* 6.0 Hz, 2H), 3.95 - 3.89 (m, 3H), 3.82 (t, *J =* 8.1 Hz, 2H); **LRMS** (ES) m/z 550.6 (M⁺ + 1).

### Example 312: Synthesis of Compound 312, 1-((5-(5-(difluoromethyl)-1,3,4-oxadiazole-2-yl)pyridine-2-yl)methyl)-6-fluoro-3-(1-(oxetan-3-yl)az etidine-3-yl)-5-(1H-pyrazole-4-yl)-1,3-dihydro-2H-benzo[d]imidazole-2-one

### [Step 1] Synthesis of the compound 312

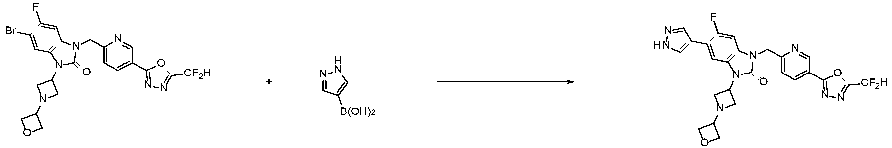

The 5-bromo-1-((5-(5-(difluoromethyl)-1,3,4-oxadiazole-2-yl)pyridine-2-yl)methyl)-6-fluoro-3-(1-(oxet an-3-yl)azetidine-3-yl)-1,3-dihydro-2H-benzo[d]imidazole-2-one (0.150 g, 0.272 mmol) prepared in the step 1 of the compound 310, (1H-pyrazole-4-yl)boronic acid (0.037 g, 0.326 mmol), [1,1'-bis(di-tert-butylphosphino)ferrocene]palladium dichloride (0.009 g, 0.014 mmol) and cesium carbonate (0.177 g, 0.544 mmol) were mixed in 1,4-dioxane (2 mL)/water (0.5 mL) at room temperature, after which the resulting mixture was irradiated with microwave, then heated at 100°C for 20 minutes, and then a reaction was finished by lowering a temperature to room temperature. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.074 g, 50.2%) in a brown solid form.

**¹H NMR** (400 MHz, CH₃OD) δ 9.19 (d, *J* = 1.6 Hz, 1H), 8.46 (dd, *J* = 8.2, 2.2 Hz, 1H), 8.01 (s, 2H), 7.88 (d, *J* = 6.2 Hz, 1H), 7.60 (d, *J =* 8.1 Hz, 1H), 7.22 (t, *J =* 51.6 Hz, 1H), 6.99 (d, *J* = 10.6 Hz, 1H), 5.31 (s, 2H), 5.23 - 5.15 (m, 1H), 4.90 - 4.79 (m, 2H), 4.65 (dd, *J* = 6.9, 4.9 Hz, 2H), 4.18 (t, *J* = 7.7 Hz, 2H), 4.13 - 4.08 (m, 1H), 3.96 (t, *J =* 8.3 Hz, 2H); **LRMS** (ES) m/z 539.5 (M⁺ + 1).

### Example 313: Synthesis of Compound 313, 3-((5-(5-(difluoromethyl)-1,3,4-oxadiazole-2-yl)pyridine-2-yl)methyl)-6-(1-isopropylpiperidine-4-yl )benzo[d]oxazole-2(3H)-one

### [Step 1] Synthesis of the compound 313

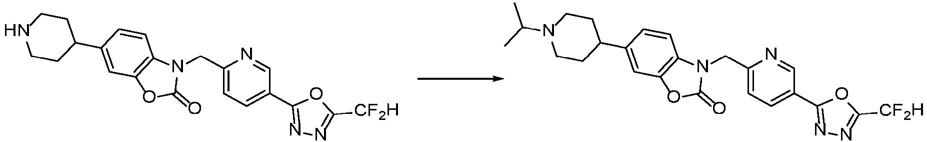

3-((5-(5-(difluoromethyl)-1,3,4-oxadiazole-2-yl)pyridine-2-yl)methyl)-6-(piperidine-4-yl)be nzo[d]oxazole-2(3H)-one (0.100 g, 0.234 mmol) and acetone (0.020 g, 0.351 mmol) were dissolved in dichloromethane (5 mL) at room temperature, after which sodium triacetoxyborohydride (0.099 g, 0.468 mmol) was added into the resulting solution and stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 10%) and concentrated to obtain a title compound (0.036 g, 32.8%) in an orange solid form.

**¹H NMR** (400 MHz, CDCl₃) δ 9.30 (d, *J* = 1.4 Hz, 1H), 8.39 (dd, *J* = 8.2, 2.2 Hz, 1H), 7.52 (d, *J =* 8.2 Hz, 1H), 7.15 (d, *J* = 1.4 Hz, 1H), 7.08 - 6.82 (m, 3H), 5.21 (s, 2H), 3.16 (d, *J =* 11.5 Hz, 2H), 3.03 - 2.99 (m, 1H), 2.60 - 2.54 (m, 1H), 2.40 (td, *J =* 11.5, 3.4 Hz, 2H), 2.03 - 1.87 (m, 4H), 1.16 (d, *J =* 6.6 Hz, 6H); **LRMS** (ES) m/z 470.5 (M⁺ + 1).

### Example 314: Synthesis of Compound 314, 3-((5-(5-(difluoromethyl)-1,3,4-oxadiazole-2-yl)pyridine-2-yl)methyl)-6-(1-(oxetan-3-yl)piperidine-4-yl)benzo[d]oxazole-2(3H)-one

### [Step 1] Synthesis of the compound 314

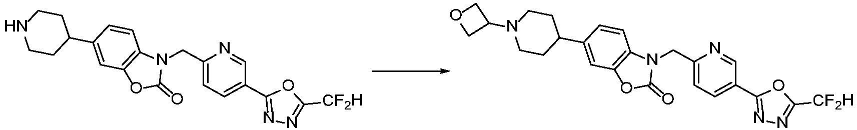

3-((5-(5-(difluoromethyl)-1,3,4-oxadiazole-2-yl)pyridine-2-yl)methyl)-6-(piperidine-4-yl)be nzo[d]oxazole-2(3H)-one (0.100 g, 0.234 mmol) and oxetan-3-one (0.025 g, 0.351 mmol) were dissolved in dichloromethane (5 mL) at room temperature, after which sodium triacetoxyborohydride (0.099 g, 0.468 mmol) was added into the resulting solution and stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 10%) and concentrated, after which an obtained product was purified again via chromatography (SiO₂, 4 g cartridge; ethyl acetate/1%-hexane aqueous solution = 70 to 100%) and concentrated to obtain a title compound (0.062 g, 54.8%) in a yellow solid form.

**¹H NMR** (400 MHz, CDCl₃) δ 9.30 (d, *J =* 1.5 Hz, 1H), 8.39 (dd, *J* = 8.2, 2.2 Hz, 1H), 7.53 (d, *J =* 8.2 Hz, 1H), 7.15 (d, *J =* 1.4 Hz, 1H), 7.08 - 6.82 (m, 3H), 5.21 (s, 2H), 4.70 - 4.64 (m, 4H), 3.53 - 3.50 (m, 1H), 2.88 (d, *J =* 11.3 Hz, 2H), 2.58 - 2.52 (m, 1H), 1.97 - 1.77 (m, 6H); LRMS (ES) m/z 484.5 (M⁺ + 1).

### Example 315: Synthesis of Compound 315, 6-(1-cyclobutylpiperidine-4-yl)-3-((5-(5-(difluoromethyl)-1,3,4-oxadiazole-2-yl)pyridine-2-yl)methy l)benzo[d]oxazole-2(3H)-one

### [Step 1] Synthesis of the compound 315

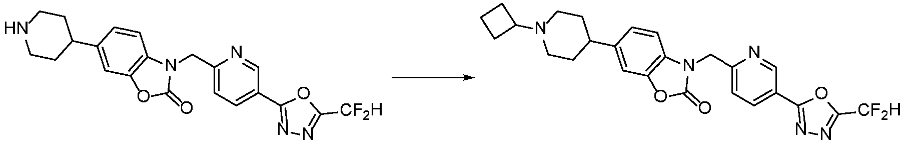

3-((5-(5-(difluoromethyl)-1,3,4-oxadiazole-2-yl)pyridine-2-yl)methyl)-6-(piperidine-4-yl)be nzo[d]oxazole-2(3H)-one (0.100 g, 0.234 mmol) and cyclobutanone (0.025 g, 0.351 mmol) were dissolved in dichloromethane (5 mL) at room temperature, after which sodium triacetoxyborohydride (0.099 g, 0.468 mmol) was added into the resulting solution and stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 10%) and concentrated to obtain a title compound (0.025 g, 22.2%) in a brown solid form.

**¹H NMR** (400 MHz, CDCl₃) δ 9.31 (d, *J =* 1.4 Hz, 1H), 8.39 (dd, *J* = 8.2, 2.2 Hz, 1H), 7.53 (d, *J =* 8.2 Hz, 1H), 7.14 (d, *J* = 1.4 Hz, 1H), 7.08 -6.82 (m, 3H), 5.22 (s, 2H), 3.27 (d, *J* = 10.9 Hz, 2H), 3.01 - 2.97 (m, 1H), 2.65 - 2.57 (m, 2H), 2.26 - 2.22 (m, 2H), 2.18 - 2.10 (m, 3H), 2.02 - 1.98 (m, 2H), 1.91 - 1.88 (m, 2H), 1.79 - 1.77 (m, 1H), 1.76 - 1.71 (m, 1H); **LRMS** (ES) m/z 482.6 (M⁺+ 1).

### Example 316: Synthesis of Compound 316, tert-butyl 4-(3-((5-(5-(difluoromethyl)-1,3,4-oxadiazole-2-yl)pyridine-2-yl)methyl)-5-fluoro-2-oxo-2,3-dihydr obenzo[d]oxazole-6-yl)piperazine-1-carboxylate

### [Step 1] Synthesis of 4,5-difluoro-2-nitrophenol

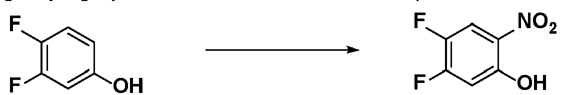

3,4-difluorophenol (7.160 g, 55.039 mmol) and iron (III) nitrate-9H₂O (22.236 g, 55.039 mmol) were mixed in ethanol (50 mL) at room temperature, after which the resulting mixture was heated under reflux for 16 hours and cooled down to room temperature. Then, solvent was removed from the reaction mixture under reduced pressure, after which ethyl acetate (20 mL) and hexane (10 mL) were inserted into the resulting concentrate and stirred to filter out a precipitated solid, then washed with hexane, and then dried to obtain a title compound (7.850 g, 81.5%) in a yellow solid form.

### [Step 2] Synthesis of tert-butyl 4-(2-fluoro-5-hydroxy-4-nitrophenyl)piperazine-1-carboxylate

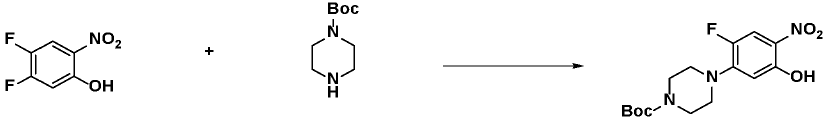

The 4,5-difluoro-2-nitrophenol (7.850 g, 44.834 mmol) prepared in the step 1, tert-butyl piperazine-1-carboxylate (16.702 g, 89.668 mmol) and potassium carbonate (12.393 g, 89.668 mmol) were mixed in toluene (50 mL) at room temperature, after which the resulting mixture was heated under reflux for 18 hours and cooled down to room temperature. Then, water was poured into the reaction mixture and an extraction was performed with ethyl acetate. An organic layer was washed with saturated sodium chloride aqueous solution, then dehydrated with anhydrous sodium sulfate, then filtered, and then concentrated under reduced pressure. Ethyl acetate (20 mL) and hexane (10 mL) were inserted into the resulting concentrate and stirred to filter out a precipitated solid, then washed with hexane, and then dried to obtain a title compound (8.300 g, 54.2%) in a yellow solid form.

### [Step 3] Synthesis of tert-butyl 4-(4-amino-2-fluoro-5-hydroxyphenyl)piperazine-1-carboxylate

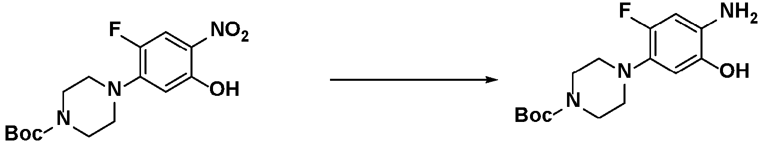

The tert-butyl 4-(2-fluoro-5-hydroxy-4-nitrophenyl)piperazine-1-carboxylate (0.780 g, 2.285 mmol) prepared in the step 2 was dissolved in ethanol (50 mL) at room temperature, after which Raney nickel was slowly added into the resulting solution and stirred at the same temperature for 18 hours in the presence of a hydrogen balloon attached thereto. The reaction mixture was filtered via a celite pad to remove a solid therefrom, after which solvent was removed from the resulting filtrate under reduced pressure, and then a title compound was used without an additional purification process (0.500 g, 70.3%, white solid).

### [Step 4] Synthesis of tert-butyl 4-(5-fluoro-2-oxo-2,3-dihydrobenzo[d]oxazole-6-yl)piperazine-1-carboxylate

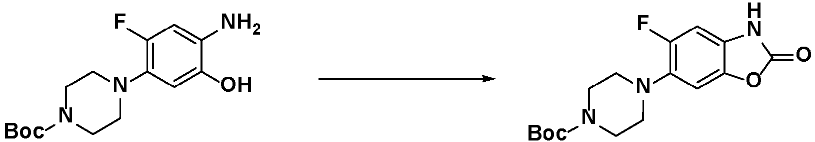

The tert-butyl 4-(4-amino-2-fluoro-5-hydroxyphenyl)piperazine-1-carboxylate (0.500 g, 1.606 mmol) prepared in the step 3 and 1,1'-carbonyldiimidazole (CDI, 0.391 g, 2.409 mmol) were dissolved in tetrahydrofuran (10 mL) at room temperature, after which the resulting solution was stirred at the same temperature for 18 hours. Water was poured into the reaction mixture and an extraction was performed with ethyl acetate. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; ethyl acetate/hexane = 0 to 30%) and concentrated to obtain a title compound (0.205 g, 37.8%) in a white solid form.

### [Step 5] Synthesis of the compound 316

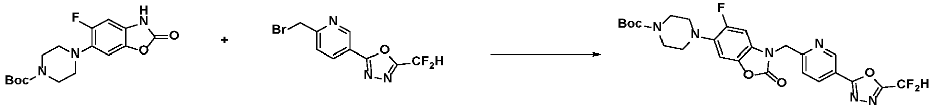

The tert-butyl 4-(5-fluoro-2-oxo-2,3-dihydrobenzo[d]oxazole-6-yl)piperazine-1-carboxylate (0.205 g, 0.608 mmol) prepared in the step 4 was dissolved in N,N-dimethylformamide (10 mL) at 0°C, after which sodium hydride (60.00%, 0.028 g, 0.699 mmol) was added into the resulting solution and stirred at the same temperature for 30 minutes. 2-(6-(bromomethyl)pyridine-3-yl)-5-(difluoromethyl)-1,3,4-oxadiazole (0.212 g, 0.729 mmol) was added into the reaction mixture and further stirred at room temperature for 2 hours. Water was poured into the reaction mixture and an extraction was performed with ethyl acetate. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; ethyl acetate/hexane = 0 to 50%) and concentrated to obtain a title compound (0.200 g, 60.2%) in a yellow solid form.

**¹H NMR** (400 MHz, CDCl₃) δ 9.30 (dd, *J* = 2.2, 0.7 Hz, 1H), 8.41 (dd, J= 8.2, 2.2 Hz, 1H), 7.54 (dd, *J* = 8.2, 0.7 Hz, 1H), 7.08 (s, 0.25H), 6.95 (s, 0.5H), 6.92 (d, *J* = 6.8 Hz, 1H), 6.82 (d, *J* = 10.6 Hz, 1H), 6.82 (s, 0.25H), 5.16 (s, 2H), 3.59 (t, *J =* 5.0 Hz, 4H), 2.96 (t, *J =* 4.8 Hz, 4H), 1.48 (s, 9H).

### Example 317: Synthesis of Compound 317, 3-((5-(5-(difluoromethyl)-1,3,4-oxadiazole-2-yl)pyridine-2-yl)methyl)-5-fluoro-6-(4-methylpiperazi ne-1-yl)benzo[d]oxazole-2(3H)-one

### [Step 1] Synthesis of 3-((5-(5-(difluoromethyl)-1,3,4-oxadiazole-2-yl)pyridine-2-yl)methyl)-5-fluoro-6-(piperazine-1-yl)b enzo[d]oxazole-2(3H)-one

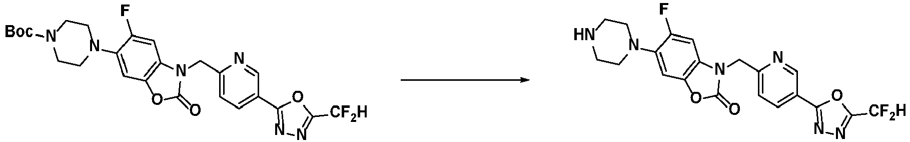

Tert-butyl 4-(3-((5-(5-(difluoromethyl)-1,3,4-oxadiazole-2-yl)pyridine-2-yl)methyl)-5-fluoro-2-oxo-2,3-dihydr obenzo[d]oxazole-6-yl)piperazine-1-carboxylate (0.300 g, 0.549 mmol) and trifluoroacetic acid (0.420 mL, 5.489 mmol) were dissolved in dichloromethane (10 mL) at room temperature, after which the resulting solution was stirred at the same temperature for 18 hours. Solvent was removed from the reaction mixture under reduced pressure, after which saturated sodium hydrogen carbonate aqueous solution was poured into the resulting concentrate, and then an extraction was performed with dichloromethane. An organic layer was washed with saturated sodium chloride aqueous solution, then dehydrated with anhydrous sodium sulfate, then filtered, and then concentrated under reduced pressure. A title compound was used without an additional purification process (0.210 g, 85.7%, yellow solid).

### [Step 2] Synthesis of the compound 317

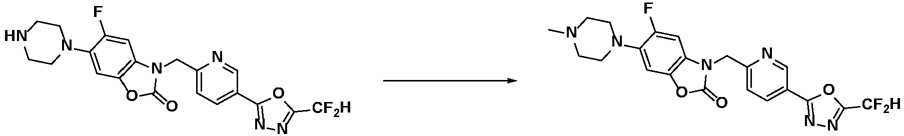

The 3-((5-(5-(difluoromethyl)-1,3,4-oxadiazole-2-yl)pyridine-2-yl)methyl)-5-fluoro-6-(piperazine-1-yl)b enzo[d]oxazole-2(3H)-one (0.110 g, 0.246 mmol) prepared in the step 1, formaldehyde (37.00% solution, 0.037 mL, 0.493 mmol) and sodium triacetoxyborohydride (0.104 g, 0.493 mmol) were dissolved in dichloromethane (10 mL) at room temperature, after which the resulting solution was stirred at the same temperature for 18 hours. Water was poured into the reaction mixture and an extraction was performed with dichloromethane. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; ethyl acetate/hexane = 0 to 30%) and concentrated to obtain a title compound (0.061 g, 53.8%) in a white solid form.

**¹H NMR** (400 MHz, CDCl₃) δ 9.30 (dd, J= 2.2, 0.7 Hz, 1H), 8.41 (dd, *J* = 8.2, 2.2 Hz, 1H), 7.54 (dd, *J* = 8.2, 0.6 Hz, 1H), 7.08 (s, 0.25H), 6.95 (d, *J* = 6.9 Hz, 1H), 6.95 (s, 0.5H), 6.82 (s, 0.25H), 6.80 (d, *J =* 10.7 Hz, 1H), 5.16 (s, 2H), 3.07 (t, *J =* 4.7 Hz, 4H), 2.67 ~ 2.63 (m, 4H), 2.37 (s, 3H).

### Example 318: Synthesis of Compound 318, 3-((5-(5-(difluoromethyl)-1,3,4-oxadiazole-2-yl)pyridine-2-yl)methyl)-5-fluoro-6-(4-(oxetan-3-yl)pi perazine-1-yl)benzo[d]oxazole-2(3H)-one

### [Step 1] Synthesis of the compound 318

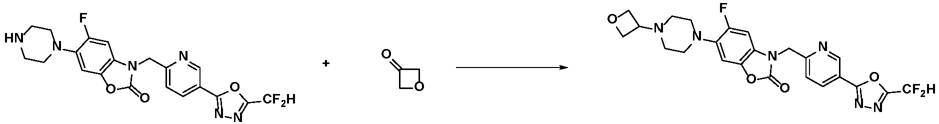

3-((5-(5-(difluoromethyl)-1,3,4-oxadiazole-2-yl)pyridine-2-yl)methyl)-5-fluoro-6-(piperazi ne-1-yl)benzo[d]oxazole-2(3H)-one (0.100 g, 0.224 mmol), 3-oxetane (0.026 mL, 0.448 mmol) and sodium triacetoxyborohydride (0.095 g, 0.448 mmol) were dissolved in dichloromethane (10 mL) at room temperature, after which the resulting solution was stirred at the same temperature for 18 hours. Water was poured into the reaction mixture and an extraction was performed with dichloromethane. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; ethyl acetate/hexane = 0 to 30%) and concentrated to obtain a title compound (0.040 g, 35.5%) in a white solid form.

**¹H NMR** (400 MHz, CDCl₃) δ 9.31 (dd, J= 2.2, 0.7 Hz, 1H), 8.41 (dd, *J* = 8.2, 2.2 Hz, 1H), 7.55 (dd, *J =* 8.2, 0.6 Hz, 1H), 7.08 (s, 0.25H), 6.96 ~ 6.95 (m, 1H), 6.95 (s, 0.5H), 6.82 (s, 0.25H), 6.82 ~ 6.80 (m, 1H), 5.17 (s, 2H), 4.73 ~ 4.64 (m, 4H), 3.62 ~ 3.56 (m, 1H), 3.10 ~ 3.08 (m, 4H), 2.55 ~ 2.52 (m, 4H).

### Example 319: Synthesis of Compound 319, 1-((5-(5-(difluoromethyl)-1,3,4-oxadiazole-2-yl)pyridine-2-yl)methyl)-6-fluoro-5-(4-methylpiperazi ne-1-yl)-3-(oxetan-3-yl)-1,3-dihydro-2H-benzo[d]imidazole-2-one

### [Step 1] Synthesis of tert-butyl 4-(2-fluoro-4-nitro-5-(oxetan-3-ylamino)phenyl)piperazine-1-carboxylate

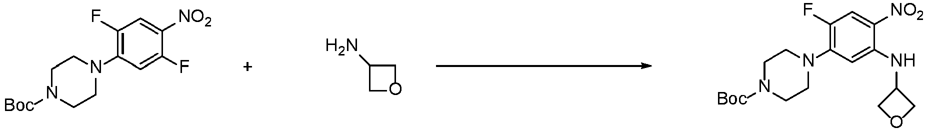

Tert-butyl 4-(2,5-difluoro-4-nitrophenyl)piperazine-1-carboxylate (4.640 g, 13.515 mmol), oxetan-3-amine (1.087 g, 14.866 mmol), potassium carbonate (3.736 g, 27.029 mmol) and potassium iodide (0.224 g, 1.351 mmol) were dissolved in N,N-dimethylformamide (60 mL) at room temperature, after which the resulting solution was stirred at 80°C for 18 hours, and then a reaction was finished by lowering a temperature to room temperature. Water was poured into the reaction mixture and an extraction was performed with ethyl acetate. An organic layer was washed with saturated sodium chloride aqueous solution, then dehydrated with anhydrous magnesium sulfate, then filtered, and then concentrated under reduced pressure. A title compound was used without an additional purification process (5.520 g, 103.0%, yellow solid).

### [Step 2] Synthesis of tert-butyl 4-(4-amino-2-fluoro-5-(oxetan-3-ylamino)phenyl)piperazine-1-carboxylate

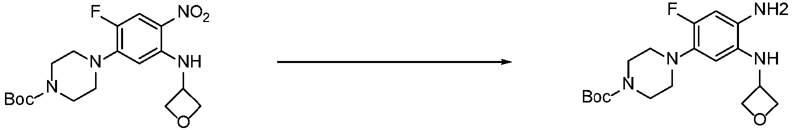

The tert-butyl 4-(2-fluoro-4-nitro-5-(oxetan-3-ylamino)phenyl)piperazine-1-carboxylate (5.520 g, 13.925 mmol) prepared in the step 1 was dissolved in methanol (60 mL) and stirred at room temperature, after which 10%-Pd/C (550 mg) was slowly added into the resulting solution at the same temperature and stirred at the same temperature for 18 hours in the presence of a hydrogen balloon attached thereto. The reaction mixture was filtered via a celite pad to remove a solid therefrom, after which solvent was removed from the resulting filtrate under reduced pressure, and then a title compound was used without an additional purification process (4.870 g, 95.4%, violet solid).

### [Step 3] Synthesis of tert-butyl 4-(6-fluoro-3-(oxetan-3-yl)-2-oxo-2,3-dihydro-1H-benzo[d]imidazole-5-yl)piperazine-1-carboxylat e

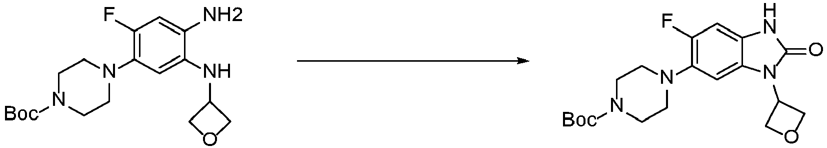

The tert-butyl 4-(4-amino-2-fluoro-5-(oxetan-3-ylamino)phenyl)piperazine-1-carboxylate (4.870 g, 13.290 mmol) prepared in the step 2, triethylamine (1.852 mL, 13.290 mmol) and 1,1'-carbonyldiimidazole (CDI, 2.801 g, 17.277 mmol) were dissolved in dichloromethane (70 mL) at room temperature, after which the resulting solution was heated under reflux for 18 hours, and then a reaction was finished by lowering a temperature to room temperature. Solvent was removed from the reaction mixture under reduced pressure, after which the resulting concentrate was purified via column chromatography (SiO₂, 80 g cartridge; methanol/dichloromethane = 0 to 5%) and concentrated to obtain a title compound (4.710 g, 90.3%) in a violet solid form.

### [Step 4] Synthesis of tert-butyl 4-(1-((5-(5-(difluoromethyl)-1,3,4-oxadiazole-2-yl)pyridine-2-yl)methyl)-6-fluoro-3-(oxetan-3-yl)-2 -oxo-2,3-dihydro-1H-benzo[d]imidazole-5-yl)piperazine-1-carboxylate

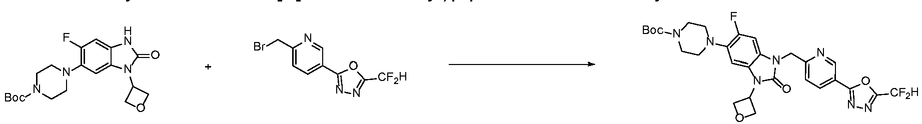

The tert-butyl 4-(6-fluoro-3-(oxetan-3-yl)-2-oxo-2,3-dihydro-1H-benzo[d]imidazole-5-yl)piperazine-1-carboxylat e (2.000 g, 5.096 mmol) prepared in the step 3 was dissolved in N,N-dimethylformamide (25 mL), after which sodium hydride (60.00%, 0.306 g, 7.645 mmol) was added into the resulting solution at 0°C, then stirred at the same temperature for 30 minutes, and then further stirred at room temperature for 4 hours. Water was poured into the reaction mixture and an extraction was performed with ethyl acetate. An organic layer was washed with saturated sodium chloride aqueous solution, then dehydrated with anhydrous magnesium sulfate, then filtered, and then concentrated under reduced pressure. The resulting concentrate was purified via column chromatography (SiO₂, 40 g cartridge; ethyl acetate/hexane = 30 to 70%) and concentrated to obtain a title compound (1.960 g, 63.9%) in a brown solid form.

### [Step 5] Synthesis of 1-((5-(5-(difluoromethyl)-1,3,4-oxadiazole-2-yl)pyridine-2-yl)methyl)-6-fluoro-3-(oxetan-3-yl)-5-(pi perazine-1-yl)-1,3-dihydro-2H-benzo[d]imidazole-2-one

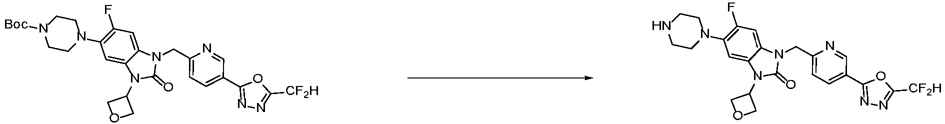

The tert-butyl 4-(1-((5-(5-(difluoromethyl)-1,3,4-oxadiazole-2-yl)pyridine-2-yl)methyl)-6-fluoro-3-(oxetan-3-yl)-2 -oxo-2,3-dihydro-1H-benzo[d]imidazole-5-yl)piperazine-1-carboxylate (1.960 g, 3.258 mmol) prepared in the step 4 and trifluoroacetic acid (2.495 mL, 32.580 mmol) were dissolved in dichloromethane (20 mL) at room temperature, after which the resulting solution was stirred at the same temperature for 5 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 24 g cartridge; methanol/dichloromethane = 0 to 30%) and concentrated to obtain a title compound (1.000 g, 61.2%) in a brown solid form.

### [Step 6] Synthesis of the compound 319

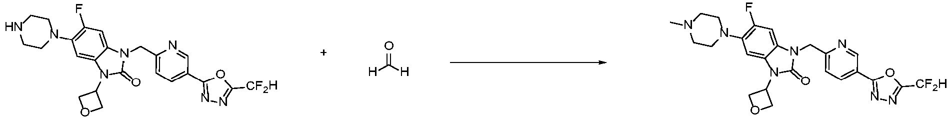

The 1-((5-(5-(difluoromethyl)-1,3,4-oxadiazole-2-yl)pyridine-2-yl)methyl)-6-fluoro-3-(oxetan-3-yl)-5-(pi perazine-1-yl)-1,3-dihydro-2H-benzo[d]imidazole-2-one (0.100 g, 0.199 mmol) prepared in the step 5, sodium triacetoxyborohydride (0.085 g, 0.399 mmol) and formaldehyde (0.018 g, 0.598 mmol) were dissolved in dichloromethane (2 mL) at room temperature, after which the resulting solution was stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.077 g, 75.0%) in a light yellow solid form.

**¹H NMR** (400 MHz, CDCl₃) δ 9.27 (d, *J* = 1.6 Hz, 1H), 8.33 (dd, *J* = 8.2, 2.2 Hz, 1H), 7.41 (dd, *J =* 12.0, 7.7 Hz, 2H), 6.92 (t, *J =* 51.6 Hz, 1H), 6.81 (d, *J =* 11.2 Hz, 1H), 5.66 - 5.60 (m, 1H), 5.19 (s, 2H), 5.14 (d, *J* = 6.7 Hz, 4H), 3.11 (t, *J* = 4.4 Hz, 4H), 2.70 - 2.50 (m, 4H), 2.36 (s, 3H); **LRMS** (ES) m/z 516.3 (M⁺+ 1).

### Example 320: Synthesis of Compound 320, 5-(4-cyclobutylpiperazine-1-yl)-1-((5-(5-(difluoromethyl)-1,3,4-oxadiazole-2-yl)pyridine-2-yl)meth yl)-6-fluoro-3-(oxetan-3-yl)-1,3-dihydro-2H-benzo[d]imidazole-2-one

### [Step 1] Synthesis of the compound 320

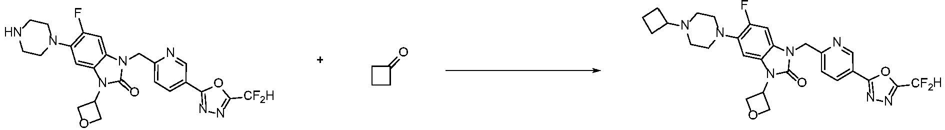

The 1-((5-(5-(difluoromethyl)-1,3,4-oxadiazole-2-yl)pyridine-2-yl)methyl)-6-fluoro-3-(oxetan-3-yl)-5-(pi perazine-1-yl)-1,3-dihydro-2H-benzo[d]imidazole-2-one (0.100 g, 0.199 mmol) prepared in the step 5 of the compound 319, sodium triacetoxyborohydride (0.085 g, 0.399 mmol) and cyclobutanone (0.042 g, 0.598 mmol) were dissolved in dichloromethane (2 mL) at room temperature, after which the resulting solution was stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.073 g, 66.3%) in a light yellow solid form.

**¹H NMR** (400 MHz, CDCl₃) δ 9.27 (dd, *J* = 2.1, 0.6 Hz, 1H), 8.32 (dd, *J* = 8.2, 2.2 Hz, 1H), 7.42 (d, *J* = 7.5 Hz, 2H), 6.92 (t, *J =* 51.6 Hz, 1H), 6.81 (d, *J =* 11.2 Hz, 1H), 5.66 - 5.59 (m, 1H), 5.19 (s, 2H), 5.19 - 5.10 (m, 4H), 3.11 (t, *J* = 4.5 Hz, 4H), 2.87 - 2.79 (m, 1H), 2.70 - 2.45 (m, 4H), 2.16 - 2.02 (m, 2H), 1.98 - 1.89 (m, 2H), 1.77 - 1.65 (m, 2H); **LRMS** (ES) m/z 556.3 (M⁺ + 1).

### Example 321: Synthesis of Compound 321, 1-((5-(5-(difluoromethyl)-1,3,4-oxadiazole-2-yl)pyridine-2-yl)methyl)-6-fluoro-3-(oxetan-3-yl)-5-(4 -(oxetan-3-yl)piperazine-1-yl)-1,3-dihydro-2H-benzo[d]imidazole-2-one

### [Step 1] Synthesis of the compound 321

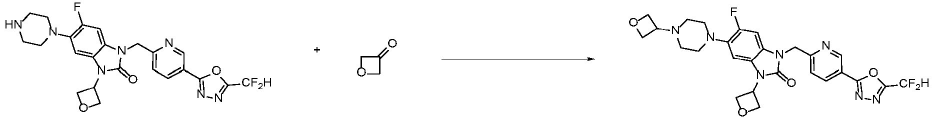

The 1-((5-(5-(difluoromethyl)-1,3,4-oxadiazole-2-yl)pyridine-2-yl)methyl)-6-fluoro-3-(oxetan-3-yl)-5-(pi perazine-1-yl)-1,3-dihydro-2H-benzo[d]imidazole-2-one (0.100 g, 0.199 mmol) prepared in the step 5 of the compound 319, sodium triacetoxyborohydride (0.085 g, 0.399 mmol) and oxetan-3-one (0.043 g, 0.598 mmol) were dissolved in dichloromethane (2 mL) at room temperature, after which the resulting solution was stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.081 g, 72.5%) in a light yellow solid form.

**¹H NMR** (400 MHz, CDCl₃) δ 9.26 (d, *J =* 2.1 Hz, 1H), 8.33 (dd, *J* = 8.2, 2.2 Hz, 1H), 7.43 (d, *J* = 7.4 Hz, 2H), 6.92 (t, J= 51.6 Hz, 1H), 6.83 (d, *J =* 11.2 Hz, 1H), 5.65 - 5.60 (m, 1H), 5.19 (s, 2H), 5.14 (td, *J* = 9.8, 5.3 Hz, 4H), 4.66 (td, *J =* 12.1, 5.9 Hz, 4H), 3.61 - 3.55 (m, 1H), 3.13 (t, *J* = 4.6 Hz, 4H), 2.70 - 2.50 (m, 4H); **LRMS** (ES) m/z 558.3 (M⁺+ 1).

### Example 322: Synthesis of Compound 322, 5-(4-((1H-indole-4-yl)methyl)piperazine-1-yl)-1-((5-(5-(difluoromethyl)-1,3,4-oxadiazole-2-yl)pyrid ine-2-yl)methyl)-6-fluoro-3-(oxetan-3-yl)-1,3-dihydro-2H-benzo[d]imidazole-2-one

### [Step 1] Synthesis of the compound 322

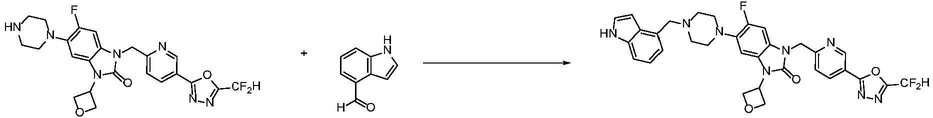

The 1-((5-(5-(difluoromethyl)-1,3,4-oxadiazole-2-yl)pyridine-2-yl)methyl)-6-fluoro-3-(oxetan-3-yl)-5-(pi perazine-1-yl)-1,3-dihydro-2H-benzo[d]imidazole-2-one (0.100 g, 0.199 mmol) prepared in the step 5 of the compound 319, sodium triacetoxyborohydride (0.085 g, 0.399 mmol) and 1H-indole-4-carbaldehyde (0.087 g, 0.598 mmol) were dissolved in dichloromethane (2 mL) at room temperature, after which the resulting solution was stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.078 g, 61.9%) in a light yellow solid form.

**¹H NMR** (400 MHz, CDCl₃) δ 9.27 (d, *J =* 1.6 Hz, 1H), 8.32 (dd, *J* = 8.1, 2.2 Hz, 2H), 7.40 (t, *J* = 8.3 Hz, 2H), 7.31 (d, *J* = 7.8 Hz, 1H), 7.21 (t, *J* = 2.8 Hz, 1H), 7.17 - 7.10 (m, 2H), 6.92 (t, *J* = 51.6 Hz, 1H), 6.80 (d, *J =* 11.2 Hz, 1H), 6.74 - 6.73 (m, 1H), 5.66 - 5.59 (m, 1H), 5.19 (s, 2H), 5.12 (d, *J* = 6.8 Hz, 4H), 3.88 (s, 2H), 3.10 (t, *J* = 4.3 Hz, 4H), 2.80 - 2.60 (m, 4H); **LRMS** (ES) m/z 632.4 (M⁺ + 1).

### Example 323: Synthesis of Compound 323, 5-(4-((1H-pyrazole-4-yl)methyl)piperazine-1-yl)-1-((5-(5-(difluoromethyl)-1,3,4-oxadiazole-2-yl)py ridine-2-yl)methyl)-6-fluoro-3-(oxetan-3-yl)-1,3-dihydro-2H-benzo[d]imidazole-2-one

### [Step 1] Synthesis of the compound 323

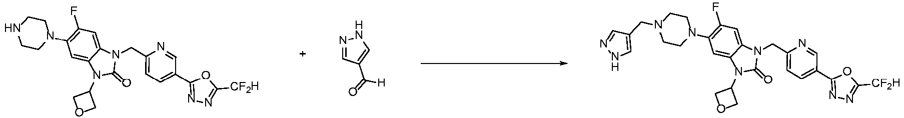

The 1-((5-(5-(difluoromethyl)-1,3,4-oxadiazole-2-yl)pyridine-2-yl)methyl)-6-fluoro-3-(oxetan-3-yl)-5-(pi perazine-1-yl)-1,3-dihydro-2H-benzo[d]imidazole-2-one (0.100 g, 0.199 mmol) prepared in the step 5 of the compound 319, sodium triacetoxyborohydride (0.085 g, 0.399 mmol) and 1H-pyrazole-4-carbaldehyde (0.057 g, 0.598 mmol) were dissolved in dichloromethane (2 mL) at room temperature, after which the resulting solution was stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 10%) and concentrated to obtain a title compound (0.094 g, 80.7%) in a light yellow solid form.

**¹H NMR** (400 MHz, CDCl₃) δ 9.26 (s, 1H), 8.32 (dd, *J* = 8.2, 1.8 Hz, 1H) 7.54 (s, 2H), 7.43 - 7.39 (m, 2H), 6.92 (t, *J =* 50.8 Hz, 1H), 6.81 (d, *J =* 11.3 Hz, 1H), 5.66 - 5.59 (m, 1H), 5.19 - 5.12 (m, 6H), 3.56 (s, 2H), 3.20 - 3.00 (m, 4H), 2.80 - 2.50 (m, 4H); **LRMS** (ES) m/z 582.3 (M⁺+ 1).

### Example 324: Synthesis of Compound 324, 1-((5-(5-(difluoromethyl)-1,3,4-oxadiazole-2-yl)pyridine-2-yl)methyl)-6-fluoro-3-(oxetan-3-yl)-5-(4 -(pyridine-3-ylmethyl)piperazine-1-yl)-1,3-dihydro-2H-benzo[d]imidazole-2-one

### [Step 1] Synthesis of the compound 324

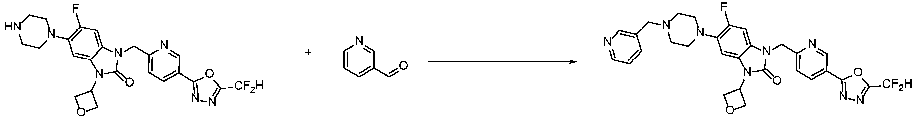

The 1-((5-(5-(difluoromethyl)-1,3,4-oxadiazole-2-yl)pyridine-2-yl)methyl)-6-fluoro-3-(oxetan-3-yl)-5-(pi perazine-1-yl)-1,3-dihydro-2H-benzo[d]imidazole-2-one (0.100 g, 0.199 mmol) prepared in the step 5 of the compound 319, sodium triacetoxyborohydride (0.085 g, 0.399 mmol) and nicotinaldehyde (0.064 g, 0.598 mmol) were dissolved in dichloromethane (2 mL) at room temperature, after which the resulting solution was stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.085 g, 72.0%) in a light yellow solid form.

**¹H NMR** (400 MHz, CDCl₃) δ 9.26 (d, *J =* 1.6 Hz, 1H), 8.55 (d, *J =* 1.5 Hz, 1H), 8.50 (dd, *J* = 4.7, 1.4 Hz, 1H), 8.32 (dd, *J* = 8.2, 2.2 Hz, 1H), 7.72 (d, *J* = 7.8 Hz, 1H), 7.42 (t, *J =* 7.2 Hz, 2H), 7.26 (dd, *J* = 8.1, 4.5 Hz, 1H), 6.92 (t, *J* = 51.6 Hz, 1H), 6.82 (d, *J =* 11.2 Hz, 1H), 5.66 - 5.59 (m, 1H), 5.19 (s, 2H), 5.16 - 5.10 (m, 4H), 3.60 (s, 2H), 3.10 (t, *J* = 4.4 Hz, 4H), 2.80 - 2.60 (m, 4H); **LRMS** (ES) m/z 593.6 (M⁺ + 1).

### Example 325: Synthesis of Compound 325, 1-((5-(5-(difluoromethyl)-1,3,4-oxadiazole-2-yl)pyridine-2-yl)methyl)-6-fluoro-3-(oxetan-3-yl)-5-(4 -(pyridine-3-ylmethyl)piperazine-1-yl)-1,3-dihydro-2H-benzo[d]imidazole-2-one

### [Step 1] Synthesis of the compound 325

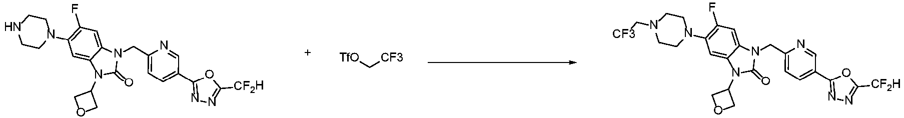

The 1-((5-(5-(difluoromethyl)-1,3,4-oxadiazole-2-yl)pyridine-2-yl)methyl)-6-fluoro-3-(oxetan-3-yl)-5-(pi perazine-1-yl)-1,3-dihydro-2H-benzo[d]imidazole-2-one (0.100 g, 0.199 mmol) prepared in the step 5 of the compound 319, triethylamine (0.056 mL, 0.399 mmol) and 2,2,2-trifluoroethyl trifluoromethanesulfonate (0.093 g, 0.399 mmol) were dissolved in dichloromethane (2 mL) at room temperature, after which the resulting solution was stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.102 g, 87.3%) in a light yellow solid form.

**¹H NMR** (400 MHz, CDCl₃) δ 9.28 (d, *J =* 1.6 Hz, 1H), 8.34 (dd, *J* = 8.2, 2.2 Hz, 1H), 7.44 (d, *J* = 8.2 Hz, 2H), 6.92 (t, *J* = 51.6 Hz, 1H), 6.83 (d, *J =* 11.2 Hz, 1H), 5.67 - 5.60 (m, 1H), 5.20 (s, 2H), 5.18 - 5.11 (m, 4H), 3.12 (t, *J =* 4.6 Hz, 4H), 3.04 (q, *J =* 9.5 Hz, 2H), 2.88 (t, *J =* 4.5 Hz, 4H); **LRMS** (ES) m/z 584.2 (M⁺+ 1).

### Example 326: Synthesis of Compound 326, 1-((5-(5-(difluoromethyl)-1,3,4-oxadiazole-2-yl)pyridine-2-yl)methyl)-3-(2-(dimethylamino)ethyl)-6 -fluoro-5-(pyridine-3-yl)-1,3-dihydro-2H-benzo[d]imidazole-2-one

### [Step 1] Synthesis of the compound 326

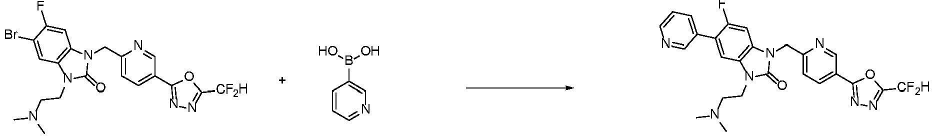

5-bromo-1-((5-(5-(difluoromethyl)-1,3,4-oxadiazole-2-yl)pyridine-2-yl)methyl)-3-(2-(dimet hylamino)ethyl)-6-fluoro-1,3-dihydro-2H-benzo[d]imidazole-2-one (0.150 g, 0.293 mmol), pyridine-3-ylboronic acid (0.054 g, 0.440 mmol), [1,1'-bis(di-tert-butylphosphino)ferrocene]palladium(II) dichloride (Pd(dtbpf)Cl₂, 0.019 g, 0.029 mmol) and cesium carbonate (0.239 g, 0.733 mmol) were mixed in 1,4-dioxane (9 mL)/water (3 mL), after which the resulting mixture was irradiated with microwave, then heated at 100°C for 20 minutes, and then a reaction was finished by lowering a temperature to room temperature. Water was poured into the reaction mixture and an extraction was performed with ethyl acetate. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; methanol/dichloromethane = 0 to 10%) and concentrated to obtain a title compound (0.060 g, 40.1%) in a brown oil form.

**¹H NMR** (400 MHz, CDCl₃) δ 9.32 (d, *J =* 1.6 Hz, 1H), 8.78 (s, 1H), 8.62 ~ 8.61 (m, 1H), 8.39 (dd, *J* = 8.2, 2.2 Hz, 1H), 7.88 ~ 7.86 (m, 1H), 7.50 (d, *J* = 8.2 Hz, 1H), 7.40 (dd, *J* = 7.9, 4.9 Hz, 1H), 7.13 ~ 7.11 (m, 1H), 7.08 (s, 0.25H), 6.95 (s, 0.5H), 6.91 (d, *J* = 9.8 Hz, 1H), 6.82 (s, 0.25H), 5.31 (s, 2H), 4.15 ~ 4.11 (m, 2H), 2.79 ~ 2.75 (m, 2H), 2.40 (s, 6H).

### Example 327: Synthesis of Compound 327, 1-((5-(5-(difluoromethyl)-1,3,4-oxadiazole-2-yl)pyridine-2-yl)methyl)-3-(2-(dimethylamino)ethyl)-6 -fluoro-5-(pyridine-4-yl)-1,3-dihydro-2H-benzo[d]imidazole-2-one

### [Step 1] Synthesis of the compound 327

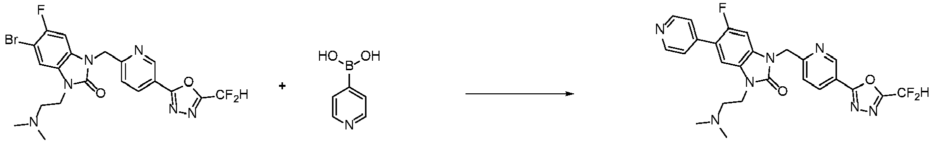

5-bromo-1-((5-(5-(difluoromethyl)-1,3,4-oxadiazole-2-yl)pyridine-2-yl)methyl)-3-(2-(dimet hylamino)ethyl)-6-fluoro-1,3-dihydro-2H-benzo[d]imidazole-2-one (0.150 g, 0.293 mmol), pyridine-4-ylboronic acid (0.054 g, 0.440 mmol), [1,1'-bis(di-tert-butylphosphino)ferrocene]palladium(II) dichloride (Pd(dtbpf)Cl₂, 0.019 g, 0.029 mmol) and cesium carbonate (0.239 g, 0.733 mmol) were mixed in 1,4-dioxane (9 mL)/water (3 mL), after which the resulting mixture was irradiated with microwave, then heated at 100°C for 20 minutes, and then a reaction was finished by lowering a temperature to room temperature. Water was poured into the reaction mixture and an extraction was performed with ethyl acetate. An organic layer was washed with saturated sodium chloride aqueous solution, then dehydrated with anhydrous sodium sulfate, then filtered, and then concentrated under reduced pressure. The resulting concentrate was purified via column chromatography (SiO₂, 12 g cartridge; methanol/dichloromethane = 0 to 10%) and concentrated to obtain a title compound (0.050 g, 33.5%) in a brown oil form.

**¹H NMR** (400 MHz, CDCl₃) δ 9.31 (s, 1H), 8.69 (d, *J =* 4.9 Hz, 2H), 8.39 (dd, *J* = 8.2, 1.8 Hz, 1H), 7.51 ~ 7.46 (m, 3H), 7.14 (d, *J* = 6.0 Hz, 1H), 7.08 (s, 0.25H), 6.95 (s, 0.5H), 6.91 (d, *J =* 10.1 Hz, 1H), 6.82 (s, 0.25H), 5.30 (s, 2H), 4.11 (t, *J =* 6.6 Hz, 2H), 2.74 (t, *J =* 6.5 Hz, 2H), 2.37 (s, 6H).

### Example 328: Synthesis of Compound 328, 1-((5-(5-(difluoromethyl)-1,3,4-oxadiazole-2-yl)pyridine-2-yl)methyl)-6-fluoro-5-(4-methylpiperazi ne-1-yl)-3-(pyridine-3-yl)-1,3-dihydro-2H-benzo[d]imidazole-2-one

### [Step 1] Synthesis of tert-butyl 4-(2-fluoro-4-nitro-5-(pyridine-3-ylamino)phenyl)piperazine-1-carboxylate

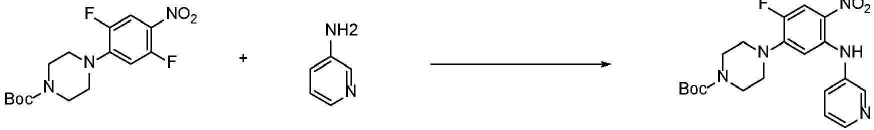

Pyridine-3-amine (0.663 g, 7.049 mmol) was dissolved in tetrahydrofuran (40 mL), after which lithium bis(trimethylsilyl)amide (26.00%, 4.536 g, 7.049 mmol) was slowly added into the resulting solution and stirred while maintaining a temperature at 0°C and then tert-butyl 4-(2,5-difluoro-4-nitrophenyl)piperazine-1-carboxylate (2.200 g, 6.408 mmol) was added thereinto and stirred at room temperature for 18 hours. Water was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 24 g cartridge; ethyl acetate/hexane = 0 to 100%) and concentrated to obtain a title compound (0.504 g, 18.8%) in a red solid form.

### [Step 2] Synthesis of tert-butyl 4-(4-amino-2-fluoro-5-(pyridine-3-ylamino)phenyl)piperazine-1-carboxylate

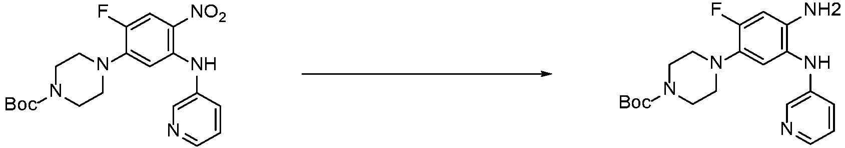

The tert-butyl 4-(2-fluoro-4-nitro-5-(pyridine-3-ylamino)phenyl)piperazine-1-carboxylate (0.504 g, 1.207 mmol) prepared in the step 1 was dissolved in methanol (5 mL)/tetrahydrofuran (2 mL) and stirred at room temperature, after which 10%-Pd/C (50 mg) was slowly added into the resulting solution at the same temperature and stirred at the same temperature for 18 hours in the presence of a hydrogen balloon attached thereto. The reaction mixture was filtered via a celite pad to remove a solid therefrom, after which solvent was removed from the resulting filtrate under reduced pressure, and then a title compound was used without an additional purification process (0.460 g, 98.3%, brown solid).

### [Step 3] Synthesis of tert-butyl 4-(6-fluoro-2-oxo-3-(pyridine-3-yl)-2,3-dihydro-1H-benzo[d]imidazole-5-yl)piperazine-1-carboxyla te

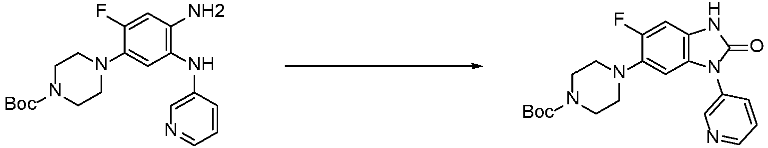

The tert-butyl 4-(4-amino-2-fluoro-5-(pyridine-3-ylamino)phenyl)piperazine-1-carboxylate (0.430 g, 1.110 mmol) prepared in the step 2, triethylamine (0.155 mL, 1.110 mmol) and 1,1'-carbonyldiimidazole (CDI, 0.216 g, 1.332 mmol) were dissolved in dichloromethane (6 mL) at room temperature, after which the resulting solution was heated under reflux for 18 hours, and then a reaction was finished by lowering a temperature to room temperature. Solvent was removed from the reaction mixture under reduced pressure, after which the resulting concentrate was purified via column chromatography (SiO₂, 12 g cartridge; ethyl acetate/hexane = 0 to 30%) and concentrated to obtain a title compound (0.449 g, 97.9%) in a brown solid form.

### [Step 4] Synthesis of tert-butyl 4-(1-((5-(5-(difluoromethyl)-1,3,4-oxadiazole-2-yl)pyridine-2-yl)methyl)-6-fluoro-2-oxo-3-(pyridine -3-yl)-2,3-dihydro-1H-benzo[d]imidazole-5-yl)piperazine-1-carboxylate

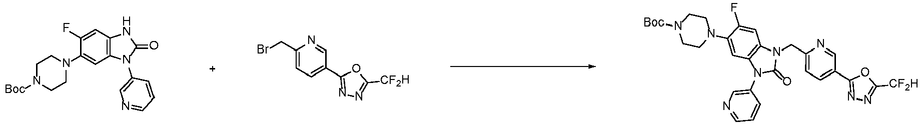

The tert-butyl 4-(6-fluoro-2-oxo-3-(pyridine-3-yl)-2,3-dihydro-1H-benzo[d]imidazole-5-yl)piperazine-1-carboxyla te (0.440 g, 1.064 mmol) prepared in the step 3 was dissolved in N,N-dimethylformamide (6 mL), after which sodium hydride (60.00%, 0.064 g, 1.596 mmol) was slowly added into the resulting solution and stirred for 30 minutes while maintaining a temperature at 0°C. Then, 2-(6-(bromomethyl)pyridine-3-yl)-5-(difluoromethyl)-1,3,4-oxadiazole (0.340 g, 1.171 mmol) was added into the resulting mixture and further stirred at room temperature for 4 hours. Water was poured into the reaction mixture and an extraction was performed with ethyl acetate. An organic layer was washed with saturated sodium chloride aqueous solution, then dehydrated with anhydrous magnesium sulfate, then filtered, and then concentrated under reduced pressure. The resulting concentrate was purified via column chromatography (SiO₂, 12 g cartridge; ethyl acetate/hexane = 0 to 100%) and concentrated to obtain a title compound (0.490 g, 74.0%) in a brown solid form.

### [Step 5] Synthesis of 1-((5-(5-(difluoromethyl)-1,3,4-oxadiazole-2-yl)pyridine-2-yl)methyl)-6-fluoro-5-(piperazine-1-yl)-3-(pyridine-3-yl)-1,3-dihydro-2H-benzo[d]imidazole-2-one

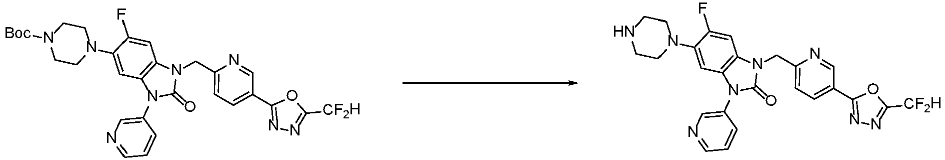

The tert-butyl 4-(1-((5-(5-(difluoromethyl)-1,3,4-oxadiazole-2-yl)pyridine-2-yl)methyl)-6-fluoro-2-oxo-3-(pyridine -3-yl)-2,3-dihydro-1H-benzo[d]imidazole-5-yl)piperazine-1-carboxylate (0.490 g, 0.787 mmol) prepared in the step 4 and trifluoroacetic acid (0.603 mL, 7.870 mmol) were dissolved in dichloromethane (5 mL) at room temperature, after which the resulting solution was stirred at the same temperature for 5 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 12 g cartridge; methanol/dichloromethane = 0 to 60%) and concentrated to obtain a title compound (0.267 g, 64.9%) in a brown solid form.

### [Step 6] Synthesis of the compound 328

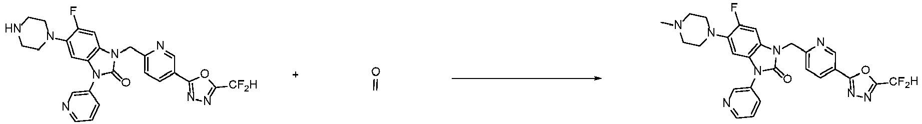

The 1-((5-(5-(difluoromethyl)-1,3,4-oxadiazole-2-yl)pyridine-2-yl)methyl)-6-fluoro-5-(piperazine-1-yl)-3-(pyridine-3-yl)-1,3-dihydro-2H-benzo[d]imidazole-2-one (0.130 g, 0.249 mmol) prepared in the step 5, sodium triacetoxyborohydride (0.105 g, 0.498 mmol) and formaldehyde (37.00%, 0.061 g, 0.746 mmol) were dissolved in dichloromethane (2 mL) at room temperature, after which the resulting solution was stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.086 g, 64.5%) in a brown solid form.

**¹H NMR** (400 MHz, CDCl₃) δ 9.28 (d, *J* = 1.6 Hz, 1H), 8.84 (d, *J* = 2.3 Hz, 1H), 8.65 (dd, *J* = 4.7, 1.3 Hz, 1H), 8.35 (dd, *J* = 8.2, 2.2 Hz, 1H), 7.93 (td, *J* = 5.0, 2.7 Hz, 1H), 7.52 - 7.49 (m, 2H), 6.92 (t, *J* = 51.6 Hz, 1H), 6.87 (d, *J =* 11.1 Hz, 1H), 6.73 (d, *J* = 7.0 Hz, 1H), 5.26 (s, 2H), 3.10 - 2.90 (m, 4H), 2.70 - 2.40 (m, 4H), 2.32 (s, 3H); **LRMS** (ES) m/z 537.5 (M⁺ + 1).

### Example 329: Synthesis of Compound 329, 1-((5-(5-(difluoromethyl)-1,3,4-oxadiazole-2-yl)pyridine-2-yl)methyl)-6-fluoro-5-(4-(oxetan-3-yl)pi perazine-1-yl)-3-(pyridine-3-yl)-1,3-dihydro-2H-benzo[d]imidazole-2-one

### [Step 1] Synthesis of the compound 329

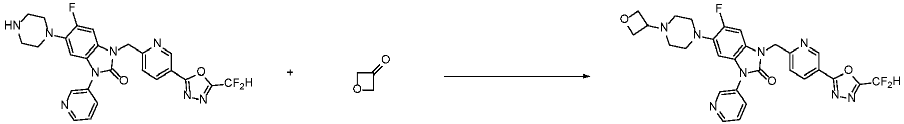

The 1-((5-(5-(difluoromethyl)-1,3,4-oxadiazole-2-yl)pyridine-2-yl)methyl)-6-fluoro-5-(piperazine-1-yl)-3-(pyridine-3-yl)-1,3-dihydro-2H-benzo[d]imidazole-2-one (0.130 g, 0.249 mmol) prepared in the step 5 of the compound 328, sodium triacetoxyborohydride (0.105 g, 0.498 mmol) and oxetan-3-one (0.054 g, 0.746 mmol) were dissolved in dichloromethane (2 mL) at room temperature, after which the resulting solution was stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.113 g, 78.4%) in a brown solid form.

**¹H NMR** (400 MHz, CDCl₃) δ 9.29 (d, *J* = 1.5 Hz, 1H), 8.84 (d, *J* = 2.3 Hz, 1H), 8.67 (dd, *J* = 4.8, 1.5 Hz, 1H), 8.36 (dd, *J* = 8.2, 2.2 Hz, 1H), 7.95 - 7.92 (m, 1H), 7.54 - 7.50 (m, 2H), 6.92 (t, *J* = 51.6 Hz, 1H), 6.89 (d, *J* = 11.1 Hz, 1H), 6.75 (d, *J =* 7.0 Hz, 1H), 5.27 (s, 2H), 4.64 (td, *J =* 11.6, 5.5 Hz, 4H), 3.60 - 3.53 (m, 1H), 3.03 (t, *J* = 4.5 Hz, 4H), 2.60 - 2.45 (m, 4H); **LRMS** (ES) m/z 579.3 (M⁺ + 1).

### Example 330: Synthesis of Compound 330, 1-((5-(5-(difluoromethyl)-1,3,4-oxadiazole-2-yl)pyridine-2-yl)methyl)-6-fluoro-3-methyl-5-(6-(morp holinomethyl)pyridine-3-yl)-1,3-dihydro-2H-benzo[d]imidazole-2-one

### [Step 1] Synthesis of the compound 330

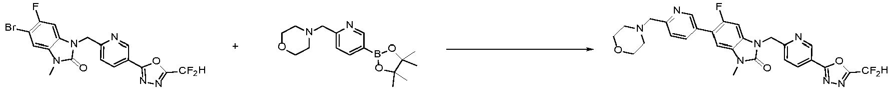

The 5-bromo-1-((5-(5-(difluoromethyl)-1,3,4-oxadiazole-2-yl)pyridine-2-yl)methyl)-6-fluoro-3-methyl-1 ,3-dihydro-2H-benzo[d]imidazole-2-one (0.200 g, 0.440 mmol) prepared in the step 4 of the compound 147, 4-((5-(4,4,5,5-tetramethyl-1,3,2-dioxaborolane-2-yl)pyridine-2-yl)methyl)morpholine (0.161 g, 0.528 mmol), [1,1'-bis(di-tert-butylphosphino)ferrocene]palladium(II) dichloride (Pd(dtbpf)Cl₂, 0.014 g, 0.022 mmol) and cesium carbonate (0.170 g, 0.881 mmol) were mixed in 1,4-dioxane (2.5 mL)/water (0.6 mL) at room temperature, after which the resulting mixture was irradiated with microwave, then heated at 100°C for 20 minutes, and then a reaction was finished by lowering a temperature to room temperature. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.188 g, 77.5%) in a light brown solid form.

**¹H NMR** (400 MHz, CDCl₃) δ 9.25 (d, *J* = 1.6 Hz, 1H), 8.66 (s, 1H), 8.32 (dd, J= 8.2, 2.2 Hz, 1H), 7.78 (td, *J* = 5.0, 2.7 Hz, 1H), 7.45 (d, *J* = 8.2 Hz, 2H), 6.95 (d, *J* = 6.2 Hz, 1H), 6.91 (t, *J* = 51.6 Hz, 1H), 6.86 (d, *J* = 9.8 Hz, 1H), 5.25 (s, 2H), 3.71 (t, *J =* 4.6 Hz, 4H), 3.67 (s, 2H), 3.46 (s, 3H), 2.51 (t, *J* = 4.4 Hz, 4H); **LRMS** (ES) m/z 553.4 (M⁺+ 1).

### Example 331: Synthesis of Compound 331, 3-((5-(5-(difluoromethyl)-1,3,4-oxadiazole-2-yl)pyridine-2-yl)methyl)-6-(1-methylpiperidine-4-yl)b enzo[d]thiazole-2(3H)-one

### [Step 1] Synthesis of tert-butyl 4-(2-oxo-2,3-dihydrobenzo[d]thiazole-6-yl)-3,6-dihydropyridine-1(2H)-carboxylate

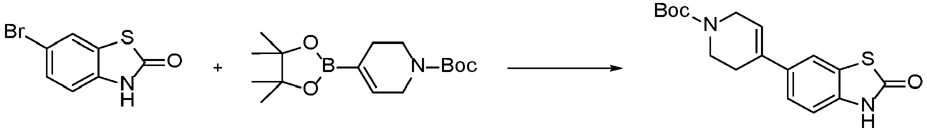

6-bromobenzo[d]thiazole-2(3H)-one (1.500 g, 6.519 mmol), tert-butyl 4-(4,4,5,5-tetramethyl-1,3,2-dioxaborolane-2-yl)-3,6-dihydropyridine-1(2H)-carboxylate (2.419 g, 7.823 mmol), [1,1'-bis(diphenylphosphino)ferrocene]dichloropalladium(II) (Pd(dppf)Cl₂, 0.239 g, 0.326 mmol) and cesium carbonate (6.372 g, 19.558 mmol) were dissolved in 1,4-dioxane (30 mL)/water (10 mL) at room temperature, after which the resulting solution was stirred at 100°C for 18 hours, and then a reaction was finished by lowering a temperature to room temperature. Saturated sodium hydrogen carbonate aqueous solution was poured into the reaction mixture and an extraction was performed with ethyl acetate. An organic layer was washed with saturated sodium chloride aqueous solution, then dehydrated with anhydrous magnesium sulfate, then filtered, and then concentrated under reduced pressure. The resulting concentrate was purified via column chromatography (SiO₂, 24 g cartridge; ethyl acetate/hexane = 5 to 30%) and concentrated to obtain a title compound (0.700 g, 14.7%) in a white solid form.

### [Step 2] Synthesis of tert-butyl 4-(2-oxo-2,3-dihydrobenzo[d]thiazole-6-yl)piperidine-1-carboxylate

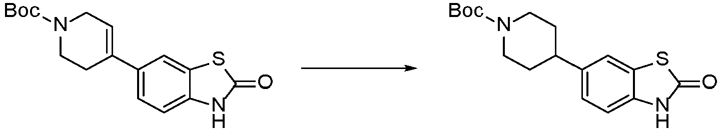

The tert-butyl 4-(2-oxo-2,3-dihydrobenzo[d]thiazole-6-yl)-3,6-dihydropyridine-1(2H)-carboxylate (0.700 g, 2.106 mmol) prepared in the step 1 was dissolved in methanol (50 mL) and stirred at room temperature, after which 10%-Pd/C (70 mg) was slowly added into the resulting solution at the same temperature and stirred at 50°C for 18 hours in the presence of a hydrogen balloon attached thereto, and then a reaction was finished by lowering a temperature to room temperature. The reaction mixture was filtered via a celite pad to remove a solid therefrom, after which solvent was removed from the resulting filtrate under reduced pressure, and then a title compound was used without an additional purification process (0.217 g, 30.8%, light yellow solid).

### [Step 3] Synthesis of tert-butyl 4-(3-((5-(5-(difluoromethyl)-1,3,4-oxadiazole-2-yl)pyridine-2-yl)methyl)-2-oxo-2,3-dihydrobenzo[d ]thiazole-6-yl)piperidine-1-carboxylate

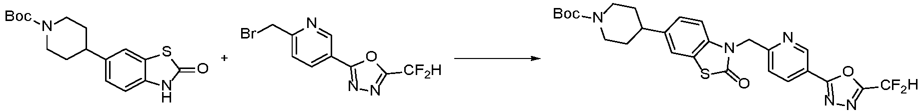

The tert-butyl 4-(2-oxo-2,3-dihydrobenzo[d]thiazole-6-yl)piperidine-1-carboxylate (0.217 g, 0.649 mmol) prepared in the step 2, 2-(6-(bromomethyl)pyridine-3-yl)-5-(difluoromethyl)-1,3,4-oxadiazole (0.207 g, 0.714 mmol), potassium carbonate (0.135 g, 0.973 mmol) and potassium iodide (0.011 g, 0.065 mmol) were dissolved in N,N-dimethylformamide (3 mL) at room temperature, after which the resulting solution was stirred at 100°C for 18 hours, and then a reaction was finished by lowering a temperature to room temperature. Saturated sodium hydrogen carbonate aqueous solution was poured into the reaction mixture and an extraction was performed with ethyl acetate. An organic layer was washed with saturated sodium chloride aqueous solution, then dehydrated with anhydrous magnesium sulfate, then filtered, and then concentrated under reduced pressure. The resulting concentrate was purified via column chromatography (SiO₂, 4 g cartridge; ethyl acetate/hexane = 10 to 20%) and concentrated to obtain a title compound (0.100 g, 28.4%) in a yellow solid form.

### [Step 4] Synthesis of 3-((5-(5-(difluoromethyl)-1,3,4-oxadiazole-2-yl)pyridine-2-yl)methyl)-6-(piperidine-4-yl)benzo[d]th iazole-2(3H)-one

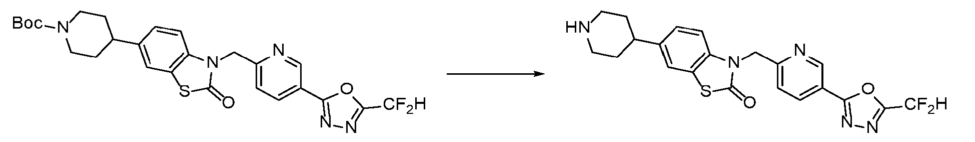

The tert-butyl 4-(3-((5-(5-(difluoromethyl)-1,3,4-oxadiazole-2-yl)pyridine-2-yl)methyl)-2-oxo-2,3-dihydrobenzo[d ]thiazole-6-yl)piperidine-1-carboxylate (0.100 g, 0.184 mmol) prepared in the step 3 and trifluoroacetic acid (0.014 mL, 0.184 mmol) were dissolved in dichloromethane (2 mL) at room temperature, after which the resulting solution was stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. A title compound was used without an additional purification process (0.064 g, 78.4%, yellow oil).

### [Step 5] Synthesis of the compound 331

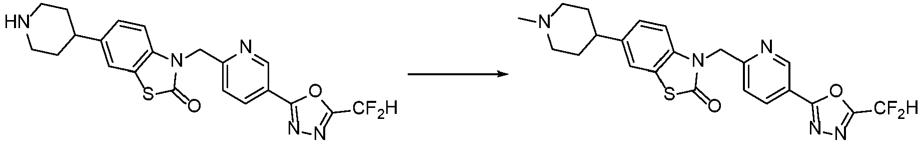

The 3-((5-(5-(difluoromethyl)-1,3,4-oxadiazole-2-yl)pyridine-2-yl)methyl)-6-(piperidine-4-yl)benzo[d]th iazole-2(3H)-one (0.064 g, 0.144 mmol) prepared in the step 4, formaldehyde (37.00% solution, 0.016 mL, 0.216 mmol) and sodium triacetoxyborohydride (0.061 g, 0.289 mmol) were dissolved in dichloromethane (2 mL) at room temperature, after which the resulting solution was stirred at the same temperature for 18 hours. Saturated sodium chloride aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.055 g, 83.3%) in a white solid form.

**¹H NMR** (400 MHz, CDCl₃) δ 9.31 (s, 1H), 8.36 (d, *J* = 8.0 Hz, 1H), 7.45 (d, *J* = 8.2 Hz, 1H), 7.35 (s, 1H), 7.14 (d, *J =* 8.2 Hz, 1H), 7.08 - 6.82 (m, 2H), 5.36 (s, 2H), 3.20 (d, *J* = 8.9 Hz, 1H), 2.61 - 2.58 (m, 1H), 2.51 (s, 3H), 2.33 - 2.32 (m, 2H), 2.06 - 2.04 (m, 2H), 1.91 - 1.88 (m, 2H); **LRMS** (ES) m/z 458.4 (M⁺ + 1).

### Example 332: Synthesis of Compound 332, 3-((5-(5-(difluoromethyl)-1,3,4-oxadiazole-2-yl)pyridine-2-yl)methyl)-5-fluoro-6-(6-(morpholinome thyl)pyridine-3-yl)benzo[d]oxazole-2(3H)-one

### [Step 1] Synthesis of the compound 332

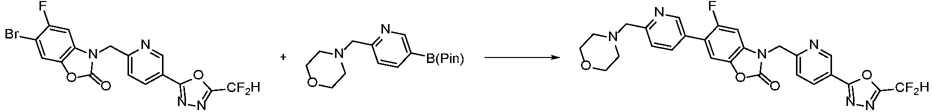

6-bromo-3-((5-(5-(difluoromethyl)-1,3,4-oxadiazole-2-yl)pyridine-2-yl)methyl)-5-fluorobe nzo[d]oxazole-2(3H)-one (0.100 g, 0.227 mmol), 4-((5-(4,4,5,5-tetramethyl-1,3,2-dioxaborolane-2-yl)pyridine-2-yl)methyl)morpholine (0.083 g, 0.272 mmol), [1,1'-bis(di-tert-butylphosphino)ferrocene]palladium(II) dichloride (Pd(dtbpf)Cl₂, 0.007 g, 0.011 mmol) and cesium carbonate (0.148 g, 0.453 mmol) were mixed in 1,4-dioxane (1.5 mL)/water (0.5 mL) at room temperature, after which the resulting mixture was irradiated with microwave, then heated at 100°C for 30 minutes, and then a reaction was finished by lowering a temperature to room temperature. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 2.5%) and concentrated to obtain a title compound (0.009 g, 7.4%) in a brown solid form.

**¹H NMR** (400 MHz, CDCl₃) δ 9.33 (d, *J =* 1.8 Hz, 1H), 8.71 (s, 1H), 8.46 (dd, *J* = 8.2, 2.2 Hz, 1H), 7.83 (d, *J* = 7.1 Hz, 1H), 7.62 - 7.60 (m, 2H), 7.30 (d, *J =* 6.0 Hz, 1H), 7.09 - 6.83 (m, 2H), 5.24 (s, 2H), 3.82 (brs, 6H), 2.64 (brs, 4H); **LRMS** (ES) m/z 539.4 (M⁺+ 1).

### Example 333: Synthesis of Compound 333, 3-((5-(5-(difluoromethyl)-1,3,4-oxadiazole-2-yl)pyridine-2-yl)methyl)-6-(6-(morpholinomethyl)pyri dine-3-yl)benzo[d]thiazole-2(3H)-one

### [Step 1] Synthesis of the compound 333

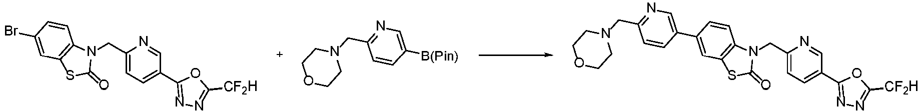

6-bromo-3-((5-(5-(difluoromethyl)-1,3,4-oxadiazole-2-yl)pyridine-2-yl)methyl)benzo[d]thi azole-2(3H)-one (0.100 g, 0.228 mmol), 4-((5-(4,4,5,5-tetramethyl-1,3,2-dioxaborolane-2-yl)pyridine-2-yl)methyl)morpholine (0.083 g, 0.273 mmol), [1,1'-bis(di-tert-butylphosphino)ferrocene]palladium(II) dichloride (Pd(dtbpf)Cl₂, 0.007 g, 0.011 mmol) and cesium carbonate (0.148 g, 0.455 mmol) were mixed in 1,4-dioxane (1.5 mL)/water (0.5 mL) at room temperature, after which the resulting mixture was irradiated with microwave, then heated at 100°C for 30 minutes, and then a reaction was finished by lowering a temperature to room temperature. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 2.5%) and concentrated to obtain a title compound (0.034 g, 27.8%) in a light brown solid form.

**¹H NMR** (400 MHz, CDCl₃) δ 9.32 (d, *J =* 1.6 Hz, 1H), 8.75 (d, *J =* 2.1 Hz, 1H), 8.38 (dd, *J =* 8.2, 2.2 Hz, 1H), 7.82 (dd, *J* = 8.1, 2.3 Hz, 1H), 7.67 (d, *J =* 1.7 Hz, 1H), 7.51 - 7.45 (m, 3H), 7.23 (d, *J* = 8.4 Hz, 1H), 7.08 - 6.82 (m, 1H), 5.41 (s, 2H), 3.79 - 3.75 (m, 6H), 2.59 (brs, 4H); **LRMS** (ES) m/z 537.4 (M⁺+ 1).

### Example 334: Synthesis of Compound 334, 3-((5-(5-(difluoromethyl)-1,3,4-oxadiazole-2-yl)pyridine-2-yl)methyl)-5-fluoro-6-(1-methylpiperidin e-4-yl)benzo[d]oxazole-2(3H)-one

### [Step 1] Synthesis of the compound 334

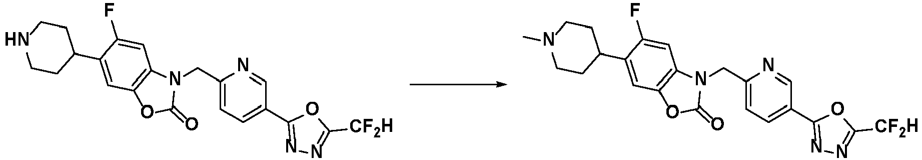

3-((5-(5-(difluoromethyl)-1,3,4-oxadiazole-2-yl)pyridine-2-yl)methyl)-5-fluoro-6-(piperidin e-4-yl)benzo[d]oxazole-2(3H)-one (0.100 g, 0.225 mmol) and formaldehyde (37.00% solution, 0.025 mL, 0.337 mmol) were dissolved in dichloromethane (5 mL) at room temperature, after which sodium triacetoxyborohydride (0.095 g, 0.449 mmol) was added into the resulting solution and stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.060 g, 58.2%) in a yellow solid form.

**¹H NMR** (400 MHz, CDCl₃) δ 9.32 (s, 1H), 8.42 (d, *J* = 7.9 Hz, 1H), 7.56 (d, *J* = 8.2 Hz, 1H), 7.15 (d, *J* = 5.6 Hz, 1H), 6.95 (t, *J* = 51.6 Hz, 1H), 6.78 (d, *J =* 9.0 Hz, 1H), 5.18 (s, 2H), 3.12 - 3.09 (m, 2H), 2.94 - 2.88 (m, 1H), 2.43 (s, 3H), 2.06 - 2.05 (m, 2H), 1.94 - 1.84 (m, 4H); **LRMS** (ES) m/z 460.4 (M⁺+ 1).

### Example 335: Synthesis of Compound 335, 3-((5-(5-(difluoromethyl)-1,3,4-oxadiazole-2-yl)pyridine-2-yl)methyl)-5-fluoro-6-(1-isopropylpiperi dine-4-yl)benzo[d]oxazole-2(3H)-one

### [Step 1] Synthesis of the compound 335

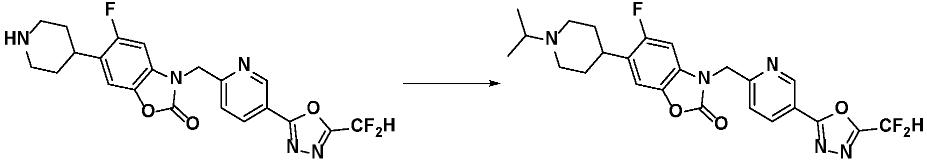

3-((5-(5-(difluoromethyl)-1,3,4-oxadiazole-2-yl)pyridine-2-yl)methyl)-5-fluoro-6-(piperidin e-4-yl)benzo[d]oxazole-2(3H)-one (0.100 g, 0.225 mmol) and acetone (0.025 mL, 0.337 mmol) were dissolved in dichloromethane (5 mL) at room temperature, after which sodium triacetoxyborohydride (0.095 g, 0.449 mmol) was added into the resulting solution and stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.056 g, 51.2%) in a yellow solid form.

**¹H NMR** (400 MHz, CDCl₃) δ 9.31 (d, *J =* 1.7 Hz, 1H), 8.41 (dd, *J* = 8.2, 2.2 Hz, 1H), 7.56 (d, *J =* 16.2 Hz, 1H), 7.17 (d, *J* = 5.7 Hz, 1H), 7.08 - 6.82 (m, 1H), 6.76 (d, *J =* 9.0 Hz, 1H), 5.18 (s, 2H), 3.12 - 3.09 (m, 2H), 2.92 - 2.91 (m, 2H), 2.40 - 2.35 (m, 2H), 1.88 - 1.87 (m, 4H), 1.16 (d, *J* = 6.2 Hz, 6H); **LRMS** (ES) m/z 488.5 (M⁺ + 1).

### Example 336: Synthesis of Compound 336, 3-((5-(5-(difluoromethyl)-1,3,4-oxadiazole-2-yl)pyridine-2-yl)methyl)-5-fluoro-6-(1-(oxetan-3-yl)pi peridine-4-yl)benzo[d]oxazole-2(3H)-one

### [Step 1] Synthesis of the compound 336

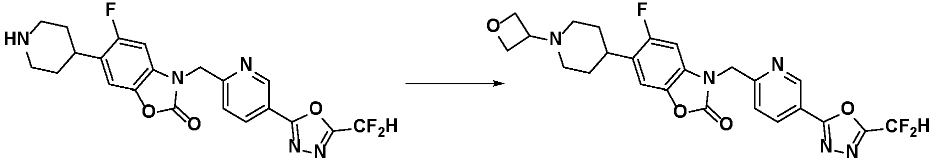

3-((5-(5-(difluoromethyl)-1,3,4-oxadiazole-2-yl)pyridine-2-yl)methyl)-5-fluoro-6-(piperidin e-4-yl)benzo[d]oxazole-2(3H)-one (0.100 g, 0.635 mmol) and oxetan-3-one (0.061 mL, 0.953 mmol) were dissolved in dichloromethane (5 mL) at room temperature, after which sodium triacetoxyborohydride (0.269 g, 1.271 mmol) was added into the resulting solution and stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.042 g, 13.2%) in a white solid form.

**¹H NMR** (400 MHz, CDCl₃) δ 9.32 (d, *J =* 1.6 Hz, 1H), 8.42 (dd, *J* = 8.2, 2.2 Hz, 1H), 7.56 (d, *J =* 8.2 Hz, 1H), 7.16 (d, *J =* 5.6 Hz, 1H), 7.08 - 6.82 (m, 1H), 6.79 (d, *J =* 9.0 Hz, 1H), 5.32 (s, 2H), 4.74 - 4.70 (m, 4H), 3.58 - 3.57 (m, 1H), 2.93 - 2.92 (m, 3H), 2.03 - 2.02 (m, 2H), 1.65 - 1.64 (m, 4H); **LRMS** (ES) m/z 502.4 (M⁺ + 1).

### Example 337: Synthesis of Compound 337, tert-butyl 4-(5-(3-((5-(5-(difluoromethyl)-1,3,4-oxadiazole-2-yl)pyridine-2-yl)methyl)-5-fluoro-2-oxo-2,3-dihy drobenzo[d]oxazole-6-yl)pyrimidine-2-yl)piperazine-1-carboxylate

### [Step 1] Synthesis of the compound 337

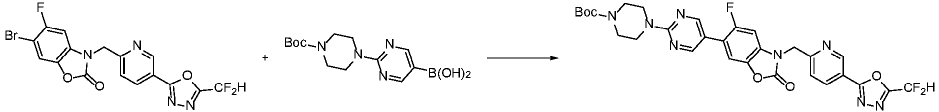

6-bromo-3-((5-(5-(difluoromethyl)-1,3,4-oxadiazole-2-yl)pyridine-2-yl)methyl)-5-fluorobe nzo[d]oxazole-2(3H)-one (0.500 g, 1.133 mmol), 2-(4-(tert-butoxycarbonyl)piperazine-1-yl)pyrimidine-5-yl)boronic acid (0.418 g, 1.360 mmol), [1,1'-bis(di-tert-butylphosphino)ferrocene]palladium(II) dichloride (Pd(dtbpf)Cl₂, 0.037 g, 0.057 mmol) and cesium carbonate (0.739 g, 2.267 mmol) were mixed in 1,4-dioxane (9 mL)/water (3 mL) at room temperature, after which the resulting mixture was irradiated with microwave, then heated at 100°C for 30 minutes, and then a reaction was finished by lowering a temperature to room temperature. Saturated sodium hydrogen carbonate aqueous solution was poured into the reaction mixture and an extraction was performed with dichloromethane. An organic layer was washed with saturated sodium chloride aqueous solution, then dehydrated with anhydrous magnesium sulfate, then filtered, and then concentrated under reduced pressure. The resulting concentrate was purified via column chromatography (SiO₂, 12 g cartridge; ethyl acetate/hexane = 10 to 70%) and concentrated to obtain a title compound (0.200 g, 28.3%) in a light brown solid form.

**¹H NMR** (400 MHz, CDCl₃) δ 9.33 (d, *J =* 1.9 Hz, 1H), 8.51 (s, 2H), 8.45 (dd, *J* = 8.2, 2.1 Hz, 1H), 7.60 (d, *J* = 8.2 Hz, 1H), 7.23 (d, *J* = 5.9 Hz, 1H), 7.09 - 6.83 (m, 2H), 5.23 (s, 2H), 3.93 - 3.91 (m, 4H), 3.57 - 3.55 (m, 4H), 1.52 (s, 9H); **LRMS** (ES) m/z 625.2 (M⁺+ 1).

### Example 338: Synthesis of Compound 338, 3-((5-(5-(difluoromethyl)-1,3,4-oxadiazole-2-yl)pyridine-2-yl)methyl)-5-fluoro-6-(2-(4-methylpiper azine-1-yl)pyrimidine-5-yl)benzo[d]oxazole-2(3H)-one

### [Step 1] Synthesis of 3-((5-(5-(difluoromethyl)-1,3,4-oxadiazole-2-yl)pyridine-2-yl)methyl)-5-fluoro-6-(2-(piperazine-1-y I)pyrimidine-5-yl)benzo[d]oxazole-2(3H)-one

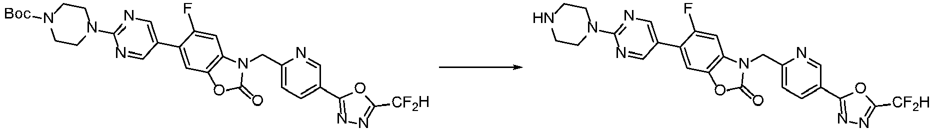

Tert-butyl 4-(5-(3-((5-(5-(difluoromethyl)-1,3,4-oxadiazole-2-yl)pyridine-2-yl)methyl)-5-fluoro-2-oxo-2,3-dihy drobenzo[d]oxazole-6-yl)pyrimidine-2-yl)piperazine-1-carboxylate (0.241 g, 0.386 mmol) and trifluoroacetic acid (0.295 mL, 3.859 mmol) were dissolved in dichloromethane (5 mL) at room temperature, after which the resulting solution was stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. A title compound was used without an additional purification process (0.132 g, 65.2%, brown solid).

### [Step 2] Synthesis of the compound 338

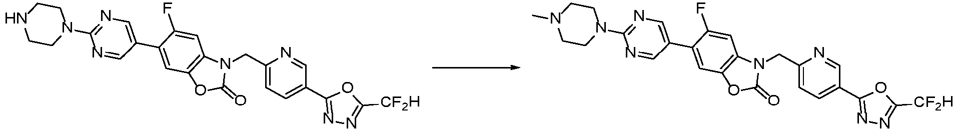

The 3-((5-(5-(difluoromethyl)-1,3,4-oxadiazole-2-yl)pyridine-2-yl)methyl)-5-fluoro-6-(2-(piperazine-1-y I)pyrimidine-5-yl)benzo[d]oxazole-2(3H)-one (0.070 g, 0.133 mmol) prepared in the step 1 and formaldehyde (37.00% solution, 0.015 mL, 0.200 mmol) were dissolved in dichloromethane (5 mL) at room temperature, after which sodium triacetoxyborohydride (0.057 g, 0.267 mmol) was added into the resulting solution and stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 47 g cartridge; methanol/dichloromethane = 0 to 5%) and concentrated to obtain a title compound (0.046 g, 64.0%) in a light yellow solid form.

**¹H NMR** (400 MHz, DMSO-d₆) δ 9.14 (d, *J* = 1.8 Hz, 1H), 8.55 (d, *J* = 1.4 Hz, 2H), 8.47 (dd, *J =* 8.2, 2.2 Hz, 1H), 7.75 (d, *J =* 11.9 Hz, 1H), 7.69 - 7.43 (m, 2H), 7.36 (d, *J =* 10.0 Hz, 1H), 5.35 (s, 2H), 3.79 - 3.77 (m, 4H), 2.38 - 2.36 (m, 4H), 2.22 (s, 3H); **LRMS** (ES) m/z 539.1 (M⁺+ 1).

### Example 339: Synthesis of Compound 339, 3-((5-(5-(difluoromethyl)-1,3,4-oxadiazole-2-yl)pyridine-2-yl)methyl)-5-fluoro-6-(2-(4-(oxetan-3-yl )piperazine-1-yl)pyrimidine-5-yl)benzo[d]oxazole-2(3H)-one

### [Step 1] Synthesis of the compound 339

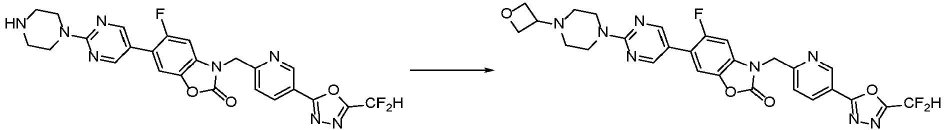

3-((5-(5-(difluoromethyl)-1,3,4-oxadiazole-2-yl)pyridine-2-yl)methyl)-5-fluoro-6-(2-(pipera zine-1-yl)pyrimidine-5-yl)benzo[d]oxazole-2(3H)-one (0.060 g, 0.114 mmol) and oxetan-3-one (0.012 g, 0.172 mmol) were dissolved in dichloromethane (5 mL) at room temperature, after which sodium triacetoxyborohydride (0.048 g, 0.229 mmol) was added into the resulting solution and stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 2.5%) and concentrated to obtain a title compound (0.026 g, 39.1%) in a light yellow solid form.

**¹H NMR** (400 MHz, DMSO-d₆) δ 9.13 (d, *J =* 2.0 Hz, 1H), 8.56 (d, *J =* 1.2 Hz, 2H), 8.47 (dd, *J* = 8.2, 2.2 Hz, 1H), 7.75 (d, *J* = 8.3 Hz, 1H), 7.69 - 7.47 (m, 2H), 7.37 (d, *J* = 10.0 Hz, 1H), 5.35 (s, 2H), 4.57 (t, *J* = 6.5 Hz, 2H), 4.49 (t, *J* = 6.1 Hz, 2H), 3.81 - 3.80 (m, 4H), 3.50 - 3.49 (m, 1H), 2.34 - 2.33 (m, 4H); **LRMS** (ES) m/z 581.5 (M⁺ + 1)

### Example 340: Synthesis of Compound 340, 1-(4-(5-(difluoromethyl)-1,3,4-oxadiazole-2-yl)-2-fluorobenzyl)-6-fluoro-3-methyl-5-(6-(morpholin omethyl)pyridine-3-yl)-1,3-dihydro-2H-benzo[d]imidazole-2-one

### [Step 1] Synthesis of the compound 340

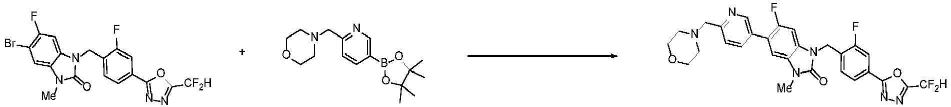

The 5-bromo-1-(4-(5-(difluoromethyl)-1,3,4-oxadiazole-2-yl)-2-fluorobenzyl)-6-fluoro-3-methyl-1,3-dih ydro-2H-benzo[d]imidazole-2-one (0.200 g, 0.424 mmol) prepared in the step 1 of the compound 148, 4-((5-(4,4,5,5-tetramethyl-1,3,2-dioxaborolane-2-yl)pyridine-2-yl)methyl)morpholine (0.155 g, 0.509 mmol), cesium carbonate (0.277 g, 0.849 mmol) and [1,1'-bis(di-tert-butylphosphino)ferrocene]palladium(II) dichloride (Pd(dtbpf)Cl₂, 0.014 g, 0.021 mmol) were mixed in 1,4-dioxane (2 mL)/water (0.6 mL) at room temperature, after which the resulting mixture was irradiated with microwave, then heated at 100°C for 20 minutes, and then a reaction was finished by lowering a temperature to room temperature. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.135 g, 55.9%) in a brown solid form.

**¹H NMR** (400 MHz, CDCl₃) δ 8.67 (s, 1H), 7.83 (d, *J* = 8.7 Hz, 2H), 7.79 (d, *J* = 8.1 Hz, 1H), 7.51 - 7.45 (m, 2H), 6.96 (d, *J =* 6.2 Hz, 1H), 6.90 (t, *J* = 51.6 Hz, 1H), 6.81 (d, *J =* 9.8 Hz, 1H), 5.17 (s, 2H), 3.72 (t, *J* = 4.5 Hz, 4H), 3.67 (s, 2H), 3.46 (s, 3H), 2.60 - 2.45 (m, 4H); **LRMS** (ES) m/z 569.35 (M⁺ + 1).

### Example 341: Synthesis of Compound 341, tert-butyl 4-(5-(1-((5-(5-(difluoromethyl)-1,3,4-oxadiazole-2-yl)pyridine-2-yl)methyl)-6-fluoro-3-methyl-2-ox o-2,3-dihydro-1H-benzo[d]imidazole-5-yl)pyrimidine-2-yl)piperazine-1-carboxylate

### [Step 1] Synthesis of the compound 341

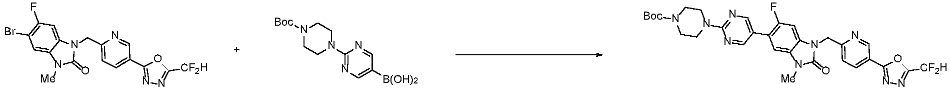

The 5-bromo-1-((5-(5-(difluoromethyl)-1,3,4-oxadiazole-2-yl)pyridine-2-yl)methyl)-6-fluoro-3-methyl-1 ,3-dihydro-2H-benzo[d]imidazole-2-one (1.500 g, 3.302 mmol) prepared in the step 4 of the compound 147, (2-(4-(tert-butoxycarbonyl)piperazine-1-yl)pyrimidine-5-yl)boronic acid (1.221 g, 3.963 mmol), cesium carbonate (2.152 g, 6.605 mmol) and [1,1'-bis(di-tert-butylphosphino)ferrocene]palladium(II) dichloride (Pd(dtbpf)Cl₂, 0.108 g, 0.165 mmol) were mixed in 1,4-dioxane (9 mL)/water (3 mL) at room temperature, after which the resulting mixture was irradiated with microwave, then heated at 100°C for 20 minutes, and then a reaction was finished by lowering a temperature to room temperature. The reaction mixture was filtered via a glass filter to remove a solid therefrom, after which saturated sodium hydrogen carbonate aqueous solution was poured into the resulting filtrate, and then an extraction was performed with ethyl acetate. An organic layer was washed with saturated sodium chloride aqueous solution, then dehydrated with anhydrous magnesium sulfate, then filtered, and then concentrated under reduced pressure. The resulting concentrate was purified via column chromatography (SiO₂, 24 g cartridge; ethyl acetate/hexane = 50 to 70%) and concentrated to obtain a title compound (0.590 g, 28.0%) in a brown solid form.

**¹H NMR** (400 MHz, CDCl₃) δ 9.29 (d, *J =* 1.9 Hz, 1H), 8.48 (s, 2H), 8.35 (dd, *J* = 8.2, 2.2 Hz, 1H), 7.47 (d, *J* = 8.2 Hz, 1H), 6.92 (t, *J =* 51.6 Hz, 1H), 6.89 (d, *J* = 6.2 Hz, 1H), 6.86 (d, *J* = 9.8 Hz, 1H), 5.27 (s, 2H), 3.86 (t, *J* = 5.1 Hz, 4H), 3.52 (t, *J* = 5.0 Hz, 4H), 3.49 (s, 3H), 1.49 (s, 9H); **LRMS** (ES) m/z 638.38 (M⁺ + 1).

### Example 342: Synthesis of Compound 342, 1-((5-(5-(difluoromethyl)-1,3,4-oxadiazole-2-yl)pyridine-2-yl)methyl)-6-fluoro-3-methyl-5-(2-(4-me thylpiperazine-1-yl)pyrimidine-5-yl)-1,3-dihydro-2H-benzo[d]imidazole-2-one

### [Step 1] Synthesis of the compound 342

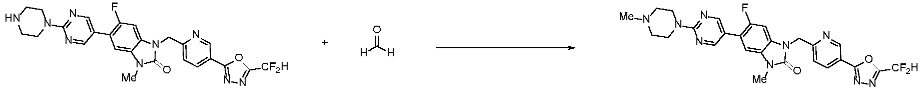

The 1-((5-(5-(difluoromethyl)-1,3,4-oxadiazole-2-yl)pyridine-2-yl)methyl)-6-fluoro-3-methyl-5-(2-(piper azine-1-yl)pyrimidine-5-yl)-1,3-dihydro-2H-benzo[d]imidazole-2-one (0.150 g, 0.279 mmol) prepared in the step 1 of the compound 341, sodium triacetoxyborohydride (0.118 g, 0.558 mmol) and formaldehyde (37.00%, 0.068 g, 0.837 mmol) were dissolved in dichloromethane (2 mL) at room temperature, after which the resulting solution was stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.141 g, 91.9%) in a brown solid form.

**¹H NMR** (400 MHz, CDCl₃) δ 9.27 (d, *J =* 1.9 Hz, 1H), 8.44 (d, *J =* 1.2 Hz, 2H), 8.33 (dd, *J* = 8.2, 2.2 Hz, 1H), 7.45 (d, *J* = 8.2 Hz, 1H), 6.92 (t, *J =* 51.6 Hz, 1H), 6.88 (d, *J =* 6.2 Hz, 1H), 6.84 (d, *J* = 9.8 Hz, 1H), 5.25 (s, 2H), 3.88 (t, *J* = 4.9 Hz, 4H), 3.47 (s, 3H), 2.48 (t, *J =* 5.0 Hz, 4H), 2.34 (s, 3H); **LRMS** (ES) m/z 552.35 (M⁺ + 1).

### Example 343: Synthesis of Compound 343, 1-((5-(5-(difluoromethyl)-1,3,4-oxadiazole-2-yl)pyridine-2-yl)methyl)-6-fluoro-3-methyl-5-(2-(4-(ox etan-3-yl)piperazine-1-yl)pyrimidine-5-yl)-1,3-dihydro-2H-benzo[d]imidazole-2-one

### [Step 1] Synthesis of the compound 343

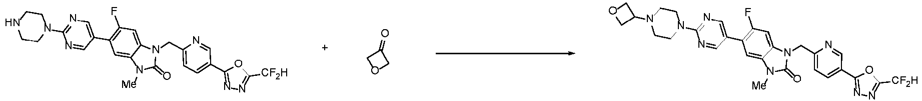

The 1-((5-(5-(difluoromethyl)-1,3,4-oxadiazole-2-yl)pyridine-2-yl)methyl)-6-fluoro-3-methyl-5-(2-(piper azine-1-yl)pyrimidine-5-yl)-1,3-dihydro-2H-benzo[d]imidazole-2-one (0.150 g, 0.279 mmol) prepared in the step 1 of the compound 341, sodium triacetoxyborohydride (0.118 g, 0.558 mmol) and oxetan-3-one (0.060 g, 0.837 mmol) were dissolved in dichloromethane (2 mL) at room temperature, after which the resulting solution was stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.115 g, 69.1%) in a brown solid form.

**¹H NMR** (400 MHz, CDCl₃) δ 9.27 (d, *J =* 1.6 Hz, 1H), 8.45 (d, *J =* 1.4 Hz, 2H), 8.34 (dd, *J* = 8.2, 2.2 Hz, 1H), 7.46 (d, *J* = 8.2 Hz, 1H), 6.92 (t, *J =* 51.6 Hz, 1H), 6.88 (d, *J =* 6.2 Hz, 1H), 6.85 (d, *J* = 9.8 Hz, 1H), 5.26 (s, 2H), 4.67 (td, *J* = 7.5, 5.6 Hz, 4H), 3.91 (t, *J* = 4.9 Hz, 4H), 3.55 - 3.47 (m, 1H), 3.47 (s, 3H), 2.40 (t, *J* = 4.9 Hz, 4H); **LRMS** (ES) m/z 594.24 (M⁺ + 1).

### Example 344: Synthesis of Compound 344, tert-butyl 4-(5-(3-((5-(5-(difluoromethyl)-1,3,4-oxadiazole-2-yl)pyridine-2-yl)methyl)-5-fluoro-2-oxo-2,3-dihy drobenzo[d]oxazole-6-yl)pyridine-2-yl)piperazine-1-carboxylate

### [Step 1] Synthesis of tert-butyl 4-(5-(5-fluoro-2-oxo-2,3-dihydrobenzo[d]oxazole-6-yl)pyridine-2-yl)piperazine-1-carboxylate

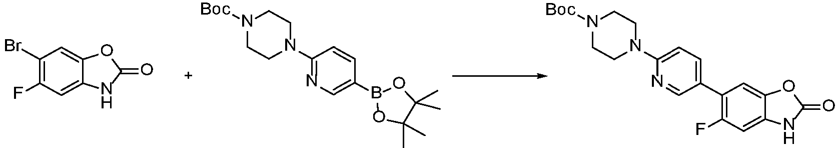

6-bromo-5-fluorobenzo[d]oxazole-2(3H)-one (0.500 g, 2.155 mmol), tert-butyl 4-(5-(4,4,5,5-tetramethyl-1,3,2-dioxaborolane-2-yl)pyridine-2-yl)piperazine-1-carboxylate (1.007 g, 2.586 mmol), [1,1'-bis(di-tert-butylphosphino)ferrocene]palladium(II) dichloride (Pd(dtbpf)Cl₂, 0.070 g, 0.108 mmol) and cesium carbonate (1.404 g, 4.310 mmol) were mixed in 1,4-dioxane (4 mL)/water (1 mL) at room temperature, after which the resulting mixture was irradiated with microwave, then heated at 100°C for 30 minutes, and then a reaction was finished by lowering a temperature to room temperature. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. Diethylether (20 mL) was put into the resulting concentrate and stirred, after which a precipitated solid was filtered, then washed with diethylether, and then dried to obtain a title compound (0.783 g, 87.7%) in a brown solid form.

### [Step 2] Synthesis of the compound 344

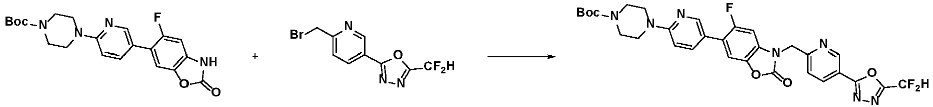

The tert-butyl 4-(5-(5-fluoro-2-oxo-2,3-dihydrobenzo[d]oxazole-6-yl)pyridine-2-yl)piperazine-1-carboxylate (0.783 g, 1.889 mmol) prepared in the step 1, 2-(6-(bromomethyl)pyridine-3-yl)-5-(difluoromethyl)-1,3,4-oxadiazole (0.603 g, 2.078 mmol), potassium carbonate (0.392 g, 2.834 mmol) and potassium iodide (0.031 g, 0.189 mmol) were dissolved in N,N-dimethylformamide (15 mL) at room temperature, after which the resulting solution was stirred at 100°C for 18 hours, and then a reaction was finished by lowering a temperature to room temperature. Solvent was removed from the reaction mixture under reduced pressure, after which saturated sodium hydrogen carbonate aqueous solution was poured into the resulting concentrate, and then an extraction was performed with ethyl acetate. An organic layer was washed with saturated sodium chloride aqueous solution, then dehydrated with anhydrous magnesium sulfate, then filtered, and then concentrated under reduced pressure. Diethylether was put into the resulting concentrate and stirred, after which a precipitated solid was filtered, then washed with diethylether, and then dried to obtain a title compound (0.794 g, 67.4%) in a gray solid form.

**¹H NMR** (400 MHz, CDCl₃) δ 9.33 (d, *J =* 1.9 Hz, 1H), 8.44 (dd, *J* = 8.2, 2.1 Hz, 1H), 8.34 (s, 1H), 7.67 (d, *J* = 7.4 Hz, 1H), 7.59 (d, *J =* 8.2 Hz, 1H), 7.26 (d, *J* = 6.0 Hz, 1H), 7.09 - 6.83 (m, 2H), 6.74 (d, *J* = 8.2 Hz, 1H), 5.23 (s, 2H), 3.62 - 3.60 (m, 8H), 1.51 (s, 9H); LRMS (ES) m/z 624.4 (M⁺+ 1).

### Example 345: Synthesis of Compound 345, 3-((5-(5-(difluoromethyl)-1,3,4-oxadiazole-2-yl)pyridine-2-yl)methyl)-5-fluoro-6-(6-(piperazine-1-y I)pyridine-3-yl)benzo[d]oxazole-2(3H)-one

### [Step 1] Synthesis of the compound 345

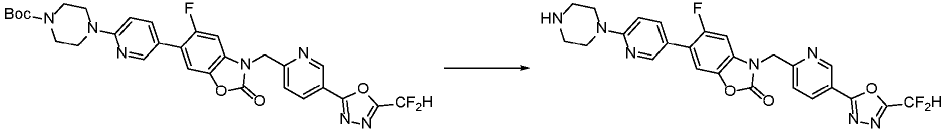

Tert-butyl 4-(5-(3-((5-(5-(difluoromethyl)-1,3,4-oxadiazole-2-yl)pyridine-2-yl)methyl)-5-fluoro-2-oxo-2,3-dihy drobenzo[d]oxazole-6-yl)pyridine-2-yl)piperazine-1-carboxylate (0.045 g, 0.072 mmol) and trifluoroacetic acid (0.028 mL, 0.361 mmol) were dissolved in dichloromethane (3 mL) at room temperature, after which the resulting solution was stirred at the same temperature for 4 hours. Solvent was removed from the reaction mixture under reduced pressure, after which saturated sodium hydrogen carbonate aqueous solution and dichloromethane were put into the resulting concentrate and stirred to filter out a precipitated solid, then washed with hexane, and then dried to obtain a title compound (0.023 g, 60.9%) in a light yellow solid form.

**¹H NMR** (400 MHz, DMSO-d₆) δ 9.15 (d, *J* = 1.9 Hz, 1H), 8.47 (dd, *J* = 8.2, 2.2 Hz, 1H), 8.28 (s, 1H), 7.75 (d, *J* = 8.3 Hz, 1H), 7.71 - 7.45 (m, 3H), 7.33 (d, *J =* 10.0 Hz, 1H), 6.89 (d, *J* = 8.6 Hz, 1H), 5.35 (s, 2H), 3.46 - 3.36 (m, 6H), 2.86 (brs, 2H); **LRMS** (ES) m/z 524.1 (M⁺ + 1).

### Example 346: Synthesis of Compound 346, 3-((5-(5-(difluoromethyl)-1,3,4-oxadiazole-2-yl)pyridine-2-yl)methyl)-5-fluoro-6-(6-(4-methylpiper azine-1-yl)pyridine-3-yl)benzo[d]oxazole-2(3H)-one

### [Step 1] Synthesis of the compound 346

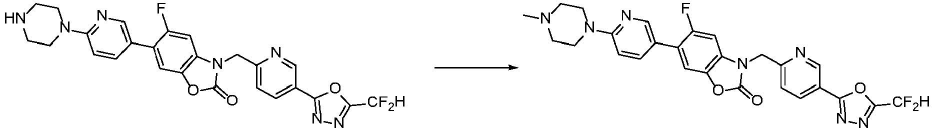

3-((5-(5-(difluoromethyl)-1,3,4-oxadiazole-2-yl)pyridine-2-yl)methyl)-5-fluoro-6-(6-(pipera zine-1-yl)pyridine-3-yl)benzo[d]oxazole-2(3H)-one (0.200 g, 0.382 mmol) and formaldehyde (37.00% solution, 0.043 mL, 0.573 mmol) were dissolved in dichloromethane (5 mL) at room temperature, after which sodium triacetoxyborohydride (0.162 g, 0.764 mmol) was added into the resulting solution and stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 10%) and concentrated to obtain a product. Then, diethylether was inserted into the resulting product and stirred to filter out a precipitated solid, then washed with diethylether, and then dried to obtain a title compound (0.011 g, 5.4%) in a yellow solid form.

**¹H NMR** (400 MHz, CDCl₃) δ 9.33 (d, *J =* 1.9 Hz, 1H), 8.44 (dd, *J* = 7.5, 2.8 Hz, 1H), 8.33 (s, 1H), 7.66 - 7.64 (m, 1H), 7.59 (d, *J* = 8.2 Hz, 1H), 7.25 (d, *J =* 4.5 Hz, 1H), 6.96 - 6.83 (m, 2H), 6.73 (d, *J* = 8.9 Hz, 1H), 5.23 (s, 2H), 3.72 (brs, 4H), 2.68 (brs, 4H), 2.45 (s, 3H); **LRMS** (ES) m/z 538.4 (M⁺+ 1).

### Example 347: Synthesis of Compound 347, 3-((5-(5-(difluoromethyl)-1,3,4-oxadiazole-2-yl)pyridine-2-yl)methyl)-6-(6-(4-ethylpiperazine-1-yl) pyridine-3-yl)-5-fluorobenzo[d]oxazole-2(3H)-one

### [Step 1] Synthesis of the compound 347

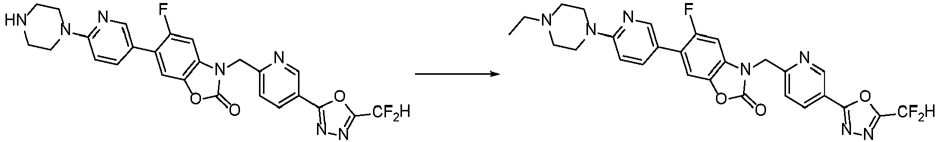

3-((5-(5-(difluoromethyl)-1,3,4-oxadiazole-2-yl)pyridine-2-yl)methyl)-5-fluoro-6-(6-(pipera zine-1-yl)pyridine-3-yl)benzo[d]oxazole-2(3H)-one (0.200 g, 0.382 mmol), acetaldehyde (0.032 mL, 0.573 mmol) and sodium triacetoxyborohydride (0.162 g, 0.764 mmol) were dissolved in dichloromethane (4 mL) at room temperature, after which the resulting solution was stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.011 g, 5.2%) in a yellow solid form.

**¹H NMR** (400 MHz, CDCl₃) δ 9.33 (d, *J =* 2.1 Hz, 1H), 8.44 (dd, *J* = 8.2, 2.2 Hz, 1H), 8.32 (brs, 1H), 7.65 - 7.63 (m, 1H), 7.58 (d, *J* = 8.2 Hz, 1H), 7.25 (d, *J* = 6.0 Hz, 1H), 6.95 - 6.82 (m, 2H), 6.72 (d, *J* = 8.9 Hz, 1H), 3.70 - 3.67 (m, 4H), 2.64 - 2.63 (m, 4H), 2.54 (q, *J* = 7.2 Hz, 2H), 1.19 (t, *J =* 7.2 Hz, 3H); **LRMS** (ES) m/z 552.4 (M⁺+ 1).

### Example 348: Synthesis of Compound 348, 3-((5-(5-(difluoromethyl)-1,3,4-oxadiazole-2-yl)pyridine-2-yl)methyl)-5-fluoro-6-(5-morpholinopyri dine-3-yl)benzo[d]oxazole-2(3H)-one

### [Step 1] Synthesis of 5-fluoro-6-(5-morpholinopyridine-3-yl)benzo[d]oxazole-2(3H)-one

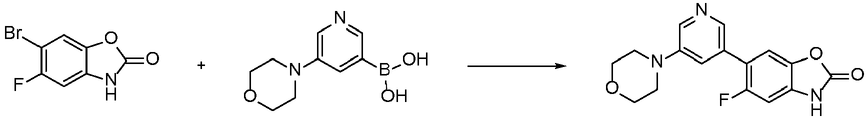

6-bromo-5-fluorobenzo[d]oxazole-2(3H)-one (0.200 g, 0.862 mmol), (2-morpholinopyridine-4-yl)boronic acid (0.215 g, 1.034 mmol), [1,1'-bis(di-tert-butylphosphino)ferrocene]palladium(II) dichloride (Pd(dtbpf)Cl₂, 0.028 g, 0.043 mmol) and cesium carbonate (0.562 g, 1.724 mmol) were dissolved in 1,4-dioxane (15 mL)/water (0.5 mL) at room temperature, after which the resulting solution was stirred at 100°C for 18 hours, and then a reaction was finished by lowering a temperature to room temperature. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; ethyl acetate/hexane = 10 to 50%) and concentrated to obtain a title compound (0.064 g, 23.5%) in a yellow solid form.

### [Step 2] Synthesis of the compound 348

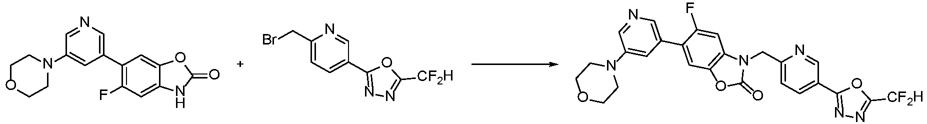

The 5-fluoro-6-(5-morpholinopyridine-3-yl)benzo[d]oxazole-2(3H)-one (0.064 g, 0.203 mmol), 2-(6-(bromomethyl)pyridine-3-yl)-5-(difluoromethyl)-1,3,4-oxadiazole (0.071 g, 0.244 mmol) prepared in the step 1, potassium carbonate (0.042 g, 0.304 mmol) and potassium iodide (0.003 g, 0.020 mmol) were dissolved in N,N-dimethylformamide (2 mL) at room temperature, after which the resulting solution was stirred at 100°C for 18 hours, and then a reaction was finished by lowering a temperature to room temperature. Solvent was removed from the reaction mixture under reduced pressure, after which the resulting concentrate was purified via column chromatography (SiO₂, 4 g cartridge; ethyl acetate/hexane = 10 to 50%) and concentrated to obtain a title compound (0.016 g, 15.0%) in a yellow solid form.

**¹H NMR** (400 MHz, CDCl₃) δ 9.33 (d, *J =* 1.8 Hz, 1H), 8.46 (dd, *J* = 8.2, 2.1 Hz, 1H), 8.30 (d, *J* = 2.5 Hz, 1H), 8.24 (s, 1H), 7.62 (d, *J* = 8.2 Hz, 1H), 7.37 (s, 1H), 7.31 (d, *J* = 5.9 Hz, 1H), 7.09 - 6.83 (m, 2H), 5.25 (s, 2H), 3.92 (t, *J* = 4.8 Hz, 4H), 3.29 (t, *J* = 4.8 Hz, 4H); **LRMS** (ES) m/z 525.1 (M⁺ + 1).

### Example 349: Synthesis of Compound 349, 3-((5-(5-(difluoromethyl)-1,3,4-oxadiazole-2-yl)pyridine-2-yl)methyl)-5-fluoro-6-(6-(4-isopropylpip erazine-1-yl)pyridine-3-yl)benzo[d]oxazole-2(3H)-one

### [Step 1] Synthesis of the compound 349

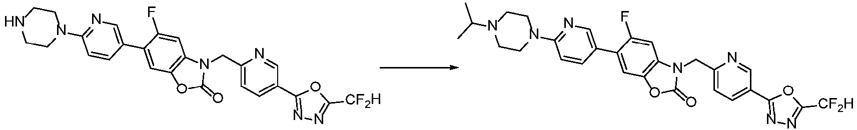

3-((5-(5-(difluoromethyl)-1,3,4-oxadiazole-2-yl)pyridine-2-yl)methyl)-5-fluoro-6-(6-(pipera zine-1-yl)pyridine-3-yl)benzo[d]oxazole-2(3H)-one (0.200 g, 0.382 mmol), acetone (0.042 mL, 0.573 mmol) and sodium triacetoxyborohydride (0.162 g, 0.764 mmol) were dissolved in dichloromethane (4 mL) at room temperature, after which the resulting solution was stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.056 g, 25.9%) in a brown solid form.

**¹H NMR** (400 MHz, CDCl₃) δ 9.32 (d, *J =* 1.8 Hz, 1H), 8.43 (dd, *J* = 8.2, 2.0 Hz, 1H), 8.32 (s, 1H), 7.63 (d, *J* = 8.9 Hz, 1H), 7.58 (d, *J =* 8.2 Hz, 1H), 7.24 (d, *J* = 6.0 Hz, 1H), 7.08 - 6.82 (m, 2H), 6.71 (d, *J* = 8.9 Hz, 1H), 5.22 (s, 2H), 3.70 (t, *J* = 4.8 Hz, 4H), 2.92 - 2.89 (m, 1H), 2.77 (t, *J* = 4.9 Hz, 4H), 1.16 (d, *J =* 6.5 Hz, 6H); **LRMS** (ES) m/z 566.2 (M⁺ + 1).

### Example 350: Synthesis of Compound 350, 3-((5-(5-(difluoromethyl)-1,3,4-oxadiazole-2-yl)pyridine-2-yl)methyl)-5-fluoro-6-(6-(4-(oxetan-3-yl )piperazine-1-yl)pyridine-3-yl)benzo[d]oxazole-2(3H)-one

### [Step 1] Synthesis of the compound 350

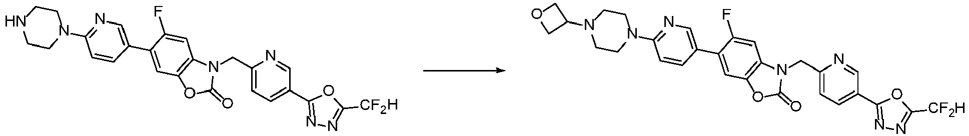

3-((5-(5-(difluoromethyl)-1,3,4-oxadiazole-2-yl)pyridine-2-yl)methyl)-5-fluoro-6-(6-(pipera zine-1-yl)pyridine-3-yl)benzo[d]oxazole-2(3H)-one (0.200 g, 0.382 mmol), oxetan-3-one (0.037 mL, 0.573 mmol) and sodium triacetoxyborohydride (0.162 g, 0.764 mmol) were dissolved in dichloromethane (4 mL) at room temperature, after which the resulting solution was stirred at the same temperature for 18 hours. Saturated sodium chloride aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 2.5%) and concentrated to obtain a title compound (0.011 g, 5.0%) in a yellow solid form.

**¹H NMR** (400 MHz, CDCl₃) δ 9.33 (d, *J =* 1.9 Hz, 1H), 8.44 (dd, *J* = 8.2, 2.2 Hz, 1H), 8.33 (s, 1H), 7.66 (d, *J* = 8.9 Hz, 1H), 7.59 (d, *J =* 8.2 Hz, 1H), 7.25 (d, *J* = 6.0 Hz, 1H), 7.09 - 6.83 (m, 2H), 6.74 (d, *J* = 8.9 Hz, 1H), 5.23 (s, 2H), 4.73 (brs, 4H), 3.71 - 3.61 (m, 6H), 2.53 (brs, 4H); **LRMS** (ES) m/z 580.3 (M⁺ + 1).

### Example 351: Synthesis of Compound 351, 3-((5-(5-(difluoromethyl)-1,3,4-oxadiazole-2-yl)pyridine-2-yl)methyl)-5-fluoro-6-(6-(4-(2,2,2-trifluo roethyl)piperazine-1-yl)pyridine-3-yl)benzo[d]oxazole-2(3H)-one

### [Step 1] Synthesis of the compound 351

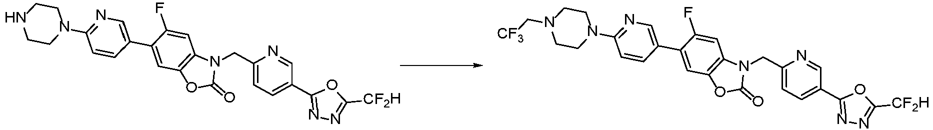

3-((5-(5-(difluoromethyl)-1,3,4-oxadiazole-2-yl)pyridine-2-yl)methyl)-5-fluoro-6-(6-(pipera zine-1-yl)pyridine-3-yl)benzo[d]oxazole-2(3H)-one (0.200 g, 0.382 mmol), 2,2,2-trifluoroethyl trifluoromethanesulfonate (0.133 g, 0.573 mmol) and potassium carbonate (0.106 g, 0.764 mmol) were dissolved in acetonitrile (5 mL) at room temperature, after which the resulting solution was heated under reflux for 18 hours, and then a reaction was finished by lowering a temperature to room temperature. Saturated sodium hydrogen carbonate aqueous solution was poured into the reaction mixture and an extraction was performed with dichloromethane. An organic layer was washed with saturated sodium chloride aqueous solution, then dehydrated with anhydrous magnesium sulfate, then filtered, and then concentrated under reduced pressure. The resulting concentrate was purified via column chromatography (SiO₂, 4 g cartridge; ethyl acetate/hexane = 10 to 50%) and concentrated to obtain a title compound (0.028 g, 12.1%) in a yellow solid form.

**¹H NMR** (400 MHz, CDCl₃) δ 9.33 (d, *J =* 1.8 Hz, 1H), 8.44 (dd, *J* = 8.2, 2.0 Hz, 1H), 8.33 (s, 1H), 7.65 (d, *J* = 8.5 Hz, 1H), 7.59 (d, *J =* 8.2 Hz, 1H), 7.25 (d, *J* = 6.0 Hz, 1H), 7.09 - 6.83 (m, 2H), 6.73 (d, *J* = 8.9 Hz, 1H), 5.23 (s, 2H), 3.66 - 3.65 (m, 4H), 3.06 (q, *J* = 9.5 Hz, 2H), 2.82 (t, *J* = 4.9 Hz, 4H); **LRMS** (ES) m/z 606.2 (M⁺ + 1).

### Example 352: Synthesis of Compound 352, tert-butyl 4-(4-(3-((5-(5-(difluoromethyl)-1,3,4-oxadiazole-2-yl)pyridine-2-yl)methyl)-2-oxo-2,3-dihydrobenz o[d]thiazole-6-yl)-1H-pyrazole-1-yl)piperidine-1-carboxylate

### [Step 1] Synthesis of methyl 6-((6-bromo-2-oxobenzo[d]thiazole-3(2H)-yl)methyl)nicotinate

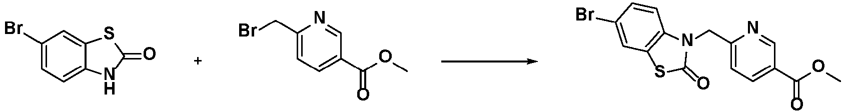

6-bromobenzo[d]thiazole-2(3H)-one (1.000 g, 4.346 mmol), methyl 6-(bromomethyl)nicotinate (2.000 g, 8.693 mmol), potassium carbonate (1.201 g, 8.693 mmol) and potassium iodide (0.072 g, 0.435 mmol) were dissolved in N,N-dimethylformamide (15 mL) at room temperature, after which the resulting solution was stirred at 100°C for 18 hours, and then a reaction was finished by lowering a temperature to room temperature. Saturated sodium hydrogen carbonate aqueous solution was poured into the reaction mixture and an extraction was performed with dichloromethane. An organic layer was washed with saturated sodium chloride aqueous solution, then dehydrated with anhydrous magnesium sulfate, then filtered, and then concentrated under reduced pressure. The resulting concentrate was purified via column chromatography (SiO₂, 24 g cartridge; ethyl acetate/hexane = 5 to 15%) and concentrated to obtain a product. Then, diethylether was inserted into the resulting product and stirred to filter out a precipitated solid, then washed with diethylether, and then dried to obtain a title compound (0.700 g, 42.5%) in a light yellow solid form.

### [Step 2] Synthesis of methyl 6-((6-(1-(1-(tert-butoxycarbonyl)piperidine-4-yl)-1H-pyrazole-4-yl)-2-oxobenzo[d]thiazole-3(2H)-y l)methyl)nicotinate

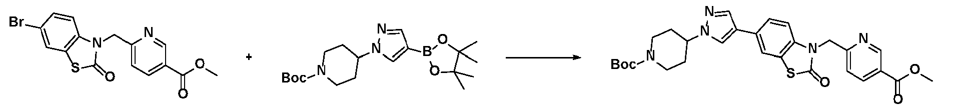

The methyl 6-((6-bromo-2-oxobenzo[d]thiazole-3(2H)-yl)methyl)nicotinate (0.700 g, 1.846 mmol) prepared in the step 1, tert-butyl 4-(4-(4,4,5,5-tetramethyl-1,3,2-dioxaborolane-2-yl)-1H-pyrazole-1-yl)piperidine-1-carboxylate (0.836 g, 2.215 mmol), [1,1'-bis(di-tert-butylphosphino)ferrocene]palladium(II) dichloride (Pd(dtbpf)Cl₂, 0.060 g, 0.092 mmol) and cesium carbonate (1.203 g, 3.692 mmol) were dissolved in 1,4-dioxane (12 mL)/water (3 mL) at room temperature, after which the resulting solution was stirred at 100°C for 18 hours, and then a reaction was finished by lowering a temperature to room temperature. Saturated sodium hydrogen carbonate aqueous solution was poured into the reaction mixture and an extraction was performed with dichloromethane. An organic layer was washed with saturated sodium chloride aqueous solution, then dehydrated with anhydrous magnesium sulfate, then filtered, and then concentrated under reduced pressure. The resulting concentrate was purified via column chromatography (SiO₂, 12 g cartridge; ethyl acetate/hexane = 20 to 60%) and concentrated to obtain a title compound (0.920 g, 90.7%) in a white solid form.

### [Step 3] Synthesis of tert-butyl 4-(4-(3-((5-(hydrazinecarbonyl)pyridine-2-yl)methyl)-2-oxo-2,3-dihydrobenzo[d]thiazole-6-yl)-1H-pyrazole-1-yl)piperidine-1-carboxylate

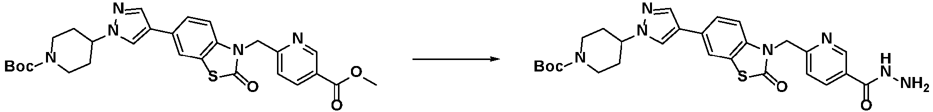

The methyl 6-((6-(1-(1-(tert-butoxycarbonyl)piperidine-4-yl)-1H-pyrazole-4-yl)-2-oxobenzo[d]thiazole-3(2H)-y l)methyl)nicotinate (0.920 g, 1.674 mmol) prepared in the step 2 and hydrazine monohydrate (0.813 mL, 16.738 mmol) were dissolved in ethanol (10 mL) at room temperature, after which the resulting solution was stirred at 80°C for 18 hours, and then a reaction was finished by lowering a temperature to room temperature. A precipitated solid was filtered, then washed with ethanol, and then dried to obtain a title compound (0.717 g, 77.9%) in a white solid form.

### [Step 4] Synthesis of the compound 352

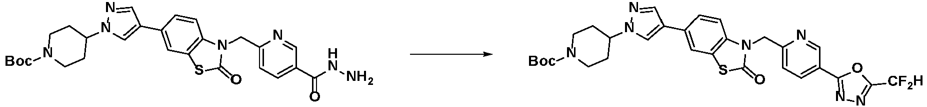

The tert-butyl 4-(4-(3-((5-(hydrazinecarbonyl)pyridine-2-yl)methyl)-2-oxo-2,3-dihydrobenzo[d]thiazole-6-yl)-1H-pyrazole-1-yl)piperidine-1-carboxylate (0.717 g, 1.304 mmol) prepared in the step 3 and imidazole (0.266 g, 3.913 mmol) were dissolved in dichloromethane (30 mL) at room temperature, after which 2,2-difluoroacetic anhydride (0.487 mL, 3.913 mmol) was added into the resulting solution, then heated under reflux for 18 hours, and then a reaction was finished by lowering a temperature to room temperature. Saturated sodium hydrogen carbonate aqueous solution was poured into the reaction mixture and an extraction was performed with dichloromethane. An organic layer was washed with saturated sodium chloride aqueous solution, then dehydrated with anhydrous magnesium sulfate, then filtered, and then concentrated under reduced pressure. The resulting concentrate was purified via column chromatography (SiO₂, 12 g cartridge; ethyl acetate/hexane = 5 to 40%) and concentrated to obtain a title compound (0.856 g, 107.6%) in a light yellow solid form.

**¹H NMR** (400 MHz, CDCl₃) δ 9.32 (d, *J =* 1.9 Hz, 1H), 8.37 (dd, *J* = 8.2, 2.2 Hz, 1H), 7.74 (s, 1H), 7.64 (s, 1H), 7.57 (d, *J =* 1.6 Hz, 1H), 7.47 (d, *J =* 8.2 Hz, 1H), 7.36 (dd, *J* = 8.4, 1.7 Hz, 1H), 7.10 (d, *J* = 8.4 Hz, 1H), 7.08 - 6.82 (m, 1H), 5.38 (s, 2H), 4.33 - 4.27 (m, 1H), 2.98 - 2.90 (m, 2H), 2.18 (d, *J =* 10.8 Hz, 2H), 1.97 - 1.93 (m, 2H), 1.50 (s, 9H); **LRMS** (ES) m/z 610.3 (M⁺ + 1).

### Example 353: Synthesis of Compound 353, 3-((5-(5-(difluoromethyl)-1,3,4-oxadiazole-2-yl)pyridine-2-yl)methyl)-6-(1-(1-methylpiperidine-4-yl )-1H-pyrazole-4-yl)benzo[d]thiazole-2(3H)-one

### [Step 1] Synthesis of the compound 353

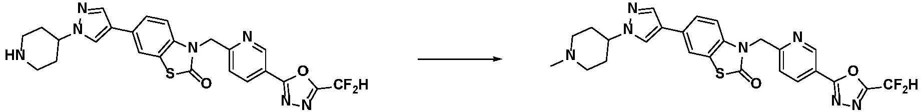

3-((5-(5-(difluoromethyl)-1,3,4-oxadiazole-2-yl)pyridine-2-yl)methyl)-6-(1-(piperidine-4-yl )-1H-pyrazole-4-yl)benzo[d]thiazole-2(3H)-one (0.100 g, 0.196 mmol) and formaldehyde (38.00% solution, 0.022 mL, 0.294 mmol) were dissolved in dichloromethane (4 mL) at room temperature, after which sodium triacetoxyborohydride (0.083 g, 0.393 mmol) was added into the resulting solution and stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.068 g, 66.2%) in a light yellow solid form.

**¹H NMR** (400 MHz, CDCl₃) δ 9.28 (s, 1H), 8.33 (dd, *J* = 8.2, 1.6 Hz, 1H), 7.70 (s, 1H), 7.64 (s, 1H), 7.54 (s, 1H), 7.44 (d, *J* = 8.2 Hz, 1H), 7.33 (d, *J* = 8.4 Hz, 1H), 7.07 (s, 1H), 7.05 - 6.81 (m, 1H), 5.35 (s, 2H), 4.20 - 4.16 (m, 1H), 3.04 (d, *J =* 11.6 Hz, 1H), 2.36 (s, 3H), 2.26 - 2.15 (m, 6H); **LRMS** (ES) m/z 524.3 (M⁺ + 1).

### Example 354: Synthesis of Compound 354, 3-((5-(5-(difluoromethyl)-1,3,4-oxadiazole-2-yl)pyridine-2-yl)methyl)-6-(1-(1-isopropylpiperidine-4 -yl)-1H-pyrazole-4-yl)benzo[d]thiazole-2(3H)-one

### [Step 1] Synthesis of the compound 354

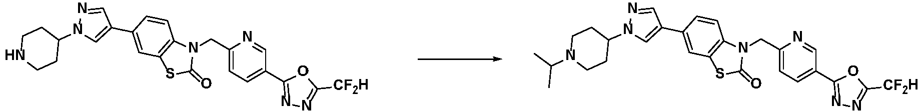

3-((5-(5-(difluoromethyl)-1,3,4-oxadiazole-2-yl)pyridine-2-yl)methyl)-6-(1-(piperidine-4-yl )-1H-pyrazole-4-yl)benzo[d]thiazole-2(3H)-one (0.100 g, 0.196 mmol) and sodium triacetoxyborohydride (0.083 g, 0.393 mmol) were dissolved in dichloromethane (4 mL) at room temperature, after which acetone (0.022 mL, 0.294 mmol) was added into the resulting solution and stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.025 g, 23.1%) in a light orange solid form.

**¹H NMR** (400 MHz, CDCl₃) δ 9.32 (d, *J =* 1.9 Hz, 1H), 8.36 (dd, *J* = 8.2, 2.2 Hz, 1H), 7.72 (s, 1H), 7.66 (s, 1H), 7.56 (d, *J =* 1.6 Hz, 1H), 7.46 (d, *J =* 8.2 Hz, 1H), 7.35 (dd, *J* = 8.4, 1.6 Hz, 1H), 7.10 - 6.82 (m, 2H), 5.38 (s, 2H), 4.19 - 4.15 (m, 1H), 3.09 - 3.08 (m, 2H), 2.89 - 2.88 (m, 2H), 2.41 - 2.40 (m, 2H), 2.26 - 2.24 (m, 2H), 2.08 - 2.06 (m, 2H), 1.14 (d, *J =* 6.0 Hz, 6H); **LRMS** (ES) m/z 552.4 (M⁺ + 1).

### Example 355: Synthesis of Compound 355, 3-((5-(5-(difluoromethyl)-1,3,4-oxadiazole-2-yl)pyridine-2-yl)methyl)-6-(1-(1-(oxetan-3-yl)piperidi ne-4-yl)-1H-pyrazole-4-yl)benzo[d]thiazole-2(3H)-on

### [Step 1] Synthesis of the compound 355

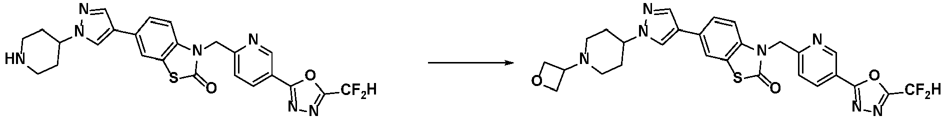

3-((5-(5-(difluoromethyl)-1,3,4-oxadiazole-2-yl)pyridine-2-yl)methyl)-6-(1-(piperidine-4-yl )-1H-pyrazole-4-yl)benzo[d]thiazole-2(3H)-one (0.100 g, 0.196 mmol) and sodium triacetoxyborohydride (0.083 g, 0.393 mmol) were dissolved in dichloromethane (4 mL) at room temperature, after which 3-oxetanone (0.019 mL, 0.294 mmol) was added into the resulting solution and stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; ethyl acetate/hexane = 50 to 90%) and concentrated to obtain a title compound (0.045 g, 40.5%) in a light yellow solid form.

**¹H NMR** (400 MHz, CDCl₃) δ 9.31 (d, *J =* 1.9 Hz, 1H), 8.36 (dd, *J* = 8.2, 2.2 Hz, 1H), 7.73 (s, 1H), 7.65 (s, 1H), 7.56 (d, *J =* 1.4 Hz, 1H), 7.47 (d, *J =* 8.2 Hz, 1H), 7.35 (dd, *J* = 8.4, 1.6 Hz, 1H), 7.10 - 6.82 (m, 2H), 5.38 (s, 2H), 4.72 - 4.64 (m, 4H), 4.21 - 4.17 (m, 1H), 3.58 - 3.55 (m, 1H), 2.93 - 2.91 (m, 2H), 2.26 - 2.23 (m, 2H), 2.16 - 2.06 (m, 4H); LRMS (ES) m/z 566.3 (M⁺ + 1).

### Example 356: Synthesis of Compound 356, 3-((5-(5-(difluoromethyl)-1,3,4-oxadiazole-2-yl)pyridine-2-yl)methyl)-6-(1-(1-(2,2,2-trifluoroethyl)p iperidine-4-yl)-1H-pyrazole-4-yl)benzo[d]thiazole-2(3H)-one

### [Step 1] Synthesis of the compound 356

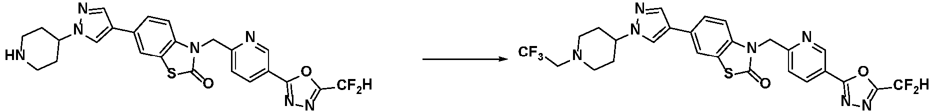

3-((5-(5-(difluoromethyl)-1,3,4-oxadiazole-2-yl)pyridine-2-yl)methyl)-6-(1-(piperidine-4-yl )-1H-pyrazole-4-yl)benzo[d]thiazole-2(3H)-one (0.100 g, 0.196 mmol), 2,2,2-trifluoroethyl trifluoromethanesulfonate (0.042 mL, 0.294 mmol) and potassium carbonate (0.054 g, 0.393 mmol) were dissolved in acetonitrile (4 mL) at room temperature, after which the resulting solution was stirred at 80°C for 18 hours, and then a reaction was finished by lowering a temperature to room temperature. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; ethyl acetate/hexane = 20 to 60%) and concentrated to obtain a title compound (0.075 g, 64.6%) in a light yellow solid form.

**¹H NMR** (400 MHz, CDCl₃) δ 9.32 (d, *J =* 2.0 Hz, 1H), 8.37 (dd, *J* = 8.2, 2.1 Hz, 1H), 7.73 (s, 1H), 7.65 (s, 1H), 7.57 (s, 1H), 7.47 (d, *J =* 8.2 Hz, 1H), 7.36 (dd, *J* = 8.4, 1.3 Hz, 1H), 7.11 - 6.82 (m, 2H), 5.39 (s, 2H), 4.21 - 4.15 (m, 1H), 3.15 - 3.04 (m, 4H), 2.62 (t, *J =* 10.9 Hz, 2H), 2.20 - 2.07 (m, 4H); **LRMS** (ES) m/z 592.2 (M⁺ + 1).

### Example 357: Synthesis of Compound 357, 3-((5-(5-(difluoromethyl)-1,3,4-oxadiazole-2-yl)pyridine-2-yl)methyl)-6-(1-(1-((1-fluorocyclopropyl )methyl)piperidine-4-yl)-1H-pyrazole-4-yl)benzo[d]thiazole-2(3H)-one

### [Step 1] Synthesis of the compound 357

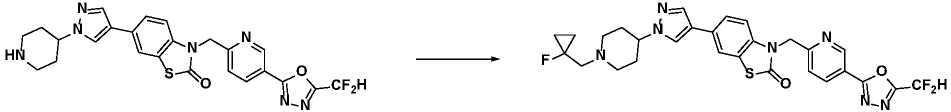

3-((5-(5-(difluoromethyl)-1,3,4-oxadiazole-2-yl)pyridine-2-yl)methyl)-6-(1-(piperidine-4-yl )-1H-pyrazole-4-yl)benzo[d]thiazole-2(3H)-one (0.100 g, 0.196 mmol) and 1-fluorocyclopropane-1-carbaldehyde (0.026 g, 0.294 mmol) were dissolved in dichloromethane (4 mL) at room temperature, after which sodium triacetoxyborohydride (0.083 g, 0.393 mmol) was added into the resulting solution and stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; ethyl acetate/hexane = 50 to 90%) and concentrated to obtain a title compound (0.067 g, 58.7%) in a light yellow solid form.

**¹H NMR** (400 MHz, CDCl₃) δ 9.30 (s, 1H), 8.35 (d, *J =* 8.2 Hz, 2H), 7.72 (s, 1H), 7.65 (s, 1H), 7.55 (s, 1H), 7.45 (d, *J =* 8.2 Hz, 1H), 7.35 (d, *J =* 8.4 Hz, 1H), 7.09 - 6.82 (m, 2H), 5.37 (s, 2H), 4.21 - 4.16 (m, 1H), 3.23 (d, *J =* 11.6 Hz, 2H), 2.82 (d, *J =* 21.4 Hz, 2H), 2.36 (t, *J =* 11.3 Hz, 2H), 2.22 - 2.16 (m, 2H), 2.13 - 2.05 (m, 2H), 1.15 - 1.07 (m, 2H), 0.68 - 0.62 (m, 2H); LRMS (ES) m/z 582.3 (M⁺ + 1).

### Example 358: Synthesis of Compound 358, tert-butyl 4-(4-(3-((5-(5-(difluoromethyl)-1,3,4-oxadiazole-2-yl)pyridine-2-yl)methyl)-5-fluoro-2-oxo-2,3-dihy drobenzo[d]oxazole-6-yl)-1H-pyrazole-1-yl)piperidine-1-carboxylate

### [Step 1] Synthesis of the compound 358

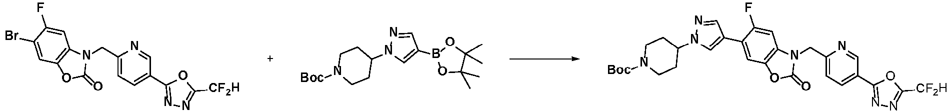

6-bromo-3-((5-(5-(difluoromethyl)-1,3,4-oxadiazole-2-yl)pyridine-2-yl)methyl)-5-fluorobe nzo[d]oxazole-2(3H)-one (2.000 g, 4.534 mmol), tert-butyl 4-(4-(4,4,5,5-tetramethyl-1,3,2-dioxaborolane-2-yl)-1H-pyrazole-1-yl)piperidine-1-carboxylate (2.053 g, 5.440 mmol), [1,1'-bis(di-tert-butylphosphino)ferrocene]palladium(II) dichloride (Pd(dtbpf)Cl₂, 0.148 g, 0.227 mmol) and cesium carbonate (2.954 g, 9.067 mmol) were mixed in 1,4-dioxane (20 mL)/water (5 mL) at room temperature, after which the resulting mixture was irradiated with microwave, then heated at 100°C for 20 minutes, and then a reaction was finished by lowering a temperature to room temperature. Solvent was removed from the reaction mixture under reduced pressure, after which the resulting concentrate was purified via column chromatography (SiO₂, 40 g cartridge; ethyl acetate/hexane = 20 to 70%) and concentrated to obtain a title compound (0.491 g, 17.7%) in a light orange solid form.

**¹H NMR** (400 MHz, CDCl₃) δ 9.33 (d, *J =* 1.8 Hz, 1H), 8.44 (dd, *J* = 8.2, 2.2 Hz, 1H), 7.83 (s, 1H), 7.78 (d, *J* = 2.2 Hz, 1H), 7.58 (d, *J =* 8.2 Hz, 1H), 7.39 (d, *J* = 5.9 Hz, 1H), 7.08 - 6.83 (m, 2H), 5.21 (s, 2H), 4.36 - 4.30 (m, 1H), 2.96 - 2.89 (m, 2H), 2.19 (d, *J =* 10.3 Hz, 2H), 2.00 - 1.96 (m, 2H), 1.51 (s, 9H); **LRMS** (ES) m/z 612.2 (M⁺ + 1).

### Example 359: Synthesis of Compound 359, 3-((5-(5-(difluoromethyl)-1,3,4-oxadiazole-2-yl)pyridine-2-yl)methyl)-5-fluoro-6-(1-(piperidine-4-yl )-1H-pyrazole-4-yl)benzo[d]oxazole-2(3H)-one

### [Step 1] Synthesis of the compound 359

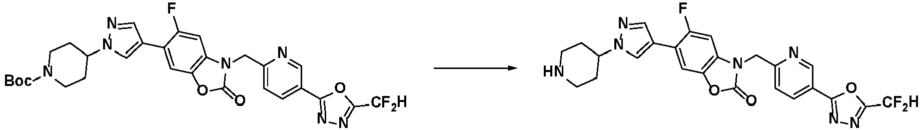

Tert-butyl 4-(4-(3-((5-(5-(difluoromethyl)-1,3,4-oxadiazole-2-yl)pyridine-2-yl)methyl)-5-fluoro-2-oxo-2,3-dihy drobenzo[d]oxazole-6-yl)-1H-pyrazole-1-yl)piperidine-1-carboxylate (0.591 g, 0.966 mmol) and trifluoroacetic acid (0.370 mL, 4.832 mmol) were dissolved in dichloromethane (5 mL) at room temperature, after which the resulting solution was stirred at the same temperature for 6 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. A title compound was used without an additional purification process (0.515 g, 104.2%, light brown solid).

**¹H NMR** (400 MHz, DMSO-d₆) δ 9.14 (d, *J* = 1.9 Hz, 1H), 8.47 (dd, *J* = 8.2, 2.1 Hz, 1H), 8.18 (s, 1H), 7.94 (s, 1H), 7.81 (d, *J* = 6.1 Hz, 1H), 7.74 (d, *J =* 8.2 Hz, 1H), 7.70 - 7.45 (m, 1H), 7.31 (d, *J =* 10.4 Hz, 1H), 5.32 (s, 2H), 4.43 - 4.40 (m, 1H), 3.36 - 3.27 (m, 2H), 2.90 - 2.88 (m, 2H), 2.12 (d, *J* = 11.9 Hz, 1H), 2.02 - 1.99 (m, 2H); **LRMS** (ES) m/z 512.0 (M⁺ + 1).

### Example 360: Synthesis of Compound 360, 3-((5-(5-(difluoromethyl)-1,3,4-oxadiazole-2-yl)pyridine-2-yl)methyl)-5-fluoro-6-(1-(1-methylpiperi dine-4-yl)-1H-pyrazole-4-yl)benzo[d]oxazole-2(3H)-one

### [Step 1] Synthesis of the compound 360

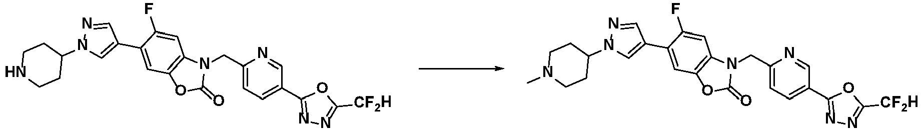

3-((5-(5-(difluoromethyl)-1,3,4-oxadiazole-2-yl)pyridine-2-yl)methyl)-5-fluoro-6-(1-(piperi dine-4-yl)-1H-pyrazole-4-yl)benzo[d]oxazole-2(3H)-one (0.100 g, 0.196 mmol) and formaldehyde (38.00% solution, 0.021 mL, 0.293 mmol) were dissolved in dichloromethane (5 mL) at room temperature, after which sodium triacetoxyborohydride (0.083 g, 0.391 mmol) was added into the resulting solution and stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 10%) and concentrated to obtain a title compound (0.023 g, 22.4%) in a light yellow solid form.

**¹H NMR** (400 MHz, CDCl₃) δ 9.32 (s, 1H), 8.43 (dd, *J* = 8.1, 1.6 Hz, 1H), 7.81 - 7.80 (m, 2H), 7.58 (d, *J* = 8.2 Hz, 1H), 7.38 (d, *J* = 5.8 Hz, 1H), 7.08 - 6.83 (m, 2H), 5.21 (s, 2H), 4.26 - 4.25 (m, 1H), 3.15 - 3.14 (m, 2H), 2.46 (s, 3H), 2.36 - 2.19 (m, 6H); **LRMS** (ES) m/z 526.2 (M+ + 1).

### Example 361: Synthesis of Compound 361, 3-((5-(5-(difluoromethyl)-1,3,4-oxadiazole-2-yl)pyridine-2-yl)methyl)-5-fluoro-6-(1-(1-isopropylpip eridine-4-yl)-1H-pyrazole-4-yl)benzo[d]oxazole-2(3H)-one

### [Step 1] Synthesis of the compound 361

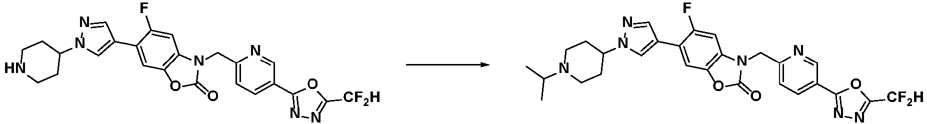

3-((5-(5-(difluoromethyl)-1,3,4-oxadiazole-2-yl)pyridine-2-yl)methyl)-5-fluoro-6-(1-(piperi dine-4-yl)-1H-pyrazole-4-yl)benzo[d]oxazole-2(3H)-one (0.100 g, 0.196 mmol) and acetone (0.022 mL, 0.293 mmol) were dissolved in dichloromethane (5 mL) at room temperature, after which sodium triacetoxyborohydride (0.083 g, 0.391 mmol) was added into the resulting solution and stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.018 g, 16.6%) in a light yellow solid form.

**¹H NMR** (400 MHz, CDCl₃) δ 9.32 (d, *J =* 1.9 Hz, 1H), 8.43 (dd, *J* = 8.2, 2.1 Hz, 1H), 7.81 - 7.80 (m, 2H), 7.57 (d, *J* = 8.2 Hz, 1H), 7.38 (d, *J =* 5.8 Hz, 1H), 7.08 - 6.82 (m, 2H), 5.21 (s, 2H), 4.20 - 4.15 (m, 1H), 3.51 - 3.49 (m, 2H), 3.11 - 3.08 (m, 1H), 2.37 - 2.36 (m, 2H), 2.25 - 2.19 (m, 2H), 2.13 - 2.11 (m, 2H), 1.32 - 1.13 (m, 6H); **LRMS** (ES) m/z 554.5 (M⁺ + 1).

### Example 362: Synthesis of Compound 362, 3-((5-(5-(difluoromethyl)-1,3,4-oxadiazole-2-yl)pyridine-2-yl)methyl)-5-fluoro-6-(1-(1-(oxetan-3-yl )piperidine-4-yl)-1H-pyrazole-4-yl)benzo[d]oxazole-2(3H)-one

### [Step 1] Synthesis of the compound 362

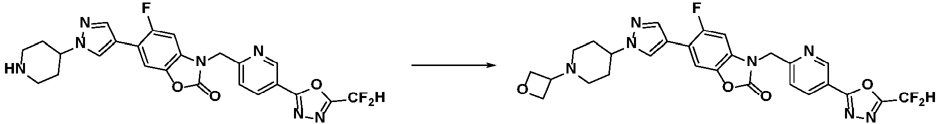

3-((5-(5-(difluoromethyl)-1,3,4-oxadiazole-2-yl)pyridine-2-yl)methyl)-5-fluoro-6-(1-(piperi dine-4-yl)-1H-pyrazole-4-yl)benzo[d]oxazole-2(3H)-one (0.100 g, 0.196 mmol) and 3-oxetanone (0.019 mL, 0.293 mmol) were dissolved in dichloromethane (5 mL) at room temperature, after which sodium triacetoxyborohydride (0.083 g, 0.391 mmol) was added into the resulting solution and stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 2.5%) and concentrated to obtain a title compound (0.056 g, 5.1%) in a light yellow solid form.

**¹H NMR** (400 MHz, CDCl₃) δ 9.32 (d, *J =* 1.8 Hz, 1H), 8.43 (dd, *J* = 8.2, 2.2 Hz, 1H), 7.81 - 7.80 (m, 2H), 7.58 (d, *J* = 8.2 Hz, 1H), 7.38 (d, *J =* 5.9 Hz, 1H), 7.08 - 6.83 (m, 2H), 5.32 (s, 2H), 4.72 - 4.64 (m, 4H), 4.24 - 4.19 (m, 1H), 3.58 - 3.49 (m, 1H), 2.91 (d, *J =* 9.6 Hz, 1H), 2.23 - 2.22 (m, 2H), 2.18 - 2.03 (m, 4H); **LRMS** (ES) m/z 568.4 (M⁺ + 1).

### Example 363: Synthesis of Compound 363, 3-((5-(5-(difluoromethyl)-1,3,4-oxadiazole-2-yl)pyridine-2-yl)methyl)-5-fluoro-6-(1-(1-(2,2,2-trifluo roethyl)piperidine-4-yl)-1H-pyrazole-4-yl)benzo[d]oxazole-2(3H)-one

### [Step 1] Synthesis of the compound 363

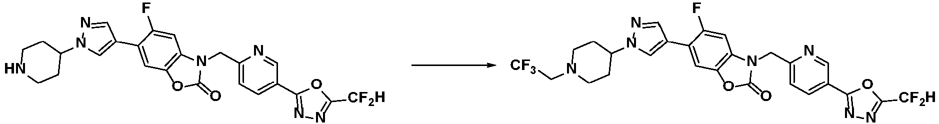

3-((5-(5-(difluoromethyl)-1,3,4-oxadiazole-2-yl)pyridine-2-yl)methyl)-5-fluoro-6-(1-(piperi dine-4-yl)-1H-pyrazole-4-yl)benzo[d]oxazole-2(3H)-one (0.100 g, 0.196 mmol), 2,2,2-trifluoroethyl trifluoromethanesulfonate (0.042 mL, 0.293 mmol) and potassium carbonate (0.054 g, 0.391 mmol) were dissolved in acetonitrile (4 mL) at room temperature, after which the resulting solution was stirred at 80°C for 18 hours, and then a reaction was finished by lowering a temperature to room temperature. Solvent was removed from the reaction mixture under reduced pressure, after which water was poured into a resulting concentrate, and an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; ethyl acetate/hexane = 10 to 40%) and concentrated to obtain a title compound (0.006 g, 5.2%) in a yellow solid form.

**¹H NMR** (400 MHz, CDCl₃) δ 9.33 (d, *J =* 1.8 Hz, 1H), 8.43 (dd, *J* = 8.2, 2.1 Hz, 1H), 7.82 (s, 1H), 7.79 (d, *J =* 2.2 Hz, 1H), 7.58 (d, *J =* 8.2 Hz, 1H), 7.39 (d, *J =* 5.9 Hz, 1H), 7.08 - 6.83 (m, 2H), 5.21 (s, 2H), 4.21 - 4.20 (m, 1H), 3.16 - 3.05 (m, 4H), 2.63 (t, *J* = 10.5 Hz, 1H), 2.19 - 2.12 (m, 4H); **LRMS** (ES) m/z 594.3 (M⁺ + 1).

### Example 364: Synthesis of Compound 364, 3-((5-(5-(difluoromethyl)-1,3,4-oxadiazole-2-yl)pyridine-2-yl)methyl)-5-fluoro-6-(2-(4-methylpiper azine-1-yl)pyridine-4-yl)benzo[d]oxazole-2(3H)-one

### [Step 1] Synthesis of the compound 364

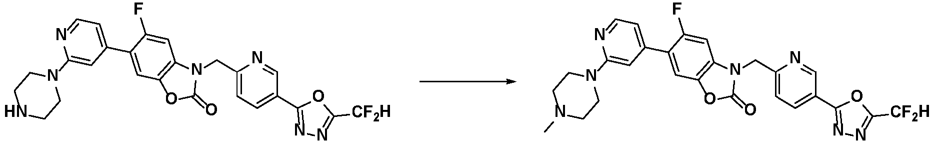

3-((5-(5-(difluoromethyl)-1,3,4-oxadiazole-2-yl)pyridine-2-yl)methyl)-6-(2-(piperazine-1-yl )pyridine-4-yl)benzo[d]thiazole-2(3H)-one (0.100 g, 0.191 mmol) and formaldehyde (38.00% solution, 0.021 mL, 0.287 mmol) were dissolved in dichloromethane (5 mL) at room temperature, after which sodium triacetoxyborohydride (0.081 g, 0.382 mmol) was added into the resulting solution and stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.072 g, 70.1%) in a white solid form.

**¹H NMR** (400 MHz, CDCl₃) δ 9.32 (s, 1H), 8.44 (dd, *J* = 8.2, 2.1 Hz, 1H), 8.24 (d, *J* = 5.6 Hz, 1H), 7.59 (d, *J* = 8.2 Hz, 1H), 7.31 (d, *J* = 5.8 Hz, 1H), 7.08 - 6.82 (m, 2H), 6.74 (brs, 2H), 5.23 (s, 2H), 3.62 (t, *J* = 4.9 Hz, 4H), 2.55 (t, *J =* 4.9 Hz, 4H), 2.37 (s, 3H); **LRMS** (ES) m/z 538.3 (M+ + 1).

### Example 365: Synthesis of Compound 365, 3-((5-(5-(difluoromethyl)-1,3,4-oxadiazole-2-yl)pyridine-2-yl)methyl)-5-fluoro-6-(2-(4-isopropylpip erazine-1-yl)pyridine-4-yl)benzo[d]oxazole-2(3H)-one

### [Step 1] Synthesis of the compound 365

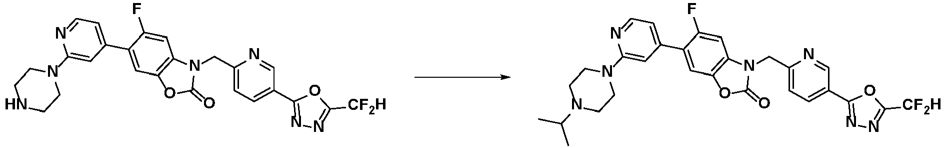

3-((5-(5-(difluoromethyl)-1,3,4-oxadiazole-2-yl)pyridine-2-yl)methyl)-6-(2-(piperazine-1-yl )pyridine-4-yl)benzo[d]thiazole-2(3H)-one (0.100 g, 0.191 mmol) and acetone (0.021 mL, 0.287 mmol) were dissolved in dichloromethane (5 mL) at room temperature, after which sodium triacetoxyborohydride (0.081 g, 0.382 mmol) was added into the resulting solution and stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 5%) and concentrated to obtain a title compound (0.072 g, 66.6%) in a white solid form.

**¹H NMR** (400 MHz, CDCl₃) δ 9.31 (d, *J =* 1.5 Hz, 1H), 8.43 (dd, *J* = 8.2, 2.1 Hz, 1H), 8.23 (d, *J* = 5.7 Hz, 1H), 7.59 (d, *J* = 8.2 Hz, 1H), 7.31 (d, *J =* 5.8 Hz, 1H), 7.08 - 7.82 (m, 2H), 6.73 (brs, 2H), 5.22 (s, 2H), 3.60 (t, *J* = 4.9 Hz, 4H), 2.76 - 2.72 (m, 1H), 2.65 (t, *J* = 4.9 Hz, 4H), 1.10 (d, *J* = 6.5 Hz, 6H); **LRMS** (ES) m/z 566.1 (M⁺ + 1).

### Example 366: Synthesis of Compound 366, 3-((5-(5-(difluoromethyl)-1,3,4-oxadiazole-2-yl)pyridine-2-yl)methyl)-5-fluoro-6-(2-(4-(oxetan-3-yl )piperazine-1-yl)pyridine-4-yl)benzo[d]oxazole-2(3H)-one

### [Step 1] Synthesis of the compound 366

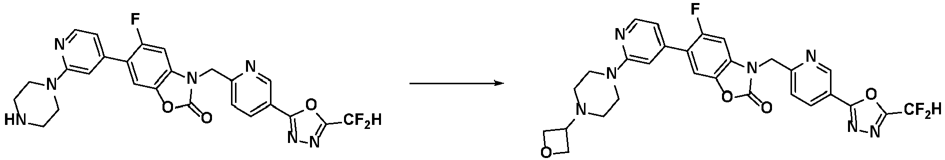

3-((5-(5-(difluoromethyl)-1,3,4-oxadiazole-2-yl)pyridine-2-yl)methyl)-6-(2-(piperazine-1-yl )pyridine-4-yl)benzo[d]thiazole-2(3H)-one (0.100 g, 0.191 mmol) and 3-oxetanone (0.018 mL, 0.287 mmol) were dissolved in dichloromethane (5 mL) at room temperature, after which sodium triacetoxyborohydride (0.081 g, 0.382 mmol) was added into the resulting solution and stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 2.5%) and concentrated to obtain a title compound (0.030 g, 27.1%) in a white solid form.

**¹H NMR** (400 MHz, CDCl₃) δ 9.32 (d, *J =* 2.0 Hz, 1H), 8.44 (dd, *J* = 8.2, 2.1 Hz, 1H), 8.25 (d, *J =* 5.2 Hz, 1H), 7.60 (d, *J* = 8.2 Hz, 1H), 7.32 (d, *J* = 5.9 Hz, 1H), 7.09 - 6.83 (m, 2H), 6.77 - 6.74 (m, 2H), 5.23 (s, 2H), 4.73 - 4.67 (m, 4H), 4.15 (t, *J =* 7.2 Hz, 4H), 3.57 - 3.54 (m, 1H), 2.47 (t, *J* = 5.0 Hz, 4H); **LRMS** (ES) m/z 580.4 (M⁺ + 1).

### Example 367: Synthesis of Compound 367, 6-(2-(4-cyclobutylpiperazine-1-yl)pyridine-4-yl)-3-((5-(5-(difluoromethyl)-1,3,4-oxadiazole-2-yl)py ridine-2-yl)methyl)-5-fluorobenzo[d]oxazole-2(3H)-one

### [Step 1] Synthesis of the compound 367

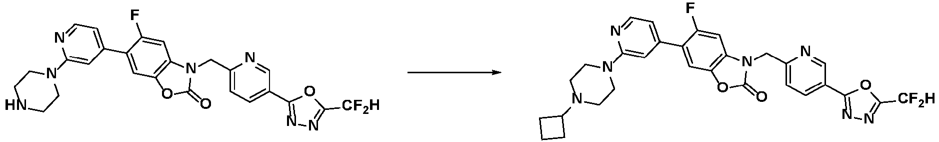

3-((5-(5-(difluoromethyl)-1,3,4-oxadiazole-2-yl)pyridine-2-yl)methyl)-6-(2-(piperazine-1-yl )pyridine-4-yl)benzo[d]thiazole-2(3H)-one (0.100 g, 0.191 mmol) and cyclobutanone (0.021 g, 0.287 mmol) were dissolved in dichloromethane (5 mL) at room temperature, after which sodium triacetoxyborohydride (0.081 g, 0.382 mmol) was added into the resulting solution and stirred at the same temperature for 18 hours. Saturated sodium hydrogen carbonate aqueous solution was poured into the reaction mixture, after which an extraction was performed with dichloromethane, then filtered via a plastic filter to remove a solid residue and an aqueous solution layer therefrom, and then concentrated under reduced pressure. The resulting concentrate was purified via column chromatography (SiO₂, 4 g cartridge; methanol/dichloromethane = 0 to 2.5%) and concentrated to obtain a title compound (0.052 g, 47.1%) in a white solid form.

**¹H NMR** (400 MHz, CDCl₃) δ 9.31 (d, *J =* 1.8 Hz, 1H), 8.44 (dd, *J* = 8.2, 2.1 Hz, 1H), 8.24 (d, *J* = 5.3 Hz, 1H), 7.59 (d, *J* = 8.2 Hz, 1H), 7.31 (d, *J* = 5.8 Hz, 1H), 7.08 - 6.82 (m, 2H), 6.74 - 6.43 (m, 2H), 5.23 (s, 2H), 3.61 (t, *J* = 4.9 Hz, 4H), 2.80 - 2.76 (m, 1H), 2.11 - 2.07 (m, 2H), 1.97 - 1.92 (m, 2H), 1.77 - 1.73 (m, 2H); **LRMS** (ES) m/z 578.4 (M⁺ + 1).

### Example 368: Synthesis of Compound 368, 3-((5-(5-(difluoromethyl)-1,3,4-oxadiazole-2-yl)pyridine-2-yl)methyl)-5-fluoro-6-(2-(piperazine-1-y I)pyridine-4-yl)benzo[d]oxazole-2(3H)-one

### [Step 1] Synthesis of tert-butyl 4-(4-(3-((5-(5-(difluoromethyl)-1,3,4-oxadiazole-2-yl)pyridine-2-yl)methyl)-5-fluoro-2-oxo-2,3-dihy drobenzo[d]oxazole-6-yl)pyridine-2-yl)piperazine-1-carboxylate

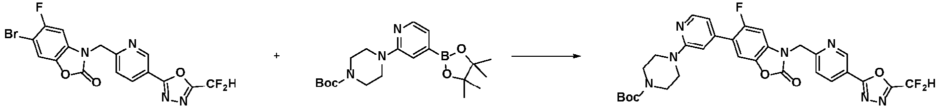

6-bromo-3-((5-(5-(difluoromethyl)-1,3,4-oxadiazole-2-yl)pyridine-2-yl)methyl)-5-fluorobe nzo[d]oxazole-2(3H)-one (2.000 g, 4.534 mmol), tert-butyl 4-(4-(4,4,5,5-tetramethyl-1,3,2-dioxaborolane-2-yl)pyridine-2-yl)piperazine-1-carboxylate (2.118 g, 5.440 mmol), [1,1'-bis(di-tert-butylphosphino)ferrocene]palladium(II) dichloride (Pd(dtbpf)Cl₂, 0.148 g, 0.227 mmol) and cesium carbonate (2.954 g, 9.067 mmol) were mixed in 1,4-dioxane (10 mL)/water (2.5 mL) at room temperature, after which the resulting mixture was irradiated with microwave, then heated at 100°C for 20 minutes, and then a reaction was finished by lowering a temperature to room temperature. Solvent was removed from the reaction mixture under reduced pressure, after which the resulting concentrate was purified via column chromatography (SiO₂, 24 g cartridge; ethyl acetate/hexane = 5 to 20%) and concentrated to obtain a title compound (1.260 g, 44.6%) in a gray solid form.

### [Step 2] Synthesis of the compound 368

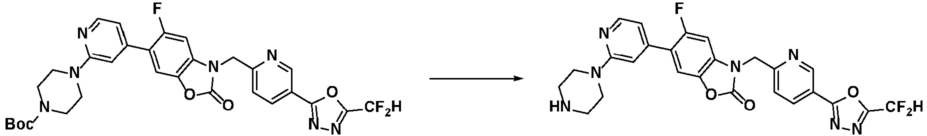

The tert-butyl 4-(4-(3-((5-(5-(difluoromethyl)-1,3,4-oxadiazole-2-yl)pyridine-2-yl)methyl)-5-fluoro-2-oxo-2,3-dihy drobenzo[d]oxazole-6-yl)pyridine-2-yl)piperazine-1-carboxylate (1.000 g, 1.604 mmol) prepared in the step 1 and trifluoroacetic acid (0.982 mL, 12.829 mmol) were dissolved in dichloromethane (10 mL) at room temperature, after which the resulting solution was stirred at the same temperature for 5 hours. Saturated sodium hydrogen carbonate aqueous solution and dichloromethane were put into the reaction mixture and stirred to filter out a precipitated solid, then washed with dichloromethane, and then dried to obtain a title compound (0.830 g, 98.9%) in a pink solid form.

**¹H NMR** (400 MHz, DMSO-d₆) δ 9.14 (d, *J* = 1.8 Hz, 1H), 8.49 (dd, *J* = 8.2, 2.2 Hz, 1H), 8.22 (d, *J* = 5.2 Hz, 1H), 7.77 (d, *J* = 8.3 Hz, 1H), 7.73 - 7.45 (m, 2H), 7.41 (d, *J* = 10.2 Hz, 1H), 7.04 (s, 1H), 6.92 (d, *J* = 4.8 Hz, 1H), 5.38 (s, 2H), 3.75 (brs, 4H), 3.17 (brs, 4H); LRMS (ES) m/z 524.1 (M⁺ + 1).

### Protocol for measuring and analyzing the activity of the inventive compound

### <Experimental Example 1> Identification of HDAC enzyme activity inhibition (in vitro)

A selective HDAC6 inhibitor is important for selectivity of HDAC1 inhibition, which is a cause of side effects, and thus HDAC1/6 enzyme selectivity and cell selectivity (HDAC1: histone acetylation/HDAC6: tubulin acetylation) were identified.

### 1. Experimental method

A HDAC enzyme inhibitory capacity of a test material was measured by using HDAC1 Fluorimetric Drug Discovery Assay Kit (Enzolifesciences: BML-AK511) and HDAC6 human recombinant (Calbiochem: 382180). For a HDAC1 assay, samples were treated at a concentration of 100, 1000 and 10000 nM. For a HDAC6 assay, samples were treated at a concentration of 0.1, 1, 10, 100 and 1000 nM. After the above sample treatment, a reaction was continued at 37°C for 60 minutes, then treated with a developer, and then subjected to a reaction at 37°C for 30 minutes, after which fluorescence intensity (Ex 390, Em 460) was measured by using FlexStatin3 (Molecular device).

### 2. Experimental results

The results of measuring HDAC enzyme inhibitory activity obtained according to the above experimental method are shown in a following table 2.

**[Table 2]**

| **Compound** | **HDAC6 IC₅₀ (µM)** | **HDAC1 IC₅₀ (µM)** | **Compound** | **HDAC6 IC₅₀ (µM)** | **HDAC1 IC₅₀ (µM)** |
|---|---|---|---|---|---|
| **1** | 0.236 | >10 | **185** | 0.202 | >10 |
| **2** | 0.203 | >10 | **186** | 0.554 | >10 |
| **3** | 0.115 | >10 | **187** | 0.064 | >10 |
| **4** | 0.178 | >10 | **188** | 0.030 | >10 |
| **5** | 0.340 | >10 | **189** | 0.037 | >10 |
| **6** | 0.203 | >10 | **190** | 0.046 | >10 |
| **7** | 0.264 | >10 | **191** | 0.037 | >10 |
| **8** | 0.171 | >10 | **192** | 0.079 | >10 |
| **9** | 0.332 | >10 | **193** | 0.048 | >10 |
| **10** | 0.222 | >10 | **194** | 0.084 | >10 |
| **11** | 0.241 | >10 | **195** | 0.050 | >10 |
| **12** | 0.160 | >10 | **196** | 0.126 | >10 |
| **13** | 0.161 | >10 | **197** | 0.235 | >10 |
| **14** | 0.209 | >10 | **198** | 0.195 | >10 |
| **15** | 0.193 | >10 | **199** | 0.175 | >10 |
| **16** | 0.239 | >10 | **200** | 0.040 | >10 |
| **17** | 0.214 | >10 | **201** | 0.070 | >10 |
| **18** | 0.267 | >10 | **202** | 0.139 | >10 |
| **19** | 2.422 | >10 | **203** | 0.105 | >10 |
| **20** | 0.285 | >10 | **204** | 0.140 | >10 |
| **21** | 0.299 | >10 | **205** | 0.739 | >10 |
| **22** | 0.109 | >10 | **206** | 0.058 | >10 |
| **23** | 0.241 | >10 | **207** | 0.173 | >10 |
| **24** | 0.137 | >10 | **208** | 0.013 | >10 |
| **25** | 0.084 | >10 | **209** | 0.017 | >10 |
| **26** | 0.209 | >10 | **210** | 0.038 | >10 |
| **27** | 0.110 | >10 | **211** | 0.048 | >10 |
| **28** | 0.064 | >10 | **212** | 0.010 | >10 |
| **29** | 0.210 | >10 | **213** | 0.014 | >10 |
| **30** | 0.444 | >10 | **214** | 0.015 | >10 |
| **31** | 0.108 | >10 | **215** | 0.020 | >10 |
| **32** | 0.228 | >10 | **216** | 0.018 | >10 |
| **33** | 0.169 | >10 | **217** | 0.006 | >10 |
| **34** | 0.235 | >10 | **218** | 0.044 | >10 |
| **35** | 0.203 | >10 | **219** | 0.033 | >10 |
| **36** | 0.131 | >10 | **220** | 0.018 | >10 |
| **37** | 0.158 | >10 | **221** | 0.008 | >10 |
| **38** | 0.202 | >10 | **222** | 0.021 | >10 |
| **39** | 0.167 | >10 | **223** | 0.016 | >10 |
| **40** | 0.155 | >10 | **224** | 0.008 | >10 |
| **41** | 0.092 | >10 | **225** | 0.011 | >10 |
| **42** | 0.106 | >10 | **226** | 0.010 | >10 |
| **43** | 0.121 | >10 | **227** | 0.032 | >10 |
| **44** | 0.076 | >10 | **228** | 0.047 | >10 |
| **45** | 0.121 | >10 | **229** | 0.312 | >10 |
| **46** | 0.103 | >10 | **230** | 0.016 | >10 |
| **47** | 0.126 | >10 | **231** | 0.009 | >10 |
| **48** | 0.022 | >10 | **232** | 0.643 | >10 |
| **49** | 0.035 | >10 | **233** | 0.107 | >10 |
| **50** | 0.039 | >10 | **234** | 0.054 | >10 |
| **51** | 0.032 | >10 | **235** | 0.103 | >10 |
| **52** | 0.034 | >10 | **236** | 0.068 | >10 |
| **53** | 0.037 | >10 | **237** | 1.137 | >10 |
| **54** | 0.039 | >10 | **238** | 0.203 | >10 |
| **55** | 0.039 | >10 | **239** | 0.051 | >10 |
| **56** | 0.034 | >10 | **240** | 0.027 | >10 |
| **57** | 0.036 | >10 | **241** | 0.094 | >10 |
| **58** | 0.028 | >10 | **242** | 0.046 | >10 |
| **59** | 0.038 | >10 | **243** | 0.017 | >10 |
| **60** | 0.031 | >10 | **244** | 0.074 | >10 |
| **61** | 0.032 | >10 | **245** | 0.684 | >10 |
| **62** | 0.259 | >10 | **246** | 0.023 | >10 |
| **63** | 0.057 | >10 | **247** | 0.009 | >10 |
| **64** | 0.044 | >10 | **248** | 0.006 | >10 |
| **65** | 0.084 | >10 | **249** | 0.018 | >10 |
| **66** | 0.072 | >10 | **250** | 0.018 | >10 |
| **67** | 0.041 | >10 | **251** | 0.374 | >10 |
| **68** | 0.034 | >10 | **252** | 0.207 | >10 |
| **69** | 0.028 | >10 | **253** | 0.271 | >10 |
| **70** | 0.031 | >10 | **254** | 0.472 | >10 |
| **71** | 0.019 | >10 | **255** | 0.048 | >10 |
| **72** | 0.026 | >10 | **256** | 0.008 | >10 |
| **73** | 0.080 | >10 | **257** | 0.012 | >10 |
| **74** | 0.070 | >10 | **258** | 0.013 | >10 |
| **75** | 0.046 | >10 | **259** | 0.014 | >10 |
| **76** | 0.031 | >10 | **260** | 0.045 | >10 |
| **77** | 0.035 | >10 | **261** | 0.057 | >10 |
| **78** | 0.079 | >10 | **262** | 0.107 | >10 |
| **79** | 0.066 | >10 | **263** | 0.059 | >10 |
| **80** | 0.061 | >10 | **264** | 0.061 | >10 |
| **81** | 0.015 | >10 | **265** | 0.046 | >10 |
| **82** | 0.023 | >10 | **266** | 0.039 | >10 |
| **83** | 0.049 | >10 | **267** | 0.042 | >10 |
| **84** | 0.036 | >10 | **268** | 0.020 | >10 |
| **85** | 0.031 | >10 | **269** | 0.006 | >10 |
| **86** | 0.028 | >10 | **270** | 0.019 | >10 |
| **87** | 0.063 | >10 | **271** | 0.027 | >10 |
| **88** | 0.038 | >10 | **272** | 0.029 | >10 |
| **89** | 0.038 | >10 | **273** | 0.014 | >10 |
| **90** | 0.031 | >10 | **274** | 0.012 | >10 |
| **91** | 0.058 | >10 | **275** | 0.016 | >10 |
| **92** | 0.062 | >10 | **276** | 0.015 | >10 |
| **93** | 0.040 | >10 | **277** | 0.021 | >10 |
| **94** | 0.045 | >10 | **278** | 0.017 | >10 |
| **95** | 0.051 | >10 | **279** | 0.015 | >10 |
| **96** | 0.036 | >10 | **280** | 0.018 | >10 |
| **97** | 0.032 | >10 | **281** | 0.018 | >10 |
| **98** | 0.028 | >10 | **282** | 0.016 | >10 |
| **99** | 0.037 | >10 | **283** | 0.023 | >10 |
| **100** | 0.039 | >10 | **284** | 0.059 | >10 |
| **101** | 0.044 | >10 | **285** | 0.033 | >10 |
| **102** | 0.059 | >10 | **286** | 0.009 | >10 |
| **103** | 0.342 | >10 | **287** | 0.014 | >10 |
| **104** | 0.283 | >10 | **288** | 0.010 | >10 |
| **105** | 0.640 | >10 | **289** | 0.004 | >10 |
| **106** | 0.391 | >10 | **290** | 0.007 | >10 |
| **107** | 0.174 | >10 | **291** | 0.009 | >10 |
| **108** | 0.148 | >10 | **292** | 0.003 | >10 |
| **109** | 0.623 | >10 | **293** | 0.008 | >10 |
| **110** | 0.234 | >10 | **294** | 0.011 | >10 |
| **111** | 0.322 | >10 | **295** | 0.010 | >10 |
| **112** | 0.864 | >10 | **296** | 0.005 | >10 |
| **113** | 0.065 | >10 | **297** | 0.010 | >10 |
| **114** | 0.088 | >10 | **298** | 0.009 | >10 |
| **115** | 0.077 | >10 | **299** | 0.013 | >10 |
| **116** | 0.084 | >10 | **300** | 0.013 | >10 |
| **117** | 0.024 | >10 | **301** | 0.029 | >10 |
| **118** | 0.042 | >10 | **302** | 0.038 | >10 |
| **119** | 0.080 | >10 | **303** | 0.029 | >10 |
| **120** | 1.313 | >10 | **304** | 0.037 | >10 |
| **121** | 3.153 | >10 | **305** | 0.050 | >10 |
| **122** | >5.00 | >10 | **306** | 0.034 | >10 |
| **123** | 0.792 | >10 | **307** | 0.399 | >10 |
| **124** | 0.950 | >10 | **308** | 0.456 | >10 |
| **125** | 0.602 | >10 | **309** | 0.053 | >10 |
| **126** | 0.029 | >10 | **310** | 0.006 | >10 |
| **127** | 0.459 | >10 | **311** | 0.012 | >10 |
| **128** | 0.798 | >10 | **312** | 0.006 | >10 |
| **129** | >3.00 | >10 | **313** | 0.165 | >10 |
| **130** | 0.288 | >10 | **314** | 0.112 | >10 |
| **131** | 0.606 | >10 | **315** | 0.118 | >10 |
| **132** | 0.281 | >10 | **316** | 0.091 | >10 |
| **133** | 0.020 | >10 | **317** | 0.015 | >10 |
| **134** | 0.020 | >10 | **318** | 0.056 | >10 |
| **135** | 0.019 | >10 | **319** | 0.039 | >10 |
| **136** | 0.012 | >10 | **320** | 0.041 | >10 |
| **137** | 0.019 | >10 | **321** | 0.053 | >10 |
| **138** | 0.019 | >10 | **322** | 0.029 | >10 |
| **139** | 0.031 | >10 | **323** | 0.035 | >10 |
| **140** | 0.054 | >10 | **324** | 0.042 | >10 |
| **141** | 0.012 | >10 | **325** | 0.070 | >10 |
| **142** | 0.018 | >10 | **326** | 0.160 | >10 |
| **143** | 0.071 | >10 | **327** | 0.058 | >10 |
| **144** | 0.077 | >10 | **328** | 0.085 | >10 |
| **145** | 0.315 | >10 | **329** | 0.127 | >10 |
| **146** | 0.130 | >10 | **330** | 0.022 | >10 |
| **147** | 0.015 | >10 | **331** | 0.061 | >10 |
| **148** | 0.061 | >10 | **332** | 0.043 | >10 |
| **149** | 0.040 | >10 | **333** | 0.038 | >10 |
| **150** | 0.043 | >10 | **334** | 0.064 | >10 |
| **151** | 0.055 | >10 | **335** | 0.075 | >10 |
| **152** | 0.034 | >10 | **336** | 0.044 | >10 |
| **153** | 0.016 | >10 | **337** | 0.672 | >10 |
| **154** | 0.042 | >10 | **338** | 0.047 | >10 |
| **155** | 0.020 | >10 | **339** | 0.052 | >10 |
| **156** | 0.102 | >10 | **340** | 0.065 | >10 |
| **157** | 0.159 | >10 | **341** | 0.208 | >10 |
| **158** | 0.138 | >10 | **342** | 0.016 | >10 |
| **159** | 0.071 | >10 | **343** | 0.019 | >10 |
| **160** | 0.092 | >10 | **344** | 0.671 | >10 |
| **161** | 0.040 | >10 | **345** | 0.022 | >10 |
| **162** | 0.039 | >10 | **346** | 0.047 | >10 |
| **163** | 0.091 | >10 | **347** | 0.062 | >10 |
| **164** | 0.061 | >10 | **348** | 0.025 | >10 |
| **165** | 0.010 | >10 | **349** | 0.055 | >10 |
| **166** | 0.034 | >10 | **350** | 0.033 | >10 |
| **167** | 0.138 | >10 | **351** | 0.151 | >10 |
| **168** | 0.040 | >10 | **352** | 0.170 | >10 |
| **169** | 0.021 | >10 | **353** | 0.019 | >10 |
| **170** | 0.211 | >10 | **354** | 0.016 | >10 |
| **171** | 0.040 | >10 | **355** | 0.023 | >10 |
| **172** | 0.034 | >10 | **356** | 0.084 | >10 |
| **173** | 0.031 | >10 | **357** | 0.042 | >10 |
| **174** | 0.050 | >10 | **358** | 0.130 | >10 |
| **175** | 0.050 | >10 | **359** | 0.033 | >10 |
| **176** | 0.042 | >10 | **360** | 0.027 | >10 |
| **177** | 0.071 | >10 | **361** | 0.023 | >10 |
| **178** | 0.020 | >10 | **362** | 0.031 | >10 |
| **179** | 0.145 | >10 | **363** | 0.075 | >10 |
| **180** | 0.038 | >10 | **364** | 0.026 | >10 |
| **181** | 0.068 | >10 | **365** | 0.033 | >10 |
| **182** | 0.118 | >10 | **366** | 0.052 | >10 |
| **183** | 0.073 | >10 | **367** | 0.312 | >10 |
| **184** | 0.137 | >10 | **368** | 0.018 | >10 |

Referring to the table 2, it may be identified that 1,3,4-oxadiazole derivative compounds according to the present invention show excellent HDAC1/6 enzyme selectivity.

## Claims

1. Compounds represented by a following chemical formula I, stereoisomers thereof or pharmaceutically acceptable salts thereof: 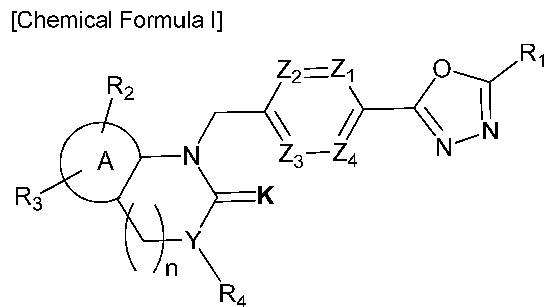 wherein,
Z₁ to Z₄ are each independently N or CRₐ, in which Rₐ is H, X, C₁-C₄ alkyl or O-(C₁-C₄ alkyl) and Rₐ can be different from each other when CRₐ is 2 or more;
K is O or S;
R₁ is CX₃ or CX₂H; 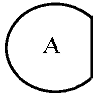 is C₆-C₁₂ arylene or C₂-C₁₀ heteroarylene;
R₂ and R₃ are each independently H, X, C₁-C₄ alkyl, C₁-C₄ haloalkyl, C₆-C₁₂ aryl, C₂-C₁₀ heteroaryl, C₃-C₁₀ cycloalkyl, C₂-C₁₀ heterocycloalkyl, C₂-C₁₀ heterocycloalkenyl, N(R_{b})(R_{c}), NH-(C₁-C₄ alkyl)-N(Rₚ)(R_{c}), NH-O-(C₁-C₄ alkyl) or NHC(=O)-R₅,
in which at least one H of C₁-C₄ alkyl, C₆-C₁₂ aryl, C₂-C₁₀ heteroaryl, C₂-C₁₀ heterocycloalkenyl or C₂-C₁₀ heterocycloalkyl can be each independently substituted with X, C₁-C₄ alkyl, C₁-C₄ haloalkyl, C₃-C₁₀ cycloalkyl, C₆-C₁₂ aryl, C₂-C₁₀ heteroaryl, C₂-C₁₀ heterocycloalkyl, (C₁-C₄ alkyl)-(C₂-C₁₀ heteroaryl), (C₁-C₄ alkyl)-(C₂-C₁₀ heterocycloalkyl), (C₂-C₁₀ heterocycloalkyl)-(C₁-C₄ alkyl), (C₂-C₁₀ heterocycloalkyl)-(C₁-C₄ haloalkyl), (C₂-C₁₀ heterocycloalkyl)-(C₃-C₁₀ cycloalkyl), (C₂-C₁₀ heterocycloalkyl)-(C₂-C₁₀ heterocycloalkyl), (C₂-C₁₀ heterocycloalkyl)-(C₁-C₄ alkyl)-(C₃-C₁₀ halocycloalkyl), (C₁-C₄ alkyl)-(C₃-C₁₀ cycloalkyl), S(O₂)-(C₁-C₄ alkyl), C(=O)-N(R_{b})(R_{c}), C(=O)-R₆, -N(R_{b})(R_{c}), (C₁-C₄ alkyl)-N(R_{b})(R_{c}), O-(C₁-C₄ alkyl), (C₁-C₄ alkyl)-O-(C₁-C₄ alkyl), C(=O)O-(C₂-C₁₀ heterocycloalkyl), (C₁-C₄ alkyl)-C(=O)-R₇ or (C₂-C₁₀ heterocycloalkyl)-C(=O)-R₈;
Y is CH, N, O or S {in which R₄ is null when Y is O or S};
R₄ is H, C₁-C₄ alkyl, C₃-C₇ cycloalkyl, C₂-C₁₀ heterocycloalkyl, (C₁-C₄ alkyl)-(C₂-C₁₀ heterocycloalkyl), C₆-C₁₂ aryl, C₂-C₁₀ heteroaryl or C(=O)-R₉,
in which at least one H of C₁-C₄ alkyl, C₂-C₁₀ heterocycloalkyl or (C₁-C₄ alkyl)-(C₂-C₁₀ heterocycloalkyl) can be each independently substituted with X, C₁-C₄ alkyl, C₁-C₄ haloalkyl, O-(C₁-C₄ alkyl), -N(R_{b})(R_{c}), C₃-C₁₀ cycloalkyl, C₂-C₁₀ heterocycloalkyl, (C₂-C₁₀ heterocycloalkyl)-C(=O)**-**R₁₀**,** S(O₂)-(C₁-C₄ alkyl), C(=O)-R₁₁**,** (C₁-C₄ alkyl)-O-(C₁-C₄ alkyl), C₆-C₁₂ aryl, C₂-C₁₀ heteroaryl, (C₁-C₄ alkyl)-(C₂-C₁₀ heteroaryl), (C₂-C₁₀ heterocycloalkyl)-(C₂-C₁₀ heterocycloalkyl) or C(=O)-(C₁-C₄ alkyl)-O-(C₁-C₄ alkyl);
R₅, R₆, R₇, R₈, R₉, R₁₀ and R₁₁ are each independently H, C₁-C₄ alkyl, (C₁-C₄ alkyl)-OH, (C₁-C₄ alkyl)-O-(C₁-C₄ alkyl), C₂-C₁₀ heterocycloalkyl, C₆-C₁₂ aryl, C₂-C₁₀ heteroaryl, O-(C₁-C₄ alkyl), C₃-C₇ cycloalkyl or (C₁-C₄ alkyl)-N(R_{b})(R_{c}),
in which at least one H of C₆-C₁₂ aryl or C₂-C₁₀ heteroaryl can be each independently substituted with C₁-C₄ alkyl, X or C₁-C₄ haloalkyl;
R_{b} and R_{c} are each independently H, C₁-C₄ alkyl, C₆-C₁₂ aryl, C₂-C₁₀ heteroaryl, C₃-C₁₀ cycloalkyl, C₂-C₁₀ heterocycloalkyl, (C₁-C₄ alkyl)NH(C₁-C₄ alkyl), (C₁-C₄ alkyl)N(C₁-C₄ alkyl)₂, (C₁-C₄ alkyl)-O-(C₁-C₄ alkyl), C(=O)-(C₁-C₄ alkyl), C(=O)-(C₂-C₁₀ heteroaryl), C(=O)-(C₂-C₁₀ heterocycloalkyl) or C(=O)-(C₃-C₁₀ cycloalkyl);
X is an halogen atom; and
n is any one integer selected from 0, 1, 2 and 3.

2. The compounds represented by the chemical formula I, stereoisomers thereof or pharmaceutically acceptable salts thereof according to claim 1, wherein, in the above chemical formula I,
Z₁ to Z₄ are each independently N or CRₐ, in which Rₐ is H, X, C₁-C₄ alkyl or O-(C₁-C₄ alkyl) and Rₐ can be different from each other when CRₐ is 2 or more;
K is O or S;
R₁ is CX₃ or CX₂H; 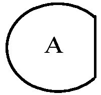
is C₆-C₁₂ arylene or C₂-C₁₀ heteroarylene, in which C₂-C₁₀ heteroarylene can comprise at least one N;
R₂ and R₃ are each independently H, X, C₁-C₄ haloalkyl, C₆-C₁₂ aryl, C₂-C₁₀ heterocycloalkyl, C₂-C₁₀ heterocycloalkenyl, N(R_{b})(R_{c}) or C₂-C₁₀ heteroaryl,
in which at least one H of C₆-C₁₂ aryl, C₂-C₁₀ heteroaryl, C₂-C₁₀ heterocycloalkenyl or C₂-C₁₀ heterocycloalkyl can be each independently substituted with X, C₁-C₄ alkyl, C₁-C₄ haloalkyl, C₃-C₁₀ cycloalkyl, C₂-C₁₀ heterocycloalkyl, (C₁-C₄ alkyl)-(C₂-C₁₀ heteroaryl), (C₁-C₄ alkyl)-(C₂-C₁₀ heterocycloalkyl), (C₂-C₁₀ heterocycloalkyl)-(C₁-C₄ alkyl), (C₂-C₁₀ heterocycloalkyl)-(C₁-C₄ haloalkyl), (C₂-C₁₀ heterocycloalkyl)-(C₃-C₁₀ cycloalkyl), (C₂-C₁₀ heterocycloalkyl)-(C₂-C₁₀ heterocycloalkyl), (C₂-C₁₀ heterocycloalkyl)-(C₁-C₄ alkyl)-(C₃-C₁₀ halocycloalkyl), S(O₂)-(C₁-C₄ alkyl), C(=O)-R₆, O-(C₁-C₄ alkyl) or (C₂-C₁₀ heterocycloalkyl)-C(=O)-R₈;
Y is CH, N, O or S {in which R₄ is null when Y is O or S};
R₄ is C₁-C₄ alkyl, C₂-C₁₀ heterocycloalkyl, C₂-C₁₀ heteroaryl or (C₁-C₄ alkyl)-(C₂-C₁₀ heterocycloalkyl),
in which at least one H of C₁-C₄ alkyl, C₂-C₁₀ heterocycloalkyl or (C₁-C₄ alkyl)-(C₂-C₁₀ heterocycloalkyl) can be each independently substituted with C₁-C₄ alkyl, C₁-C₄ haloalkyl, O-(C₁-C₄ alkyl), -N(R_{b})(R_{c}), C₃-C₁₀ cycloalkyl, C₂-C₁₀ heterocycloalkyl, (C₂-C₁₀ heterocycloalkyl)-C(=O)-R₁₀, S(O₂)-(C₁-C₄ alkyl), C(=O)-R₁₁**,** (C₁-C₄ alkyl)-O-(C₁-C₄ alkyl), C₆-C₁₂ aryl, (C₂-C₁₀ heterocycloalkyl)-(C₂-C₁₀ heterocycloalkyl) or (C₁-C₄ alkyl)-(C₂-C₁₀ heteroaryl);
R₆, R₈, R₁₀ and R₁₁ are each independently C₁-C₄ alkyl, (C₁-C₄ alkyl)-OH, (C₁-C₄ alkyl)-O-(C₁-C₄ alkyl), C₂-C₁₀ heterocycloalkyl, C₆-C₁₂ aryl, C₂-C₁₀ heteroaryl or O-(C₁-C₄ alkyl),
in which at least one H of C₆-C₁₂ aryl or C₂-C₁₀ heteroaryl can be each independently substituted with C₁-C₄ alkyl or C₁-C₄ haloalkyl;
R_{b} and R_{c} are each independently H, C₁-C₄ alkyl or C(=O)-(C₁-C₄ alkyl);
X is an halogen atom; and
n is any one integer selected from 0, 1, 2 and 3.

3. The compounds represented by the chemical formula I, stereoisomers thereof or pharmaceutically acceptable salts thereof according to claim 1, wherein, in the above chemical formula I,
C₂-C₁₀ heterocycloalkyl is 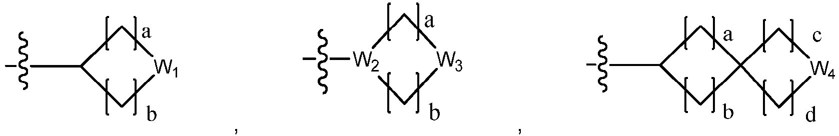 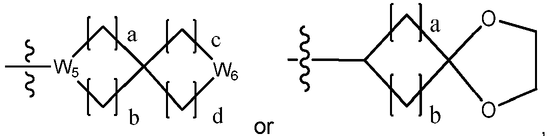
in which W₁ to W₆ are each independently N, NH, O, S or SO₂, and
a to d are each independently an integer of 1, 2 or 3.

4. The compounds represented by the chemical formula I, stereoisomers thereof or pharmaceutically acceptable salts thereof according to claim 1, wherein the compounds represented by the above chemical formula I comprise compounds represented by a following chemical formula II: 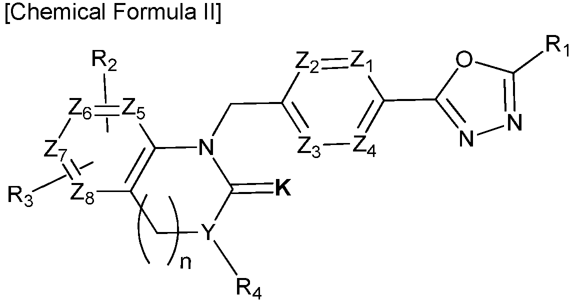 wherein,
Z₁ to Z₄ are each independently N or CRₐ, in which Rₐ is H, X, C₁-C₄ alkyl or O-(C₁-C₄ alkyl) and Rₐ can be different from each other when CRₐ is 2 or more;
Z₅ to Z₈ are each independently CR₂, CR₃, CH or N, in which Z₅ to Z₈ comprise CR₂, CR₃, CH and N, comprise CR₂, CR₃ and two Ns, or comprise CR₂, CR₃ and two CHs;
K is O or S;
R₁ is CX₃ or CX₂H;
R₂ and R₃ are each independently H, X, C₁-C₄ alkyl, C₁-C₄ haloalkyl, C₆-C₁₂ aryl, C₂-C₁₀ heteroaryl, C₃-C₁₀ cycloalkyl, C₂-C₁₀ heterocycloalkyl, C₂-C₁₀ heterocycloalkenyl, N(R_{b})(R_{c}), NH-(C₁-C₄ alkyl)-N(R_{b})(R_{c}), NH-O-(C₁-C₄ alkyl) or NHC(=O)-R₅,
in which at least one H of C₁-C₄ alkyl, C₆-C₁₂ aryl, C₂-C₁₀ heteroaryl, C₂-C₁₀ heterocycloalkenyl or C₂-C₁₀ heterocycloalkyl can be each independently substituted with X, C₁-C₄ alkyl, C₁-C₄ haloalkyl, C₃-C₁₀ cycloalkyl, C₆-C₁₂ aryl, C₂-C₁₀ heteroaryl, C₂-C₁₀ heterocycloalkyl, (C₁-C₄ alkyl)-(C₂-C₁₀ heteroaryl), (C₁-C₄ alkyl)-(C₂-C₁₀ heterocycloalkyl), (C₂-C₁₀ heterocycloalkyl)-(C₁-C₄ alkyl), (C₂-C₁₀ heterocycloalkyl)-(C₁-C₄ haloalkyl), (C₂-C₁₀ heterocycloalkyl)-(C₃-C₁₀ cycloalkyl), (C₂-C₁₀ heterocycloalkyl)-(C₂-C₁₀ heterocycloalkyl), (C₂-C₁₀ heterocycloalkyl)-(C₁-C₄ alkyl)-(C₃-C₁₀ halocycloalkyl), (C₁-C₄ alkyl)-(C₃-C₁₀ cycloalkyl), S(O₂)-(C₁-C₄ alkyl), C(=O)-N(R_{b})(R_{c}), C(=O)-R₆, -N(R_{b})(R_{c}), (C₁-C₄ alkyl)-N(R_{b})(R_{c}), O-(C₁-C₄ alkyl), (C₁-C₄ alkyl)-O-(C₁-C₄ alkyl), C(=O)O-(C₂-C₁₀ heterocycloalkyl), (C₁-C₄ alkyl)-C(=O)-R₇ or (C₂-C₁₀ heterocycloalkyl)-C(=O)-R₈;
Y is CH, N, O or S {in which R₄ is null when Y is O or S};
R₄ is H, C₁-C₄ alkyl, C₃-C₇ cycloalkyl, C₂-C₁₀ heterocycloalkyl, (C₁-C₄ alkyl)-(C₂-C₁₀ heterocycloalkyl), C₆-C₁₂ aryl, C₂-C₁₀ heteroaryl or C(=O)-R₉,
in which at least one H of C₁-C₄ alkyl, C₂-C₁₀ heterocycloalkyl or (C₁-C₄ alkyl)-(C₂-C₁₀ heterocycloalkyl) can be each independently substituted with X, C₁-C₄ alkyl, C₁-C₄ haloalkyl, O-(C₁-C₄ alkyl), -N(R_{b})(R_{c}), C₃-C₁₀ cycloalkyl, C₂-C₁₀ heterocycloalkyl, (C₂-C₁₀ heterocycloalkyl)-C(=O)-R₁₀**,** S(O₂)-(C₁-C₄ alkyl), C(=O)-R₁₁**,** (C₁-C₄ alkyl)-O-(C₁-C₄ alkyl), C₆-C₁₂ aryl, C₂-C₁₀ heteroaryl, (C₁-C₄ alkyl)-(C₂-C₁₀ heteroaryl), (C₂-C₁₀ heterocycloalkyl)-(C₂-C₁₀ heterocycloalkyl) or C(=O)-(C₁-C₄ alkyl)-O-(C₁-C₄ alkyl);
R₅, R₆, R₇, R₈, R₉, R₁₀ and R₁₁ are each independently H, C₁-C₄ alkyl, (C₁-C₄ alkyl)-OH, (C₁-C₄ alkyl)-O-(C₁-C₄ alkyl), C₂-C₁₀ heterocycloalkyl, C₆-C₁₂ aryl, C₂-C₁₀ heteroaryl, O-(C₁-C₄ alkyl), C₃-C₇ cycloalkyl or (C₁-C₄ alkyl)-N(R_{b})(R_{c}),
in which at least one H of C₆-C₁₂ aryl or C₂-C₁₀ heteroaryl can be each independently substituted with C₁-C₄ alkyl, X or C₁-C₄ haloalkyl;
R_{b} and R_{c} are each independently H, C₁-C₄ alkyl, C₆-C₁₂ aryl, C₂-C₁₀ heteroaryl, C₃-C₁₀ cycloalkyl, C₂-C₁₀ heterocycloalkyl, (C₁-C₄ alkyl)NH(C₁-C₄ alkyl), (C₁-C₄ alkyl)N(C₁-C₄ alkyl)₂, (C₁-C₄ alkyl)-O-(C₁-C₄ alkyl), C(=O)-(C₁-C₄ alkyl), C(=O)-(C₂-C₁₀ heteroaryl), C(=O)-(C₂-C₁₀ heterocycloalkyl) or C(=O)-(C₃-C₁₀ cycloalkyl);
X is an halogen atom; and
n is any one integer selected from 0, 1, 2 and 3.

5. The compounds represented by the chemical formula I, stereoisomers thereof or pharmaceutically acceptable salts thereof according to claim 4, wherein,
Z₁ to Z₄ are each independently N or CRₐ, in which Rₐ is H, X, C₁-C₄ alkyl or O-(C₁-C₄ alkyl) and Rₐ can be different from each other when CRₐ is 2 or more;
Z₅ to Z₈ are each independently CR₂, CR₃, CH or N, in which Z₅ to Z₈ comprise CR₂, CR₃, CH and N or comprise CR₂, CR₃ and two CHs (however, Z₆ is N when Z₅ to Z₈ comprise CR₂, CR₃, CH and N);
K is O or S;
R₁ is CX₃ or CX₂H;
R₂ and R₃ are each independently H, X, C₁-C₄ haloalkyl, C₆-C₁₂ aryl, C₂-C₁₀ heterocycloalkyl, C₂-C₁₀ heterocycloalkenyl, N(R_{b})(R_{c}) or C₂-C₁₀ heteroaryl,
in which at least one H of C₆-C₁₂ aryl, C₂-C₁₀ heteroaryl, C₂-C₁₀ heterocycloalkenyl or C₂-C₁₀ heterocycloalkyl can be each independently substituted with X, C₁-C₄ alkyl, C₁-C₄ haloalkyl, C₃-C₁₀ cycloalkyl, C₂-C₁₀ heterocycloalkyl, (C₁-C₄ alkyl)-(C₂-C₁₀ heteroaryl), (C₁-C₄ alkyl)-(C₂-C₁₀ heterocycloalkyl), (C₂-C₁₀ heterocycloalkyl)-(C₁-C₄ alkyl), (C₂-C₁₀ heterocycloalkyl)-(C₁-C₄ haloalkyl), (C₂-C₁₀ heterocycloalkyl)-(C₃-C₁₀ cycloalkyl), (C₂-C₁₀ heterocycloalkyl)-(C₂-C₁₀ heterocycloalkyl), (C₂-C₁₀ heterocycloalkyl)-(C₁-C₄ alkyl)-(C₃-C₁₀ halocycloalkyl), S(O₂)-(C₁-C₄ alkyl), C(=O)-R₆, O-(C₁-C₄ alkyl) or (C₂-C₁₀ heterocycloalkyl)-C(=O)-R₈;
Y is CH, N, O or S {in which R₄ is null when Y is O or S};
R₄ is C₁-C₄ alkyl, C₂-C₁₀ heterocycloalkyl, C₂-C₁₀ heteroaryl or (C₁-C₄ alkyl)-(C₂-C₁₀ heterocycloalkyl),
in which at least one H of C₁-C₄ alkyl, C₂-C₁₀ heterocycloalkyl or (C₁-C₄ alkyl)-(C₂-C₁₀ heterocycloalkyl) can be each independently substituted with C₁-C₄ alkyl, C₁-C₄ haloalkyl, O-(C₁-C₄ alkyl), -N(R_{b})(R_{c}), C₃-C₁₀ cycloalkyl, C₂-C₁₀ heterocycloalkyl, (C₂-C₁₀ heterocycloalkyl)-C(=O)-R₁₀, S(O₂)-(C₁-C₄ alkyl), C(=O)-R₁₁**,** (C₁-C₄ alkyl)-O-(C₁-C₄ alkyl), C₆-C₁₂ aryl, (C₂-C₁₀ heterocycloalkyl)-(C₂-C₁₀ heterocycloalkyl) or (C₁-C₄ alkyl)-(C₂-C₁₀ heteroaryl);
R₆, R₈, R₁₀ and R₁₁ are each independently C₁-C₄ alkyl, (C₁-C₄ alkyl)-OH, (C₁-C₄ alkyl)-O-(C₁-C₄ alkyl), C₂-C₁₀ heterocycloalkyl, C₆-C₁₂ aryl, C₂-C₁₀ heteroaryl or O-(C₁-C₄ alkyl),
in which at least one H of C₆-C₁₂ aryl or C₂-C₁₀ heteroaryl can be each independently substituted with C₁-C₄ alkyl or C₁-C₄ haloalkyl;
R_{b} and R_{c} are each independently H, C₁-C₄ alkyl or C(=O)-(C₁-C₄ alkyl);
X is an halogen atom; and
n is any one integer selected from 0, 1, 2 and 3.

6. The compounds represented by the chemical formula I, stereoisomers thereof or pharmaceutically acceptable salts thereof according to claim 4, wherein, in the above chemical formula II,
Z₁ to Z₄ are each independently N or CRₐ, in which Rₐ is H, X, C₁-C₄ alkyl or O-(C₁-C₄ alkyl) and Rₐ can be different from each other when CRₐ is 2 or more;
Z₅ to Z₈ are each independently CR₂, CR₃, CH or N, in which Z₅ to Z₈ comprise CR₂, CR₃, CH and N or comprise CR₂, CR₃ and two CHs (however, Z₆ is N when Z₅ to Z₈ comprise CR₂, CR₃, CH and N);
K is O or S;
R₁ is CX₃ or CX₂H;
R₂ and R₃ are each independently H, X, C₁-C₄ haloalkyl, C₆-C₁₂ aryl, C₂-C₁₀ heterocycloalkyl, C₂-C₁₀ heterocycloalkenyl, N(R_{b})(R_{c}) or C₂-C₁₀ heteroaryl,
in which at least one H of C₆-C₁₂ aryl, C₂-C₁₀ heteroaryl, C₂-C₁₀ heterocycloalkenyl or C₂-C₁₀ heterocycloalkyl can be each independently substituted with X, C₁-C₄ alkyl, C₁-C₄ haloalkyl, C₃-C₁₀ cycloalkyl, C₂-C₁₀ heterocycloalkyl, (C₁-C₄ alkyl)-(C₂-C₁₀ heteroaryl), (C₁-C₄ alkyl)-(C₂-C₁₀ heterocycloalkyl), (C₂-C₁₀ heterocycloalkyl)-(C₁-C₄ alkyl), (C₂-C₁₀ heterocycloalkyl)-(C₁-C₄ haloalkyl), (C₂-C₁₀ heterocycloalkyl)-(C₃-C₁₀ cycloalkyl), (C₂-C₁₀ heterocycloalkyl)-(C₂-C₁₀ heterocycloalkyl), (C₂-C₁₀ heterocycloalkyl)-(C₁-C₄ alkyl)-(C₃-C₁₀ halocycloalkyl), S(O₂)-(C₁-C₄ alkyl), C(=O)-R₆, O-(C₁-C₄ alkyl) or (C₂-C₁₀ heterocycloalkyl)-C(=O)-R₈;
Y is N, O or S {in which R₄ is null when Y is O or S};
R₄ is C₁-C₄ alkyl, C₂-C₁₀ heterocycloalkyl, C₂-C₁₀ heteroaryl or (C₁-C₄ alkyl)-(C₂-C₁₀ heterocycloalkyl),
in which at least one H of C₁-C₄ alkyl, C₂-C₁₀ heterocycloalkyl or (C₁-C₄ alkyl)-(C₂-C₁₀ heterocycloalkyl) can be each independently substituted with C₁-C₄ alkyl, C₁-C₄ haloalkyl, O-(C₁-C₄ alkyl), -N(R_{b})(R_{c}), C₃-C₁₀ cycloalkyl, C₂-C₁₀ heterocycloalkyl, (C₂-C₁₀ heterocycloalkyl)-C(=O)-R₁₀, S(O₂)-(C₁-C₄ alkyl), C(=O)-R₁₁**,** (C₁-C₄ alkyl)-O-(C₁-C₄ alkyl), C₆-C₁₂ aryl, (C₂-C₁₀ heterocycloalkyl)-(C₂-C₁₀ heterocycloalkyl) or (C₁-C₄ alkyl)-(C₂-C₁₀ heteroaryl);
R₆, R₈, R₁₀ and R₁₁ are each independently C₁-C₄ alkyl, (C₁-C₄ alkyl)-OH, (C₁-C₄ alkyl)-O-(C₁-C₄ alkyl), C₂-C₁₀ heterocycloalkyl, C₆-C₁₂ aryl, C₂-C₁₀ heteroaryl or O-(C₁-C₄ alkyl),
in which at least one H of C₆-C₁₂ aryl or C₂-C₁₀ heteroaryl can be each independently substituted with C₁-C₄ alkyl or C₁-C₄ haloalkyl;
R_{b} and R_{c} are each independently H, C₁-C₄ alkyl or C(=O)-(C₁-C₄ alkyl);
X is an halogen atom; and
n is any one integer selected from 0, 1, 2 and 3.

7. The compounds represented by the chemical formula I, stereoisomers thereof or pharmaceutically acceptable salts thereof according to claim 1, wherein, in the above chemical formula I,
Z₁ to Z₄ are each independently N or CRₐ, in which Rₐ is H or X, and Rₐ can be different from each other when CRₐ is 2 or more;
K is O;
R₁ is CF₃ or CF₂H; 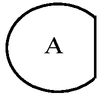 is phenylene or pyridinylene,
R₂ and R₃ are each independently H, X, C₁-C₄ haloalkyl, C₆-C₁₂ aryl, C₂-C₁₀ heterocycloalkyl, C₂-C₁₀ heterocycloalkenyl, N(R_{b})(R_{c}) or C₂-C₁₀ heteroaryl,
in which at least one H of C₆-C₁₂ aryl, C₂-C₁₀ heteroaryl, C₂-C₁₀ heterocycloalkenyl or C₂-C₁₀ heterocycloalkyl can be each independently substituted with X, C₁-C₄ alkyl, C₁-C₄ haloalkyl, C₃-C₁₀ cycloalkyl, C₂-C₁₀ heterocycloalkyl, (C₁-C₄ alkyl)-(C₂-C₁₀ heteroaryl), (C₁-C₄ alkyl)-(C₂-C₁₀ heterocycloalkyl), (C₂-C₁₀ heterocycloalkyl)-(C₁-C₄ alkyl), (C₂-C₁₀ heterocycloalkyl)-(C₁-C₄ haloalkyl), (C₂-C₁₀ heterocycloalkyl)-(C₃-C₁₀ cycloalkyl), (C₂-C₁₀ heterocycloalkyl)-(C₂-C₁₀ heterocycloalkyl), (C₂-C₁₀ heterocycloalkyl)-(C₁-C₄ alkyl)-(C₃-C₁₀ halocycloalkyl), S(O₂)-(C₁-C₄ alkyl), C(=O)-R₆, O-(C₁-C₄ alkyl) or (C₂-C₁₀ heterocycloalkyl)-C(=O)-R₈;
Y is N, O or S {in which R₄ is null when Y is O or S};
R₄ is C₁-C₄ alkyl, C₂-C₁₀ heterocycloalkyl, C₂-C₁₀ heteroaryl or (C₁-C₄ alkyl)-(C₂-C₁₀ heterocycloalkyl),
in which at least one H of C₁-C₄ alkyl, C₂-C₁₀ heterocycloalkyl or (C₁-C₄ alkyl)-(C₂-C₁₀ heterocycloalkyl) can be each independently substituted with C₁-C₄ alkyl, C₁-C₄ haloalkyl, O-(C₁-C₄ alkyl), -N(R_{b})(R_{c}), C₃-C₁₀ cycloalkyl, C₂-C₁₀ heterocycloalkyl, (C₂-C₁₀ heterocycloalkyl)-C(=O)-R₁₀, S(O₂)-(C₁-C₄ alkyl), C(=O)-R₁₁**,** (C₁-C₄ alkyl)-O-(C₁-C₄ alkyl), C₆-C₁₂ aryl, (C₂-C₁₀ heterocycloalkyl)-(C₂-C₁₀ heterocycloalkyl) or (C₁-C₄ alkyl)-(C₂-C₁₀ heteroaryl);
R₆ is C₁-C₄ alkyl, O-(C₁-C₄ alkyl) or (C₁-C₄ alkyl)-OH;
R₈ is O-(C₁-C₄ alkyl);
R₁₀ is C₁-C₄ alkyl;
R₁₁ is C₁-C₄ alkyl, (C₁-C₄ alkyl)-OH, (C₁-C₄ alkyl)-O-(C₁-C₄ alkyl), C₂-C₁₀ heterocycloalkyl, C₆-C₁₂ aryl, C₂-C₁₀ heteroaryl or O-(C₁-C₄ alkyl), in which at least one H of C₆-C₁₂ aryl or C₂-C₁₀ heteroaryl can be each independently substituted with C₁-C₄ alkyl or C₁-C₄ haloalkyl;
R_{b} and R_{c} are each independently H, C₁-C₄ alkyl or C(=O)-(C₁-C₄ alkyl);
X is F, Cl or Br; and
n is 0 or 1.

8. The compounds represented by the chemical formula I, stereoisomers thereof or pharmaceutically acceptable salts thereof according to claim 1, wherein the compounds are selected from the group consisting of compounds represented by following compounds 1 to 368:
| Compo und | Structure | Compound | Structure |
|---|---|---|---|
| 1 | 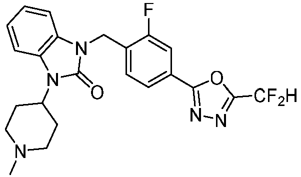 | 2 | 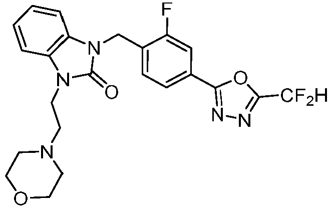 |
| 3 | 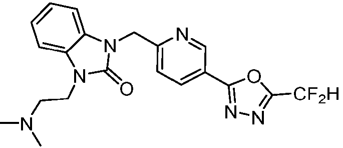 | 4 | 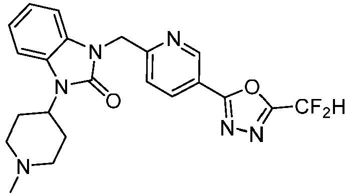 |
| 5 | 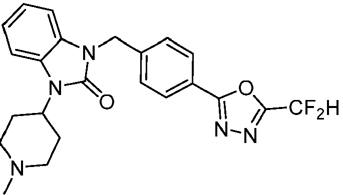 | 6 | 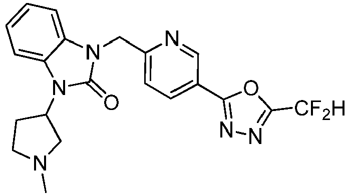 |
| 7 | 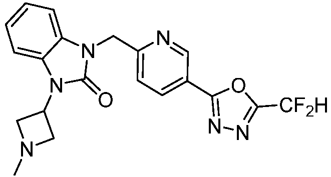 | 8 | 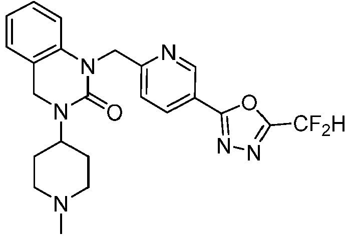 |
| 9 | 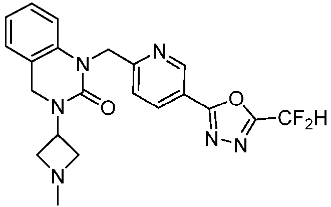 | 10 | 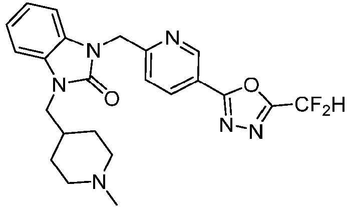 |
| 11 | 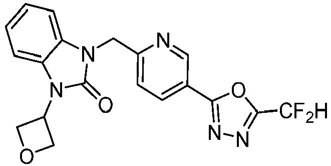 | 12 | 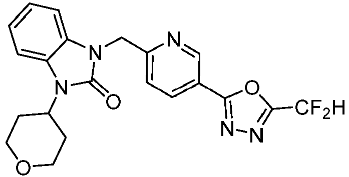 |
| 13 | 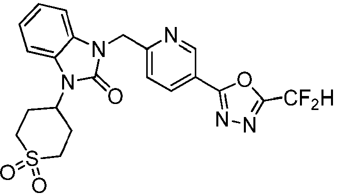 | 14 | 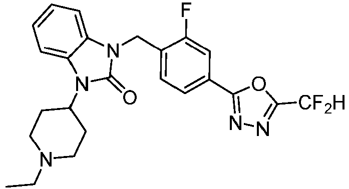 |
| 15 | 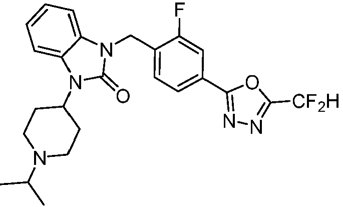 | 16 | 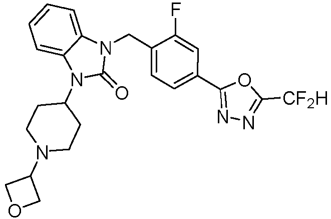 |
| 17 | 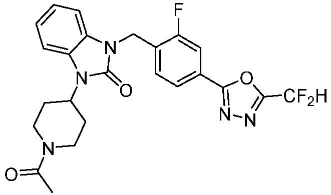 | 18 | 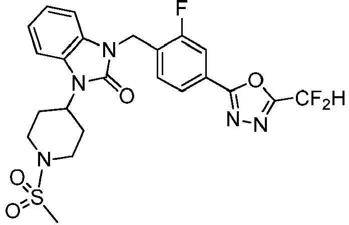 |
| 19 | 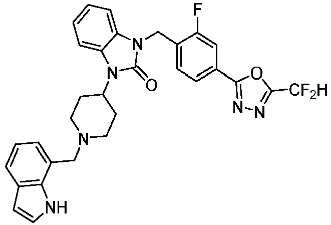 | 20 | 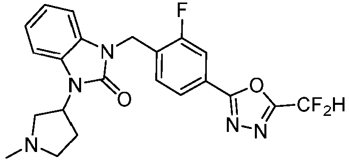 |
| 21 | 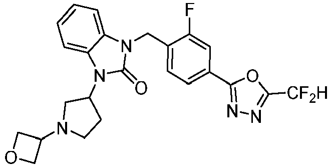 | 22 | 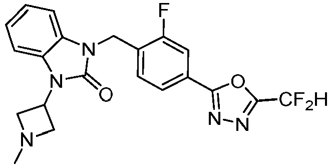 |
| 23 | 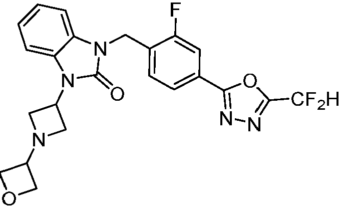 | 24 | 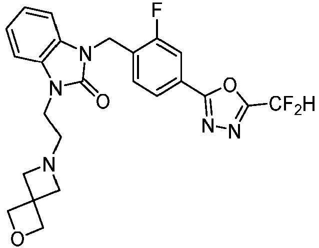 |
| 25 | 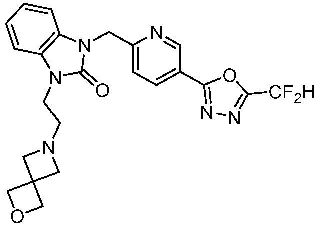 | 26 | 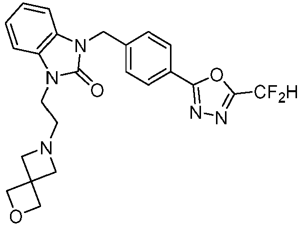 |
| 27 | 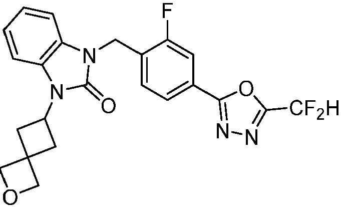 | 28 | 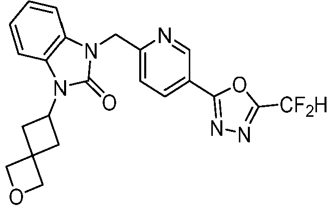 |
| 29 | 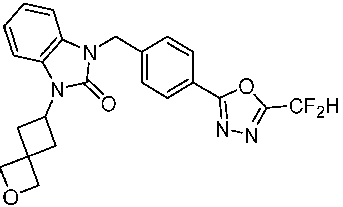 | 30 | 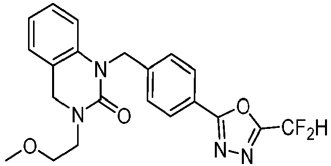 |
| 31 | 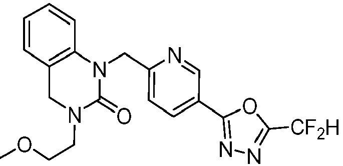 | 32 | 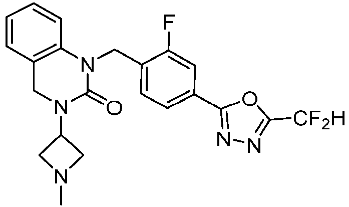 |
| 33 | 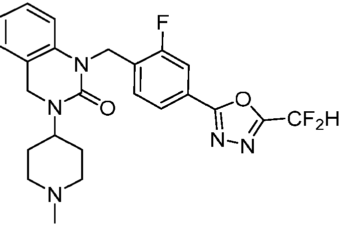 | 34 | 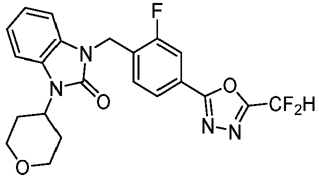 |
| 35 | 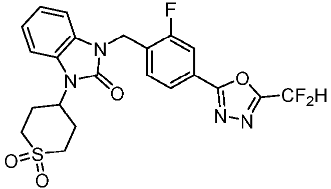 | 36 | 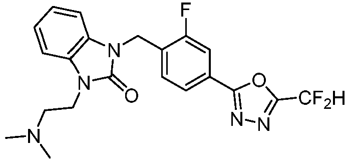 |
| 37 | 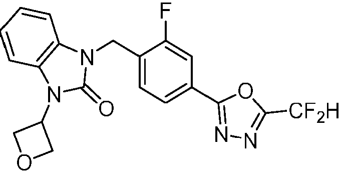 | 38 | 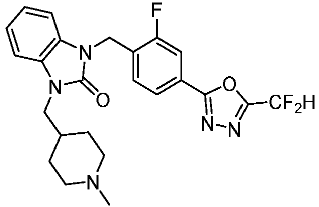 |
| 39 | 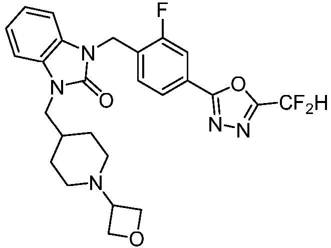 | 40 | 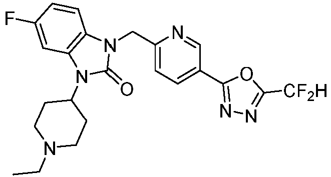 |
| 41 | 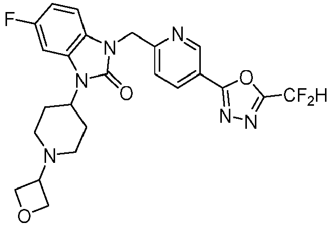 | 42 | 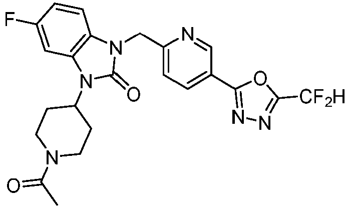 |
| 43 | 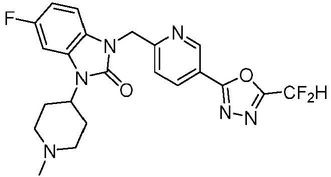 | 44 | 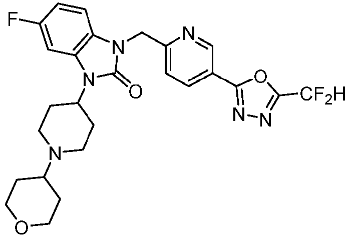 |
| 45 | 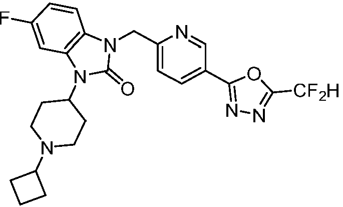 | 46 | 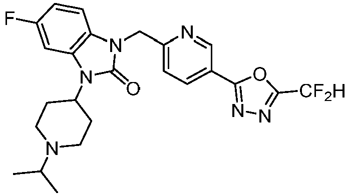 |
| 47 | 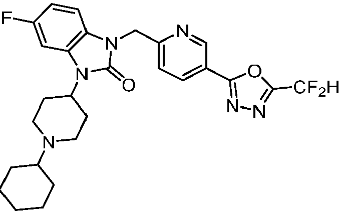 | 48 | 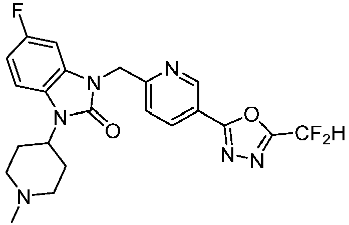 |
| 49 | 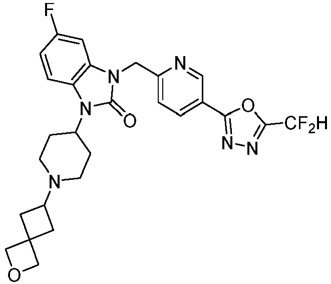 | 50 | 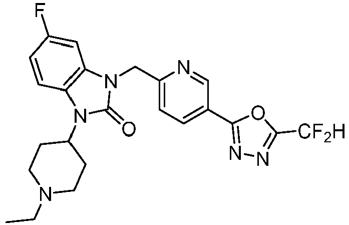 |
| 51 | 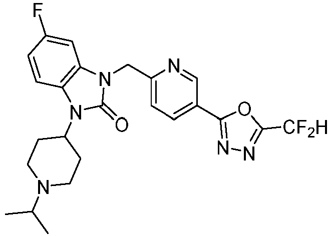 | 52 | 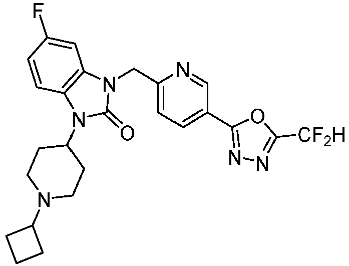 |
| 53 | 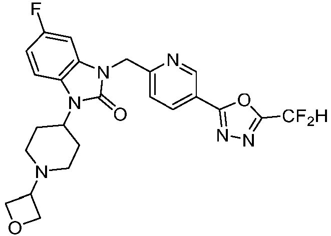 | 54 | 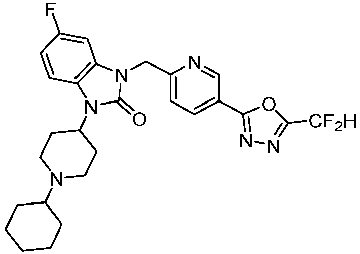 |
| 55 | 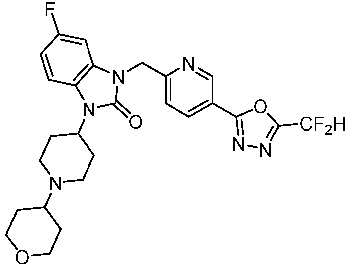 | 56 | 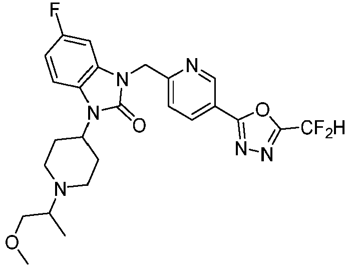 |
| 57 | 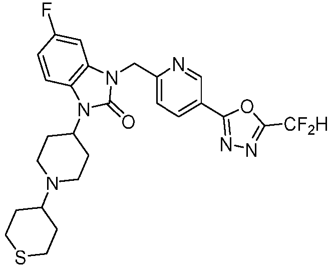 | 58 | 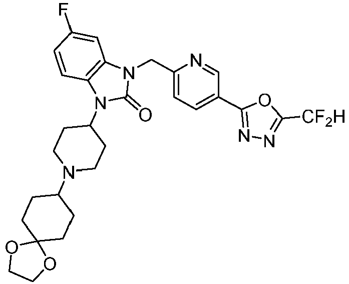 |
| 59 | 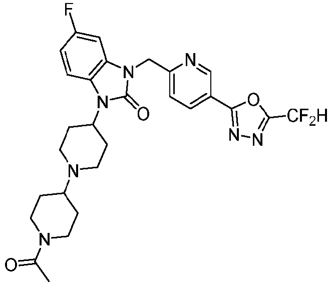 | 60 | 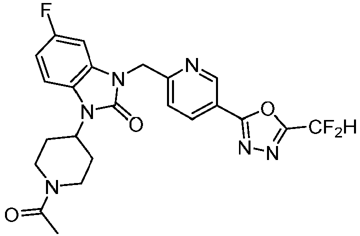 |
| 61 | 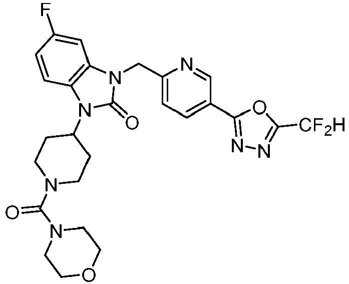 | 62 | 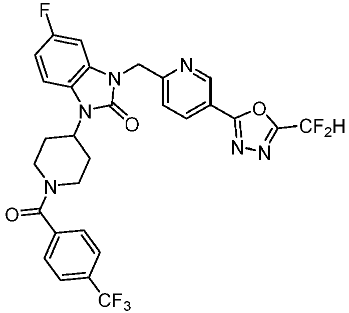 |
| 63 | 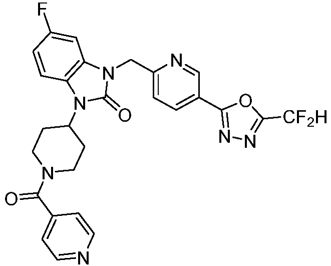 | 64 | 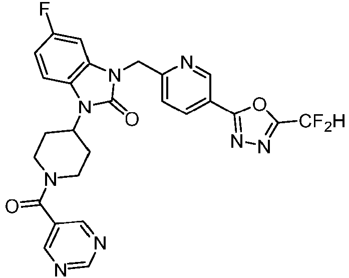 |
| 65 | 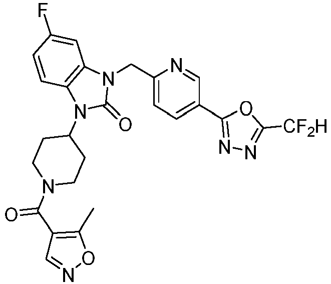 | 66 | 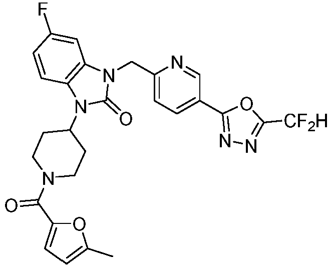 |
| 67 | 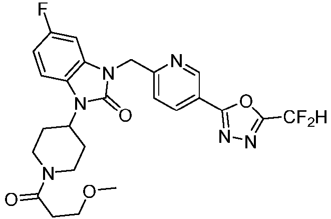 | 68 | 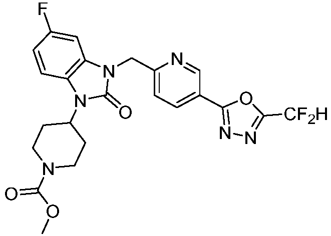 |
| 69 | 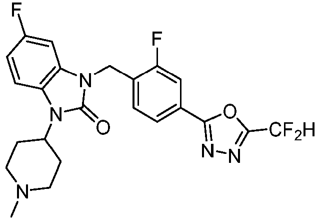 | 70 | 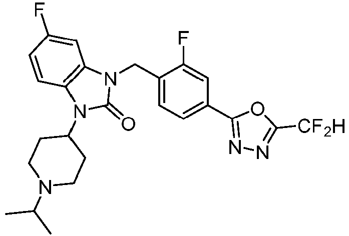 |
| 71 | 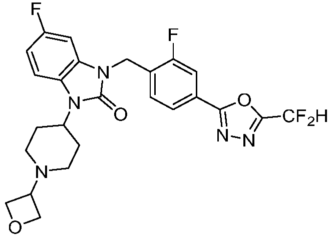 | 72 | 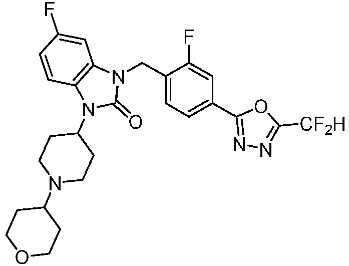 |
| 73 | 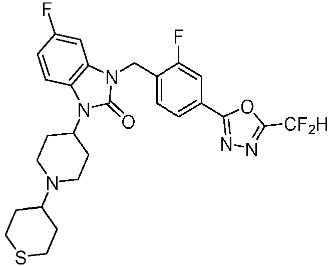 | 74 | 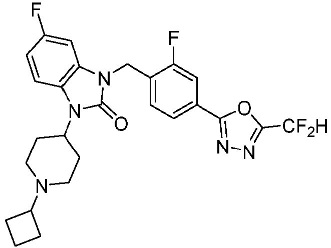 |
| 75 | 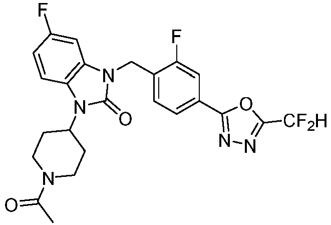 | 76 | 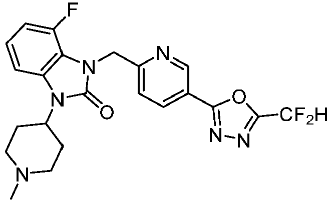 |
| 77 | 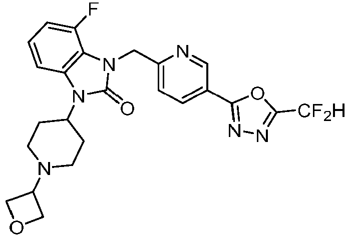 | 78 | 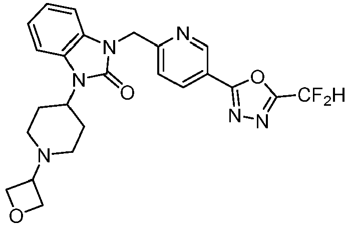 |
| 79 | 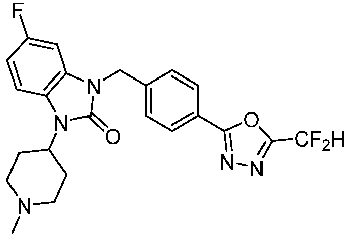 | 80 | 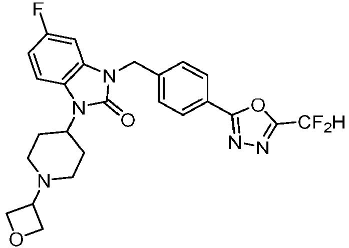 |
| 81 | 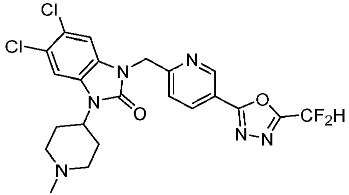 | 82 | 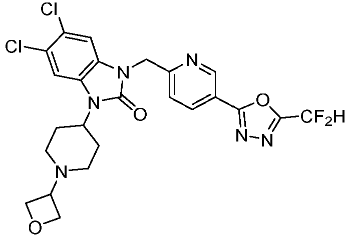 |
| 83 | 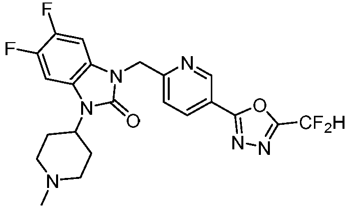 | 84 | 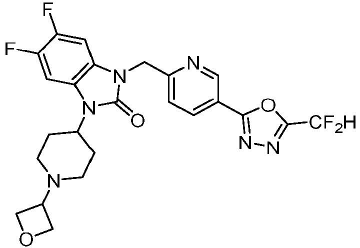 |
| 85 | 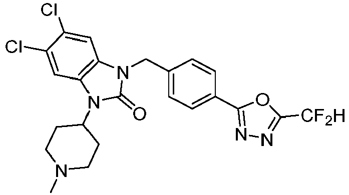 | 86 | 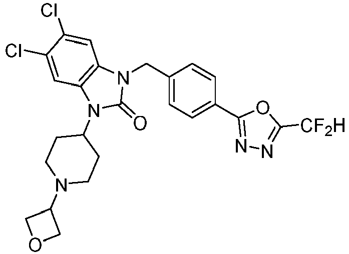 |
| 87 | 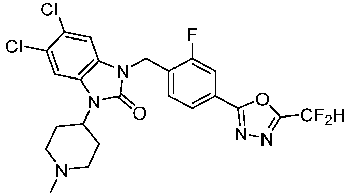 | 88 | 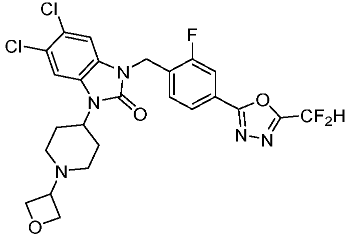 |
| 89 | 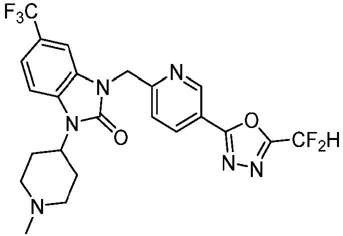 | 90 | 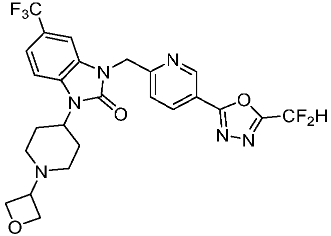 |
| 91 | 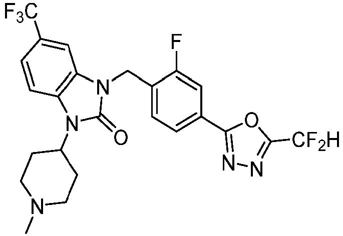 | 92 | 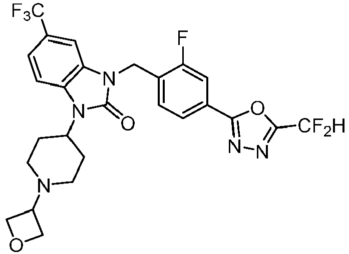 |
| 93 | 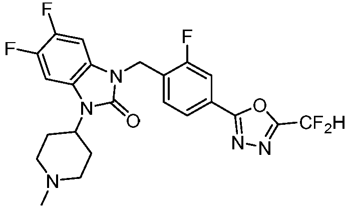 | 94 | 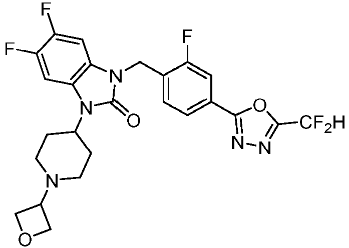 |
| 95 | 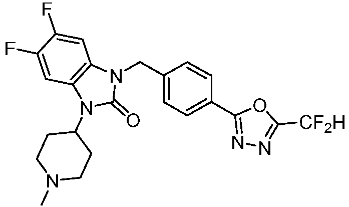 | 96 | 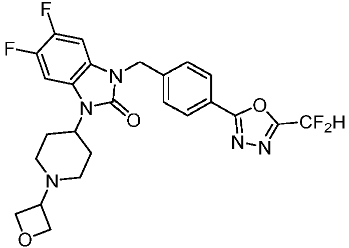 |
| 97 | 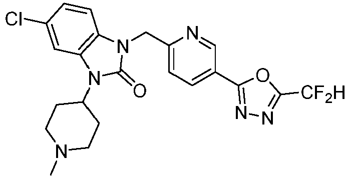 | 98 | 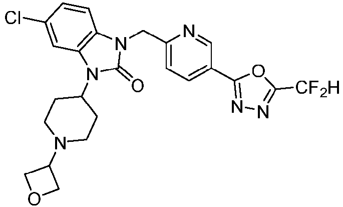 |
| 99 | 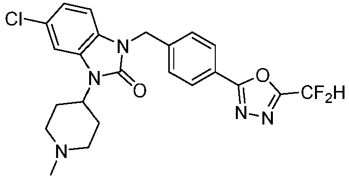 | 100 | 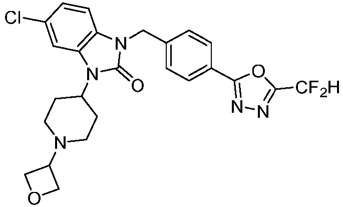 |
| 101 | 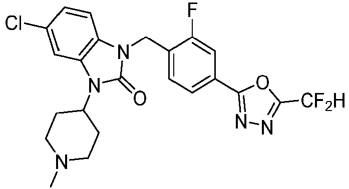 | 102 | 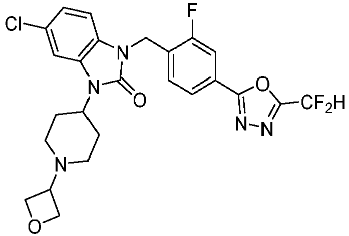 |
| 103 | 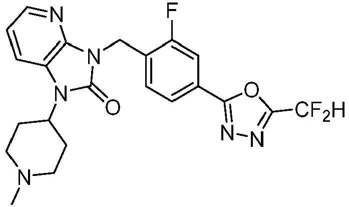 | 104 | 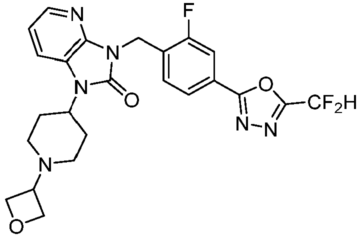 |
| 105 | 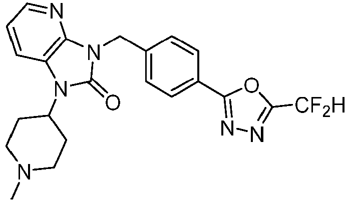 | 106 | 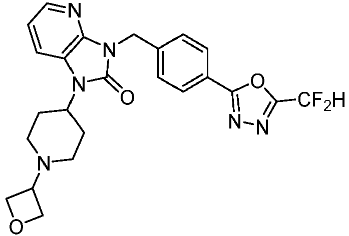 |
| 107 | 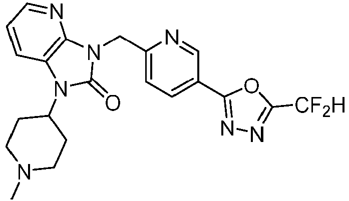 | 108 | 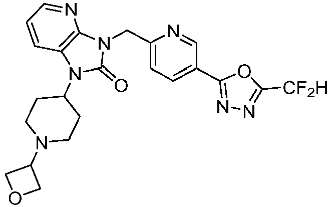 |
| 109 | 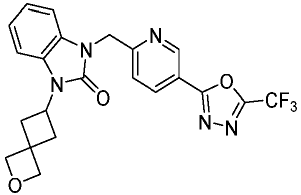 | 110 | 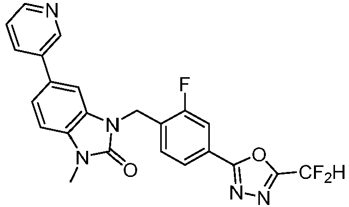 |
| 111 | 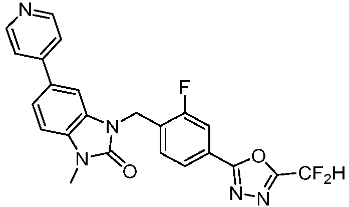 | 112 | 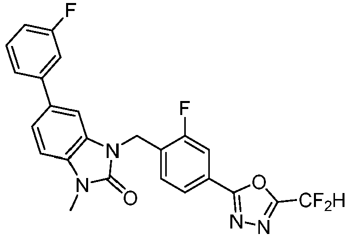 |
| 113 | 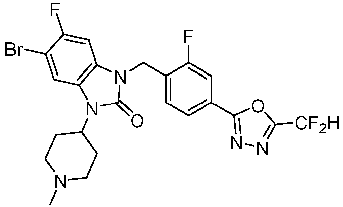 | 114 | 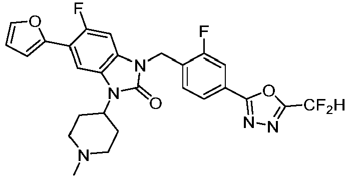 |
| 115 | 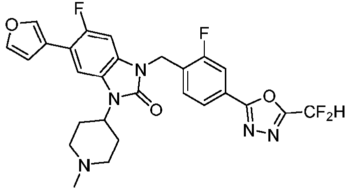 | 116 | 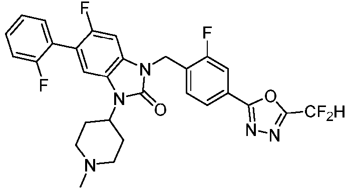 |
| 117 | 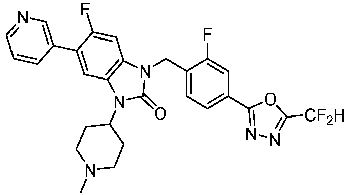 | 118 | 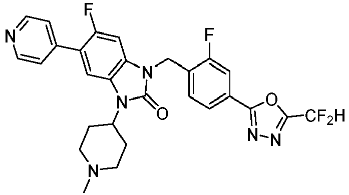 |
| 119 | 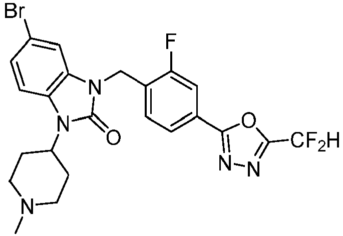 | 120 | 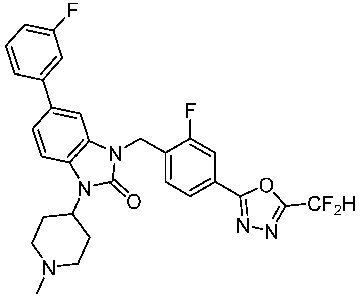 |
| 121 | 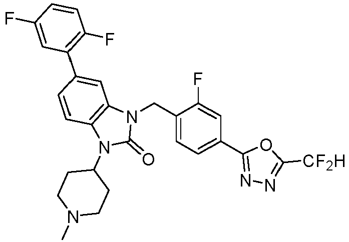 | 122 | 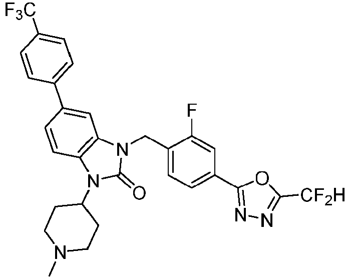 |
| 123 | 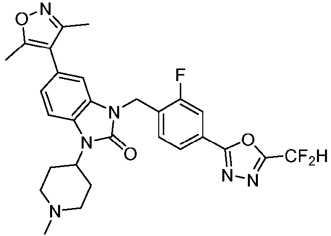 | 124 | 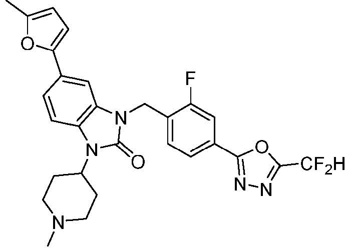 |
| 125 | 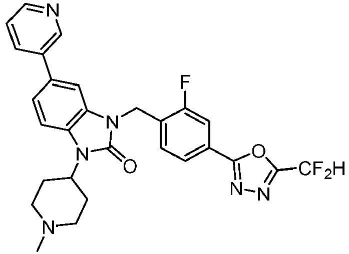 | 126 | 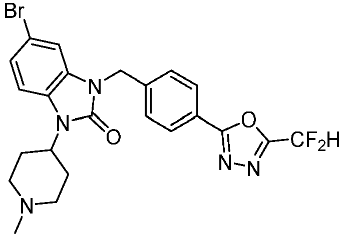 |
| 127 | 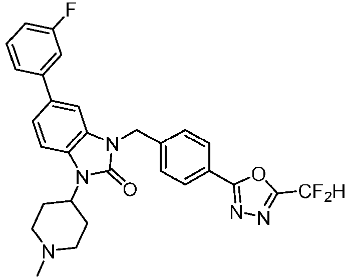 | 128 | 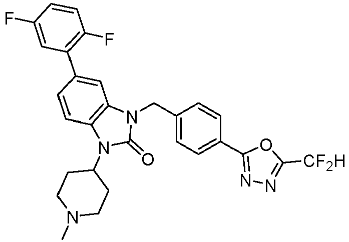 |
| 129 | 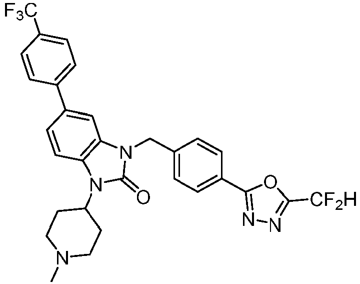 | 130 | 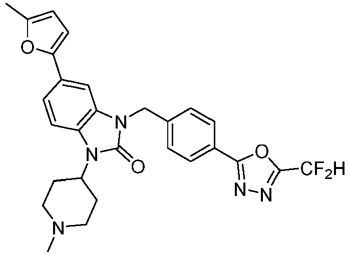 |
| 131 | 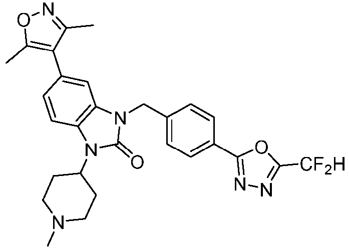 | 132 | 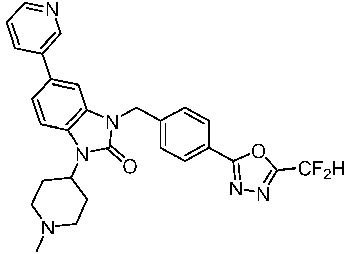 |
| 133 | 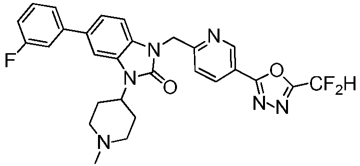 | 134 | 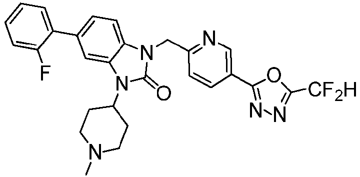 |
| 135 | 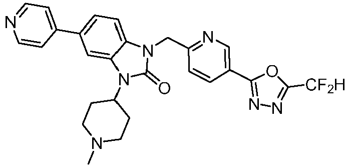 | 136 | 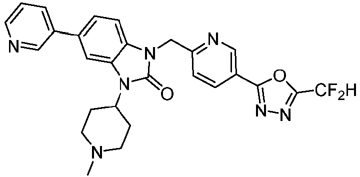 |
| 137 | 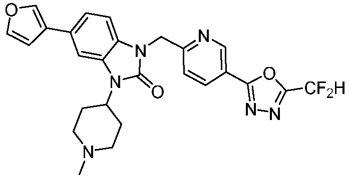 | 138 | 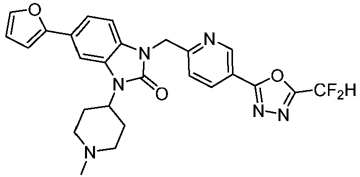 |
| 139 | 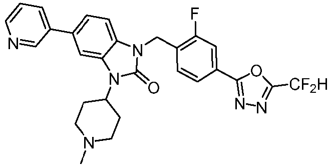 | 140 | 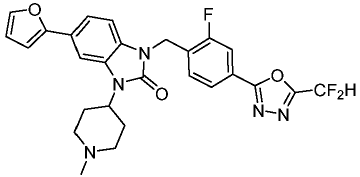 |
| 141 | 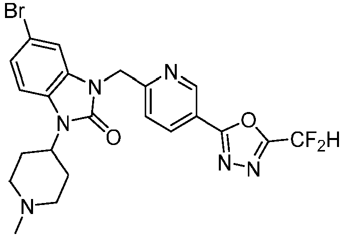 | 142 | 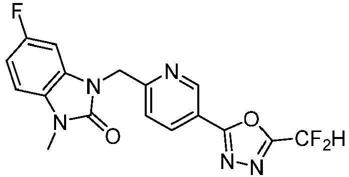 |
| 143 | 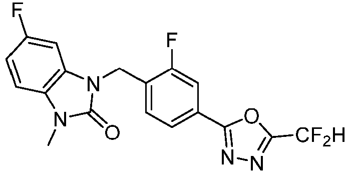 | 144 | 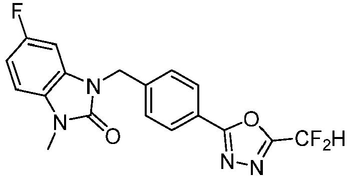 |
| 145 | 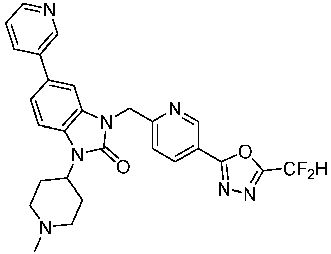 | 146 | 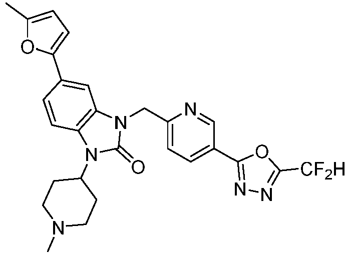 |
| 147 | 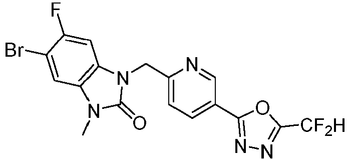 | 148 | 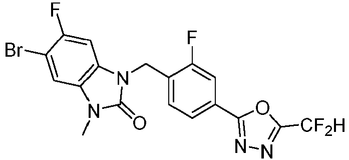 |
| 149 | 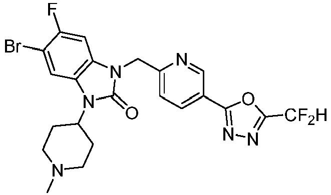 | 150 | 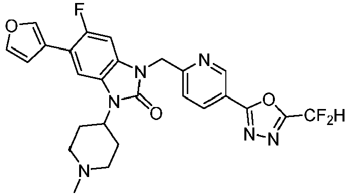 |
| 151 | 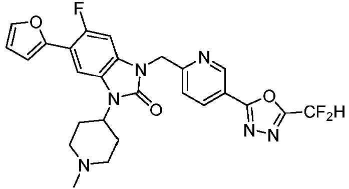 | 152 | 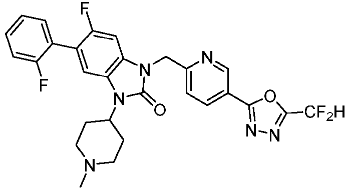 |
| 153 | 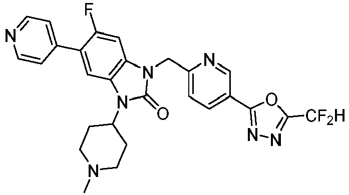 | 154 | 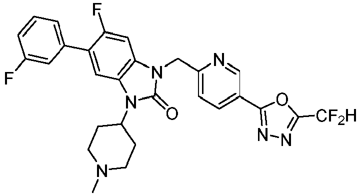 |
| 155 | 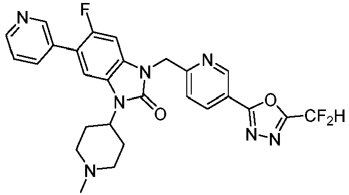 | 156 | 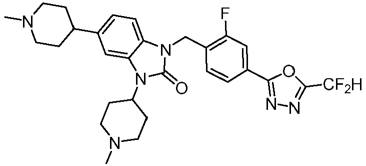 |
| 157 | 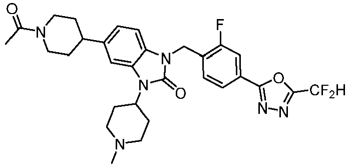 | 158 | 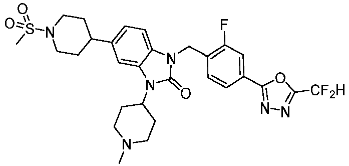 |
| 159 | 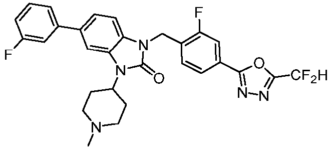 | 160 | 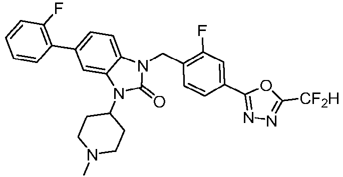 |
| 161 | 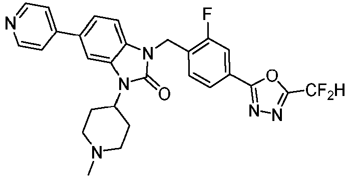 | 162 | 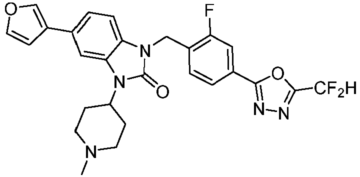 |
| 163 | 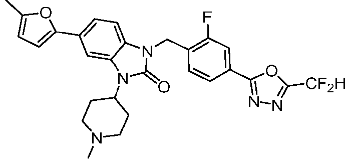 | 164 | 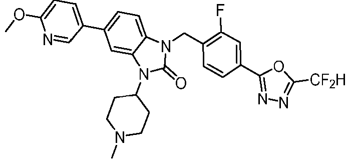 |
| 165 | 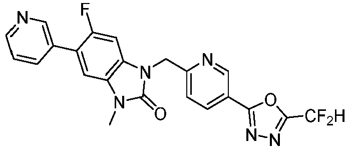 | 166 | 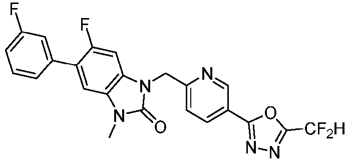 |
| 167 | 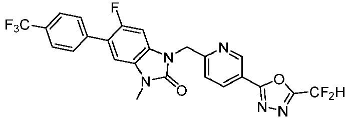 | 168 | 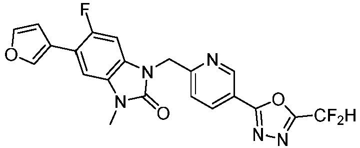 |
| 169 | 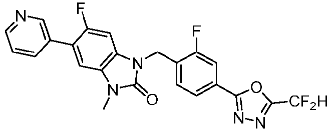 | 170 | 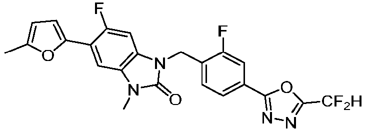 |
| 171 | 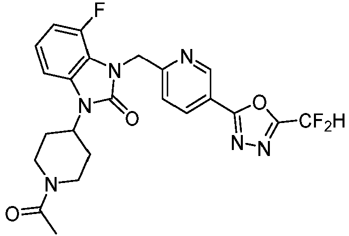 | 172 | 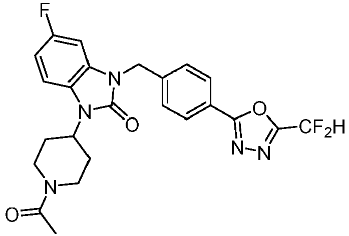 |
| 173 | 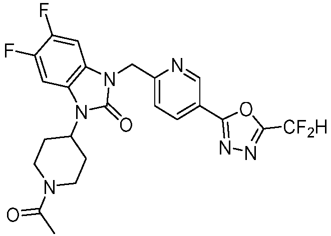 | 174 | 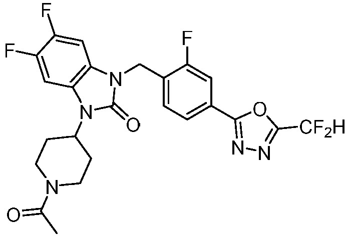 |
| 175 | 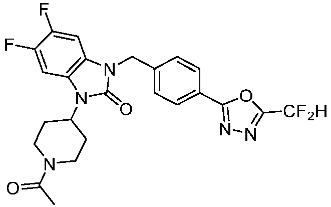 | 176 | 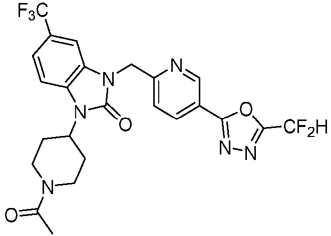 |
| 177 | 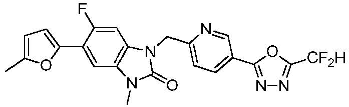 | 178 | 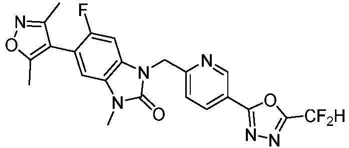 |
| 179 | 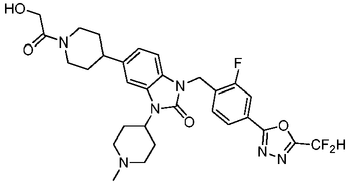 | 180 | 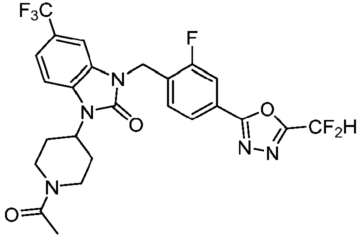 |
| 181 | 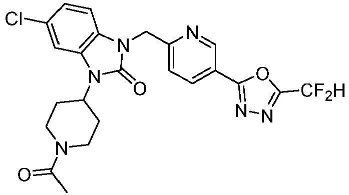 | 182 | 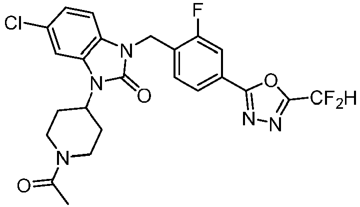 |
| 183 | 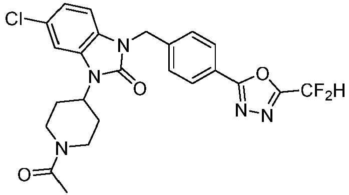 | 184 | 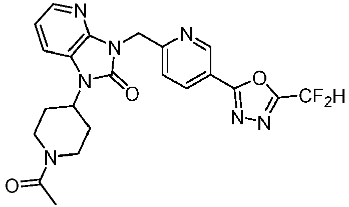 |
| 185 | 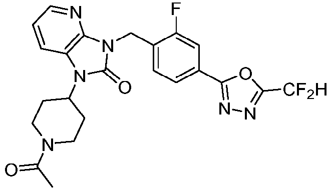 | 186 | 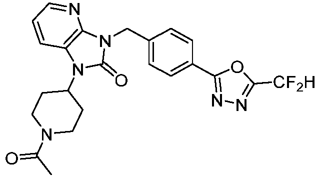 |
| 187 | 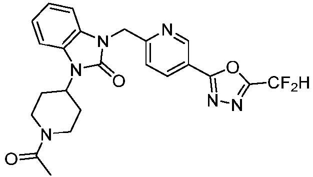 | 188 | 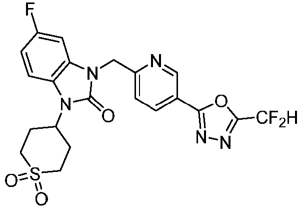 |
| 189 | 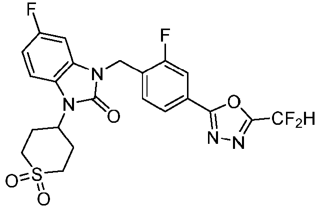 | 190 | 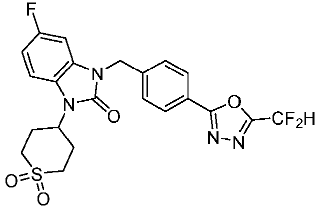 |
| 191 | 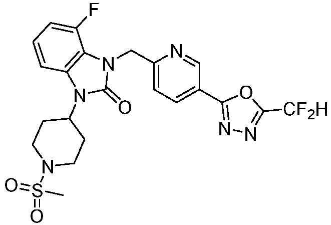 | 192 | 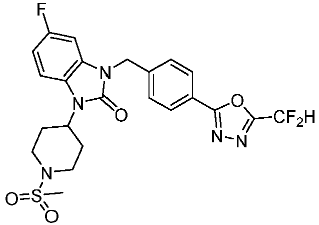 |
| 193 | 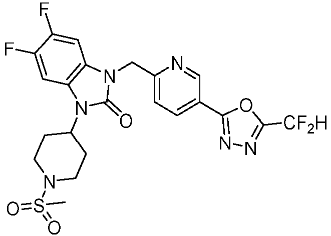 | 194 | 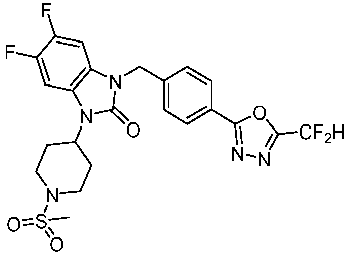 |
| 195 | 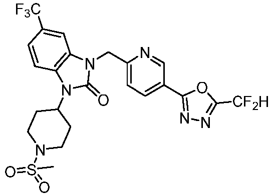 | 196 | 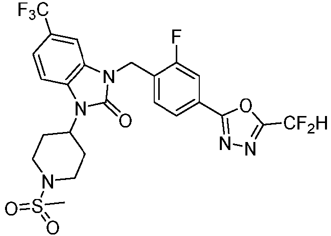 |
| 197 | 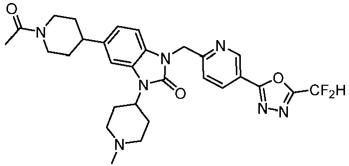 | 198 | 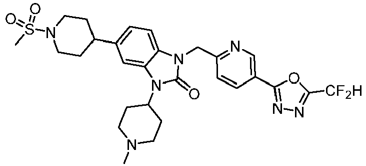 |
| 199 | 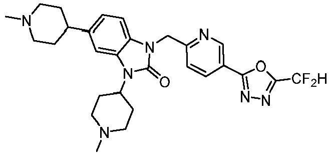 | 200 | 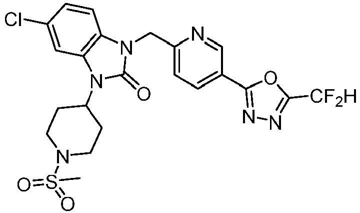 |
| 201 | 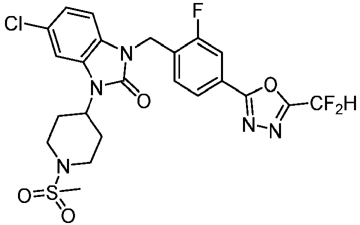 | 202 | 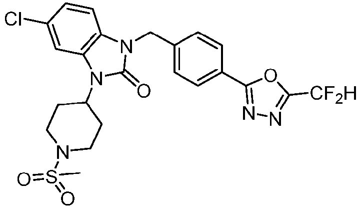 |
| 203 | 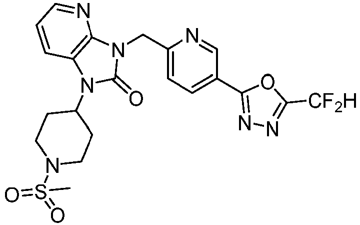 | 204 | 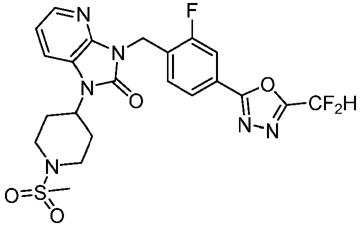 |
| 205 | 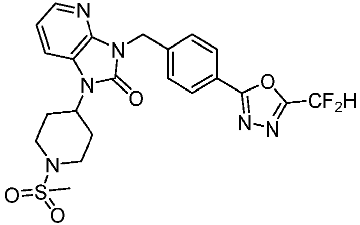 | 206 | 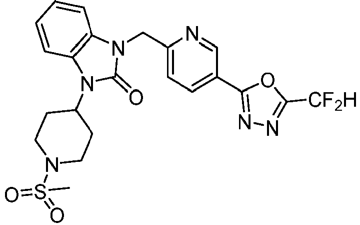 |
| 207 | 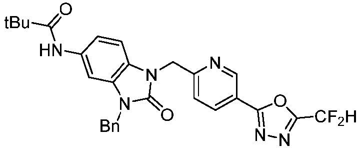 | 208 | 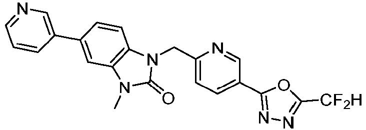 |
| 209 | 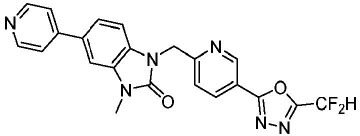 | 210 | 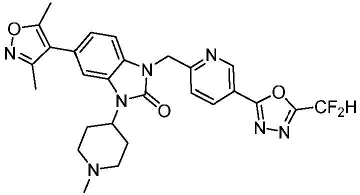 |
| 211 | 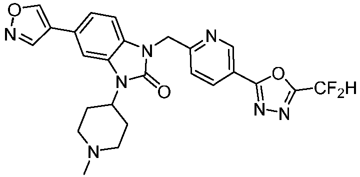 | 212 | 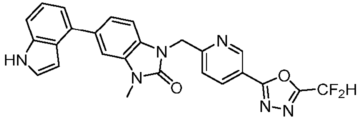 |
| 213 | 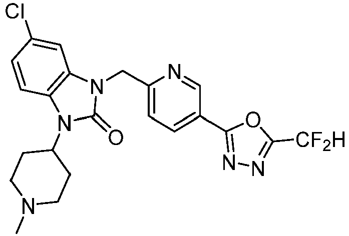 | 214 | 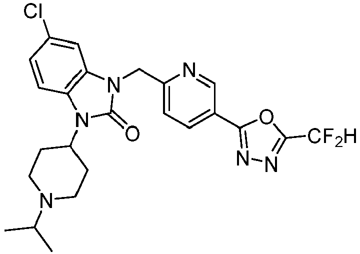 |
| 215 | 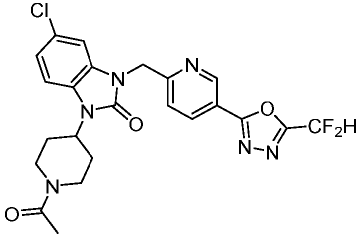 | 216 | 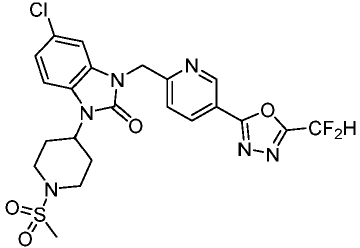 |
| 217 | 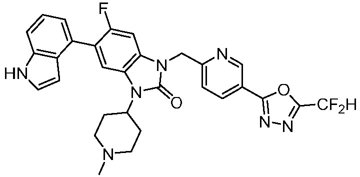 | 218 | 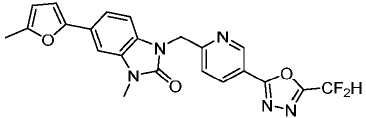 |
| 219 | 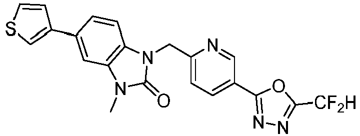 | 220 | 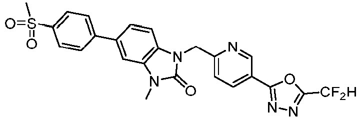 |
| 221 | 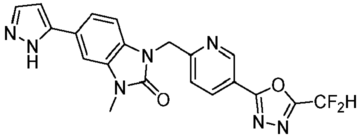 | 222 | 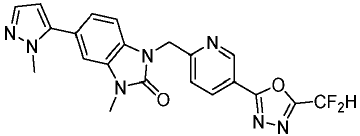 |
| 223 | 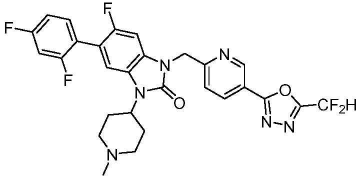 | 224 | 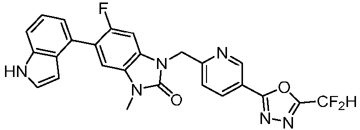 |
| 225 | 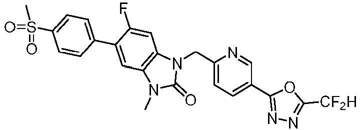 | 226 | 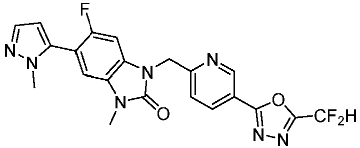 |
| 227 | 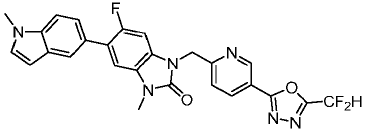 | 228 | 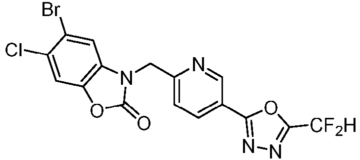 |
| 229 | 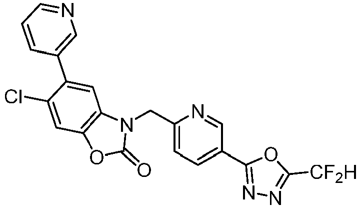 | 230 | 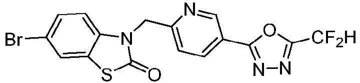 |
| 231 | 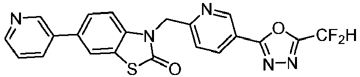 | 232 | 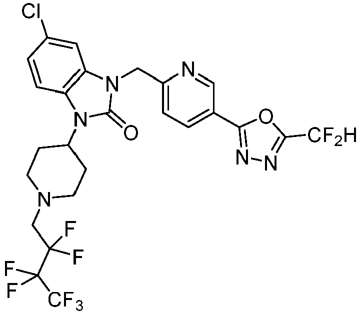 |
| 233 | 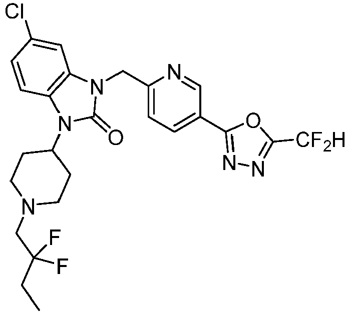 | 234 | 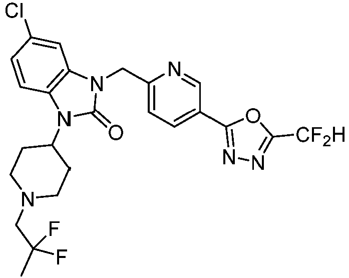 |
| 235 | 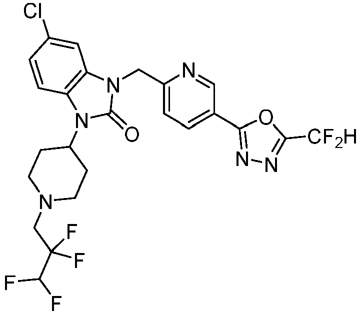 | 236 | 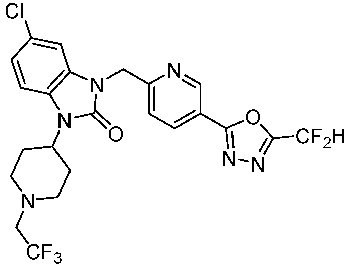 |
| 237 | 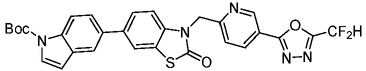 | 238 | 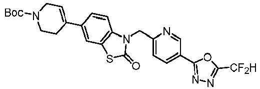 |
| 239 | 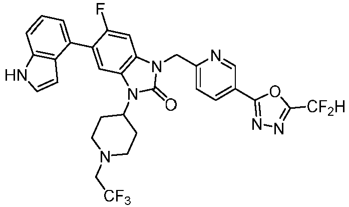 | 240 | 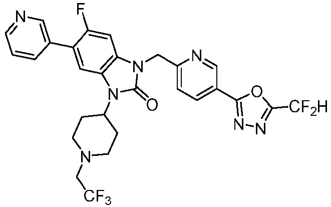 |
| 241 | 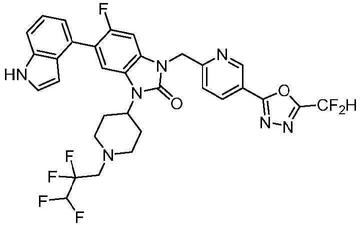 | 242 | 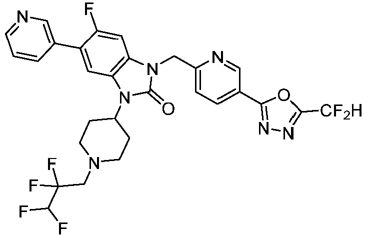 |
| 243 | 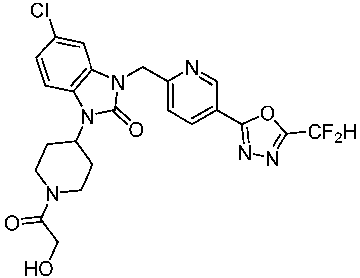 | 244 | 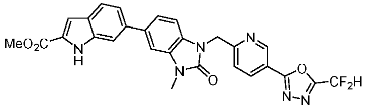 |
| 245 | 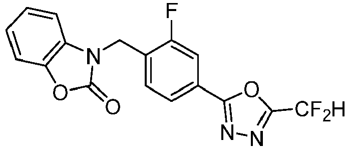 | 246 | 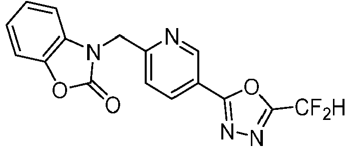 |
| 247 | 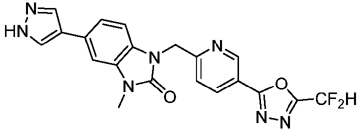 | 248 | 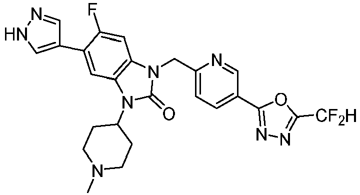 |
| 249 | 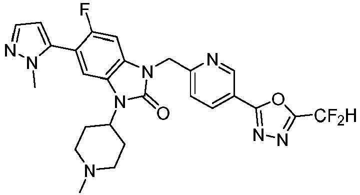 | 250 | 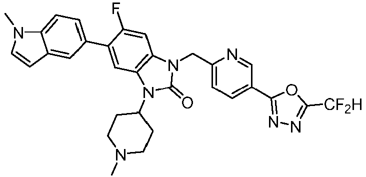 |
| 251 | 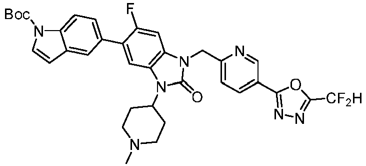 | 252 | 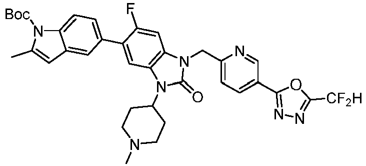 |
| 253 | 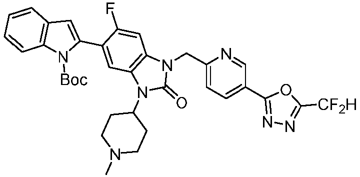 | 254 | 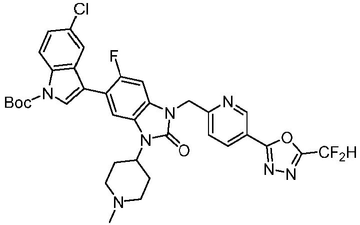 |
| 255 | 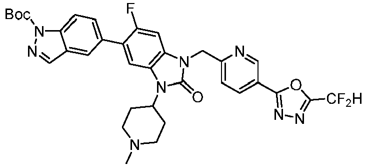 | 256 | 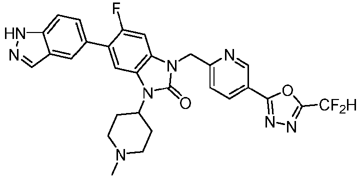 |
| 257 | 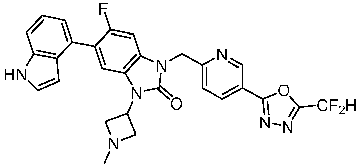 | 258 | 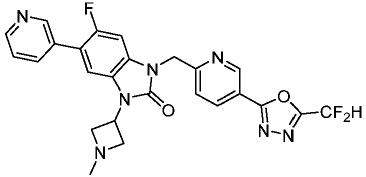 |
| 259 | 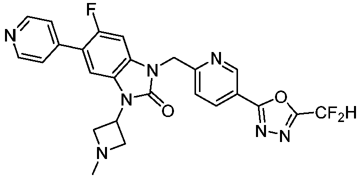 | 260 | 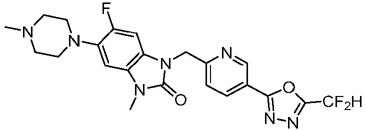 |
| 261 | 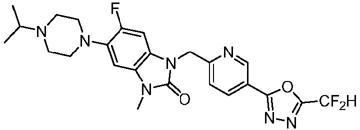 | 262 | 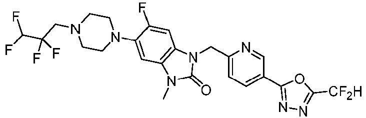 |
| 263 | 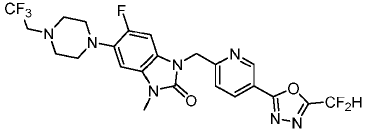 | 264 | 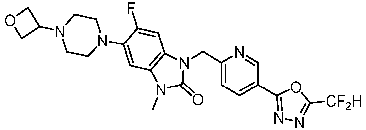 |
| 265 | 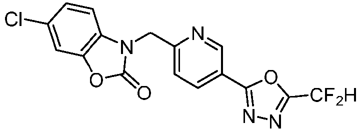 | 266 | 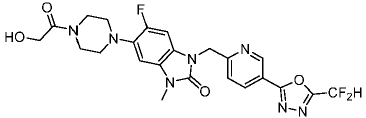 |
| 267 | 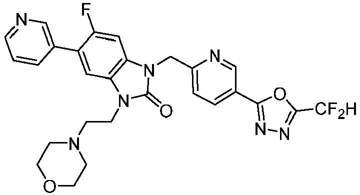 | 268 | 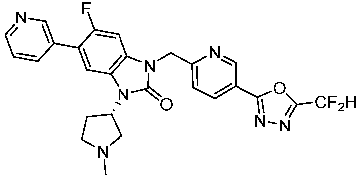 |
| 269 | 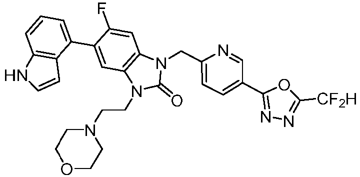 | 270 | 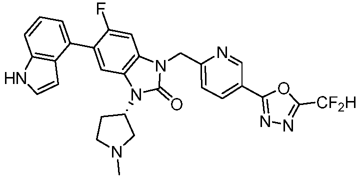 |
| 271 | 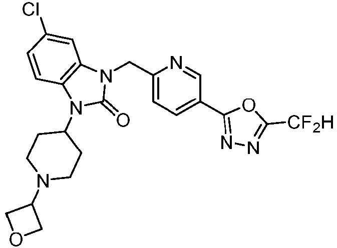 | 272 | 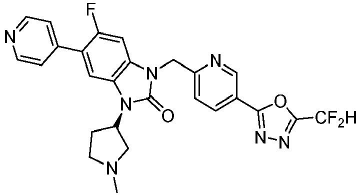 |
| 273 | 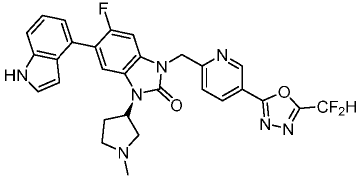 | 274 | 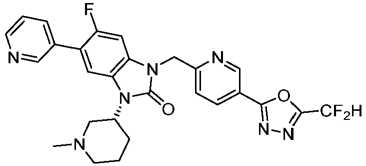 |
| 275 | 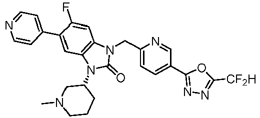 | 276 | 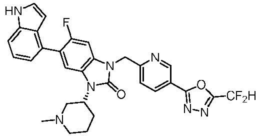 |
| 277 | 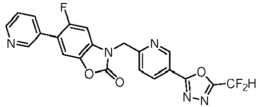 | 278 | 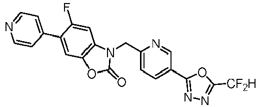 |
| 279 | 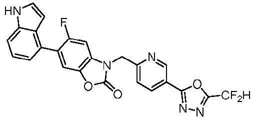 | 280 | 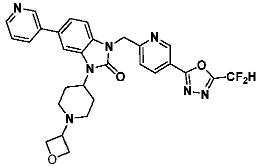 |
| 281 | 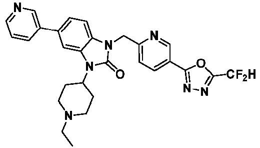 | 282 | 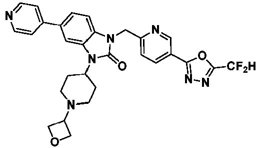 |
| 283 | 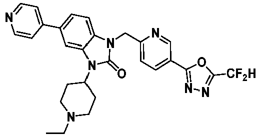 | 284 | 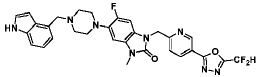 |
| 285 | 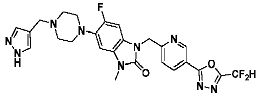 | 286 | 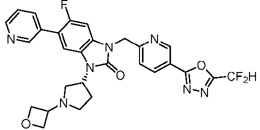 |
| 287 | 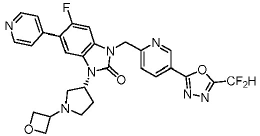 | 288 | 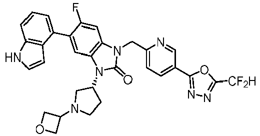 |
| 289 | 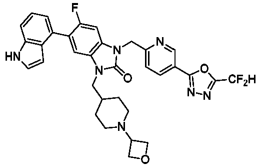 | 290 | 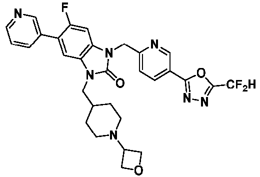 |
| 291 | 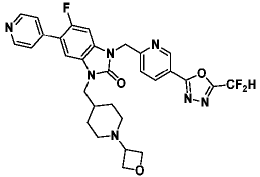 | 292 | 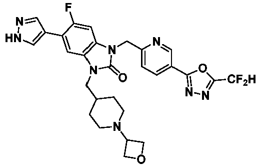 |
| 293 | 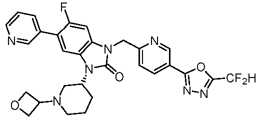 | 294 | 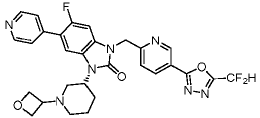 |
| 295 | 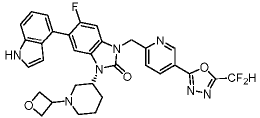 | 296 | 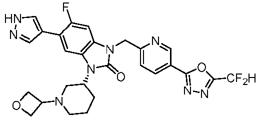 |
| 297 | 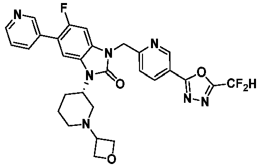 | 298 | 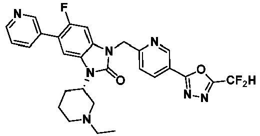 |
| 299 | 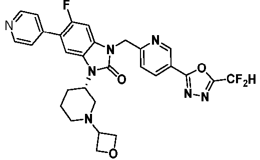 | 300 | 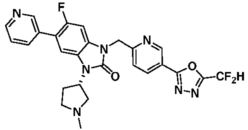 |
| 301 | 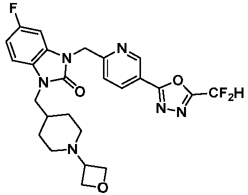 | 302 | 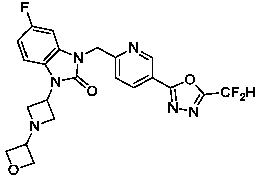 |
| 303 | 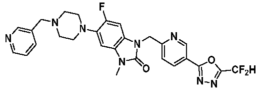 | 304 | 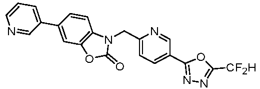 |
| 305 | 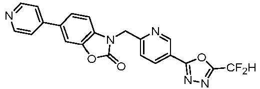 | 306 | 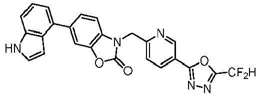 |
| 307 | 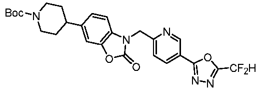 | 308 | 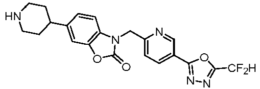 |
| 309 | 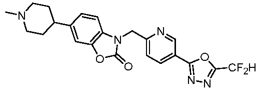 | 310 | 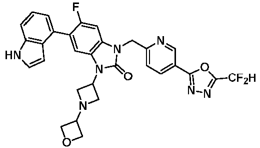 |
| 311 | 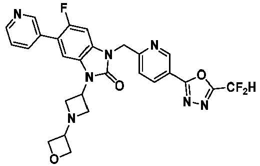 | 312 | 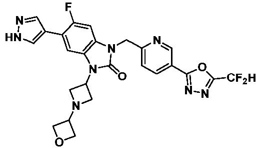 |
| 313 | 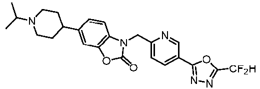 | 314 | 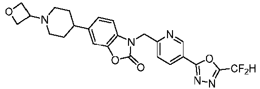 |
| 315 | 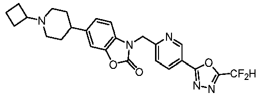 | 316 | 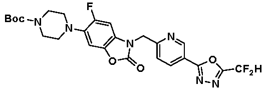 |
| 317 | 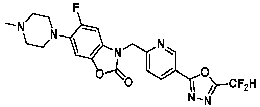 | 318 | 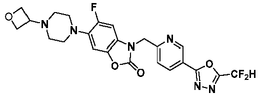 |
| 319 | 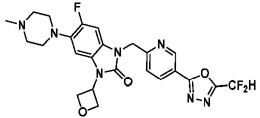 | 320 | 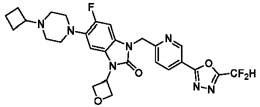 |
| 321 | 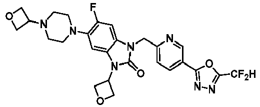 | 322 | 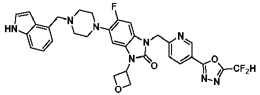 |
| 323 | 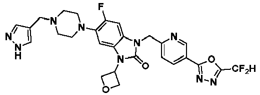 | 324 | 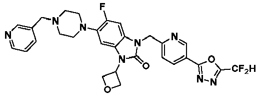 |
| 325 | 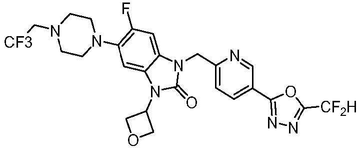 | 326 | 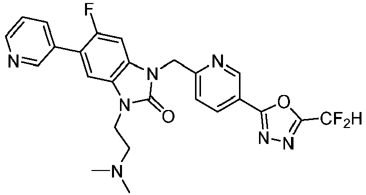 |
| 327 | 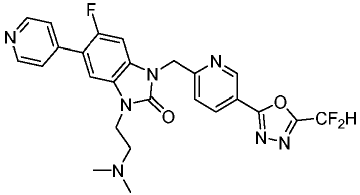 | 328 | 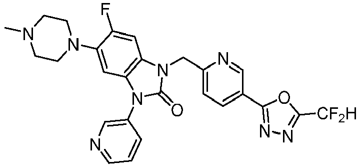 |
| 329 | 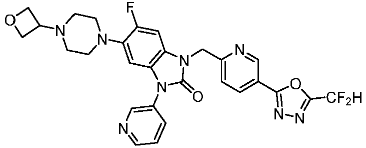 | 330 | 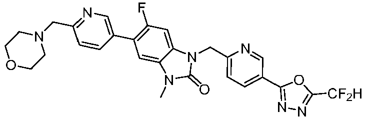 |
| 331 | 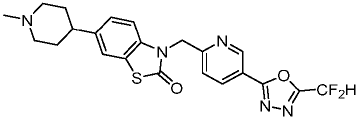 | 332 | 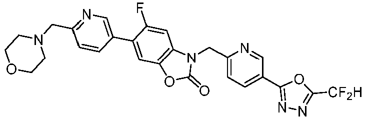 |
| 333 | 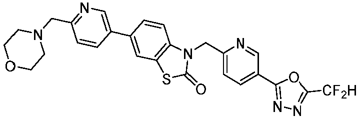 | 334 | 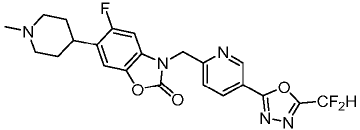 |
| 335 | 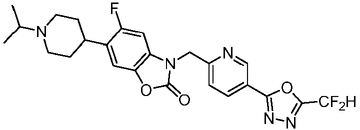 | 336 | 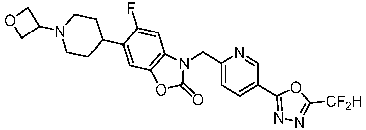 |
| 337 | 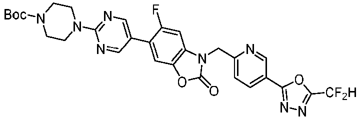 | 338 | 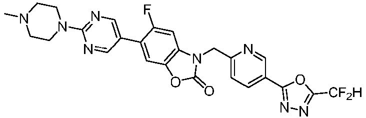 |
| 339 | 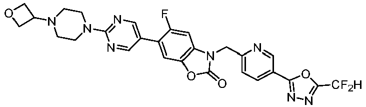 | 340 | 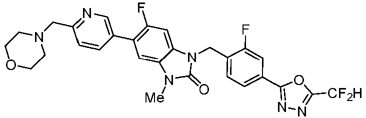 |
| 341 | 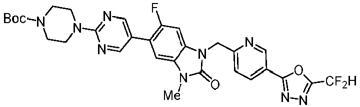 | 342 | 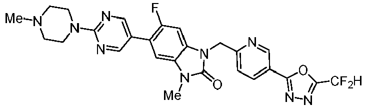 |
| 343 | 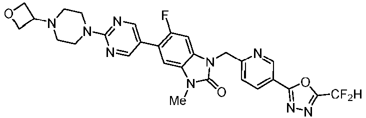 | 344 | 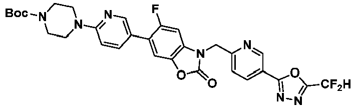 |
| 345 | 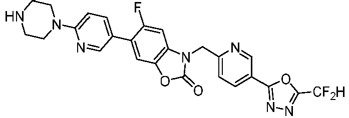 | 346 | 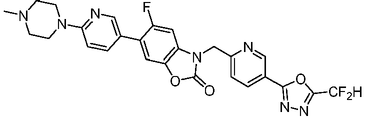 |
| 347 | 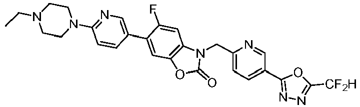 | 348 | 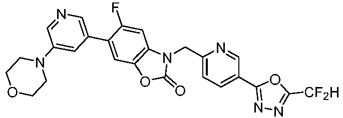 |
| 349 | 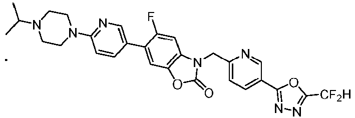 | 350 | 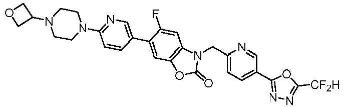 |
| 351 | 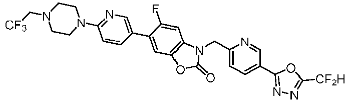 | 352 | 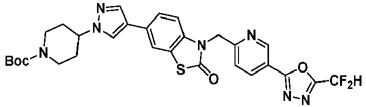 |
| 353 | 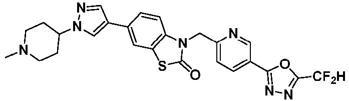 | 354 | 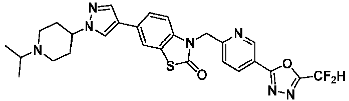 |
| 355 | 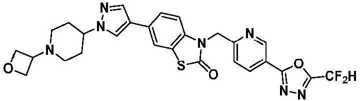 | 356 | 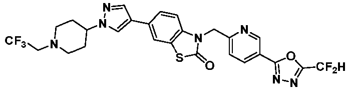 |
| 357 | 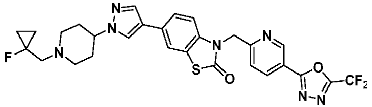 | 358 | 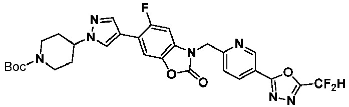 |
| 359 | 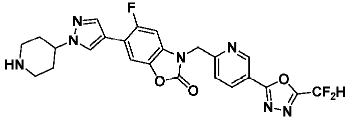 | 360 | 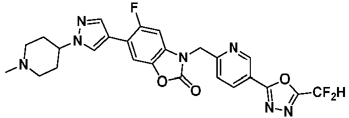 |
| 361 | 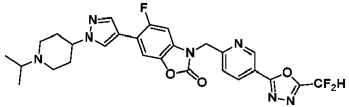 | 362 | 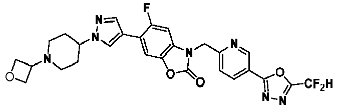 |
| 363 | 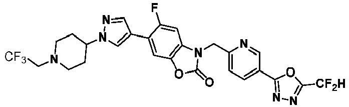 | 364 | 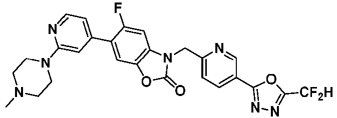 |
| 365 | 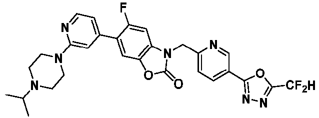 | 366 | 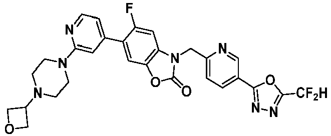 |
| 367 | 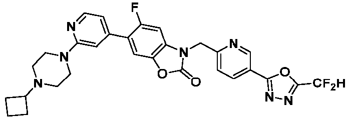 | 368 | 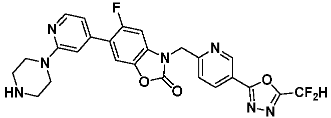 |

9. A pharmaceutical composition comprising the compounds represented by the chemical formula I according to any one of claims 1 to 8, stereoisomers thereof or pharmaceutically acceptable salts thereof as an effective component.

10. The pharmaceutical composition according to claim 9, wherein said pharmaceutical composition is for use in preventing or treating histone deacetylase 6 activity-related diseases.

11. The pharmaceutical composition for use according to claim 10, wherein said histone deacetylase 6 activity-related diseases are at least one selected from the group consisting of infectious diseases, neoplasm, endocrinopathy, nutritional and metabolic diseases, mental and behavioral disorders, neurological diseases, eye and ocular adnexal diseases, circulatory diseases, respiratory diseases, digestive diseases, skin and subcutaneous tissue diseases, musculoskeletal system and connective tissue diseases, and teratosis, deformities and chromosomal aberration.

12. The compounds represented by the chemical formula I according to any one of claims 1 to 8, stereoisomers thereof or pharmaceutically acceptable salts thereof for use in preventing or treating histone deacetylase 6 activity-related diseases.

## Patentansprüche

1. Verbindungen, die durch die folgende chemische Formel I dargestellt sind, Stereoisomere davon oder pharmazeutisch annehmbare Salze davon: 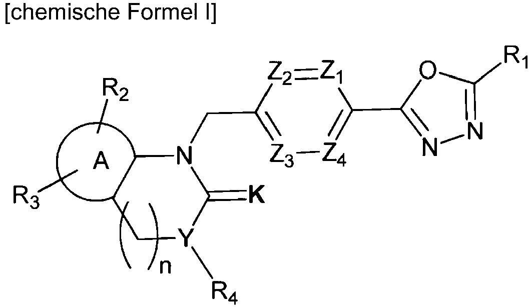 worin
Z₁ bis Z₄ jeweils unabhängig voneinander N oder CRₐ sind, worin Rₐ H, X, C₁-C₄-Alkyl oder O-(C₁-C₄-Alkyl) ist und die Rₐ unterschiedlich sein können, wenn 2 oder mehr CRₐ vorhanden sind;
K O oder S ist;
R₁ CX₃ oder CX₂H ist; 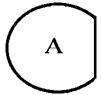 C₆-C₁₂-Arylen oder C₂-C₁₀-Heteroarylen ist;
R₂ und R₃ jeweils unabhängig voneinander H, X, C₁-C₄-Alkyl, C₁-C₄-Halogenalkyl, C₆-C₁₂-Aryl, C₂-C₁₀-Heteroaryl, C₃-C₁₀-Cycloalkyl, C₂-C₁₀-Heterocycloalkyl, C₂-C₁₀-Heterocycloalkenyl, N(R_{b})(R_{c}), NH-(C₁-C₄-Alkyl)-N(R_{b})(R_{c}), NH-O-(C₁-C₄-Alkyl) oder NHC(=O)-R₅ sind;
wobei gegebenenfalls zumindest ein H von C₁-C₄-Alkyl, C₆-C₁₂-Aryl, C₂-C₁₀-Heteroaryl, C₂-C₁₀-Heterocycloalkenyl oder C₂-C₁₀-Heterocycloalkyl jeweils unabhängig durch X, C₁-C₄-Alkyl, C₁-C₄-Halogenalkyl, C₃-C₁₀-Cycloalkyl, C₆-C₁₂-Aryl, C₂-C₁₀-Heteroaryl, C₂-C₁₀-Heterocycloalkyl, (C₁-C₄-Alkyl)-(C₂-C₁₀-heteroaryl), (C₁-C₄-Alkyl)-(C₂-C₁₀-heterocycloalkyl), (C₂-C₁₀-Heterocycloalkyl)-(C₁-C₄-alkyl), (C₂-C₁₀-Heterocycloalkyl)-(C₁-C₄-halogenalkyl), (C₂-C₁₀-Heterocycloalkyl)-(C₃-C₄₀-cycloalkyl), (C₂-C₁₀-Heterocycloalkyl)-(C₂-C₁₀-heterocycloalkyl), (C₂-C₁₀-Heterocycloalkyl)-(C₁-C₄ -alkyl)-(C₃-C₁₀)-halogencycloalkyl), (C₁-C₄-Alkyl)-(C₃-C₁₀-cycloalkyl), S(O₂)-(C₁-C₄-Alkyl), C(=O)-N(R_{b})(R_{c}), C(=O)-R₆, -N(R_{b})(R_{c}), (C₁-C₄-Alkyl)-N(R_{b})(R_{c}), O-(C₁-C₄-Alkyl), (C₁-C₄-Alkyl)-O-(C₁-C₄-alkyl), C(=O)O-(C₂-C₁₀-Heterocycloalkyl), (C₁-C₄-Alkyl)-C(=O)-R₇ oder (C₂-C₁₀-Heterocycloalkyl)- C(=O)-R₈ ersetzt ist;
Y CH, N, O oder S ist {wobei R₄ nicht vorhanden ist, wenn Y O oder S ist};
R₄ H, C₁-C₄-Alkyl, C₃-C₇-Cycloalkyl, C₂-C₁₀-Heterocycloalkyl, (C₁-C₄-Alkyl)-(C₂-C₁₀-Heterocycloalkyl), C₆-C₁₂-Aryl, C₂-C₄₀-Heteroaryl oder C(=O)-R₉ ist,
wobei gegebenenfalls zumindest ein H von C₁-C₄-Alkyl, C₂-C₁₀-Heterocycloalkyl oder (C₁-C₄-Alkyl)-(C₂-C₁₀-heterocycloalkyl) jeweils unabhängig durch X, C₁-C₄-Alkyl, C₁-C₄-Halogenalkyl, O-(C₁-C₄-Alkyl), -N(R_{b})(R_{c}), C₃-C₁₀-Cycloalkyl, C₂-C₁₀-Heterocycloalkyl, (C₂-C₁₀-Heterocycloalkyl)- C(=O)-R₁₀, S(O₂)-(C₁-C₄-Alkyl), C(=O)-R₁₁**,** (C₁-C₄-Alkyl)-O-(C₁-C₄-alkyl), C₆-C₁₂-Aryl, C2-C10 -Heteroaryl, (C₁-C₄-Alkyl)-(C₂-C₁₀-heteroaryl), (C₂-C₁₀-Heterocycloalkyl)-(C₂-C₁₀-heterocycloalkyl) oder C(=O)-(C₁-C₄-Alkyl)-O-(C₁-C₄-alkyl) ersetzt ist;
R₅, R₆, R₇, R₈, R₉, R₁₀ und R₁₁ jeweils unabhängig voneinander H, C₁-C₄-Alkyl, (C₁-C₄-Alkyl)-OH, (C₁-C₄-Alkyl)-O-(C₁-C₄-alkyl), C₂-C₁₀-Heterocycloalkyl, C₆-C₁₂-Aryl, C₂-C₁₀-Heteroaryl, O-(C₁-C₄-Alkyl), C₃-C₇-Cycloalkyl oder (C₁-C₄-Alkyl)-N(Rb)(Rc) sind,
wobei gegebenenfalls zumindest ein H von C₆-C₁₂-Aryl oder C₂-C₁₀-Heteroaryl jeweils unabhängig durch C₁-C₄-Alkyl, X oder C₁-C₄-Halogenalkyl ersetzt ist;
R_{b} und R_{c} jeweils unabhängig voneinander H, C₁-C₄-Alkyl, C₆-C₁₂-Aryl, C₂-C₁₀-Heteroaryl, C₃-C₁₀-Cycloalkyl, C₂-C₁₀-Heterocycloalkyl, (C₁-C₄-Alkyl)NH(C₁-C₄-alkyl), (C₁-C₄-Alkyl)-N(C₁-C₄-alkyl)₂, (C₁-C₄-Alkyl)-O-(C₁-C₄-alkyl), C(=O)-(C₁-C₄-Alkyl), C(=O)-(C₂-C₁₀-Heteroaryl), C(=O)-(C₂-C₁₀-Heterocycloalkyl) oder C(=O)-(C₃-C₁₀-Cycloalkyl) sind;
X ein Halogenatom ist; und
n jeweils eine ganze Zahl ist, die aus 0, 1, 2 und 3 ausgewählt ist.

2. Verbindungen, die durch die chemische Formel I dargestellt sind, Stereoisomere davon oder pharmazeutisch annehmbare Salze davon nach Anspruch 1, wobei in der obigen chemischen Formel I:
Z₁ bis Z₄ jeweils unabhängig voneinander N oder CRₐ sind, worin Rₐ H, X, C₁-C₄-Alkyl oder O-(C₁-C₄-Alkyl) ist und die Rₐ unterschiedlich sein können, wenn 2 oder mehr CR vorhanden sind;
K O oder S ist;
R₁ CX₃ oder CX₂H ist; 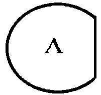 C₆-C₁₂-Arylen oder C₂-C₁₀-Heteroarylen ist, wobei das C₂-C₁₀-Heteroarylen gegebenenfalls zumindest ein N umfasst;
R₂ und R₃ jeweils unabhängig voneinander H, X, C₁-C₄-Halogenalkyl, C₆-C₁₂-Aryl, C₂-C₁₀-Heterocycloalkyl, C₂-C₁₀-Heterocycloalkenyl, N(R_{b})(R_{c}) oder C₂-C₁₀-Heteroaryl sind, wobei gegebenenfalls zumindest ein H von C₆-C₁₂-Aryl, C₂-C₁₀-Heteroaryl, C₂-C₁₀-Heterocycloalkenyl oder C₂-C₄₀-Heterocycloalkyl jeweils unabhängig durch X, C₁-C₄-Alkyl, C₁-C₄-Halogenalkyl, C₃-C₁₀-Cycloalkyl, C₂-C₄₀-Heterocycloalkyl, (C₁-C₄-Alkyl)-(C₂-C₁₀-heteroaryl), (C₁-C₄-Alkyl)-(C₂-C₁₀-heterocycloalkyl), (C₂-C₁₀-Heterocycloalkyl)-(C₁-C₄-alkyl), (C₂-C₁₀-Heterocycloalkyl)-(C₁-C₄-halogenalkyl), (C₂-C₁₀-Heterocycloalkyl)-(C₃-C₁₀-cycloalkyl), (C₂-C₁₀-Heterocycloalkyl)-(C₂-C₁₀-heterocycloalkyl), (C₂-C₁₀-Heterocycloalkyl)-(C₁-C₄-alkyl)-(C₃-C₁₀-halogencycloalkyl), S(O₂)-(C₁-C₄-Alkyl), C(=O)-R₆, O-(C₁-C₄-Alkyl) oder (C₂-C₁₀-Heterocycloalkyl)-C(=O)-R₈ ersetzt ist;
Y CH, N, O oder S ist {wobei R nicht vorhanden ist, wenn Y O oder S ist};
R₄ C₁-C₄-Alkyl, C₂-C₁₀-Heterocycloalkyl, C₂-C₁₀-Heteroaryl oder (C₁-C₄-Alkyl)-(C₂-C₁₀-heterocycloalkyl) ist,
wobei gegebenenfalls zumindest ein H von C₁-C₄-Alkyl, C₂-C₁₀-Heterocycloalkyl oder (C₁-C₄-Alkyl)-(C₂-C₁₀-heterocycloalkyl) jeweils unabhängig durch C₁-C₄-Alkyl, C₁-C₄-Halogenalkyl, O-(C₁-C₄-Alkyl), -N(R_{b})(R_{c}), C₃-C₁₀-Cycloalkyl, C₂-C₁₀-Heterocycloalkyl, (C₂-C₁₀-Heterocycloalkyl)-C(=O)-R₁₀, S(O₂)-(C₁-C₄-Alkyl), C(=O)-R₁₁, (C₁-C₄-Alkyl)-O-(C₁-C₄-alkyl), C₆-C₁₂-Aryl, (C₂-C₁₀-Heterocycloalkyl)-(C₂-C₁₀-heterocycloalkyl) oder (C₁-C₄-Alkyl)-(C₂-C₁₀-heteroaryl) ersetzt ist;
R₆, R₈, R₁₀ und R₁₁ jeweils unabhängig voneinander C₁-C₄-Akyl, (C₁-C₄-Alkyl)-OH, (C₁-C₄-Alkyl)-O-(C₁-C₄-alkyl), C₂-C₁₀-Heterocycloalkyl, C₆-C₁₂-Aryl, C₂-C₁₀-Heteroaryl oder O-(C₁-C₄-Alkyl) sind, wobei gegebenenfalls zumindest ein H von C₆-C₁₂-Aryl oder C₂-C₁₀-Heteroaryl jeweils unabhängig durch C₁-C₄-Alkyl oder C₁-C₄-Halogenalkyl ersetzt ist;
R_{b} und R_{c} jeweils unabhängig voneinander H, C₁-C₄-Alkyl oder C(=O)-(C₁-C₄-Alkyl) sind;
X ein Halogenatom ist; und
n jeweils eine ganze Zahl ist, die aus 0, 1, 2 und 3 ausgewählt ist.

3. Verbindungen, die durch die chemische Formel I dargestellt sind, Stereoisomere davon oder pharmazeutisch annehmbare Salze davon nach Anspruch 1, wobei in der obigen chemischen Formel I:
C₂-C₁₀-Heterocycloalkyl 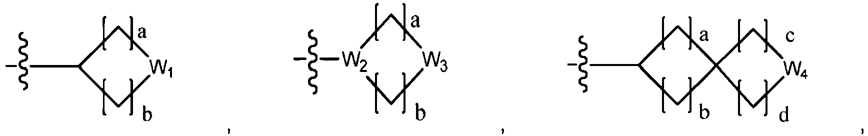 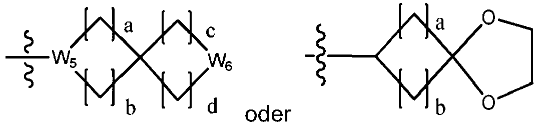 ist, worin W₁ bis W₆ jeweils unabhängig voneinander N, NH, O, S oder SO₂ sind und
a bis d jeweils unabhängig voneinander die ganze Zahl 1, 2 oder 3 sind.

4. Verbindungen, die durch die chemische Formel I dargestellt sind, Stereoisomere davon oder pharmazeutisch annehmbare Salze davon nach Anspruch 1, wobei die durch die obige chemische Formel I dargestellten Verbindungen durch die folgende chemische Formel II dargestellte Verbindungen umfassen: 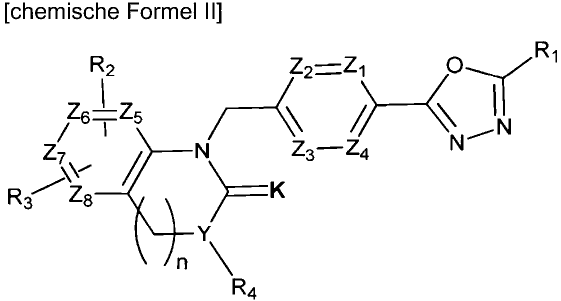 worin
Z₁ bis Z₄ jeweils unabhängig voneinander N oder CRₐ sind, worin Rₐ H, X, C₁-C₄-Alkyl oder O-(C₁-C₄-Alkyl) ist und die Rₐ unterschiedlich sein können, wenn 2 oder mehr CR vorhanden sind;
Z₅ bis Z₈ jeweils unabhängig voneinander CR₂, CR₃, CH oder N sind, worin Z₅ bis Z₈ CR₂, CR₃, CH und N umfassen, CR₂, CR₃ und zwei N umfassen oder CR₂, CR₃ und zwei CH umfassen;
K O oder S ist;
R₁ CX₃ oder CX₂H ist;
R₂ und R₃ jeweils unabhängig voneinander H, X, C₁-C₄-Alkyl, C₁-C₄-Halogenalkyl, C₆-C₁₂-Aryl, C₂-C₁₀-Heteroaryl, C₃-C₁₀-Cycloalkyl, C₂-C₄₀-Heterocycloalkyl, C₂-C₁₀-Hetero-cycloalkenyl, N(R_{b})(R_{c}), NH-(C₁-C₄-Alkyl)-N(R_{b})(R_{c}), NH-O-(C₁-C₄-Alkyl) oder NHC(=O)-R₅ sind,
wobei gegebenenfalls zumindest ein H von C₁-C₄-Alkyl, C₆-C₁₂-Aryl, C₂-C₁₀-Heteroaryl, C₂-C₁₀-Heterocycloalkenyl oder C₂-C₁₀-Heterocycloalkyl jeweils unabhängig durch X, C₁-C₄-Alkyl, C₁-C₄-Halogenalkyl, C₃-C₁₀-Cycloalkyl, C₆-C₁₂-Aryl, C₂-C₁₀-Heteroaryl, C₂-C₁₀-Heterocycloalkyl, (C₁-C₄-Alkyl)-(C₂-C₁₀-heteroaryl), (C₁-C₄-Alkyl)-(C₂-C₁₀-heterocycloalkyl), (C₂-C₁₀-Heterocycloalkyl)-(C₁-C₄-alkyl), (C₂-C₁₀-Heterocycloalkyl)-(C₁-C₄-halogenalkyl), (C₂-C₁₀-Heterocycloalkyl)-(C₃-C₁₀-cycloalkyl), (C₂-C₁₀-Heterocycloalkyl)-(C₂-C₁₀-heterocyclo-alkyl), (C₂-C₁₀-Heterocycloalkyl)-(C₁-C₄-alkyl)-(C₃-C₁₀-halogencycloalkyl), (C₁-C₄-Alkyl)-(C₃-C₁₀-cycloalkyl), S(O₂)-(C₁-C₄-Alkyl), C(=O)- N(R_{b})(R_{c}), C(=O)-R₆, -N(R_{b})(R_{c}), (C₁-C₄-Alkyl)-N(R_{b})(R_{c}), O-(C₁-C₄-Alkyl), (C₁-C₄-Alkyl)-O-(C₁-C₄-alkyl), C(=O)O-(C₂-C₁₀-Heterocyclo-alkyl), (C₁-C₄-Alkyl)-C(=O)-R₇ oder (C₂-C₁₀-Heterocycloalkyl)-C(=O)-R₈ ersetzt ist;
Y CH, N, O oder S ist {wobei R₄ nicht vorhanden ist, wenn Y O oder S ist};
R₄ H, C₁-C₄-Alkyl, C₃-C₇-Cycloalkyl, C₂-C₁₀-Heterocycloalkyl, (C₁-C₄-Alkyl)-(C₂-C₁₀-heterocycloalkyl), C₆-C₁₂-Aryl, C₂-C₁₀-Heteroaryl oder C(=O)-R₉ ist,
wobei gegebenenfalls zumindest ein H von C₁-C₄-Alkyl, C₂-C₁₀-Heterocycloalkyl oder (C₁-C₄-Alkyl)-(C₂-C₁₀-heterocycloalkyl) jeweils unabhängig durch X, C₁-C₄-Alkyl, C₁-C₄-Halogenalkyl, O- (C₁-C₄-Alkyl), -N(R_{b})(Rc), C₃-C₁₀-Cycloalkyl, C₂-C₁₀-Heterocycloalkyl, (C₂-C₁₀-Heterocycloalkyl)-C(=O)-R₁₀, S(O₂)-(C₁-C₄-Alkyl), C(=O)-R₁₁, (C₁-C₄-Alkyl)-O-(C₁-C₄-alkyl), C₆-C₁₂-Aryl, C₂-C₁₀-Heteroaryl, (C₁-C₄-Alkyl)-(C₂-C₁₀-heteroaryl), (C₂-C₄₀-Heterocycloalkyl)-(C₂-C₁₀-heterocycloalkyl) oder C(=O)-(C₁-C₄-Alkyl)-O-(C₁-C₄-alkyl) ersetzt ist;
R₅, R₆, R₇, R₈, R₉, R₁₀ und R₁₁ jeweils unabhängig voneinander H, C₁-C₄-Alkyl, (C₁-C₄-Alkyl)-OH, (C₁-C₄-Alkyl)-O-(C₁-C₄-alkyl), C₂-C₁₀-Heterocycloalkyl, C₆-C₁₂-Aryl, C₂-C₁₀-Heteroaryl, O-(C₁-C₄-Alkyl), C₃-C₇-Cycloalkyl oder (C₁-C₄-Alkyl)-N(R_{b})(R_{c}) sind,
wobei gegebenenfalls zumindest ein H von C₆-C₁₂-Aryl oder C₂-C₁₀-Heteroaryl jeweils unabhängig durch C₁-C₄-Alkyl, X oder C₁-C₄-Halogenalkyl ersetzt ist;
R_{b} und R_{c} jeweils unabhängig voneinander H, C₁-C₄-Alkyl, C₆-C₁₂-Aryl, C₂-C₁₀-Heteroaryl, C₃-C₁₀-Cycloalkyl, C₂-C₁₀-Heterocycloalkyl, (C₁-C₄-Alkyl)NH(C₁-C₄-alkyl), (C₁-C₄-Alkyl)-N(C₁-C₄-alkyl)₂, (C₁-C₄-Alkyl)-O-(C₁-C₄-alkyl), C(=O)-(C₁-C₄-Alkyl), C(=O)-(C₂-C₁₀-Heteroaryl), C(=O)-(C₂-C₁₀-Heterocycloalkyl) oder C(=O)-(C₃-C₁₀-Cycloalkyl) sind;
X ein Halogenatom ist; und
n jeweils eine ganze Zahl ist, die aus 0, 1, 2 und 3 ausgewählt ist.

5. Verbindungen, die durch die chemische Formel I dargestellt sind, Stereoisomere davon oder pharmazeutisch annehmbare Salze davon nach Anspruch 4, worin:
Z₁ bis Z₄ jeweils unabhängig voneinander N oder CRₐ sind, worin Rₐ H, X, C₁-C₄-Alkyl oder O-(C₁-C₄-Alkyl) ist und die Rₐ unterschiedlich sein können, wenn 2 oder mehr CR vorhanden sind;
Z₅ bis Z₈ jeweils unabhängig voneinander CR₂, CR₃, CH oder N sind, wobei Z₅ bis Z₈ CR₂, CR₃, CH und N umfassen oder CR₂, CR₃ und zwei CH umfassen (wobei Z₆ jedoch N ist, wenn Z₅ bis Z₈ CR₂, CR₃, CH und N umfassen);
K O oder S ist;
R₁ CX₃ oder CX₂H ist;
R₂ und R₃ jeweils unabhängig voneinander H, X, C₁-C₄-Halogenalkyl, C₆-C₁₂-Aryl, C₂-C₁₀-Heterocycloalkyl, C₂-C₁₀-Heterocycloalkenyl, N(R_{b})(R_{c}) oder C₂-C₁₀-Heteroaryl sind,
wobei gegebenenfalls zumindest ein H von C₆-C₁₂-Aryl, C₂-C₁₀-Heteroaryl, C₂-C₁₀-Heterocycloalkenyl oder C₂-C₁₀-Heterocycloalkyl jeweils unabhängig durch X, C₁-C₄-Alkyl, C₁-C₄-Halogenalkyl, C₃-C₁₀-Cycloalkyl, C₂-C₁₀-Heterocycloalkyl, (C₁-C₄-Alkyl)-(C₂-C₁₀-heteroaryl), (C₁-C₄-Alkyl)-(C₂-C₁₀-heterocycloalkyl), (C₂-C₁₀-Heterocycloalkyl)-(C₁-C₄-alkyl), (C₂-C₁₀-Heterocycloalkyl)-(C₁-C₄-halogenalkyl), (C₂-C₁₀-Heterocycloalkyl)-(C₃-C₁₀-cycloalkyl), (C₂-C₁₀-Heterocycloalkyl)-(C₂-C₁₀-heterocycloalkyl), (C₂-C₁₀-Heterocycloalkyl)-(C₁-C₄-alkyl)-(C₃-C₁₀-halogencycloalkyl), S(O₂)-(C₁-C₄-Alkyl), C(=O)-R₆, O-(C₁-C₄-Alkyl) oder (C₂-C₁₀-Heterocyclo-alkyl)-C(=O)-R₈ ersetzt ist;
Y CH, N, O oder S ist {wobei R₄ nicht vorhanden ist, wenn Y O oder S ist};
R₄ C₁-C₄-Alkyl, C₂-C₁₀-Heterocycloalkyl, C₂-C₁₀-Heteroaryl oder (C₁-C₄-Alkyl)-(C₂-C₁₀-heterocycloalkyl) ist,
wobei gegebenenfalls zumindest ein H von C₁-C₄-Alkyl, C₂-C₁₀-Heterocycloalkyl oder (C₁-C₄-Alkyl)-(C₂-C₁₀-heterocycloalkyl) jeweils unabhängig durch C₁-C₄-Alkyl, C₁-C₄-Halogenalkyl, O-(C₁-C₄-Alkyl), -N(R_{b})(R_{c}), C₃-C₁₀-Cycloalkyl, C₂-C₁₀-Heterocycloalkyl, (C₂-C₁₀-Hetero-cycloalkyl)-C(=O)-R₁₀, S(O₂)-(C₁-C₄-alkyl), C(=O)-R₁₁, (C₁-C₄-Alkyl)-O-(C₁-C₄-alkyl), C₆-C₁₂-Aryl, (C₂-C₁₀-Heterocycloalkyl)-(C₂-C₁₀-heterocycloalkyl) oder (C₁-C₄-Alkyl)-(C₂-C₁₀-heteroaryl) ersetzt ist;
R₆, R₈, R₁₀ und R₁₁ jeweils unabhängig voneinander C₁-C₄-Alkyl, (C₁-C₄-Alkyl)-OH, (C₁-C₄-Alkyl)-O-(C₁-C₄-alkyl), C₂-C₁₀-Heterocycloalkyl, C₆-C₁₂-Aryl, C₂-C₁₀-Heteroaryl oder O-(C₁-C₄-alkyl) sind,
wobei gegebenenfalls zumindest ein H von C₆-C₁₂-Aryl oder C₂-C₁₀-Heteroaryl jeweils unabhängig durch C₁-C₄-Alkyl oder C₁-C₄-Halogenalkyl ersetzt ist;
R_{b} und R_{c} jeweils unabhängig voneinander H, C₁-C₄-Alkyl oder C(=O)-(C₁-C₄-Alkyl) sind;
X ein Halogenatom ist; und
n jeweils eine ganze Zahl ist, die aus 0, 1, 2 und 3 ausgewählt ist.

6. Verbindungen, die durch die chemische Formel I dargestellt sind, Stereoisomere davon oder pharmazeutisch annehmbare Salze davon nach Anspruch 4, wobei in der obigen chemischen Formel II:
Z₁ bis Z₄ jeweils unabhängig voneinander N oder CRₐ sind, worin Rₐ H, X, C₁-C₄-Alkyl oder O-(C₁-C₄-Alkyl) ist und die Ra unterschiedlich sein können, wenn 2 oder mehr CR vorhanden sind;
Z₅ bis Z₈ jeweils unabhängig voneinander CR₂, CR₃, CH oder N sind, wobei Z₅ bis Z₈ CR₂, CR₃, CH und N umfassen oder CR₂, CR₃ und zwei CH umfassen (wobei Z₆ jedoch N ist, wenn Z₅ bis Z₈ CR₂, CR₃, CH und N umfassen);
K O oder S ist;
R₁ CX₃ oder CX₂H ist;
R₂ und R₃ jeweils unabhängig voneinander H, X, C₁-C₄-Halogenalkyl, C₆-C₁₂-Aryl, C₂-C₁₀-Heterocycloalkyl, C₂-C₁₀-Heterocycloalkenyl, N(R_{b})(R_{c}) oder C₂-C₁₀-Heteroaryl sind,
wobei gegebenenfalls zumindest ein H von C₆-C₁₂-Aryl, C₂-C₁₀-Heteroaryl, C₂-C₁₀-Heterocycloalkenyl oder C₂-C₁₀-Heterocycloalkyl jeweils unabhängig durch X, C₁-C₄-Alkyl, C₁-C₄-Halogenalkyl, C₃-C₁₀-Cycloalkyl, C₂-C₁₀-Heterocycloalkyl, (C₁-C₄-Alkyl)-(C₂-C₁₀-heteroaryl), (C₁-C₄-Alkyl)-(C₂-C₁₀-heterocycloalkyl), (C₂-C₁₀-Heterocycloalkyl)-(C₁-C₄-alkyl), (C₂-C₁₀-Heterocycloalkyl)-(C₁-C₄-halogenalkyl), (C₂-C₁₀-Heterocycloalkyl)-(C₃-C₁₀-cycloalkyl), (C₂-C₁₀-Heterocycloalkyl)-(C₂-C₁₀-heterocycloalkyl), (C₂-C₁₀-Heterocycloalkyl)-(C₁-C₄-alkyl)-(C₃-C₁₀-halogencycloalkyl), S(O₂)-(C₁-C₄-alkyl), C(=O)-R₆, O-(C₁-C₄-alkyl) oder (C₂-C₁₀-Heterocycloalkyl)-C(=O)-R₈ ersetzt ist;
Y N, O oder S ist {wobei R₄ nicht vorhanden ist, wenn Y O oder S ist};
R₄ C₁-C₄-Alkyl, C₂-C₁₀-Heterocycloalkyl, C₂-C₁₀-Heteroaryl oder (C₁-C₄-Alkyl)-(C₂-C₁₀-heterocycloalkyl) ist,
wobei gegebenenfalls zumindest ein H von C₁-C₄-Alkyl, C₂-C₁₀-Heterocycloalkyl oder (C₁-C₄-Alkyl)-(C₂-C₁₀-heterocycloalkyl) jeweils unabhängig durch C₁-C₄-Alkyl, C₁-C₄-Halogenalkyl, O-(C₁-C₄-Alkyl), -N(R_{b})(R_{c}), C₃-C₁₀-Cycloalkyl, C₂-C₁₀-Heterocycloalkyl, (C₂-C₁₀-Hetero-cycloalkyl)-C(=O)-R₁₀, S(O₂)-(C₁-C₄-Alkyl), C(=O)-R₁₁, (C₁-C₄-Alkyl)-O-(C₁-C₄-alkyl), C₆-C₁₂-Aryl, (C₂-C₁₀-Heterocycloalkyl)-(C₂-C₁₀-heterocycloalkyl) oder (C₁-C₄-Alkyl)-(C₂-C₁₀-heteroaryl) ersetzt ist;
R₆, R₈, R₁₀ und R₁₁ jeweils unabhängig voneinander C₁-C₄-Alkyl, (C₁-C₄-Alkyl)-OH, (C₁-C₄-Alkyl)-O-(C₁-C₄-alkyl), C₂-C₁₀-Heterocycloalkyl, C₆-C₁₂-Aryl, C₂-C₁₀-Heteroaryl oder O-(C₁-C₄-alkyl) sind,
wobei gegebenenfalls zumindest ein H von C₆-C₁₂-Aryl oder C₂-C₁₀-Heteroaryl jeweils unabhängig durch C₁-C₄-Alkyl oder C₁-C₄-Halogenalkyl ersetzt ist;
R_{b} und R_{c} jeweils unabhängig voneinander H, C₁-C₄-Alkyl oder C(=O)-(C₁-C₄-Alkyl) sind;
X ein Halogenatom ist; und
n jeweils eine ganze Zahl ist, die aus 0, 1, 2 und 3 ausgewählt ist.

7. Verbindungen, die durch die chemische Formel I dargestellt sind, Stereoisomere davon oder pharmazeutisch annehmbare Salze davon nach Anspruch 1, wobei in der obigen chemischen Formel I:
Z₁ bis Z₄ jeweils unabhängig voneinander N oder CRₐ sind, worin Rₐ H oder X ist und die Rₐ unterschiedlich sein können, wenn 2 oder mehr CRₐ vorhanden sind;
K O ist;
R₁ CF₃ oder CF₂H ist; 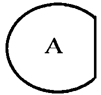 Phenylen oder Pyridinylen ist,
R₂ und R₃ jeweils unabhängig voneinander H, X, C₁-C₄-Halogenalkyl, C₆-C₁₂-Aryl, C₂-C₁₀-Heterocycloalkyl, C₂-C₁₀-Heterocycloalkenyl, N(R_{b})(R_{c}) oder C₂-C₁₀-Heteroaryl sind,
wobei gegebenenfalls zumindest ein H von C₆-C₁₂-Aryl, C₂-C₁₀-Heteroaryl, C₂-C₁₀-Heterocycloalkenyl oder C₂-C₁₀-Heterocycloalkyl jeweils unabhängig durch X, C₁-C₄-Alkyl, C₁-C₄-Halogenalkyl, C₃-C₁₀-Cycloalkyl, C₂-C₁₀-Heterocycloalkyl, (C₁-C₄-Alkyl)-(C₂-C₁₀-heteroaryl), (C₁-C₄-Alkyl)-(C₂-C₁₀-heterocycloalkyl), (C₂-C₁₀-Heterocycloalkyl)-(C₁-C₄-alkyl), (C₂-C₁₀-Heterocycloalkyl)-(C₁-C₄-halogenalkyl), (C₂-C₁₀-Heterocycloalkyl)-(C₃-C₁₀-cycloalkyl), (C₂-C₁₀-Heterocycloalkyl)-(C₂-C₁₀-heterocycloalkyl), (C₂-C₁₀-Heterocycloalkyl)-(C₁-C₄-alkyl)-(C₃-C₁₀-halogencycloalkyl), S(O₂)-(C₁-C₄-Alkyl), C(=O)-R₆, O-(C₁-C₄-Alkyl) oder (C₂-C₁₀-Heterocyclo-alkyl)-C(=O)-R₈ ersetzt ist;
Y N, O oder S ist {wobei R₄ nicht vorhanden ist, wenn Y O oder S ist};
R₄ C₁-C₄-Alkyl, C₂-C₁₀-Heterocycloalkyl, C₂-C₁₀-Heteroaryl oder (C₁-C₄-Alkyl)-(C₂-C₁₀-heterocycloalkyl) ist,
wobei gegebenenfalls zumindest ein H von C₁-C₄-Alkyl, C₂-C₁₀-Heterocycloalkyl oder (C₁-C₄-Alkyl)-(C₂-C₁₀-heterocycloalkyl) jeweils unabhängig durch C₁-C₄-Alkyl, C₁-C₄-Halogenalkyl, O-(C₁-C₄-Alkyl), -N(R_{b})(R_{c}), C₃-C₁₀-Cycloalkyl, C₂-C₁₀-Heterocycloalkyl, (C₂-C₁₀-Hetero-cycloalkyl)-C(=O)- R₁₀, S(O₂)-(C₁-C₄-Alkyl), C(=O)-R₁₁, (C₁-C₄-Alkyl)-O-(C₁-C₄-alkyl), C₆-C₁₂-Aryl, (C₂-C₁₀-Heterocycloalkyl)-(C₂-C₁₀-heterocycloalkyl) oder (C₁-C₄-Alkyl)-(C₂-C₁₀-heteroaryl) ersetzt ist;
R₆ C₁-C₄-Alkyl, O-(C₁-C₄-Alkyl) oder (C₁-C₄-Alkyl)-OH ist;
R₈ O-(C₁-C₄-Alkyl) ist;
R₁₀ C₁-C₄-Alkyl ist;
R₁₁ C₁-C₄-Alkyl, (C₁-C₄-Alkyl)-OH, (C₁-C₄-Alkyl)-O-(C₁-C₄-alkyl), C₂-C₁₀-Heterocyclo-alkyl, C₆-C₁₂-Aryl, C₂-C₁₀-Heteroaryl oder O-(C₁-C₄-Alkyl) ist,
wobei gegebenenfalls zumindest ein H von C₆-C₁₂-Aryl oder C₂-C₁₀-Heteroaryl jeweils unabhängig durch C₁-C₄-Alkyl oder C₁-C₄-Halogenalkyl ersetzt ist;
R_{b} und R_{c} jeweils unabhängig voneinander H, C₁-C₄-Alkyl oder C(=O)-(C₁-C₄-Alkyl) sind;
X F, Cl oder Br ist; und
n = 0 oder 1 ist.

8. Verbindungen, die durch die chemische Formel I dargestellt sind, Stereoisomere davon oder pharmazeutisch annehmbare Salze davon nach Anspruch 1, wobei die Verbindungen aus der Gruppe ausgewählt sind, die aus Verbindungen besteht, die durch die folgenden Verbindungen 1 bis 368 dargestellt sind:
| Verbindung | Struktur | Verbindung | Struktur |
|---|---|---|---|
| 1 | 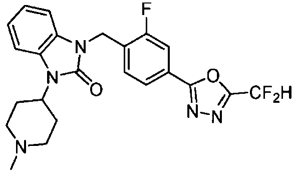 | 2 | 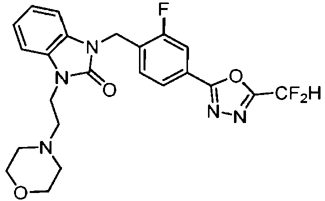 |
| 3 | 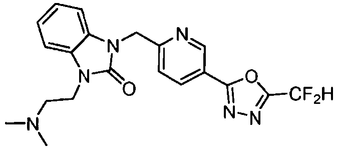 | 4 | 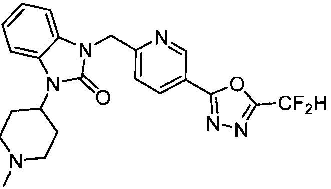 |
| 5 | 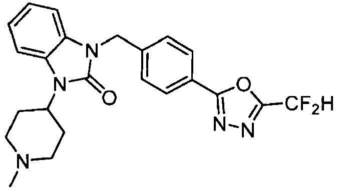 | 6 | 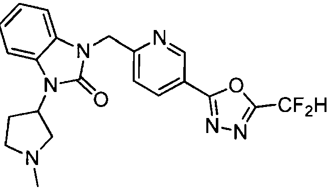 |
| 7 | 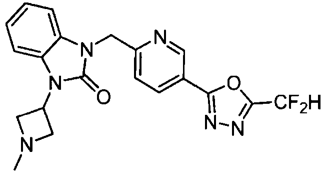 | 8 | 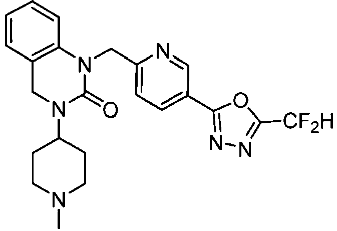 |
| 9 | 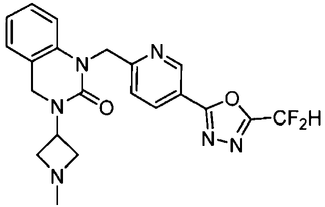 | 10 | 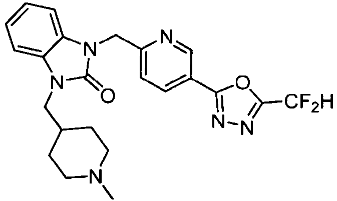 |
| 11 | 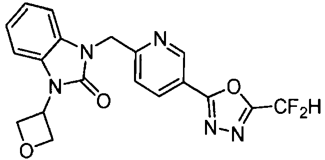 | 12 | 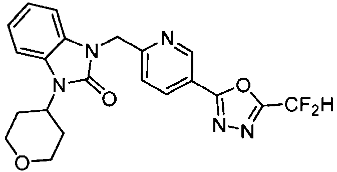 |
| 13 | 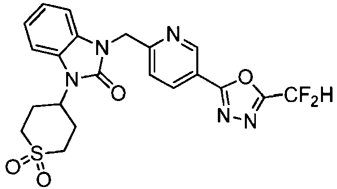 | 14 | 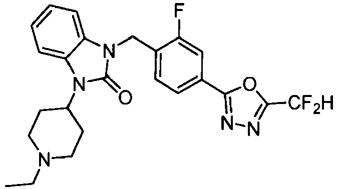 |
| 15 | 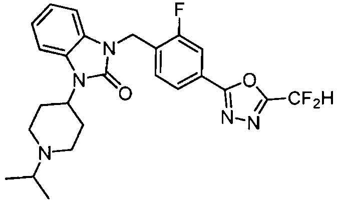 | 16 | 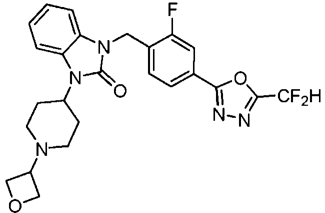 |
| 17 | 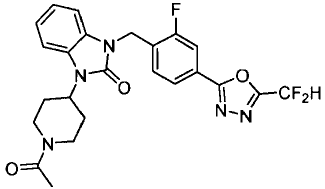 | 18 | 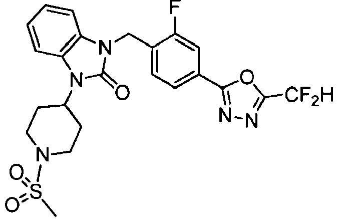 |
| 19 | 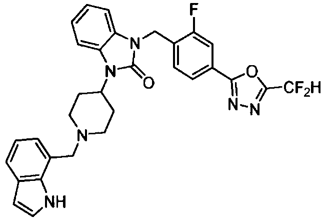 | 20 | 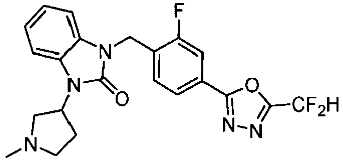 |
| 21 | 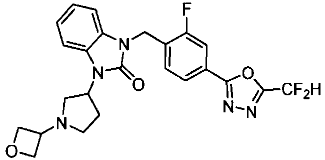 | 22 | 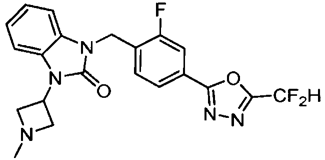 |
| 23 | 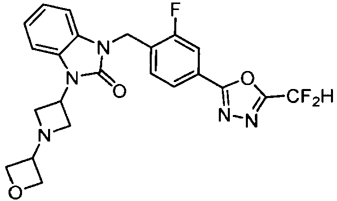 | 24 | 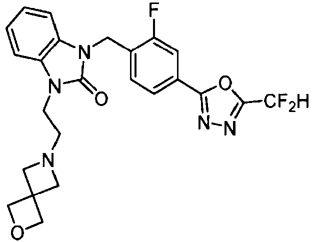 |
| 25 | 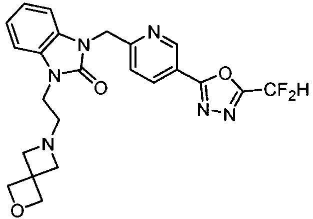 | 26 | 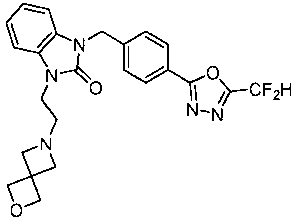 |
| 27 | 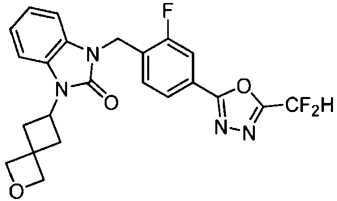 | 28 | 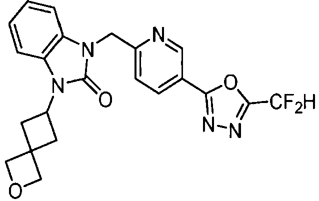 |
| 29 | 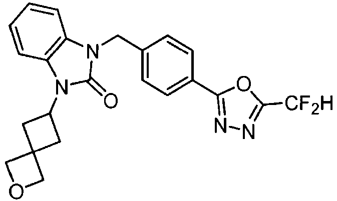 | 30 | 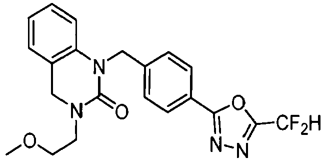 |
| 31 | 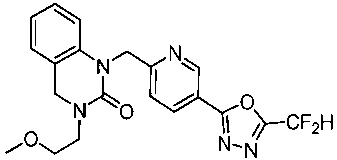 | 32 | 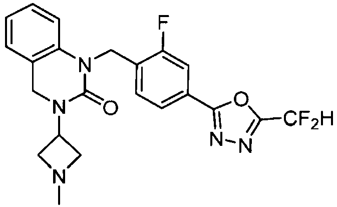 |
| 33 | 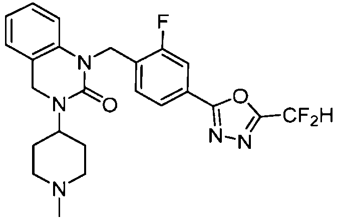 | 34 | 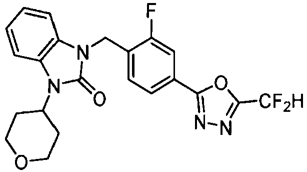 |
| 35 | 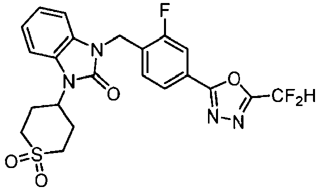 | 36 | 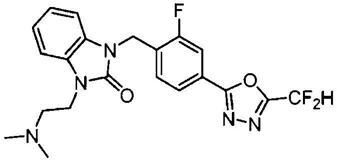 |
| 37 | 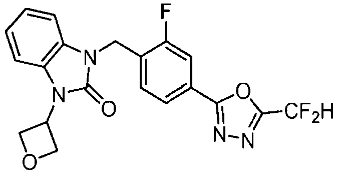 | 38 | 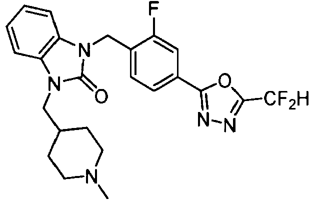 |
| 39 | 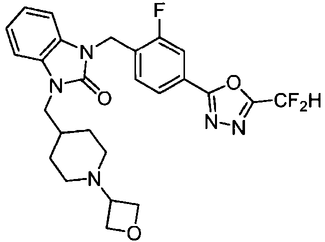 | 40 | 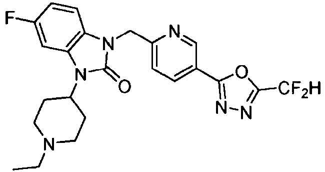 |
| 41 | 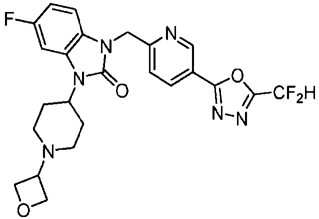 | 42 | 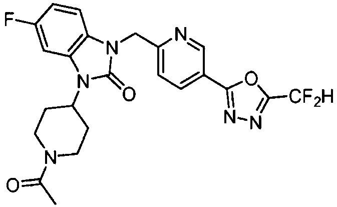 |
| 43 | 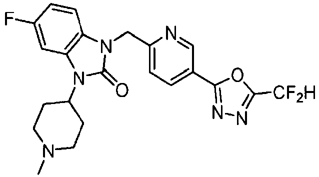 | 44 | 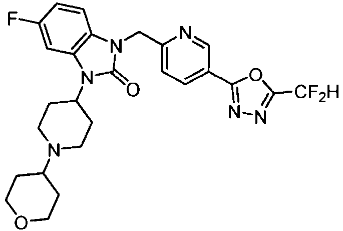 |
| 45 | 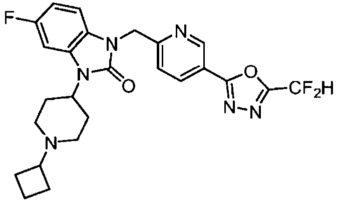 | 46 | 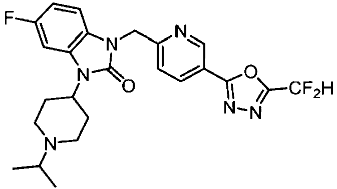 |
| 47 | 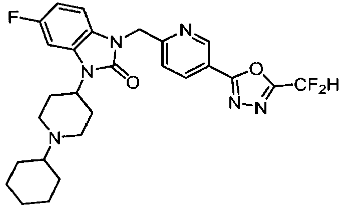 | 48 | 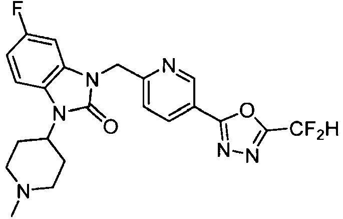 |
| 49 | 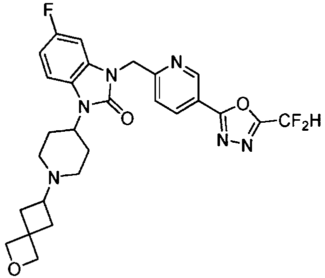 | 50 | 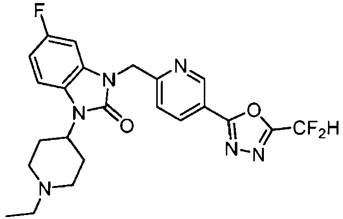 |
| 51 | 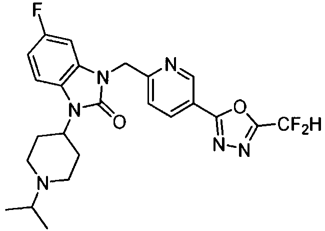 | 52 | 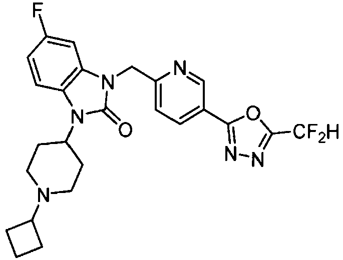 |
| 53 | 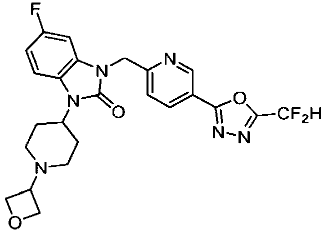 | 54 | 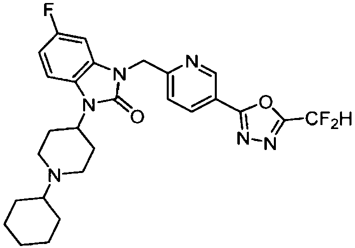 |
| 55 | 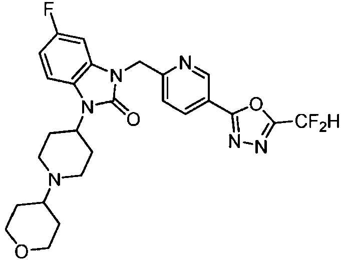 | 56 | 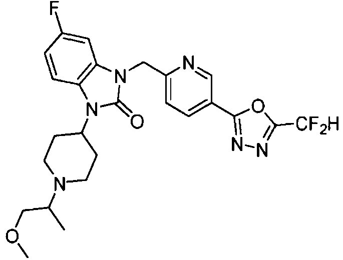 |
| 57 | 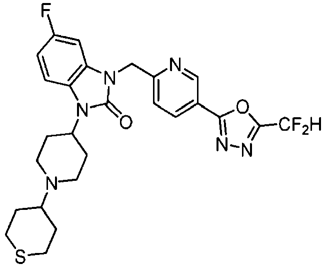 | 58 | 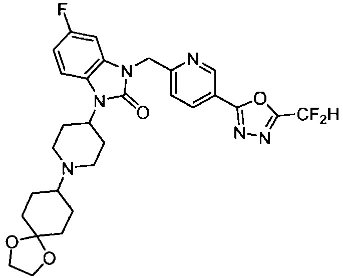 |
| 59 | 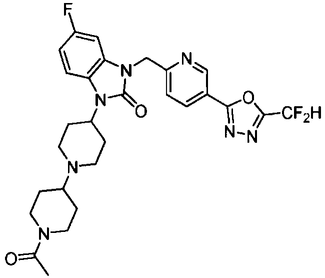 | 60 | 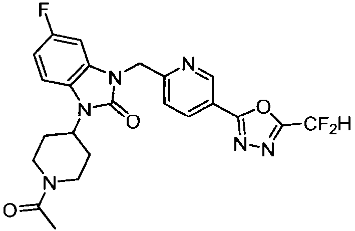 |
| 61 | 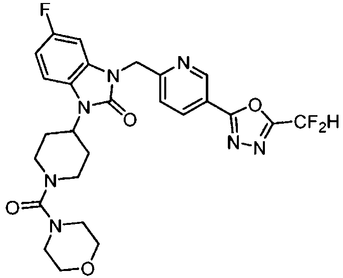 | 62 | 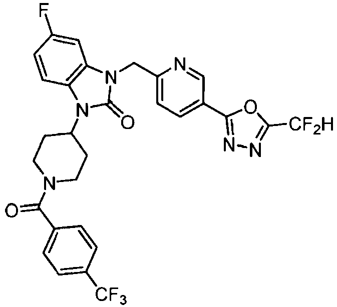 |
| 63 | 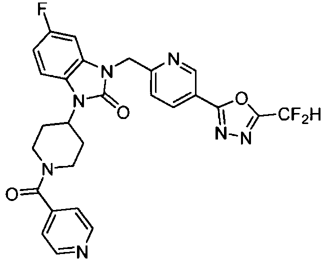 | 64 | 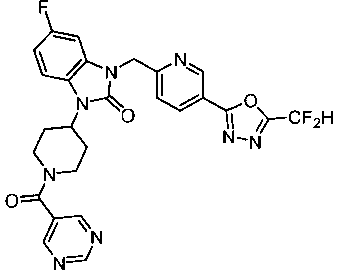 |
| 65 | 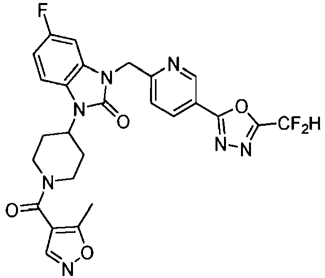 | 66 | 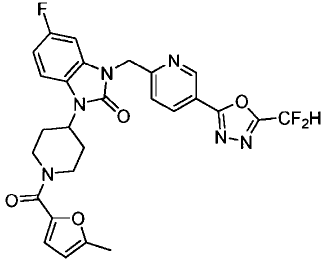 |
| 67 | 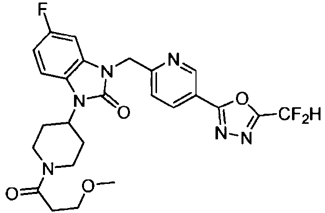 | 68 | 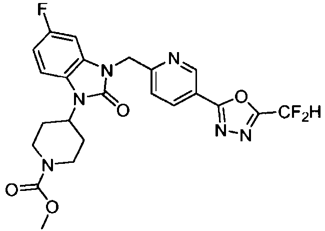 |
| 69 | 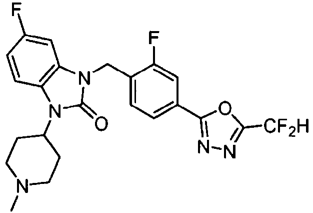 | 70 | 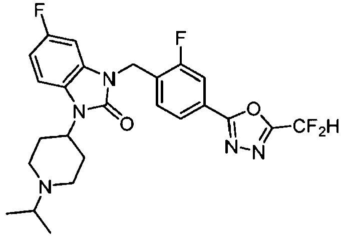 |
| 71 | 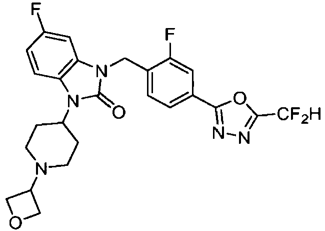 | 72 | 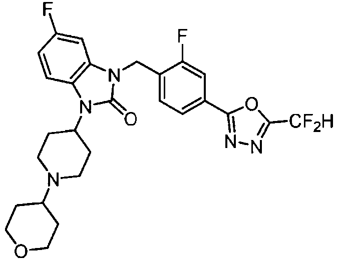 |
| 73 | 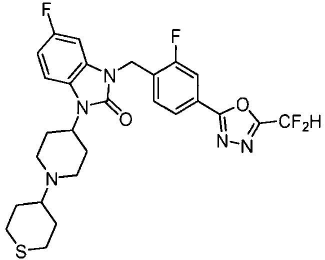 | 74 | 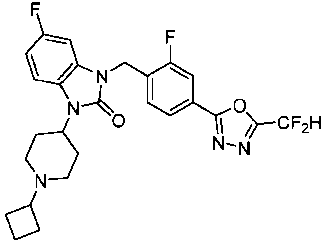 |
| 75 | 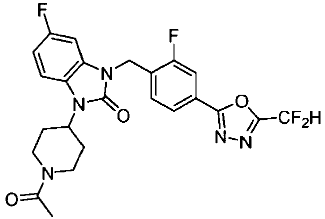 | 76 | 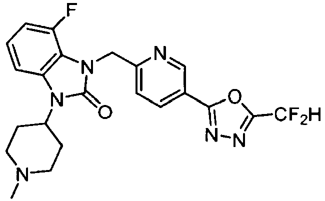 |
| 77 | 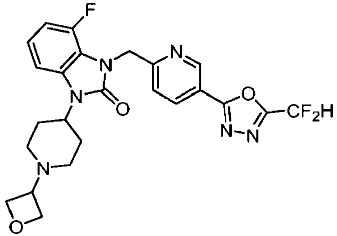 | 78 | 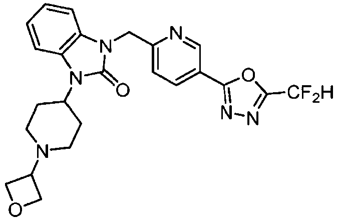 |
| 79 | 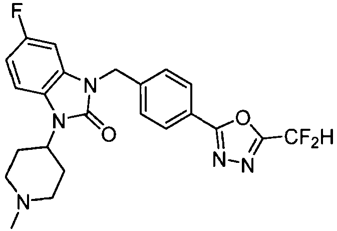 | 80 | 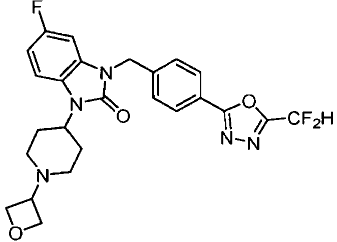 |
| 81 | 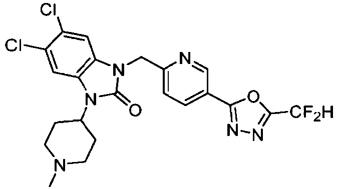 | 82 | 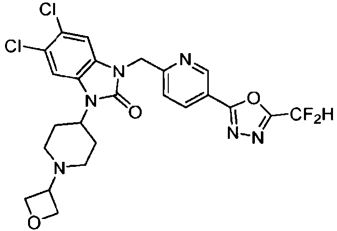 |
| 83 | 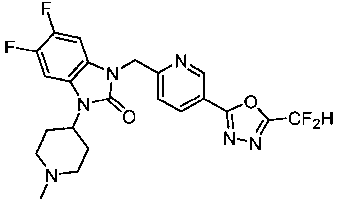 | 84 | 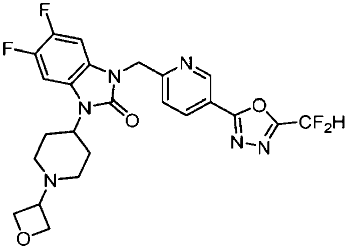 |
| 85 | 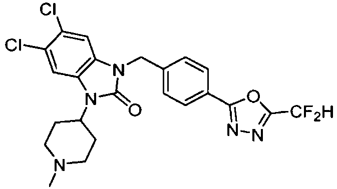 | 86 | 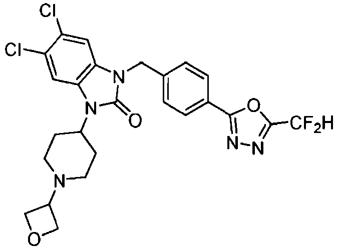 |
| 87 | 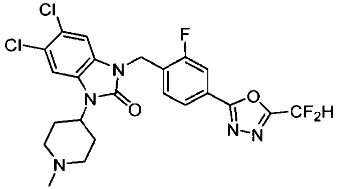 | 88 | 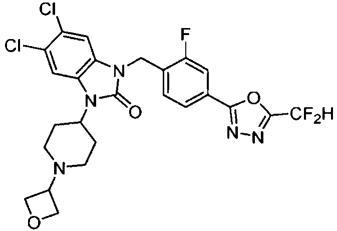 |
| 89 | 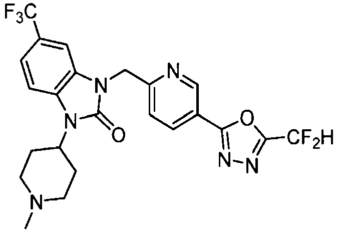 | 90 | 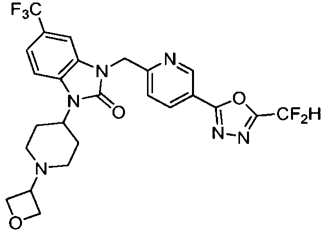 |
| 91 | 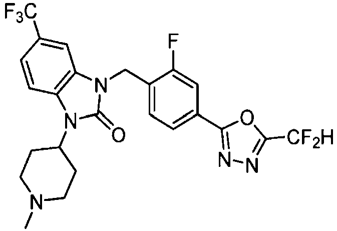 | 92 | 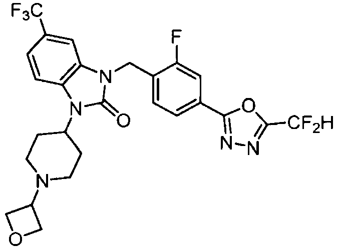 |
| 93 | 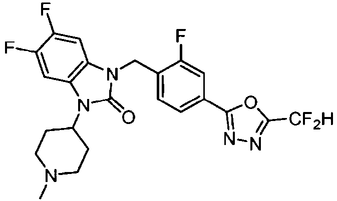 | 94 | 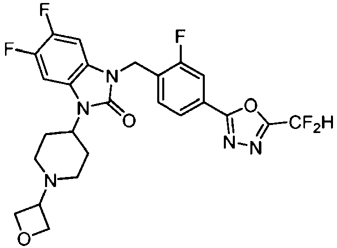 |
| 95 | 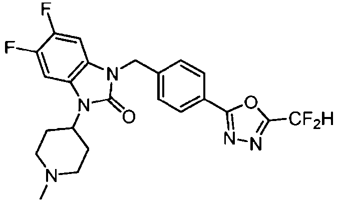 | 96 | 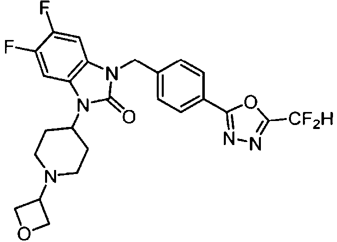 |
| 97 | 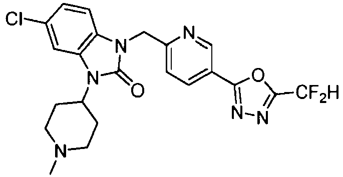 | 98 | 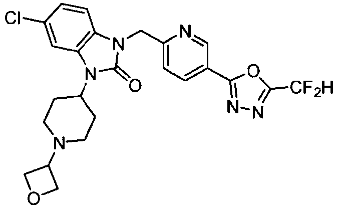 |
| 99 | 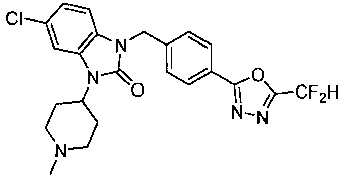 | 100 | 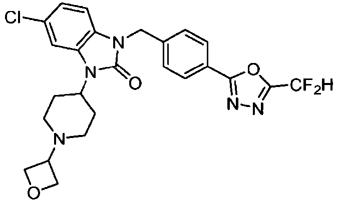 |
| 101 | 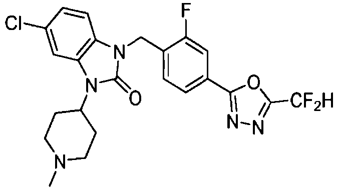 | 102 | 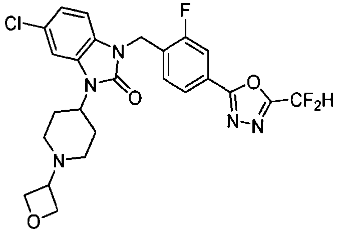 |
| 103 | 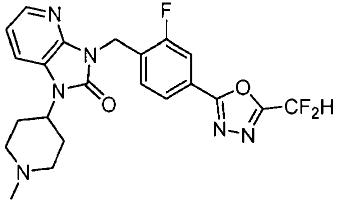 | 104 | 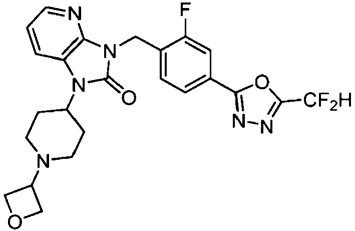 |
| 105 | 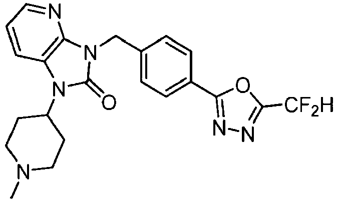 | 106 | 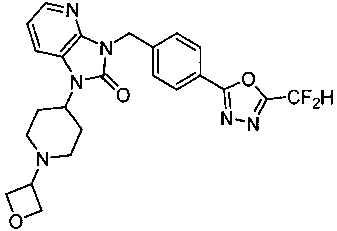 |
| 107 | 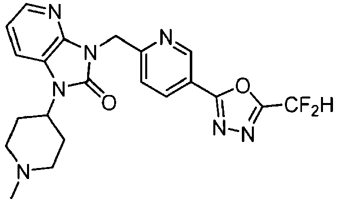 | 108 | 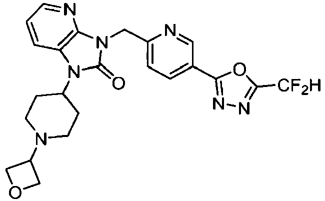 |
| 109 | 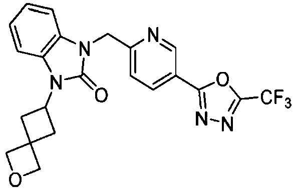 | 110 | 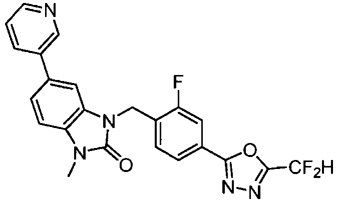 |
| 111 | 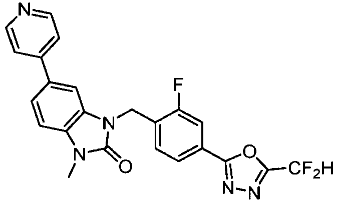 | 112 | 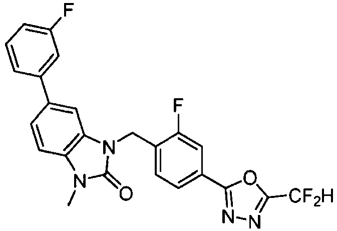 |
| 113 | 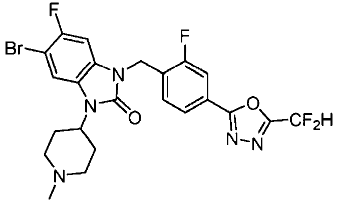 | 114 | 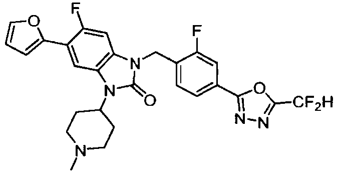 |
| 115 | 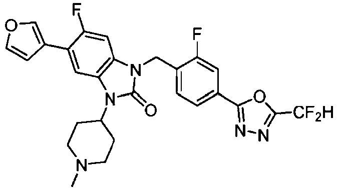 | 116 | 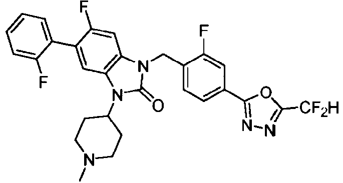 |
| 117 | 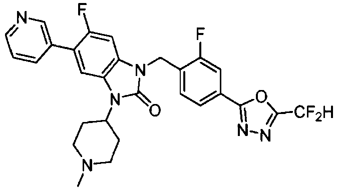 | 118 | 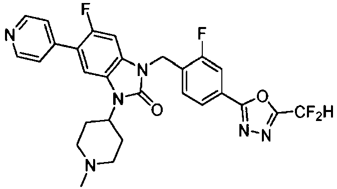 |
| 119 | 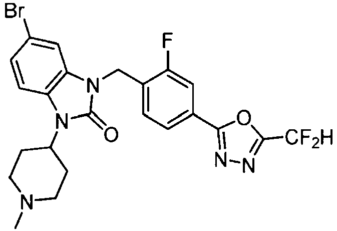 | 120 | 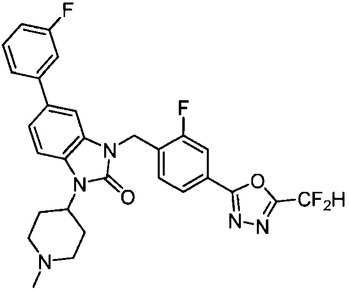 |
| 121 | 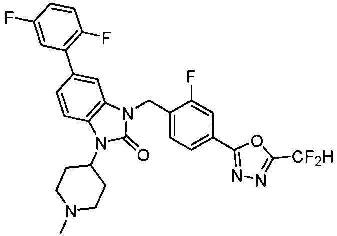 | 122 | 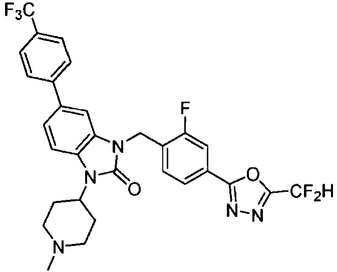 |
| 123 | 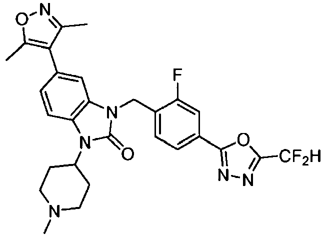 | 124 | 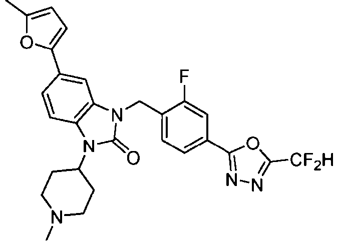 |
| 125 | 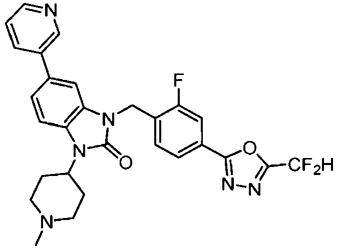 | 126 | 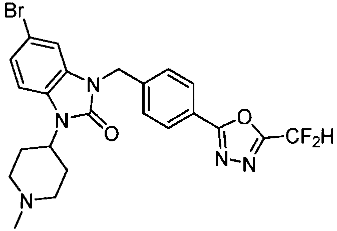 |
| 127 | 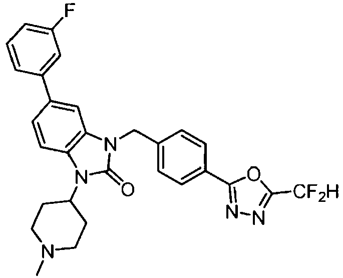 | 128 | 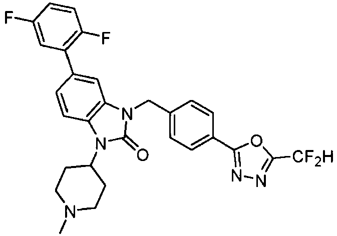 |
| 129 | 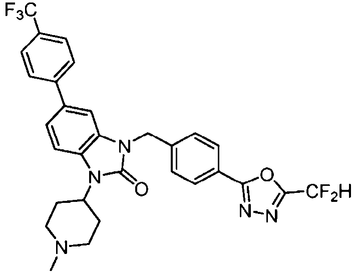 | 130 | 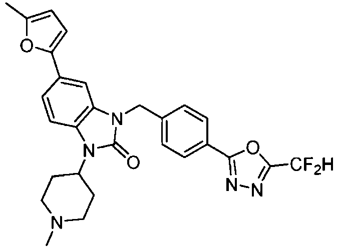 |
| 131 | 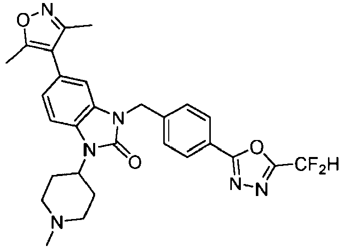 | 132 | 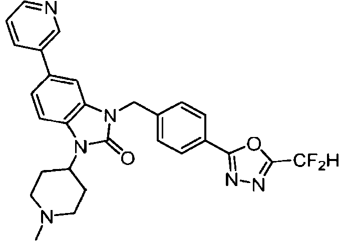 |
| 133 | 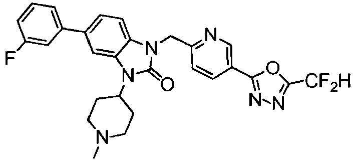 | 134 | 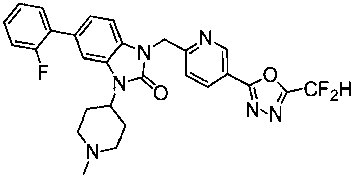 |
| 135 | 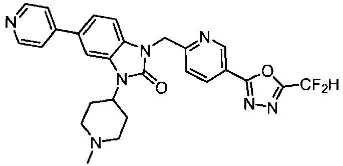 | 136 | 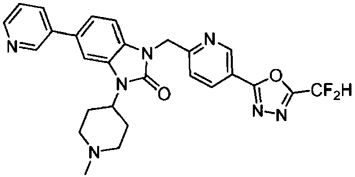 |
| 137 | 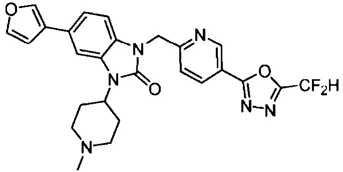 | 138 | 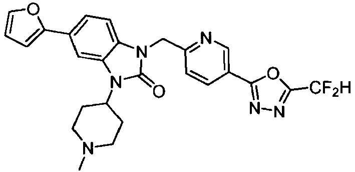 |
| 139 | 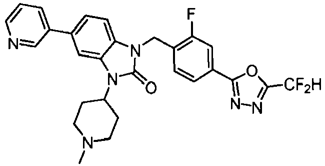 | 140 | 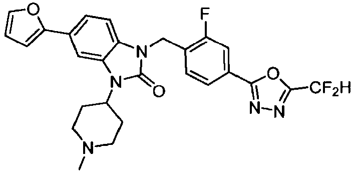 |
| 141 | 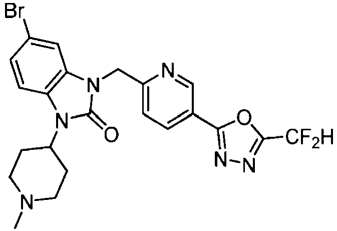 | 142 | 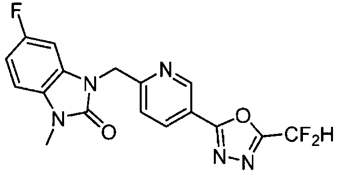 |
| 143 | 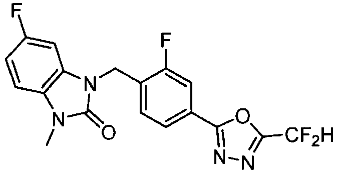 | 144 | 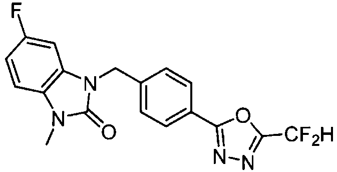 |
| 145 | 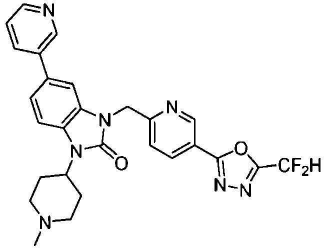 | 146 | 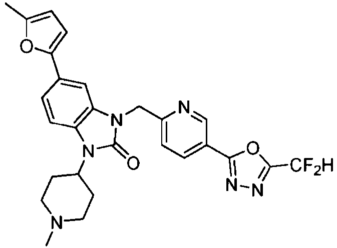 |
| 147 | 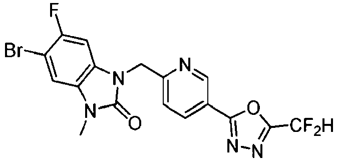 | 148 | 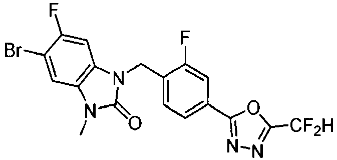 |
| 149 | 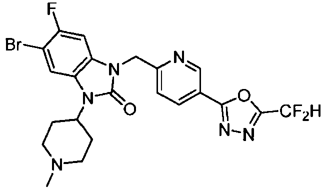 | 150 | 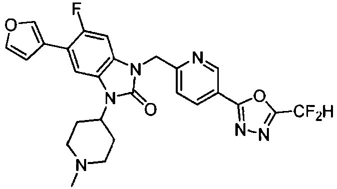 |
| 151 | 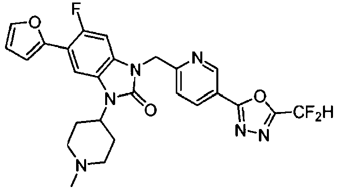 | 152 | 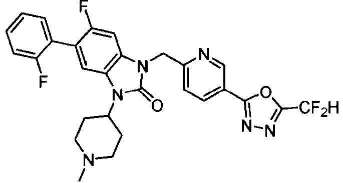 |
| 153 | 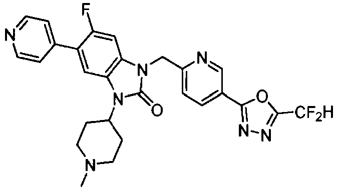 | 154 | 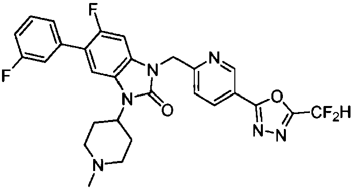 |
| 155 | 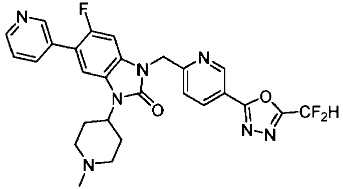 | 156 | 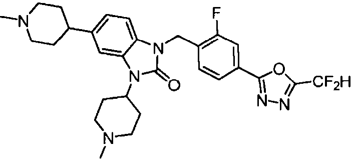 |
| 157 | 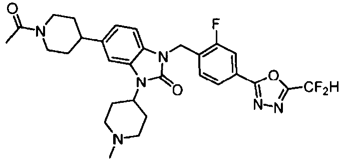 | 158 | 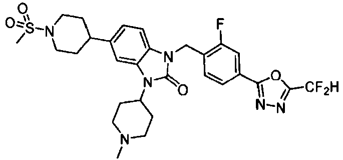 |
| 159 | 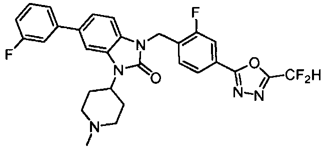 | 160 | 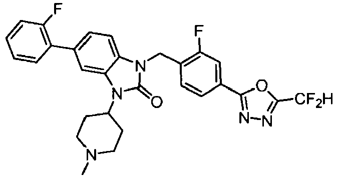 |
| 161 | 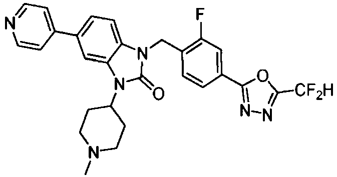 | 162 | 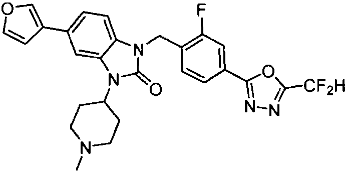 |
| 163 | 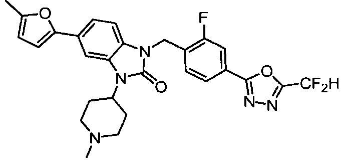 | 164 | 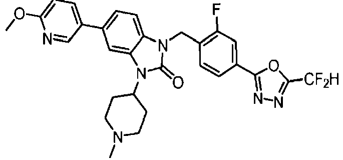 |
| 165 | 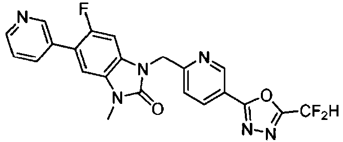 | 166 | 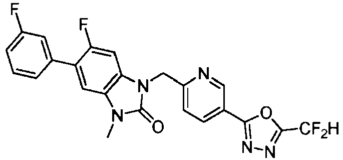 |
| 167 | 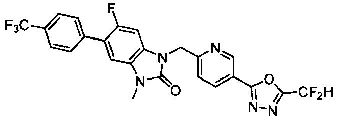 | 168 | 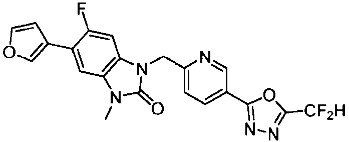 |
| 169 | 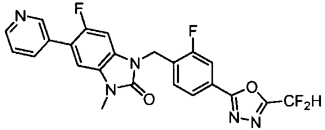 | 170 | 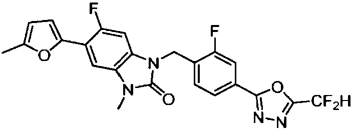 |
| 171 | 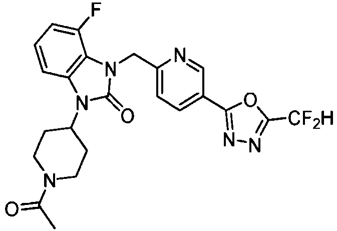 | 172 | 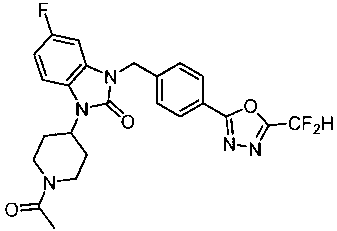 |
| 173 | 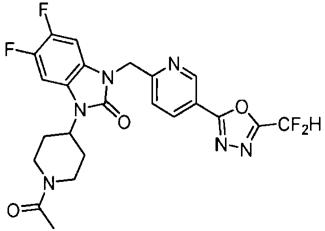 | 174 | 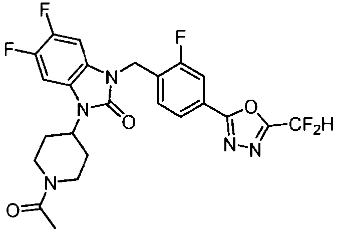 |
| 175 | 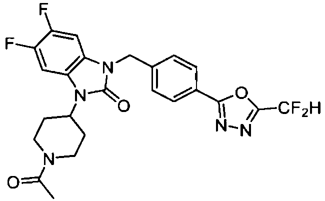 | 176 | 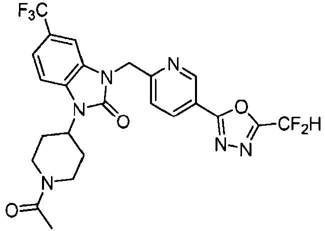 |
| 177 | 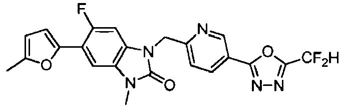 | 178 | 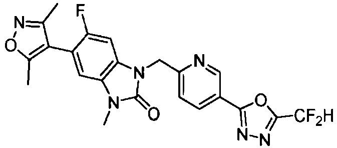 |
| 179 | 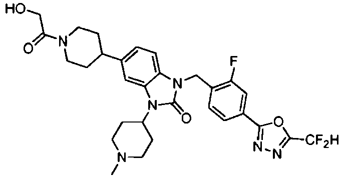 | 180 | 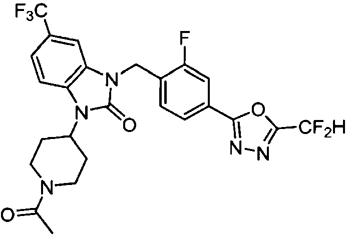 |
| 181 | 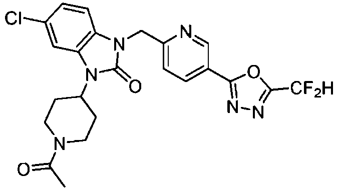 | 182 | 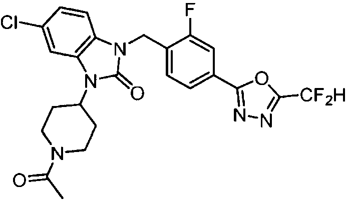 |
| 183 | 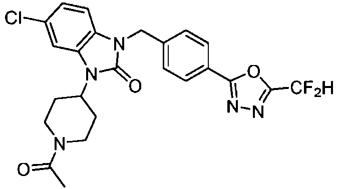 | 184 | 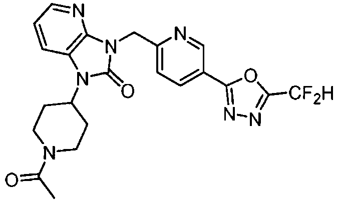 |
| 185 | 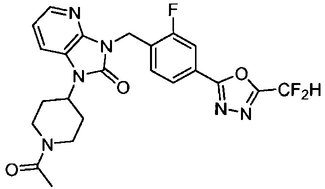 | 186 | 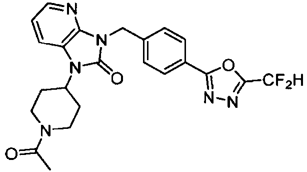 |
| 187 | 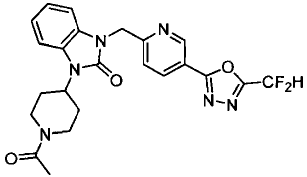 | 188 | 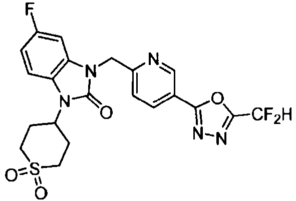 |
| 189 | 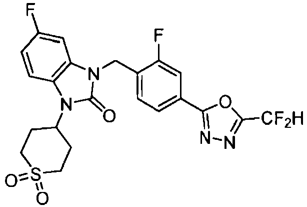 | 190 | 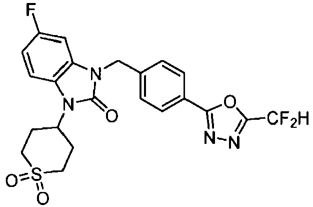 |
| 191 | 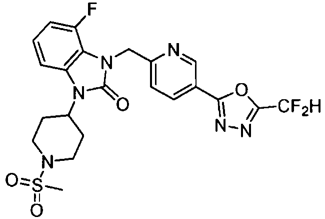 | 192 | 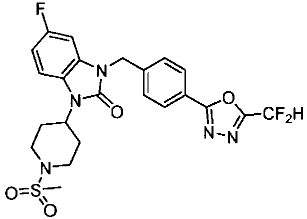 |
| 193 | 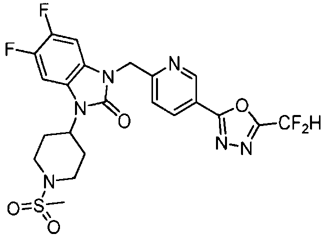 | 194 | 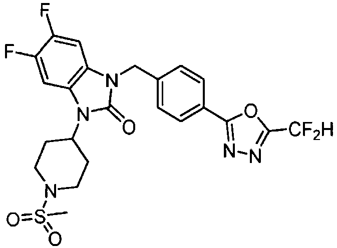 |
| 195 | 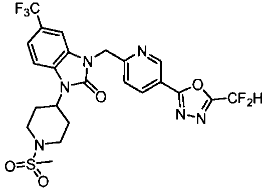 | 196 | 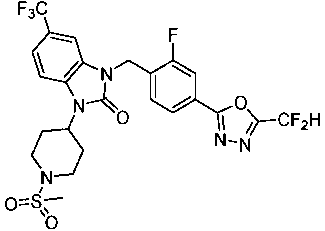 |
| 197 | 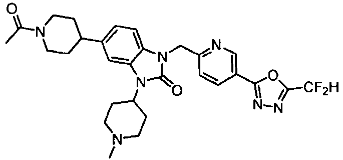 | 198 | 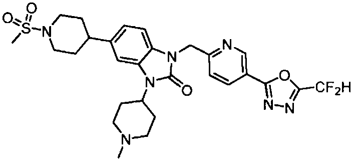 |
| 199 | 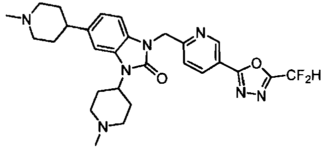 | 200 | 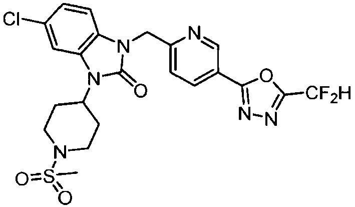 |
| 201 | 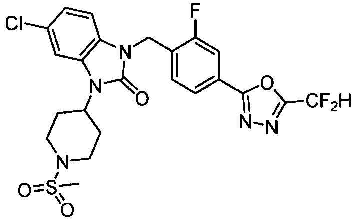 | 202 | 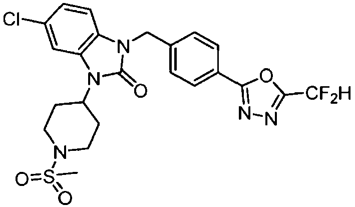 |
| 203 | 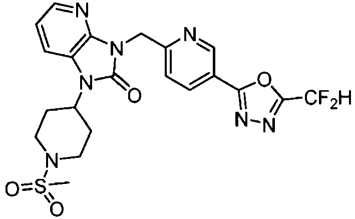 | 204 | 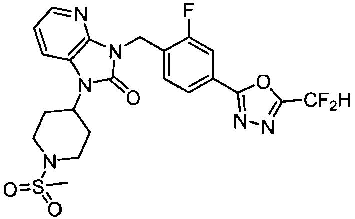 |
| 205 | 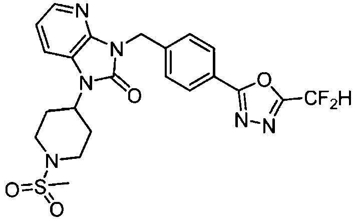 | 206 | 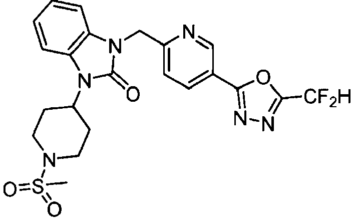 |
| 207 | 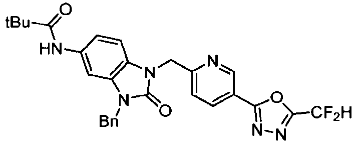 | 208 | 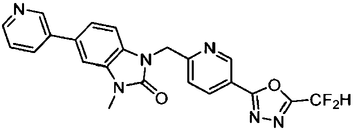 |
| 209 | 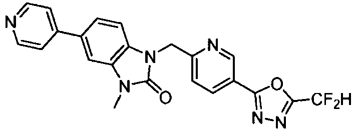 | 210 | 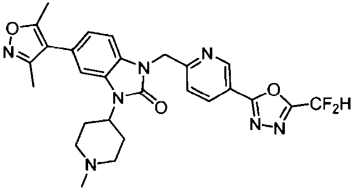 |
| 211 | 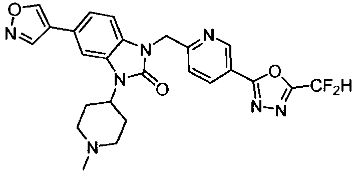 | 212 | 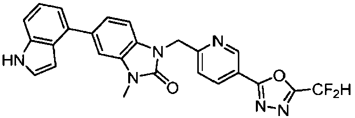 |
| 213 | 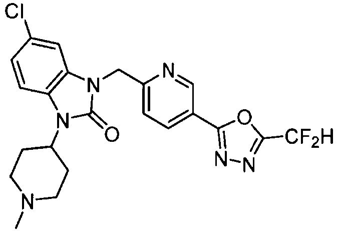 | 214 | 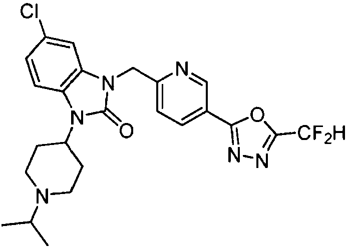 |
| 215 | 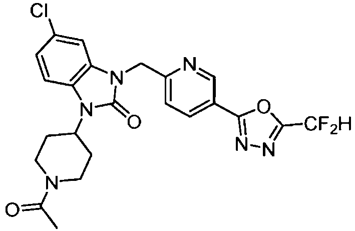 | 216 | 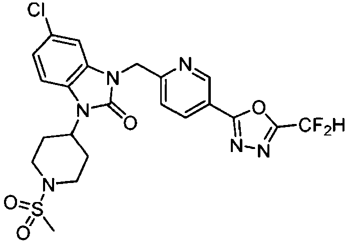 |
| 217 | 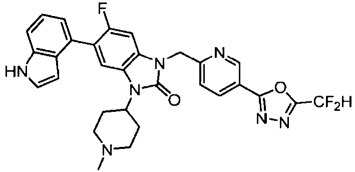 | 218 | 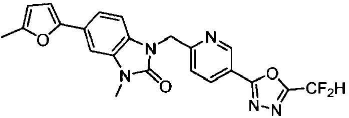 |
| 219 | 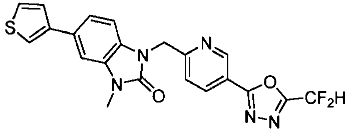 | 220 | 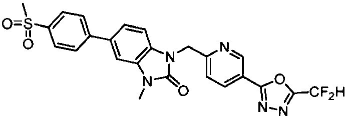 |
| 221 | 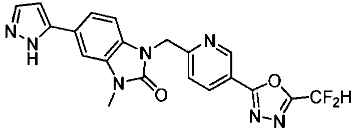 | 222 | 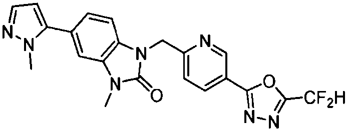 |
| 223 | 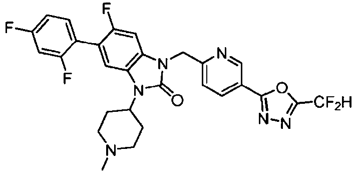 | 224 | 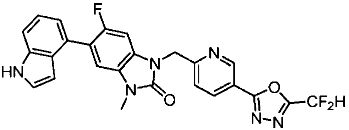 |
| 225 | 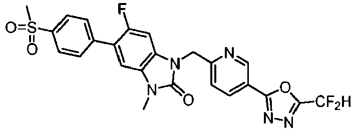 | 226 | 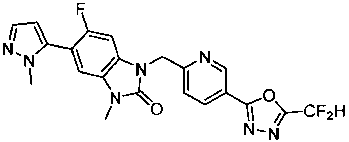 |
| 227 | 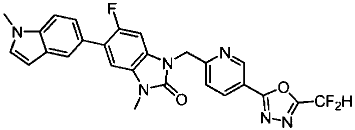 | 228 | 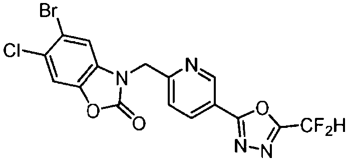 |
| 229 | 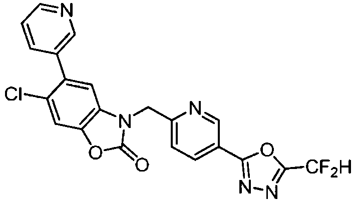 | 230 | 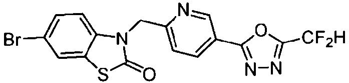 |
| 231 | 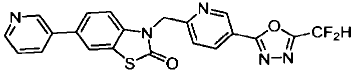 | 232 | 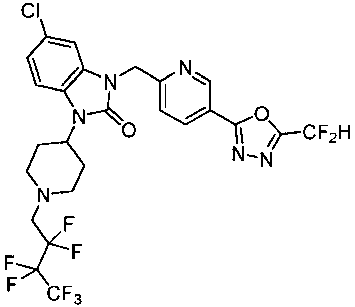 |
| 233 | 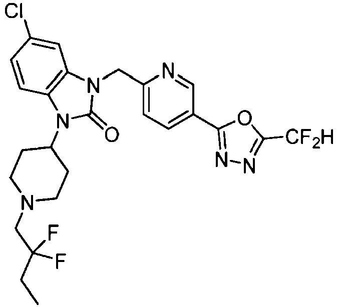 | 234 | 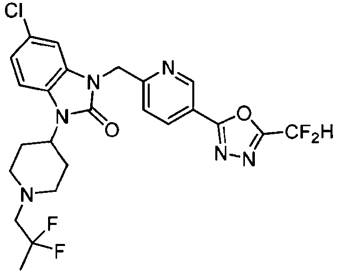 |
| 235 | 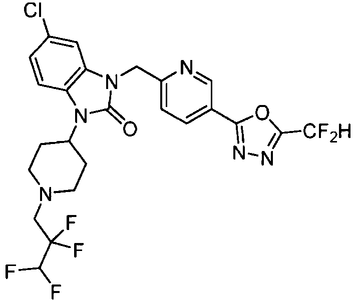 | 236 | 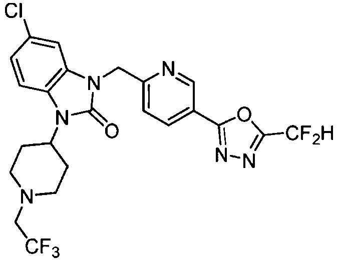 |
| 237 | 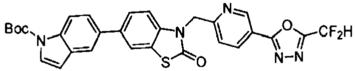 | 238 | 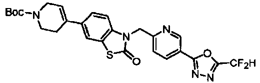 |
| 239 | 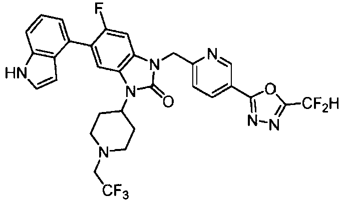 | 240 | 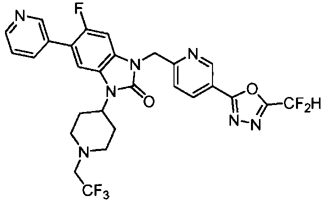 |
| 241 | 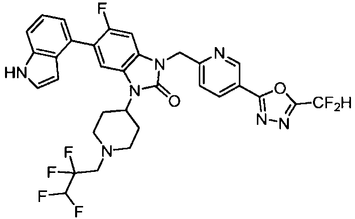 | 242 | 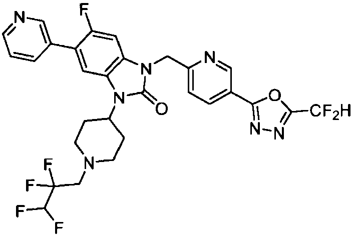 |
| 243 | 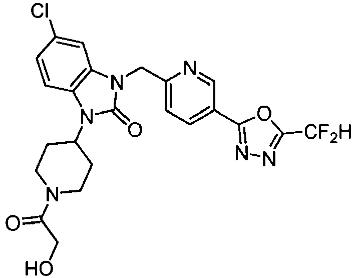 | 244 | 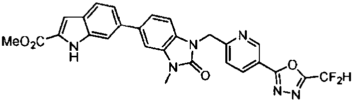 |
| 245 | 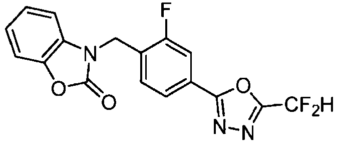 | 246 | 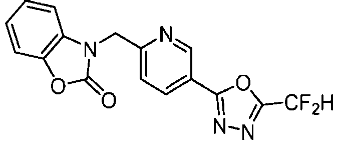 |
| 247 | 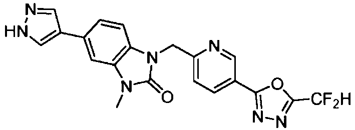 | 248 | 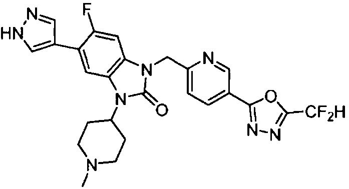 |
| 249 | 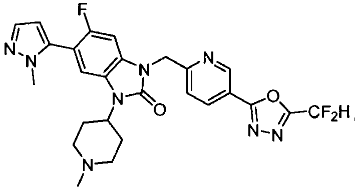 | 250 | 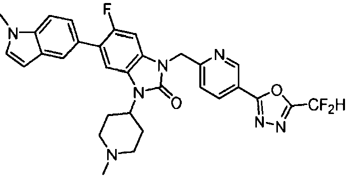 |
| 251 | 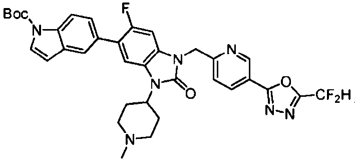 | 252 | 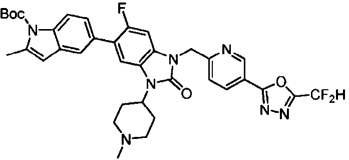 |
| 253 | 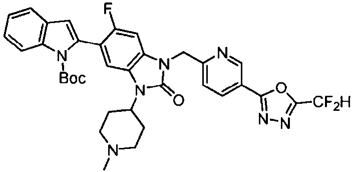 | 254 | 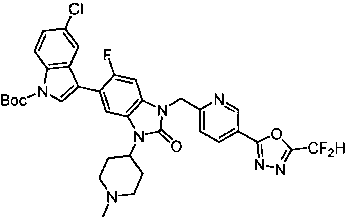 |
| 255 | 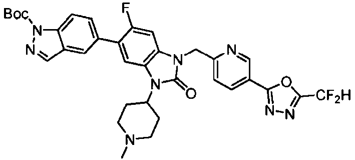 | 256 | 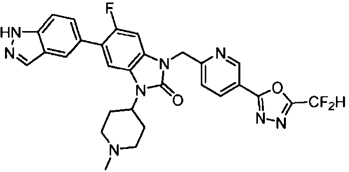 |
| 257 | 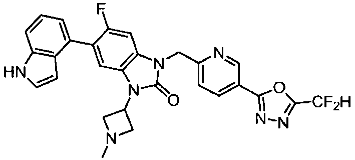 | 258 | 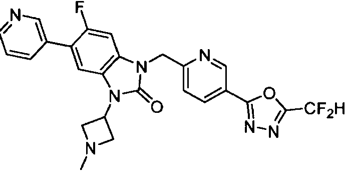 |
| 259 | 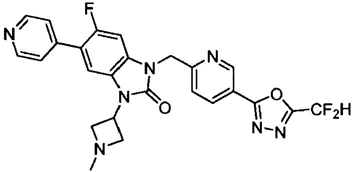 | 260 | 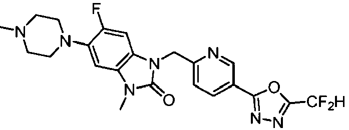 |
| 261 | 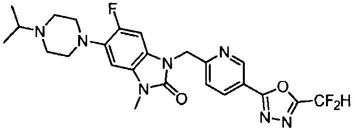 | 262 | 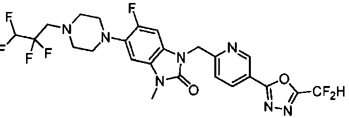 |
| 263 | 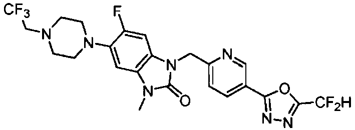 | 264 | 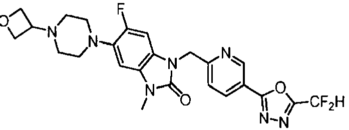 |
| 265 | 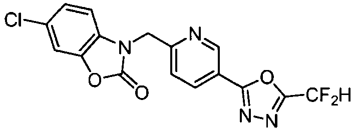 | 266 | 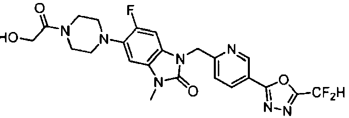 |
| 267 | 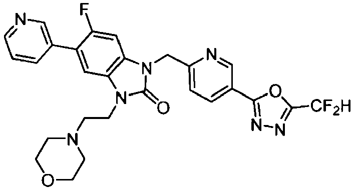 | 268 | 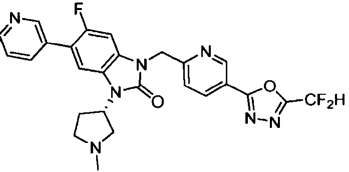 |
| 269 | 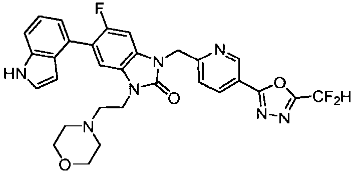 | 270 | 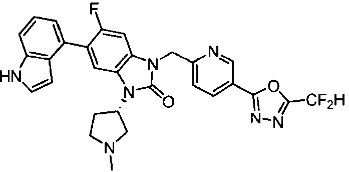 |
| 271 | 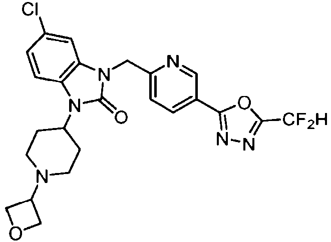 | 272 | 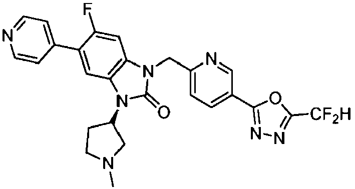 |
| 273 | 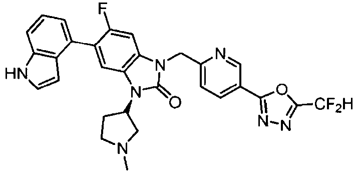 | 274 | 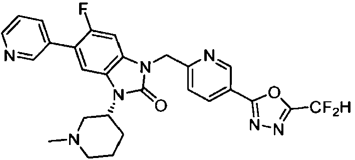 |
| 275 | 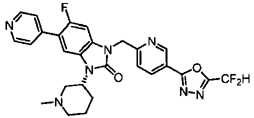 | 276 | 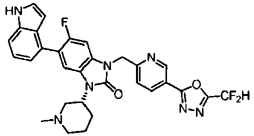 |
| 277 | 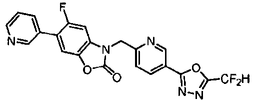 | 278 | 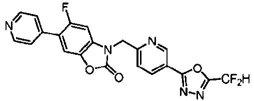 |
| 279 | 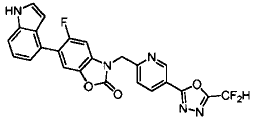 | 280 | 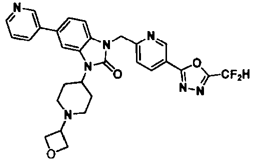 |
| 281 | 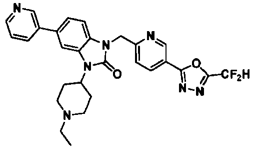 | 282 | 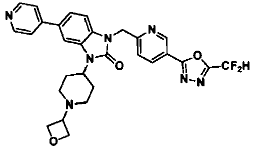 |
| 283 | 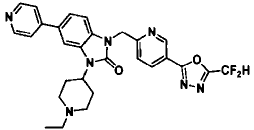 | 284 | 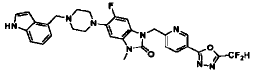 |
| 285 | 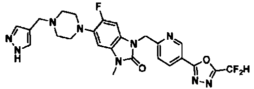 | 286 | 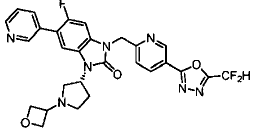 |
| 287 | 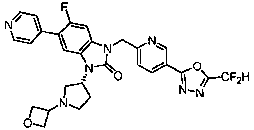 | 288 | 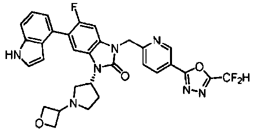 |
| 289 | 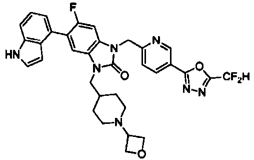 | 290 | 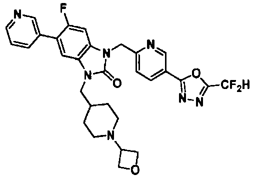 |
| 291 | 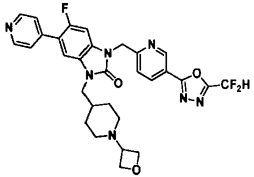 | 292 | 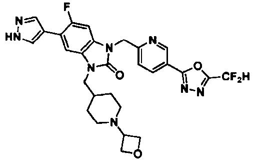 |
| 293 | 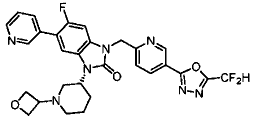 | 294 | 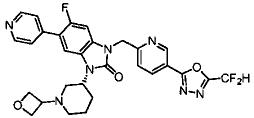 |
| 295 | 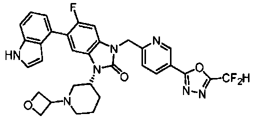 | 296 | 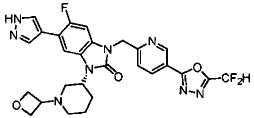 |
| 297 | 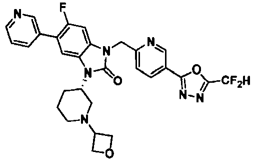 | 298 | 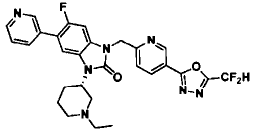 |
| 299 | 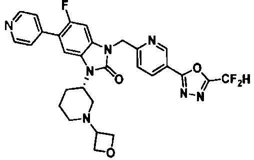 | 300 | 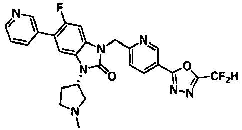 |
| 301 | 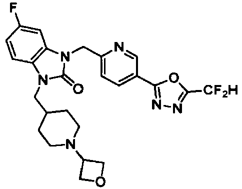 | 302 | 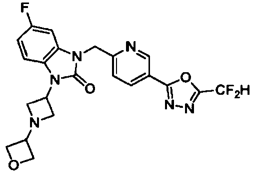 |
| 303 | 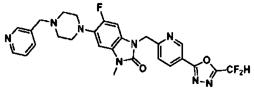 | 304 | 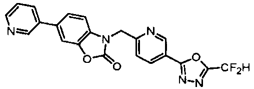 |
| 305 | 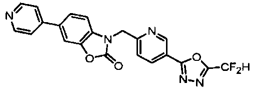 | 306 | 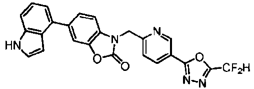 |
| 307 | 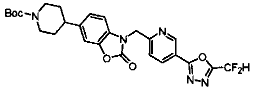 | 308 | 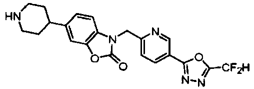 |
| 309 | 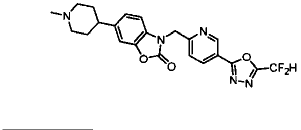 | 310 | 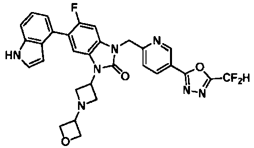 |
| 311 | 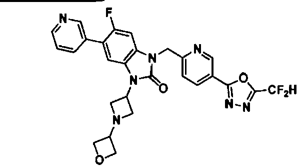 | 312 | 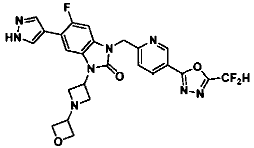 |
| 313 | 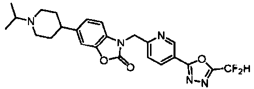 | 314 | 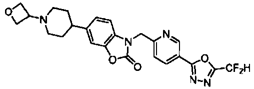 |
| 315 | 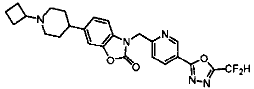 | 316 | 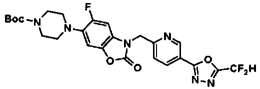 |
| 317 | 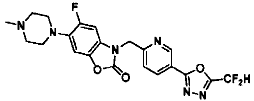 | 318 | 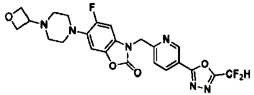 |
| 319 | 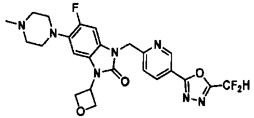 | 320 | 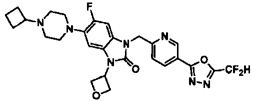 |
| 321 | 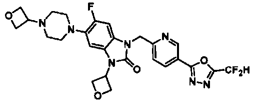 | 322 | 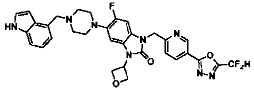 |
| 323 | 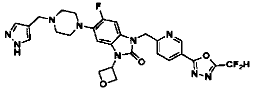 | 324 | 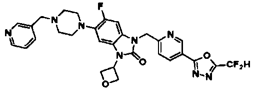 |
| 325 | 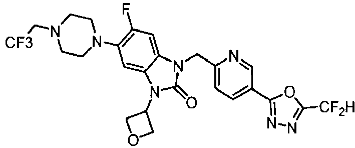 | 326 | 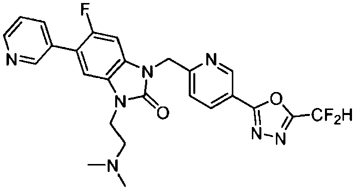 |
| 327 | 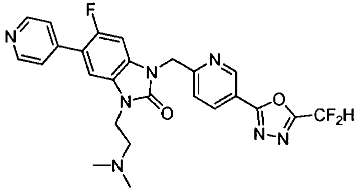 | 328 | 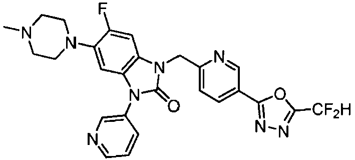 |
| 329 | 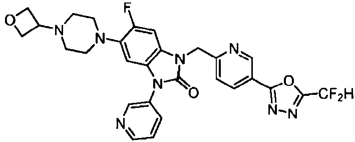 | 330 | 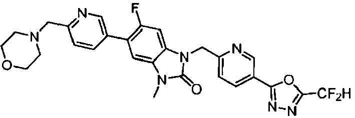 |
| 331 | 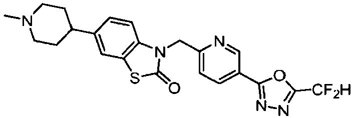 | 332 | 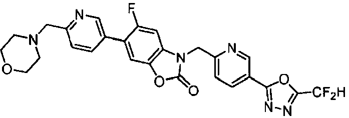 |
| 333 | 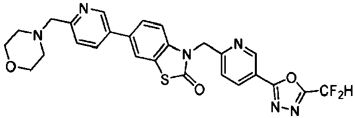 | 334 | 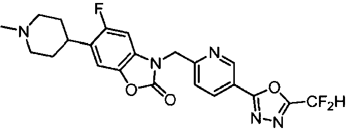 |
| 335 | 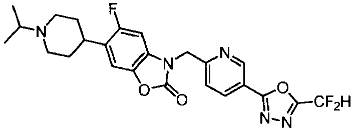 | 336 | 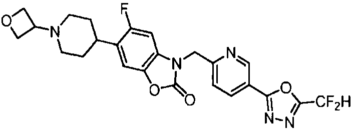 |
| 337 | 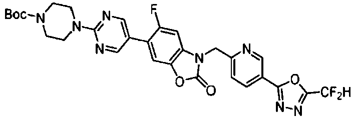 | 338 | 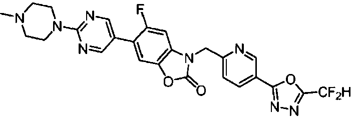 |
| 339 | 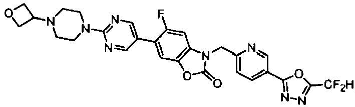 | 340 | 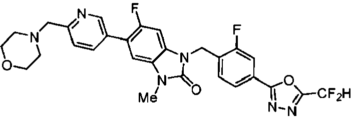 |
| 341 | 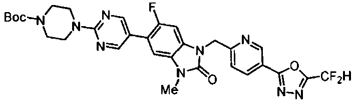 | 342 | 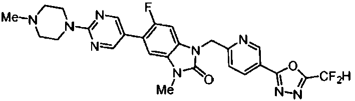 |
| 343 | 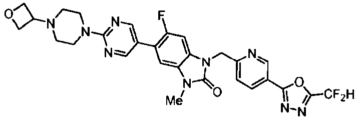 | 344 | 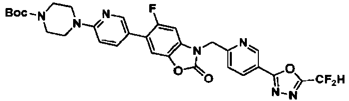 |
| 345 | 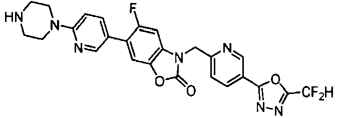 | 346 | 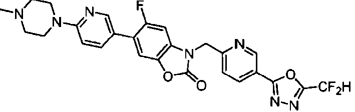 |
| 347 | 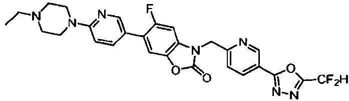 | 348 | 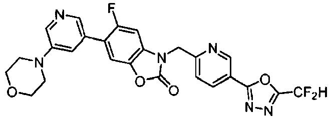 |
| 349 | 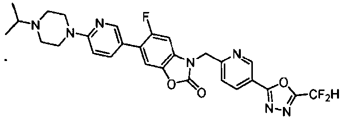 | 350 | 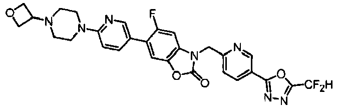 |
| 351 | 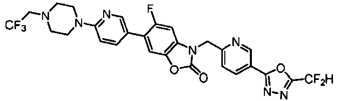 | 352 | 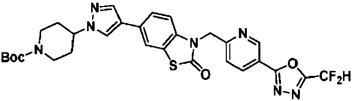 |
| 353 | 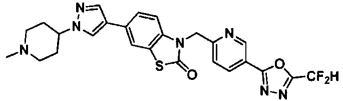 | 354 | 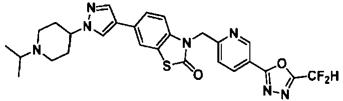 |
| 355 | 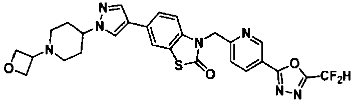 | 356 | 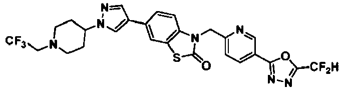 |
| 357 | 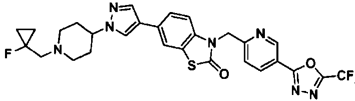 | 358 | 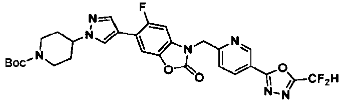 |
| 359 | 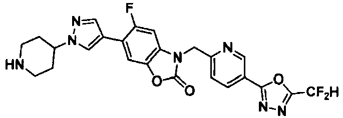 | 360 | 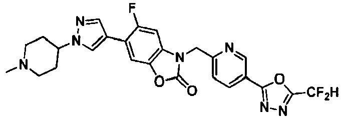 |
| 361 | 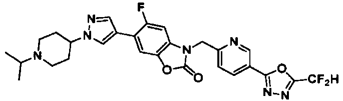 | 362 | 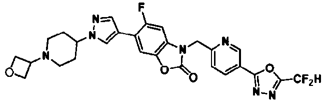 |
| 363 | 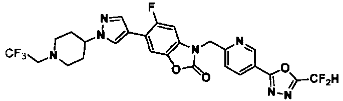 | 364 | 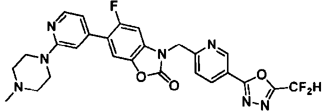 |
| 365 | 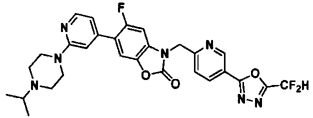 | 366 | 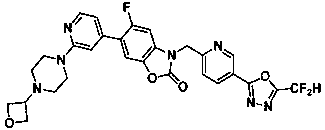 |
| 367 | 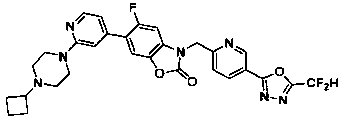 | 368 | 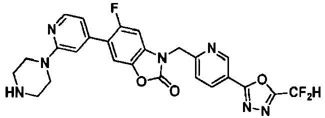 |

9. Pharmazeutische Zusammensetzung, die Verbindungen, die durch die chemische Formel I dargestellt sind, nach einem der Ansprüche 1 bis 8, Stereoisomere davon oder pharmazeutisch annehmbare Salze davon als Wirkbestandteil umfasst.

10. Pharmazeutische Zusammensetzung nach Anspruch 9, wobei die pharmazeutische Zusammensetzung zur Verwendung bei der Prävention oder Behandlung von mit Histon-deacetylase-6-Aktivität zusammenhängenden Erkrankungen nützlich ist.

11. Pharmazeutische Zusammensetzung nach Anspruch 10, wobei die mit Histon-deacetylase-6-Aktivität zusammenhängenden Erkrankungen zumindest eine, ausgewählt aus der aus Infektionskrankheiten, Neoplasie, Endokrinopathie, ernährungsbedingten oder Stoffwechselerkrankungen, psychischen und Verhaltensstörungen, neurologischen Erkrankungen, Augenerkrankungen und Erkrankungen oder okularen Adnexe, Kreislauferkrankungen, Atemwegserkrankungen, Verdauungserkrankungen, Hauterkrankungen und Erkrankungen des subkutanen Gewebes, Erkrankungen des muskuloskelettalen Systems und des Bindegewebes und Fehlbildungen, Missbildungen und Chromosomenaberrationen bestehenden Gruppe sind.

12. Verbindungen, die durch die chemische Formel I dargestellt sind, nach einem der Ansprüche 1 bis 8, Stereoisomere davon oder pharmazeutisch annehmbare Salze davon zur Verwendung bei der Prävention oder Behandlung von mit Histondeacetylase-6-Aktivität zusammenhängenden Erkrankungen.

## Revendications

1. Composés représentés par la formule chimique I qui suit, leurs stéréoisomères ou leurs sels pharmaceutiquement acceptables : 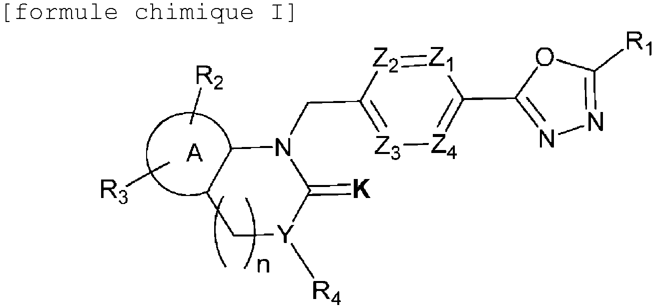 dans lesquels
chacun de Z₁ à Z₄ est indépendamment N ou CRₐ où Rₐ est H, X, un alkyle en C₁ à C₄ ou un O-(alkyle en C₁ à C₄), et les Rₐ peuvent être mutuellement différents quand CRₐ est au nombre de 2 ou plus ;
K est O ou S ;
R₁ est CX₃ ou CX₂H ; 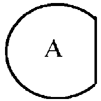 est un arylène en C₄ à C₁₂ ou un hétéroarylène en C₂ à C₁₀ ;
chacun de R₂ et R₃ est indépendamment H, X, un alkyle en C₁ à C₄, un halogénoalkyle en C₁ à C₄, un aryle en C₆ à C₁₂, un hétéroaryle en C₂ à C₁₀, un cycloalkyle en C₃ à C₁₀, un hétérocycloalkyle en C₂ à C₁₀, un hétérocycloalcényle en C₂ à C₁₀, N(R_{b})(R_{c}), un NH-(alkyle en C₁ à C₄)-N(R_{b})(R_{c}), un NH-O-(alkyle en C₁ à C₄) ou NHC(=O)-R₅,
où au moins un H de l'alkyle en C₁ à C₄, de l'aryle en C₆ à C₁₂, de l'hétéroaryle en C₂ à C₁₀, de l'hétérocycloalcényle en C₂ à C₁₀ ou de l'hétérocycloalkyle en C₂ à C₁₀ peut être indépendamment remplacé par X, un alkyle en C₁ à C₄, un halogénoalkyle en C₁ à C₄, un cycloalkyle en C₃ à C₁₀, un aryle en C₆ à C₁₂, un hétéroaryle en C₂ à C₁₀, un hétérocycloalkyle en C₂ à C₁₀, un (alkyle en C₁ à C₄)-(hétéroaryle en C₂ à C₁₀), un (alkyle en C₁ à C₄)-(hétérocycloalkyle en C₂ à C₁₀), un (hétérocycloalkyle en C₂ à C₁₀)-(alkyle en C₁ à C₄), un (hétérocycloalkyle en C₂ à C₁₀)-(halogénoalkyle en C₁ à C₄), un (hétérocycloalkyle en C₂ à C₁₀)-(cycloalkyle en C₃ à C₁₀), un (hétérocycloalkyle en C₂ à C₁₀)-(hétérocycloalkyle en C₂ à C₁₀), un (hétérocycloalkyle en C₂ à C₁₀)-(alkyle en C₁ à C₄)- (halogénocycloalkyle en C₃ à C₁₀), un (alkyle en C₁ à C₄)-(cycloalkyle en C₃ à C₁₀), un S(O)₂-(alkyle en C₁ à C₄), C(=O)-N(R_{b})(R_{c}), C(=O)-R₆, -N(R_{b})(R_{c}), un (alkyle en C₁ à C₄)-N(R_{b})(R_{c}), un O- (alkyle en C₁ à C₄), un (alkyle en C₁ à C₄)-O-(alkyle en C₁ à C₄), un C(=O)O-(hétérocycloalkyle en C₂ à C₁₀), un (alkyle en C₁ à C₄)-C(=O)-R₇ ou un (hétérocycloalkyle en C₂ à C₁₀)-C(=O)-R₈ ;
Y est CH, N, O ou S {où R₄ n'existe pas quand Y est O ou S} ;
R₄ est H, un alkyle en C₁ à C₄, un cycloalkyle en C₃ à C₇, un hétérocycloalkyle en C₂ à C₁₀, un (alkyle en C₁ à C₄)-(hétérocycloalkyle en C₂ à C₁₀), un aryle en C₆ à C₁₂, un hétéroaryle en C₂ à C₁₀ ou C(=O)-R₉,
où au moins un H de l'alkyle en C₁ à C₄, de l'hétérocycloalkyle en C₂ à C₁₀, ou du (alkyle en C₁ à C₄)-(hétérocycloalkyle en C₂ à C₁₀) peut être indépendamment remplacé par X, un alkyle en C₁ à C₄, un halogénoalkyle en C₁ à C₄, un O- (alkyle en C₁ à C₄), -N(R_{b})(C_{c}), un cycloalkyle en C₃ à C₁₀, un hétérocycloalkyle en C₂ à C₁₀, un (hétérocycloalkyle en C₂ à C₁₀)-C(=O)-R₁₀, un S(O)₂-(alkyle en C₁ à C₄), C(=O)-R₁₁, un (alkyle en C₁ à C₄)-O-(alkyle en C₁ à C₄), un aryle en C₆ à C₁₂, un hétéroaryle en C₂ à C₁₀, un (alkyle en C₁ à C₄)-(hétéroaryle en C₂ à C₁₀), un (hétérocycloalkyle en C₂ à C₁₀)-(hétérocycloalkyle en C₂ à C₁₀) ou un C(=O)-(alkyle en C₁ à C₄)-O-(alkyle en C₁ à C₄) ;
chacun de R₅, R₆, R₇, R₈, R₉, R₁₀ et R₁₁ est indépendamment H, un alkyle en C₁ à C₄, un (alkyle en C₁ à C₄)-OH, un (alkyle en C₁ à C₄)-O-(alkyle en C₁ à C₄), un hétérocycloalkyle en C₂ à C₁₀, un aryle en C₆ à C₁₂, un hétéroaryle en C₂ à C₁₀, un O-(alkyle en C₁ à C₄), un cycloalkyle en C₃ à C₇ ou un (alkyle en C₁ à C₄)-N(R_{b})(R_{c}),
où au moins un H de l'aryle en C₆ à C₁₂ ou de l'hétéroaryle en C₂ à C₁₀ peut être indépendamment remplacé par un alkyle en C₁ à C₄, X, ou un halogénoalkyle en C₁ à C₄ ;
chacun de R_{b} et R_{c} est indépendamment H, un alkyle en C₁ à C₄, un aryle en C₆ à C₁₂, un hétéroaryle en C₂ à C₁₀, un cycloalkyle en C₃ à C₁₀, un hétérocycloalkyle en C₂ à C₁₀, un (alkyle en C₁ à C₄)-NH-(alkyle en C₁ à C₄), un (alkyle en C₁ à C₄)-N-(alkyle en C₁ à C₄)₂, un (alkyle en C₁ à C₄)-O-(alkyle en C₁ à C₄), un C(=O)-(alkyle en C₁ à C₄), un C(=O)-(hétéroaryle en C₂ à C₁₀), un C(=O)-(hétérocycloalkyle en C₂ à C₁₀) ou un C(=O)-(cycloalkyle en C₃ à C₁₀) ;
X est un atome d'halogène ; et
n est un entier quelconque choisi parmi 0, 1, 2 et 3.

2. Composés représentés par la formule chimique I, leurs stéréoisomères ou leurs sels pharmaceutiquement acceptables, selon la revendication 1, dans lesquels, dans la formule chimique I ci-dessus,
chacun de Z₁ à Z₄ est indépendamment N ou CRₐ, où Rₐ est H, X, un alkyle en C₁ à C₄ ou un O-(alkyle en C₁ à C₄), et les Rₐ peuvent être mutuellement différents quand CRₐ est au nombre de 2 ou plus ;
K est O ou S ;
R₁ est CX₃ ou CX₂H ; 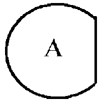 est un arylène en C₄ à C₁₂ ou un hétéroarylène en C₂ à C₁₀, où l'hétéroarylène en C₂ à C₁₀ peut comprendre au moins un N ;
chacun de R₂ et R₃ est indépendamment H, X, un halogénoalkyle en C₁ à C₄, un aryle en C₆ à C₁₂, un hétérocycloalkyle en C₂ à C₁₀, un hétérocycloalcényle en C₂ à C₁₀, N(R_{b})(R_{c}) ou un hétéroaryle en C₂ à C₁₀,
où au moins un H de l'aryle en C₆ à C₁₂, de l'hétéroaryle en C₂ à C₁₀, de l'hétérocycloalcényle en C₂ à C₁₀ ou de l'hétérocycloalkyle en C₂ à C₁₀ peut être indépendamment remplacé par X, un alkyle en C₁ à C₄, un halogénoalkyle en C₁ à C₄, un cycloalkyle en C₃ à C₁₀, un hétérocycloalkyle en C₂ à C₁₀, un (alkyle en C₁ à C₄)-(hétéroaryle en C₂ à C₁₀), un (alkyle en C₁ à C₄)-(hétérocycloalkyle en C₂ à C₁₀), un (hétérocycloalkyle en C₂ à C₁₀)-(alkyle en C₁ à C₄), un (hétérocycloalkyle en C₂ à C₁₀)-(halogénoalkyle en C₁ à C₄), un (hétérocycloalkyle en C₂ à C₁₀)-(cycloalkyle en C₃ à C₁₀), un (hétérocycloalkyle en C₂ à C₁₀)-(hétérocycloalkyle en C₂ à C₁₀), un (hétérocycloalkyle en C₂ à C₁₀)-(alkyle en C₁ à C₄)-(halogénocycloalkyle en C₃ à C₁₀), un S(O)₂-(alkyle en C₁ à C₄), C(=O)-R₆, un O-(alkyle en C₁ à C₄) ou un (hétérocycloalkyle en C₂ à C₁₀)-C(=O)-R₈ ;
Y est CH, N, O ou S {où R₄ n'existe pas quand Y est O ou S} ;
R₄ est un alkyle en C₁ à C₄, un hétérocycloalkyle en C₂ à C₁₀, un hétéroaryle en C₂ à C₁₀ ou un (alkyle en C₁ à C₄)-(hétérocycloalkyle en C₂ à C₁₀),
où au moins un H de l'alkyle en C₁ à C₄, de l'hétérocycloalkyle en C₂ à C₁₀ ou du (alkyle en C₁ à C₄)-(hétérocycloalkyle en C₂ à C₁₀) peut être indépendamment remplacé par un alkyle en C₁ à C₄, un halogénoalkyle en C₁ à C₄, un O- (alkyle en C₁ à C₄), -N(R_{b})(R_{c}), un cycloalkyle en C₃ à C₁₀, un hétérocycloalkyle en C₂ à C₁₀, un (hétérocycloalkyle en C₂ à C₁₀)-C(=O)-R₁₀, un S(O₂)-(alkyle en C₁ à C₄), C(=O)-R₁₁, un (alkyle en C₁ à C₄)-O-(alkyle en C₁ à C₄), un aryle en C₆ à C₁₂, un (hétérocycloalkyle en C₂ à C₁₀)-(hétérocycloalkyle en C₂ à C₁₀) ou un (alkyle en C₁ à C₄)-(hétéroaryle en C₂ à C₁₀) ;
chacun de R₆, R₈, R₁₀ et R₁₁ est indépendamment un alkyle en C₁ à C₄, un (alkyle en C₁ à C₄)-OH, un (alkyle en C₁ à C₄)-O-(alkyle en C₁ à C₄), un hétérocycloalkyle en C₂ à C₁₀, un aryle en C₆ à C₁₂, un hétéroaryle en C₂ à C₁₀ ou un O-(alkyle en C₁ à C₄),
où au moins un H de l'aryle en C₆ à C₁₂ ou de l'hétéroaryle en C₂ à C₁₀ peut être indépendamment remplacé par un alkyle en C₁ à C₄ ou un halogénoalkyle en C₁ à C₄ ;
chacun de R_{b} et R_{c} est indépendamment H, un alkyle en C₁ à C₄ ou un C(=O)-(alkyle en C₁ à C₄) ;
X est un atome d'halogène ; et
n est un entier quelconque choisi parmi 0, 1, 2 et 3.

3. Composés représentés par la formule chimique I, leurs stéréoisomères ou leurs sels pharmaceutiquement acceptables, selon la revendication 1, dans lesquels, dans la formule chimique I ci-dessus,
l'hétérocycloalkyle en C₂ à C₁₀ est 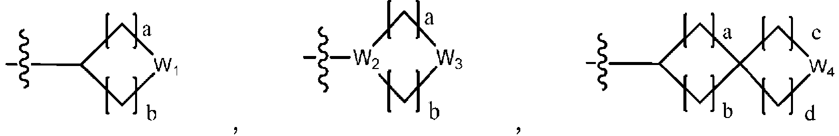 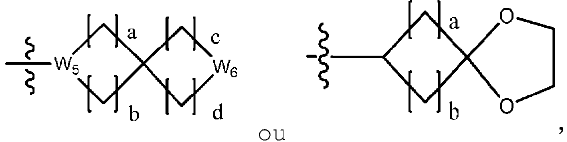
où chacun de W₁ à W₆ est indépendamment N, NH, O, S ou SO₂, et
chacun de a à d est indépendamment l'entier 1, 2 ou 3.

4. Composés représentés par la formule chimique I, leurs stéréoisomères ou leurs sels pharmaceutiquement acceptables, selon la revendication 1, dans lesquels les composés représentés par la formule chimique I ci-dessus comprennent les composés représentés par la formule chimique II suivante : 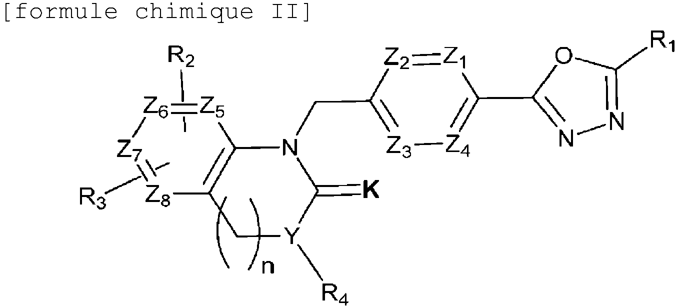 dans lesquels
chacun de Z₁ à Z₄ est indépendamment N ou CRₐ, où Rₐ est H, X, un alkyle en C₁ à C₄ ou un O- (alkyle en C₁ à C₄), et les Rₐ peuvent être mutuellement différents quand CRₐ est au nombre de 2 ou plus ;
chacun de Z₅ à Z₈ est indépendamment CR₂, CR₃, CH ou N, où Z₅ à Z₈ comprennent CR₂, CR₃, CH et N, comprennent CR₂, CR₃ et deux N, ou comprennent CR₂, CR₃ et deux CH ;
K est O ou S ;
R₁ est CX₃ ou CX₂H ;
chacun de R₂ et R₃ est indépendamment H, X, un alkyle en C₁ à C₄, un halogénoalkyle en C₁ à C₄, un aryle en C₆ à C₁₂, un hétéroaryle en C₂ à C₁₀, un cycloalkyle en C₃ à C₁₀, un hétérocycloalkyle en C₂ à C₁₀, un hétérocycloalcényle en C₂ à C₁₀, N(R_{b})(R_{c}), un NH-(alkyle en C₁ à C₄)-N-(R_{b})(R_{c}), un NH-O-(alkyle en C₁ à C₄) ou NHC(=O)-R₅,
où au moins un H de l'alkyle en C₁ à C₄, de l'aryle en C₆ à C₁₂, de l'hétéroaryle en C₂ à C₁₀, de l'hétérocycloalcényle en C₂ à C₁₀ ou de l'hétérocycloalkyle en C₂ à C₁₀ peut être indépendamment remplacé par X, un alkyle en C₁ à C₄, un halogénoalkyle en C₁ à C₄, un cycloalkyle en C₃ à C₁₀, un aryle en C₆ à C₁₂, un hétéroaryle en C₂ à C₁₀, un hétérocycloalkyle en C₂ à C₁₀, un (alkyle en C₁ à C₄)-(hétéroaryle en C₂ à C₁₀), un (alkyle en C₁ à C₄)-(hétérocycloalkyle en C₂ à C₁₀), un (hétérocycloalkyle en C₂ à C₁₀)-(alkyle en C₁ à C₄), un (hétérocycloalkyle en C₂ à C₁₀)-(halogénoalkyle en C₁ à C₄), un (hétérocycloalkyle en C₂ à C₁₀)-(cycloalkyle en C₃ à C₁₀), un (hétérocycloalkyle en C₂ à C₁₀)-(hétérocycloalkyle en C₂ à C₁₀), un (hétérocycloalkyle en C₂ à C₁₀)-(alkyle en C₁ à C₄)-(halogénocycloalkyle en C₃ à C₁₀), un (alkyle en C₁ à C₄)-(cycloalkyle en C₃ à C₁₀), un S(O₂)-(alkyle en C₁ à C₄), C(=O)-N(R_{b})(R_{c}), C(=O)-R₆, -N(R_{b})(R_{c}), un (alkyle en C₁ à C₄)-N(R_{b})(R_{c}), un O-(alkyle en C₁ à C₄), un (alkyle en C₁ à C₄)-O-(alkyle en C₁ à C₄), un C(=O)O-(hétérocycloalkyle en C₂ à C₁₀), un (alkyle en C₁ à C₄)-C(=O)-R₇ ou un (hétérocycloalkyle en C₂ à C₁₀)-C(=O)-R₈ ;
Y est CH, N, O ou S {où R₄ n'existe pas quand Y est O ou S} ;
R₄ est H, un alkyle en C₁ à C₄, un cycloalkyle en C₃ à C₇, un hétérocycloalkyle en C₂ à C₁₀, un (alkyle en C₁ à C₄)-(hétérocycloalkyle en C₂ à C₁₀), un aryle en C₆ à C₁₂, un hétéroaryle en C₂ à C₁₀ ou C(=O)-R₉,
où au moins un H de l'alkyle en C₁ à C₄, de l'hétérocycloalkyle en C₂ à C₁₀, ou du (alkyle en C₁ à C₄)-(hétérocycloalkyle en C₂ à C₁₀) peut être indépendamment remplacé par X, un alkyle en C₁ à C₄, un halogénoalkyle en C₁ à C₄, un O- (alkyle en C₁ à C₄) , -N(R_{b})(C_{c}), un cycloalkyle en C₃ à C₁₀, un hétérocycloalkyle en C₂ à C₁₀, un (hétérocycloalkyle en C₂ à C₁₀) -C (=O) -R₁₀, un S(O)₂- (alkyle en C₁ à C₄), C(=O)-R₁₁, un (alkyle en C₁ à C₄) -O-(alkyle en C₁ à C₄), un aryle en C₆ à C₁₂, un hétéroaryle en C₂ à C₁₀, un (alkyle en C₁ à C₄) - (hétéroaryle en C₂ à C₁₀), un (hétérocycloalkyle en C₂ à C₁₀) - (hétérocycloalkyle en C₂ à C₁₀) ou un C(=O)-(alkyle en C₁ à C₄)-O-(alkyle en C₁ à C₄) ;
chacun de R₅, R₆, R₇, R₈, R₉, R₁₀ et R₁₁ est indépendamment H, un alkyle en C₁ à C₄, un (alkyle en C₁ à C₄)-OH, un (alkyle en C₁ à C₄)-O-(alkyle en C₁ à C₄), un hétérocycloalkyle en C₂ à C₁₀, un aryle en C₆ à C₁₂, un hétéroaryle en C₂ à C₁₀, un O-(alkyle en C₁ à C₄), un cycloalkyle en C₃ à C₇ ou un (alkyle en C₁ à C₄) -N (R_{b}) (R_{c}),
où au moins un H de l'aryle en C₆ à C₁₂ ou de l'hétéroaryle en C₂ à C₁₀ peut être indépendamment remplacé par un alkyle en C₁ à C₄, X, ou un halogénoalkyle en C₁ à C₄ ;
chacun de R_{b} et R_{c} est indépendamment H, un alkyle en C₁ à C₄, un aryle en C₆ à C₁₂, un hétéroaryle en C₂ à C₁₀, un cycloalkyle en C₃ à C₁₀, un hétérocycloalkyle en C₂ à C₁₀, un (alkyle en C₁ à C₄)-NH-(alkyle en C₁ à C₄), un (alkyle en C₁ à C₄)-N-(alkyle en C₁ à C₄)₂, un (alkyle en C₁ à C₄)-O-(alkyle en C₁ à C₄), un C(=O)-(alkyle en C₁ à C₄), un C(=O)-(hétéroaryle en C₂ à C₁₀), un C(=O)-(hétérocycloalkyle en C₂ à C₁₀) ou un C(=O)-(cycloalkyle en C₃ à C₁₀) ;
X est un atome d'halogène ; et
n est un entier quelconque choisi parmi 0, 1, 2 et 3.

5. Composés représentés par la formule chimique I, leurs stéréoisomères ou leurs sels pharmaceutiquement acceptables, selon la revendication 4, dans lesquels
chacun de Z₁ à Z₄ est indépendamment N ou CRₐ, où Rₐ est H, X, un alkyle en C₁ à C₄ ou un O-(alkyle en C₁ à C₄), et les Rₐ peuvent être mutuellement différents quand CRₐ est au nombre de 2 ou plus ;
chacun de Z₅ à Z₈ est indépendamment CR₂, CR₃, CH ou N, où Z₅ à Z₅ comprennent CR₂, CR₃, CH et N ou comprennent CR₂, CR₃ et deux CH (toutefois Z₆ est N quand Z₅ à Z₈ comprennent CR₂, CR₃, CH et N) ;
K est O ou S ;
R₁ est CX₃ ou CX₂H ;
chacun de R₂ et R₃ est indépendamment H, X, un halogénoalkyle en C₁ à C₄, un aryle en C₆ à C₁₂, un hétérocycloalkyle en C₂ à C₁₀, un hétérocycloalcényle en C₂ à C₁₀, N(R_{b})(R_{c}) ou un hétéroaryle en C₂ à C₁₀,
où au moins un H de l'aryle en C₆ à C₁₂, de l'hétéroaryle en C₂ à C₁₀, de l'hétérocycloalcényle en C₂ à C₁₀ ou de l'hétérocycloalkyle en C₂ à C₁₀ peut être indépendamment remplacé par X, un alkyle en C₁ à C₄, un halogénoalkyle en C₁ à C₄, un cycloalkyle en C₃ à C₁₀, un hétérocycloalkyle en C₂ à C₁₀, un (alkyle en C₁ à C₄)-(hétéroaryle en C₂ à C₁₀), un (alkyle en C₁ à C₄)-(hétérocycloalkyle en C₂ à C₁₀), un (hétérocycloalkyle en C₂ à C₁₀) - (alkyle en C₁ à C₄), un (hétérocycloalkyle en C₂ à C₁₀) - (halogénoalkyle en C₁ à C₄), un (hétérocycloalkyle en C₂ à C₁₀) - (cycloalkyle en C₃ à C₁₀), un (hétérocycloalkyle en C₂ à C₁₀) - (hétérocycloalkyle en C₂ à C₁₀), un (hétérocycloalkyle en C₂ à C₁₀) -(alkyle en C₁ à C₄)-(halogénocycloalkyle en C₃ à C₁₀), un S(O)₂-(alkyle en C₁ à C₄), C(=O)-R₆, un O-(alkyle en C₁ à C₄) ou un (hétérocycloalkyle en C₂ à C₁₀)-C(=O)-R₈;
Y est CH, N, O ou S {où R₄ n'existe pas quand Y est O ou S} ;
R₄ est un alkyle en C₁ à C₄, un hétérocycloalkyle en C₂ à C₁₀, un hétéroaryle en C₂ à C₁₀ ou un (alkyle en C₁ à C₄)-(hétérocycloalkyle en C₂ à C₁₀),
où au moins un H de l'alkyle en C₁ à C₄, de l'hétérocycloalkyle en C₂ à C₁₀ ou du (alkyle en C₁ à C₄)-(hétérocycloalkyle en C₂ à C₁₀) peut être indépendamment remplacé par un alkyle en C₁ à C₄, un halogénoalkyle en C₁ à C₄, un O- (alkyle en C₁ à C₄), -N(R_{b})(R_{c}), un cycloalkyle en C₃ à C₁₀, un hétérocycloalkyle en C₂ à C₁₀, un (hétérocycloalkyle en C₂ à C₁₀) -C(=O)-R₁₀, un S(O₂)-(alkyle en C₁ à C₄), C(=O)-R₁₁, un (alkyle en C₁ à C₄)-O-(alkyle en C₁ à C₄), un aryle en C₆ à C₁₂, un (hétérocycloalkyle en C₂ à C₁₀)-(hétérocycloalkyle en C₂ à C₁₀) ou un (alkyle en C₁ à C₄)-(hétéroaryle en C₂ à C₁₀) ;
chacun de R₆, R₈, R₁₀ et R₁₁ est indépendamment un alkyle en C₁ à C₄, un (alkyle en C₁ à C₄)-OH, un (alkyle en C₁ à C₄)-O-(alkyle en C₁ à C₄), un hétérocycloalkyle en C₂ à C₁₀, un aryle en C₆ à C₁₂, un hétéroaryle en C₂ à C₁₀ ou un O-(alkyle en C₁ à C₄),
où au moins un H de l'aryle en C₆ à C₁₂ ou de l'hétéroaryle en C₂ à C₁₀ peut être indépendamment remplacé par un alkyle en C₁ à C₄ ou un halogénoalkyle en C₁ à C₄ ;
chacun de R_{b} et R_{c} est indépendamment H, un alkyle en C₁ à C₄ ou un C(=O)- (alkyle en C₁ à C₄) ;
X est un atome d'halogène ; et
n est un entier quelconque choisi parmi 0, 1, 2 et 3.

6. Composés représentés par la formule chimique I, leurs stéréoisomères ou leurs sels pharmaceutiquement acceptables, selon la revendication 4, dans lesquels, dans la formule chimique II ci-dessus,
chacun de Z₁ à Z₄ est indépendamment N ou CRₐ, où Rₐ est H, X, un alkyle en C₁ à C₄ ou un O-(alkyle en C₁ à C₄), et les Rₐ peuvent être mutuellement différents quand CRₐ est au nombre de 2 ou plus ;
chacun de Z₅ à Z₈ est indépendamment CR₂, CR₃, CH ou N,
où Z₅ à Z₈ comprennent CR₂, CR₃, CH et N ou comprennent CR₂, CR₃ et deux CH (toutefois Z₆ est N quand Z₅ à Z₈ comprennent CR₂, CR₃, CH et N) ;
K est O ou S ;
R₁ est CX₃ ou CX₂H ;
chacun de R₂ et R₃ est indépendamment H, X, un halogénoalkyle en C₁ à C₄, un aryle en C₆ à C₁₂, un hétérocycloalkyle en C₂ à C₁₀, un hétérocycloalcényle en C₂ à C₁₀, N(R_{b})(R_{c}) ou un hétéroaryle en C₂ à C₁₀,
où au moins un H de l'aryle en C₆ à C₁₂, de l'hétéroaryle en C₂ à C₁₀, de l'hétérocycloalcényle en C₂ à C₁₀ ou de l'hétérocycloalkyle en C₂ à C₁₀ peut être indépendamment remplacé par X, un alkyle en C₁ à C₄, un halogénoalkyle en C₁ à C₄, un cycloalkyle en C₃ à C₁₀, un hétérocycloalkyle en C₂ à C₁₀, un (alkyle en C₁ à C₄)-(hétéroaryle en C₂ à C₁₀), un (alkyle en C₁ à C₄)-(hétérocycloalkyle en C₂ à C₁₀), un (hétérocycloalkyle en C₂ à C₁₀)-(alkyle en C₁ à C₄), un (hétérocycloalkyle en C₂ à C₁₀)-(halogénoalkyle en C₁ à C₄), un (hétérocycloalkyle en C₂ à C₁₀)-(cycloalkyle en C₃ à C₁₀), un (hétérocycloalkyle en C₂ à C₁₀)-(hétérocycloalkyle en C₂ à C₁₀), un (hétérocycloalkyle en C₂ à C₁₀) -(alkyle en C₁ à C₄) - (halogénocycloalkyle en C₃ à C₁₀), un S(O)₂-(alkyle en C₁ à C₄), C(=O)-R₆, un 0- (alkyle en C₁ à C₄) ou un (hétérocycloalkyle en C₂ à C₁₀) -C(=O)-R₈ ;
Y est CH, N, O ou S {où R₄ n'existe pas quand Y est O ou S} ;
R₄ est un alkyle en C₁ à C₄, un hétérocycloalkyle en C₂ à C₁₀, un hétéroaryle en C₂ à C₁₀ ou un (alkyle en C₁ à C₄) - (hétérocycloalkyle en C₂ à C₁₀),
où au moins un H de l'alkyle en C₁ à C₄, de l'hétérocycloalkyle en C₂ à C₁₀ ou du (alkyle en C₁ à C₄)-(hétérocycloalkyle en C₂ à C₁₀) peut être indépendamment remplacé par un alkyle en C₁ à C₄, un halogénoalkyle en C₁ à C₄, un O-(alkyle en C₁ à C₄), -N(R_{b})(R_{c}), un cycloalkyle en C₃ à C₁₀, un hétérocycloalkyle en C₂ à C₁₀, un (hétérocycloalkyle en C₂ à C₁₀) -C(=O)-R₁₀, un S(O₂)- (alkyle en C₁ à C₄), C(=O)-R₁₁, un (alkyle en C₁ à C₄)-O-(alkyle en C₁ à C₄) , un aryle en C₆ à C₁₂, un (hétérocycloalkyle en C₂ à C₁₀)-(hétérocycloalkyle en C₂ à C₁₀) ou un (alkyle en C₁ à C₄)-(hétéroaryle en C₂ à C₁₀) ;
chacun de R₆, R₈, R₁₀ et R₁₁ est indépendamment un alkyle en C₁ à C₄, un (alkyle en C₁ à C₄)-OH, un (alkyle en C₁ à C₄)-O- (alkyle en C₁ à C₄), un hétérocycloalkyle en C₂ à C₁₀, un aryle en C₆ à C₁₂, un hétéroaryle en C₂ à C₁₀ ou un O- (alkyle en C₁ à C₄),
où au moins un H de l'aryle en C₆ à C₁₂ ou de l'hétéroaryle en C₂ à C₁₀ peut être indépendamment remplacé par un alkyle en C₁ à C₄ ou un halogénoalkyle en C₁ à C₄ ;
chacun de R_{b} et R_{c} est indépendamment H, un alkyle en C₁ à C₄ ou un C(=O)- (alkyle en C₁ à C₄) ;
X est un atome d'halogène ; et
n est un entier quelconque choisi parmi 0, 1, 2 et 3.

7. Composés représentés par la formule chimique I, leurs stéréoisomères ou leurs sels pharmaceutiquement acceptables, selon la revendication 1 dans lesquels, dans la formule chimique I ci-dessus,
chacun de Z₁ à Z₄ est indépendamment N ou CRₐ, où Rₐ est H ou X, et les Rₐ peuvent être mutuellement différents quand CRₐ est au nombre de 2 ou plus ;
K est O ;
R₁ est CF₃ ou CF₂H ; 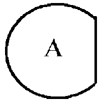 est le phénylène ou le pyridinylène,
chacun de R₂ et R₃ est indépendamment H, X, un halogénoalkyle en C₁ à C₄, un aryle en C₆ à C₁₂, un hétérocycloalkyle en C₂ à C₁₀, un hétérocycloalcényle en C₂ à C₁₀, N(R_{b})(R_{c})ou un hétéroaryle en C₂ à C₁₀,
où au moins un H de l'aryle en C₆ à C₁₂, de l'hétéroaryle en C₂ à C₁₀, de l'hétérocycloalcényle en C₂ à C₁₀ ou de l'hétérocycloalkyle en C₂ à C₁₀ peut être indépendamment remplacé par X, un alkyle en C₁ à C₄, un halogénoalkyle en C₁ à C₄, un cycloalkyle en C₃ à C₁₀, un hétérocycloalkyle en C₂ à C₁₀, un (alkyle en C₁ à C₄)-(hétéroaryle en C₂ à C₁₀), un (alkyle en C₁ à C₄)-(hétérocycloalkyle en C₂ à C₁₀), un (hétérocycloalkyle en C₂ à C₁₀)-(alkyle en C₁ à C₄), un (hétérocycloalkyle en C₂ à C₁₀)-halogénoalkyle en C₁ à C₄), un (hétérocycloalkyle en C₂ à C₁₀)-(cycloalkyle en C₃ à C₁₀), un (hétérocycloalkyle en C₂ à C₁₀)-(hétérocycloalkyle en C₂ à C₁₀), un (hétérocycloalkyle en C₂ à C₁₀) - (alkyle en C₁ à C₄) - (halogénocycloalkyle en C₃ à C₁₀), un S(O₂)-(alkyle en C₁ à C₄) , C(=O)-R₆, un O- (alkyle en C₁ à C₄) ou un (hétérocycloalkyle en C₂ à C₁₀)-C(=O)-R₈ ;
Y est N, O ou S {où R₄ n'existe pas quand Y est O ou S} ;
R₄ est un alkyle en C₁ à C₄, un hétérocycloalkyle en C₂ à C₁₀, un hétéroaryle en C₂ à C₁₀ ou un (alkyle en C₁ à C₄)-(hétérocycloalkyle en C₂ à C₁₀),
où au moins un H de l'alkyle en C₁ à C₄, de l'hétérocycloalkyle en C₂ à C₁₀ ou du (alkyle en C₁ à C₄)-(hétérocycloalkyle en C₂ à C₁₀) peut être indépendamment remplacé par un alkyle en C₁ à C₄, un halogénoalkyle en C₁ à C₄, un O- (alkyle en C₁ à C₄), -N(R_{b})(C_{c}), un cycloalkyle en C₃ à C₁₀, un hétérocycloalkyle en C₂ à C₁₀, un (hétérocycloalkyle en C₂ à C₁₀) -C(=O)-R₁₀, un S(O)₂- (alkyle en C₁ à C₄), C(=O)-R₁₁, un (alkyle en C₁ à C₄)-O- (alkyle en C₁ à C₄), un aryle en C₆ à C₁₂, un (hétérocycloalkyle en C₂ à C₁₀)-(hétérocycloalkyle en C₂ à C₁₀) ou un (alkyle en C₁ à C₄)-(hétéroaryle en C₂ à C₁₀) ;
R₆ est un alkyle en C₁ à C₄, un O-(alkyle en C₁ à C₄) ou un (alkyle en C₁ à C₄)-OH ;
R₈ est un O-(alkyle en C₁ à C₄);
R₁₀ est un alkyle en C₁ à C₄ ;
R₁₁ est un alkyle en C₁ à C₄, un (alkyle en C₁ à C₄)-OH, un (alkyle en C₁ à C₄)-O-(alkyle en C₁ à C₄) , un hétérocycloalkyle en C₂ à C₁₀, un aryle en C₆ à C₁₂, un hétéroaryle en C₂ à C₁₀ ou un O-(alkyle en C₁ à C₄), où au moins un H de l'aryle en C₆ à C₁₂ ou de l'hétéroaryle en C₂ à C₁₀ peut être indépendamment substitué par un alkyle en C₁ à C₄ ou un halogénoalkyle en C₁ à C₄ ;
chacun de R_{b} et R_{c} est indépendamment H, un alkyle en C₁ à C₄ ou un C(=O)-(alkyle en C₁ à C₄);
X est F, Cl ou Br ; et
n vaut 0 ou 1.

8. Composés représentés par la formule chimique I, leurs stéréoisomères ou leurs sels pharmaceutiquement acceptables, selon la revendication 1, dans lesquels les composés sont choisis dans le groupe constitué par les composés représentés par les composés 1 à 368 suivants :
| Composé | Structure | Composé | Structure |
|---|---|---|---|
| 1 | 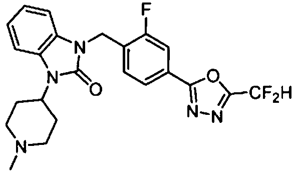 | 2 | 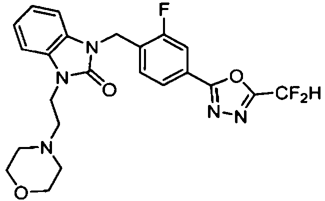 |
| 3 | 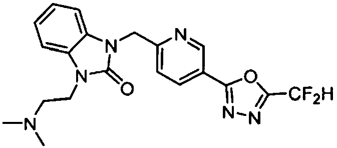 | 4 | 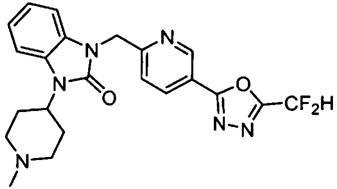 |
| 5 | 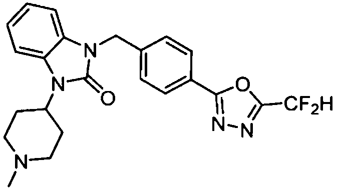 | 6 | 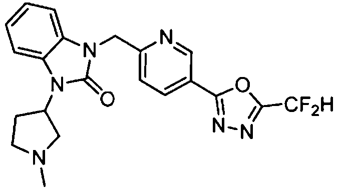 |
| 7 | 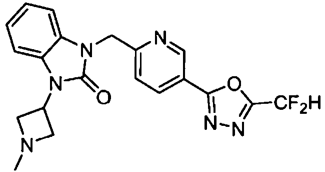 | 8 | 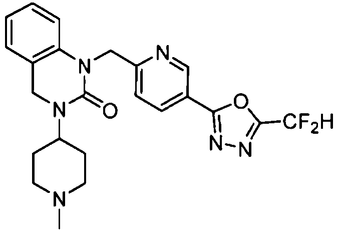 |
| 9 | 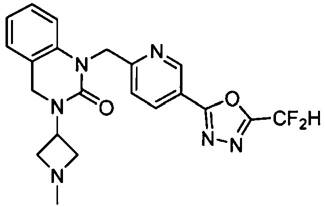 | 10 | 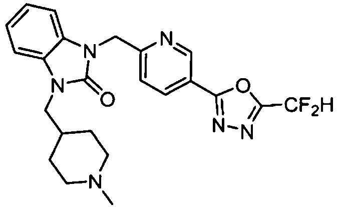 |
| 11 | 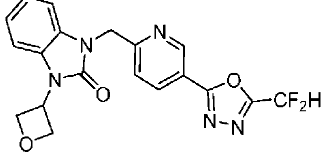 | 12 | 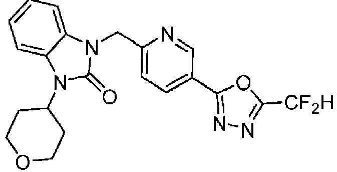 |
| 13 | 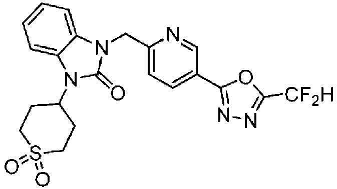 | 14 | 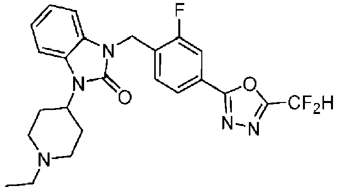 |
| 15 | 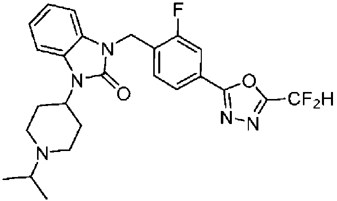 | 16 | 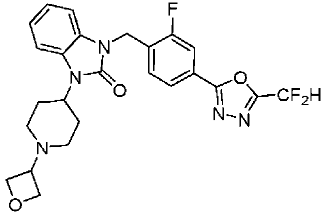 |
| 17 | 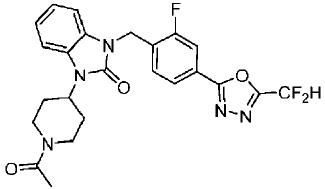 | 18 | 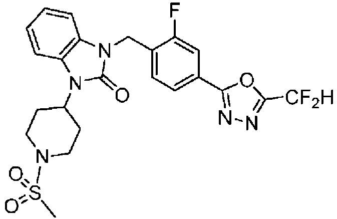 |
| 19 | 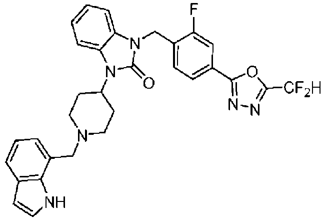 | 20 | 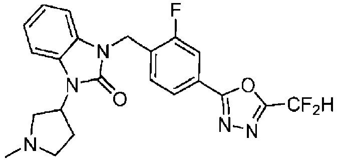 |
| 21 | 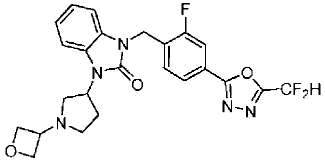 | 22 | 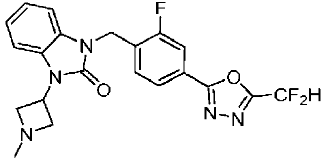 |
| 23 | 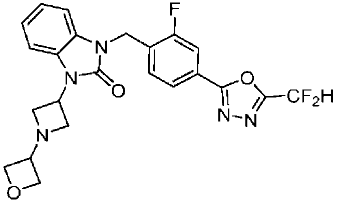 | 24 | 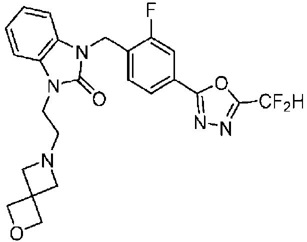 |
| 25 | 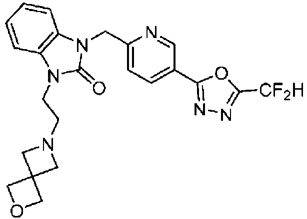 | 26 | 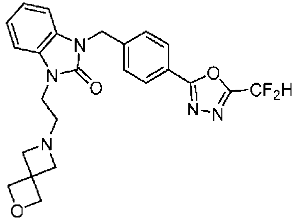 |
| 27 | 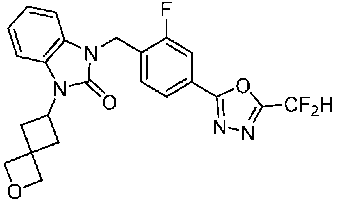 | 28 | 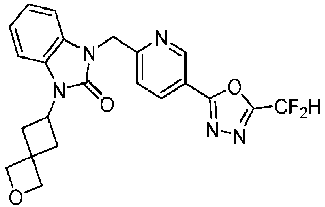 |
| 29 | 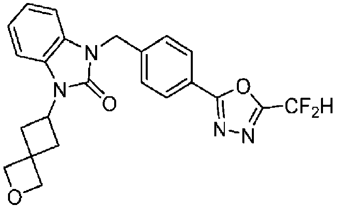 | 30 | 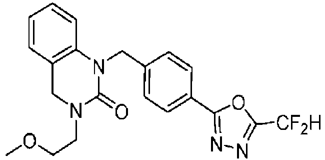 |
| 31 | 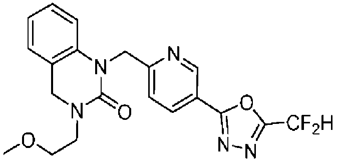 | 32 | 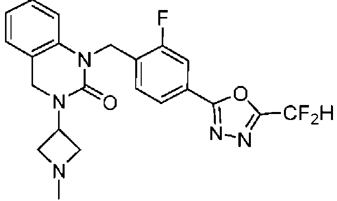 |
| 33 | 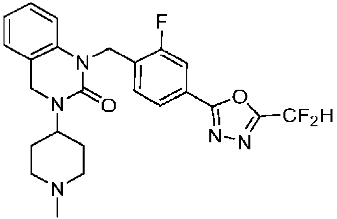 | 34 | 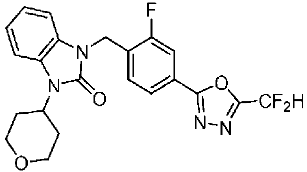 |
| 35 | 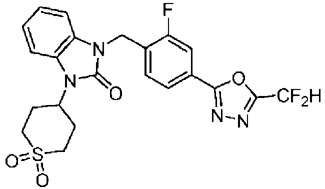 | 36 | 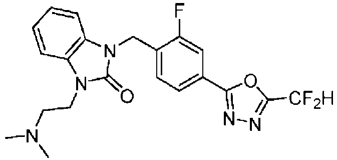 |
| 37 | 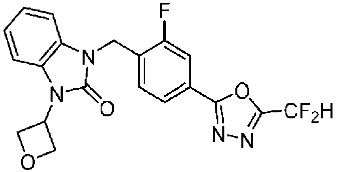 | 38 | 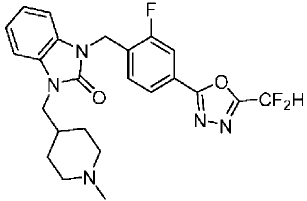 |
| 39 | 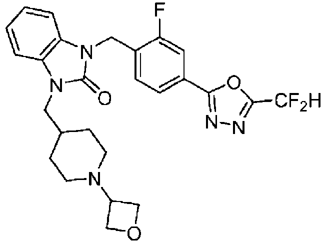 | 40 | 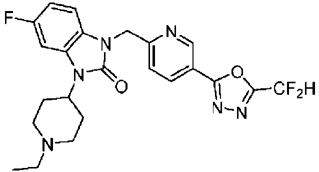 |
| 41 | 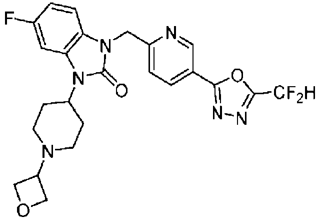 | 42 | 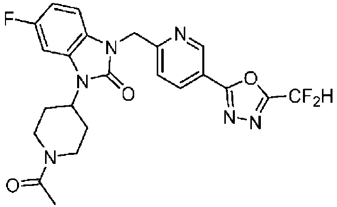 |
| 43 | 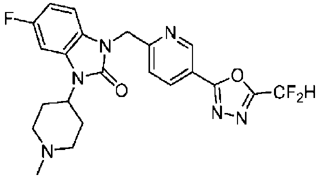 | 44 | 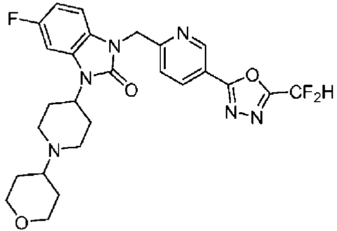 |
| 45 | 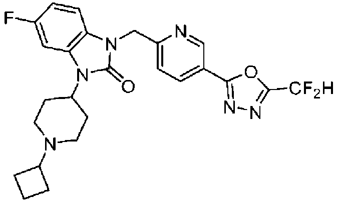 | 46 | 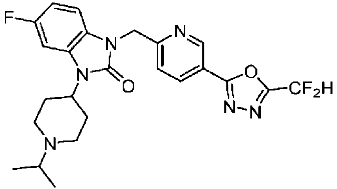 |
| 47 | 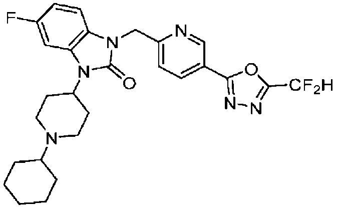 | 48 | 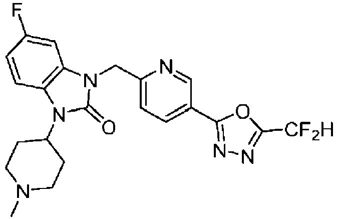 |
| 49 | 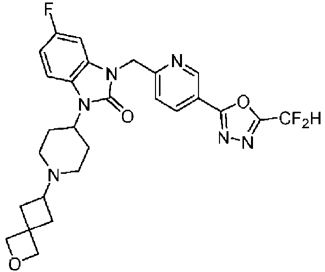 | 50 | 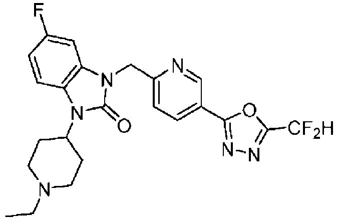 |
| 51 | 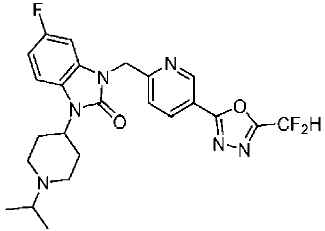 | 52 | 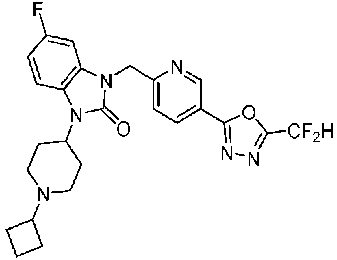 |
| 53 | 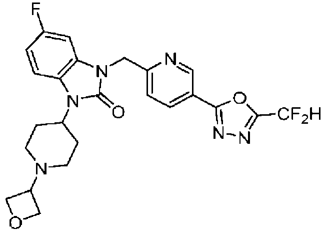 | 54 | 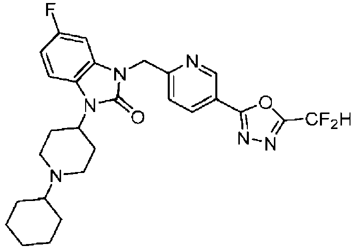 |
| 55 | 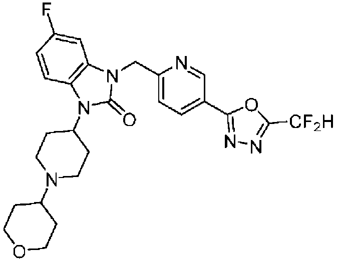 | 56 | 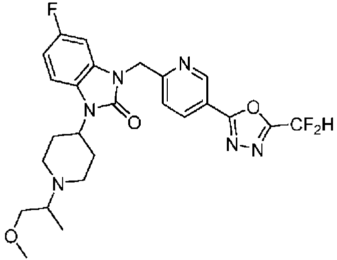 |
| 57 | 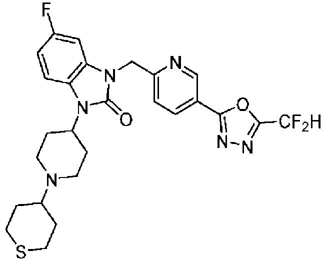 | 58 | 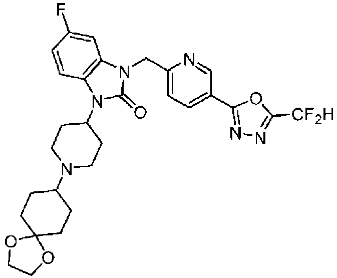 |
| 59 | 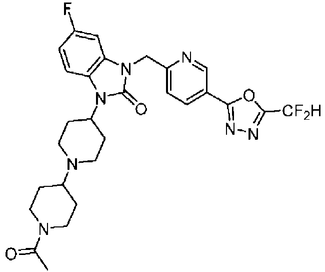 | 60 | 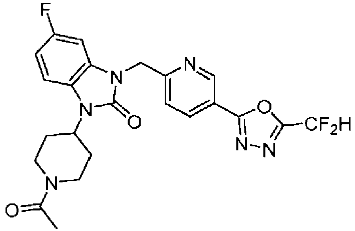 |
| 61 | 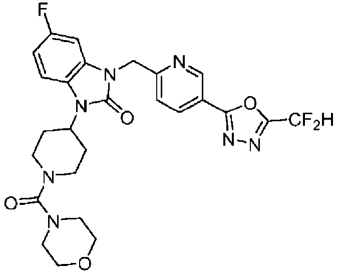 | 62 | 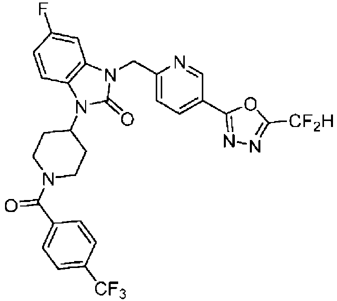 |
| 63 | 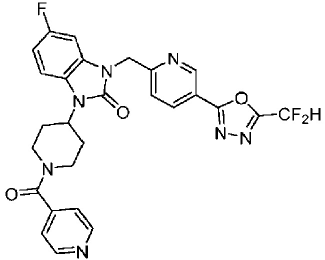 | 64 | 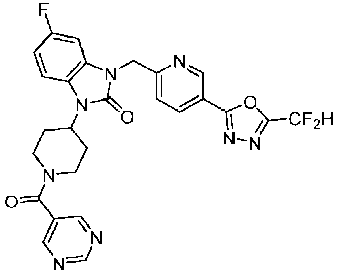 |
| 65 | 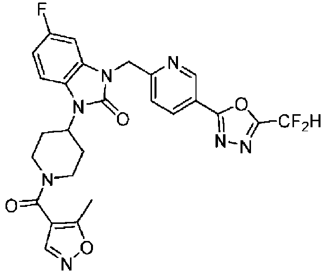 | 66 | 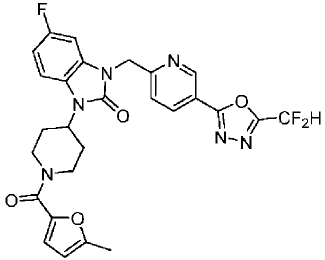 |
| 67 | 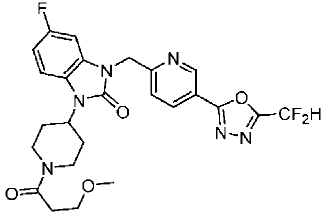 | 68 | 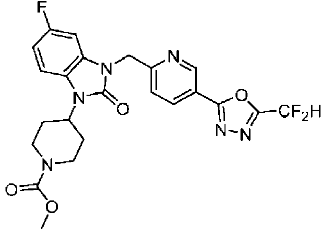 |
| 69 | 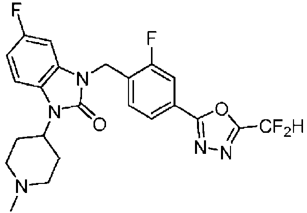 | 70 | 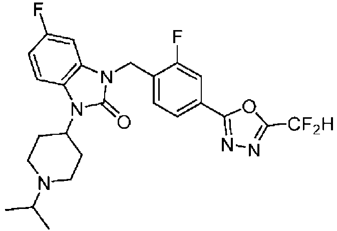 |
| 71 | 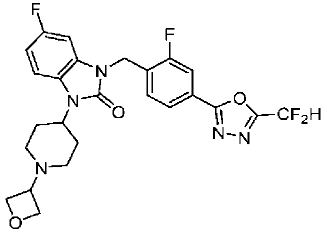 | 72 | 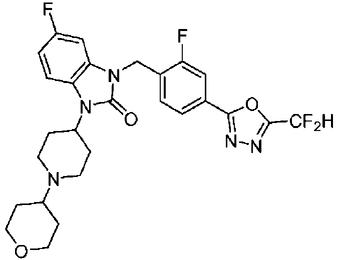 |
| 73 | 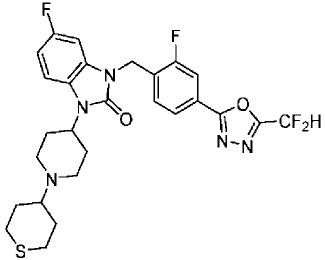 | 74 | 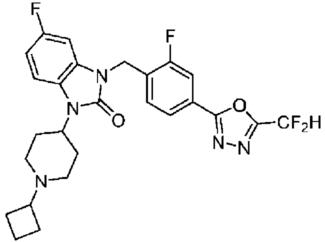 |
| 75 | 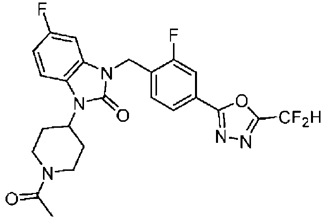 | 76 | 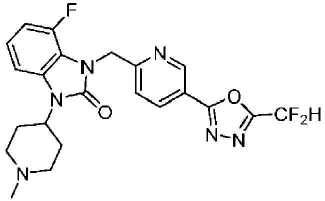 |
| 77 | 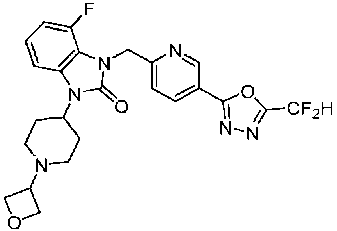 | 78 | 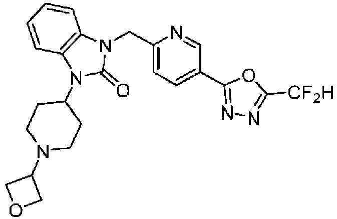 |
| 79 | 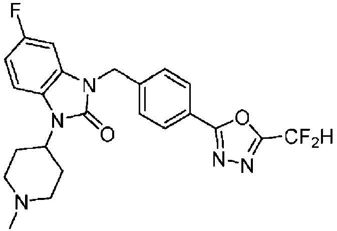 | 80 | 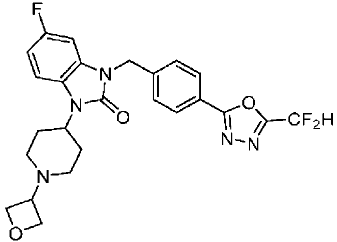 |
| 81 | 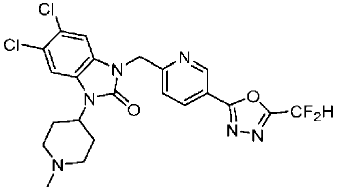 | 82 | 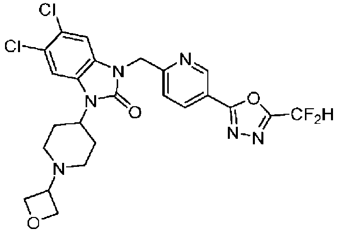 |
| 83 | 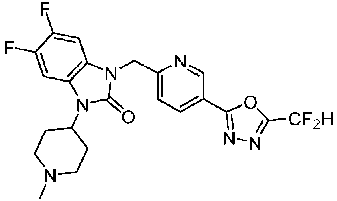 | 84 | 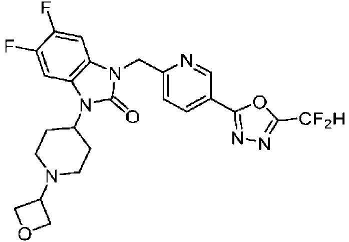 |
| 85 | 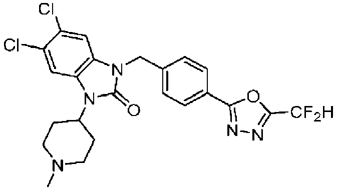 | 86 | 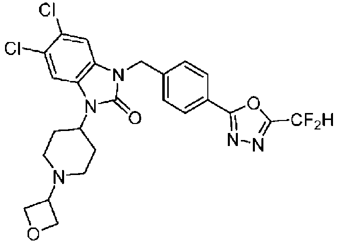 |
| 87 | 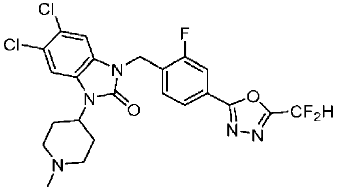 | 88 | 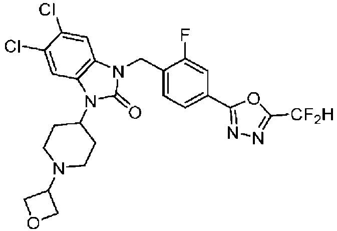 |
| 89 | 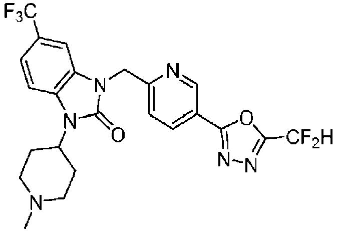 | 90 | 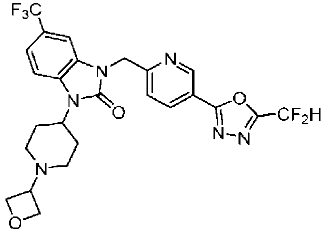 |
| 91 | 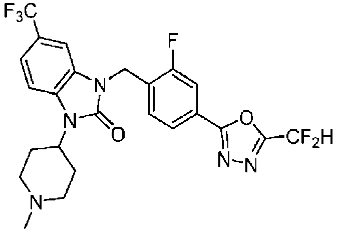 | 92 | 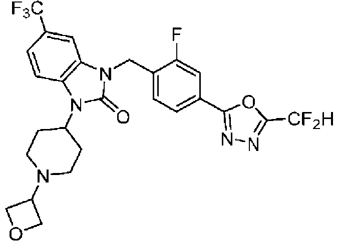 |
| 93 | 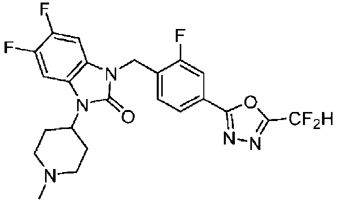 | 94 | 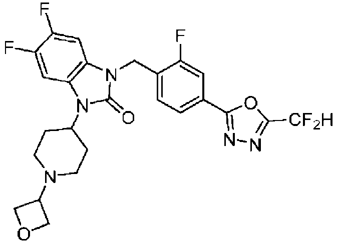 |
| 95 | 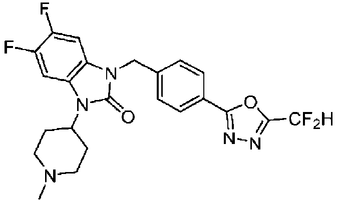 | 96 | 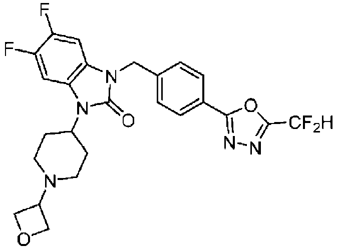 |
| 97 | 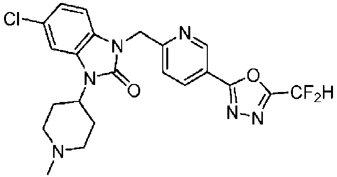 | 98 | 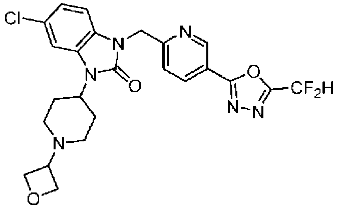 |
| 99 | 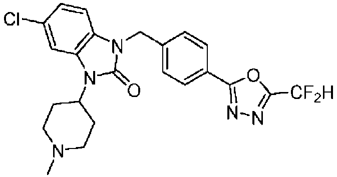 | 100 | 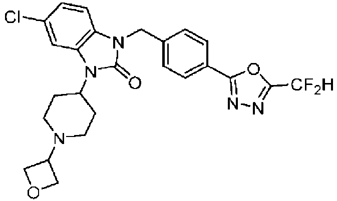 |
| 101 | 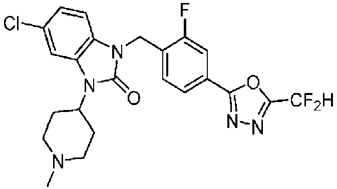 | 102 | 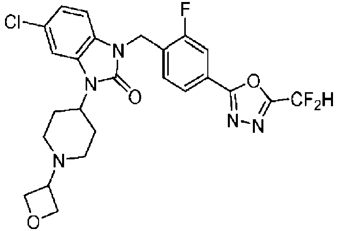 |
| 103 | 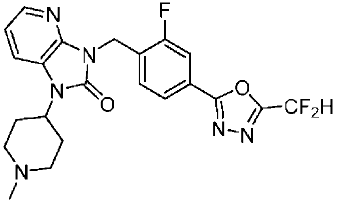 | 104 | 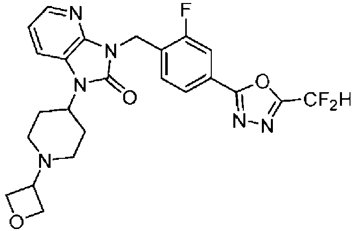 |
| 105 | 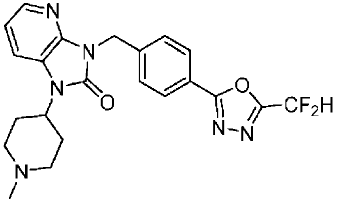 | 106 | 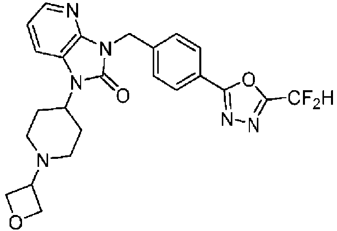 |
| 107 | 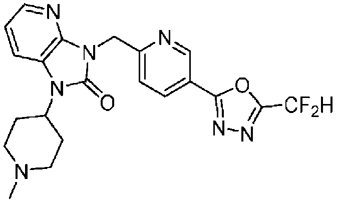 | 108 | 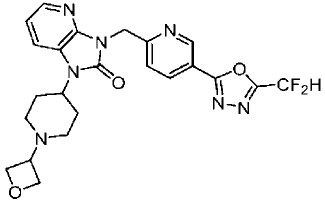 |
| 109 | 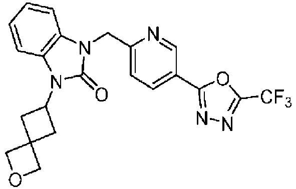 | 110 | 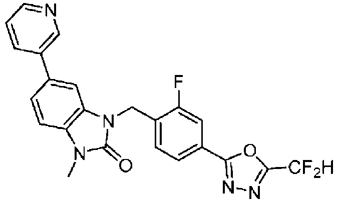 |
| 111 | 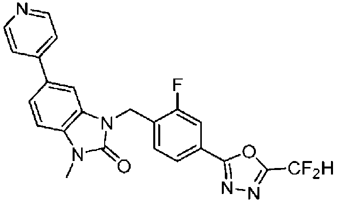 | 112 | 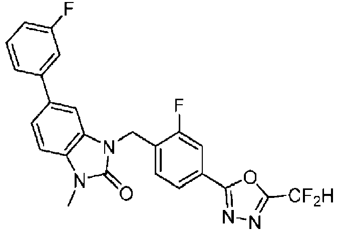 |
| 113 | 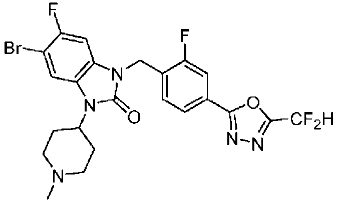 | 114 | 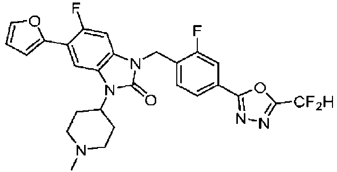 |
| 115 | 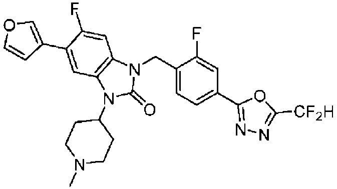 | 116 | 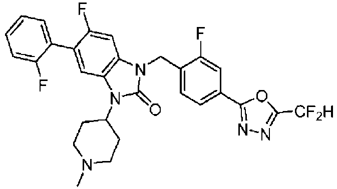 |
| 117 | 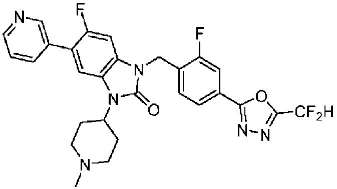 | 118 | 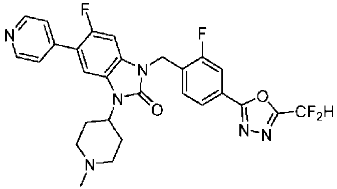 |
| 119 | 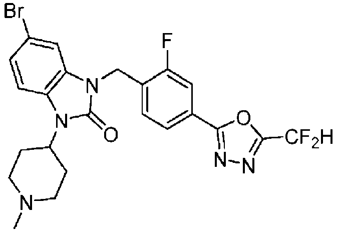 | 120 | 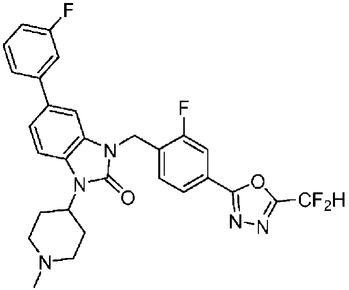 |
| 121 | 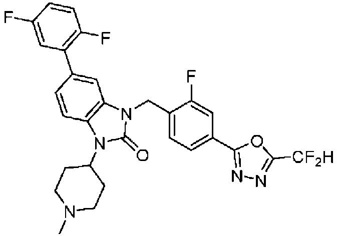 | 122 | 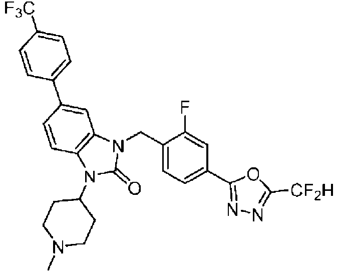 |
| 123 | 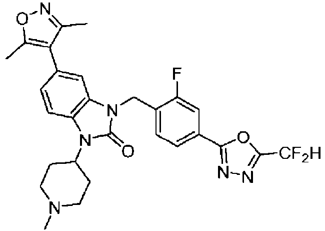 | 124 | 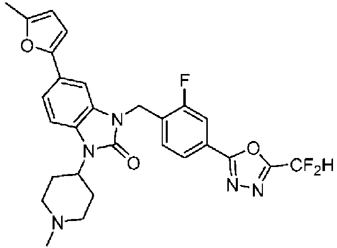 |
| 125 | 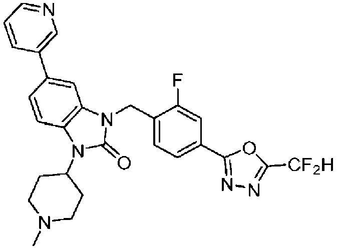 | 126 | 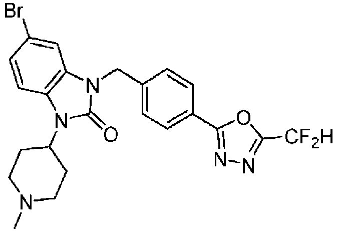 |
| 127 | 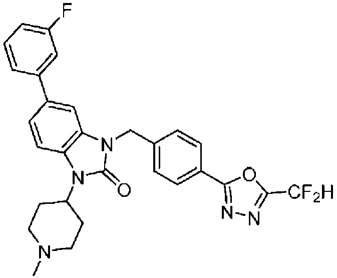 | 128 | 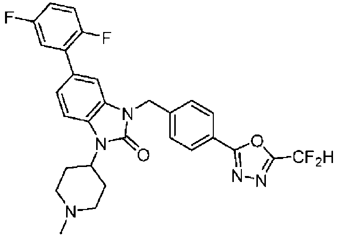 |
| 129 | 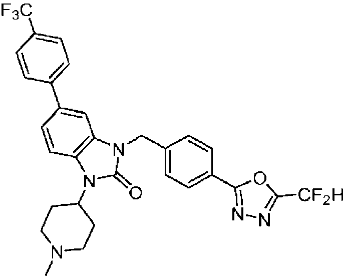 | 130 | 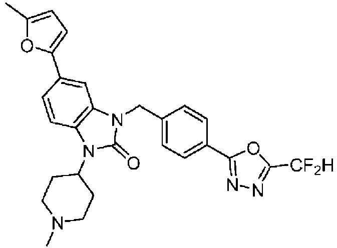 |
| 131 | 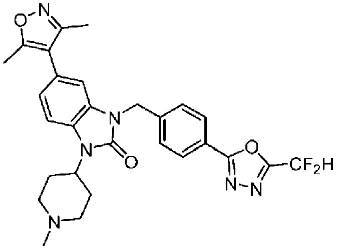 | 132 | 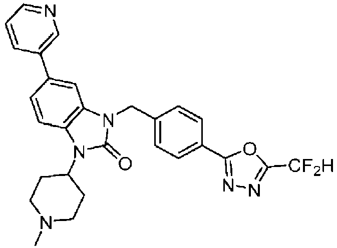 |
| 133 | 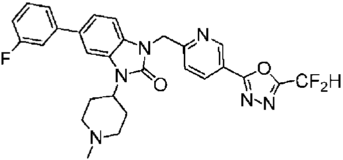 | 134 | 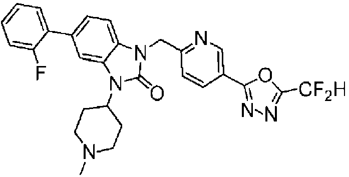 |
| 135 | 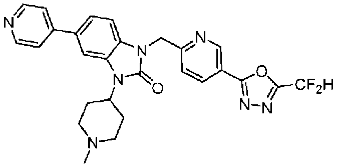 | 136 | 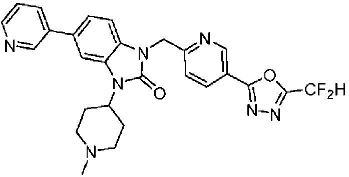 |
| 137 | 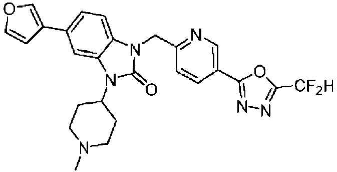 | 138 | 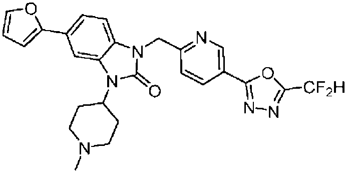 |
| 139 | 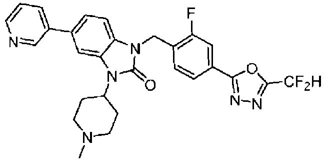 | 140 | 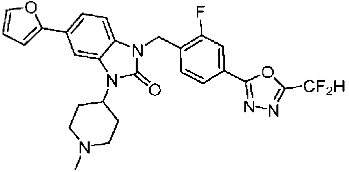 |
| 141 | 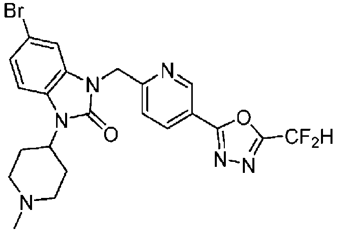 | 142 | 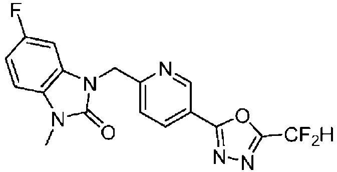 |
| 143 | 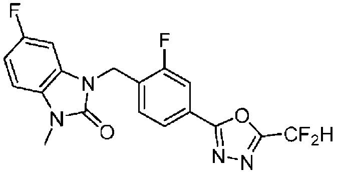 | 144 | 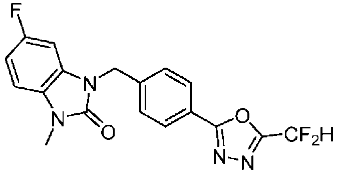 |
| 145 | 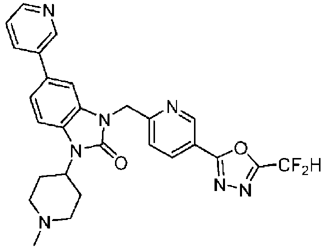 | 146 | 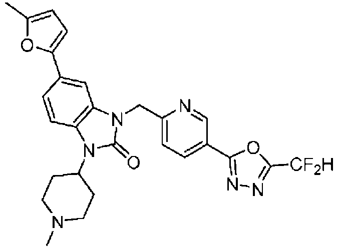 |
| 147 | 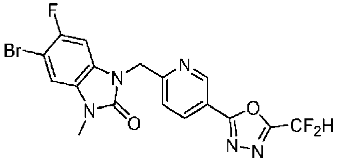 | 148 | 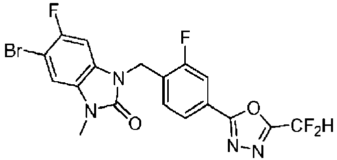 |
| 149 | 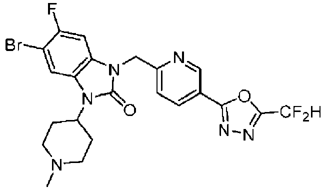 | 150 | 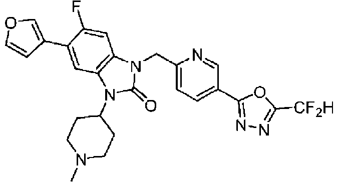 |
| 151 | 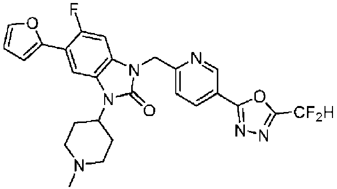 | 152 | 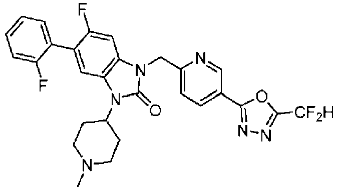 |
| 153 | 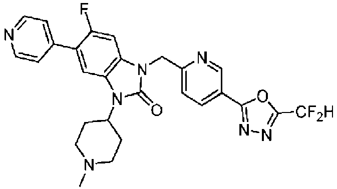 | 154 | 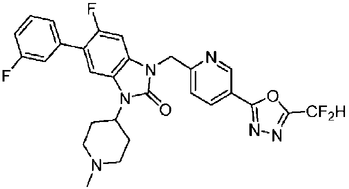 |
| 155 | 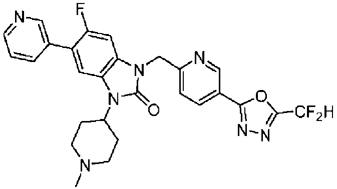 | 156 | 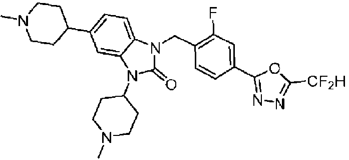 |
| 157 | 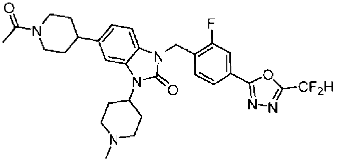 | 158 | 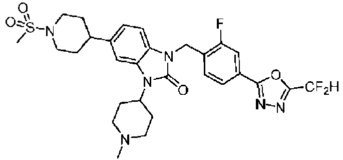 |
| 159 | 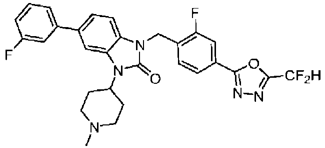 | 160 | 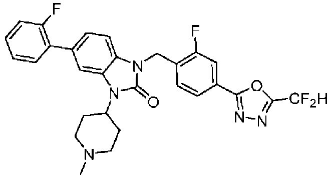 |
| 161 | 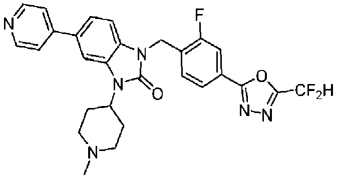 | 162 | 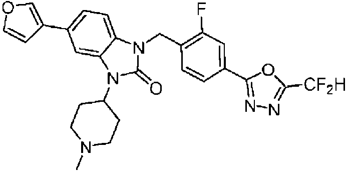 |
| 163 | 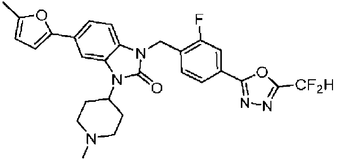 | 164 | 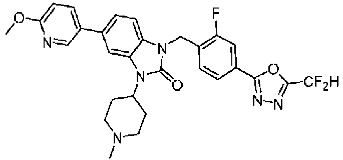 |
| 165 | 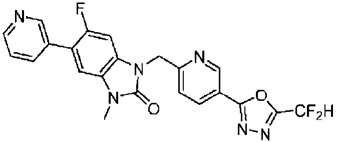 | 166 | 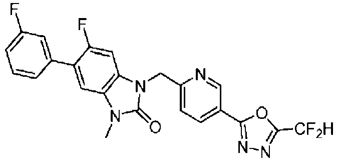 |
| 167 | 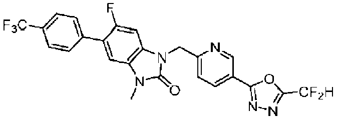 | 168 | 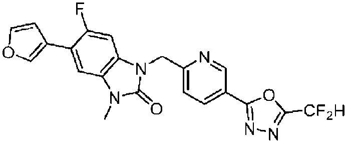 |
| 169 | 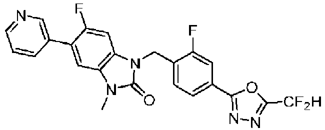 | 170 | 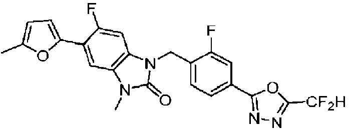 |
| 171 | 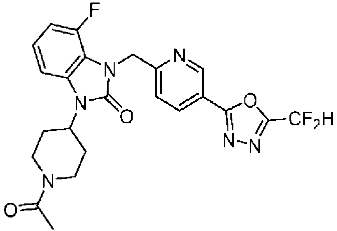 | 172 | 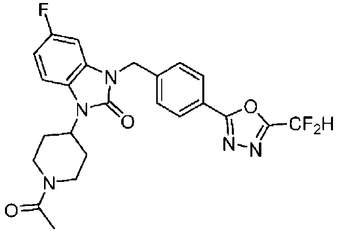 |
| 173 | 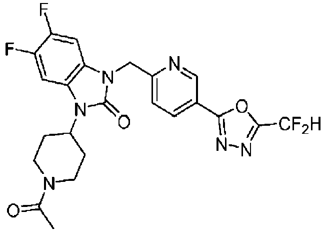 | 174 | 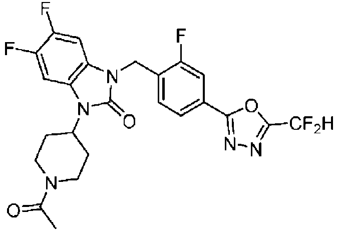 |
| 175 | 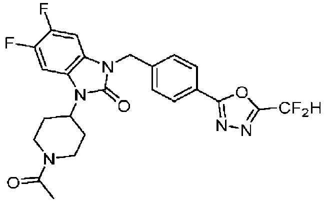 | 176 | 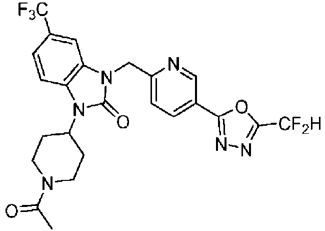 |
| 177 | 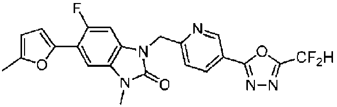 | 178 | 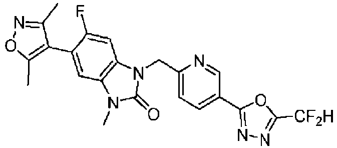 |
| 179 | 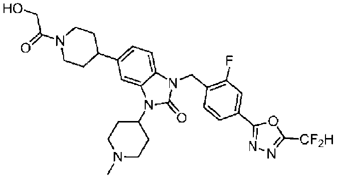 | 180 | 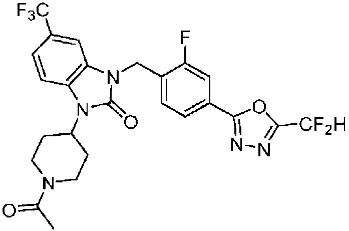 |
| 181 | 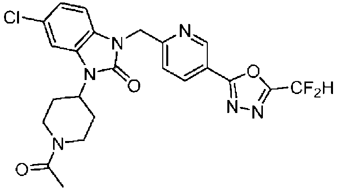 | 182 | 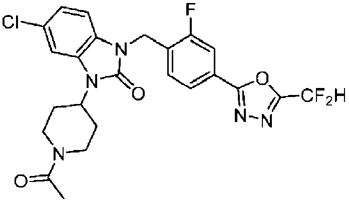 |
| 183 | 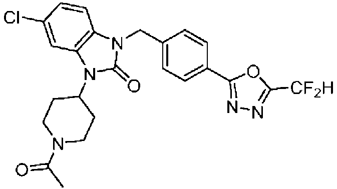 | 184 | 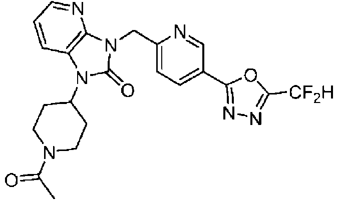 |
| 185 | 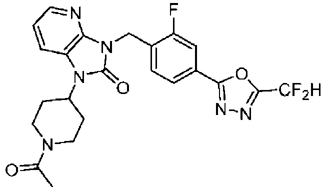 | 186 | 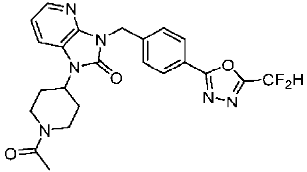 |
| 187 | 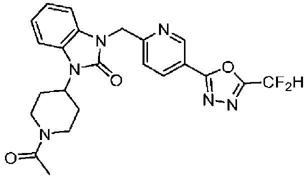 | 188 | 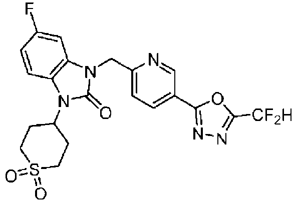 |
| 189 | 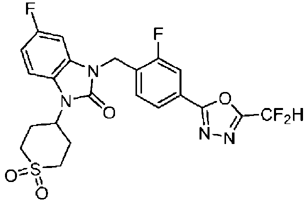 | 190 | 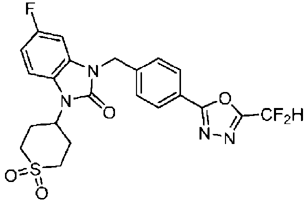 |
| 191 | 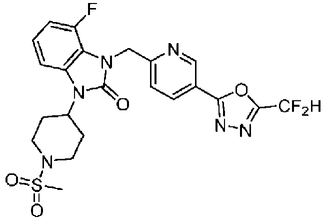 | 192 | 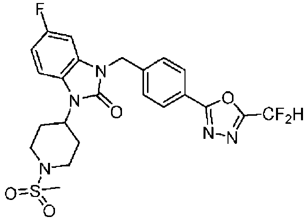 |
| 193 | 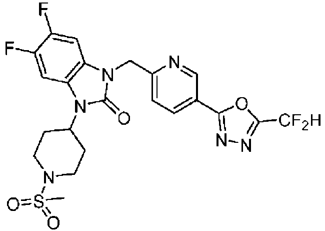 | 194 | 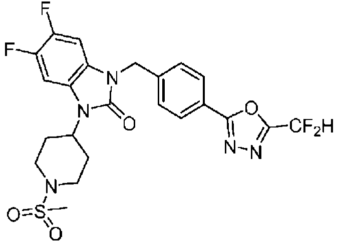 |
| 195 | 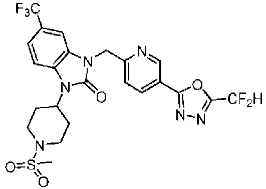 | 196 | 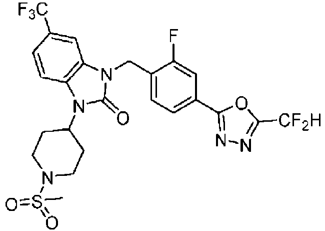 |
| 197 | 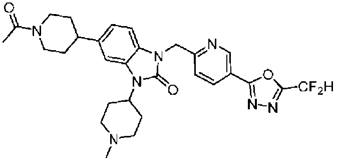 | 198 | 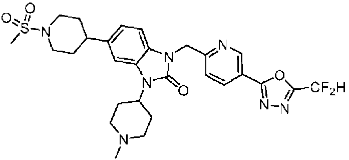 |
| 199 | 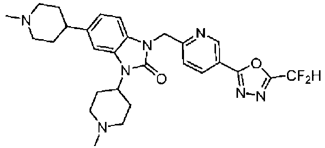 | 200 | 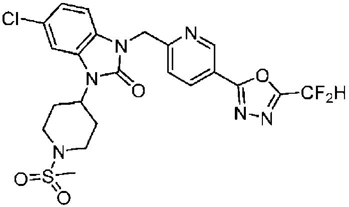 |
| 201 | 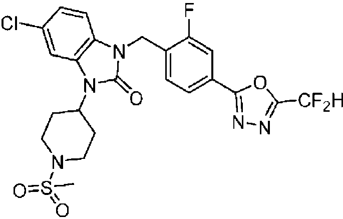 | 202 | 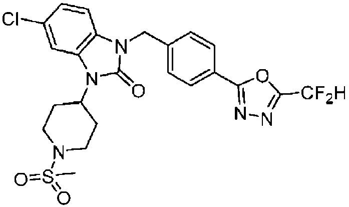 |
| 203 | 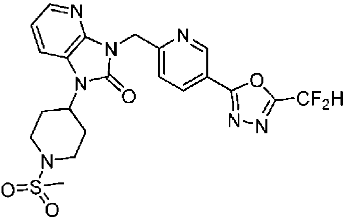 | 204 | 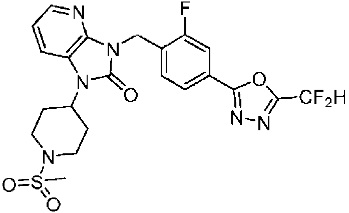 |
| 205 | 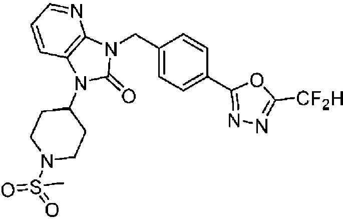 | 206 | 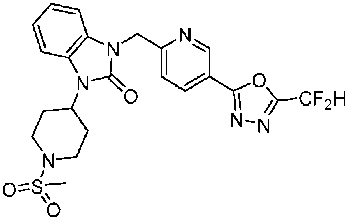 |
| 207 | 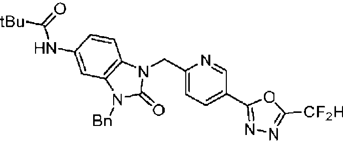 | 208 | 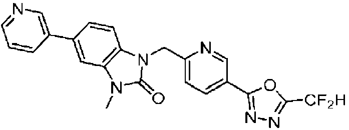 |
| 209 | 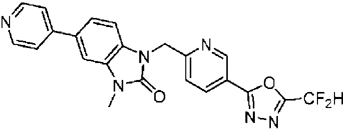 | 210 | 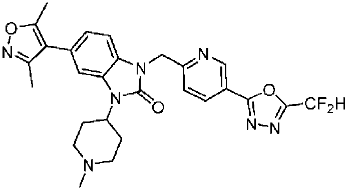 |
| 211 | 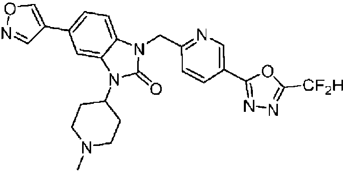 | 212 | 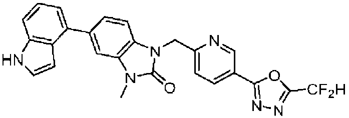 |
| 213 | 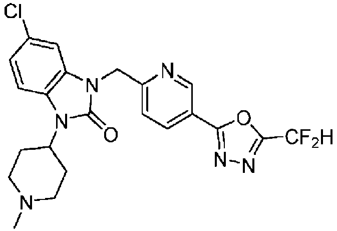 | 214 | 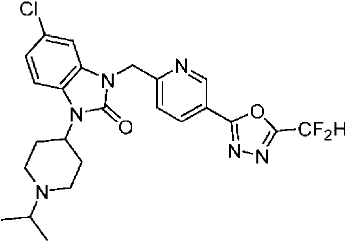 |
| 215 | 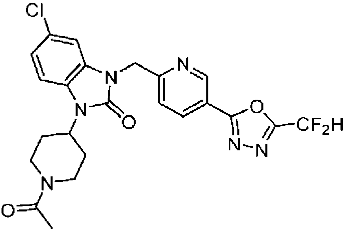 | 216 | 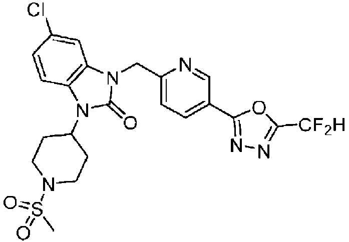 |
| 217 | 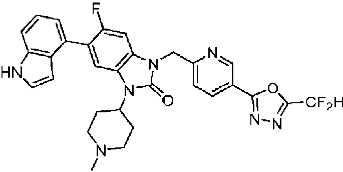 | 218 | 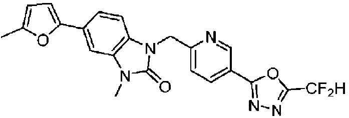 |
| 219 | 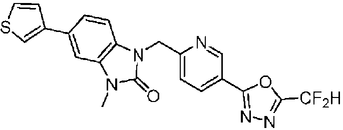 | 220 | 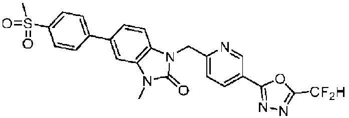 |
| 221 | 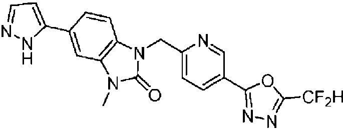 | 222 | 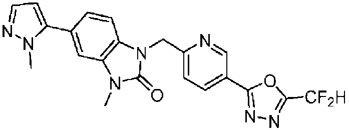 |
| 223 | 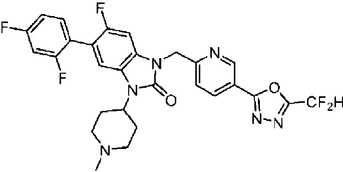 | 224 | 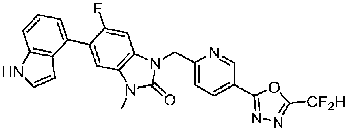 |
| 225 | 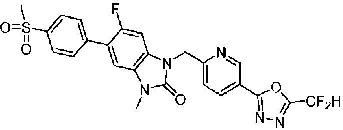 | 226 | 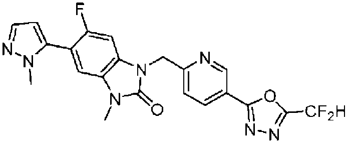 |
| 227 | 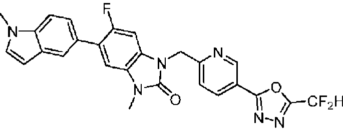 | 228 | 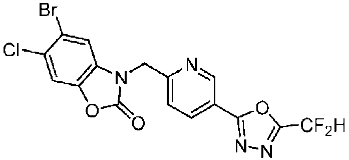 |
| 229 | 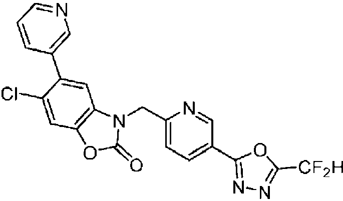 | 230 | 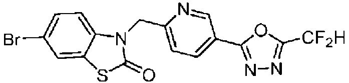 |
| 231 | 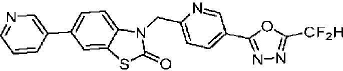 | 232 | 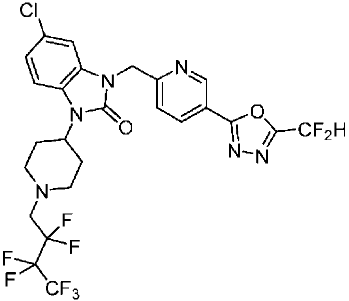 |
| 233 | 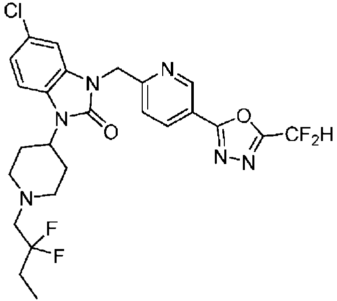 | 234 | 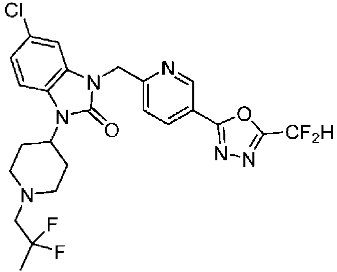 |
| 235 | 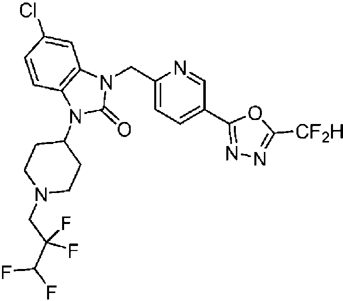 | 236 | 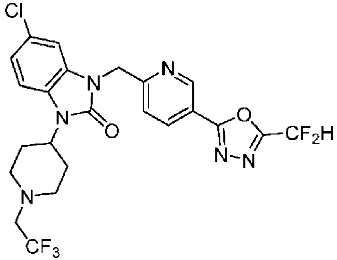 |
| 237 | 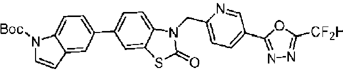 | 238 | 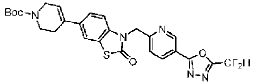 |
| 239 | 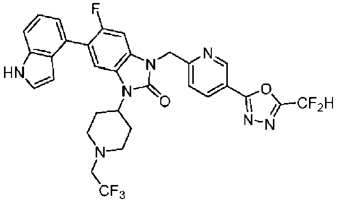 | 240 | 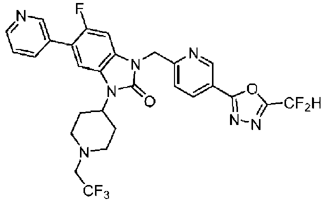 |
| 241 | 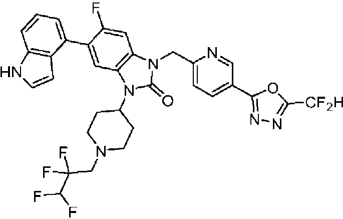 | 242 | 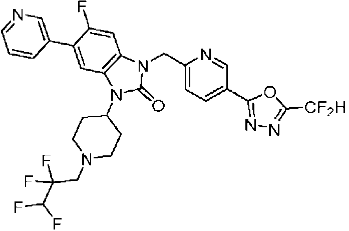 |
| 243 | 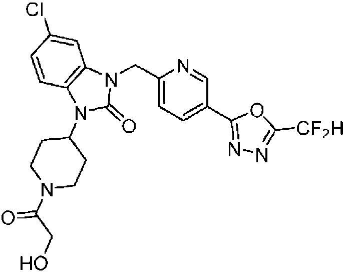 | 244 | 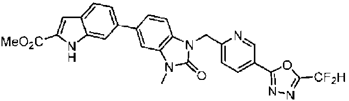 |
| 245 | 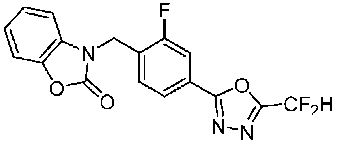 | 246 | 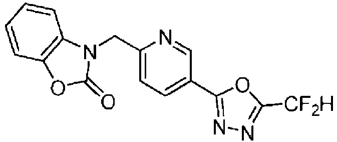 |
| 247 | 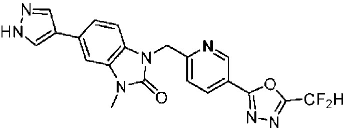 | 248 | 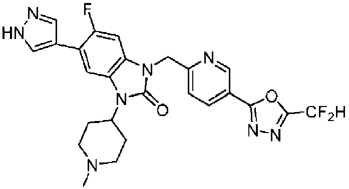 |
| 249 | 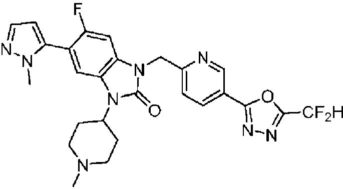 | 250 | 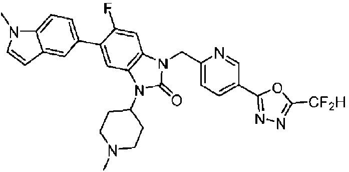 |
| 251 | 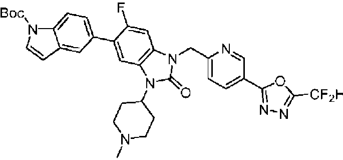 | 252 | 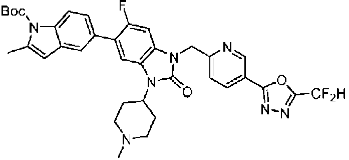 |
| 253 | 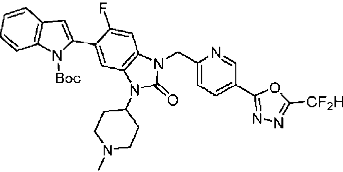 | 254 | 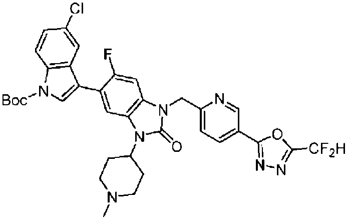 |
| 255 | 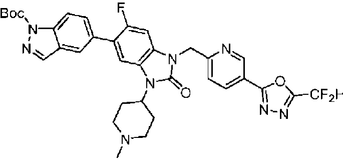 | 256 | 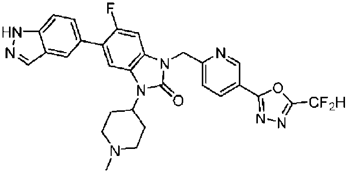 |
| 257 | 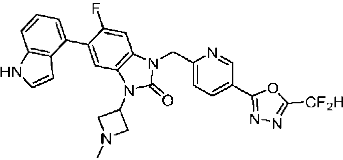 | 258 | 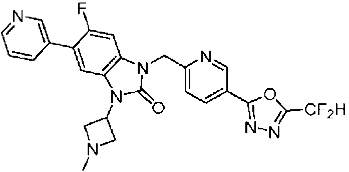 |
| 259 | 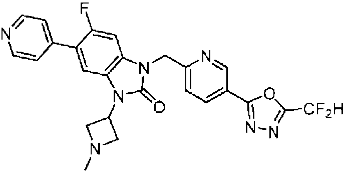 | 260 | 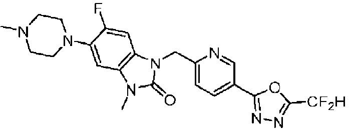 |
| 261 | 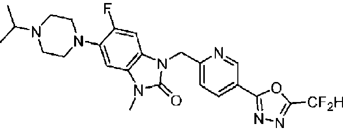 | 262 | 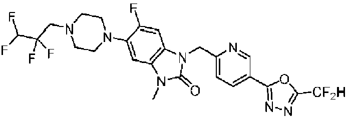 |
| 263 | 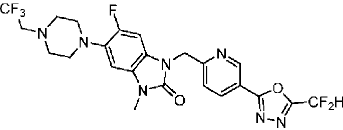 | 264 | 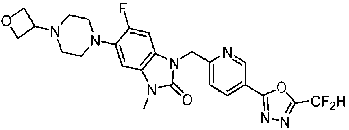 |
| 265 | 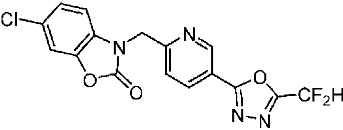 | 266 | 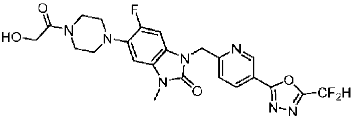 |
| 267 | 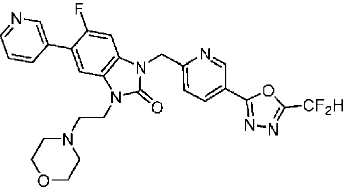 | 268 | 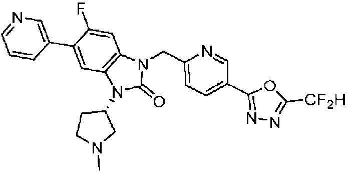 |
| 269 | 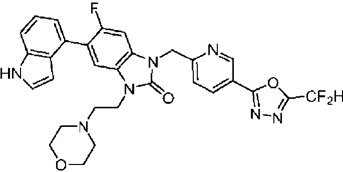 | 270 | 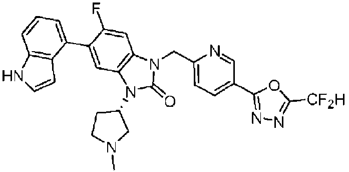 |
| 271 | 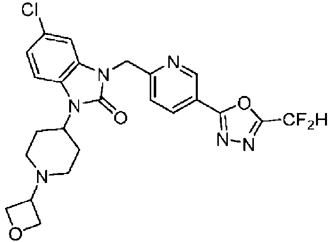 | 272 | 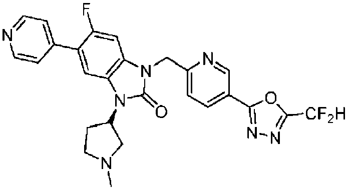 |
| 273 | 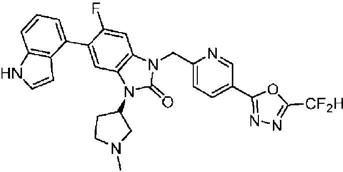 | 274 | 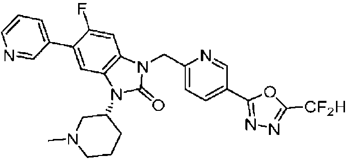 |
| 275 | 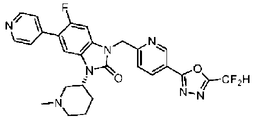 | 276 | 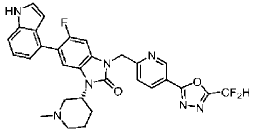 |
| 277 | 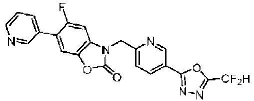 | 278 | 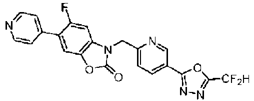 |
| 279 | 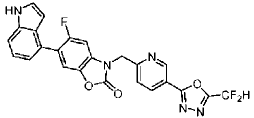 | 280 | 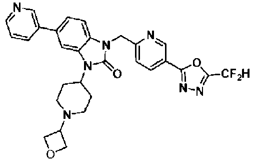 |
| 281 | 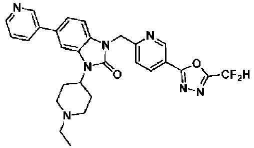 | 282 | 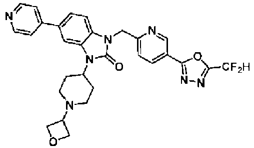 |
| 283 | 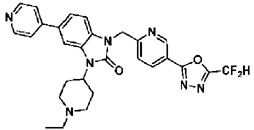 | 284 | 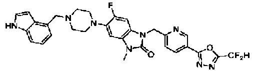 |
| 285 | 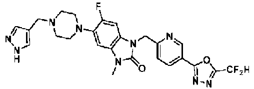 | 286 | 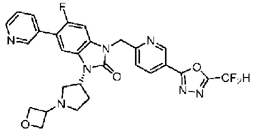 |
| 287 | 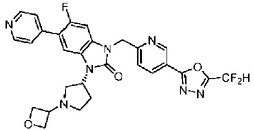 | 288 | 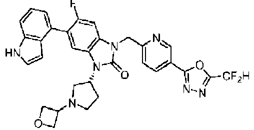 |
| 289 | 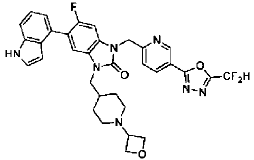 | 290 | 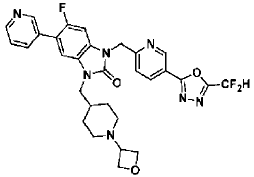 |
| 291 | 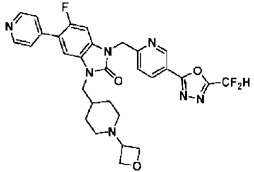 | 292 | 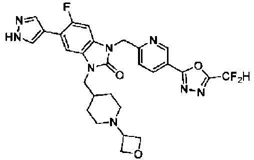 |
| 293 | 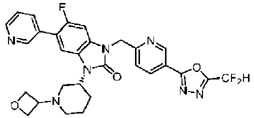 | 294 | 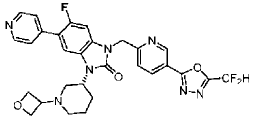 |
| 295 | 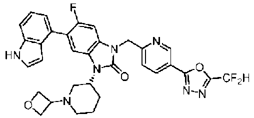 | 296 | 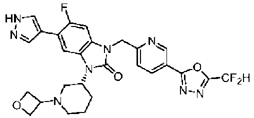 |
| 297 | 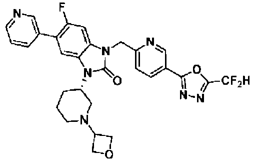 | 298 | 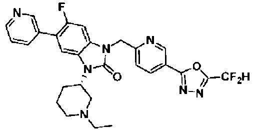 |
| 299 | 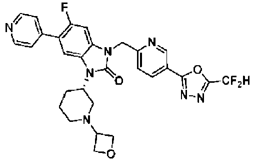 | 300 | 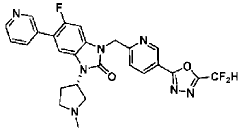 |
| 301 | 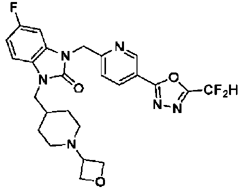 | 302 | 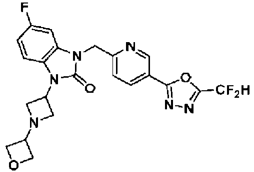 |
| 303 | 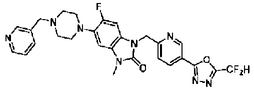 | 304 | 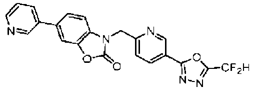 |
| 305 | 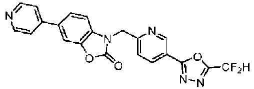 | 306 | 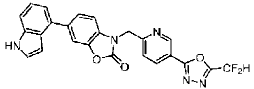 |
| 307 | 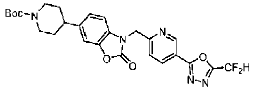 | 308 | 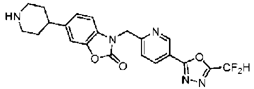 |
| 309 | 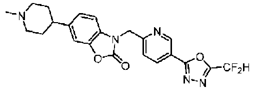 | 310 | 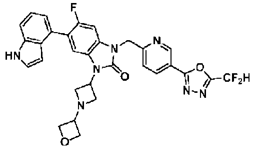 |
| 311 | 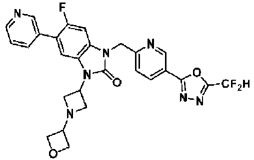 | 312 | 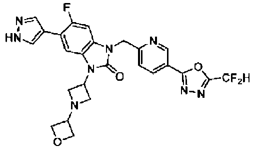 |
| 313 | 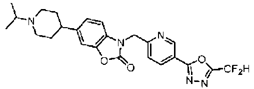 | 314 | 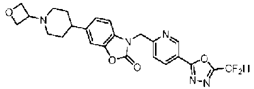 |
| 315 | 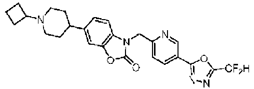 | 316 | 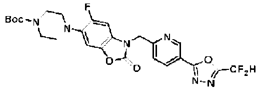 |
| 317 | 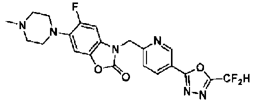 | 318 | 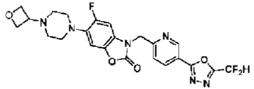 |
| 319 | 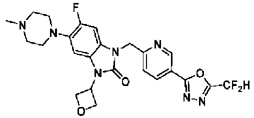 | 320 | 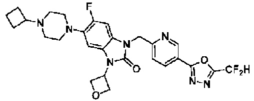 |
| 321 | 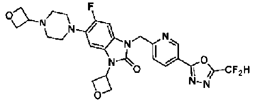 | 322 | 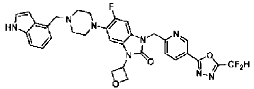 |
| 323 | 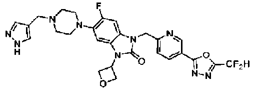 | 324 | 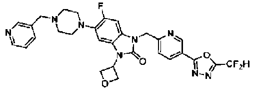 |
| 325 | 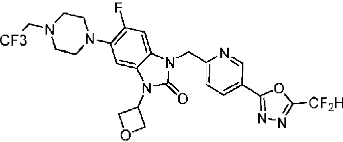 | 326 | 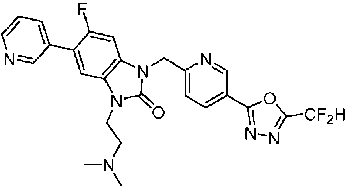 |
| 327 | 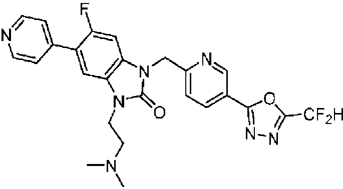 | 328 | 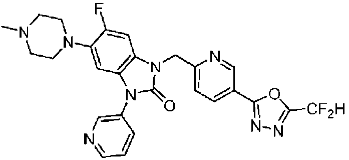 |
| 329 | 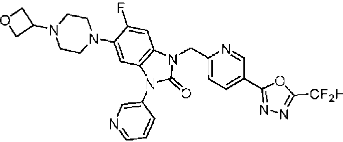 | 330 | 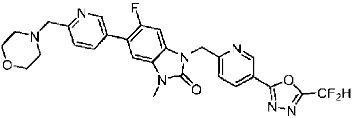 |
| 331 | 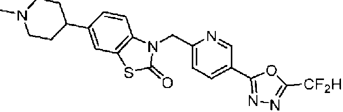 | 332 | 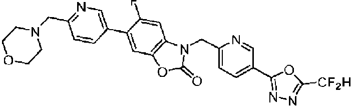 |
| 333 | 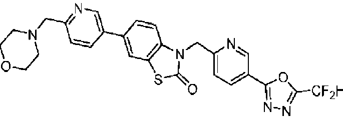 | 334 | 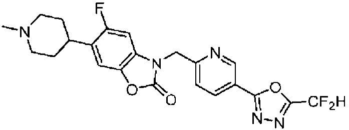 |
| 335 | 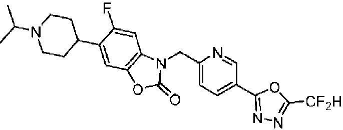 | 336 | 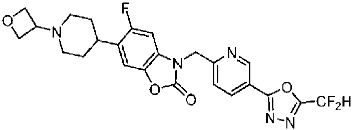 |
| 337 | 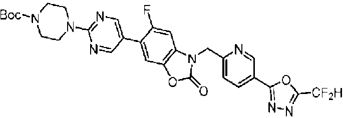 | 338 | 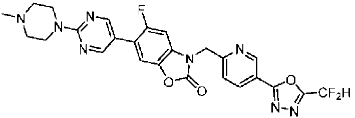 |
| 339 | 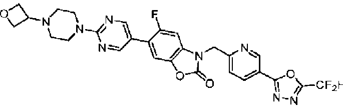 | 340 | 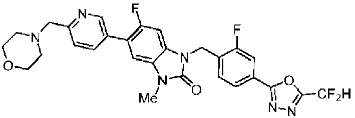 |
| 341 | 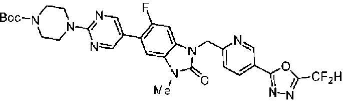 | 342 | 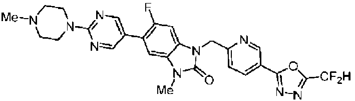 |
| 343 | 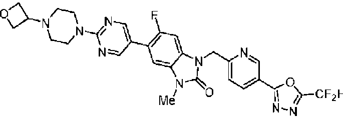 | 344 | 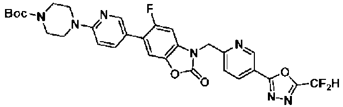 |
| 345 | 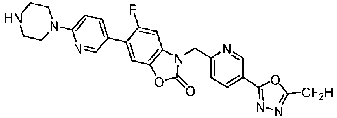 | 346 | 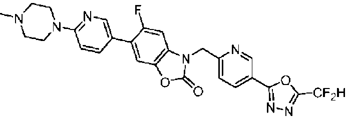 |
| 347 | 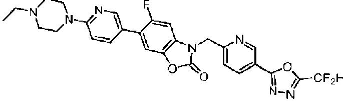 | 348 | 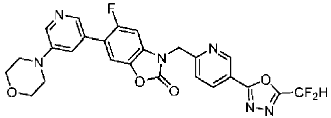 |
| 349 | 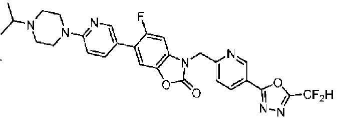 | 350 | 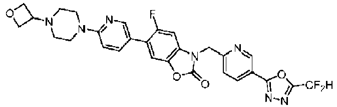 |
| 351 | 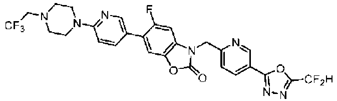 | 352 | 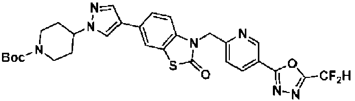 |
| 353 | 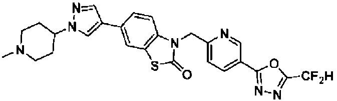 | 354 | 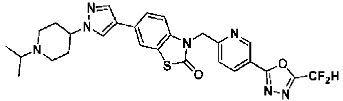 |
| 355 | 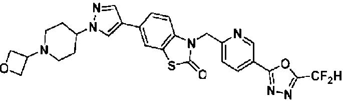 | 356 | 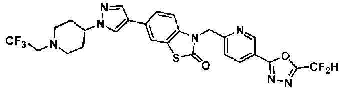 |
| 357 | 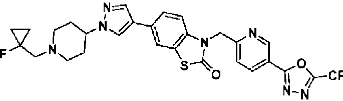 | 358 | 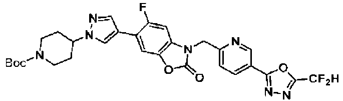 |
| 359 | 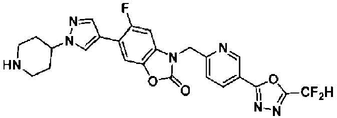 | 360 | 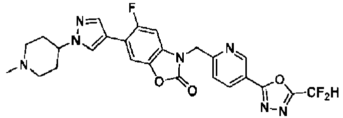 |
| 361 | 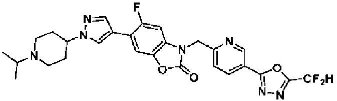 | 362 | 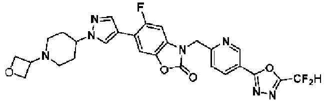 |
| 363 | 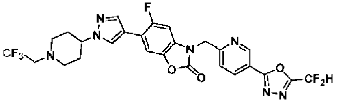 | 364 | 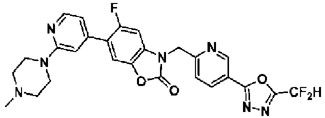 |
| 365 | 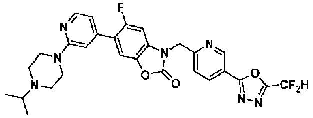 | 366 | 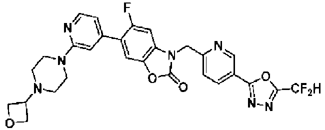 |
| 367 | 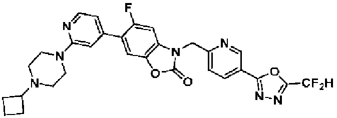 | 368 | 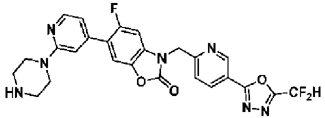 |

9. Composition pharmaceutique comprenant les composés représentés par la formule chimique I selon l'une quelconque des revendications 1 à 8, leurs stéréoisomères ou leurs sels pharmaceutiquement acceptables, en tant que principe actif.

10. Composition pharmaceutique selon la revendication 9, laquelle composition pharmaceutique est destinée à être utilisée dans la prévention ou le traitement de maladies associées à l'activité de l'histone désacétylase 6.

11. Composition pharmaceutique pour une utilisation selon la revendication 10, dans laquelle lesdites maladies associées à l'activité de l'histone désacétylase 6 sont au moins l'une choisie dans le groupe constitué par les maladies infectieuses, un néoplasme, une endocrinopathie, les maladies nutritionnelles et métaboliques, les troubles mentaux et comportementaux, les maladies neurologiques, les maladies annexielles oculaires et des yeux, les maladies circulatoires, les maladies respiratoires, les maladies digestives, les maladies de la peau et du tissu sous-cutané, les maladies du système musculosquelettique et du tissu conjonctif, et un tératisme, les déformités et une aberration chromosomique.

12. Composés représentés par la formule chimique I selon l'une quelconque des revendications 1 à 8, leurs stéréoisomères ou leurs sels pharmaceutiquement acceptables, pour une utilisation dans la prévention ou le traitement de maladies associées à l'activité de l'histone désacétylase 6.
